# EUROPEAN PATENT APPLICATION

(11) **EP 4 585 265 A2**
(43) Date of publication of application: **16.07.2025**
(21) Application number: 25179074.7
(22) Date of filing: 16.03.2022
(51) Int. Cl.: A61P 29/00

(54) **HETEROARYL INHIBITORS OF PLASMA KALLIKREIN**

(30) Priority: 17.03.2021 US 202163162468 P
(62) Divisional of application: 22716137.9
(71) Applicant: Takeda Pharmaceutical Company Limited, 1-1, Doshomachi 4-chome, Chuo-ku Osaka-Shi, Osaka (JP)
(72) Inventor: PAPAIOANNOU, Nikolaos, Lexington, 02421 (US); TRAVINS, Jeremy Mark, Lexington, 02421 (US); FINK, Sarah Jocelyn, Lexington, 02421 (US); ELLARD, John Mark, Saffron Walden, CB10 iXL (GB); RAE, Alastair, Saffron Walden, CB10 1XL (GB); SPENCER, Jonathan Andrew, Saffron Walden, CB10 iXL (GB); RANKIN, Stuart Shane, Saffron Walden, CB10 1XL (GB); CHAPMAN, Robert Stuart Laurie, Saffron Walden, CB10 1XL (GB)
(74) Representative: Hambleton, Bernadette Angelina

(57) **Abstract**

The present invention provides compounds and compositions thereof which are useful as inhibitors of plasma kallikrein and which exhibit desirable characteristics for the same.

## Description

This application claims the benefit of US 63/162,468, filed March 17, 2021.

### I. BACKGROUND OF THE INVENTION

Plasma Kallikrein (PKa) is a serine protease zymogen in blood that is converted to its catalytically active form by coagulation factor XIIa, and contributes to the innate inflammatory response and intrinsic cascade of blood coagulation. The mechanisms that lead to the activation of this pathway *in vivo* include interactions with polyphosphates released from activated platelets and deficiency of C1 inhibitor (C1-INH), the primary physiological inhibitor of PKa. PKa-mediated cleavage of high-molecular weight kininogen generates the potent vasodilator and pro-inflammatory nonapeptide bradykinin (BK), which activates the bradykinin 2 receptor. Subsequent cleavage of BK by carboxypeptidases generates des-Arg9-BK, which activates the B1 receptor. Both B1 and B2 receptors are expressed by vascular, glial, and neuronal cell types, with the highest levels of retinal expression detected in the ganglion cell layer and inner and outer nuclear layers. Activation of B1 and B2 receptors causes vasodilation and increases vascular permeability.

PKa is also associated with a number of disorders, such as hereditary angioedema (HAE), an autosomal dominant disease characterized by painful, unpredictable, recurrent attacks of inflammation affecting the hands, feet, face, abdomen, urogenital tract, and the larynx. Prevalence for HAE is uncertain but is estimated to be approximately 1 case per 50,000 persons without known differences among ethnic groups. HAE is caused by deficient (Type I) or dysfunctional (Type II) levels of C1-INH, which inhibits PKa, bradykinin, and other serine proteases in the blood. Individuals with hereditary angioedema (HAE) are deficient in C1-INH and consequently undergo excessive bradykinin generation, which in turn cause painful, debilitating, and potentially fatal swelling attacks. If left untreated, HAE can result in a mortality rate as high as 40% primarily due to upper airway obstruction.

### II. SUMMARY OF THE INVENTION

The present disclosure is based on, at least in part, the development of a number of compounds which bind to plasma kallikrein and effectively inhibit its activity. Accordingly, provided herein are compounds and uses thereof for targeting plasma kallikrein and/or treating plasma kallikrein-mediated diseases and disorders.

In some embodiments, the present invention provides a compound of Formula (I): 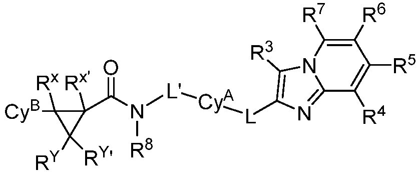 or a pharmaceutically acceptable salt thereof, wherein each of Cy^{A}, Cy^{B}, L, L', R^{x}, R^{x'}, R^{Y}, R^{Y'}, R³, R⁴, R⁵, R⁶, R⁷, and R⁸ is defined and described in classes and subclasses herein, both singly and in combination. In certain embodiments, the present invention provides compounds of Formulae (I)-(VI-c), as defined and described in classes and subclasses herein. In certain embodiments, the present invention provides novel intermediates and processes for preparing compounds disclosed herein. The disclosure also extends to pharmaceutical compositions comprising any one of the same, and use of compounds or compositions herein for treatment, in particular treatment of autoimmune disease, such as HAE or diabetic macular edema.

In some embodiments, the present invention also provides methods of using compounds of Formulae (I)-(VI-c).

Advantageously, the compounds of the present disclosure have therapeutic activity and/or adequate levels of bioavailability and/or adequate half-life for use as a therapeutic.

### III. DETAILED DESCRIPTION OF CERTAIN EMBODIMENTS

### A. Definitions

Compounds of this invention include those described generally above, and are further illustrated by the classes, subclasses, and species disclosed herein. As used herein, the following definitions shall apply unless otherwise indicated. For purposes of this invention, the chemical elements are identified in accordance with the Periodic Table of the Elements, CAS version, Handbook of Chemistry and Physics, 75th Ed. Additionally, general principles of organic chemistry are described in "Organic Chemistry", Thomas Sorrell, University Science Books, Sausalito: 1999, and "March's Advanced Organic Chemistry", 5th Ed., Ed.: Smith, M.B. and March, J., John Wiley & Sons, New York: 2001, the entire contents of which are hereby incorporated by reference.

The abbreviations used herein have their conventional meaning within the chemical and biological arts. The chemical structures and formulae set forth herein are constructed according to the standard rules of chemical valency known in the chemical arts.

The term "aliphatic" or "aliphatic group", as used herein, means a straight-chain (i.e., unbranched) or branched, substituted or unsubstituted hydrocarbon chain that is completely saturated or that contains one or more units of unsaturation, or a monocyclic hydrocarbon or bicyclic hydrocarbon that is completely saturated or that contains one or more units of unsaturation, but which is not aromatic (also referred to herein as "carbocyclyl," "cycloaliphatic" or "cycloalkyl"), that has a single point of attachment to the rest of the molecule. Unless otherwise specified, aliphatic groups contain 1-6 aliphatic carbon atoms. In some embodiments, aliphatic groups contain 1-5 aliphatic carbon atoms. In some embodiments, aliphatic groups contain 1-4 aliphatic carbon atoms. In some embodiments, aliphatic groups contain 1-3 aliphatic carbon atoms, and in yet other embodiments, aliphatic groups contain 1-2 aliphatic carbon atoms. In some embodiments, "cycloaliphatic" (or "carbocyclyl" or "cycloalkyl") refers to a monocyclic C₃-C₇ hydrocarbon that is completely saturated or that contains one or more units of unsaturation, but which is not aromatic, that has a single point of attachment to the rest of the molecule. Suitable aliphatic groups include, but are not limited to, linear or branched, substituted or unsubstituted alkyl, alkenyl, alkynyl groups and hybrids thereof such as (cycloalkyl)alkyl, (cycloalkenyl)alkyl or (cycloalkyl)alkenyl.

The term "heteroatom" means one or more of oxygen, sulfur, nitrogen, phosphorus, or silicon (including, any oxidized form of nitrogen, sulfur, phosphorus, or silicon; the quaternized form of any basic nitrogen or; a substitutable nitrogen of a heterocyclic ring, for example N (as in 3,4-dihydro-2H-pyrrolyl), NH (as in pyrrolidinyl) or NR⁺ (as in N-substituted pyrrolidinyl)).

The term "unsaturated," as used herein, means that a moiety has one or more units of unsaturation.

The term "alkylene" refers to a bivalent alkyl group. An "alkylene chain" is a polymethylene group, i.e., -(CH₂)ₙ-, wherein n is a positive integer, preferably from 1 to 6, from 1 to 4, from 1 to 3, from 1 to 2, or from 2 to 3. A substituted alkylene chain is a polymethylene group in which one or more methylene hydrogen atoms are replaced with a substituent. Suitable substituents include those described below for a substituted aliphatic group.

The term "halogen" means F, Cl, Br, or I.

The term "aryl" refers to monocyclic and bicyclic ring systems having a total of five to 10 ring members, wherein at least one ring in the system is aromatic and wherein each ring in the system contains three to seven ring members. The term "aryl" may be used interchangeably with the term "aryl ring". In some embodiments, an 8-10 membered bicyclic aryl group is an optionally substituted naphthyl ring. In certain embodiments of the present invention, "aryl" refers to an aromatic ring system which includes, but not limited to, phenyl, biphenyl, naphthyl, anthracyl and the like, which may bear one or more substituents. Also included within the scope of the term "aryl," as it is used herein, is a group in which an aromatic ring is fused to one or more non-aromatic rings, such as indanyl, phthalimidyl, naphthimidyl, phenanthridinyl, or tetrahydronaphthyl, and the like.

The terms "heteroaryl" and "heteroar-" refer to groups having 5 to 10 ring atoms, preferably 5, 6, or 9 ring atoms; having 6, 10, or 14 π electrons shared in a cyclic array; and having, in addition to carbon atoms, from one to five heteroatoms. Heteroaryl groups include, without limitation, thienyl, furanyl, pyrrolyl, imidazolyl, pyrazolyl, triazolyl, tetrazolyl, oxazolyl, isoxazolyl, oxadiazolyl, thiazolyl, isothiazolyl, thiadiazolyl, pyridyl, pyridazinyl, pyrimidinyl, pyrazinyl, indolizinyl, purinyl, naphthyridinyl, and pteridinyl. The terms "heteroaryl" and "heteroar-", as used herein, also include groups in which a heteroaromatic ring is fused to one or more aryl, cycloaliphatic, or heterocyclyl rings, where the radical or point of attachment is on the heteroaromatic ring (or in the case of a bivalent fused heteroarylene ring system, at least one radical or point of attachment is on a heteroaromatic ring). Nonlimiting examples include indolyl, isoindolyl, benzothienyl, benzofuranyl, dibenzofuranyl, indazolyl, benzimidazolyl, benzthiazolyl, quinolyl, isoquinolyl, cinnolinyl, phthalazinyl, quinazolinyl, quinoxalinyl, 4H-quinolizinyl, carbazolyl, acridinyl, phenazinyl, phenothiazinyl, phenoxazinyl, tetrahydroquinolinyl, tetrahydroisoquinolinyl, and pyrido[2,3-b]-1,4-oxazin-3(4H)-one. A heteroaryl group may be mono- or bicyclic. The term "heteroaryl" may be used interchangeably with the terms "heteroaryl ring," "heteroaryl group," or "heteroaromatic," any of which terms include rings that are optionally substituted.

As used herein, the terms "heterocyclyl," "heterocyclic radical," and "heterocyclic ring" are used interchangeably and refer to a stable 5- to 7-membered monocyclic or 7-10-membered bicyclic heterocyclic moiety that is either saturated or partially unsaturated, and having, in addition to carbon atoms, one or more, preferably one to four, heteroatoms, as defined above. When used in this context in reference to a ring atom, the term "nitrogen" includes a substituted nitrogen. As an example, in a saturated or partially unsaturated ring having 0-3 heteroatoms selected from oxygen, sulfur or nitrogen, the nitrogen may be N (as in 3,4-dihydro-2H-pyrrolyl), NH (as in pyrrolidinyl), or ⁺NR (as in *N-*substituted pyrrolidinyl).

A heterocyclic ring can be attached to its pendant group at any heteroatom or carbon atom that results in a stable structure and any of the ring atoms can be optionally substituted. Examples of such saturated or partially unsaturated heterocyclic radicals include, without limitation, tetrahydrofuranyl, tetrahydrothiophenyl pyrrolidinyl, piperidinyl, pyrrolinyl, tetrahydroquinolinyl, tetrahydroisoquinolinyl, decahydroquinolinyl, oxazolidinyl, piperazinyl, dioxanyl, dioxolanyl, diazepinyl, oxazepinyl, thiazepinyl, morpholinyl, and quinuclidinyl. The terms "heterocyclyl," "heterocyclyl ring," "heterocyclic group," "heterocyclic moiety," and "heterocyclic radical," are used interchangeably herein, and also include groups in which a heterocyclyl ring is fused to one or more aryl, heteroaryl, or cycloaliphatic rings, such as indolinyl, 3H-indolyl, chromanyl, phenanthridinyl, or tetrahydroquinolinyl, where the radical or point of attachment is on the heterocyclyl ring. A heterocyclyl group may be mono- or bicyclic. The term "heterocyclylalkyl" refers to an alkyl group substituted by a heterocyclyl, wherein the alkyl and heterocyclyl portions independently are optionally substituted.

As used herein, the term "partially unsaturated" refers to a ring moiety that includes at least one double or triple bond. The term "partially unsaturated" is intended to encompass rings having multiple sites of unsaturation, but is not intended to include aryl or heteroaryl moieties, as herein defined.

As used herein and unless otherwise specified, the suffix "-ene" is used to describe a bivalent group. Thus, any of the terms above can be modified with the suffix "-ene" to describe a bivalent version of that moiety. For example, a bivalent carbocycle is "carbocycylene", a bivalent aryl ring is "arylene", a bivalent benzene ring is "phenylene", a bivalent heterocycle is "heterocyclylene", a bivalent heteroaryl ring is "heteroarylene", a bivalent alkyl chain is "alkylene", a bivalent alkenyl chain is "alkenylene", a bivalent alkynyl chain is "alkynylene", and so forth.

As described herein, compounds of the invention may, when specified, contain "optionally substituted" moieties. In general, the term "substituted," whether preceded by the term "optionally" or not, means that one or more hydrogens of the designated moiety are replaced with a suitable substituent. "Substituted" applies to one or more hydrogens that are either explicit or implicit from the structure (e.g., 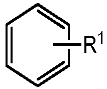 refers to at least 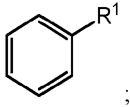 and 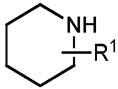 refers to at least 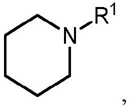 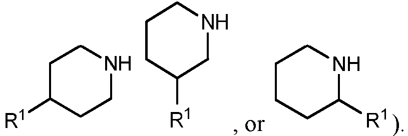 In addition, in a polycyclic ring system, substituents may, unless otherwise indicated, replace a hydrogen on any individual ring (e.g., 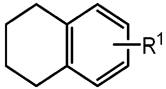 refers to at least 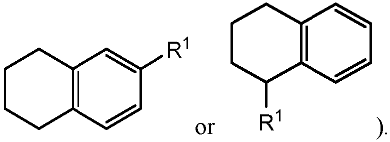 Unless otherwise indicated, an "optionally substituted" group may have a suitable substituent at each substitutable position of the group, and when more than one position in any given structure may be substituted with more than one substituent selected from a specified group, the substituent may be either the same or different at every position. Combinations of substituents envisioned by this invention are preferably those that result in the formation of stable or chemically feasible compounds. The term "stable," as used herein, refers to compounds that are not substantially altered when subjected to conditions to allow for their production, detection, and, in certain embodiments, their recovery, purification, and use for one or more of the purposes disclosed herein.

Suitable monovalent substituents on a substitutable carbon atom of an "optionally substituted" group are independently halogen; -(CH₂)₀₋₄R°; -(CH₂)₀₋₄OR°; -O(CH₂)₀₋₄R°, -O(CH₂)₀₋₄C(O)OR°; -O(CH₂)₀₋₄OR°; -(CH₂)₀₋₄CH(OR°)₂; -(CH₂)₀₋₄SR°; -(CH₂)₀₋₄Ph, which may be substituted with R°; -(CH₂)₀₋₄O(CH₂)₀₋₁Ph which may be substituted with R°; -CH=CHPh, which may be substituted with R°; -(CH₂)₀₋₄O(CH₂)₀₋₁-pyridyl which may be substituted with R°; -NO₂; -CN; - N₃; -(CH₂)₀₋₄N(R°)₂; -(CH₂)₀₋₄N(R°)C(O)R°; -N(R°)C(S)R°; -(CH₂)₀₋₄N(R°)C(O)NR°₂; - N(R°)C(S)NR°₂; -(CH₂)₀₋₄N(R°)C(O)OR°; -N(R°)N(R°)C(O)R°; -N(R°)N(R°)C(O)NR°₂; - N(R°)N(R°)C(O)OR°; -(CH₂)₀₋₄C(O)R°; -C(S)R°; -(CH₂)₀₋₄C(O)OR°; -(CH₂)₀₋₄C(O)SR°; - (CH₂)₀₋₄C(O)OSiR°₃; -(CH₂)₀₋₄OC(O)R°; -OC(O)(CH₂)₀₋₄SR°, -SC(S)SR°; -(CH₂)₀₋₄SC(O)R°; -(CH₂)₀₋₄C(O)NR°₂; -C(S)NR°₂; -C(S)SR°; -SC(S)SR°, -(CH₂)₀₋₄OC(O)NR°₂; -C(O)N(OR°)R°; -C(O)C(O)R°; - C(O)CH₂C(O)R°; -C(NOR°)R°; -(CH₂)₀₋₄SSR°; -(CH₂)₀₋₄S(O)₂R°; -(CH₂)₀₋₄S(O)₂OR°; -(CH₂)₀₋₄OS(O)₂R°; -S(O)₂NR°₂; - (CH₂)₀₋₄S(O)R°; -N(R°)S(O)₂NR°₂; -N(R°)S(O)₂R°; -N(OR°)R°; - C(NH)NR°₂; -P(O)₂R°; -P(O)R°₂; -OP(O)R°₂; -OP(O)(OR°)₂; SiR°₃; -(C₁₋₄ straight or branched alkylene)O-N(R°)₂; or -(C₁₋₄ straight or branched alkylene)C(O)O-N(R°)₂, wherein each R° may be substituted as defined below and is independently hydrogen, C₁₋₆ aliphatic, -CH₂Ph, -O(CH₂)₀₋₁Ph, - CH₂-(5-6 membered heteroaryl ring), or a 5-6 membered saturated, partially unsaturated, or aryl ring having 0-4 heteroatoms independently selected from nitrogen, oxygen, or sulfur, or, notwithstanding the definition above, two independent occurrences of R°, taken together with their intervening atom(s), form a 3-12 membered saturated, partially unsaturated, or aryl mono- or bicyclic ring having 0-4 heteroatoms independently selected from nitrogen, oxygen, or sulfur, which may be substituted as defined below.

Suitable monovalent substituents on R° (or the ring formed by taking two independent occurrences of R° together with their intervening atoms), are independently halogen, -(CH₂)₀₋₂R^{•}, - (haloR•), -(CH₂)₀₋₂OH, -(CH₂)₀₋₂OR^{•}, -(CH₂)₀₋₂CH(OR^{•})₂; -O(haloR•), -CN, -N₃, -(CH₂)₀₋₂C(O)R^{•}, - (CH₂)₀₋₂C(O)OH, -(CH₂)₀₋₂C(O)OR^{•}, -(CH₂)₀₋₂SR^{•}, -(CH₂)₀₋₂SH, -(CH₂)₀₋₂NH₂, -(CH₂)₀₋₂NHR^{•}, - (CH₂)₀₋₂NR^{•}₂, -NO₂, -SiR^{•}₃, -OSiR^{•}₃, -C(O)SR^{•}, -(C₁₋₄ straight or branched alkylene)C(O)OR^{•}, or - SSR^{•} wherein each R^{•} is unsubstituted or where preceded by "halo" is substituted only with one or more halogens, and is independently selected from C₁₋₄ aliphatic, -CH₂Ph, -O(CH₂)₀₋₁Ph, or a 5-6-membered saturated, partially unsaturated, or aryl ring having 0-4 heteroatoms independently selected from nitrogen, oxygen, or sulfur. Suitable divalent substituents on a saturated carbon atom of R° include =O and =S.

Suitable divalent substituents on a saturated carbon atom of an "optionally substituted" group include the following: =O, =S, =NNR^{#}₂, =NNHC(O)R^{#}, =NNHC(O)OR^{#}, =NNHS(O)₂R^{#}, =NR^{#}, =NOR^{#}, -O(C(R^{#}₂))₂₋₃O-, or -S(C(R^{#}₂))₂₋₃S-, wherein each independent occurrence of R^{#} is selected from hydrogen, C₁₋₆ aliphatic which may be substituted as defined below, or an unsubstituted 5-6 membered saturated, partially unsaturated, or aryl ring having 0-4 heteroatoms independently selected from nitrogen, oxygen, or sulfur. Suitable divalent substituents that are bound to vicinal substitutable carbons of an "optionally substituted" group include: -O(CR^{#}₂)₂₋₃O-, wherein each independent occurrence of R^{#} is selected from hydrogen, C₁₋₆ aliphatic which may be substituted as defined below, or an unsubstituted 5-6-membered saturated, partially unsaturated, or aryl ring having 0-4 heteroatoms independently selected from nitrogen, oxygen, or sulfur.

Suitable substituents on the aliphatic group of R^{#} include halogen, -R^{•}**,** - (haloR^{•}), -OH, - **OR**^{•}**,** -O(haloR^{•}), -CN, -C(O)OH, -C(O)OR^{•}, -NH₂, -NHR^{•}, -NR^{•}₂, or -NO₂, wherein each R^{•} is unsubstituted or where preceded by "halo" is substituted only with one or more halogens, and is independently C₁₋₄ aliphatic, -CH₂Ph, -O(CH₂)₀₋₁Ph, or a 5-6 membered saturated, partially unsaturated, or aryl ring having 0-4 heteroatoms independently selected from nitrogen, oxygen, or sulfur.

Suitable substituents on a substitutable nitrogen of an "optionally substituted" group include -R^{†}, -NR^{†}₂, -C(O)R^{†}, -C(O)OR^{†}, -C(O)C(O)R^{†}, -C(O)CH₂C(O)R^{†}, -S(O)₂R^{†}, -S(O)₂NR^{†}₂, -C(S)NR^{†}₂, - C(NH)NR^{†}₂, or -N(R^{†})S(O)₂R^{†}; wherein each R^{†} is independently hydrogen, C₁₋₆ aliphatic which may be substituted as defined below, unsubstituted -OPh, or an unsubstituted 5-6-membered saturated, partially unsaturated, or aryl ring having 0-4 heteroatoms independently selected from nitrogen, oxygen, or sulfur, or, notwithstanding the definition above, two independent occurrences of R^{t}, taken together with their intervening atom(s) form an unsubstituted 3-12 membered saturated, partially unsaturated, or aryl mono- or bicyclic ring having 0-4 heteroatoms independently selected from nitrogen, oxygen, or sulfur.

Suitable substituents on the aliphatic group of R^{†} are independently halogen, **-R^{•}, -** (haloR**^{•}**), -OH, **-OR^{•},** -O(haloR**^{•}**), -CN, -C(O)OH, -C(O)OR**^{•}**, -NH₂, -NHR**^{•}**, -NR**^{•}**₂, or -NO₂, wherein each R**^{•}** is unsubstituted or where preceded by "halo" is substituted only with one or more halogens, and is independently C₁₋₄ aliphatic, -CH₂Ph, -O(CH₂)₀₋₁Ph, or a 5-6-membered saturated, partially unsaturated, or aryl ring having 0-4 heteroatoms independently selected from nitrogen, oxygen, or sulfur.

As used herein, the term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. For example, S. M. Berge et al., describe pharmaceutically acceptable salts in detail in J. Pharmaceutical Sciences, 1977, 66, 1-19, incorporated herein by reference.

In certain embodiments, the neutral forms of the compounds are regenerated by contacting the salt with a base or acid and isolating the parent compound in the conventional manner. In some embodiments, the parent form of the compound differs from the various salt forms in certain physical properties, such as solubility in polar solvents.

Unless otherwise stated, structures depicted herein are also meant to include all isomeric (e.g., enantiomeric, diastereomeric, and geometric (or conformational)) forms of the structure; for example, the R and S configurations for each asymmetric center, Z and E double bond isomers, and Z and E conformational isomers. Therefore, single stereochemical isomers as well as enantiomeric, diastereomeric, and geometric (or conformational) mixtures of the present compounds are within the scope of the invention. Unless otherwise stated, all tautomeric forms of the compounds of the invention are within the scope of the invention. Additionally, unless otherwise stated, structures depicted herein are also meant to include compounds that differ only in the presence of one or more isotopically enriched atoms. For example, compounds having the present structures including the replacement of hydrogen by deuterium or tritium, or the replacement of a carbon by a ¹³C- or ¹⁴C-enriched carbon are within the scope of this invention. Such compounds are useful, for example, as analytical tools, as probes in biological assays, or as therapeutic agents in accordance with the present invention. In some embodiments, compounds of the present disclosure are provided as a single enantiomer or single diastereoisomer. Single enantiomer refers to an enantiomeric excess of 80% or more, such as 81, 82, 83, 84, 85, 86, 87, 88, 89, 90, 91, 92, 93, 94, 95, 96, 97, 98 or 99%. Single diastereoisomer excess refers to an excess of 80% or more, for example 81, 82, 83, 84, 85, 86, 87, 88, 89, 90, 91, 92, 93, 94, 95, 96, 97, 98 or 99%.

The term "oxo," as used herein, means an oxygen that is double bonded to a carbon atom, thereby forming a carbonyl.

The symbol " 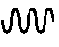 ", except when used as a bond to depict unknown or mixed stereochemistry, denotes the point of attachment of a chemical moiety to the remainder of a molecule or chemical formula.

The articles "a" and "an" are used herein to refer to one or to more than one (i.e., to at least one) of the grammatical object of the article. By way of example, "an element" means one element or more than one element.

A "dosing regimen" (or "therapeutic regimen"), as that term is used herein, is a set of unit doses (typically more than one) that are administered individually to a subject, typically separated by periods of time. In some embodiments, a given therapeutic agent has a recommended dosing regimen, which may involve one or more doses. In some embodiments, a dosing regimen comprises a plurality of doses each of which are separated from one another by a time period of the same length; in some embodiments, a dosing regimen comprises a plurality of doses and at least two different time periods separating individual doses.

As will be understood from context, a "reference" compound is one that is sufficiently similar to a particular compound of interest to permit a relevant comparison. In some embodiments, information about a reference compound is obtained simultaneously with information about a particular compound. In some embodiments, information about a reference compound is historical. In some embodiments, information about a reference compound is stored, for example in a computer-readable medium. In some embodiments, comparison of a particular compound of interest with a reference compound establishes identity with, similarity to, or difference of the particular compound of interest relative to the compound.

As used herein, the phrase "therapeutic agent" refers to any agent that has a therapeutic effect and/or elicits a desired biological and/or pharmacological effect, when administered to a subject.

As used herein, the term "therapeutically effective amount" refers to an amount of a therapeutic agent that confers a therapeutic effect on the treated subject, at a reasonable benefit/risk ratio applicable to any medical treatment. The therapeutic effect may be objective (i.e., measurable by some test or marker) or subjective (i.e., subject gives an indication of or feels an effect). In particular, the "therapeutically effective amount" refers to an amount of a therapeutic agent effective to treat, ameliorate, or prevent a desired disease or condition, or to exhibit a detectable therapeutic or preventative effect, such as by ameliorating symptoms associated with the disease, preventing or delaying the onset of the disease, and/or also lessening the severity or frequency of symptoms of the disease. A therapeutically effective amount is commonly administered in a dosing regimen that may comprise multiple unit doses. For any particular therapeutic agent, a therapeutically effective amount (and/or an appropriate unit dose within an effective dosing regimen) may vary, for example, depending on route of administration, on combination with other pharmaceutical agents. Also, the specific therapeutically effective amount (and/or unit dose) for any particular subject may depend upon a variety of factors including the disorder being treated and the severity of the disorder; the activity of the specific therapeutic agent employed; the specific composition employed; the age, body weight, general health, sex and diet of the subject; the time of administration, route of administration, and/or rate of excretion or metabolism of the specific therapeutic agent employed; the duration of the treatment; and like factors as is well known in the medical arts.

As used herein, the term "treatment" (also "treat" or "treating") refers to any administration of a substance (e.g., provided compositions) that partially or completely alleviates, ameliorates, relives, inhibits, delays onset of, reduces severity of, and/or reduces incidence of one or more symptoms, features, and/or causes of a particular disease, disorder, and/or condition. Such treatment may be of a subject who does not exhibit signs of the relevant disease, disorder and/or condition and/or of a subject who exhibits only early signs of the disease, disorder, and/or condition. Alternatively or additionally, such treatment may be of a subject who exhibits one or more established signs of the relevant disease, disorder and/or condition. In some embodiments, treatment may be of a subject who has been diagnosed as suffering from the relevant disease, disorder, and/or condition. In some embodiments, treatment may be of a subject known to have one or more susceptibility factors that are statistically correlated with increased risk of development of the relevant disease, disorder, and/or condition.

### B. Compounds

In some embodiments, a provided compound is of Formula (I): 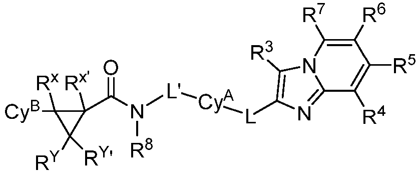
or a pharmaceutically acceptable salt thereof,
wherein:
   Cy^{A} is a phenylene or a 5- to 6-membered monocyclic heteroarylene having 1-3 heteroatoms independently selected from oxygen, nitrogen, and sulfur, or a 7- to 12-membered bicyclic heteroarylene having 1-4 heteroatoms independently selected from oxygen, nitrogen, and sulfur, wherein Cy^{A} is substituted with 0-4 -R^{A} groups;
      each R^{A} is independently selected from oxo, halogen, -CN, -C(O)R, -C(O)₂R, -C(O)N(R)₂, -NO₂, - N(R)₂, -N(R)C(O)R, -N(R)C(O)₂R, -N(R)S(O)₂R, -OR, -OC(O)R, -OC(O)N(R)₂, -SR, -S(O)R, - S(O)₂R, -S(O)N(R)₂, -S(O)₂N(R)₂, or an optionally substituted group selected from C₁₋₆ aliphatic, phenyl, 3- to 7-membered saturated or partially unsaturated monocyclic carbocyclyl, or 3- to 7-membered saturated or partially unsaturated monocyclic heterocyclyl having 1-2 heteroatoms selected from oxygen, nitrogen, or sulfur;
   each R is independently hydrogen or an optionally substituted C₁₋₆ aliphatic group;
   each R^{Y} and R^{Y'} is independently selected from hydrogen, halogen, and an optionally substituted C₁₋₆ aliphatic group;
   each R^{x} and R^{x}' is independently selected from hydrogen, halogen, or -CN;
   Cy^{B} is selected from phenyl, 8- to 10-membered bicyclic aryl, a 5- to 6-membered heteroaryl having 1-3 heteroatoms independently selected from oxygen, nitrogen, and sulfur, or a 7- to 10-membered heteroaryl having 1-4 heteroatoms independently selected from oxygen, nitrogen, and sulfur, wherein Cy^{B} is substituted with 0-5 -R^{B} groups; or
   Cy^{B} and R^{x}, together with their intervening atoms, form a 6- to 12-membered spirocyclic ring system having 0-4 heteroatoms independently selected from oxygen, nitrogen, and sulfur, wherein the ring or rings formed by Cy^{B} and R^{x} may be substituted with 0-4 -R^{B} groups;
      each R^{B} is independently selected from oxo, halogen, -CN, -C(O)R, -C(O)₂R, -C(O)N(R)₂, -NO₂, - N(R)₂, -N(R)C(O)R, -N(R)C(O)₂R, -N(R)S(O)₂R, -OR, -OC(O)R, -OC(O)N(R)₂, -SR, -S(O)R, - S(O)₂R, -S(O)N(R)₂, -S(O)₂N(R)₂, or an optionally substituted group selected from C₁₋₆ aliphatic, a 3- to 7-membered saturated or partially unsaturated monocyclic heterocyclyl having 1-2 heteroatoms selected from oxygen, nitrogen, or sulfur, or a 5- to 6-membered heteroaryl having 1-4 heteroatoms independently selected from oxygen, nitrogen, and sulfur;
   L is an optionally substituted C₁₋₃ hydrocarbon chain, wherein 1 to 3 methylene units are optionally and independently replaced with -O-, -NR^{z}-, -S-, -SO-, or -SO₂-; or L is an optionally substituted 5- to 6-membered saturated or partially unsaturated heterocyclene, having 1-3 heteroatoms independently selected from oxygen, nitrogen, and sulfur;
      each R^{z} is independently selected from hydrogen, -(CH₂)₀₋₃OR, -(CH₂)₀₋₃C(O)OR, or an optionally substituted C₁₋₆ aliphatic group;
   L' is a covalent bond or an optionally substituted C₁₋₃ hydrocarbon chain, wherein 1 to 3 methylene units are optionally and independently replaced with -O-, -NR^{z}-, -S-, -SO-, or SO₂-;
   each R³, R⁴, R⁵, R⁶, and R⁷ is independently selected from hydrogen or -L^{C}-R^{C}, wherein
      each L^{C} is independently selected from a covalent bond or an optionally substituted C₁₋₆ hydrocarbon chain, wherein 1 to 3 methylene units are optionally and independently replaced with -O- or -NR-;
      each R^{C} is independently selected from oxo, halogen, -CN, -C(O)R, -C(O)₂R, -C(O)N(R)₂, -NO₂, -N(R)₂, -N(R)C(O)R, -N(R)C(O)₂R, -N(R)S(O)₂R, -OR, -OC(O)R, - OC(O)N(R)₂, -SR, -S(O)R, -S(O)₂R, -S(O)N(R)₂, -S(O)₂N(R)₂, Cy^{C}, or an optionally substituted group selected from C₁-₆ aliphatic;
         each Cy^{C} is independently selected from a 3- to 7-membered saturated or partially unsaturated monocyclic carbocyclyl, a 3- to 7-membered saturated or partially unsaturated monocyclic heterocyclyl having 1-2 heteroatoms selected from oxygen, nitrogen, or sulfur, a 5- to 6-membered monocyclic heteroaryl having 1-3 heteroatoms independently selected from oxygen, nitrogen, and sulfur, phenyl, a 6- to 12- membered saturated or partially unsaturated fused bicyclic heterocyclyl having 1-3 heteroatoms independently selected from oxygen, nitrogen, or sulfur, a bridged bicycle, or a 6- to 12- membered saturated or partially unsaturated bicyclic spiroheterocyclyl having 1-3 heteroatoms independently selected from oxygen, nitrogen, or sulfur, wherein Cy^{C} is substituted with 0-4 -L^{D}-R^{D} groups;
         each L^{D} is independently selected from a covalent bond or an optionally substituted C₁₋₆ hydrocarbon chain, wherein 1 to 3 methylene units are optionally and independently replaced with -O- or -NR-;
         each R^{D} is independently selected from oxo, halogen, -CN, -C(O)R, -C(O)₂R, -C(O)N(R)₂, -NO₂, -N(R)₂, -N(R)C(O)R, -N(R)C(O)₂R, -N(R)S(O)₂R, -OR, -OC(O)R, -OC(O)N(R)₂, -SR, -S(O)R, -S(O)₂R, -S(O)N(R)₂, -S(O)₂N(R)₂, or an optionally substituted group selected from C₁-₆ aliphatic, phenyl, a 3- to 7-membered saturated or partially unsaturated monocyclic carbocyclyl, or a 3- to 7-membered saturated or partially unsaturated monocyclic heterocyclyl having 1-2 heteroatoms selected from oxygen, nitrogen, or sulfur; and
   R⁸ is selected from hydrogen, -OR, or an optionally substituted C₁₋₆ aliphatic group.

It will be appreciated that, "oxo" refers a double bonded oxygen substitution on a carbon "C=O", where the carbon atom is part of the structure or group that is substituted by oxo. For example, where Cy^{C} is substituted with -L^{D}-R^{D}, and where L^{D} is a covalent bond and R^{D} is oxo, the carbon atom substituted with oxo (i.e., the carbon in C=O) is part of Cy^{C} (e.g., a structure of Cy^{C} being cyclopentyl substituted with -L^{D}-R^{D} at the 2-position, where L^{D} is a covalent bond and R^{D} is oxo corresponds to

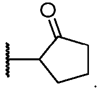

In some embodiments, Cy^{A} is a phenylene or a 5- to 6-membered monocyclic heteroarylene having 1-3 heteroatoms independently selected from oxygen, nitrogen, and sulfur, wherein Cy^{A} is substituted with 0-4 R^{A} groups. In some embodiments, Cy^{A} is a 5- to 6-membered monocyclic heteroarylene having 1-3 heteroatoms independently selected from oxygen, nitrogen, and sulfur, or a 7-to 12-membered bicyclic heteroarylene having 1-4 heteroatoms independently selected from oxygen, nitrogen, and sulfur, wherein Cy^{A} is substituted with 0-4 -R^{A} groups.

In some embodiments, Cy^{A} is a phenylene, wherein Cy^{A} is substituted with 0-4 -R^{A} groups. In some embodiments, Cy^{A} is a phenylene, wherein Cy^{A} is substituted with 0-2 -R^{A} groups.

In some embodiments, Cy^{A} is a 5- to 6-membered monocyclic heteroarylene having 1-3 heteroatoms independently selected from oxygen, nitrogen, and sulfur, wherein Cy^{A} is substituted with 0-4 -R^{A} groups.

In some embodiments, Cy^{A} is a 6-membered monocyclic heteroarylene having 1-3 heteroatoms independently selected from oxygen, nitrogen, and sulfur, wherein Cy^{A} is substituted with 0-4 -R^{A} groups. In some embodiments, Cy^{A} is a 6-membered monocyclic heteroarylene having 1-3 nitrogen heteroatoms, wherein Cy^{A} is substituted with 0-4 R^{A} groups. In some embodiments, Cy^{A} is a pyridinediyl substituted with 0-1 R^{A} groups. In some embodiments, Cy^{A} is a pyrimidinediyl substituted with 0-1 R^{A} groups. In some embodiments, Cy^{A} is a pyridazinediyl substituted with 0-1 R^{A} groups. In some embodiments, Cy^{A} is a triazinediyl substituted with 0-1 R^{A} groups.

In some embodiments, Cy^{A} is a 5-membered monocyclic heteroarylene having 1-3 heteroatoms independently selected from oxygen, nitrogen, and sulfur, wherein Cy^{A} is substituted with 0-2 -R^{A} groups. In some embodiments, Cy^{A} is an unsubstituted thiadiazolediyl. In some embodiments, Cy^{A} is an unsubstituted oxadiazolediyl. In some embodiments, Cy^{A} is an unsubstituted triazolediyl.

In some embodiments, Cy^{A} is a 7- to 12-membered bicyclic heteroarylene having 1-4 heteroatoms independently selected from oxygen, nitrogen, and sulfur, wherein Cy^{A} is substituted with 0-4 -R^{A} groups. In some embodiments, Cy^{A} is a 8- to 12-membered bicyclic heteroarylene having 1-4 heteroatoms independently selected from oxygen, nitrogen, and sulfur, wherein Cy^{A} is substituted with 0-4 -R^{A} groups. In some embodiments, Cy^{A} is a 9-membered bicyclic heteroarylene having 3-4 heteroatoms independently selected from oxygen and nitrogen, wherein Cy^{A} is substituted with 0-1 -R^{A} groups. In some embodiments, Cy^{A} is a 10-membered bicyclic heteroarylene having 3-4 heteroatoms independently selected from oxygen and nitrogen, wherein Cy^{A} is substituted with 0-1 -R^{A} groups.

In some embodiments, Cy^{A} is selected from the group consisting of: 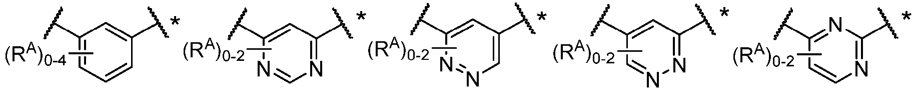 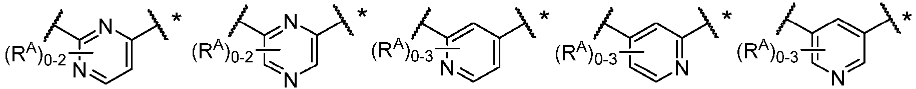 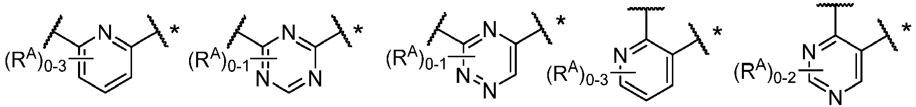 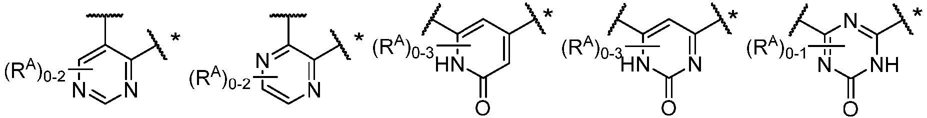 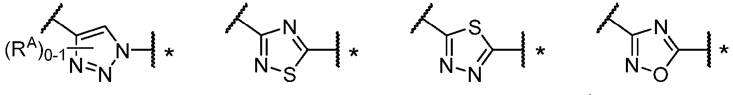 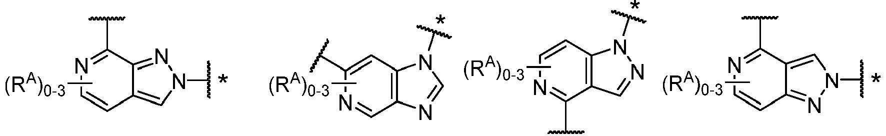 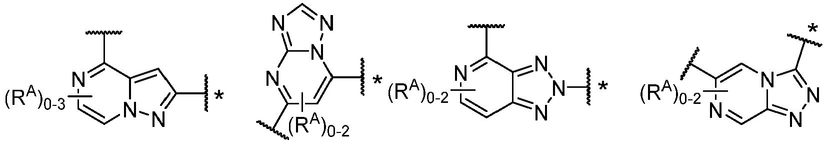 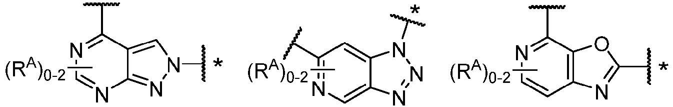 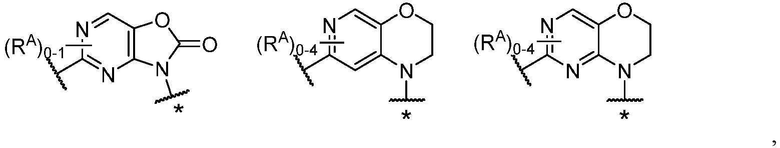 wherein * represents point of attachment to L.

In some embodiments, Cy^{A} is selected from the group consisting of: 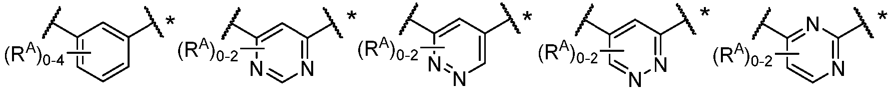 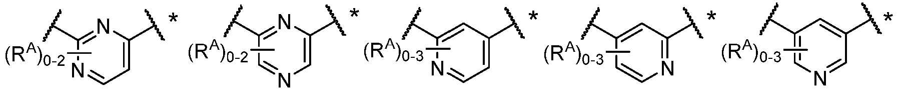 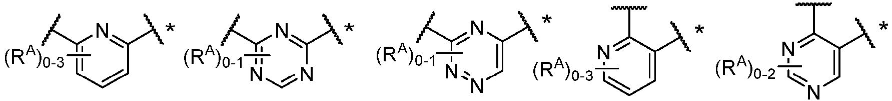 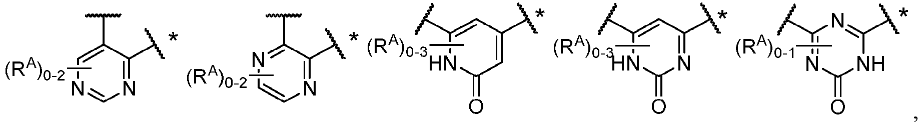 wherein * represents the point of attachment to L.

In some embodiments, Cy^{A} is selected from the group consisting of: 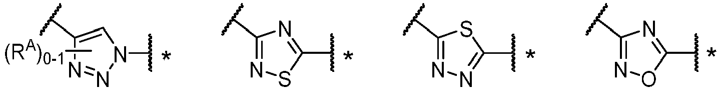 wherein * represents the point of attachment to L.

In some embodiments, Cy^{A} is selected from the group consisting of: 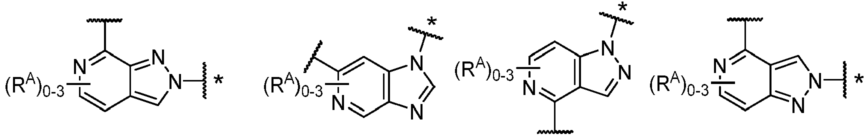 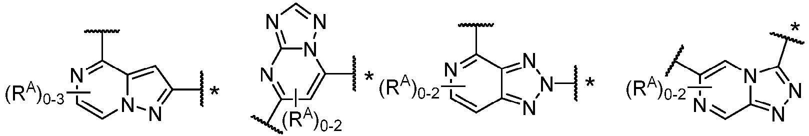 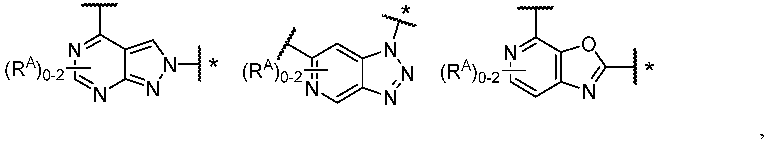 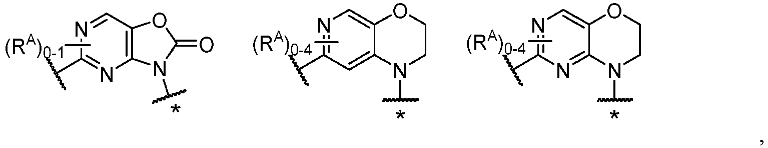 wherein * represents the point of attachment to L.

In some embodiments, Cy^{A} is selected from the group consisting of: 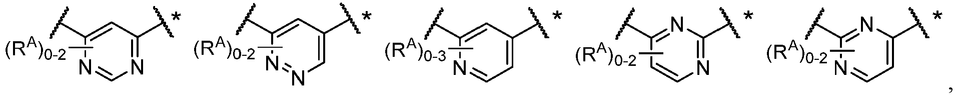 wherein * represents the point of attachment to L.

In some embodiments, Cy^{A} is selected from the group consisting of: 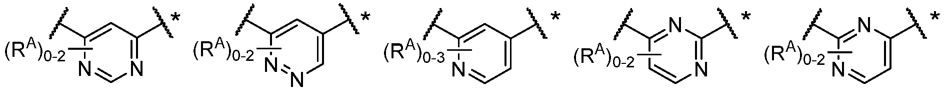 in particular: 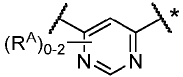 wherein * represents the point of attachment to L.

In some embodiments, each R^{A} is independently selected from oxo, halogen, -CN, -C(O)₂R, -N(R)₂, -OR, -SR, -S(O)R, -S(O)₂R, or an optionally substituted group selected from C₁-₆ aliphatic, 3- to 7-membered saturated or partially unsaturated monocyclic carbocyclyl, or 3- to 7-membered saturated or partially unsaturated monocyclic heterocyclyl having 1-2 heteroatoms selected from oxygen, nitrogen, or sulfur.

In some embodiments, Cy^{A} is: 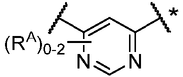 wherein * represents the point of attachment to L.

In some embodiments, Cy^{A} is: 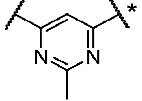 wherein * represents the point of attachment to L.

In one embodiment, Cy^{A} comprising 0 R^{A} groups, i.e. Cy^{A} is unsubstituted.

In one embodiment, Cy^{A} comprises 1 R^{A} group, for example as described herein, in particular methyl.

In one embodiment, Cy^{A} comprises 2 R^{A} groups, for example independently selected from the groups/atoms described herein.

In some embodiments, substituents on an optionally substituted R^{A} group are independently halogen, -(CH₂)₀₋₄OR°, or -(CH₂)₀₋₄N(R°)₂, wherein each R° is independently as defined above and described in classes and subclasses herein.

In some embodiments, a single instance of R^{A} is oxo. In some embodiments, a single instance of R^{A} is halogen. In some embodiments, a single instance of R^{A} is fluorine. In some embodiments, a single instance of R^{A} is chlorine. In some embodiments, a single instance of R^{A} is -CN. In some embodiments, a single instance of R^{A} is -C(O)₂R. In some embodiments, a single instance of R^{A} is -N(R)₂. In some embodiments, a single instance of R^{A} is -OR. In some embodiments, a single instance of R^{A} is -OMe. In some embodiments, a single instance of R^{A} is -SR. In some embodiments, a single instance of R^{A} is -SR, wherein R is optionally substituted C₁₋₆ aliphatic. In some embodiments, a single instance of R^{A} is -S(O)R. In some embodiments, a single instance of R^{A} is -S(O)R, wherein R is optionally substituted C₁₋₆ aliphatic. In some embodiments, a single instance of R^{A} is -S(O)₂R. In some embodiments, a single instance of R^{A} is -S(O)₂R, wherein R is optionally substituted C₁₋₆ aliphatic. In some embodiments, a single instance of R^{A} is -OR, wherein R is optionally substituted C₁₋₆ aliphatic. In some embodiments, a single instance of R^{A} is -OR, wherein R is C₁₋₆ aliphatic, optionally substituted with -(CH₂)₀₋₄R°, wherein R° is phenyl optionally substituted with -OR**^{•},** wherein R**^{•}** is independently as defined above and described in classes and subclasses herein. It will be appreciated that references herein to embodiments in which "a single instance" of a substituent is defined are not limited to monosubstituted embodiments. For example, "[i]n some embodiments, a single instance of R^{A} is oxo" includes embodiments in which at least one instance of R^{A} is oxo and which may comprise one or more additional R^{A} groups as defined herein.

In some embodiments, a single instance of R^{A} is C₁₋₆ aliphatic substituted with halogen. In some embodiments, a single instance of R^{A} is CF₃. In some embodiments, a single instance of R^{A} is C₁₋₆ aliphatic substituted with -(CH₂)₀₋₄OR°, wherein R° is selected from hydrogen or C₁₋₆ aliphatic. In some embodiments, a single instance of R^{A} is C₁₋₆ aliphatic substituted with -(CH₂)₀₋₄N(R°)₂, wherein each R° is independently selected from hydrogen or C₁₋₆ aliphatic.

In some embodiments, a single instance of R^{A} is optionally substituted 3- to 7-membered saturated or partially unsaturated monocyclic carbocyclyl. In some embodiments, a single instance of R^{A} is optionally substituted cyclopropyl.

In some embodiments, a single instance of R^{A} is optionally substituted 3- to 7-membered saturated or partially unsaturated monocyclic heterocyclyl having 1-2 heteroatoms selected from oxygen, nitrogen, or sulfur. In some embodiments, a single instance of R^{A} is optionally substituted 3- to 7-membered saturated monocyclic heterocyclyl having 1-2 heteroatoms selected from oxygen and nitrogen. In some embodiments, a single instance of R^{A} is optionally substituted oxetanyl. In some embodiments, a single instance of R^{A} is oxetanyl optionally substituted with halogen or -(CH₂)₀₋₄OR°. In some embodiments, a single instance of R^{A} is pyrrolidinyl.

In some embodiments, Cy^{B} is selected from phenyl, a 5- to 6-membered heteroaryl having 1-3 heteroatoms independently selected from oxygen, nitrogen, and sulfur or a 7- to 10-membered heteroaryl having 1-4 heteroatoms independently selected from oxygen, nitrogen, and sulfur, wherein Cy^{B} is substituted with 0-4 -R^{B} groups.

In some embodiments, Cy^{B} is selected from phenyl or a 5- to 6-membered heteroaryl having 1-3 heteroatoms independently selected from oxygen, nitrogen, and sulfur, wherein Cy^{B} is substituted with 0-4 -R^{B} groups.

In some embodiments, Cy^{B} is selected from phenyl or a 5- to 6-membered heteroaryl having 1-3 heteroatoms independently selected from oxygen, nitrogen, and sulfur, wherein Cy^{B} is substituted with 0-4 -R^{B} groups, for example pyrimidinyl substituted with 0-4 -R^{B} groups, such as 0 or 1 group (in particular wherein 1 group is methyl).

In some embodiments, Cy^{B} is phenyl, wherein Cy^{B} is substituted with 0-4 -R^{B} groups. In some embodiments, Cy^{B} is phenyl, wherein Cy^{B} is substituted with 0-3 -R^{B} groups.

In some embodiments, Cy^{B} is a 6-membered heteroaryl having 1-3 heteroatoms independently selected from oxygen, nitrogen, and sulfur, wherein Cy^{B} is substituted with 0-4 -R^{B} groups. In some embodiments, Cy^{B} is a 6-membered heteroaryl having 1-3 nitrogens, wherein Cy^{B} is substituted with 0-4 -R^{B} groups. In some embodiments, Cy^{B} is a pyrimidinyl group substituted with 0-2 -R^{B} groups. In some embodiments, Cy^{B} is a pyridinyl group substituted with 0-2 -R^{B} groups. In some embodiments, Cy^{B} is a pyrazinyl group substituted with 0-1 -R^{B} groups. In some embodiments, Cy^{B} is a pyridazinyl group substituted with 0-1 -R^{B} groups. In some embodiments, Cy^{B}is a 1,3,5-triazinyl group substituted with 0-1 -R^{B} groups.

In some embodiments, Cy^{B} is a 5-membered heteroaryl having 1-3 heteroatoms independently selected from oxygen, nitrogen, and sulfur, wherein Cy^{B} is substituted with 0-4 -R^{B} groups. In some embodiments, Cy^{B} is a 5-membered heteroaryl having 1-2 heteroatoms independently selected from sulfur and nitrogen, wherein Cy^{B} is substituted with 0-4 -R^{B} groups. In some embodiments, Cy^{B} is a thienyl group substituted with 0-2 -R^{B} groups. In some embodiments, Cy^{B} is a thiazolyl group substituted with 0-1 -R^{B} groups. In some embodiments, Cy^{B} is a thiadiazolyl group substituted with 0-1 -R^{B} groups.

In some embodiments, Cy^{B} is selected from the group consisting of:

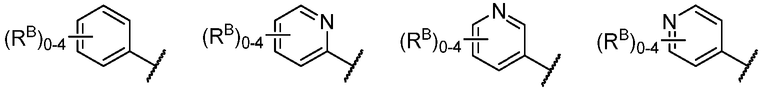

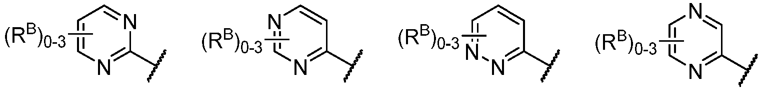

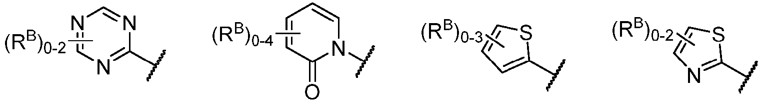

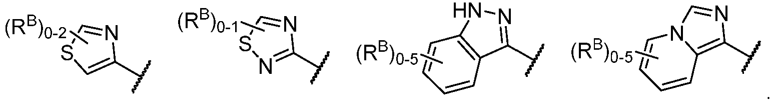

In some embodiments, Cy^{B} is selected from the group consisting of:

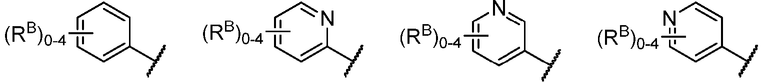

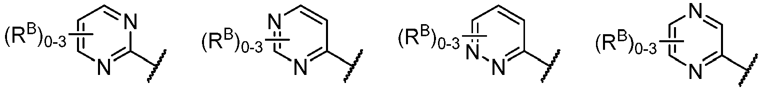

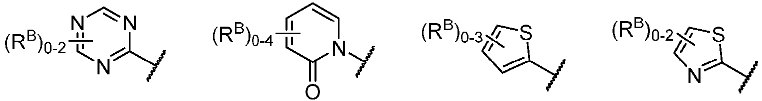

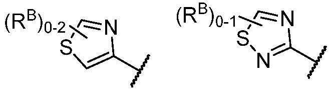

In some embodiments, Cy^{B} is selected from the group consisting of:

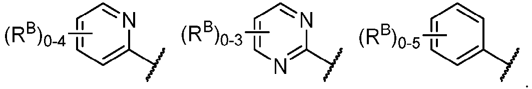

In some embodiments, Cy^{B} is selected from the group consisting of: 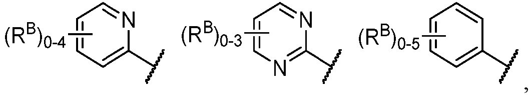 in particular

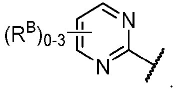

In some embodiments, Cy^{B} is:

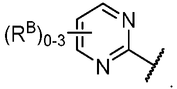

In some embodiments, Cy^{B} and R^{x}, together with their intervening atoms, form a 6- to 12-membered spirocyclic ring system having 0-4 heteroatoms independently selected from oxygen, nitrogen, and sulfur, wherein the ring or rings formed by Cy^{B} and R^{x} may be substituted with 0-4 -R^{B} groups. It will be appreciated that references herein to the number of atoms in a spirocyclic ring system (e.g., 6- to 12-membered) include the depicted cyclopropyl ring.

In some embodiments, Cy^{B} and R^{x}, together with their intervening atoms, form a 6- to 12-membered spirocyclic ring system having 0-1 nitrogen heteroatoms, wherein the ring or rings formed by Cy^{B} and R^{x} may be substituted with 1-3 -R^{B} groups.

In some embodiments, Cy^{B} and R^{x}, together with their intervening atoms, form a 6- to 12-membered spirocyclic ring system selected from:

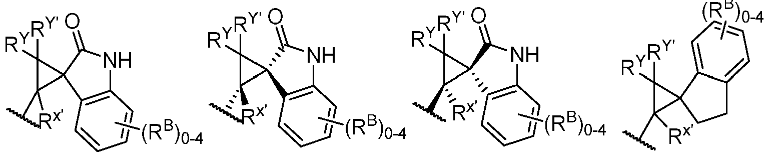

In some embodiments, each R^{B} is independently selected from oxo, halogen, -CN, -NO₂, - N(R)₂, -N(R)C(O)₂R, -OR, or an optionally substituted group selected from C₁₋₆ aliphatic or a 5-membered heteroaryl having 1-4 heteroatoms independently selected from oxygen, nitrogen, and sulfur.

In some embodiments, substituents on an optionally substituted R^{B} group are independently selected from oxo, halogen, and -(CH₂)₀₋₄OR°, wherein R° is as defined above and described in classes and subclasses herein.

In some embodiments, a single instance of R^{B} is oxo. In some embodiments, a single instance of R^{B} is halogen. In some embodiments, a single instance of R^{B} is fluorine. In some embodiments, a single instance of R^{B} is chlorine. In some embodiments, a single instance of R^{B} is -CN. In some embodiments, a single instance of R^{B} is -NO₂. In some embodiments, a single instance of R^{B} is - N(R)₂. In some embodiments, a single instance of R^{B} is -NH₂. In some embodiments, a single instance of R^{B} is -N(R)C(O)₂R. In some embodiments, a single instance of R^{B} is -OR. In some embodiments, a single instance of R^{B} is -OMe.

In some embodiments, a single instance of R^{B} is optionally substituted C₁-₆ aliphatic. In some embodiments, a single instance of R^{B} is C₁₋₆ aliphatic substituted with halogen. In some embodiments, a single instance of R^{B} is methyl. In some embodiments, a single instance of R^{B} is CF₃. In some embodiments, a single instance of R^{B} is CF₂.

In some embodiments, a single instance of R^{B} is -N(R)C(O)₂R, wherein each R is independently selected from hydrogen or C₁₋₆ aliphatic optionally substituted with -(CH₂)₀₋₄R°.

In some embodiments, a single instance of R^{B} is -OR, wherein each R is independently selected from hydrogen or C₁₋₆ aliphatic optionally substituted with halogen, -(CH₂)₀₋₄OR°, or (CH₂)₀₋₄C(O)OR°.

In some embodiments, a single instance of R^{B} is a 5-membered heteroaryl having 1-4 heteroatoms independently selected from oxygen, nitrogen, and sulfur. In some embodiments, a single instance of R^{B} is tetrazolyl.

In some embodiments, each of R^{x} and R^{x'} is independently selected from hydrogen and halogen. In some embodiments R^{x} is H. In some embodiments R^{x'} is H. In some embodiments, each of R^{x} and R^{x'} is hydrogen. In some embodiments, one of R^{x} and R^{x'} is hydrogen and the other is halogen.

In some embodiments, each of R^{Y} and R^{Y'} is independently selected from hydrogen and halogen.. In some embodiments R^{Y} is H. Is some embodiments R^{Y'} is H.

In some embodiments, each of R^{Y} and R^{Y'} is hydrogen.

In some embodiments, R^{Y} is an optionally substituted C₁₋₆ aliphatic group.

In some embodiments, R^{Y} is an optionally substituted C₁₋₆ aliphatic group and R^{Y'} is hydrogen. In some embodiments, R^{Y} is substituted with -(CH₂)₀₋₄OR°, wherein R° is as defined above and described in classes and subclasses herein.

In some embodiments, L is an optionally substituted C₁₋₃ hydrocarbon chain, wherein 1-3 methylene units are optionally replaced with -O-, -NR^{z}-, -S-, or -SO₂-. In some embodiments, L is an optionally substituted C₁₋₃ hydrocarbon chain, wherein 1 methylene unit is optionally replaced with -O-, -NR^{z}-, -S-, or -SO₂-. In some embodiments, L is an optionally substituted C₁₋₃ hydrocarbon chain, wherein 1 methylene unit is optionally replaced with -O-, -NR^{z}-, -S-, or -SO₂-, in particular -NR^{z}-.

In some embodiments, L is an optionally substituted C₁ hydrocarbon chain.

In some embodiments, L is an optionally substituted C₁ hydrocarbon chain, wherein the 1 methylene unit is replaced with 5-membered saturated or partially unsaturated heterocyclene having 1 nitrogen heteroatom, optionally substituted with -(CH₂)₀₋₄OR°, wherein R° is as defined above and described in classes and subclasses herein.

In some embodiments, L is -CH₂-. In some embodiments, L is optionally substituted 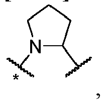 wherein * represents the point of attachment to Cy^{A}. In some embodiments, L is optionally substituted 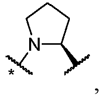 wherein * represents the point of attachment to Cy^{A}. In some embodiments, L is optionally substituted 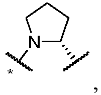 wherein * represents the point of attachment to Cy^{A}. In some embodiments, L is 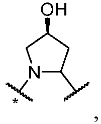 wherein * represents the point of attachment to Cy^{A}. In some embodiments, L is 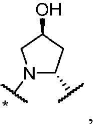 wherein * represents the point of attachment to Cy^{A}. In some embodiments, L is 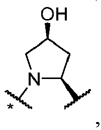 wherein * represents the point of attachment to Cy^{A}.

In some embodiments, L is an optionally substituted C₂ hydrocarbon chain, wherein 1 methylene unit is optionally replaced with -NR^{z}- or -O-. In some embodiments, L is an optionally substituted C₂ hydrocarbon chain, wherein 1 methylene unit is optionally replaced with -NR^{z}- or -O-, in particular -NR^{z}-. In some embodiments, L is an optionally substituted C₂ hydrocarbon chain, wherein the methylene unit connected to Cy^{A} is replaced with -NR^{z}- or -O-. In some embodiments, L is an optionally substituted C₂ hydrocarbon chain, wherein the methylene unit connected to Cy^{A} is replaced with -NR^{z}-. In some embodiments, L is an optionally substituted C₂ hydrocarbon chain, wherein the methylene unit connected to Cy^{A} is replaced with -NR^{z}-, and wherein R^{z} is selected from hydrogen, - (CH₂)₀₋₃C(O)OR, or an optionally substituted C₁₋₆ aliphatic group. In some embodiments, L is an optionally substituted C₂ hydrocarbon chain, wherein the methylene unit connected to Cy^{A} is replaced with -NR^{z}-, and wherein R^{z} is selected from hydrogen, -(CH₂)₀₋₃C(O)OR, or an optionally substituted C₁₋₆ aliphatic group (such as methyl).

In some embodiments, R^{z} is selected from H and C₁₋₆ aliphatic group, such as H or methyl, in particular methyl.

In some embodiments, L is an optionally substituted C₂ hydrocarbon chain, wherein the methylene unit connected to Cy^{A} is replaced with -O-.

In some embodiments, L is *-NHCH(Me)-, wherein * represents the point of attachment to Cy^{A}. In some embodiments, L is 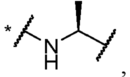 wherein * represents the point of attachment to Cy^{A}. In some embodiments, L is 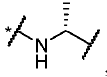 , wherein * represents the point of attachment to Cy^{A}. In some embodiments, L is *-NHCH₂-, wherein * represents the point of attachment to Cy^{A}. In some embodiments, L is *-N(CH₃)CH₂-, wherein * represents the point of attachment to Cy^{A}. In some embodiments, L is 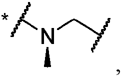 wherein * represents the point of attachment to Cy^{A}. In some embodiments, L is 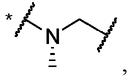 wherein * represents the point of attachment to Cy^{A}. In some embodiments, L is *-OCH(Me)-, wherein * represents the point of attachment to Cy^{A}. In some embodiments, L is *-OCH₂-, wherein * represents the point of attachment to Cy^{A}.

In some embodiments, L comprises a two-atom spacer between Cy^{A} and

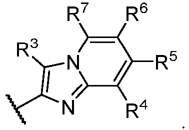

In some embodiments, L is an optionally substituted 5- to 6-membered saturated or partially unsaturated heterocyclene, having 1-3 heteroatoms independently selected from oxygen, nitrogen, and sulfur. In some embodiments, L is an optionally substituted 5-membered saturated or partially unsaturated heterocyclene, having 1 heteroatom independently selected from oxygen, nitrogen, and sulfur. In some embodiments, L is an optionally substituted pyrrolidinediyl group. In some embodiments, L is optionally substituted 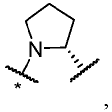 wherein * represents the point of attachment to Cy^{A}.

In some embodiments, optional substituents on L are independently selected from -(CH₂)₀₋₄R°, -(CH₂)₀₋₄OR°, -(CH₂)₀₋₄OC(O)R°, and -(CH₂)₀₋₄N(R°)₂, wherein each R° is independently as defined above and described in classes and subclasses herein.

In some embodiments, L' is a covalent bond or a methylene unit optionally substituted with -(CH₂)₀₋₄R°, wherein R° is independently as defined above and described in classes and subclasses herein. In some embodiments, R° is hydrogen or C₁₋₆ aliphatic.

In some embodiments, L' is a covalent bond.

In some embodiments, each of R³, R⁴, R⁵, R⁶, and R⁷ is independently selected from hydrogen or L^{C}-R^{C}, wherein each L^{C} is independently selected from a covalent bond or an optionally substituted C₁₋₆ hydrocarbon chain, wherein 1 to 3 methylene units are optionally and independently replaced with -O- or -NR-; and wherein each R^{C} is independently selected from halogen, -CN, -C(O)R, - C(O)₂R, -C(O)N(R)₂, -N(R)₂, -N(R)C(O)R, -N(R)C(O)₂R, -N(R)S(O)₂R, -S(O)₂R, -S(O)₂N(R)₂, Cy^{C}, or an optionally substituted group selected from C₁₋₆ aliphatic.

In some embodiments, R³ is selected from hydrogen or L^{C}-R^{C}, wherein L^{C} is a covalent bond and R^{C} is halogen. In some embodiments, R³ is chloro. In some embodiments, R³ is fluoro. In some embodiments R³ is H.

In some embodiments, R⁴ is selected from hydrogen or L^{C}-R^{C}, wherein L^{C} is selected from a covalent bond or an optionally substituted C₁₋₆ hydrocarbon chain, wherein 1 to 3 methylene units are optionally and independently replaced with -O- or -NR-; and wherein R^{C} is selected from halogen, -CN, -C(O)R, -C(O)₂R, -C(O)N(R)₂, -N(R)₂, -N(R)C(O)R, -N(R)C(O)₂R, -N(R)S(O)₂R, -OR, -S(O)₂R, - S(O)₂N(R)₂, Cy^{C}, or an optionally substituted group selected from C₁₋₆ aliphatic. In some embodiments L^{C} is a covalent bond.

In some embodiments, R⁴ is selected from hydrogen or L^{C}-R^{C}, wherein L^{C} is a covalent bond and wherein R^{C} is selected from halogen, -CN, -C(O)R, -C(O)₂R, -C(O)N(R)₂, -N(R)₂, - N(R)C(O)R, -N(R)C(O)₂R, -N(R)S(O)₂R, -OR, -S(O)₂R, -S(O)₂N(R)₂, Cy^{C}, or an optionally substituted group selected from C₁₋₆ aliphatic. In some embodiments, R⁴ is chloro. In some embodiments, R⁴ is fluoro.

In some embodiments, R⁴ is selected from the group consisting of:

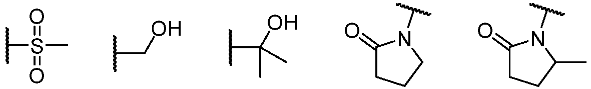

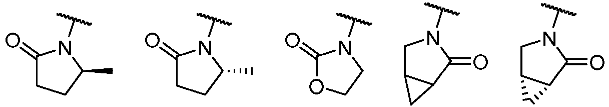

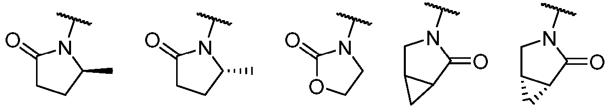

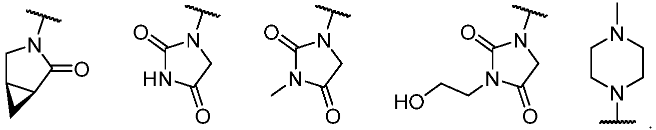

In some embodiments, R⁴ is selected from the group consisting of: 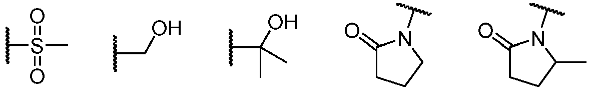 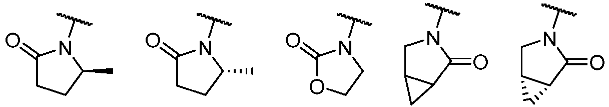 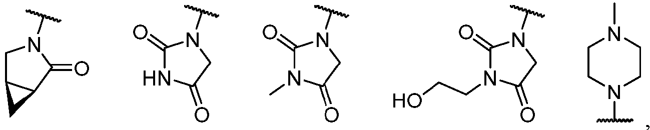 in particular

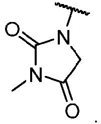

In some embodiments of R⁴, optional substituents on a C₁₋₆ aliphatic group are selected from -(CH₂)₀₋₄R°, -(CH₂)₀₋₄OR°, -CN, -(CH₂)₀₋₄N(R°)₂, and -(CH₂)₀₋₄C(O)OR°, wherein each R° is independently as defined above and described in classes and subclasses herein.

In some embodiments of R⁴, Cy^{C} is selected from a 3- to 7-membered saturated or partially unsaturated monocyclic heterocyclyl having 1-2 heteroatoms selected from oxygen, nitrogen, or sulfur, a 5- to 6-membered monocyclic heteroaryl having 1-3 heteroatoms independently selected from oxygen, nitrogen, and sulfur, a 6- to 12- membered saturated or partially unsaturated fused bicyclic heterocyclyl having 1-3 heteroatoms independently selected from oxygen, nitrogen, or sulfur, a bridged bicycle, or a 6- to 12- membered saturated or partially unsaturated bicyclic spiroheterocyclyl having 1-3 heteroatoms independently selected from oxygen, nitrogen, or sulfur, wherein Cy^{C} is substituted with 0-4 -L^{D}-R^{D} groups. In some embodiments of R⁴, Cy^{C} is a 5-membered saturated or partially unsaturated monocyclic heterocyclyl having 1-2 heteroatoms selected from oxygen, nitrogen, or sulfur.

In some embodiments, Cy^{C} is substituted with 0 L^{D}-R^{D} groups. In some embodiments, Cy^{C} is substituted with 1 L^{D}-R^{D} groups. In some embodiments, Cy^{C} is substituted with 2 L^{D}-R^{D} groups. In some embodiments, Cy^{C} is substituted with 3 L^{D}-R^{D} groups. In some embodiments, Cy^{C} is substituted with 4 L^{D}-R^{D} groups.

In some embodiments L^{D} is a covalent bond. In some embodiments R^{D} is optionally substituted C₁₋₆ aliphatic, such as methyl.

In some embodiments of R⁴, Cy^{C} is selected from the group consisting of:

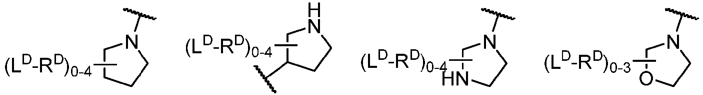

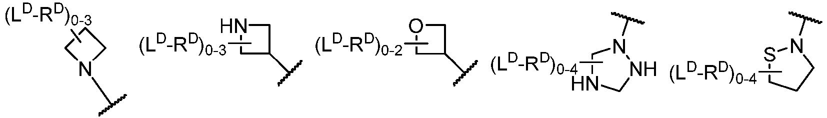

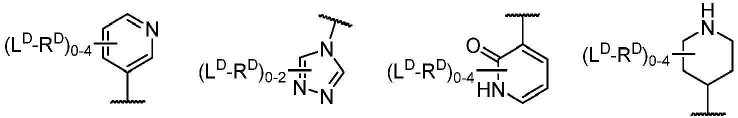

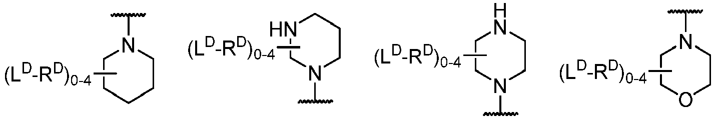

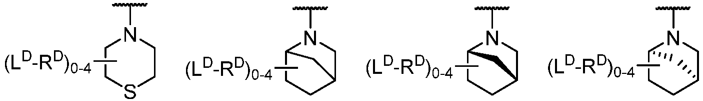

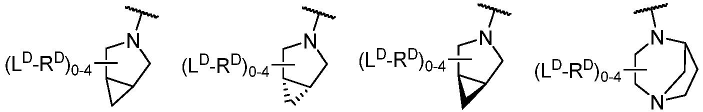

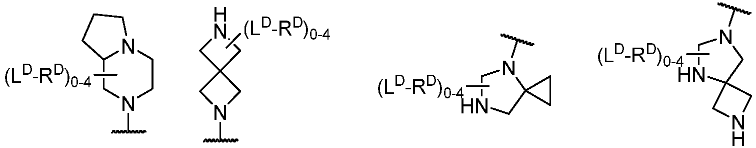

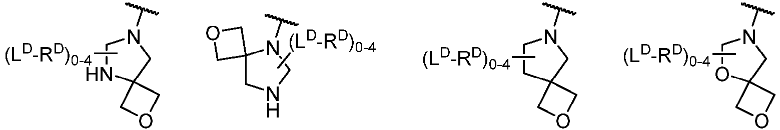

In some embodiments of R⁴, R^{D} is selected from oxo, halogen, -C(O)₂R, -N(R)₂, -OR, or an optionally substituted group selected from C₁₋₆ aliphatic, phenyl, a 3- to 7-membered saturated or partially unsaturated monocyclic carbocyclyl, or a 3- to 7-membered saturated or partially unsaturated monocyclic heterocyclyl having 1-2 heteroatoms selected from oxygen, nitrogen, or sulfur.

In some embodiments of a R^{D} group of R⁴, optional substituents on R^{D} are selected from halogen, -(CH₂)₀₋₄R°, -(CH₂)₀₋₄OR°, -(CH₂)₀₋₄N(R°)₂, -(CH₂)₀₋₄C(O)OR°, and -OP(O)(OR°)₂, wherein each R° is independently as defined above and described in classes and subclasses herein.

In some embodiments of R⁴, L^{D} is a covalent bond.

In some embodiments, R⁵ is hydrogen.

In some embodiments, R⁵ is L^{C}-R^{C}, wherein L^{C} is a covalent bond and R^{C} is Cy^{C}. In some embodiments, Cy^{C} is a cyclopropyl group.

In some embodiments, R⁶ is selected from hydrogen or L^{C}-R^{C}, wherein L^{C} is a covalent bond, and wherein R^{C} is selected from halogen, -N(R)₂, -OR, Cy^{C}, or an optionally substituted C₁₋₆ - aliphatic group.

In some embodiments of R⁶, Cy^{C} is a cyclopropyl group substituted with 0-4 L^{D}-R^{D} groups. In some embodiments of R⁶, Cy^{C} is a cyclopropyl group substituted with methyl or halogen. In some embodiments, L^{D} is a covalent bond and R^{D} is selected from halogen and optionally substituted C₁₋₆ aliphatic.

In some embodiments, R⁷ is selected from hydrogen or L^{C}-R^{C}, wherein L^{C} is a covalent bond, and wherein R^{C} is Cy^{C}.

In some embodiments, R⁷ is hydrogen.

In some embodiments of R⁷, Cy^{C} is:

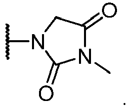

In some embodiments, R⁸ is hydrogen.

In some embodiments, R⁸ is selected from -OR or an optionally substituted C₁₋₆ aliphatic group.

In some embodiments, a provided compound is of Formula (II): 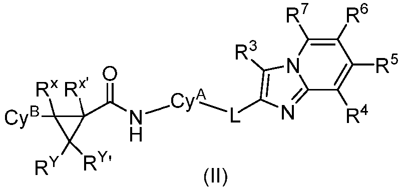
or a pharmaceutically acceptable salt thereof,
wherein each of Cy^{A}, Cy^{B}, L, R^{x}, R^{x'}, R^{Y}, R^{Y'}, R³, R⁴, R⁵, R⁶, and R⁷ is defined and described in classes and subclasses herein, both singly and in combination.

It will be understood that, unless otherwise specified or prohibited by the foregoing definition of Formula (II), embodiments of variables Cy^{A}, Cy^{B}, L, R^{x}, R^{x'}, R^{Y}, R^{Y'}, R³, R⁴, R⁵, R⁶, and R⁷ as defined above and described in classes and subclasses herein, also apply to compounds of Formula (II), both singly and in combination.
In some embodiments, a provided compound is of Formula (III-a), Formula (III-b), or Formula (III-c): 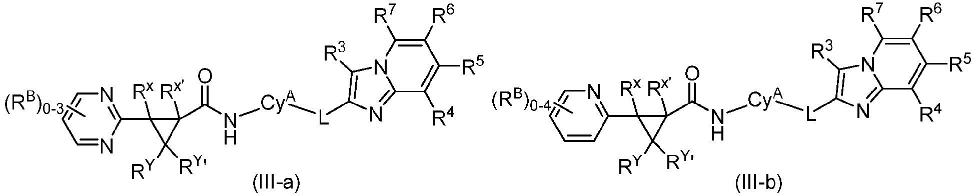 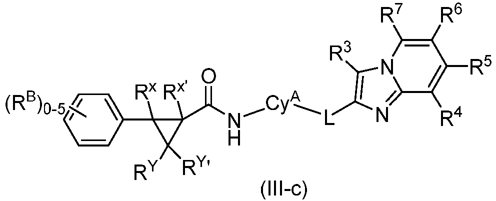
or a pharmaceutically acceptable salt thereof,
wherein each of Cy^{A}, R^{B}, L, R^{x}, R^{x'}, R^{Y}, R^{Y'}, R³, R⁴, R⁵, R⁶, and R⁷ is defined and described in classes and subclasses herein, both singly and in combination, in particular formula (III-a).

It will be understood that, unless otherwise specified or prohibited by the foregoing definitions of Formulae (III-a), (III-b), and (III-c), embodiments of variables Cy^{A}, R^{A}, L, R^{x}, R^{x'}, R^{Y}, R^{Y'}, R³, R⁴, R⁵, R⁶, and R⁷ as defined above and described in classes and subclasses herein, also apply to compounds of Formulae (III-a), (III-b), and (III-c), both singly and in combination.

In some embodiments, a provided compound is of Formula (IV-a), Formula (IV-b), Formula (IV-c), Formula (IV-d), Formula (IV-e), or Formula (IV-e): 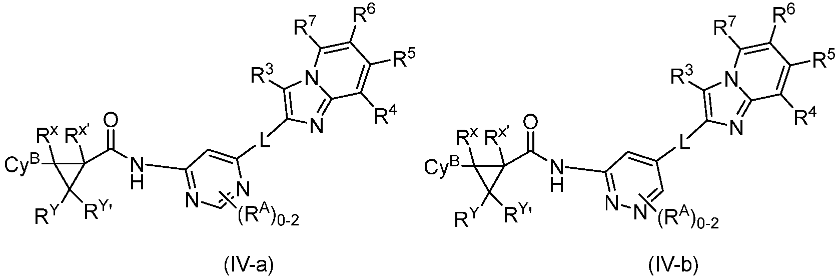 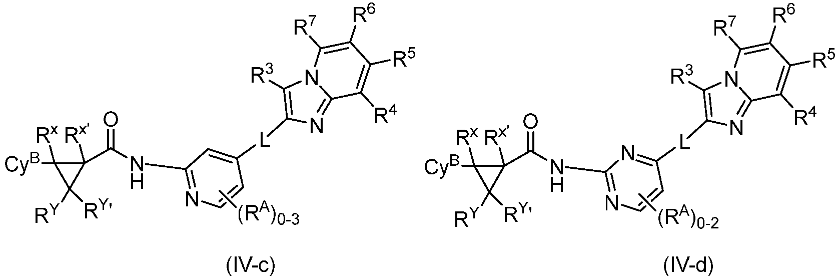 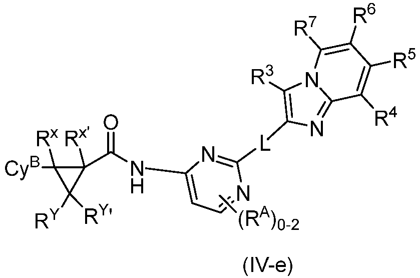 or a pharmaceutically acceptable salt thereof, in particular formula (IV-a).

It will be understood that, unless otherwise specified or prohibited by the foregoing definitions of Formulae (IV-a), (IV-b), (IV-c), (IV-d), and (IV-e), embodiments of variables Cy^{B}, R^{A}, L, R^{x}, R^{x'}, R^{Y}, R^{Y'}, R³, R⁴, R⁵, R⁶, and R⁷ as defined above and described in classes and subclasses herein, also apply to compounds of Formulae (IV-a), (IV-b), (IV-c), (IV-d), and (IV-e), both singly and in combination.

In some embodiments, a provided compound is of Formula (V-a), Formula (V-b), or Formula (V-c): 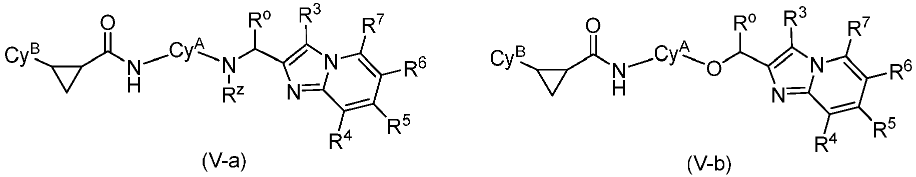 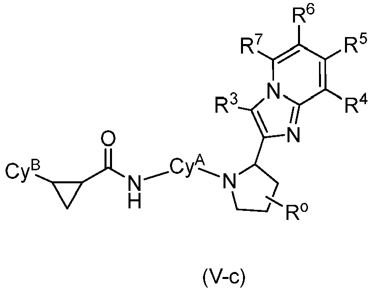
or a pharmaceutically acceptable salt thereof,
wherein each of Cy^{A}, Cy^{B}, R^{z}, R°, R³, R⁴, R⁵, R⁶, and R⁷ is defined and described in classes and subclasses herein, both singly and in combination, in particular formula (V-a).

It will be understood that, unless otherwise specified or prohibited by the foregoing definitions of Formulae (V-a), (V-b), and (V-c), embodiments of variables Cy^{A}, Cy^{B}, R^{z}, R°, R³, R⁴, R⁵, R⁶, and R⁷ as defined above and described in classes and subclasses herein, also apply to compounds of Formulae (V-a), (V-b), and (V-c), both singly and in combination.

In some embodiments, a provided compound is of Formula (V-a-1), Formula (V-b-1), or Formula (V-c-1): 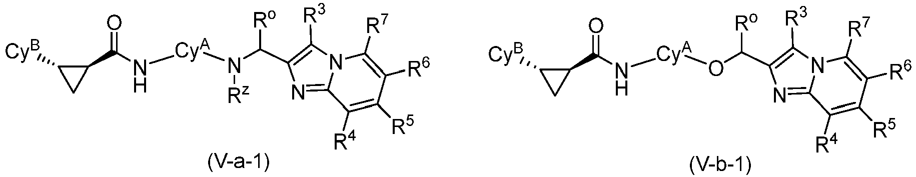 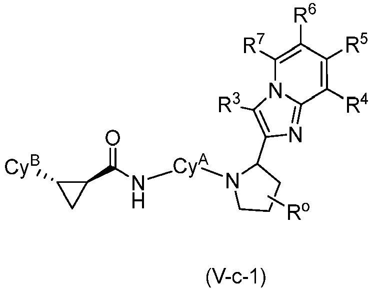
or a pharmaceutically acceptable salt thereof,
wherein each of Cy^{A}, Cy^{B}, R^{z}, R°, R³, R⁴, R⁵, R⁶, and R⁷ is defined and described in classes and subclasses herein, both singly and in combination, in particular formula (V-a-1).

It will be understood that, unless otherwise specified or prohibited by the foregoing definitions of Formulae (V-a), (V-b), (V-c), (V-a-1), (V-b-1), and (V-c-1), embodiments of variables Cy^{A}, Cy^{B}, R^{z}, R°, R³, R⁴, R⁵, R⁶, and R⁷ as defined above and described in classes and subclasses herein, also apply to compounds of Formulae (V-a), (V-b), (V-c), (V-a-1), (V-b-1), and (V-c-1), both singly and in combination.

In some embodiments, a provided compound is of Formula (VI-a), Formula (VI-b), or Formula (VI-c): 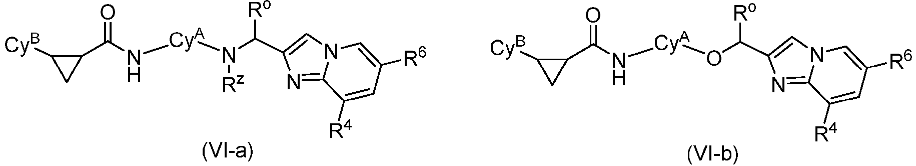 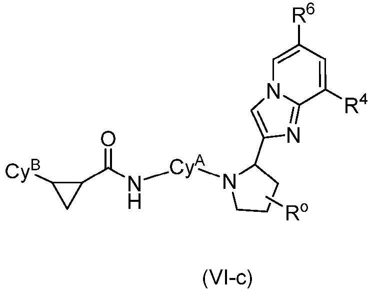
or a pharmaceutically acceptable salt thereof,
wherein each of Cy^{A}, Cy^{B}, R^{z}, and R° is defined and described in classes and subclasses herein, both singly and in combination;
   R⁴ is L^{C}-R^{C}, wherein L^{C} is a covalent bond and R^{C} is Cy^{C}, wherein Cy^{C} is a 5-membered saturated or partially unsaturated monocyclic heterocyclyl having 1-2 heteroatoms selected from nitrogen, and wherein Cy^{C} is substituted with 0-4 -L^{D}-R^{D} groups;
   R⁶ is L^{C}-R^{C}, wherein L^{C} is a covalent bond and R^{C} is C₁₋₆ aliphatic or Cy^{C}, wherein Cy^{C} is cyclopropyl optionally substituted with halogen, in particular (VI-a).

It will be understood that, unless otherwise specified or prohibited by the foregoing definitions of Formulae (VI-a), (VI-b), and (VI-c), embodiments of variables Cy^{A}, Cy^{B}, R^{z}, and R° as defined above and described in classes and subclasses herein, also apply to compounds of Formulae (VI-a), (VI-b), and (VI-c), both singly and in combination.

In some embodiments of Formulae (VI-a), (VI-b), and (VI-c), R⁴ is a ring selected from:

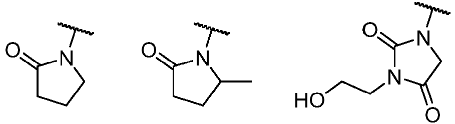

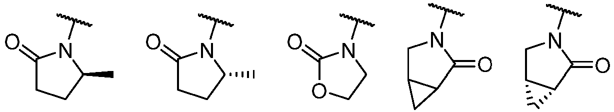

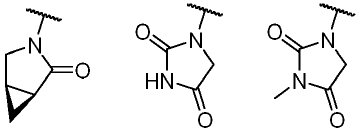

In some embodiments of Formulae (VI-a), (VI-b), and (VI-c), R⁴ is a ring selected from:

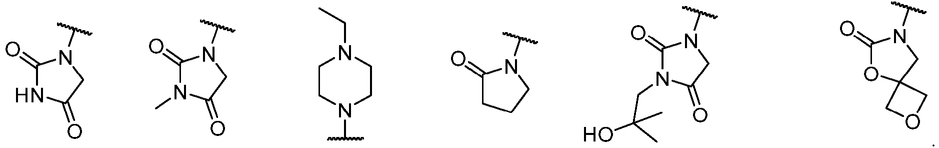

In some embodiments of Formulae (VI-a), (VI-b), and (VI-c), R⁴ is a ring selected from: 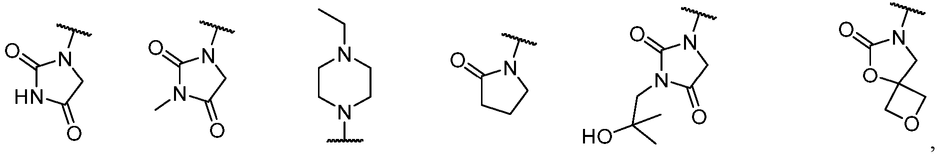 in particular:

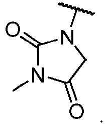

In some embodiments of Formulae (VI-a), (VI-b), and (VI-c), R⁴ is:

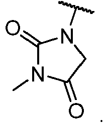

In certain embodiments of provided compounds (i.e., of any species not otherwise defined and of any for Formula (I) - (VI-c), the moiety: 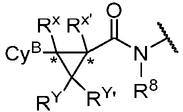 (including where one or more of R^{x}, R^{x'}, R^{Y}, R^{Y'}, or R⁸ is hydrogen) is in the relative trans configuration with respect to the Cy^{B} and amide group attached to the two stereocenters marked with an *. In other words, it will be appreciated that "trans" in the context of the moiety: 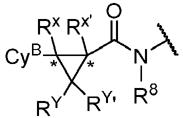 is meant a compound comprising a mixture of: 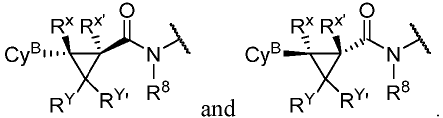 In some embodiments, such a mixture is a racemic mixture.

In certain embodiments of provided compounds (i.e., of any species not otherwise defined and of any of Formula (I) - (VI-c), the absolute stereochemistry of the moiety: 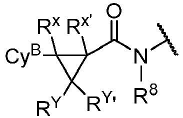 is as follows:

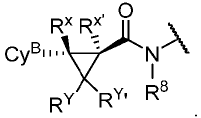

In certain embodiments of provided compounds (i.e., of any species not otherwise defined and of any of Formula (I) - (VI-c), the absolute stereochemistry of the moiety: 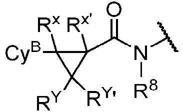 is as follows:

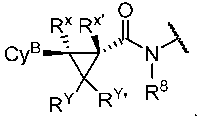

In some embodiments, a provided compound is selected from the group consisting of the following **Individual** compounds:
(**I-1**); (I-2); (**I-3**); (**I-4)**; (I-5); (1S,2S)-2-(3-chlorophenyl)-N-(6-(((6-cyclopropyl-8-((1-methylpiperidin-4-yl)amino)imidazo[1,2-a]pyridin-2-yl)methyl)amino)pyrimidin-4-yl)cyclopropane-1-carboxamide; (**I-6**); (**I-7**); (1S,25)-2-(3-chlorophenyl)-N-(6-(((6-cyclopropyl-8-(3-(hydroxymethyl)-4-methylpiperazin-1-yl)imidazo[1,2-a]pyridin-2-yl)methyl)amino)pyrimidin-4-yl)cyclopropane-1-carboxamide; **(1-8);** (**I-9**); (**I-10**); **(1-11);** (**I-12**); (**I-13**); (**I-14**); (**I-15**); (1S,2S)-2-(3-chlorophenyl)-N-(6-(((6-cyclopropyl-8-((R)-3-(dimethylamino)pyrrolidin-1-yl)imidazo[1,2-a]pyridin-2-yl)methyl)amino)pyrimidin-4-yl)cyclopropane-1-carboxamide; (1S,2S)-2-(3-chlorophenyl)-N-(6-(((6-cyclopropyl-8-((S)-3-(dimethylamino)pyrrolidin-1-yl)imidazo[1,2-a]pyridin-2-yl)methyl)amino) pyrimidin-4-yl)cyclo propane-1-carboxamide; (**I-16**); **(I-17); (I-18); (I-19); (I-20); (I-21);** (**I-22)**; **(I-23); (I-24); (I-25); (I-26); (I-27); (I-28); (I-29); (I-30); (I-31); (I-32); (I-33); (I-34); (I-35); (I-36); (I-37); (I-38); (I-39); (I-40); (I-41); (I-42); (I-43); (I-44); (I-45); (I-46); (I-47); (I-48);** *rac-*(1*S**,2*S**)-2-(5-chloro-2-cyanophenyl)-*N*-(6-(((6-cyclopropylimidazo[1,2-a]pyridin-2-yl)methyl)amino)pyrimidin-4-yl)cyclopropane-1-carboxamide; (**I-49**); *rac*-(*1S*,*2*S**)*-N-*(6-(((6-cyclopropylimidazo[1,2-a]pyridin-2-yl)methyl)amino)pyrimidin-4-yl)-2-(2,5-dichlorophenyl)cyclo propane-1-carboxamide; **(I-50);** *rac*-(1*S**,2*S**)-2-(3-chloro-6-cyano-2-fluoro phenyl)-*N*-(6-(((6-cyclopropylimidazo[1,2-a]pyridin-2-yl)methyl)amino)pyrimidin-4-yl)cyclopropane-1-carboxamide; **(I-51); (I-52); (I-53); (I-54); (I-55); (I-56); (I-57); (I-58); (I-59); (I-60); (I-61); (I-62); (I-63); (I-64); (I-65); (I-66); (I-67); (I-68);** *rac*-(1*S**,2*S**)-2-(3-chlorophenyl)-*N*-(6-(((6-cyclopropyl-8-(2-methyl-3-oxomorpholino)imidazo[1,2-a]pyridin-2-yl)methyl)amino)pyrimidin-4-yl)cyclopropane-1-carboxamide; **(I-69); (I-70);** *rac*-(1S*,2S*)-2-(3-chlorophenyl)-N-(6-(((6-cyclo propyl-8-(hydroxy methyl)imidazo[1,2-*a*]pyridin-2-yl)methyl)amino)pyrimidin-4-yl)cyclopropane-1-carboxamide; **(I-71); (I-72); (I-73); (I-74); (I-75); (I-76);** (1*S*,2*S*)-2-(3-chlorophenyl)-*N*-(6-(((6-cyclopropyl-8-(3-oxomorpholino)imidazo[1,2-*a*]pyridin-2-yl)methyl)amino)pyrimidin-4-yl)cyclopropane-1-carboxamide; (1*R*,2*R*)-2-(3-chlorophenyl)-*N*-(6-(((6-cyclopropyl-8-(3-oxomorpholino) imidazo[1,2-*a*]pyridin-2-yl)methyl)amino)pyrimidin-4-yl)cyclopropane-1-carboxamide; **(I-77); (I-78); (I-79); (I-80); (I-81);** (1*S*,2*S*)-2-(5-chloro-2-cyanophenyl)-*N*-(4-(((6-cyclopropyl-8-(2-oxopyrrolidin-1-yl)imidazo[1,2-a]pyridin-2-yl)methyl)amino)pyridin-2-yl)cyclopropane-1-carboxamide; (1R,2R)-2-(5-chloro-2-cyanophenyl)-N-(4-(((6-cyclopropyl-8-(2-oxopyrrolidin-1-yl)imidazo[1,2-a]pyridin-2-yl)methyl)amino)pyridin-2-yl)cyclopropane-1-carboxamide; **(I-82); (I-83); (I-84); (I-85); (I-86);** *rac-*(1*S*,*2*S**)-2-(3-chlorophenyl)-*N*-(4-(((6-cyclopropylimidazo[1,2-*a*]pyridin-2-yl)methyl)amino)-5-fluoropyridin-2-yl)cyclopropane-1-carboxamide; **(I-87); (I-88); (I-89); (I-90); (I-91);** (1*S*,2*S*)-2-(3-chlorophenyl)-*N*-(4-(((6-cyclopropyl-8-((*S*)-2-methyl-5-oxopyrrolidin-1-yl)imidazo[1,2-*a*]pyridin-2-yl)methyl)amino)pyridin-2-yl)cyclopropane-1-carboxamide; (1*S*,2*S*)-2-(3-chlorophenyl)-N-(4-(((6-cyclopropyl-8-((*R*)-2-methyl-5-oxopyrrolidin-1-yl)imidazo[1,2-*a*]pyridin-2-yl)methyl)amino)pyridin-2-yl)cyclopropane-1-carboxamide; **(I-92); (I-93); (I-94);** (1*S*,2*S*)-2-(3-chlorophenyl)-*N*-(4-(((6-cyclopropyl-8-((1*R*,4*S*)-3-oxo-2-azabicyclo[2.2.1]heptan-2-yl)imidazo[1,2-a]pyridin-2-yl)methyl)amino)pyridin-2-yl)cyclopropane-1-carboxamide; (1*S*,2*S*)-2-(3-chlorophenyl)-N-(4-(((6-cyclopropyl-8-((1*S*,4*R*)-3-oxo-2-azabicyclo[2.2.1]heptan-2-yl)imidazo[1,2-*a*]pyridin-2-yl)methyl)amino)pyridin-2-yl)cyclopropane-1-carboxamide; **(I-95); (I-96); (I-97);** (1*S*,2*S*)-2-(3-chlorophenyl)-*N*-(4-(((6-cyclopropyl-8-((1*S*,5*R*)-2-oxo-3-azabicyclo[3.1.0]hexan-3-yl)imidazo[1,2-*a*]pyridin-2-yl)methyl)amino)pyridin-2-yl)cyclopropane-1-carboxamide; (1*S*,2*S*)-2-(3-chloropheny1)-*N-*(4-(((6-cyclopropyl-8-((1*R*,5*S*)-2-oxo-3-azabicyclo[3.1.0]hexan-3-yl)imidazo[1,2-*a*]pyridin-2-yl)methyl)amino)pyridin-2-yl)cyclopropane-1-carboxamide; **(I-98); (I-99); (I-100); (I-101); (I-102); (I-103); (I-104); (I-105); (I-106); (I-107); (I-108); (I-109); (I-110); (1-111); (I-112); (I-113); (I-114); (I-115); (1-116); (I-117); (I-118); (1-119); (I-120); (1-121); (I-122); (I-123); (I-124); (I-125); (I-126); (I-127); (I-128);** (1*S*,2*S*)-2-(3-chlorophenyl)-*N*-(4-(((6-cyclopropyl-8-(*N-*methylmethylsulfonamido)imidazo[1,2-*a*]pyridin-2-yl)methyl)amino)pyridin-2-yl)cyclopropane-1-carboxamide; **(I-129); (I-130); (I-131); (I-132); (I-133);** (1*S*,2*S*)-2-(3-chlorophenyl)-*N*-(4-(((6-cyclopropyl-8-(3-fluoroazetidin-3-yl)imidazo[1,2-a]pyridin-2-yl)methyl)amino)pyridin-2-yl)cyclopropane-1-carboxamide; **(I-134); (I-135); (I-137); (I-138); (I-139); (I-140); (I-141);** ((1*S*,2*S*)-2-(3-chlorophenyl)-*N*-(4-((1-(6-cyclopropylimidazo[1,2-*a*]pyridin-2-yl)-2,2,2-trifluoroethyl)amino)pyridin-2-yl)cyclopropane-1-carboxamide; **(I-142);** (1*S*,2*S*)-*N-*(4-(((8-acetamido-6-cyclopropylimidazo[1,2-a]pyridin-2-yl)methyl)amino)pyridin-2-yl)-2-(3-chlorophenyl)cyclopropane-1-carboxamide; **(I-143); (I-144); (I-145); (I-146); (I-147); (I-148); (1-149); (I-150); (I-151); (I-152); (I-153); (I-154); (I-155); (I-156); (I-157); (I-158); (1-159); (I-160); (I-161); (I-162); (I-163); (I-164); (I-165); (I-166); (I-167); (I-168); ((1-169); (I-170); (1-171); (I-172); (I-173); (I-174); (I-175); (I-176); (I-177); (I-178); (I-179); (I-180); (1-181); (I-182); (I-183); (I-184); (I-185); (I-186); (I-187); (I-188); (I-189); (I-190); (1-191); (I-192); (I-193); (I-194); (I-195); (I-196); (I-197); ((I-198); (I-199); (I-200); (1-201); (I-202); (I-203); (I-204); (I-205); (I-206); (I-207); (I-208); (I-209); (I-210); (I-211); (I-212); (I-213); (I-214); (1-216); (I-217); (I-218); (I-219); (I-220); (1-221); (I-222); (I-223); (I-224); (I-225); (I-226); (I-227); (I-228); (I-229); ((I-230); (1-231); (I-232); (I-233); (I-234); (I-235); (I-236); (I-237); (I-238); (I-239); (I-240); (I-241); (I-242); (I-243); (I-244); (I-245); (I-246); (I-247); (I-248);** (1S,2S)-2-(4-chloropyridin-2-yl)-N-(6-((2R,4S)-2-(6-cyclopropylimidazo[1,2-a]pyridin-2-yl)-4-hydroxypyrrolidin-1-yl)pyrimidin-4-yl)cyclopropane-1-carboxamide; (1R,2R)-2-(4-chloropyridin-2-yl)-N-(6-((2R,4S)-2-(6-cyclopropylimidazo[1,2-a]pyridin-2-yl)-4-hydroxypyrrolidin-1-yl)pyrimidin-4-yl)cyclopropane-1-carboxamide; **(I-249);** (1S,2S)-2-(4-chloropyridin-2-yl)-N-(6-((2R,4S)-2-(6-cyclopropylimidazo[1,2-a]pyridin-2-yl)-4-hydroxypyrrolidin-1-yl)pyrimidin-4-yl)cyclopropane-1-carboxamide; (1R,2R)-2-(4-chloropyridin-2-yl)-N-(6-((2R,4S)-2-(6-cyclopropylimidazo[1,2-a]pyridin-2-yl)-4-hydroxypyrrolidin-1-yl)pyrimidin-4-yl)cyclopropane-1-carboxamide; **(I-250); (I-251); (I-252); (I-253); (I-254); (I-255); (I-256); (I-257), (I-258);** (1S,2S)-2-(3-amino-5-chlorophenyl)-N-(6-(((6-cyclopropylimidazo[1,2-a]pyridin-2-yl)methyl)amino)pyrimidin-4-yl)cyclopropane-1-carboxamide; (1R,2R)-2-(3-amino-5-chlorophenyl)-N-(6-(((6-cyclopropylimidazo [1,2-a]pyridin-2-yl)methyl)amino)pyrimidin-4-yl)cyclopropane-1-carboxamide; **(I-259); (I-260); (I-261); (I-262); (I-263); (I-264); (I-265); (I-266); (I-267);** (1R,2R)-2-(4-chloropyridin-2-yl)-N-(6-(((6-cyclopropylimidazo[1,2-a]pyridin-2-yl)methyl)amino)pyrimidin-4-yl)cyclopropane-1-carboxamide; (1S,2S)-2-(4-chloropyridin-2-yl)-N-(6-(((6-cyclopropylimidazo[1,2-a]pyridin-2-yl)methyl)amino) pyrimidin-4-yl)cyclopropane-1-carboxamide; **(I-268); (I-269); (I-270);** (1R,2R)-2-(4-chloropyridin-2-yl)-N-(4-(((6-cyclopropylimidazo[1,2-a]pyridin-2-yl)methyl)amino)pyridin-2-yl)cyclopropane-1-carboxamide; (1S,2S)-2-(4-chloropyridin-2-yl)-N-(4-(((6-cyclopropylimidazo[1,2-a]pyridin-2-yl)methyl)amino)pyridin-2-yl)cyclopropane-1-carboxamide; **(I-271); (I-272); I-273);** (1R,2R)-6'-chloro-N-(4-(((6-cyclopropylimidazo[1,2-a]pyridin-2-yl)methyl)amino)pyridin-2-yl)-2',3'-dihydrospiro[cyclopropane-1,1'-indene]-2-carboxamide; (1S,2S)-6'-chloro-N-(4-(((6-cyclopropyl imidazo[1,2-a]pyridin-2-yl)methyl)amino)pyridin-2-yl)-2',3'-dihydrospiro[cyclopropane-1,1'-indene]-2-carboxamide; **(I-274); (I-275); (I-276); (I-277); (I-278); (I-279); (I-280); (1-281); (I-282); (I-283); (I-284); (I-285); (I-286); (I-287); (I-288); (I-289); (I-290); (1-291); (I-292); (I-293); (I-294); (I-295); (I-296); (I-297); (I-298); (I-299); (I-300); (I-301); (I-302); (I-303); (I-304); (I-305); (I-306); (I-307); (I-308); (I-309); (I-310);** rac-(1*S**,2*S*%*)-2-(5-chloro-2-(2-methoxyethoxy)phenyl)-*N*-(6-(((6-cyclopropyl imidazo[1,2-a]pyridin-2-yl)methyl)amino)pyrimidin-4-yl)cyclopropane-1-carboxamide; **(I-311); (I-312); (I-313); (I-314);** *rac*-(1*S**,2*S**)-2-(6-cyano-2-fluoro-3-methoxyphenyl)-N-(6-(((6-cyclopropylimidazo[1,2-*a*]pyridin-2-yl)methyl)amino)pyrimidin-4-yl)cyclopropane-1-carboxamide; **(I-315); (1-316); (1-317); (I-318); (I-319); (I-320); (I-321);** (1*S*,2*S*)-*N*-(6-(((6-cyclopropyl-8-((1*R*,5*S*)-2-oxo-3-azabicyclo[3.1.0]hexan-3-yl)imidazo[1,2-*a*]pyridin-2-yl)methyl)amino)pyrimidin-4-yl)-2-(4-methylpyrimidin-2-yl)cyclopropane-1-carboxamide; (1*R*,2*R*)-*N*-(6-(((6-cyclopropyl-8-((1*R*,5*S*)-2-oxo-3-azabicyclo[3.1.0]hexan-3-yl)imidazo[1,2-*a*]pyridin-2-yl)methyl)amino)pyrimidin-4-yl)-2-(4-methyl pyrimidin-2-yl)cyclopropane-1-carboxamide; (1*S*,2*S*)-*N*-(6-(((6-cyclopropyl-8-((1*S*,5*R*)-2-oxo-3-azabicyclo[3.1.0]hexan-3-yl)imidazo[1,2-*a*]pyridin-2-yl)methyl)amino)pyrimidin-4-yl)-2-(4-methyl pyrimidin-2-yl)cyclopropane-1-carboxamide; (1*R*,2*R*)-*N-*(6-(((6-cyclopropyl-8-((1*S*,5*R*)-2-oxo-3-azabicyclo[3.1.0]hexan-3-yl)imidazo[1,2-*a*]pyridin-2-yl)methyl)amino)pyrimidin-4-yl)-2-(4-methylpyrimidin-2-yl)cyclopropane-1-carboxamide; **(I-322); (I-323); (I-324); (I-325); (I-326); (I-327); (I-328); (I-329); (I-330); (I-331); (I-332); (I-333);** (1*R*,2*R*)-2-(3-chlorophenyl)-*N*-(6-(((6-cyclopropyl-8-(3-methyl-2,4-dioxoimidazolidin-1-yl)imidazo[1,2-*a*]pyridin-2-yl)methyl)amino)pyrimidin-4-yl)-2-fluorocyclopropane-1-carboxamide; (1*S*,2*S*)-2-(3-chlorophenyl)-*N*-(6-(((6-cyclopropyl-8-(3-methyl-2,4-dioxoimidazolidin-1-yl)imidazo[1,2-*a*]pyridin-2-yl)methyl)amino)pyrimidin-4-yl)-2-fluorocyclo propane-1-carboxamide; (1*R*,2*S*)-2-(3-chlorophenyl)-*N*-(6-(((6-cyclopropyl-8-(3-methyl-2,4-dioxoimidazolidin-1-yl)imidazo[1,2-*a*]pyridin-2-yl)methyl)amino)pyrimidin-4-yl)-2-fluorocyclo propane-1-carboxamide; (1*S*,2*R*)-2-(3-chlorophenyl)-*N*-(6-(((6-cyclopropyl-8-(3-methyl-2,4-dioxoimidazolidin-1-yl)imidazo[1,2-*a*]pyridin-2-yl)methyl)amino)pyrimidin-4-yl)-2-fluorocyclo propane-1-carboxamide; **(I-334); (I-335); (I-336);** (1*S*,2*S*)-2-(*S*-chloro-2-cyanophenyl)-N-(6-(((6-cyclopropyl-8-(2-oxopyrrolidin-1-yl)imidazo[1,2-*a*]pyridin-2-yl)methyl)amino)pyrimidin-4-yl)cyclopropane-1-carboxamide; (1*R*,2*R*)-2-(5-chloro-2-cyanophenyl)-N-(6-(((6-cyclopropyl-8-(2-oxopyrrolidin-1-yl)imidazo[1,2-*a*]pyridin-2-yl)methyl)amino)pyrimidin-4-yl)cyclopropane-1-carboxamide; **(I-337); (I-338); (I-339); (I-340); (I-341); (I-342); (I-343); (I-344); (I-345); (I-346); ((I-347); (I-348); (I-349); (I-350); (I-351); (I-352); (I-353); (I-354); (I-355); (I-356); (I-357); (I-358); (I-359); (I-360); (I-361); (I-362); (I-363); (I-364); (I-365); (I-366); (I-367); (I-368); (I-369); (I-370); (I-371); (I-372); (I-373); (I-374); (I-375); (I-376); (I-377); (I-378); (I-379); (I-380); (I-381); (I-382); (I-383); (I-384); (I-385); (I-386); (I-387); (I-388); (I-389); (I-390); (I-391); (I-392); (I-393); (I-394); (I-395); (I-396); (I-397); (I-398); (I-399); (I-400); (I-401); (I-402); (I-403); (I-404); (I-405); (I-406); (I-407); (I-408); (I-409); (I-410); (I-411); (I-412); (I-413); (I-414); (I-415); (I-416 (I-417); (I-418); (I-419); (I-420); (I-421); (I-422); (I-423); (I-424); (I-425); (I-426); (I-427); (I-428); (I-429); (I-430); (I-431); (I-432); (I-433); (I-434); (I-435); (I-436); (I-437); (I-438); (I-439); (I-440); (1-441); (I-442); (I-443); (I-444); (I-445);** (1S,2S)-N-(6-(((R)-1-(6-cyclopropyl-8-((1S,5R)-2-oxo-3-azabicyclo[3.1.0]hexan-3-yl)imidazo[1,2-a]pyridin-2-yl)ethyl)amino)pyrimidin-4-yl)-2-(4-methylpyrimidin-2-yl)cyclopropane-1-carboxamide; (1S,2S)-N-(6-(((R)-1-(6-cyclopropyl-8-((1R,5S)-2-oxo-3-azabicyclo[3.1.0]hexan-3-yl)imidazo[1,2-a]pyridin-2-yl)ethyl)amino)pyrimidin-4-yl)-2-(4-methylpyrimidin-2-yl)cyclopropane-1-carboxamide; **(I-446); (I-447); (I-448);** (1S,2S)-N-(6-(((6-cyclopropyl-8-(3-methyl-2,4-dioxoimidazolidin-1-yl)imidazo[1,2-a]pyridin-2-yl)methyl)amino) pyrimidin-4-yl)-2-(4-methylthiazol-2-yl)cyclopropane-1-carboxamide; (1R,2R)-N-(6-(((6-cyclopropyl-8-(3-methyl-2,4-dioxoimidazolidin-1-yl)imidazo[1,2-a]pyridin-2-yl)methyl)amino)pyrimidin-4-yl)-2-(4-methylthiazol-2-yl)cyclopropane-1-carboxamide; N-(6-((1-(6-cyclopropyl-8-(3-methyl-2,4-dioxo imidazolidin-1-yl)imidazo[1,2-a]pyridin-2-yl)ethyl)amino)pyrimidin-4-yl)-2-(4-methylpyrimidin-2-yl)cyclopropane-1-carboxamide; **(I-449); (I-450); (I-451); (I-452); (I-453);** (1R,2R)-N-(6-(((R)-1-(6-cyclopropyl-8-(3-methyl-2,4-dioxoimidazolidin-1-yl)imidazo[1,2-a]pyridin-2-yl)ethyl)amino) pyrimidin-4-yl)-2-(4-methylpyrimidin-2-yl)cyclopropane-1-carboxamide; (1R,2R)-N-(6-(((S)-1-(6-cyclopropyl-8-(3-methyl-2,4-dioxoimidazolidin-1-yl)imidazo[1,2-a]pyridin-2-yl)ethyl)amino) pyrimidin-4-yl)-2-(4-methylpyrimidin-2-yl)cyclopropane-1-carboxamide, N-(6-((1-(6-cyclopropyl-8-(3-methyl-2,4-dioxoimidazolidin-1-yl)imidazo[1,2-a]pyridin-2-yl)ethyl)amino)-2-methylpyrimidin-4-yl)-2-(4-methylpyrimidin-2-yl)cyclopropane-1-carboxamide; **(I-454); (I-455); (I-456); (I-457); (I-458); (I-459);** (1S,2S)-2-(3-chlorophenyl)-N-(6-(((6-cyclopropyl-8-((1R,5S)-2-oxo-3-azabicyclo [3.1.0]hexan-3-yl)imidazo[1,2-a]pyridin-2-yl)methyl)amino)pyrimidin-4-yl)cyclopropane-1-carboxamide; (1S,2S)-N-(5-((6-cyclopropyl-8-((1S,5R)-2-oxo-3-azabicyclo[3.1.0]hexan-3-yl)imidazo[1,2-a]pyridin-2-yl)methoxy)pyridazin-3-yl)-2-(4-methylpyrimidin-2-yl)cyclopropane-1-carboxamide; **(I-460); (I-461); (I-462); (I-463); (I-464); (I-465); (I-466); (I-467); (I-468); (I-469); (I-470); (I-471); (I-472); (I-473); (I-474); (I-475); (I-476); (I-477); (I-478); (I-479); (I-480); (I-481); (I-482); (I-483); (I-484); (I-485); (I-486); (I-487); (I-488); (I-489); (I-490); (I-491); (I-492); (I-493); (I-494); (I-495); (I-496); (I-497); (I-498); (I-499); (I-500); (I-501); (I-502); (I-503); (I-504); (I-505); (I-506); (I-507); (I-508);** (1R,2R,3S)-2-(3-chlorophenyl)-N-(6-(((6-cyclopropyl imidazo[1,2-a]pyridin-2-yl)methyl)amino)pyrimidin-4-yl)-3-methylcyclopropane-1-carboxamide; (1R,2R,3R)-2-(3-chlorophenyl)-N-(6-(((6-cyclopropylimidazo[1,2-a]pyridin-2-yl)methyl)amino) pyrimidin-4-yl)-3-methylcyclopropane-1-carboxamide; (1S,2S,3S)-2-(3-chlorophenyl)-N-(6-(((6-cyclopropylimidazo[1,2-a]pyridin-2-yl)methyl)amino)pyrimidin-4-yl)-3-methylcyclopropane-1-carboxamide; (1S,2S,3R)-2-(3-chlorophenyl)-N-(6-(((6-cyclopropylimidazo[1,2-a]pyridin-2-yl)methyl)amino)pyrimidin-4-yl)-3-methylcyclopropane-1-carboxamide; **(I-509); (I-510); (I-511); (I-512); (I-513); (I-514); (I-515); (I-516); (I-517); (I-518); (I-519); (I-520); (1-521); (I-522); (I-523); (I-524); (I-525); (I-526); (I-527); (I-528); (I-529); (I-530); (1-531); (I-532); (I-533); (I-534); (I-535); (I-536); (I-537); (I-538); (I-539); (I-540);** e **(I-541); (I-542); (I-543); (I-544); (I-545);** (1R,3R)-3-(3-chlorophenyl)-N-(6-(((6-cyclopropyl-8-(3-methyl-2,4-dioxoimidazolidin-1-yl)imidazo[1,2-a]pyridin-2-yl)methyl)amino)pyrimidin-4-yl)-2,2-difluorocyclopropane-1-carboxamide; (1S,3S)-3-(3-chlorophenyl)-N-(6-(((6-cyclopropyl-8-(3-methyl-2,4-dioxoimidazolidin-1-yl)imidazo [1,2-a]pyridin-2-yl)methyl)amino)pyrimidin-4-yl)-2,2-difluorocyclopropane-1-carboxamide; **(I-546); (I-547); (I-548);** (1S,25)-N-(6-(((R)-1-(6-cyclopropyl-8-(3-methyl-2,4-dioxoimidazolidin-1-yl)imidazo[1,2-a]pyridin-2-yl)ethyl)amino)-2-methylpyrimidin-4-yl)-2-fluoro-2-(4-methylpyrimidin-2-yl)cyclopropane-1-carboxamide; (1R,2R)-N-(6-(((R)-1-(6-cyclopropyl-8-(3-methyl-2,4-dioxoimidazolidin-1-yl)imidazo[1,2-a]pyridin-2-yl)ethyl)amino)-2-methylpyrimidin-4-yl)-2-fluoro-2-(4-methylpyrimidin-2-yl)cyclopropane-1-carboxamide; **(I-549); (I-550); (I-551); (I-552); (I-553); (I-554); (I-555); (I-556); (I-557); (I-558); (I-559); (I-560); (1-561); (I-562); (I-563); (I-564); (I-565); (I-566); (I-567); (I-568); (I-569); (I-570); (1-571); (I-572); (I-573); (I-574); (I-575); (I-576); (I-577); (I-578); (I-579); (I-580); (1-581); (I-582); (I-583); (I-584); (I-585); (I-586); (I-587); (I-588); (I-589); (I-590),** Isomer 2; (1S*,2S*)-2-(3-chlorophenyl)-N-((S)-1-(1-((6-cyclopropyl imidazo[1,2-a]pyridin-2-yl)methyl)-1H-1,2,3-triazol-4-yl)ethyl)cyclopropane-1-carboxamide; (1S,2S)-2-(3-chlorophenyl)-N-((S)-1-(1-((6-cyclopropylimidazo[1,2-a]pyridin-2-yl)methyl)-1H-1,2,3-triazol-4-yl)ethyl)cyclopropane-1-carboxamide; (1R,2R)-2-(3-chlorophenyl)-N-((S)-1-(1-((6-cyclopropyl imidazo[1,2-a]pyridin-2-yl)methyl)-1H-1,2,3-triazol-4-yl)ethyl)cyclopropane-1-carboxamide; (1S*,2S*)-2-(3-chlorophenyl)-N-((R)-1-(1-((6-cyclopropylimidazo[1,2-a]pyridin-2-yl)methyl)-1H-1,2,3-triazol-4-yl)ethyl)cyclopropane-1-carboxamide; (1S,2S)-2-(3-chlorophenyl)-N-((R)-1-(1-((6-cyclopropylimidazo[1,2-a]pyridin-2-yl)methyl)-1H-1,2,3-triazol-4-yl)ethyl)cyclopropane-1-carboxamide; (1R,2R)-2-(3-chlorophenyl)-N-((R)-1-(1-((6-cyclopropylimidazo[1,2-a]pyridin-2-yl)methyl)-1H-1,2,3-triazol-4-yl)ethyl)cyclopropane-1-carboxamide; **(I-591); (I-592); (I-593); (I-594); (I-595); (I-596); (I-597); (I-598); (I-599); (I-600); (1-601); (I-602); (I-603); (I-604); (I-605); (I-606); (I-607); (I-608); (I-609); (I-610); (1-611); (I-612 (I-613); (I-614); (I-615); (I-616); (I-617); (I-618); (I-619); (I-620); (1-621); (I-622); (I-623); (I-624); (I-625); (I-626); (I-627); (I-628); (I-629); (I-630); (I-631); (I-632); (I-633); (I-634);** (1R,2R)-N-(2-((6-cyclopropyl-8-(3-methyl-2,4-dioxoimidazolidin-1-yl)imidazo[1,2-a]pyridin-2-yl)methyl)-2H-pyrazolo[3,4-d]pyrimidin-4-yl)-2-(4-methylpyrimidin-2-yl)cyclopropane-1-carboxamide; (1S,2S)-N-(2-((6-cyclopropyl-8-(3-methyl-2,4-dioxoimidazolidin-1-yl)imidazo[1,2-a]pyridin-2-yl)methyl)-2H-pyrazolo[3,4-d]pyrimidin-4-yl)-2-(4-methylpyrimidin-2-yl)cyclopropane-1-carboxamide; **(I-635); (I-636); (I-637); (I-638); (I-639); (I-640); (I-641); (I-642); (I-643); (I-644); (I-645); (I-646); (I-647); (I-648); (I-649); (I-650); (I-651); (I-652); (I-653); (I-654); (I-655); (I-656); (I-657); (I-658); (I-659); (I-660); (1-661); (I-662); (I-663); (I-664); (I-665); (I-666); (I-667); (I-668); (I-669); (I-670); (1-671); (I-672); (I-673); (I-674); (I-675); (I-676); (I-677); (I-678);** and **(I-679);** and pharmaceutically acceptable salts thereof.

Compounds explicitly disclosed herein may be claimed as an individual compound, including where there is no reference to stereochemistry.

Processes for preparing compounds of the disclosure are described herein below.

### C. Pharmaceutical Compositions

In another aspect, the present invention provides pharmaceutical compositions comprising a compound of the present disclosure, including Formulae (I)-(VI-c) or a compound of Formulae (I)-(VI-c) and examples in combination with a pharmaceutically acceptable excipient (e.g., carrier).

The pharmaceutical compositions include optical isomers, diastereomers, or pharmaceutically acceptable salts of the inhibitors disclosed herein. A compound of Formulae (I)-(VI-c) included in the pharmaceutical composition may be covalently attached to a carrier moiety, as described above. Alternatively, a compound of Formulae (I)-(VI-c) included in the pharmaceutical composition is not covalently linked to a carrier moiety.

A "pharmaceutically acceptable carrier," as used herein refers to pharmaceutical excipients, for example, pharmaceutically, physiologically, acceptable organic or inorganic carrier substances suitable for enteral or parenteral application that do not deleteriously react with the active agent. Suitable pharmaceutically acceptable carriers include water, salt solutions (such as Ringer's solution), alcohols, oils, gelatins, and carbohydrates such as lactose, amylose or starch, fatty acid esters, hydroxymethycellulose, and polyvinyl pyrrolidine. Such preparations can be sterilized and, if desired, mixed with auxiliary agents such as lubricants, preservatives, stabilizers, wetting agents, emulsifiers, salts for influencing osmotic pressure, buffers, coloring, and/or aromatic substances and the like that do not deleteriously react with the compounds of the invention.

The compounds of the invention can be administered alone or can be coadministered to the subject. Coadministration is meant to include simultaneous or sequential administration of the compounds individually or in combination (more than one compound). The preparations can also be combined, when desired, with other active substances (e.g. to reduce metabolic degradation).

In some embodiments, a compound as described herein can be incorporated into a pharmaceutical composition for administration by methods known to those skilled in the art and described herein for provided compounds.

### D. Formulations

Compounds of the present invention can be prepared and administered in a wide variety of oral, parenteral, and topical dosage forms. Thus, the compounds of the present invention can be administered by injection (e.g. intravenously, intramuscularly, intracutaneously, subcutaneously, intraduodenally, or intraperitoneally). In some embodiments compounds of the present disclosure are administered orally. Also, the compounds described herein can be administered by inhalation, for example, intranasally. Additionally, the compounds of the present invention can be administered transdermally. It is also envisioned that multiple routes of administration (e.g., intramuscular, oral, transdermal) can be used to administer the compounds of the invention. Accordingly, the present invention also provides pharmaceutical compositions comprising a pharmaceutically acceptable carrier or excipient and one or more compounds of the invention.

For preparing pharmaceutical compositions from the compounds of the present invention, pharmaceutically acceptable carriers can be either solid or liquid. Solid form preparations include powders, tablets, pills, capsules, cachets, suppositories, and dispersible granules. A solid carrier can be one or more substance that may also act as diluents, flavoring agents, binders, preservatives, tablet disintegrating agents, or an encapsulating material.

In powders, the carrier is a finely divided solid in a mixture with the finely divided active component. In tablets, the active component is mixed with the carrier having the necessary binding properties in suitable proportions and compacted in the shape and size desired.

The powders and tablets preferably contain from 5% to 70% of the active compound. Suitable carriers are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as a carrier providing a capsule in which the active component with or without other carriers, is surrounded by a carrier, which is thus in association with it. Similarly, cachets and lozenges are included. Tablets, powders, capsules, pills, cachets, and lozenges can be used as solid dosage forms suitable for oral administration.

For preparing suppositories, a low melting wax, such as a mixture of fatty acid glycerides or cocoa butter, is first melted and the active component is dispersed homogeneously therein, as by stirring. The molten homogeneous mixture is then poured into convenient sized molds, allowed to cool, and thereby to solidify.

Liquid form preparations include solutions, suspensions, and emulsions, for example, water or water/propylene glycol solutions. For parenteral injection, liquid preparations can be formulated in solution in aqueous polyethylene glycol solution.

When parenteral application is needed or desired, particularly suitable admixtures for the compounds of the invention are injectable, sterile solutions, preferably oily or aqueous solutions, as well as suspensions, emulsions, or implants, including suppositories. In particular, carriers for parenteral administration include aqueous solutions of dextrose, saline, pure water, ethanol, glycerol, propylene glycol, peanut oil, sesame oil, polyoxyethylene-block polymers, and the like. Ampoules are convenient unit dosages. The compounds of the invention can also be incorporated into liposomes or administered via transdermal pumps or patches. Pharmaceutical admixtures suitable for use in the present invention include those described, for example, in Pharmaceutical Sciences (17th Ed., Mack Pub. Co., Easton, PA) and WO 96/05309.

Aqueous solutions suitable for oral use can be prepared by dissolving the active component in water and adding suitable colorants, flavors, stabilizers, and thickening agents as desired. Aqueous suspensions suitable for oral use can be made by dispersing the finely divided active component in water with viscous material, such as natural or synthetic gums, resins, methylcellulose, sodium carboxymethylcellulose, and other well-known suspending agents.

Also included are solid form preparations that are intended to be converted, shortly before use, to liquid form preparations for oral administration. Such liquid forms include solutions, suspensions, and emulsions. These preparations may contain, in addition to the active component, colorants, flavors, stabilizers, buffers, artificial and natural sweeteners, dispersants, thickeners, solubilizing agents, and the like.

The pharmaceutical preparation is preferably in unit dosage form. In such form the preparation is subdivided into unit doses containing appropriate quantities of the active component. The unit dosage form can be a packaged preparation, the package containing discrete quantities of preparation, such as packeted tablets, capsules, and powders in vials or ampoules. Also, the unit dosage form can be a capsule, tablet, cachet, or lozenge itself, or it can be the appropriate number of any of these in packaged form.

The quantity of active component in a unit dose preparation may be varied or adjusted according to the particular application and the potency of the active component. The composition can, if desired, also contain other compatible therapeutic agents.

Some compounds may have limited solubility in water and therefore may require a surfactant or other appropriate co-solvent in the composition. Such co-solvents include: Polysorbate 20, 60, and 80; Pluronic F-68, F-84, and P-103; cyclodextrin; and polyoxyl 35 castor oil. Such co-solvents are typically employed at a level between about 0.01 % and about 2% by weight.

Viscosity greater than that of simple aqueous solutions may be desirable to decrease variability in dispensing the formulations, to decrease physical separation of components of a suspension or emulsion of formulation, and/or otherwise to improve the formulation. Such viscosity building agents include, for example, polyvinyl alcohol, polyvinyl pyrrolidone, methyl cellulose, hydroxy propyl methylcellulose, hydroxyethyl cellulose, carboxymethyl cellulose, hydroxy propyl cellulose, chondroitin sulfate and salts thereof, hyaluronic acid and salts thereof, and combinations of the foregoing. Such agents are typically employed at a level between about 0.01% and about 2% by weight.

The compositions of the present invention may additionally include components to provide sustained release and/or comfort. Such components include high molecular weight, anionic mucomimetic polymers, gelling polysaccharides, and finely-divided drug carrier substrates. These components are discussed in greater detail in USP: 4,911,920; 5,403,841; 5,212,162; and 4,861,760..

### E. Effective Dosages

Pharmaceutical compositions provided by the present invention include compositions wherein the active ingredient is contained in a therapeutically effective amount, i.e., in an amount effective to achieve its intended purpose. The actual amount effective for a particular application will depend, *inter alia,* on the condition being treated. For example, when administered in methods to treat HAE, such compositions will contain an amount of active ingredient effective to achieve the desired result (e.g. inhibiting PKa and/or decreasing the amount of bradykinin in a subject).

The dosage and frequency (single or multiple doses) of compound administered can vary depending upon a variety of factors, including route of administration; size, age, sex, health, body weight, body mass index, and diet of the recipient; nature and extent of symptoms of the disease being treated (e.g., the disease responsive to PKa inhibition); presence of other diseases or other health-related problems; kind of concurrent treatment; and complications from any disease or treatment regimen. Other therapeutic regimens or agents can be used in conjunction with the methods and compounds of the invention.

For any provided compound or test agent, the therapeutically effective amount can be initially determined from cell culture assays. Target concentrations will be those concentrations of active compound(s) that are capable of decreasing PKa enzymatic activity as measured, for example, using the methods described.

Therapeutically effective amounts for use in humans may be determined from animal models. For example, a dose for humans can be formulated to achieve a concentration that has been found to be effective in animals. The dosage in humans can be adjusted by monitoring PKa inhibition and adjusting the dosage upwards or downwards, as described above.

Dosages may be varied depending upon the requirements of the patient and the compound being employed. The dose administered to a patient, in the context of the present invention, should be sufficient to effect a beneficial therapeutic response in the patient over time. The size of the dose also will be determined by the existence, nature, and extent of any adverse side effects.

In one aspect, compounds provided herein display one or more improved pharmacokinetic (PK) properties (e.g., Cₘₐₓ, tₘₐₓ, Cₘᵢₙ, t_{1/2}, AUC, CL, bioavailability, etc.) when compared to a reference compound. In some embodiments, a reference compound is a PKa inhibitor known in the art. In some embodiments, a reference compound is a PKa inhibitor selected from those disclosed in WO 2019/178129.

In some embodiments a compound of the disclosure or a pharmaceutical composition comprising the same is provided as a unit dose.

### F. Methods of Treatment

The present disclosure provides compounds and pharmaceutical compositions comprising the same for use in medicine i.e. for use in treatment. The present disclosure further provides the use of any compounds described herein for inhibiting the activity of PKa, which would be beneficial to treatment of PKa-mediated diseases and conditions. Exemplary PKa-mediated disorders include edema, which refers to swelling in the whole body of a subject or a part thereof due to inflammation or injury when small blood vessels become leaky and releases fluid into nearby tissues. In some examples, the edema is HAE. In other examples, the edema occurs in eyes, *e.g*., diabetic macular edema (DME). The present disclosure provides methods of inhibiting the activity of PKa. In certain embodiments, the application provides a method of inhibiting the activity of PKa *in vitro* via contacting any of the compounds described herein with PKa molecules in a sample, such as a biological sample. In certain embodiments, the application provides a method of inhibiting the activity of PKa *in vivo* via delivering an effective amount of any of the compounds described herein to a subject in need of the treatment through a suitable route.

In certain embodiments, the methods comprise administering to a subject in need thereof (*e.g.,* a subject such as a human patient, for example with edema) any of the compounds described herein or a pharmaceutically acceptable salt thereof. In certain embodiments, the methods comprise administering a compound of Formulae (I)-(VI-c), or a pharmaceutically acceptable salt or composition thereof, to a subject in need thereof. In some embodiments, the method comprises administering a pharmaceutical composition comprising a compound of Formulae (I)-(VI-c), or a pharmaceutically acceptable salt to a subject in need thereof.

In certain embodiments, the subject to be treated by any of the methods described herein is a human patient having, suspected of having, or at risk for edema, for example, HAE or diabetic macular edema (DME). A subject having an edema can be identified by routine medical examination, *e.g.,* laboratory tests. A subject suspected of having an edema might show one or more symptoms of the disease/disorder. A subject at risk for edema can be a subject having one or more of the risk factors associated with the disease, for example, deficiency in C1-INH as for HAE.

In certain embodiments, provided herein are methods of alleviating one or more symptoms of HAE in a human patient who is suffering from an HAE attack. Such a patient can be identified by routine medical procedures. An effective amount of one or more of the provided compounds can be given to the human patient via a suitable route, for example, those described herein. The compounds described herein may be used alone, or may be used in combination with other anti-HAE agents, for example, a C1 esterase inhibitor (e.g., Cinryze^{®} or Berinert^{®}), a PKa inhibitor *(e.g.,* ecallantide or lanadelumab) or a bradykinin B2 receptor antagonist (e.g., Firazyr^{®}).

In other embodiments, provided herein are methods or reducing the risk of HAE attack in a human HAE patient who is in quiescent stage. Such a patient can be identified based on various factors, including history of HAE attack. An effective amount of one or more of the compounds can be given to the human patient *via* a suitable route, for example, those described herein. The compounds described herein may be used alone, or may be used in combination with other anti-HAE agents, for example, a C1 esterase inhibitor (e.g., Cinryze^{®} or Berinert^{®}), a PKa inhibitor (e.g., ecallantide or lanadelumab) or a bradykinin B2 receptor antagonist (e.g., Firazyr^{®}).

In some embodiments, provided herein is prophylactic treatment of HAE in human patients having risk to HAE attacks with one or more of the compounds described herein. In some embodiments, patients suitable for prophylactic treatment of HAE are human subjects suffering from HAE (e.g., having history of HAE attacks). In some embodiments, patients suitable for such prophylactic treatment are human subjects where a physician determines a history of HAE attacks warrants a prophylactic approach (e.g., human subjects experiencing more than a particular average number of attacks over a time period, including by way of nonlimiting example, one, two, or more attacks per month). Alternatively, patients suitable for the prophylactic treatment may be human subjects having no HAE attack history but bearing one or more risk factors for HAE (e.g., family history, genetic defects in C1-INH gene, etc.) Such prophylactic treatment may involve the compounds described herein as the sole active agent, or involve additional anti-HAE agents, such as those described herein.

In certain embodiments, provided herein are methods for preventing or reducing edema in an eye of a subject (e.g., a human patient). In some examples, the human patient is a diabetic having, suspected of having, or at risk for diabetic macular edema (DME). DME is the proliferative form of diabetic retinopathy characterized by swelling of the retinal layers, neovascularization, vascular leak, and retinal thickening in diabetes mellitus due to leaking of fluid from blood vessels within the macula. To practice this method, an effective amount of one or more of the compounds described herein, or pharmaceutically acceptable salts thereof, may be delivered into the eye of the subject where treatment is needed. For example, the compound may be delivered topically, by intraocular injection, or intravitreal injection. A subject may be treated with the compound as described herein, either as the sole active agent, or in combination with another treatment for DME. Non-limiting examples of treatment for DME include laser photocoagulation, steroids, VEGF pathway targeting agents (e.g., Lucentis^{®} (ranibizumab) or Eylea^{®} (aflibercept)), and/or anti-PDGF agents.

In certain embodiments, the methods disclosed herein comprise administering to the subject an effective amount of a compound of Formulae (I)-(VI-c), or a pharmaceutically acceptable salt or composition thereof. In some embodiments, the effective amount is a therapeutically effective amount. In some embodiments, the effective amount is a prophylactically effective amount.

In certain embodiments, the subject being treated is an animal. The animal may be of either sex and may be at any stage of development. In certain embodiments, the subject is a mammal. In certain embodiments, the subject being treated is a human. In certain embodiments, the subject is a domesticated animal, such as a dog, cat, cow, pig, horse, sheep, or goat. In certain embodiments, the subject is a companion animal, such as a dog or cat. In certain embodiments, the subject is a livestock animal, such as a cow, pig, horse, sheep, or goat. In certain embodiments, the subject is a zoo animal. In another embodiment, the subject is a research animal such as a rodent *(e.g.,* mouse, rat), dog, pig, or non-human primate. In certain embodiments, the animal is a genetically engineered animal. In certain embodiments, the animal is a transgenic animal.

Certain methods described herein may comprise administering one or more additional pharmaceutical agent(s) in combination with the compounds described herein. The additional pharmaceutical agent(s) may be administered at the same time as the compound of Formulae (I)-(VI-c), or at different times than the compound of Formulae (I)-(VI-c). For example, the compound of Formulae (I)-(VI-c) and any additional pharmaceutical agent(s) may be on the same dosing schedule or different dosing schedules. All or some doses of the compound of Formulae (I)-(VI-c) may be administered before all or some doses of an additional pharmaceutical agent, after all or some does an additional pharmaceutical agent, within a dosing schedule of an additional pharmaceutical agent, or a combination thereof. The timing of administration of the compound of Formulae (I)-(VI-c) and additional pharmaceutical agents may be different for different additional pharmaceutical agents.

Also provided is use of a compound of the present disclosure for the manufacture of a medicament for a condition/disease disclosed herein.

In certain embodiments, the additional pharmaceutical agent comprises an agent useful in the treatment of an edema, such as HAE or DME. Examples of such agents are provided herein.

While we have described a number of embodiments of this invention, it is apparent that our basic examples may be altered to provide other embodiments that utilize the compounds and methods of this invention. Therefore, it will be appreciated that the scope of this invention is to be defined by the appended claims rather than by the specific embodiments that have been represented by way of examples.

In the context of this specification "comprising" is to be interpreted as "including". Embodiments of the invention comprising certain features/elements are also intended to extend to alternative embodiments "consisting" or "consisting essentially" of the relevant elements/features. Where technically appropriate, embodiments of the invention may be combined.

Technical references such as patents and applications are incorporated herein by reference.

The background section of this specification contains relevant technical information and may be used as basis for amendment. Subject headings herein are employed to divide the document into sections and are not intended to be used to construe the meaning of the disclosure provided herein.

The present specification claims priority from U.S. Provisional Application No. 63/162,468 (filed March 17, 2021) incorporated herein by reference. This application may be used as basis for corrections to the present specification, especially in respect of chemical structures disclosed therein.

### IV. Examples

In certain embodiments, the Examples describe compounds comprising one or more stereocenters, where a particular stereocenter is designated "S*" or "R*." In both cases, the depiction of the "*" generally indicates that the exact configuration is unknown (e.g., for a compound with a single stereocenter, the depiction R*- or S*- indicates that either the R- or S-isomer was isolated, but the configuration at the stereocenter of the particular isomer isolated was not determined).

It will be appreciated that compounds described within the Examples may comprise more than one stereocenter. As described above, single stereochemical isomers as well as enantiomeric, diastereomeric, and geometric (or conformational) mixtures of the present compounds are within the scope of the invention. Within a particular compound name, where more than one "S*" or "R*" appear within a single pair of parentheses (e.g., "(1S*,2S*)"), it is understood that the S* and/or R* configurations are relative to each other. For example, a compound denoted "(1S*,2S*)-" or "(1R*,2R*)-" would be understood to refer specifically to either the "(1S,2S)-" or "(1R,2R)-" isomer, but not the "(1S,2R)-" or "(1R,2S)-" isomers. Furthermore, a compound denoted "rac-(1S*,2S*)-" or "rac-(1R*,2R*)-" would be understood to include a racemic mixture of the "(1S,2S)-" and "(1R,2R)-" isomers. Similarly, a compound denoted "(1S*,2R*)-" or "(1R*,2S*)-" would be understood to refer specifically to either the "(1R,2S)-" or "(1S,2R)-" isomer, but not the "(1S,2S)-" or "(1R,2R)-" isomers. In addition, a compound denoted "rac-(1R*,2S*)-" or "rac-(1S*,2R*)-" would be understood to include a racemic mixture of the "(1R,2S)-" and "(1S,2R)-" isomers. In certain embodiments, the Examples include schemes that depict compounds with one or more stereocenters. In some embodiments, the symbol "&" followed by a number appears adjacent to a stereocenter. In such cases, it is understood to include a mixture of both configurations (e.g., R- and S-) at that position.

In some embodiments, the term "or" followed by a number appears adjacent to a stereocenter. In such cases, it is understood to denote either an "R-" or "S-" isomer, but the particular isomer was not determined.

In some embodiments, the numbering following the symbol "&" or term "or" refers to one stereocenter's relation to another stereocenter in that compound. For example, where two stereocenters in a compound are each denoted with the same number (e.g., two instances of "&1"), it is understood that the configurations are relative to each other (e.g., if the structure is drawn as (S,S) and both stereocenters are denoted "&1", it is understood to include a mixture of the (S,S) and (R,R) isomers, but not the (S,R) or (R,S) isomers). However, where each stereocenter is denoted with a different number (e.g., one instance of "&1" and one instance of "&2"), it is understood that that the configurations may be independent to each other (e.g., if the structure is drawn (S,S) and one stereocenter is denoted "&1" and one is denoted "&2," it is understood to include a mixture of the (S,S), (S,R), (R,S), and (R,R) isomers).

In one embodiment, Examples 449 to 453 can be prepared as follows:

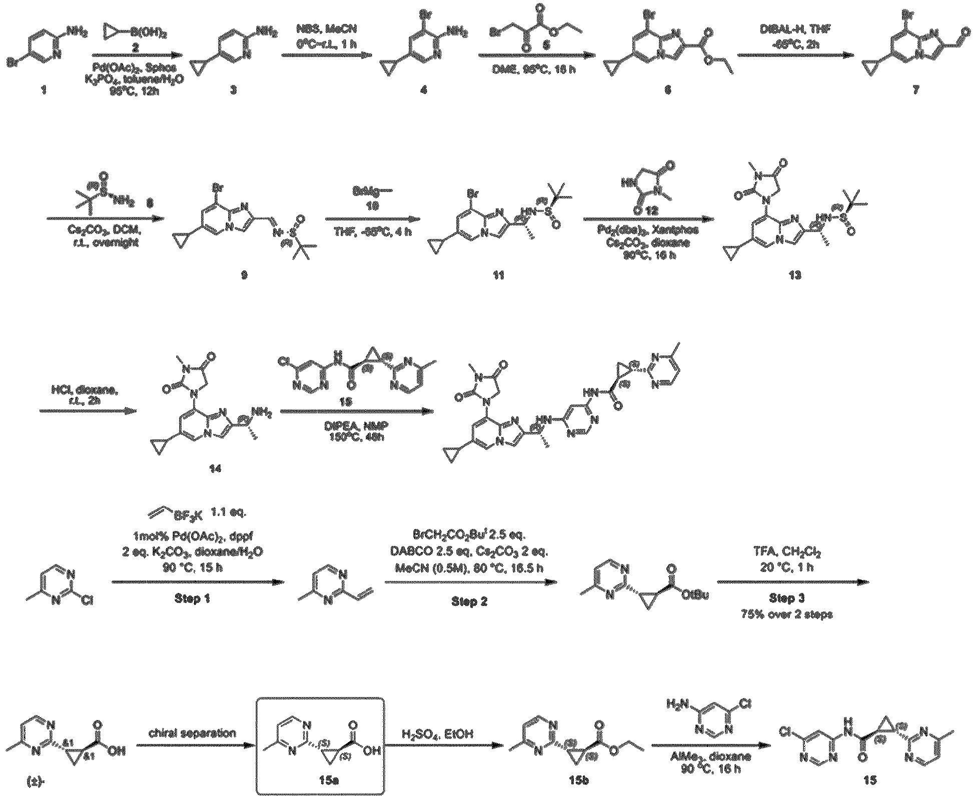

### SYNTHESIS OF INTERMEDIATES:

### Intermediate 1 4,6-dichloro-2-(methoxymethyl)pyrimidine

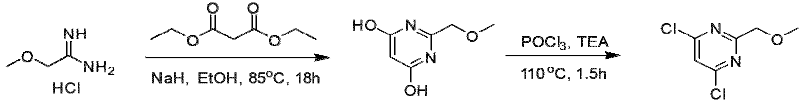

To a mixture of 2-methoxyacetimidamide hydrochloride (5 g, 40.32 mmol) and diethyl malonate (9 g, 56.82 mmol) in EtOH (50 mL) was added NaH (5.6 g, 60% suspension in paraffin oil, 142.05 mmol) in small portions at room temperature and the resulting mixture was heated to 85 °C for 18h. The reaction was cooled, the pH was adjusted to ~ 3 with 4 N HCl solution, and the mixture was extracted with CHCl₃/IPA (3/1; 100 mL x 8). The combined organic layers were washed with brine (50mL), dried over Na₂SO₄ and concentrated *in vacuo* to afford **2-(methoxymethyl)pyrimidine-4,6-diol intermediate 2** (4.8 g, 76%) as an off-white solid, which was used without further purification. ESI-MS [M +H]+: 157.1.

A mixture of intermediate 2 (2.5 g, 16 mmol) and TEA (2.4 g, 24 mmol) in POCl₃ (15.7 g, 103 mmol) was stirred at 110°C for 1.5h. The reaction was cooled to room temperature and concentrated. Ice-water (50 mL) was added and the mixture was extracted with EtOAc (50 mL x 3). The combined organic layers were washed with brine (50 mL), dried over Na₂SO₄ and concentrated *in vacuo.* The residue was purified by silica gel chromatography (EtOAc /PE from 0 to 10%) to provide intermediate 1 (950 mg, 30.6 % yield) as a yellow solid.

### Intermediate 3: 2-(2-((tert-butyldimethylsilyl)oxy)ethoxy)-4,6-dichloropyrimidine

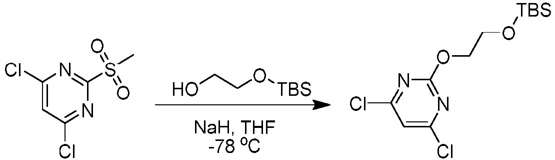

To a solution of 2-((tert-butyldimethylsilyl)oxy)ethan-1-ol (1.76 g, 10 mmol) in THF (30 mL) was added NaH (480 mg, 60% suspension in paraffin oil, 12 mmol) at 0°C slowly. The resulting suspension was stirred at 0°C for 10 min followed by addition of a solution of 4,6-dichloro-2-(methylsulfonyl) pyrimidine (2.27 g, 10 mmol) in THF (10 mL). After stirring at room temperature for 3h, the reaction was quenched with saturated aqueous NH₄Cl (30 mL) and extracted with EtOAc (50 mL x 3). The combined organic layers were washed with brine (50 mL), dried over Na₂SO₄, concentrated *in vacuo* and the crude product was purified by silica gel chromatography (eluent: PE / EtOAc = 5/1) to give intermediate 3 (1.3 g, 40%) as a yellow solid. ESI-MS [M +H]+: 323.1.

### Intermediate 4: 4,6-dichloro-2-(difluoromethyl)pyrimidine

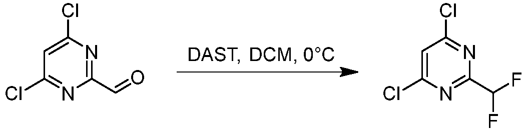

To a mixture of 4,6-dichloropyrimidine-2-carbaldehyde (150 mg, 0.86 mmol) in DCM (3 mL) was added DAST (694 mg, 4.3 mmol) at 0°C under N₂, followed by stirring for 2 hours at room temperature under N₂. The reaction was quenched with H₂O (0.5 mL), then concentrated *in vacuo* and purified by preparative TLC (eluent: PE/EtOAc=6/1) to give **intermediate 4** as a white solid (80 mg, 47%).

### Intermediate 5: 4,6-dichloro-2-(fluoromethyl)pyrimidine

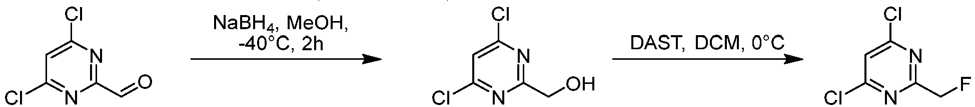

To a mixture of 4,6-dichloropyrimidine-2-carbaldehyde (200 mg, 1.14 mmol) in MeOH (3 mL) was added NaBH₄ (86 mg, 2.28 mmol) at -40°C under N₂. The reaction mixture was stirred at -40°C for 1 h under N₂. The reaction was quenched with H₂O (3 mL) and extracted with EtOAc (10 mL x 3). The organic layers were washed with brine (20 mL), dried over Na₂SO₄ then concentrated *in vacuo* to give (4,6-dichloropyrimidin-2-yl)methanol **intermediate 6** as a white solid (140 mg, crude), which was used directly in the next step. ESI-MS [M +H]+: 179.1

A mixture of **intermediate 6** (100 mg, 0.56 mmol) in DCM (5 mL) was added DAST (451 mg, 2.8 mmol) at 0°C under N₂, followed by stirring for 2 h at RT under N₂. The mixture was quenched with H₂O (20 mL) and extracted with EtOAc (10 mL x3). The organic layers were concentrated *in vacuo* and purified by preparative TLC ((eluent: PE/EtOAc=6/1) to give **intermediate 5** as a white solid (50 mg, 50%). ESI-MS [M +H]⁺: 181.2

### Intermediate 7: 2-bromo-6-(2-morpholinoethyl)pyridin-4-amine

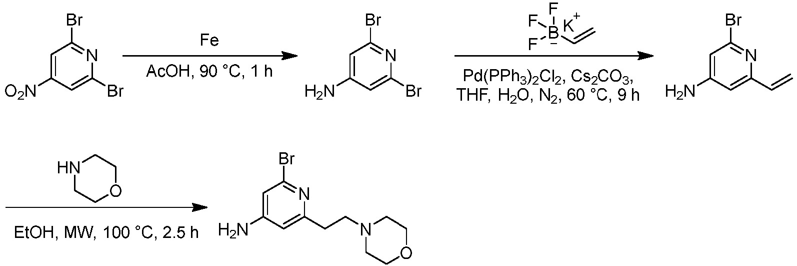

To a mixture of 2,6-dibromo-4-nitropyridine (2.3 g, 8.2 mmol) in AcOH (15 ml) was added iron powder (2.3 mg, 41 mmol) at room temperature, followed by stirring at 90°C for 1 h. The mixture was filtered and concentrated *in vacuo* to afford 2,6-dibromopyridin-4-amine **intermediate 8** (3.15 g, crude) as a yellow solid, which was used without further purification. ESI-MS [M +H]+: 281.1.

To a mixture of **intermediate 8** (3.0 g, 11.9 mmol), potassium trifluoro(vinyl)borate (1.6 g, 11.9 mmol) and Cs₂CO₃ (11.6 g, 35.7 mmol) in THF (27 ml) and water (3 mL) was added Pd(PPh₃)₂Cl₂ (417 mg, 0.60 mmol) at room temperature. The mixture was stirred at 60°C for 9 h and cooled to room temperature. Water (50 mL) was added to the mixture and extracted with EtOAc (3 x 50 mL). The combined organic layers were washed with brine (60 mL), dried over anhydrous Na₂SO₄, filtered and concentrated *in vacuo* to give the crude, which was purified by silica gel chromatography (eluent: PE/EtOAc = 3/1) to give 2-bromo-6-vinylpyridin-4-amine **intermediate 9** (584 mg, 25 %) as a white solid. ESI-MS [M +H]+: 199.1.

To a solution of **intermediate 9** (580 mg, 2.9 mmol) in EtOH (4 mL) was added morpholine (5.0 g, 58 mmol). The mixture was stirred at 100°C in a microwave reactor for 2.5 h. The reaction was concentrated *in vacuo.* The residue was purified by silica gel chromatography (eluent: MeOH/DCM = 0 - 10%) to afford 800 mg yellow solid, which was purified again by preparative TLC (eluent: DCM: MeOH = 7: 1) to afford **intermediate 7** (419 mg, 51%) as pale-yellow solid. ESI-MS [M +H]+: 286.1.

### Intermediate 10: 4-chloro-2-((6-cyclopropylimidazo[-1,2-a]pyridin-2-yl)methyl)-2H-[1,2,3]triazolo[4,5-c]pyridine

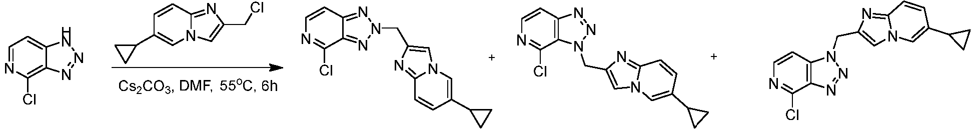

A mixture of 4-chloro-1H-[1,2,3]triazolo[4,5-c]pyridine (3.5 g, 22.6 mmol), 2-(chloromethyl)-6-cyclopropylimidazo[1,2-a]pyridine (5.6 g, 27.1 mmol) and Cs₂CO₃ (14.7 g, 45.2 mmol) in DMF (50 mL) was stirred at 55°C for 6 h. After cooling to room temperature, the reaction was quenched with water (500 mL) and extracted with EtOAc (300 mL x 3). The combined organic layers were washed with brine (300 mL), dried over Na₂SO₄, concentrated *in vacuo* to give the crude, which was purified by Prep-HPLC to give **intermediate 10** (100 mg, 1.4%), 4-chloro-3-((6-cyclopropylimidazo[1,2-a]pyridin-2-yl)methyl)-3H-[1,2,3]triazolo[4,5-c]pyridine (250 mg, 3.4%) and 4-chloro-1-((6-cyclopropylimidazo[1,2-a]pyridin-2-yl)methyl)-1H-[1,2,3]triazolo[4,5-c]pyridine (1.5 g, 21%) as yellow solids. ESI-MS [M +H] +: 325.1

### Intermediate 11: - 4-chloro-6-methyl-1,3,5-triazin-2-amine4

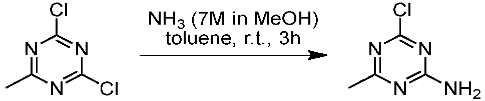

A mixture of 2,4-dichloro-6-methyl-1,3,5-triazine (700 mg,4.29mmol) in NH₃/MeOH (9 mL, 7M) and toluene (10 mL) was stirred at room temperature for 3h. The mixture was concentrated *in vacuo* to give the crude, which was purified by silica gel chromatography (eluent: DCM/MeOH =10/1) to give **intermediate** 11 (300 mg, 49%) as a white solid. ESI-MS [M +H]+: 145.2.

### Intermediate 12: 4-chloro-6-methyl-2H-pyrazolo[4,3-c]pyridine

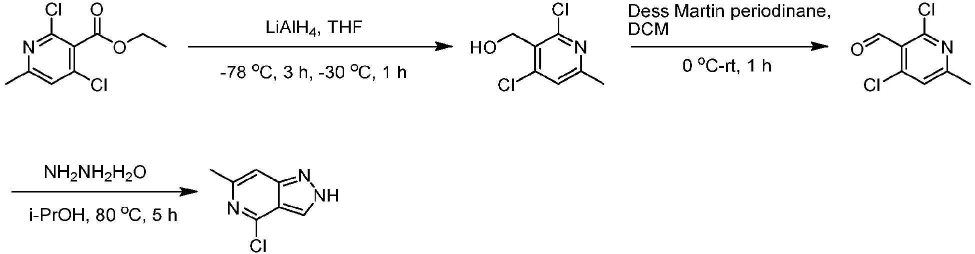

To a solution of ethyl 2,4-dichloro-6-methylnicotinate (2.0 g, 8.54 mmol) in THF (30 mL) was added LiAlH₄ (42 mL, 42 mmol) at -78°C. The resulting mixture was stirred at -78°C for 3 h and at -30°C for another 1 h under N₂. After cooling back the mixture to -78°C, a solution of saturated aq. NH₄Cl (100 mL) solution was added and extracted with EtOAc (3 x 40 mL). The combined organic layers were washed with brine (50 mL), dried over Na₂SO₄ and concentrated *in vacuo.* The residue was purified by silica gel chromatography (EtOAc /PE from 0 to 10%) to give (2,4-dichloro-6-methylpyridin-3-yl)methanol **intermediate 13** (1.2 g, 73%) as a colorless oil. ESI-MS [M +H]+: 192.0. A mixture of **intermediate 13** (1.2 g, 6.25 mmol) in DCM (30 mL) was added Dess Martin periodinane (3.18 g, 7.5 mmol) at 0°C and warmed to room temperature for 1 h under N₂. The mixture quenched with saturated aq. Na₂S₂O₃ (50 mL) and extracted with DCM (3 x 50 mL). The combined organic layers were washed with brine (50 mL), dried over Na₂SO₄ and concentrated *in vacuo.* The residue was purified by preparative TLC (eluent: PE/EtOAc = 10/1) to give 2,4-dichloro-6-methylnicotinaldehyde **intermediate 14** (1 g, 84%) as a white solid. ESI-MS [M +H]+: 190.0.

To a mixture of **intermediate 14** (1 g, 5.26 mmol) in i-PrOH (15 mL) was added hydrazine hydrate (1.5 mL, 45.6 mmol) and the mixture was stirred at 80°C for 5 h under N₂. The reaction was quenched with water (30 mL) and the mixture was extracted with DCM (3 x 30 mL). The combined organic layers were washed with brine (50 mL), dried over Na₂SO₄ and concentrated *in vacuo.* The crude product was purified by preparative TLC (eluent: PE/EtOAc = 10/1) to give **intermediate 12** (370 mg, 42%) as a white oil. ESI-MS [M +H]⁺: 168.0.

### Intermediate 15: 2,4-dichloro-6-((4-methoxybenzyl)oxy)-1,3,5-triazine

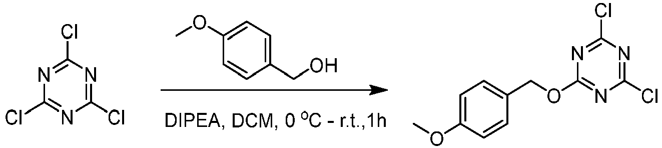

To a mixture of 2,4,6-trichloro-1,3,5-triazine (550 mg, 3.0 mmol) and (4-methoxyphenyl)methanol (414 mg, 3.0 mmol) in DCM (10 mL) was added DIPEA (774 mg, 6.0 mmol) at 0°C and then stirred at room temperature under N₂ for 1 h. The resulting mixture was evaporated to afford crude **intermediate 15** (1.2 g, crude) as a yellow oil which was used directly in next step. ESI-MS [M +H] +: 286.0

### Intermediate 16: 4,6-dichloro-2-((4-methoxybenzyl)oxy)pyrimidine

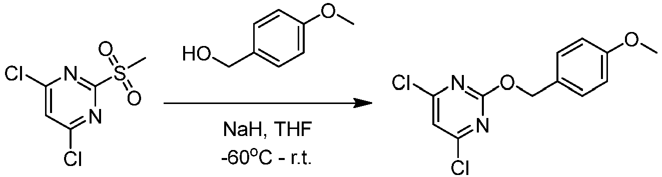

To a mixture of 4,6-dichloro-2-(methylsulfonyl)pyrimidine (800 mg, 3.54 mmol) in THF (15 mL) was added NaH (254 mg, 11 mmol) at -60°C under N₂. After stirring the mixture at -60°C for 1h, a solution of (4-methoxyphenyl)methanol (586 mg,4.24 mmol) in THF (5 mL) was added dropwise at -60°C. Then the mixture was warmed to room temperature and stirred for 1h. The reaction was quenched with saturated aq. NH₄Cl (50 mL) and extracted with EtOAc (3 x 50 mL). The combined organic layers were washed with brine (30 mL), dried over anhydrous Na₂SO₄, and concentrated *in vacuo* to give the crude, which was purified by silica gel chromatography (eluent: MeOH/DCM = 1/50) to give **intermediate 16** (400 mg, 40%) as a yellow solid. ESI-MS [M +H]+: 285.1

### Intermediate 17: 4,6-dichloro-2-(pyrrolidin-1-yl)pyrimidine

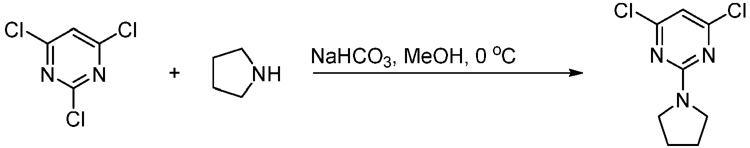

A suspension of 2,4,6-trichloropyrimidine (1.0 g, 5.5 mmol) and NaHCO₃ (1.4 g, 16 mmol) in MeOH (7.0 mL) was stirred at 0°C. A solution of pyrrolidine (0.46 mL, 5.5 mmol) in MeOH (3 mL) was added dropwise and the mixture was stirred at 0°C for 3 h. The reaction mixture was allowed to warm to room temperature, filtered, concentrated *in vacuo.* The residue was purified by column chromatography on silica gel, eluting with a gradient of 0-10 % EtOAc in cyclohexane to give the **intermediate 17** (420 mg, 35 %). ¹H NMR (400 MHz, CDCl₃) δ 6.51 (s, 1H), 3.60 - 3.55 (m, 4H), 2.01 - 1.96 (m, 4H).

### Intermediate 18: ethyl 4,6-dichloropyrimidine-2-carboxylate

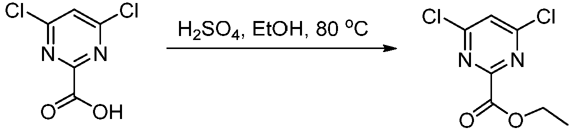

A solution of 4,6-dichloropyrimidine-2-carboxylic acid (300 mg, 1.6 mmol) and H₂SO₄ (8.3 µL, 0.16 mmol) in EtOH (8.0 mL) was stirred at 80°C for 16 h, followed by cooling to room temperature and concentrated *in vacuo.* The residue was dissolved in DCM (15 mL) and washed with NaHCO₃ (sat. aq., 10 mL), dried over MgSO₄ and concentrated *in vacuo* to give **intermediate 18** (270 mg, 78 %) as a colourless oil. ESI-MS (M+H): 221.0, ¹H NMR (400 MHz, CDCl₃) δ 7.58 (s, 1H), 4.54 (q, J=7.0 Hz, 2H), 1.46 (t, J=7.3 Hz, 3H).

### Intermediate 19: 2-(2-((tert-butyldimethylsilyl)oxy)ethyl)-4,6-dichloropyrimidine

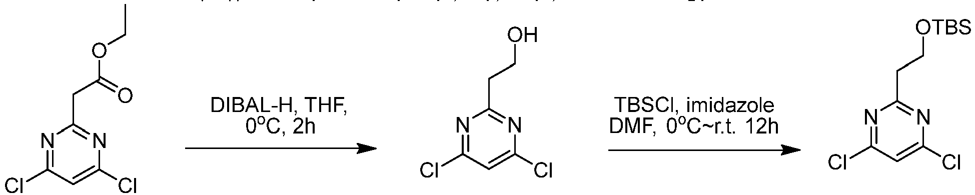

To a solution of ethyl 2-(4,6-dichloropyrimidin-2-yl)acetate (117.5 mg, 0.5 mmol) in THF (5 mL) was added DIBALH (1.0 M in hexane, 1.0 mL, 1.0 mmol) at 0°C. After stirring the mixture at room temperature for 2h, the reaction was quenched with Na₂SO₄ ·10 H₂O. The resulting solution was filtered and the filter cake was washed with DCM/MeOH (10/1, 50 mL). The filtrate was concentrated *in vacuo* to give the crude product **2-(4,6-dichloropyrimidin-2-yl)ethan-1-ol intermediate 20** as a yellow solid (109 mg, crude), which was used without further purification. ESI-MS [M +H]+: 193.1 To a solution of intermediate 20 (96 mg, 0.5 mmol) in DMF (5 mL) was added imidazole (51 mg, 0.75 mmol) and TBSCl (83 mg, 0.55 mmol) at 0°C. After stirring at room temperature for 3 h, the reaction was quenched with water (20 mL) then extracted with EtOAc (3 x 20 mL). The combined organic layers were washed with brine (30 mL), dried over Na₂SO₄ and concentrated *in vacuo* to give the crude product, which was purified by silica gel chromatography (PE : EA =10:1) to give **intermediate 19** as a colorless oil (75 mg, 49%, two steps). ESI-MS [M +H]+: 307.1

### Intermediate 21: 2-(((tert-butyldimethylsilyl)oxy)methyl)-4,6-dichloropyrimidine

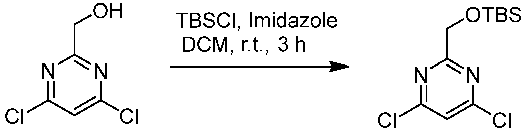

To a mixture of (4,6-dichloropyrimidin-2-yl)methanol (1.48 g crude) and imidazole (1.135 g, 16.686 mmol) in DCM (50 mL) was added TBSCl (1.89 g, 12.515 mmol) at 0°C. After stirring at room temperature for 3 h, the reaction mixture was quenched with water (40 mL) and extracted with DCM (3 x 50 mL). The combined organic layers were washed with brine (50 mL), dried over anhydrous Na₂SO₄, filtered and concentrated *in vacuo.* The residue was purified by silica gel column chromatography (eluent: PE/EtOAc = 4/1) to give **intermediate 21** (1.72 g, 70%) as yellow oil. ESI-MS [M +H]+: 293.1.

### Intermediate 22: 4,6-dichloro-N,N-dimethylpyrimidin-2-amine

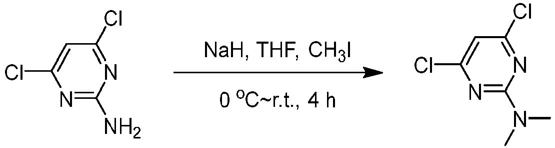

To a solution of 4,6-dichloropyrimidin-2-amine (1.0 g, 6.1 mmol) in THF (40 mL) was added NaH (1 g, 25 mmol, 60% dispersion in mineral oil) slowly at 0°C. The reaction mixture was stirred at 0°C for 1 h, then CH₃I (1.7 g, 12 mmol) was added at 0°C. After stirring at room temperature for another 3 h, the reaction was quenched with water (40 mL) then extracted with EtOAc (3 x 50 mL). The combined organics were dried over Na₂SO₄ and concentrated *in vacuo* to give the crude, which was purified by silica gel column chromatography (eluent: PE/ EtOAc = 2/1) to afford **intermediate 22** (640 mg, 55 %) as a yellow solid. ESI-MS: [M + H]⁺, 192.0

### Intermediate 23: 2-(5-chloro-2-nitrophenyl)cyclopropane-1-carboxamide

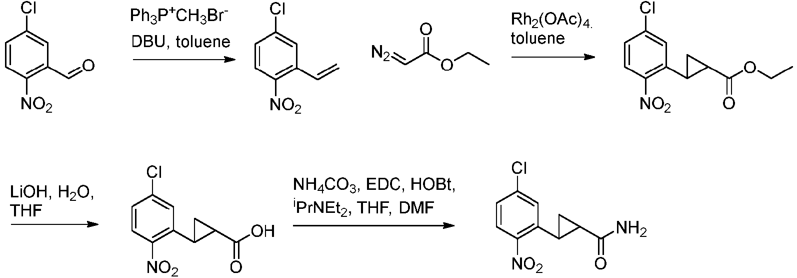

DBU (2.9 mL, 19.4 mmol) was added to a stirred solution of 5-chloro-2-nitrobenzaldehyde (3.0 g, 16.17 mmol) and methyltriphenylphosphonium bromide (6.93 g, 19.4 mmol) in dry toluene (50 mL). The mixture was stirred at room temperature for 16 h, then (NH₄)₂CO₃ (sat. aq.) was added. The mixture was extracted with EtOAc (×3) and the organic extracts were dried (hydrophobic frit) and concentrated *in vacuo.* The resulting oil was purified by dry flash silica chromatography eluting with a gradient of 0-50 % EtOAc in cyclohexane to give **4-chloro-1-nitro-2-vinylbenzene interemediate 24** (450 mg, 15 %). ¹H NMR (400 MHz, CDCl₃) δ 7.95 - 7.90 (m, 1H), 7.61 - 7.57 (m, 1H), 7.38 (dd, J=2.1, 8.7 Hz, 1H), 7.23 - 7.12 (m, 1H), 5.76 (d, J=17.2 Hz, 1H), 5.54 (d, J=10.9 Hz, 1H).

A solution of ethyl diazoacetate (0.60 mL, 5.72 mmol) in toluene (5 mL) was added dropwise over 2 h to a solution of **intermediate 24** (350 mg, 1.91 mmol) and Rh₂(OAc)₄ (17 mg, 0.038 mmol) in toluene (5 mL) at 80°C for 16 h, followed by cooling to room temperature and concentrated *in vacuo* purifiction by dry flash silica chromatography eluting with a gradient of 0-20 % EtOAc in cyclohexane to **give ethyl 2-(5-chloro-2-nitrophenyl)cyclopropane-1-carboxylate intermeidate 25** as a 2:1 mixture of diastereoisomers (255 mg, 50 %). ¹H NMR (400 MHz, CDCl₃) δ 7.92 (d, J=9.1 Hz, 1H), 7.36 (d, J=8.6 Hz, 1H), 7.23 (s, 1H), 4.30 - 4.19 (m, 2H), 3.02 - 2.95 (m, 1H), 1.90 - 1.83 (m, 1H), 1.72 - 1.67 (m, 1H), 1.35 - 1.28 (m, 4H).

LiOH (222 mg, 9.27 mmol) was added to a solution of **intermediate 25** (500 mg, 1.85 mmol) in THF/water (1:1; 10 mL) and the mixture was stirred at room temperature for 16 h. The reaction mixture was acidified to pH1 with HCl (1 M, aq.) and extracted with DCM. The organic extract was dried (hydrophobic frit) and concentrated *in vacuo* to give **2-(5-chloro-2-nitrophenyl)cyclopropane-1-carboxylic acid intermediate 26** as a mixture of diastereoisomers (356 mg, 79 %). ¹H NMR (400 MHz, DMSO) δ 8.06 (d, J=8.7 Hz, 1H), 7.67 - 7.60 (m, 2H), 7.52 (d, J=1.8 Hz, 1H), 2.80 - 2.73 (m, 1H), 1.98 - 1.91 (m, 1H), 1.65 - 1.60 (m, 1H), 1.51 - 1.46 (m, 1H). ESI-MS (M+H)+: 240

A solution of **intermediate 26** (150 mg, 0.62 mmol), EDC·HCl (116 mg, 0.745 mmol), HOBt (101 mg, 0.745 mmol), (NH₄)₂CO₃ (581 mg, 3.10 mmol) and DIPEA (0.65 mL, 3.72 mmol) in THF (10 mL) and DMF (10 mL) was stirred at room temperature for 16 h. Brine was added and the mixture was extracted with EtOAc. The organic extract was dried (hydrophobic frit) and concentrated *in vacuo.* The crude product was purified by dry flash silica chromatography eluting with a gradient of 0-100 % EtOAc in cyclohexane to give **intermediate 23** as a mixture of diastereoisomers (56 mg, 37 %). ¹H NMR (400 MHz, CDCl₃) δ 7.89 (d, J=8.6 Hz, 1H), 7.36 (dd, J=2.0, 8.8 Hz, 1H), 7.26 (s, 1H), 5.66 (d, J=54.9 Hz, 2H), 2.97 - 2.87 (m, 1H), 1.76 - 1.64 (m, 2H), 1.34 - 1.22 (m, 1H).

### Intermediate 27: 2-(6-cyano-2-fluoro-3-methoxyphenyl)cyclopropane-1-carboxamide

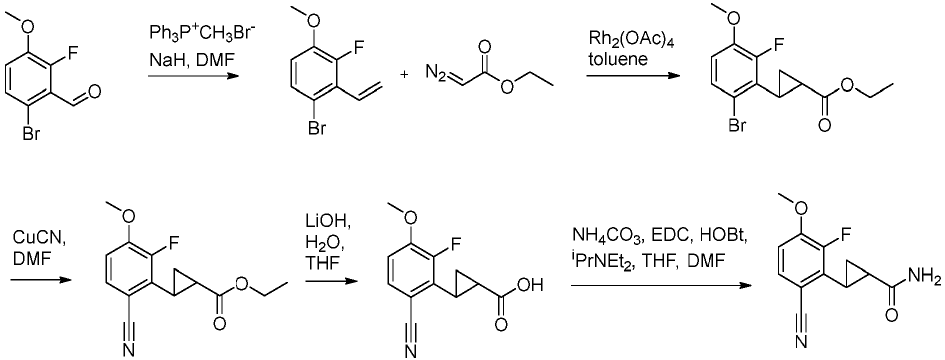

NaH (60 % in mineral oil; 0.15 g, 6.44 mmol) was added to an ice-cooled solution of methyltriphenyl phosphonium bromide (2.30 g, 6.44 mmol) in DMF (50 mL) at 0°C, followed by warm to room temperature and stirred for 30 min. After cooling to 0°C 6-Bromo-2-fluoro-3-methoxybenzaldehyde (5.78 g, 16.17 mmol) was added portion-wise. The mixture was stirred at room temperature for 16 h, then NH₄Cl (sat. aq.) was added. The mixture was extracted with EtOAc (×3) and the organic extracts were dried over MgSO₄ and concentrated *in vacuo.* The resulting oil was purified by flash silica chromatography eluting with a gradient of 0-15 % EtOAc in isohexane to give **bromo-3-fluoro-4-methoxy-2-vinylbenzene intermediate 28** as a clear oil (790 mg, 64 %). ¹H NMR (400 MHz, CDCl₃) δ 7.29 (dd, J=2.1, 8.7 Hz, 1H), 6.79 - 6.65 (m, 2H), 5.92 (d, J=17.9 Hz, 1H), 5.65 (d, J=11.6 Hz, 1H), 3.88 (s, 3H).

A solution of ethyl diazoacetate (1.1 mL, 10.3 mmol) in toluene (3 mL) was added dropwise over 3 h to a solution of **intermediate 28** (790 mg, 3.43 mmol) and Rh₂(OAc)₄ (30 mg, 0.069 mmol) in toluene (7 mL) at 80°C. The reaction mixture was stirred at 80°C for 16 h, cooled to room temperature and concentrated *in vacuo.* The crude product was partially purified by dry flash silica chromatography eluting with a gradient of 0-30 % ethyl acetate in isohexane to give a ~1:1 mixture of **ethyl 2-(6-bromo-2-fluoro-3-methoxyphenyl)cyclopropane-1-carboxylate intermediate 29** and the starting alkene (724 mg). The impure product was carried forward without further purification to the next step. A mixture of ethyl **intermediate 29** (~50 % purity; 724 mg, 1.14 mmol), and CuCN (204 mg, 2.28 mmol) in DMF (11 mL) was heated at 150°C for 16 h. The mixture was cooled to room temperature, then diluted with EtOAc and 1:4 NH₄OH (sat. aq.) in NH₄Cl (sat. aq.). The organic phase was separated and the aqueous phase further extracted with EtOAc (×2). The organic extracts were dried (MgSO₄) and concentrated *in vacuo.* The crude product was purified by dry flash silica chromatography eluting with 0-40 % EtOAc in isohexane to give the desired product **ethyl 2-(6-cyano-2-fluoro-3-methoxyphenyl)cyclopropane-1-carboxylate intermediate 30** as a mixture of isomers (175 mg, 58 %). This was used without further purification Using a similar procedure to that for **Intermediate 23, intermediate 27** (67 mg) was synthesised from **intermediate 30** (175 mg, 0.66 mmol) and was used without further purification in the next step.

### Intermediate 31: 2-(3-chlorophenyl)-2-fluorocyclopropane-1-carboxamide

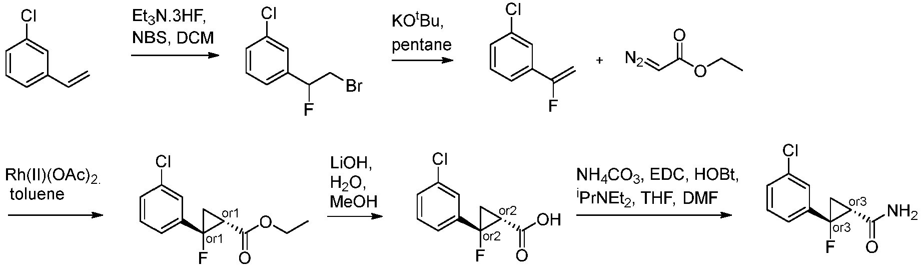

NEt₃·3HF (7.1 mL, 43.29 mmol) was added dropwise to an ice-cooled solution of 3-chlorostyrene (1.8g, 14.43 mmol) and NBS (3.08g, 17.32 mmol) in DCM (15 mL) at 0°C and the mixture was stirred at room temperature for 16 h. Further NBS (1.50 g, 8.43 mmol) was added and the mixture was stirred at room temperature for 7 h. The solution was poured into NH₄OH (sat. aq.), then extracted with EtOAc (×5). The extracts were washed with HCl (1 M aq.), dried (MgSO₄) and concentrated *in vacuo.* The crude product was purified by dry flash silica chromatography eluting with pentane to give **rac-1-(2-bromo-1-fluoroethyl)-3-chlorobenzene intermediate 32** as a clear oil (1.61 g, 47 %). ¹H NMR (400 MHz, CDCl₃) δ 7.38 - 7.33 (m, 3H), 7.26 - 7.21 (m, 1H), 5.68 - 5.52 (m, 1H), 3.71 - 3.55 (m, 2H). KO^{t}Bu (1.52 g, 13.56 mmol) was added portion-wise to an ice-cooled solution of **intermediate 32** (1.61 g, 6.78 mmol) in pentane (40 mL) at 0°C and the mixture was stirred at 35°C for 1.5 h. The suspension was poured into iced water, then the mixture was extracted with pentane (×2). The extract was a dried (MgSO₄) and concentrated *in vacuo* to give **1-chloro-3-(1-fluorovinyl)benzene intermediate 33** as a clear oil (0.93 g, 88 %). ¹H NMR (400 MHz, CDCl₃) δ 7.53 (dd, J=1.9, 1.9 Hz, 1H), 7.45 - 7.41 (m, 1H), 7.34 - 7.30 (m, 2H), 5.06 (dd, J=3.6, 49.2 Hz, 1H), 4.90 (dd, J=3.8, 17.7 Hz, 1H).

Using a similar procedure to that employed for **intermediate 29** starting from **intermediate 33** (200 mg, 1.28 mmol) was synethesised **ethyl 2-(3-chlorophenyl)-2-fluorocyclopropane-1-carboxylate intermediate 34** as a mixture of diastereoisomers (279 mg, 90 %). ¹H NMR (400 MHz, CDCl₃) δ 7.49-7.47 (m, 0.5H, Isomer A), 7.37-7.29 (m, 2.5H, Isomer A+B), 7.18 - 7.14 (m, 0.5H, Isomer B), 4.29 - 4.18 (m, 2H), 2.61-2.51 (m, 0.5H, Isomer A), 2.36 - 2.27 (m, 0.5H, Isomer B), 2.21 - 2.15 (m, 0.5H, Isomer A), 2.01 - 1.93 (m, 0.5H, Isomer B), 1.89 - 1.78 (m, 0.5H, Isomer B), 1.65 - 1.58 (m, 0.5H, Isomer A), 1.35 - 1.28 (m, 3H).

Using a similar procedure to **intermediate 27** starting from **intermediate 34** (279 mg, 1.15 mmol) was synthesised **intermediate 31** as a mixture of diastereoisomers (yellow solid, 179 mg, 99 %) which was used without further purification.

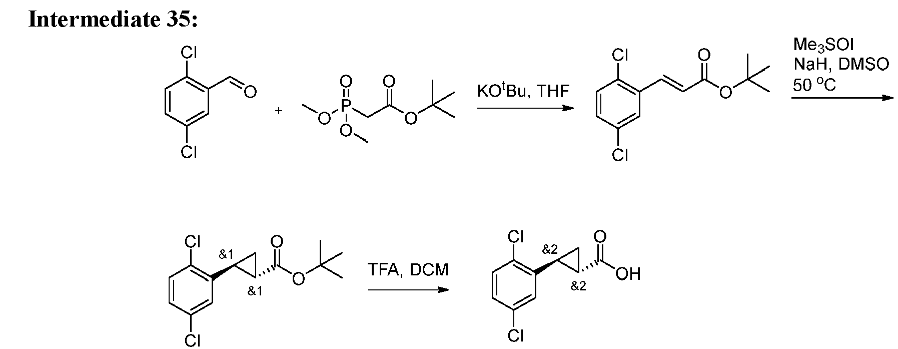

KO^{t}Bu (505 mg, 4.50 mmol) was added to a stirred solution of 2,5-dichlorobenzaldehyde (525 mg, 3.00 mmol) and tert-butyl 2-(dimethoxyphosphoryl)acetate (673 mg, 3.00 mmol) in THF (30 mL). The mixture was stirred at room temperature for 16 h, then diluted with EtOAc (30 mL). The solution was washed with water (30 mL) and brine (30 mL), then dried (hydrophobic frit) and concentrated *in vacuo* to give ***tert-butyl (E)-3-(2,5-dichlorophenyl)acrylate* intermediate 36** (840 mg, ~90 % purity, 96 %) as a clear oil that was used in the next reaction without further purification. ¹H NMR (400 MHz, CDCl₃) δ 7.91 (d, J=17.1 Hz, 1H), 7.58 (d, J=2.4 Hz, 1H), 7.34 (d, J=8.5 Hz, 1H), 7.27 (d, J=2.9 Hz, 1H), 6.36 (d, J=15.0 Hz, 1H), 1.48 (s, 9H).

NaH (60 % in mineral oil, 137 mg, 3.43 mmol) was added portion-wise to a solution of trimethylsulfoxonium iodide (754 mg, 3.43 mmol) in DMSO (5 mL). The resulting mixture was stirred for 30 min at room temperature followed by 1h at 40°C, with regular sonication to assist solubilisation. A solution of **intermediate 36** (780 mg, 2.86 mmol) in DMSO (1 mL) was then added dropwise and the resulting mixture was stirred at 60°C for 16 h. The mixture was diluted with water (25 mL) and extracted with EtOAc (2 x 10 mL), then the combined organics were washed with water and brine, dried (hydrophobic frit) and concentrated *in vacuo.* The resulting oil was purified by dry flash silica chromatography eluting with a gradient of 5-50 % Et₂O in cyclohexane to give ***rac-(1S*2S*)-tert-butyl 2-(2,5-dichlorophenyl)cyclopropane-1-carboxylate* intermediate 37** as an oil (466 mg, 56 %). ¹H NMR (400 MHz, CDCl₃) δ 7.29 (d, J=8.4 Hz, 1H), 7.13 (dd, J=2.4, 8.7 Hz, 1H), 6.97 (d, J=2.5 Hz, 1H), 2.63 (ddd, J=4.5, 6.7, 9.0 Hz, 1H), 1.75 - 1.70 (m, 1H), 1.59 - 1.54 (m, 1H), 1.48 (s, 9H), 1.27 - 1.22 (m, 1H). TFA (1.2 mL) was added to a solution of **intermediate 37** (450 mg, 1.57 mmol) in DCM (20 mL) and the mixture was stirred at room temperature for 16 h. The solvents were removed *in vacuo* to give **intermediate 35** as a grey solid (354 mg, 97 %) which was used without further purification.

The compounds in Table A1 were synthesized using a similar method to intermediate 35 starting from the appropriate benzaldehyde derivatives. **Table A1**

| | |
|---|---|
| 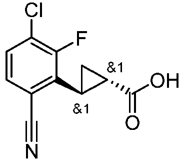 **Intermediate 38:** *rac*-(1*S**,2*S**)-2-(3-chloro-6-cyano-2-fluorophenyl) cyclopropane-1-carboxylic acid used without purification | 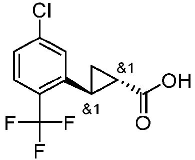 **Intermediate 39:** *rac*-(1*S**,2*S**)-2-(5-chloro-2-(trifluoromethyl) phenyl)cyclopropane-1-carboxylic acid ESI-MS (M+H)+: 263 |
| 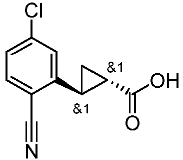 **Intermediate 40:** *rac*-(1*S**,2*S**)-2-(5-chloro-2-cyanophenyl)cyclopropane-1-carboxylic acid | ESI-MS (M+H)+: 220, ¹H NMR (400 MHz, DMSO) δ 12.53 - 12.52 (m, 1H), 7.86 (d, J=7.9 Hz, 1H), 7.51 (d, J=7.9 Hz, 1H), 7.38 (s, 1H), 2.69 - 2.64 (m, 1H), 1.59 - 1.53 (m, 2H). 1H obscured by DMSO signal. |
| 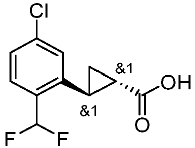 **Intermediate 41:** *rac*-(1*S**,2*S**)-2-(5-chloro-2-(difluoromethyl)phenyl)cyclopropane-1-carboxylic acid | ESI-MS (M+H)+: 245, ¹H NMR (400 MHz, DMSO) δ 7.62 (d, J=8.1 Hz, 1H), 7.49 (d, J=8.3 Hz, 1H), 7.46 - 7.17 (m, 2H), 2.70 - 2.64 (m, 1H), 1.99 - 1.92 (m, 1H), 1.60 - 1.40 (m, 2H). |
| 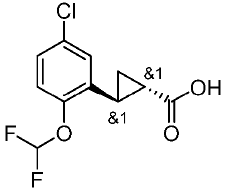 **Intermediate 42:** *rac*-(1*S**,2*S**)-2-(5-chloro-2-(difluoromethoxy) phenyl)cyclopropane-1-carboxylic acid ESI-MS (M+H)+: 261 | 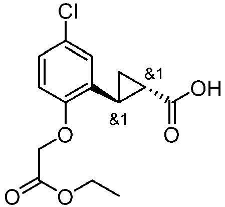 **Intermediate 43:** *rac*-(1*S**,2*S**)-2-(5-chloro-2-(2-ethoxy-2-oxoethoxy)phenyl)cyclopropane-1-carboxylic acid ESI-MS (M+H)+: 297 |
| 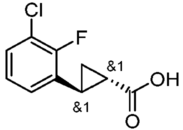 **Intermediate 44:** *rac*-(1*S**,2*S**)-2-(3-chloro-2-fluorophenyl)cyclopropane-1-carboxylic acid | ESI-MS (M+H)+: 213, ¹H NMR (400 MHz, CDCl₃) 7.26 (1H, s), 7.30 - 7.22 (1H, m), 7.00 (1H, dd, J=8.0, 8.0 Hz), 6.89 (1H, dd, J=6.7, 6.7 Hz), 2.78 - 2.70 (1H, m), 1.98 - 1.91 (1H, m), 1.43 (1H, ddd, J=4.6, 6.8, 8.3 Hz); |
| 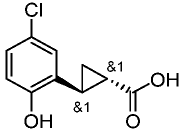 **Intermediate 45:** *rac*-(1*S**,2*S**)-2-(5-chloro-2-hydroxyphenyl)cyclopropane-1-carboxylic acid | ESI-MS (M+H)+: 211 |
| 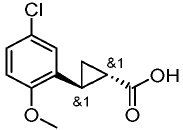 **Intermediate 46:** *rac*-(1*S**,2*S**)-2-(5-chloro-2-methoxyphenyl)cyclopropane-1-carboxylic acid | ESI-MS (M+H)+: 227, ¹H NMR (400 MHz, CDCl₃) δ , 7.15 (dd, J=2.3, 8.6 Hz, 1H), 6.87 (d, J=2.3 Hz, 1H), 6.77 (d, J=8.6 Hz, 1H), 3.84 (s, 3H), 2.76 (ddd, J=4.0, 6.8, 9.1 Hz, 1H), 1.85 - 1.79 (m, 1H), 1.42 - 1.36 (m, 1H). |

### Intermediate 47: rac-(1S*,2S*)-2-(5-chloro-2-hydroxyphenyl)cyclopropane-1-carboxamide

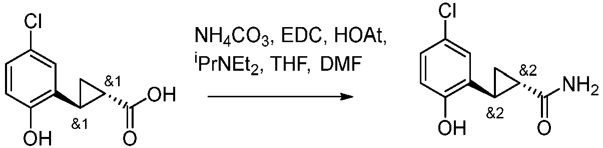

A solution of **intermediate 45** (100 mg, 0.47 mmol), EDC (108 mg, 0.56 mmol), HOAt (70 mg, 0.52 mmol), (NH₄)₂CO₃ (452 mg, 4.70 mmol) and DIPEA (0.37 mL, 2.12 mmol) in THF (4 mL) and DMF (4 mL) was stirred at 50°C for 16 h. Water was added and the mixture was extracted with EtOAc. The organic extract was washed with brine and concentrated *in vacuo.* The crude product was purified by dry flash silica chromatography eluting with a gradient of 5-100 % EtOAc in cyclohexane to give **intermediate 47** as a mixture of diastereoisomers (73 mg, 73 %). ESI-MS (M+H)-: 210, ¹H NMR (400 MHz, CDCl₃) δ , 6.99 (dd, J=2.3, 8.6 Hz, 1H), 6.82 (d, J=2.0 Hz, 1H), 6.77 (d, J=8.6 Hz, 1H), 6.45 - 6.36 (m, 1H), 6.00 - 5.89 (m, 1H), 2.60 - 2.51 (m, 1H), 1.70 - 1.61 (m, 0H), 1.57 - 1.48 (m, 1H), 1.29 - 1.18 (m, 1H).

The compounds in Table A2 were synthesized using a similar method to **intermediate 47** starting from the appropriate carboxylic acids. **Table A2**

| Compound | Coupling partner | Analytical Data |
|---|---|---|
| 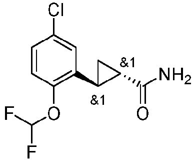 **Intermediate 48:** *rac-*(1*S*,*2*S**)-2-(5-chloro-2-(difluoromethoxy) phenyl)cyclopropane-1-carboxamide | *rac-*(1*S*,*2*S**)-2-(5-chloro-2-(difluoro methoxy) phenyl)cyclopropan e-1-carboxylic acid | compound used without purification |
| 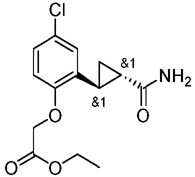 **Intermediate 49:** *rac*-ethyl 2-(2-((1*S**,2*S**)-2-carbamoylcyclopropyl)-4-chlorophenoxy)acetate | **Intermediate 43** | ESI-MS (M+H)+: 298, ¹HNMR (400 MHz, DMSO) δ 7.58 - 7.54 (m, 1H), 7.18 (dd, J=2.6, 8.8 Hz, 1H), 6.95 - 6.89 (m, 3H), 4.85 (s, 2H), 4.18 (q, J=7.1 Hz, 2H), 2.50 - 2.47 (m, 1H), 1.91 - 1.85 (m, 1H), 1.30-1.28 (m, 1H), 1.26-1.20 (m, 4H). |

### Intermediate 50: (1S*,2S*)-2-(3-chloro-4-fluorophenyl)cyclopropanecarboxylic acid

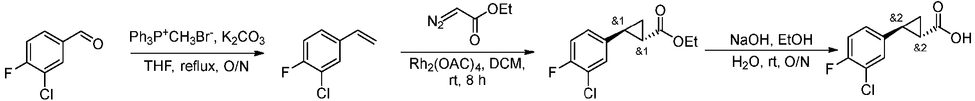

5-chloro-2-fluorobenzaldehyde (5.0 g, 30 mmol) was added to a stirred suspension of potassium carbonate (8.0 g, 60 mmol) and methyltriphenylphonium bromide (17.0 g, 50 mmol) in anhydrous THF (150mL) under nitrogen. After stirring at reflux overnight, the reaction mixture was cooled and filtered. The filtrate was concentrated *in vacuo* and the residue was purified by silica gel column chromatography (petroleum ether) to afford ***2-chloro-1-fluoro-4-vinylbenzene* intermediate 51** (3.5 g, 71%) as a colourless oil. ¹H NMR (400 MHz, CDCl₃,) δ 7.43 (dd, J= 7.2, 2.0 Hz, 1H), 7.27-7.23 (m, 1H), 7.08 (t, *J =* 9.6 Hz, 1H), 6.44-6.57 (m, 1H), 5.68 (d, *J =* 17.6 Hz, 1H), 5.27 (d, *J =* 10.8 Hz, 1H). To a suspension of **intermediate 51** (1.0 g, 6.4 mmol) and diacetoxyrhodium (0.57 g, 1.3 mmol) in dichloromethane (40mL) was added ethyl diazoacetate (4.4 g, 38.4 mmol) in DCM (20mL) over 8 hours. After addition, the mixture was filtered, and the filtrate was concentrated *in vacuo.* The residue was purified by silica gel column chromatography (petroleum ether: ethyl acetate = 10:1) to afford *rac-****(1S*,2S*)-ethyl 2-(3-chloro-4-fluorophenyl) cyclopropanecarboxylate* intermediate 52** (0.75 g, 48%) as a colourless oil. 1H NMR (400 MHz, CDCl₃,) δ 7.13 (dd, J = 7.2, 2.0 Hz, 1H), 7.04 (t, J = 8.8 Hz, 1H), 6.99-6.95 (m, 1H), 4.18 (q, J = 7.2 Hz, 2H),2.50-2.45 (m, 1H), 1.87-1.83 (m, 1H), 1.62-1.57 (m, 1H), 1.34-1.23 (m, 4H).

To a solution of **intermediate 52** (0.5 g, 2.1 mmol) in ethanol (40 mL) and water (10 mL) was added sodium hydroxide (0.3 g, 8.3 mmol). The mixture was stirred at room temperature overnight the concentrated *in vacuo.* The residue was diluted with water (20 mL), and acidified with 1N HCl to pH = 1-2. The resulting precipitate was collected by filtration. The filter cake was dried in vacuum to afford **intermediate 50** (0.3 g, 68%) as a white solid. ESI-MS [M +H] +: 214.9, ¹H NMR (400 MHz, CDCl₃): δ 7.15 (dd, *J =* 6.8 Hz, 2.0 Hz, 1H), 7.05 (t, *J =* 8.8 Hz, 1H), 7.01-6.97 (m, 1H), 2.58-2.53 (m, 1H), 1.88-1.84 (m, 1H), 1.68-1.64 (m,1H), 1.38-1.33 (m, 1H).

The compounds in Table A3 were synthesized using a similar method to *rac* **intermediate 50** starting from the appropriate benzaldehyde derivatives: **Table A3**

| | |
|---|---|
| 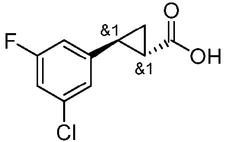 | **Intermediate 53:** *(rac)-(1S*,2S*)-2-(3-chloro-5-fluorophenyl)cyclopropanecarboxylic acid* ESI-MS [M +H] +: 214.9, δ 12.39 (s, 1H), 7.23 (dt, *J =* 8.8, 2.0 Hz, 1H), 7.19 (s, 1H), 7.07 (dt, *J =* 10.4, 2.0 Hz, 1H), 2.49-2.44 (m, 1H), 1.94-1.90 (m, 1H), 1.46-1.39 (m, 2H). |
| 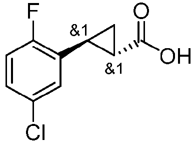 | **Intermediate 54:** *(rac)-(1S*,2S*)-2-(5-chloro-2-*fluorophenyl)cyclopropanecarboxylic acid ESI-MS [M +H] +: 214.9, δ 7.33-7.29 (m, 1H), 7.25-7.19 (m, 2H), 2.47-2.42 (m, 1H), 1.93-1.88 (m, 1H), 1.49-1.39 (m, 2H). |

### Intermediate 55: (1S,2S)-2-(4-methylpyrimidin-2-yl)cyclopropane-1-carboxamide

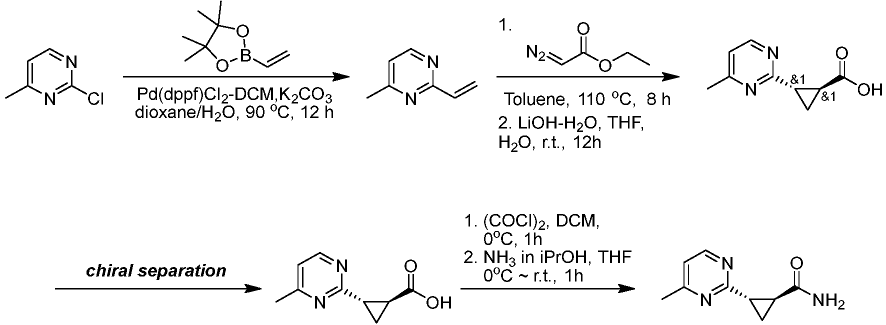

A mixture of 2-chloro-4-methylpyrimidine (6.4 g, 50 mmol), 4,4,5,5-tetramethyl-2-vinyl-1,3,2-dioxaborolane (10 g, 65 mmol), Pd(dppf)Cl₂-DCM (2 g, 2.5 mmol) and K₂CO₃ (17.25 g, 125 mmol) in dioxane (100 mL) and H₂O (5 ml) was stirred at 90°C for 12 h. The reaction mixture was treated with H₂O (50 mL) and extracted with EA (50 ml x 3). The combined organic layers were washed with brine (50 mL), dried over Na₂SO₄ and concentrated *in vacuo* (at 30°C). The crude product was purified by silica column chromatography (PE/EtOAc = 5/1) to give ***4-methyl-2-vinylpyrimidine* intermediate 56** (4.8 g, 80%) as yellow oil. ESI-MS [M +H]⁺: 121.2.

A solution of **intermediate 56** (4.8 g, 40 mmol) and ethyl 2-diazoacetate (9.12 g, 80 mmol) in toluene (70 mL) was refluxed at 110°C for 8 h. The reaction was concentrated *in vacuo* and the crude product was purified by silica gel column (PE/EA = 5/1) to give ***rac-ethyl (1S*, 2S*)-2-(4-methylpyrimidin-2-yl)cyclopropane-1-carboxylate* intermediate 57** (3.6 g, 43.7%) as yellow oil. ESI-MS [M +H]⁺: 207.2. A mixture of **intermediate 57** (3.6 g, 17.5 mmol) and LiOH-OH (1.4 g, 35 mmol) in THF/H₂O (20 mL / 10 mL) was stirred at room temperature for 12 h. The reaction was concentrated *in vacuo* to remove THF and the pH of the residue was adjusted to 3 by HCl (2N). The white solid was precipitated, and mixture was filtered, dried to give ***rac-(1S*,2S*)-2-(4-methylpyrimidin-2-yl)cyclopropane-1-carboxylic acid* intermediate 58** (2.4 g, 77%) as a white solid. ESI-MS [M +H]+: 179.2. Purity: 95%. 1H NMR (400 MHz, DMSO) δ 12.45 (s, 1H), 8.51 (d, J = 5.1 Hz, 1H), 7.20 (d, J = 5.1 Hz, 1H), 2.58 - 2.52 (m, 1H), 2.41 (S, 3H), 2.07 - 2.03 (m, 1H), 1.54 - 1.45 (m, 2H).

### Separation of intermediate 58

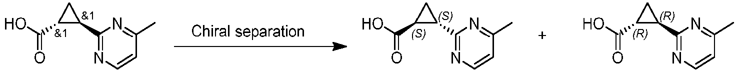

The mixture was separated using chiral column separation (Daicel CHIRALPAK AY, 250mm L × 50 mm I.D., 10 µm, EtOH/HOAc) = 100/0.1(V/V), 65 mL/min, 38 °C) to give two enantiomers: **(1S,2S)-2-(4-methylpyrimidin-2-yl)cyclopropane-1-carboxylic acid intermediate 59** (first eluting isomer): and **(1R,2R)-2-(4-methylpyrimidin-2-yl)cyclopropane-1-carboxylic acid intermediate 60** (second eluting isomer).

**Intermediate 59:** ESI-MS [M + H ]+: 179.1. Purity: 95%. 1H NMR (400 MHz, DMSO) δ 12.45 (s, 1H), 8.51 (d, J = 5.1 Hz, 1H), 7.20 (d, J = 5.1 Hz, 1H), 2.57 - 2.52 (m, 1H), 2.41 (S, 3H), 2.07 - 2.03 (m, 1H), 1.53 - 1.46 (m, 2H). RT = 4.6 min, 99% e.e.

**Intermediate 60: :** ESI-MS [M + H ]+: 179.1. Purity: 95%. 1H NMR (400 MHz, DMSO) δ 12.44 (s, 1H), 8.51 (d, J = 5.1 Hz, 1H), 7.20 (d, J = 5.1 Hz, 1H), 2.56 - 2.51 (m, 1H), 2.41 (S, 3H), 2.07 - 2.03 (m, 1H), 1.53 - 1.46 (m, 2H). RT = 5.9 min, 99% e.e.

To a solution of **intermediate 59** (1.1 g, 6.1 mmol) in dry DCM (20 mL) was added (COCl)₂ (1.56 g, 12.3 mmol) at 0°C slowly and was stirred at 0 °C for another 1 h. The reaction mixture was concentrated *in vacuo* and the resulting acid chloride was dissolved in dry THF (20 mL), cooled to 0 °C and then added NH3 (20 mL, 2 M solution in iPrOH). The resulting solution was stirred at room temperature for another 1 h and concentrated *in vacuo* to give crude, which was purified with silica gel chromatography (eluent: DCM/MeOH = 20/1) to furnish **intermediate 55** (900 mg, 81.2%) as a yellow solid. ESI-MS [M +H]+: 178.1.

### Intermediate 61: rac-ethyl (1R*2R*)-2-fluoro-2-(4-methylpyrimidin-2-yl)cyclopropane-1-carboxylate

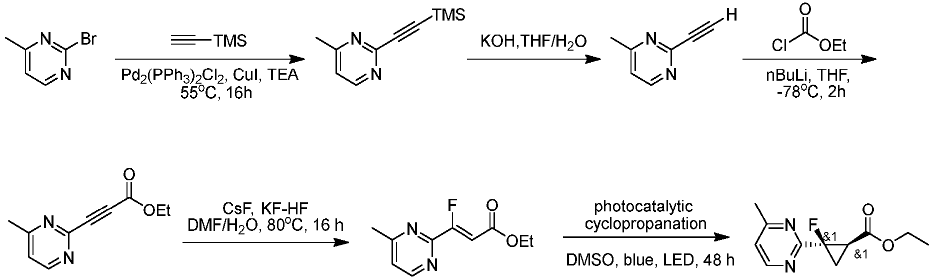

To a solution of 2-bromo-4-methylpyrimidine (10 g, 58 mmol) in TEA (29.4 g, 290 mmol) were added ethynyltrimethylsilane (17.1g, 174.4 mmol), Pd₂(PPh₃)₂Cl₂ (2.04 g, 2.9 mmol) and CuI (2.21 g, 11.6 mmol) under N₂ protection. The resulting reaction was stirred at 50°C for 16 h, cooled to room temperature and concentrated to give the crude, which was purified by silica gel chromatography (EtOAc/PE = 1/3) to give **4-methyl-2-((trimethylsilyl)ethynyl)pyrimidine intermediate 62** (9 g, 82%) as a yellow solid. ESI-MS [M +H]⁺: 191.1.

To a solution of **intermediate 62** (9 g, 47.4 mmol) in THF (36 mL) was added KOH (8 g, 142 mmol) in water (140 mL). After stirring at room temperature for 2h, the reaction was extracted with EtOAc/MeOH (10/1, 3 x 200 mL). The combined organics were washed by brine (200 mL), concentrated *in vacuo* and the residue purified by silica gel chromatography (EA/PE = 1/1) to give **2-ethynyl-4-methylpyrimidine intermediate 63** (1.5 g, 81%) as a yellow solid. ESI-MS [M + H]⁺: 119.1.

To a solution of **intermediate 63** (1 g, 8.4 mmol) in THF (15 mL) was added n-BuLi (3.87 mL, 2.4M solution in hexane 9.3 mmol) at -78°C under N₂ and the resulting mixture was stirred at -78°C for 2 h. Ethyl carbonochloridate (3.8 g, 39.3 mmol) was added and the resulting mixture was stirred at -78°C for another 1 h. The pH of reaction was adjusted to 7 with HCl (1N) and extracted with EtOAc (3 x 80 mL). The combined organic layers were washed with brine (60 mL), dried over Na₂SO₄ and concentrated *in vacuo* to give the crude, which was purified by silica gel chromatography (EtOAc/PE = 1/4) to give **ethyl 3-(4-methylpyrimidin-2-yl)propiolate intermediate 64** (780 mg, 49%) as a white solid. ESI-MS [M + Na]⁺: 191.1

To a solution of **intermediate 64** (780 mg, 4.1 mmol) in DMF/H₂O (15 mL/0.3 mL) was added KF-HF (350 mg, 4.5 mmol) and CsF (3.12 g, 20.5 mmol). The reaction mixture was stirred at 90°C for 16 h. Water (60 mL) was added to the reaction and extracted with EtOAc (3 x 800 mL). The combined organic layers were washed with brine, dried over Na₂SO₄, concentrated *in vacuo* to give the crude, which was purified by silica gel chromatography (EtOAc/PE = 1/5) to give **ethyl (Z)-3-fluoro-3-(4-methylpyrimidin-2-yl)acrylate intermediate 65** (370 mg, 43%) as colorless oil. ESI-MS [M + H]⁺: 211.1

A flame dried 10 mL round bottom flask equipped with a magnetic stir bar was charged with **intermediate 65** (370 mg, 1.76 mmol), 4CzIPN (2,4,5,6-tetra(9H-carbazol-9-yl)isophthalonitrile, 69 mg, 0.088 mmol), triethylammonium bis(catecholato)iodomethylsilicate (1.284 g, 2.647 mmol) and anhydrous DMSO (5 mL). The resulting solution was degassed (Vac/N₂, 3 cycles) and placed in front of two blue LEDs (Kessil, H150, 32W). The lamps were arranged on both sides of the flask as close as possible to the surface. A fan for cooling was mounted over the setup. The reaction was left under irradiation for 48 h. The resulting reaction was poured into water (50 mL) and extracted with EtOAc:MeOH (10:1, 3 x 50 mL). The organic layers were washed with brine (50 mL), dried over Na₂SO₄, concentrated *in vacuo* and purified by silica gel chromatography (EtOAc/PE = 1/4) to give **intermediate 61** (140 mg, 35%) as yellow oil. ESI-MS [M + H]+: 225.2.

### Intermediate 66:rac-ethyl (1S*,2S*)-2-(5-fluoro-4-methylpyrimidin-2-yl)cyclopropane-1-carboxylate

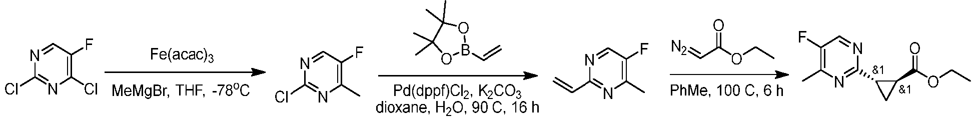

To a mixture of 2,4-dichloro-5-fluoropyrimidine (5 g, 30.1 mmol) in THF (80 mL) was added Fe(acac)₃ (1.17 g, 3.31 mmol). After cooling to -78°C, CH₃MgBr (3M solution in Et₂O, 17 mL, 51.5 mmol) was added dropwise. After stirring at -78°C for 2 h under N₂, the reaction was quenched with saturated aqueous NH₄Cl (20 mL) and extracted with EtOAc ( 3 x 30 mL). The combined organic layers were washed with brine (30 mL), dried over anhydrous Na₂SO₄, and concentrated *in vacuo* to give the crude, which was purified by column chromatography (eluent: PE/EtOAc = 15/1) to give **2-chloro-5-fluoro-4-methylpyrimidine intermediate 67** (3.8 g, 86%) as a yellow oil. ESI-MS [M +H]+: No MS.

To a mixture of **intermediate 67** (3.8 g, 26 mmol), 4,4,5,5-tetramethyl-2-vinyl-1,3,2-dioxaborolane (4.8 g, 31.2 mmol) and K₂CO₃ (10.8 g, 78 mmol) in dioxane/H₂O (80/20 mL) was added Pd(dppf)Cl₂ (952 mg, 1.3 mmol). The resulting reaction mixture was stirred at 90°C for 16 h under N₂. After cooling to 25°C, water (50 mL) was added and the mixture was extracted with EtOAc (3 x 30 mL). The combined organic layers were washed with brine (60 mL), dried over anhydrous Na₂SO₄, and concentrated *in vacuo* to give the crude, which was purified by column chromatography (eluent: PE/EtOAc = 15/1) to give **5-fluoro-4-methyl-2-vinylpyrimidine intermediate 68** (2 g, 56%) as a yellow oil. ESI-MS [M +H]⁺: No MS.

To a solution of **intermediate 68** (500 mg, 3.62 mmol) in toluene (5 mL) was added ethyl 2-diazoacetate (1.24 g, 10.87 mmol). The resulting reaction mixture was stirred at 100°C for 6 h under N₂, cooled to room temperature and then concentrated *in vacuo* to give the crude, which was purified by silica gel chromatography (eluent: PE/EtOAc = 5/1) to give **intermediate 66** (200 mg, 25%) as a yellow oil. ESI-MS [M +H]+: 225.2.

### Intermediate 69: rac-(1S*,2S*)-2-(4-methylthiazol-2-yl)cyclopropane-1-carboxamide

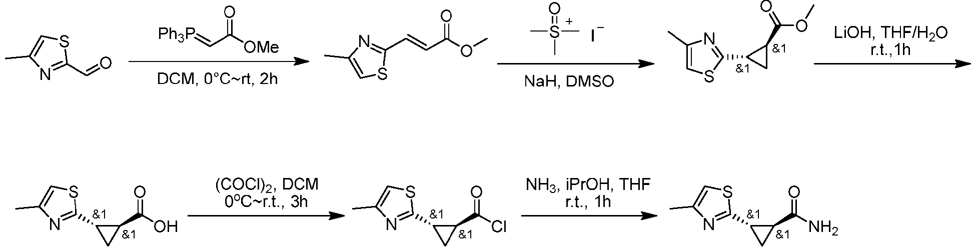

To a mixture of 4-methylthiazole-2-carbaldehyde (500 mg, 3.94 mmol) in dry DCM (20.0 mL) was added methyl 2-(triphenyl-l5-phosphaneylidene)acetate (1.32 g, 3.94 mmol) at 0°C and the resulting reaction mixture was stirred at rt for 2 h. Water (50 mL) was added and the mixture was extracted with DCM (3 x 30 mL). The combined organic layers were washed with brine (50 mL), dried over anhydrous Na₂SO₄, and concentrated *in vacuo* to give the crude, which was purified by column chromatography (eluent: PE/EtOAc = 10/1) to give **methyl (E)-3-(4-methylthiazol-2-yl)acrylate intermediate 70** (300 mg, 41.6 %) as a yellow oil. ESI-MS [M +H]⁺: 184.2.

A mixture of trimethylsulfoxonium iodide (0.726 g, 3.3 mmol) and NaH (132 mg, 60%, 3.3 mmol) in THF (25 mL) was stirred at room temperature for 40 min. Then a solution of **ethyl (E)-3-(2-methylthiazol-4-yl)acrylate intermediate 70** (200 mg, 1.1 mmol) in DMSO (5 mL) was added and stirred at 50°C for 2 h. The mixture was quenched with saturated aqueous NaHCO₃ (30 mL) then extracted with EtOAc (3 x 30 mL). The combined organic layers were washed with brine (50 mL), dried over Na₂SO₄, concentrated *in vacuo* to give the crude product, which was purified with preparative TLC (eluent: PE/EtOAc = 4/1) to give **rac-methyl (1S*,2S*)-2-(4-methylthiazol-2-yl)cyclopropane-1- carboxylate intermediate 71(**50 mg, 23 %). ESI-MS [M +H]+: 198.2

A mixture of **intermediate 71** (100 mg, 0.5 mmol) and LiOH-H₂O (63 mg, 1.5 mmol) in THF/H₂O (2 mL/2 mL) was stirred at room temperature for 1 h. The pH of the mixture was adjusted to 6 with HCl solution (4M, aq.) and then concentrated *in vacuo* to give **rac-(1S*,2S*)-2-(4-methylthiazol-2- yl)cyclopropane-1-carboxylic acid intermediate 72** as a white solid (150 mg, crude), which was used directly in the next step. ESI-MS [M +H]⁺: 184.1.

To a mixture of **intermediate 72** (150 mg, crude) in DCM (5 mL) was added (COCl)₂ (192 mg, 1.52 mmol) at 0°C. The mixture was stirred for 3 h at room temperature and then concentrated *in vacuo* to give ***rac-(1S*,2S*)-* 2-(4-methylthiazol-2-yl)cyclopropane-1-carbonyl chloride intermediate 73** (150 mg, crude), which was used directly in the next step. ESI-MS [M +H]⁺: 198.1.

To a solution of NH₃ in IPA (3 mL) was added a solution of **intermediate 73** (150 mg, crude from previous step) in THF (1 mL) at 0°C under N₂. The resulting mixture was allowed to warm to room temperature and stirred for 1 h then concentrated *in vacuo* and purified by preparative TLC (eluent: DCM/MeOH=10/1) to give **intermediate 69** as a white solid (60 mg, 66%). ESI-MS [M +H]⁺: 183.0.

### Intermediate 74: rac-ethyl (1S*,2S*)-2-(2-methylthiazol-4-yl)cyclopropane-1-carboxylate

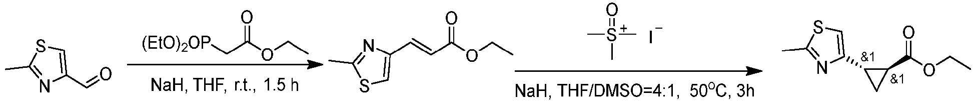

To a mixture of NaH (202 mg, 60% suspension in paraffin oil, 5.06 mmol) in THF (20 mL) was added ethyl 2-(diethoxyphosphoryl) acetate (1.1 g, 5.06 mmol). The reaction mixture was stirred at room temperature for 0.5 h. Then a solution of 2-methylthiazole-4-carbaldehyde (586 mg, 4.6 mmol) in THF (5 mL) was added. The reaction was stirred at room temperature for another 1h. The mixture was quenched with saturated aqueous NaHCO₃ (20 mL). The aqueous phase was extracted with EtOAc (3 x 30 mL). The combined organic layers were washed with brine (50 mL), dried over Na₂SO₄, concentrated *in vacuo* to give the crude product, which was purified with flash chromatography (eluent: PE/ EtOAc = 2/1) to give the product **ethyl (E)-3-(2-methylthiazol-4-yl)acrylate intermediate 75** as a white solid. (700 mg, 77%). ESI-MS [M +H]+: 198.1
A mixture of trimethylsulfoxonium iodide (1.1 g, 2.25 mmol) and NaH (182 mg, 4.55 mmol) in THF (24 mL) was stirred at room temperature for 40 min. Then a solution of **intermediate 75** (690 mg, 3.5 mmol) in DMSO (6 mL) was added and stirred at 50°C for 2 h. The mixture was quenched with saturated aqueous NaHCO₃ (30 mL), extracted with EtOAc (3 x 30 mL). The combined organic layers were washed with brine (50 mL), dried over Na₂SO₄ then concentrated *in vacuo* to give the crude product, which was purified with preparative TLC (eluent: PE/EtOAc = 4/1) to give rac-ethyl **intermediate 74** as a colorless oil. (360 mg, 48 %) ESI-MS [M +H]+: 212.2

### Intermediate 76: (1R*,3R*)-2,2-difluoro-3-(4-methylpyrimidin-2-yl)cyclopropane-1-carboxylic acid

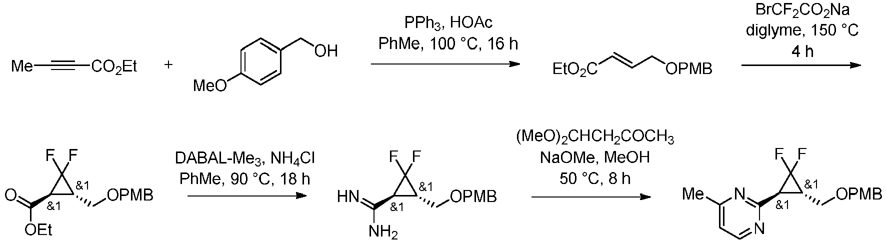

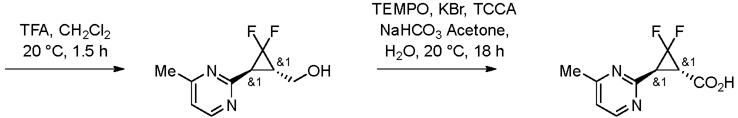

A solution of ethyl tetrolate (7.22 g, 64.4 mmol), 4-methoxybenzyl alcohol (9.34 g, 67.6 mmol), triphenylphosphine (844 mg, 3.22 mmol) and acetic acid (736 µL, 12.9 mmol) in toluene (65 ml) was heated at 100°C for 16 h. After being cooled to room temperature, the mixture was concentrated *in vacuo* and the resulting oil loaded onto a silica gel column (350 g) equilibrated with heptane : ethyl acetate = 85:15. The column was eluted with this mixture (3L) to obtain **ethyl (E)-4-((4-methoxy benzyl)oxy)but-2-enoate intermediate 77** as a colourless oil (11.92 g) used without further purification. ESI-MS [M + Na] 273, ¹H NMR (400 MHz, CDCl₃, ppm) δ 7.27 (d, *J* 7 Hz, 2H), 6.97 (dt, *J* 16, 4 Hz, 1H), 6.89 (d, *J* 7 Hz, 2H), 6.11 (dt, *J* 16, 2 Hz, 1H), 4.49 (s, 2H), 4.20 (q, *J* 7 Hz, 2H), 4.15 (dd, *J* 4, 2 Hz, 2H), 3.81 (s, 3H), 1.29 (t, *J* 7 Hz, 3H).

**Intermediate 77** (11.92 g, 47.6 mmol) was heated to 150°C under nitrogen. A solution of sodium bromodifluoroacetate (28.1 g, 143 mmol) in anhydrous diglyme (48 mL) was added via syringe pump over 4 h with vigorous stirring. After the addition was complete the mixture was stirred for another 10 min and allowed to cool to room temperature. Heptane (300 mL) and water (250 mL) were added, the layers were separated, and the organic layer was passed through a sinter funnel to remove a small amount of precipitate. The aqueous layer was extracted with heptane (2 × 50 mL). The combined organic solutions were washed with water (3 × 250 mL), dried (MgSO₄), filtered and concentrated *in vacuo* to obtain crude **(1S*,3S*)-2,2-difluoro-3-(((4-methoxybenzyl)oxy)methyl)cyclopropane-1-carboxylate intermediate 78** as a brown oil. ¹H NMR (400 MHz, CDCl₃, ppm) δ 7.25 (d, *J* 9 Hz, 2H), 6.89 (d, *J* 9 Hz, 2H), 4.49 (d, *J* 12 Hz, 1H), 4.44 (d, *J* 12 Hz, 1H), 4.20 (q, *J* 7 Hz, 2H), 3.81 (s, 3H), 3.58 (d, *J* 7Hz, 2H), 2.66-2.56 (m, 1H), 2.27 (dd, *J* 12, 8 Hz, 1H), 1.28 (t, *J* 7 Hz, 3H). ¹⁹F NMR (373 MHz, CDCl₃, ppm) δ -134.9 (ddd, *J* 155, 13, 1 Hz, 1F), -135.6 (dd, *J* 155, 13 Hz, 1F). An oven-dried 3-neck flask (250 mL) equipped with stir bar, reflux condenser and nitrogen inlet was charged with ammonium chloride (6.29 g, 118 mmol) and dry toluene (50 mL). The mixture was cooled to 0°C and DABAL-Me₃ (30.2 g, 118 mmol) was added in one portion. The mixture was stirred for another 10 min and a solution of **ethyl (1S*,3S*)-2,2-difluoro-3-(((4-methoxybenzyl)oxy)methyl)cyclopropane-1-carboxylate intermediate 78** (7.07 g, 23.5 mmol) in dry toluene (66 mL) was added via syringe. The cooling bath was removed, and the mixture was heated to 90°C (DrySyn) for 18 h. The mixture was cooled to room temperature and slowly poured into a stirred mixture of methanol : water =9:1 (400 mL) and Dicalite (100 g). After being stirred for another 20 min (until all effervescence had ceased), the solids were filtered. The resulting filter cake was washed with methanol (4 × 100 mL). The combined filtrates were concentrated under reduced pressure, and the resulting residue was co-evaporated with acetonitrile (3 × 200 mL) and then taken up in additional acetonitrile (100 mL). The solids were removed by filtration and washed with acetonitrile (3 × 25 mL). The combined filtrates were concentrated *in vacuo* to obtain crude **(1S*,3S*)-2,2-difluoro-3-(((4-methoxybenzyl)oxy) methyl)cyclopropane-1-carboximidamide intermediate 79** (ca 10 g), as a brown gum, which was used in the next step without further purification. ESI-MS [M + H]⁺ 271. 1,1-Dimethoxypropan-2-one (3.42 mL, 28.2 mmol) and a solution of sodium methoxide (1 M in methanol, freshly prepared, 47 mL, 47 mmol) were added sequentially to a solution of crude **intermediate 79** (~ 10 g; assumed 23.5 mmol amidine) in anhydrous methanol (60 mL). The resulting solution was heated at 50°C for 8 h. The mixture was concentrated *in vacuo,* and the residue was taken up in tert-butyl methyl ether (200 mL) and saturated aqueous sodium bicarbonate (150 ml). The layers were separated, and the aqueous layer was extracted with tert-butyl methyl ether (50 mL). The combined organic layers were washed with brine and dried (MgSO₄). The solution was filtered and concentrated *in vacuo* to obtain a brown oil, which was purified on a silica cartridge (Silicycle, Silaprep 220 g, 120 ml/min) using a gradient of heptane : ethyl acetate = 7:3-55:45 (over 12 column volumes) to afford **2-((1S*,3S*)-2,2-difluoro-3-(((4-methoxybenzyl)oxy)methyl)cyclopropyl)-4-methylpyrimidine intermediate 80** (4.44 g, 59% over 2 steps), as a light yellow oil. ESI-MS [M + H]⁺ 321, ¹H NMR (400 MHz, CDCl₃, ppm) δ 8.50 (d, *J* 5 Hz, 1H), 7.27 (d, *J* 8 Hz, 2H), 7.02 (d, *J* 5 Hz, 1H), 6.88 (d, *J* 8 Hz, 2H), 4.54 (d, *J* 12 Hz, 1H), 4.47 (d, *J* 12 Hz, 1H), 3.80 (s, 3H), 3.76-3.67 (m, 2H), 3.05-2.95 (m, 1H), 2.86 (dd, *J* 14, 7 Hz, 1H), 2.50 (s, 3H).

¹⁹F NMR (373 MHz, CDCl₃, ppm) δ -133.0 (dd, *J* 155, 14 Hz, 1F), -139.2 (ddd, *J* 155, 14, 3 Hz, 1F). **Intermediate 80** (710 mg, 2.22 mmol) was dissolved in DCM (40.5 mL), and trifluoroacetic acid (4.0 mL, 52.2 mmol) was added followed by water (500 µL, 27.8 mmol). The reaction mixture was stirred at room temperature for 1.5 h. The mixture was neutralised (to pH7) by the addition of solid sodium bicarbonate and water (45 mL). The aqueous layer was separated and extracted with DCM (3 × 45 mL). The organic solutions were combined, washed with water (45 mL), dried over Na₂SO₄ and concentrated *in vacuo* to give a red solid. Purification by flash chromatography (loading the sample in solution in ethyl acetate, Silicycle, Silaprep 25 g cartridge), eluting with a gradient of heptane : ethyl acetate = 90:10-0:100, then ethyl acetate : methanol = 100:0-80:20) to afford **((*1S**,*3S**)-2,2-difluoro-3-(4-methylpyrimidin-2-yl)cyclopropyl)methanol intermediate 81** (353 mg, yield 80%), as a colourless solid. ESI-MS [M + H]⁺ 201, ¹H NMR (396 MHz, CDCl₃) δ 8.51 (d, *J* 5.4 Hz, 1H), 7.04 (d, *J* 5.4 Hz, 1H), 3.98-3.87 (m, 2H), 3.04-2.90 (m, 2H), 2.51 (s, 3H).

¹⁹F NMR (373 MHz, CDCl₃) δ -133.8 - -134.3 (m, 1F), -138.7 (ddd, *J* 157, 13, 2 Hz, 1F). **Intermediate 81** (590 mg, 2.95 mmol) was dissolve in acetone (30 mL) and saturated aqueous sodium bicarbonate (8.7 mL) was added to obtain a cloudy solution. The solution was cooled to 0°C in an ice bath, and potassium bromide (71 mg, 0.60 mmol) and (2,2,6,6-tetramethylpiperidin-1-yl)oxyl (TEMPO, 9 mg, 0.058 mmol) were added. Trichloroisocyanuric acid (1.378 g, 5.93 mmol ) was added in small portions over 10 minutes. The reaction mixture was allowed to reach room temperature and stirred at room temperature for 18h. The reaction was quenched by the addition of isopropanol (2 mL) and acetone (30 mL), and the mixture was filtered. The filtrate was concentrated *in vacuo* to remove acetone and isopropanol. The resulting aqueous solution was diluted with water, filtered again, and the solid was washed with DCM (30 mL). The biphasic filtrate was concentrated *in vacuo* to remove the DCM. The aqueous solution (pH 7) was rendered basic (pH 11) by the addition of aqueous sodium hydroxide (4M). The aqueous solution was washed with DCM (3 × 15 mL), neutralised (pH 7) by adding hydrochloric acid (2M), and then washed again with DCM (3 × 15 mL). Finally, the aqueous solution was acidified (pH 1) by adding hydrochloric acid (2M) and the product was extracted into DCM (4 × 15 mL). The pH of the aqueous layer was found to be ~2.5, therefore, more hydrochloric acid (2M) was added and further extractions with DCM (4 × 15 mL) were performed. The combined organics were concentrated *in vacuo* to obtain **(1S*,3S*)-2,2-difluoro-3-(4-methylpyrimidin-2-yl)cyclopropane-1-carboxylic acid intermediate 81** (447 mg, 71%), as a pale pink solid. ESI-MS [M + H]⁺ 215, ¹H NMR (396 MHz, CDCl₃): δ 8.59 (d, *J 5.4* Hz, 1H, H-2'), 7.13 (d, *J 5.4* Hz, 1H, H-3'), 3.79 (ddd, *J* 13, 7.7, 1.4 Hz, 1H, H-2), 3.51 (dd, *J* 13, 7.7 Hz, 1H, H-3), 2.55 (s, 3H, H-5').

¹⁹F NMR (373 MHz, CDCl₃) δ -130.8 (dd, *J* 150, 13 Hz, 1F), -135.3 - -135.7 (m, 1F).

### Intermediate 82: rac-methyl (1S*,3S*)-2,2-difluoro-3-(4-methylpyrimidin-2-yl)cyclopropane-1-carboxylate

To a mixture of **intermediate 81 (** (150 mg, 0.7 mmol) in CH₃OH (10 mL) was added SOCl₂ (167 mg, 1.7 mmol). The reaction mixture was stirred at 40°C for 3 h. The reaction mixture was poured into water (50 mL) then extracted with EtOAc (3 x 50 mL). The combined organics were washed with brine (50 mL), dried over Na₂SO₄, concentrated *in vacuo* to give the crude, which was purified by flash chromatography (PE/EtOAc = 5/1) to get **intermediate 82** as colorless oil (120 mg, yield 75%). ESI-MS [M +H] +: 229

### Intermediate 83: rac-ethyl (1S*,2S*)-2-(4-methyl-1,3,5-triazin-2-yl)cyclopropane-1-carboxylate

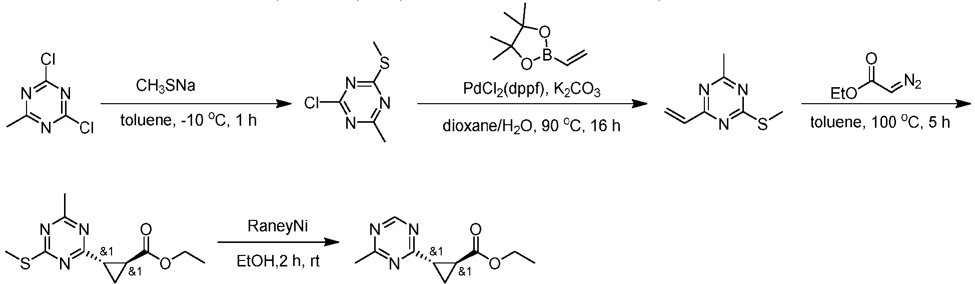

To a solution of 2,4-dichloro-6-methyl-1,3,5-triazine (2 g, 12.2 mmol) in toluene (20 mL) was added CH₃SNa (769 mg, 10.9 mmol) slowly at -10°C. The mixture was stirred for 1 h at -10°C then water (20 mL) was added. The mixture was extracted with EtOAc (3 x 30 mL). The combined organic layers were washed with brine (20 mL), dried over Na₂SO₄ then concentrated *in vacuo* to give the crude, which was purified by column chromatography (PE : EtAOc = 3 : 1) to give **2-chloro-4-methyl-6-(methylthio)-1,3,5-triazine intermediate 84** (1 g, 47.6%) as a white solid. ESI-MS [M +H]⁺: 176.0. To a mixture of **intermediate 84** (495 mg, 2.81 mmol) in dioxane (15 mL) and water (1.5 mL) was added 4,4,5,5-tetramethyl-2-vinyl-1,3,2-dioxaborolane (1.2 g, 3.15 mmol), PdCl₂(dppf) (206 mg, 0.28 mmol) and K₂CO₃ (1.16 g, 8.43 mmol) at 25°C. The resulting reaction mixture was then stirred at 90°C for 16 h then cooled to room temperature. The reaction mixture was filtered through Celite ^{®} and the filter cake was washed with DCM/MeOH (10/1, 50 mL). The filtrate was concentrated *in vacuo* to give the crude, which was purified by column chromatography (eluent: PE/EtAOc=20/1 ~ 10/1) to give **2-methyl-4-(methylthio)-6-vinyl-1,3,5-triazine intermediate 85** (250 mg, 53.2%) as yellow oil. ESI-MS [M +H]⁺: 168.0. To a mixture of intermediate 85 (250 mg, 1.5 mmol) in toluene (15 mL) was added ethyl 2-diazoacetate (512 mg, 4.5 mmol). The resulting reaction mixture was stirred at 100°C for 5 h under a N₂ atmosphere. The reaction was cooled to room temperature then filtered. The filtrate was concentrated to give the crude product, which was purified by silica column chromatography (eluent: PE/EtAOc = 3/1) to give ***rac*-ethyl (1S*, 2S*)-2-(4-methyl-6-(methylthio)-1,3,5-triazin-2-yl)cyclopropane-1-carboxylate intermediate 86** (100 mg, 26.3%) as a yellow solid. ESI-MS [M +H]⁺: 254.1.

A mixture of **intermediate 86** (100 mg, 0.4 mmol) and RaneyNi (100 mg) in EtOH (3 mL) was stirred at room temperature for 2 h. The reaction mixture was filtered, the filtrate was concentrated *in vacuo* to give **intermediate 83** (60 mg, 72.4%) as colorless oil, which was used into next step without further purification. ESI-MS [M +H]⁺: 208.1.

### Intermediate 87: rac-ethyl (1S*,2S*)-2-(4,6-dimethylpyrimidin-2-yl)cyclopropane-1-carboxylate

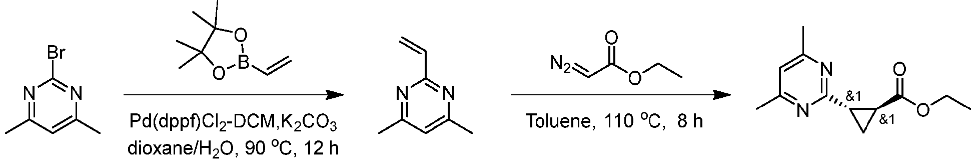

A mixture of 2-bromo-4,6-dimethylpyrimidine (900 mg, 4.8 mmol), 4,4,5,5-tetramethyl-2-vinyl-1,3,2-dioxaborolane (875 mg, 5.7 mmol), Pd(dppf)Cl₂-DCM (192 mg, 0.23 mmol) and K₂CO₃ (1.9 g, 14.1 mmol) in dioxane (30 mL) and water (3 ml) was stirred at 90°C for 12 h. Water (30 mL) was added to the reaction, extracted with EA (3 x 50 mL). The combined organic layers were washed with brine (50 mL), dried over Na₂SO₄, concentrated *in vacuo* to give the crude, which was purified by silica column chromatography (PE/EtOAc = 5/1) to give 5-(prop-1-en-2-yl)-2,3-dihydro-1H-inden-4-amine **intermediate 88** (500 mg, 78%) as a yellow solid. ESI-MS [M +H]⁺: 135.2.

A mixture of 4**,6-dimethyl-2-vinylpyrimidine intermediate 88** (500 mg, 3.7 mmol) and ethyl 2-diazoacetate (843 mg, 7.4 mmol) in toluene (30 mL) was heated at 110°C for 8 h. The reaction was cooled to room temperature and concentrated *in vacuo* to give the crude, which was purified by silica gel column (PE/EtOAc = 5/1) to give **intermediate 87** (250 mg, 31%) as a yellow solid. ESI-MS [M +H]⁺: 221.2.

The compounds in Table A4 were synthesized using a similar method to **intermediate 87** starting from the appropriate heteroaryl bromide or chloride derivatives: **Table A4**

| | |
|---|---|
| 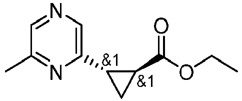 **Intermediate 89:** *rac-ethyl (1S*,2S*)-2-(6-methylpyrazin-2-yl)cyclopropane-1-carboxylate* ESI-MS [M +H]⁺: 207.1 | 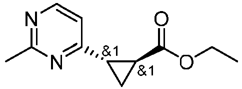 **Intermediate 90:** *rac*-ethyl (1S*,2S*)-2-(2-methylpyrimidin-4-yl)cyclopropane-1-carboxylate ESI-MS [M +H] ⁺: 207.1 |
| 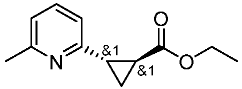 **Intermediate 91:** *rac*-ethyl (1S*,2S*)-2-(6-methylpyridin-2-yl)cyclopropane-1-carboxylate ESI-MS [M + H ]⁺: 206.1 | 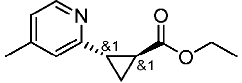 **Intermediate 92:** *rac*-ethyl (1S*,2S*)-2-(4-methylpyridin-2-yl)cyclopropane-1-carboxylate ESI-MS [M + H ]⁺: 206.1 |
| 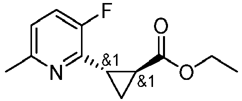 **Intermediate 93:** *rac*-ethyl (1S*, 2S*)-2-(3-fluoro-6-methylpyridin-2-yl)cyclopropane-1-carboxylate ESI-MS [M +H] ⁺: 224.1 | 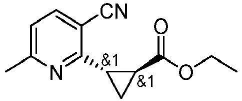 **Intermediate 94:** *rac*-ethyl (1S*,2S*)-2-(3-cyano-6-methylpyridin-2-yl)cyclopropane-1-carboxylate ESI-MS [M +H]⁺: 231.1 |
| 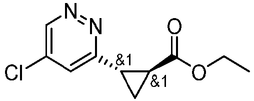 **Intermediate 95:** *rac*-ethyl (1S*,2S*)-2-(5-chloropyridazin-3-yl)cyclopropane-1-carboxylate ESI-MS [M +H]⁺: 227.2 | 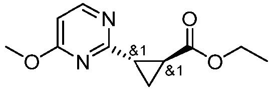 **Intermediate 96:** *rac-*ethyl (*1S*,2S**)-2-(4-methoxypyrimidin-2-yl)cyclopropane-1-carboxylate ESI-MS [M +H]⁺: 223.1 |
| 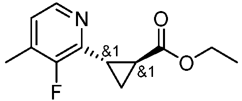 | **Intermediate 97:** *rac*-ethyl (1S*,2S*)-2-(3-fluoro-4-methyl pyridin-2-yl)cyclopropane-1-carboxylate ESI-MS [M +H]⁺: 244.2 |

### Intermediate 98: rac-(1S*,2S*)-2-(4-(fluoromethyl)pyrimidin-2-yl)cyclopropane-1-carboxamide

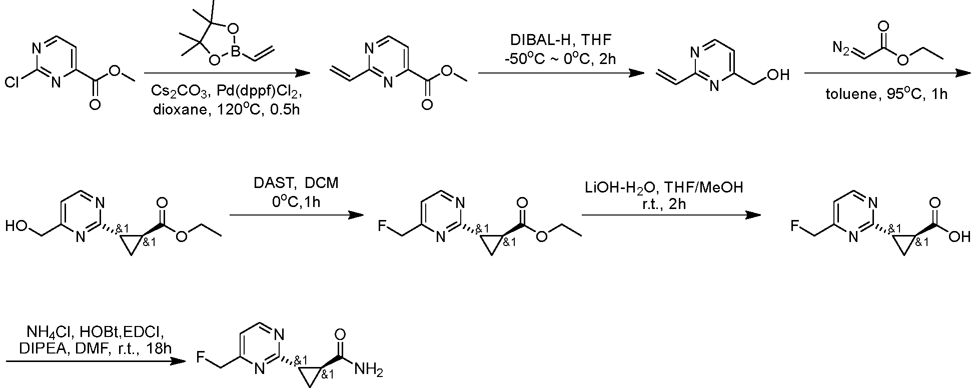

To a mixture of methyl 2-chloropyrimidine-4-carboxylate ( 1.2 g, 6.97 mmol), 4,4,5,5-tetramethyl-2-vinyl-1,3,2-dioxaborolane (1.61 g, 10.4 mmol) and Cs₂CO₃ (6.8 g, 20.9 mmol) in dioxane/H₂O (20/5 mL) was added Pd(dppf)Cl₂ (254.9 mg, 0.35mmol). The resulting reaction mixture was stirred at 120°C for 5 h under N₂. After cooling to room temperature, water (50 mL) was added and the mixture was extracted with EtOAc (3 x 50 mL). The combined organic layers were washed with brine (60 mL), dried over anhydrous Na₂SO₄, and concentrated *in vacuo* to give the crude, which was purified by column chromatography (eluent: PE/EtOAc = 15/1) to give **methyl 2-vinylpyrimidine-4-carboxylate intermediate 99** (700mg, 63.6%) as a yellow oil. ESI-MS [M +H]⁺: 165.2.

To a solution of **intermediate 99** (300 mg,1.8 mmol) in THF(6 mL) was added DIBAL-H (4.57 ml,1.0M in hexane) at -50⁰C. The mixture was stirred at 0⁰C for 2h, water(10 mL) was added and the resulting mixture was extracted with EtOAc (20 mL x 3). The combined organic layers were washed with brine (30 mL), dried over Na₂SO₄, filtered and concentrated *in vacuo* to give the crude, which was purified by preparative TLC(PE/EA = 5/1) to give **(2-vinylpyrimidin-4-yl)methanol intermediate 100** (180 mg, 73%) as a yellow solid. ESI-MS [M +H]+: 137.2.

To a solution of **intermediate 100** (180 mg, 1.3 mmol) in toluene (5 mL) was added ethyl 2-diazoacetate (444.7 mg, 3.97 mmol). The resulting reaction mixture was stirred at 95°C for 1 h under N₂. The reaction was then cooled to room temperature and concentrated *in vacuo* to give the crude, which was purified by preparative TLC (eluent: PE/EtOAc = 3/1) to give ***rac*-ethyl (1S*,2S*)-2-(4-(hydroxymethyl)pyrimidin-2-yl)cyclopropane-1-carboxylate intermediate 101** (100 mg, 34.6%) as a yellow oil. ESI-MS [M +H]+: 223.2.

To a solution of **intermediate 101** (100 mg,0.45 mmol) in DCM (3 mL) was added DAST (93.3 mg, 0.58 mmol) at 0°C, After stirring the resulting reaction mixture was stirred at 0°C for 1 h under N₂, water (20 mL) was added and extracted with EtOAc (20 mL x 3). The combined organic layers were washed with brine (20 mL), dried over Na₂SO₄, filtered and concentrated *in vacuo* to give the crude, which was purified by preparative TLC (PE/EtOAc= 5/1) to give ***rac*-ethyl (1S*,2S*)-2-(4-(fluoromethyl)pyrimidin-2-yl)cyclopropane-1-carboxylate intermediate 102** (60 mg, 60%) as a yellow oil. ESI-MS [M +H]+: 225.2.

Using a similar procedure to that for **intermediate 23, intermediate 98** (30 mg, 60%) was synthesised from **intermediate 102** (60 mg,0.26 mmol). ESI-MS [M +H]+: 196.2.

### Intermediate 103: rac-(1S*, 2S*)-2-(4-(difluoromethyl)pyrimidin-2-yl)cyclopropane-1-carboxamide

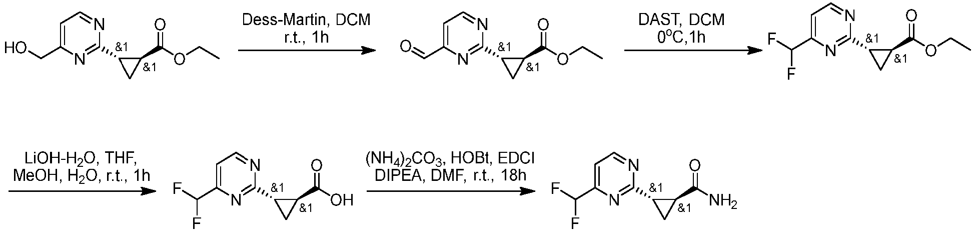

To a solution of *rac*-ethyl (1S*, 2S*)-2-(4-(hydroxymethyl)pyrimidin-2-yl)cyclopropane-1-carboxylate (200 mg, 0.9 mmol) in DCM (5 mL) was added Dess-Martin periodinane (457.9 mg, 1.08 mmol). The resulting reaction mixture was stirred at room temperature for 1 h under N₂. The reaction was quenched with saturated aqueous NH₄Cl (15 mL) and extracted with DCM (3 x 20 mL). The combined organic layers were washed with brine (30 mL), dried over Na₂SO₄, concentrated *in vacuo* to give the crude, which was purified by preparative TLC (eluent: PE/EtOAc = 5/1) to give ***rac*-ethyl (1S*, 2S*)-2-(4-formylpyrimidin-2-yl)cyclopropane-1-carboxylate intermediate 104** (170 mg, 85%) as a yellow solid. ESI-MS [M +H]+: 221.2.

To a solution of **intermediate 104** (170 mg, 0.77 mmol) in DCM (3 mL) was added DAST (149.5 mg,0.93 mmol) at 0°C, After stirring the resulting reaction mixture at 0°C for 1 h under N₂, water (20 mL) was added to the reaction, extracted with EtOAc (3 x 20 mL). The combined organic layers were washed with brine (30 mL), dried over Na₂SO₄, filtered and concentrated *in vacuo* to give the crude, which was purified by preparative TLC (PE / EtOAc= 5/1) to give **rac-ethyl (1S*, 2S*)-2-(4-(difluoromethyl)pyrimidin-2-yl)cyclopropane-1-carboxylate intermediate 105** (100 mg, 53.4%) as a yellow oil. ESI-MS [M +H]+: 243.2. Using a similar procedure to that for intermediate 23, **intermediate 103** (13 mg, 50%) was synthesised from **intermediate 105** (100 mg,0.41 mmol). ESI-MS [M +H]+: 196.2.

### Intermediate 106: rac-(1S*,2S*)-2-(4-(trifluoromethyl)pyrimidin-2-yl)cyclopropane-1-carboxamide

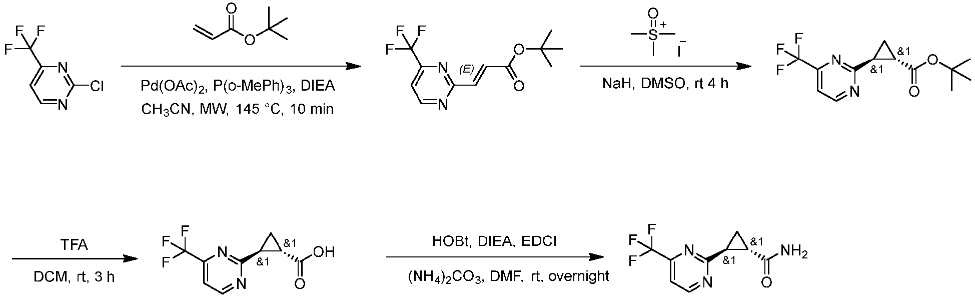

To a mixture of 2-chloro-4-(trifluoromethyl)pyrimidine (2 g, 11 mmol) and tert-butyl acrylate (4.21 g, 32.9 mmol) in CH₃CN (10 mL) was added Pd(OAc)₂ (490 mg, 2.19 mmol), P(*o*-MePh)₃ (1 g, 3.29 mmol) and DIEA (5.66 g, 43.83 mmol). The mixture was stirred at 145°C for 10 min under microwave irradiation. After cooling to 25°C, the reaction was filtered through Celite^{®}. The filtrate was concentrated *in vacuo* to give the residue, which was purified by silica gel chromatography (eluent: EtOAc/PE = 1/100 ~ 20/1) to afford **tert-butyl (E)-3-(4-(trifluoromethyl)pyrimidin-2-yl)acrylate intermediate 107** (615 mg, 19%) as pale-yellow sticky oil. ESI-MS [M +H]+: 275.1 To a mixture of trimethylsulfoxonium iodide (672 mg, 3.05 mmol) in DMSO (5 mL) was added NaH (122 mg, 60% purity in mineral oil, 3.05 mmol) at room temperature. The resulting mixture was stirred for 1 h under N₂ then a solution of **intermediate 107** (465 mg, 1.7 mmol) in DMSO (2 mL) was added. The resulting mixture was stirred at room temperature for 3 h. The reaction was quenched with saturated aqueous NH₄Cl (40 mL) then extracted with EtOAc (5 x 30 mL). The combined organic layers were dried over Na₂SO₄, filtered and concentrated *in vacuo.* The residue was purified by silica gel chromatography (EtOAc/PE = 1/100 ~ 10/1) to afford ***rac*-tert-butyl (1S*,2S*)-2-(4-(trifluoromethyl)pyrimidin-2-yl)cyclopropane-1-carboxylate intermediate 108** (235 mg, 48%) as pale-yellow oil. ESI-MS [M +H]+: 289.1

A mixture of **intermediate 108** (235 mg, 0.815 mmol) and TFA (2 mL) in DCM (1 mL) was stirred at room temperature for 3 h. The reaction was concentrated *in vacuo.* The residue was diluted with water (10 mL), then basified with 1 N NaOH solution (3 mL) to pH = 9, and washed with EtOAc (2 x 30 mL). The separated aqueous phase was acidified with 1 N HCl (2 mL) to pH = 5, and then extracted with DCM (3 x 15 mL). The combined organic phase was dried over Na₂SO₄, filtered and concentrated *in vacuo* to afford ***rac*-(1S*,2S*)-2-(4-(trifluoromethyl)pyrimidin-2-yl)cyclopropane-1-carboxylic acid intermediate 109** (140 mg, crude) as white solid, which was used in next step without purification. ESI-MS [M +H]+: 233.1

To a mixture of **intermediate 109** (120 mg, 0.517 mmol), and (NH₄)₂CO₃ (199 mg, 2.067 mmol) in DMF (2 mL) was added HOBt (105 mg, 0.775 mmol), DIPEA (149 mg, 0.775 mmol) and EDCI (333 mg, 2.585 mmol). The mixture was stirred at room temperature overnight. The mixture was diluted with water (30 mL), and then extracted with EtOAc (3 x 20 mL). The combined organic phase was dried over Na₂SO₄, filtered and concentrated *in vacuo.* The residue was purified by silica gel chromatography (eluent: EtOAc/PE = 1/100 - 100/1) to afford **intermediate 106** (80 mg, 67%) as white solid. ESI-MS [M +H]+: 232.1

### Intermediate 110: rac-(1S*,2S*)-2-(4-chloropyridin-2-yl)cyclopropane-1-carboxamide

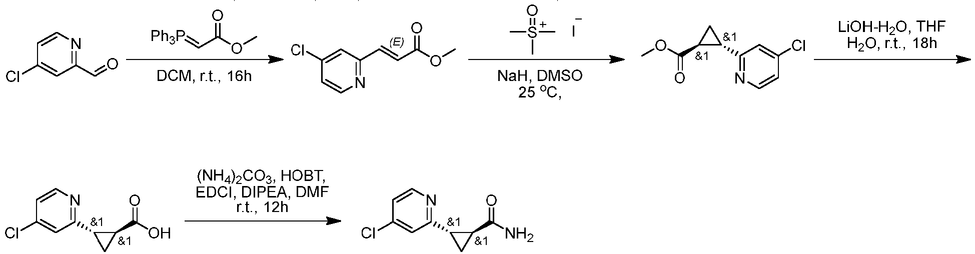

A mixture of 4-chloropicolinaldehyde (2 g, 14.2 mmol) and methyl 2-(triphenyl-15-phosphaneylidene) acetate (2.4 g, 14.2 mmol) in DCM (30 mL) was stirred at room temperature for 16h. The reaction was quenched with water (100 mL) then extracted with DCM (3 x 50 mL). The combined organic layers were washed with brine (30 mL), dried over Na₂SO₄ and concentrated *in vacuo.* The residue was purified by silica gel column chromatography (eluent: EtOAc /PE from 0 to 20%) to afford **methyl (E)-3-(4-chloropyridin-2-yl)acrylate intermediate 111** (2.5 g, 89 % yield) as a white solid. ESI-MS [M +H]+: 198.1
To a mixture of trimethylsulfoxonium iodide (8.4 g, 38.1 mmol) in DMSO (50 mL) was added NaH (1.5 g, 38.1 mmol) and the mixture was stirred at room temperature for 2h. **intermediate 111** (2.5 g, 12.7 mmol) in DMSO (10 mL) was added and the resulting mixture was stirred at room temperature for 16h. The reaction was quenched with saturated NH₄Cl solution (100 mL) then extracted with EtOAc (3 x 100 mL). The combined organic layers were washed with brine (100 mL), dried over Na₂SO₄ and concentrated *in vacuo.* The residue was purified by silica gel column chromatography (eluent: EtOAc /PE from 0 to 20%) to afford ***rac*-methyl (1S*,2S*)-2-(4-chloropyridin-2-yl)cyclopropane-1-carboxylate intermediate 112** (0.7 g, 26 % yield) as a yellow oil. ESI-MS [M +H]+: 212.2 A mixture of **intermediate 112** (340 mg, 1.6 mmol) and LiOH-H₂O (135 mg, 3.2 mmol) in THF/H2O(5 mL/5 mL) was stirred at room temperature for 18h. The pH of the reaction mixture was adjusted to pH ~3 with 1N HCl and the mixture was extracted with IPA/CHCl₃ (3/1, 5 x 30 mL) The combined organic layers were washed with brine (30 mL), dried over Na₂SO₄ and concentrated *in vacuo* to afford ***rac-*(1S*,2S*)-2-(4-chloropyridin-2-yl)cyclopropane-1-carboxylic acid intermediate 113** (300 mg, crude) as an off-white solid, which was used in the next step directly. ESI-MS [M +H]+: 198.2 A mixture of **intermediate 113** (300 mg, 1.52 mmol), (NH₄)₂CO₃ (670 mg, 7.6 mmol), HOBt (410 mg, 3.04 mmol), EDCI (578 mg, 3.04 mmol) and DIEA (588 mg, 4.56 mmol) in DMF (5 mL) was stirred at room temperature for 12h. The mixture was quenched with water (150 mL) and extracted with EtOAc (5 x 50 mL). The combined organic layers were washed with brine (50 mL), dried over Na₂SO₄ and concentrated *in vacuo.* The residue was purified by silica gel column chromatography (EtOAc / PE from 0 to 50%) to give **intermediate 110**(220 mg, 73%) as an off-white solid. ESI-MS [M +H]+: 197.2 The mixture was separated using SFC (SFC80, Daicel CHIRALPAK AD-H, 250mm × 20 mm I.D., 5µm, CO2/MEOH = 74/26, 50 g/min, 35°C) to give two enantiomers: **(1S,2S)-2-(4-chloropyridin-2-yl)cyclopropane-1-carboxamide intermediate 114** (0.1 g, first eluting isomer, Rt = 2.6 min, 99.9% e.e) and **(1R,2R)-2-(4-chloropyridin-2-yl)cyclopropane-1-carboxamide intermediate 115** (0.1 g, second eluting isomer, Rt = 5.5 min, 99.9% e.e)

### Intermediate 116: rac-(1S*,2S*)-2-(3-cyano-6-methylpyridin-2-yl)cyclopropane-1-carboxamide

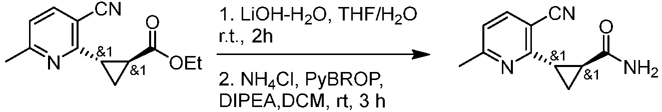

To a mixture of **intermediate 94** (690 mg, 3 mmol) in THF (10 mL) and water (10 mL) was added LiOH-H₂O (370 mg, 9 mmol). The reaction was stirred at room temperature for 2 h. The pH of the reaction was adjusted to 6 with HCl (1N), and extracted with DCM/MeOH (10/1, 3 x 30 mL). The combined organic layers were dried over Na₂SO₄, filtered and concentrated *in vacuo.* The residue was purified by silica gel chromatography (eluent: DCM/MeOH/HCOOH = 100/10/1) to give ***rac-*(1S*,2S*)-2-(3-cyano-6-methylpyridin-2-yl)cyclopropane-1-carboxylic acid intermediate 117** (330 mg, 54%) as a white solid. ESI-MS [M +H]+: 203.1.

A mixture of **intermediate 117** (202 mg, 1mmol) and NH₄Cl (106 mg, 2 mol), PyBROP (699 mg, 1.5 mmol), DIEA (516 mg, 4 mmol) in DCM (10 mL) was stirred at RT for 2 h. After diluting the mixture with DCM (20 mL), the resulting solution was washed with saturated aqueous NaHCO₃ (30 mL), water (20 mL) then brine (30 mL), dried over Na₂SO₄ and concentrated *in vacuo.* The residue was purified by preparative TLC (eluent: DCM/MeOH = 20/1) to afford **intermediate 116** (160 mg, 80%) as a white solid. ESI-MS [M +H]+: 202.1.

### Intermediate 118 rac-(1S*,2S*)-2-(5-chloro-2-cyanophenyl)cyclopropane-1-carboxamide

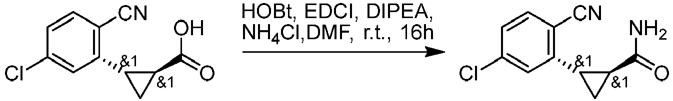

A mixture of **intermediate 40** (245 mg, 1.1 mmol), NH₄Cl (350 mg, 6.6 mol), HOBt (297 mg, 12.2 mmol), EDCI (420 mg, 2.2 mmol) and DIPEA (851 mg, 6.6 mmol) in DMF (10 mL) was stirred at room temperature under N₂ for 16 h. The reaction was poured onto water (20 ml) and extracted with EtOAc (3 x 20 ml), The combined organic layers were washed with brine, dried over Na₂SO₄ and concentrated to give the crude, which was purified with preparative TLC (eluent: DCM/MeOH = 20/1) to give the **intermediate 118** (215 mg, 88%) as white solid. ESI-MS [M +H]+: 221.1.

### Intermediate 119: (1S,2S)-2-(3-chlorophenyl)cyclopropane-1-carboxamide

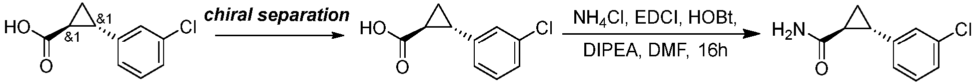

**intermediate 1149** (10 g) was separated using SFC (SFC80, Daicel CHIRALPAK AD-H 250mm × 20 mm I.D., 5µm, CO₂/EtOH = 86/14, 50 g/min, 35°C) to give **(1S,2S)-2-(3-chlorophenyl)cyclopropane-1-carboxylic acid intermediate 120** (4.5 g, first eluting isomer, Rt = 3.0 min, 99.9% e.e.) and **(1R,2R)-2-(3-chlorophenyl)cyclopropane-1-carboxylic acid intermediate 121** (4.3 g, second eluting isomer, Rt = 4.0 min, 99.9% e.e.)

To a solution of **intermediate 120** (250 mg, 1.28 mmol) in DMF (4 mL) was added (NH₄)₂CO₃(247 mg, 2.56 mmol), HOBt (260 mg, 1.92 mmol), EDCI (372 mg, 1.92 mmol) and DIPEA (498 mg, 3.84 mmol). After stirring at room temperature for 12 h, water (20 mL) was added then the reaction was extracted with EtOAc (5 x 50 mL). The combined organic layers were washed with brine (30 mL), dried over anhydrous Na₂SO₄, and concentrated *in vacuo* to give the crude, which was purified by silica gel column chromatography (eluent: DCM/MeOH = 15/1) to give **intermediate 119** (210 mg, 84%) as a white solid. ESI-MS [M +H]+: 196.1

### Intermediate 122 rac-methyl (1S*,3S*)-3-(3-chlorophenyl)-2,2-difluorocyclopropane-1-carboxylate

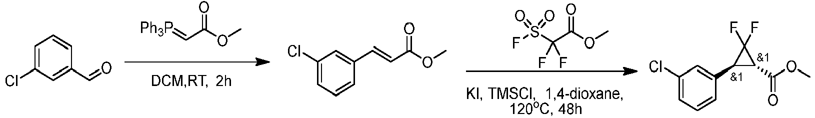

A mixture of 3-chlorobenzaldehyde (10 g, 71.4 mmol) and methyl 2-(triphenyl-15-phosphaneylidene) acetate (23.9 g, 71.4 mmol) in DCM (200 mL) was stirred at room temperature for 2h. The reaction was quenched with water (300 mL) then extracted with DCM (3 x 200 mL). The combined organic layers were washed with brine (200 mL), dried over Na₂SO₄ and concentrated *in vacuo.* The residue was purified by silica gel column chromatography (eluent: EtOAc /PE from 0 to 10%) to afford **methyl (E)-3-(3-chlorophenyl)acrylate intermediate 123** (13.5 g, 96 % yield) as a white solid.

A mixture of **intermediate 123** (1.0 g, 5.1 mmol), dry KI (2.5 g, 15.3 mmol), TMSCl (1.7 g, 15.3 mmol) and methyl 2,2-difluoro-2-(fluorosulfonyl)acetate (2.9 g, 15.3 mmol) in 1,4-dioxane (10 mL) was stirred at 120°C for 48h in a sealed tube. After cooled to room temperature, the reaction was quenched with water (40 mL) and extracted with EtOAc (3 x 50 mL). The combined organic layers were washed with brine (50 mL), dried over Na₂SO₄ and concentrated *in vacuo.* The residue was purified by silica gel column chromatography (eluent: EtOAc /PE from 0 to 10%) to afford **intermediate 122** (200 mg, 16 % yield) as a yellow oil.

### Intermediate 124: rac-(1S*,2S*)-2-(4-chloropyrimidin-2-yl)cyclopropane-1-carboxamide

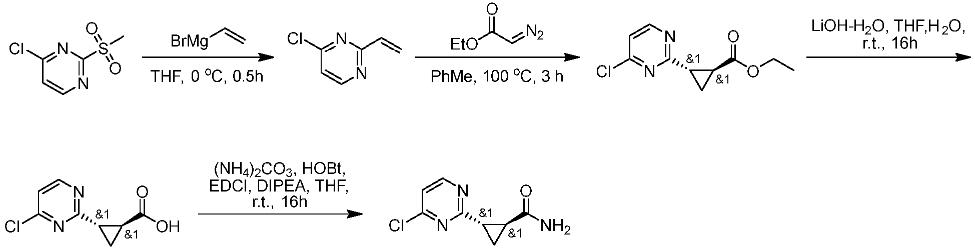

To a mixture of 4-chloro-2-(methylsulfonyl)pyrimidine (200 mg, 1.04 mmol) in THF (10 mL) was added vinylmagnesium bromide (2.0M solution in THF, 0.52 mL, 1.04 mmol) under N₂ at 0°C. After stirring at 0°C for 0.5 h, the reaction was quenched with water (20 mL) and extracted with EtOAc (3 x 15 mL). The combined organic layers were washed with brine (20 mL), dried over anhydrous Na₂SO₄, and concentrated to give **4-chloro-2-vinylpyrimidine intermediate 125** (150 mg, crude) as a yellow oil, which was used in the next step without further purification. ESI-MS [M +H]+: 141.1. To a solution of **intermediate 125** (150 mg, 1.07 mmol) in toluene (5 mL) was added ethyl 2-diazoacetate (360 mg, 3.21 mmol). The resulting reaction mixture was stirred at 100°C for 3 h under N₂. After cooling to room temperature, the reaction concentrated *in vacuo* to give the crude, which was purified by silica gel chromatography (eluent: PE/EtOAc = 3/1) to give ***rac*-ethyl (1S*,2S*)-2-(4-chloropyrimidin-2-yl)cyclopropane-1-carboxylate intermediate 126** (50 mg, 21%) as a yellow solid. ESI-MS [M +H]+: 227.2.

Using a similar procedure to that for **intermediate 23, intermediate 124** (15 mg, 76%) was synthesised from **intermediate 126** (25 mg, 0.11 mmol). ESI-MS [M +H]⁺: 198.2

### Intermediate 127: rac-(1S*,2S*)-2-(6-chloropyrimidin-4-yl)cyclopropane-1-carboxamide

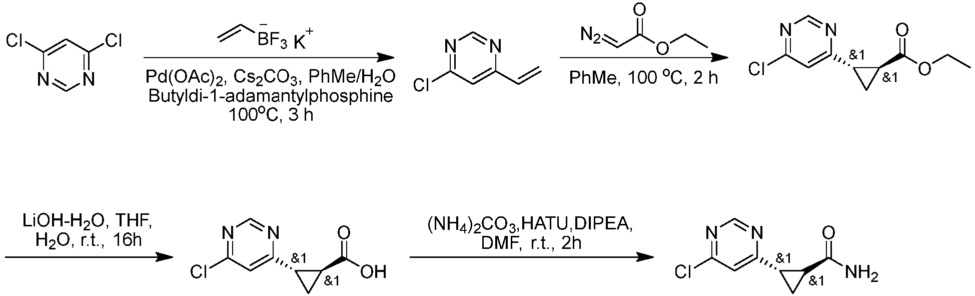

A mixture of 4,6-dichloropyrimidine (6 g, 40.2 mmol), potassium ethenyltrifluoroborate (5.4 g, 44.2 mol), Pd(OAc)₂ (900 mg, 4 mmol), butyldi-1-adamantylphosphine (2 g, 6 mmol) and Cs₂CO₃ (26 g, 80.4 mmol) in toluene (120 mL) and water (12 mL) was stirred at 100°C for 3 h. The reaction mixture was diluted with water (300 mL) then extracted with EtOAc (2 x 100 mL). The combined organics were washed with brine (100 mL), dried over Na₂SO₄, concentrated *in vacuo* to give the crude product, which was purified by silica gel column chromatography (eluant: PE : EtOAc = 10 : 1) to give 4-**chloro-6-vinylpyrimidine intermediate 128** (1.7 g, 30.0% yield) as a colorless oil.

To a solution of **intermediate 128** (1.7 g, 12.1 mmol) in toluene (40 mL) was added dropwise a solution of ethyl 2-diazoacetate (4.14 g, 36.3 mmol) in toluene (20 mL). The resulting solution was stirred at 100°C under N₂ for 2 h, cooled to room temperature and concentrated *in vacuo* to give the crude product. The crude product was purified by silica gel column chromatography (eluant: PE/EtOAc = 10/1) to give **ethyl *rac*-ethyl (1S*,2S*)-2-(6-chloropyrimidin-4-yl)cyclopropane-1-carboxylate intermediate 129** (810 mg, 29.5 % yield) as colorless oil. ESI-MS [M +H]+: 227.1

A mixture of **intermediate 129** (400 mg, 1.66 mmol) and LiOH (120 mg, 5 mmol) in THF (10 mL) and water (2 mL) was stirred at 25°C for 16 h. After removing THF, the mixture was acidified to pH ~ 3 with HCl (1M) then extracted with EtOAc (2 x 20 mL). The combined organics were washed with brine (50 mL), dried over Na₂SO₄ then concentrated *in vacuo* to give the crude **rac-(1S*,2S*)-2-(6-chloropyrimidin-4-yl)cyclopropane-1-carboxylic acid intermediate 130** (400 mg, crude). ESI-MS [M +H]+: 199.1

A mixture of **intermediate 130** (400 mg, 2 mmol), HATU (384 mg, 4 mmol) and DIPEA (755 mg, 6 mmol) in DMF (20 mL) was stirred at 25°C for 10 mins. (NH₄)₂CO₃ (384 mg, 4 mmol) was added then the mixture was stirred at 25°C for 2 h. The mixture was diluted with water (200 mL) then extracted with EtOAc (3 x 50 mL). The combined organics were washed with brine (100 mL), dried over Na₂SO₄ then concentrated *in vacuo* to give the crude product. The crude product was trituated with PE:DCM = 1:1 to give **intermediate 127** as an off-white solid. (300 mg, 75% yield). ESI-MS [M +H]+: 198.1

### Intermediate 131: rac-methyl (1S*,2S*)-2-(3-chlorophenyl)-3-fluorocyclopropane-1-carboxylate

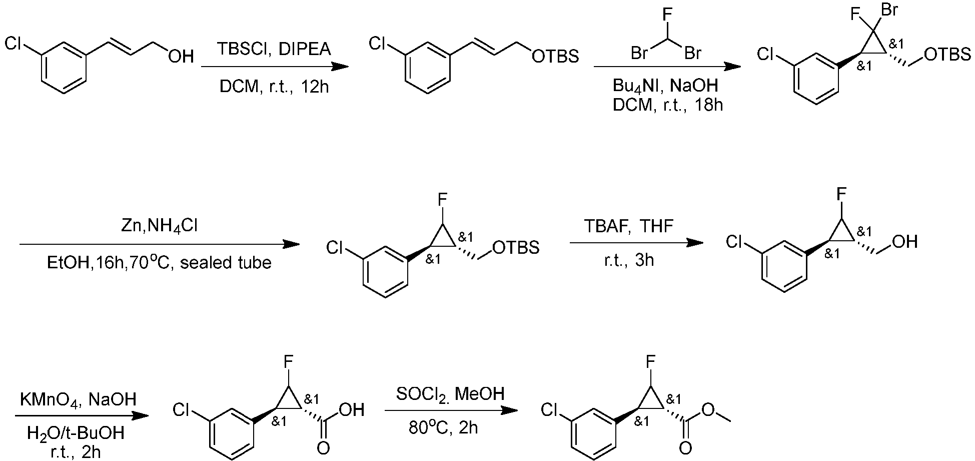

To a solution of (E)-3-(3-chlorophenyl)prop-2-en-1-ol (300 mg, 1.78 mmol) and DIPEA (807 mg, 6.23 mmol) in DCM (10 mL) was added TBSCl (538 mg, 3.56 mmol) . The resulting mixture was stirred at room temperature for 12 h. Water (30 mL) was added to the reaction, extracted with DCM (3 x 30 mL). The combined organic layers were washed with brine (30 mL), dried over anhydrous Na₂SO₄, and concentrated *in vacuo* to give the crude, which was purified by silica gel column chromatography (eluent: PE/EtOAc = 1/1) to give **(E)-tert-butyl((3-(3-chlorophenyl)allyl)oxy)dimethylsilane intermediate 132** (300 mg, 59%) as a yellow oil. ESI-MS [M +H]+: 283.1.

A mixture of **intermediate 132** (300 mg, 1.06 mmol), dibromofluoromethane (305 mg, 1.59 mmol) and Bu₄NI (39 mg, 0.106 mmol) in DCM (1 mL)/ 50% NaOH aqueous(1 mL). The mixture was degassed with N₂ for 1 min, then stirred at 25°C in a sealed tube for 18 h. The reaction mixture was quenched with water (10 mL) and extracted with EtOAc (3 x 30 mL). The combined organic layers were washed with brine (20 mL), dried over anhydrous Na₂SO₄, and concentrated *in vacuo* to give the crude product, which was purified by silica gel column chromatography (eluent: PE/EtOAc = 100/0) to give *rac-***(((1S*,3S*)-2-bromo-3-(3-chlorophenyl)-2-fluorocyclopropyl)methoxy)(tert-butyl)dimethylsilane intermediate 133** (160 mg, 38.3%) as a yellow oil. ESI-MS [M +H]+: 393.1.

A mixture of **intermediate 133** (160 mg, 0.4 mmol), Zn(240 mg, 3.6 mmol) and NH₄Cl(196 mg, 3.6 mmol) in EtOH (5 mL) was degassed with N₂ for 1 min then stirred in a sealed tube at 70°C for 16 h. After cooling to room temperature, the reaction mixture was filtered through Celite^{®} and the filter cake was washed with DCM/MeOH (10/1, 40 mL). The filtrate was concentrated to give the crude, which was purified by silica gel column chromatography (eluent: DCM/MeOH=50/1 ~ 20/1) to give ***rac*-tert-butyl(((1S*,2S*)-2-(3-chlorophenyl)-3-fluorocyclopropyl)methoxy)dimethylsilane intermediate 134** (120 mg, 93.7%) as a yellow oil. ESI-MS [M +H]+: 315.1.

To a mixture of **intermediate 134** (120 mg, 0.38 mmol) in THF (10 mL) was added TBAF (250 mg, 0.95 mmol). The reaction mixture was stirred at 25°C for 3 h under N₂. Water (10 mL) was added, and the reaction mixture was extracted with EtOAc (3 x 30 mL). The combined organic layers were washed with brine (30 mL), dried over anhydrous Na₂SO₄, and concentrated *in vacuo* to give the crude product, which was purified by silica gel column chromatography (eluent: PE/EtOAc = 1/1) to give ***rac-*((1S*,2S*)-2-(3-chlorophenyl)-3-fluorocyclopropyl)methanol intermediate 135** (90 mg, 90%) as yellow oil. ESI-MS [M +H]+: 201.1.

To a mixture of **intermediate 135** (90 mg, 0.45 mmol) in t-BuOH (2 mL) was added a mixture of NaOH (144 mg, 3.6 mmol) and KMnO₄ (213 mg, 1.35 mmol) in water (1.5 ml). After stirring at 25°C for 2 h under N₂, Water (20 mL) was added, the mixture was extracted with DCM (3 x 30 mL). The combined organic layers were washed with brine (10 mL), dried over anhydrous Na₂SO₄, and concentrated *in vacuo* to give the crude product, which was purified by silica gel column chromatography (eluent: DCM/MeOH = 15/1) to give ***rac*-(1S*,2S*)-2-(3-chlorophenyl)-3-fluorocyclo propane-1-carboxylic acid intermediate 136** (70 mg, 72.6%) as a yellow oil. ESI-MS [M +H]+: 215.1.

To a mixture of **intermediate 136** (70 mg, 0.32 mmol) in MeOH (4 mL) was added SOCl₂ (0.4 ml). The reaction mixture was stirred at 80°C for 2 h under N₂, cooled to room temperature then concentrated *in vacuo* to give the crude product, which was purified by preparative TLC (eluent: DCM/MeOH = 20/1) to give **intermediate 131** (40 mg, 54%) as a yellow solid. ESI-MS [M +H]+: 229.1.

### Intermediate 137: rac-(1S*,2S*)-2-(5-chloropyridazin-3-yl)cyclopropane-1-carboxamide

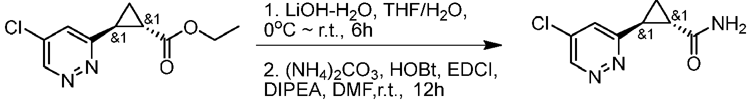

Using a similar procedure to that for **intermediate 23, intermediate 137** (5 mg, 12%) was synthesised from **intermediate 95** (210 mg, 0.93 mmol). ESI-MS [M +H]+: 198.2.

### Intermediate 138: rac-(1S*,2S*)-2-(5-chloro-3-cyanothiophen-2-yl)cyclopropane-1-carboxylic acid

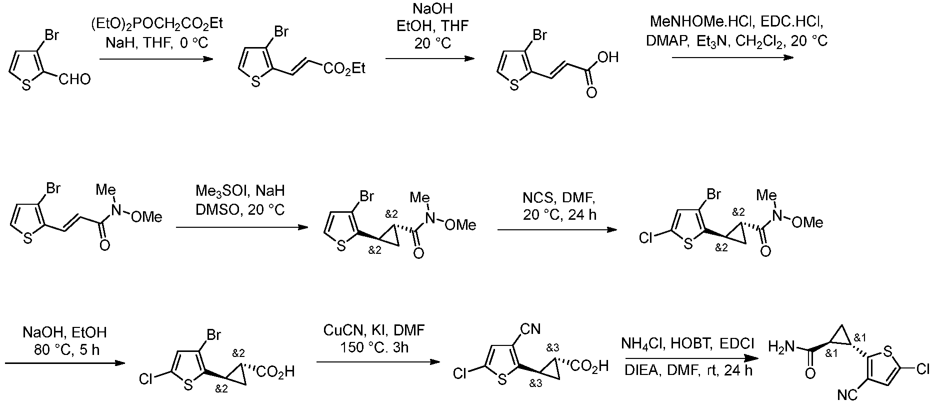

Sodium hydride (60% oil dispersion, 2.76 g, 69.0 mmol) was suspended in anhydrous THF (500 mL) under nitrogen at 0°C. Triethyl phosphonoacetate (14.7 g, 65.5 mmol) was added dropwise over 5 min and the mixture was stirred for 30 min. 3-Bromo-2-thiophenecarboxaldehyde (10.0 g, 52.4 mmol) was added dropwise over 5 min. The mixture was allowed to warm to room temperature and stirred for a further 18 h. The reaction mixture was poured into saturated aqueous ammonium chloride (100 mL) and extracted with methyl *tert*-butyl ether (3 × 100 mL). The combined organic extracts were washed with brine (2 x 100 mL), dried (MgSO₄), filtered and concentrated *in vacuo* to give a pale yellow solid. The solid was purified by column chromatography (200 g silica gel, eluting with 0-25% ethyl acetate in heptane) to give **ethyl (E)-3-(3-bromothiophen-2-yl)acrylate intermediate 139** (10.9 g, 79%), as a colourless solid. ESI-MS [M + H]⁺ 263, ¹H NMR (400 MHz, CDCl₃) δ 7.83 (dd, *J* 15.7, 1.2 Hz, 1H), 7.33 (d, *J* 5.4 Hz, 1H), 7.03 (d, *J* 5.4 Hz, 1H), 6.30 (d, *J* 15.7 Hz, 1H), 4.27 (q, *J* 7.3 Hz, 2H), 1.33 (t, *J* 7.3 Hz, 3H).

Aqueous sodium hydroxide (2 M, 85.7 mL, 171 mmol) was added to a solution of **intermediate 139** (10.9 g, 41.6 mmol) in a mixture of THF (120 mL) and ethanol (60 mL), and the mixture was stirred for 18 h at room temperature. The mixture was acidified to pH 2 using hydrochloric acid (2M, 100 mL) and extracted with ethyl acetate (3 x 200 mL). The combined extracts were washed with water (2 x 100 mL), brine (2 x 100 mL), dried (MgSO₄), filtered and concentrated *in vacuo* to give **(E)-3-(3-bromothiophen-2-yl)acrylic acid intermediate 140** (9.26 g, 96% ), as a colourless solid. ESI-MS [M - H]⁻ 231/233, ¹H NMR (400 MHz, CD₃SOCD₃) δ 12.63 (s, 1H), 7.85 (d, *J* 5.4 Hz, 1H), 7.63 (d, *J* 16.3 Hz, 1H), 7.25 (d, *J* 5.4 Hz, 1H), 6.30 (d, *J* 16.3 Hz, 1H).

A solution of **intermediate 140** (9.26 g, 39.7 mmol) and N-ethyl-N'N'-diemthylaminopropyl-carbodiimide hydrochloride (10.66 g, 55.63 mmol) in DCM (150 mL) under nitrogen was cooled to 0°C. N,N-Dimethylaminopyridine (5.29 g, 43.3 mmol), N,O-dimethylhydroxylamine hydrochloride (4.88 g, 50.1 mmol) and triethylamine (11.6 mL, 83.4 mmol) were added and the reaction mixture was stirred for 18 h at room temperature. Further N-ethyl-N'N'-diemthylaminopropylcarbodiimide hydrochloride (2.13 g, 11.1 mmol), N,O-dimethylhydroxylamine hydrochloride (0.976 g, 10.0 mmol) and triethylamine (2.3 mL, 17 mmol) were added. The reaction mixture was stirred for another 18 h at room temperature. The reaction mixture was diluted with ethyl acetate (500 mL) and the solution was washed with water (2 x 100 mL), brine (2 x 100 mL), dried (MgSO₄), filtered and concentrated *in vacuo* to give a yellow oil. Purification by flash chromatography (Silica gel, eluting with 0-80% ethyl acetate in heptane) gave **(E)-3-(3-bromothiophen-2-yl)-N-methoxy-N-methylacrylamide intermediate 141** (8.39 g, 76%), as a colourless solid. ESI-MS [M + H]⁺ 276/278¹H NMR (400 MHz, CDCl₃) δ 7.88 (d, *J* 15.7 Hz, 1H), 7.30 (d, *J* 5.4 Hz, 1H), 7.02 (d, *J* 5.4 Hz, 1H), 6.90 (d, *J* 15.7 Hz, 1H), 3.76 (s, 3H), 3.30 (s, 3H).

Sodium hydride (60% oil dispersion, 2.57 g, 107 mmol) was washed with heptane (2 × 50 mL) and suspended in anhydrous DMSO (30 mL) under nitrogen at room temperature. A solution of trimethylsulfoxonium iodide (21.8 g, 99.0 mmol) in anhydrous DMSO (90 mL) was added dropwise over 10 min to give a cloudy suspension. The mixture was stirred at room temperature for 1 h, during which time it became a pale- yellow solution. Then a solution **intermediate 141** (8.39 g, 30.4 mmol) in anhydrous DMSO (30 mL) was added dropwise over 5 min. The mixture was stirred at room temperature overnight, diluted with ethyl acetate (250 mL) and the solution was washed with water (3 × 70 mL), brine (3 × 80 mL), dried (MgSO₄), filtered and concentrated *in vacuo* to give a yellow oil. Purification by column chromatography (silica gel, eluting with 0-80% ethyl acetate in heptane) gave **(*rac*)-(1S*,2S*)-2-(3-bromothiophen-2-yl)-N-methoxy-N-methylcyclopropane-1-carboxamide intermediate 142** (7.46 g, 85%), as a colourless oil. ESI-MS [M + H]⁺ 290/292, ¹H NMR (400 MHz, CDCl₃) δ 7.06 (d, *J* 5.2 Hz, 1H), 6.91 (d, *J* 5.2 Hz, 1H), 3.75 (s, 3H), 3.25 (s, 3H), 2.65 (ddd, *J* 9.6, 6.0, 4.0 Hz, 1H), 2.45 (br s, 1H), 1.70 (ddd, *J* 9.6, 5.4, 4.0 Hz, 1H), 1.30 (ddd, *J* 8.5, 6.0, 4.0 Hz, 1H). N-Chlorosuccinimide (2.02 g, 15.2 mmol) was added to a solution of **intermediate 142** (4.0 g, 13.78 mmol) in DMF (103 mL) at room temperature under nitrogen. The mixture was stirred for 24 h, diluted with ethyl acetate and washed with aqueous sodium thiosulfate (10%, 3 × 40 mL). The organic solution was washed with water (4 × 50 mL), brine (4 × 50 mL), dried (MgSO₄), filtered and concentrated *in vacuo* to give a yellow oil. Analysis by LCMS showed the reaction was incomplete. Consequently, the oil was dissolved in DMF (20 mL) at room temperature and more N-chlorosuccinimide (707 mg, 5.28 mmol) was added. The reaction mixture was stirred for 6 h at room temperature. The reaction mixture was diluted with ethyl acetate (200 mL) and washed with aqueous sodium thiosulfate (10%, 3 × 60 mL), water (4 × 80 mL), brine (4 × 80 mL), dried (MgSO₄), filtered and concentrated *in vacuo* to give a yellow oil. Purification by column chromatography (silica gel, eluting with 0-80% ethyl acetate in heptane) gave **(*rac*)-(1S*,2S*)-2-(3-bromo-5-chlorothiophen-2-yl)-N-methoxy-N-methylcyclopropane-1-carboxamide intermediate 143** (3.80 g, 85 %), as a colourless solid. ESI-MS [M + H]⁺ 326/328, ¹H NMR (400 MHz, CDCl₃) δ 6.75 (s, 1H), 3.77 (s, 3H), 3.25 (s, 3H), 2.60-2.55 (m, 1H), 2.42 (m, 1H), 1.70-1.65 (m, 1H), 1.26-1.22 (m, 1H).

A solution of sodium hydroxide (2 M in water, 11.72 mL, 23.45 mmol) was added to a solution of **intermediate 143** (3.8 g, 11.72 mmol) in EtOH (46.0 mL) at room temperature under nitrogen. The mixture was stirred at 80°C for 5 h. The mixture was acidified to pH 2 using hydrochloric acid (2M, 15 mL) and extracted with ethyl acetate (2 × 100 mL). The extracts were washed with water (2 × 60 mL), brine (2 × 60 mL), dried (MgSO₄), filtered and concentrated *in vacuo.* The residue was recrystallised from ethyl acetate in heptane to give **(*rac*)-(1S*,2S*)-2-(3-bromo-5-chlorothiophen-2-yl)-cyclopropane-1-carboxylic acid intermediate 144** (3.18 g, 96%), as a colourless solid. ESI-MS [M - H]⁻ 281/283, ¹H NMR (400 MHz, CD₃SOCD₃) δ 12.52 (s, 1H), 7.15 (s, 1H), 2.38 (ddd, *J* 9.7, 5.4, 3.6 Hz, 1H), 1.80-1.76 (m, 1H), 1.48-1.43 (m, 1H), 1.29-1.24 (m, 1H).

A mixture of **intermediate 144** (1.00 g, 3.55 mmol), copper cyanide (956 mg, 10.6 mmol) and potassium iodide (59 mg, 0.35 mmol) in anhydrous DMF (4 mL) was heated at 150°C under nitrogen for 3 h. After being cooled to room temperature, the mixture was diluted with ethyl acetate (100 mL) and filtered through short pad of Dicalite (2 g). The filtrate was acidified with hydrochloric acid (2M, 6 mL) to give pH 2. The aqueous layer was separated and extracted with ethyl acetate (2 x 100 mL). the combined organic solutions were washed with brine (2 x 50 mL), dried (MgSO₄), filtered and concentrated under reduced pressure. The residue was purified by reverse phase chromatography, eluting with a gradient of acetonitrile and 0.1% aqueous formic acid = 10:90 to 90:10 over 10 column volumes. Fractions containing product were pooled, concentrated *in vacuo* and freeze-dried to give **(*rac*)-(1*S**,2*S**)-2-(5-chloro-3-cyanothiophen-2-yl)-cyclopropane-1-carboxylic acid intermediate 145** (545 mg, 69%), as a colourless solid. ESI-MS [M - H]⁻ 226/228, ¹H NMR (400 MHz, CD₃SOCD₃) δ 12.67 (s, 1H), 7.47 (s, 1H), 2.65 (ddd, *J* 10, 6, 4.4 Hz, 1H), 2.00 (ddd, *J* 9.2*,* 4.8, 4.4 Hz, 1H), 1.56 (dt, *J* 10, 4.8 Hz, 1H), 1.41 (ddd, *J* 9.2, 6, 4.8 Hz, 1H).

A mixture of **intermediate 145** (270 mg, 1.19 mmol), NH₄Cl (378 mg, 7.14 mol), HOBt (321 mg, 2.38 mmol), EDCI (455 mg, 2.38 mmol) and DIPEA (921 mg, 7.14 mmol) in DMF (12 mL) was stirred at room temperature under N₂ for 24 h. The reaction was poured into water (20 ml) then extracted with EtOAc (3 x 30 ml). The combined organic layers were washed with brine (30 ml), dried with Na₂SO₄ then concentrated *in vacuo* to give the crude, which was purified by silica gel chromatography (DCM/MeOH = 30:1) to give **intermediate 138** (210 mg, 78% ) as a yellow solid. ESI-MS [M +H]+: 227.1.

### Intermediate 146: methyl rac-(1R*,2S*)-5'-chloro-1'-methyl-2'-oxospiro[cyclopropane-1,3'-indoline]-2-carboxylate

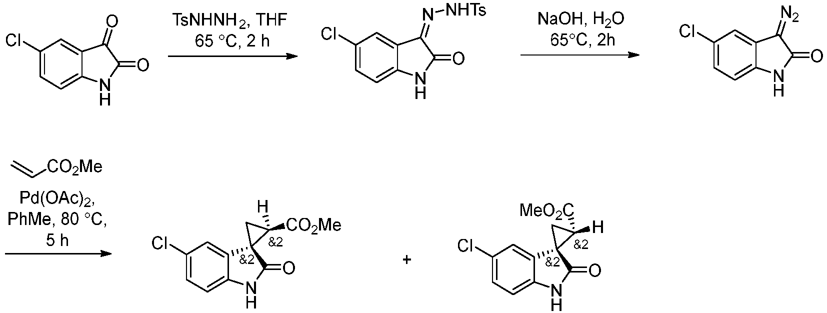

5-Chloroisatin (20.0 g, 110 mmol) was suspended in THF (400 mL). Tosylhydrazine (21.5 g, 115 mmol) was added, and the suspension was heated at reflux for 2 h. The reaction mixture was allowed to cool to room temperature, and the product was collected by filtration, washed with cold methanol and dried *in vacuo.* **(Z)-N'-(5-Chloro-2-oxoindolin-3-ylidene)-4-methylbenzenesulfonohydrazide intermediate 147** (34.6 g, 90%) was obtained as a yellow solid. ESI-MS [M + H]⁺ 350. δ 12.46 (s, 1H), 11.30 (s, 1H), 7.88 (d, *J* 7.9 Hz, 2H), 7.45 (d, *J* 7.9 Hz, 2H and d, *J* 2.4 Hz, 1H), 7.39 (dd, *J* 8.8, 2.4 Hz, 1H), 6.91 (d, *J* 8.8 Hz, 1H), 2.39 (s, 3H).

**Intermediate 147** (12.0 g, 38.1 mmol) was treated with a solution of sodium hydroxide (3.04 g, 76.1 mmol) in water (375 mL). The reaction mixture was stirred at 65°C for 2 h, and then allowed to cool to room temperature. The reaction mixture was neutralized (from pH 11 to pH7) by the gradual addition of solid carbon dioxide with cooling in an ice-water bath. The mixture was filtered and the solid dried *in vacuo* to give **5-chloro-3-diazoindolin-2-one intermediate 148** (7.01 g, 99%), as an orange solid. ¹H NMR (400 MHz, DMSO): δ 10.78 (s, 1H), 7.58 (d, *J* 1.8 Hz, 1H), 7.12 (dd, *J* 8.5, 1.8 Hz, 1H), 6.89 (d, *J* 8.5 Hz, 1H).

A mixture of **intermediate 148** (3.40 g, 17.6 mmol), methyl acrylate (3.40 mL, 37.5 mmol) and palladium acetate (478 mg, 2.13 mmol) in anhydrous toluene (100 mL) was heated under nitrogen at 80°C for 5 h. The reaction mixture was concentrated under reduced pressure. The residue was dissolved in ethyl acetate (40 mL), adsorbed onto silica gel (35 g), and purified by flash chromatography (Biotage Isolera, 330 g silica cartridge, eluting with 25% ethyl acetate in heptane for 2 column volumes, then 25-75% ethyl acetate in heptane over 12 column volumes). Fractions were collected together to give a mixture of the impure desired product isomers. This material was combined with another batch, prepared on the same scale in the same manner already described, to give a red solid (2.128 g). Two other impure batches prepared previously (0.150 g and 0.317 g respectively) were also added. The combined crude material (2.595 g) was adsorbed onto silica gel (10 g) with acetone (40 mL) and re-purified by flash chromatography (eluting with 10% methyl *tert*-butyl ether in toluene for 1 column volume, followed by 10-33% methyl *tert*-butyl ether in toluene over 8 column volumes and 33-50% methyl *tert*-butyl ether in toluene over 3 column volumes). First eluted was the (1R*,2R*)-isomer (structure assigned by NMR) Methyl *rac*-(1R*, 2S*)-5'-chloro-2'-oxospiro[cyclopropane-1,3'-indoline]-2-carboxylate **intermediate 149** ESI-MS [M + H]⁺ : 252. ¹H NMR (400 MHz, CDCl₃) δ 8.44 (s, 1H), 7.37 (d, *J* 2.4 Hz, 1H), 7.21 (dd, *J* 8.5, 2.4 Hz, 1H), 6.87 (d, *J* 8.5 Hz, 1H), 3.72 (s, 3H), 2.73 (dd, *J* 8.5, 7.5 Hz, 1H), 2.15 (dd, *J* 7.5, 4.5 Hz, 1H), 2.06 (dd, *J* 8.5, 4.5 Hz, 1H).

Second eluted was methyl (1R*,2S*)-isomer (structure assigned by NMR) **intermediate 146** (1.230 g, 12%), as an orange solid. ESI-MS [M + H]⁺ : 252, ¹H NMR (400 MHz, CDCl₃) δ 8.35 (s, 1H), 7.20 (dd, *J* 8.5, 1.8 Hz, 1H), 6.87 (d, *J* 8.5 Hz, 1H), 6.81 (d, *J* 1.8 Hz, 1H), 3.74 (s, 3H), 2.67 (t, *J* 8.5 Hz, 1H), 2.41 (dd, *J* 8.5, 5 Hz, 1H), 1.84 (dd, *J* 8.5, 5 Hz, 1H).

### Intermediate 150: rac-(1R*,2S*)-5'-chloro-1'-methyl-2'-oxospiro[cyclopropane-1,3'-indoline]-2-carboxamide

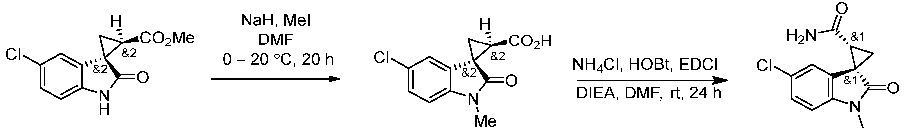

A solution of methyl *rac*-(1R*,2S*)-5'-chloro-2'-oxospiro[cyclopropane-1,3'-indoline]-2-carboxylate (630 mg, 2.50 mmol) in anhydrous THF (100 mL) was added to a stirred suspension of sodium hydride (120 mg, 60% oil dispersion, 5.00 mmol) in anhydrous THF (25 mL) under nitrogen at 0 °C. The mixture was stirred at room temperature for 1.5 h then re-cooled to 0 °C. Iodomethane (0.32 mL, 5.00 mmol) was added and the mixture was stirred at 0 °C for 2 h and then at room temperature for 18 h. The mixture was treated with aqueous citric acid (10% w/v, 18 mL) and the aqueous layer was extracted with ethyl acetate (3 × 50 mL) and dichloromethane (30 mL). The combined organic solutions were washed with brine (2 × 50 mL), dried (MgSO₄) and concentrated under reduced pressure. The residue was purified by reversed-phase column chromatography, eluting with a gradient of 5-70% acetonitrile in water containing 0.1% formic acid over 14 column volumes. Fractions containing the product were pooled and concentrated *in vacuo* to give **(1R*,2S*)-5'-chloro-1'-methyl-2'-oxospiro[cyclopropane-1,3'-indoline]-2-carboxylic acid intermediate 151** (304 mg, 48%), as a colourless solid. ESI-MS [M + H]⁺ 252, ¹H NMR (400 MHz, CDCl₃) δ 7.34 (dd, *J* 8*,* 2 Hz, 1H), 6.95 (d, *J* 8 Hz, 1H), 6.86 (d, *J* 2 Hz, 1H), 3.38 (s, 3H), 2.89 (dd, *J* 9*.*7*,* 8.5 Hz, 1H), 2.32 (dd, *J* 8.5, 5.3 Hz, 1H), 2.16 (dd, *J* 9.7*,* 5.3 Hz, 1H).

A mixture of **intermediate 151** (300 mg, 1.19 mmol), NH₄Cl (378 mg, 7.14 mol), HOBt (321 mg, 2.38 mmol), EDCI (455 mg, 2.38 mmol) and DIPEA (921 mg, 7.14 mmol) in DMF (12 mL) was stirred at room temperature under N₂ for 24 h. The reaction was poured into water (20 ml) and extracted with EtOAc (30 ml x 4). The combined organic layers were washed with brine (30 ml), dried over Na₂SO₄ then concentrated *in vacuo* to give the crude, which was purified by silica gel chromatography (eluant: DCM/MeOH = 30:1) to give **intermediate 150** (250 mg, 84% ) as a yellow solid. ESI-MS [M +H]+: 251.1.

### Intermediate 158: rac-(1R*,2S*,3R*)-2-(3-chlorophenyl)-3-(hydroxymethyl)cyclopropane-1-carboxamide

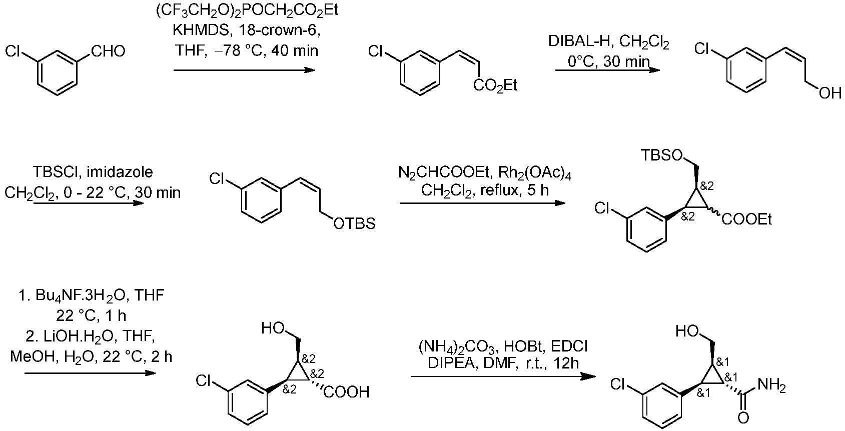

To a -78°C solution of ethyl *bis*(2,2,2-trifluoroethyl)phosphonoacetate (2.51 g, 7.56 mmol) and 18-crown-6 (9.51 g, 36.0 mmol) in anhydrous THF (100 mL) was added potassium *bis*(trimethylsilyl)amide (11% w/v in toluene, 15.6 mL, 7.56 mmol) dropwise over 5 min. After being stirred for 10 min, a solution of 3-chlorobenzaldehyde (816 µL, 7.20 mmol) in anhydrous THF (5 mL) was added over *ca.* 5 min. After being stirred for 40 min the reaction was quenched by the addition of saturated aqueous ammonium chloride (50 mL) and the resulting suspension was allowed to warm to room temperature. Most of the solvent was removed under reduced pressure and the biphasic mixture was diluted with ethyl acetate (100 mL). The layers were separated, and the organic layer was washed with water 3 times, and brine. The solution was dried (MgSO₄), filtered and concentrated under reduced pressure to give a light-yellow oil. Purification on silica gel (40 g), eluting with a gradient of heptane:ethyl acetate = 100:0 to 97:3 afforded **ethyl (Z)-3-(3-chlorophenyl)acrylate intermediate 152** (1.43 g, 86%), as a colourless liquid. ¹H NMR (400 MHz, CDCl₃, ppm) δ 7.58-7.56 (m, 1H), 7.44-7.40 (m, 1H), 7.32-7.27 (m, 2H), 6.88 (d, *J* 13 Hz, 1H), 5.99 (d, *J* 13 Hz, 1H), 4.18 (q, *J* 7 Hz, 2H), 1.25 (t, *J* 7 Hz, 3H).

A solution of **intermediate 152** (1.43 g, 6.80 mmol) in anhydrous DCM (47 mL) under nitrogen was cooled in an ice bath and a solution of diisobutylaluminium hydride (25% in toluene, 10.0 mL, 15.0 mmol) was added dropwise over *ca.* 10 min. After addition was complete, the mixture was stirred for another 20 min before being poured into 5% aqueous sodium potassium tartrate (200 mL). The mixture was vigorously stirred for 1 h and filtered through a plug of Dicalite, washing the solid with DCM. The filtrate layers were separated and the aqueous layer extracted once with DCM. The combined organic layers were dried (MgSO₄), filtered and evaporated to obtain **(Z)-3-(3-chlorophenyl)prop-2-en-1-ol intermediate 153** (1.20 g, 105% mass recovery), as a light yellow oil. The material was judged pure by LCMS and NMR and used in the next step without further purification. ¹H NMR (400 MHz, CDCl₃, ppm) δ 7.31-7.18 (m, 3H, *overlap with* CDCl₃), 7.11-7.07 (m, 1H), 6.51 (d, *J* 12 Hz, 1H), 5.93 (dt, *J* 12, 6 Hz, 1H), 4.42 (dd, *J* 6, 2 Hz, 2H).

A solution of *tert*-butyldimethylsilyl chloride (1.29 g, 8.54 mmol) in DCM (4 mL) was added dropwise to a stirred solution of (Z)-3-(3-chlorophenyl)prop-2-en-1-ol (1.20 g, 6.80 mmol) and imidazole (581 mg, 8.54 mmol) in DCM (35 mL) at 0°C. After addition was complete, the cooling bath was removed, and the suspension was stirred for another 30 min. The mixture was transferred to a separating funnel and washed with water (100 mL). The aqueous layer was extracted once with DCM (10 mL) and the combined organic solutions were dried overnight (MgSO₄), filtered and concentrated *in vacuo.* The residue was purified on silica gel (40 g) eluting with a gradient of heptane:ethyl acetate = 100:0 to 95:5 to afford **(Z)-*tert*-butyl((3-(3-chlorophenyl)allyl)oxy)dimethylsilane intermediate 154** (1.93 g, 96%), as a colourless oil. ESI-MS [M - H]⁻ 281. ¹H NMR (400 MHz, CDCl₃, ppm) δ 7.32-7.18 (m, 4H, *overlap with* CDCl₃), 7.09-7.05 (m, 1H), 6.46-6.40 (m, 1H), 5.88 (dt, *J* 12, 6 Hz, 1H), 4.39 (dd, *J* 6, 2 Hz, 2H), 0.90 (s, 9H), 0.06 (s, 6H).

A solution of **intermediate 154** (1.21 g, 4.27 mmol) and Rh₂(OAc)₄ (37 mg, 0.085 mmol) in anhydrous DCM (12 mL) was heated to reflux. Once a steady reflux was established, a solution of ethyl diazoacetate (85% in DCM, 2.11 mL, 17.1 mmol) in anhydrous DCM (6.8 mL) was added *via* syringe pump over 5 h. At the end of the addition the reaction was judged complete by LCMS, and the solvent was evaporated to obtain an orange oil, which was purified by flash chromatography on silica gel (50 g), eluting with a gradient of heptane: ethyl acetate = 100:0 to 90:10 to obtain ethyl **(1R*,2R*,3S*) and (1R*,2S*,3R*)-2-(((*tert-*butyldimethylsilyl)oxy)methyl)-3-(3-chlorophenyl)cyclopropane-1-carboxylate intermediate 155** (1:1 mixture of isomers, 1.28 g, containing *ca.* 6% w/w diethyl fumarate, 77%), as a colourless oil. ESI-MS [M + H]⁺ 369/371; ¹H NMR (400 MHz, CDCl₃, ppm) δ 7.34 (s, 1H), 7.28 (s, 1H), 7.21-7.14 (m, 6H), 4.21-4.15 (m, 2H), 4.09-3.99 (m, 2H), 3.97 (dd, *J* 11, 8.5 Hz, 1H), 3.90 (dd, *J* 11, 5 Hz, 1H), 3.59 (dd, *J* 11, 6 Hz, 1H), 3.26 (dd, *J* 11, 8 Hz, 1H), 2.83 (dd, *J* 10, 5 Hz, 1H), 2.72 (t, *J* 9 Hz, 1H), 2.15 (t, *J* 9 Hz, 1H), 2.07-2.00 (m, 1H), 1.98 (t, *J* 5 Hz, 1H), 1.85 (ddd, *J* 14, 9, 5.4 Hz, 1H), 1.32 (s, *J* 7 Hz, 3H), 1.18 (t, *J* 7 Hz, 3H), 0.93 (s, 9H), 0.83 (s, 9H), 0.07 (s, 6 H), 0.05 (s, 6 H), -0.08 (s, 6H), -0.11 (s, 6H).

### Synthesis of rac-(1R*,2S*,3R*)-2-(3-chlorophenyl)-3-(hydroxymethyl)cyclopropane-1-carboxylic acid intermediate 156 and rac-(1R*,2R*,3S*)-2-(3-chlorophenyl)-3-(hydroxymethyl)cyclopropane-1-carboxylic acid intermediate 157

A freshly prepared solution of tetrabutylammonium fluoride trihydrate (2.07 g, 6.56 mmol) in THF (8 mL) was added to a solution of ethyl (1R*,2R*,3S*) and (1R*,2S*,3R*)-2-(((*tert-*butyldimethylsilyl)oxy)methyl)-3-(3-chlorophenyl)cyclopropane-1-carboxylate (1:1 mixture of isomers, 1.21 g, 3.28 mmol) in THF (8 mL). After 1 h the mixture was diluted with ethyl acetate (100 mL), washed with saturated aqueous sodium bicarbonate (2 × 50 mL) and brine, dried (MgSO₄), and concentrated under reduced pressure to obtain a dark brown oil that was used in the next step without further treatment.

The mixture of hydroxyesters was dissolved in a mixture of THF (6.4 mL) and methanol (3.2 mL) and aqueous lithium hydroxide (2 M, 6.4 mL, 12.8 mmol) was added at room temperature. After 2 h the reaction was quenched with hydrochloric acid (2 M, 10 mL). Ethyl acetate (100 mL) was added, the layers were separated, and the aqueous layer extracted once with ethyl acetate. The combined organic solutions were washed with brine, dried (Na₂SO₄), filtered and concentrated under reduced pressure to obtain a brown oil (ca. 950 mg). Purification by reversed-phase column chromatography, eluting with a gradient of MeCN:H₂O (50 mL/min, each containing 0.1% formic acid v/v; 10:90 to 35:65 over 12 column volumes). Mixed fractions containing the desired product isomers were pooled and purified again by reversed-phase chromatography (Biotage C₁₈ SNAP Ultra cartridge, 60 g; 50 mL/min, MeCN:H₂O each containing 0.1% formic acid v/v, 10:90 to 55:45 over 12 CV). Fractions containing the separated isomers were pooled to obtain (1R*,2S*,3R*)-2-(3-chlorophenyl)-3-(hydroxymethyl)cyclopropane-1-carboxylic acid as a colourless solid (268 mg, 36%) and (1R*,2R*,3S*)-2-(3-chlorophenyl)-3-(hydroxymethyl)cyclopropane-1-carboxylic acid as an off-white solid (213 mg, 29%). Assignment of the relative stereochemistry was accomplished by 2D NOESY NMR.

(1R*,2S*,3R*)-Isomer **intermediate 156** ESI-MS [M - H]⁻ 225/227, ¹H NMR (400 MHz, CD₃OD, ppm) δ 7.33-7.20 (m, 4H), 3.40 (dd, *J* 12, 7 Hz, 1H), 3.23 (dd, *J* 12, 7 Hz, 1H), 2.80 (dd, *J* 10, 5 Hz, 1H), 2.09 (t, *J 5* Hz, 1H), 2.03-1.95 (m, 1H).

(1R*,2R*,3S*)-Isomer **intermediate 157** ESI-MS [M - H]⁻ 225/227 ¹H NMR (400 MHz, CD₃OD, ppm) δ 7.31-7.17 (m, 4H), 3.96 (dd, *J* 11, 8 Hz, 1H), 3.85 (dd, *J* 11, 8 Hz, 1H), 2.78 (t, J9 Hz, 1H), 2.19 (t, *J* 9 Hz, 1H), 1.88 (pentet, *J* 8 Hz, 1H).

A mixture of **intermediate 156** (100 mg, 0.44 mmol), (NH₄)₂CO₃ (84.5 mg, 0.88 mmol), HOBt (90.4 mg,0.66mmol), EDCI(126.5 mg,0.66 mmol) and DIPEA(170.3 mg,1.32 mmol) in DMF(5 mL) was stirred at room temperature for 18h. Water (20 mL) was added then the reaction was extracted with EtOAc (3 x 30 mL). The combined organic layers were washed with brine (30 mL), dried over Na₂SO₄, filtered and concentrated *in vacuo* to give the crude, which was purified by silica gel chromatography (eluant: DCM/MeOH=20/1) to give **intermediate 158** (38 mg, 38%) as a white solid. ESI-MS [M +H]+: 226.1.

### Intermediate 159 rac-(1S*,2S*)-2-(5-methoxy-2-oxopyridin-1(2H)-yl)cyclopropane-1-carboxamide

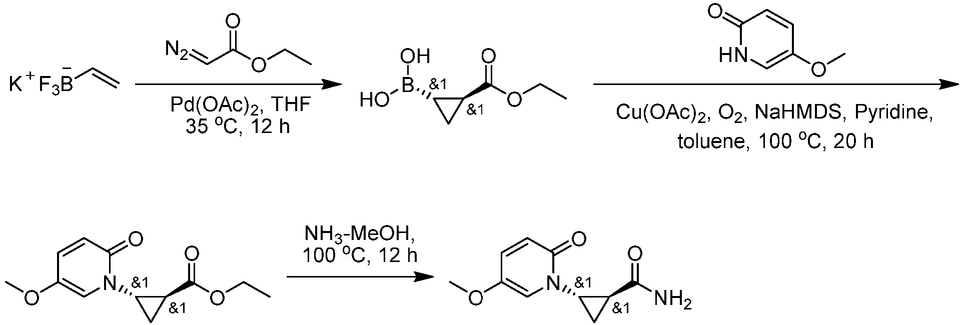

A mixture of trifluoro(vinyl)borate (3.0 g, 22.5 mmol), ethyl 2-diazoacetate (5.1 g, 45 mmol) and Pd(OAc)₂ (505 mg, 2.25 mmol) in THF (30 ml) was stirred at 35°C for 12 h. The reaction mixture was filtered and the filter cake was washed by DCM (400 ml). The filtrate was concentrated *in vacuo* to give the crude ***rac-*((1S*,2S*)-2-(ethoxycarbonyl)cyclopropyl)boronic acid intermediate** 160 (9.0 g crude), which was used into next step without further purification. ESI-MS: [M + H]⁺, 159.2

A mixture of **intermediate 160** (312 mg, 2.5 mmol), 5-methoxypyridin-2(1H)-one (1.18 g, 7.5 mmol), pyridine (987 mg, 12.5 mmol), NaHMDS (1.25 ml, 2.5 mmol) and Cu(OAc)₂ (452 mg, 2.5 mmol) in toluene (10 ml) was stirred at 100°C for 20 h. The mixture was cooled to room temperature, filtered and the filter cake was washed by EtOAc (100 mL). The filtrate was concentrated *in vacuo* to give the crude, which was purified by silica gel chromatography (eluant: EtOAc/PE = 4/1) to afford ***rac*-ethyl (1S*,2S*)-2-(5-methoxy-2-oxopyridin-1(2H)-yl)cyclopropane-1-carboxylate intermediate** 161 (125 mg, 20%) as a yellow oil. ESI-MS: [M + H]⁺, 238.2

A mixture of **intermediate 161** (100 mg, 0.1 mmol) and NH₃ in MeOH (7M, 10 ml, 70 mmol) were stirred at 100°C for 12 h in a sealed tube. The reaction was cooled to room temperature and concentrated *in vacuo* to give the crude which was purified by preparative TLC (eluant: DCM:MeOH = 10:1) to afford **intermediate 159** (65 mg, 75%) as a white solid. ESI-MS: [M + H]⁺, 209.2

### Intermediate 162: rac-(1S*,2S*)-2-(4-methoxypyridin-2-yl)cyclopropane-1-carboxamide

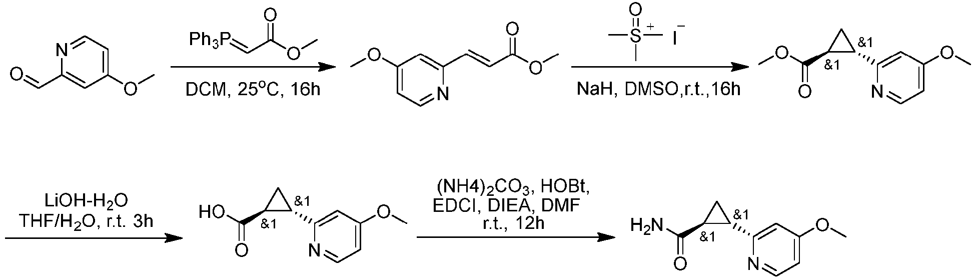

A mixture of 4-methoxypicolinaldehyde (3 g, 21.9 mmol) and methyl 2-(triphenyl-15-phosphaneylidene)acetate (7.3 g, 21.9 mmol) in DCM (30 mL) was stirred at room temperature for 16h. The reaction was quenched with water (100 mL) then extracted with DCM (3 x 50 mL). The combined organic layers were washed with brine (60 mL), dried over Na₂SO₄ and concentrated *in vacuo.* The residue was purified by silica gel column chromatography (eluent: EtOAc /PE from 0 to 20%) to afford **methyl (E)-3-(4-methoxypyridin-2-yl)acrylate intermediate 163** (3.3 g, 79 % yield) as a white solid. ESI-MS: [M + H]⁺, 194.2

To a mixture of trimethylsulfoxonium iodide (6.9 g, 31.2 mmol) in DMSO (50 mL) was added NaH (1.2 g, 60% suspension in paraffin oil, 31.2 mmol). The resulting mixture was stirred at room temperature for 2h. A solution of **intermediate 163** (2.0 g, 10.4 mmol) in DMSO (10 mL) was added and reaction mixture was stirred at room temperature for 16h. The reaction was quenched with saturated NH₄Cl (60 mL) then extracted with EtOAc (3 x 100 mL). The combined organic layers were washed with brine (100 mL), dried over Na₂SO₄ and concentrated. The residue was purified by silica gel column chromatography (eluent: EtOAc /PE from 0 to 20%) to afford ***rac*-methyl (1S*,2S*)-2-(4-methoxypyridin-2-yl)cyclopropane-1-carboxylate intermediate 164** (1.3 g, 60 % yield) as a yellow oil. ESI-MS: [M + H]⁺, 208.2

Using a similar procedure to that for **intermediate 23, intermediate 162** (0.65 g, 50%) was synthesised from **intermediate 164** (1.3 g, 6.3 mmol). ESI-MS: [M + H]⁺, 193.2.

### Intermediate 165: rac-tert-butyl (4-((1S*,2S*)-2-carbamoylcyclopropyl)-6-methoxypyridin-2-yl)carbamate

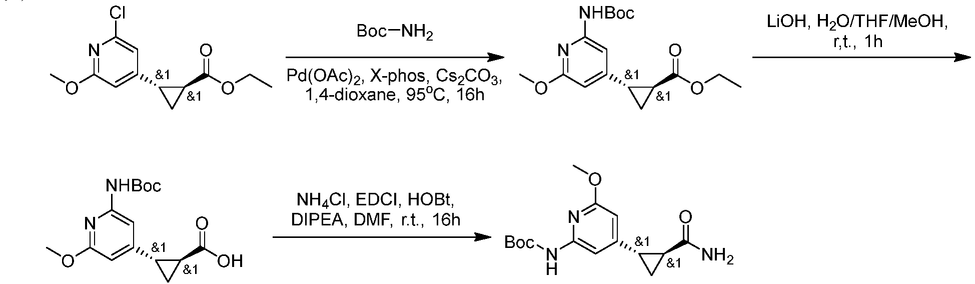

A mixture of *rac*-ethyl (1S*,2S*)-2-(2-chloro-6-methoxypyridin-4-yl)cyclopropane-1-carboxylate (300 mg, 1.17 mmol), tert-butyl carbamate (1.1 g, 9.36 mmol), Pd(OAc)₂ (26 mg, 0.117 mmol), X-phos (112 mg, 0.234 mmol) and Cs₂CO₃ (1.14 g, 3.51 mmol) in 1,4-dioxane (10 mL) was stirred at 90°C for 16 h under N₂. The reaction mixture was cooled to room temperature, filtered through Celite^{®}, and then the filter cake was washed with DCM/MeOH (10/1, 50 mL). The filtrate was concentrated *in vacuo* to give the crude, which was purified by silica gel chromatography (eluent: EtOAc/PE = 1/30) to give ***rac-*ethyl (1S*,2S*)-2-(2-((tert-butoxycarbonyl)amino)-6-methoxypyridin-4-yl)cyclopropane-1-carboxylate intermediate 166** (320 mg, 81%) as a white solid. ESI-MS [M +H]⁺: 337.1.

A mixture of **intermediate 166** (320 mg, 0.951 mmol) and LiOH·H₂O (160 mg, 3.81mmol) in THF (5 mL) / MeOH (5 mL) / H₂O (4 mL) was stirred at room temperature for 2 h. The reaction mixture was poured into water (40 mL) then the pH of the was acidified to 4~5 with HCl (2 N). The reaction mixture was extracted with DCM/MeOH (10/1, 3 x 40 ml). The combined organics were washed with brine (50 mL), dried over Na₂SO₄ then concentrated *in vacuo* to give ***rac*-(1S*,2S*)-2-(2-((tert-butoxycarbonyl)amino)-6-methoxypyridin-4-yl)cyclopropane-1-carboxylic acid intermediate 167** (293 mg crude) as a white solid. ESI-MS [M +H]⁺: 309.1.

A mixture of **intermediate 167** (293 mg, 0.951 mmol), NH₄Cl (509 mg, 9.51 mmol), EDCI (365 mg, 1.90 mmol), HOBt (257 mg, 1.90 mmol) and DIPEA (737 mg, 5.71 mmol) in DMF (10 mL) was stirred at 25°C for 16 h. The reaction mixture was poured into water (30 mL) then extracted with EtOAc (3 x 40 ml). The combined organics were washed with brine (40 mL), dried over Na₂SO₄ then concentrated *in vacuo* to give the crude, which was purified by silica gel chromatography (eluent: EtOAc/PE = 1/1) to give **intermediate 165** (240 mg, 82%) as a white solid. ESI-MS [M + H]⁺: 308.1.

### Intermediate 168: rac-(1R*,2R*)-6'-chloro-2',3'-dihydrospiro[cyclopropane-1,1'-indene]-2-carboxamide

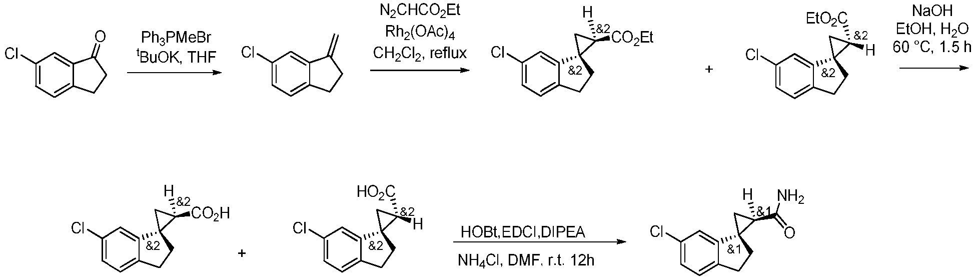

A solution of potassium tert-butoxide (97.0 mL, 1.0 M in THF, 97.0 mmol) was added to a suspension of methyltriphenylphosphonium bromide (34.7 g, 97.1 mmol) in anhydrous THF (150 mL) under nitrogen at 0 °C. The bright yellow suspension was stirred for 1 h and a solution of 6-chloroindan-1-one (8.09 g, 48.6 mmol) in anhydrous THF (75 mL) was added over 5 min. Once the addition was complete the cooling bath was removed. After 1 h, saturated aqueous ammonium chloride (100 mL) was added slowly. After being stirred for 10 min, the THF was removed *in vacuo,* and the residue was diluted with ethyl acetate (150 mL) and water (100 mL). The layers were separated, and the organic phase washed with brine, dried (MgSO₄) and concentrated *in vacuo* onto silica gel (50 g). This material was applied to the top of a chromatography column (200 g silica gel) which was eluted with heptane to afford **6-chloro-1-methylene-2,3-dihydro-1H-indene intermediate 169** (7.24 g, 90%) as a light-yellow oil. ¹H NMR (400 MHz, CDCl₃, ppm): δ 7.44 (s, 1H), 7.18-7.16 (m, 2H), 5.44 (t, *J* 2 Hz, 1H), 5.07 (t, *J* 2 Hz, 1H), 2.95-2.92 (m, 2H), 2.84-2.80 (m, 2H).

A solution of ethyl diazoacetate (85%, 10.0 g, 104 mmol) in anhydrous DCM (20 mL) was added *via* syringe pump over 5 h to a stirred solution of **intermediate 169** (5.69 g, 34.6 mmol) and rhodium acetate (153 mg, 0.346 mmol) in anhydrous DCM (80 mL) under nitrogen at reflux. The dark green-blue mixture was concentrated *in vacuo,* dissolved in toluene and applied to the top of a silica gel column (500 g), which was eluted with 1-4% methyl *tert-*butyl ether in heptane in 1% increments to give a mixture of **ethyl (1R*,2R*)-6'-chloro-2',3'-dihydrospiro[cyclopropane-1,1'-indene]-2-carboxylate intermediate 170 and ethyl (1R*,2S*)-6'-chloro-2',3'-dihydrospiro[cyclopropane-1,1'-indene]-2-carboxylates intermediate 171** (ratio 6:5, 3.932 g, 45%). Purification of several aliquots (10 × 7 mg) of the isomeric mixture by preparative HPLC was performed:
First eluted was a colourless oil (10 mg, RT = 5.6 min, minor isomer). This was assigned as the (1R*,2S*) **intermediate 171** by n.O.e experiments. ¹H NMR (400 MHz, CDCl₃, ppm): δ 7.18 (s, 1H), 7.10 (m, 2H), 4.06 (dq, *J* 11, 7 Hz, 1H), 3.99 (dq, *J* 11, 7 Hz, 1H), 3.05 (ddd, *J* 8.2, 10, 16 Hz, 1H), 2.86 (ddd, *J* 1.5, 8.9, 16 Hz, 1H), 2.39 (ddd, *J* 8.9, 10, 13 Hz, 1H), 2.10 (dd, *J* 6.4, 8 Hz, 1H), 1.92 (ddd, *J* 1.5, 8.2, 13 Hz, 1H), 1.84 (dd, *J* 5.6, 6.4 Hz, 1H), 1.41 (dd, *J* 5.6, 8 Hz, 1H), 1.16 (t, *J* 7 Hz, 3H).
Second eluted was a colourless oil (16 mg, RT = 6.0 min, major isomer). This was assigned as the (1R*,2R*) **intermediate 170** by n.O.e experiments. ¹H NMR (400 MHz, CDCl₃, ppm): δ 7.10 (m, 2H), 6.65 (s, 1H), 4.15 (m, 2H), 2.96 (m, 2H), 2.27 (m, 2H), 2.00 (dd, *J* 6, 8.5 Hz, 1H), 1.65 (dd, *J* 5, 6 Hz, 1H), 1.39 (dd, *J* 5, 8.5 Hz, 1H), 1.25 (t, J 7.2 Hz, 3H).

A solution of sodium hydroxide (1.88 g, 46.9 mmol) in water (23 mL) was treated with a solution of a mixture of ethyl **intermediate 170 and intermediate 171** (ratio 6:5, 3.92 g, 15.6 mmol) in ethanol (75 mL) under nitrogen. The mixture was heated at 60 °C for 90 min then was cooled to room temperature, concentrated *in vacuo,* and the residue was diluted with water (70 mL). Hydrochloric acid (2M) was added to give pH 1 which precipitated a gummy solid. The gum was extracted into ethyl acetate (3 × 70 mL), which was dried (Na₂SO₄) and concentrated *in vacuo* to give a pale brown solid. Recrystallisation from a mixture of boiling heptane (70 mL) and ethyl acetate (20 mL) gave **(1R*,2R*)-6'-chloro-2',3'-dihydrospiro[cyclopropane-1,1'-indene]-2-carboxylic acid and (1R*,2S*)-6'-chloro-2',3'-dihydrospiro[cyclopropane-1,1'-indene]-2-carboxylic acid intermediate 172** (1169 mg, 30%), as colourless needles. ESI-MS [M - H]⁻ 221/223, ¹H NMR (400 MHz, DMSO, ppm): δ 12.28 (br s, 1H), 7.22 (d, *J* 8 Hz, 1H), 7.17 (dd, *J* 8*,* 1.7 Hz, 1H), 6.98 (d, *J* 1.7 Hz, 1H), 2.99-2.86 (m, 2H), 2.24-2.11 (m, 2H), 2.00 (dd, *J* 6, 8.5 Hz, 1H), 1.48 (dd, *J* 5, 8.5 Hz, 1H), 1.44 (dd, *J* 5, 6 Hz, 1H).

A mixture of **intermediate 172** (230 mg, 1.04 mmol), NH₄Cl (220 mg, 4.14 mmol), HOBt (351 mg, 2.6 mmol), EDCI (500 mg, 2.6 mmol ) and DIPEA (671 mg, 5.2 mmol) in DMF (15 mL) was stirred at room temperature for 12 h under N₂. The reaction was quenched with water (30mL) then extracted with EtOAc (3 x 30 mL). The combined organic layers were washed with brine (50 mL), dried over Na₂SO₄, then concentrated *in vacuo* to give the crude, which was triturated with DCM (10 mL) to give **intermediate 168** (180 mg, 78%) as a white solid. ESI-MS [M +H]+: 222.1

### Intermediate 173: rac-(1S*2S*)-2-(3-chlorophenyl)-1-fluorocyclopropane-1-carboxamide

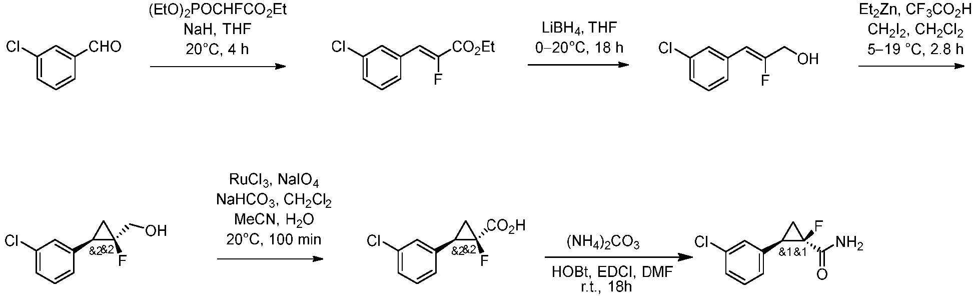

A solution of triethyl 2-fluorophosphonoacetate (12.1 g, 50 mmol) in anhydrous THF (25 mL) was added dropwise via syringe over 40 min to a stirred suspension of sodium hydride (2.00 g, 60% oil dispersion, 50 mmol) in anhydrous THF (50 mL) under nitrogen at room temperature. The resulting orange mixture was stirred for 55 min, and a solution of 3-chlorobenzaldehyde (2.81 g, 20.0 mmol) in anhydrous THF (15 mL) was added dropwise over 30 min. The resulting mixture was stirred for 4 h, and then treated with aqueous ammonium chloride (10% w/v, 75 mL). The product was extracted into methyl tert-butyl ether (100 mL and 50 mL) and the organic solutions were washed with water (2 × 50 mL), dried (Na₂SO₄) and concentrated *in vacuo.* The residue was dissolved in a little heptane (with warming) and purified by flash chromatography on silica gel (150 g), eluting with heptane followed by heptane : ethyl acetate = 50 : 1. Fractions containing product were combined and concentrated *in vacuo* to give a colourless solid (3.371 g, 74%), which consisted of a 5: 1 mixture of *E* and Z isomers. Following the discovery that the desired Z-isomer is a solid and the undesired E-isomer is a liquid, the 5:1 mixture of isomers (2.79 g) was suspended in 7 mL heptane and stored for 24 h at -20 °C. The crystals were collected by filtration and washed with cold heptane to give ethyl **Z-(3-chlorophenyl)-2-fluoropropanoate intermediate 174** (2.017 g), as a colourless solid.
¹H NMR (400 MHz, CDCl₃, ppm) Z-isomer: δ 7.64 (s, 1H), 7.52-7.50 (m, 1H), 7.36-7.31 (m, 2H), 6.85 (d, *J* 34 Hz, 1H), 4.36 (q, *J* 7.2 Hz, 2H), 1.39 (t, *J* 7.2 Hz, 3H).
¹H NMR (400 MHz, CDCl₃, ppm) E-isomer: δ 7.47 (s, 1H), 7.32-7.28 (m, 3H), 6.84 (d, J22 Hz, 1H), 4.25 (q, *J* 7.2 Hz, 2H), 1.25 (t, *J* 7.2 Hz, 3H).
¹⁹F NMR (373 MHz, CDCl₃, ppm, no reference) δ -115.3 (d, *J* 21 Hz, minor isomer), -122.9 (d, *J* 34 Hz, major isomer).

Lithium borohydride (789 mg, 37.6 mmol) was added in portions to a stirred solution of **intermediate 174** (2.147 g, 9.40 mmol) in anhydrous THF (50 mL) under nitrogen with cooling in an ice-water bath. After 10 min, the bath was removed, and the mixture was stirred at room temperature 17.5 h. The mixture was cooled in an ice-water bath and treated with saturated aqueous ammonium chloride (25 mL). Ethyl acetate (30 mL) and water (30 mL) were added and the layers were separated. The aqueous layer was extracted with ethyl acetate (3 × 50 mL) and the combined extracts were washed with brine (30 mL), dried (Na₂SO₄) and concentrated *in vacuo.* The residue was dissolved in ethyl acetate and adsorbed onto Dicalite. Purification by flash chromatography (100 g silica gel) eluting with heptane:ethyl acetate = 3:1 gave **(Z)-3-(3-chlorophenyl)-2-fluoroprop-2-en-1-ol intermediate 175,** as a colourless oil (1.840 g, containing 10% w/w ethyl acetate, 95%), which crystallised on standing. ¹H NMR (400 MHz, CDCl₃, ppm) δ 7.52 (s, 1H), 7.36 (d, *J* 7.6 Hz, 1H), 7.25 (t, *J* 8 Hz, 1H), 7.21 (m, 1H), 5.75 (d, *J* 38 Hz, 1H), 4.30 (dd, *J* 7*,* 13.5 Hz, 2H), 1.85 (t, *J* 7 Hz, 1H). ¹⁹F NMR (373 MHz, CDCl₃, ppm, no reference) δ -111.1 (dt, *J 38,* 13.5 Hz).

A solution of trifluoroacetic acid (2.58 mL, 33.5 mmol) in anhydrous DCM (5 mL) was added dropwise over 17 min to a solution of diethyl zinc (33.5 mL, 1.0 M in hexanes, 33.5 mmol) in anhydrous DCM (66 mL) under nitrogen, keeping the internal temperature between 5 °C and 7 °C by means of an ice-water bath. After 20 min, a solution of diiodomethane (2.70 mL, 33.5 mmol) in anhydrous DCM (5 mL) was added dropwise over 12 min (internal temperature 5 °C). The mixture was stirred for 26 min and then a solution **intermediate 175** (1.561 g, 8.37 mmol) in anhydrous DCM (10 mL) was added dropwise over 12 min (internal temperature 3-5 °C). After another 12 min the cooling bath was taken away, and the mixture was allowed to warm to room temperature. The mixture was stirred for 2.8 h, treated with treated with saturated aqueous ammonium chloride (25 mL), initially dropwise. Water (50 mL) was added and the layers were separated. The aqueous layer was extracted with DCM (3 × 40 mL) and the combined extracts were dried (Na₂SO₄) and concentrated *in vacuo.* Purification by flash chromatography (100 g silica gel) eluting with heptane: ethyl acetate = 4:1 gave **((1S*,2S*)-2-(3-chlorophenyl)-1-fluorocyclopropyl)methanol intermediate 176,** as an almost colourless oil (1.169 g, 70%). ¹H NMR (400 MHz, CDCl₃, ppm) δ 7.26-7.20 (m, 3H), 7.15-7.13 (m, 1H), 4.01 (ddd, *J* 5.6, 13, 20 Hz, 1H), 3.86 (ddd, *J* 6.4, 13, 20 Hz, 1H), 2.21 (ddd, *J* 4.4, 8, 10.4 Hz, 1H), 1.94-1.90 (m, 1H), 1.49 (dt, J20, 8 Hz, 1H), 1.32 (dt, J20, 8 Hz, 1H). ¹⁹F NMR (376 MHz, CDCl₃, ppm, no reference) δ -200.2 (dddt, *J* 4, 11, 13, 20 Hz).

**intermediate 176** (1.160 g, 5.79 mmol) was dissolved in a mixture of DCM (40 mL) and acetonitrile (40 mL) under nitrogen at room temperature. Water (60 mL) was added followed by sodium bicarbonate (3.205 g, 38.2 mmol) and sodium *meta*-periodate (6.946 g, 32.5 mmol). Finally, ruthenium (III) chloride (240 mg, 1.16 mmol) was added and the mixture was stirred vigorously for 100 min. The mixture was diluted with DCM (30 mL) and water (30 mL) and acidified with 2M hydrochloric acid to pH1. The mixture was filtered through a pad of Dicalite, and the filter cake was washed with DCM (100 mL). The filtrate layers were separated, the aqueous layer was extracted with DCM (2 × 50 mL) and the combined extracts were dried (Na₂SO₄) and concentrated *in vacuo.* The resulting black oily residue was adsorbed onto silica gel (10 g) with DCM and purified by flash chromatography, eluting with heptane : ethyl acetate : acetic acid = 50:50:1 to give (1R*,2R*)-2-(3-chlorophenyl)-1-fluorocyclopropane-1-carboxylic acid, as a pale grey oil (912 mg, 73%). This material was combined with another batch of similar quality (96 mg) and re-purified by flash chromatography on silica gel (100 g), eluting with heptane : ethyl acetate : acetic acid = 50:50:1 to give **(1S*,2S*)-2-(3-chlorophenyl)-1-fluorocyclopropane-1-carboxylic acid intermediate 177,** as pale grey oil (1043 mg, 68%, containing the following residual solvents: acetic acid (6.1% w/w), DCM (2.7% w/w) and ethyl acetate (1.3% w/w)). ESI-MS [M - H]⁻ 213/215, ¹H NMR (400 MHz, CDCl₃, ppm) δ 7.28-7.26 (m, 3H), 7.16-7.14 (m, 1H), 2.91 (ddd, *J* 2.4, 9.2, 11.2 Hz, 1H), 2.00 (ddd, *J* 2.4, 7.2, 10 Hz, 1H), 1.87 (ddd, *J* 7.2, 9.2, 16 Hz, 1H). ¹⁹F NMR (373 MHz, CDCl₃, ppm, no reference) δ -207.0 (apparent dd, *J* 10, 17 Hz).

A mixture of **intermediate 177** (370 mg,1.72 mmol), (NH₄)₂CO₃ (331 mg, 3.45 mmol), HOBt (348.3 mg, 2.58 mmol), EDCI (497.9 mg, 2.58 mmol) and DIPEA (665.6 mg, 5.16 mmol) in DMF (5 mL) was stirred at room temperature for 18h. Water (20 mL) was added then the mixture was extracted with EtOAc (3 x 30 mL). The combined organic layers were washed with brine (20 mL), dried over Na₂SO₄, filtered and concentrated *in vacuo* to give the crude, which was purified by silica gel chromatography (eluant: PE/EtOAc = 2/1) to give **intermediate 173** (180 mg, 49.2%) as a white solid. ESI-MS [M +H]+: 214.1.

### Intermediate 178: rac-(1S*,2S*)-2-(5-chlorothiophen-2-yl)cyclopropane-1-carboxamide

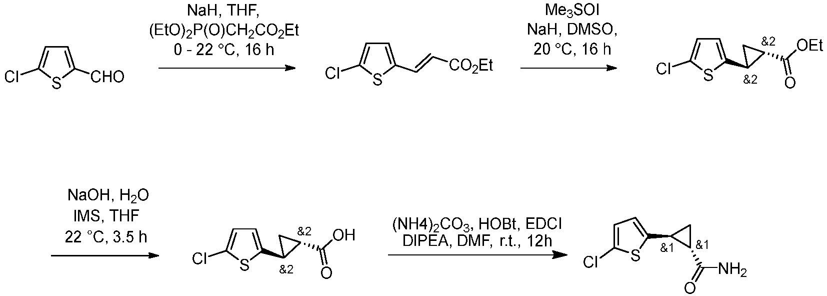

Sodium hydride (60% dispersion in oil, 1.074 g, 26.9 mmol) was suspended in anhydrous THF (150 mL) at 0°C under nitrogen. Triethyl phosphonoacetate (4.80 mL, 24.2 mmol) was added dropwise over 5 min. The mixture was stirred for 45 min. 5-Chloro-2-thiophenecarboxaldehyde (2.20 mL, 20.7 mmol) was added dropwise over 5 min. The mixture was allowed to warm to room temperature and stirred for a further 16 h. The mixture was poured into saturated aqueous ammonium chloride (300 mL) and extracted with methyl *tert*-butyl ether (3 × 100 mL). The combined extracts were washed with brine (150 mL), dried (MgSO₄), filtered and concentrated *in vacuo* to give a light brown oil (5.055 g). Purification by flash column chromatography (SiliCycle SiliaSep cartridge, 120 g) eluting with 5% ethyl acetate in heptane for 1 column volume followed by 5-25% ethyl acetate in heptane over 10 column volumes gave **ethyl (E)-3-(5-chlorothiophen-2-yl)acrylate intermediate 179** (3.585 g, 80%) as a pale yellow oil. ¹H NMR (400 MHz, CDCl₃, ppm) δ 7.63 (d, *J* 15.7 Hz, 1H), 7.02 (d, *J* 4.2 Hz, 1H), 6.87 (d, *J* 4.2 Hz, 1H), 6.10 (d, *J* 15.7 Hz, 1H), 4.24 (q, *J* 7.0 Hz, 2H), 1.32 (t, *J* 7.0 Hz, 3H).

Sodium hydride (60% dispersion in oil, 0.381 g, 9.53 mmol) was washed with heptane (2 × 10 mL) under nitrogen prior to use and then anhydrous DMSO (12 mL) was added. To this was added a solution of trimethylsulfoxonium iodide (2.058 g, 9.35 mmol) in anhydrous DMSO (14 mL) dropwise over 15 min to give a cloudy suspension. The mixture was stirred at room temperature for 1 h, during which time the mixture became a pale-yellow solution. To this was added a solution of ETHYL? **intermediate 179** (1.350 g, 6.23 mmol) in anhydrous DMSO (4 mL) dropwise over 5 min. The mixture was stirred for 16 h and then poured into water (50 mL) and extracted with ethyl acetate (4 × 30 mL). The combined extracts were washed with water (2 × 50 mL) and brine (50 mL). The extracts were dried (Na₂SO₄), filtered and concentrated *in vacuo* to give an orange oil. Purification by flash column chromatography (SiliCycle SiliaSep cartridge, 40 g) eluting with 5% ethyl acetate in heptane for 1 column volume followed by 5-30% ethyl acetate in heptane over 15 column volumes gave ethyl **(1R*,2R*)-2-(5-chlorothiophen-2-yl)cyclopropane-1-carboxylate intermediate 180** (0.496 g, 35%) as a pale yellow oil. ¹H NMR (400 MHz, CDCl₃, ppm) δ 6.69 (d, *J* 4.2 Hz, 1H), 6.58 (d, *J* 4.4 Hz, 1H), 4.17 (q, *J* 7.1 Hz, 2H), 2.58 (ddd, *J* 9*,* 5, 4 Hz, 1H), 1.88 (ddd, *J* 8*,* 5, 4 Hz, 1H), 1.58 (ddd, *J* 9*,* 6, 5 Hz, 1H), 1.28 (t, *J* 7.1 Hz, 3H), 1.25 (ddd, *J* 8*,* 6, 4 Hz, 1H).

**Intermediate 180** (0.643 g, 2.79 mmol) was dissolved in a mixture of aqueous sodium hydroxide (2 M, 3.5 mL, 7.00 mmol), THF (3 mL) and industrial methylated spirit (3 mL). The mixture was stirred at room temperature for 2 h 15 min and then acidified to pH 5 using aqueous hydrochloric acid (2 M). The mixture was diluted with water (10 mL) and extracted with ethyl acetate (3 × 20 mL). The combined extracts were washed with water (20 mL) and brine (20 mL), dried (MgSO₄), filtered and concentrated *in vacuo* to give a pale-yellow oil. The aqueous phase was found to contain product and so the pH was reduced to pH 3 with hydrochloric acid. The aqueous phase was extracted further with ethyl acetate (3 × 20 mL), and the extracts were dried (MgSO₄), filtered and concentrated *in vacuo* to give a pale-yellow oil. The two oils (ca. 0.7 g) were combined and purified by column chromatography on silica gel (21 g) eluting with 10% ethyl acetate in heptane + 1% acetic acid to give **(1R*,2R*)-2-(5-chlorothiophen-2-yl)cyclopropane-1-carboxylic acid intermediate 181** (0.549 g, 84%) as a pale-yellow oil that solidified on standing. ESI-MS [M - H]⁻ 201, ¹H NMR (400 MHz, CDCl₃, ppm) δ 6.71 (d, *J* 3.6 Hz, 1H), 6.60 (d, *J* 3.0 Hz, 1H), 2.67 (ddd, *J* 10, 7, 4 Hz, 1H), 1.90 (ddd, J9, 5, 4 Hz, 1H), 1.65 (ddd, *J* 10, 5, 5 Hz, 1H), 1.36 (ddd, *J* 9*,* 7, 5 Hz, 1H).

A solution of **intermediate 181** (260 mg, 1.28 mmol), (NH₄)₂CO₃(247 mg, 2.56 mmol), HOBt (260 mg, 1.92 mmol), EDCI (372 mg, 1.92 mmol), and DIPEA (498 mg, 3.84 mmol) ) in DMF (4 mL) was stirred at room temperature for 12 h. Water (20 mL) was added to the reaction which was then extracted with EtOAc (5 x 50 mL). The combined organic layers were washed with brine (30 mL), dried over anhydrous Na₂SO₄, and concentrated *in vacuo* to give the crude, which was purified by silica gel chromatography (eluent: DCM/MeOH = 15/1) to give **intermediate 178** (180 mg, 69%) as a white solid. ESI-MS [M +H]+: 202.1

### Intermediate 182: rac-(1S*2S*)-2-(4-chlorothiophen-2-yl)cyclopropane-1-carboxamide

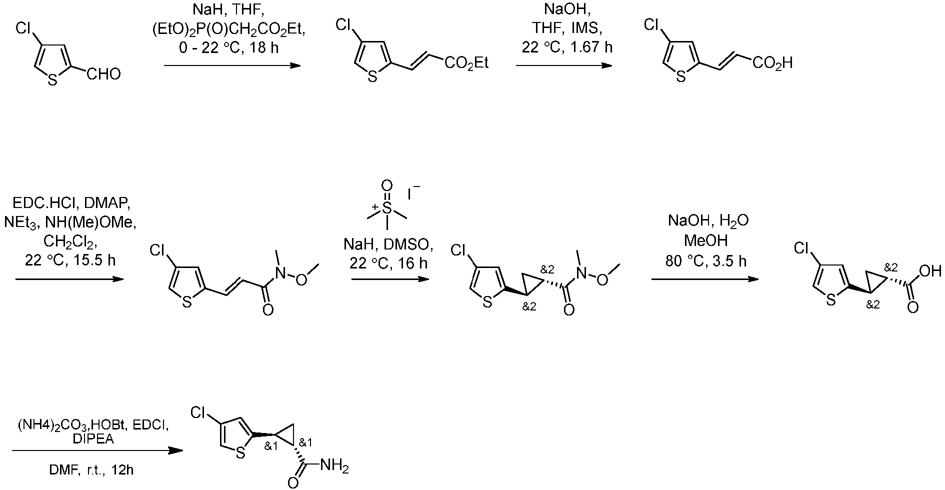

Sodium hydride (60% dispersion in oil, 1.074 g, 26.9 mmol) was suspended in anhydrous THF (150 mL) under nitrogen at 0°C. Triethyl phosphonoacetate (4.80 mL, 24.2 mmol) was added dropwise over 5 min and the mixture was stirred for 1 h. 4-Chloro-2-thiophenecarboxaldehyde (2.20 mL, 20.7 mmol) was added dropwise over 5 min. The mixture was warmed to room temperature and stirred for a further 18 h. The reaction mixture was poured into saturated aqueous ammonium chloride (300 mL) and extracted with methyl *tert*-butyl ether (3 × 100 mL). The combined organic extracts were washed with brine (150 mL), dried (MgSO₄), filtered and concentrated *in vacuo* to give a dark red solid (5.156 g). The solid was purified by silica gel column chromatography (100 g), eluting with 10% ethyl acetate in heptane to give ethyl **(E)-3-(4-chlorothiophen-2-yl)acrylate intermediate 183** (3.355 g, 75%), as an orange solid. ESI-MS [M - OEt]⁺ 171, ¹H NMR (400 MHz, CDCl₃, ppm) δ 7.65 (d, *J* 15.7 Hz, 1H), 7.13 (s, 1H), 7.11 (s, 1H), 6.24 (d, *J* 15.7 Hz, 1H), 4.25 (q, *J* 7.1 Hz, 2H), 1.32 (t, *J* 7.1 Hz, 3H).

**Intermediate 183** (2.703 g, 12.5 mmol) was dissolved in a mixture of THF (15 mL) and industrial methylated spirit (15 mL). Aqueous sodium hydroxide (2M, 17 mL, 34 mmol) was added and the mixture was stirred at room temperature for 1 h 40 min. The mixture was acidified to pH 2 using hydrochloric acid (2M). The mixture was diluted with water (40 mL) and extracted with ethyl acetate (3 × 50 mL). The extracts were dried (MgSO₄), filtered and concentrated *in vacuo* to give **(E)-3-(4-chlorothiophen-2-yl)acrylic acid intermediate 184** (2.325 g, 99% ), as a pale brown solid. ESI-MS [M - CO₂H]⁻ 143, ¹H NMR (400 MHz, CDCl₃, ppm) δ 7.75 (d, *J* 15.7 Hz, 1H), 7.19 (s, 1H), 7.16 (s, 1H), 6.25 (d, *J* 15.7 Hz, 1H).

**intermediate 184** (2.325 g, 12.3 mmol), 1-ethyl-3-(3-(N,N-dimethylamino)propyl)carbodiimide (3.287 g, 17.2 mmol), triethylamine (1.90 mL, 25.9 mmol), (4-dimethylamino)pyridine (1.637 g, 13.4 mmol) and N,O-dimethylhydroxylamine hydrochloride (1.516 g, 15.5 mmol) were mixed together in DCM (40 mL) and stirred at room temperature under nitrogen for 15.5 h. The mixture was diluted with DCM (50 mL) and washed with water (2 × 75 mL) then brine (75 mL). The organic phase was dried (MgSO₄), filtered and concentrated *in vacuo* to give a red oil (4.074 g). Purification by flash column chromatography (SiliCycle SiliaSep cartridge, 120 g), eluting with 5% ethyl acetate in heptane for 2 column volumes and then 5-50% ethyl acetate in heptane over 15 column volumes gave **(E)-3-(4-chlorothiophen-2-yl)-N-methoxy-N-methylacrylamide intermediate 185** (2.143 g, 75%) as a pale-yellow oil. ESI-MS [M + H]⁺ 232, ¹H NMR (400 MHz, CDCl₃, ppm) δ 7.70 (d, *J* 15.7 Hz, 1H), 7.12 (s, 1H), 7.11 (s, 1H), 6.83 (d, *J* 15.7 Hz, 1H), 3.75 (s, 3H), 3.29 (s, 3H).

Sodium hydride (60% dispersion in oil, 0.660 g, 16.3 mmol) was washed with heptane (2 × 15 mL) and suspended in anhydrous DMSO (10 mL). To this suspension was added a solution of trimethylsulfoxonium iodide (3.310 g, 15.0 mmol) in anhydrous DMSO (30 mL) dropwise over 15 min to give a cloudy suspension. The mixture was stirred at room temperature under nitrogen for 1 h, during which time it became a pale-yellow translucent solution. To this was added a solution of **intermediate 185** (2.143 g, 9.3 mmol) in anhydrous DMSO (5 mL) dropwise over 5 min. The mixture was stirred at room temperature for 16 h, poured into water (100 mL) and extracted with ethyl acetate (5 x 50 mL). The combined organic extracts were washed with water (2 × 100 mL) and brine (100 mL). The organic solution was dried (MgSO₄), filtered and concentrated *in vacuo* to give a yellow oil. Purification by flash column chromatography (SiliCycle SiliaSep cartridge, 40 g), eluting with heptane for 2 column volume followed by 5-20% ethyl acetate in heptane over 15 column volumes and then 20% ethyl acetate in heptane for 5 column volumes gave **(1S*,2S*)-2-(4-chlorothiophen-2-yl)-N-methoxy-N-methylcyclopropane-1-carboxamide intermediate 186** (1.748 g, 77%) as a yellow oil. ESI-MS [M + H]⁺ 246. ¹H NMR (400 MHz, CDCl₃, ppm) δ 6.87 (d, *J* 1.8 Hz, 1H), 6.69 (s, 1H), 3.74 (s, 3H), 3.24 (s, 3H), 2.63-2.58 (m, 1H), 2.43 (br s, 1H), 1.67-1.63 (m, 1H), 1.31-1.27 (m, 1H).

**intermediate 186** (1.748 g, 7.10 mmol) was dissolved in methanol (14 mL) and aqueous sodium hydroxide (2M, 8.0 mL, 16 mmol) was added. The mixture was stirred at 80°C (oil bath) under nitrogen for 3.5 h. The mixture was cooled to room temperature and then acidified to pH 2 using 2M aqueous hydrochloric acid. The mixture was diluted with water (20 mL) and extracted with ethyl acetate (3 × 30 mL). The extracts were dried (MgSO₄), filtered and concentrated *in vacuo* to give a pale-yellow oil (1.851 g). Purification by flash column chromatography (SiliCycle SiliaSep cartridge, 120 g), eluting with 5% ethyl acetate in heptane + 1% acetic acid for 2 column volumes followed by 5-60% ethyl acetate in heptane + 1% acetic acid over 15 column volumes) gave a pale-yellow oil, which crystallised on standing (1.338 g). Recrystallisation from hot heptane gave **(1S*,2S*)-2-(4-chlorothiophen-2-yl)cyclopropane-1-carboxylic acid intermediate 187** (0.601 g, 42%), as a colourless solid. ESI-MS [M - H]⁻ 201, ¹H NMR (400 MHz, CDCl₃, ppm) δ 6.90 (d, *J* 1.2 Hz, 1H), 6.71 (s, 1H), 2.70 (ddd, *J* 10, 6, 4 Hz, 1H), 1.93 (ddd, *J* 9, 6, 5 Hz, 1H), 1.68 (ddd, *J* 10, 7, 5 Hz, 1H), 1.38 (ddd, *J* 9*,* 7, 4 Hz, 1H).

A mixture of *rac*-**intermediate 187** (300 mg, 1.49 mmol), (NH₄)₂CO₃ (713 mg, 7.43 mmol), HOBt (402 mg, 2.98 mmol), EDCI (566 mg, 2.98 mmol) and DIPEA (577 mg, 4.47 mmol) in DMF (10 mL) was stirred at room temperature for 12h. The mixture was quenched with water (100 mL) then extracted with EtOAc (5 x 50 mL). The combined organic layers were washed with brine (50 mL), dried over Na₂SO₄ and concentrated *in vacuo.* The residue was purified by silica gel chromatography (eluant: EtOAc /PE from 0 to 30%) to afford **intermediate 182** (250 mg, 83%) as an off-white solid. ESI-MS [M +H]⁺: 202.1.

### Intermediate 188: rac-tert-butyl (3-((1S*,2S*)-2-carbamoylcyclopropyl)-5-chlorophenyl)carbamate

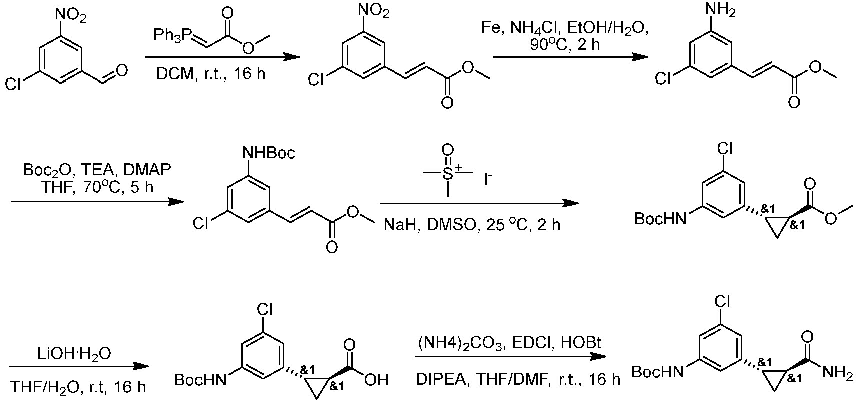

To a solution of 3-chloro-5-nitrobenzaldehyde (1.75 g, 9.5 mmol) in DCM (50 mL) was added methyl 2-(triphenyl-phosphanylidene)acetate (3.8 g, 11.3 mmol) and the mixture was stirred at room temperature for 16 h. The mixture was quenched with water (100 mL) and extracted with DCM (3 x 50 mL). The combined organic layers were washed with brine (50 mL), dried over Na₂SO₄ and concentrated *in vacuo.* The residue was purified by silica gel chromatography (eluent: EtOAc /PE from 0 to 15%) to give **methyl (E)-3-(3-chloro-5-nitrophenyl)acrylate intermediate 189** (2 g, 88%) as a white solid. ESI-MS [M +H]⁺: 242.1.

A mixture of **intermediate 189** (2 g, 8.3 mmol), Fe (4.6 g, 83 mmol) and NH₄Cl (4.4 g, 83 mmol) in EtOH/H₂O (60 mL/20mL) was stirred at 90°C for 2 h. The reaction mixture was poured into water (100 mL) and filtered. The filtrate was extracted with EtOAc (3 x 40 ml). The combined organics were washed with brine (40 mL), dried over Na₂SO₄ and concentrated *in vacuo.* The residue was purified by silica gel chromatography (eluent: EtOAc /PE from 0 to 15%) to give **methyl (E)-3-(3-amino-5-chlorophenyl)acrylate intermediate 190** (1.75 g, 98%) as a yellow solid. ESI-MS [M +H]⁺: 212.1.

To a mixture of methyl (**intermediate 189** (1.9 g, 9 mmol), Et₃N (2.73 g, 27 mmol) and DMAP (1.65 g, 13.5 mmol) in THF (50 mL) was added Boc₂O (3.93 g, 18 mmol) and the mixture was stirred at 70°C for 5 h. The mixture was cooled to room temperature, diluted with water (100 mL) then extracted with EtOAc (3 x 50 mL). The combined organics were washed with brine (50 mL), dried over Na₂SO₄ and concentrated. The residue was purified by silica gel chromatography (eluent: EtOAc /PE from 0 to 15%) to give **methyl (E)-3-(3-((tert-butoxycarbonyl)amino)-5-chlorophenyl)acrylate intermediate 190a** (2.3 g, 82%) as a white solid. ESI-MS [M -55]⁺: 256.1.

To a stirred solution of trimethylsulfoxonium iodide (960 mg, 4.4 mmol) in dry DMSO (20 mL) was added NaH (180 mg, 4.4 mmol) at 25°C and the mixture was stirred at 25°C for 1 h. A solution of **intermediate 190a** (1 g, 3.2 mmol) in DMSO (10 mL) was then added dropwise. The mixture was stirred at 25°C for 2 h, quenched with water (100 mL) and extracted with EtOAc (2 x 100 ml). The combined organics were washed with water (3 x 100 mL) and brine (100 mL), dried over Na₂SO₄, concentrated *in vacuo* and purified by silica gel chromatography (eluent: EtOAc /PE from 0 to 15%) to give ***rac*-methyl (1S*,2S*)-2-(3-((tert-butoxycarbonyl)amino)-5-chlorophenyl)cyclopropane-1-carboxylate intermediate 191** (240 mg, 23%) as a white solid. ESI-MS [M + Na]⁺: 348.2.

Using a similar procedure to that for **intermediate 23, intermediate 188** (220 mg, 76%) was synthesised from **intermediate 191** (240 mg, 0.74 mmol). ESI-MS [M + Na]+: 333.1.

### Intermediate 192: rac-(1S*,2S*)-2-(2-chloro-6-methoxypyridin-4-yl)cyclopropane-1-carboxamide

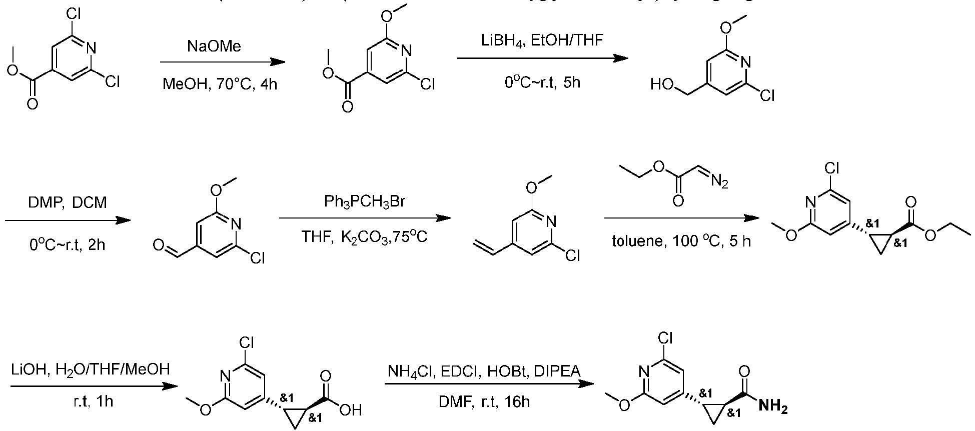

Na (2.23 g, 97.0 mmol) was added to a MeOH (100 mL) with stirring at 0°C in portions and the mixture was stirred at room temperature for 1h. Methyl 2,6-dichloroisonicotinate (10 g, 48.54 mmol) was added in portions and the resulting mixture was stirred at 70°C for 4 h. The reaction mixture was adjusted to pH 9~10 by NaHCO₃ aqueous (100 mL) and MeOH was removed *in vacuo.* The mixture was extracted with EtOAc (3 x 50 ml). The combined organics were washed with brine (50 mL), dried over Na₂SO₄ and concentrated *in vacuo.* The residue was purified by silica gel chromatography (eluent: EtOAc/PE = 1/5) to give **methyl 2-chloro-6-methoxyisonicotinate intermediate 193** (7.5 g, 77%) as a white solid. ESI-MS [M +H]⁺: 202.2.

To a stirred solution of **intermediate 193** (4.5 g, 22.32 mmol) in THF (60 mL) and EtOH (20 mL) was added LiBH₄ (972 mg, 44.64 mmol) in portions at 0°C and the mixture was stirred at room temperature for 5 h. The reaction mixture was quenched with saturated NH₄Cl aqueous (100 mL) and extracted with EtOAc (3 x 100 ml). The combined organics were washed with brine (100 mL), dried over Na₂SO₄, concentrated *in vacuo* to give **(2-chloro-6-methoxypyridin-4-yl)methanol intermediate** 194 (3.8 g, 98%) as a white solid which was used directly in the next step. ESI-MS [M +H]⁺: 174.1.

To a stirred solution of **intermediate 194** (2.15 g, 12.38 mmol) in DCM (80 mL) was added Dess-Martin periodinane (7.88 g, 18.58 mmol) in portions at 0°C. The mixture was stirred at room temperature for 2 h, filtered and rinsed with DCM (50 mL). The filtrate was washed with saturated NaHCO₃ aqueous (80 mL) and brine (80 mL), dried over Na₂SO₄ and concentrated *in vacuo.* The residue was purified by silica gel chromatography (eluent: EtOAc/PE = 1/10) to give **2-chloro-6-methoxyisonicotinaldehyde intermediate 195** (2.0 g, 94%) as a colorless syrup. ESI-MS [M + H]⁺: 172.1.

To a solution of **intermediate 195** (3.2 g, 18.65 mmol) in THF (50 mL) was added bromo(methyl)triphenyl-λ5-phosphane (9.99 g, 27.98 mmol) and K₂CO₃ (7.73 g, 55.95 mmol) at room temperature. The mixture was stirred at 70°C for 16 h. The reaction mixture was poured into water (100 mL) and extracted with EtOAc (3 x 80 ml). The combined organics were washed with brine (80 mL), dried over Na₂SO₄ then concentrated *in vacuo* to give the crude, which was purified by silica gel chromatography (eluent: EtOAc/PE = 1/5) to give **2-chloro-6-methoxy-4-vinylpyridine intermediate 196** (2.2 g, 70%) as a yellow oil. ESI-MS [M + H]⁺: 170.1.

To a stirred solution of **intermediate 196** (2.2 g, 12.97 mmol) in toluene (40 mL) was added ethyl 2-diazoacetate (4.43 g, 38.91 mmol) in three portions over 2 h at 100°C and the mixture was stirred at 100°C for another 3 h. The reaction mixture was cooled, quenched with water (100 mL) and extracted with EtOAc (3 x 50 mL). The combined organic layers were washed with brine (50 mL), dried over Na₂SO₄ and concentrated *in vacuo.* The residue was purified by silica gel chromatography (EtOAc /PE from 0 to 50%) concentrated to give the crude, which was purified by silica gel chromatography (eluent: EtOAc/PE = 1/5) to give ***rac*-ethyl (1S*,2S*)-2-(2-chloro-6-methoxypyridin-4-yl)cyclopropane-1-carboxylate intermediate 197** (1.06 g, 32%, trans-racemic) as a yellow oil. ESI-MS [M + H]⁺: 256.1.

Using a similar procedure to that for **intermediate 165, intermediate 192** (75 mg, 77%) was synthesised from **intermediate 197** (110 mg, 0.43 mmol). ESI-MS [M + H]⁺: 227.1.

### Intermediate 198: rac-(1S*,2S*)-2-(6-chloro-3-cyanopyridin-2-yl)cyclopropane-1-carboxamide

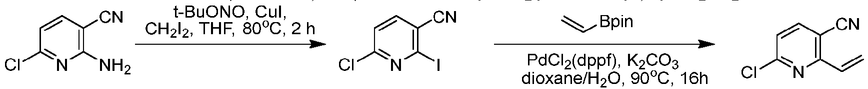

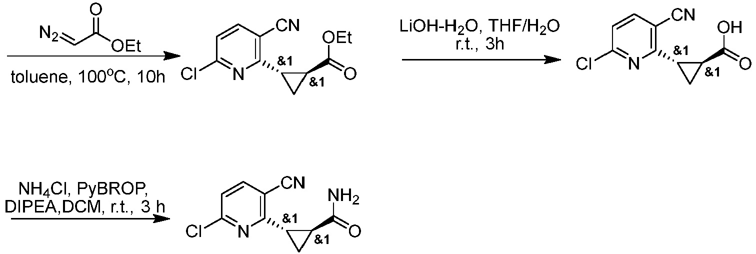

A mixture of 2-amino-6-chloronicotinonitrile (857 mg, 5.6 mmol), t-BuONO (2.3 g, 22.4 mmol), CH₂I₂ (12 g, 44.8 mmol) and CuI (1.6 g, 8.4 mmol) in THF (30 mL) was stirred at 85°C for 2 h. The reaction was concentrated *in vacuo* then the residue was dissolved in EtOAc (100 mL). The organics were washed with saturated Na₂S₂O₃ solution (40 mL), saturated NaHCO₃ solution (40 mL) and brine (40 mL). The organic phase was dried over Na₂SO₄, concentrated *in vacuo* to give the crude product, which was purified by silica gel chromatography (eluent: EtOAc /PE from 0 to 10%) to give **6-chloro-2-iodonicotinonitrile intermediate 199** as a yellow solid. (900 mg, 61%). ESI-MS [M +H]⁺: 264.9.

To a solution of **intermediate 199** (530 mg, 2 mmol) in 1,4-dioxane (12 mL) and water (3 mL) was added K₂CO₃ (828 mg, 6 mmol), Pd(dppf)Cl₂ (146 mg, 0.2 mmol) and 4,4,5,5-tetramethyl-2-vinyl-1,3,2-dioxaborolane (308 mg, 2 mmol). The resulting mixture was stirred at 90°C overnight. After cooling to room temperature, the mixture was diluted with water (50 mL) and extracted with EtOAc (3 x 30 mL). The combined organic layers were washed with brine (30 mL), dried over Na₂SO₄, filtered, and concentrated *in vacuo.* The residue was purified by silica gel chromatography column (eluent: EtOAc /PE from 0 to 10%) to give **6-chloro-2-vinylnicotinonitrile intermediate 200** (250 mg, 76 %) as white solid. ESI-MS [M +H]⁺: 165.0.

To a reaction mixture of **intermediate 200** (250 mg, 1.5 mol) in toluene (10 mL) was added ethyl 2-diazoacetate (513 mg, 4.5 mol). The reaction was stirred at 100°C for 10 h. The reaction was cooled to room temperature and concentrated *in vacuo* to give the crude, which was purified by silica gel chromatography (eluent: EtOAc /PE from 0 to 10%) to afford ***rac*-ethyl (1S*,2S*)-2-(6-chloro-3-cyanopyridin-2-yl)cyclopropane-1-carboxylate intermediate 201** (230 mg, 60%) as white solid. ESI-MS [M +H]+: 251.0.

Using a similar procedure to that for **intermediate 116, intermediate 198** (120 mg, 69%) was synthesised from **intermediate 201** (230 mg, 0.92 mmol). ESI-MS [M +H]+: 222.1.

### Intermediate 202: rac-tert-butyl (tert-butoxycarbonyl)(4-((1S*,2S*)-2-carbamoylcyclopropyl)-2-chlorophenyl)carbamate

To a solution of 3-chloro-4-nitrobenzaldehyde (1.5 g, 8.08 mmol) in THF (40 mL) was added ethyl (triphenylphosphoranylidene)acetate (3.66 g, 10.51 mmol) and the mixture was stirred at room temperature for 16 h. The reaction mixture was concentrated *in vacuo* to give the crude, which was purified by silica gel chromatography (eluent: EtOAc/PE = 1/10) to give **ethyl (E)-3-(3-chloro-4-nitrophenyl)acrylate intermediate 203** (1.8 g, 87%) as a yellow solid. ESI-MS [M +H]⁺: 256.1.

A mixture of **intermediate 203** (490 mg, 1.92 mmol) and Zn (1.26 g, 19.3 mmol) in AcOH (10 mL) was stirred at room temperature for 16 h. The reaction mixture was poured into water (30 mL), adjusted to pH 9~10 with NaHCO₃ solution and filtered. The filtrate was extracted with EtOAc (3 x 50 ml). The combined organics were washed with brine (50 mL), dried over Na₂SO₄, concentrated *in vacuo* to give the crude, which was purified by silica gel chromatography (eluent: EtOAc/PE = 1/10) to give **ethyl (E)-3-(4-amino-3-chlorophenyl)acrylate intermediate 204** (390 mg, 90%) as a yellow solid. ESI-MS [M +H]⁺: 226.1.

To a mixture of **intermediate 204** (390 mg, 1.73 mmol), Et₃N (875 mg, 8.65 mmol) and DMAP (106 mg, 0.865 mmol) in DCM (10 mL) was added dropwise Boc₂O (943 mg, 4.32 mmol) and the mixture was stirred at room temperature for 16 h. The reaction mixture was diluted with DCM (50 mL) and washed with water (40 mL) and brine (40 mL), dried over Na₂SO₄ then concentrated *in vacuo* to give the crude, which was purified by silica gel chromatography (eluent: EtOAc/PE = 1/10) to give **ethyl (E)-3-(4-(di-(tert-butoxycarbonyl)amino)-3-chlorophenyl)acrylate intermediate 205** (260 mg, 35%) as a yellow solid. ESI-MS [M + Na]⁺: 448.1.

To a stirred solution of trimethylsulfoxonium iodide (242 mg, 1.1 mmol) in dry DMSO (5 mL) was added NaH (44 mg, 1.1 mmol) at 25°C. After stirring the mixture for 1h, a solution of **intermediate 205** (260 mg, 0.61 mmol) in DMSO (1 mL) was added dropwise and stirred at 25°C for another 2 h. The reaction was quenched with water (80 mL) then extracted with EtOAc (3 x 30 ml). The combined organics were washed with brine (2 x 30 mL), dried over Na₂SO₄ then concentrated *in vacuo* to give the crude, which was purified by silica gel chromatography (eluent: EtOAc/PE = 1/10) to give ***rac*-ethyl (1S*,2S*)-2-(4-(bis(tert-butoxycarbonyl)amino)-3-chlorophenyl)cyclopropane-1-carboxylate intermediate 206** (100 mg, 37%) as a white solid. ESI-MS [M + Na]⁺: 462.1.

Using a similar procedure to that for **intermediate 165, intermediate 202** (40 mg, 77%) was synthesised from **intermediate 206** (95 mg, 0.216 mmol). ESI-MS [M + Na]⁺: 333.1.

### Intermediate 207: rac-(1S*,2S*)-2-(5-chloro-2-nitrophenyl)cyclopropane-1-carboxamide

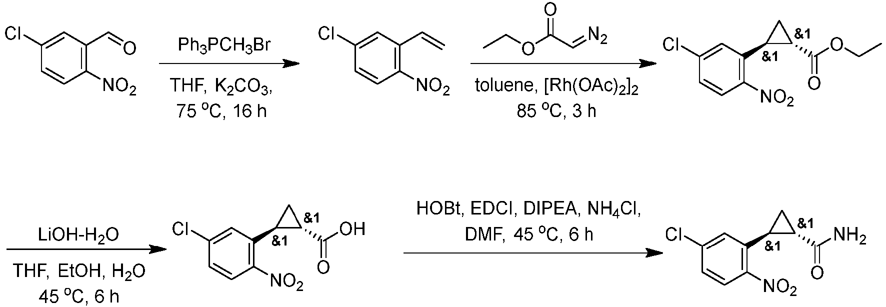

To a solution of 5-chloro-2-nitrobenzaldehyde (5.0 g, 27 mmol) in THF (100 mL), was added Ph₃PCH₃Br (11.0 g, 32 mmol) and K₂CO₃ (7.4 g, 54 mmol) at room temperature. The reaction mixture was stirred at 75°C for 16 h. Water (200 ml) was added and the mixture was extracted with EtOAc (3 x 100 mL). The combined organics were washed with brine (100 ml), dried over Na₂SO₄ and concentrated *in vacuo* to give the crude which was purified by silica gel chromatography (eluent: EtOAc : PE=1:50) to afford **4-chloro-1-nitro-2-vinylbenzene intermediate 208** (2.3 g, 46.5%) as a yellow oil. 1H NMR (400 MHz, DMSO) δ 8.02 (d, J = 8.7 Hz, 1H), 7.90 (d, J = 2.3 Hz, 1H), 7.64 - 7.61 (m, 1H), 7.02 - 6.95 (m, 1H), 6.03 (d, J = 17.3 Hz, 1H), 5.58 (d, J = 11.1 Hz, 1H).

Ethyl 2-diazoacetate (2.8 g, 24.6 mmol) was added slowly to a mixture of **intermediate 208** (1.5 g, 8.2 mmol and [Rh(OAc)₂]₂ (73 mg, 0.164 mmol) in toluene (30 ml) at 85°C. The reaction mixture was stirred at the same temperature for 3h. After cooling to room temperature, the mixture was concentrated *in vacuo* to give the crude which was purified by silica gel chromatography (eluent: EtOAc : PE = 1:40) to **afford *rac*-ethyl (1S*,2S*)-2-(5-chloro-2-nitrophenyl)cyclopropane-1-carboxylate intermediate 209** (700 mg, 32%) as a yellow oil.
ESI-MS: [M + H]+, 270.1

Using a similar procedure to that for **intermediate 165 intermediate 207** (400 mg, 64% in 2 steps) was synthesised from **intermediate 209** (700 mg, 2.6 mmol). ESI-MS: [M + H]+, 241.1

### Intermediate 210 Rac-methyl (1S*,2R*)-5'-chloro-2'-oxospiro[cyclopropane-1,3'-indoline]-2-carboxylate and Intermediate 211rac-methyl (1S*,2S*)-5'-chloro-2'-oxospiro[cyclopropane-1,3'-indoline]-2-carboxylate

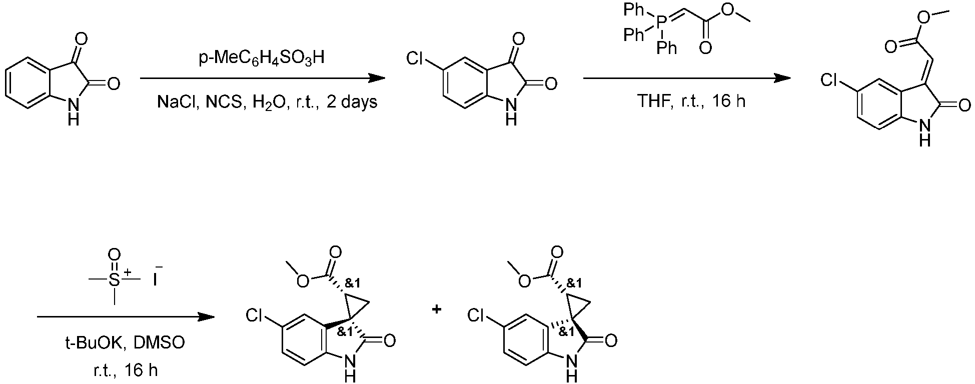

A mixture of indoline-2,3-dione (3.5 g, 24 mmol), p-MeC₆H₄SO₃H (6.8 g, 36 mmol), NaCl (2.8 g, 48 mmol) and NCS (4.8 g, 36 mmol) in water (50 mL) was stirred at room temperature for 2 days. The precipitates were collected by filtration and dried in an oven to give **5-chloroindoline-2,3-dione intermediate 212** (4 g, 93%) as an orange solid. ESI-MS [M +H]+: 182.1.

To a solution of 5-chloroindoline-2,3-dione (4 g, 22 mmol) in THF (20 mL) was added **intermediate 212** (8 g, 24 mmol). The reaction mixture was stirred at room temperature for 16 h. then concentrated *in vacuo.* The resulting residue purified by silica gel chromatography (eluent: PE/EtOAc = 2/1) to give **methyl (E)-2-(5-chloro-2-oxoindolin-3-ylidene)acetate intermediate 213** (1.7 g, 33%) as an orange solid . ESI-MS : [M+H]⁺: 238.1.

A mixture of trimethylsulfoxonium iodide (2.3 g, 10.5 mmol) and t-BuOK (10.5 mL, 10.5 mmol, 1M in THF) in DMSO (5 mL) was stirred at room temperature for 1 h under N₂. Then a solution of **intermediate 213** (1.7 g, 7 mmol) in DMSO/THF (5 mL/10 mL) was added. The reaction mixture was stirred at room temperature for 16 h then acidified to pH 7 with HCl (1 M, aq.). The mixture was extracted with EtOAc (3 x 50 mL). The combined organic layers were washed with brine (2 x 20 mL), dried over Na₂SO₄, concentrated *in vacuo* to give the crude, which was purified by column chromatography (eluent: DCM/MeOH = 20/1) to give **intermediate 210** (isomer 1: 250 mg, 14%) and **intermediate 211** (isomer 2: 300 mg, 17%) as yellow solids. ESI-MS [M +H]+: 252.0.

### Intermediate 214 rac-(1S*,2S*)-2-(3-chloro-2-nitrophenyl)cyclopropane-1-carboxamide

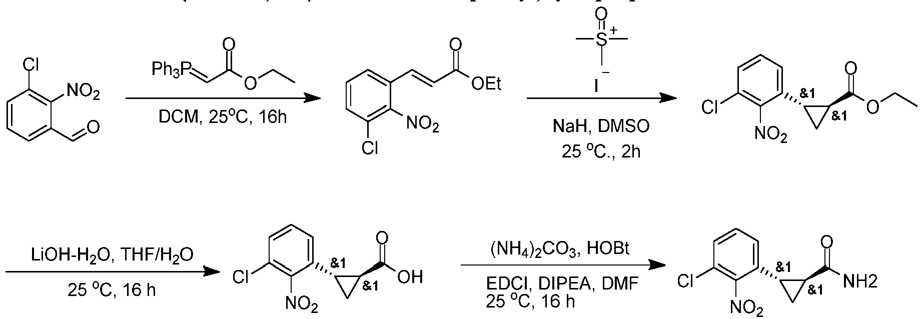

A mixture of 3-chloro-2-nitrobenzaldehyde (2 g, 10.7 mmol) and ethyl 2-(triphenyl-15-phosphanylidene)acetate (4.5 g, 12.9 mmol) in DCM (80 mL ) was stirred at 25°C for 16 h under N₂. The mixture concentrated to give a crude product which was purified by flash column chromatography (eluent: PE : EtOAc = 0 to 100%) to give **ethyl (E)-3-(3-chloro-2-nitrophenyl)acrylate intermediate 215** (1.5 g, 55% yield) as an off-white solid. ESI-MS [M +H]+: 256.0

To a mixture of NaH (410 mg, 10 mmol) in DMSO (10 mL) was added trimethylsulfoxonium iodide (2.01 g, 9 mmol) slowly at 25°C under N₂. The reaction mixture was then stirred at 25°C for 0.5 h until a clear solution was obtained. **intermediate 215** (1.3 g, 5 mmol) in DMSO (5 mL) was added and the mixture was stirred at 25°C for 2 h. The mixture was quenched with saturated NH₄Cl solution (150 mL) and extracted with EtOAc (3 x 50 mL). The organic layers were washed with water (30 mL), brine (30 mL), dried over Na₂SO₄ and concentrated *in vacuo.* The crude product was purified by silica gel column chromatography (eluent: MeOH /DCM from 0 to 10%) to give ***rac*-ethyl (1S*,2S*)-2-(3-chloro-2-nitrophenyl)cyclopropane-1-carboxylate intermediate 216** (300 mg, 22% yield) as a yellow oil. ESI-MS [M +H]+: 270.1

**Intermediate 214** was synthesized using a similar procedure to that for intermediate 23, **intermediate 214** (225 mg, 85% yield) was synthesised from **intermediate 216**(300 mg, 1.11 mmol). ESI-MS [M +H]+: 241.1

### Intermediate 217: rac-(1S*,2S*)-2-(2-cyano-5-methoxyphenyl)cyclopropane-1-carboxamide

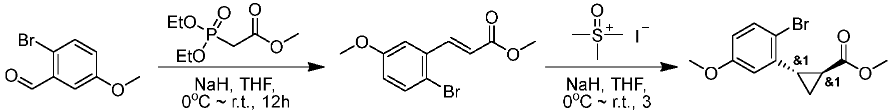

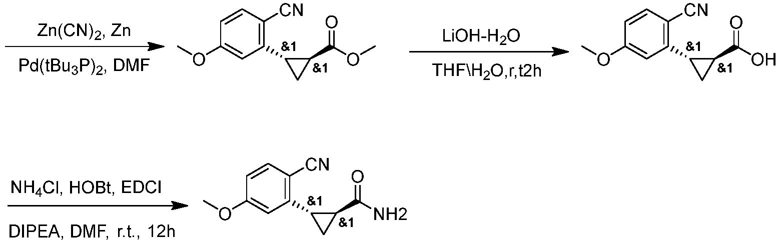

To a solution of methyl 2-(diethoxyphosphoryl)acetate (1.1 mL, 5.75 mmol) in THF (10 mL) was added NaH (220 mg, 5.5 mmol) at 0°C. The mixture was stirred for 5 min then 2-bromo-5-methoxybenzaldehyde (1.07 g, 5.0 mmol) in THF (10 mL) was added dropwise. The mixture was stirred at room temperature for 12h. Water (50 mL) was added and the mixture extracted with EtOAc (3 x 40 mL). The combined organic layers were washed with brine (40 mL), dried over Na₂SO₄ and concentrated *in vacuo* to give the crude, which was purified by silica gel chromatography (eluent: PE/EtOAc= 10/1) to give **methyl (E)-3-(2-bromo-5-methoxyphenyl)acrylate intermediate 218(1.2** g, 89%) as a white solid. ESI-MS [M +H]+: 271.1.

NaH (320 mg,13.3 mmol) was added to a solution of trimethylsulfoxonium iodide (2.93 g, 13.3 mmol) in DMSO (50 mL). The mixture was stirred at room temperature for 2h. Methyl ( **intermediate 218** (2.0 g, 7.4 mmol) in DMSO (30mL) was added to the mixture and stirring was continued at room temperature for 2h. Water (300 mL) was added and the mixture was extracted with EtOAc (3 x 100 mL). The combined organic layers were washed with brine (3 x 50 mL), dried over Na₂SO₄ and concentrated to give the crude, which was purified by silica gel chromatography (eluent: PE / EtOAc = 30/1) to **give *rac*-methyl (1S*,2S*)-2-(2-bromo-5-methoxyphenyl)cyclopropane-1-carboxylate intermediate 219** (1.1 g, 52%) as a yellow solid. ESI-MS [M +H]+: 285.2.

To a mixture of **intermediate 219** (330 mg, 1.11 mmol), Zn(CN)₂ (129.8 mg, 1.11 mmol) and Zn (7.2 mg, 0.111 mmol) in DMF (5 mL) was added Pd(t-BuP)₂ (56.7 mg, 0.111 mmol). The resulting reaction mixture was stirred at 80°C for 4h under N₂ then cooled to room temperature. Water (60 mL) was added and the mixture was extracted with EtOAc (3 x 30 mL). The combined organic layers were washed with brine (40 mL), dried over Na2SO4 and concentrated *in vacuo* to give the crude, which was purified by silica gel chromatography (eluent: DCM/MeOH = 50/1) to give ***rac-*methyl (1S*,2S*)-2-(2-cyano-5-methoxyphenyl)cyclopropane-1-carboxylate intermediate 220** (125 mg, 51%) as a yellow solid. ESI-MS [M +H]+: 232.2.

**Intermediate 217** (300mg, 85.7%) was syntheized using a similar procedure to that for **intermediate 165,** from **intermediate 220** (500 mg, 2.16 mmol). ESI-MS [M +H]+: 217.2.

### Intermediate 221: Rac-(1S*,2S*)-2-(5-chloro-1H-indazol-3-yl)cyclopropane-1-carboxamide

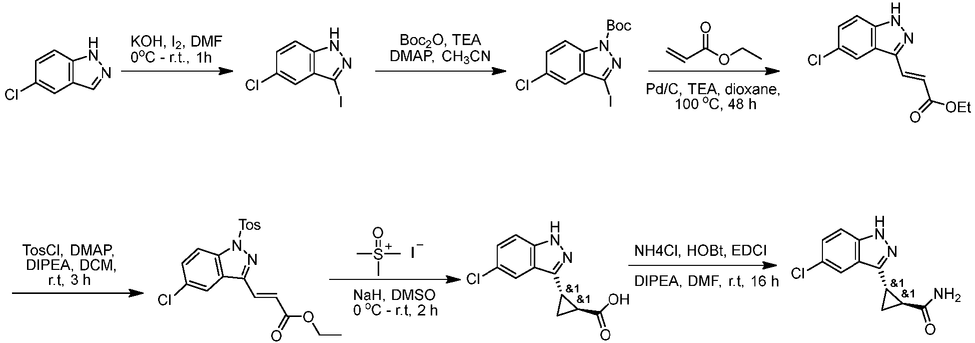

To a mixture of 5-chloro-1H-indazole (5.2 g, 34 mmol) in dry DMF (50 mL) was added KOH (7.6 g,136 mmol) and **I₂** (17.2 g, 68 mmol) at 0°C under N₂. The mixture was stirred for 1 h at 0°C and then at room temperature for another 1 h. The reaction was quenched with water (500 mL) and extracted with EtOAc (3 x 300 mL). The organic layers were washed with brine (200 ml), dried over Na₂SO₄ and concentrated *in vacuo* to give crude **5-chloro-3-iodo-1H-indazole intermediate 222** (9.0 g, crude) which used in the next step without further purification. ESI-MS [M +H]⁺: 279.9.

To a mixture of **intermediate 222** (6.4 g, crude) in dry MeCN (80 mL) was added TEA (4.6 g, 46 mmol), DMAP (280 mg, 2.3 mmol) and Boc₂O (5.9 g, 27.6 mmol). The reaction was stirred at room temperature for 16 h under N₂ then poured into water (200 mL) and extracted with EtOAc (3 x 100 mL). The combined organics were washed with brine (100 mL), dried over Na₂SO₄ and concentrated *in vacuo.* The residue was purified by silica gel column (eluent: EtOAc/ PE= 10%) to give **tert-butyl 5-chloro-3-iodo-1H-indazole-1-carboxylate intermediate 223** (8.2 g, 94%) as white solid. ESI-MS [M - 55]+: 322.9.

To a solution of **intermediate 223** (5 g, 13.2 mmol) in dry 1,4-dioxane (250 mL) were added ethyl acrylate (26 g, 264 mmol), Pd/C (2.75 g, 2.6 mmol) and TEA (26 g, 264 mmol). The mixture was stirred at 100°C for 48 h under N₂, cooled to room temperature, filtered with through Celite^{®} and washed with EtOAc (2 x 50 mL). The organic layers were concentrated and purified by chromatography (eluent: EtOAc/ PE= 15%) to give **ethyl (E)-3-(5-chloro-1H-indazol-3-yl)acrylate intermediate 224** (2 g, 61%) as a yellow solid. ESI-MS [M +H] ⁺: 251.1.

To a solution of **intermediate 224** (2.2 g, 8.8 mmol) in dry DCM (100 mL) was added TsCl (2.5 g, 13.2 mmol), DMAP (322 mg, 2.6 mmol) and DIPEA (3.4 g, 26.4 mmol). After stirring for 3 h under N₂, the reaction mixture was poured into water (100 mL) and extracted with DCM (3 x 100 ml). The combined organics were washed with brine (100 mL), dried over Na₂SO₄ and concentrated *in vacuo.* The residue was purified by silica gel chromatography (eluent: EtOAc/ PE= 10%) to give **ethyl (E)-3-(5-chloro-1-tosyl-1H-indazol-3-yl)acrylate intermediate 225** (1.7 g, 48%) as a white solid. ESI-MS [M +H] ⁺: 405.0.

To a solution of NaH (504 mg, 12.6 mmol) in dry DMSO (20 mL) was added trimethylsulfoxonium iodide (2.7 g, 12.6 mmol) in DMSO (20 mL). The mixture was stirred at 0°C for 0.5 h then **intermediate 225** (1.7 g, 4.2 mmol) in dry DMSO (10 mL) was added. After stirring at room temperature for 2 h under N₂, the mixture was poured into water (200 mL) and extracted with EtOAc (2 x 100 mL). The water layers were adjusted pH to 5 with HCl (5 ml, 1M, aq.), then extracted with EtOAc (2 x 100 mL). The combined organic layers were dried over Na₂SO₄ and concentrated *in vacuo* and purified by column (DCM / MeOH = 10 / 1) to give ***rac*-(1S*,2S*)-2-(5-chloro-1H-indazol-3-yl)cyclopropane-1-carboxylic acid intermediate 226** (500 mg, 50%) as a white solid. ESI-MS [M +H] +: 237.1.

To a solution of **intermediate 226** (500 mg, 2.1 mmol) in dry DMF (10 mL) was added NH₄Cl (1.1 g, 21 mmol), HOBt (368 mg, 2.7 mmol), EDCI (515 mg, 2.7 mmol) and DIPEA (812 mg, 6.3 mmol). The reaction was stirred at room temperature for 16 h under N₂. Water (100 ml) was added and extracted with EtOAc (3 x 50 mL). The organic layers were washed with brine (50 mL), dried over Na₂SO₄ and concentrated *in vacuo.* The residue was concentrated and purified by silica gel chromatography (eluent: DCM / MeOH = 10:1 ) to give **intermediate 221** (200 mg, 40%) as a white solid. ESI-MS [M +H] ⁺: 236.1.

### Intermediate 227: ethyl (1R*,2R*,3S*)-2-(3-chlorophenyl)-3-methylcyclopropane-1-carboxylate

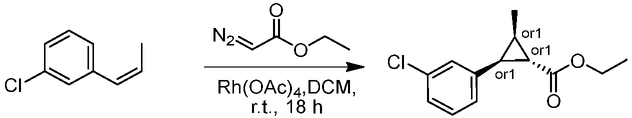

To a mixture of (Z)-1-chloro-3-(prop-1-en-1-yl)benzene ( 1.52 g, 10.0 mmol) and Rh(OAc)₄ ( 221 mg, 0.5 mmol) in DCM (30 mL) was added ethyl 2-diazoacetate ( 2.28 g, 20.0 mmol) dropwise. After stirring at room temperature for 18 h, the reaction mixture was diluted with water (100 mL) and extracted with DCM (3 x 50 mL). The combined organic layers were washed with brine (50 mL), dried over Na₂SO₄ and concentrated *in vacuo.* The residue was purified by silica gel chromatography (eluent: DCM / PE from 0 to 100%) to afford **intermediate 227** (isomer 1, 650 mg, 25%; isomer 2, 500 mg, 21%; isomer 3, 400 mg, 17%; isomer 4, 80 mg, 3.4%) as light-yellow oils.

### Intermediate 228: rac-(1S*2S*)-2-(5-chloropyridin-3-yl)cyclopropane-1-carboxamide

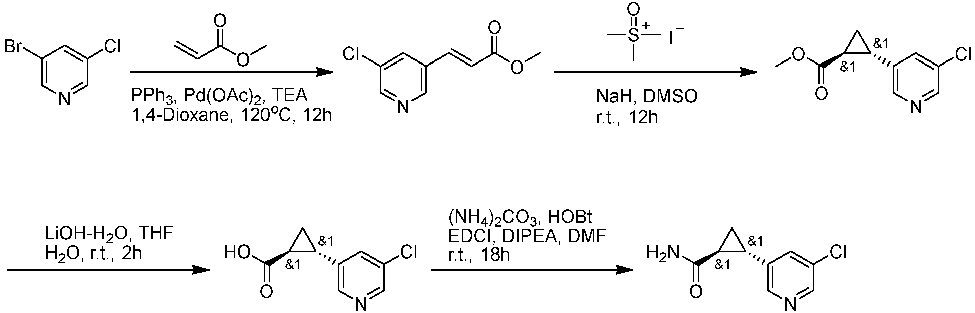

A mixture of 3-bromo-5-chloropyridine (2 g, 10.4 mmol), methyl acrylate (4.7 g, 52.0 mmol) , PPh₃ (545mg, 2.08mmol), Pd(OAc)₂ (466 mg, 2.08 mmol), TEA (5.25 g, 52.0 mmol) and 1,4-dioxane (20 mL) was stirred at 120°C or 12h. The reaction was diluted with water (30 mL) then extracted with EtOAc (3 x 40 mL). The combined organic layers were washed with brine, dried over Na₂SO₄, filtered and concentrated *in vacuo* to give the crude, which was purified by silica gel chromatography (eluent: DCM\MeOH=100\1) to give **methyl (E)-3-(5-chloropyridin-3-yl)acrylate** (500 mg, 25%) as a white solid. ESI-MS [M +H]+: 198.2.

To a solution of trimethylsulfoxonium iodide (600 mg, 91.3 mmol) in DMSO (20 mL) was added NaH (220 mg, 60% dispersion in mineral oil, 91.3 mmol. The reaction mixture was stirred at room temperature for 1h and then a solution of methyl (E)-3-(5-chloropyridin-3-yl)acrylate (600 mg, 30.3 mmol) in DMSO (30 mL) was added. The reaction mixture was stirred at room temperature for 12h. The reaction was quenched with water (60 mL) and extracted with EtOAc (3 x 60 mL). The combined organic layers were washed with brine, dried over Na₂SO₄, filtered and concentrated *in vacuo* to give the crude, which was purified by silica gel chromatography (eluent: PE\EA= 4\1) to give ***rac-*methyl *(1S*,2S*)*-2-(5-chloropyridin-3-yl)cyclopropane-1-carboxylate intermediate 229** (220 mg, 34%) as a white solid. ESI-MS [M +H]+: 212.1

Using a similar procedure to that for **intermediate 23, intermediate 228** (130 mg, 65%) was synthesised from **intermediate 229** (220 mg, 1.11 mmol). ESI-MS [M +H]+: 197.1.

### Intermediate 230: rac-(1S*,2S*)-2-(2-chloropyridin-4-yl)cyclopropane-1-carboxamide

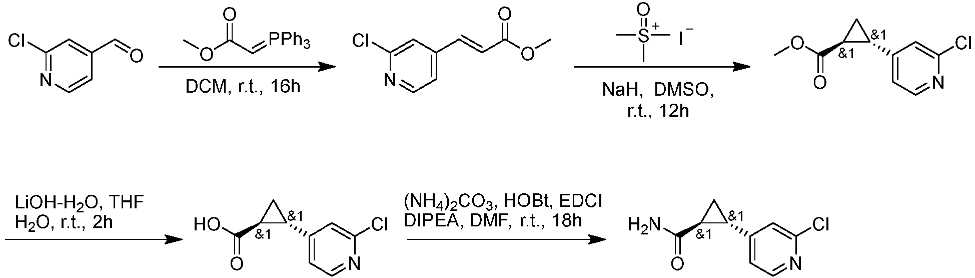

A mixture of 2-chloroisonicotinaldehyde (2 g, 14.0 mmol), methyl 2-(triphenyl-15-phosphanylidene)acetate (5.67 g, 16.96 mmol) and DCM (40 mL) was stirred at room temperature for 16h. The reaction was concentrated *in vacuo* to give the crude, which was purified by silica gel chromatography (eluent: PE \ EtOAc = 10\1) to give **methyl (E)-3-(2-chloropyridin-4-yl)acrylate intermediate 231** (1.8 g, 64%) as a White solid. ESI-MS [M +H]+: 198.2.

To a solution of Trimethylsulfoxonium iodide (600 mg, 91.3 mmol) in DMSO (20 mL) was added NaH (220mg, 60% dispersion in mineral oil, 91.3mmol) . After stirring the resulting mixture at room temperature for 1h, a solution of methyl (E)-3-(5-chloropyridin-3-yl)acrylate (600 mg, 30.3 mmol) in DMSO (30 mL) was added and then stirred at room temperature for another 12h. The reaction was quenched with water (60 mL), extracted with EtOAc (3 x 60 mL). The combined organic layers were washed with brine, dried over Na₂SO₄, filtered and concentrated to give the crude, which was purified by silica gel chromatography (eluent: PE/EtOAc= 5/1) to ***rac*-methyl (1S*,2S*)-2-(2-chloropyridin-4-yl)cyclopropane-1-carboxylate intermediate 232** (220 mg, 34.3%) as a white solid. ESI-MS [M +H]+: 212.1

A mixture of **intermediate 232** (220 mg, 1.11 mmol), LiOH-H₂O (137 mg, 3.33 mmol) in THF/ H₂O (2 mL / 5mL) was stirred at room temperature for 2h. The reaction was concentrated, then the pH of the residue was adjusted to ~ 4 with HCl (1.0 N, aq.) and extracted with EA (3 x 30 mL). The combined organic layers were washed with brine, dried over Na₂SO₄, filtered and concentrated to give ***rac*-(1S*,2S*)-2-(2-chloropyridin-4-yl)cyclopropane-1-carboxylic acid intermediate 233** (200mg, crude) as a white solid, which was used into next step without further purification. ESI-MS [M +H]+: 198.2.

A mixture of **intermediate 233** (200 mg, crude), (NH₄)₂CO₃ (195 mg, 2.03 mmol), HOBt (205.5 mg, 1.52 mmol), EDCI (293.3 mg, 1.52 mmol), DIPEA (134.8 mg, 1.045 mmol) and DMF(3 mL) was stirred at room temperature for 12h. The reaction was washed with water (20 mL), extracted with EtOAc (3 x 30 mL). The combined organic layers were washed with brine, dried over Na₂SO₄, filtered and concentrated to give the crude, which was purified by silica gel chromatography (eluent: DCM/MeOH = 20/1) to give **intermediate 230** (130 mg, 65%) as a white solid. ESI-MS [M +H]+: 197.1.

### Intermediate: 234 rac-ethyl (1S*,2S*)-2-(5-methyl-1,2,4-thiadiazol-3-yl)cyclopropane-1-carboxylate

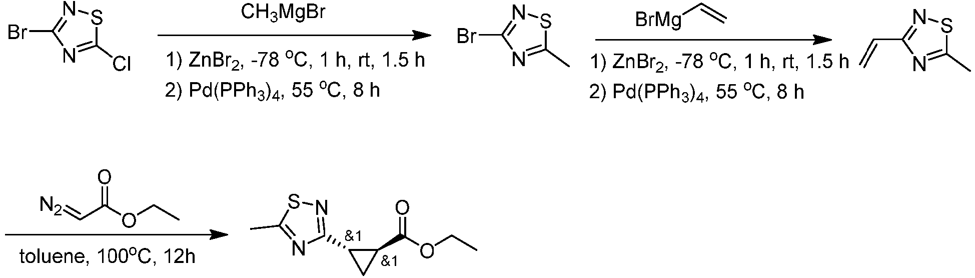

To a mixture of ZnBr₂ (3.26 g, 14mmoL) in THF (60 mL) and was added CH₃MgBr (4.7 mL, 3M solution in THF, 14 mmol) at -78°C for 1h. The resulting mixture was stirred at room temperature for 1h and then were added 3-bromo-5-chloro-1,2,4-thiadiazole (2 g, 10 mmol) and Pd(PPh₃)₄ (1.1 g, 1 mmol). After stirring at 55°C for 8h, the reaction was washed with water (50 mL) and extracted with EtOAc (3 x 50 mL). The combined organic layers were washed with brine (50 mL), dried over Na₂SO₄, concentrated *in vacuo* to give the crude, which was purified with silica gel column chromatography (eluent: PE/EtOAc = 5/1) to give **3-bromo-5-methyl-1,2,4-thiadiazole intermediate 235** (550 mg, 31%) as colorless oil. ESI-MS [M +H]+: 199.0.

To a mixture of ZnBr₂ (250 mg, 1.96 mmol) in THF (30 mL) and was added CH₃MgBr (0.65 mL, 1.96 mmol, 3M solution in THF) at -78°C for 1h. The resulting mixture was stirred at room temperature for 1h and then **intermediate 235** (250 mg, 1.6 mmol) and Pd(PPh₃)₄ (185 mg, 0.16 mmol) were added. After stirring at 55°C for 8h, the reaction was washed with saturated aqueous NH₄Cl (30 mL), extracted with EtOAc (3 x 30 mL). The combined organic layers were washed with brine (30 mL), dried over Na₂SO₄ then concentrated *in vacuo* to give the crude, which was purified preparative TLC (eluent: PE/EtOAc = 5/1) to give **5-methyl-3-vinyl-1,2,4-thiadiazole intermediate 236** (120 mg, 60%) as a yellow oil. ESI-MS [M +H]+: 127.1.

To a solution of **intermediate 236** (120 mg, 0.95 mmol) in toluene (5 mL) was added ethyl 2-diazoacetate (760 mg, 6.66 mmol). The resulting reaction mixture was stirred at 100°C for 12 h. The reaction was cooled to room temperature and then concentrated *in vacuo* to give the crude, which was purified by silica gel chromatography (eluent: PE/EtOAc = 5/1) to give **intermediate 234** (60 mg, 30%) as a yellow oil. ESI-MS [M +H]+: 213.2.

### Intermediate 237: rac-ethyl (1S*,2S*)-1-fluoro-2-(4-methylpyrimidin-2-yl)cyclopropane-1-carboxylate

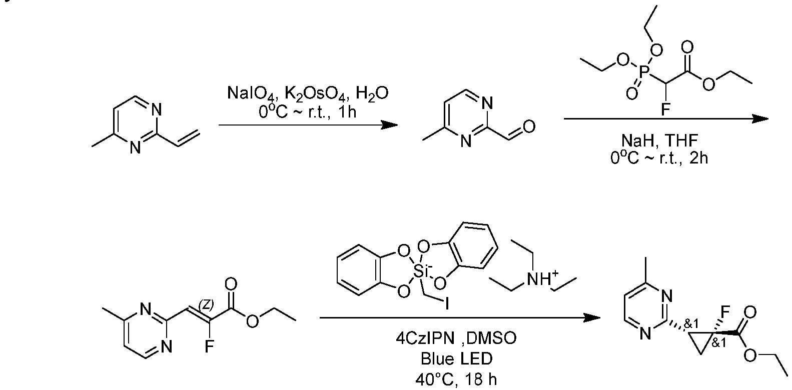

A mixture of **intermediate 56** (2 g, 16.7 mmol), NaIO₄(14.2 g, 66.8 mmol) and K₂OsO₄ (564.4 mg, 1.7 mmol) in THF (80 mL) and water (20 mL) was stirred at room temperature for 1 h. The reaction was diluted water (60 mL), extracted with EtOAc (3 x 60 mL). The combined organic layers were washed with brine (60 mL), dried over Na₂SO₄, filtered and concentrated *in vacuo* to give the crude, which was purified silica gel column chromatography (eluent: PE/EA = 20/1-10/1) to give **4-methylpyrimidine-2-carbaldehyde intermediate 238** (300 mg, 15%) as a yellow oil. ESI-MS [M +H]+:123.2.

To a solution of ethyl 2-(diethoxyphosphoryl)-2-fluoroacetate (895 mg, 3.7 mmol) in THF (30 mL) was added NaH (180 mg, 4.5 mmol, 60% dispersion in mineral oil) at 0°C. The reaction mixture was stirred for 30min and then a solution of **intermediate 238** (300 mg, 2.5 mmol) in THF (5 mL) was added. After stirring at room temperature for another 2h, the reaction was quenched with saturated aqueous (50 mL) and extracted with EtOAc (3 x 50 mL). The combined organic layers were washed with brine (50 mL), dried over Na₂SO₄ and concentrated *in vacuo* to give the crude, which was purified with silica gel column chromatography (eluent: PE/EA = 10/1-5/1) to give **ethyl (Z)-2-fluoro-3-(4-methylpyrimidin-2-yl)acrylate intermediate 239** (315 mg, 59%) as yellow oil. ESI-MS [M +H]+:211.2.

To a mixture of **intermediate 239** (300 mg, 1.43 mmol) in DMSO (5 mL) were added triethylammonium bis(catecholato)iodomethylsilicate (1 g, 2.14 mmol) and 4CzIPN (54 mg, 0.07 mmol) . The resulting solution was degassed with N₂ and placed in front of two blue LEDs (Kessil, H150, 32W). The reaction was stirred for 18 h. The reaction was diluted with water (30 mL) and extracted with EtOAc (3 x 50 mL). The combined organic layers were washed with brine (50 mL), dried over Na₂SO₄ then concentrated *in vacuo* to give the crude, which was purified with preparative TLC (PE/EA=2/1 ) to give **intermediate 237** as yellow oil. ESI-MS [M +H]+: 224.2.

### Intermediate 240: (1S,2S)-2-(3-((trimethylsilyl)ethynyl)phenyl)cyclopropane-1-carboxamide

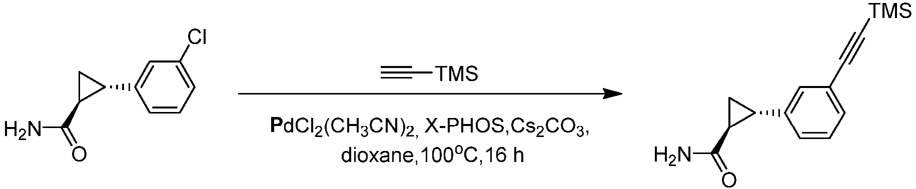

To a solution of **intermediate 119** (255 mg, 1.30 mmol) in 1,4-Dioxane(5 mL) were added ethynyltrimethylsilane (384 mg, 3.9 mmol), X-PHOS (31 mg, 0.06 mmol), Cs₂CO₃ (937 mg, 2.86 mmol) and PdCl₂(CH₃CN)₂ (17 mg, 0.06 mmol). After stirring at 100°C for 16h, the reaction was cooled to room temperature, diluted with water (50 mL) then extracted with EtOAc (3 x 50 mL). The combined organic layers were washed with brine (40 mL), dried over Na₂SO₄, filtered and concentrated *in vacuo* to give the crude, which was purified by silica gel chromatography (MeOH/ DCM = 1/20) to give **intermediate 240** (180 mg, 53.4%) as a yellow solid. ESI-MS [M +H]+: 258.1.

### Intermediate 241: rac-(1S*, 2S*)-2-(7-chloro-8-fluoroimidazo[1,5-a]pyridin-1-yl)cyclopropanecarboxylic acid

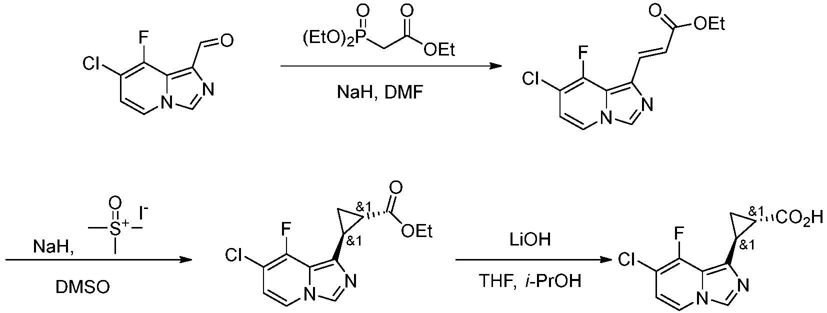

To a solution of ethyl 2-(diethoxyphosphoryl)acetate (6.8 g, 30.3 mmol) in dry DMF (100 mL) cooled at 0°C under N₂ was added NaH (60%, 910 mg, 37.88 mmol) in portions. The reaction mixture was stirred at 0°C for 1 h before 7-chloro-8-fluoroimidazo[1,5-a]pyridine-1-carbaldehyde (5 g, 25.25 mmol) was added dropwise. The resulting mixture was allowed to warm to room temperature and stirred overnight. Once the reaction was complete by TLC, the reaction was quenched with saturated NH₄Cl solution and extracted with ethyl acetate (50 mL×3). The combined organic layers were washed with brine, dried over anhydrous sodium sulphate, filtered, and concentrated *in vacuo.* The residue was purified by silica gel column chromatography (petroleum ether: ethyl acetate = 3:1) to afford **ethyl 3-(7-chloro-8-fluoroimidazo[1,5-a]pyridin-1-yl)acrylate intermediate 242** (3.2 g, 47%) as a yellow solid. ESI-MS [M+H]⁺ 269.1

To a solution of trimethylsulfoxonium iodide (5.25 g, 23.88 mmol) in dry DMSO (100 mL) was added NaH (60%, 860 mg, 35.82 mmol) in portions at 0°C. The reaction mixture was stirred at this temperature for 30 min before **intermediate 242** (3.2 g, 11.94 mmol) was added. The resulting mixture was stirred at room temperature overnight then quenched with saturated NH₄Cl solution, extracted with **ethyl acetate** (50 mL×3). The combined organic layers were washed with brine, dried over sodium sulfate, filtrated, concentrated *in vacuo* and purified by chromatography (petrol ether : ethyl acetate = 3:1) to give ***rac-(1S*,* 2S*)-ethyl 2-(7-chloro-8-fluoroimidazo[1,5-a]pyridin-1-yl)cyclopropanecarboxylate intermediate 243** (440 mg, 13%) as a yellow solid. ESI-MS [M+H]⁺ 283.1

To a solution of **intermediate 243** (440 mg, 1.56 mmol) in THF (10 mL), iPrOH (10 mL) and water (5 mL) was added lithium hydroxide hydrate (197 mg, 4.68 mmol). The resulting reaction mixture was stirred at room temperature for 4 hrs. After evaporation, the residue was diluted with water (5 mL) and acidified with 1 N HCl to pH~5. The precipitate was filtered and dried under vacuum to give **intermediate 241** (280 mg, 71%) as a white solid. ESI-MS 255.0 [M+H]⁺, ¹H NMR (400 MHz, DMSO) δ 8.37 (d, *J* = 2.4 Hz, 1H), 8.16 (d, *J* = 7.2 Hz, 1H), 6.72 (t, *J* = 6.8 Hz, 1H), 2.66-2.64 (m, 1H), 1.97-1.93 (m, 1H), 1.51-1.43 (m, 2H).

### Intermediate 244: rac-(1S*,2S*)-2-(3-bromophenyl)cyclopropanecarboxylic acid

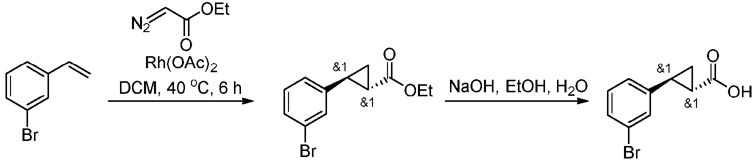

To a solution of 1-bromo-3-vinylbenzene (5 g, 27.32 mmol) in DCM (50 mL) was added Rh(OAc)₂ (765 mg, 2.73 mmol). The mixture was heated to 40°C and a solution of ethyl diazoacetate (15.7 g, 136.66 mmol) in DCM (300 mL) was added dropwise over 6 hrs. After the addition, the reaction mixture was cooled to room temperature and filtered through a short pad of Celite. The filtrate was concentrated *in vacuo* and purified by silica gel column chromatography (petroleum ether: ethyl acetate = 20:1) to afford ***rac-(1S*,2S*)*-ethyl 2-(3-bromophenyl)cyclopropanecarboxylate intermediate 245** (3.9 g, 53%) as a colorless oil. ESI-MS [M+H]⁺ 269.0

To a solution of *trans*-ethyl 2-(3-bromophenyl)cyclopropanecarboxylate **intermediate 245?** (1.8 g, 6.7 mmol) in EtOH (30 mL) was added sodium hydroxide (550 mg, 13.8 mmol). After stirring at room temperature for 1 hr, the mixture was cooled to 0°C, and HCl (1M, 10 mL) was added. The suspension was filtered and the filter cake was washed with water and dried under vacuum to afford **intermediate 244** (1.4 g, 88%) as a white solid. ESI-MS [M+H]⁺242.8, ¹H NMR (400 MHz, CDCl₃): δ 7.35 (d, *J* = 7.6 Hz, 1H), 7.25 (s, 1H), 7.15 (d, *J =* 8.0 Hz, 1H), 7.04 (d, *J =* 7.6 Hz, 1H), 2.59-2.54 (m, 1H), 1.92-1.82 (m, 1H), 1.69-1.64 (m, 1H), 1.42-1.36 (m,1H).

### Intermediate 246:rac-(1S*,2S*)-2-(3-cyclopropyiphenyl)cyclopropanecarboxylic acid

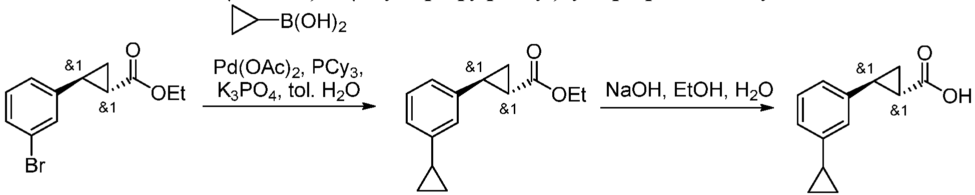

To a solution of *trans*-ethyl 2-(3-bromophenyl)cyclopropanecarboxylate (2.0 g, 7.5 mmol) in tol/H₂O (40 mL/8 mL), was added cyclopropylboronic acid (9.7 g, 113 mmol), Pd(OAc)₂ (840 mg, 3.75 mmol), tricyclohexyl phosphine (PCy₃, 2.1 g, 7.5 mmol and K₃PO₄ (4.8 g, 22.5 mmol). The mixture was stirred at 100°C under N₂ overnight. Once the reaction was complete by TLC, the mixture was cooled to room temperature and quenched with water then extracted with ethyl acetate. The combined organic layers were washed with brine, dried and concentrated *in vacuo.* The residue was purified by silica gel column chromatography (petroleum ether: ethyl acetate = 20:1) to afford ***rac-(1S*,2S*)*-ethyl 2-(3-cyclopropylphenyl)cyclopropanecarboxylate intermediate 247** (1.3 g, 87%) as a yellow oil.

To a solution of **intermediate 247** (1.3 g, 5.65 mmol) in ethanol (10 mL) and water (4 mL) was added sodium hydroxide (452 mg, 11.3 mmol). After the mixture was stirred at room temperature for 1.5 hrs, the reaction was complete by TLC. The mixture was concentrated *in vacuo.* The residue was diluted with water and then acidified with 1M HCl to pH = 5. The mixture was extracted with ethyl acetate and the combined organic layers were washed with brine then concentrated *in vacuo* to afford **intermediate 246** (1.05 g, 92%) as a yellow solid. ESI-MS [M-H]⁻201.0, ¹H NMR (400 MHz, DMSO) δ 12.27 (s, 1H), 7.13 (t, *J* =7.6 Hz, 1H), 6.90-6.87 (m, 3H), 2.36-2.32 (m, 1H), 1.89-1.77 (m, 2H), 1.42-1.38 (m, 1H), 1.34-1.30 (m, 1H), 0.93-0.88 (m, 2H), 0.68-0.64 (m, 2H).

### Intermediate 248: rac-(1S*,2S*)-2-(3-methoxyphenyl)cyclopropanecarboxylic acid

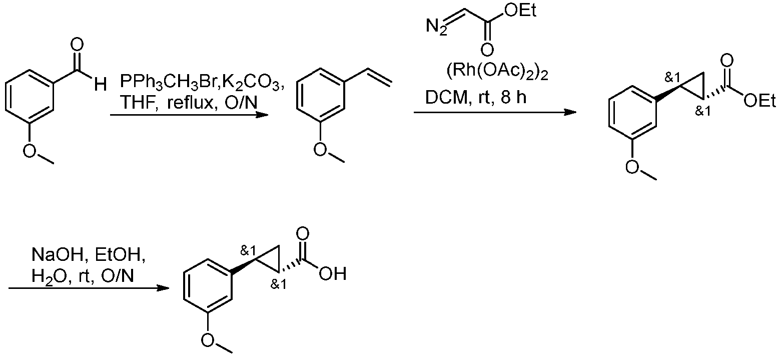

3-methoxybenzaldehyde (20 g, 147 mmol) was added to a stirred suspension of potassium carbonate (36.5 g, 264.6 mmol) and methyltriphenylphonium bromide (78.7 g, 220.5 mmol) in anhydrous THF (300mL) under nitrogen. The reaction was stirred at reflux overnight. After cooling to room temperature, the mixture was filtered, and the filtrate was concentrated *in vacuo.* The residue was purified by silica gel column chromatography (petroleum ether) to afford **1-methoxy-3-vinylbenzene intermediate 249** (15.6 g, 79%) as a colourless oil. ESI-MS [M+H]⁺135.1

To a suspension of **intermediate 249** (9.6 g, 71.6 mmol) and diacetoxyrhodium (2.5 g, 5.72 mmol) in DCM (100mL) was added ethyl diazoacetate (20 g, 173.9 mmol) in DCM (150 mL) dropwise over 8 hours. After the addition, the reaction mixture was filtered, and the filtrate was concentrated *in vacuo.* The residue was purified by silica gel column chromatography (petroleum ether: ethyl acetate = 20:1) to afford ***rac-(1S*,2S*)--*ethyl 2-(3-methoxyphenyl)cyclopropanecarboxylate intermediate 250** (5.8 g, 37%) as a colorless oil.

To a solution of **intermediate 250** (5.8 g, 26.3 mmol) in ethanol (40 mL) and water (10 mL) was added sodium hydroxide (3.1 g, 71.9 mmol). The mixture was stirred at room temperature overnight. Once the reaction was complete by LCMS, the mixture was concentrated *in vacuo.* The residue was diluted with water (20 mL) and then acidified with 1M HCl to *p*H = 1-2. The resulting precipitate was collected by filtration and dried under vacuum to afford **intermediate 248** (4.26 g, 85%) as a white solid. ESI-MS [2M-H]⁻ 382.9, 1H NMR (400 MHz, CDCl₃) δ 7.20 (t, J = 8.0 Hz, 1H), 6.75 (d, J = 8.8 Hz, 1H), 6.69 (d, J = 7.6 Hz, 1H), 6.65 (s, 1H), 3.80 (s, 3H), 2.60 - 2.55 (m, 1H), 1.94 - 1.86 (m, 1H), 1.68 - 1.62 (m, 1H), 1.45 - 1.35 (m, 1H).

### Intermediate 251: (6-cyclopropylimidazo[1,2-a]pyridin-2-yl)methanamine

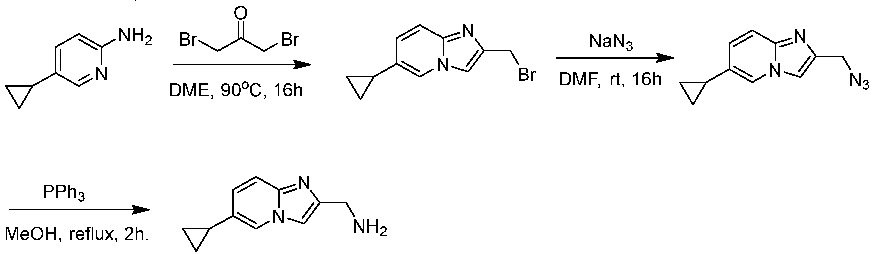

A mixture of 5-cyclopropylpyridin-2-amine (670 mg, 5.0 mmol) and 1,3-dibromopropan-2-one (1.61 g, 7.5 mmol) in DME (20.0 mL) was stirred at 90°C under N₂ for 16 h. The reaction mixture was cooled to room temperature, quenched with sat. NaHCO₃ solution (50 mL) and extracted with EtOAc (3 x 50 ml). The combined organic layers were washed with brine (50 mL), dried over Na₂SO₄ and concentrated *in vacuo.* The crude residue was purified by column chromatography (eluent: DCM/MeOH=50/1 ~ 30/1) to give the product **2-(bromomethyl)-6-cyclopropylimidazo[1,2-a]pyridine intermediate 252** (880 mg, 70 %) as a yellow solid. ESI-MS [M +H]⁺: 252.2.

### Synthesis of 2-(azidomethyl)-6-cyclopropylimidazo[1,2-a]pyridine

A mixture of **intermediate 252** (753 mg, 3 mmol) and NaN₃ (244 mg, 3.75 mmol) in DMF (10.0 mL) was stirred at room temperature under N₂ for 16 h. The mixture was diluted with EtOAc (100 mL) and washed with brine (3 x 50 mL). The organic layers were dried over Na₂SO₄ and concentrated *in vacuo* to give 2-(azidomethyl)-6-cyclopropylimidazo[1,2-a]pyridine (730 mg, crude) as a yellow solid, which was used for the next step without purification. ESI-MS [M +H]⁺: 214.2.

A mixture of 2-(azidomethyl)-6-cyclopropylimidazo[1,2-a]pyridine (730 mg, crude) and PPh₃ (983 mg, 3.75 mmol) in MeOH (25 mL) was stirred at reflux for 2 h. The mixture was concentrated *in vacuo* and purified by preparative TLC (eluent: DCM/MeOH = 10/1) to give **intermediate 251** (450 mg, 81% over 2 steps ) as a yellow oil. ESI-MS [M +H]+: 188.2

### Intermediate 253: 2-((tert-butyldimethylsilyl)oxy)-N-((6-cyclopropylimidazo[1,2-a]pyridin-2-yl)methyl)ethan-1-amine

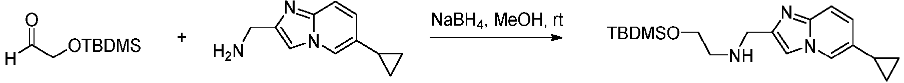

**Intermediate 251** (50 mg, 0.28 mmol) and 2-((tert-butyldimethylsilyl)oxy)acetaldehyde (76 µL, 0.40 mmol) were dissolved in MeOH (1.0 mL) and stirred at room temperature for 2 h. NaBH₄ (30 mg, 0.80 mmol) was then added and the reaction stirred at room temperature for a further 1 h. The reaction was then quenched with water (5 mL) and extracted into DCM (3 x 10 mL), the combined organics were dried over MgSO₄ then concentrated *in vacuo.* The residue was purified by silica gel chromatography, eluting with a gradient of 0-20 % (7N NH₃ in MeOH) in EtOAc to give **intermediate 253** (47 mg, 51 %). This was used directly without further purification.

### Intermediate 254: 1-(6-cyclopropylimidazo[1,2-a]pyridin-2-yl)-2,2,2-trifluoroethan-1-one

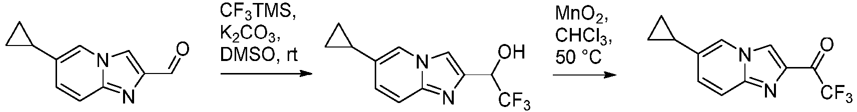

To a solution of 6-cyclopropylimidazo[1,2-a]pyridine-2-carbaldehyde (100 mg, 0.54 mmol) and trimethyl(trifluoromethyl)silane (99 mg, 0.70 mmol) in DMSO (2.0 mL) was added K₂CO₃ (7.4 mg, 0.05 mmol) and the reaction mixture was stirred at room temperature for 16 h. The mixture was poured onto ice water and extracted with ethyl acetate (3×20 mL). The organics were dried over MgSO₄ and concentrated *in vacuo* to give **1-(6-cyclopropylimidazo[1,2-a]pyridin-2-yl)-2,2,2-trifluoroethan-1-ol intermediate 255** (100 mg, 73 %) which was used without further purification. ESI-MS (M+H)+: 257.2, ¹H NMR (400 MHz, CDCl₃) δ 7.89 (s, 1H), 7.55 (s, 1H), 7.50 (d, J=9.5 Hz, 1H), 7.00 (dd, J=1.7, 9.3 Hz, 1H), 5.23 - 5.17 (m, 1H), 1.94 - 1.86 (m, 1H), 1.02 - 0.96 (m, 2H), 0.71 - 0.66 (m, 2H). OH not observed

A suspension of **intermediate 255** (400 mg, 1.6 mmol), manganese(IV) oxide (1.4 g, 16 mmol) in chloroform (15 mL) was stirred at 50°C for 16 h. The mixture was cooled to room temperature, filtered through Celite^{®}, washed with DCM (30 mL) and concentrated *in vacuo* to give **intermediate 254** (350 mg, 88 %) which was used without further purification. ESI-MS (M+H)+: 273.2

### Intermediate 256: (6-isopropylimidazo[1,2-a]pyridin-2-yl)methanamine

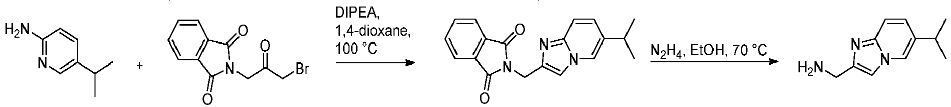

A solution of 5-isopropylpyridin-2-amine (500 mg, 3.7 mmol), **intermediate 311** (1100 mg, 4.0 mmol) and DIPEA (0.96 mL, 5.5 mmol) in 1,4-dioxane (37 mL) was stirred at 100°C for 16 h. The mixture was cooled to room temperature and concentrated *in vacuo.* The residue was purified by silica gel chromatography eluting with a gradient of 50-100 % EtOAc in cyclohexane to give **2-((6-Isopropylimidazo[1,2-a]pyridin-2-yl)methyl)isoindoline-1,3-dione intermediate 257** (480 mg, 41 %) as a red oil. ESI-MS (M+H)+: 320.1, 8.38 (1H, s), 8.03 - 7.94 (4H, m), 7.88 (1H, s), 7.48 (1H, d, J=9.3 Hz), 7.27 (1H, dd, J=1.8, 9.3 Hz), 4.97 (2H, s), 3.01 - 2.93 (1H, m), 1.30 (6H, d, J=6.8 Hz);

Using a similar procedure to that for **intermediate 278, intermediate 256** (272 mg, 97 %) was synthesised from **intermediate 257** (480 mg, 1.50 mmol) using hydrazine hydrate. ESI-MS (M+H)+: 190.1, δ 8.33 - 8.33 (m, 1H), 7.69 (s, 1H), 7.41 - 7.38 (m, 1H), 7.18 - 7.15 (m, 1H), 3.80 - 3.79 (m, 2H), 2.95 - 2.86 (m, 1H), 1.26 - 1.23 (m, 6H).

### Intermediate 258: 2-(azidomethyl)-N,N-dimethylimidazo[1,2-a]pyridin-6-amine

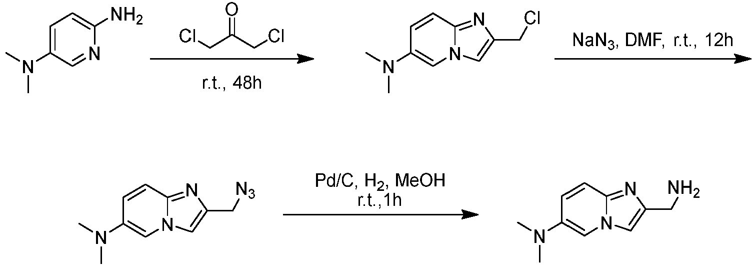

A solution of N⁵,N⁵-dimethylpyridine-2,5-diamine (1 g, 7.3 mmol) in 1,3-dichloropropan-2-one (2.9 g, 21.9 mmol) was stirred at room temperature for 48h to give ***2-(2-((tert-butyldimethylsilyl)oxy)ethoxy)-4,6-dichloropyrimidine.*** The reaction mixture was used into next step without purification. ESI-MS [M +H]+: 210.1.

To the solution from previous step was added DMF (30 mL), followed by NaN₃ (4.7 g, 73 mmol). The reaction was stirred at room temperature for 12h. The reaction was quenched with water (50 mL) and extracted with EtOAc (3 x 50 mL). The combined organic layers were washed with brine (50 mL), dried over anhydrous Na₂SO₄ and concentrated *in vacuo* to give the crude product, which was purified by silica gel chromatography (eluent: PE/EtOAc = 1/1) to give ***2-(azidomethyl)-N,N-dimethylimidazo[1,2-a]pyridin-6-amine* intermediate 259** (300 mg, 19% (2 steps)) as a yellow solid. ESI-MS [M +H]+: 217.2.
A solution of **intermediate 259** (300 mg, 1.39 mmol) and Pd/C (30 mg) in MeOH (10 mL) was stirred at room temperature under H₂ atmosphere for 1h. The reaction mixture was filtered through a pad of celite, washed with MeOH (3 x 30 mL). The filtrate was concentrated *in vacuo* to give **intermediate 258** (210 mg, 79.5%) as a yellow solid, which was used in the next step without further purification. ESI-MS [M +H]+: 191.2.

### Intermediate 260: (3-chloro-6-cyclopropylimidazo[1,2-a]pyridin-2-yl)methanamine

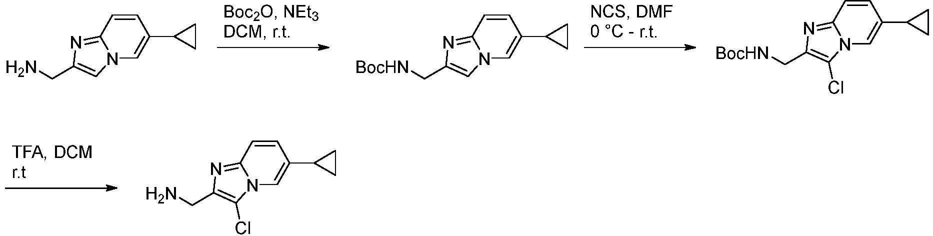

A mixture of **intermediate 251** (1.0 g, 5.3 mmol), di-*tert*-butyl decarbonate (1.4 g, 6.4 mmol) and NEt₃ (1.1 mL, 8.0 mmol) in DCM (55 mL) was stirred at room temperature for 18 h. A solution of NaHCO₃ (sat. aq., 50 mL) was added and the mixture was extracted with DCM (3 × 50 mL). The combined organics were dried (MgSO₄), filtered and concentrated *in vacuo.* The residue was purified by column chromatography on silica gel eluting with 20-100 % EtOAc in cyclohexane to give **tert-butyl ((6-cyclopropylimidazo[1,2-a]pyridin-2-yl)methyl)carbamate intermediate 261** (930 mg, 60 %) as a yellow solid. ESI-MS (M+H)+: 288.2, ¹H NMR (400 MHz, DMSO) δ 8.38 (s, 1H), 7.63 (s, 1H), 7.40 (d, J=9.3 Hz, 1H), 7.32 (dd, J=5.7, 5.7 Hz, 1H), 7.00 (dd, J=1.9, 9.2 Hz, 1H), 4.25 (d, J=6.1 Hz, 2H), 2.01 - 1.93 (m, 1H), 1.45 (s, 9H), 1.00 - 0.94 (m, 2H), 0.75 - 0.70 (m, 2H).

NCS (0.10 g, 0.76 mmol) was added to a solution of **intermediate 261** (0.20 g, 0.70 mmol) in DMF (5.0 mL) at 0°C under a N₂ atmosphere. The mixture was stirred at 0°C for 4 h then quenched with water. The mixture was extracted with EtOAc. The combined organic layers were dried (MgSO₄), filtered and concentrated *in vacuo* to give **tert-butyl ((3-chloro-6-cyclopropylimidazo[1,2-a]pyridin-2-yl)methyl)carbamate intermediate 262** (0.11 g, 49 %). ESI-MS (M+H)+: 322.3, ¹H NMR (400 MHz, DMSO) δ 8.14 (s, 1H), 7.55 (dd, J=0.6, 9.4 Hz, 1H), 7.32 - 7.26 (m, 1H), 7.11 (dd, J=1.7, 9.1 Hz, 1H), 4.30 (d, J=5.7 Hz, 2H), 2.18 - 2.10 (m, 1H), 1.44 (s, 9H), 1.05 - 0.99 (m, 2H), 0.84 - 0.78 (m, 2H).

A solution of **intermediate 262** (0.11 g, 0.34 mmol) in TFA (0.26 mL) and DCM (5.0 mL) was stirred at room temperature for 3 h. The mixture was loaded onto an SCX cartridge, washed with MeOH and eluted with 7 N NH₃ in MeOH to give **intermediate 260** (0.060 g, 79 %) which was used directly in the next step. ESI-MS (M+H)+: 222.2

### Intermediate 263: 2-(aminomethyl)-6-cyclopropylimidazo[1,2-a]pyridine-8-carbonitrile

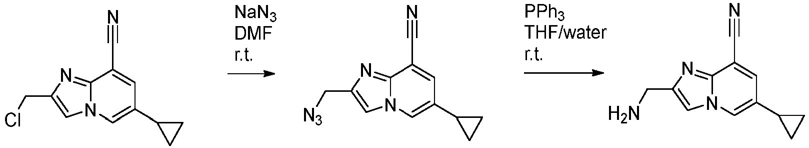

2-(Chloromethyl)-6-cyclopropylimidazo[1,2-a]pyridine-8-carbonitrile (630 mg, 2.70 mmol) and NaN₃ (230 mg, 3.5 mmol) were combined in DMF (5.0 mL) and stirred at room temperature under a nitrogen atmosphere for 18 h. The mixture was diluted with EtOAc (50 mL) and washed with water (2 × 50 mL) and brine (50 mL), then dried over MgSO₄, filtered and concentrated *in vacuo* to give **2-(azidomethyl)-6-cyclopropylimidazo[1,2-a]pyridine-8-carbonitrile intermediate 264** (580 mg, 89 %) as a purple oil. ESI-MS [M+H]+: 239.2, ¹H NMR (400 MHz, DMSO) δ 8.72 (dd, J=0.5, 1.7 Hz, 1H), 8.02 (s, 1H), 7.80 (d, J=1.8 Hz, 1H), 4.56 (s, 2H), 2.03 - 1.96 (m, 1H), 0.99 - 0.94 (m, 2H), 0.80 - 0.75 (m, 2H).

**Intermediate 264** (580 mg, 2.4 mmol) and triphenyl phosphine (1.3 g, 4.8 mmol) were combined in THF (11 mL) and water (1.0 mL) and stirred at room temperature for 24 h. The mixture was then concentrated *in vacuo,* dissolved in DCM and treated with HCl in dioxane (4.0 M). The precipitate was collected by filtration and washed with DCM to give **intermediate 263** (630 mg, 90 %) as a pale yellow solid. ESI-MS [M+H]+: 213.2, ¹H NMR (400 MHz, DMSO) δ 8.81 (s, 1H), 8.38 (br s, 3H), 8.06 (s, 1H), 7.85 (s, 1H), 4.19 (d, J=5.6 Hz, 2H), 2.04 - 1.97 (m, 1H), 1.02 - 0.95 (m, 2H), 0.82 - 0.76 (m, 2H).

### Intermediate 265: 6-cyclopropyl-2-(hydroxymethyl)imidazo[1,2-a]pyridine-8-carbonitrile

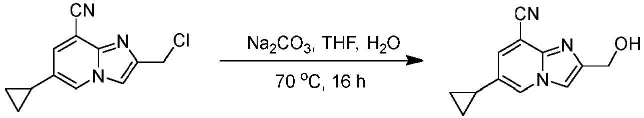

A mixture of 2-(chloromethyl)-6-cyclopropylimidazo[1,2-a]pyridine-8-carbonitrile (0.55 g, 2.0 mmol) and Na₂CO₃ (1.3 g, 12 mmol) in a mixed solvent of THF/H₂O (V/V =1/1, 20 mL) was stirred at 70°C for 16 h. After cooling to room temperature, the reaction mixture was extracted with EtOAc/MeOH (10/1, 3 x 30 mL). The combined organic layers were concentrated and purified by prep-TLC (eluent: DCM/MeOH = 20/1) to give **intermediate 265** (0.17 g, 40%) as a yellow solid. ESI-MS [M +H]⁺: 214.2

### Intermediate 266: (2-(aminomethyl)-6-cyclopropylimidazo[1,2-a]pyridin-8-yl)methanol hydrochloride

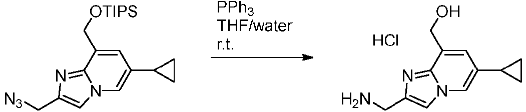

A mixture of 2-(azidomethyl)-6-cyclopropyl-8-(((tri*iso*propylsilyl)oxy)methyl) imidazo[1,2-a]pyridine (0.80 g, 2.0 mmol), PPh₃ (1.2 g, 4.5 mmol) and water (1.0 mL) in THF (10 mL) was stirred at room temperature for 18 h. The mixture was concentrated *in vacuo.* The material was divided, and 0.95 g of the 2.0 g residue was dissolved in DCM and washed with HCl solution (2.0 M aq.) and water. The combined aqueous layers were concentrated *in vacuo.* The residue was dissolved in MeOH and MP-carbonate resin (0.4 g) was added. The mixture was stirred gently for 1 h then filtered. The filtrate was concentrated *in vacuo* to give **intermediate 266** see also intermediate 266a in Example 98 (0.15 g, 20 %) as a yellow gum. ESI-MS (M+H)+: 218.2, ¹H NMR (400 MHz, DMSO) δ 8.98 - 8.85 (m, 3H), 8.80 (s, 1H), 8.34 (s, 1H), 7.66 (s, 1H), 4.89 (s, 2H), 4.43 - 4.35 (m, 2H), 3.22 (s, 1H), 2.19 - 2.10 (m, 1H), 1.15 - 1.07 (m, 2H), 0.88 - 0.84 (m, 2H).

### Intermediate 267: 1-(2-(aminomethyl)-6-cyclopropylimidazo[1,2-a]pyridin-8-yl)ethan-1-one

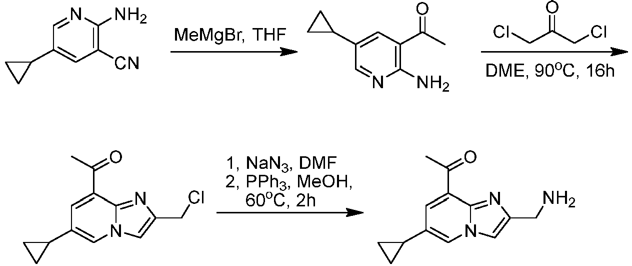

To a solution of 2-amino-5-cyclopropylnicotinonitrile (400 mg, 2.52 mmol) in THF (15 mL) was added CH₃MgBr (6.72 ml, 3M in THF, 20.16 mmol) drop-wise at 0°C. Then the mixture was stirred at 60°C for 2.5 h, quenched with sat. NH₄Cl solution (30 mL) and extracted with EtOAc/MeOH (10/1) (3 x 30 mL). The organic layers were concentrated *in vacuo* to give the crude product, which was purified by silica gel chromatography (eluent: DCM/MeOH = 0 ~ 5%) to give **1-(2-amino-5-cyclopropylpyridin-3-yl)ethan-1-one intermediate 268** as a light yellow solid (300 mg, 67%). ESI-MS [M +H]⁺: 177.2.

A solution of **intermediate 268** (600 mg, 3.41 mmol) and 1,3-dichloropropan-2-one (859 mg, 6.82 mmol) in DME (20 mL) was stirred at 90°C for 16 h under N₂. The reaction was cooled to room temperature and concentrated *in vacuo* to give the crude product, which was purified by silica gel chromatography (eluent: DCM/MeOH = 0 ~ 20%) to give **1-(2-(chloromethyl)-6-cyclopropylimidazo[1,2-a]pyridin-8-yl)ethan-1-one intermediate 269** as a brown solid (550 mg, 65%). ESI-MS [M +H]⁺: 249.1.

A solution of **intermediate 269** (550 mg, 2.02 mmol) and NaN₃ (180 mg, 2.77 mmol) in DMF (10 mL) was stirred at room temperature for 3 h under N₂. The mixture was diluted with EtOAc (50 mL) and washed with H₂O (3 x 50 mL). The organic layers were concentrated *in vacuo* to give **1-(2-(azidomethyl)-6-cyclopropylimidazo[1,2-a]pyridin-8-yl)ethan-1-one intermediate 270** as a brown solid (450 mg, crude), which was used into next step directly. ESI-MS [M +H]⁺: 256.2.

A solution of **intermediate 270** (450 mg, crude) and PPh₃ (692 mg, 2.64 mmol) in MeOH (20 mL) was stirred at 60°C for 2 h. The mixture was cooled to room temperature and concentrated *in vacuo.* The residue was purified by column chromatography (eluent: DCM/MeOH = 0 ~ 20%) to give **intermediate 267** as a light yellow oil (320 mg, 79%). ESI-MS [M +H]⁺: 230.1

### Intermediate 271: 1-(2-(aminomethyl)-6-cyclopropylimidazo[1,2-a]pyridin-8-yl)ethan-1-ol

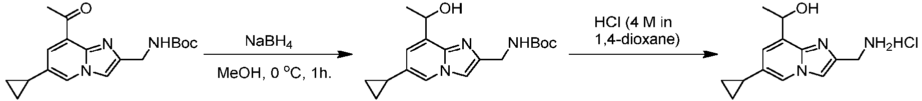

To solution of tert-butyl ((8-acetyl-6-cyclopropylimidazo[1,2-a]pyridin-2-yl)methyl)carbamate (300 mg, 0.91 mmol) in MeOH (20 mL) was added NaBH₄ (17.1 mg, 0.45 mmol) at 0°C. After stirring at 0°C for 1h, the mixture was quenched with water (20 mL) and concentrated to give the crude product which was purified by preparative TLC (eluent: DCM/MeOH = 15/1) to give **tert-butyl ((6-cyclopropyl-8-(1-hydroxyethyl)imidazo[1,2-a]pyridin-2-yl)methyl)carbamate intermediate 272** as a colourless oil (180 mg, 60%). ESI-MS [M +H]⁺: 332.1

A mixture of **intermediate 272** (180 mg, 0.54 mmol) in HCl (4 M solution in 1,4-dioxane, 6 mL) was stirred at room temperature for 1h. The mixture was concentrated *in vacuo* to give **intermediate 271** as the hydrochloric acid salt (180 mg, crude) as a yellow solid. ESI-MS [M +H]⁺: 232.1

### Intermediate 273: ethyl 3-(2-(aminomethyl)-6-cyclopropylimidazo[1,2-a]pyridin-8-yl)propanoate

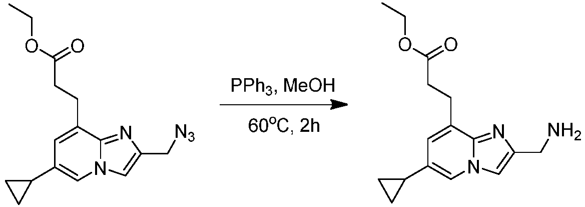

To a solution of ethyl 3-(2-(azidomethyl)-6-cyclopropylimidazo[1,2-a]pyridin-8-yl)propanoate (240 mg, 0.77 mmol) in MeOH (7 mL) was added PPh₃ (301 mg, 1.15 mmol). The resulting mixture was heated to 60°C and stirred for 2 h under N₂. The reaction mixture was cooled to room temperature and concentrated *in vacuo* to give the crude product, which was purified by preparative TLC (eluent: MeOH/DCM = 1/9) to give **intermediate 273** (130 mg, 59%) as yellow oil. ESI-MS [M +H]+: 288.2.

### Intermediate 274: (6-cyclopropyl-8-((2,2-diethoxyethoxy)methyl)imidazo[1,2-a]pyridin-2-yl)methanamine

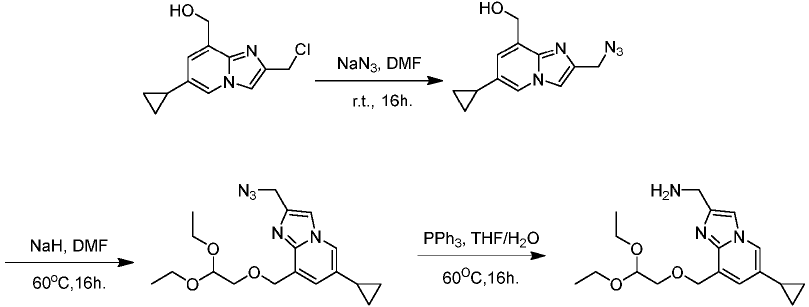

To a mixture of (2-(azidomethyl)-6-cyclopropylimidazo[1,2-a]pyridin-8-yl)methanol (1 g, 4.1 mmol) in DME(20 mL) was added NaH (0.39 g, 16.4 mmol) at 0°C .The reaction mixture was stirred at 0°C for 30 min, 2-bromo-1,1-diethoxyethane (0.8 g, 4.1 mmol) was add slowly. The resulting mixture was stirred at 60°C for 16h under N₂. After cooling to 25°C, water (50 mL) was added and the mixture was extracted with EtOAc (3 x 30 mL). The combined organic layers were washed with brine (60 mL), dried over anhydrous Na₂SO₄, and concentrated *in vacuo* to give the crude, which was purified by silica gel chromatography (eluent: DCM/MeOH = 100/1) to give **2-(azidomethyl)-6-cyclopropyl-8-((2,2-diethoxyethoxy)methyl)imidazo[1,2-a]pyridine intermediate 275** (780 mg, 53%) as a white solid. ESI-MS [M +H]+: 360.2.

A mixture of **intermediate 275** (780 mg, 2.17 mmol) and PPh₃ (1.1 g, 4.34 mmol) in a solution of THF (20 mL) and water (5 mL) was stirred at 60°C for 16h under N₂. Water (50 mL) was added and the mixture was extracted with EtOAc (3 x 30 mL). The combined organic layers were washed with brine (60 mL), dried over anhydrous Na₂SO₄, and concentrated *in vacuo* to give the crude, which was purified by column chromatography (eluent: DCM/MeOH = 100/1) to give **intermediate 274** (450 mg, 62%) as a white solid. ESI-MS [M +H]+: 334.2.

### Intermediate 276: ethyl 2-(2-(aminomethyl)-6-cyclopropylimidazo[1,2-a]pyridin-8-yl)acetate

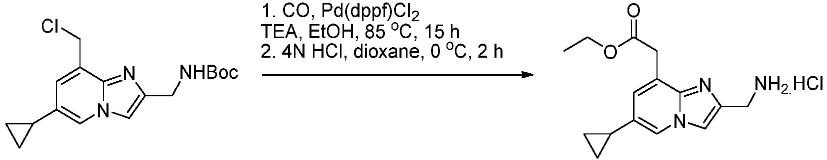

A mixture of **intermediate 387**(0.35 g, 1.0 mmol), Pd(dppf)Cl₂ (82 mg, 0.11 mmol) and Et₃N (2.0 mL) in EtOH (10 mL) was stirred at 85°C under CO for 15 h. After cooling to room temperature, the reaction mixture was concentrated *in vacuo* and purified by silica gel chromatography (eluent: DCM/MeOH = 0 ~ 10%) to give **ethyl 2-(2-(((tert-butoxycarbonyl)amino)methyl)-6-cyclopropylimidazo[1,2-a]pyridin-8-yl)acetate intermediate 277** (0.20 g, 54%) as a brown oil. ESI-MS [M +H]+: 374.2.

A mixture of **intermediate 277** (0.20 g, 0.54 mmol) in HCl (5 mL, 4 N in dioxane) was stirred at room temperature for 2 h. The reaction mixture was concentrated *in vacuo* to give **intermediate 276** (0.15 g, quant) as a yellow solid (hydrochloric acid salt), which was used for the next step directly without further purification. ESI-MS [M +H]+: 274.2.

### Intermediate 278: 3-(2-(aminomethyl)-6-cyclopropylimidazo[1,2-a]pyridin-8-yl)oxetan-3-ol

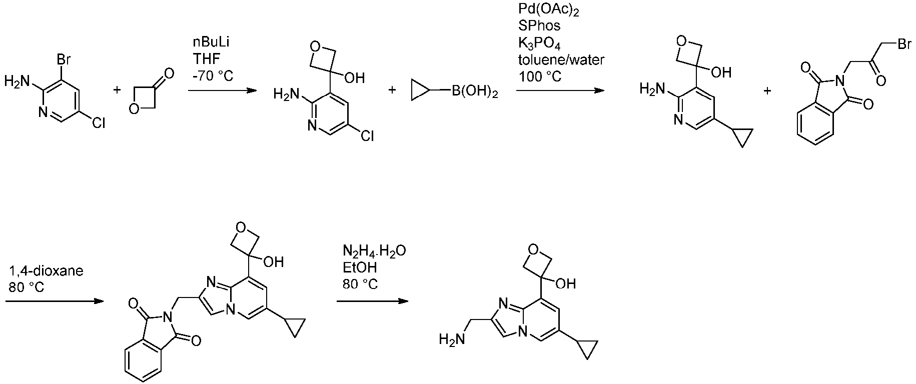

nBuLi in hexanes (9.2 mL, 22 mmol) was added dropwise to a stirred solution of **intermediate 505** (1.1 g, 5.5 mmol) in anhydrous THF (40 mL) at -70°C under a nitrogen atmosphere. The mixture was stirred -70°C for 1 h then a solution of oxetan-3-one (1.8 mL, 22 mmol) in THF (10 mL) was added slowly over 10 min. The resultant mixture was warmed to room temperature and stirred for 1 h. NH₄Cl (sat. aq., 15 mL) was added and the mixture was extracted with EtOAc (3 × 25 mL). The combined organics were dried over MgSO₄, filtered and concentrated *in vacuo.* The residue was purified by column chromatography on silica gel, eluting with 0-10 % MeOH in DCM to give **3-(2-amino-5-chloropyridin-3-yl)oxetan-3-ol intermediate 279** (460 mg, 42 %) as a pale brown solid. ESI-MS [M+H]+: 201.1, ¹H NMR (400 MHz, CDCl₃) δ 8.01 (d, J=2.4 Hz, 1H), 7.45 (d, J=2.4 Hz, 1H), 5.04 - 5.00 (m, 4H), 4.90 - 4.87 (m, 2H), 2.76 (br s, 1H).

A mixture of **intermediate 279** (550 mg, 2.8 mmol), cyclopropylboronic acid (350 mg, 4.1 mmol), SPhos (110 mg, 0.28 mmol), Pd(OAc)₂ (62 mg, 0.28 mmol) and K₃PO₄ (2.1 g, 9.6 mmol) in toluene (30 mL) and water (3.0 mL) was degassed with nitrogen then stirred at 100°C for 18 h under a nitrogen atmosphere. The mixture was cooled and filtered through Celite^{®} with EtOAc (75 mL). The filtrate was dried over MgSO₄, filtered and concentrated *in vacuo.* The residue was purified by column chromatography on silica gel, eluting with 0-10 % MeOH in DCM to give **3-(2-amino-5-cyclopropylpyridin-3-yl)oxetan-3-ol intermediate 280** (400 mg, 70 %) as a pale yellow solid. ESI-MS [M+H]+: 207.1, ¹H NMR (400 MHz, CDCl₃) δ 7.85 (d, J=1.9 Hz, 1H), 7.16 (d, J=2.3 Hz, 1H), 5.07 (dd, J=0.8, 7.3 Hz, 2H), 4.89 (dd, J=0.8, 7.3 Hz, 2H), 4.75 (br s, 2H), 2.95 (br s, 1H), 1.85 - 1.78 (m, 1H), 0.94 - 0.88 (m, 2H), 0.62 - 0.57 (m, 2H).

A mixture of **intermediate 280** (210 mg, 1.0 mmol) and **intermediate 311** (280 mg, 1.0 mmol) in 1,4-dioxane (4.0 mL) was stirred at 80°C for 18 h. The mixture was cooled to room temperature and diluted with EtOAc and K₂CO₃ (10 % aq.). The mixture was separated and the aqueous layer was further extracted with EtOAc. The combined organic layers were washed with brine (sat. aq.), dried over MgSO₄, filtered and concentrated *in vacuo.* The residue was purified by column chromatography on silica gel, eluting with 1-6 % MeOH in DCM to give **2-((6-cyclopropyl-8-(3-hydroxyoxetan-3-yl)imidazo[1,2-a]pyridin-2-yl)methyl)isoindoline-1,3-dione intermediate 281** (210 mg, 54 %) as a light yellow solid. ESI-MS [M+H]+: 390.2, ¹H NMR (400 MHz, CDCl₃) δ 7.88 - 7.85 (m, 2H), 7.76 - 7.75 (m, 1H), 7.74 - 7.71 (m, 2H), 7.47 (s, 1H), 7.36 (s, 1H), 7.24 (d, J=1.6 Hz, 1H), 5.02 - 4.99 (m, 4H), 4.77 (d, J=7.3 Hz, 2H), 2.01 - 1.88 (m, 1H), 1.01 - 0.95 (m, 2H), 0.70 - 0.65 (m, 2H).

A mixture of **intermediate 281** (120 mg, 0.31 mmol) and hydrazine hydrate (87 µL, 1.8 mmol) in EtOH (3.0 mL) was heated at reflux for 1 h. The mixture was cooled to room temperature, filtered and concentrated *in vauco.* The residue was redissolved in MeOH, filtered and concentrated *in vacuo.* The residue was purified by column chromatography on reverse phase silica gel, eluting with 30 % MeCN in water (0.1 % NH₅CO₃) to give **intermediate 278** (0.029 g, 36 %) as an off-white solid. ¹H NMR (400 MHz, MeOD) δ , 8.19 (s, 1H), 7.84 (s, 1H), 7.21 (s, 1H), 5.37 (d, J=6.8 Hz, 2H), 4.25 (s, 2H), 3.35 (s, 2H), 2.04 - 1.94 (m, 1H), 1.03 - 0.96 (m, 2H), 0.76 - 0.71 (m, 2H), exchangeable OH and NH not observed.

### Intermediate 282: (6-cyclopropyl-8-(3-fluorooxetan-3-yl)imidazo[1,2-a]pyridin-2-yl)methanamine

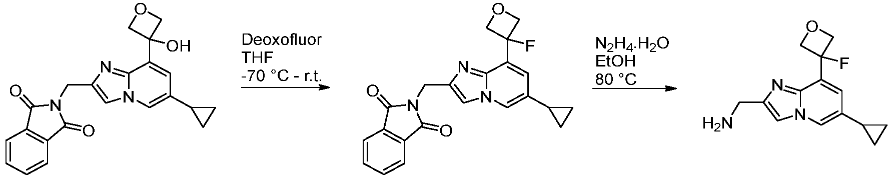

Deoxofluor^{®} (50 % in THF, 0.6 mL, 1.6 mmol) was added to a solution of **intermediate 281** (420 mg, 1.1 mmol) in DCM (20 mL) at -70°C. The mixture warmed to 0°C and stirred for 3 h. Further Deoxofluor^{®} (50 % in THF, 0.3 mL, 0.82 mmol) was added and the mixture was stirred at 0°C for a further 2 h. The mixture was warmed to room temperature and stirred for 18 h. The mixture was cooled to 0°C and NaHCO₃ (sat. aq., 15 mL) was added. The mixture was separated and the organic layer was dried over MgSO₄, filtered and concentrated *in vacuo* The residue was purified by column chromatography on silica gel, eluting with 20-60 % EtOAc in cyclohexane to give **2-((6-cyclopropyl-8-(3-fluorooxetan-3-yl)imidazo[1,2-a]pyridin-2-yl)methyl)isoindoline-1,3-dione intermediate 283** the title compound (190 mg, 44 %) as a colourless solid. ESI-MS [M+H]+: 392.1, ¹H NMR (400 MHz, CDCl₃) δ 7.91 - 7.86 (m, 2H), 7.79 (s, 1H), 7.75 - 7.70 (m, 2H), 7.43 (s, 1H), 6.95 (s, 1H), 5.53 - 5.41 (m, 2H), 5.08 (s, 2H), 5.06 - 4.95 (m, 2H), 1.90 - 1.82 (m, 1H), 0.98 - 0.90 (m, 2H), 0.66 - 0.60 (m, 2H).

A mixture of **intermediate 283** (180 mg, 0.46 mmol) in EtOH (5 mL) and hydrazine monohydrate (67 µL, 1.4 mmol) was stirred at reflux for 1 h. The mixture was cooled and concentrated *in vacuo.* The residue was dissolved in a mixture of DCM/MeOH (9:1, 8 mL), filtered and concentrated *in vacuo* to give **intermediate 282** (110 mg, 95 %) as a viscous pale brown oil. ESI-MS [M+H]+: 262.0, ¹H NMR (400 MHz, CDCl₃) δ 7.88 (s, 1H), 7.44 (s, 1H), 6.97 (s, 1H), 5.56 - 5.46 (m, 2H), 5.14 - 5.04 (m, 2H), 4.02 (s, 2H), 1.01 - 0.93 (m, 2H), 0.70 - 0.64 (m, 2H), cyclopropyl CH signal obscured by water signal.

### Intermediate 284: tert-butyl 3-(2-(aminomethyl)-6-cyclopropylimidazo[1,2-a]pyridin-8-yl)-3-fluoroazetidine-1-carboxylate

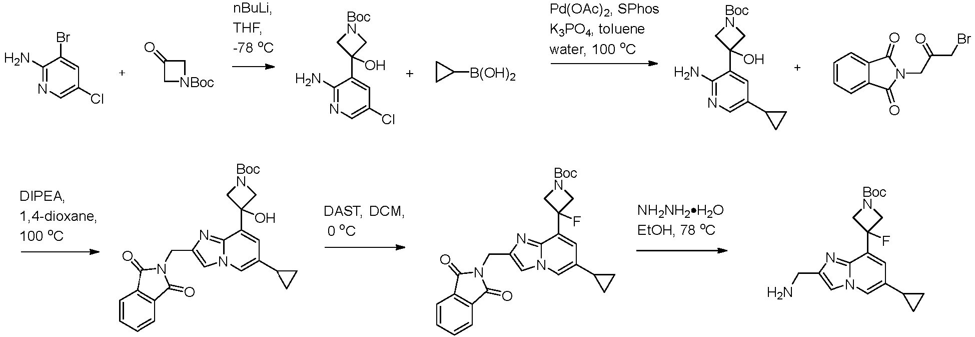

Using a similar procedure to that for **intermediate 281, *tert-butyl 3-(6-cyclopropyl-2-((1,3-dioxoisoindolin-2-yl)methyl)imidazo[1,2-a]pyridin-8-yl)-3-hydroxyazetidine-1-carboxylate* intermediate 285** (512 mg) was synthesised from **intermediate 505** (2.0 g, 9.64 mmol) and tert-butyl 3-oxoazetidine-1-carboxylate (6.6 g, 38.56 mmol). ESI-MS (M+H)+: 489.2, ¹H NMR (400 MHz, CDCl₃) δ 7.88 - 7.86 (m, 2H), 7.74 - 7.71 (m, 2H), 7.46 (s, 1H), 7.16 (s, 1H), 6.99 (d, J=1.5 Hz, 1H), 5.01 (s, 2H), 4.26 - 4.22 (m, 2H), 4.19 - 4.12 (m, 2H), 1.92 - 1.85 (m, 1H), 1.46 (s, 9H), 1.00 - 0.94 (m, 2H), 0.68 - 0.63 (m, 2H).

DAST (0.20 mL, 1.54 mmol) was added dropwise to a stirred solution of **intermediate 285** (250 mg, 0.512 mmol) in DCM (20 mL) at 0°C. The reaction was stirred at 0°C for 30 min. The reaction was quenched with NaHCO₃ solution (sat. aq., 20 mL) and extracted with DCM (2 x 20 mL), the organics were combined, dried (MgSO₄) and concentrated *in vacuo.* The residue was purified by silica gel chromatography, eluting with a gradient of 0-100 % EtOAc in cyclohexane to give ***tert-butyl 3-(6-cyclopropyl-2-((1,3-dioxoisoindolin-2-yl)methyl)imidazo[1,2-a]pyridin-8-yl)-3-fluoroazetidine-1-carboxylate* intermediate 286** (160 mg, 64 %). ESI-MS (M+H)+: 491.2, ¹H NMR (400 MHz, CDCl₃) δ 7.90 - 7.87 (m, 2H), 7.76 - 7.72 (m, 3H), 7.38 (s, 1H), 6.97 (s, 1H), 5.05 (s, 2H), 4.89 - 4.78 (m, 2H), 4.30 (dd, J=10.2, 22.9 Hz, 2H), 1.89 - 1.81 (m, 1H), 1.52 (s, 9H), 0.97 - 0.91 (m, 2H), 0.65 - 0.60 (m, 2H).

Using a similar procedure to that **forintermediate 278, intermediate 284** (110 mg, 93 %) was synthesised from **intermediate 286** (160 mg, 0.326 mmol) using hydrazine hydrate. ESI-MS (M+H)+: 361.1, ¹H NMR (400 MHz, CDCl₃) δ 7.85 (s, 1H), 7.40 (s, 1H), 6.98 (d, J=1.4 Hz, 1H), 4.87 (dd, J=10.4, 22.8 Hz, 2H), 4.33 (dd, J=10.2, 22.6 Hz, 2H), 3.97 (s, 2H), 1.93 - 1.85 (m, 1H), 1.50 (s, 9H), 0.99 - 0.93 (m, 2H), 0.70 - 0.65 (m, 2H). Two exchangeable protons (NH₂) not visible

### Intermediate 287: (6-cyclopropyl-8-(3-fluoro-1-methylazetidin-3-yl)imidazo[1,2-a]pyridin-2-yl)methanamine

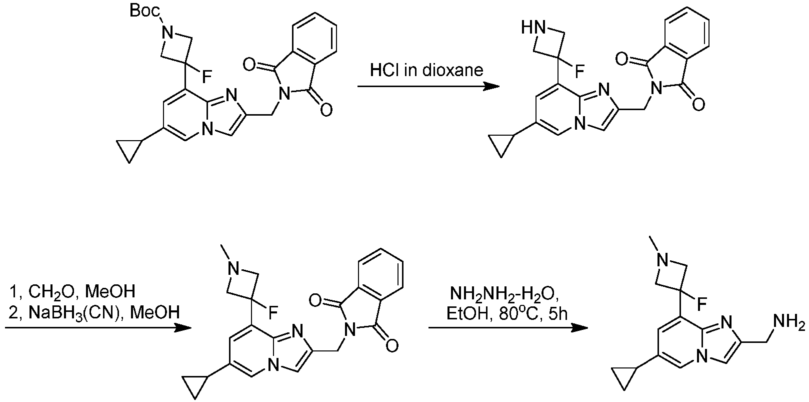

To a solution of intermediate **286** (110 mg, 0.22 mmol) in MeOH (10 mL) was added HCl (4M solution in dioxane, 5 mL). The resulting mixture was stirred at room temperature for 1 h and concentrated *in vacuo* to *give* **2-((6-cyclopropyl-8-(3-fluoroazetidin-3-yl)imidazo[1,2-a]pyridin-2-yl)methyl)isoindoline-1,3-dione Intermediate 288** as a hydrochloric acid salt (90 mg, 96%) as a yellow oil, which was used in next step without purification. ESI-MS [M +H]+: 391.1.

To a solution of intermediate **288** hydrochloride (89.5 mg, 0.21 mmol), paraformaldehyde (63 mg, 2.1 mmol) and NaBH₃CN ( (93 mg, 1.47 mmol) in MeOH (10 mL) was stirred at room temperature for 4 h. The mixture was then quenched with water (20 mL) and extracted with EtOAc (3 x 20 mL). The combined organic layers were washed with brine (50 mL), dried over anhydrous Na₂SO₄ and concentrated *in vacuo* to give the crude, which was purified by silica gel chromatography (DCM/MeOH= 20/1) to give **2-((6-cyclopropyl-8-(3-fluoro-1-methylazetidin-3-yl)imidazo[1,2-a]pyridin-2-yl)methyl)isoindoline-1,3-dione intermediate 289** (51 mg, 60%) as a yellow oil. ESI-MS [M +H]+: 405.2

To a solution of **intermediate 289** (50 mg, 0.12 mmol) in EtOH (10 mL) was added N₂H₄⁻ H₂O (1 mL). After stirring at 80°C for 5 h, the reaction mixture was filtered and the filtrate was concentrated *in vacuo* to give **intermediate 287** (30 mg, 91%) as a white solid, which was used in next step without purification. ESI-MS [M +H]⁺: 275.1.

### Intermediate 290: tert-butyl 3-(2-(aminomethyl)-6-cyclopropylimidazo[1,2-a]pyridin-8-yl)-3-hydroxyazetidine-1-carboxylate

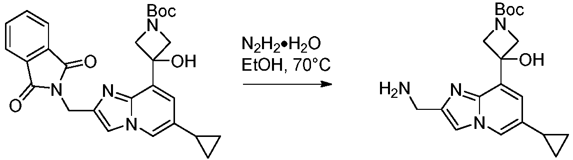

Using a similar procedure to that for **intermediate 278, intermediate 290** (180 mg, 99 %) was synthesised from **intermediate 285** (250 mg, 0.51 mmol) using hydrazine hydrate. ¹H NMR (400 MHz, CDCl₃) δ 7.79 (s, 1H), 7.38 (s, 1H), 7.01 - 6.99 (m, 1H), 4.29 (d, J=9.3 Hz, 2H), 4.24 - 4.19 (m, 2H), 3.97 (d, J=0.6 Hz, 2H), 1.97 - 1.88 (m, 1H), 1.47 (s, 9H), 1.02 - 0.96 (m, 2H), 0.71 - 0.66 (m, 2H). NH₂ and OH not observed.

### Intermediate 291: 3-(2-(aminomethyl)-6-cyclopropylimidazo[1,2-a]pyridin-8-yl)-1-methylazetidin-3-ol

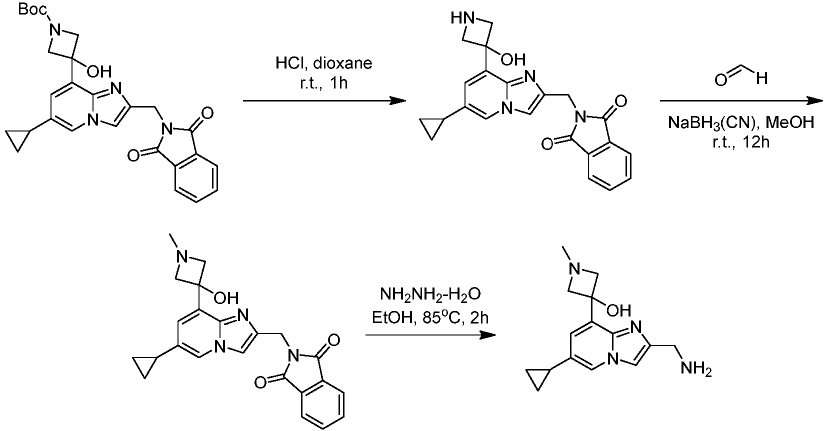

To a solution of **intermediate 285** (3.1 g, 6.35 mmol) in dioxane (20 mL) was added HCl (8 mL, 4 M solution in dioxane, 32 mmol). The resulting mixture was stirred at room temperature for 1h. The solution was concentrated *in vacuo* to give the residue, which was washed with saturated aqueous NaHCO₃ (50 mL), extracted with EtOAc (3 x 70 mL). The combined organic layers were washed with brine (70 mL), dried over Na₂SO₄, concentrated *in vacuo* to give the crude product, which was purified with silica gel chromatography (eluent: DMC/MeOH = 15/1) to give **2-((6-cyclopropyl-8-(3-hydroxyazetidin-3-yl)imidazo[1,2-a]pyridin-2-yl)methyl)isoindoline-1,3-dione intermediate 292** (1.5 g, 61%) as a yellow solid. ESI-MS [M +H]⁺: 389.1.

To a solution of **intermediate 292** (1.5 g, 3.86 mmol) in MeOH (30 mL) were added paraformaldehyde (232 mg, 7.72 mmol) and NaBH₃(CN) (487 mg, 7.72 mmol). The resulting mixture was stirred at room temperature for 12h. Then water (50 mL) was added and the mixture extracted with EtOAc (3 x 70 mL). The combined organic layers were washed with brine (50 mL), dried over Na₂SO₄ and concentrated *in vacuo* give the crude product, which was purified with silica gel chromatography (eluent: DCM/MeOH = 20/1) to give **2-((6-cyclopropyl-8-(3-hydroxy-1-methylazetidin-3-yl)imidazo[1,2-a]pyridin-2-yl)methyl)isoindoline-1,3-dione intermediate 293** (1.1 g, 71%) as a yellow solid. ESI-MS [M +H]⁺: 403.2.

A solution of **intermediate 293** (1.1 g, 2.74 mmol) in NH₂NH₂-H₂O (2 mL) in EtOH (20 mL) was stirred at 85°C for 2h. After cooling to the room temperature, the white solid was precipitated and filtered. The filtrate was concentrated *in vacuo* to give **intermediate 291**(700 mg crude), which was used into next step without further purification. ESI-MS [M +H]⁺: 273.2.

### Intermediate 294: tert-butyl 3-((2-(aminomethyl)-6-cyclopropylimidazo[1,2-a]pyridin-8-yl)(hydroxy)methyl)azetidine-1-carboxylate

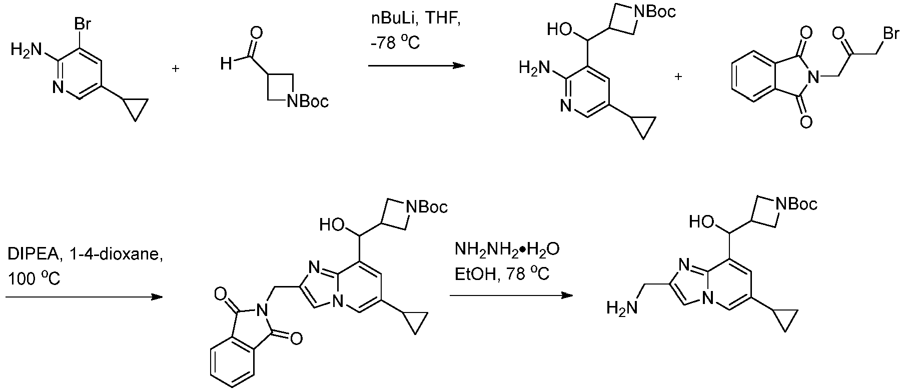

Using a similar procedure to that for**intermediate 278, intermediate 294** (80 mg) was synthesised from **intermediate 309** (633 mg, 2.97 mmol) and tert-butyl 3-formylazetidine-1-carboxylate (1.10 g, 5.94 mmol). ESI-MS (M+H)+: 373.2

### Intermediate 295: 4-(2-(aminomethyl)-6-cyclopropylimidazo[1,2-a]pyridin-8-yl)-1-methylpiperidin-4-ol

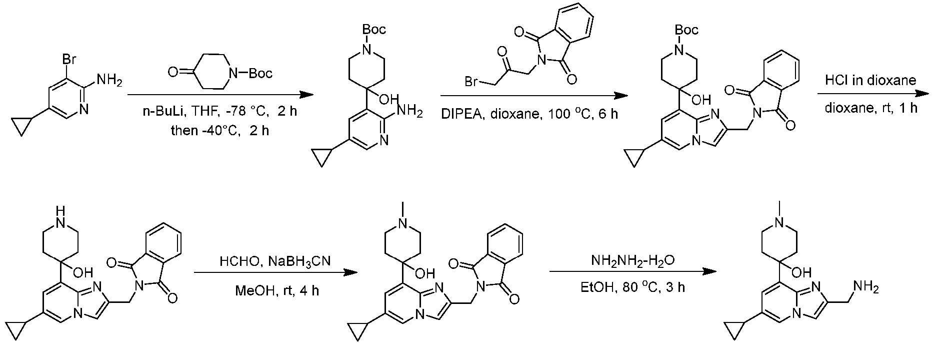

Using a similar procedure to that for**intermediate 278, tert-butyl 4-(6-cyclopropyl-2-((1,3-dioxoisoindolin-2-yl)methyl)imidazo[1,2-a]pyridin-8-yl)-4-hydroxypiperidine-1-carboxylate intermediate 296** (937 mg, 76%) was synthesised from **intermediate 309** (500 mg, 2.35 mmol) and tert-butyl 4-oxopiperidine-1-carboxylate (1.9 g, 9.54 mmol). ESI-MS [M +H]+ 517.2

To a solution of **intermediate 296** (937 mg, 1.82 mmol) in dioxane (3 mL) was added HCl (4 M in dioxane, 3 mL) at room temperature and stirred for 1 h. The reaction mixture was concentrated to give **2-((6-cyclopropyl-8-(4-hydroxypiperidin-4-yl)imidazo[1,2-a]pyridin-2-yl)methyl)isoindoline-1,3-dione hydrochloride intermediate 297** (866 mg, crude) as a white solid, which was used in next step without purification. ESI-MS [M + H ]⁺: 417.2.

A mixture of **intermediate 297** (866 mg, 2.08 mmol) and HCHO (779 mg, 9.60 mmol) in MeOH (20 mL) was added NaBH₃CN (302 mg, 4.80 mmol) at room temperature. The mixture was stirred at rt for 4 h. H₂O (30 mL) was added and extracted with EtOAc (3 x 30 mL). The combined organic layers were washed with brine (30 mL), dried over Na₂SO₄ and concentrated *in vacuo* give the crude product, which was purified by silica gel chromatography (eluent: DCM/MeOH=9/1) to give **2-((6-cyclopropyl-8-(4-hydroxy-1-methylpiperidin-4-yl)imidazo[1,2-a]pyridin-2-yl)methyl) isoindoline-1,3-dione intermediate 298** (822 mg, 92%) as a yellow oil. ESI-MS [M + H ]⁺: 431.2.

A solution of **intermediate 298** (400 mg, 0.93 mmol) in EtOH (15 mL) was added NH₂NH₂-H₂O (0.5 mL) at room temperature. Then the mixture was stirred at 80°C for 3 h and then cooled to room temperature. The reaction mixture was filtered and the filtrate was concentrated *in vacuo* to give **intermediate 295** (407 mg, crude) as a yellow solid, which was used directly into next step without further purification. ESI-MS [M + H ]⁺: 301.2.

### Intermediate 299: (6-cyclopropyl-8-(4-fluoro-1-methylpiperidin-4-yl)imidazo[1,2-a]pyridin-2-yl)methanamine

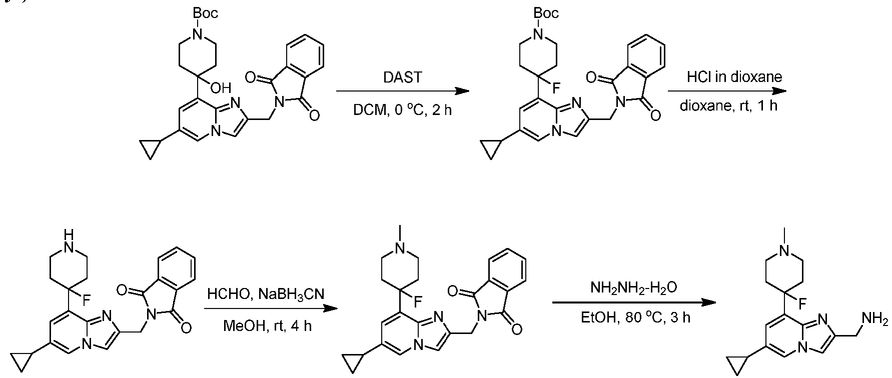

To a solution of tert-butyl 4-(6-cyclopropyl-2-((1,3-dioxoisoindolin-2-yl)methyl)imidazo[1,2-a]pyridin-8-yl)-4-hydroxy piperidine-1-carboxylate (517 mg, 1.0 mmol) in DCM (10 mL) was added a solution of DAST (177 mg, 1.1 mmol) in DCM (5 mL) dropwise at 0°C. After stirring at 0°C for 2 h under N₂, the mixture was quenched with water (20 mL) and extracted by DCM (3 x 20 mL). The combined organic layers were washed with brine (20 mL) and concentrated *in vacuo* to give the crude product, which was purified by silica gel chromatography (eluent: PE/EtOAc = 2/1) to give tert-butyl **4-(6-cyclopropyl-2-((1,3-dioxoisoindolin-2-yl)methyl)imidazo[1,2-a]pyridin-8-yl)-4-fluoropiperidine-1-carboxylate intermediate 300** (447 mg, 86%) as a yellow solid. ESI-MS [M + H]⁺: 519.2.

To a solution of **intermediate 300** (347 mg, 0.67 mmol) in dioxane (2 mL) was added HCl (4 M in dioxane, 2 mL) at room temperature and stirred at rt for 1 h. The reaction mixture was concentrated *in vacuo* to give **2-((6-cyclopropyl-8-(4-fluoropiperidin-4-yl)imidazo[1,2-a]pyridin-2-yl)methyl)isoindoline-1,3-dione hydrochloric acid salt intermediate 301** (300 mg, crude) as a white solid which was used in next step without purification. ESI-MS [M + H ]⁺: 419.2.

To a mixture of **intermediate 301** (200 mg, 0.44 mmol) and paraformaldehyde (66 mg, 2.2 mmol) in MeOH (5 mL) was added NaBH₃CN (70 mg, 1.1 mmol) at room temperature. After stirring for 4 h, water (30 mL) was added and extracted with EtOAc (3 x 50 mL). The combined organic layers were washed with brine (30 mL), dried over Na₂SO₄ and concentrated *in vacuo* to give the crude product, which was purified by preparative TLC (eluent: DCM/MeOH = 10/1) to give **2-((6-cyclopropyl-8-(4-fluoro-1-methylpiperidin-4-yl)imidazo[1,2-a]pyridin-2-yl)methyl)isoindoline-1,3-dione intermediate 302** (160 mg, 84%) as a white solid. ESI-MS [M + H ]⁺: 433.2.

To a mixture of **intermediate 302** (312 mg, 0.72 mmol) in EtOH (20 mL) was added NH₂NH₂-H₂O (225 mg, 3.6 mmol) at room temperature. After stirring at 80°C for 3 h, the reaction mixture was filtered and the filtrate was concentrated to give **intermediate 299** (200 mg, crude) as a yellow solid, which was used directly in the next step without further purification. ESI-MS [M + H ]⁺: 303.2.

### Intermediate 303: tert-butyl 4-(2-(aminomethyl)-6-cyclopropylimidazo[1,2-a]pyridin-8-yl)piperazine-1-carboxylate

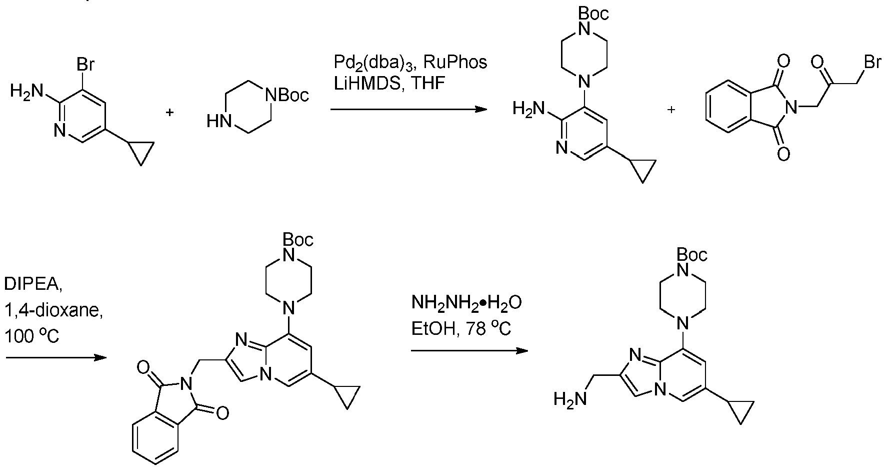

A mixture of **intermediate 309** (1.00 g, 4.69 mmol), 1-Boc-piperazine (1.31 g, 7.04 mmol), Pd₂(dba)₃ (0.43 g, 0.47 mmol), and RuPhos (0.44 g, 0.94 mmol) and degassed with N₂ for 10 min. THF (40 mL) and LiHMDS (1M in THF, 11.7 mL, 11.7 mmol) were added and the resulting reaction mixture was heated to 65°C for 90 min. The reaction mixture was allowed to cool to room temperature and was quenched with NH₄Cl (sat. aq., 100 mL) and extracted with EtOAc (3 × 75 mL). The combined organics were dried over MgSO₄ and concentrated *in vacuo.* The residue was purified by silica gel chromatography, eluting with a gradient of 0-100 % EtOAc in cyclohexane to give **tert-Butyl 4-(2-amino-5-cyclopropylpyridin-3-yl)piperazine-1-carboxylate intermediate 304** (0.940 g, 63 %). ESI-MS (M+H)+: 319.3, ¹H NMR (400 MHz, CDCl₃): δ ppm, 7.67 (1H, d, J = 2.0 Hz), 6.86 (1H, d, J = 2.0 Hz), 4.55 (2H, br s), 3.56 (4H, br s), 2.84 (4H, t, J = 4.7 Hz), 1.82 - 1.74 (1H, m), 1.49 (9H, s), 0.91 - 0.85 (2H, m), 0.60 - 0.55 (2H, m).
**Intermediate 304** (940 mg, 2.95 mmol) and **intermediate 311** (1.02 g, 3.25 mmol) were dissolved in 1,4-dioxane (90 mL) and DIPEA (0.77 mL, 4.43 mmol) was added. The reaction mixture was heated to 100°C and stirred at this temperature for 4.5 h. The reaction mixture was cooled to room temperature and concentrated *in vacuo.* The residue was purified by silica gel chromatography, eluting with a gradient of 0-100 % EtOAc in cyclohexane to give **tert-Butyl 4-(6-cyclopropyl-2-((1,3-dioxoisoindolin-2-yl)methyl)imidazo[1,2-a]pyridin-8-yl)piperazine-1-carboxylate intermediate 305** (1.38 g, 93 %). ESI-MS (M+H)+: 502.2, ¹H NMR (400 MHz, CDCl₃): δ ppm, 7.87 (2H, dd, J = 3.1, 5.5 Hz), 7.72 (2H, dd, J = 3.1, 5.5 Hz), 7.44 (1H, dd, J = 1.0, 1.0 Hz), 7.35 (1H, d, J = 1.0 Hz), 6.14 (1H, d, J = 1.0 Hz), 5.03 (2H, s), 3.62 (4H, t, J = 5.1 Hz), 3.41 (4H, br s), 1.84 - 1.76 (1H, m), 1.49 (9H, s), 0.91 - 0.85 (2H, m), 0.62 - 0.57 (2H, m).

Using a similar procedure to that **forintermediate 278, intermediate 303** (1.14 g, 85 %) was synthesised from **intermediate 305** (1.38 g, 2.75 mmol) using hydrazine hydrate. ESI-MS (M+H)+: 372.3, ¹H NMR (400 MHz, CDCl₃): δ ppm, 7.52 (1H, s), 7.31 (1H, s), 6.19 (1H, d, J = 1.6 Hz), 3.98 (2H, s), 3.69 (4H, t, J = 4.9 Hz), 3.43 (4H, t, J = 4.9 Hz), 1.86 - 1.79 (1H, m), 1.49 (9H, s), 0.94 - 0.89 (2H, m), 0.66 - 0.61 (2H, m).
Using a similar procedure to that used forintermediate 303, the compounds in Table A5 were prepared starting from **intermediate 309** and an appropriate coupling partner. **Table A5**

| | | |
|---|---|---|
| 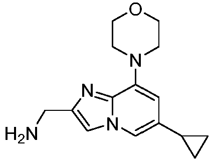 | (6-cyclopropyl-8-morpholinoimidazo[1,2-*a*]pyridin-2-yl)methanamine | Used without further purification |
| | Coupling Partner: morpholine | |

### Intermediate 306: (6-cyclopropyl-8-(4-cyclopropylpiperazin-1-yl)imidazo[1,2-a]pyridin-2-yl)methanamine

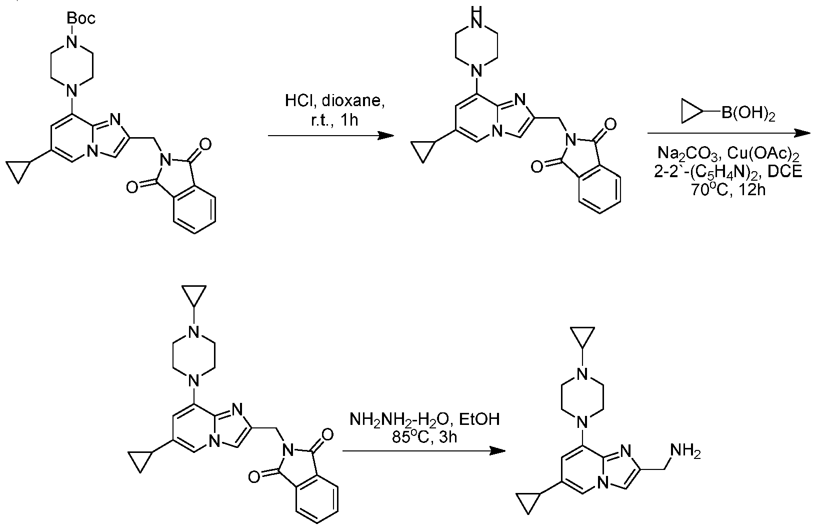

To a solution of **intermediate 305** (7 g, 13.97 mmol) in dioxane (30 mL) was added HCl (13.75 mL, 4 M solution in dioxane, 55 mmol). The resulting mixture was stirred at room temperature for 1h. The reaction was concentrated *in vacuo* to give the residue, which was washed with saturated aqueous NaHCO₃ (150 mL) and extracted with EtOAc (3 x 70 mL). The combined organic layers were washed with brine (70 mL), dried over Na₂SO₄, concentrated *in vacuo* to give the crude residue, which was purified with silica gel chromatography (eluent: DMC/MeOH = 15/1) to give **2-((6-cyclopropyl-8-(piperazin-1-yl)imidazo[1,2-a]pyridin-2-yl)methyl)isoindoline-1,3-dione intermediate 307** (4.5 g, 81.8%) as a yellow solid. ESI-MS [M +H]⁺: 402.1.

To a mixture of **intermediate 307** (750 mg, 1.87 mmol), cyclopropylboronic acid (322 mg, 3.74 mmol) and Na₂CO₃ (396 mg, 3.74 mmol) in DCE (10 mL) were added Cu(OAc)₂ (338 mg, 1.87 mmol) and 2,2'-bipyridine (292 mg, 1.87 mmol) in DCE (10 mL). After stirring at 70°C for 12 h, the reaction mixture was cooled to room temperature and filtered. The resulting filtrate was washed with water (30 mL) and extracted with DCM (30 mL x 3). The combined organic layers were washed with brine (50 mL), dried over Na₂SO₄ then concentrated *in vacuo* to give the crude, which was purified by silica gel chromatography (eluant: DCM/MeOH= 20/1) to give the product **2-((6-cyclopropyl-8-(4-cyclopropylpiperazin-1-yl)imidazo[1,2-a]pyridin-2-yl)methyl)isoindoline-1,3-dione intermediate** 308 (250 mg, 30%) as a yellow oil. ESI-MS [M +H]+: 442.2

A mixture of **intermediate 308** (150 mg, 0.34 mmol) and NH₂NH₂-H₂O(170 mg, 3.40 mmol) in EtOH (20 mL) was stirred at 85°C for 3 h. The reaction was cooled to room temperature and the mixture was filtered. The filtrate was concentrated *in vacuo* to give the crude product **intermediate 306** (120 mg, crude) as a yellow oil which was used in next step without purification. ESI-MS [M +H]+: 312.2

### Intermediate 309: 3-bromo-5-cyclopropylpyridin-2-amine

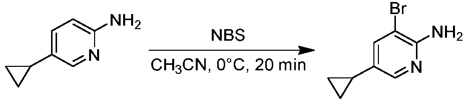

To a stirred solution of 5-cyclopropylpyridin-2-amine (10 g, 74 mmol) in CH₃CN (200 mL) was added NBS (20 g, 111.9 mmol) at 0°C and then the mixture was stirred at room temperature for 20 min. The reaction mixture was poured into water (250 mL) and extracted with EtOAc (250 mL×3). The combined organics were washed with brine (250 mL), dried over Na₂SO₄. The organic layers were concentrated *in vacuo* and purified by silica gel chromatography (eluent: PE: EA=10:1) to give **intermediate 309** (10 g, yield 62 %) ESI-MS [M +H] +: 213.0

### Intermediate 310: 2-((8-bromo-6-cyclopropylimidazo[1,2-a]pyridin-2-yl)methyl)isoindoline-1,3-dione

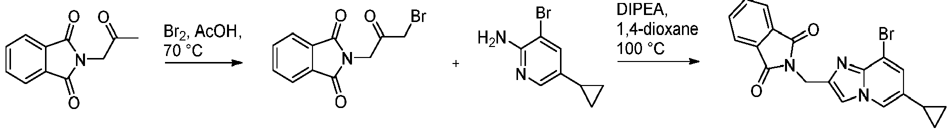

A few drops of a solution of bromine (7.6 mL, 150 mmol) in AcOH (80 mL) was added to a stirred solution of 2-(2-oxopropyl)isoindoline-1,3-dione (20 g, 98 mmol) at 70°C. The reaction was stirred until the solution went colourless. The remainder of the bromine/AcOH solution was added dropwise over 2h. The reaction mixture was stirred at 70°C for a further 2 h. The mixture was cooled to room temperature and concentrated *in vacuo.* The residue was dissolved in DCM (300 mL) and washed with Na₂S₂O₃ solution (1.0 M, 75 mL) and Na₂CO₃ solution (10 % aq., 2 × 150 mL). The combined organics were dried over MgSO₄ and concentrated *in vacuo.* The residue was triturated with hot diethyl ether, filtered and dried to give **2-(3-bromo-2-oxopropyl)isoindoline-1,3-dione intermediate 311** the title compound (22.6 g, 81 %) as a white solid. ¹H NMR (400 MHz, CDCl₃) δ 7.92 - 7.87 (m, 2H), 7.79 - 7.73 (m, 2H), 4.78 (s, 2H), 4.01 (s, 2H).

A solution of **intermediate 311** (12 g, 39 mmol), **intermediate 309** (7.5 g, 35 mmol) and DIPEA (9.2 mL, 53 mmol) in 1,4-dioxane (350 mL) was heated at 100°C for 16 h. The mixture was cooled to room temperature and the volume was reduced by a half by concentrating *in vacuo.* The mixture was diluted with DCM (200 mL) and washed with NaHCO₃ solution (sat. aq., 150 mL) and brine (150 mL). The organics were dried over MgSO₄ and concentrated *in vacuo.* The residue was purified by column chromatography on silica gel, eluting with a gradient of 0-100 % EtOAc in cyclohexane to give **intermediate 310** (7.2 g, 52 %). ESI-MS (M+H)+: 396.1, 398.1 δ 8.35 (d, J=1.0 Hz, 1H), 7.99 - 7.94 (m, 2H), 7.94 - 7.91 (m, 3H), 7.39 (d, J=1.5 Hz, 1H), 4.94 (s, 2H), 2.02 - 1.94 (m, 1H), 0.96 (m, 2H), 0.76 - 0.71 (m, 2H).

### Intermediate 312:2-((8-chloro-6-cyclopropylimidazo[1,2-a]pyridin-2-yl)methyl)isoindoline-1,3-dione

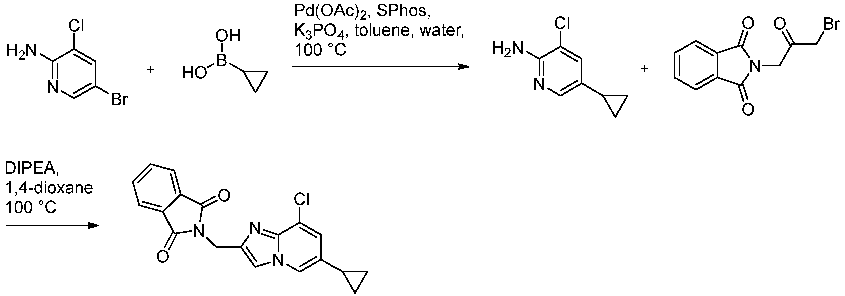

**Intermediate 312** was synthesized using a similar method to**intermediate** 310, starting from 5-bromo-3-chloropyridin-2-amine. ESI-MS (M+H)+: 352.1, ¹H NMR (400 MHz, CDCl₃) δ 7.91 - 7.85 (m, 2H), 7.76 - 7.70 (m, 3H), 7.46 (s, 1H), 6.99 (s, 1H), 5.10 (s, 2H), 1.87 - 1.80 (m, 1H), 0.99 - 0.89 (m, 2H), 0.67 - 0.60 (m, 2H).

### Intermediate 313: (8-chloro-6-cyclopropylimidazo[1,2-a]pyridin-2-yl)methanamine

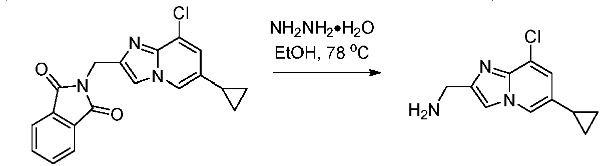

Using a similar procedure to that for**intermediate 278, intermediate 313** (75 mg, 78 %) was synthesised from **intermediate 312** (152 mg, 0.432 mmol) using hydrazine hydrate. ESI-MS (M+H)+: 222.2, ¹H NMR (400 MHz, MeOD) δ 8.18 - 8.15 (m, 1H), 7.76 - 7.73 (m, 1H), 7.20 - 7.17 (m, 1H), 3.97 (s, 2H), 2.00 - 1.89 (m, 1H), 1.04 - 0.96 (m, 2H), 0.77 - 0.69 (m, 2H), exchangeable protons not observed.

### Intermediate 314: 1-(2-(aminomethyl)-6-cyclopropylimidazo[1,2-a]pyridin-8-yl)pyrrolidin-2-one

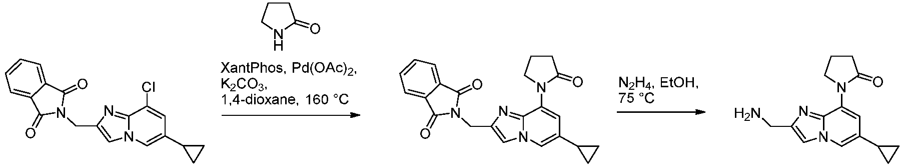

Pyrrolidine-2-one (0.65 mL, 8.5 mmol), **intermediate 312** (2000 mg, 5.7 mmol), Xantphos (660 mg, 1.1 mmol) and K₂CO₃ (1600 mg, 11 mmol) in 1,4-dioxane (20 mL) was degassed for 5 min with N₂. Pd(OAc)₂ (130 mg, 0.57 mmol) was added and the reaction mixture was heated in a microwave at 160°C for 2 h. The mixture was cooled to room temperature, filtered through Celite^{®} and concentrated *in vacuo.* The residue was purified by silica gel chromatography eluting with a gradient of 20-100 % EtOAc in cyclohexane to give **2-((6-cyclopropyl-8-(2-oxopyrrolidin-1-yl)imidazo[1,2-a]pyridin-2-yl)methyl)isoindoline-1,3-dione intermediate 315** (1.0 g, 44 %). ESI-MS (M+H)+: 401.3, ¹H NMR (400 MHz, CDCl₃) δ 7.87 (dd, J=3.0, 5.5 Hz, 2H), 7.72 (dd, J=3.1, 5.5 Hz, 2H), 7.69 - 7.68 (m, 1H), 7.45 (s, 1H), 7.24 (d, J=1.5 Hz, 1H), 5.02 (s, 2H), 4.27 (dd, J=7.1, 7.1 Hz, 2H), 2.58 (t, J=8.2 Hz, 2H), 2.22 - 2.13 (m, 2H), 1.90 - 1.82 (m, 1H), 0.95 - 0.89 (m, 2H), 0.68 - 0.62 (m, 2H). Using a similar procedure to that for**intermediate 278, intermediate 314** (600 mg, 89 %) was synthesised from **intermediate 315** (1.0 g, 2.50 mmol) using hydrazine hydrate. ESI-MS (M+H)+: 271.3, ¹H NMR (400 MHz, CDCl₃) δ 7.77 (s, 1H), 7.40 (s, 1H), 7.19 (d, J=1.5 Hz, 1H), 4.28 (t, J=7.2 Hz, 2H), 3.99 (d, J=0.6 Hz, 2H), 2.62 (t, J=8.2 Hz, 2H), 2.28 - 2.20 (m, 2H), 1.93 - 1.85 (m, 1H), 0.97 - 0.91 (m, 2H), 0.72 - 0.66 (m, 2H). NH₂ not observed. Compounds in Table A6 were synthesized using a similar method to **intermediate 315** from **intermediate 312** and the appropriate coupling partner, followed by treatment with hydrazine hydrate.

**Table A6:**

| | | |
|---|---|---|
| 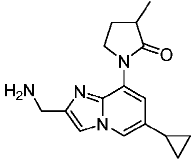 | 1-(2-(aminomethyl)-6-cyclo propylimidazo[1,2-a]pyridin-8-yl)-3-methyl pyrrolidin-2-one from **3-methylpyrrolidin-2-one** | ESI-MS (M+H)+: 285.2, ¹H NMR (400 MHz, MeOD) δ 8.24 - 8.23 (m, 1H), 7.80 (s, 1H), 7.15 (d, J=1.6 Hz, 1H), 4.06 (s, 2H), 4.02 - 3.97 (m, 2H), 2.87 - 2.76 (m, 1H), 2.55 - 2.46 (m, 1H), 2.04 - 1.96 (m, 2H), 1.34 (d, J=7.2 Hz, 3H), 1.05 - 0.99 (m, 2H), 0.80 - 0.75 (m, 2H). NH₂ not observed |
| 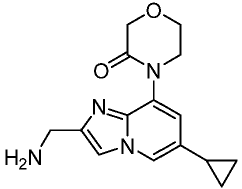 | 4-(2-(aminomethyl)-6-cyclo propylimidazo[1,2-a]pyridin-8-yl)morpholin-3-one from **morpholin-3-one** | ESI-MS [M+H]+: 287.2, ¹H NMR (400 MHz, MeOD) δ 8.25 - 8.24 (m, 1H), 7.74 - 7.73 (m, 1H), 7.13 (d, J=1.6 Hz, 1H), 4.36 (s, 2H), 4.15 - 4.12 (m, 2H), 3.93 - 3.93 (m, 2H), 3.84 - 3.80 (m, 2H), 2.02 - 1.94 (m, 1H), 1.02 - 0.97 (m, 2H), 0.78 - 0.73 (m, 2H), exchangeable NH₂ signal not observed. |
| 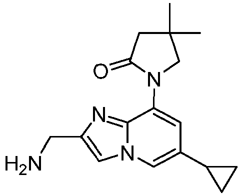 | 1-(2-(aminomethyl)-6-cyclo propylimidazo[1,2-a]pyridin-8-yl)-4,4-dimethylpyrrolidin-2-one from **4,4-dimethyl-2-pyrrolidinone** | ESI-MS [M+H]+: 299, ¹H NMR (400 MHz, MeOD) δ 8.18 (s, 1H), 7.73 (s, 1H), 7.11 (d, J=1.5 Hz, 1H), 3.97 (s, 2H), 3.78 (s, 2H), 2.48 (s, 2H), 2.01 - 1.93 (m, 1H), 1.32 (s, 6H), 1.02 - 0.95 (m, 2H), 0.77 - 0.71 (m, 2H). |
| 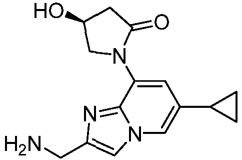 | (*S*)-1-(2-(aminomethyl)-6-cyclo propylimidazo[1,2-a]pyridin-8-yl)-4-hydroxy pyrrolidin-2-one from **(*S*)-2-((6-cyclopropyl-8-(4-hydroxy-2-oxopyrrolidin-1-yl)imidazo[1,2-*a*]pyridin-2-yl)methyl)isoindoline-1,3-dione** | ESI-MS (M+H)+: 287.2, ¹H NMR (400 MHz, MeOD) δ 8.24 - 8.24 (m, 1H), 7.76 - 7.75 (m, 1H), 7.16 (d, J=1.5 Hz, 1H), 4.64 - 4.64 (m, 1H), 4.37 (dd, J=5.4, 10.7 Hz, 1H), 3.96 (s, 2H), 3.80 - 3.76 (m, 1H), 3.02 (dd, J=6.5, 17.4 Hz, 1H), 2.55 - 2.49 (m, 1H), 2.04 - 1.96 (m, 1H), 1.05 - 0.99 (m, 2H), 0.80 - 0.75 (m, 2H), exchangeable protons not observed. |
| 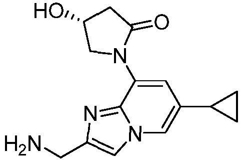 | (*R*)-1-(2-(aminomethyl)-6-cyclopropylimidazo[1,2-*a*]pyridin-8-yl)-4-hydroxy pyrrolidin-2-one from **(*R*)-2-((6-cyclopropyl-8-(4-hydroxy-2-oxopyrrolidin-1-yl)imidazo[1,2-*a*]pyridin-2-yl)methyl)isoindoline-1,3-dione** | ESI-MS (M+H)+: 287, ¹H NMR (400 MHz, MeOD) δ 8.24 - 8.24 (m, 1H), 7.76 - 7.75 (m, 1H), 7.17 - 7.16 (m, 1H), 4.64 - 4.64 (m, 1H), 4.37 (dd, J=5.4, 10.7 Hz, 1H), 3.97 - 3.96 (m, 2H), 3.80 - 3.76 (m, 1H), 3.02 (dd, J=6.5, 17.3 Hz, 1H), 2.55 - 2.49 (m, 1H), 2.04 - 1.96 (m, 1H), 1.05 - 0.99 (m, 2H), 0.80 - 0.75 (m, 2H), exchangeable protons not observed. |
| 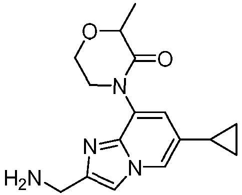 | 4-(2-(aminomethyl)-6-cyclopropylimidazo[1,2-*a*]pyridin-8-yl)-2-methylmorpholin-3-one from **2-((6-cyclopropyl-8-(2-methyl-3-oxomorpholino) imidazo [1,2-*a*]pyridin-2-yl)methyl)isoindoline-1,3-dione** | ESI-MS (M+H)+: 301.2, ¹H NMR (400 MHz, DMSO) δ 8.34 - 8.33 (m, 1H), 7.73 (s, 1H), 6.97 - 6.96 (m, 1H), 4.39 - 4.35 (m, 1H), 4.09 (s, 1H), 4.00 - 3.96 (m, 2H), 3.84 - 3.80 (m, 2H), 3.75 - 3.70 (m, 1H), 1.98 - 1.92 (m, 1H), 1.41 (d, J=6.8 Hz, 3H), 0.97 - 0.92 (m, 2H), 0.72 - 0.67 (m, 2H), exchangeable protons not observed. |

### Intermediate 316: 1-(2-(aminomethyl)-6-cyclopropylimidazo[1,2-a]pyridin-8-yl)imidazolidine-2,4-dione

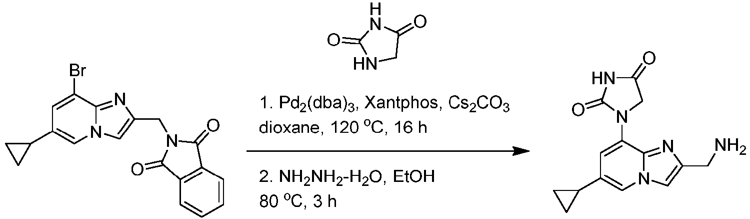

A mixture of **intermediate 310** (350 mg, 0.886 mmol), imidazolidine-2,4-dione (354 mg, 3.544 mmol), Pd₂(dba)₃ (162 mg, 0.177 mmol), Xantphos (154 mg, 0.266 mmol) and Cs₂CO₃ (866 mg, 2.658 mmol) in 1,4-dioxane (20 ml) were stirred at 120 °C for 16 h. The reaction mixture was allowed to reach room temperature, quenched with water (50 mL) and extracted with EtOAc (3 x 50 ml). The combined organics were washed with brine (50 mL), dried over Na₂SO₄ and concentrated *in vacuo.* The crude residue was purified by preparative TLC (eluent: DCM:MeOH = 15:1) to afford **2-((6-cyclopropyl-8-(2,4-dioxoimidazolidin-1-yl)imidazo[1,2-a]pyridin-2-yl)methyl)isoindoline-1,3-dione intermediate 317** (70 mg, 19%) as a yellow solid.
ESI-MS: [M + H]⁺, 416.1

A solution of **intermediate 317** (70 mg, 0.17 mmol) and NH₂NH₂-H₂O (0.2 mL) in EtOH (1 ml) was stirred at 80 °C for 3 h. The reaction mixture was allowed to reach room temperature, solid was filtered and filtrate was concentrated to give **intermediate 316** (40 mg, crude) which was used in next step without further purification.
ESI-MS: [M + H]⁺, 286.2
Using a similar procedure to that used for **intermediate 316,** the compounds in Table A7 were prepared from **intermediate 310** and an appropriate coupling partner, followed by treatment with hydrazine hydrate. **Table A7** (¹H NMR (400 MHz, DMSO unless indicated otherwise):

| | | |
|---|---|---|
| 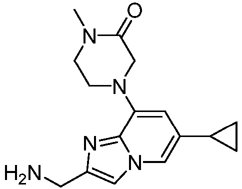 | 4-(2-(aminomethyl)-6-cyclo propylimidazo[1,2-a]pyridin-8-yl)-1-methylpiperazin-2-one from | δ 7.97 (s, 1H), 7.63 (s, 1H), 6.20 (d, J=1.5 Hz, 1H), 4.12 (s, 2H), 3.92 (m, 2H), 3.83 (s, 2H), 3.50 (m, 2H), 3.22 (s, 2H), 2.96 (s, 3H), 1.97 - 1.88 (m, 1H), 0.93 (m, 2H), 0.77 - 0.72 (m, 2H). |
| | **1-methylpiperazin-2-one** | |
| 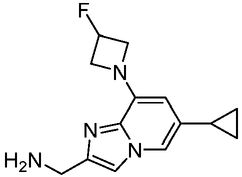 | (6-cyclopropyl-8-(3-fluoro azetidin-1-yl)imidazo[1,2-a]pyridin-2-yl)methanamine from **3-fluoroazetidine** | ESI-MS (M+H)+: 261.2. |
| 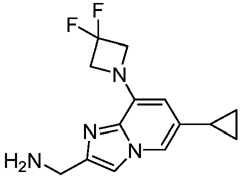 | (6-cyclopropyl-8-(3,3-difluoro azetidin-1-yl)imidazo[1,2-a] pyridin-2-yl)methanamine from **3,3-difluoro azetidine** | ESI-MS (M+H)+: 279.2. |
| 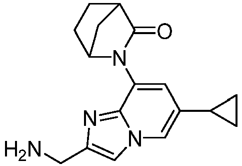 | 2-(2-(aminomethyl)-6-cyclo propylimidazo[1,2-*a*]pyridin-8-yl) -2-azabicyclo[2.2.1]heptan-3-one from **2-azabicyclo[2.2.1]heptan-3-one** | δ 8.17 (s, 1H), 7.69 (s, 1H), 7.29 (s, 1H), 5.47 (s, 1H), 3.81 (s, 2H), 2.83 (m, 1H), 2.02 (m, 1H), 1.97 - 1.73 (m, 4H), 1.65 - 1.53 (m, 2H), 0.94 - 0.90 (m, 2H), 0.67 - 0.61 (m, 2H). |
| 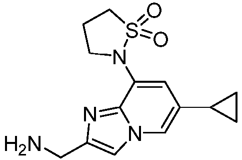 | 2-(2-(aminomethyl)-6-cyclo propylimidazo[1,2-*a*]pyridin-8-yl) isothiazolidine1,1-dioxide from **isothiazolidine 1,1-dioxide** | **ESI-MS** (M+H)+: 307.2 |
| 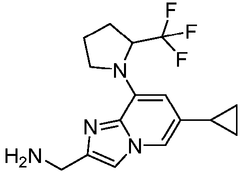 | (6-cyclopropyl-8-(2-(trifluoro methyl)pyrrolidin-1-yl)imidazo [1,2-a]pyridin-2-yl)methanamine from **2-(trifluoromethyl)pyrrolidine** | **δ** 7.83 (s, 1H), 7.61 (s, 1H), 6.42 - 6.33 (m, 1H), 6.10 (s, 1H), 3.94 - 3.85 (m, 1H), 3.81 (s, 2H), 3.47 (s, 1H), 2.34 - 2.21 (m, 1H), 2.17 - 2.05 (m, 3H), 1.95 - 1.86 (m, 1H), 0.92 (m, 2H), 0.77 - 0.67 (m, 2H). |
| 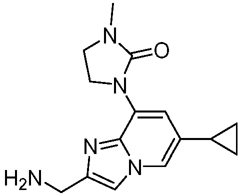 | 1-(2-(aminomethyl)-6-cyclo propylimidazo[1,2-*a*]pyridin-8-yl)-3-methylimidazolidin-2-one from **1-methylimidazolidin-2-one** | **δ** 7.79 (s, 1H), 7.40 (s, 1H), 7.03 (d, J=1.6 Hz, 1H), 5.04 (dd, J=6.3, 13.6 Hz, 1H), 3.99 (d, J=1.8 Hz, 2H), 2.67 - 2.61 (m, 2H), 2.59 - 2.45 (m, 1H), 1.93 - 1.86 (m, 1H), 1.82 - 1.74 (m, 1H), 1.07 (d, J=6.3 Hz, 3H), 0.97 - 0.93 (m, 2H), 0.72 - 0.67 (m, 2H). |
| 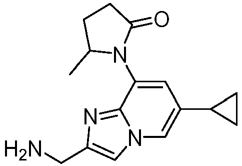 | 1-(2-(aminomethyl)-6-cyclo propylimidazo[1,2-*a*]pyridin-8-yl)-5-methylpyrrolidin-2-one from **5-methylpyrrolidin-2-one** | ESI-MS (M+H)+: 415.4: δ 7.89 - 7.86 (m, 2H), 7.74 - 7.70 (m, 3H), 7.44 (s, 1H), 7.07 - 7.06 (m, 1H), 5.13 - 5.04 (m, 1H), 5.03 - 5.02 (m, 2H), 2.63 - 2.58 (m, 2H), 2.48 - 2.39 (m, 1H), 1.90 - 1.82 (m, 1H), 1.75 - 1.65 (m, 1H), 1.00 (d, J=6.3 Hz, 3H), 0.95 - 0.90 (m, 2H), 0.68 - 0.63 (m, 2H). |
| 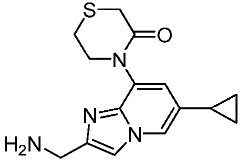 | 4-(2-(aminomethyl)-6-cyclo propylimidazo[1,2-*a*]pyridin-8-yl)thiomorpholin-3-one from **thiomorpholin-3-one** | **δ** 8.31 (d, J=1.5 Hz, 1H), 7.73 (s, 1H), 6.93 (d, J=1.5 Hz, 1H), 4.01 (t, J=5.8, 2H), 3.80 (s, 2H), 3.47 (s, 2H), 3.18 (m, 2H), 1.99 - 1.91 (m, 1H), 0.97 - 0.91 (m, 2H), 0.71 - 0.66 (m, 2H). |
| 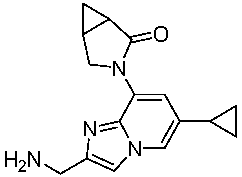 | 3-(2-(aminomethyl)-6-cyclo propylimidazo[1,2-*a*]pyridin-8-yl)-3-azabicyclo[3.1.0]hexan-2-one from **3-azabicyclo[3.1.0]hexan-2-one** | ESI-MS (M+H)+: 283.1, **δ** 7.73 (s, 1H), 7.38 (s, 1H), 7.09 (s, 1H), 4.52 (m, 1H), 4.12 (m, 1H), 3.99 (s, 2H), 2.12 - 2.02 (m, 2H), 1.93 - 1.83 (m, 1H), 1.28 - 1.21 (m, 2H), 0.99 - 0.87 (m, 2H), 0.69 - 0.64 (m, 2H). |
| 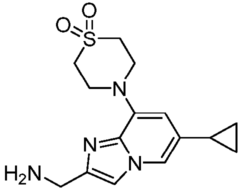 | 4-(2-(aminomethyl)-6-cyclo propyl imidazo[1,2*-a*]pyridin-8-yl)thiomorpholine 1,1-dioxide from **thiomorpholine 1,1-dioxide** | **δ** 7.94 (s, 1H), 7.60 (s, 1H), 6.28 (s, 1H), 4.12 - 4.11 (m, 4H), 3.78 (s, 2H), 3.24 (m, 4H), 1.93 - 1.85 (m, 1H), 0.92 - 0.86 (m, 2H), 0.75 - 0.70 (m, 2H). |
| 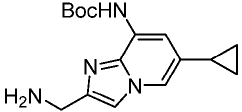 | *tert*-butyl (2-(aminomethyl)-6-cyclopropylimidazo[1,2-*a*] pyridin-8-yl)carbamate from ***tert*-Butyl carbamate** | ESI-MS (M+H)+: 303.1 |

### Intermediate 318: 2-(methoxymethyl)-1-methylpiperazine

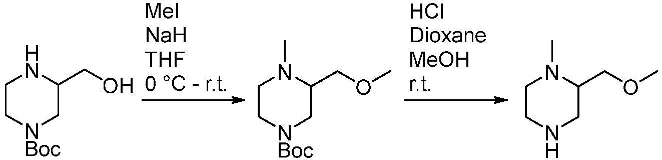

NaH (60 % in mineral oil, 0.041 g, 1.0 mmol) was added to a stirred solution of tert-butyl 3-(hydroxymethyl)piperazine-1-carboxylate (0.20 g, 0.93 mmol) in THF (5.0 mL) at 0°C. The mixture was stirred at 0°C for 30 min then MeI (0.063 mL, 1.0 mmol) was added. The mixture was stirred at 0°C for 2 h then quenched with water and extracted with DCM (3 ×). The combined organic layers were dried over MgSO₄, filtered and concentrate-d *in vacuo.* The residue was purified by column chromatography on silica gel, eluting with 0-20 % (7 N NH₃ in MeOH) in DCM to give **tert-butyl 3-(methoxymethyl)-4-methylpiperazine-1-carboxylate intermediate 319** (0.060 g, 28 %). ¹H NMR (400 MHz, CDCl₃) δ 3.97 - 3.77 (m, 2H), 3.52 - 3.37 (m, 2H), 3.35 (s, 3H), 3.07 - 2.92 (m, 2H), 2.77 - 2.70 (m, 1H), 2.34 (s, 3H), 2.24 - 2.14 (m, 1H), 2.14 - 2.06 (m, 1H), 1.46 (s, 9H).

A mixture of **intermediate 319** (0.060 g, 0.25 mmol) and HCl (4.0 M in 1,4-dioxane, 0.61 mL, 2.5 mmol) in MeOH (1.0 mL) was stirred at room temperature for 2 h. The mixture was concentrated *in vacuo* to give **intermediate 318** (0.035 g, quant.) as an off-white foam which was used without further purification.

### Intermediate 320: 3-methylimidazolidin-4-one

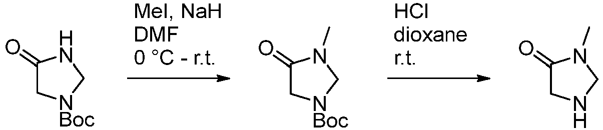

NaH (60 % in mineral oil, 0.20 g, 4.9 mmol) was added to a stirred solution of *tert*-butyl 4-oxoimidazolidine-1-carboxylate (0.76 g, 4.1 mmol) in DMF (5.0 mL) at 0°C. After 20 min MeI (0.36 mL, 5.7 mmol) was added and the mixture was warmed to room temperature and stirred for 2 h. NaHCO₃ (sat. aq.) was added and the mixture was extracted with EtOAc (3 ×). The combined organic layers were washed with brine (sat. aq.), dried over MgSO₄, filtered and concentrated *in vacuo.* The residue was purified by column chromatography on silica gel, eluting with 0-100 % EtOAc in DCM to give **tert-butyl 3-methyl-4-oxoimidazolidine-1-carboxylate intermediate 321** (0.54 g, 66 %) as an off-white solid. ESI-MS (M+H)+: 201.2, ¹H NMR (400 MHz, CDCl₃) δ 4.71 (d, J=8.3 Hz, 2H), 3.97 - 3.87 (m, 2H), 2.96 (s, 3H), 1.48 (s, 9H).

A mixture of **intermediate 321** (0.54 g, 2.7 mmol) and HCl solution (4.0 M in 1,4-dioxane, 6.7 mL, 27 mmol) in MeOH (5.0 mL) was stirred at room temperature for 2 h. The mixture was concentrated *in vacuo* and the residue was loaded onto an SCX cartridge and washed with a mixture of MeOH and DCM. The product was eluted with (7 N NH₃ in MeOH) in DCM to give **intermediate 320** (0.23 g, 85 %) as a clear oil. ESI-MS (M+H)+: 101.3, ¹H NMR (400 MHz, CDCl₃) δ 4.39 (s, 2H), 3.47 (d, J=7.3 Hz, 2H), 2.86 (s, 3H).

### Intermediate 322: 3-methylimidazolidine-2,4-dione

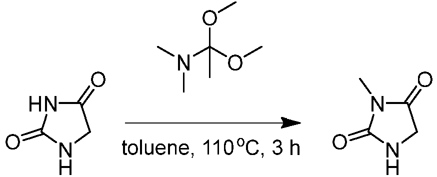

To a solution of imidazolidine-2,4-dione (1 g, 10 mmol) in dry toluene (25 mL) was added 1,1-dimethoxy-N,N-dimethylethan-1-amine (4 g, 30 mmol). The reaction mixture was stirred at 110°C for 3 h under N₂ and then cooled to 0°C for 15 minutes and filtered. The filtrate was concentrated and the crude residue was purified by silica gel chromatography (eluent: DCM/MeOH = 10/1) to give **intermediate 322** (400 mg, 34%) as a white solid. ESI-MS [M +H]⁺: 115.1.

### Intermediate 323: 4-(2-(aminomethyl)-6-cyclopropylimidazo[1,2-a]pyridin-8-yl)piperazin-2-one

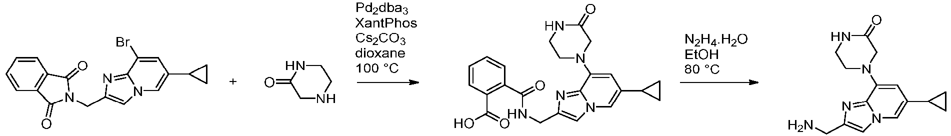

A mixture of **intermediate 310** (0.50 g, 1.3 mmol), piperazin-2-one (0.19 g, 1.9 mmol), Pd₂(dba)₃ (0.12 g, 0.13 mmol), XantPhos (0.15 g, 0.25 mmol) and Cs₂CO₃ (0.82 g, 2.5 mmol) in 1,4-dioxane (13 mL) was degassed with nitrogen then stirred at 100°C for 2.5 h. The mixture was filtered through Celite^{®} with a mixture of 7 N NH₃ in MeOH and DCM and concentrated *in vacuo.* The residue was purified by column chromatography on silica gel (40 g), eluting with 1-100 % (7 N NH₃ in MeOH) in DCM to give **2-(((6-cyclopropyl-8-(3-oxopiperazin-1-yl)imidazo[1,2-a]pyridin-2-yl)methyl)carbamoyl)benzoic acid intermediate 324** the title compound (0.68 g, quant.), which was used directly in the next step. ESI-MS (M+H)+: 434.4.

A mixture of **intermediate 324** (0.68 g, 1.6 mmol) and hydrazine hydrate (0.98 mL, 16 mmol) in ethanol (16 mL) was stirred at 80°C for 18 h. The mixture was loaded onto an SCX cartridge and washed with a mixture of MeOH and DCM. The product was eluted with 7 N NH₃ in MeOH in DCM to give **intermediate 323** (0.18 g, 39 %) as a yellow gum, which was used without further purification. ESI-MS (M+H)+: 286.3.

### Intermediate 325: 2-((6-cyclopropyl-8-(2-oxo-3-azabicyclo[3.1.0]hexan-3-yl)imidazo[1,2-a]pyridin-2-yl)methyl)isoindoline-1,3-dione

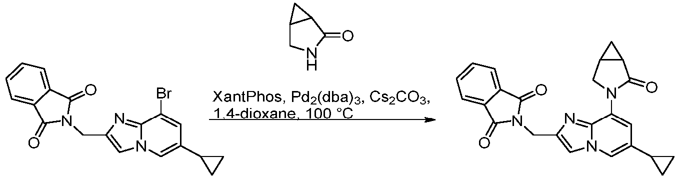

3-azabicyclo[3.1.0]hexan-2-one (270 mg, 2.8 mmol), **intermediate 310** (730 mg, 1.9 mmol), XantPhos (210 mg, 0.37 mmol), and Cs₂CO₃ (1.2 g, 3.7 mmol) were dissolved in 1,4-dioxane (10 mL) and degassed with nitrogen for 5 min. Pd₂(dba)₃ (170 mg, 0.185 mmol) was added and the reaction mixture was heated to 100°C and stirred for 4 h. The reaction mixture was cooled to room temperature, filtered through Celite^{®} and concentrated *in vacuo.* The residue was purified by silica gel chromatography eluting with a gradient of 20-100 % EtOAc in cyclohexane to give **intermediate 325** (580 mg, 76 %) which was used without further purification. ESI-MS (M+H)+: 413.2 ¹H NMR (400 MHz, CDCl₃): δ 7.88 (dd, J=3.0, 5.4 Hz, 2H), 7.73 (dd, J=3.0, 5.4 Hz, 2H), 7.66 (dd, J=0.7, 1.5 Hz, 1H), 7.43 (s, 1H), 7.14 (d, J=1.5 Hz, 1H), 5.01 (s, 2H), 4.45 (dd, J=5.9, 10.5 Hz, 1H), 4.18 - 4.14 (m, 1H), 2.08 - 2.03 (m, 1H), 2.01 - 1.95 (m, 1H), 1.87 - 1.80 (m, 1H), 1.20 - 1.14 (m, 1H), 0.93 - 0.86 (m, 3H), 0.66 - 0.61 (m, 2H).

### Intermediate 326: 1-(2-(aminomethyl)-6-cyclopropylimidazo[1,2-a]pyridin-8-yl)-3-tritylimidazolidin-2-one

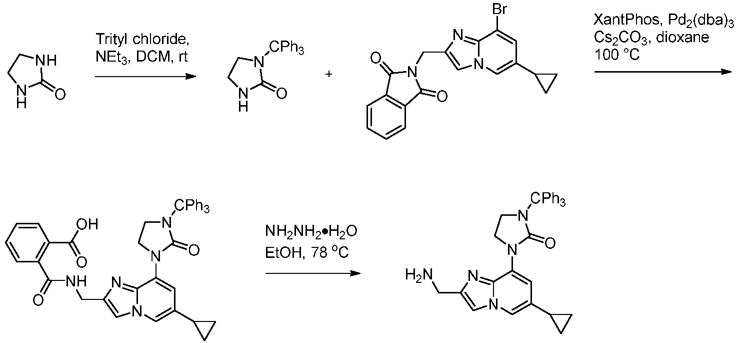

Triethylamine (1.6 mL, 11.62 mmol) was added to a stirred solution of imidazolidin-2-one (500 mg, 5.81 mmol) and trityl chloride (2.43 g, 8.71 mmol) in DCM (15 mL). The reaction was stirred at room temperature for 18 h. The reaction was diluted with water (50 mL) and extracted with DCM (3 x 30 mL). The organics were combined, dried over MgSO₄ then concentrated *in vacuo.* The residue was purified by silica gel chromatography, eluting with a gradient of 0-10 % MeOH in DCM to give **1-tritylimidazolidin-2-one intermediate 327(1.44** g, 75 %). ESI-MS (M+H)+: 327.2, δ 7.39 (d, J=7.5 Hz, 6H), 7.29 (dd, J=7.7, 7.7 Hz, 6H), 7.19 (dd, J=7.2, 7.2 Hz, 3H), 6.40 (s, 1H), 3.31 - 3.19 (m, 4H). **Intermediate 310** (300 mg, 0.76 mmol), **intermediate 327** (370 mg, 1.14 mmol), Xantphos (88 mg, 0.15 mmol) and Cs₂CO₃ (490 mg, 1.51 mmol) were suspended in dioxane and degassed with N₂ for 5 min. Pd₂(dba)₃ (69 mg, 0.0757 mmol) was added and the reaction was heated to 100°C, under a N₂ environment, for 4 h. The reaction was cooled to room temperature and filtered through Celite^{®}. The Celite^{®} was washed with DCM:MeOH (20 mL 1:1), the filtrate was concentrated *in vacuo.* The residue was purified by silica gel chromatography, eluting with a gradient of 0-10 % (7N NH₃ in MeOH) in DCM to give **2-(((6-cyclopropyl-8-(2-oxo-3-tritylimidazolidin-1-yl)imidazo[1,2-a]pyridin-2-yl)methyl)carbamoyl)benzoic acid intermediate 328** (320 mg, 64 %). ESI-MS (M+H)+: 662.4, ¹H NMR (400 MHz, DMSO) δ 8.10 (s, 1H), 7.84 (s, 1H), 7.62 (d, J=7.0 Hz, 2H), 7.46 (d, J=7.6 Hz, 6H), 7.34 (dd, J=7.7, 7.7 Hz, 7H), 7.23 (dd, J=7.3, 7.3 Hz, 4H), 7.14 (d, J=1.5 Hz, 1H), 4.51 (d, J=5.5 Hz, 2H), 4.34 (dd, J=7.4, 7.4 Hz, 2H), 3.48 (dd, J=7.5, 7.5 Hz, 2H), 1.91 - 1.83 (m, 1H), 0.91 - 0.85 (m, 2H), 0.62 - 0.57 (m, 2H), Exchangeable protons not seen.

Hydrazine hydrate (0.13 mL, 2.42 mmol) was added to a stirred solution of **intermediate 328** (320 mg, 0.484 mmol) in ethanol (5.0 mL) the reaction was then heated to 78°C for 18 h. The reaction was cooled to room temperature and loaded onto a 5.0 g SCX cartridge in MeOH, washed with 3:1 DCM:MeOH and eluted with 3:1 DCM:7N NH₃ in MeOH. The eluent was concentrated *in vacuo* to give **intermediate 326** (140 mg, 57 %), used in the next step without further purification. ESI-MS (M+H)+: 514.3, ¹H NMR (400 MHz, DMSO) δ 8.12 (d, J=1.4 Hz, 1H), 7.67 (s, 1H), 7.46 (d, J=7.5 Hz, 6H), 7.33 (dd, J=7.7, 7.7 Hz, 6H), 7.23 (dd, J=7.3, 7.3 Hz, 3H), 7.11 (d, J=1.5 Hz, 1H), 4.31 (dd, J=7.5, 7.5 Hz, 2H), 3.81 (s, 2H), 3.29 - 3.21 (m, 2H), 3.18 (s, 1H), 1.91 - 1.83 (m, 1H), 0.91 - 0.85 (m, 2H), 0.61 - 0.56 (m, 2H).

### Intermediate 329: tert-butyl 4-(2-(aminomethyl)-6-cyclopropylimidazo[1,2-a]pyridin-8-yl)-3-oxopiperazine-1-carboxylate

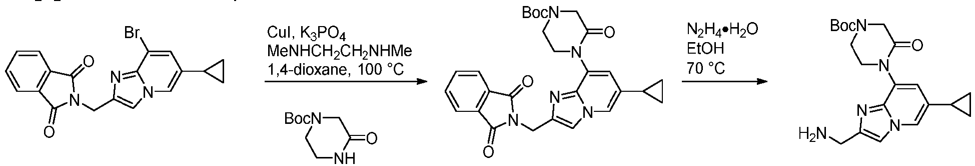

A mixture of **intermediate 310** (0.30 g, 0.76 mmol), te*r*t-butyl 3-oxopiperazine-1-carboxylate (0.20 g, 0.98 mmol), CuI (0.029 g, 0.15 mmol) and *N*,*N*'-dimethylethylenediamine (0.033 mL, 0.30 mmol) in 1,4-dioxane (3.0 mL) was degassed with nitrogen and stirred at 115°C for 18 h. K₃PO₄ (0.21 g, 0.98 mmol) and further CuI (0.029 g, 0.15 mmol) were added and the mixture was stirred at 115°C for 18 h. The mixture was diluted with EtOAc and washed with NH₄OH (dil. aq.) and brine (sat. aq.). The organic layer was passed through a phase separator and concentrated *in vacuo.* The residue was purified by column chromatography on silica gel, eluting with 0-20 % MeOH in DCM to give ***tert-butyl 4-(6-cyclopropyl-2-((1,3-dioxoisoindolin-2-yl)methyl)imidazo[-1,2-a]pyridin-8-yl)-3-oxopiperazine-1-carboxylate*** (0.12 g, 32 %) as a beige solid. ESI-MS (M+H)+: 516, ¹H NMR (400 MHz, DMSO) δ 8.27 (d, J=10.7 Hz, 1H), 7.96 - 7.87 (m, 4H), 7.77 (d, J=9.3 Hz, 1H), 7.37 (d, J=9.3 Hz, 1H), 4.88 (s, 2H), 4.12 (s, 1H), 3.84 - 3.64 (m, 3H), 3.48 - 3.39 (m, 2H), 1.96 - 1.89 (m, 1H), 1.45 (d, J=18.2 Hz, 9H), 0.96 - 0.89 (m, 2H), 0.69 - 0.64 (m, 2H).

Hydrazine monohydrate (0.27 mL, 5.5 mmol) was added to a stirred solution of *tert*-butyl 4-(6-cyclopropyl-2-((1,3-dioxo*iso*indolin-2-yl)methyl)imidazo[1,2-a]pyridin-8-yl)-3-oxopiperazine-1-carboxylate (0.28 g, 0.55 mmol) in EtOH (1.7 mL). The mixture was stirred at 70°C for 1 h. The mixture was diluted with EtOH and filtered with further EtOH. The filtrate was concentrated *in vacuo.* The residue was loaded onto an SCX cartridge and washed with MeOH. The product was eluted with 20% (7 N NH₃ in MeOH) in DCM and concentrated *in vacuo* to give **intermediate 329** (0.13 g, 59 %) as a yellow gum. ESI-MS (M+H)+: 386.

### Intermediate 330: 1-(2-(aminomethyl)-6-cyclopropylimidazo[1,2-a]pyridin-8-yl)-4-methylpiperazin-2-one

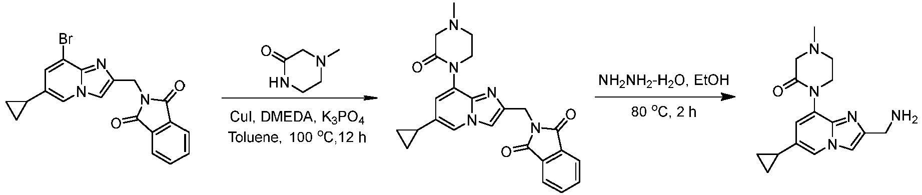

To a mixture of **intermediate 310** (500 mg,1.26 mmol), 4-methylpiperazin-2-one (287.3 mg, 2.52 mmol) and K₃PO₄ (801.3 mg, 3.78 mmol) in toluene (10 mL) were added CuI (47.9 mg, 0.25 mmol) and DMEDA (221.7 mg, 2.52 mmol). The reaction mixture was stirred at 100 ⁰C for 12 h under N₂ and cooled to room temperature. The reaction mixture was filtered through celite and the filter cake was washed with EtOAc (10 mL). The filtrate was concentrated *in vacuo* to give the crude product, which was purified by column chromatography (PE/EtOAc = 4/1) to give **2-((6-cyclopropyl-8-(4-methyl-2-oxopiperazin-1-yl)imidazo[1,2-a]pyridin-2-yl)methyl)isoindoline-1,3-dione intermediate 331** (120 mg, 22%) as a white solid. ESI-MS [M +H]+: 430.2.

To a solution of **intermediate 331** (120 mg, 0.28 mmol) in EtOH (5 mL) was added hydrazine hydrate (77 mg, 1.54 mmol). The reaction mixture was stirred at 80 ⁰C for 2 h and cooled to room temperature. The reaction mixture was filtered through celite and the filter cake was washed with DCM/MeOH (30/1, 100 mL). The filtrate was concentrated *in vacuo* to give the crude, which was purified by preparative TLC (DCM/MeOH = 30/1) to give **intermediate 330** (60 mg, 72%) as a white solid. ESI-MS [M +H]+: 300.2.

### Intermediate 332: (6-cyclopropyl-8-(1-methylazetidin-3-yl)imidazo[1,2-a]pyridin-2-yl)methanamine

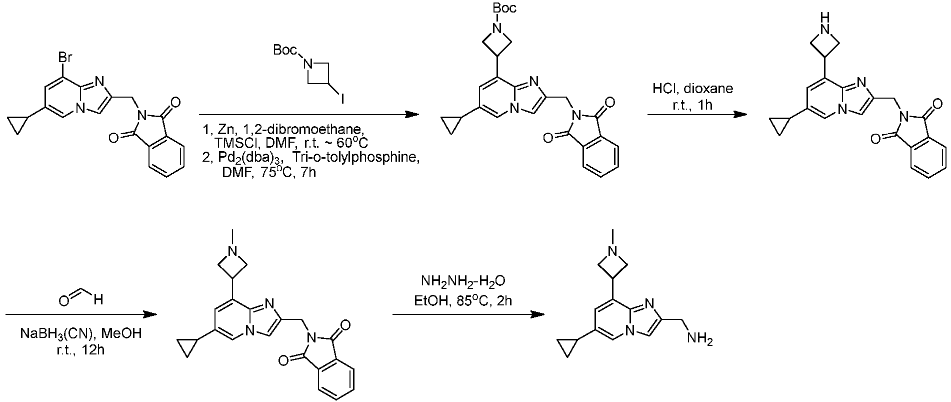

1,2-Dibromoethane (95 mg, 0.51 mmol) was added to a suspension of zinc dust (3.28 g, 50.1 mmol) in dry DMF (50 mL), and the reaction mixture was heated to reflux for 1 h. After cooling to room temperature, the reaction mixture was treated with trimethylsilyl chloride (55 mg, 0.51 mmol) and stirred for 1 h. A solution of tert-butyl 3-iodoazetidine-1-carboxylate (2.86 g, 10.1 mmol) in DMF (15 mL) was then added drop-wise. The reaction was stirred for 1 h at 60°C and cooled to room temperature. To this mixture were added **intermediate 310** (2 g, 5.05 mmol) and Pd₂(dba)₃ (467 mg, 0.51 mmol) and tri-o-tolylphosphine (310 mg, 1.02 mmol) and the mixture was heated at 75°C for 7 h. After cooling to room temperature the reaction mixture was filtered through Celite and the filtrate was concentrated *in vacuo,* then treated with EtOAc (3 x 50 mL) and saturated aqueous sodium carbonate solution. The combined organic layers were dried over Na₂SO₄, filtered and concentrated *in vacuo* to give the crude, which was purified with silica gel chromatography (eluent: DCM/MeOH = 20/1) to give **intermediate 333** (320 mg, 13%) as a yellow solid. ESI-MS [M +H]⁺: 473.2.

To a solution of **intermediate 333** (320 mg, 0.68 mmol) in dioxane (10 mL) was added HCl (2 mL, 4 M solution in dioxane, 4 mmol). The resulting mixture was stirred at room temperature for 1h and then concentrated *in vacuo* to give the residue, which was washed with saturated aqueous NaHCO₃ (30 mL), extracted with EtOAc (3 x 30 mL). The combined organic layers were washed with brine (30 mL), dried over Na₂SO₄, concentrated *in vacuo* to give the crude, which was purified with silica gel chromatography (eluent: DMC/MeOH = 15/1) to give **2-((8-(azetidin-3-yl)-6-cyclopropylimidazo[1,2-a]pyridin-2-yl)methyl)isoindoline-1,3-dione intermediate 334** (190 mg, 75%) as a yellow solid. ESI-MS [M +H]⁺: 373.1.

To a solution of **intermediate 334** (190 mg, 0.51 mmol) in MeOH (10 mL) was added paraformaldehyde (46 mg, 1.53 mmol) and NaBH₃(CN) (97 mg, 1.53 mmol). After stirring the resulting mixture at room temperature for 12h, water (30 mL) was added and the mixture extracted with EtOAc (3 x 30 mL). The combined organic layers were washed with brine (30 mL), dried over Na₂SO₄, concentrated *in vacuo* give the crude, which was purified with silica gel chromatography (eluent: DCM/MeOH = 20/1) to give **2-((6-cyclopropyl-8-(1-methylazetidin-3-yl)imidazo[1,2-a]pyridin-2-yl) methyl)isoindoline-1,3-dione intermediate 335** (140 mg, 71%) as a yellow solid. ESI-MS [M +H]⁺: 387.2.

A solution of **intermediate 335** (140 mg, 0.36 mmol) in NH₂NH₂-H₂O (0.5 mL) in EtOH (10 mL) was stirred at 85°C for 2h. After cooling to the room temperature, the white solid was precipitated and filtered. The filtrate was concentrated *in vacuo* to give **intermediate 331** (80 mg crude) as yellow oil, which was used into next step without further purification. ESI-MS [M +H]⁺: 257.2.

### Intermediate 336: tert-butyl 3-(2-(aminomethyl)-6-cyclopropylimidazo[1,2-a]pyridin-8-yl)azetidine-1-carboxylate

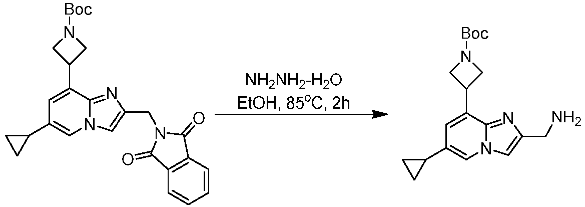

To a solution of **intermediate 333** (150 mg, 0.32 mmol) in NH₂NH₂-H₂O (0.5 mL) in EtOH (10 mL) was stirred at 85°C for 2h. After cooling to the room temperature, the white solid was precipitated and filtered. The filtrate was concentrated *in vacuo* to give **intermediate 336** (100 mg crude) as a yellow solid, which was used without further purification. ESI-MS [M +H]⁺: 343.2.

### Intermediate 337: (6-cyclopropyl-8-(4-(oxetan-3-yl)piperazin-1-yl)imidazo[1,2-a]pyridin-2-yl)methanamine

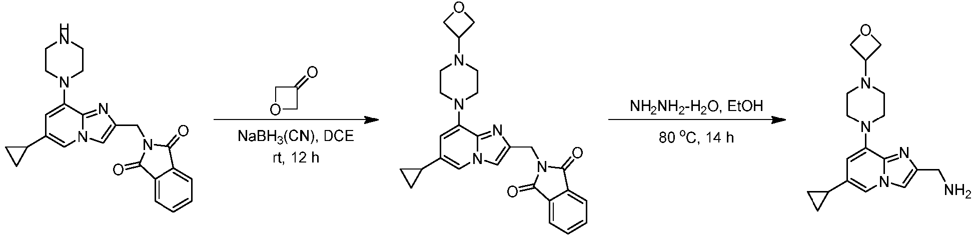

To a mixture of 2-((6-cyclopropyl-8-(piperazin-1-yl)imidazo[1,2-a]pyridin-2-yl)methyl)isoindoline-1,3-dione (50 mg, 0.12 mmol) and oxetan-3-one(17 mg, 0.24 mmol ) in DCE (10 mL) was added NaBH₃CN (23 mg, 0.37 mmol) slowly at 0°C. The reaction mixture was stirred at room temperature for 12 h. The resulting mixture was quenched with water (20 mL) and the aqueous phase was extracted with EtOAc (3 x 20 mL). The combined organic layers were washed with brine (30 mL), dried over Na₂SO₄ and concentrated *in vacuo* to give the crude, which was purified by Pre-TLC (DCM/MeOH= 10/1) to give **2-((6-cyclopropyl-8-(4-(oxetan-3-yl)piperazin-1-yl)imidazo[1,2-a]pyridin-2-yl)methyl)isoindoline-1,3-dione intermediate 355** (30 mg, 54%) as a yellow oil. ESI-MS [M +H]+: 458.2

To a solution of **intermediate 355** (30 mg, 0.066 mmol)) in EtOH (2 mL) was added NH₂NH₂-H₂O (0.2 mL). The reaction mixture was stirred at 85°C for 14 h. The resulting mixture was filtered, and the filtrate was concentrated *in vacuo* to give **intermediate 337** (200 mg, crude) as a yellow oil, which was used in next step without purification. ESI-MS [M +H]+: 328.2

### Intermediate 338: tert-butyl (6-cyclopropyl-2-((1,3-dioxoisoindolin-2-yl)methyl)imidazo[1,2-a]pyridin-8-yl)carbamate

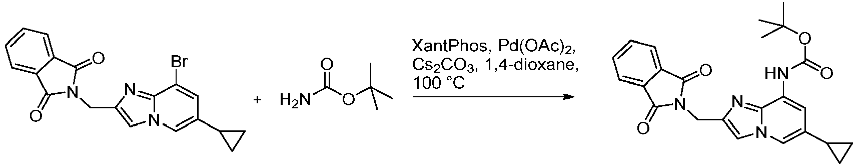

*tert*-Butyl carbamate (330 mg, 2.8 mmol), **intermediate 310** (1000 mg, 2.5 mmol), XantPhos (29 mg, 0.500 mmol), and Cs₂CO₃ (1600 mg, 5.1 mmol) were dissolved in 1,4-dioxane (15 mL) and degassed with nitrogen for 10 min. Pd(OAc)₂ (57 mg, 0.25 mmol) was added and the reaction mixture was heated in a microwave at 130°C for 3 h. The reaction mixture was cooled to room temperature, filtered through Celite^{®}, washed with methanol and concentrated *in vacuo.* The residue was purified by silica gel chromatography eluting with a gradient of 30-60 % EtOAc in cyclohexane to give **intermediare 338** (704 mg, 65 %). ESI-MS (M+H)+: 433.2, ¹H NMR (400 MHz, CDCl₃) δ 7.88 - 7.86 (m, 2H), 7.73 - 7.70 (m, 3H), 7.58 (s, 1H), 7.47 - 7.45 (m, 2H), 4.99 (s, 2H), 1.53 (s, 9H), 0.91 - 0.86 (m, 2H), 0.68 - 0.63 (m, 2H)

### Intermediate 339: N-(2-(aminomethyl)-6-cyclopropylimidazo[1,2-a]pyridin-8-yl)methanesulfonamide

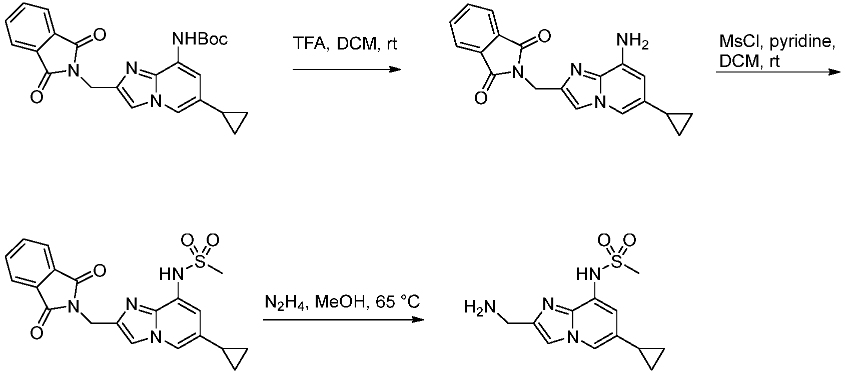

Trifluoroacetic acid (0.64 mL, 8.3 mmol) was added to a solution of **intermediate 338** (900 mg, 2.1 mmol) in DCM (10 mL). The reaction mixture was stirred at room temperature for 6 h. The reaction mixture was quenched with Na₂CO₃ (sat. aq., 20 mL) and extracted with DCM (3 × 75 mL). The combined organics were dried over a hydrophobic frit and concentrated *in vacuo* to give **2-((8-amino-6-cyclopropylimidazo[1,2-a]pyridin-2-yl)methyl)isoindoline-1,3-dione intermediate 340** (700 mg, quant.) which was used without further purification. ESI-MS (M+H)+: 333.2

Methanesulfonyl chloride (0.16 mL, 2.1 mmol) was added to a solution of **intermediate 340** (700 mg, 2.1 mmol) and pyridine (0.51 mL, 6.3 mmol) in DCM (0.3 mL) was stirred at room temperature for 16 h. The mixture was quenched with water (5 mL) and extracted with DCM (3×25 mL). The organics were dried over a hydrophobic frit and concentrated *in vacuo* to give **N-(6-cyclopropyl-2-((1,3-dioxoisoindolin-2-yl)methyl)imidazo[1,2-a]pyridin-8-yl)methanesulfonamide intermediate 341** the title compound (820 mg, 95 %). This was used without further purification. ***Synthesis of N-(2-(aminomethyl)-6-cyclopropylimidazo[1,2-a]pyridin-8-yl)methanesulfonamide***

Using a similar procedure to that for **intermediate 278,** *N*-(2-(aminomethyl)-6-cyclopropylimidazo[1,2-*a*]pyridin-8-yl)methanesulfonamide (260 mg, 95 %) was synthesised from **intermediate 341** (400 mg, 0.975 mmol) using hydrazine hydrate. ESI-MS (M+H)+: 281.1

### Intermediate 342: N-(2-(((6-chloropyrimidin-4-yl)amino)methyl)-6-cyclopropylimidazo[1,2-a]pyridin-8-yl)-N-methylacetamide

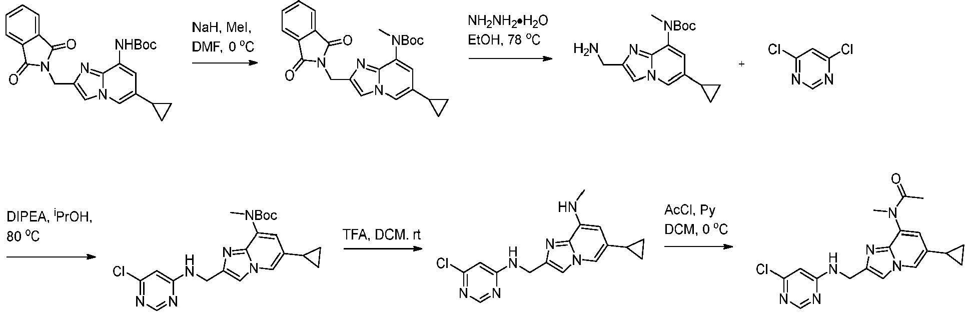

NaH (60 % in mineral oil, 55.5 mg, 1.39 mmol) was added to stirred solution of **intermediate 338** (500 mg, 1.16 mmol) in DMF (10 mL) at 0°C. The reaction was stirred at 0°C for 30 min. MeI (86 µL, 1.39 mmol) was then added and the reaction stirred at 0°C for 2 h. The reaction was quenched with water (15 mL) and extracted with EtOAc (3 x 15 mL). The organics were combined, dried over MgSO₄ and concentrated *in vacuo.* The residue was purified by silica gel chromatography, eluting with a gradient of 0-100 % EtOAc in cyclohexane to give ***tert-butyl* (6-cyclopropyl-2-((1,3-dioxoisoindolin-2-yl)methyl)imidazo[1,2-a]pyridin-8-yl)(methyl)carbamate intermediate 343** (350 mg, 68 %). ¹H NMR (400 MHz, CDCl₃) δ 7.88 (dd, J=3.0, 5.3 Hz, 2H), 7.73 (dd, J=3.0, 5.6 Hz, 2H), 7.68 (s, 1H), 7.40 (s, 1H), 6.83 - 6.78 (m, 1H), 5.06 (s, 2H), 3.34 (s, 3H), 1.88 - 1.80 (m, 1H), 1.37 (s, 9H), 0.95 - 0.89 (m, 2H), 0.64 - 0.59 (m, 2H).

Using a similar procedure to that **forintermediate 278, tert-butyl (2-(aminomethyl)-6-cyclopropylimidazo[1,2-a]pyridin-8-yl)(methyl)carbamate intermediate 344** (260 mg) was synthesised from **intermediate 343** (350 mg, 0.79 mmol) using hydrazine hydrate. ESI-MS (M+H)+: 317.4

DIPEA (0.44 mL, 2.56 mmol) was added to a stirred solution of 4,6-dichloropyrimidine (150 mg, 0.85 mmol) **intermediate 344** (260 mg, 0.85 mmol) in isopropanol (10 mL). The reaction was then heated at 80°C for 18 h. The reaction was cooled to room temperature and partitioned between water (30 mL) and EtOAc (30 mL), the layers were separated and the aqueous layer was further extracted with EtOAc (2 x 20 mL). The organics were combined, dried over MgSO₄ and concentrated *in vacuo* to give ***tert-butyl* (2-(((6-chloropyrimidin-4-yl)amino)methyl)-6-cyclopropylimidazo[1,2-a]pyridin-8-yl)(methyl)carbamate intermediate 345** (160 mg, 40 %). ESI-MS (M+H)+: 429.3, ¹H NMR (400 MHz, CDCl₃) δ 8.36 (s, 1H), 7.75 (s, 1H), 7.43 (s, 1H), 6.86 (s, 1H), 6.41 (s, 1H), 4.70 - 4.60 (m, 2H), 3.35 (s, 3H), 1.92 - 1.83 (m, 1H), 1.42 (s, 9H), 1.00 - 0.92 (m, 2H), 0.69 - 0.62 (m, 2H). One exchangeable (NH) not visible.
TFA (0.114 mL, 1.49 mmol) was added to a stirred solution of **intermediate 345** (160 mg, 0.373 mmol) in DCM (5.0 mL) and stirred at room temperature for 2 h. The reaction was quenched with NaHCO₃ solution (sat. aq., 20 mL) and extracted with DCM (3 x 20 mL), the organics were combined, dried over MgSO₄ and concentrated *in vacuo* to give **2-(((6-chloropyrimidin-4-yl)amino)methyl)-6-cyclopropyl-N-methylimidazo[1,2-a]pyridin-8-amine intermediate 346** (120 mg, 98 %). ESI-MS (M+H)+: 329.3

Acetyl chloride (26 µL, 0.36 mmol) was added to a stirred solution of **intermediate 346** (120 mg, 0.36 mmol) and pyridine (88 µL, 1.09 mmol) in DCM (1.0 mL) at 0°C. The reaction was stirred at 0°C for 1 hour. The reaction was diluted with water (10 mL) and extracted with DCM (3 x 10 mL). The organics were combined, dried over MgSO₄ and concentrated *in vacuo.* The residue was purified by silica gel chromatography, eluting with a gradient of 0-10 % (7N NH₃ in MeOH) in DCM to give **intermediate 342** (100 mg, 74 %). ESI-MS (M+H)+: 371.3, ¹H NMR (400 MHz, CDCl₃) δ 8.36 (s, 1H), 7.88 (s, 1H), 7.55 (s, 1H), 6.86 (s, 1H), 6.62 (s, 1H), 6.44 (s, 1H), 4.75 - 4.63 (m, 2H), 3.33 (s, 3H), 1.97 - 1.81 (m, 4H), 1.01 (d, J=8.1 Hz, 2H), 0.72 - 0.64 (m, 2H).

### Intermediate 347: N-(2-(aminomethyl)-6-cyclopropylimidazo[1,2-a]pyridin-8-yl)-N-methylmethanesulfonamide

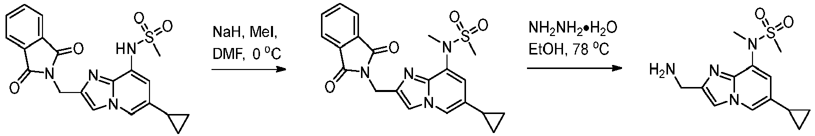

NaH (60 % in mineral oil, 23.4 mg, 0.59 mmol) was added to stirred solution of **intermediate 341** (200 mg, 0.49 mmol) in DMF (5 mL) at 0°C. The reaction was stirred at 0°C for 30 min. MeI (36 µL, 0.59 mmol) was then added and the reaction stirred at 0°C for 4 h. The reaction was quenched with water (15 mL) and extracted with EtOAc (3 x 15 mL). The organics were combined, dried over MgSO₄ and concentrated *in vacuo.* The residue was purified by silica gel chromatography, eluting with a gradient of 0-100 % EtOAc in cyclohexane to give **N-(6-cyclopropyl-2-((1,3-dioxoisoindolin-2-yl)methyl)imidazo[1,2-a]pyridin-8-yl)-N-methylmethanesulfonamide intermediate 348** (177 mg, 86 %). ESI-MS (M+H)+: 425.1, ¹H NMR (400 MHz, CDCl₃) δ 7.86 (dd, J=3.0, 5.6 Hz, 2H), 7.79 (d, J=1.0 Hz, 1H), 7.73 (dd, J=3.2, 5.4 Hz, 2H), 7.49 (s, 1H), 7.01 (d, J=1.5 Hz, 1H), 5.02 (s, 2H), 3.40 (s, 3H), 3.01 (s, 3H), 1.89 - 1.80 (m, 1H), 0.97 - 0.91 (m, 2H), 0.67 - 0.63 (m, 2H).

Using a similar procedure to that for **intermediate 278, intermediate 347** (100 mg, 81 %) was synthesised from **intermediate 348** (177 mg, 0.42 mmol) using hydrazine hydrate. ESI-MS (M+H)+: 295.1

### Intermediate 349: (6-cyclopropyl-8-(4H-1,2,4-triazol-4-yl)imidazo[1,2-a]pyridin-2-yl)methanamine

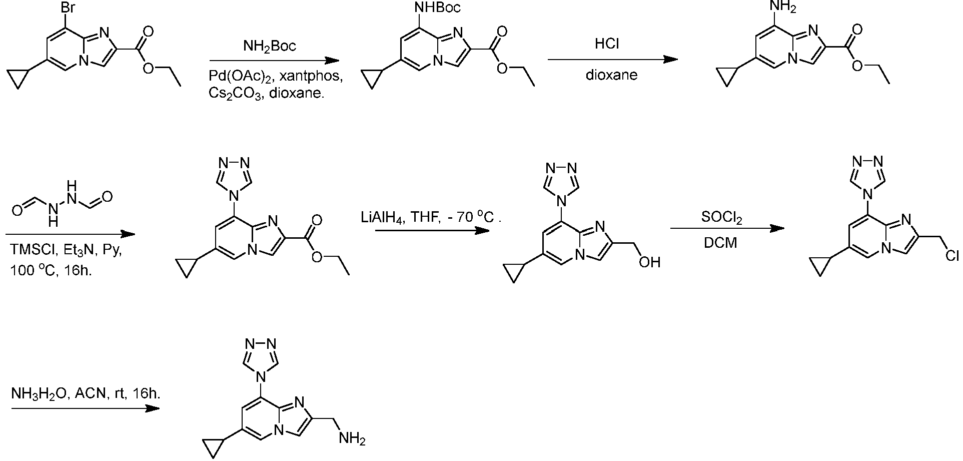

To a mixture of **intermediate 451** (1.3 g, 4.22 mmol), tert-butyl carbamate (740.7 mg, 6.33 mmol) and Cs₂CO₃ (3.4 g, 10.55 mmol) in dioxane (20 mL) was added Pd(OAc)₂ (94.5 mg, 0.42 mmol) and Xantphos (159.5 g, 0.42 mmol). The reaction mixture was stirred at 95°C for 16 h under N₂. The reaction mixture was cooled to room temperature, then filtered through celite and the filter cake was washed with DCM/MeOH (10/1, 50 mL). The filtrate was concentrated *in vacuo* to give the crude, which was purified by silica gel chromatography (eluent: DCM/MeOH=40/1) to give **ethyl 8-((tert-butoxycarbonyl)amino)-6-cyclopropylimidazo[1,2-a]pyridine-2-carboxylate intermediate 350** as a yellow solid (1.3 g, 89 %). ESI-MS [M +H]⁺: 346.2.

A solution of **intermediate 350** (1.3 g, 3.77 mmol) in HCl (4M in dioxane, 30 mL) was stirred at room temperature for 2 h. The mixture was concentrated *in vacuo* to give **ethyl 8-amino-6-cyclopropylimidazo[1,2-a]pyridine-2-carboxylate intermediate 351** (924 mg, crude) as a yellow solid, which was used for the next step directly without purification. ESI-MS [M +H]⁺: 246.1.

To a solution of **intermediate 351** (924 mg, crude), N'-formylformohydrazide (995.3 mg, 11.31 mmol) and Et₃N (2.66 g, 26.39 mmol) in pyridine (20 mL) was added TMSCl (2.85 g, 26.39 mmol) at 0°C. Then the mixture was stirred at 100°C for 16 h under N₂. After cooling to room temperature, the mixture was concentrated *in vacuo* to get the crude product, which was purified by silica gel chromatography (eluent: DCM/MeOH=50/1 ~ 40/1) to give **ethyl 6-cyclopropyl-8-(4H-1,2,4-triazol-4-yl)imidazo[1,2-a]pyridine-2-carboxylate intermediate 352** as a yellow solid (500 mg, 44%). ESI-MS [M +H]⁺: 298.2.

To a solution of **intermediate 352** (500 mg, 1.68 mmol) in THF (20.0 mL) was stirred LiAlH₄ (8.4 mL, 8.4 mmol) at - 70°C under N₂. After stirring at - 70°C for 3 h, the reaction mixture was quenched with water (20 mL) at -70°C, warmed to room temperature and stirred for 10 min. The suspension was filtered and the filtrate was concentrated *in vacuo* and purified by preparative TLC (eluent: DCM/MeOH=50/1 ~ 40/1) to give **(6-cyclopropyl-8-(4H-1,2,4-triazol-4-yl)imidazo[1,2-a]pyridin-2-yl)methanol intermediate 353** (120 mg, 28%) as a light yellow solid. ESI-MS [M +H]+: 256.1.

To a solution of **intermediate 353** (120 mg, 0.47 mmol) in DCM (10.0 mL) was added SOCl₂ (1.0 mL) at 0°C and it was stirred at room temperature for 1 h. Then the reaction mixture was concentrated *in vacuo* to give **2-(chloromethyl)-6-cyclopropyl-8-(4H-1,2,4-triazol-4-yl)imidazo[1,2-a]pyridine intermediate 354** (120 mg, crude) as a yellow oil, which was used for the next step directly without purification. ESI-MS [M +H]+: 274.1.

To a solution of **intermediate 354** (120 mg, 0.44 mmol) in CH₃CN (2.0 mL) was added NH₃ (25 wt% in water, 2.0 mL) at room temperature and stirred for 16h. The mixture was then concentrated to give **(6-cyclopropyl-8-(4H-1,2,4-triazol-4-yl)imidazo[1,2-a]pyridin-2-yl)methanamine intermediate 349** (100 mg, crude) as a yellow oil. which was used for the next step without purification. ESI-MS [M +H]+: 255.1.

### Intermediate 356: 3-(2-(aminomethyl)-6-cyclopropylimidazo[1,2-a]pyridin-8-yl)oxazolidin-2-one

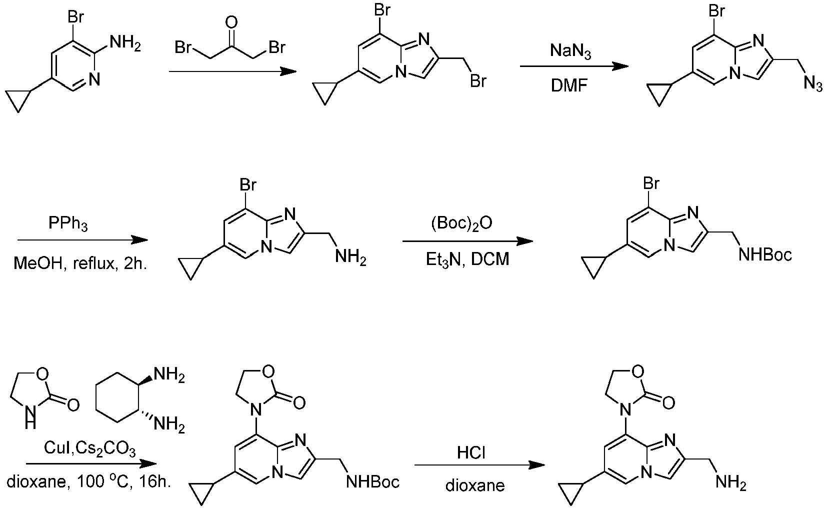

A mixture of **intermediate 309** (1.06 g, 5.0 mmol) and 1,3-dibromopropan-2-one (1.62 g, 7.5 mmol) in DME (20.0 mL) was stirred at 90°C under N₂ for 16 h. The reaction mixture was warmed to room temperature, quenched with sat. NaHCO3 solution (50 mL) and extracted with EtOAc (3 x 50 ml). The combined organics were washed with brine (50 mL), dried over Na₂SO₄ and concentrated *in vacuo.* The crude residue was purified by column chromatography (eluent: DCM/MeOH=50/1 ~ 30/1) to give the product **8-bromo-2-(bromomethyl)-6-cyclopropylimidazo[1,2-a]pyridine intermediate 357** (1.2 g, 73 %) as a yellow solid. ESI-MS [M +H]⁺: 330.2.

A mixture of **intermediate 357** (1.0 g, 3.0 mmol) and NaN₃ (244 mg, 3.75 mmol) in DMF (10.0 mL) was stirred at room temperature under N₂ for 16 h. The mixture was diluted with EtOAc (100 mL) and washed with brine (3 x 50 mL). The organic layer was dried over Na₂SO₄ and concentrated *in vacuo* to give **2-(azidomethyl)-8-bromo-6-cyclopropylimidazo[1,2-a]pyridine intermediate 358** (900 mg, crude) as a yellow solid which was used for the next step without purification. ESI-MS [M +H]⁺: 292.2.

A mixture of **intermediate 358** (870 mg, 3.0 mmol) and PPh₃ (983 mg, 3.75 mmol) in MeOH (25 mL) was stirred at reflux for 2 h. The mixture was concentrated *in vacuo* and purified by preparative TLC (eluent: DCM/MeOH = 10/1) to give **(8-bromo-6-cyclopropylimidazo[1,2-a]pyridin-2-yl)methanamine intermediate 359** (550 mg, 69 %) as a yellow oil. ESI-MS [M +H]⁺: 266.2.

A mixture of **intermediate 359** (550 mg, 2.08 mmol), (Boc)₂O (679 mg, 3.11 mmol) and Et₃N (630 mg, 6.24 mmol) in DCM (20 mL) was stirred at room temperature for 16 h. The mixture was quenched with water (50 mL) and extracted with DCM (3 x 50 ml). The combined organics were washed with brine (50 mL), dried over Na₂SO₄ and concentrated *in vacuo.* The residue was purified by preparative TLC (eluent: DCM/MeOH = 20/1) to give **tert-butyl ((8-bromo-6-cyclopropylimidazo[1,2-a]pyridin-2-yl)methyl)carbamate intermediate 360** (520 mg, 69 %) as a yellow solid. ESI-MS [M +H]⁺: 366.2.

A mixture of **intermediate 360** (100 mg, 0.27 mmol), oxazolidin-2-one (47.7 mg, 0.55 mmol), (1R,2R)-cyclohexane-1,2-diamine (30.8 mg, 0.27 mmol), CuI (51.3 mg, 0.27 mmol) and Cs₂CO₃ (179.3 mg, 0.55 mmol) in dioxane (10 mL) was stirred at 100°C under N₂ for 16 h. The reaction mixture was allowed to reach room temperature, quenched with water (50 mL) and extracted with DCM (3 x 50 ml). The combined organics were washed with brine (50 mL), dried over Na₂SO₄ and concentrated *in vacuo.* The residue was purified by preparative TLC (eluent: DCM/MeOH = 10/1) to give the product **tert-butyl ((6-cyclopropyl-8-(2-oxooxazolidin-3-yl)imidazo[1,2-a]pyridin-2-yl)methyl)carbamate intermediate 361** (50 mg, 50 %) as a light yellow solid. ESI-MS [M +H]⁺: 373.2.

A mixture of **intermediate 361** (50 mg, 0.13 mmol) in HCl/dioxane(4M, 5.0 mL) was stirred at room temperature for 2 h. The resulting solution was concentrated to give **intermediate 356** (35 mg, crude) as a yellow solid which was used for the next step directly. ESI-MS [M +H]⁺: 273.2.

Using a similar procedure to that used **forintermediate 356,** the compounds in Table A8 were prepared from **intermediate 360** and an appropriate coupling partner, followed by treatment with HCl in dioxane. **Table A8**

| | | |
|---|---|---|
| 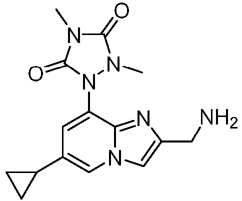 | 1-(2-(aminomethyl)-6-cyclopropyl imidazo[1,2-a]pyridin-8-yl)-2,4-dimethyl-1,2,4-triazolidine-3,5-dione hydrochloric acid salt from **1,4-dimethyl-1,2,4-triazolidine-3,5-dione** | ESI-MS [M +H]+: 315.2 |
| 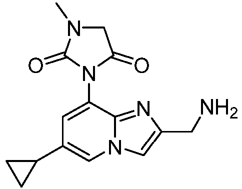 | 3-(2-(aminomethyl)-6-cyclopropylimidazo[1,2-a]pyridin-8-yl)-1-methylimidazolidine-2,4-dione from **1-methylimidazolidine-2,4-dione** | ESI-MS [M +H]+: 300.1 |

### Intermediate 362: 1-(2-(aminomethyl)-6-cyclopropylimidazo[1,2-a]pyridin-8-yl)-4-methylpiperazine-2,5-dione hydrochloride

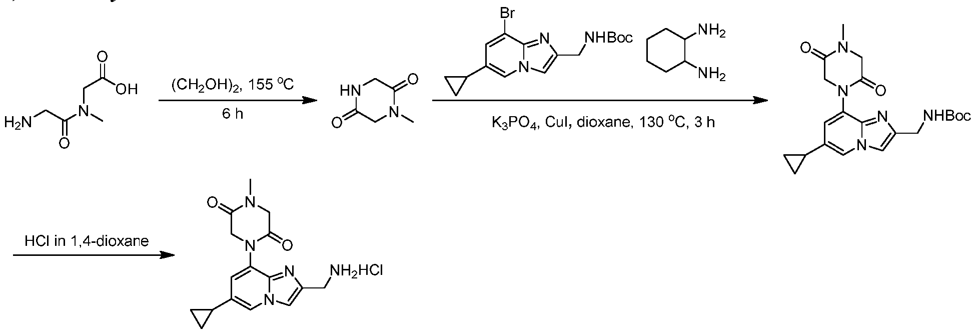

A solution of N-glycyl-N-methylglycine (1.1 g, 7.53 mmol) in (CH₂OH)₂ (10 mL) was stirred at 155°C for 6 h. The reaction was cooled to room temperature and concentrated *in vacuo* to give the crude, which was purified by silica gel chromatography (eluent: DCM/MeOH = 25/1) to give **1-methylpiperazine-2,5-dione intermediate 363** (800 mg, 83%) as a white solid. ESI-MS: [M + H]+, 129.2.

A mixture of **intermediate 360** (200 mg, 0.55 mmol), **intermediate 363** (210 mg, 1.64 mmol), cyclohexane-1,2-diamine (31 mg, 0.27 mmol), K₃PO₄ (232 mg, 1.09 mmol) and CuI (21 mg, 0.11 mmol) in 1,4-dioxane (10 ml) was stirred at 130°C for 3 h. The reaction mixture was cooled to room temperature, filtered through celite and the filter cake was washed with DCM (60 mL). The filtrate was concentrated *in vacuo* to give the crude, which was purified by silica gel chromatography (eluent: DCM/MeOH = 100/7) to give **tert-butyl ((6-cyclopropyl-8-(4-methyl-2,5-dioxopiperazin-1-yl)imidazo[1,2-a]pyridin-2-yl)methyl)carbamate intermediate 364** (60 mg, 26%) as a yellow solid. ESI-MS: [M + H]⁺, 314.2

To a solution of **intermediate 364** (50 mg, 0.12 mmol) in 1,4-dioxane (1 ml) was added HCl (4 M solution in dioxane, 1.5 ml, 6 mmol). The resulting mixture was stirred at room temperature for 1 h and concentrated *in vacuo* to give **intermediate 362** (60 mg, 100%) which was used to the next step without further purification. ESI-MS: [M + H]+, 314.2

### Intermediate 365: 1-(2-(aminomethyl)-6-cyclopropylimidazo[1,2-a]pyridin-8-yl)-3-methylimidazolidine-2,4-dione

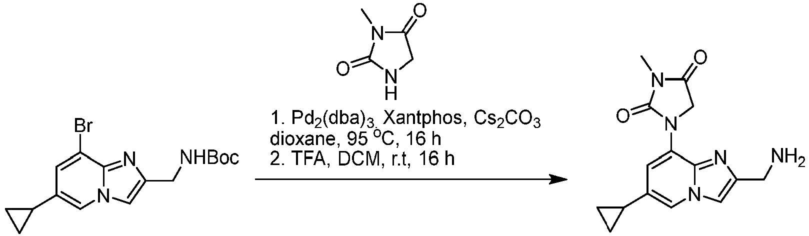

To a mixture of **intermediate 322** (224 mg, 2 mmol), **intermediate 360** (365 mg, 1 mmol) and Cs₂CO₃ (975 mg, 3 mmol) in dry 1,4-dioxane (10 mL) was added Pd₂(dba)₃ (183 mg, 0.2 mmol) and XantPhos (116 mg, 0.2 mmol). The reaction was stirred at 100°C for 16 h under N₂, quenched with water (50 mL) and extracted with EtOAc (30 mL x 3). The organic layers were washed with brine (30 mL), dried with Na₂SO₄, concentrated *in vacuo* and purified by silica gel chromatography (eluent: DCM/MeOH = 10/1)to give **tert-butyl ((6-cyclopropyl-8-(3-methyl-2,4-dioxoimidazolidin-1-yl)imidazo[1,2-a]pyridin-2-yl)methyl)carbamate intermediate 366** (150 mg, 37%) as a yellow solid. ESI-MS [M +H]⁺: 400.0.

To a mixture of **intermediate 366** (500 mg, 1.25 mmol) in dry DCM (10 mL) was added TFA (2 ml). The reaction mixture was stirred at room temperature for 16 h under N₂ and then neutralized with methanolic ammonia solution (7M). The resulting solution was concentrated *in vacuo* and purified by silica gel chromatography (eluent: DCM/MeOH = 10/1) to give **intermediate 365**(150 mg, 40%) as a white solid. ESI-MS [M +H]⁺: 300.1. Using a similar procedure to that used for **intermediate 365,** the compounds in Table A9 were prepared from **intermediate 360** and an appropriate coupling partner, followed by treatment with TFA in dichloromethane or HCl in dioxane. **Table A9**

| **Structure** | **Coupling Partner** | **Analytical Data** |
|---|---|---|
| 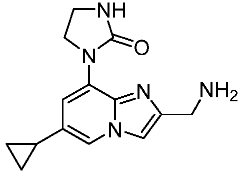 | 1-(2-(aminomethyl)-6-cyclopropyl imidazo[1,2-a]pyridin-8-yl) imidazolidin-2-one hydrochloride from **imidazolidin-2-one** | ESI-MS: [M + H]+, 272.2 |
| 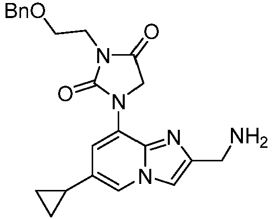 | 1-(2-(aminomethyl)-6-cyclopropyl imidazo[1,2-a]pyridin-8-yl)-3-(2-(benzyloxy)ethyl)imidazolidine-2,4-dione hydrochloride from **intermediate 373** | ESI-MS [M + H]⁺: 420.2 |
| 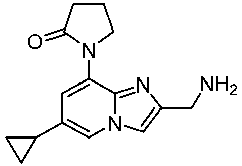 | 1-(2-(aminomethyl)-6-cyclopropyl imidazo[1,2-a]pyridin-8-yl)pyrrolidin-2-one hydrochloride from **pyrrolidin-2-one** | ESI-MS [M +H]⁺: 271.1 |
| 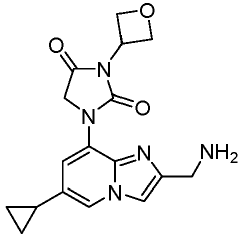 | 1-(2-(aminomethyl)-6-cyclopropyl imidazo[1,2-a]pyridin-8-yl)-3-(oxetan-3-yl)imidazolidine-2,4-dione from **intermediate 374** | ESI-MS [M +H] +: 342.2 |
| 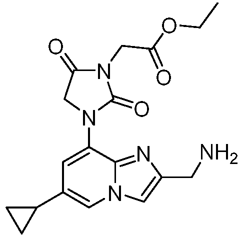 | ethyl 2-(3-(2-(aminomethyl)-6-cyclo propylimidazo[1,2-a]pyridin-8-yl)-2,5-dioxoimidazolidin-1-yl)acetate from **intermediate 375** | ESI-MS [M +H]⁺: 372.2 |
| 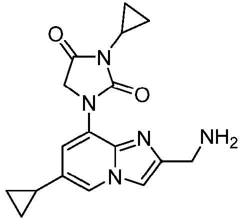 | 1-(2-(aminomethyl)-6-cyclopropyl imidazo[1,2-a]pyridin-8-yl)-3-cyclopropylimidazolidine-2,4-dione hydrochloride from **intermediate 376** | ESI-MS [M +H]+: 326.2 |
| 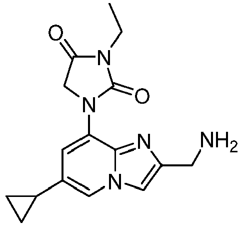 | 1-(2-(aminomethyl)-6-cyclopropyl imidazo[1,2-a]pyridin-8-yl)-3-ethylimidazolidine-2,4-dione hydrochloride from 3-ethylimidazolidine-2,4-dione | ESI-MS [M +H]⁺: 314.2 |
| 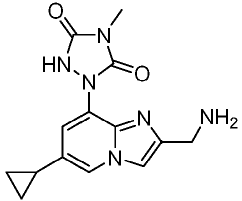 | 1-(2-(aminomethyl)-6-cyclopropyl imidazo[1,2-a]pyridin-8-yl)-4-methyl-1,2,4-triazolidine-3,5-dione hydrochloride from **4-methyl-1,2,4-triazolidine-3,5-dione** | ESI-MS [M +H]+: 301.2 |
| 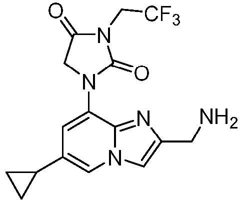 | 1-(2-(aminomethyl)-6-cyclopropyl imidazo[1,2-a]pyridin-8-yl)-3-(2,2,2-trifluoroethyl)imidazolidine-2,4-dione from **intermediate 378** | ESI-MS [M +H]⁺: 368.2 |
| 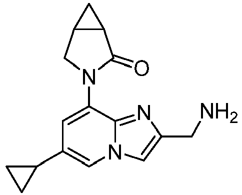 | 3-(2-(aminomethyl)-6-cyclo propylimidazo[1,2-a]pyridin-8-yl)-3-azabicyclo[3.1.0]hexan-2-one hydrochloride from **3-azabicyclo[3.1.0]hexan-2-one** | ESI-MS [M +H]+: 283.2 |
| 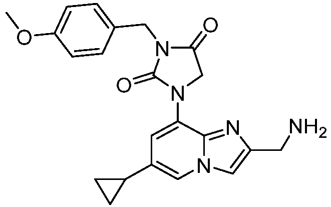 | 1-(2-(aminomethyl)-6-cyclopropyl imidazo[1,2-a]pyridin-8-yl)-3-(4-methoxybenzyl)imidazolidine-2,4-dione hydrochloride from **intermediate 379** | ESI-MS: [M + H]+, 406.2 |
| 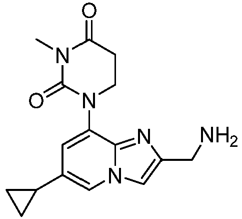 | 1-(2-(aminomethyl)-6-cyclopropyl imidazo[1,2-a]pyridin-8-yl)-3-methyl dihydropyrimidine-2,4(1H,3H)-dione hydrochloride from **intermediate 380** | ESI-MS [M +H]+: 314.1 |
| 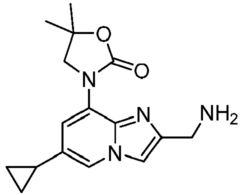 | 3-(2-(aminomethyl)-6-cyclopropyl imidazo[1,2-a]pyridin-8-yl)-5,5-di methyloxazolidin-2-one hydrochloride from **5,5-dimethyloxazolidin-2-one** | ESI-MS [M +H]+: 301.2 |

### Intermediate 367: 1-(2-(aminomethyl)-6-cyclopropyl-3-fluoroimidazo[1,2-a]pyridin-8-yl)-3-methylimidazolidine-2,4-dione

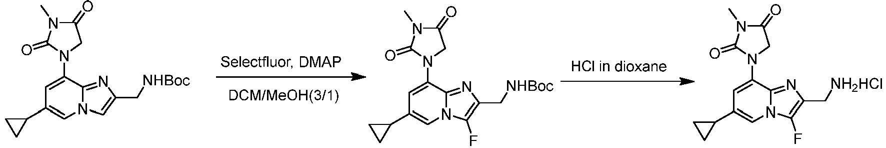

A mixture of **intermediate 366** (200 mg, 0.5 mmol), Selectfluor (354 mg, 1.0 mmol) and DMAP (61 mg, 0.5 mmol) in DCM/MeOH (3.0 mL/1.0 mL) was stirred at room temperature for 16h. The reaction was concentrated *in vacuo* to give the crude product, which was purified by silica gel chromatography (eluent: DCM/MeOH=30/1 ~ 10/1) to give **tert-butyl ((6-cyclopropyl-3-fluoro-8-(3-methyl-2,4-dioxoimidazolidin-1-yl)imidazo[1,2-a]pyridin-2-yl)methyl)carbamate intermediate 368** as a white solid (120 mg, 58%). ESI-MS [M +H]⁺: 418.2.

A mixture of **intermediate 368** (84 mg, 0.2 mmol) in HCl (4 M in dioxane, 5 mL) was stirred at room temperature for 1 h. The reaction mixture was concentrated *in vacuo* to give **intermediate 367** as the hydrochloric acid salt (80 mg, crude) as a white solid, which was used directly in the next step. ESI-MS [M +H]⁺: 318.2.

### Intermediate 369: 1-(2-(aminomethyl)-6-(1-fluorocyclopropyl)imidazo[1,2-a]pyridin-8-yl)-3-methylimidazolidine-2,4-dione

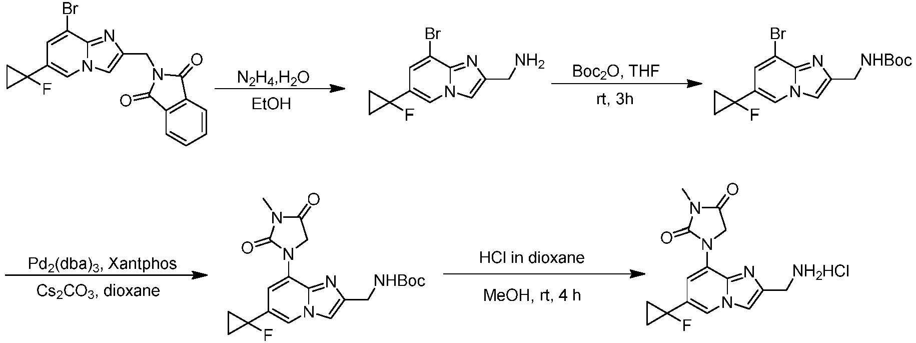

To a solution of 2-((8-bromo-6-(1-fluorocyclopropyl)imidazo[1,2-a]pyridin-2-yl)methyl)isoindoline-1,3-dione (200 mg, 0.48 mmol) in EtOH (10 mL) was added N₂H₄⁻H₂O (1 mL). The reaction mixture was stirred at 65°C for 2 h and then allowed to reach room temperature. The solution was filtered and the filtrate was concentrated *in vacuo* to give **(8-bromo-6-(1-fluorocyclopropyl)imidazo[1,2-a]pyridin-2-yl)methanamine intermediate 370** (100 mg, crude) as a white solid, which was used in the next step without further purification. ESI-MS [M +H]⁺: 284.1.

To a solution of **intermediate 370** (100 mg, crude) in dry THF (10 mL ) was added Boc₂O (152 mg, 0.70 mmol) and trimethylamine (106 mg, 1.05 mmol). After stirring for 3 h at room temperature, water (10 mL) was added and the mixture was extracted with EtOAc (3 x 10 mL). The combined organic layers were washed with brine (20 mL), dried over anhydrous Na₂SO₄ then concentrated to give the crude, which was purified by preparative TLC (eluent: PE/EtOAc = 3/1) to give **tert-butyl ((8-bromo-6-(1-fluorocyclopropyl)imidazo[1,2-a]pyridin-2-yl)methyl)carbamate intermediate 371** (120 mg, 65% in 2 steps) as a white solid. ESI-MS [M +H]⁺: 384.2.

To a mixture of **intermediate 371** (120 mg, 0.31 mmol), **intermediate 322** (71 mg, 0.62 mmol), and Cs₂CO₃ (303 mg, 0.93 mmol) in dioxane (10 mL) was added Pd₂(dba)₃ (28 mg, 0.031mmol) and Xantphos (18 mg, 0.031 mmol). The reaction mixture was stirred at 90°C for 16 h under N₂ and cooled to room temperature. The reaction mixture was filtered through celite and the filter cake was washed with DCM/MeOH (10/1, 20 mL). The filtrate was concentrated *in vacuo* to give the crude, which was purified by silica gel chromatography (eluent: DCM/MeOH=50/1 ~ 20/1) to give **intermediate 372** (100 mg, 77%) as a yellow solid. ESI-MS [M +H]+: 418.2.

To a solution of **intermediate 372** (100 mg, 0.24 mmol) in MeOH (5 mL) was added HCl (4M solution in dioxane, 5 mL). The resulting mixture was stirred at room temperature for 4 h and then concentrated *in vacuo* to give **intermediate 369** as the hydrochloric acid salt (80 mg, 85%) as a yellow oil which was used in next step without purification. ESI-MS [M +H]+: 318.1.

### Intermediate 373: 3-(2-(benzyloxy)ethyl)imidazolidine-2,4-dione

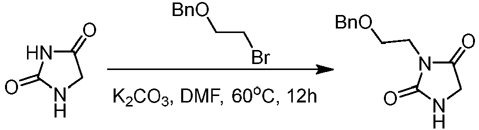

A solution of imidazolidine-2,4-dione (500 mg, 5 mmol), ((2-bromoethoxy)methyl)benzene (1.18 g, 5.5 mmol) and K₂CO₃ (1.38 g, 10 mmol) in DMF (10 mL) was stirred at 60°C for 12 h. The reaction mixture was cooled to room temperature, poured into water (50 mL) and extracted with EtOAc (2 x 50 ml). The combined organic layers were washed with brine (100 mL), dried over Na₂SO₄ then concentrated *in vacuo* to give the crude product, which was purified by silica gel chromatography (eluent: EtOAc/PE = 1/1) to give **intermediate 373** (720 mg, 62%) as a white solid. ESI-MS [M +H]⁺: 235.1.

### Intermediate 374: 3-(oxetan-3-yl)imidazolidine-2,4-dione

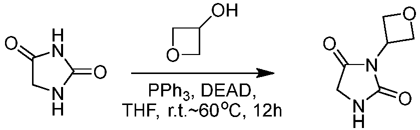

To a solution of imidazolidine-2,4-dione (3 g, 30 mmol), oxetan-3-ol (6.66 g, 90 mmol) and PPh₃ (15.78 g, 60 mmol) in THF (50 mL) was added DEAD (10.44 g, 60 mmol). The resulting mixture was stirred at 60°C for 12h, then water (100 mL) was added. The mixture was extracted with EtOAc (3 x 80 mL). The combined organic layers were washed with brine (80 mL), dried over Na₂SO₄ and concentrated *in vacuo* to give the crude product, which was purified with silica gel chromatography (eluent: DCM/MeOH = 15/1) to give **intermediate 374** (1 g, 21%) as a yellow solid. ESI-MS [M +H]⁺:157.2.

### Intermediate 375: ethyl 2-(2,5-dioxoimidazolidin-1-yl)acetate

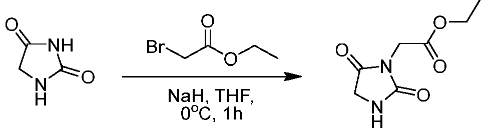

To a solution of imidazolidine-2,4-dione (2.0 g, 20.0 mmol) in dry THF (50 mL) was added NaH (1.2 g, 30 mmol) at 0°C. The mixture was stirred for 0.5 h, then ethyl 2-bromoacetate (3.3 g, 20.0 mmol) was added. The reaction was stirred at room temperature for another 1 h, then quenched with sat. NH₄Cl solution (50 mL) and extracted with EtOAc (2 x 50 mL). The combined organic layers were washed with brine (50 mL), dried over anhydrous Na₂SO₄ and concentrated inn vacuo to give the crude product, which was purified by silica gel chromatography (eluent: DCM/MeOH=10/1) to give **intermediate 375** (500 mg, 13%) as a brown oil. ESI-MS [M +H] ⁺: 187.2.

### Intermediate 376: 3-cyclopropylimidazolidine-2,4-dione

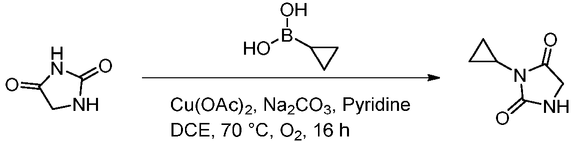

To a mixture of imidazolidine-2,4-dione (1 g, 10 mmol), cyclopropylboronic acid (903 mg, 10.5 mmol), Cu(OAc)₂ (3.73 g, 20.5 mmol), and Na₂CO₃ (2.17 g, 20.5 mmol) in DCE (100 mL) was added pyridine (3.95 g, 50 mmol). After stirring at 70°C for 16 h under O₂, the reaction mixture was filtered through celite and the filter cake was washed with DCM/MeOH (10/1, 100 mL). The filtrate was concentrated *in vacuo* to give the crude product, which was purified by silica gel chromatography (eluent: MeOH/DCM=1/100 ~ 1/5) to afford **intermediate 376** (190 mg, 14%) as white solid. ESI-MS [M+H]+: 141.2 .

### Intermediate 378: 3-(2,2,2-trifluoroethyl)imidazolidine-2,4-dione

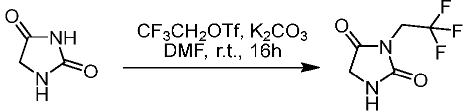

To a mixture of imidazolidine-2,4-dione (1 g, 10 mmol) in DMF (30 mL) was added K₂CO₃ (2.76 g, 20 mmol) and CF₃CH₂OTf (2.55 g, 11 mmol). The reaction was stirred at room temperature under N₂ for 16 h. The reaction was poured into water (50 ml) and extracted with EtOAc (3 x 50 ml). The combined organic layers were washed with brine (50 mL), dried over anhydrous Na₂SO₄ and concentrated *in vacuo* to give the crude, which was purified by silica gel chromatography (eluent: DCM/MeOH = 40/1) to give the **intermediate 378** (1 g, 55%) as yellow solid. ESI-MS [M +H]⁺: 183.0.

### Intermediate 379: 3-(4-methoxybenzyl)imidazolidine-2,4-dione

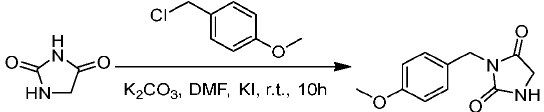

A mixture of imidazolidine-2,4-dione (1.0 g, 10 mmol), 1-(chloromethyl)-4-methoxybenzene (1.56 g, 10 mmol), K₂CO₃ (2.07 g, 15 mmol) and KI (166 mg, 1 mmol) in DMF (40 ml) was stirred at rt for 10 h. The reaction was quenched with water (50 mL) and extracted with EtOAc (3 x 50 mL). The combined organic layers were washed with brine (50 mL), dried over anhydrous Na₂SO₄ and concentrated *in vacuo* to give the crude product, which was purified by silica gel chromatography (eluent: PE/EtOAc = 1/1) to give **intermediate 379** (1.0 g, 45%) as a yellow solid. ESI-MS: [M + H]+, 221.2.

### Intermediate 380: 3-methyldihydropyrimidine-2,4(1H,3H)-dione

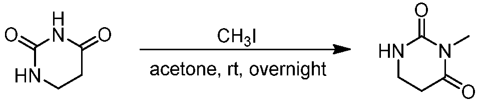

To a mixture of dihydropyrimidine-2,4(1H,3H)-dione (500 mg, 4.38 mmol) in acetone (5 mL) was added K₂CO₃ (1.09 g, 7.89 mmol) and iodomethane (1.12 g, 7.89 mmol) successively. After stirring at room temperature overnight, the resulting mixture was filtered through celite and the filter cake was washed with DCM/MeOH (10/1, 15 mL). The filtrate was concentrated *in vacuo* to give the crude, which was purified by was purified by silica gel chromatography (eluent: MeOH/DCM = 1/100 ~ 20/1) to give **intermediate 380** (44 mg, 8%) as white solid. ESI-MS [M +H]+: 129.1.

### Intermediate 381: (6-cyclopropyl-8-(4-methylpiperazin-1-yl)imidazo[1,2-a]pyridin-2-yl)methanamine

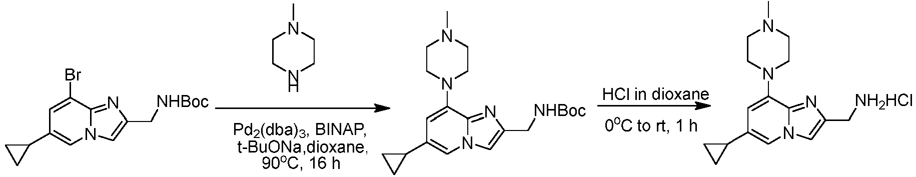

To a mixture of tert-butyl ((7-bromo-5-cyclopropylpyrazolo[1,5-a]pyridin-2-yl)methyl)carbamate (2 g, 5.5 mmol), 1-methylpiperazine (1.64 g, 16.4 mmol) and t-BuONa (1.58 g, 16.4 mmol) in 1,4-dioxane (50 mL) was added Pd₂(dba)₃ (504 mg, 0.55 mmol) and Xantphos (636 mg, 1.1 mmol). After stirring at 90°C for 16 h under N₂, the reaction mixture was filtered through celite and the filter cake was washed with DCM/MeOH (10/1, 50 mL). The filtrate was concentrated *in vacuo* to give the crude product, which was purified by silica gel chromatography (eluent: DCM/ MeOH = 10/1) to give **tert-butyl ((6-cyclopropyl-8-(4-methylpiperazin-1-yl)imidazo[1,2-a]pyridin-2-yl)methyl)carbamate intermediate 382** (900 mg, 43%) as yellow solid. ESI-MS [M + H]⁺: 386.2.

To a solution of **intermediate 382** (900 mg, 2.3 mmol) in dioxane (10 mL) was added HCl (4M solution in dioxane, 10 mL). The resulting mixture was stirred at room temperature for 1 h and then concentrated *in vacuo* to give **intermediate 381** as the hydrochloric acid salt (920 mg, crude) as a yellow solid, which was used in the next step without further purification. ESI-MS [M + H]⁺: 286.2. Using a similar procedure to that used for**intermediate 381,** the compounds in Table A10 were prepared from **intermediate 360** and an appropriate coupling partner, followed by treatment HCl in dioxane.

**Table A10**

| | | |
|---|---|---|
| 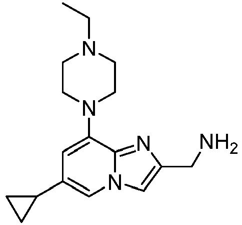 | (6-cyclopropyl-8-(4-ethylpiperazin-1-yl)imidazo[1,2-a]pyridin-2-yl)methanamine hydrochloride from **1-ethylpiperazine** | ESI-MS [M +H]+: 300.1 |
| 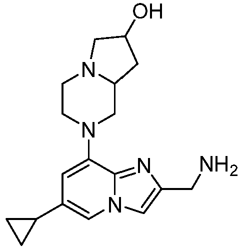 | 2-(2-(aminomethyl)-6-cyclopropylimidazo[1,2-a]pyridin-8-yl)octahydropyrrolo[1,2-a]pyrazin-7-ol hydrochloride from **octahydropyrrolo[1,2-a]pyrazin-7-ol** | ESI-MS [M +H]+: 327.2 |
| 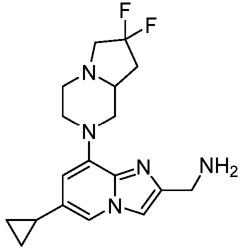 | (6-cyclopropyl-8-(7,7-difluorohexahydropyrrolo[1,2-a]pyrazin-2(1H)-yl)imidazo[1,2-a]pyridin-2-yl)methanamine hydrochloride from 7,7-**difluorooctahydro pyrrolo[1,2-a]pyrazine** | ESI-MS [M +H]+: 163.2 |
| 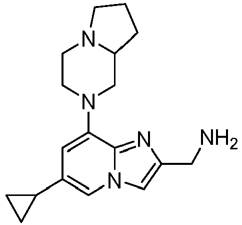 | (6-cyclopropyl-8-(hexahydropyrrolo[1,2-a]pyrazin-2(1H)-yl) imidazo[1,2-a]pyridin-2-yl)methanamine hydrochloride from **octahydropyrrolo[1,2-a]pyrazine** | ESI-MS [M +H]⁺: 312.2 |

### Intermediate 383: 1-(2-(aminomethyl)-6-cyclopropylimidazo[1,2-a]pyridin-8-yl)-3-(2-(2-methoxyethoxy)ethyl)imidazolidine-2,4-dione

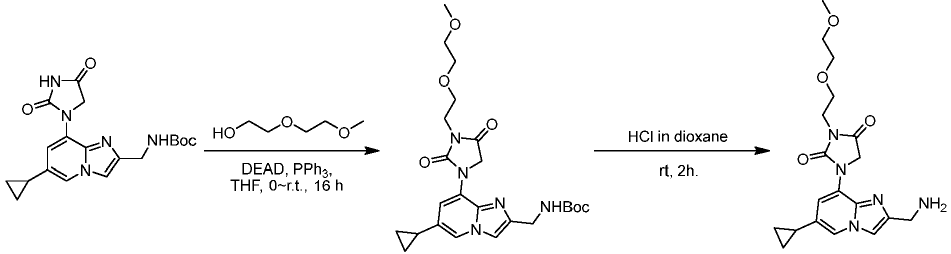

To a mixture of tert-butyl ((6-cyclopropyl-8-(2,4-dioxoimidazolidin-1-yl)imidazo[1,2-a]pyridin-2-yl)methyl)carbamate (145 mg, 0.38 mmol) and PPh₃ (199 mg, 0.76 mol) in THF (10 mL) was added 2-(2-methoxyethoxy)ethan-1-ol (91 mg, 0.76 mol) at 0°C. Diethylazodicarboxylate (198 mg, 1.14 mmol) was added dropwise to the reaction and the mixture was stirred at room temperature under N₂ for 16h. The reaction was concentrated *in vacuo* to give the crude, which was purified by preparative TLC (eluent: DCM/MeOH: 15/1) to give ***tert-butyl* ((6-cyclopropyl-8-(3-(2-(2-methoxyethoxy)ethyl)-2,4-dioxoimidazolidin-1-yl)imidazo[1,2-a]pyridin-2-yl)methyl)carbamate** (50 mg, 27 %) as yellow oil. ESI-MS [M +H]⁺: 488.2.

To a solution of tert-butyl ((6-cyclopropyl-8-(3-(2-(2-methoxyethoxy)ethyl)-2,4-dioxo imidazolidin-1-yl)imidazo[1,2-a]pyridin-2-yl)methyl)carbamate (50 mg, 0.1 mol) in dioxane (2 mL) was added HCl (4M solution in dioxane, 2 mL). After stirring at room temperature for 2 h, the mixture was basified (pH 8) with sat. aqueous NaHCO₃ solution then extracted with DCM (3 x 20 mL). The combined organic layers were washed with brine (30 mL), dried over anhydrous Na₂SO₄ and concentrated *in vacuo* to give the crude product, which was purified with preparative TLC (eluent: DCM/MeOH = 10/1) to afford **intermediate 383** (38 mg, 98 %) as yellow solid. ESI-MS [M +H]⁺: 388.2.

### Intermediate 384:1-((2-(aminomethyl)-6-cyclopropylimidazo[1,2-a]pyridin-8-yl)methyl)-3-methyl imidazolidine-2,4-dione hydrochloride

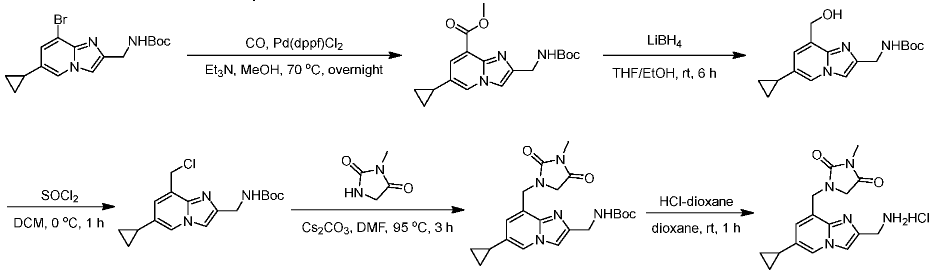

To a mixture of **intermediate 360** (500 mg, 1.37 mmol) and Et₃N (693 mg, 6.85 mmol) in MeOH (15 ml) was added Pd(dppf)Cl₂ (102 mg, 0.14 mmol). The mixture was stirred at 70°C under CO overnight and cooled to room temperature. The reaction mixture was filtered and the filtrate was concentrated *in vacuo* to give the crude, which was purified by silica gel chromatography (eluent: PE/EtOAc=1/2) to give **methyl 2-(((tert-butoxycarbonyl)amino)methyl)-6-cyclopropylimidazo[1,2-a]pyridine-8-carboxylate intermediate 385** (410 mg, 87%) as an orange solid. ESI-MS [M + H ]⁺: 346.2.

To a solution of **intermediate 385** (420 mg, 1.22 mmol) in THF/EtOH (5/1, 24 mL) was added LiBH₄ (80 mg, 3.67 mmol). The reaction mixture was stirred at room temperature for 6 h, then quenched with saturated NH₄Cl (10 mL) and extracted with EtOAc (3 x 20 mL). The combined organic layers were washed with brine (20 mL), dried over anhydrous Na₂SO₄, and concentrated *in vacuo* to give **tert-butyl ((6-cyclopropyl-8-(hydroxymethyl)imidazo[1,2-a]pyridin-2-yl)methyl)carbamate intermediate 386** (397 mg, crude) as a yellow solid. ESI-MS [M + H ]⁺: 318.2.

To a solution of **intermediate 386** (350 mg, 1.10 mmol) in DCM (3.5 mL) was added SOCl₂ (131 mg, 1.10 mmol) at 0°C for 1 h. The resulting mixture was concentrated *in vacuo* to give **tert-butyl ((8-(chloromethyl)-6-cyclopropylimidazo[1,2-a]pyridin-2-yl)methyl)carbamate intermediate 387** (413 mg, crude) as a yellow solid which was used in next step without purification. ESI-MS [M + H ]⁺: 336.2.

A mixture of tert-butyl ((8-(chloromethyl)-6-cyclopropylimidazo[1,2-a]pyridin-2-yl)methyl) carbamate (413 mg, crude), **intermediate 322** (146 mg, 1.28 mmol), and Cs₂CO₃ (1.251 g, 3.84 mmol) in DMF (10.0 ml) was stirred in a sealed tube at 95°C for 3 h. The resulting mixture was cooled to room temperature and concentrated *in vacuo* to give the crude, which was purified by column chromatography (eluent: DCM/MeOH=10/1) to give **tert-butyl ((6-cyclopropyl-8-((3-methyl-2,4-dioxoimidazolidin-1-yl)methyl)imidazo[1,2-a]pyridin-2-yl)methyl)carbamate intermediate 388** (82 mg, 18%) as a yellow solid. ESI-MS [M + H ]⁺: 414.2.

To a solution of **intermediate 388** (82 mg, 0.20 mmol) in dioxane (2 ml) was added HCl (4 M solution in dioxane, 2 mL, 0.8 mmol) at room temperature. After stirring the resulting mixture for 1 h, the mixture was concentrated *in vacuo* to give **intermeidate 384** (83 mg, crude) as a yellow solid which was used in the next step without purification. ESI-MS [M + H ]⁺: 350.1.

### Intermediate 389: 1-(6-cyclopropyl-2-((methylamino)methyl)imidazo[1,2-a]pyridin-8-yl)-3-methylimidazolidine-2,4-dione

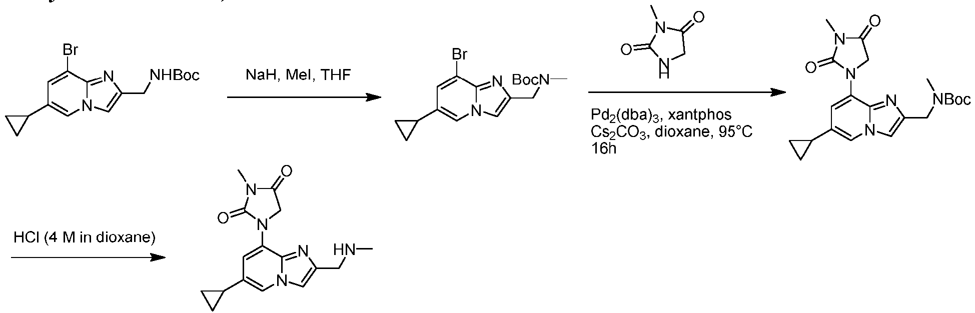

To a solution of **intermediate 360** (200 mg, 0.55 mmol) in THF (6 mL) was added NaH (32.9 mg, 60% in oil, 0.82 mmol) at 0°C. The reaction mixture was stirred at 0°C for 0.5 h under N₂ and then a solution of MeI (116.4 mg, 0.82 mmol) in THF (1 mL) was added. After stirring the mixture at room temperature for 2h, the mixture was quenched with sat. aq. NH₄Cl solution (30 mL) and extracted with EtOAc (3 x 30 mL). The combined organic layers were washed with brine (20 mL), dried over Na₂SO₄ and concentrated. The crude residue was purified by preparative TLC (eluent: PE/EtOAc = 2/1) to give **intermediate 390 (170 mg, 82%) as a white solid. ESI-MS [M +H]+: 380.2.**

To a mixture of **intermediate 390** (170 mg, 0.45 mmol), **intermediate 322** (154 mg, 1.35 mmol), and Cs₂CO₃ (440 mg, 1.35 mmol) in dioxane (5 mL) was added Pd₂(dba)₃ (41 mg, 0.045 mmol) and XantPhos (52 mg, 0.09 mmol). The reaction mixture was stirred at 95°C for 16 h under N₂. The reaction mixture was cooled to room temperature, filtered through Celite^{®} and the filter cake was washed with DCM/MeOH (10/1, 20 mL). The filtrate was concentrated to give the crude residue, which was purified by preparative TLC (eluent: DCM/MeOH = 20/1) to give **tert-butyl ((6-cyclopropyl-8-(3-methyl-2,4-dioxoimidazolidin-1-yl)imidazo[1,2-a]pyridin-2-yl)methyl)(methyl)carbamate intermediate 391** (80 mg, 43%) as a white solid. ESI-MS [M +H]+: 414.2.

### Intermediate 392: 1-(6-cyclopropyl-2-((methylamino)methyl)imidazo[1,2-a]pyridin-8-yl)imidazolidine-2,4-dione

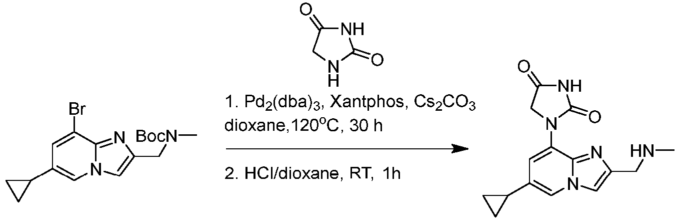

To a solution of **intermediate 390** (1.14 g, 3 mmol) in dioxane (30 mL) were added imidazolidine-2,4-dione (1.2 g, 12 mmol), Cs₂CO₃ (2.93 g, 9 mmol), Xantphos (520 mg, 0.9 mmol) and Pd₂(dba)₃ (411 mg, 0.45 mmol) under N₂. The resulting reaction was then stirred at 120°C for 16 h and cooled to room temperature. The reaction mixture was poured into water (50 mL) and extracted with EtOAc (3 x 50 ml). The combined organics were washed with brine (50 mL), dried over Na₂SO₄ then concentrated to give the crude product, which was purified by silica gel chromatography (eluent: EtOAc /PE = 4/1) to give **intermediate 393** (650 mg, 54%) as a yellow solid. ESI-MS [M + H]⁺: 400.2.

To a solution of **intermediate 393**(650 mg, 1.63 mmol) in MeOH (10 mL) was added HCl/dioxane (10 ml, 6 mol/L in dioxane). The resulting reaction was stirred at room temperature for 1 h and then concentrated *in vacuo* to give intermediate 392 (650 mg, crude) as a yellow solid which was used in next step without purification. ESI-MS [M + H]⁺: 300.2.
Using a similar procedure to that used for**intermediate 392,** the compounds in Table A11 were prepared from **intermediate 393** and an appropriate coupling partner, followed by treatment with HCl in dioxane. **Table A11**

| | |
|---|---|
| 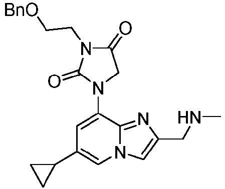 | 13-(2-(benzyloxy)ethyl)-1-(6-cyclopropyl-2-((methylamino) methyl)imidazo[1,2-a]pyridin-8-yl)imidazolidine-2,4-dione hydrochloride ESI-MS [M + H]⁺: 434.2 |
| | From **3-(2-(benzyloxy) ethyl)imidazolidine-2,4-dione** |

### Intermediate 394: 1-(6-cyclopropyl-2-((ethylamino)methyl)imidazo[1,2-a]pyridin-8-yl)-3-methylimidazolidine-2,4-dione

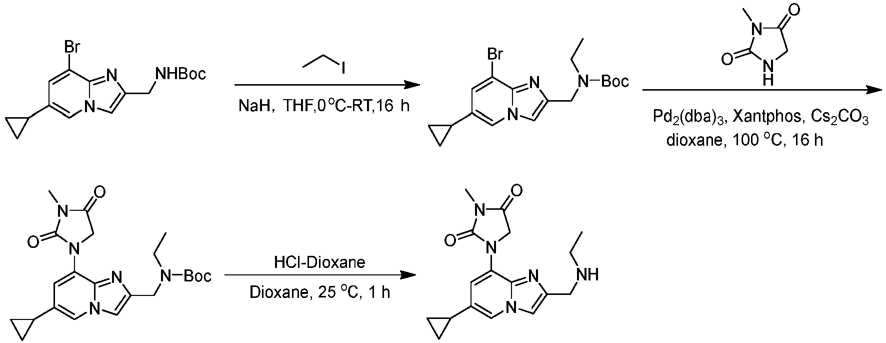

Using a similar procedure to that for**intermediate 391, intermediate 394** (60 mg) was synthesised from **intermediate 360** and ethyl iodide. ESI-MS [M +H]+: 328.2.

### Intermediate 395: benzyl ((6-cyclopropyl-8-(3-methyl-2,4-dioxoimidazolidin-1-yl)imidazo[1,2-a]pyridin-2-yl)methyl)glycinate

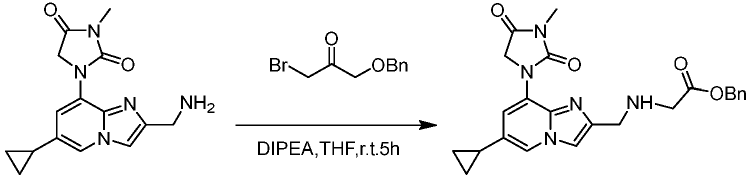

To a solution of 1-(2-(aminomethyl)-6-cyclopropylimidazo[1,2-a]pyridin-8-yl)-3-methylimidazolidine-2,4-dione (299 mg, 1.0 mmol) and DIPEA (645 mg, 5.0 mmol) in THF (20 mL) was added 1-(benzyloxy)-3-bromopropan-2-one (242 mg, 1.0 mmol) at 0°C. The mixture was stirred at room temperature for 5h, quenched with water (30 mL) and extracted with EtOAc (3 x 10 mL). The combined organic layers were washed with brine (20 mL), dried over anhydrous Na₂SO₄ and concentrated *in vacuo* to give the crude product, which was purified purified with preparative TLC (eluent: DCM/MeOH = 10/1) to give the product **intermediate 395** as a yellow oil (190 mg, 42%). ESI-MS [M +H]+: 448.1.

### Intermediate 396: 1-(6-cyclopropyl-2-((methylamino)methyl)imidazo[1,2-a]pyridin-8-yl)-3-(2-hydroxy-2-methylpropyl)imidazolidine-2,4-dione

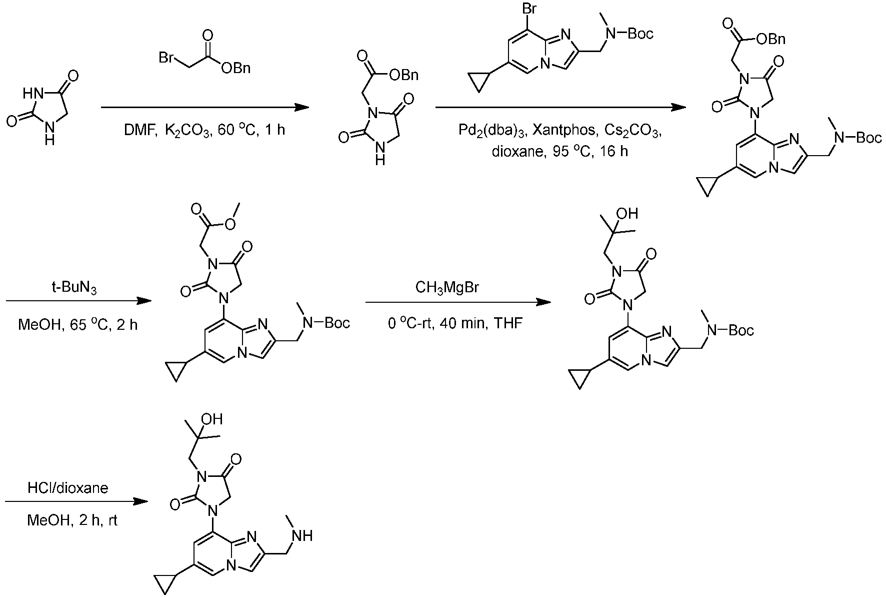

To a solution of imidazolidine-2,4-dione (2 g, 20.0 mmol) in DMF (20 mL) was added benzyl 2-bromoacetate (3.2 g, 14.0 mmol) and K₂CO₃ (5.5 g, 40.0 mmol). After stirring the mixture was stirred at 60°C for 1 h, quenched with water (50 mL) and extracted with EtOAc (3 x 30 mL). The combined organics were washed with brine (50 mL), dried over Na₂SO₄ and concentrated *in vacuo* to give **benzyl 2-(2,5-dioxoimidazolidin-1-yl)acetate intermediate 397** (2.5 g, 50.4%) as a white solid. ESI-MS [M +Na]⁺: 271.1.

A mixture of **-intermediate 397** (2.35 g, 9.5 mmol), **intermediate 390**(900 mg, 2.4 mmol), Pd₂(dba)₃ (433 mg, 0.2 mmol), Xantphos (548 mg, 0.95 mmol) and Cs₂CO₃ (2.3 g, 7.0 mmol) in dioxane (20 mL) was stirred at 95°C for 16 h under N₂. The mixture was warmed to room temperature, quenched with water (50 mL) and extracted with DCM (3 x 50 ml). The combined organics were washed with brine (50 mL), dried over Na₂SO₄ and concentrated. The residue was purified by silica gel chromatography (eluant: PE : EtAOc = 1 : 1) to give **benzyl 2-(3-(2-(((tert-butoxycarbonyl)(methyl)amino)methyl)-6-cyclopropyl imidazo[1,2-a]pyridin-8-yl)-2,5-dioxoimidazolidin-1-yl)acetate intermediate 398** (600 mg, 46%) as a yellow solid. ESI-MS [M +H]⁺: 548.2.

To a solution of **intermediate 398** (600 mg, 1.09 mmol) in MeOH (10 mL) was added t-BuN₃ (1.6 g, 21.9 mmol) and the mixture was stirred at 65°C for 2 h under N₂. The mixture was cooled to room temperature, filtered and the filtrate was concentrated *in vacuo* to give the crude product which was purified by silica gel chromatography (eluent: DCM/MeOH = 20/1) to give **methyl 2-(3-(2-(((tert-butoxycarbonyl)(methyl)amino)methyl)-6-cyclopropylimidazo[1,2-a]pyridin-8-yl)-2,5-dioxoimidazolidin-1-yl)acetate intermediate 399** (450 mg, 87.5%) as a yellow solid. ESI-MS [M +H]⁺: 472.2.

To a solution of **intermediate 399** (300 mg, 0.63 mmol) in THF (10 mL) was added CH₃MgBr (2.1 mL, 3 M in THF, 6.3 mmol) at 0°C and then mixture was stirred at room temperature for 40 min. The mixture was quenched with sat. NH₄Cl solution (50 mL) and extracted with EtOAc (3 x 30 mL). The combined organic layers were washed with brine (30 mL), dried over Na₂SO₄ and concentrated *in vacuo.* The residue was purified by preparative TLC (eluent: DCM/MeOH = 16/1) to give **tert-butyl ((6-cyclopropyl-8-(3-(2-hydroxy-2-methylpropyl)-2,4-dioxoimidazolidin-1-yl)imidazo[1,2-a]pyridin-2-yl)methyl)(methyl)carbamate** (30 mg, 10%) as a yellow solid. ESI-MS [M +H]⁺: 472.2. To a solution of *tert*-butyl ((6-cyclopropyl-8-(3-(2-hydroxy-2-methylpropyl)-2,4-dioxoimidazolidin-1-yl)imidazo[1,2-a]pyridin-2-yl)methyl)(methyl)carbamate (30 mg, 0.063 mmol) in dioxane (2 mL) was added HCl/dioxane (2 mL) at room temperature and the mixture was stirred for 2 h. The mixture was concentrated *in vacuo* to give **intermediate 396** (30 mg, crude) as a yellow solid which was used in next step without purification. ESI-MS [M +H]⁺: 372.2.

### Intermediate 400: 1-(2-(1-aminoethyl)-7-cyclopropylimidazo[1,2-a]pyridin-5-yl)-3-methylimidazolidine-2,4-dione

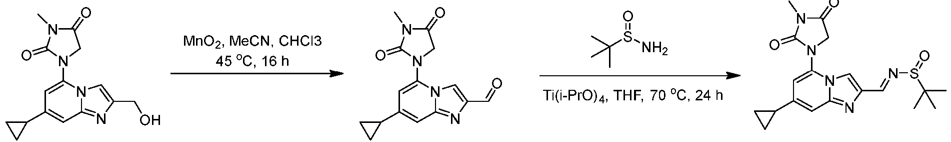

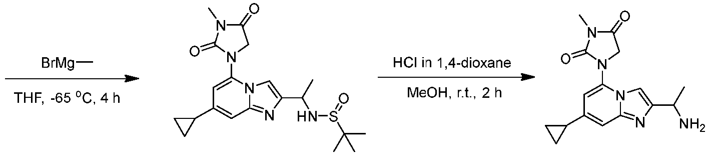

A mixture of **intermediate 463** (300 mg, 1.0 mmol) and MnO₂ (1.3 g, 15 mmol) in CHCl₃ (3.0 ml) and MeCN (3.0 mL) was stirred at 45°C for 16 h. The reaction mixture was filtered through celite and the filter cake was washed with DCM/MeOH (10/1, 50 mL). The filtrate was concentrated *in vacuo* to give the crude, which was purified by silica gel chromatography (eluent: PE/EA = 1/2) to give **7-cyclopropyl-5-(3-methyl-2,4-dioxoimidazolidin-1-yl)imidazo[1,2-a]pyridine-2-carbaldehyde intermediate 401** (200 mg, 67%) as a yellow solid. ESI-MS: [M + H]⁺,299.1

To a solution of **intermediate 401** (180 mg, 0.6 mmol) and 2-methylpropane-2-sulfinamide (218 mg, 1.8 mmol) in THF (6 mL) was added Ti(i-PrO)₄ (1.02 g, 3.6 mmol) dropwise. The resulting mixture was stirred at 70°C for another 24 h. The reaction was quenched with water (50 mL) then extracted with EtOAc (3 x 30 mL). The combined organic layers were washed with brine (20 mL), dried over anhydrous Na₂SO₄, and concentrated *in vacuo* to give the crude, which was purified by silica gel chromatography (eluent, DCM/MeOH = 30/1) to give **(E)-N-((7-cyclopropyl-5-(3-methyl-2,4-dioxoimidazolidin-1-yl)imidazo[1,2-a]pyridin-2-yl)methylene)-2-methylpropane-2-sulfinamide** (100 mg, 41%) as a yellow solid. ESI-MS: [M + H]⁺, 402.1

A solution of (E)-N-((7-cyclopropyl-5-(3-methyl-2,4-dioxoimidazolidin-1-yl)imidazo[1,2-a]pyridin-2-yl)methylene)-2-methylpropane-2-sulfinamide (100 mg, 0.25 mmol) in THF (4 mL) was cooled to -65°C. Methylmagnesium bromide (3M solution in THF, 0.292 ml, 0.875 mmol) was added dropwise. The resulting mixture was stirred at -65°C for 4 h under N₂. The reaction was quenched with saturated aqueous NH₄Cl (20 mL) then extracted with DCM (3 x 20 ml), The combined organic layers were washed with brine (20 mL), dried over anhydrous Na₂SO₄, and concentrated *in vacuo* to give the crude product, which was purified by silica gel chromatography (eluent:, DCM/MeOH = 25/1) to give **N-(1-(7-cyclopropyl-5-(3-methyl-2,4-dioxoimidazolidin-1-yl)imidazo[1,2-a]pyridin-2-yl)ethyl)-2-methylpropane-2-sulfinamide intermediate 402** (18 mg, 17%) as a yellow solid. ESI-MS: [M +H]⁺, 418.1

To a mixture of **intermediate 402**(50 mg, 0.12 mmol) in MeOH (1 mL) was added HCl (4M solution in dioxane, 0.5 mL). The resulting mixture was stirred at room temperature for 2 h. The reaction mixture was diluted with DCM (20 ml) and concentrated *in vacuo* to give the crude, which was purified by preparative TLC (eluent, DCM/MeOH = 8/1) to give **intermediate 400** (20 mg, 53%) as a yellow solid. ESI-MS: [M +H]⁺,314.1

### Intermediate 403: 1-(2-(1-aminoethyl)-6-cyclopropylimidazo[1,2-a]pyridin-8-yl)-3-methylimidazolidin-2-one

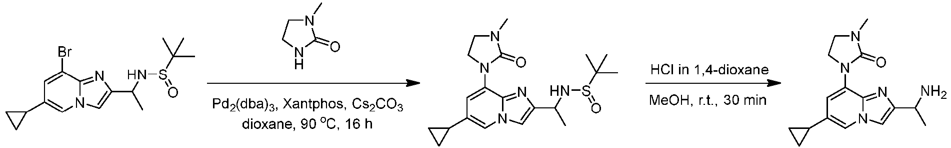

To a mixture of **intermediate 417** (200 mg, 0.52 mmol), 1-methylimidazolidin-2-one (156 mg, 1.56 mmol) and Cs₂CO₃ (509 mg, 1.56 mmol) in 1,4-dioxane (10 mL) were added Pd₂(dba)₃ (48 mg, 0.052 mmol) and Xantphos (60 mg, 0.104 mmol). The mixture was stirred at 90°C for 16 h under N₂. The reaction mixture was cooled to room temperature, filtered through celite and the filter cake was washed with DCM/MeOH (10/1, 30 mL). The filtrate was concentrated *in vacuo* to give the crude product, which was purified by column chromatography (eluent: EtOAc/PE = 1/2) to give **N-(1-(6-cyclopropyl-8-(3-methyl-2-oxoimidazolidin-1-yl)imidazo[1,2-a]pyridin-2-yl)ethyl)-2-methylpropane-2-sulfinamide intermediate 404** (180 mg, 86%) as a yellow solid. ESI-MS [M + H]⁺: 404.2.

To a stirred solution of **intermediate 404** (215 mg, 0.53 mmol) in MeOH (5 mL) was added HCl (4 M solution in 1,4-dioxane, 5 mL). The mixture was stirred at room temperature for 30 min. The reaction mixture was concentrated *in vacuo* and a solution of NH₃ (7 M solution in MeOH, 5 mL) was added. The resulting mixture was stirred for 10 min and concentrated *in vacuo* to give the crude, which was purified by preparative TLC (eluent: DCM/MeOH = 10/1) to give **intermediate 403** (110 mg, 69%) as a white solid. ESI-MS [M + H]⁺: 300.2.

### Intermediate 405: 1-(2-(1-aminoethyl)-6-cyclopropylimidazo[1,2-a]pyridin-8-yl)-3-(2-(benzyloxy)ethyl)imidazolidine-2,4-dione

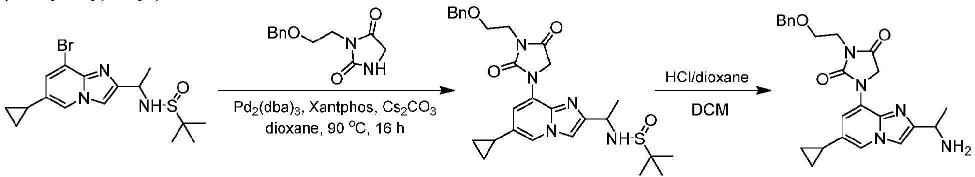

To a solution of **intermediate 417** (300 mg, 0.78 mmol) in dioxane (10 mL) were added **intermediate 373** (548 mg, 2.34 mmol), Pd₂(dba)₃ (143 mg, 0.16 mmol), Xantphos (181 mg, 0.31 mmol) and Cs₂CO₃ (1019 mg, 3.13 mmol). After stirring the mixture at 95°C for 16 h under N₂, it was cooled to room temperature, diluted with water (50 mL) and extracted with EtOAc (3 x 50 mL). The combined organic layers were washed with brine (50 mL), dried over Na₂SO₄ then concentrated to give the crude, which was purified by silica gel (eluant: DCM: MeOH=50: 1) to give **N-(1-(8-(3-(2-(benzyl oxy)ethyl)-2,4-dioxoimidazolidin-1-yl)-6-cyclopropylimidazo[1,2-a]pyridin-2-yl)ethyl)-2-methyl propane-2-sulfinamide intermediate 406** as yellow solid. (300 mg, 72% yield). ESI-MS [M +H]⁺: 538.2

To a solution of **intermediate 406** (300 mg, 0.55 mmol) in DCM (10 mL) was added HCl (4M solution in dioxane, 1 mL) and then the mixture was stirred at 0°C for 1 h. The resulting solution was concentrated and basified by NH₃/MeOH (30 mL) and concentrated to dryness. The residue was purified by preparative TLC (DCM: NH₃/MeOH=12:1) to give **intermediate 405** as yellow solid. (170 mg, 71% yield). ESI-MS [M +H]⁺: 434.2

### Intermediate 407: (R)-((2-(1-aminoethyl)-6-cyclopropylimidazo[1,2-a]pyridin-8-yl)imino)dimethyl-λ⁶-sulfanone

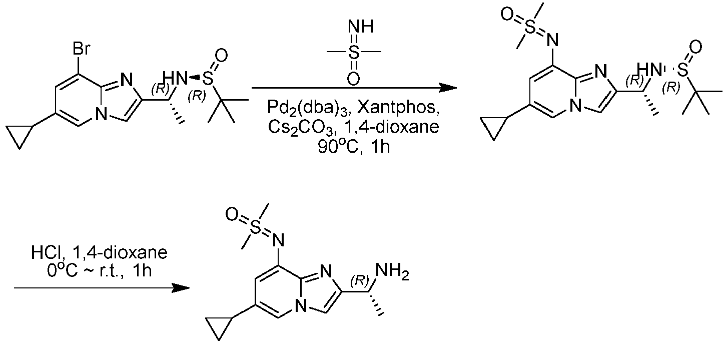

To a mixture of **intermediate 427** (350 mg, 0.91 mmol), iminodimethyl-λ⁶-sulfanone (213.9 mg, 2.3 mmol) and Cs₂CO₃ (743 mg, 2.28 mmol) in 1,4-dioxane (20 mL) was added Pd₂(dba)₃ (0.091 mmol) and Xantphos (104 mg, 0.18 mmol). The reaction mixture was stirred at 90°C for 1h and cooled to room temperature. The resulting solution was filtered through Celite^{®} and the filter cake was washed with DCM/MeOH (10/1, 50 mL). The filtrate was concentrated *in vacuo* to give the crude, which was purified by preparative TLC (eluent: DCM/MeOH = 20/1) to give **(R)-N-((R)-1-(6-cyclopropyl-8-((dimethyl(oxo)-λ⁶-sulfaneylidene)amino)imidazo[1,2-a]pyridin-2-yl)ethyl)-2-methylpropane-2-sulfinamide intermediate 408** (210 mg, 58%) as a white solid. ESI-MS [M +H]+: 397.2

To a solution of **intermediate 408** (210 mg, 0.53 mmol) in 1,4-dioxane (10 mL) was added HCl (4M solution in 1,4-dioxane, 1 mL) at 0°C. The reaction was stirred at room temperature for 1h and concentrated *in vacuo* to give the crude, which was purified by preparative TLC (DCM/MeOH/NH₄OH=10/1/0.1) to give **intermediate 407** (140 mg, 90%) as a yellow solid. ESI-MS [M +H]+: 293.1

### Intermediate 409: (R)-1-(2-(1-aminoethyl)-6-cyclopropylimidazo[1,2-a]pyridin-8-yl)-3,5,5-trimethylimidazolidine-2,4-dione

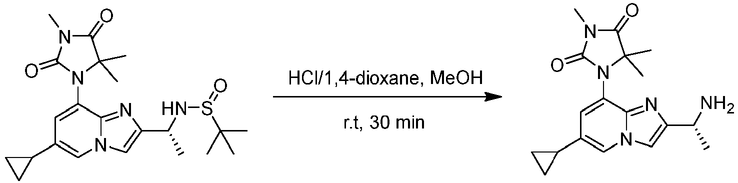

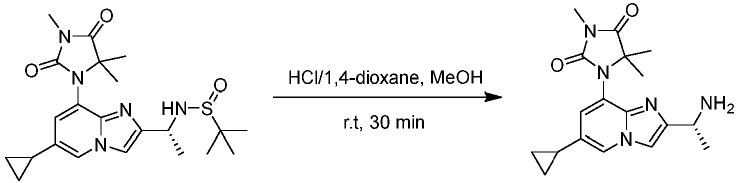

A mixture of 5,5-dimethylimidazolidine-2,4-dione (4.0 g, 0.031 mol) and KOH (1.9 g, 0.034 mol) were dissolved in EtOH (60 mL) by stirring at 95°C for 10 min. After removing the solvent by evaporation, the resulting hydantoin potassium salt was dried under vacuum at 45°C. The residue was dissolved in DMF (60 mL) and then was added MeI (4.4 g, 0.031 mol). After stirring at 25°C for 5 h, the reaction mixture was filtered, and the filtrate was concentrated *in vacuo* to give the crude product, which was purified by silica gel chromatography (eluent: PE/EtOAc = 10/1) to give **3,5,5-trimethyl imidazolidine-2,4-dione intermediate 410** (0.50 g, 11%) as a yellow solid. ESI-MS [M +H]⁺: 143.0.

A mixture of **intermediate 410** (0.44 g, 3.1 mmol), N-((R)-1-(8-bromo-6-cyclopropylimidazo[1,2-a]pyridin-2-yl)ethyl)-2-methylpropane-2-**sulfinamide** (0.30 g, 0.78 mmol), Pd₂(dba)₃ (0.15 g, 0.16 mmol), Xantphos (0.18 g, 0.31 mmol) and Cs₂CO₃ (0.75 g, 2.3 mmol) in toluene (10 mL) was stirred in a sealed tube. After degassing with N₂ for 1min, the reaction was irradiated in microwave at 130°C for 2 h. The reaction mixture was diluted in DCM/MeOH (50 mL, 10/1), filtered and the filtrate was concentrated *in vacuo.* The residue was purified by silica gel chromatography (eluent: PE/EtOAc = 10/1) to give **N-((*R*)-1-(6-cyclopropyl-8-(3,5,5-trimethyl-2,4-dioxoimidazolidin-1-yl)imidazo[1,2-a]pyridin-2-yl)ethyl)-2-methylpropane-2-sulfinamide intermediate 411** (0.13 g, 37%) as a yellowy solid. ESI-MS [M + H]⁺: 446.2.

To a solution of **intermediate 411** (0.13 g, 0.29 mmol) in MeOH (2.0 mL) was added HCl (4.0 M solution in 1,4-dioxane, 3.0 mL). The resulting mixture was stirred at room temperature for 30 min and concentrated *in vacuo.* Then a solution of NH₃ (7 M in MeOH 5.0 mL) was added to the residue and stirred for 10 min. The mixture was concentrated *in vacuo* to give the crude, which was purified by preparative TLC (eluent: DCM/NH₃ (7 M in MeOH) = 10/1) to give (**intermediate 409** (80 mg, 80%) as a white solid. ESI-MS [M + H]⁺: 342.2.

### Intermediate 412: (R)-1-(2-(1-aminoethyl)-6-cyclopropylimidazo[1,2-a]pyridin-8-yl)imidazolidine-2,4-dione

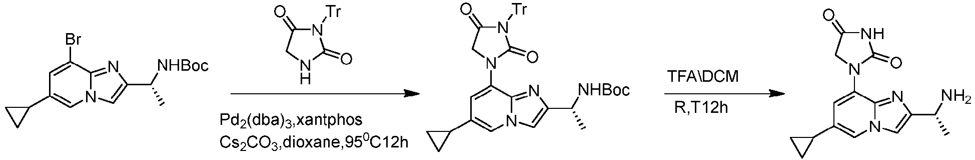

To a mixture of **intermediate 564** (500 mg, 1.3 mmol), **intermediate 280** (889 mg, 2.6 mmol) and Cs₂CO₃ (1.27 g, 3.9 mmol) in dioxane (10 mL) was added Pd₂(dba)₃ (174 mg, 0.19 mmol) and xantPhos (150 mg, 0.26 mmol). The reaction mixture was stirred at 95°C for 12 h under N₂. Then the reaction mixture was was filtered through Celite^{®} and the filter cake was washed with DCM/MeOH (10/1, 100 mL). The filtrate was concentrated *in vacuo* to give the crude, which was purified by preparative TLC (eluent: DCM/MeOH=20/1) to give **tert-butyl (R)-(1-(6-cyclopropyl-8-(2,4-dioxo-3-tritylimidazolidin-1-yl)imidazo[1,2-a]pyridin-2-yl)ethyl)carbamate intermediate 413** (300 mg, 36%) as a yellow solid. ESI-MS [M +H]+: 642.2.

A solution of **intermediate 413** (300 mg, 0.47 mmol) in TFA (2 mL) and DCM (2 mL) was stirred at room temperature for 12 h. The mixture was quenched with NaHCO₃ (sat. aq., 50 mL) and extracted with DCM (50 mL x 3). The combined organic layers were washed with brine (50 mL), dried over Na₂SO₄ and concentrated *in vacuo.* The residue was purified by column chromatography (eluent: DCM/MeOH = 0-10%) to give **intermediate 412** (100 mg, 71 %) as a white solid. ESI-MS [M +H]⁺: 300.2

### Intermediate 414: (R)-3-(2-(1-aminoethyl)-6-cyclopropylimidazo[1,2-a]pyridin-8-yl)oxazolidin-2-one

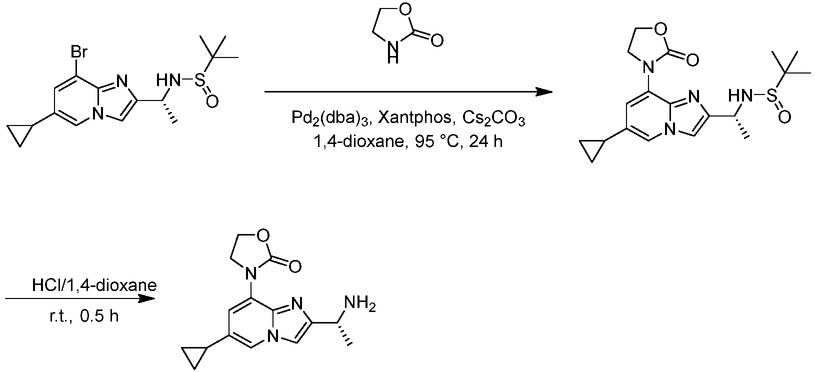

To a mixture of N-((R)-1-(8-bromo-6-cyclopropylimidazo[1,2-a]pyridin-2-yl)ethyl)-2-methylpropane-2-sulfinamide (295 mg, 0.78 mmol), oxazolidin-2-one (205 mg, 2.34 mmol) and Cs₂CO₃ (750 mg, 2.3 mmol) and Xantphos (93 mg, 0.16 mmol) in 1,4-dioxane (15 mL) was added Pd₂(dba)₃ (73 mg, 0.080 mmol) and the reaction mixture was stirred at 95°C for 24 h under N₂. The reaction mixture was filtered through Celite^{®} and the filter cake was washed with DCM/MeOH (10/1, 100 mL). The filtrate was concentrated *in vacuo* to give the crude, which was purified by column chromatography (eluent: EtOAc/PE = 0 - 100%) to afford **N-((R)-1-(6-cyclopropyl-8-(2-oxooxazolidin-3-yl)imidazo[1,2-a]pyridin-2-yl)ethyl)-2-methylpropane-2-sulfinamide intermediate 415** (240 mg, 79%) as an off-yellow solid. ESI-MS [M +H] +: 391.2

To a mixture of **intermediate 415** (240 mg, 0.62 mmol) in MeOH (3 mL) was added HCl/1,4-dioxane (5 mL, 4.0 M in 1,4-dioxane ). The reaction was stirred at room temperature for 0.5 h. The mixture was quenched with NaHCO₃ (sat. aq., 50 mL) and extracted with EtOAc (50 mL x 3). The combined organic layers were washed with brine (50 mL), dried over Na₂SO₄ and concentrated *in vacuo.* The residue was purified by column chromatography (eluent: MeOH/DCM = 0-10% ) with to afford **intermediate 414** (140 mg, 79%) as an yellow solid. ESI-MS [M +H] +: 287.1

### Intermediate 416: 1-(6-cyclopropyl-2-(1-(methylamino)ethyl)imidazo[1,2-a]pyridin-8-yl)-3-methylimidazolidine-2,4-dione

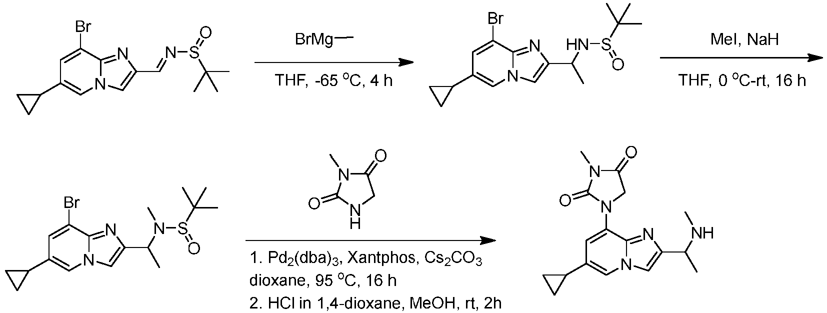

To a mixture of (E)-N-((8-bromo-6-cyclopropylimidazo[1,2-a]pyridin-2-yl)methylene)-2-methylpropane-2-sulfinamide (1 g, 2.7 mmol) in THF (25 mL) was added CH₃MgBr (2.7 mL, 3 M in THF, 8.1 mmol) at - 65°C and then stirred for 4 h under N₂. Then the mixture was quenched with sat. NH₄Cl solution (20 mL) at -65°C, warmed to room temperature and extracted with DCM (3 x 30 mL). The combined organic layers were washed with brine (20 mL), dried over anhydrous Na₂SO₄, and concentrated *in vacuo* to give **N-(1-(8-bromo-6-cyclopropylimidazo[1,2-a]pyridin-2-yl)ethyl)-2-methylpropane-2-sulfinamide intermediate 417** (860 mg, crude) as a yellow solid. ESI-MS [M +H]⁺: 384.2.

To a solution of **intermediate 417** (500 mg, crude) in THF (20 mL) was added NaH (76 mg, 1.9 mmol) at 0°C. The mixture was stirred for 0.5 h. Then a solution of MeI (269.8 mg, 1.9 mmol) in THF (2 mL) was added to the reaction mixture and it was stirred at room temperature for 16 h. The reaction was quenched with water (10 mL) and extracted with EtOAc (3 x 20 mL). The combined organic layers were washed with brine (20 mL), dried over anhydrous Na₂SO₄ and concentrated to give the crude product, which was purified by preparative TLC (eluent: DCM/MeOH = 10/1) to give **N-(1-(8-bromo-6-cyclopropylimidazo[1,2-a]pyridin-2-yl)ethyl)-N,2-dimethylpropane-2-sulfinamide**
**intermediate 418** (370 mg, 58% in 2 steps) as a yellow solid. ESI-MS [M +H]⁺: 400.1.

To a solution of **intermediate 418** (370 mg, 0.93 mmol) in dioxane (10 mL) was added 3-methylimidazolidine-2,4-dione (424 mg, 3.72 mmol), Pd₂(dba)₃ (164.9 mg, 0.18 mmol), Xantphos (208.1 mg, 0.36 mmol) and Cs₂CO₃ (909.5 mg, 2.79 mmol). After stirring the mixture at 95°C for 16 h under N₂, the mixture was filtered through celite and the filter cake was washed with DCM/MeOH (10/1, 50 mL). The filtrate was concentrated *in vacuo* to give the crude product, which was purified by silica gel chromatography (eluent: DCM/MeOH=50/1 ~ 20/1) to give **N-(1-(6-cyclopropyl-8-(3-methyl-2,4-dioxoimidazolidin-1-yl)imidazo[1,2-a]pyridin-2-yl)ethyl)-N,2-dimethylpropane-2-sulfinamide intermediate 419** (300 mg, 75%) as a yellow solid. ESI-MS [M +H]⁺: 432.2.

To a solution of **intermediate 419** (300 mg, 0.70 mmol) in MeOH (5 mL) was added HCl (4M solution in dioxane, 5 mL). After stirring for 2 h, the reaction mixture was concentrated *in vacuo* to give **intermediate 416** as the hydrochloric acid salt (200 mg, 79%) as a yellow solid which was used in next step without purification. ESI-MS [M +H]+: 328.1.

### Intermediate 420: 1-(2-(aminomethyl-d2)-6-cyclopropylimidazo[1,2-a]pyridin-8-yl)-3-methylimidazolidine-2,4-dione

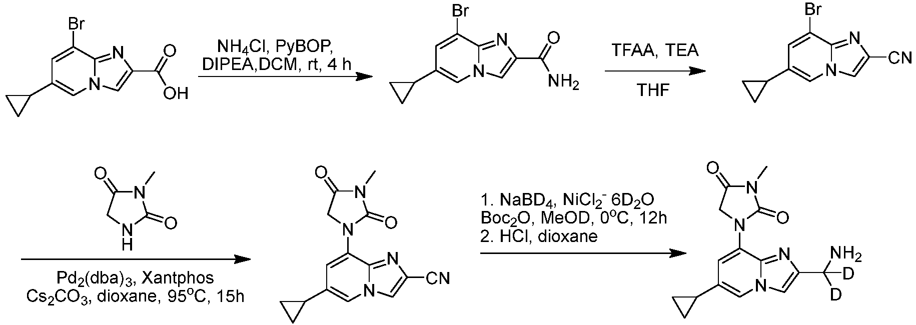

A solution of 8-bromo-6-cyclopropylimidazo[1,2-a]pyridine-2-carboxylic acid (1.12 g, crude), NH₄Cl (424 mg, 8 mmol), PyBOP (2796 mg, 6 mmol) and DIPEA (2064 mg, 16 mmol) in DCM (30 mL) was stirred at room temperature for 3 h. The reaction mixture was diluted with DCM (50 mL), washed with sat.NaHCO₃ solution (50 mL) and water (50 mL). The organic layer was dried over anhydrous Na₂SO₄ and concentrated *in vacuo* to get the crude, which was purified by silica gel chromatography (eluent: DCM/MeOH = 10/1) to afford **8-bromo-6-cyclopropylimidazo[1,2-a]pyridine-2-carboxamide intermediate 421** (840 mg, 75%) as yellow solid. ESI-MS [M +H]⁺: 280.0.

To a solution of **intermediate 421** (560 mg, 2 mmol) in TEA (2 ml) and THF (10 mL) was added TFAA (1 mL) at 0°C. After stirring for 2 h, the reaction was quenched with water (30 mL) and extracted with EtOAc (3 x 30 mL). The combined organic layers were washed with brine (20 mL), dried over anhydrous Na₂SO₄, and concentrated to give the crude product, which was purified by column chromatography (eluent: DCM/MeOH = 30/1) to give the **8-bromo-6-cyclopropylimidazo[1,2-a]pyridine-2-carbonitrile intermediate 422** (290 mg, 56%) as yellow solid. ESI-MS [M +H]+: 262.0.

To a mixture of **intermediate 422** (290 mg, 1.1 mmol) and **intermediate 322** (376 mg, 3.3 mmol) and Cs₂CO₃ (1076 mg, 3.3 mmol) in dioxane (20 mL) was added Pd₂(dba)₃ (100 mg, 0.11 mmol) and Xantphos (127 mg, 0.22 mmol). The reaction was stirred at 95°C under N₂ for 15 h, cooled to room temperature, filtered through celite and the filter cake was washed with DCM/MeOH (10/1, 50 mL). The filtrate was concentrated *in vacuo* to give the crude, which was purified by preparative TLC (eluent: DCM/MeOH = 20/1) to give the **6-cyclopropyl-8-(3-methyl-2,4-dioxoimidazolidin-1-yl)imidazo[1,2-a]pyridine-2-carbonitrile intermediate 423** (180 mg, 56 %) as yellow solid. ESI-MS [M +H]+: 296.1.

To a mixture of **intermediate 423** (180 mg, 0.61 mmol) and NiCl₂ 6D₂O (7 mg, 0.03 mmol) in MeOD (10 mL) was added Boc₂O (266 mg, 1.22mmol). After stirring at room temperature under N₂ for 30 min, the reaction mixture was treated with NaBD₄ (154 mg, 3.66 mmol) slowly in small portions. The mixture was stirred at 0°C under N₂ for 1h and then at room temperature for 12 h. The reaction was quenched with sat. aqueous NH₄Cl solution (30 mL) and extracted with ethyl acetate (3 x 30 mL). The combined organic layers were dried over anhydrous Na₂SO₄ and concentrated *in vacuo* to give the crude, which was purified by preparative TLC (eluent: DCM/MeOH = 15/1) to give the **tert-butyl((6-cyclopropyl-8-(3-methyl-2,4-dioxoimidazolidin-1-yl)imidazo[1,2-a]pyridin-2-yl)methyl-d2) carbamate intermediate 424** (100 mg, 41 %) as yellow solid. ESI-MS [M +H]+: 402.2.

To a solution of tert-butyl ((6-cyclopropyl-8-(3-methyl-2,4-dioxoimidazolidin-1-yl)imidazo[1,2-a]pyridin-**2**-yl)methyl-d2)carbamate (100 mg, 0.25 mol) in dioxane (2 mL). was added HCl (4M solution in dioxane, 2 mL). After stirring at room temperature for 1 h., the mixture was basified (pH 8) with sat. aqueous NaHCO₃ solution and the mixture was extracted with DCM (3 x 20 mL). The combined organic layers were washed with brine (30 mL), dried over anhydrous Na₂SO₄ and concentrated *in vacuo* to give the crude product, which was purified by preparative TLC (eluent: DCM /MeOH = 10/1) to afford **intermediate 420** (55 mg, 73 %) as a white solid. ESI-MS [M +H]+: 302.1.

### Intermediate 425: (R)-1-(2-(1-aminoethyl)-6-cyclopropylimidazo[1,2-a]pyridin-8-yl)-3-methylimidazolidine-2,4-dione

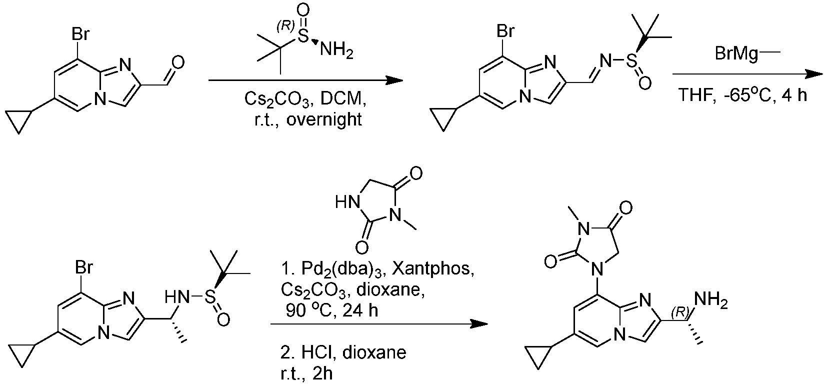

To a solution of **intermediate 457** (4.2 g, 15.8 mmol) in DCM (80 mL) was added (R)-2-methylpropane-2-sulfinamide (2.5 g, 0.21 mmol) and Cs₂CO₃ (10.33 g, 0.21 mmol). The reaction mixture was stirred at room temperature overnight, then poured into water (100 mL) and extracted with EtOAc (100 mL×3). The combined organics were washed with brine (100 mL), dried over Na₂SO₄. The reaction mixture was concentrated *in vacuo* and purified by silica gel chromatography (eluent: PE: EtOAc =5:1) to give **(R)-N-((8-bromo-6-cyclopropylimidazo[1,2-a]pyridin-2-yl)methylene)-2-methylpropane-2-sulfinamide intermediate 426** (4.2 g, yield 72%). ESI-MS [M +H] +: 368.0

To a solution of (**intermediate 426** (200 mg, 0.54 mmol) in THF (5 mL) was added methylmagnesium bromide (0.72 mL, 2.16 mmol) at -65°C and the mixture was stirred for 4 h at the same temperature. The reaction mixture was quenched with NH₄Cl solution (sat. aq., 50 mL) at -65°C, stirred for 10 min then extracted with EtOAc (50 mL× 3). The combined organics were washed with brine (50 mL), dried over Na₂SO₄ and concentrated *in vacuo.* The residue was purified by silica gel chromatography (eluent: PE: EtOAc =5:1) to give **(R)-N-((R)-1-(8-bromo-6-cyclopropylimidazo[1,2-a]pyridin-2-yl)ethyl)-2-methylpropane-2-sulfinamide intermediate 427** (160 mg, yield 77 %). ESI-MS [M +H] +: 384.1

To a solution of **intermediate 427** (400 mg, 1.05 mmol) in 1,4-dioxane (40 mL) were added **intermediate 322** (190 mg, 1.56 mmol), Pd₂(dba)₃ (100 mg, 0.105 mmol), XantPhos (120 mg, 0.21 mmol), and Cs₂CO₃ (1.15g, 3.09 mmol) at 90°C for 16 h under N₂. The reaction mixture was filtered and the filter cake was washed with DCM/MeOH (10/1, 50 mL). The filtrate was concentrated *in vacuo* to give the crude residue, which was purified by silica gel chromatography (eluent: DCM/MeOH=50/1 ~ 20/1) to give **(R)-N-((R)-1-(6-cyclopropyl-8-(3-methyl-2,4-dioxoimidazolidin-1-yl)imidazo[1,2-a]pyridin-2-yl)ethyl)-2-methylpropane-2-sulfinamide intermediate 428** (400 mg, 91 %). ESI-MS [M +H] +: 418. 2

A mixture of **intermediate 428** (70 mg, 0.17 mmol) in HCl/dioxane (4M, 2 mL) was stirred at room temperature for 1 h. The reaction mixture was poured into ammonia in methanol (7M, 2 mL) and concentrated *in vacuo* to give the crude residue, which was purified by silica gel chromatography (eluent: DCM: MeOH=10:1) to give **intermediate 425** a white solid (50 mg, yield 95 %). ESI-MS [M +H] +: 314.

### Intermediate 429: (R)-1-(2-(1-aminopropyl)-6-cyclopropylimidazo[1,2-a]pyridin-8-yl)-3-mthyl imidazolidine-2,4-dione

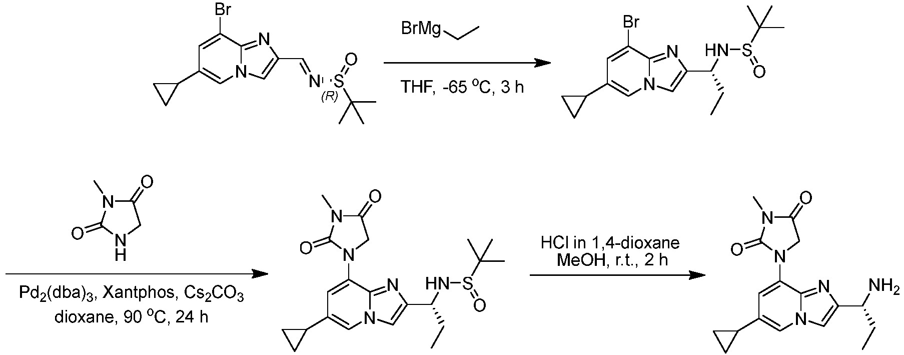

Using a similar procedure to that for **intermediate 425, intermediate 429** (243 mg) was synthesised from (R,E)-N-((8-bromo-6-cyclopropylimidazo[1,2-a]pyridin-2-yl)methylene)-2-methylpropane-2-sulfinamide and ethylmagnesium bromide. ESI-MS [M +H]+: 328.1

### Intermediate 430: 3-(2-((R)-1-aminoethyl)-6-cyclopropylimidazo[1,2-a]pyridin-8-yl)-3-azabicyclo[3.1.0]hexan-2-one

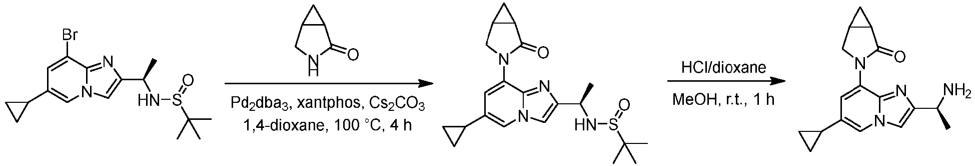

To a mixture of N-((R)-1-(8-bromo-6-cyclopropylimidazo[1,2-a]pyridin-2-yl)ethyl)-2-methylpropane-2-sulfinamide (0.75 g, 1.9 mmol), 3-azabicyclo[3.1.0]hexan-2-one (0.19 g, 1.9 mmol) and Cs₂CO₃ (1.9 g, 5.7 mmol) in 1,4-dioxane (30 mL) was added Pd₂(dba)₃ (0.36 g, 0.38 mmol) and Xantphos (0.45 g, 0.76 mmol). The resulting mixture was stirred at 100°C for 4 h under N₂ and cooled to room temperature. The reaction mixture was filtered through Celite^{®} and the filter cake was washed with DCM/MeOH (10/1, 50 mL). The filtrate was concentrated *in vacuo* to give the crude, which was purified by silica gel column chromatography (eluent: DCM/MeOH =0 ~ 5%) to afford **N-((1R)-1-(6-cyclopropyl-8-(2-oxo-3-azabicyclo[3.1.0]hexan-3-yl)imidazo[1,2-a]pyridin-2-yl)ethyl)-2-methylpropane-2-sulfinamide intermediate 431** (0.76 g, 97 %) as a yellow solid. ESI-MS: [M + H]⁺, 401.2

To a mixture of **intermediate 431** (0.76 g, 1.9 mmol) in MeOH (10 mL) was added HCl (5 mL, 20 mmol, 4M in 1,4-dioxane,) at 0°C. The reaction mixture was stirred at room temperature for 1 h and concentrated *in vacuo.* The resulting residue was basified (pH = 8) with NH₃/ MeOH (5 mL, 35 mmol, 7M in MeOH) at 0°C. The mixture was diluted with DCM (90 mL), filtered and the filter cake was washed by DCM (3 x 40 ml). The filtrate was concentrated *in vacuo* to give the crude, which was purified by column chromatography (eluent: MeOH/DCM = 1/8) to afford **intermediate 430** (0.54 g, 96 %) as a yellow solid. ESI-MS: [M + H]⁺, 297.1

### Intermediate 432: (R)-1-(2-(1-aminoethyl)-6-cyclopropylimidazo[1,2-a]pyridin-8-yl)pyrrolidin-2-one

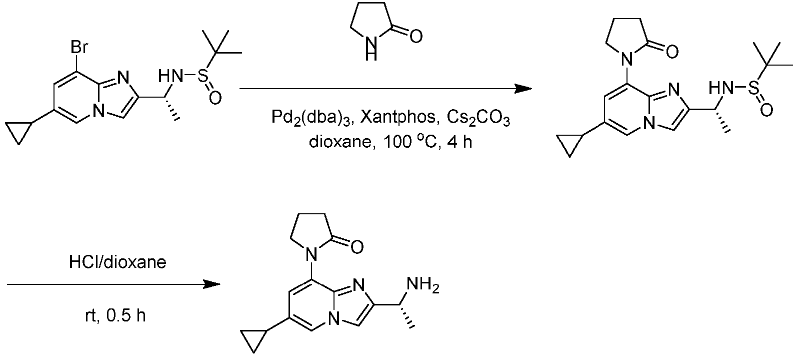

A mixture of N-((R)-1-(8-bromo-6-cyclopropylimidazo[1,2-a]pyridin-2-yl)ethyl)-2-methylpropane-2-**sulfinamide** (300 mg, 0.78 mmol), pyrrolidin-2-one (266 mg, 3.12 mmol), Pd₂(dba)₃ (143 mg, 0.156 mmol), XantPhos (180 mg, 0.312 mmol) and Cs₂CO₃ (763 mg, 2.34 mmol) in dioxane (15 mL) was stirred at 100°C for 4 h under N₂. The reaction mixture was filtered through Celite^{®} and the filter cake was washed with DCM/MeOH (10/1, 50 mL). The filtrate was concentrated *in vacuo* to give the crude, which was purified by column chromatography (eluent: DCM/MeOH=0 ~ 10/1) to give **N-((R)-1-(6-cyclopropyl-8-(2-oxopyrrolidin-1-yl)imidazo[1,2-a] pyridin-2-yl)ethyl)-2-methylpropane-2-sulfinamide intermediate 433** (250 mg, 83 %) as a yellow oil. ESI-MS [M +H] +: 389.2

To a mixture of **intermediate 433** (250 mg, 0.64 mmol) in MeOH (3 mL) was added HCl (5 mL, 4M solution in dioxane). The resulting mixture was stirred at room temperature for 0.5 h. The reaction mixture was concentrated *in vacuo* to give **intermediate 432** as the hydrochloric acid salt (250 mg, quant) as a yellow solid. ESI-MS [M +H]+: 285.1.

### Intermediate 434: 1-(2-(1-amino-2,2,2-trifluoroethyl)-6-cyclopropylimidazo[1,2-a]pyridin-8-yl)-3-methylimidazolidine-2,4-dione

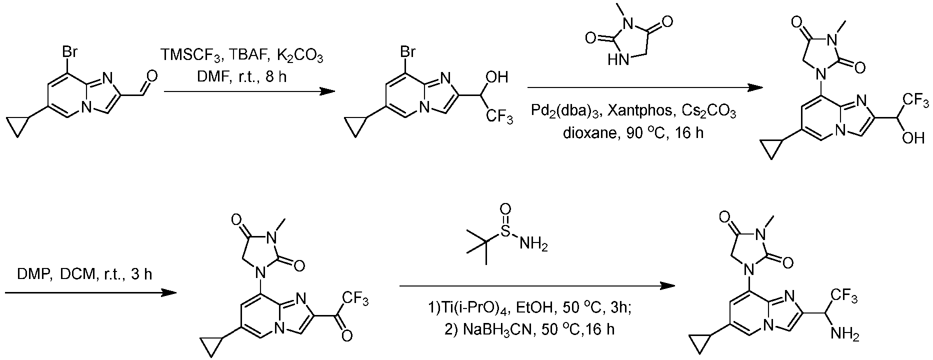

To a solution of **intermediate 457** (1.5 g, 5.68 mmol) in DMF (30 ml) were added K₂CO₃ (78 mg, 0.568 mmol) and TMSCF₃ (1.2 g, 8.52 mmol). The resulting reaction was stirred at room temperature for 4 h, then TBAF (8.52 mL, 8.52 mmol) was added dropwise. After stirring for another 4 h, the mixture was quenched with water (200 mL) and extracted with DCM (3 x 100 mL). The combined organic layers were washed with brine (200 mL), dried over anhydrous Na₂SO₄, and concentrated *in vacuo* to give the crude, which was purified by silica gel chromatography (eluent: PE/EtOAc = 1/1) to give **1-(8-bromo-6-cyclopropylimidazo[1,2-a]pyridin-2-yl)-2,2,2-trifluoroethan-1-ol intermediate 435** (1.2 g, 63%) as a yellow solid. ESI-MS: [M + H]⁺, 335.2.

To a solution of **intermediate 435** (1.0 g, 2.99 mmol), **intermediate 322** (1.0 g, 8.97 mmol) and Cs₂CO₃ (2.4 g, 7.48 mmol) in 1,4-dioxane (30 mL) was added Pd₂(dba)₃ (274 mg, 0.299 mmol) and Xantphos (346 mg, 0.598 mmol). After stirring at 90°C for 16 h under N₂, the reaction mixture was cooled to room temperature, filtered through celite and the filter cake was washed with DCM/MeOH (10/1, 100 mL). The filtrate was concentrated *in vacuo* to give the crude, which was purified by silica gel chromatography (eluent: DCM/MeOH= 20/1) to give **1-(6-cyclopropyl-2-(2,2,2-trifluoro-1-hydroxyethyl)imidazo[1,2-a]pyridin-8-yl)-3-methylimidazolidine-2,4-dione intermediate 436** (800 mg, 73 %) as a yellow solid. ESI-MS: [M + H]⁺,369.1.

To a solution of **intermediate 436** (800 mg, 2.17 mmol) in DCM (20 mL) was added DMP (2.76 g, 6.51 mmol). The resulting mixture was stirred at room temperature for 3 h and then quenched with saturated aqueous Na₂S₂O₃ solution (30 mL) and extracted with DCM (3 x 50 mL). The combined organic layers were washed with brine (80 mL), dried over anhydrous Na₂SO₄, and concentrated *in vacuo* to give the crude, which was purified by silica gel chromatography (eluent: DCM/MeOH = 25/1) to give **1-(6-cyclopropyl-2-(2,2,2-trifluoroacetyl)imidazo[1,2-a]pyridin-8-yl)-3-methylimidazolidine-2,4-dione intermediate 437** (550 mg, 69%) as a yellow solid. ESI-MS: [M + H]⁺, 367.2.

To a solution of **intermediate 437** (350 mg, 0.96 mmol) and 2-methylpropane-2-sulfinamide (1.74 g, 14.40 mmol) in EtOH (20 mL) was added Ti(*i*-PrO)₄ (2.73 g, 9.6 mmol) dropwise. The resulting reaction was stirred at 50°C for 3 h, and then NaBH₃CN (363 mg, 5.76 mmol) was added. The resulting mixture was stirred at 50°C for another 16 h. The reaction was quenched with water (30 mL) then extracted with DCM (3 x 30 mL). The combined organic layers were washed with brine (50 mL), dried over anhydrous Na₂SO₄, and concentrated *in vacuo* to give the crude, which was purified by silica gel chromatography (eluent: DCM/MeOH = 10/1) to give **intermediate 434** (150 mg, 42%) as a white solid. ESI-MS: [M+H]⁺,368.1.

### Intermediate 438: 1-(2-(1-amino-2-methoxyethyl)-6-cyclopropylimidazo[1,2-a]pyridin-8-yl)-3-methylimidazolidine-2,4-dione

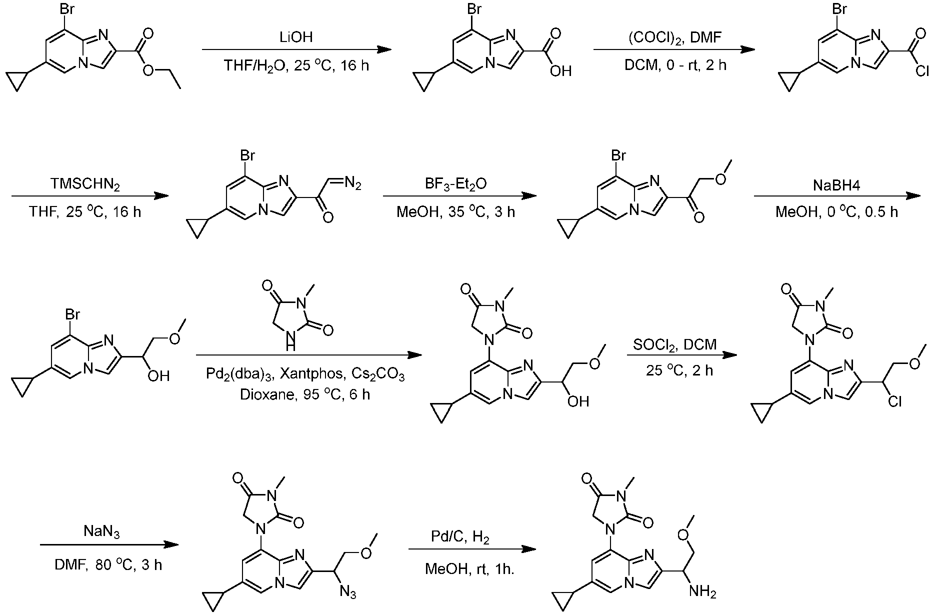

To a solution of **intermediate 451** (3.25 g, 10.51 mmol) in THF (20 mL), water (20 mL), and EtOH (4 mL) was added LiOH·H2O (882 mg, 21.02 mmol). After stirring at 25°C for 16 h, the resulting mixture was concentrated to give the crude, which was diluted with water (10 mL) and then acidified to pH 5 by adding HCl (1M aq.). The aqueous layer was extracted with EtOAc (3 x 30 mL), washed with brine (30 mL), dried over Na₂SO₄ and concentrated *in vacuo* to give **8-bromo-6-cyclopropylimidazo[1,2-a]pyridine-2-carboxylic acid intermediate 439** (2 g, 68%) as a white solid. ESI-MS [M + H ]⁺: 281.0.

To a solution of **intermediate 439** (2 g, 7.11 mmol) in DCM (50 mL) was added oxalyl chloride (1.8 g, 14.18 mmol) and catalytic amount of DMF at 0°C. After stirring at 25°C for 2 h, the resulting mixture was concentrated to give **8-bromo-6-cyclopropylimidazo[1,2-a]pyridine-2-carbonyl chloride intermediate 440** (2 g). This crude product was used for next step without further purification.

To a solution of **intermediate 440** (2 g, crude) in THF (15 mL) was added TMSCHN₂ (10 mL, 2 M solution in hexane) at 0°C. After stirring at 25°C for 16 h, the resulting mixture was concentrated to give **1-(8-bromo-6-cyclopropylimidazo[1,2-a]pyridin-2-yl)-2-diazoethan-1-one intermediate 441** (2 g) as a colorless oil,. This crude product was used to next step without further purification. ESI-MS [M + H ]+: 305.1.

To a solution of **intermediate 441** (2 g, crude) in MeOH was added boron trifluoride etherate (0.6 mL, 48% BF₃) at 0°C. After stirring at 35°C for 3 h, the resulting mixture was concentrated *in vacuo* to give the crude, which was purified by silica gel chromatography (eluent: EtOAc/PE = 0 ~ 100/1) to give **1-(8-bromo-6-cyclopropylimidazo[1,2-a]pyridin-2-yl)-2-methoxyethan-1-one intermediate 442** (900 mg, 41%) as a yellow solid. ESI-MS [M + H ]+: 309.0.

To a solution of **intermediate 442** (450 mg, 1.46 mmol) in MeOH (110 mL) was added NaBH₄ (110 mg, 2.91 mmol) at 0°C. After stirring at 25°C for 1 h, the resulting mixture was quenched with saturated aqueous NH₄Cl (20 mL) and extracted with EtOAc (3 x 20 mL). The combined organic layers were washed with brine (20 mL), dried over Na₂SO₄, and concentrated *in vacuo* to give the crude product, which was purified by silica gel chromatography (eluent: EtOAc/PE = 0 ~ 100/1) to give **1-(8-bromo-6-cyclopropylimidazo[1,2-a]pyridin-2-yl)-2-methoxyethan-1-ol intermediate 443** (200 mg, 44%) as a colorless oil. ESI-MS [M + H ]+: 311.0.

To a mixture of **intermediate 443** (150 mg, 0.48 mmol), **intermediate 322** (82 mg, 0.72 mmol), and Cs₂CO₃ (470 mg, 1.44 mmol) in dioxane (5 mL) were added Pd₂(dba)₃ (82 mg, 0.090 mmol) and Xantphos (110 mg, 0.190 mmol). The reaction mixture was stirred at 95°C for 16 h under N₂ and, after cooling to room temperature, the mixture was filtered through celite and the filter cake was washed with EtOAc (20 mL). The filtrate was concentrated to give the crude product, which was purified by column chromatography (eluent: EtOAc/PE = 0 ~ 100/1) to give **1-(6-cyclopropyl-2-(1-hydroxy-2-methoxyethyl)imidazo[1,2-a]pyridin-8-yl)-3-methylimidazolidine-2,4-dione intermediate 444** (120 mg, 73% yield) as a yellow solid,. ESI-MS [M + H ]+: 345.2.

To a solution of **intermediate 444** (100 mg, 0.29 mmol) in DCM (5 mL) was added SOCl₂ (1.0 mL) at 0°C. After stirring at 25°C for 2 h, the resulting mixture was concentrated to give the **1-(2-(1-chloro-2-methoxyethyl)-6-cyclopropylimidazo[1,2-a]pyridin-8-yl)-3-methylimidazolidine-2,4-dione intermediate 445** (100 mg). The crude colorless oil was used for next step directly without further purification.

To a solution of **intermediate 445** (100 mg, crude) in DMF (5 mL) was added NaN₃ (63 mg, 0.99 mmol) at room temperature and the reaction mixture was stirred at 80°C for 3 h. The reaction was cooled to room temperature, quenched with water (30 mL) and extracted with EtOAc (3 x 30 mL). The combined organic layers were concentrated *in vacuo* to give **1-(2-(1-azido-2-methoxyethyl)-6-cyclopropylimidazo[1,2-a]pyridin-8-yl)-3-methylimidazolidine-2,4-dione intermediate 446** (100 mg, crude) as a yellow oil. ESI-MS [M +H]⁺: 370.1.

To a solution of **intermediate 446** (100 mg, crude) in MeOH (5 mL) was added Pd/C (10 mg) at room temperature and the reaction mixture was stirred for 1 h. The mixture was then filtered and the filtrate was concentrated *in vacuo* to give **intermediate 438** (60 mg, crude) as a yellow oil which was used in next step without purification. ESI-MS [M +H]⁺: 344.1.

### Intermediate 4471-(2-(1-amino-2-hydroxyethyl)-6-cyclopropylimidazo[1,2-a]pyridin-8-yl)-3-methylimidazolidine-2,4-dione

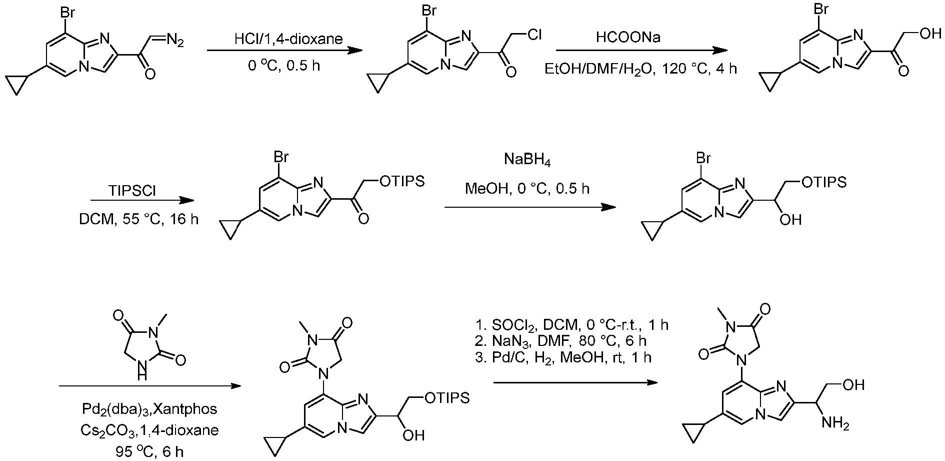

To a mixture of **1 intermediate 441** (2.1 g, 7.1 mmol) in THF (50 mL) was added HCl (4M solution in dioxane, 5 mL) at 0°C and stirred for 0.5 h. The reaction mixture was concentrated *in vacuo* to give the crude, which was purified by column chromatography (eluent: PE/EtOAc = 2/1) to give 1-**(8-bromo-6-cyclopropylimidazo[1,2-a]pyridin-2-yl)-2-chloroethan-1-one intermediate 448** (1.0 g, 45%) as a yellow solid. ESI-MS [M +H]+:313.0.

A mixture of **intermediate 448** (1.1 g, 3.5 mmol), HCOONa (0.72 g, 11 mmol) in a solution of EtOH (28 mL), DMF (10 mL) and water (8 mL) was stirred at 120°C for 4h. After cooling to 25°C, water (40 mL) was added, the reaction mixture was extracted by DCM (3 x 40 mL). The organic layers were concentrated *in vacuo* to give the crude, which was purified by silica gel chromatography (eluent: PE/EtOAc = 2/1) to give **1-(8-bromo-6-cyclopropylimidazo[1,2-a]pyridin-2-yl)-2-hydroxyethan-1-one intermediate 449** (0.54 g, 52%) as a pale solid. ESI-MS [M +H]+:295.1.

To a mixture of **intermediate 449** (0.54 g, 1.8 mmol) and imidazole (0.87 g, 13 mmol) in DCM (30 mL) was added TIPSCl (1.8 g, 9.2 mmol). The reaction was stirred at 55°C for 16 h under N₂. After cooling to 25°C, water (50 mL) was added and the mixture was extracted with EtOAc (3 x 30 mL). The combined organic layers were washed with brine (60 mL), dried over anhydrous Na₂SO₄, and concentrated *in vacuo* to give the crude, which was purified by silica gel chromatography (eluent: PE/EtOAc = 2/1) to give **1-(8-bromo-6-cyclopropylimidazo[1,2-a]pyridin-2-yl)-2-((triisopropylsilyl)oxy)ethan-1-one intermediate 450** (0.65 g, 80 %) as a pale solid. ESI-MS [M +H]+:451.1.

Using a similar procedure to that for **intermediate 438, intermediate 447** (170 mg, crude) was synthesised from **intermediate 450.** ESI-MS [M +H]+:330.1

### Intermediate 451 1-(2-(chloromethyl)-6-cyclopropylimidazo[1,2-a]pyridin-8-yl)-3-methylimidazolidine-2,4-dione

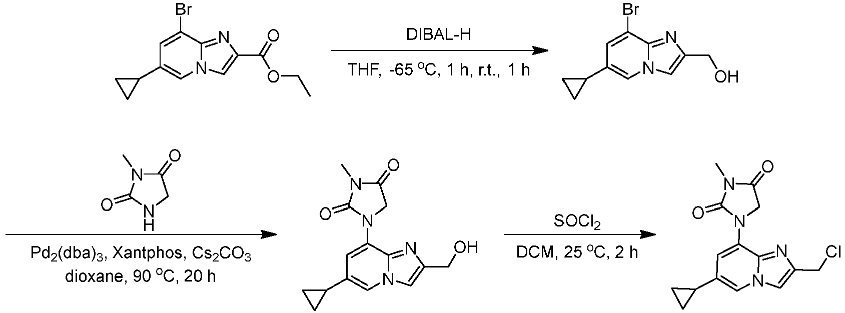

A mixture of **intermediate 309** (4.9 g, 23 mmol), ethyl 3-bromo-2-oxopropanoate (5.4 g, 28 mmol) in DME (100 mL) was stirred at 90°C for 24 h under N₂. After cooling to room temperature, the reaction was quenched with saturated aq. Na₂CO₃ (20 mL) and extracted with EtOAc (3 x 200 mL). The combined organic layers were washed with saturated brine solution (20 mL), dried over anhydrous Na₂SO₄, and concentrated *in vacuo* to give the crude, which was purified by column chromatography on silica gel (eluent: PE/EtOAc = 1/1) to give **ethyl 8-bromo-6-cyclopropylimidazo[1,2-a]pyridine-2-carboxylate intermediate 452** (4.0 g, 56%) as a yellow solid. ESI-MS [M +H]⁺: 309.1

A mixture of **intermediate 452** (4.0 g, 13 mmol) in THF(60 mL) was added DIBAL-H (1M in hexane, 40 mL, 40 mmol) dropwise. The resulting mixture was stirred at -65°C for 1 h and then warmed to 25°C and stirred for another 1 h under N₂. The reaction was quenched with NaOH (aqueous 10%, 30 mL) and extracted with EtOAc (4 x 50 mL). The combined organic layers were washed with saturated brine solution (30 mL), dried over anhydrous Na₂SO₄, and concentrated *in vacuo* to give the crude, which was purified by column chromatography on silica gel (eluent: PE/EtOAc = 3/1) to give **(8-bromo-6-cyclopropylimidazo[1,2-a]pyridin-2-yl)methanol intermediate 453** (2.0 g, 58%) as a yellow solid. ESI-MS [M +H]+: 267.1

To a mixture of **intermediate 453** (0.86 g, 3.2 mmol), **intermediate 322** (0.74 g, 6.5 mmol) and Cs₂CO₃ (2.6 g , 8.0 mmol) in dioxane (12 mL) were added Pd₂(dba)₃ (0.30 g, 0.33 mmol) and Xantphos(0.37 g, 0.64 mmol). The reaction mixture was stirred at 90°C for 20 h under N₂. After cooling to room temperature, the reaction mixture was filtered through Celite^{®} and the filter cake was washed with DCM/MeOH (10/1, 100 mL). The filtrate was concentrated *in vacuo* to give the crude, which was purified by column chromatography on silica gel (eluent: MeOH/DCM = 1/20) to give **1-(6-cyclopropyl-2-(hydroxymethyl)imidazo[1,2-a]pyridin-8-yl)-3-methylimidazolidine-2,4-dione intermediate 454** (0.44 g, 46%) as a yellow solid. ESI-MS [M +H]+: 300.1.

To a solution of **intermediate 454** (0.40 g, 1.3 mmol) in DCM(10 mL) was added SOCl₂ (3.0 mL). The resulting mixture was stirred at 25°C for 2 h under N₂. The reaction mixture was concentrated *in vacuo* to give **intermediate 451** (0.42 g, quant) as a yellow solid which was used to the next step without further purification. ESI-MS [M +H]+: 319.1.

### Intermediate 455 6-cyclopropyl-8-(3-methyl-2,4-dioxoimidazolidin-1-yl)imidazo[1,2-a]pyridine-2-carbaldehyde

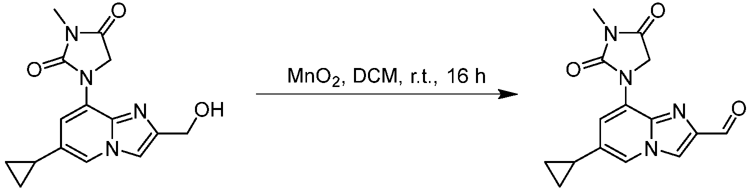

A mixture of 1-(6-cyclopropyl-2-(hydroxymethyl)imidazo[1,2-a]pyridin-8-yl)-3-methylimidazolidine-2,4-dione (0.30 g, 1.0 mmol) and MnO₂ (0.87 g, 10 mmol) in DCM (10.0 mL) was stirred at room temperature for 16 h. The reaction mixture was filtered through Celite^{®} and the filter cake was washed with DCM/MeOH (50/1, 50 mL). The filtrate was concentrated *in vacuo* to give the crude, which was purified by column chromatography on silica gel (eluent: DCM/MeOH = 50/1 ~ 20/1) to give **intermediate 455** (0.22 g, 74 %) as a yellow solid. ESI-MS [M +H]⁺: 299.2.

### Intermediate 456 1-(6-cyclopropyl-2-(1-hydroxyethyl)imidazo[1,2-a]pyridin-8-yl)-3-methylimidazolidine-2,4-dione

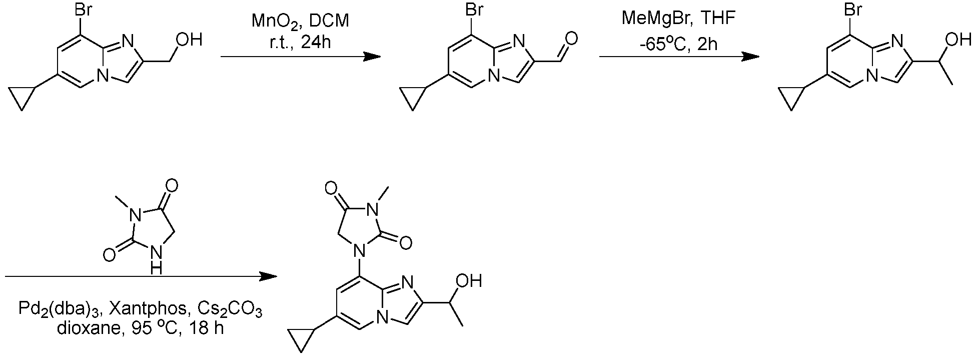

A mixture of **intermediate 453** (10 g, 37.45 mmol) and MnO₂ (9.8 g, 112 mmol) was stirred at room temperature for 12h. Second batch of MnO₂ (9.8 g, 112 mmol) was added and stirred at room temperature for another 12h. The reaction mixture was filtered through a short silica pad and washed with EtOAc (500 mL). The filtrate was concentrated *in vacuo* to give **8-bromo-6-cyclopropylimidazo[1,2-a]pyridine-2-carbaldehyde intermediate 457** (8 g, 80.8%) as a yellow solid. ESI-MS [M +H]⁺: 266.2.

To a solution of **intermediate 457** (8 g, 30 mmol) in dry THF (100 mL) was added MeMgBr (30 mL, 3M solution in Et2O, 90 mmol) at -65°C slowly and the resulting solution was stirred at -65°C for 2h. The reaction was quenched with saturated aqueous NH₄Cl (70 mL) at -65°C, stirred and warmed to room temperature then extracted with EtOAc (4 x 75 mL). The combined organic layers were washed with brine (75 mL), dried over Na₂SO₄ then concentrated *in vacuo* to give the crude product, which was purified with silica gel chromatography (eluent: DCM/MeOH = 15 / 1) to give **1-(8-bromo-6-cyclopropylimidazo[1,2-a]pyridin-2-yl)ethan-1-olintermediate 458** (7.3 g, 86.9%) as a yellow solid. ESI-MS [M +H]⁺: 281.2.

A mixture of **intermediate 458** (7.3 g, 25.98 mmol), **intermediate** 322 (8.9 g, 77.9 mmol), Pd₂(dba)₃ (2.38 g, 2.6 mmol), Xantphos (3 g, 5.2 mmol) and Cs₂CO₃ (25 g, 77 mmol) in dioxane (75 mL) was stirred at 95°C for 18h. The reaction mixture was cooled to room temperature, filtered through celite, washed with EtoAc (3 x 75 mL). The combined filtrate was washed with brine (80 mL), dried over Na₂SO₄ then concentrated *in vacuo* to give the crude product, which was purified with silica gel chromatography (eluent: DCM/MeOH = 15/1) to give **intermediate 456** (6.1 g, 75%) as a yellow solid. ESI-MS [M +H]⁺: 315.2.

### Intermediate 459: 3-(2-((tert-butyldimethylsilyl)oxy)ethyl)-1-(6-cyclopropyl-2-(hydroxymethyl)imidazo[1,2-a]pyri din-8-yl)imidazolidine-2,4-dione

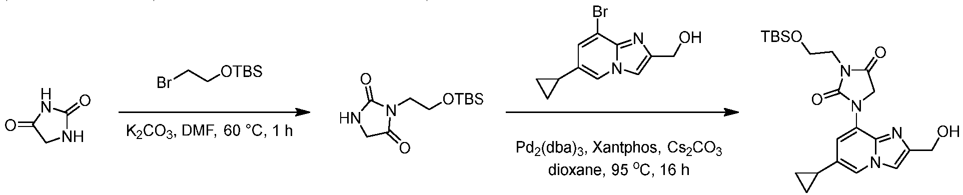

To the mixture of imidazolidine-2,4-dione (5 g, 59 mmol), K₂CO₃ (20.7 g, 150 mmol) in DMF (40 mL) was added (2-bromoethoxy)(tert-butyl)dimethylsilane (9.57 g, 40 mmol). The resulting reaction mixture was stirred at 60°C for 1 h under N₂. After cooling to room temperature, water (50 mL) was added and the mixture was extracted with EtOAc (3 x 30 mL). The combined organic layers were washed with brine (60 mL), dried over anhydrous Na₂SO₄ and concentrated *in vacuo* to give the crude product, which was purified by silica gel chromatography (eluent: MeOH/DCM = 0 ~ 1/10) to afford **3-(2-((tert-butyldimethylsilyl)oxy)ethyl)imidazolidine-2,4-dione intermediate 460** (710 mg, 5.5 % )as pale solid. ESI-MS [M +H]⁺: 259.2.

To a mixture of **intermediate 460** (720 mg, 2.79 mmol), **intermediate 453** (250 mg, 0.93 mmol), and Cs₂CO₃ (909 mg, 2.79 mmol) in dioxane (35 mL) was added Pd₂(dba)₃ (170 mg, 0.186 mmol) and Xantphos (215 mg, 0.372 mmol). The reaction mixture was stirred at 95°C for 16 h under N₂ and then cooled to room temperature. The solution was filtered through celite and the filter cake was washed with DCM/MeOH (10/1, 50 mL). The filtrate was concentrated *in vacuo* to give the crude, which was purified by column chromatography (eluent: EtOAc/PE=1/100~100/1) to afford **intermediate 459** (300 mg, 73%) as yellow solid. ESI-MS [M +H]⁺: 445.3.

### Intermediate 460: methyl 2-(3-(6-cyclopropyl-2-(hydroxymethyl)imidazo[1,2-a[pyridin-8-yl)-2,5-dioxoimidazolidin-1-yl)acetate

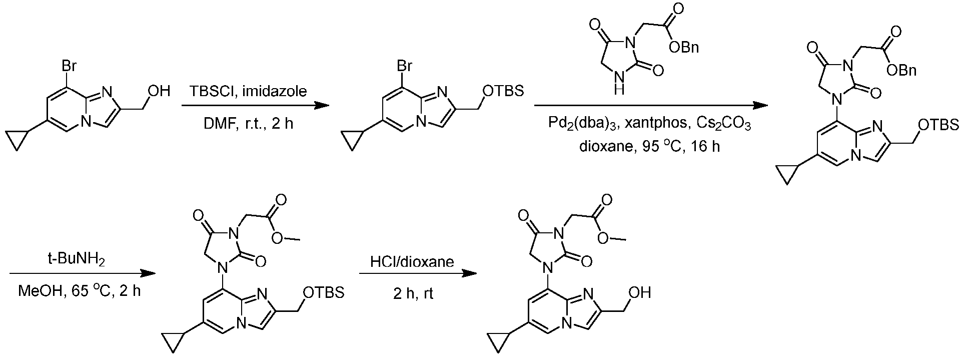

To a solution of **intermediate 453** (2 g, 7.5 mmol) in DMF (20 mL) was added tert-butylchlorodimethylsilane (1.69 g, 11.2 mmol) and imidazole (1.53 g, 22.5 mmol). The mixture was stirred at 25°C for 2 h, then water (50 mL) was added and the mixture was extracted with EtOAc (30 mL x 3). The combined organic layers were washed with brine (30 mL), dried over Na₂SO₄ and concentrated *in vacuo.* The resulting residue was purified by column chromatography (eluent: PE : EtAOc = 1 : 1) to give **8-bromo-2-(((tert-butyldimethylsilyl)oxy)methyl)-6-cyclopropylimidazo[1,2-a]pyridine intermediate 461** (2.6 g, 91%) as a purple oil. ESI-MS [M +H]⁺: 381.1.

To a solution of **intermediate 397** (3.1 g, 12.6 mmol) in dioxane (20 mL) was added **intermediate 461** (1.2 g, 3.15 mmol), Pd₂(dba)₃ (577 mg, 0.63 mmol), Xantphos (729 mg, 1.26 mmol) and Cs₂CO₃ (3.0 g, 9.45 mmol). The mixture was stirred at 95°C for 16 h under N₂, cooled to room temperature, quenched with water (50 mL) and extracted with EtOAc (3 x 50 ml). The combined organics were washed with brine (50 mL), dried over Na₂SO₄ and concentrated. The crude residue was purified by column chromatography (eluent: PE : EtAOc = 1 : 1) to give **intermediate 398** (1 g, 58%) as a yellow oil. ESI-MS [M +H]⁺: 549.2.

To a mixture of **intermediate 398** (1 g, 1.82 mmol) in MeOH (15 mL) was added t-BuN₃ (2.7 g, 36.4 mmol). The mixture was stirred at 65°C for 2 h under N₂ then cooled to room temperature and filtered. The filtrate was concentrated to give the crude product which was purified by column chromatography (eluent: DCM/MeOH = 20/1) to give **methyl 2-(3-(2-(((tertbutyldimethylsilyl)oxy)methyl)-6-cyclopropylimidazo[1,2-a]pyridin-8-yl)-2,5-dioxoimidazolidin-1-yl)acetate intermediate 462**(800 mg, 93%) as a yellow solid. ESI-MS [M +H]⁺: 473.2.

To a solution of **intermediate 462** (500 mg, 1.05 mmol) in MeOH (3 mL) was added HCl/dioxane (5.0 mL, 20 mmol) at 0°C and the mixture was stirred at RT for 2 h. Then the mixture was quenched with water (50 mL) and extracted with EtOAc (3 x 50 ml). The combined organics were washed with brine (50 mL), dried over Na₂SO₄ and concentrated *in vacuo.* The residue was was purified by preparative TLC (eluent: DCM/MeOH = 10/1) to give **intermediate 460** (370 mg, 98%) as a yellow solid. ESI-MS [M +H]⁺: 359.1.

### Intermediate 463: 3-(6-cyclopropyl-2-(hydroxymethyl)imidazo[1,2-a[pyridin-8-yl)-3-azabicyclo[3.1.0]hexan-2-one

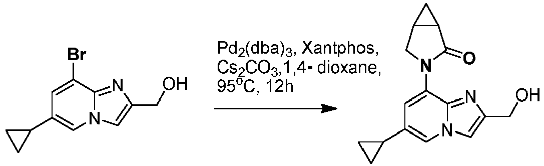

To a mixture of **intermediate 453** (300 mg, 1.12 mmol), 3-azabicyclo[3.1.0]hexan-2-one (217.3 mg, 2.24 mmol) and Cs₂CO₃ (1.1 g, 3.36 mmol) in 1,4-dioxane (5 mL) was added Pd₂(dba)₃ (102.5 mg, 0.112 mmol) and Xantphos (129.6 mg, 0.224 mmol). The reaction mixture was stirred at 95°C for 12 h under N₂ and cooled to room temperature. The solution was filtered through Celite^{®} and the filter cake was washed with DCM/MeOH (10/1, 100 mL). The filtrate was concentrated *in vacuo* to give the crude, which was purified by preparative TLC (DCM/MeOH=20/1) to give **intermediate 463** (250 mg, 79%) as a yellow solid. ESI-MS [M +H]+: 284.2.

### Intermediate 464: 1-(7-cyclopropyl-2-(hydroxynæthyl)imidazo[1,2-a]pyridin-5-yl)-3-methylimidazolidine-2,4-dione

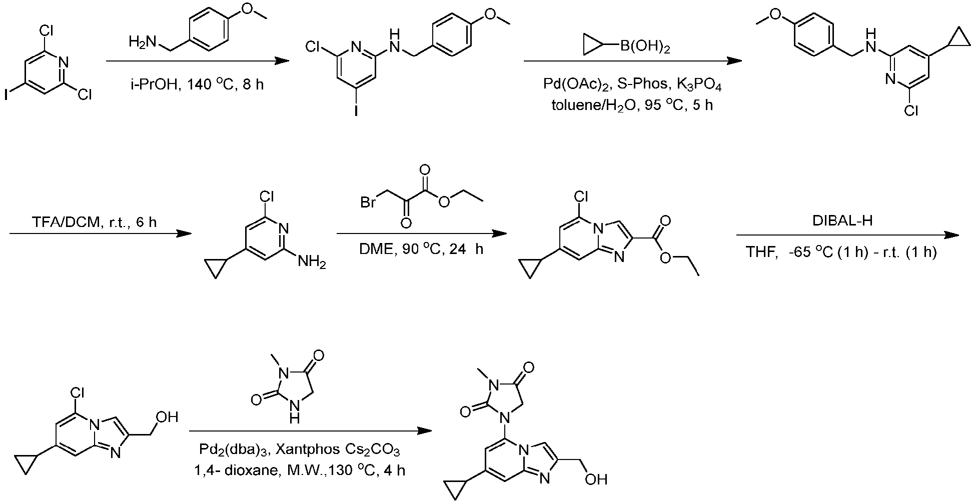

A mixture of 2,6-dichloro-4-iodopyridine (5.0 g, 18.26 mmol) and (4-methoxyphenyl)methanamine (12.5 g, 91.12 mmol) in i-PrOH (100 mL) was stirred in a sealed tube at 140°C for 8 h. The resulting mixture was concentrated *in vacuo* to give the crude, which was purified by silica gel chromatography (eluent: PE/EtOAc = 20/1) to give **6-chloro-4-iodo-N-(4-methoxybenzyl)pyridin-2-amine intermediate 465** (2.2 g, 32%) as a white solid. ESI-MS: [M + H]⁺: 374.9.

To a mixture of **intermediate 465** (2.5 g, 6.67 mmol), cyclopropylboronic acid (575 mg, 6.69 mmol), and K₃PO₄ (4.25 g, 20.02 mmol) in H₂O (6 mL) and toluene (60 mL) was added Pd(OAc)₂ (299 mg, 1.33 mmol) and S-Phos (1.1 g, 2.68 mmol). The reaction mixture was stirred at 95°C for 5 h under N₂. The reaction mixture was filtered through celite and the filter cake was washed with EtOAc (50 mL). The filtrate was concentrated *in vacuo* to give the crude, which was purified by silica gel chromatography (eluent: PE/EtOAc = 10/1) to give **6-chloro-4-cyclopropyl-N-(4-methoxybenzyl)pyridin-2-amine intermediate 466** ( 1.4 g, 73%) as a yellow solid. ESI-MS: [M + H]⁺: 289.1.

To a solution of **intermediate 466** (2.5 g, 8.66 mmol) in DCM (10 mL) was added TFA (2 mL). The reaction mixture was stirred at room temperature for 6 h. After cooling to 0°C, the mixture was adjusted to pH 8 by adding saturated NaHCO₃ solution. The resulting mixture was then extracted with DCM (300 mL x 3). The combined organic layers were washed with brine (50 mL), dried over anhydrous Na₂SO₄ and concentrated *in vacuo* to give the crude, which was purified by silica gel chromatography (eluent: PE/EA = 2/1) to give **6-chloro-4-cyclopropylpyridin-2-amine intermediate 467** (1.4 g, 95.9%) as a yellow solid. ESI-MS: [M + H]⁺, 289.1.

To a solution of **intermediate 467** (1.4 g, 8.30 mmol) in DME (30 mL) was added ethyl 3-bromo-2-oxopropanoate (1.9 g, 9.74 mmol). The reaction mixture was stirred at 90°C for 24 h under N₂. After cooling to room temperature, water (100 mL) was added and then extracted with DCM (400 mL x 3). The combined organic layers were washed with brine (100 mL), dried over anhydrous Na₂SO₄, and concentrated *in vacuo* to give the crude, which was purified by silica gel chromatography (eluent: EA/PE = 3/2) to **give ethyl 5-chloro-7-cyclopropylimidazo[1,2-a]pyridine-2-carboxylate intermediate 468** (740 mg, 34 %) as a yellow solid. ESI-MS: [M + H]⁺, 265.1.

To a solution of **intermediate 468** (2.5 g, 9.47 mmol) in THF (50 mL) was added DIBAL-H (1M solution in hexanes, 18.94 mL, 18.94 mmol) dropwise at -65°C. The resulting reaction was stirred at -65°C for 1 h under N₂, then warmed to room temperature for another 1 h. The reaction was quenched with H₂O (5 mL) at 0°C, followed by NaOH (5 mL, 15% aqueous solution) and H₂O (20 mL). The reaction mixture was warmed to room temperature and stirred for another 15 min then extracted with EtOAc (50 mL x 3). The combined organic layers were washed with brine (80 mL), dried over anhydrous Na₂SO₄, and concentrated *in vacuo* to give the crude, which was purified by silica chromatography (eluent: DCM/MeOH = 25/1) to give **(5-chloro-7-cyclopropylimidazo[1,2-a]pyridin-2-yl)methanol intermediate 469** (1.48 g, 70 %) as a yellow solid. ESI-MS: [M + H]⁺, 223.0

To a solution of **intermeidate 469** (230 mg, 1.04 mmol), **intermediate 322** (948 mg, 8.32 mmol) and Cs₂CO₃ (1.02 g, 3.12 mmol) in 1,4-dioxane (10 mL) was added Pd₂(dba)₃ (192 mg, 0.21 mmol) and XantPhos (243 mg, 0.42 mmol). The reaction mixture was stirred in a sealed tube under microwave at 130°C for 4 h. The reaction mixture was filtered through celite and the filter cake was washed with DCM/MeOH (10/1, 100 mL). The filtrate was concentrated *in vacuo* to give the crude, which was purified by silica gel chromatography (eluent: DCM/MeOH = 25/1) to give **intermediate 464** (300 mg, 96 %) as a yellow solid. ESI-MS: [M + H]⁺,301.1

### Intermediate 470: (6-cyclopropyl-8-(4-methylpiperazin-1-yl)imidazo[1,2-a]pyridin-2-yl)methanol

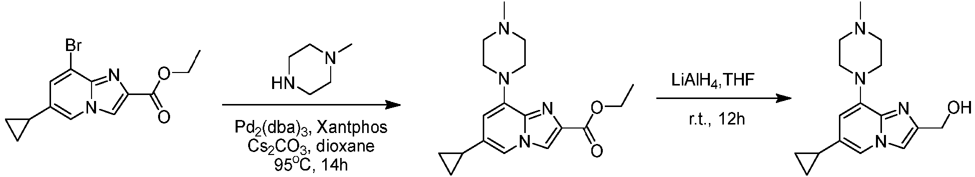

To a mixture of **intermediate 452** (500 mg,1.6 mmol), 1-methylpiperazine (320 mg, 3.2 mmol) and Cs₂CO₃ (1.56 g, 4.8 mmol) in 1,4-dioxane (5 mL) was added Pd₂(dba)₃ (146.5 mg,0.16 mmol) and Xantphos (185.2 mg,0.32 mmol). The resulting mixture was stirred at 95°C for 14h under N₂. The reaction mixture was filtered through Celite^{®} and the filter cake was washed with DCM / MeOH (10 / 1, 10 mL). The filtrate was concentrated *in vacuo* to give the crude, which was purified by preparative TLC (DCM/MeOH = 20/1) to give **ethyl 6-cyclopropyl-8-(4-methylpiperazin-1-yl)imidazo[1,2-a]pyridine-2-carboxylate(**300 mg, 57%) as a white solid. ESI-MS [M +H]+: 329.2.

To a mixture of 6-cyclopropyl-8-(4-methylpiperazin-1-yl)imidazo[1,2-a]pyridine-2-carboxylate (300 mg, 0.91 mmol) in THF (10 mL) was added LiAlH₄ (103.4 mg, 2.73 mmol) at 0°C. After stirring at room temperature for 12h, the reaction was quenched with saturated aq. NH₄Cl (25 mL) then extracted with EtOAc (3 x 30 mL). The combined organic layers were washed with brine, dried over Na₂SO₄, filtered and concentrated *in vacuo* to give the crude, which was purified by preparative TLC (DCM/MeOH = 60/1) to give **intermediate 470** (180 mg, 69%) as a white solid. ESI-MS [M +H]+: 287.2.

### Intermediate 471: (8-(benzyloxy)-6-cyclopropylimidazo[1,2-a]pyridin-2-yl)methanamine

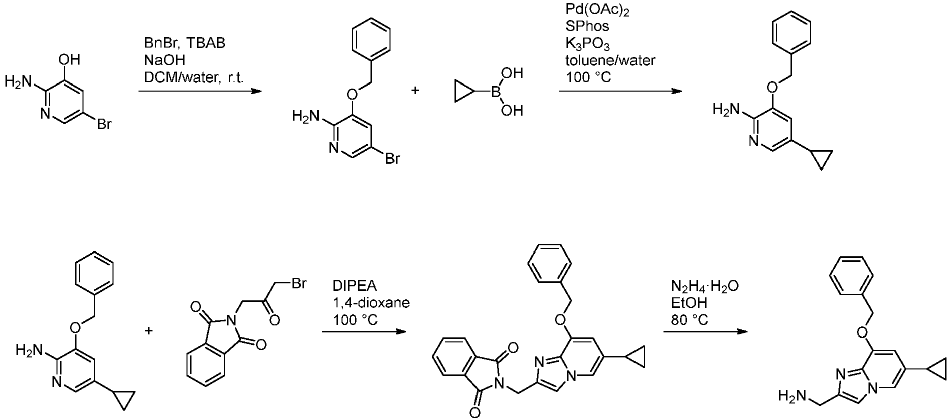

2-Amino-5-bromopyridin-3-ol (13 g, 67 mmol) was added portionwise to a vigorously stirred mixture of NaOH (15 g, 380 mmol) in water (24 mL) and DCM (40 mL). TBAB (0.36 g, 1.1 mmol) was added and the mixture was stirred at room temperature for 15 min. A solution of benzyl bromide (13 g, 74 mmol) in DCM (40 mL) was added and the mixture was stirred at room temperature for 18 h. Water (100 mL) was added and the mixture was extracted with DCM (3 × 100 mL). The combined organic layers were dried (MgSO₄), filtered and concentrated *in vacuo.* The residue was purified by column chromatography on silica gel, eluting with 20-80 % EtOAc in cyclohexane to give **3-(benzyloxy)-5-bromopyridin-2-amine intermediate 472(13** g, 67 %) as a yellow solid. ESI-MS (M+H)+: 281.1, 279.1 δ 7.60 - 7.59 (m, 1H), 7.53 - 7.50 (m, 2H), 7.44 - 7.34 (m, 3H), 7.29 (d, J=2.0 Hz, 1H), 5.96 (s, 2H), 5.17 (s, 2H).

A mixture of **intermediate 472** (13 g, 45 mmol), cyclopropylboronic acid (5.8 g, 67 mmol), Pd(OAc)₂ (0.50 g, 2.2 mmol), SPhos (1.8 g, 4.5 mmol) and K₃PO₄ (33 g, 160 mmol) in toluene (130 mL) and water (13 mL) was degassed with N₂ then stirred at 100°C under a N₂ atmosphere for 18 h. The mixture was cooled and the organic layer was decanted and filtered through Celite^{®}. The filtrate was diluted with EtOAc and the organic layer further decanted and repeatedly filtered through Celite^{®}. The combined filtrates were concentrated *in vacuo* and the residue was purified by column chromatography on silica gel, eluting with 20-100 % EtOAc in cyclohexane to give **3-(benzyloxy)-5-cyclopropylpyridin-2-amine intermediate 473** (8.7 g, 89 %) as a yellow solid. ESI-MS (M+H)+: 241.3, δ 7.51 - 7.49 (m, 2H), 7.42 - 7.31 (m, 4H), 6.77 (d, J=1.9 Hz, 1H), 5.41 (s, 2H), 5.12 (s, 2H), 1.80 - 1.73 (m, 1H), 0.85 - 0.79 (m, 2H), 0.58 - 0.53 (m, 2H).

A mixture of 3-(benzyloxy)-5-cyclopropylpyridin-2-amine (4.1 g, 17 mmol), **intermediate** 311 (5.2 g, 18.6 mmol) and DIPEA (3.2 mL, 19 mmol) in 1,4-dioxane (340 mL) was stirred at 100°C for 18 h then concentrated *in vacuo.* The residue was dissolved in DCM (250 mL) and washed with water (150 mL), then a solution of NaHCO₃ (sat. aq., 150 mL), then brine (sat. aq., 150 mL) and filtered through a phase separator then concentrated *in vacuo.* The residue was purified by column chromatography on silica gel, eluting with 0-100 % EtOAc in cyclohexane to give ***2-((8-(benzyloxy)-6-cyclopropylimidazol1,2-a]pyridin-2-yl)methyl)isoindoline-1,3-dione* intermediate 474** the title compound (2.4 g, 34 %) as a green solid. ESI-MS (M+H)+: 424.4 δ 7.94 - 7.86 (m, 5H), 7.71 (s, 1H), 7.49 - 7.46 (m, 2H), 7.43 - 7.35 (m, 3H), 6.44 (d, J=1.1 Hz, 1H), 5.25 (s, 2H), 4.84 (s, 2H), 1.91 - 1.83 (m, 1H), 0.92 - 0.86 (m, 2H), 0.68 - 0.63 (m, 2H).

A mixture of **intermediate 474** (0.50 g, 1.2 mmol) and hydrazine monohydrate (0.74 mL, 12 mmol) in EtOH (12 mL) was stirred at 80°C for 1 h. The mixture was cooled to room temperature, loaded onto an SCX cartridge and washed with a mixture of DCM and MeOH (1:1). The product was eluted with a mixture of DCM and 7 N NH₃ in MeOH (1:1) to give the **intermediate 471** (450 mg, quantitative) as a yellow gum. ESI-MS (M+H)+: 294.2 δ 7.95 (s, 1H), 7.63 (s, 1H), 7.51 (d, J=6.9 Hz, 2H), 7.46 - 7.37 (m, 3H), 6.44 (s, 1H), 5.26 (s, 2H), 3.77 (s, 2H), 1.94 - 1.86 (m, 1H), 0.93 - 0.87 (m, 2H), 0.71 - 0.66 (m, 2H). NH₂ not observed

### Intermediate 475: 3-((2-(aminomethyl)-6-cyclopropylimidazo[1,2-a]pyridin-8-yl)oxy)-1-methylpyrrolidin-2-one

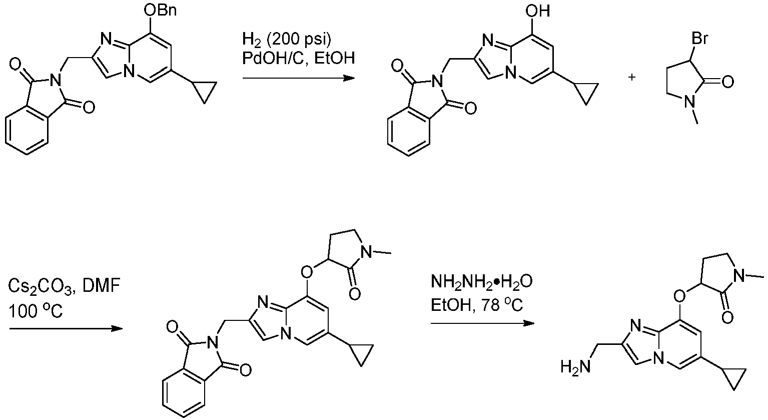

**Intermediate 474** (1.90 g, 4.49 mmol) and palladium hydroxide (10 mol% on carbon, 1.9 g, 1.35 mmol) were suspended in ethanol (90 mL) and degassed with N₂ for 5 min. The reaction was then placed under H₂ (200 psi) and the reaction stirred at room temperature for 18 h. The reaction was filtered through Celite^{®} which was then washed with MeOH (50 mL). The reaction was concentrated *in vacuo.* The residue was purified by silica gel chromatography, eluting with a gradient of 0-20 % MeOH in DCM to give ***2-((6-cyclopropyl-8-hydroxyimidazo[1,2-a]pyridin-2-yl)methyl)isoindoline-1,3-dione* intermediate 476** (790 mg, 53 %). ESI-MS (M+H)+: 334.1 δ 7.98 - 7.90 (m, 4H), 7.84 (d, J=1.0 Hz, 1H), 7.74 (s, 1H), 6.26 - 6.23 (m, 1H), 4.90 (s, 2H), 1.91 - 1.76 (m, 1H), 0.94 - 0.88 (m, 2H), 0.65 - 0.60 (m, 2H).

**Intermediate 476** (200 mg, 0.60 mmol), 3-bromo-1-methylpyrrolidin-2-one (117 mg, 0.66 mmol) and Cs₂CO₃ (215 mg, 0.66 mmol) were suspended in DMF (3.0 mL) and the reaction was heated to 100°C for 30 min. The reaction was cooled to room temperature and diluted with EtOAc (10 mL) and NaHCO₃ (sat. aq., 15 mL), the layers were separated and the aqueous layer was further extracted with EtOAc (2 x 30 mL). The combined organics were dried over MgSO₄ then concentrated *in vacuo.* The residue was purified by silica gel chromatography, eluting with a gradient of 0-20 % (7N NH₃ in MeOH) in DCM to give ***2-((6-cyclopropyl-8-((1-methyl-2-oxopyrrolidin-3-yl)oxy)imidazo[1,2-a]pyridin-2-yl)methyl)isoindoline-1,3-dione* intermediate 477** (200 mg, 78 %). ESI-MS (M+H)+: 431.3, ¹H NMR (400 MHz, CDCl₃) δ 7.88 - 7.85 (m, 2H), 7.71 (dd, J=3.0, 5.6 Hz, 2H), 7.48 (s, 1H), 7.39 (s, 1H), 6.76 - 6.73 (m, 1H), 5.30 (s, 2H), 5.26 (dd, J=5.4, 8.0 Hz, 1H), 3.62 - 3.55 (m, 1H), 3.40 - 3.32 (m, 1H), 2.61 - 2.38 (m, 2H), 1.87 - 1.78 (m, 2H), 1.47 - 1.43 (m, 1H), 0.92 - 0.86 (m, 2H), 0.66 - 0.61 (m, 2H).

Using a similar procedure to that for **intermediate 278, intermediate 475** (125 mg, 89 %) was synthesised from **intermediate 477** (200 mg, 0.46 mmol) using hydrazine hydrate. ESI-MS (M+H)+: 301.2, ¹H NMR (400 MHz, MeOD) δ 7.88 (s, 1H), 7.65 (s, 1H), 6.76 (s, 1H), 5.30 (dd, J=6.2, 7.8 Hz, 1H), 3.93 (s, 2H), 3.66 - 3.59 (m, 1H), 3.56 - 3.47 (m, 1H), 2.96 (s, 3H), 2.72 - 2.60 (m, 1H), 2.32 - 2.23 (m, 1H), 1.99 - 1.92 (m, 1H), 1.01 - 0.92 (m, 2H), 0.79 - 0.74 (m, 2H). The compound in Table A12 were synthesized using a similar method to **intermediate 476,** starting from **intermediate 477** and the appropriate alkyl halide. **Table A12**

| Compound | Alkyl halide | Analytical data |
|---|---|---|
| 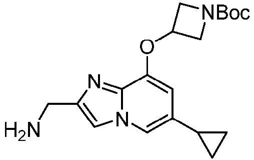 tert-butyl 3-((2-(aminomethyl)-6-cyclopropylimidazo[1,2-a]pyridin-8-yl)oxy)azetidine-1-carboxylate | *tert*-butyl 3-iodoazetidine-1-carboxylate | δ 8.02 (s, 1H), 7.70 (s, 1H), 6.18 (d, J=1.3 Hz, 1H), 5.30 - 5.23 (m, 1H), 4.39 (dd, J=5.6, 8.1 Hz, 2H), 3.91 (dd, J=2.9, 9.5 Hz, 2H), 1.98 - 1.90 (m, 1H), 1.45 (s, 9H), 0.95 (ddd, J=4.4, 6.4, 8.4 Hz, 2H), 0.76 - 0.71 (m, 2H) |

### Intermediate 478: (6-cyclopropyl-8-(2-methoxypyridin-3-yl)imidazo[1,2-a]pyridin-2-yl)methanamine

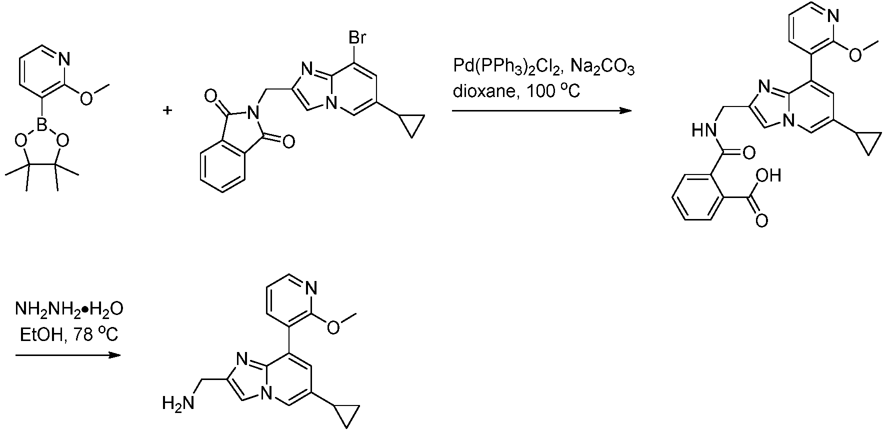

**Intermediate 310** (500 mg, 1.26 mmol), 2-methoxy-3-(4,4,5,5-tetramethyl-1,3,2-dioxaborolan-2-yl)pyridine (326 mg, 1.39 mmol) and Na₂CO₃ (1M aq., 3.8 mL, 3.8 mmol) were suspended in 1,4-dioxane (5.0 mL) and degassed with N₂ for 5 min. Pd(PPh₃)Cl₂ (89 mg, 0.126 mmol) was then added and the reaction was heated to 100°C under a N₂ environment for 30 min. The reaction was cooled to room temperature and filtered through Celite^{®}, which was then washed with DCM:MeOH 2:1 (10 mL). The reaction was concentrated *in vacuo.* The residue was purified by silica gel chromatography, eluting with a gradient of 0-100 % (7N NH₃ in MeOH) in DCM to give ***2-(((6-cyclopropyl-8-(2-methoxypyridin-3-yl)imidazo[1,2-a]pyridin-2-yl)methyl)carbamoyl)benzoic acid* intermediate 479** (550 mg, 98 %). ESI-MS (M+H)+: 443.4, ¹H NMR (400 MHz, CDCl₃) δ 8.27 - 8.26 (1H, m), 8.06 - 8.06 (2H, m), 7.74 - 7.73 (2H, m), 7.56 - 7.54 (2H, m), 7.17 - 7.17 (2H, m), 6.98 (1H, s), 6.82 - 6.82 (1H, m), 3.81 (2H, s), 3.47 (3H, s), 1.85 - 1.76 (1H, m), 0.94 -0.87 (2H, m), 0.62-0.58 (2H, m)

Using a similar procedure to that **forintermediate 278, intermediate 478** (370 mg, 99 %) was synthesised from **intermediate 479** (550 mg, 1.26 mmol) using hydrazine hydrate. ESI-MS (M+H)+: 295.3, ¹H NMR (400 MHz, MeOD) δ 8.14 - 8.11 (m, 2H), 7.76 (dd, J=2.0, 7.3 Hz, 1H), 7.67 (s, 1H), 7.01 - 6.97 (m, 2H), 3.88 (s, 2H), 3.79 (s, 3H), 1.93 - 1.85 (m, 1H), 0.93 - 0.87 (m, 2H), 0.67 - 0.62 (m, 2H).

### Alternative route to Intermediate 273: ethyl 3-(2-(aminomethyl)-6-cyclopropylimidazo[1,2-alpyridin-8-yl)propanoate

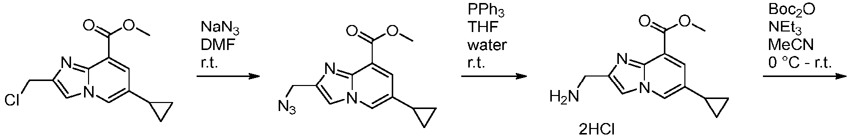

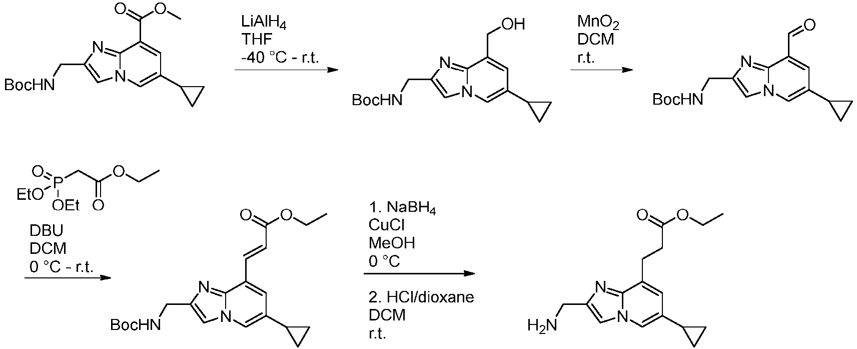

Sodium azide (1.1 g, 17 mmol) was added to a mixture of methyl 2-(chloromethyl)-6-cyclopropylimidazo[1,2-*a*]pyridine-8-carboxylate hydrochloride (3.4 g, 11 mmol) and NEt₃ (2.6 g, 26 mmol) in DMF (25 mL). The mixture was stirred at room temperature under a nitrogen atmosphere for 72 h. The mixture was poured into EtOAc (330 mL) and washed with water (330 mL), brine (sat. aq., 330 mL), water (330 mL), dried over MgSO₄, filtered and concentrated *in vacuo* to give ***methyl 2-(azidomethyl)-6-cyclopropylimidazo[1,2-a]pyridine-8-carboxylate* intermediate 480** the title compound (2.7 g, 77 %) as a brown oil. ESI-MS [M+H]+: 272.2, δ 8.62 (dd, J=0.6, 1.8 Hz, 1H), 7.96 (m, 1H), 7.64 (d, J=1.9 Hz, 1H), 4.55 (s, 2H), 3.89 (s, 3H), 2.06 - 1.99 (m, 1H), 0.99 - 0.93 (m, 2H), 0.75 - 0.70 (m, 2H).

A mixture of **intermediate 479** (2.7 g, 10 mmol) and triphenylphosphine (5.3, 20 mmol) in THF (45 mL) and water (5.0 mL) was stirred at room temperature for 18 h. The mixture was concentrated *in vacuo* then dissolved in DCM and treated with HCl (4.0 M in 1,4-dioxane). The precipitate was collected by filtration and washed with Et₂O to give **methyl 2-(aminomethyl)-6-cyclopropylimidazo[1,2-*a*]pyridine-8-carboxylate dihydrochloride intermediate 481** (2.4 g, 76 %) as a cream solid. ESI-MS [M+H]+: 246.2, δ 8.98 (s, 1H), 8.62 (br s, 3H), 8.25 (s, 1H), 8.12 (s, 1H), 4.36 (s, 2H), 4.00 (s, 3H), 2.18 - 2.14 (m, 1H), 1.10 - 1.04 (m, 2H), 0.86 - 0.82 (m, 2H). **Alternative route to Intermediate 385:** Di-*tert*-butyl dicarbonate (1.7 g, 7.6 mmol) was added to a mixture of methyl2-(aminomethyl)-6-cyclopropylimidazo[1,2-a]pyridine-8-carboxylate **dihydrochloride** (2.4 g, 7.6 mmol) and NEt₃ (5.3 mL, 38 mmol) in MeCN (25 mL) 0°C. The mixture allowed to warm to room temperature and stirred for 18 h under a nitrogen atmosphere. The mixture was diluted with NaHCO₃ (sat. aq., 50 mL) and water (50 mL) then extracted with DCM (100 mL × 3). The combined organic layers were dried over MgSO₄, filtered and concentrated *in vacuo* to the title compound (2.2 g, 82 %) as a yellow gum. δ 8.59 (s, 1H), 7.70 (s, 1H), 7.36 - 7.34 (m, 1H), 4.23 (d, J=5.6 Hz, 2H), 3.88 (s, 3H), 2.04 - 1.95 (m, 1H), 1.40 (s, 9H), 0.98 - 0.91 (m, 2H), 0.73 - 0.67 (m, 2H), exchangeable NH not observed.

**Alternative route to Intermediate 386**LiAlH₄ (1.0 M in THF, 6.3 mL, 6.3 mmol) was added dropwise to a solution of **intermediate 385** (2.2 g, 6.3 mmol) in THF (41 mL) at -40°C under a nitrogen atmosphere. The mixture was stirred at -40°C for 30 min then warmed to 0°C and stirred for 30 min then warmed to room temperature and stirred for 30 min. The mixture was cooled to 0°C and further LiAlH₄ (1.0 M in THF, 3.2 mL, 3.2 mmol) was added. The mixture was warmed to room temperature and stirred for 45 min. The mixture was cooled to 0°C and water (1.5 mL) and NaOH (20 % aq., 0.35 mL) were added. The mixture was warmed to room temperature and stirred for 15 min. Et₂O (10 mL) was added and the mixture was filtered through Celite^{®} with THF and concentrated *in vacuo.* The residue was redissolved in THF (21 mL) and cooled to -40°C under a nitrogen atmosphere. LiAlH₄ (1.0 M in THF, 2.8 mL, 2.8 mmol) was added dropwise and the mixture was stirred at -40°C for 1.5 h. Further LiAlH₄ (1.0 M in THF, 2.8 mL, 2.8 mmol) was added and the mixture was stirred at -40°C for a further 30 min. The mixture was warmed to room temperature and stirred for 2 h. The mixture was cooled to 0°C and water (0.21 mL), NaOH (20 % aq , 0.16 mL) and water (0.66 mL) were added slowly. The mixture was warmed to room temperature and stirred for 15 min. MgSO₄ was added and the mixture was filtered through Celite^{®} with THF and concentrated *in vacuo* to give the title compound (1.5 g, 75 %) as a yellow gum. ESI-MS [M+H]+: 318.2, δ 8.22 (s, 1H), 7.58 (s, 1H), 7.29 - 7.26 (m, 1H), 6.97 - 6.95 (m, 1H), 5.30 (t, J=5.7 Hz, 1H), 4.75 (d, J=5.3 Hz, 2H), 4.19 (d, J=5.9 Hz, 2H), 1.97 - 1.89 (m, 1H), 1.40 (s, 9H), 0.95 - 0.89 (m, 2H), 0.68 - 0.63 (m, 2H).

### Intermediate 482: tert-butyl ((6-cyclopropyl-8-formylimidazo[1,2-a]pyridin-2-yl)mthyl)carbamate

To a solution of **intermediate 386** (400 mg, 1.3 mmol) in DCM (15 mL) was added MnO₂ (1.1 g, 13 mmol). The mixture was stirred at room temperature for 2 h under a nitrogen atmosphere. The mixture was filtered through Celite^{®} with DCM then THF (x 3) and concentrated *in vacuo* to give **intermediate 482**(210 mg, 32 %) as a yellow oil. ESI-MS [M+H]+: 316.2, ¹ δ 10.48 (s, 1H), 8.70 (s, 1H), 7.75 (s, 1H), 7.42 - 7.36 (m, 1H), 6.87 (s, 1H), 4.27 (d, J=5.9 Hz, 2H), 2.08 - 1.99 (m, 1H), 1.41 (s, 9H), 1.01 - 0.94 (m, 2H), 0.77 - 0.71 (m, 2H).

### Intermediate 483: ethyl (E)-3-(2-(((tert-butoxycarbonyl)amino)methyl)-6-cyclopropylimidazo[1,2-alpyridin-8-yl)acrylate

DBU (85 µl, 0.57 mmol) was added slowly to a solution of **intermediate 482**(180 mg, 0.57 mmol) and triethyl phosphonoacetate (140 mg, 0.628 mmol) in DCM (10 mL) at 0°C under a nitrogen atmosphere. The mixture was stirred at room temperature for 72 h. The mixture was diluted with water (20 mL) and brine (sat. aq., 30 mL) and extracted with DCM (3 x 50 mL). The combined organic layers were passed through a phase separator and concentrated *in vacuo.* The residue was purified by column chromatography on silica gel, eluting with 1-5 % NH₃ in MeOH in DCM to give **intermediate 483** (270 mg, quant.) as a yellow oil which was used directly in the next step without further purification. ESI-MS [M+H]+: 386.3.

### Intermediate 484: ethyl 3-(2-(((tert-butoxycarbonyl)amino)methyl)-6-cyclopropylimidazo[1,2-a]pyridin-8-yl)propanoate

NaBH₄ (240 mg, 6.2 mmol) was added to a mixture of **intermediate 483** (240 mg, 0.62 mmol) and CuCl (62 mg, 0.62 mmol) in MeOH (10 mL) in three portions at 0°C under a nitrogen atmosphere. The mixture was stirred at 0°C for 3 h. The mixture was diluted with NaHCO₃ (sat. aq., 5.0 mL), water (50 mL) and NaHCO₃ (sat. aq., 45 mL) and extracted with EtOAc (3 × 75 mL). The combined organic layers were dried over MgSO₄, filtered and concentrated *in vacuo* to give **intermediate 484** (280 mg, quant.) as a yellow oil which was used directly in the next step without further purification. ESI-MS [M+H]+: 388.3.

HCl (4.0 M in 1,4-dioxane, 0.9 mL, 3.6 mmol) was added to a solution of **intermediate 484** (280 mg, 0.72 mmol) in DCM (2 mL). The mixture was stirred at room temperature for 90 min then concentrated *in vacuo* to give **intermediate 273** (250 mg, quant.) as a yellow oil which was used directly in the next step. ESI-MS [M+H]+: 288.3.

### Intermediate 485: (6-cyclopropyl-8-(methylsulfonyl)imidazo[1,2-a]pyridin-2-yl)methanol

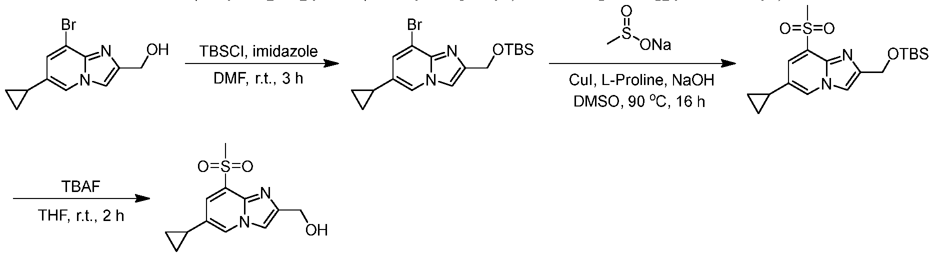

To a mixture of **intermediate 453** (0.20 g, 0.75 mmol), imidazole (0.77 g, 11 mmol) in DMF (10 mL) was added TBSCl (0.85 g, 5.6 mmol). The mixture was stirred at room temperature for 3 h under N₂. The reaction was quenched with water (20 mL) and extracted with EtOAc (3 x 50 mL). The combined organic layers were washed with saturated brine solution (30 mL), dried over Na₂SO₄, and concentrated *in vacuo* to give the crude, which was purified by silica gel chromatography (eluent: PE/EtOAc = 20/1 ~ 5/1) to give **intermediate 461** (0.15 g, 52%) as a yellow oil. ESI-MS [M +H]+: 383.1

To a mixture of **intermediate 461** (0.96 g, 2.5 mmol) in DMSO (25 mL) were added sodium methanesulfinate (2.6 g, 25 mmol), NaOH (0.10 g, 2.5 mmol), L-Proline (0.29 g, 2.5 mmol) and CuI (0.49 g, 2.6 mmol). The mixture was stirred at 90°C for 16 h. After cooling to room temperature, the reaction was quenched with water (30 mL) and extracted with EtOAc (3 x 50 mL). The combine organic layers were washed with saturated brine solution (60 mL), dried over Na₂SO₄, and concentrated *in vacuo* to give the crude, which was purified by column chromatography on silica gel (eluent: PE/ EtOAc = 10/1 ~ 3/1) to give ***2-(((tert-butyldimethylsilyl)oxy)methyl)-6-cyclopropyl-8-(methylsulfonyl)-imidazo[1,2-a]pyridine* Intermediate 486** (0.59 g, 62%) as a yellow solid. ESI-MS [M +H]+: 381.1

To a mixture **intermediate 486** (0.30 g, 0.79 mmol) in THF (10 mL) was added TBAF (1.6 mL, 1.6 mmol). After stirring at room temperature for 2 h, the mixture was concentrated *in vacuo* to give the crude **intermediate 485** (0.10 g, 48%) as a white solid. ESI-MS [M +H]+: 267.2

### Intermediate 487: (6-cyclopropyl-8-(methylsulfonyl)imidazo[1,2-a]pyridin-2-yl)methanamine

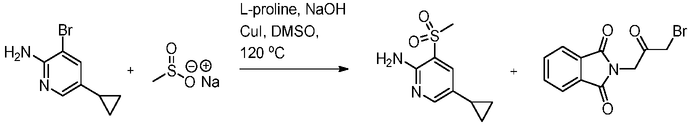

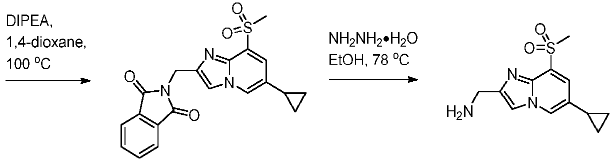

**intermediate 309** (250 mg, 1.17 mmol), sodium methanesulfinate (299 mg, 2.93 mmol), L-proline (135 mg, 1.17 mmol), NaOH (47 mg, 1.17 mmol) and CuI (223 mg, 1.17 mmol) were suspended in DMSO (1.5 mL), the reaction was degassed with N₂ for 5 min. The reaction was then heated at 120°C for 18 h. The reaction was cooled to room temperature then filtered through Celite^{®}. The Celite^{®} was then washed with 10 % MeOH in DCM (20 mL). The washings were concentrated *in vacuo,* and the residue was purified by silica gel chromatography, eluting with a gradient of 25-100 % EtOAc in cyclohexane to give ***5-cyclopropyl-3-(methylsulfonyl)pyridin-2-amine* intermediate 488** the title compound (145 mg, 58 %). ¹H NMR (400 MHz, CDCl₃) δ 8.23 - 8.18 (m, 1H), 7.67 (dd, J=4.0, 4.0 Hz, 1H), 5.76 (s, 2H), 3.07 - 3.06 (m, 3H), 1.88 - 1.79 (m, 1H), 1.00 - 0.83 (m, 2H), 0.68 - 0.53 (m, 2H).

DIPEA (0.24 mL, 1.37 mmol) was added to a stirred solution of **intermediate 488** (145 mg, 0.686 mmol) and **intermediate 311** (194 mg, 0.686 mmol) in 1,4-dioxane (7.0 mL). The reaction was heated at 100°C for 18 h. The reaction was cooled to room temperature and the reaction was concentrated *in vacuo.* The residue was dissolved in EtOAc (100 mL) and washed with NaHCO₃ (sat. aq., 2 x 40 mL) and brine (50 mL). The combined organics were dried over MgSO₄ and concentrated *in vacuo.* The residue was purified by silica gel chromatography, eluting with a gradient of 20-100 % EtOAc in cyclohexane to give ***2-((6-cyclopropyl-8-(methylsulfonyl)imidazo[1,2-a]pyridin-2-yl)methyl)isoindoline-1,3-dione* intermediate 489** (100 mg, 37 %). ¹H NMR (400 MHz, CDCl₃) δ 8.00 (d, J=1.0 Hz, 1H), 7.89 - 7.86 (m, 2H), 7.74 (dd, J=2.9, 5.4 Hz, 2H), 7.68 - 7.65 (m, 1H), 7.55 (s, 1H), 5.11 (s, 2H), 3.51 (s, 2H), 1.96 - 1.84 (m, 1H), 1.04 - 0.94 (m, 2H), 0.72 - 0.63 (m, 2H).

Using a similar procedure to that for **intermediate 278, intermediate 487** (67 mg) was synthesised from **intermediate 489** (100 mg, 0.253 mmol) using hydrazine hydrate. The product was used without further purification.

### Intermediate 490: (R)-1-(6-cyclopropyl-8-(methylsulfonyl)imidazo[1,2-a]pyridin-2-yl)ethan-1-amine

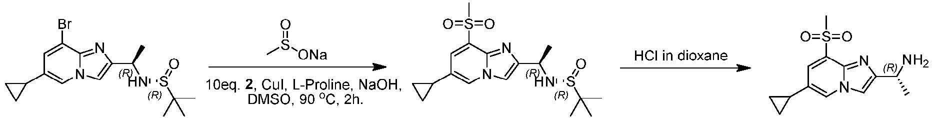

To a mixture of **intermediate 427** (200 mg, 0.52 mmol), sodium methanesulfinate (530 mg, 5.2 mmol), L-Proline (60 mg, 0.52 mmol) and NaOH (21 mg, 0.52 mmol) in DMSO (6 mL) was added CuI (99 mg, 0.52 mmol). The reaction mixture was stirred at 90°C for 2 h under N₂ and cooled to room temperature. The solution was filtered through celite and the filter cake was washed with DCM/MeOH (10/1, 30 mL). The filtrate was concentrated *in vacuo* to give the crude, which was purified by preparative TLC (eluent: PE/EtOAc = 20/1) to give **(R)-N-((R)-1-(6-cyclopropyl-8-(methylsulfonyl)imidazo[1,2-a]pyridin-2-yl)ethyl)-2-methylpropane-2-sulfinamide intermediate 491** (150 mg, 50%) as a yellow oil. ESI-MS [M +H]+: 384.2.

To a solution of **intermediate 491** (150 mg, 0.39 mmol) in dioxane (5 mL) was added HCl (4M solution in dioxane, 5 mL). The resulting mixture was stirred at room temperature for 1 h. and was concentrated *in vacuo* to give **intermediate 490** as the hydrochloric acid salt (100 mg, crude) as a yellow oil which was used in next step without purification. ESI-MS [M +H]+: 280.1.

### Intermediate 492: 1-(6-cyclopropyl-8-(næthylsulfonyl)imidazo[1,2-a]pyridin-2-yl)-N-methylmethanamine

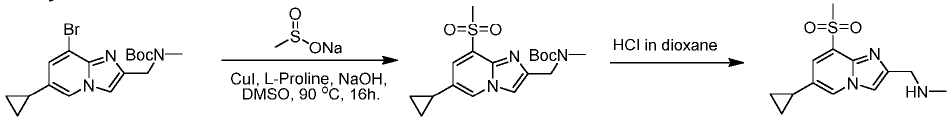

To a mixture of **intermediate 390** (200 mg, 0.53 mmol), sodium methanesulfinate (540 mg, 5.3 mmol), *L*-Proline (61 mg, 0.53 mmol) and NaOH (21 mg, 0.53 mmol) in DMSO (6 mL) was added CuI (101 mg, 0.53 mmol). The reaction mixture was stirred at 90°C for 16 h under N₂ and then cooled to room temperature. The solution was filtered through celite and the filter cake was washed with DCM/MeOH (10/1, 30 mL). The filtrate was concentrated *in vacuo* to give the crude product, which was purified by preparative TLC (eluent: PE/EtOAc = 1/1) to give **(tert-butyl ((6-cyclopropyl-8-(methylsulfonyl)imidazo[1,2-a]pyridin-2-yl)methyl)(methyl)carbamate intermediate 493** (160 mg, 80%) as a yellow solid. ESI-MS [M +H]+: 380.2.

To a solution of **intermediate 493** (160 mg, 0.42 mmol) in dioxane (5 mL) was added HCl (4M solution in dioxane, 5 mL). The resulting mixture was stirred at room temperature for 1 h and then concentrated *in vacuo* to give **intermediate 492** as the hydrochloric acid salt (110 mg, crude) as a yellow solid which was used in next step without purification. ESI-MS [M +H]+: 280.1.

### Intermediate 494: 1-(8-bromo-6-methylimidazo[1,2-a]pyridin-2-yl)ethan-1-ol

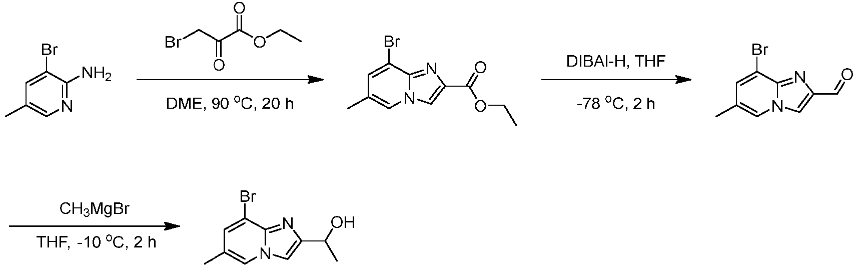

To a mixture of 3-bromo-5-methylpyridin-2-amine (5.0 g, 27 mmol) and ethyl 3-bromo-2-oxopropanoate (6.3 g, 32 mmol) in DME (60 mL) was stirred at 90°C for 20 h. The reaction mixture was cooled to room temperature and was quenched with water (50 mL), extracted with EtOAc (50 mL x 3). The combined organic layers were concentrated *in vacuo* to give the crude, which was purified by column chromatography (eluent: EtOAc/PE = 0 - 100%) to afford e**thyl 8-bromo-6-methylimidazo[1,2-a]pyridine-2-carboxylate intermediate 495** (3.5 g, 46%) as yellow solid. ESI-MS [M +H]+:283.0.

To a solution of **intermediate 495** (3.5 g, 12.4 mmol) in THF (50 mL) was added DIBAL-H (37 mL, 37 mmol, 1M in hexane) at -78°C. The reaction mixture was stirred at -78°C for 2 h. The reaction was quenched at - 65°C by MeOH (30 mL) and 15% NaOH (aq., 30 mL). The mixture was then allowed to room temperature, water (40 mL) was added and extracted with EtOAc (100 mL x 3). The combined organic layers were washed with brine (50 mL), dried over Na₂SO₄ and concentrated *in vacuo.* The residue was purified by column chromatography (eluent: EtOAc/PE = 0 - 80%) to afford 8-**bromo-6-methylimidazo[1,2-a]pyridine-2-carbaldehyde intermediate 496** (2.4 g, 81%) as yellow solid. ESI-MS [M +H]+:239.0.

To a solution of **intermediate 496** (1.2 g, 5.0 mmol) in THF (25 mL) was added CH₃MgBr (5.0 mL, 15 mmol, 3M in ether) at -10°C and the reaction mixture was stirred at this temperature for 2 h. The reaction was quenched with NH₄Cl (sat, a.q., 50 mL) at -10°C and extracted with EtOAc (30 mL x 3). The combined organic layers were washed with brine (50 mL), dried over Na₂SO₄ and concentrated *in vacuo.* The residue was purified by column chromatography (eluent: EtOAc/PE = 0 - 80%) to afford **intermediate 494** (1.0 g, 79%) as a yellow solid. ESI-MS [M +H]+:255.0.

### Intermediate 497: (R)-1-(2-(1-aminoethyl)-6-methylimidazo[1,2-a]pyridin-8-yl)-3-methylimidazolidine-2,4-dione

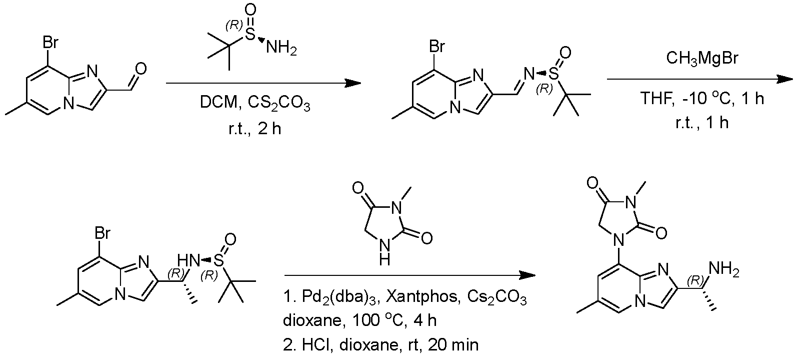

To a mixture of 8-bromo-6-methylimidazo[1,2-a]pyridine-2-carbaldehyde (12.0 g, 50 mmol) in DCM (200 mL) was added (R)-2-methylpropane-2-sulfinamide (9.1 g, 75 mmol) and Cs₂CO₃ (32.7 g, 100 mmol). The resulting mixture was stirred at room temperature for 4 h. The reaction mixture was poured into water (200 mL) and extracted with DCM (3 x 200 mL). The combined organics were washed with brine (200 mL), dried over Na₂SO₄ and concentrated *in vacuo.* The residue was purified by silica gel chromatography (eluent: EtOAc/PE = 0 ~ 50%) to give **(R,E)-N-((8-bromo-6-methylimidazo[1,2-a]pyridin-2-yl)methylene)-2-methylpropane-2-sulfinamide intermediate 498** (10 g, yield 58%) as a yellow oil. ESI-MS [M +H] +: 342.0

To a mixture of **intermediate 498** (400 mg, 1.17 mmol) in THF (10 mL) was added methylmagnesium bromide (1.17 mL, 3 M in THF, 3.50 mmol) at - 10°C. The resulting mixture was stirred at -10°C for 1 h and then room temperature for 1 h. The reaction mixture was quenched with saturated aq. NH₄Cl (50 mL) and extracted with EtOAc (3 x 30 mL). The combined organics were washed with brine (30 mL), dried over Na₂SO₄ and concentrated *in vacuo.* The residue was purified by silica gel chromatography (eluent: EtOAc/PE = 0 ~ 40%) to give **(R)-N-((R)-1-(8-bromo-6-methylimidazo[1,2-a]pyridin-2-yl)ethyl)-2-methylpropane-2-sulfinamide intermediate 499** (260 mg, yield 62%) as a yellow oil. ESI-MS [M +H] +: 358.0

A mixture of intermediate **499** (260 mg, 0.73 mmol), **intermediate 322** (400 mg, 3.51 mmol), Pd₂(dba)₃ (134 mg, 0.15 mmol), XantPhos (169 mg, 0.29 mmol) and Cs₂CO₃ (713 mg, 2.19 mmol) in 1,4-dioxane (6 mL) was stirred at 100°C for 4 h under N₂. The reaction mixture was cooled to room temperature, then filtered through Celite^{®} and the filter cake was washed with DCM/MeOH (10/1, 50 mL). The filtrate was concentrated *in vacuo* to give the crude, which was purified by silica gel chromatography (eluent: DCM/MeOH= 0~ 5%) to give **(R)-2-methyl-N-((R)-1-(6-methyl-8-(3-methyl-2,4-dioxoimidazolidin-1-yl)imidazo[1,2-a]pyridin-2-yl)ethyl)propane-2-sulfinamide intermediate 500** (230 mg, 81%) as a yellow solid. ESI-MS [M +H] +: 392. 2

A mixture of **intermediate 500** (230 mg, 0.59 mmol) in HCl/dioxane (2 mL) was stirred at room temperature for 20 min. The reaction mixture was poured into NH₃ in methanol (10 mL) and concentrated *in vacuo* to give the crude, which was purified by silica gel chromatography (eluent: DCM/MeOH= 0~ 10%) to get **intermediate 497** (150 mg, yield 88%) as a white solid. ESI-MS [M +H] +: 288. 1

### Intermediate 501: 3-(2-((R)-1-aminoethyl)-6-methylimidazo[1,2-a]pyridin-8-yl)-3-azabicyclo [3.1.0]hexan-2-one

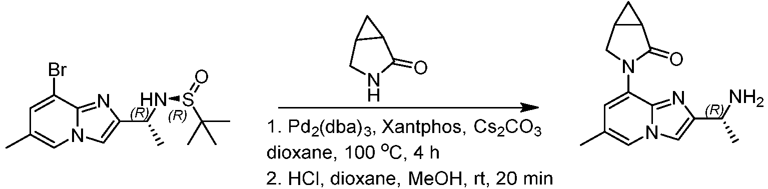

Using a similar procedure to that for **intermediate 497, interemediate 501** hydrochloric acid salt (140 mg) was synthesised from **intermediate 499** (250 mg, 0.7 mmol) and 3-azabicyclo[3.1.0]hexan-2-one (135.8 mg, 1.4 mmol). ESI-MS [M +H]+: 271.2

### Intermediate 502: (R)-1-(2-(1-aminoethyl)-6-ethylimidazo[1,2-a[pyridin-8-yl)-3-methylimidazolidine-2,4-dione

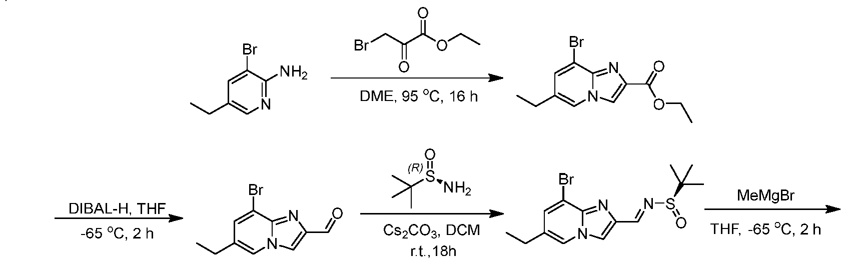

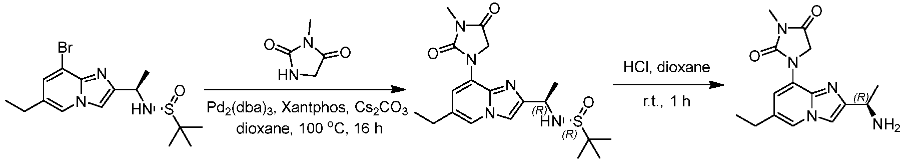

Using a similar procedure to that for **intermediate 497, intermediate 502** (0.50 g (0.50 g) was synthesised from 3-bromo-5-ethylpyridin-2-amine. ESI-MS [M +H]+: 302.1

### Intermediate 503: 1-(2-(aminomethyl)-6-ethylimidazo[1,2-a]pyridin-8-yl)-3-methylimidazolidine-2,4-dione

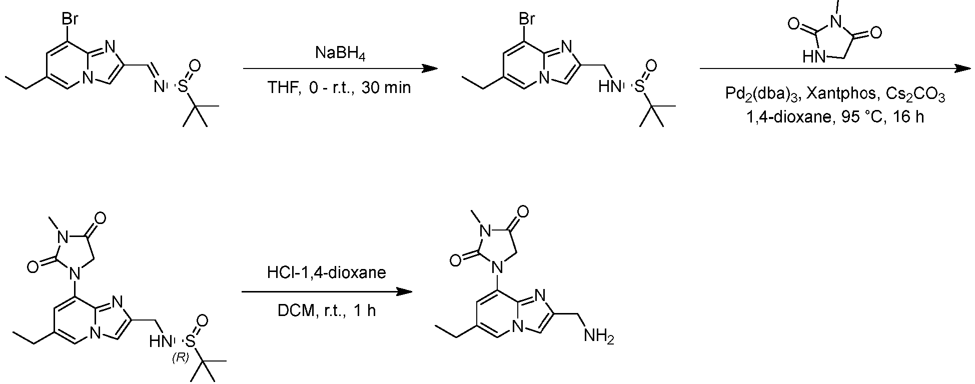

NaBH₄ (88 mg, 2.3 mmol) was added to a solution of (R,E)-N-((8-bromo-6-ethylimidazo[1,2-a]pyridin-2-yl)methylene)-2-methylpropane-2-sulfinamide (200 mg, 0.56 mmol) in THF (8 mL) at 0°C and the mixture was stirred at room temperature for 0.5 h. The reaction was quenched with NaHCO₃ (sat. aq., 50 mL) and extracted with EtOAc (50 mL x 3). The combined organic layers were washed with brine (50 mL), dried over Na₂SO₄ and concentrated *in vacuo.* The residue was purified by column chromatography (eluting: EtOAc/PE = 0 - 100%) to afford (R)-N-((8-bromo-6-**ethylimidazo[1,2-a]pyridin-2-yl)methyl)-2-methylpropane-2-sulfinamide intermediate 504** (200 mg, yield 99%) as pale yellow solid. ESI-MS [M +H]+: 358.1

To a mixture of **intermediate 504** (380 mg, 1.1 mmol) and **intermediate 322** (480 mg, 4.2 mmol) in 1,4-dioxane (20 mL) was added Pd₂(dba)₃ (150 mg, 0.16 mmol), Xantphos (180 mg, 0.32 mmol) and Cs₂CO₃ (1.1 mg, 3.2 mmol). The mixture was stirred at 95°C for 16 h under N₂. The reaction mixture was filtered through Celite^{®} and the filter cake was washed with DCM/MeOH (10/1, 100 mL). The filtrate was concentrated *in vacuo* to give the crude, which was purified by column chromatography (eluting: MeOH/DCM = 0 - 4%) to afford **(*R*)-N-((6-ethyl-8-(3-methyl-2,4-dioxoimidazolidin-1-yl)imidazo[1,2-a]pyridin-2-yl)methyl)-2-methylpropane-2-sulfinamide intermediate 505** (270 mg, yield 64%) as pale yellow solid. ESI-MS [M +H]+: 392.1

To a mixture of **intermediate 505** (270 mg, 0.69 mmol) in DCM (5 mL) was added HCl (4M in dioxane, 1.7 mL, 6.8 mmol). The mixture was stirred at room temperature for 1h. The mixture was concentrated *in vacuo.* The residue was treated with NH₃ (7M in MeOH, 5 mL) and the resulting solution was stirred at room temperature for 5 min then concentrated *in vacuo.* The residue was purified by column chromatography (eluting: MeOH/DCM = 0 - 20%) to afford **intermediate** 503 (180 mg, yield 91%) as white solid.
ESI-MS [M +H]+: 288.1

### Intermediate 506 1-(2-(1-aminoethyl)-6-chloroimidazo[1,2-a]pyridin-8-yl)-3-methylimidazolidine-2,4-dione

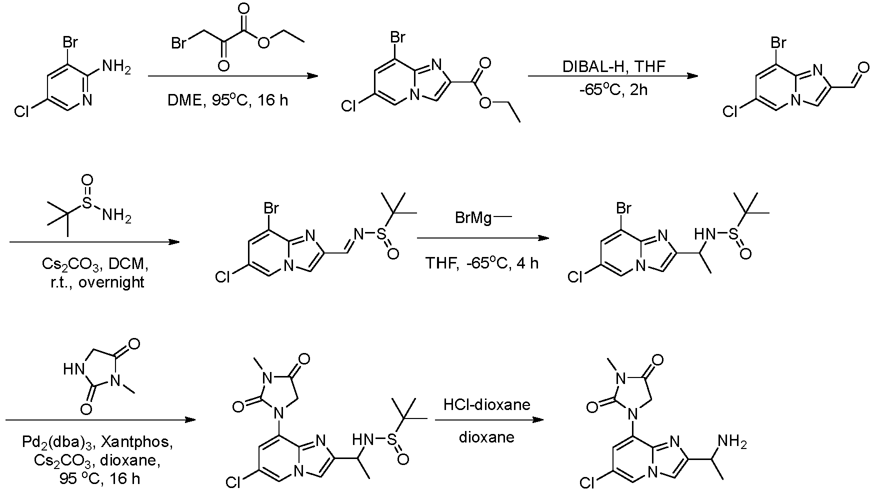

Using a similar procedure to that for **intermediate 497, intermediate 506** (0.71 mg) was synthesised from **3-bromo-5-chloropyridin-2-amine.** ESI-MS [M +H]⁺: 308.1

### Intermediate 507: 1-(2-(aminonæthyl)-6-chloroimidazo[1,2-a]pyridin-8-yl)-3-mthylimidazolidine-2,4-dione

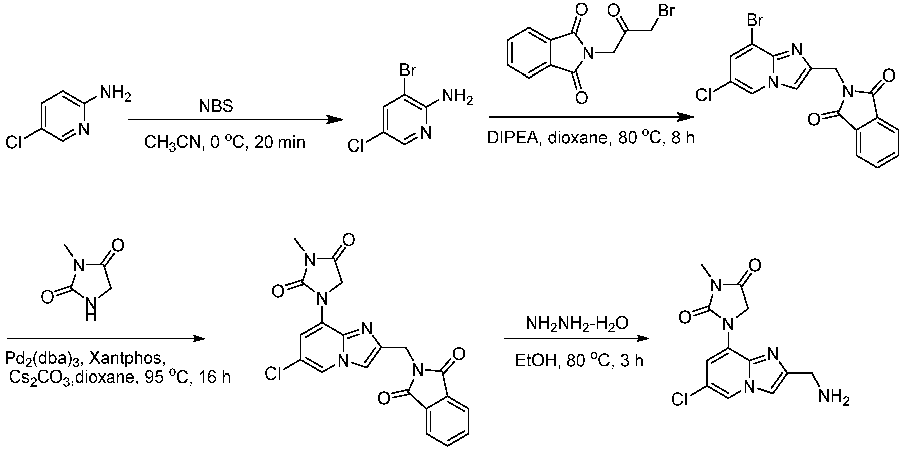

To a mixture of 5-chloropyridin-2-amine (3.0 g, 23.44 mmol) in CH₃CN (30 mL) was added a solution of NBS (4.2 g, 23.44 mmol) in CH₃CN (20 mL) at 0°C and stirred for 20 min. The reaction was quenched with water (50 mL) and extracted with EtOAc (3 x 50 mL). The combined organic layers were washed with brine (50 mL), dried over anhydrous Na₂SO₄ and concentrated *in vacuo* to give the crude product, which was purified by silica gel chromatography (eluting: EtOAc/PE = 1/10) to give 3-**bromo-5-chloropyridin-2-amine intermediate 508** (3.5 g, 72 %) as a brown solid. ESI-MS [M +H]⁺: 207.2.

To a solution of **intermediate 508** (1.0 g, 4.85 mmol) in dioxane (50 mL) was added **intermediate 311** (2.7 g, 9.71 mmol) and DIPEA (3.1 g, 24.30 mol). The reaction mixture was stirred at 80°C for 8h. After cooling to room temperature, the reaction mixture was diluted with EtOAc (100 mL) and washed with brine (50 mL). The organic layer was dried and concentrated *in vacuo* to give the crude product, which was purified by silica gel chromatography (eluent: PE/EtOAc = 10/1) to give 2-**((8-bromo-6-chloroimidazo[1,2-a]pyridin-2-yl)methyl)isoindoline-1,3-dione intermediate 509** (300 mg, 16%) as a yellow solid. ESI-MS [M +H]⁺: 390.1.

To a mixture of **intermediate 509** (200 mg, 0.51 mmol), **intermediate 322** (116 mg, 1.02 mmol) and Cs₂CO₃ (499 mg,1.53 mmol) in dioxane (20 mL) was added Pd₂(dba)₃ (46 mg, 0.05 mmol) and Xantphos (58 mg, 0.10 mmol). The reaction mixture was stirred at 95°C for 16h, cooled to room temperature, filtered through celite and the filter cake was washed with DCM/MeOH (10/1, 50 mL). The filtrate was concentrated *in vacuo* to give the crude product, which was purified by silica gel chromatography (eluting: PE/ EtOAc = 1/2) to give **2-((6-chloro-8-(3-methyl-2,4-dioxoimidazolidin-1-yl)imidazo[1,2-a]pyridin-2-yl)methyl)isoindoline-1,3-dione intermediate 510** (150 mg, 69 %) as a brown oil. ESI-MS [M +H]+: 424.0.

To a solution of **intermediate 510** (150 mg, 0.35 mmol) in EtOH (15 mL) was added NH₂NH₂-H₂O (0.2 mL). The reaction mixture was stirred at 80°C for 3h and then cooled to room temperature. The resulting solution was filtered and filtrate was concentrated *in vacuo* to give the crude, which was purified by preparative TLC (eluting: DCM/MeOH=10/1) to give **intermediate 507** (60 mg, 58%) as a white solid. ESI-MS [M +H]+:294.1.

### Intermediate 511: (6-chloro-8-(4-methylpiperazin-1-yl)imidazo[1,2-a]pyridin-2-yl)methanamine

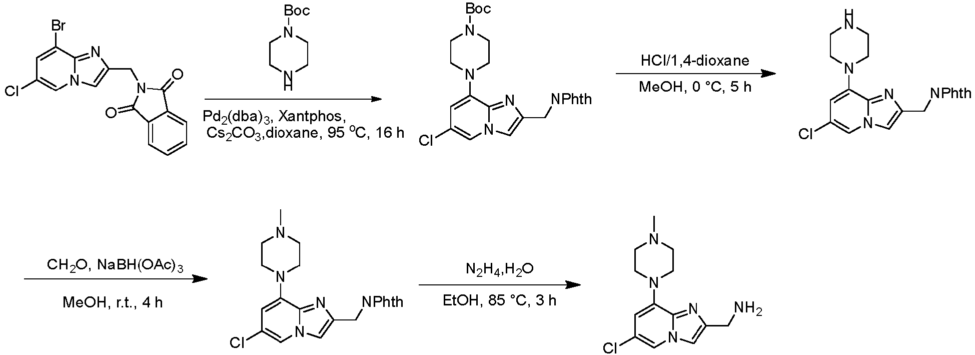

To a solution of **intermediate 509** (200 mg, 0.51 mmol), tert-butyl piperazine-1-carboxylate (190 mg, 1.02 mmol) and Cs₂CO₃ (499 mg,1.53 mmol) in dioxane (20 mL) were added Pd₂(dba)₃ (46 mg, 0.05 mmol) and Xantphos (58 mg, 0.10 mmol). The reaction mixture was stirred at 95°C for 16h, cooled to room temperature, filtered through celite and the filter cake was washed with DCM/MeOH (10/1, 50 mL). The filtrate was concentrated *in vacuo* to give the crude product, which was purified by column chromatography (eluting: PE/ EtOAc = 1/2) to give **tert-butyl 4-(6-chloro-2-((1,3-dioxoisoindolin-2-yl)methyl)imidazo[1,2-a]pyridin-8-yl)piperazine-1-carboxylate intermediate 512** (151 mg, 60 %) as a brown oil. ESI-MS [M +H]+: 496.0

To a solution of **intermediate 512** (0.40 g, 0.81 mmol) in MeOH (10 mL) was added HCl ( 1.0 mL, 4.0 mmol, 4M in 1,4-dioxane). The mixture was stirred at 0°C for 5 h and evaporated to give **2-((6-chloro-8-(piperazin-1-yl)imidazo[1,2-a]pyridin-2-yl)methyl)isoindoline-1,3-dione intermediate 513** (0.30 g, quant) as a light yellow oil, which was used in next step without purification. ESI-MS [M +H]+: 396.2

A solution of **intermediate 513** (0.3 g, crude), formaldehyde (0.19 g, 6.2 mmol, 37wt.% in H₂O) and NaBH(OAc)₃ (0.92 g, 4.3 mmol) in MeOH (10 mL) was stirred at room temperature for 4 h. Water (20 mL) was added and extracted with EtOAc (3 x 20 mL). The combined organic layers were washed with brine (50 mL), dried over Na₂SO₄, concentrated *in vacuo* to give the crude, which was purified by column chromatography on silica gel (eluent: DCM/MeOH = 20/1) to give **2-((6-chloro-8-(4-methylpiperazin-1-yl)imidazo[1,2-a]pyridin-2-yl)methyl)isoindoline-1,3-dione intermediate 514** (0.15 g, 45%) as a yellow oil. ESI-MS [M +H]+: 410.2

To a solution of **intermediate 514** (0.15 g, 0.37 mmol)) in EtOH (10 mL) was added N₂H₄•H₂O (92 mg, 1.9 mmol, 80% purity). The mixture was stirred at 85°C for 3 h and cooled to room temperature. The mixture was filtered, and the filtrate was concentrated *in vacuo* to give **intermediate 511** (0.12 g, crude) as a yellow oil, which was used in next step without purification. ESI-MS [M +H]+: 280.2.

### Intermediate 515: (R)-1-(2-(1-aminoethyl)-6-methoxyimidazo[1,2-a]pyridin-8-yl)-3-methylimidazolidine-2,4-dione

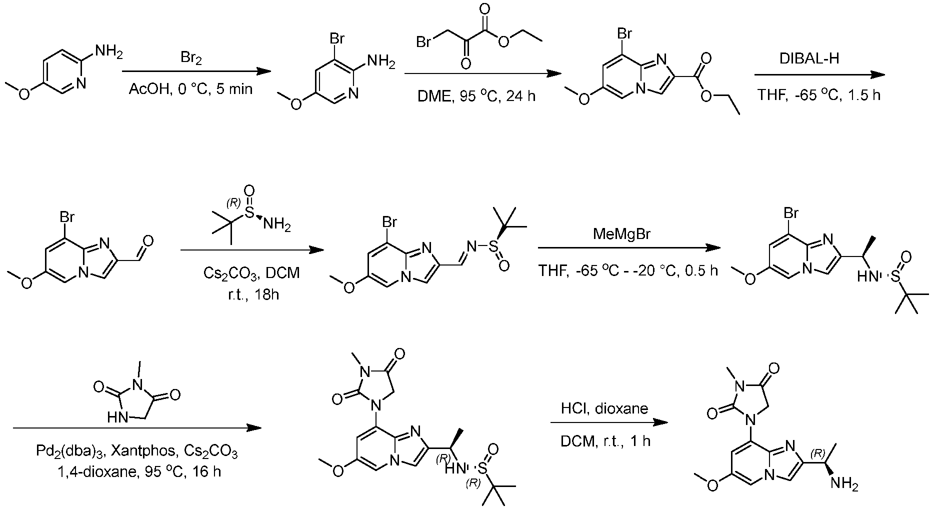

To a solution of 5-methoxypyridin-2-amine (5 g, 40 mmol) in AcOH (35 mL) at 0°C was added Br₂ (2.2 mL, 40 mmol). The mixture was stirred at 0°C for 5 min and quenched with water (20 mL). The mixture was neutralized with saturated aq. NaHCO₃ (100 mL) and extracted with DCM (3 x 40 mL). The combined organic layers were washed with brine (200 mL), dried over Na₂SO₄ and concentrated *in vacuo.* The residue was purified by silica gel column chromatography (eluent: EtOAc/PE = 0 ~ 20%) to afford **3-bromo-5-methoxypyridin-2-amine intermediate 516** (2.2 g, yield 27%) as brown solid. ESI-MS [M +H]+: 203.1

To a mixture of **intermediate 516** (2.4 g, 12 mmol) in DME (50 mL) was added ethyl 3-bromo-2-oxopropanoate (3.5 g, 18 mmol). The mixture was stirred at 95°C for 24 h under N₂ and cooled to room temperature. The mixture was quenched with saturated aq. NaHCO₃ (50 mL) and extracted with DCM (3 x 50 mL). The combined organic layers were washed with brine (50 mL), dried over Na₂SO₄ and concentrated *in vacuo.* The residue was purified by flash column chromatography (eluent: EtOAc/PE = 0 ~ 40%) to afford ethyl **8-bromo-6-methoxyimidazo[1,2-a]pyridine-2-carboxylate intermediate 517** (2.1 g, yield 59%) as brown solid. ESI-MS [M +H]+: 299.1

To a mixture of ethyl **intermediate 517** (2.1 g, 7.0 mmol) in THF (40 mL) was added dropwise DIBAL-H (1M in hexane, 22 mL, 22 mmol) at - 65°C. After stirring at -65°C for 1.5 h, the mixture was quenched with NaOH (15%, aq. 40 mL) and extracted with EtOAc (3 x 40 mL). The combined organic layers were washed with brine (50 mL), dried over Na₂SO₄ and concentrated *in vacuo.* The residue was purified by silica gel column chromatography (eluent: EtOAc/PE = 0 ~ 50%) to afford **8-bromo-6-methoxyimidazo[1,2-a]pyridine-2-carbaldehyde intermediate 518** (1.1 g, yield 62%) as white solid. ESI-MS [M +H]+: 255.1

To a mixture of **intermediate 518** (1.1 g, 4.3 mmol) and (R)-2-methylpropane-2-sulfinamide (0.63 g, 5.2 mmol) in DCM (25 mL) was added Cs₂CO₃ (2.8 g, 8.6 mmol). After stirring at room temperature for 18h, the mixture was quenched with water (50 mL) and extracted with EtOAc (3 x 40 mL). The combined organic layers were washed with brine (50 mL), dried over Na₂SO₄ and concentrated *in vacuo.* The residue was purified by silica gel column chromatography (eluent: EtOAc/PE = 0 ~ 50%) to afford **(R,E)-N-((8-bromo-6-methoxyimidazo[1,2-a]pyridin-2-yl)methylene)-2-methylpropane-2-sulfinamide intermediate 519** (0.56 g, yield 36%) as pale-yellow solid. ESI-MS [M +H]+: 358.1

To a mixture of **intermediate 519** (0.56 g, 1.7 mmol) in THF (20 mL) was added methylmagnesium bromide (1.7 mL, 5.1 mmol, 3M in ether) at - 65°C. After warming to - 20°C in 30 min, the mixture was quenched with saturated aq. NH₄Cl (30 mL) and extracted with EtOAc (3 x 30 mL). The combined organic layers were washed with brine (50 mL), dried over Na₂SO₄ and concentrated *in vacuo.* The residue was purified by silica gel column chromatography (eluent: MeOH/DCM = 0 ~ 5%) to afford **(R)-N-((R)-1-(8-bromo-6-methoxyimidazo[1,2-a]pyridin-2-yl)ethyl)-2-methylpropane-2-sulfinamide intermediate 520** (0.54 g, yield 85%) as pale-yellow solid. ESI-MS [M +H]+: 374.1

A mixture of **intermediate 520** (0.54 g, 1.5 mmol), **intermediate 322** (0.83 g, 7.3 mmol), Pd₂(dba)₃ (0.20 g, 0.22 mmol), Xantphos (0.25 g, 0.44 mmol) and Cs₂CO₃ (1.2 mg, 3.6 mmol) in 1,4-dioxane (20 mL) was stirred at 95°C for 16 h under N₂. The mixture was filtered through Celite^{®} and the filter cake was washed with DCM/MeOH (10/1, 50 mL). The filtrate was concentrated *in vacuo* to give the crude, which was purified by silica gel chromatography (eluent: MeOH/DCM= 0~4%) to afford **(R)-N-((R)-1-(6-methoxy-8-(3-methyl-2,4-dioxoimidazolidin-1-yl)imidazo [1,2-a] pyridin-2-yl)ethyl)-2-methylpropane-2-sulfinamide intermediate 521** (0.55 g, yield 92 %) as pale-yellow solid. ESI-MS [M +H]+: 408.2

To a mixture of **intermediate 521** (0.55 g, 1.4 mmol) in DCM (5.0 mL) was added HCl (3.3 mL, 14 mmol, 4M in 1,4-dioxane,). The mixture was stirred at room temperature for 1 h and concentrated *in vacuo.* NH₃/MeOH (10 mL, 7M in MeOH) was added to the resulting residue and the mixture stirred for 15 min. The mixture was concentrated *in vacuo* and the residue was purified by silica gel chromatography (eluent: MeOH/DCM= 0~18%) to afford **intermediate 515** (0.28 g, yield 69%) as white solid. ESI-MS [M +H]+: 304.1

### Intermediate 522: (S*)-1-(2-(1-aminoethyl)-6-(difluoromethyl)imidazo[1,2-a]pyridin-8-yl)-3-methylimidazolidine-2,4-dione

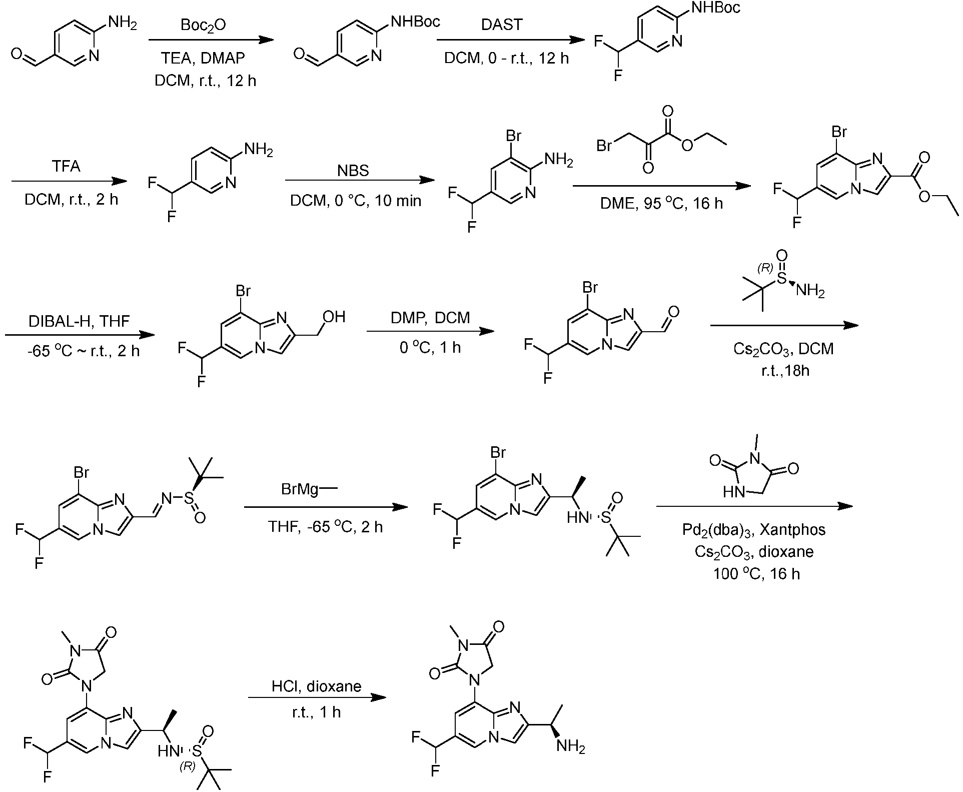

To a mixture of 6-aminonicotinaldehyde (5.0 g, 41 mmol) in DCM (80 mL) were added Boc₂O (9.4 g, 43 mmol), DMAP (0.25 g, 2.0 mmol) at room temperature and the mixture was stirred for 12 h. The mixture was quenched with water (100 mL) and extracted with DCM (3 x 100 mL). The combined organic layers were washed with brine (100 mL), dried over Na₂SO₄ and concentrated *in vacuo.* The residue was purified by silica gel column chromatography (eluent: EtOAc/PE from 0 to 20%) to afford **tert-butyl (5-formylpyridin-2-yl)carbamate intermediate 523** (7.2 g, 79%) as a white solid. ESI-MS [M +H]+: 223.1

To a mixture of **intermediate 523** (7.2 g, 32 mmol) in DCM (100 mL) was added DAST (17 mL, 96 mmol) at 0°C. The mixture was stirred at room temperature for 12 h. The reaction was quenched with saturated aq. NaHCO₃ (100 mL) then extracted with DCM (3 x 100mL). The combined organic layers were washed with brine (100 mL), dried over Na₂SO₄ and concentrated *in vacuo.* The residue was purified by silica gel column chromatography (eluent: EtOAc/PE from 0 to 20%) to afford **tert-butyl (5-(difluoromethyl) pyridin-2-yl) carbamate intermediate 524** (5.8 g,76%) as a white solid.ESI-MS [M +H]+: 245.1

To a mixture of **intermediate 524(**5.8 g, 24 mmol) in DCM (100 mL) was added TFA (17 mL, 240 mmol) at room temperature. The mixture was stirred at room temperature for 2 h. The reaction was quenched with NH₃/MeOH (7 M in MeOH, 20 mL) until pH>7 and then water (100 mL) was added. The mixture was extracted with DCM (3 x 100 mL). The combined organic layers were washed with brine (100 mL), dried over Na₂SO₄ and concentrated *in vacuo.* The residue was purified by silica gel column chromatography (eluent: EtOAc/PE from 0 to 30%) to give **5-(difluoromethyl)pyridin-2-amine intermediate 525** (3.5 g, 95%) as a white solid. ESI-MS [M +H]+: 145.0

NBS (4.8 g, 27 mmol) was added to a solution of **intermediate 525** (3.5 g, 24 mmol) in DCM (30 mL) at 0°C. After stirring at 0°C for 10 min, the mixture was quenched with water (100 mL), and then neutralized with saturated aq. NH₄Cl (100 mL). The aqueous phase was extracted with DCM (3 x 100 mL). The combined organic layers were washed with brine (100 mL), dried over Na₂SO₄ and concentrated *in vacuo.* The residue was purified by silica gel column chromatography (eluent: EtOAc/PE from 0 to 20%) to afford **3-bromo-5-(difluoromethyl)pyridin-2-amine intermediate 526** (3.2 g, yield 58%) as brown solid. ESI-MS [M +H]+: 223.1

To a solution of **intermediate 526** (3.2 g, 14.4mmol) in DME (50 mL) was added ethyl 3-bromo-2-oxopropanoate (4.5 g, 24 mmol). After stirring stirred at 95°C for 24 h under N₂, the reaction mixture was cooled to room temperature. The mixture was then quenched with water (100 mL) and extracted with EtOAc (3 x 100 mL). The combined organic layers were washed with brine (100 mL), dried over Na₂SO₄ and concentrated *in vacuo.* The residue was purified by silica gel column chromatography (eluent: EtOAc/PE from 0 to 40%) to afford **ethyl 8-bromo-6-(difluoromethyl)imidazo[1,2-a]pyridine-2-carboxylate intermediate 527** (2.0 g, yield 41%) as brown solid. ESI-MS [M +H]+: 319.1

DIBAL-H (19 mL, 19 mmol) was added dropwise to a solution of **intermediate 527** (2.0 g, 6.3 mmol) in THF (40 mL) at - 65°C. The reaction mixture was further stirred at -65°C for 1 h and then at room temperature for another 1 h. The mixture was quenched with NaOH (aq., 15%, 40 mL) and extracted with EtOAc (3 x 40 mL). The combined organic layers were washed with brine (100 mL), dried over Na₂SO₄ and concentrated *in vacuo.* The residue was purified by silica gel column chromatography (eluent: EtOAc/PE from 0 to 50%) to afford **(8-bromo-6-(difluoromethyl)imidazo[1, 2-a]pyridin-2-yl)methanol intermediate 528** (1.6 g, yield 92%) as white solid. ESI-MS [M +H]+: 277.1

Dess Martin periodinane (6.1 g, 15 mmol) was added to a solution of **intermediate 528**(1.6 g, 5.8 mmol) at 0°C. After stirring at 0°C for 1 h, the reaction was quenched with saturated aq. Na₂S₂O₃ (40 mL). The mixture was cooled to 0°C and basified with saturated aq. NaHCO₃ (40 mL) until pH > 7. The resulting mixture was the extracted with DCM (3 x 50 mL). The combined organic layers were washed with brine (50 mL), dried over Na₂SO₄ and concentrated *in vacuo.* The residue was purified by silica gel column chromatography (eluent: EtOAc/PE from 0 to 50%) to afford **8-bromo-6-(difluoromethyl)imidazo[1,2-a]pyridine-2-carbaldehyde intermediate 529** (1.4 g, yield 92%) as white solid. ESI-MS [M +H]+: 275.1

To a mixture of **intermediate 529** (1.4 g, 5.0 mmol) and (R)-2-methylpropane-2-sulfinamide (0.72 g, 6.0 mmol) in DCM (30 mL) was added Cs₂CO₃ (3.2 g, 10 mmol). After stirring at room temperature for 18h, the mixture was filtered through Celite^{®} and the filter cake was washed with DCM/MeOH (10/1, 50 mL). The filtrate was concentrated *in vacuo* to give the crude, which was purified by silica gel column chromatography (eluent: EtOAc/PE from 0 to 50%) to afford (R,E)-N-((8-**bromo-6-(difluoromethyl)imidazo[1,2-a]pyridin-2-yl)methylene)-2-methylpropane-2-sulfinamide intermediate 530** (1.4 g, yield 74%) as pale-yellow solid. ESI-MS [M +H]+: 378.1

### Synthesis of (R)-N-((R)-1-(8-bromo-6-(difluoromethyl)imidazo[1,2-a]pyridin-2-yl)ethyl)-2-methylpropane-2-sulfinamide and (S)-N-((R)-1-(8-bromo-6-(difluoromethyl)imidazo[1,2-a[pyridin-2-yl)ethyl)-2-methylpropane-2-sulfinamide

Methylmagnesium bromide ((2.5 mL, 7.4 mmol, 3 M in ether) was added dropwise to a mixture of **intermediate 530** (1.4 g, 3.7 mmol) in THF (40 mL) at - 65°C. After warming to - 20°C for 30 min, the mixture was quenched with saturated aq. NH₄Cl (50 mL) and extracted with EtOAc (3 x 50 mL). The combined organic layers were washed with brine (100 mL), dried over Na₂SO₄ and concentrated *in vacuo.* The residue was purified by silica gel chromatography (eluent: MeOH/DCM from 0 to 8%) to afford isomer 1 (0.5 g, yield 34%) and isomer 2 (0.5g, yield 34%) as pale yellow solids (stereochemistry not assigned). ESI-MS [M +H]+: 394.1 The following 2 steps were carried out on isomer 1 and isomer 2 of **1-(8-bromo-6-(difluoromethyl)imidazo[1,2-a]pyridin-2-yl)ethyl)-2-methylpropane-2-sulfinamide** - isomer 1 is shown in full, the yield for isomer 2 (after step 2) is given also.

A mixture of (S*)-N-((R)-1-(8-bromo-6-(difluoromethyl)imidazo[1,2-a]pyridin-2-yl)ethyl)-2-methylpropane-2-sulfinamide (0.20 g, 0.5 mmol, isomer 1), **intermediate 322** (0.23 g, 2.0 mmol), Pd₂(dba)₃ (0.069 g, 0.075 mmol), Xantphos (0.087 g, 0.15 mmol) and Cs₂CO₃ (0.49 g, 1.5 mmol) in 1,4-dioxane (10 mL) was stirred at 95°C for 16 h under N₂. The mixture was cooled to room temperature and filtered through Celite^{®}. The filter cake was washed with DCM/MeOH (10/1, 50 mL) and the filtrate was concentrated *in vacuo* to give the crude, which was purified by silica gel chromatography (eluent: DCM/MeOH=0 ~ 5%) to afford **(S*)-N-((R)-1-(6-(difluoromethyl)-8-(3-methyl-2,4-dioxoimidazolidin-1-yl)imidazo[1,2-a]pyridin-2-yl)ethyl)-2-methylpropane-2-sulfinamide,** isomer 1 (40 mg, yield 18 %) as pale-yellow solid. ESI-MS [M +H]+: 428.1

To a mixture of (S*)-N-((R)-1-(6-(difluoromethyl)-8-(3-methyl-2,4-dioxoimidazolidin-1-yl)imidazo[1,2-a]pyridin-2-yl)ethyl)-2-methylpropane-2-sulfinamide (40 mg, 0.09 mmol) in DCM (5.0 mL) was added HCl (3.3 mL, 14 mmol, 4M in 1,4-dioxane). The mixture was stirred at room temperature for 1 h and concentrated *in vacuo.* NH₃/MeOH (7M in MeOH, 10 mL) was added to the resulting residue and the mixture was stirred for 15 min. The solution was concentrated *in vacuo* and the residue was purified by preparative TLC to afford **intermediate 522** (30 mg, yield 91%) as white solid. ESI-MS [M +H]+: 324.1.1
Isomer 2 of **1-(8-bromo-6-(difluoromethyl)imidazo[1,2-a]pyridin-2-yl)ethyl)-2-methylpropane-2-sulfinamide** was also carried through these steps to give the desired product in 30% yield.

### Intermediate 531: 4-(2-(aminomethyl)-6-cyclopropylimidazo[1,2-a]pyridin-8-yl)-6-methyl-4,6-diazaspiro[2.4]heptane-5,7-dione

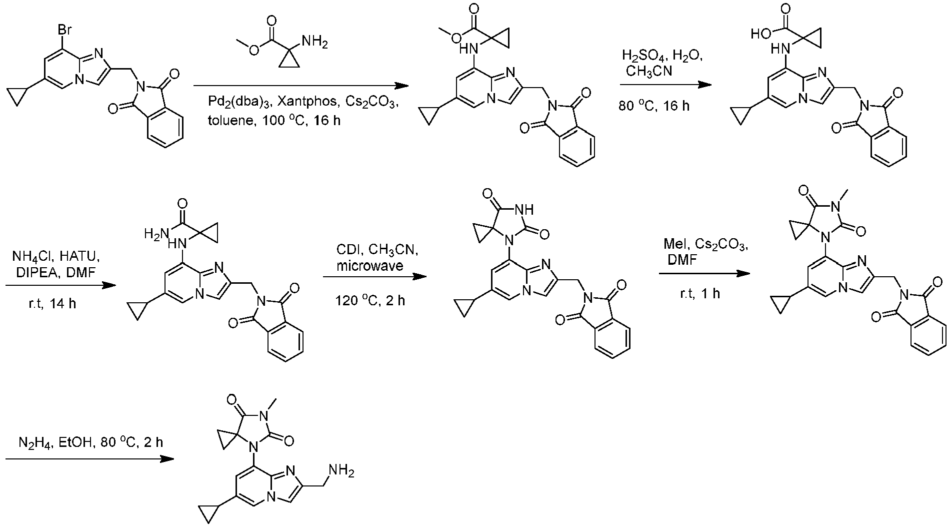

To a mixture of **intermediate 310** (2 g, 5.05 mmol), methyl 1-aminocyclopropane-1-carboxylate (2.91 g, 25.3 mmol) and Cs₂CO₃ (4.94 g, 15.2 mmol) in toluene (40 mL) was added Pd₂(dba)₃ (460 mg, 0.505 mmol) and Xantphos (580 mg, 1.01 mmol). The mixture was stirred at 100°C for 16 h under N₂. The reaction mixture was cooled to room temperature, filtered through celite and the filter cake was washed with DCM/MeOH (10/1, 100 mL). The filtrate was concentrated *in vacuo* to give the crude product, which was purified by silica gel chromatography (eluent: EtOAc/PE = 1/2) to give **methyl 1-((6-cyclopropyl-2-((1,3-dioxoisoindolin-2-yl)methyl)imidazo[1,2-a]pyridin-8-yl)amino)cyclopropane-1-carboxylate intermediate 532** (730 mg, 34%) as a yellow solid. ESI-MS [M + H]⁺: 431.1.

A mixture of **intermediate 532** (730 mg, 1.70 mmol) and H₂SO₄ (3 mL, 30%) in CH₃CN (6 mL) was stirred at 80°C for 16 h. The reaction mixture was cooled to room temperature and poured into water (50 mL). The pH was adjusted to 4~5 by NaHCO₃ aqueous (40 mL) and extracted with EtOAc (3 x 50 ml). The combined organics were washed with brine (100 mL x 1), dried over Na₂SO₄, concentrated and dried *in vacuo* to give **1-((6-cyclopropyl-2-((1,3-dioxoisoindolin-2-yl)methyl)imidazo[1,2-a]pyridin-8-yl)amino)cyclopropane-1-carboxylic acid intermediate 533** (390 mg, 55%) as a yellow syrup. ESI-MS [M + H]⁺: 417.2.

To a stirred solution of **intermediate 533** (370 mg, 0.888 mmol) and NH₄Cl (475 mg, 8.88 mmol) in DMF (10 mL) was added HATU (439 mg, 1.15 mmol) and DIPEA (344 mg, 2.66 mmol). The resulting mixture was stirred at room temperature for 14 h. The reaction mixture was poured into water (100 mL) and extracted with EtOAc (3 x 50 ml). The combined organics were washed with brine (3 x 30 mL), dried over Na₂SO₄ then concentrated *in vacuo* to give the crude product, which was purified by silica gel chromatography (eluent: DCM/MeOH = 30/1) to give **1-((6-cyclopropyl-2-((1,3-dioxoisoindolin-2-yl)methyl)imidazo[1,2-a]pyridin-8-yl)amino)cyclopropane-1-carboxamide intermediate 534** (330 mg, 89%) as a pale solid. ESI-MS [M + H]⁺: 416.1.

A mixture of **intermediate 534** (330 mg, 0.794 mmol), CDI (644 mg, 3.97 mmol) and Et₃N (402 mg, 3.97 mmol) in CH₃CN (8 mL) was stirred in a sealed tube. After degassing with N₂ for 1 min, the reaction was irradiated in microwave at 120°C for 2 h. After the reaction mixture was cooled to room temperature, water (40 mL) was added then the mixture was extracted with EtOAc (40 ml x 3). The combined organics were washed with brine (40 mL), dried over Na₂SO₄ then concentrated *in vacuo* to give the crude product, which was purified by silica gel chromatography (eluent: DCM/MeOH = 30/1) to give **2-((6-cyclopropyl-8-(5,7-dioxo-4,6-diazaspiro[2.4]heptan-4-yl)imidazo[1,2-a]pyridin-2-yl)methyl)isoindoline-1,3-dione intermediate 535** (350 mg, 100%) as a yellow solid. ESI-MS [M + H]⁺: 442.1.

To a stirred solution of **intermediate 535** (350 mg, 0.793 mmol) and Cs₂CO₃ (517 mg, 1.586 mmol) in DMF (10 mL) was added MeI (225 mg, 1.586 mmol). The mixture was stirred at room temperature for 1 h. The reaction mixture was poured into water (50 mL) and extracted with EtOAc (3 x 50 ml). The combined organics were washed with brine (50 mL), dried over Na₂SO₄ then concentrated *in vacuo* to give the crude, which was purified by silica gel chromatography (eluent: EtOAc/PE = 1/2) to give **2-((6-cyclopropyl-8-(6-methyl-5,7-dioxo-4,6-diazaspiro[2.4]heptan-4-yl)imidazo[1,2-a]pyridin-2-yl)methyl)isoindoline-1,3-dione intermediate 536** (250 mg, 69%) as a pale solid. ESI-MS [M + H]⁺: 456.1.

### Synthesis of 4-(2-(aminomethyl)-6-cyclopropylimidazo[1,2-a]pyridin-8yl)-6-methyl-4,6-diazaspiro[2.4]heptane-5,7-dione

To a stirred solution of **intermediate 536** (250 mg, 0.549 mmol) in EtOH (5 mL) was added N₂H₄ (2 mL, 80% in water). The mixture was stirred at 80°C for 2 h, cooled to room temperature, filtered and the filtrate was concentrated to give the crude product, which was purified by preparative TLC (DCM/MeOH/NH₃ = 10/1/1) to give 4-(2-(aminomethyl)-6-cyclopropylimidazo[1,2-a]pyridin-8-yl)-6-methyl-4,6-diazaspiro[2.4]heptane-5,7-dione (110 mg, 61%) as a pale solid. ESI-MS [M + H]⁺: 326.1.

### Intermediate 537: 7-(2-(aminomethyl)-6-cyclopropylimidazo[1,2-a]pyridin-8-yl)-2,5-dioxa-7-azaspiro[3.4]octan-6-one

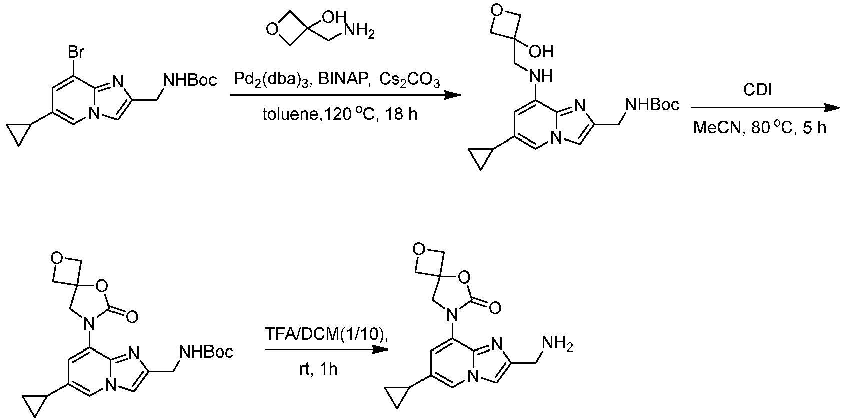

A mixture of **intermediate 360** (730 mg, 2.0 mmol), 3-(aminomethyl)oxetan-3-ol (206 mg, 2.0 mmol), Pd₂(dba)₃ (275 mg, 0.3 mmol), BINAP (373 mg, 0.6 mmol) and Cs₂CO₃ (1.3 g, 4.0 mmol) in toluene (20 mL) was stirred at 100°C for 18h under N₂. The reaction was cooled to room temperature, quenched with water (30 mL) and extracted with EtOAc (3 x 30 mL). The combined organic layers were washed with brine (20 mL), dried over anhydrous Na₂SO₄ and concentrated *in vacuo* to give the crude product, which was purified by silica gel chromatography (eluent: DCM/MeOH=50/1 ~ 20/1) to afford **tert-butyl ((6-cyclopropyl-8-(((3-hydroxyoxetan-3-yl)methyl)amino)imidazo[1,2-a]pyridin-2-yl)methyl)carbamate intermediate 538** (220 mg, 28 %) as a yellow oil. ESI-MS [M +H]⁺: 389.2.

A mixture of **intermediate 538** (220 mg, 0.57 mmol) and CDI (367 mg, 2.27 mmol) in MeCN (20 mL) was stirred at 80°C for 5h. The reaction was cooled to room temperature, quenched with water (30 mL) and extracted with EtOAc (3 x 30 mL). The combined organic layers were washed with brine (30 mL), dried over Na₂SO₄ and concentrated *in vacuo* to give the crude product, which purified by preparative TLC (eluent: MeOH/DCM = 1/15) to afford ***tert-butyl* ((6-cyclopropyl-8-(6-oxo-2,5-dioxa-7-azaspiro[3.4]octan-7-yl)imidazo[1,2-a]pyridin-2-yl)methyl)carbamate** (180 mg, 76%) as a white solid. ESI-MS [M +H]⁺: 415.2.

A solution of tert-butyl ((6-cyclopropyl-8-(6-oxo-2,5-dioxa-7-azaspiro[3.4]octan-7-yl)imidazo[1,2-a]pyridin-2-yl)methyl)carbamate (120 mg, 0.29 mmol) in TFA / DCM (0.5 mL/5.0 mL) was stirred at room temperature for 1h. The mixture was quenched with sat. NaHCO₃ solution (30 mL) then extracted with DCM (3 x 10 mL). The combined organic layers were washed with brine (30 mL), dried over Na₂SO₄ and concentrated *in vacuo* to give the crude, which was purified by preparative TLC (eluent: MeOH/DCM = 1/8) to afford **intermediate 537** (60 mg, 66%) as a yellow oil. ESI-MS [M +H]⁺: 315.2.

### Intermediate 539: (R)-7-(2-(1-aminoethyl)-6-cyclopropylimidazo[1,2-a]pyridin-8-yl)-2,5-dimethyl-2,5,7-triazaspiro[3.4]octan-6-one

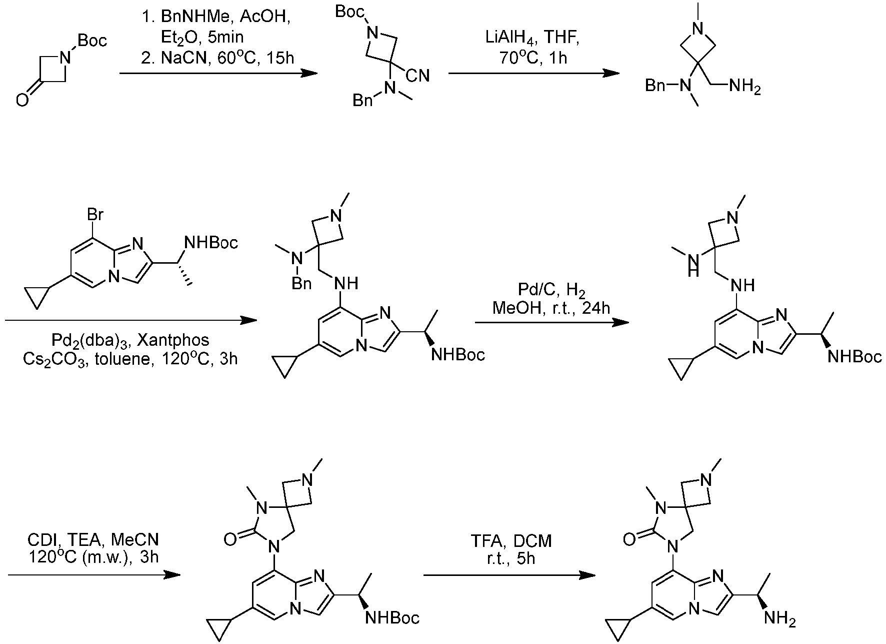

A mixture of tert-butyl 3-oxoazetidine-1-carboxylate (3 g, 17.5 mmol), N-methyl-1-phenylmethanamine (2.1 g, 17.5 mmol) and acetic acid (2.1 g, 35 mmol) in Et₂O (100 mL) was stirred at room temperature for 5 min. Then NaCN (857.5 mg, 17.5 mmol) was added and the mixture was stirred at 60°C for 15h. The reaction was quenched with saturated aq. NaHCO₃ (100 mL) and extracted with EtOAc (3 x 50 mL). The organic layers were washed with brine (50 mL), dried over Na₂SO₄, concentrated *in vacuo* to give the crude, which was purified by silica gel chromatography (eluent: PE/EtOAc = 0 - 10%) to give **tert-butyl 3-(benzyl(methyl)amino)-3-cyanoazetidine-1-carboxylate intermediate 540** (3.8 g, 73%) as a yellow solid. ESI-MS [M +H]+: 557.2

To a solution of **intermediate 540** (600 mg, 2.0 mmol) in THF (40 mL) was added LiAlH₄ (10 mL, 10 mmol, 1M in THF). After stirring at 70°C for 1h, the reaction was quenched Na₂SO₄-10H₂O at 0°C. The mixture was filtered and washed with MeOH (100 mL). The filtrate was concentrated *in vacuo* to give the crude, which was purified with preparative TLC (eluent: DMC/MeOH = 0~ 12%) to give **3-(aminomethyl)-N-benzyl-N,1-dimethylazetidin-3-amine intermediate 541** (300 mg, 69%) as a yellow solid. ESI-MS [M +H]+: 220.2

A mixture of **intermediate 541(290** mg, 1.32 mmol), **intermediate 564** (501 mg, 1.32 mmol), Pd₂(dba)₃ (120 mg, 0.13 mmol), Xantphos (117 mg, 0.2 mmol) and Cs₂CO₃ (1.1 g, 3.3 mmol) in toluene (25 mL) was stirred at 120°C for 3h. The reaction was quenched with brine (30 mL) and extracted with EtOAc (3 x 40 mL). The combined organic layers were washed with brine (40 mL), dried over Na₂SO₄ then concentrated *in vacuo* to give the crude, which was purified by silica gel chromatography (eluent: DCM/MeOH = 0 - 5%) to give **tert-butyl (R)-(1-(8-(((3-(benzyl(methyl)amino)-1-methylazetidin-3-yl)methyl)amino)-6-cyclopropylimidazo[1,2-a]pyridin-2-yl)ethyl)carbamate intermediate 542** (180 mg, 26%) as a yellow solid. ESI-MS [M +H]+: 519.2

A mixture of **intermediate 542** (180 mg, 0.35 mmol) and Pd/C (30 mg) in MeOH (20 mL) was stirred at room temperature for 24h under H₂. The reaction mixture was filtered through Celite^{®} and the filter cake was washed with MeOH (50 mL). The filtrate was concentrated *in vacuo* to give the crude, which was purified by preparative TLC (eluent: DCM/MeOH = 10/1) to give **tert-butyl (R)-(1-(6-cyclopropyl-8-(((1-methyl-3-(methylamino)azetidin-3-yl)methyl)amino)imidazo[1,2-a] pyridin-2-yl)ethyl)carbamate intermediate 543** (60 mg, 40%) as a yellow solid. ESI-MS [M +H]+: 429.2

A mixture of **intermediate 543** (60 mg, 0.14 mmol), CDI (180 mg, 1.12 mmol) and TEA (170 mg, 1.68 mmol) in MeCN (4 mL) was stirred at 120°C under microwave heating for 3h. The reaction was diluted with water (30 mL) and extracted with EtOAc (3 x 40 mL). The combined organic layers were washed with brine (40 mL), dried over Na₂SO₄ and concentrated *in vacuo* to give the crude, which was purified with preparative TLC (eluent: DCM/MeOH = 10/1) to give **tert-butyl (R)-(1-(6-cyclopropyl-8-(2,5-dimethyl-6-oxo-2,5,7-triazaspiro [3.4] octan-7-yl)imidazo [1,2-a] pyridin-2-yl)ethyl)carbamate intermediate 544** (40 mg, 63%) as a yellow solid. ESI-MS [M +H]+: 455.2

To a solution of **intermediate 544** (40 mg, 0.09 mmol) in DCM (5 mL) was added TFA (0.5 mL). The reaction was stirred at room temperature for 5h and concentrated *in vacuo* to give the crude. The residue was neutralized with NH₃ (7M solution in MeOH) and concentrated *in vacuo* to give the crude, which was purified with preparative TLC (eluent: DCM/MeOH/NH4OH = 10/1/0.1) to give **intermediate 539** (20 mg, 63%) as yellow oil. ESI-MS [M +H]+: 355.2

### Intermediate 545: 5-(2-(1-aminoethyl)-6-cyclopropylimidazo[1,2-a]pyridin-8-yl)-7-methyl-2-oxa-5,7-diazaspiro[3.4]octane-6,8-dione

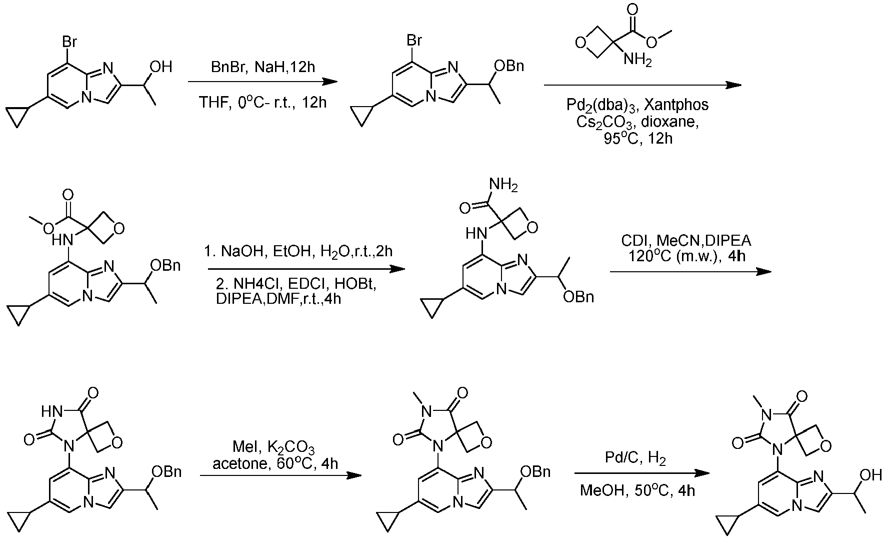

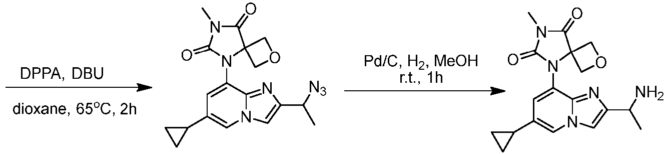

To a solution of **intermediate 458** (2.0 g, 7.1 mmol) in THF (60 mL) was slowly added NaH (430 mg, 17.9 mmol) at 0°C. After stirring 30 min, BnCl (1.8 g, 10.5 mmol) was added and the mixture was stirred at room temperature for 12 h. The mixture was quenched with saturated aq. NH₄Cl (100 mL) and extracted with EtOAc (3 x 50 mL). The combined organic layers were washed with brine (50 mL), dried over Na₂SO₄ and concentrated *in vacuo.* The residue was purified by silica gel chromatography (eluent: : EtOAc/PE from 0 to 20 %) to give **2-(1-(benzyloxy)ethyl)-8-bromo-6-cyclopropylimidazo[1,2-a]pyridine intermediate 546** (1.8 g, 68%) as a colorless oil. ESI-MS [M +H]+: 371.1.

A mixture of **intermediate 546** (1.8 g, 4.86 mmol), methyl 3-aminooxetane-3-carboxylate (764 mg, 5.83 mmol), Pd₂(dba)₃ (889 mg, 0.97 mmol), xantphos(1.12 g,1.94 mmol) and Cs₂CO₃(3.96 g, 12.1 mmol) in 1,4-dioxane(50 mL) was stirred at 95°C for 12h under N₂. The reaction mixture was filtered through Celite^{®} and the filter cake was washed with DCM/MeOH (10/1, 50 mL). The filtrate was concentrated *in vacuo* to give the crude, which was purified by silica gel chromatography (eluent: DCM/MeOH = 0 - 5%) to give **methyl 3-((2-(1-(benzyloxy)ethyl)-6-cyclopropylimidazo[1,2-a]pyridin-8-yl)amino)oxetane-3-carboxylate intermediate 547** (900 mg, 44%) as a white solid. ESI-MS [M +H]+: 422.2.

To a mixture of **intermediate 547** (900mg, 2.1 mmol) in EtOH/H₂O(12 mL/4 mL) was added LiOH-H₂O (101 mg,4.2 mmol). After stirring at room temperature for 2 h, the solution was acidified to pH=3-4 with aq. HCl (1N) and extracted with EtOAc (3 x 50 mL). The combined organic layers were washed with brine (50 mL), dried over Na₂SO₄ and concentrated *in vacuo.* The crude product was dissolved in DMF (10 mL) and NH₄Cl (597 mg,11.1 mmol), HOBt (597 mg, 4.4 mmol), EDCI (1.7 g, 8.8 mmol) and DIPEA (1.4 g,10.9 mmol) were added. After stirring at room temperature for 4h, the mixture was quenched with water (100 mL) and extracted with EtOAc (3 x 50 mL). The combined organic layers were washed with brine (50 mL), dried over Na₂SO₄ and concentrated *in vacuo* to give the crude, which was purified by silica gel chromatography (eluent: : DCM/ MeOH = 0 - 7%) to give **3-((2-(1-(benzyloxy)ethyl)-6-cyclopropylimidazo[1,2-a]pyridin-8-yl)amino)oxetane-3-carboxamide intermediate 548** (780 mg, 90%) as a yellow solid. ESI-MS [M +H]⁺: 407.2.

To a mixture of **intermediate 548** (780 mg, 1.92 mmol) in MeCN (10 mL) was added CDI (1.56 g, 9.6 mmol) and TEA (2.67 mL,19.2 mmol). After heating at 120°C for 4h in a microwave, the mixture was washed with water (50 mL,) and extracted with EtOAc (3 x 30 mL). The combined organic layers were washed with brine (30 mL), dried over Na₂SO₄ and concentrated *in vacuo.* The residue was purified by silica gel chromatography (eluent: DCM/ MeOH = 0 - 5%) to give **5-(2-(1-(benzyloxy)ethyl)-6-cyclopropylimidazo[1,2-a]pyridin-8-yl)-7-methyl-2-oxa-5,7-diazaspiro[3.4]octane-6,8-dione interemediate 549** (610 mg, 74%) as a yellow solid. ESI-MS [M +H]+: 433.2.

To a mixture of 5-(2-(1-(benzyloxy)ethyl)-6-cyclopropylimidazo[1,2-a]pyridin-8-yl)-7-methyl-2-oxa-5,7-diazaspiro[3.4]octane-6,8-**dione intermediate 549** (610 mg, 1.41 mmol) in acetone (15 mL) were added K₂CO₃ (585 mg, 4.24 mmol) and MeI (602 mg,4.24 mmol). The mixture was stirred at 60°C for 4h and cooled to room temperature. The mixture was quenched with water (30 mL,) and extracted with EtOAc (3 x 30 mL). The combined organic layers were washed with brine (30 mL), dried over Na₂SO₄ and concentrated *in vacuo.* The residue was purified by silica gel chromatography (eluent: DCM/ MeOH = 0 - 5%) to give **5-(2-(1-(benzyloxy)ethyl)-6-cyclopropylimidazo[1,2-a]pyridin-8-yl)-7-methyl-2-oxa-5,7-diazaspiro[3.4]octane-6,8-dione intermediate 550** (570 mg, 91%) as a yellow solid. ESI-MS [M +H]+: 447.2.

A mixture of **intermediate 550** (570 mg, 1.28 mmol) and Pd/C (122 mg) in MeOH (10 mL) was stirred at 50°C for 4h under H₂. The reaction mixture was filtered through Celite^{®} and the filter cake was washed with DCM/MeOH (10/1, 50 mL). The filtrate was concentrated *in vacuo* to give the crude, which was purified by silica gel chromatography (eluent: DCM/MeOH = 30/1) to give **5-(6-cyclopropyl-2-(1-hydroxyethyl)imidazo[1,2-a]pyridin-8-yl)-7-methyl-2-oxa-5,7-diazaspiro[3.4]octane-6,8-dione intermediate 551(210** mg, 42%) as a white solid. ESI-MS [M +H]+: 357.2.

To a mixture of **intermediate 551** (160 mg, 0.45 mmol) in 1,4-dioxane (10 mL) were added DPPA (1.24 g, 4.51 mmol) and DBU (684 mg,4.5 mmol). After stirring at 65 ° C for 2 h, the mixture was quenched with water (50 mL) and extracted with EtOAc (3 x 50 mL). The combined organic layers were washed with brine (50 mL), dried over Na₂SO₄ and concentrated *in vacuo.* The residue was purified by silica gel column chromatography (eluent: DCM/ MeOH = 0 ~ 30/1) to give **5-(2-(1-azidoethyl)-6-cyclopropylimidazo[1,2-a] pyridin-8-yl)-7-methyl-2-oxa-5,7-diazaspiro[3.4]octane-6,8-dione intermediate 552** (120 mg, 70%) as a grey solid. ESI-MS [M +H]+: 382.2.

To a mixture of **intermediate 552** (120 mg, 0.31 mmol) in MeOH (10 mL) was added Pd/C (24 mg). After stirring at room temperature for 1h under H₂, the reaction mixture was filtered through Celite^{®} and the filter cake was washed with DCM/MeOH (10/1, 50 mL). The filtrate was concentrated to give the crude, which was purified by silica gel chromatography (eluent: DCM/NH₃·MeOH = 20/1) to give **intermediate 545** (100 mg, 90%) as a white solid. ESI-MS [M +H]+: 356.1.

### Intermediate 553: 7-(6-cyclopropyl-2-(hydroxymethyl)imidazo[1,2-a]pyridin-8-yl)-2,5-dioxa-7-azaspiro[3.4]octan-6-one

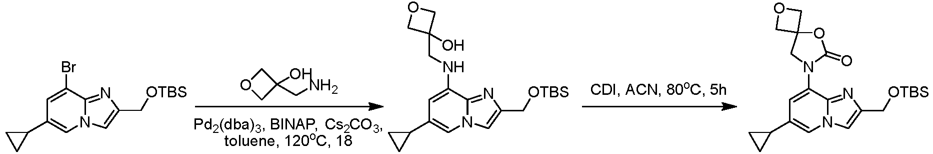

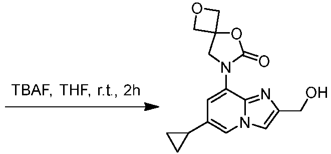

To a mixture of **intermediate 461** (500 mg, 1.31 mmol), 3-(aminomethyl)oxetan-3-ol (271 mg, 2.63 mmol), Cs₂CO₃ (1.28 g, 3.93 mmol) in toluene (50 mL) was added Pd₂(dba)₃ (240 mg, 0.262 mmol) and BINAP (326 mg, 0.524 mmol) at room temperature. Then the mixture was stirred at 120°C for 16 h under N₂ and cooled to room temperature. The reaction mixture was filtered and the filter cake was washed with DCM (100 mL). The filtrate was concentrated *in vacuo* to give the crude product which was purified by column chromatography (eluent: PE:EA= 0 to 1:1) to give **3-(((2-(((tertbutyldimethylsilyl)oxy)methyl)-6-cyclopropylimidazo[1,2-a]pyridin-8-yl)amino)methyl)oxetan-3-ol intermediate 554** (210 mg, 39.8%) as a yellow oil. ESI-MS [M +H]+: 404.2.

To a solution of **intermediate 554** (160 mg, 0.39 mmol) in ACN (20 mL) was added CDI (250 mg, 1.54 mmol) and the mixture was stirred at 80°C for 5 h. After bringing the reaction to room temperature, water (50 mL) was added and the mixture was extracted with EtOAc (3 x 50 ml). The combined organics were washed with brine (50 mL), dried over Na₂SO₄ then concentrated *in vacuo* to give the crude product, which was purified by silica gel chromatography to give **7-(2-(((tertbutyldimethylsilyl)oxy)methyl)-6-cyclopropylimidazo[1,2-a]pyridin-8-yl)-2,5-dioxa-7-azaspiro[3.4]octan-6-one intermediate 555** (76 mg, 44.7%) as a yellow oil. ESI-MS [M +H]+: 430.2.

To a solution of **intermediate 555** (76 mg, 0.18 mmol) in THF (2 mL) was added TBAF (0.36 mL, 0.36 mmol) and the mixture was stirred at room temperature for 2 h. The reaction mixture was poured into water (30 mL) and extracted with EtOAc (3 x 30 ml). The combined organics were washed with brine (20 mL), dried over Na₂SO₄ then concentrated *in vacuo* to give the crude, which was purified by silica gel chromatography (eluant: EtOAc : PE from 0 to 50%) to give **intermediate 553** (50 mg, 89%) as a white solid. ESI-MS [M +H]+: 316.1

### Intermediate 556 (R)-7-(2-(1-aminoethyl)-6-cyclopropylimidazo[1,2-a]pyridin-8-yl)-5-methyl-2-oxa-5,7-diazaspiro[3.4]octan-6-one

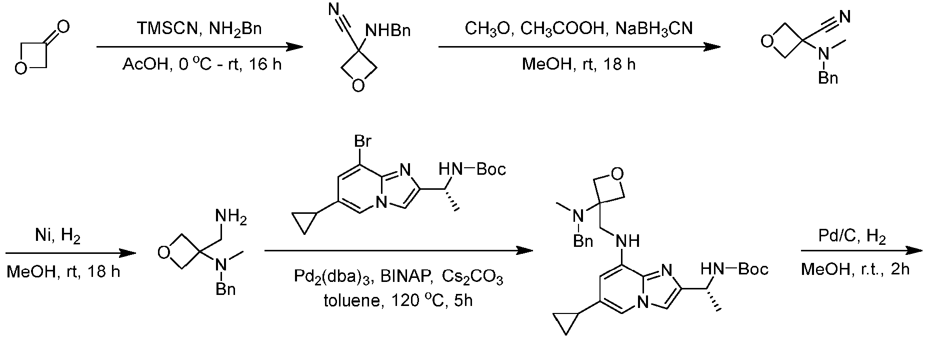

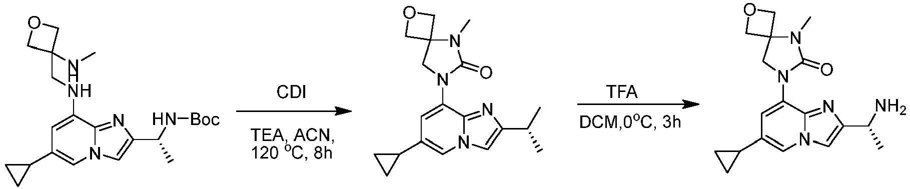

To a mixture of NH₂Bn (13 g, 0.12 mol) in AcOH (50 mL) was added oxetan-3-one (2.0 g, 28 mmol) and TMSCN (6.3 g, 64 mmol).The mixture was stirred at room temperature for 16 h. The mixture was concentrated to remove AcOH *in vacuo ,* then diluted with saturated Na₂CO₃ (sat., aq., 100 mL) and extracted by EtOAc (100 mL X 3).The combined organic layers were concentrated *in vacuo* and the residue was purified by column chromatography on silica gel, eluting with 0-20% EA/PE to afford **3-(benzylamino)oxetane-3-carbonitrile intermediate 557** (3.7 g, 70 % )as yellow oil. ESI-MS [M +H]+: 189.1.

To a mixture of **intermediate 557** (3.6 g, 19 mmol) in MeOH (60 mL) was added CH₂O (7.8 g, 0.26 mol) and CH₃COOH (0.5 mL). The mixture was stirred at room temperature for 16 h then NaBH₃CN (6.1 g, 96 mmol) was added. The mixture was stirred at room temperature for 2 h. Water (100 mL) was added and the mixture was extracted by DCM (100 mL X 3). The combined organic layers were concentrated *in vacuo* and the residue was purified by flash column chromatography, eluting with 0-20% MeOH/DCM to afford **3-(benzyl(methyl)amino)oxetane-3-carbonitrile intermediate 558** (0.50 g, 13 %) as yellow oil. ESI-MS [M +H]+:203.2.

To a mixture of **intermediate 558** (0.50 g, 2.5 mmol) in MeOH (10 mL) was added Raney-Ni and stirred at room temperature for 16 h. The mixture was filtered through Celite^{®} and the filter cake was washed with MeOH (30 mL). The filtrate was concentrated *in vacuo* and the residue was purified by column chromatography on silica gel, eluting with 0-20% MeOH/DCM to afford **3-(aminomethyl)-N-benzyl-N-methyloxetan-3-amine intermediate 559** (0.24 g, 48% )as yellow oil. ESI-MS [M +H]+:207.2.

To a mixture of **intermediate 559** (0.14 g, 0.66 mmol) , **intermediate 564** (0.15 g, 0.39 mmol), and Cs₂CO₃ (0.38 g, 1. 2 mmol) in toluene (15 mL) were added Pd₂(dba)₃ (71 mg, 0.078 mmol) and BINAP (97 mg, 0.16 mmol). The reaction mixture was stirred at 120°C for 5 h under N₂ and cooled to room temperature. The reaction mixture was filtered through Celite^{®} and the filter cake was washed with DCM/MeOH (10/1, 40 mL). The filtrate was concentrated *in vacuo* to give the crude, which was purified by preparative TLC (eluent: EA/PE=2/1) to give **tert-butyl (R)-(1-(8-(((3-(benzyl(methyl)amino)oxetan-3-yl)methyl)amino)-6-cyclopropylimidazo[1,2-a]pyridin-2-yl) ethyl)carbamate intermediate 560** (0.16 g, 82%) as a yellow oil. ESI-MS [M +H]+: 506.3

A mixture of **intermediate 560** (316 mg, 0.62 mmol) and Pd/C(316 mg) in MeOH (10 mL) was stirred at room temperature for 2 h under H₂. After stirring at 90°C for 16 h under N₂, the reaction mixture was filtered through Celite^{®} and the filter cake was washed with DCM/MeOH (10/1, 100 mL). The filtrate was concentrated *in vacuo* to give the crude, which was purified by column chromatography (eluent: DCM/MeOH=50/1 ~ 20/1) to give ***tert-*butyl (R)-(1-(6-cyclopropyl-8-(((3-(methylamino)oxetan-3-yl)methyl)amino)imidazo[1,2-a]pyridin-2-yl)ethyl)carbamate** (170 mg, 66%) as a yellow solid. ESI-MS [M +H]+: 416.2

A mixture of tert-butyl (R)-(1-(6-cyclopropyl-8-(((3-(methylamino)oxetan-3-yl)methyl)amino)imidazo[1,2-a]pyridin-2-yl)ethyl)carbamate (222 mg, 0.53 mmol), TEA (800 mg, 7.9 mmol) and CDI (700 mg, 4.3 mmol) in MeCN (6 mL) was stirred at 120°C for 8 h. The reaction was quenched with water (10 mL) and extracted with EtOAc (3 x 20 mL). The combined organic layers were washed with brine (20 mL), dried over anhydrous Na₂SO₄ and concentrated *in vacuo* to give the crude product, which was purified by column chromatography (eluent: DCM/MeOH = 20/1) to give **tert-butyl (R)-(1-(6-cyclopropyl-8-(5-methyl-6-oxo-2-oxa-5,7-diazaspiro[3.4]octan-7-yl)imidazo[1,2-a]pyridin-2-yl)ethyl)carbamate intermediate 561** (80 mg, 34%) as a white solid. ESI-MS [M +H]+: 442.2.

To a solution of **intermediate 561** (40 mg, 0.09 mmol) in DCM (4 mL) was added TFA (0.4 mL). The resulting mixture was stirred at room temperature for 3 h. After cooled to 0°C, the mixture was quenched with a solution of NH₃ (7M in MeOH, 2 mL). The resulting solution was concentrated *in vacuo* to give the crude, which was purified by preparative TLC (eluent: DCM/MeOH = 20/1) to give intermediate 556 (20 mg, 65%) as a yellow solid. ESI-MS [M +H]+: 342.1.

### Intermediate 562 (R)-7-(2-(1-aminoethyl)-6-cyclopropylimidazo[1,2-a]pyridin-8-yl)-2,5-dioxa-7-azaspiro[3.4]octan-6-one

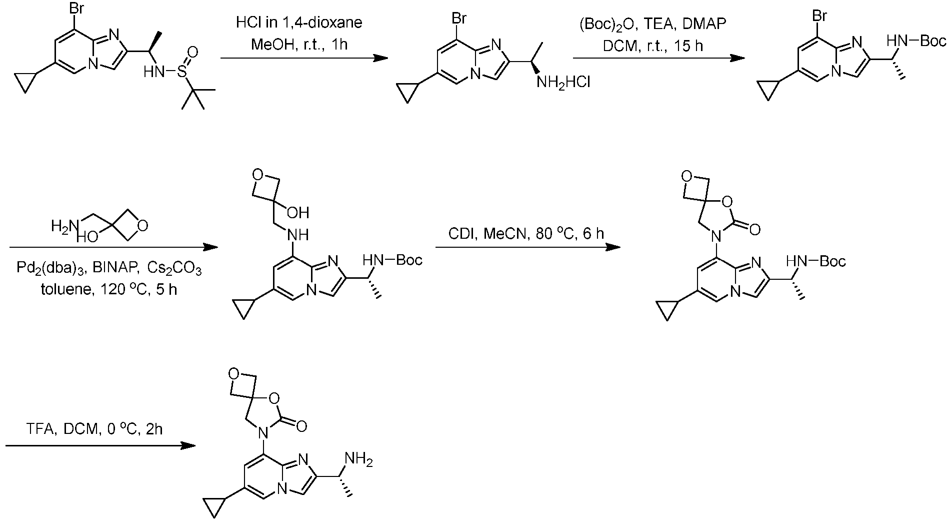

To a solution of N-((R)-1-(8-bromo-6-cyclopropylimidazo[1,2-a]pyridin-2-yl)ethyl)-2-methylpropane-2-**sulfinamide** (1.5 g, 3.9 mmol) in MeOH (10 mL) was added HCl (4M solution in 1,4-dioxane, 10 mL). The resulting mixture was stirred at room temperature for 1 h and concentrated *in vacuo* to give **(R)-1-(8-bromo-6-cyclopropylimidazo[1,2-a]pyridin-2-yl)ethan-1-amine hydrochloride intermediate 563** (1.3 g, 94%) as a yellow solid. ESI-MS: [M + H]⁺, 280.0

A mixture of **intermediate 563** (0.70 g, 2.5 mmol), DMAP (31 mg, 0.25 mmol), TEA (1.3 g, 13 mmol) and (Boc)₂O (0.83 g, 3.8 mmol) in DCM (10 mL) was stirred at room temperature for 15 h. The reaction was quenched with water (40 mL), extracted with DCM (3 x 40 mL). The combined organic layers were washed with brine (2 x 60 mL), dried over anhydrous Na₂SO₄, and concentrated *in vacuo* to give the crude, which was purified by silica gel chromatography (eluent: PE/ EtOAc = 3/2) to afford **tert-butyl (R)-(1-(8-bromo-6-cyclopropylimidazo[1,2-a]pyridin-2-yl)ethyl)carbamate intermediate 564** (0.60 g, 63%) as a yellow oil. ESI-MS: [M + H]⁺, 380.1

To a solution of **intermediate 564** (0.50 g, 1.3 mmol), 3-(aminomethyl)oxetan-3-ol (0.27 g, 2.6 mmol) and Cs₂CO₃ (1.3 g, 3.9 mmol) in toluene (50 mL) was added Pd₂(dba)₃ (0.24 g, 0.26 mmol) and BINAP (0.33 g, 0.52 mmol). After stirring at 120°C for 5 h, the reaction mixture was filtered through Celite^{®} and the filter cake was washed with DCM (50 mL x 3). The filtrate was concentrated *in vacuo* to give the crude, which was purified by silica gel chromatography (eluent: MeOH/DCM = 1/10) to afford **tert-butyl (R)-(1-(6-cyclopropyl-8-(((3-hydroxyoxetan-3-yl)methyl)amino)imidazo[1,2-a]pyridin-2-yl)ethyl)carbamate intermediate 565** (0.31 g, 58%) as a yellow solid. ESI-MS: [M + H]⁺, 403.2

A mixture of **intermediate 565** (0.31 g, 0.77 mmol) and 1,1'-carbonyldiimidazole (0.50 g, 3.1 mmol) in acetonitrile (40 mL) was stirred at 80°C for 6 h. The reaction mixture was concentrated *in vacuo* to give the crude, which was purified by silica gel chromatography (eluent: MeOH/DCM = 1/20) to afford **tert-butyl (R)-(1-(6-cyclopropyl-8-(6-oxo-2,5-dioxa-7-azaspiro[3.4]octan-7-yl)imidazo[1,2-a]pyridin-2-yl)ethyl)carbamate intermediate 566** (0.30 g, 91 %) as a yellow solid. ESI-MS: [M + H]⁺, 429.2

A mixture of **intermediate 566** (0.30 g, 0.70 mmol) and trifluoroacetic acid (1 mL) in DCM (10 mL) stirred at 0°C for 2 h. The pH of reaction mixture was adjusted to 8 with NH₃ (7M solution in MeOH, 5 mL, 35 mmol) at 0°C. The mixture was diluted with DCM (40 mL) then concentrated *in vacuo.* The residue was dissolved in DCM (20 mL). The mixture was filtered and the filtrant washed by DCM (3 x 50 mL). The combined organics were concentrated *in vacuo* to give **intermediate 562** (0.20 g, 87%) as a white solid. ESI-MS: [M + H]⁺, 329.2

### Intermediate 567: 2-(aminomethyl)-6-cyclopropyl-N,N-dimethylimidazo[1,2-a]pyridine-8-sulfonamide

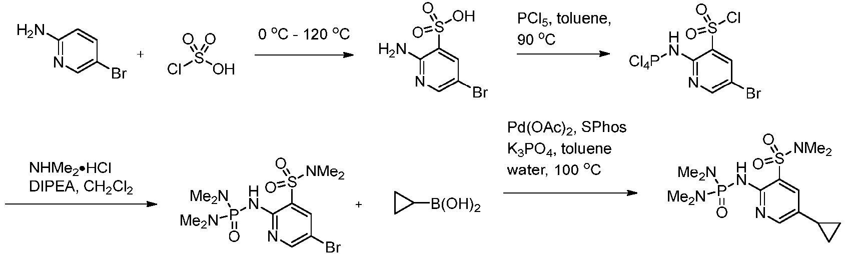

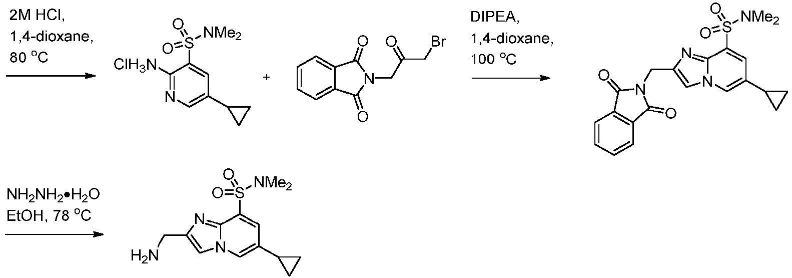

5-bromopyridin-2-amine (1.29 g, 7.45 mmol), was added portionwise to stirred sulfurochloridic acid (5.0 mL) at 0°C. The reaction was then heated to 120°C for 18 h. The reaction was cooled to room temperature and poured onto ice (250 g). The resulting precipitate was filtered, washed with water and dried to give ***2-amino-5-bromopyridine-3-sulfonic acid* intermediate 568** as a pale yellow powder (1.18 g, 58 %). ¹H NMR (400 MHz, DMSO) δ 8.15 - 8.11 (m, 2H), 7.56 (s, 1H), 7.21 (d, J=9.3 Hz, 1H), 6.80 (d, J=8.3 Hz, 1H).

**intermediate 568** (1.0 g, 3.95 mmol) and PCl₅ (1.23 g, 5.93 mmol) were suspended in toluene (10.0 mL) and the reaction was heated at 90°C for 18 h. The reaction was concentrated *in vacuo* and used in the next step without further purification ***(5-bromo-2-((tetrachloro-λ⁵***-***phosphaneyl)amino)pyridine-3-sulfonyl chloride* intermediate 569)**

DIPEA (6.9 mL, 39.50 mmol) was added to a stirred mixture of **intermediate 569** (1.75 g, 3.95 mmol) and dimethylamine hydrochloride (3.22 g, 39.50 mmol) in DCM (40 mL) and stirred at room temperature for 18 h. The reaction was diluted with DCM (60 mL) and washed with water and brine. The combined organics were dried over MgSO₄ and concentrated *in vacuo.* The residue was used in the next step without further purification. ***(2-((bis(dimethylamino)phosphoryl)amino)-5-bromo-N,N-dimethylpyridine-3-sulfonamide* intermediate 570)**

**Intermediate 570** (1.64 g, 3.95 mmol), cyclopropylboronic acid (0.51 g, 5.93 mmol), Pd(OAc)₂ (44 mg, 0.20 mmol), SPhos (160 mg, 0.40 mmol) and K₃PO₄ (2.93 g, 13.83 mmol) were suspended in toluene (20 mL) and water (2.0 mL), the reaction was degassed with N₂ for 5 min. The reaction was then heated to 100°C for 2.5 h. The reaction was cooled to room temperature, diluted with EtOAc (60 mL) and washed with water and brine. The combined organics were dried over MgSO₄ and concentrated *in vacuo.* The residue was purified by silica gel chromatography, eluting with a gradient of 0-6 % MeOH in DCM to give ***2-((bis(dimethylamino)phosphoryl)amino)-5-cyclopropyl-N,N-dimethylpyridine-3-sulfonamide* intermediate 571** (805 mg, 54 %) which was used without further purification.

**Intermediate 571** (805 mg, 2.14 mmol), was suspended HCl (2M aq., 4.3 mL, 8.58 mmol) and 1,4-dioxane (15.0 mL) and heated at 80°C for 48 h. The mixture was then cooled to room temperature and concentrated *in vacuo.* The residue was dissolved in DCM (100 mL) and washed with NaHCO₃ (sat. aq., 2 x 40 mL) and brine (50 mL), the combined organics were dried over MgSO₄ and concentrated *in vacuo* to give ***2-amino-5-cyclopropyl-N,N-dimethylpyridine-3-sulfonamide hydrochloride* intermediate 572(**430 mg, 83 %). ¹H NMR (400 MHz, DMSO) δ 8.13 (d, J=2.3 Hz, 1H), 7.47 (d, J=2.5 Hz, 1H), 6.50 (s, 2H), 2.72 (s, 6H), 1.98 - 1.90 (m, 1H), 0.94 (ddd, J=4.4, 6.3, 8.5 Hz, 2H), 0.69 - 0.64 (m, 2H).

DIPEA (0.23 mL, 1.30 mmol) was added to a stirred solution of **intermediate 572** (360 mg, 0.648 mmol) and **intermediate 311** (200 mg, 0.713 mmol) in dioxane (10.0 mL). The reaction was heated at 100°C for 18 h. The reaction was cooled to room temperature and then concentrated *in vacuo.* The residue was dissolved in EtOAc (100 mL) and washed with NaHCO₃ (sat. aq., 2 x 40 mL) and brine (50 mL), the combined organics were dried over MgSO₄ and concentrated *in vacuo.* The residue was purified by silica gel chromatography, eluting with a gradient of 0-100 % DCM in EtOAc to give ***6-cyclopropyl-2-((1,3-dioxoisoindolin-2-yl*)*methyl-N,N-dimethylimidazo[1,2-a]pyridine-8-sulfonamide* intermediate 573** (270 mg, 95 %). ESI-MS (M+H)+: 425, δ 8.57 (d, J=1.0 Hz, 1H), 7.99 - 7.91 (m, 5H), 7.53 (d, J=1.5 Hz, 1H), 4.99 (s, 2H), 2.74 (s, 6H), 2.13 - 2.05 (m, 1H), 1.01 (ddd, J=4.4, 6.4, 8.3 Hz, 2H), 0.78 - 0.72 (m, 2H).

Using a similar procedure to that for**intermediate 278, intermediate 567** (220 mg at 79 % purity) was synthesised from **intermediate 573** (285 mg, 0.671 mmol) using hydrazine hydrate. ESI-MS (M+H)+: 295.3, ¹H NMR (400 MHz, DMSO) δ 8.63 (d, J=1.0 Hz, 1H), 7.86 (s, 1H), 7.51 (d, J=1.8 Hz, 1H), 3.88 (s, 2H), 2.91 (s, 6H), 2.14 - 2.06 (m, 1H), 1.05 - 1.00 (m, 2H), 0.80 - 0.74 (m, 2H).

### Intermediate 574: 2-(2-(aminomethyl)-6-cyclopropylimidazo[1,2-a]pyridin-8-yl)propan-2-ol

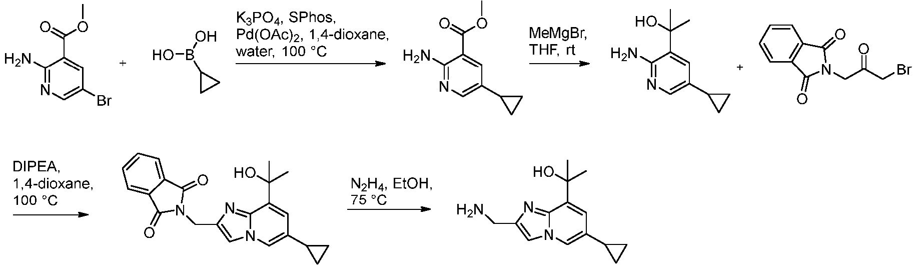

Methyl 2-amino-5-bromonicotinate (2.0 g, 8.7 mmol), cyclopropylboronic acid (1900 mg, 22 mmol), SPhos (360 mg, 0.87 mmol) and K₃PO₄ (6.4 g, 30 mmol) were suspended in 1,4-dioxane (40 mL) and water (4.0 mL) and degassed for 5 min. Pd(OAc)₂ (97 mg, 0.4 mmol) was added and the reaction mixture was stirred at 100°C for 16 h. The mixture was cooled to room temperature, diluted with EtOAc (100 mL) and washed with water (70 mL) and brine (3 × 50 mL). The organics were dried over MgSO₄ and concentrated *in vacuo.* The residue was purified by silica gel chromatography eluting with a gradient of 0-100 % EtOAc in isohexane to give ***methyl 2-amino-5-cyclopropylnicotinate* intermediate 575** (1.2 g, 72 %).
ESI-MS (M+H)+: 193.1, ¹H NMR (400 MHz, CDCl₃) δ 8.09 (d, J=2.4 Hz, 1H), 7.83 - 7.81 (m, 1H), 6.32 - 6.32 (m, 2H), 3.88 (s, 3H), 1.83 - 1.77 (m, 1H), 0.93 - 0.88 (m, 2H), 0.64 - 0.56 (m, 2H).

A solution of **intermediate 575** (1200 mg, 6.2 mmol) in THF (30 mL) was cooled to -20°C and methylmagnesium bromide (3M in Et₂O, 13 mL, 38 mmol) was added dropwise. The reaction mixture was allowed to warm up to room temperature over 16 h. The mixture was quenched with sodium bicarbonate (sat. aq., 50 mL), diluted with EtOAc (70 mL), filtered and separated. The organics were washed with brine (50 mL), dried over MgSO₄ and concentrated *in vacuo.* The residue was purified by silica gel chromatography eluting with a gradient of 0-100 % EtOAc in cyclohexane to give ***2-(2-Amino-5-cyclopropylpyridin-3-yl)propan-2-o1* intermediate 576** (750 mg, 63 %). ESI-MS (M+H)+: 193.2, ¹ δ 7.65 (d, J=2.1 Hz, 1H), 7.00 (d, J=2.1 Hz, 1H), 5.84 (s, 2H), 5.32 (s, 1H), 1.81 - 1.73 (m, 1H), 1.48 (s, 6H), 0.85 - 0.80 (m, 2H), 0.57 - 0.52 (m, 2H).
A solution of **intermediate 576** (700 mg, 3.6 mmol), **intermediate 311** (1300 mg, 4.0 mmol) and DIPEA (0.95 mL, 5.5 mmol) in 1,4-dioxane (80 mL) was stirred at 100°C for 16 h. The mixture was cooled to room temperature, diluted with EtOAc (150 mL) and washed with sodium bicarbonate (2 × 70 mL). The organics were dried over MgSO₄ and concentrated *in vacuo.* The residue was purified by silica gel chromatography eluting with a gradient of 0-100 % EtOAc in cyclohexane to give 2-((6-***cyclopropyl-8-(2-hydroxypropan-2-yl)imidazo[1,2-a]pyridin-2-yl)methyl)isoindoline-1,3-dione* intermediate 577** (800 mg, 59 %). ESI-MS (M+H)+: 376.2, ¹H NMR (400 MHz, CDCl₃) δ 7.87 - 7.85 (m, 2H), 7.73 - 7.70 (m, 2H), 7.67 (s, 1H), 7.42 (s, 1H), 6.78 (d, J=1.6 Hz, 1H), 6.38 (s, 1H), 5.02 (s, 2H), 1.89 - 1.81 (m, 1H), 1.64 (s, 6H), 0.96 - 0.90 (m, 2H), 0.64 - 0.59 (m, 2H).

Using a similar procedure to that for **intermediate 278, intermediate 574** (500 mg, 96 %) was synthesised from **intermediate 577** (800 mg, 2.13 mmol) using hydrazine hydrate. ESI-MS (M+H)+: 246.2, ¹H NMR (400 MHz, CDCl₃) δ 7.74 - 7.73 (m, 1H), 7.34 (s, 1H), 6.79 (d, J=1.6 Hz, 1H), 3.97 (s, 2H), 1.92 - 1.83 (m, 1H), 1.69 (s, 6H), 0.98 - 0.92 (m, 2H), 0.68 - 0.63 (m, 2H). OH and NH₂ not observed

### Intermediate 578 (R)-1-(2-(1-aminoethyl)-6-(1-fluorocyclopropyl)imidazo[1,2-a[pyridin-8-yl)-3-methylimidazolidine-2,4-dione

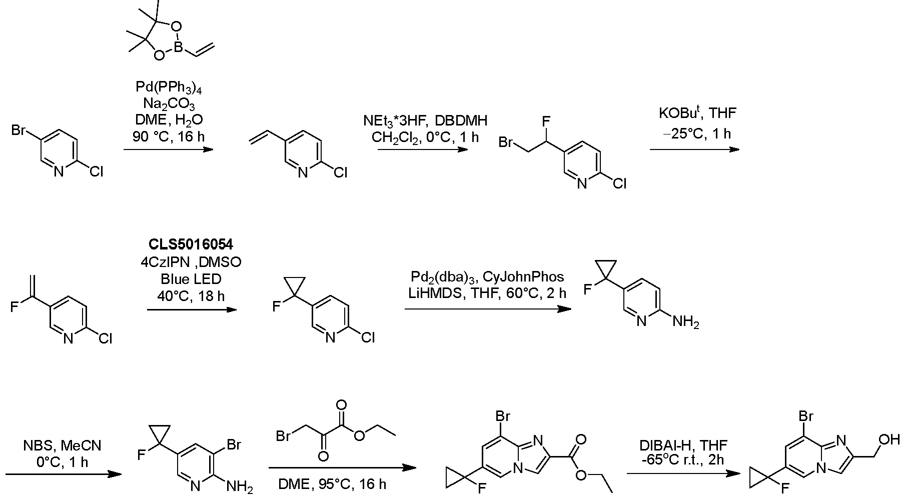

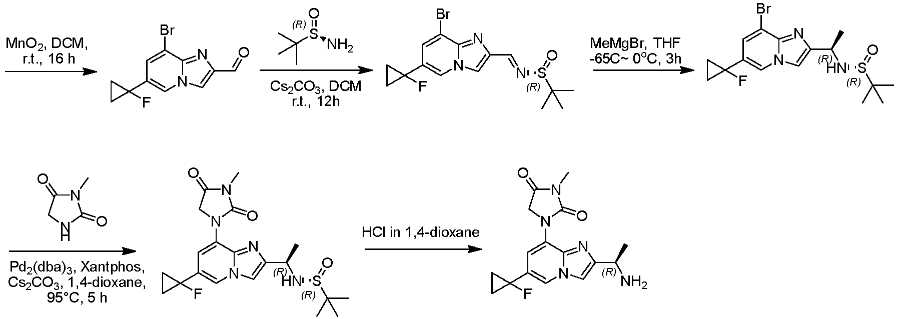

A mixture of 5-bromo-2-chloropyridine (10.0 g, 52.0 mmol), vinyl boronic acid pinacol ester (10.4 g, 67.6 mmol), 1,2-dimethoxyethane (250 mL) and Na₂CO₃ (2 M aq., 78 mL) was degassed (by vacuum/nitrogen refill cycles). Pd(PPh₃)₄ (3.00 g, 2.60 mmol) was added and the mixture was heated at 90°C for 16 h. The mixture was cooled to room temperature, diluted with ethyl acetate (250 mL) and Na₂CO₃ (10% aq.,150 mL). The layers were separated, and the organic layer was washed with brine, dried with Na₂SO₄, filtered and concentrated *in vacuo* to obtain a golden oil, which was loaded onto a silica gel column (250 g) equilibrated with heptane. Elution with heptane : ethyl acetate (1:0 - 96:4) afforded **2-chloro-5-vinylpyridine intermediate** 579 (4.71 g, 65%), as colourless liquid. ESI-MS [M + H]+: 140/142. ¹H NMR (400 MHz, CDCl₃, ppm) δ 8.37 (d, J 3 Hz, 1H), 7.70 (dd, J 9, 3 Hz, 1H), 7.29 (d, J 9 Hz, 1H), 6.67 (dd, J 18, 11 Hz, 1H), 5.81 (d, J 18 Hz, 1H), 5.42 (d, J 11 Hz, 1H).

A solution of **intermediate 579** (4.71 g, 33.7 mmol) in dichloromethane (45 mL) was added to a suspension of 1,3-dibromo-5,5-dimethylhydantoin (14.5 g, 50.6 mmol) in dichloromethane (40 mL) at 0°C. After being stirred for 30 min the mixture was treated with triethylamine trihydrofluoride (8.24 mL, 50.6 mmol). After a further 30 min the reaction was quenched by being poured into NaHCO3 (sat. aq., 250 mL). After being stirred for 30 min the mixture was diluted with ethyl acetate (250 mL). The layers were separated, and the organic layer washed with NaHSO3 (5%, aq.,100 mL), followed by NaHCO3 (sat. aq., 100 mL). The organic solution was washed with brine, dried with Na2SO4, filtered and concentrated to obtain an orange oil, which was purified on silica gel (200 g) column purification using a gradient of heptane : ethyl acetate (95:5 to 8:2) to afford **5-(2-bromo-1-fluoroethyl)-2-chloropyridine intermediate 580** (4.38 g, 54%), as a pale yellow oil. ESI-MS [M + H]+: 238/240/242. ¹H NMR (400 MHz, CDCl₃, ppm) δ 8.41 (s, 1H), 7.70 (dd, J 8, 2 Hz, 1H), 7.40 (d, J 8 Hz, 1H), 5.68 (dt, J 46, 6 Hz, 1H), 3.75-3.59 (m, 2H). 19F NMR (373MHz, CDCl₃, ppm) δ:175.1 (dt, J 46, 18 Hz, 1F).

A solution of potassium tert-butoxide (1 M in tetrahydrofuran, 29.4 ml, 29.4 mmol) was added *via* syringe pump over 60 min to a rapidly stirred solution of **intermediate 580** in anhydrous tetrahydrofuran (100 mL) at -25°C. After the addition was complete, the mixture was poured into citric acid (1 M aq., 50 mL). The pH was raised to ~7 by addition of NaHCO₃ (sat. aq., 150 mL). The mixture was diluted with methyl tert-butyl ether (300 mL) and the layers were separated. The organic layer was washed with brine (2 x 150 mL), dried (MgSO₄), filtered and concentrated *in vacuo* to obtain a light brown oil. The crude product was purified on silica gel (100 g) using a gradient of heptane : ethyl acetate (1:0 - 9:1) to afford **2-chloro-5-(1-fluorovinyl)pyridine intermediate 581** (2.46 g, 85%), as a colourless oil. ESI-MS [M + H]+ 158/160., ¹H NMR (400 MHz, CDCl₃, ppm) δ 8.59 (d, J 2 Hz, 1H), 7.78 (dd, J 9, 2 Hz, 1H), 7.36 (d, J 9 Hz, 1H), 5.11 (dd, J 49, 4 Hz, 1H), 5.02 (dd, J 18, 4 Hz, 1H). 19F NMR (376 MHz, CDCl₃, ppm) δ -108.7 (dd, J 49, 18 Hz, 1F).

An oven-dried 250 mL round bottom flask equipped with a magnetic stir bar was charged with **intermediate 581** (1.53 g, 9.74 mmol), 4CzIPN (384 mg, 0.49 mmol), triethylammonium bis(catecholato)iodomethylsilicate (7.12 g, 14.6 mmol) and anhydrous DMSO (100 mL). The resulting solution was degassed (vacuum/nitrogen refill x 3) and placed in front of two blue LED lamps (Kessil, H150 Growlights, 32W, 420-500 nm). The lamps were arranged on both sides of the flask as close as possible to its surface. A fan for cooling was mounted over the reaction flask. The reaction was left for 18 h. Proton NMR of a sample showed complete consumption of starting material. The heterogeneous mixture was poured into methyl tert-butyl ether (250 mL). NaHCO₃ (Sat., aq., 150 mL) and water (300 mL) were added with stirring. After for 10 min Dicalite (30 g) was added and the mixture was stirred for another 10 min. The mixture was filtered through a sinter funnel, washing the precipitate with additional methyl tert-butyl ether (2 × 200 mL). The clear biphasic filtrate was transferred to a separatory funnel and the layers were separated. The organic layer was washed with more saturated aqueous sodium bicarbonate (250 mL). The combined aqueous layers were extracted with methyl tert-butyl ether (100 mL). The organic solutions were combined, washed with brine (2 × 150 mL), dried (Na₂SO₄), filtered and concentrated under reduced pressure to obtain a dark yellow gum. Purification by flash chromatography on silica gel (150 g), eluting with pentane : methyl tert-butyl ether = 96:4 (500 mL), 94:6 (500 mL) and 92:8 (1L), gave **2-chloro-5-(1-fluorocyclopropyl)pyridine intermediate 582** (673 mg, 40%), as a yellow oil that crystallised upon standing. ESI-MS [M + H]+ 172/174, 1H NMR (400 MHz, CDCl₃, ppm) δ 8.28 (d, J 2 Hz, 1H), 7.56 (dd, J 8, 2 Hz, 1H), 7.33 (d, J 8 Hz, 1H), 1.62-1.52 (m, 2H), 1.12-1.05 (m, 2H).

To a stirred solution of **intermediate 582** (860 mg, 5 mmol), Pd₂(dba)₃ (458 mg, 0.5 mmol) and CyJohnPhos (350 mg, 1 mmol) in THF (50 mL) was added LiHMDS (25 ml, 25 mmol. After stirring at 60°C for 2 h under N₂, the reaction was quenched with saturated aq. NH₄Cl (20 mL) and extracted with EtOAc (3 x 30 mL). The combined organic layers were washed with brine (30 mL), dried over anhydrous Na₂SO₄, and concentrated *in vacuo* to give the crude, which was purified by silica gel chromatography (eluent: DCM/MeOH = 30/1) to give the **5-(1-fluorocyclopropyl)pyridin-2-amine intermediate 583** (450 mg, 59%) as a yellow solid. ESI-MS [M +H]⁺: 153.1.

To a stirred solution of **intermediate 583** (450 mg, 3 mmol) in MeCN (20 mL) was added a solution of NBS (587 mg, 3.3 mmol) in MeCN (5 mL) at 0°C. After stirring at 0°C for 0.5 h. water (50 mL) was added and the mixture was extracted with EtOAc (3 x 30 mL). The combined organic layers were washed with brine (60 mL), dried over anhydrous Na₂SO₄, and concentrated *in vacuo* to give the crude, which was purified by column chromatography (eluent: EA/PE = 1/5) to give the **3-bromo-5-(1-fluorocyclopropyl)pyridin-2-amine intermediate 584** (290 mg, 42 %) as a yellow solid. ESI-MS [M +H]⁺: 231.0.

To a mixture of **intermediate 584** (290 mg, 1.3 mmol) in DME (20mL) was added ethyl 3-bromo-2-oxopropanoate (761 mg, 3.9 mmol) and the mixture was stirred at 95°C for 16 h. After cooling to 25°C, water (50 mL) was added and the mixture was extracted with EtOAc (3 x 30 mL). The combined organic layers were washed with brine (60 mL), dried over anhydrous Na₂SO₄, and concentrated *in vacuo* to give the crude, which was purified by silica gel chromatography (eluent: PE/EtOAc = 5/1) to give **ethyl 8-bromo-6-(1-fluorocyclopropyl)imidazo[1,2-a]pyridine-2-carboxylate intermediate 585** (260 mg, 63%) as a yellow solid. ESI-MS [M +H]⁺: 327.0.

To a stirred solution of **intermediate 585** (260 mg, 0.8 mmol) in THF (10 mL) was slowly added DIBAL-H (2.4 mmol, 2.4 mL) at -65°C. Then the reaction was stirred at -65°C for 2h and warmed to room temperature. The reaction was quenched with water (10 mL) and extracted with EtOAc (3 x 10 mL). The combined organic layers were washed with brine (20 mL), dried over anhydrous Na₂SO₄, and concentrated *in vacuo* to give the crude product, which was purified by silica gel chromatography (eluent: EA/PE = 1/3) to give the **(8-bromo-6-(1-fluorocyclopropyl)imidazo[1,2-a]pyridin-2-yl)methanol** intermediate 586 (160 mg, 71%) as yellow solid. ESI-MS [M +H]+: 285.1.

To a mixture of **intermediate 586** (158 mg, 0.55 mmol) in DCM (20 mL) was added MnO₂ (479 mg, 5.5 mmol). The resulting mixture was stirred at room temperature for 16 h, then concentrated *in vacuo* to give the crude, which was purified by silica gel chromatography (eluent: EA/PE = 1/3) to give the **8-bromo-6-(1-fluorocyclopropyl)imidazo[1,2-a]pyridine-2-carbaldehyde intermediate 587** (142 mg, 92%) as a yellow solid. ESI-MS [M +H]+: 283.0.

To a mixture of **intermediate 587** (142 mg, 0.5 mmol) and (R)-2-methylpropane-2-sulfinamide (91 mg, 0.75 mmol) in DCM (15 mL) was added Cs₂CO₃ (326 mg, 1.0 mmol). The reaction was stirred at room temperature for 16 h, then concentrated *in vacuo* to give the crude, which was purified by silica gel chromatography (eluent: EA/PE = 1:1) to give **(R, E)-N-((8-bromo-6-(1-fluorocyclopropyl) imidazo[1,2-a]pyridin-2-yl)methylene)-2-methylpropane-2-sulfinamide intermediate 588** (160 mg, 83 %) as yellow solid. ESI-MS [M +H]+: 386.0.

To a solution of **intermediate 588** (160 mg, 0.41 mmol) in THF (10 mL) was slowly added methylmagnesium bromide (1.2 mmol, 1M in THF, 1.2 mL) at -65°C. Then the reaction was stirred at - 65°C for 3h under N₂. The reaction was quenched with saturated aq. NH₄Cl (20 mL) and extracted with EtOAc (3 x 30 mL). The combined organic layers were washed with brine (30 mL), dried over anhydrous Na₂SO₄, and concentrated *in vacuo* to give the crude, which was purified by silica gel chromatography (eluent: EA/PE = 1/1) to give the **(R)-N-((R)-1-(8-bromo-6-(1-fluorocyclopropyl) imidazo[1,2-a]pyridin-2-yl)ethyl)-2-methylpropane-2-sulfinamide intermediate 589** (120 mg, 72 %) as a yellow solid. ESI-MS [M +H]+: 402.1.

To a solution of intermediate 589 (100 mg, 0.25 mmol), **intermediate 322** (86 mg, 0.75 mmol) and Cs₂CO₃ (245 mg, 0.75 mmol) in 1,4-dioxane (20 mL) was added Pd₂(dba)₃ (23 mg, 0.025 mmol) and Xantphos (29 mg, 0.05 mmol). The reaction mixture was filtered through Celite^{®} and the filter cake was washed with DCM/MeOH (10/1, 100 mL). The filtrate was concentrated *in vacuo* to give the crude, which was purified by silica gel chromatography (eluent: MeOH / DCM= 1 / 30) to give the **(R)-N-((R)-1-(6-(1-fluorocyclopropyl)-8-(3-methyl-2,4-dioxoimidazolidin-1-yl)imidazo[1,2-a]pyridin-2-yl)ethyl)-2-methylpropane-2-sulfinamide intermediate 590** (90 mg, 83 %) as a yellow solid. ESI-MS [M +H]+: 436.2.

To a mixture of **intermediate 590** (90 mg, 0.21 mol) in 1,4-dioxane (2 mL) was added HCl (4M solution in dioxane, 2 mL). After stirring at room temperature for 1 h, the resulting reaction solution was neutralized with saturated aq. NaHCO₃ solution and extracted with DCM (3 x 20 mL). The combined organic layers were washed with brine, dried over Na₂SO₄, concentrated *in vacuo* to give the crude, which was purified by preparative TLC to give **intermediate 578** (42 mg, 61%) as a yellow solid. ESI-MS [M +H]+: 332.2.

### Intermediate 591 3-(2-(aminomethyl)-6-cyclopropylimidazo[1,2-a]pyridin-8-yl)propaeitrile

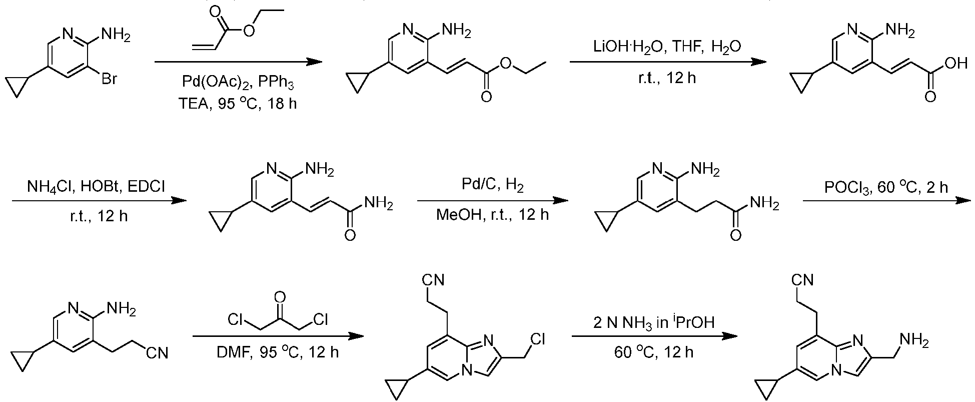

A mixture of **intermediate 309** (1.5 g, 7.0 mmol), Pd(OAc)₂ (0.16 g, 0.71 mmol), PPh₃ (0.93 g, 3.5 mmol), ethyl acrylate (1.4 g,14 mmol) and Et₃N (2.1 g, 21 mmol) in DMF (30 mL) was stirred at 95°C under N₂ for 16 h. After cooling to room temperature, the reaction mixture was quenched with water (40 mL) and extracted with EtOAc (6 x 70 mL). The combined organic layers were washed with saturated brine solution (50 mL), dried over Na₂SO₄, and concentrated *in vacuo* to give the crude, which was purified with column chromatography on silica gel (eluent: PE/EtOAc = 1/1) to give **ethyl (E)-3-(2-amino-5-cyclopropylpyridin-3-yl)acrylate intermediate 592** (0.70 g, 43%). as a yellow solid. ESI-MS [M +H]+: 233.1

A mixture of **intermediate 592** (0.70 g, 3.0 mmol) and LiOH·H₂O (0.51 g, 12 mmol) in THF (30 mL) and H₂O (10mL) was stirred at room temperature for 12 h. The reaction mixture was concentrated *in vacuo* to remove the THF and pH was adjusted to 3~4 with HCl (4 N in dioxane). The resulting mixture was concentrated *in vacuo* to give crude **(E)-3-(2-amino-5-cyclopropylpyridin-3-yl)acrylic acid intermediate 593** (0.75 g, quant), which was used to the next step directly without further purification. ESI-MS [M +H]+: 205.2

A mixture of **intermediate 593** (0.75 g, 3.7 mmol), (NH₄)₂CO₃ (1.8 g, 19 mmol), HOBt(0.99 g, 7.3 mmol), EDCI (1.4 g, 7.3 mmol),and DIPEA (2.4 g, 19 mmol) in DMF (25 mL) was stirred at room temperature for 12 h. The reaction mixture was quenched with water (20 mL) and extracted with EtOAc (8 x 50 mL). The combined organic layers were washed with saturated brine solution (50 mL), dried over Na₂SO₄, and concentrated *in vacuo* to give the crude, which was purified with column chromatography on silica gel (eluent: DCM/CH₃OH= 10/1) to give **(E)-3-(2-amino-5-cyclopropylpyridin-3-yl)acrylamide intermediate 594** (0.51 g, 68%) as a white solid. ESI-MS [M +H]+: 204.2

A mixture of **intermediate 594** (0.51 g, 2.5 mmol) in MeOH (30 mL) and Pd /C (80 mg) was stirred at room temperature under H₂ for 12 h. The mixture was filtered and the filtrate was concentrated *in vacuo* to give the crude, which was purified with column chromatography on silica gel (eluent: DCM/CH₃OH = 10/1) to give **3-(2-amino-5-cyclopropylpyridin-3-yl)propenamide intermediate 595** (0.31 g, 60%) as a yellow solid. ESI-MS [M +H]+: 205.1

A mixture of **intermediate 595** (0.31 g,1.5 mmol) in POCl₃ (15 mL) was stirred at 60°C under N₂ for 2 h. After cooling to room temperature, the reaction solution was concentrated *in vacuo* and the pH adjusted to 7~8 with saturated aq. NaHCO₃. The resulting mixture was extracted with EtOAc (3 x 30 mL). The combined organic layers were washed with saturated brine solution (30 mL), dried over Na₂SO₄ and concentrated *in vacuo* to give the crude, which was purified with preparative TLC (eluent: DCM/CH₃OH = 10/1) to give **3-(2-amino-5-cyclopropylpyridin-3-yl)propanenitrile intermediate 596** (0.12 g, 43%) as a white solid. ESI-MS [M +H]+: 187.2

To a solution of **intermediate 596**(0.12 g, 0.64 mmol) in DMF (15 mL) was added 1,3-dichloropropan-2-one (0.24 g, 1.9 mmol). The resulting mixture was stirred at 95°C under N₂ for 13 h. After cooling to room temperature, the reaction was quenched with water (20 mL) and extracted with EtOAc (3 x 30 mL). The combined organic layers were washed with saturated brine solution (20mL), dried over Na₂SO₄, and concentrated *in vacuo* to give the crude, which was purified with preparative TLC (eluent: PE/EA= 1/1) to give the **3-(2-(chloromethyl)-6-cyclopropylimidazo[1,2-a]pyridin-8-yl)propanenitrile intermediate 597** (70 mg, 42%) as a white solid. ESI-MS [M +H]+: 260.1

A mixture of **intermediate 597** (70 mg, 0.27 mmol) in NH₃(2N NH₃ in ⁱPrOH, 20 mL, 40 mmol) was stirred at 60°C in sealed tube for 14 h. After cooling to room temperature, the reaction mixture was concentrated *in vacuo* to give the **intermediate 591** (80 mg, quant), which was used without further purification. ESI-MS [M +H]+: 241.1

### Intermediate 598: 3-(6-cyclopropyl-2-(hydroxymethyl)imidazo[1,2-a]pyridin-8-yl)propaneitrile

A mixture of **intermediate 597** (0.13 g, 0.50 mmol) and Na₂CO₃ (0.16 g, 1.5 mmol) in THF (10mL) and water (10 mL) was stirred at 90°C for 16 h. After cooling to room temperature, the reaction mixture was quenched with water (10 mL) and extracted with EtOAc/MeOH (10/1, 3 x 20 mL). The combined organic layers were washed with saturated brine solution (20 mL), dried over Na₂SO₄, and concentrated *in vacuo* to give **intermediate 598** as a white soild, which was used into next step without further purification. (90 mg, 75%) ESI-MS [M +H]+: 241.2

### Intermediate 599: (R)-1-(2-(1-aminoethyl)-6-(1,1-difluoroethyl)imidazo[1,2-a]pyridin-8-yl)-3-methylimidazolidine-2,4-dione

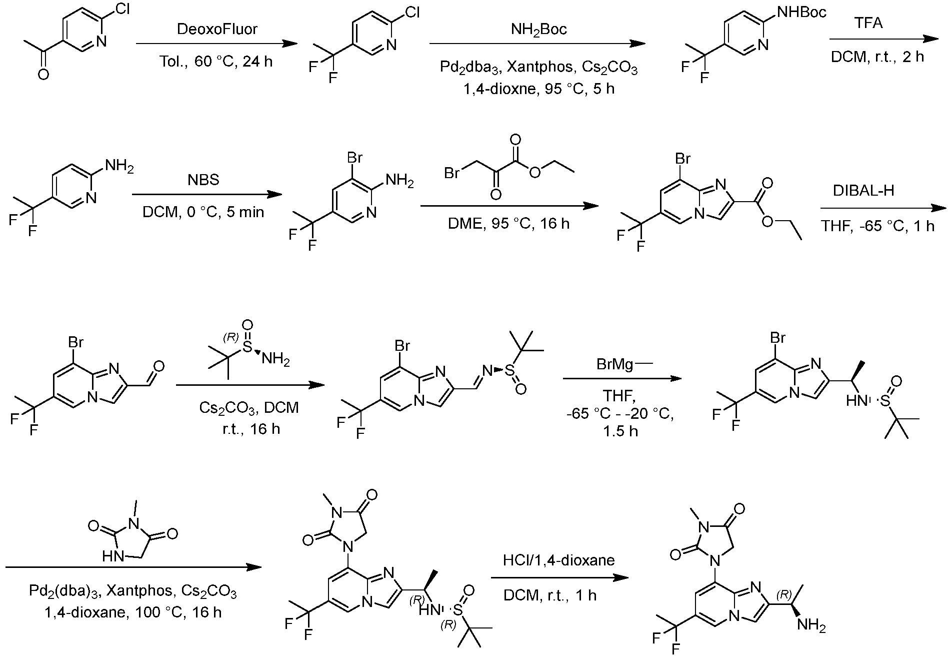

To a solution of 1-(6-chloropyridin-3-yl)ethan-1-one (6.0 g, 39 mmol) in toluene (80 mL) was added DeoxoFluor^{®} (21 mL, 0.11 mol) at room temperature and the mixture was stirred at 60°C for 24 h. The reaction was quenched with NaHCO₃ (Sat. aq., 50 mL) and extracted with EtOAc (50 mL x 3). The combined organic layers were washed with brine (50 mL), dried over Na₂SO₄ and concentrated. The residue was purified by column chromatography (eluent: EtOAc/PE = 0 - 20%) to afford **2-chloro-5-(1,1-difluoroethyl)pyridine intermediate 600** (4.3 g, yield 62%) as colorless oil. ESI-MS [M +H]+: 178.1

To a mixture of **intermediate 600** (4.3 g, 24 mmol) and tert-butyl carbamate (5.7 g, 48 mmol), Xantphos (2.8 g, 4.8 mmol) and Cs₂CO₃ (20 g, 61 mmol) in 1,4-dioxane (100 mL) was added Pd₂(dba)₃ (2.2 g, 2.4 mmol) and the mixture was stirred at 95°C for 5 h under N₂. The reaction mixture was filtered through Celite^{®} and the filter cake was washed with DCM/MeOH (10/1, 100 mL). The filtrate was concentrated *in vacuo* to give the crude, which was purified by column chromatography (eluent: EtOAc/PE = 0 - 20%) to afford **tert-butyl (5-(1,1-difluoroethyl)pyridin-2-yl)carbamate intermediate 601** (6 g, yield 97%) as a pale yellow solid. ESI-MS [M +H]+: 259.1

To a solution of **intermediate 601** (6 g, 23 mmol) in DCM (30 mL) was added TFA (5 mL) and the mixture was stirred at room temperature for 2 h. The reaction was concentrated *in vacuo* and then neutralized with NH₃ (10 mL, 7M in MeOH), and then concentrated *in vacuo.* The residue was purified by column chromatography (eluent: EtOAc/PE = 0 - 50%) to afford **5-(1,1-difluoroethyl)pyridin-2-amine intermediate 602** (2.6 g, yield 72%) as a pale yellow solid. ESI-MS [M +H]+: 159.1

To a mixture of **intermediate 602** (2.6 g, 16.5 mmol) in DCM (35 mL) was added NBS (3.2 g, 18 mmol) at 0°C. The mixture was stirred at 0°C for 5 min and quenched with NaHCO₃ (Sat. aq., 50 mL). The mixture was extracted with DCM (50 mL x 3). The combined organic layers were washed with brine (50 mL), dried over Na₂SO₄ and concentrated *in vacuo.* The residue was purified by column chromatography (eluent: EtOAc/PE = 0 - 40%) to afford **3-bromo-5-(1,1-difluoroethyl)pyridin-2-amine intermediate 603** (2.9 g, yield 75%) as pale yellow solid. ESI-MS [M +H]+: 237.1

To a mixture of **intermediate 603** (2.9 g, 12 mmol) in DME (50 mL) was added ethyl 3-bromo-2-oxopropanoate (2.6 mL, 18 mmol) and the mixture was stirred at 95°C for 16 h. The mixture was quenched with NaHCO₃ (sat. aq., 50 mL) and extracted with DCM (50 mL x 3). The combined organic layers were washed with brine (50 mL), dried over Na₂SO₄ and concentrated *in vacuo.* The residue was purified by column chromatography (eluent: EtOAc/PE = 0 - 50%) to afford **ethyl 8-bromo-6-(1,1-difluoroethyl)imidazo[1,2-a]pyridine-2-carboxylate intermediate 604** (3 g, yield 75%) as green sticky oil. ESI-MS [M+H] +: 333.1

To a mixture of **intermediate 604** (1.5 g, 4.5 mmol) in THF (40 mL) was added DIBAL-H (14 mL, 14 mmol, 1 M in hexanes) at - 65°C and the mixture was stirred at - 65°C for 1 h. The reaction was quenched at - 65°C with MeOH (5 mL) and 15% NaOH (aq., 20 mL). The mixture was allowed to warm to room temperature and extracted with EtOAc (50 mL x 3). The combined organic layers were washed with brine (50 mL), dried over Na₂SO₄ and concentrated *in vacuo.* The residue was purified by column chromatography (eluent: EtOAc/PE = 0 - 35%) to afford **8-bromo-6-(1,1-difluoroethyl)imidazo[1,2-a]pyridine-2-carbaldehyde carboxylate intermediate 605** (560 mg, yield 43%) as pale-yellow solid. ESI-MS [M + H] +: 289.1

To a mixture of **intermediate 605** (560 mg, 1.94 mmol) and (*R*)-2-methylpropane-2-sulfinamide (280 mg, 2.33 mmol) in DCM (10 mL) was added Cs₂CO₃ (1.3 g, 3.88 mmol) and the mixture was stirred at room temperature for 16 h. The reaction was filtered, and the filtrate was washed with DCM (50 mL) then concentrated *in vacuo.* The residue was purified by column chromatography (eluent: EtOAc/PE = 0 - 40%) to afford **(*R*,*E*)-N-((8-bromo-6-(1,1-difluoroethyl)imidazo[1,2-a]pyridin-2-yl)methylene)-2-methylpropane-2-sulfinamide intermediate 606** (750 mg, yield 99%) as white solid. ESI-MS [M + H] +: 392.1

To a solution of **intermediate 606** (750 mg, 1.92 mmol) in THF (20 mL) was added dropwise methylmagnesium bromide (2 mL, 6 mmol, 3M in ether) at -65°C. The mixture was warmed to -20°C and stirred for 1.5h. The reaction was quenched with NH₄Cl (Sat. aq., 50 mL) and extracted with EtOAc (50 mL x 3). The combined organic layers were washed with brine (50 mL), dried over Na₂SO₄ and concentrated *in vacuo.* The residue was purified by column chromatography (eluent: EtOAc/PE = 0 - 50%) to afford **(*R*)-*N*-((*R*)-1-(8-bromo-6-(1,1-difluoroethyl)imidazo[1,2-a]pyridin-2-yl)ethyl)-2-methylpropane-2-sulfinamide intermediate 607** (550 mg, yield 64%) as white solid. ESI-MS [M + H] +: 408.1

To a mixture of **intermediate 607** (550 mg, 1.35 mmol) and **intermediate 322** (610 mg, 5.35 mmol) in 1,4-dioxane (25 mL) was added Pd₂(dba)₃ (180 mg, 0.21 mmol), xantPhos (0.23 g, 0.40 mmol) and Cs₂CO₃ (1.1 g, 3.4 mmol). The mixture was stirred at 100°C for 16 h under N2. The reaction mixture was filtered through Celite^{®} and the filter cake was washed with DCM/MeOH (10/1, 100 mL). The filtrate was concentrated *in vacuo* to give the crude, which was purified by column chromatography (eluent: EtOAc/PE = 0 - 50%) to afford **(*R*)-*N*-((*R*)-1-(6-(1,1-difluoroethyl)-8-(3-methyl-2,4-dioxoimidazolidin-1-yl)imidazo[1,2-a]pyridin-2-yl)ethyl)-2-methylpropane-2-sulfinamide intermediate 608** (500 mg, yield 84%) as pale yellow solid. ESI-MS [M + H] +: 442.2

To a mixture of **(intermediate 608** (500 mg, 1.13 mmol) in DCM (4 mL) was added HCl (4 mL, 16 mmol, 4M in 1,4-dioxane) at 0°C. The reaction was stirring at room temperature for 1 h. The reaction was concentrated *in vacuo.* The residue was neutralized with NH₃ (10 mL, 7 M in MeOH). The reaction was stirred for 5 min and then concentrated *in vacuo.* The residue was purified by column chromatography (eluent: MeOH/DCM = 0 - 9%) to afford **intermediate 599** (340 mg, yield 89%) as white solid. ESI-MS [M + H] +: 338.2

### Intermediate 609: 1-(2-((R)-1-aminoethyl)-6-(1-fluoroethyl)imidazo[1,2-a]pyridin-8-yl)-3-methylimidazolidine-2,4-dione

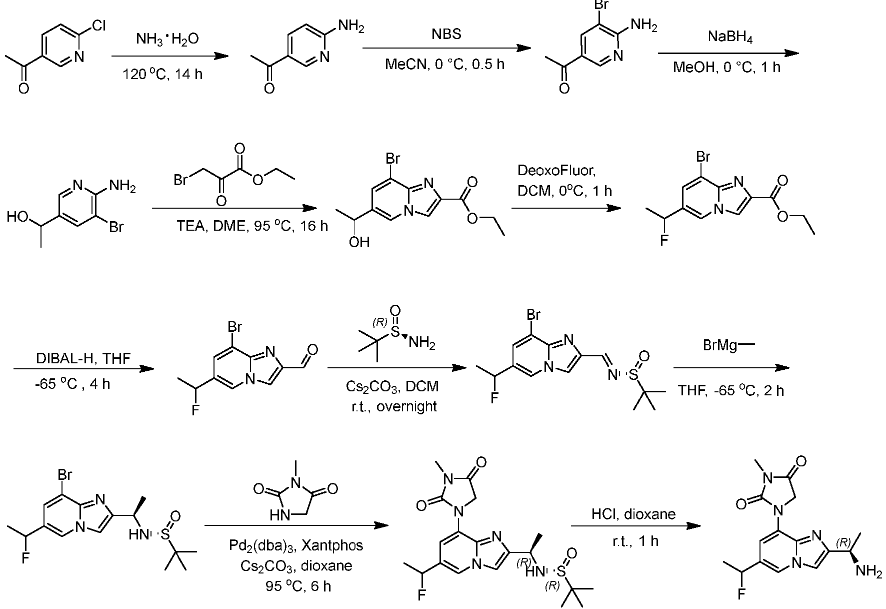

A mixture of 1-(6-chloropyridin-3-yl)ethan-1-one (4.96 g, 32 mmol) in NH₃/H₂O (60 mL) was stirred in a sealed tube at 120°C for 14 h. After cooling to room temperature, the reaction mixture was concentrated *in vacuo* to give the crude, which was purified by column chromatography (eluent: MeOH / DCM = 0 ~ 5%) to give **1-(6-aminopyridin-3-yl)ethan-1-one intermediate 610** (3.4 g, 78%) as a yellow solid. ESI-MS [M +H]+: 137.1.

To a stirred solution of **intermediate 610** (3.4 g, 25 mmol) in MeCN (120 mL) was added NBS (4.9 g in 30 mL MeCN, 27.5 mmol) at 0°C slowly. Then the reaction was stirred at 0°C for 0.5 h. The reaction mixture was quenched with Na₂SO₃ (sat. aq., 100 mL) and extracted with EtOAc (50 mL x 3). The combined organic layers were washed with brine (50 mL), dried over Na₂SO₄ and concentrated *in vacuo* to give the crude, which was purified by column chromatography (eluent: EtOAc / PE = 0 ~ 20%) to give the **1-(6-amino-5-bromopyridin-3-yl)ethan-1-one intermediate 611** (4.3 g, 80 %) as yellow solid. ESI-MS [M +H]⁺: 215.0.

To a solution of **intermediate 611** (4.3 g, 20 mmol) in MeOH (100 mL) was added NaBH₄ (2.28 g, 60 mmol) portionwise at 0°C. The resulting mixture was stirred at 0°C for 1 h. The reaction was quenched with NH₄Cl (sat. aq., 100 mL) and extracted with EtOAc (60 mL x 3). The combined organic layers were washed with brine (60 mL), dried over Na₂SO₄ and concentrated *in vacuo* to give the crude, which was purified by column chromatography (eluent: MeOH / DCM = 0 ~ 5%) to give **1-(6-amino-5-bromopyridin-3-yl)ethan-1-ol intermediate 612** (2.9 g, 67%) as yellow solid. ESI-MS [M +H]⁺: 217.0.

To a solution of **intermediate 612** (2.5 g, 11.5 mmol) in DME (100 mL) was added ethyl 3-bromo-2-oxopropanoate (6.7 g, 34.5 mmol) and TEA (3.5 g, 34.5 mmol). The reaction was stirred at 95°C for 16 h. After cooling to room temperature, the reaction mixture was quenched with NaHCO₃ (sat. aq., 100 mL) and extracted with EtOAc (50 mL x 3). The combined organic layers were washed with brine (50 mL), dried over Na₂SO₄ and concentrated *in vacuo* to give the crude, which was purified by column chromatography (eluent: EtOAc / PE = 0 ~ 30%) to give the **ethyl 8-bromo-6-(1-hydroxyethyl)imidazo[1,2-a]pyridine-2-carboxylate intermediate 613** (1.3 g, 36%) as yellow oil. ESI-MS [M +H]+: 313.0.

To a solution of **intermediate 613** (1.3 g, 4.2 mmol) in DCM (80 mL) was added DeoxoFluor^{®} (2.8 g, 12.6 mol) at 0°C. The resulting mixrture was stirred at 0°C for 1 h. The reaction was quenched with NaHCO₃ (sat. aq., 80 mL) and extracted with EtOAc (50 mL x 3). The combined organic layers were washed with brine (50 mL), dried over Na₂SO₄ and concentrated *in vacuo* to give the crude, which was purified by column chromatography (eluent: EtOAc / PE = 0 ~ 20%) to give **ethyl 8-bromo-6-(1-fluoroethyl)imidazo[1,2-a]pyridine-2-carboxylate intermediate 614** (800 mg, 61% ) as yellow oil. ESI-MS [M +H]+: 314.9.

To a stirred solution of **intermediate 614** (800 mg, 2.5 mmol) in THF (50 mL) was slowly added DIBAL-H (7.5 mL, 7.5 mmol, 1 mol/L) at -70°C. Then the reaction was stirred at -65°C for 4h. EtOAc (50 mL) and Na₂SO₄·10H₂O was added in slowly at -65°C and the reaction mixture was warmed to room temperature and stirred for 30 minutes. Water (10 mL) was added slowly at room temperature and the reaction mixture was stirred at room temperature stirred for another 10 minutes. The reaction mixture was filtered through Celite^{®} and the filter cake was washed with DCM/MeOH (10/1, 50 mL). The filtrate was concentrated *in vacuo* to give the crude, which was purified by silica gel chromatography (eluent: EtOAc / PE = 0 ~ 20%) to give the **8-bromo-6-(1-fluoroethyl)imidazo[1,2-a]pyridine-2-carbaldehyde intermediate 615** (440 mg, 65 %) as yellow solid. ESI-MS [M +H]+: 271.0.

To a reaction mixture of **intermediate 615** (432 mg, 1.6 mmol) and (R)-2-methylpropane-2-sulfinamide (290 mg, 2.4 mmol) in DCM (15 mL) was added Cs₂CO₃ (1.04 g, 3.2 mol) and the mixture was stirred at room temperature for 16 h. The mixture was quenched water (50 mL) and extracted with DCM (50 mL x 3). The combined organic layers were washed with brine (50 mL), dried over Na₂SO₄ and concentrated *in vacuo.* The residue was purified by column chromatography (eluent: EtOAc / PE = 0 ~ 50%) to give **((R)-N-((E)-(8-bromo-6-(1-fluoroethyl)imidazo[1,2-a]pyridin-2-yl)methylene)-2-methylpropane-2-sulfinamide intermediate 616** (320 mg, 54%) as yellow solid. ESI-MS [M +H]+: 374.0.

To a stirred solution of **intermediate 616** (320 mg, 0.86 mmol) in THF (10 mL) was slowly added methylmagnesium bromide (1.2 mL, 3.6 mmol, 3 mol/L) at -70°C. Then the reaction was stirred at -65°C for 2h. The reaction was quenched with NH₄Cl (sat. aq., 30 mL) and then extracted with EtOAc (30 mL x 3). The combined organic layers were washed with brine (30 mL), dried over Na₂SO₄, filtered and concentrated *in vacuo* to give the crude, which was purified by column chromatography (eluent: MeOH / DCM = 0 ~ 5%) to give **(R)-N-((1R)-1-(8-bromo-6-(1-fluoroethyl)imidazo[1,2-a]pyridin-2-yl)ethyl)-2-methylpropane-2-sulfinamide intermediate 617** (200 mg, 60 %) as yellow solid. ESI-MS [M +H]+: 390.1.

To a stirred mixture of intermediate 617 (200 mg, 0.5 mmol), **intermediate 322** (171 mg, 1.5 mmol), xantPhos (58 mg, 0.1 mmol) and Cs₂CO₃ (489 mg, 1.5 mmol) in 1,4-dioxane (15 mL) was added Pd₂(dba)₃ (46 mg, 0.05 mmol). The reaction was stirred at 95°C for 6 h under N₂. The reaction mixture was cooled to room temperature then filtered through Celite^{®} and the filter cake was washed with DCM/MeOH (10/1, 50 mL). The filtrate was concentrated *in vacuo* to give the crude, which was purified by column chromatography (eluent: MeOH / DCM = 0 ~ 3%) to give the **(R)-N-((1R)-1-(6-(1-fluoroethyl)-8-(3-methyl-2,4-dioxoimidazolidin-1-yl)imidazo[1,2-a]pyridin-2-yl)ethyl)-2-methylpropane-2-sulfinamide intermediate 618** (195 mg, 92 %) as yellow solid. ESI-MS [M +H]+: 424.1.

To a solution of **intermediate** 618 (195 mg, 0.46 mol) in 1,4-dioxane (8 mL) was added HCl (2 mL, 8 mmol, 4M solution in 1,4-dioxane). The resulting mixture was stirred at room temperature for 1 h. The reaction was concentrated *in vacuo* and neutralized with NH₃ (5 mL, 7 M in MeOH). The reaction was stirred for 5 min and concentrated *in vacuo* to give the crude, which was purified by column chromatography (eluent: MeOH / DCM = 0 ~ 10%)to afford **intermediate 609** (100 mg, 68 %) as yellow solid. ESI-MS [M +H]+: 320.1.

### Intermediate 619: 1-(2-(aminomethyl)-6-(2,2-difluorocyclopropyl)imidazo[1,2-a]pyridin-8-yl)-3-methylimidazolidine-2,4-dione

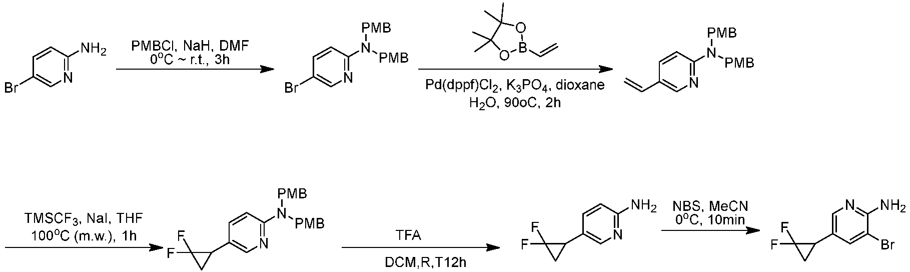

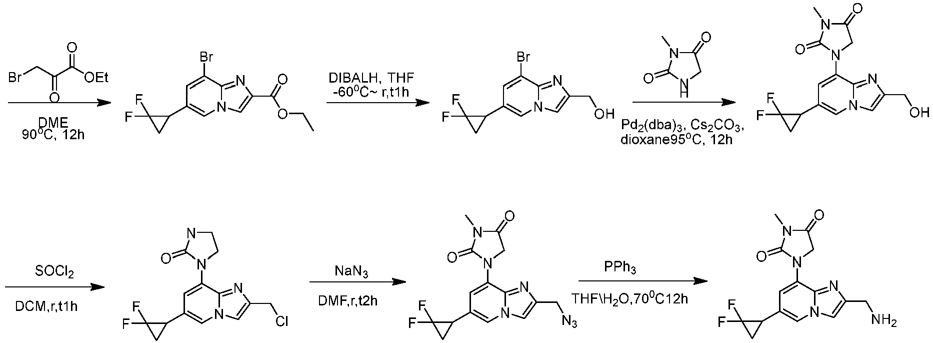

To a mixture of 5-bromopyridin-2-amine (5 g, 28.9 mmol) in DMF (80 ml) was added NaH (4.62 g, 60% in mineral oil, 115.6 mmol) at 0°C and the mixture was stirred at 0°C for 1 h. PMBCl (18 g, 115.6 mmol) was added and the resulting mixture was stirred at room temperature for 2 h. The mixture was quenched with NH₄Cl (sat. aq., 10 mL) and extracted with EtOAc (100 mL x 3). The combined organic layers were washed with brine (100 mL), dried over Na₂SO₄ and concentrated *in vacuo.* The residue was purified by column chromatography (eluent: EtOAc/PE = 0 ~ 20%) to give **5-bromo-N,N-bis(4-methoxybenzyl)pyridin-2-amine intermediate 620** (10 g, 84%) as a white solid. ESI-MS [M +H]+: 413.2.

To a mixture of **intermediate 620** (2 g, 4.8 mmol), 4,4,5,5-tetramethyl-2-vinyl-1,3,2-dioxaborolane (1.1 g, 7.3 mmol) and K₃PO₄ (2.1 g, 9.6 mmol) in 1,4-dioxane/H₂O (40/10 mL) was added Pd(dppf)Cl₂ (350.8 mg, 0.48 mmol). The resulting reaction mixture was stirred at 90°C for 2 h under N₂. After cooling to room temperature, water (100 mL) was added and the mixture was extracted with EtOAc (40 mL X 3). The combined organic layers were washed with brine (60 mL), dried over anhydrous Na₂SO₄, and concentrated *in vacuo* to give the crude, which was purified by column chromatography (eluent: EtOAc/PE = 0 ~ 20%) to give **N,N-bis(4-methoxybenzyl)-5-vinylpyridin-2-amine intermediate 621** (1.1 g, 64%) as a yellow solid. ESI-MS [M +H]⁺: 361.2.

A mixture of **intermediate 621** (800 mg, 2.2 mmol), TMSCF3(1.25 g, 8.8 mmol) , NaI (1.32 g, 8.8 mmol) in THF (3 mL) in a sealed tube was irradiated in a microwave reactor at 100°C for 4 h. The mixture was quenched with NaHCO₃ (sat. aq., 50 mL) and extracted with DCM (50 mL x 3). The combined organic layers were washed with brine (50 mL), dried over Na₂SO₄ and concentrated *in vacuo.* The residue was purified by column chromatography (eluent: EtOAc/PE = 0 - 30%) to give **5-(2,2-difluorocyclopropyl)-N,N-bis(4-methoxybenzyl)pyridin-2-amine intermediate 622** (480 mg, 53%) as a yellow solid. ESI-MS [M +H]⁺: 411.2.

A solution of **intermediate 622** (6 g, 14.6 mmol) in TFA (20 mL) and DCM (20 mL) was stirred at room temperature for 12 h. The reaction was concentrated *in vacuo.* The residue was diluted with NaHCO3 (sat. aq., 100 mL) and extracted with DCM (100 mL x 3). The combined organic layers were washed with brine (100 mL), dried over Na₂SO₄ and concentrated *in vacuo.* The residue was purified by column chromatography (eluent: MeOH/DCM = 0 - 10%) to give **5-(2,2-difluorocyclopropyl)pyridine-2-amine intermediate 623** (2 g, 80%) as a white solid. ESI-MS [M +H]⁺: 171.2

To a mixture of **intermediate 623** (2 g, 11.8 mmol) in MeCN (40 mL) was added NBS (2.3 g, 13.0 mmol) at 0°C and the reaction mixture was stirred at 0°C for 10min. The mixture was quenched with Na₂SO₃ (sat. aq., 100 mL) and extracted with DCM (100 mL x 3). The combined organic layers were washed with brine (100 mL), dried over Na₂SO₄ and concentrated *in vacuo.* The residue was purified by column chromatography (eluent: MeOH/DCM = 0 - 10%) to give **3-bromo-5-(2,2-difluorocyclopropyl)pyridine-2-amine intermediatr 624** (1.4 g, 48%) as a yellow solid. ESI-MS [M +H]⁺: 249.2.

A mixture of **intermediate 624** (800 mg, 3.2 mmol) and ethyl 3-bromo-2-oxopropanoate (1.86 g, 9.6 mmol) in DME (16 mL) was stirred at 90°C for 12 h. The mixture was quenched with NaHCO₃ (sat. aq., 100 mL) and extracted with EtOAc (100 mL x 3). The combined organic layers were washed with brine (100 mL), dried over Na₂SO₄ and concentrated *in vacuo.* The residue was purified by column chromatography (eluent: EtOAc/PE = 0 - 50%) to give **ethyl 8-bromo-6-(2,2-difluorocyclopropyl)imidazo[1,2-a]pyridine-2-carboxylate intermediate 625** (500 mg, 45%) as a yellow solid. ESI-MS [M +H]⁺: 345.2

To a solution of **intermediate 625** (100 mg, 0.29 mmol) in THF (3 mL) was added DIBALH (1.0 M solution in hexane, 0.87 mL, 0.87 mmol) at -60°C. The resulting reaction was warmed and stirred at room temperature for 1h. Na₂SO₄.10H₂O was added and the resulting mixture was stirred at room temperature for 0.5h. The mixture was filtered and the filter cake was washed with DCM/MeOH (10/1, 30 mL). The filtrate was concentrated *in vacuo* to give the crude, which was purified by preparative TLC (eluent: DCM/MeOH = 30/1) to give **(8-bromo-6-(2,2-difluorocyclopropyl)imidazo[1,2-a]pyridin-2-yl)methanol intermediate 626** (50 mg, 57%) as a white solid. ESI-MS [M +H]+: 303.1.

To a mixture of **intermediate 626** (300 mg, 0.99 mmol), **intermediate 322** (340 mg, 2.97 mmol), Cs₂CO₃ (968 mg, 2.97 mmol), and xantPhos (115 mg, 0.2 mmol) in 1,4-dioxane (10 mL) was added Pd₂(dba)₃ (137 mg, 0.15 mmol) and the reaction mixture was stirred at 95°C for 12 h under N₂. The reaction mixture was filtered through Celite^{®} and the filter cake was washed with DCM/MeOH (10/1, 100 mL). The filtrate was concentrated *in vacuo* to give the crude, which was purified by preparative TLC (eluent: DCM/MeOH=20/1) to give **1-(6-(2,2-difluorocyclopropyl)-2-(hydroxymethyl)imidazo[1,2-a]pyridin-8-yl)-3-methylimidazolidine-2,4-dione intermediate 627** (180 mg, 54%) as a yellow solid. ESI-MS [M +H]+: 337.2.

To a solution of **intermediate 627** (150 mg, 0.45 mmol) in DCM (5 mL) was added (108 mg, 0.9 mmol). The reaction mixture was stirred at room temperature for 1 h under N₂ and concentrated *in vacuo* to give **1-(2-(chloromethyl)-6-(2,2-difluorocyclopropyl)imidazo[1,2-a]pyridin-8-yl)imidazolidin-2-one intermediate 628** (160 mg, crude) as a yellow solid, which was used in the next step without further purification. ESI-MS [M +H]+: 326.2.

A mixture of **intermediate 628** (160 mg, crude) and NaN₃ (64 mg, 0.98 mmol) in DMF (3 mL) was stirred at room temperature for 2h. The mixture was quenched with water (30 mL) and extracted with EtOAc (30 mL x 3). The combined organic layers were washed with brine (20 mL), dried over Na₂SO₄ and concentrated *in vacuo* to give methyl **1-(2-(azidomethyl)-6-(2,2-difluorocyclo propyl) imidazo[1,2-a]pyridin-8-yl)-3-methylimidazolidine-2,4-dione intermediate 629** (165 mg, crude) as a yellow solid, which was used in the next step without further purification. ESI-MS [M +H]+: 362.1.

To a mixture of methyl **intermediate 629** (160 mg, crude) in THF/H₂O (10 ml/1 mL) was added PPh₃(254 mg, 0.97 mmol) and the reaction mixture was stirred at 70°C for 2 h under N₂. Water (20 mL) was added and the mixture was extracted with EtOAc (20 mL X 5). The combined organic layers were washed with brine (20 mL), dried over anhydrous Na₂SO₄, and concentrated *in vacuo* to give the crude, which was purified by preparative TLC (eluent: DCM/MeOH = 20/1) to give **intermediate 619** (90 mg, 60% over 3 steps) as a yellow solid. ESI-MS [M +H]⁺: 336.2.

### Intermediat 630 N-(2-(((6-chloropyrimidin-4-yl)amino)methyl)-6-cyclopropylimidazo[1,2-ajpyridin-8-yl)acetamide

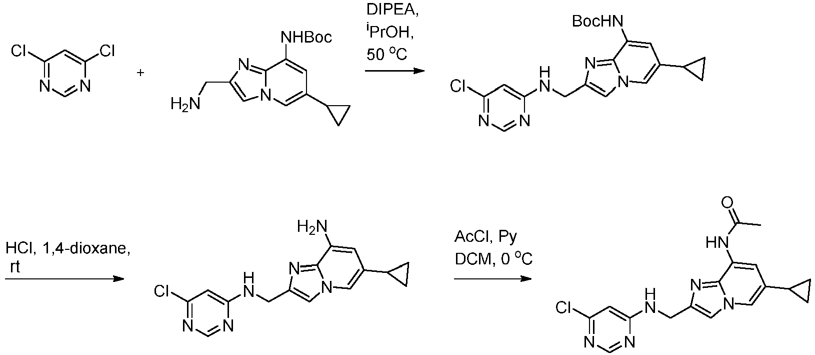

DIPEA (0.19 mL, 1.91 mmol) was added to a stirred solution of 4,6-dichloropyrimidine (95 mg, 0.64 mmol) and *tert*-butyl (2-(aminomethyl)-6-cyclopropylimidazo[1,2-*a*]pyridin-8-yl)carbamate (193 mg, 0.64 mmol) in isopropanol (5 mL), the reaction was then heated at 50°C for 18 h. The reaction was cooled to room temperature and partitioned between water (30 mL) and EtOAc (30 mL), the layers were separated and the aqueous layer was further extracted with EtOAc (2 x 20 mL). The organics were combined, dried over MgSO₄ and concentrated *in vacuo* to give **tert-butyl (2-(((6-chloropyrimidin-4-yl)amino)methyl)-6-cyclopropylimidazo[1,2-a]pyridin-8-yl)carbamate intermediate 631** (175 mg, 66 %). ESI-MS (M+H)+: 415.3, ¹H NMR (400 MHz, CDCl₃) δ 8.40 (s, 1H), 7.66 (s, 1H), 7.62 (s, 1H), 7.52 (d, J=5.1 Hz, 1H), 7.39 (s, 1H), 6.45 (d, J=0.8 Hz, 1H), 4.62 (s, 2H), 1.90 - 1.86 (m, 1H), 1.55 (s, 9H), 1.21 (d, J=7.5 Hz, 1H), 0.96 - 0.90 (m, 2H), 0.73 - 0.68 (m, 2H).

4M HCl in dioxane (0.16 mL, 0.63 mmol) was added to a stirred solution of **intermediate 631** (175 mg, 0.42 mmol) in 1,4-dioxane (5.0 mL) and stirred at room temperature for 18 h. The reaction was quenched with NaHCO₃ solution (sat. aq., 30 mL) and extracted with EtOAc (3 x 20 mL). The organics were combined, dried (MgSO₄) and concentrated *in vacuo.* The residue was purified by silica gel chromatography, eluting with a gradient of 0-10 % MeOH (7N NH₃ in MeOH) in DCM to give **2-(((6-chloropyrimidin-4-yl)amino)methyl)-6-cyclopropylimidazo[1,2-a]pyridin-8-amine intermediate 632** (61 mg, 46 %). ESI-MS (M+H)+: 315.1, ¹H NMR (400 MHz, CDCl₃) δ 8.39 (s, 1H), 7.36 (d, J=7.8 Hz, 2H), 6.43 (s, 1H), 6.11 (d, J=1.5 Hz, 1H), 6.01 (s, 1H), 4.62 - 4.62 (m, 2H), 4.39 (s, 2H), 1.84 - 1.76 (m, 1H), 0.92 - 0.87 (m, 2H), 0.65 - 0.60 (m, 2H).

Acetyl chloride (11.2 µL, 0.16 mmol) was added to a stirred solution of **intermediate 632** (50 mg, 0.16 mmol) and pyridine (38 µL, 0.48 mmol) in DCM (0.5 mL) at 0°C. The reaction was stirred at 0°C for 1 hour. The reaction was diluted with water (10 mL) and extracted with DCM (3 x 10 mL). The organics were combined, dried over MgSO₄ and concentrated *in vacuo.* The residue was purified by silica gel chromatography, eluting with a gradient of 0-10 % MeOH (7N NH₃ in MeOH) in DCM to give **intermediate 630** (35 mg, 56 %). ESI-MS (M+H)+: 397.1, ¹H NMR (400 MHz, CDCl₃) δ 8.43 (s, 1H), 8.39 (s, 1H), 7.95 (s, 1H), 7.58 (s, 1H), 7.41 (s, 1H), 6.43 (s, 1H), 5.97 - 5.97 (m, 1H), 4.63 - 4.63 (m, 2H), 2.27 - 2.26 (m, 3H), 1.88 (s, 1H), 0.98 - 0.91 (m, 2H), 0.73 - 0.66 (m, 2H).

### Intermediate 633 6-chloro-N-((6-cyclopropyl-3-fluoroimidazo[1,2-a]pyridin-2-yl)methyl)pyrimidin-4-amine

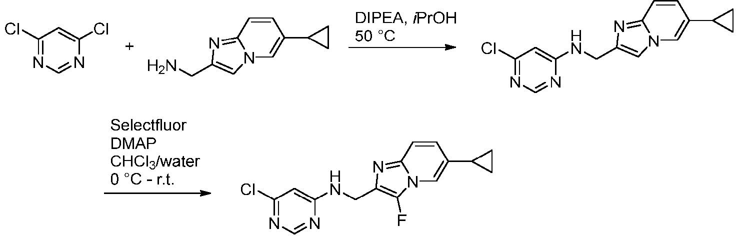

A mixture of 4,6-dichloropyrimidine (1.8 g, 12 mmol), **intermediate 251** (2.5 g, 13 mmol) and DIPEA (6.3 mL, 36 mmol) in *i*PrOH (25 mL) was stirred at 50°C for 18 h. Water was added and the precipitate was collected by filtration to give **6-chloro-N-((6-cyclopropylimidazo[1,2-a]pyridin-2-yl)methyl)pyrimidin-4-amine intermediate 634** (3.2 g, 89 %). ESI-MS (M+H)+: 300, δ 8.33 - 8.17 (m, 3H), 7.69 (s, 1H), 7.40 (d, J=9.4 Hz, 1H), 6.99 (dd, J=1.8, 9.2 Hz, 1H), 6.64 (d, J=0.7 Hz, 1H), 4.62 - 4.48 (m, 2H), 1.97 - 1.89 (m, 1H), 0.96 - 0.90 (m, 2H), 0.70 - 0.65 (m, 2H).

A mixture of **intermediate 634** (0.20 g, 0.67 mmol), Selectfluor^{™} (0.47 g, 1.3 mmol) and DMAP (0.082 g, 0.67 mmol) in CHCl₃ (5.0 mL) and water (1.0 mL) was stirred at 0°C for 6 h. The mixture was warmed to room temperature and stirred for 18 h. The reaction was quenched with water and extracted with DCM. The organic layer was passed through a hydrophobic frit and concentrated *in vacuo.* The residue was purified by column chromatography on silica gel, eluting with 0-5 % 7 N NH₃ in MeOH in DCM to give **intermediate 633** (0.042 g, 20 %). ESI-MS (M+H)+: 318.3, ¹H NMR (400 MHz, CDCl₃) δ 8.40 (s, 1H), 7.65 (d, J=0.8 Hz, 1H), 7.37 - 7.33 (m, 1H), 6.93 (dd, J=1.8, 9.4 Hz, 1H), 6.47 (s, 1H), 5.84 (s, 1H), 4.68 (s, 2H), 1.96 - 1.88 (m, 1H), 1.03 - 0.97 (m, 2H), 0.73 - 0.68 (m, 2H).

### Intermediate 635: 2-((2R,4S)-4-((tert-butyldimethylsilyl)oxy)pyrrolidin-2-yl)-6-cyclopropyl imidazo[1,2-a]pyridine

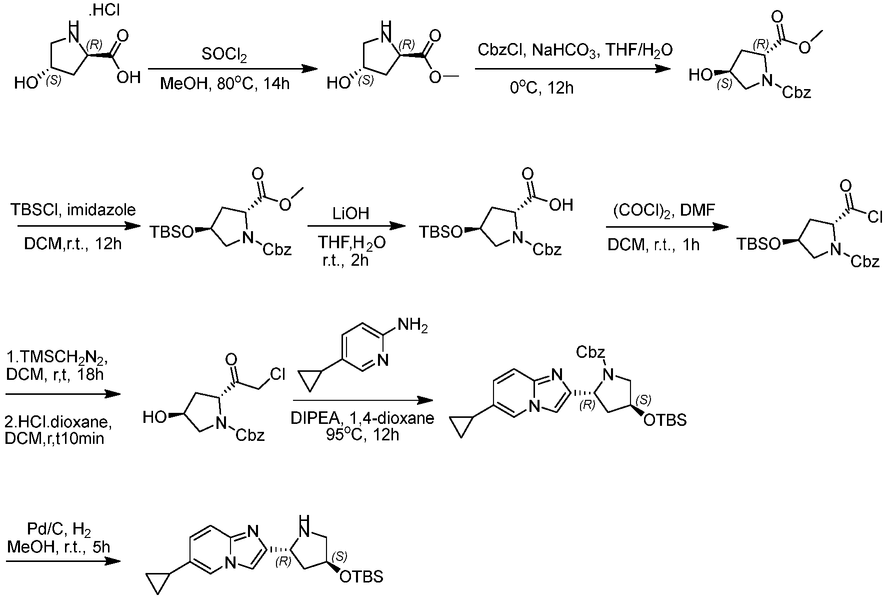

To a solution of *(2R*, *4S)*-4-hydroxypyrrolidine-2-carboxylic acid hydrochloride (5 g, 29.8 mmol) in MeOH (50 mL) was added SOCl₂ (10.6 g, 89.4 mmol) slowly at 0°C. After stirring at 80 °C for 12 h, the reaction was concentrated *in vacuo* to give **methyl (2R,4S)-4-hydroxypyrrolidine-2-carboxylate intermediate 636** (4 g, crude) as a grey solid. ESI-MS [M +H]+: 146.2.

To a solution of **intermediate 636** (4 g, 27.5 mmol) in THF (80 mL) was added saturated aqueous NaHCO₃ (60 ml), followed by CbzCl (5.6 g, 33.1 mmol) at 0 °C. The reaction mixture was stirred at 0°C for another 1 h and then extracted with EtOAc (3 x 50 mL). The combined organic layers were washed with brine, dried over Na₂SO₄ and concentrated *in vacuo* to give the crude, which was purified by silica gel chromatography (EtOAc/ PE = 1/5) to give **1-benzyl 2-methyl *(2R,4s)-4-*hydroxypyrrolidine-1,2-dicarboxylate intermediate 637** (5.8 g, 75%) as yellow oil. ESI-MS [M +H]⁺: 280.1.

A mixture of **intermediate 637** (4 g, 14.3 mmol), TMSCl (2.3 g, 21.5 mmol), imidazole(1.9 g, 28.6 mmol) in DCM (40 mL) was stirred at room temperature for 12h. The reaction was washed with water (50 mL) then extracted with EtOAc (3 x 50 mL). The combined organic layers were washed with brine, dried over Na₂SO₄, filtered and concentrated *in vacuo* to give the crude, which was purified by silica gel chromatography (EtOAc/ PE = 1/3) to give **1-benzyl 2-methyl *(2R,4S)-4-((tert*butyldimethylsilyl)oxy)pyrrolidine-1,2-dicarboxylate intermediate 638** (4 g, 71%) as yellow oil. ESI-MS [M +H]⁺: 394.1.

To a solution of **intermediate 638** (4 g, 10.2 mmol) in THF (40 mL) was added LiOH (1.25 g, 30.6 mmol) and water (4 mL). The reaction mixture was stirred at room temperature for 2 h and then concentrated *in vacuo* to remove THF. The pH of the resulting residue was adjusted to 3-4 with HCl (1.0 M) then the mixture was extracted with EtOAc (3 x 50 mL). The combined organic layers were washed with brine, dried over Na₂SO₄, filtered and concentrated *in vacuo* to give the crude ***(2R,4S)-1-*((benzyloxy)carbonyl)-4-((tert-butyldimethylsilyl)oxy)pyrrolidine-2-carboxylic acid intermediate 639** (3.3 g, 87%) as yellow oil. ESI-MS [M +H]⁺: 394.1.

To a solution of **intermediate 639** (2 g, crude from previous step) in DCM (20 mL) was added DMF (2 drops) and (COCl)₂ (1.0 g, 7.91 mmol) at 0°C. After stirring at room temperature for 1 h, the solution was concentrated to give **benzyl (2R,4S)-4-((tert-butyldimethylsilyl)oxy)-2-(chlorocarbonyl)pyrrolidine-1-carboxylate intermediate 640** (2.1 g crude) as yellow oil, which was used in next step without further purification. ESI-MS [M +H]⁺: 394.1.

To a solution of **intermediate 640** (400 mg, 1 mmol) in DCM (20 mL) was added TMSCH₂N₂ (1.0 mL, 2M in hexane). After stirring at room temperature for 12 h, the solution was concentrated *in vacuo* to give the crude, which was re-dissolved in DCM (10 mL) and then HCl (4.0 M in 1,4-dioxane, 0.5 mL) was added. After stirring at room temperature for another 10min, the solution was concentrated to give the crude, which was purified by silica gel chromatography (eluent: EtOAc/PE = 1/5) to give **benzyl (2R,4S)-2-(2-chloroacetyl)-4-hydroxypyrrolidine-1-carboxylate intermediate 641** (150 mg, 36%) as yellow oil. ESI-MS [M +H]⁺: 298.2.

A solution of **intermediate 641** (150 mg, 0.51 mmol), 5-cyclopropylpyridin-2-amine (134 mg, 1.0 mmol) and DIPEA (329 mg, 2.55 mmol) in 1.4-dioxane (10 mL) was stirred at 95°C for 12h. The resulting mixture was concentrated *in vacuo* to give the crude, which was purified with silica gel chromatography (eluent: DCM/MeOH = 15/1) to give **benzyl(2R,4S)-4-((tert-butyldimethylsilyl)oxy)-2-(6-cyclopropylimidazo[1,2-a]pyridin-2-yl)pyrrolidine-1-carboxylate intermediate 642** (150 mg, 58%) as a yellow solid. ESI-MS [M +H]⁺: 492.1.

A mixture of **intermediate 642** (150 mg, 0.31 mmol) and Pd/C (20 mg) in MeOH (10 mL) was stirred at room temperature for 5h under a H₂ atmosphere. The reaction was filtered, washed with MeOH (30 mL). The filtrate was concentrated *in vacuo* to give **intermediate 635** (120 mg, crude) as a yellow solid. ESI-MS [M +H]⁺: 358.2.

### Intermediate 643: 2-((2R,4R)-4-((tert-butyldimethylsilyl)oxy)pyrrolidin-2-yl)-6-cyclopropylimidazo[1,2-a]pyridine

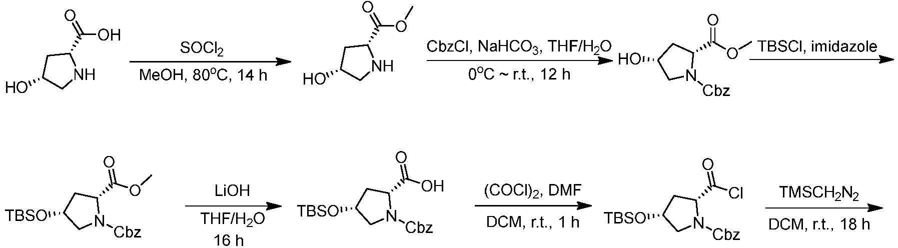

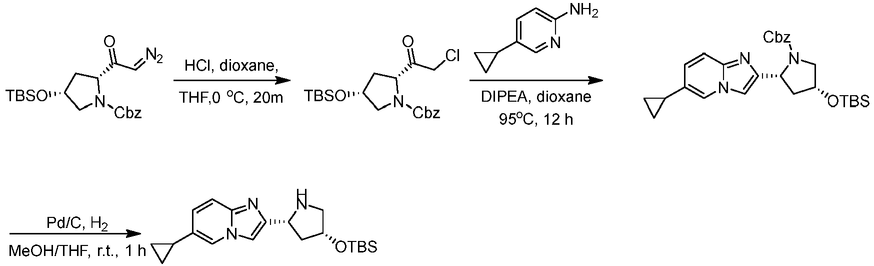

Using a similar procedure to that for **intermediate 635, intermediate 643** (30 mg) was synthesised from (2R,4R)-4-hydroxypyrrolidine-2-carboxylic acid hydrochloric acid salt. ESI-MS [M +H]+: 358.2

### Intermediate 644: (R)-6-cyclopropyl-2-(pyrrolidin-2-yl)imidazo[1,2-a]pyridine

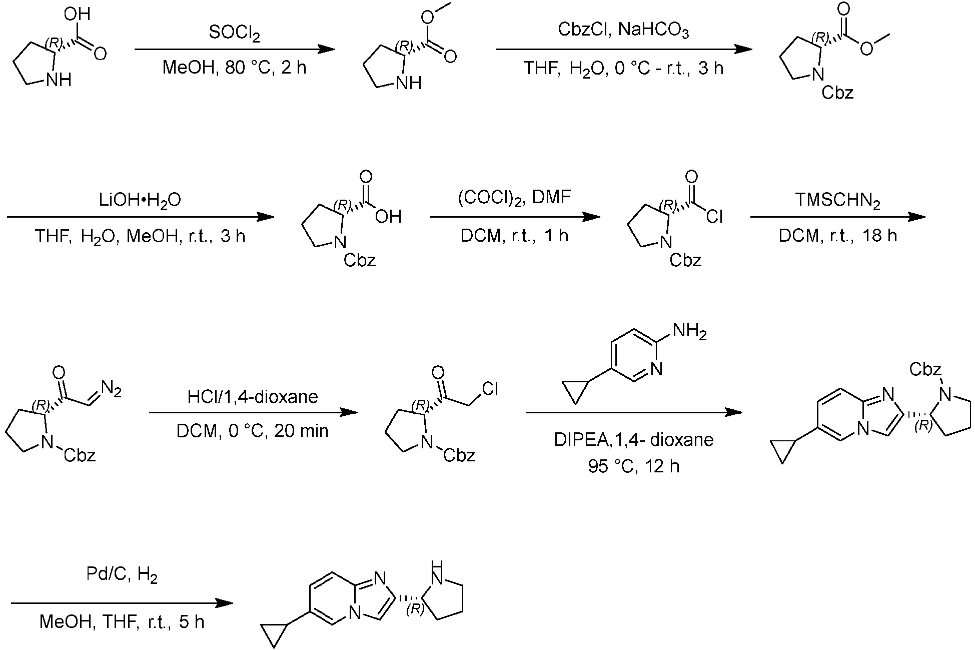

Using a similar procedure to that for **intermediate 635, (intermediate 644** (65 mg) was synthesised from D-proline. ESI-MS [M +H]+: 228.2

### Intermediate 645: 1-(2-((2R,4S)-4-((tert-butyldimethylsilyl)oxy)pyrrolidin-2-yl)-6-cyclopropylimidazo[1,2-a]pyridin-8-yl)-3-methylimidazolidine-2,4-dione

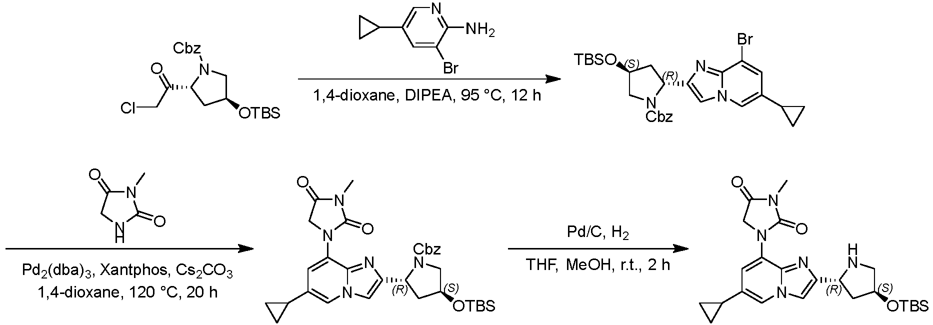

A mixture of benzyl (2R,4S)-4-((tert-butyldimethylsilyl)oxy)-2-(2-chloroacetyl)pyrrolidine-1-carboxylate (0.55 g, 1.3 mmol), **intermediate 309** (0.51 g, 2.4 mmol) and DIPEA (0.52 g, 4.0 mmol) in dioxane (5.0 mL) was stirred at 95°C for 16 h. After cooling to room temperature, the mixture was concentrated *in vacuo* to give the crude product, which was purified by column chromatography on silica gel (eluent: DCM/MeOH = 10/1) to give benzyl (2R,4S)-2-(8-bromo-6-cyclopropylimidazo[1,2-a]pyridin-2-yl)-4-((tert-butyldimethylsilyl)oxy)pyrrolidine-1-carboxylate (0.20 g, 27%) as a yellow solid. ESI-MS [M +H]+: 572.1.

To a mixture of benzyl (2R,4S)-2-(8-bromo-6-cyclopropylimidazo[1,2-a]pyridin-2-yl)-4-((tert-butyldimethylsilyl)oxy)pyrrolidine-1-carboxylate (0.30 g, 0.53 mmol), **intermediate 322** (0.12 g, 1.1 mmol), and Cs₂CO₃ (0.52 g, 1.6 mmol) in dioxane (50 mL) was added Pd₂(dba)₃ (48 mg, 0.052 mmol) and Xantphos (61 mg, 0.11 mmol). The reaction mixture was stirred at 120°C for 24 h under N₂. After cooling to room temperature, the mixture was filtered through Celite^{®} and the filter cake was washed with DCM/MeOH (10/1, 100 mL). The filtrate was concentrated *in vacuo* to give the crude, which was purified by silica gel chromatography (eluent: DCM/MeOH = 50/1 ~ 20/1) to give **benzyl (2R,4S)-4-((tert-butyldimethylsilyl)oxy)-2-(6-cyclopropyl-8-(3-methyl-2,4-dioxoimidazolidin-1-yl)imidazo[1,2-a]pyridin-2-yl)pyrrolidine-1-carboxylate** (0.31 g, 97%) as a yellow solid. ESI-MS [M +H]+: 604.1.

To a mixture of benzyl (2R,4S)-4-((tert-butyldimethylsilyl)oxy)-2-(6-cyclopropyl-8-(3-methyl-2,4-dioxoimidazolidin-1-yl)imidazo[1,2-a]pyridin-2-yl)pyrrolidine-1-carboxylate (0.31 g, 0.51 mmol) in THF (3.0 mL) and MeOH (3.0 mL) was added Pd/C (0.31 g). The mixture was stirred at room temperature for 2 h. The reaction mixture was filtered and concentrated *in vacuo* to give **intermediate 645** as a yellow solid (0.23 g, 96%), which was without further purification. ESI-MS [M +H]+: 470.1.

The compounds in Table A13 were synthesized using a similar method to **intermediate 645** starting from benzyl (2R,4S)-2-(8-bromo-6-cyclopropylimidazo[1,2-a]pyridin-2-yl)-4-((tertbutyldimethylsilyl)oxy)pyrrolidine-1-carboxylate and the appropriate coupling partner:

**Table A13**

| | | |
|---|---|---|
| 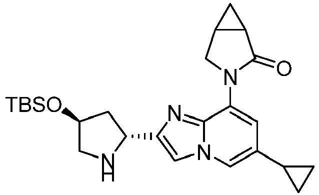 | From: 3-azabicyclo[3.1.0] hexan-2-one | 3-(2-((2RS,4RS)-4-((tert-butyldimethylsilyl)oxy)pyrrolidin e-2-yl)-6-cyclopropyl imidazo[1,2-a]pyridin-8-yl)-3-azabicyclo[3.1.0]hexan-2-one |
| | | ESI-MS (M+H)+:453.3 |
| 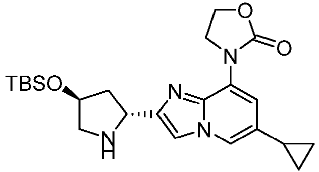 | From: oxazolidin-2-one | 3-(2-((2R,4S)-4-((tert-butyldimethylsilyl)oxy)pyrrolidin-2-yl)-6-cyclopropylimidazo[1,2-a]pyridin-8-yl)oxazolidin-2-one |
| | | ESI-MS [M +H]+: 443.2 |
| 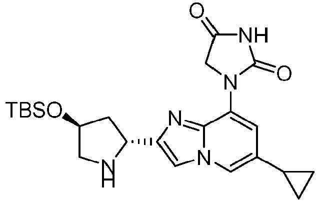 | From: imidazolidine-2,4-dione | 1-(2-((2R,4S)-4-((tert-butyldimethylsilyl)oxy)pyrrolidin-2-yl)-6-cyclopropylimidazo[1,2-a]pyridin-8-yl)imidazolidine-2,4-dione |
| | | ESI-MS [M +H]⁺: 456.2 |

### Intermediate 646: (3S,5R)-5-(6-cyclopropyl-8-(3-methyl-2,4-dioxoimidazolidin-1-yl)imidazo[1,2-a]pyridin-2-yl)pyrrolidin-3-yl propionate

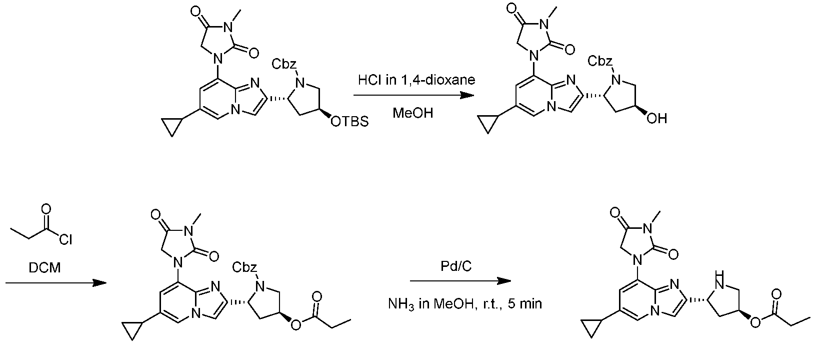

To a mixture of benzyl (2R,4S)-4-((tert-butyldimethylsilyl)oxy)-2-(6-cyclopropyl-8-(3-methyl-2,4-dioxoimidazolidin-1-yl)imidazo[1,2-a]pyridin-2-yl)pyrrolidine-1-carboxylate (210 mg, 0.35 mmol) in MeOH (2 mL) was added HCl (4M solution in dioxane, 2 mL). The mixture was stirred at 25°C for 2h then concentrated *in vacuo* to give **benzyl (2R,4S)-2-(6-cyclopropyl-8-(3-methyl-2,4-dioxoimidazolidin-1-yl)imidazo[1,2-a]pyridin-2-yl)-4-hydroxypyrrolidine-1-carboxylate intermediate 647** (170 mg, crude) as a yellow solid. ESI-MS [M +H]+: 490.2.

To a mixture of **intermediate 647** (150 mg, 0.3 mmol) in DCM (1 mL) was added propionyl chloride (1 mL) and the mixture was stirred at 25°C for 2 h under N₂. The mixture was basified with saturated aq. NaHCO₃ solution to pH 8 and then extracted with (DCM (3 x 20 mL). The combined organic layers were washed with brine (20 mL), dried over anhydrous Na₂SO₄, and concentrated *in vacuo* to give the crude product, which was purified by silica gel chromatography (eluent: PE/EtOAc = 2/1) to give **benzyl (2R,4S)-2-(6-cyclopropyl-8-(3-methyl-2,4-dioxoimidazolidin-1-yl)imidazo[1,2-a]pyridin-2-yl)-4-(propionyloxy)pyrrolidine-1-carboxylate intermediate 648** (120 mg, 72%) as a white solid. ESI-MS [M +H]⁺: 546.2.

To a mixture of intermediate 648 (100 mg, 0.18 mmol) in NH₃ (7M in MeOH, 2 mL) was added Pd/C (100 mg) and the mixture was stirred at 25°C for 5 min under H₂. The mixture was filtered and the filtrate was concentrated *in vacuo* to give **intermediate 646** (60 mg, 82%) as a white solid. ESI-MS [M +H]⁺: 412.2

### Intermediate 649: (5R)-5-(6-cyclopropylimidazo[-1,2-a]pyridin-2-yl)-3-methylpyrrolidin-3-ol

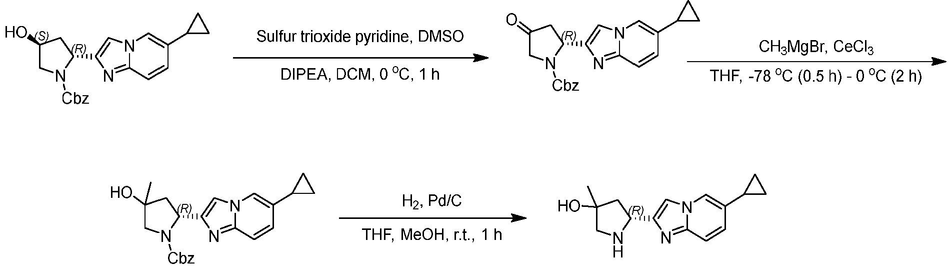

To a mixture of benzyl (2R,4S)-2-(6-cyclopropylimidazo[1,2-a]pyridin-2-yl)-4-hydroxy pyrrolidine-1-carboxylate (0.55 g, 1.5 mmol) in DCM (6 mL) was added DIPEA (0.73 mL, 4.4 mmol) dropwise at 0°C followed by sulfur trioxide pyridine (1.2 g, 7.4 mmol) in DMSO (3.0 mL) dropwise at 0°C. After stirring at 0°C for 1 h, the mixture was quenched with water (10 mL) and extracted with DCM (3 x 50 mL). The organic layers were combined and washed with brine (10 mL). The mixture was concentrated *in vacuo* and purified by column chromatography on silica gel (eluent: DCM/MeOH = 20/1) to give **benzyl (R)-2-(6-cyclopropylimidazo[1,2-a]pyridin-2-yl)-4-oxo pyrrolidine-1-carboxylate intermediate 650** (0.52 mg, 95%) as a yellow oil. ESI-MS [M + H ]⁺: 376.2.

A mixture of CeCl₃ (0.80 g, 3.2 mmol) in THF (12 mL) was added CH₃MgBr (3.4 mL, 10 mmol, 3 mol/L in diethyl ether) at -78°C and stirred for 0.5 h. Then to the resulting the mixture was added **intermediate 650** (0.48 g, 1.3 mmol) in THF (3.0 mL) at -78°C. After stirring at 0°C for 2 h, the mixture was quenched with a solution of saturated aq. NH₄Cl (10 mL) and extracted with EtOAc (3 x 60 mL). The organic layers were combined and washed with brine (10 mL) and concentrated *in vacuo.* The residue was purified by column chromatography on silica gel (eluent: DCM/MeOH=20/1) to give **benzyl (2R)-2-(6-cyclopropylimidazo[1,2-a]pyridin-2-yl)-4-hydroxy-4-methylpyrrolidine-1-carboxylate intermediate 651** (0.50 g, 98%) as a yellow oil. ESI-MS [M + H ]⁺: 392.2.

A mixture of **intermediate 651** (0.50 g, 1.3 mmol) in THF (2.5 mL) and MeOH (2.5 mL) was added 10%Pd on carbon (0.50 g) at room temperature. The mixture was stirred at room temperature under and atmosphere of H₂ for 1 h. The mixture was filtered and concentrated *in vacuo* to give **intermediate 649** (0.14 g, crude) as a yellow oil. ESI-MS [M + H ]⁺: 258.2.

### Intermediate 652: ((3S,5R)-5-(6-cyclopropylimidazo[1,2-a]pyridin-2-yl)pyrrolidin-3-yl)methanol

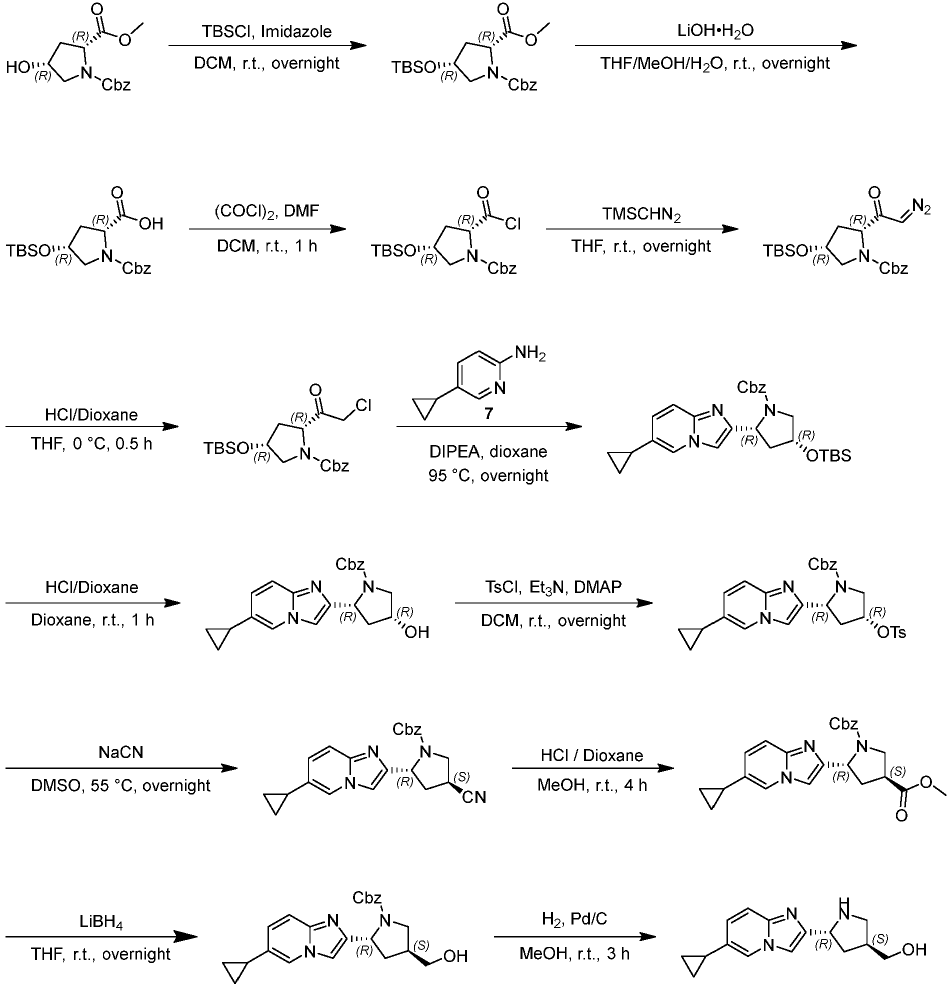

Using a similar procedure to that for **intermediate 642,** benzyl (2R,4R)-4-((tert-butyl dimethylsilyl)oxy)-2-(6-cyclopropylimidazo[1,2-a]pyridin-2-yl)pyrrolidine-1-carboxylate **intermediate 653** (1.1 g) was synthesized from **intermediate 661** (5.0 g, 18 mmol).

To a mixture of **intermediate 653** (1.1 g, 2.2 mmol) in 1,4-dioxane (10 mL) was added HCl (10 mL, 40 mmol, 4 mol/L in 1,4-dioxane) at room temperature. After stirring at room temperature for 1 h, the mixture was concentrated *in vacuo* and the residue was basified with saturated aq. NaHCO₃ (30 mL) to pH = 8. The mixture was extracted with DCM (3 x 60 mL). The organic layers were combined and concentrated *in vacuo* to give **benzyl (2R,4R)-2-(6-cyclopropylimidazo[1,2-a]pyridin-2-yl)-4-hydroxypyrrolidine-1-carboxylate intermediate 654** (0.91 g, crude) as a brown oil, which was used without purification. ESI-MS [M + H ]⁺: 378.2.

To a mixture of **intermediate 654** (0.91 g, 2.4 mmol), TsCl (1.4 g, 7.2 mmol) and Et₃N (1.0 mL, 7.2 mmol) in DCM (10 mL) was added DMAP (0.15 g, 1.2 mmol) at room temperature. After stirring at room temperature overnight, the mixture was quenched with water (20 mL) and extracted with DCM (3 x 60 mL). The combined organic layers were concentrated *in vacuo* and purified by column chromatography on silica gel (eluent: DCM/MeOH = 10/1) to give **benzyl (2R,4R)-2-(6-cyclopropylimidazo[1,2-a]pyridin-2-yl)-4-(tosyloxy)pyrrolidine-1-carboxylate intermediate 655** (0.78 g, 61%) as a yellow oil. ESI-MS [M + H ]⁺: 532.2.

A mixture of **intermediate 655** (0.78 g, 1.5 mmol) in DMSO (10 mL) was added NaCN (0.29 g, 5.9 mmol) at room temperature. After stirring at 55°C overnight, the mixture was washed with water (30 mL) and extracted with EtOAc (3 x 60 mL). The organic layers were combined and purified by column chromatography on silica gel (eluent: DCM/MeOH = 15/1) to give **benzyl (2R,4S)-4-cyano-2-(6-cyclopropylimidazo[1,2-a]pyridin-2-yl)pyrrolidine-1-carboxylate intermediate 656** (0.52 g, 91%) as an orange oil. ESI-MS [M + H ]⁺: 387.2.

A mixture **ofintermediate 656** (0.35 g, 0.90 mmol) in MeOH (4.0 mL) was added HCl (7.0 mL, 28 mmol, 4 M in 1,4-dioxane) was stirred at room temperature for 4 h. The mixture was concentrated *in vacuo* and then the residue was diluted with saturated aq. NaHCO₃ (10 mL). The aqueous was extracted with EtOAc (3 x 30 mL). The combined organic layers were concentrated *in vacuo* and purified by preparative TLC (eluent: DCM/MeOH = 40/1) to give **1-benzyl 3-methyl (3S,5R)-5-(6-cyclopropylimidazo[1,2-a]pyridin-2-yl)pyrrolidine-1,3-dicarboxylate intermediate 657** (0.21 g, 55%) as a yellow oil. ESI-MS [M + H ]⁺: 420.2.

To a mixture of **intermediate 657** (0.13 g, 0.31 mmol) in THF (3.0 mL) was added LiBH₄ (14 mg, 0.62 mmol) at 0°C. After stirring at room temperature overnight, the mixture was quenched with saturated aq. NH₄Cl (30 mL) and extracted with EtOAc (3 x 60 mL). The organic layers were combined and concentrated *in vacuo* to give **benzyl(2R,4S)-2-(6-cyclopropylimidazo[1,2-a]pyridin-2-yl)-4-(hydroxymethyl)pyrrolidine-1-carboxylate intermediate 658** (69 mg, crude) as a white solid, which was used without purification. ESI-MS [M + H ]⁺: 392.2.

To a mixture of **intermediate 658** (69 mg, 0.18 mmol) in MeOH (2.0 mL) was added 10% Pd on carbon (70 mg) at room temperature. The mixture was stirred for 3 h under an atmosphere of H₂. The mixture was filtered and concentrated *in vacuo* to give **intermediate 652** (37 mg, crude) as a yellow oil, which was used without purification. ESI-MS [M + H ]⁺: 258.2.

### Intermediate 659: 2-((2S,3S,4R)-3,4-bis((tert-butyldimethylsilyl)oxy)pyrrolidin-2-yl)-6-cyclopropylimidazo[1,2-a]pyridine

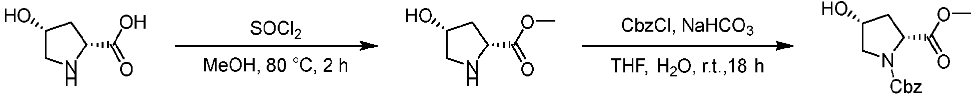

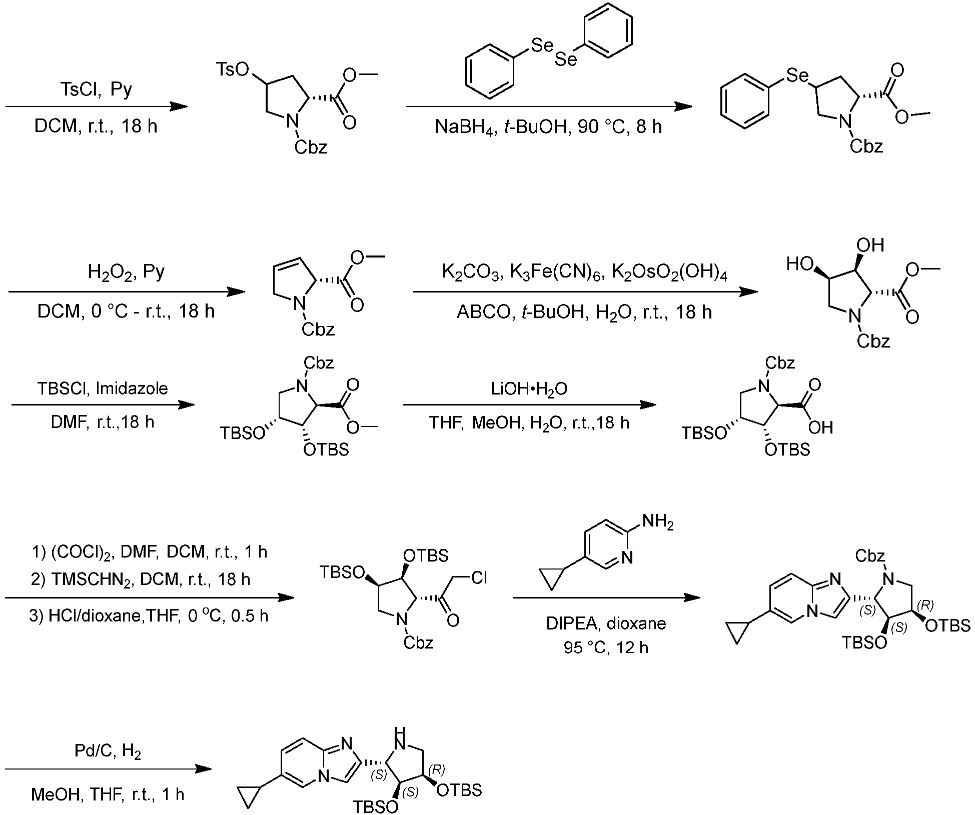

To a mixture of (2R,4R)-4-hydroxypyrrolidine-2-carboxylic acid (25 g, 0.19 mol) in MeOH (0.25 L) was added SOCl₂ (25 mL) at 0°C. The reaction mixture was then warmed to 80°C and stirred for 2 h. After cooling to room temperature, the mixture was concentrated *in vacuo* to afford **methyl (2R,4R)-4-hydroxypyrrolidine-2-carboxylate intermediate 660** (35 g, crude) as a white solid, which was used without further purification. ESI-MS [M +H] +: 146.1

To a mixture of **intermeidate 660** (35 g, 0.2 mol) and NaHCO₃ (48 g, 0.57 mol) in THF/H₂O (0.60 L/0.40 L) was added CbzCl (49 g, 0.29 mol) at 0°C. The mixture was then warmed to room temperature and stirred for 18 h. The resulting solution was extracted with EtOAc (3 x 0.30 L). The combined organic layers were washed with brine (0.30 L), dried over Na₂SO₄ and concentrated *in vacuo.* The residue was purified by column chromatography on silica gel (eluent: EtOAc/PE = 0 - 100%) to afford **1-benzyl 2-methyl (2R,4R)-4-hydroxypyrrolidine-1,2-dicarboxylate intermediate 661** (50 g, 94% for two steps) as a white solid. ESI-MS [M +H] +: 280.1

A mixture of **intermediate 661** (15 g, 54 mmol), TsCl (46 g, 81 mmol) and DMAP (20 g, 0.16 mol) in DCM (0.20 L) was stirred at room temperature for 18 h. The mixture was quenched with water (0.50 L), and then extracted with DCM (3 x 0.30 L). The combined organic layers were washed with brine (0.20 L), dried over Na₂SO₄ and concentrated *in vacuo.* The residue was purified by column chromatography on silica gel (eluent: EtOAc/PE = 0 - 30%) to afford **1-benzyl 2-methyl (2R)-4-(tosyloxy)pyrrolidine-1,2-dicarboxylate intermediate 662** (19 g, 82 %) as a white solid. ESI-MS [M +H] +: 434.1

To a mixture of 1,2-diphenyldiselane (2.6 g, 8.3 mmol) in t-BuOH (0.10 L) was added NaBH₄ (0.63 g, 17 mmol) and **intermediate 662** (6.0 g, 14 mmol) and the mixture was stirred at 90°C for 8 h. The mixture was quenched with water (0.20 L), filtered then extracted with DCM (3 x 0.10 L). The combined organic layers were washed with brine (0.10 L), dried over Na₂SO₄ and concentrated *in vacuo.* The residue was purified by column chromatography on silica gel (eluent: EtOAc/PE = 0 - 30%) to afford **1-benzyl 2-methyl (2R)-4-(phenylselanyl)pyrrolidine-1,2-dicarboxylate intermediate 663** (4 g, 68 %) as a colorless oil. ESI-MS [M +H] +: 420.1

To the mixture of **intermediate 663** (2.1 g, 5.0 mmol) and pyridine (0.56 mL, 7.0 mmol) in DCM (30 mL) was added H₂O₂ (1.4 mL, 13 mmol, 30% in water) at 0°C and the mixture was stirred at room temperature for 18 h. The mixture was quenched with saturated aq. Na₂SO₃ (30 mL) and extracted with DCM (3 x 50 mL). The combined organic layers were washed with brine (30 mL), dried over Na₂SO₄ and concentrated *in vacuo.* The residue was purified by column chromatography on silica gel (eluent: EtOAc/PE = 0 - 30%) to afford **1-benzyl 2-methyl (R)-2,5-dihydro-1H-pyrrole-1,2-dicarboxylate intermediate 664** (1.0 g, 77 %) as a colorless oil. ESI-MS [M +H] +: 262.1

To the mixture of **intermediate 664** (6.5 g, 25 mmol), K₃Fe(CN)₆ (25 g, 75 mmol), 1-azabicyclo[2.2.2]octane (0.13 g, 1.2 mmol) and K₂CO₃ (10 g, 75 mmol) in t-BuOH/ H₂O (80 mL/80 mL) was added K₂OsO₂(OH)₄ (1.1 g, 1.2 mmol). The mixture was stirred at room temperature for 18 h. then quenched with saturated aq. Na₂SO₃ (30 mL) and extracted with EA (3 x 200 mL). The combined organic layers were washed with brine (200 mL), dried over Na₂SO₄ and concentrated *in vacuo.* The residue was purified by column chromatography on silica gel (eluent: MeOH/DCM = 0 - 5%) to afford 1-benzyl **2-methyl (2R,3S,4R)-3,4-dihydroxypyrrolidine-1,2-dicarboxylate intermediate 665** (5.5 g, 75 %) as a colorless oil. ESI-MS [M +H] +: 296.1.

To a mixture of **intermediate 665** (5.2 g, 18 mmol) and imidazole (7.2 g, 0.11 mol) in DMF (50 mL) was added TBSCl (7.9 g, 53 mmol). The mixture was stirred at room temperature for 18 h then quenched with water (500 mL), and then extracted with EA (3 x 300 mL). The combined organic layers were washed with brine (200 mL), dried over Na₂SO₄ and concentrated *in vacuo.* The residue was purified by column chromatography on silica gel (eluent: EtOAc/PE = 0 - 15%) to afford **1-benzyl** 2-**methyl (2R,3S,4R)-3,4-bis((tert-butyldimethylsilyl)oxy)pyrrolidine-1,2-dicarboxylate intermediate 667** (8.2 g, 89 %) as a colorless oil. ESI-MS [M +H] +: 524.3

Using a similar procedure to that for **intermediate 635, intermediate 659** (0.10 g) was synthesised from **intermediate 667.** ESI-MS [M +H]+: 488.3

### Intermediate 668: 2-((2R,4S)-4-((tert-butyldimethylsilyl)oxy)pyrrolidin-2-yl)-6-(2-methylcyclo propyl)imidazo[1,2-a]pyridine

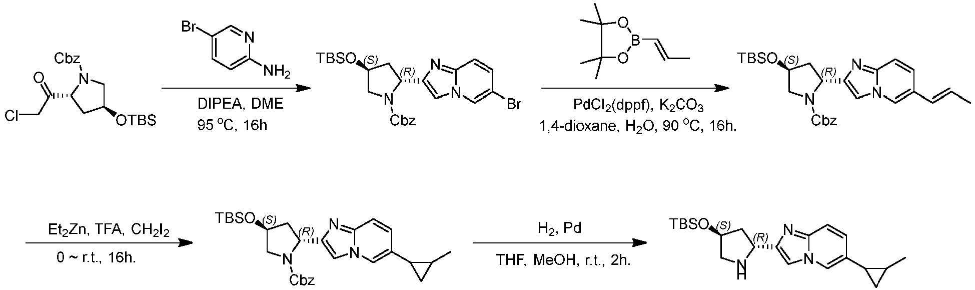

A mixture of benzyl (2R,4S)-4-((tert-butyldimethylsilyl)oxy)-2-(2-chloroacetyl)pyrrolidine-1-carboxylate (1.0 g, 2.4 mmol), 5-bromopyridin-2-amine (0.83 g, 4.8 mmol) and DIPEA (0.93 g, 7.2 mmol) in DME (30 mL) was stirred at 95°C for 16h. The resulting mixture was concentrated *in vacuo* and then extracted with DCM (3 x 50 mL). The combined organic layers were concentrated *in vacuo* to get the crude, which was purified by column chromatography (eluent: EA/PE = 1/2) to give **benzyl (2R,4S)-2-(6-bromoimidazo[1,2-a]pyridin-2-yl)-4-((tert-butyldimethylsilyl)oxy)pyrrolidine-1-carboxylate intermediate 669** (0.20 g, 16%) as a yellow oil. ESI-MS [M +H]+: 530.2.

A mixture of **intermediate 669** (0.50 g, 0.94 mmol), PdCl₂(dppf) (0.14 g, 0.19 mmol), (E)-4,4,5,5-tetramethyl-2-(prop-1-en-1-yl)-1,3,2-dioxaborolane (0.47 g, 2.8 mmol) and K₂CO₃ (0.39 g, 2.8 mmol) in s solution of 1,4-dioxane (10 mL) and water (2.0 mL) was stirred at 95°C under N₂ for 16 h. The reaction mixture was quenched with water (50 mL) and extracted with EtOAc (5 x 70 mL). The combined organic layers were washed with brine, dried over Na₂SO₄, and concentrated *in vacuo* to give the crude, which was purified with column chromatography (eluent: PE/EtOAc = 1/1) to give **benzyl (2R,4S)-4-((tert-butyldimethylsilyl)oxy)-2-(6-((E)-prop-1-en-1-yl)imidazo[1,2-a]pyridin-2-yl) pyrrolidine-1-carboxylate intermediate 670** (0.33 g, 71%) as a white solid. ESI-MS [M +H]+: 492.2.

To a solution of DCM (0.5 mL) was added Et₂Zn (0.92 mL, 1.83 mmol, 2.0 M in hexanes) under N₂ atmosphere. The solution was cooled in an ice bath and a solution of trifluoroacetic acid (209 mg, 1.83 mmol) in DCM (1.0 mL) was then dripped very slowly into the reaction mixture via syringe. Upon stirring for 10 min, a solution of CH₂l₂ (490 mg, 1.83 mmol) in DCM (1.0 mL) was added. After an additional 20 min of stirring, a solution of **intermediate 670** (90 mg, 0.1 mmol) in DCM (1.0 mL) was added, and the ice bath was removed. After the reaction mixture was stirred at room temperature for 16h, the mixture was quenched with saturated aq. NH₄Cl (10 mL) and the phases were separated. The aqueous layer was extracted with DCM (2 x 10 mL). The combined organic layers were concentrated *in vacuo* and purified by Prep-HPLC (eluent: DCM/MeOH = 10/1) to give benzyl **(2R,4S)-4-((tertbutyldimethylsilyl)oxy)-2-(6-(2-methylcyclopropyl)imidazo[1,2-a]pyridin-2-yl)pyrrolidine-1-carboxylate intermediate 671** (10 mg, 11%) as a colourless oil. ESI-MS [M +H]+: 506.2.

To a mixture of **intermediate 671** (10 mg, 0.02 mmol) in THF (1 mL) and MeOH (1 mL) was added Pd/C (10 mg). the mixture was stirred at room temperature under the H₂ for 2 h. The reaction mixture was filtered through Celite^{®} and the filter cake was washed with DCM/MeOH (10/1, 10 mL). The filtrate was concentrated *in vacuo* to give **intermediate 668** (6 mg, 81%) as a yellow oil. ESI-MS [M +H]+: 372.2.

### Intermediate 672: 6-cyclopropyl-2-((2R,3S)-3-((triisopropylsilyl)oxy)pyrrolidin-2-yl)imidazo[1,2-a]pyridine

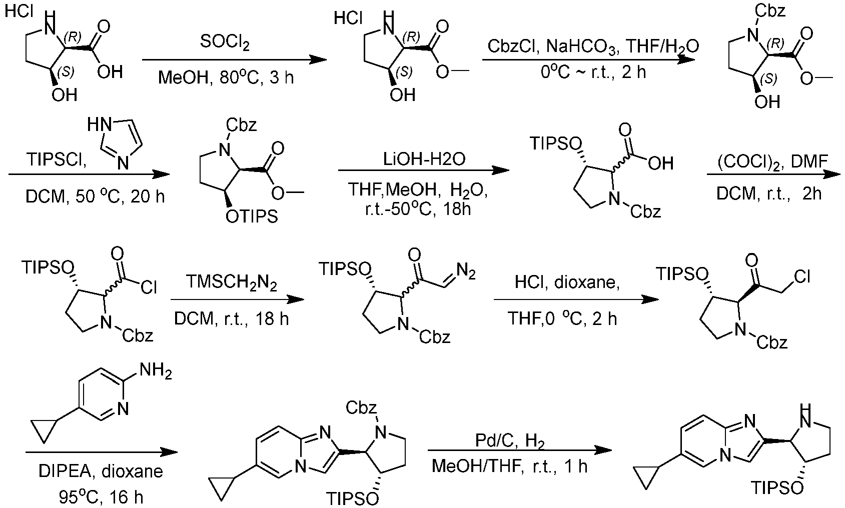

Using a similar procedure to that for **intermediate 635, intermediate 672 (35** mg) was synthesised from (2R,3S)-3-hydroxypyrrolidine-2-carboxylic acid hydrochloric acid salt. TIPSCl was used in place of TBSCl. ESI-MS [M + H ]⁺:400.3

### Intermediate 673: 6-cyclopropyl-2-((2S,3R)-3-((triisopropylsilyl)oxy)pyrrolidin-2-yl)imidazo[1,2-a]pyridine

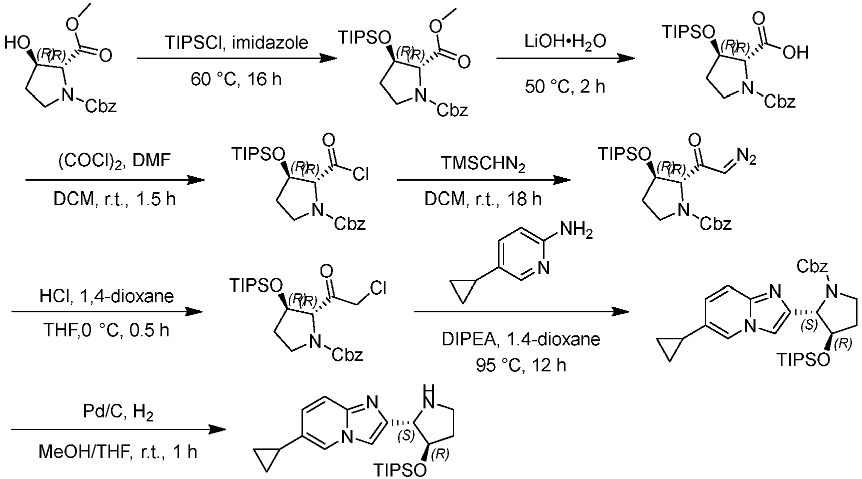

To a mixture of 1-benzyl 2-methyl (2R,3R)-3-hydroxypyrrolidine-1,2-dicarboxylate (2.0 g, 7.2 mmol) in DCM (40 mL) was added imidazole (3.4 g, 50 mmol) and TIPSCl (6.9 g, 36 mmol) at 0°C. The resulting reaction mixture was stirred at 60°C for 16 h under N₂ then cooled to room temperature. The reaction mixture was concentrated *in vacuo in vacuo* to give the crude. The residue was purified by column chromatography on silica gel (eluent: EtOAc/PE = 0% - 50%) to afford 1-benzyl **2-methyl (2R,3R)-3-((triisopropylsilyl)oxy) pyrrolidine-1,2-dicarboxylate intermediate 674** (1.6 g, 50 %) as yellow oil. ESI-MS [M + H ]⁺: 436.2.

To a mixture of **1intermediate 674** (1.6 g, 3.6 mmol) in MeOH (10 mL), THF (10 mL) and water (5 mL) was added LiOH•H₂O (1.1 g, 25 mmol) at room temperature. The resulting reaction mixture was stirred at 50°C for 2 h under N₂. The reaction mixture was acidified (pH = 4) with 1 N aq. HCl, diluted with H₂O(40 mL), then extracted with DCM (3 x 40 mL).The combined organics were concentrated *in vacuo* to afford **(2R,3R)-1-((benzyloxy y)carbonyl)-3-((triisopropylsilyl)oxy)pyrrolidine-2-carboxylic acid intermediate 675** (1.5 g, quant) as yellow oil. MS [M + H ]+: 444.2.

Using a similar procedure to that for **intermediate 635, intermediate 673** (65 mg) was synthesised from ((2R,3R)-1-((benzyloxy y)carbonyl)-3-((triisopropylsilyl)oxy)pyrrolidine-2-carboxylic acid. ESI-MS [M + H ]⁺:400.3

The present specification has been amended to remove duplication and refer to the intermediates and examples by their given number. Any errors in the amendments may be corrected as per the original specification as published in WO2022/197758.
All NMR is ¹H NMR (400 MHz, DMSO) unless indicated otherwise.

### Example 1: (1S,2S)-N-(6-(((8-(4-acetylpiperazin-1-yl)-6-cyclopropylimidazo[1,2-a]pyridin-2-yl)methyl)amino)pyrimidin-4-yl)-2-(3-chlorophenyl)cyclopropane-1-carboxamide (I-1)

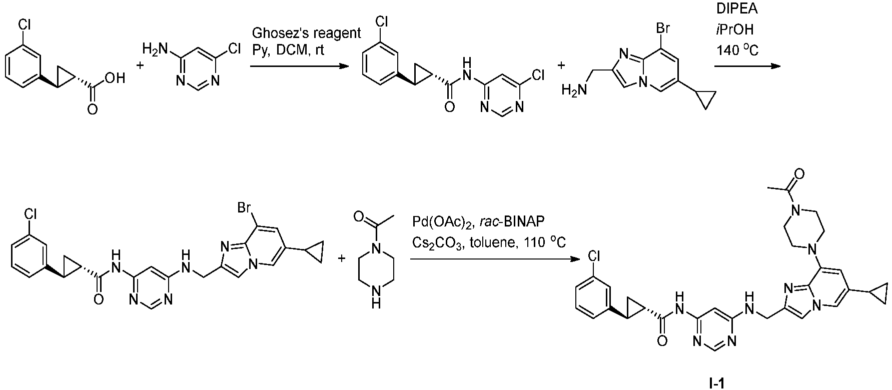

Ghosez's reagent (0.81 mL, 6.1 mmol) was added to a stirred solution of (1S,2S)-2-(3-chlorophenyl)cyclopropane-1-carboxylic acid (1.0 g, 5.1 mmol) in DCM (20 mL). The reaction was stirred at room temperature for 1.5 h, then **intermediate 846** (730 mg, 5.6 mmol) and pyridine (0.62 mL, 7.6 mmol) were added and the reaction was stirred at room temperature for 18 h. The reaction was diluted with DCM (60 mL) and NaHCO3 (sat. aq., 60 mL). The layers were separated and the aqueous layer was further extracted with DCM (2 x 30 mL). The combined organics were dried over MgSO₄ then concentrated in vacuo. The residue was purified by column chromatography on silica gel, eluting with a gradient of 0-100 % EtOAc in cyclohexane to give ***(1S,2S)-2-(3-chlorophenyl)-N-(6-chloropyrimidin-4-yl)cyclopropane-1-carboxamide* intermediate 676** (1.1 g, 70 %). ESI-MS (M+H)+: 308.1, ¹H NMR (400 MHz, CDCl₃) δ 8.63 (s, 1H), 8.24 (s, 2H), 7.24 - 7.21 (m, 2H), 7.09 (s, 1H), 7.04 - 7.00 (m, 1H), 2.66 - 2.60 (m, 1H), 1.82 - 1.77 (m, 2H), 1.51 - 1.45 (m, 1H). **Intermediate 676** (1.0 g, 3.3 mmol), **intermediate 359** (950 mg, 3.6 mmol) and DIPEA (1.1 mL, 6.5 mmol) were suspended in iPrOH (20 mL). The reaction mixture was heated in a microwave at 140 °C for 4 h. The reaction mixture was concentrated in vacuo. The residue was purified by column chromatography on silica gel, eluting with a gradient of 0-20 % (7 N NH₃ in MeOH) in DCM to give **(1S,2S)-N-(6-(((8-bromo-6-cyclopropylimidazo[1,2-a]pyridin-2-yl)methyl)amino)pyrimidin-4-yl)-2-(3-chlorophenyl)cyclopropane-1-carboxamide intermediate 677** (1.3 g, 75 %). ESI-MS (M+H)+: 537.3, 539.3, ¹H NMR (400 MHz, CDCl₃) δ 8.69 - 8.62 (m, 1H), 8.25 (s, 1H), 7.82 (s, 1H), 7.53 (s, 1H), 7.31 (s, 1H), 7.21 - 7.17 (m, 3H), 7.05 (d, J=2.0 Hz, 1H), 6.99 - 6.95 (m, 1H), 5.87 - 5.86 (m, 1H), 4.71 - 4.70 (m, 2H), 2.56 (ddd, J=4.0, 6.5, 9.2 Hz, 1H), 1.91 - 1.85 (m, 1H), 1.82 - 1.69 (m, 2H), 1.75 (s, 2H), 1.36 (ddd, J=4.7, 6.6, 8.2 Hz, 1H), 1.00 - 0.94 (m, 2H), 0.69 - 0.64 (m, 2H).

**Intermediate 677** (60 mg, 0.11 mmol), 1-acetylpiperazine (21 mg, 0.17 mmol), rac-BINAP (14 mg, 0.022 mmol), and Cs₂CO₃ (73 mg, 0.22 mmol) were suspended in toluene (4.0 mL) and * degassed with nitrogen for 5 min. Pd(OAc)₂ (2.5 mg, 0.011 mmol) was then added and the reaction was heated at 110 °C under a nitrogen atmosphere for 18 h. The reaction was cooled to room temperature, filtered through Celite^{®}, and washed with DCM:MeOH (2:1, 10 mL). The filtrate was concentrated in vacuo. The residue was purified by reverse phase preparative HPLC to give (I-1) (8.5 mg, 13 %). ESI-MS (M+H)+: 585.3, ¹H NMR (400 MHz, DMSO) δ 10.59 (s, 1H), 8.19 (s, 1H), 7.90 - 7.89 (m, 1H), 7.84 (s, 1H), 7.53 (br s, 1H), 7.34 - 7.25 (m, 4H), 7.16 - 7.13 (m, 1H), 6.18 (d, J=1.2 Hz, 1H), 4.54 (br s, 2H), 3.62 - 3.60 (m, 4H), 3.51 (d, J=4.8 Hz, 2H), 3.41 - 3.36 (m, 2H), 2.42 - 2.31 (m, 2H), 2.05 (s, 3H), 1.88 - 1.81 (m, 1H), 1.49 - 1.37 (m, 2H), 0.89 - 0.84 (m, 2H), 0.68 - 0.63 (m, 2H).

Using a similar procedure to that used for (I-1), the compounds in Table 1 were prepared from **Intermediate 677** and an appropriate coupling partner. **Table 1**

| **EG** | **Compound** | **Coupling Partner /Analytical Data** |
|---|---|---|
| **1** | 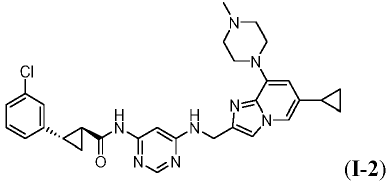 (1*S*,2*S*)-2-(3-chlorophenyl)-*N*-(6-(((6-cyclo propyl-8-(4-methylpiperazin-1-yl)imidazo [1,2-a]pyridin-2-yl)methyl)amino) pyrimidin-4-yl)cyclopropane-1-carboxamide | 1-methylpiperazine |
| | | ESI-MS (M+H)+: 557.5, δ 10.64 (1H, s), 8.24 (1H, s), 7.91 (2H, s), 7.56 (1H, s), 7.40 - 7.30 (4H, m), 7.20 (1H, d, J=7.6 Hz), 6.19 (1H, s), 4.62 - 4.62 (2H, m), 3.52 (4H, s), 3.37 (4H, s), 2.48 - 2.38 (2H, m), 2.29 (3H, s), 1.94 - 1.86 (1H, m), 1.55 - 1.42 (2H, m), 0.94 - 0.88 (2H, m), 0.73 - 0.68 (2H, m); |
| **2** | 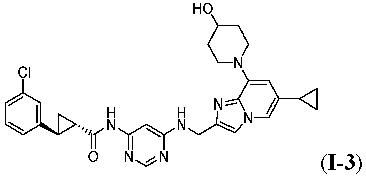 (1*S*,2*S*)-2-(3-chlorophenyl)-*N*-(6-(((6-cyclopropyl-8-(4-hydroxypiperidin-1-yl) imidazo[1,2-a]pyridin-2-yl)methyl)amino) pyrimidin-4-yl)cyclopropane-1-carboxamide | piperidin-4-ol ESI-MS (M+H)+: 558.5, δ 10.68 (s, 1H), 8.24 (s, 1H), 8.01 (s, 2H), 7.71 (s, 1H), 7.36 - 7.30 (m, 2H), 7.28 (d, J=6.3 Hz, 2H), 7.17 (d, J=7.7 Hz, 1H), 4.65 - 4.65 (m, 3H), 3.73 - 3.66 (m, 2H), 2.96 (dd, J=9.7, 9.7 Hz, 2H), 2.46 - 2.34 (m, 2H), 1.93 - 1.90 (m, 3H), 1.61 (d, J=9.5 Hz, 2H), 1.52 - 1.39 (m, 2H), 0.92 (d, J=6.5 Hz, 2H), 0.71 (d, J=4.1 Hz, 2H). |
| **3** | 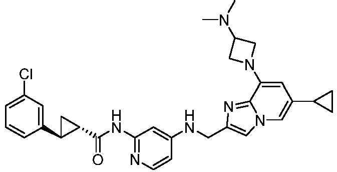 (1*S*,2*S*)-2-(3-chlorophenyl)-*N*-(4-(((6-cyclopropyl-8-(3-(dimethylamino)azetidin-1-yl)imidazo[1,2-*a*]pyridin-2-yl)methyl) amino)pyridin-2-yl)cyclopropane-1-carboxamide **(I-4)** | *N*,*N*-dimethylazetidin-3-amine |
| | | ESI-MS (M+H)+: 556.5, δ 10.38 - 10.37 (m, 1H), 7.77 (d, J=5.9 Hz, 1H), 7.70 (s, 1H), 7.53 (s, 1H), 7.42 (s, 1H), 7.36 - 7.30 (m, 1H), 7.26 (d, J=6.9 Hz, 2H), 7.18 - 7.12 (m, 2H), 6.35 (dd, J=2.1, 5.8 Hz, 1H), 5.67 (s, 1H), 4.33 (d, J=5.8 Hz, 2H), 4.24 (dd, J=7.6, 7.6 Hz, 2H), 3.88 (dd, J=6.3, 6.3 Hz, 2H), 2.42 - 2.33 (m, 2H), 2.18 (s, 6H), 1.86 - 1.78 (m, 1H), 1.49 - 1.33 (m, 2H), 0.89 - 0.83 (m, 2H), 0.67 - 0.61 (m, 2H). |
| **4** | 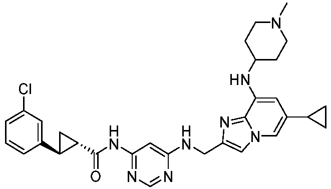 (1*S*,2*S*)-2-(3-chlorophenyl)-*N*-(6-(((6-cyclopropyl-8-((1-methylpiperidin-4-yl)amino)imidazo[1,2-*a*]pyridin-2-yl) methyl)amino)pyrimidin-4-yl)cyclopropane-1-carboxamide, formic acid salt **(I-5)** | 1-methylpiperidin-4-amine |
| | | ESI-MS (M+H)+: 571.6, δ 10.64 (s, 1H), 8.35 (s, 1H), 8.24 (s, 1H), 7.84 (s, 1H), 7.59 (s, 1H), 7.53 (s, 1H), 7.40 - 7.29 (m, 4H), 7.22 - 7.19 (m, 1H), 6.68 (s, 1H), 5.36 (d, J=8.3 Hz, 1H), 4.58 (s, 2H), 3.50 - 3.43 (m, 1H), 2.80 - 2.71 (m, 2H), 2.49 - 2.37 (m, 2H), 2.24 (s, 3H), 2.15 - 2.08 (m, 2H), 1.97 - 1.83 (m, 3H), 1.66 - 1.42 (m, 4H), 0.92 - 0.86 (m, 2H), 0.70 - 0.65 (m, 2H). |
| 5 | 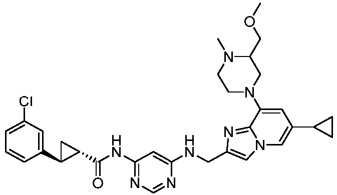 (1*S*,2*S*)-2-(3-chlorophenyl)-*N*-(6-(((6-cyclopropyl-8-(3-(methoxymethyl)-4-methylpiperazin-1-yl)imidazo[1,2-a]pyridin-2-yl)methyl)amino)pyrimidin-4-yl)cyclopropane-1-carboxamide (I-6) | 2-(methoxymethyl)-1-methylpiperazine |
| | | ESI-MS (M+H)+: 601.5, δ 10.63 (s, 1H), 8.24 (s, 1H), 7.92 - 7.86 (m, 2H), 7.57 (s, 1H), 7.39 - 7.34 (m, 1H), 7.34 - 7.29 (m, 3H), 7.20 (d, J=7.8 Hz, 1H), 6.20 (d, J=1.0 Hz, 1H), 4.61 - 4.56 (m, 2H), 4.31 - 4.26 (m, 1H), 4.06 - 4.03 (m, 1H), 3.59 (dd, J=4.2, 10.0 Hz, 1H), 3.44 - 3.39 (m, 1H), 3.33 (s, 3H), 2.91 - 2.84 (m, 2H), 2.73 - 2.64 (m, 1H), 2.46 - 2.38 (m, 4H), 2.33 (s, 3H), 1.95 - 1.87 (m, 1H), 1.55 - 1.42 (m, 2H), 0.94 - 0.89 (m, 2H), 0.72 - 0.67 (m, 2H). |
| 6 | 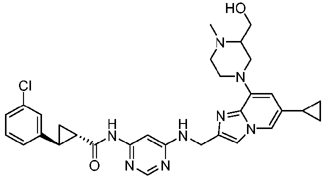 (1*S*,2*S*)-2-(3-chlorophenyl)-*N*-(6-(((6-cyclo propyl-8-(3-(hydroxymethyl)-4-methyl piperazin-1-yl)imidazo[1,2-*a*]pyridin-2-yl)methyl)amino)pyrimidin-4-yl)cyclo propane-1-carboxamide, formic acid salt **(I-7)** | (1-methylpiperazin-2-yl)methanol |
| | | ESI-MS (M+H)+: 587.4, δ 10.61 (s, 1H), 8.35 (s, 1H), 8.21 (s, 1H), 7.91 - 7.80 (m, 2H), 7.53 (s, 1H), 7.36 - 7.25 (m, 4H), 7.16 (d, J=7.8 Hz, 1H), 6.17 (s, 1H), 4.55 (s, 2H), 4.24 (s, 1H), 3.83 (d, J=11.6 Hz, 1H), 3.66 (dd, J=3.9, 11.2 Hz, 1H), 3.54 - 3.49 (m, 1H), 2.86 - 2.80 (m, 3H), 2.43 - 2.34 (m, 4H), 2.31 - 2.26 (m, 4H), 1.91 - 1.83 (m, 1H), 1.51 - 1.39 (m, 2H), 0.91-0.85 (m, 2H), 0.68-0.64 (m,2H). |
| 7 | 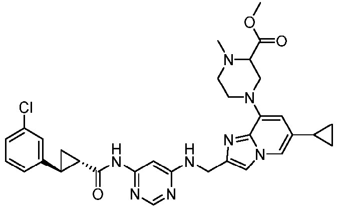 methyl4-(2-(((6-((1*S*,2*S*)-2-(3-chloro phenyl)cyclopropane-1-carboxamido) pyrimidin-4-yl)amino)methyl)-6-cyclo propylimidazo[1,2-*a*]pyridin-8-yl)-1-methylpiperazine-2-carboxylate **(I-8)** | methyl 1-methylpiperazine-2-carboxylate |
| | | ESI-MS (M+H)+: 615.4, δ 10.64 (s, 1H), 8.25 (s, 1H), 7.93 (s, 1H), 7.89 (s, 1H), 7.57 (s, 1H), 7.40 - 7.34 (m, 1H), 7.32 - 7.29 (m, 3H), 7.20 (d, J=7.8 Hz, 1H), 6.24 (d, J=1.3 Hz, 1H), 4.58 (s, 2H), 3.99 - 3.96 (m, 1H), 3.82 (s, 1H), 3.72 (s, 3H), 3.27 - 3.20 (m, 1H), 3.14 - 3.09 (m, 1H), 2.48 - 2.38 (m, 4H), 2.34 (s, 3H), 1.94 - 1.87 (m, 1H), 1.55 - 1.42 (m, 2H), 0.92 (ddd, J=4.1, 6.3, 8.4 Hz, 2H), 0.73 - 0.68 (m, 2H). |
| **8** | 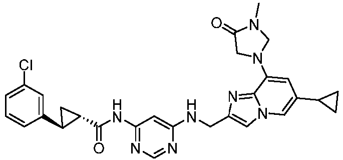 (1*S*,2*S*)-2-(3-chlorophenyl)-*N*-(6-(((6-cyclopropyl-8-(3-methyl-4-oxo imidazolidin-1-yl)imidazo[1,2-*a*]pyridin-2-yl)methyl)amino)pyrimidin-4-yl)cyclo propane-1-carboxamide **(I-9)** | 3-methylimidazolidin-4-one |
| | | ESI-MS (M+H)+: 557.5, δ 10.61 (s, 1H), 8.21 (s, 1H), 7.84 (s, 1H), 7.77 (s, 1H), 7.56 (s, 1H), 7.32 (d, J=7.5 Hz, 2H), 7.27 (d, J=5.4 Hz, 2H), 7.16 (d, J=7.7 Hz, 1H), 5.82 (d, J=1.3 Hz, 1H), 5.28 (s, 2H), 4.56 - 4.56 (m, 2H), 4.10 (s, 2H), 2.91 (s, 3H), 2.45 - 2.34 (m, 2H), 1.88 - 1.80 (m, 1H), 1.51 - 1.39 (m, 2H), 0.90 - 0.85 (m, 2H), 0.71 - 0.66 (m, 2H). |
| **9** | 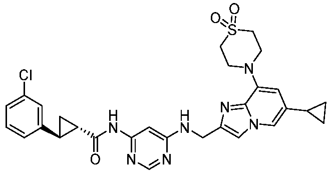 (1*S*,2*S*)-2-(3-chlorophenyl)-*N*-(6-(((6-cyclopropyl-8-(1,1-dioxidothiomorpholino)imidazo[1,2-*a*]pyridin-2-yl)methyl)amino)pyrimidin-4-yl)cyclopropane-1-carboxamide **(I-10)** | thiomorpholine 1,1-dioxide |
| | | ESI-MS (M+H)+: 592.4, ¹H NMR (400 MHz, DMSO) δ 10.1-62 (s, 1H), 8.20 (s, 1H), 7.93 (s, 1H), 7.86 (s, 1H), 7.57 (s, 1H), 7.36 - 7.26 (m, 4H), 7.16 (d, J=7.7 Hz, 1H), 6.31 (s, 1H), 4.56 - 4.55 (m, 2H), 4.14 (d, J=2.1 Hz, 4H), 3.25 (dd, J=4.8, 4.8 Hz, 4H), 2.45 - 2.34 (m, 3H), 1.92 - 1.84 (m, 1H), 1.51 - 1.39 (m, 2H), 0.91 - 0.86 (m, 2H), 0.73 - 0.68 (m, 2H). |
| **10** | 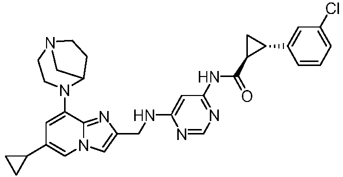 (1*S*,2*S*)-*N*-(6-(((8-(1,4-diazabicyclo[3.2.1] octan*-*4*-*yl)-6-cyclopropylimidazo[1,2-*a*] pyridin-2-yl)methyl) amino)pyrimidin-4-yl)-2-(3-chlorophenyl)cyclopropane-1-carboxamide **(I-11)** | 1,4-diazabicyclo[3.2.1]octane |
| | | ESI-MS (M+H)+: 569.2, ¹H NMR (400 MHz, MeOD) δ 8.17 (s, 1H), 7.76 (s, 1H), 7.55 (s, 1H), 7.34 (d, J = 0.8 Hz, 1H), 7.28-7.24 (m, 1H), 7.20-7.18 (m, 2H), 7.10 (d, J = 7.6 Hz, 1H), 6.32 (s, 1H), 4.64 (s, 2H), 3.42 (d, J = 11.1 Hz, 1H), 3.26-3.20 (m, 4H), 3.08 - 3.01 (m, 2H), 2.89 (d, J = 8.9 Hz, 1H), 2.75 (d, J = 12.9 Hz, 1H), 2.49-2.44 (m, 1H), 2.20-2.18 (m, 1H), 1.90 - 1.81 (m, 3H), 1.62-1.58 (m, 2H), 0.93 - 0.90 (m, 2H), 0.70-0.67 (m, 2H). |
| **11** | 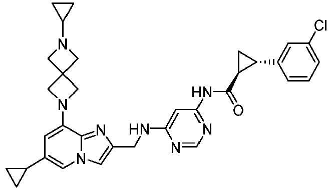 (1S,2S)-2-(3-chlorophenyl)-N-(6-(((6-cyclo propyl-8-(6-cyclopropyl-2,6-diazaspiro [3.3]heptan-2-yl)imidazo[1,2-a]pyridin-2-yl)methyl)amino)pyrimidin-4-yl)cyclo propane-1-carboxamide **(I-12)** | 2-cyclopropyl-2,6-diazaspiro[3.3]heptane |
| | | ESI-MS (M+H)+: 595.2, ¹H NMR (400 MHz, MeOD) δ 8.20 (s, 1H), 7.68 (s, 1H), 7.58 (s, 1H), 7.32 (s, 1H), 7.26 (t, J = 7.7 Hz, 1H), 7.21 - 7.17 (m, 2H), 7.10 (d, J = 7.6 Hz, 1H), 5.98 (s, 1H), 4.64 (s, 2H), 4.40 (s, 4H), 4.33 (s, 4H), 3.04 - 2.97 (m, 1H), 2.50 - 2.45 (m, 1H), 2.21 - 2.17 (m, 1H), 1.90 - 1.84 (m, 1H), 1.61 - 1.58 (m, 1H), 1.42 - 1.38 (m, 1H), 0.95 - 0.90 (m, 4H), 0.86 - 0.83 (m, 2H), 0.69 - 0.65 (m, 2H). |

### Examples 12-15

### (1S,2S)-2-(3-chlorophenyl)-N-(6-(((6-cyclopropyl-8-(3-(dimethylamino)pyrrolidin-1-ylimidazo[1,2-a]pyridin-2-yl)methyl)amino)pyrimidin-4-yl)cyclopropane-1-carboxamide (I-13)

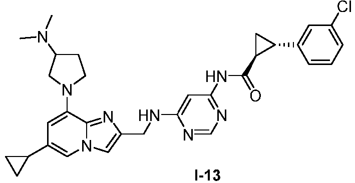

**(I-13)** was prepared in a similar manner to **(I-1)** as a mixture of diastereomers. ESI-MS [M +H] +: 571.2, δ 10.58 (s, 1H), 8.17 (s, 1H), 7.79 (s, 1H), 7.61 (s, 1H), 7.47 (s, 1H), 7.29 - 7.24 (m, 4H), 7.15-7.13 (d, J = 7.6 Hz, 1H), 5.70 (s, 1H), 4.52 (s, 2H), 3.93-3.84 (m, 2H), 3.67 - 3.62 (m, 1H), 3.50 - 3.48 (m, 1H), 2.78-2.72 (m, 1H), 2.42-2.34 (m, 2H), 2.19 (s, 6H), 1.83-1.74 (m, 2H), 1.50-1.38 (m, 3H), 0.85 - 0.81 (m, 2H), 0.64-0.60 (m, 2H).

The mixture was separated using chiral column separation [CHIRALPAK AD-H, 0.46 cm I.D × 5 cm L MEOH=100, 1 mL/min, 35°C) to give two diastereomers: (1S,2S)-2-(3-chlorophenyl)-N-(6-(((6-cyclopropyl-8-((R)-3-(dimethylamino)pyrrolidin-1-yl)imidazo[1,2-a]pyridin-2-yl)methyl)amino)pyrimidin-4-yl)cyclopropane-1-carboxamide and (1S,2S)-2-(3-chlorophenyl)-N-(6-(((6-cyclopropyl-8-((S)-3-(dimethylamino)pyrrolidin-1-yl)imidazo[1,2-a]pyridin-2-yl)methyl)amino)pyrimidin-4-yl)cyclopropane-1-carboxamide.

First eluting isomer **(I-14):** ESI-MS [M +H]+: 571.2. ¹H NMR (400 MHz, MeOD) δ 8.17 (s, 1H), 7.54 (s, 1H), 7.50 (s, 1H), 7.35 (s, 1H), 7.28-7.24 (m, 1H), 7.21-7.18 (m, 2H), 7.11-7.09 (m, 1H), 5.93 (s, 1H), 4.61 (s, 2H), 4.05 - 4.01 (m, 1H), 3.79-3.74 (m, 1H), 3.70-3.63 (m, 1H), 3.53 - 3.48 (m, 1H), 3.01 - 2.94 (m, 1H), 2.50-2.45 (m, 1H), 2.37 (s, 6H), 2.30 - 2.24 (m, 1H), 2.21-2.17 (m, 1H), 1.93 - 1.81 (m, 2H), 1.63-1.59 (m, 1H), 1.41 - 1.37 (m, 1H), 0.91-0.86 (m, 2H), 0.68-0.64 (m, 2H). RT = 6.447 min, 99.82% e.e.

Second eluting isomer **(I-15):** ESI-MS [M +H]+: 571.2. ¹H NMR (400 MHz, MeOD) δ 8.16 (s, 1H), 7.56 (s, 1H), 7.49 (s, 1H), 7.34 (s, 1H), 7.27-7.23 (m, 1H), 7.19-7.17 (m, 2H), 7.10-7.08 (m, 1H), 5.95 (s, 1H), 4.60 (s, 2H), 3.98 - 3.93 (m, 1H), 3.80-3.75 (m, 1H), 3.68 - 3.59 (m, 2H), 3.18 - 3.11 (m, 1H), 2.47 (s, 6H), 2.33-2.26 (m, 1H), 2.20-2.16 (m, 1H), 1.98-1.93 (m, 1H), 1.86-1.79 (m, 1H), 1.62-1.57 (m, 1H), 1.39 - 1.35 (m, 1H), 0.89 - 0.86 (m, 2H), 0.67-0.63 (m, 2H). RT = 8.348 min, 99.53% e.e.

### Example 13: (1S,2S)-2-(3-chlorophenyl)-N-(6-(((6-cyclopropyl-8-(3-(methylamino)pyrrolidin-1-yl)imidazo[1,2-a]pyridin-2-yl)methyl)amino)pyrimidin-4-yl)cyclopropane-1-carboxamide (I-16)

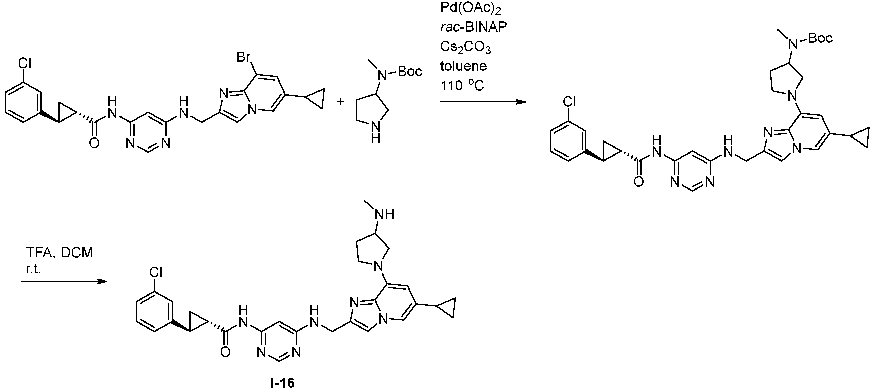

**Intermediate 677** (150 mg, 0.28 mmol), tert-butyl methyl(pyrrolidin-3-yl)carbamate (84 mg, 0.42 mmol), rac-BINAP (35 mg, 0.056 mmol), and Cs₂CO₃ (180 mg, 0.56 mmol) were suspended in toluene (5.0 mL) and degassed with N₂ for 5 min. Pd(OAc)₂ (6.3 mg, 0.028 mmol) was then added and the reaction mixture was heated at 110 °C under a nitrogen atmosphere for 18 h. The reaction mixture was cooled to room temperature and filtered through Celite^{®} and washed with DCM:MeOH (2:1, 10 mL). The reaction mixture was concentrated in vacuo. The residue was purified by column chromatography on silica gel, eluting with a gradient of 0-20 % NH₃ in MeOH in DCM to give **tert-butyl (1-(2-(((6-((1S,2S)-2-(3-chlorophenyl)cyclopropane-1-carboxamido)pyrimidin-4-yl)amino)methyl)-6-cyclopropylimidazo[1,2-a]pyridin-8-yl)pyrrolidin-3-yl)(methyl)carbamate** (120 mg, 66 %). ESI-MS (M+H)+: 657.6, ¹H NMR (400 MHz, CDCl₃) δ 8.28 (s, 1H), 8.07 (s, 1H), 7.34 - 7.31 (m, 3H), 7.25 - 7.18 (m, 3H), 7.09 (s, 1H), 7.03 - 6.99 (m, 1H), 5.79 (s, 1H), 4.60 (s, 2H), 4.08 - 4.07 (m, 1H), 3.80 (dd, J=8.1, 10.6 Hz, 1H), 3.71 - 3.64 (m, 2H), 2.87 (s, 3H), 2.61 - 2.55 (m, 1H), 2.27 - 2.18 (m, 1H), 2.13 - 2.03 (m, 1H), 1.83 - 1.69 (m, 3H), 1.50 - 1.46 (m, 10H), 1.43 - 1.37 (m, 1H), 0.91 - 0.86 (m, 2H), 0.66 - 0.61 (m, 2H).

Trifluoroacetic acid (0.28 mL, 3.7 mmol) was added to a stirred solution of tert-butyl (1-(2-(((6-((1S,2S)-2-(3-chlorophenyl)cyclopropane-1-carboxamido)pyrimidin-4-yl)amino)methyl)-6-cyclopropylimidazo[1,2-a]pyridin-8-yl)pyrrolidin-3-yl)(methyl)carbamate (120 mg, 0.22 mmol) in DCM (5.0 mL), the reaction was then stirred at room temperature for 3 h. The reaction was concentrated in vacuo. The residue was loaded on to an SCX cartridge in MeOH, washed with DCM:MeOH (3:1) and eluted with DCM:7N NH₃ in MeOH (3: 1). The product was concentrated in vacuo and purified by reverse phase preparative HPLC to give **(I-16)** (20.3 mg, 20 %) as a formic acid salt.
ESI-MS (M+H)+: 557.4, δ 10.60 (s, 1H), 8.20 (s, 1H), 7.81 (s, 1H), 7.63 (s, 1H), 7.49 (s, 1H), 7.36 - 7.26 (m, 4H), 7.16 (d, J=7.7 Hz, 1H), 5.70 (s, 1H), 4.54 (s, 2H), 3.91 (dd, J=6.3, 10.8 Hz, 1H), 3.78 - 3.71 (m, 1H), 3.68 - 3.62 (m, 1H), 3.59 - 3.53 (m, 1H), 2.44 - 2.34 (m, 6H), 2.13 - 2.05 (m, 1H), 1.85 - 1.77 (m, 2H), 1.51 - 1.39 (m, 2H), 0.88 - 0.82 (m, 2H), 0.66 - 0.61 (m, 2H).

### Example 14: (1S,2S)-2-(3-chlorophenyl)-N-(6-(((6-cyclopropyl-8-(3-(dimethylamino)-2-oxopyrrolidin-1-yl)imidazo[1,2-a]pyridin-2-yl)methyl)amino)pyrimidin-4-yl)cyclopropane-1-carboxamide (I-17)

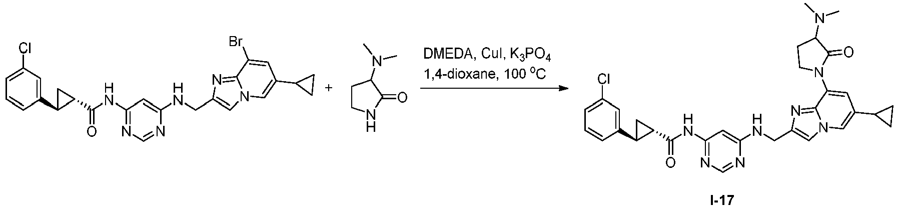

**Intermediate 677** (100 mg, 0.19 mmol), 3-(dimethylamino)pyrrolidin-2-one (36 mg, 0.28 mmol), *N*,*N*-dimethylethylenediamine (12 µL, 0.11 mmol), and K₃PO₄ (79 mg, 0.37 mmol) were suspended in 1,4-dioxane (4.0 mL) and degassed with nitrogen for 5 min. CuI (11 mg, 0.059 mmol) was then added and the reaction was heated at 100 °C under a nitrogen atmosphere for 18 h. The reaction was then cooled to room temperature and filtered through Celite^{®} and washed with DCM:MeOH (2:1, 10 mL). The reaction was concentrated *in vacuo* and the residue was purified by reverse phase preparative HPLC to give (I-17) (23 mg, 21 %).ESI-MS (M+H)+: 585.3, δ 10.64 (s, 1H), 8.28 (d, J=1.1 Hz, 1H), 8.23 (s, 1H), 7.91 (s, 1H), 7.68 (s, 1H), 7.38 - 7.32 (m, 2H), 7.30 - 7.28 (m, 2H), 7.19 (d, J=6.9 Hz, 2H), 4.60 (s, 2H), 4.16 (dd, J=2.7, 9.2 Hz, 1H), 4.07 - 4.00 (m, 1H), 3.61 (dd, J=8.8, 8.8 Hz, 1H), 2.47 - 2.38 (m, 8H), 2.29 - 2.21 (m, 1H), 2.16 - 2.08 (m, 1H), 2.00 - 1.93 (m, 1H), 1.53 - 1.42 (m, 2H), 0.99 - 0.93 (m, 2H), 0.70 - 0.66 (m, 2H).

Using a similar procedure to that used for Example 17, Example 18 was prepared from **intermeidate 677** and 4-(dimethylamino)pyrrolidin-2-one.

**Table 2**

| **EG** | **Compound** | **Coupling Partner/Analvtical Data** |
|---|---|---|
| **18** | 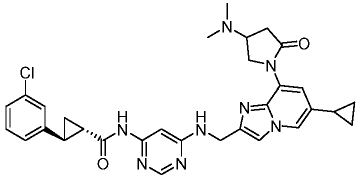 (1*S*,2*S*)-2-(3-chlorophenyl)-*N*-(6-(((6-cyclopropyl-8-(4-(dimethylamino)-2-oxo pyrrolidin-1-yl)imidazo[1,2-*a*] pyridin-2-yl)methyl)amino)pyrimidin-4-yl)cyclopropane-1-carboxamide **(I-18)** | 4-(dimethylamino)pyrrolidin-2-one |
| | | ESI-MS (M+H)+: 585.3, δ 10.63 (s, 1H), 8.28 (d, J=1.3 Hz, 1H), 8.23 (s, 1H), 7.90 - 7.89 (m, 1H), 7.69 (s, 1H), 7.38 - 7.28 (m, 4H), 7.18 (d, J=7.6 Hz, 1H), 7.15 (d, J=1.5 Hz, 1H), 4.61 (s, 2H), 4.24 (dd, J=7.5, 9.7 Hz, 1H), 4.04 (dd, J=6.1, 9.9 Hz, 1H), 3.23 - 3.14 (m, 1H), 2.66 (dd, J=8.5, 16.6 Hz, 1H), 2.51 - 2.39 (m, 3H), 2.22 (s, 6H), 1.99 - 1.91 (m, 1H), 1.54 - 1.41 (m, 2H), 0.95 (ddd, J=4.4, 6.3, 8.4 Hz, 2H), 0.70 - 0.65 (m, 2H). |

### Example 19: (1S,2S)-2-(3-chlorophenyl)-N-(6-(((6-cyclopropyl-8-(5-oxoimidazolidin-1-ylimidazo[1,2-alpyridin-2-yl)methyl)amino)pyrimidin-4-yl)cyclopropane-1-carboxamide (I-19)

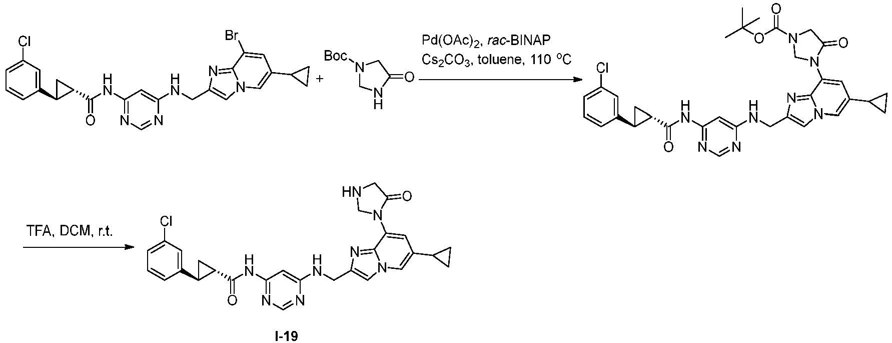

**Intermediate 677** (80 mg, 0.15 mmol), tert-butyl 4-oxoimidazolidine-1-carboxylate (42 mg, 0.22 mmol), N,N-dimethylethylenediamine (3.2 µL, 0.030 mmol), and K₃PO₄ (63 mg, 0.30 mmol) were suspended in 1,4-dioxane (4.0 mL) and degassed with nitrogen for 5 min. CuI (2.8 mg, 0.015 mmol) was then added and the reaction was heated at 100 °C under a nitrogen atmosphere for 18 h. The reaction was cooled to room temperature and filtered through Celite^{®} and washed with DCM:MeOH (2:1, 10 mL). The reaction was concentrated in vacuo. The residue was purified by column chromatography on silica gel, eluting with a gradient of 0-20 % NH₃ in MeOH in DCM to give **tert-butyl 3-(2-(((6-((1S,2S)-2-(3-chlorophenyl)cyclopropane-1-carboxamido)pyrimidin-4-yl)amino)methyl)-6-cyclopropylimidazo[1,2-a]pyridin-8-yl)-4-oxoimidazolidine-1-carboxylate intermediate 678** (50 mg, 52 %).ESI-MS (M+H)+: 643.4.
Trifluoroacetic acid (0.1 mL, 1.2 mmol) was added to a stirred solution of **intermeidate 678** (40 mg, 0.062 mmol) in DCM (2.0 mL). The reaction was stirred at room temperature for 3 h then concentrated in vacuo. The residue was loaded on to an SCX cartridge in MeOH, washed with DCM:MeOH (3:1), and eluted with DCM:NH₃ in MeOH (3:1). The product was concentrated in vacuo and purified by reverse phase preparative HPLC to give **(I-19)** (8.1 mg, 24 %).ESI-MS (M+H)+: 543.3, δ 10.62 (s, 1H), 8.23 - 8.20 (m, 2H), 7.92 - 7.85 (m, 1H), 7.67 (s, 1H), 7.36 - 7.30 (m, 3H), 7.28 - 7.26 (m, 2H), 7.16 (d, J=7.7 Hz, 1H), 5.16 (s, 2H), 4.60 - 4.60 (m, 2H), 3.64 (s, 1H), 3.44 (s, 2H), 2.45 - 2.36 (m, 2H), 1.98 - 1.90 (m, 1H), 1.51 - 1.39 (m, 2H), 0.97 - 0.91 (m, 2H), 0.67 - 0.62 (m, 2H).

### Example 20: (1S,2S)-2-(3-chlorophenyl)-N-(4-((6-cyclopropyl-8-(4-(2,2,2-trifluoroethyl) piperazin-1-yl)imidazo[1,2-a]pyridin-2-yl)methylamino)pyridin-2-yl)cyclopropane carboxamide (I-20)

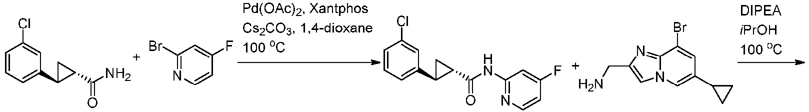

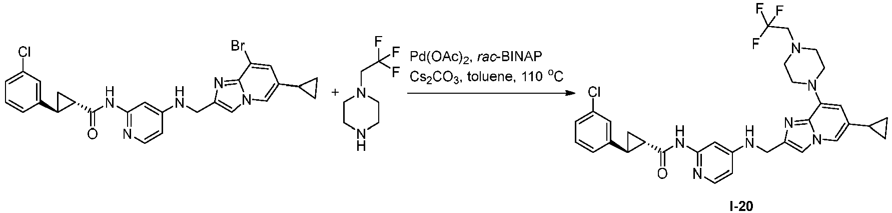

2-Bromo-4-fluoropyridine (500 mg, 2.8 mmol), **intermediate 119** (611 mg, 3.13 mmol), Xantphos (330 mg, 0.57 mmol), and Cs₂CO₃ (1.9 g, 5.7 mmol) were suspended in 1,4-dioxane (20 mL) and degassed with nitrogen for 5 min. Pd(OAc)₂ (64 mg, 0.28 mmol) was then added and the reaction was heated at 110 °C under a nitrogen atmosphere for 4 h. The reaction was cooled to room temperature and filtered through Celite^{®} and washed with DCM:MeOH (2:1, 30 mL). The reaction was concentrated *in vacuo* and the residue was purified by column chromatography on silica gel, eluting with a gradient of 0-60 % EtOAc in cyclohexane to give ***(1S,2S)-2-(3-chlorophenyl)-N-(4-fluoropyridin-2-yl)cyclopropane-1-carboxamide* intermediate 873** (630 mg, 77 %).¹H NMR (400 MHz, CDCl₃) δ , 8.20 - 8.11 (m, 1H), 8.01 (dd, J=2.0, 11.1 Hz, 1H), 7.23 - 7.18 (m, 2H), 7.08 (dd, J=2.7, 2.7 Hz, 1H), 7.07 - 7.01 (m, 2H), 6.77 - 6.72 (m, 1H), 2.63 - 2.56 (m, 1H), 1.80 - 1.73 (m, 2H), 1.42 - 1.37 (m, 1H).

**Intermediate 873** (400 mg, 1.4 mmol), **intermediate 359** (370 mg, 1.4 mmol), and DIPEA (0.72 mL, 4.1 mmol) were suspended in iPrOH (20 mL). The reaction mixture was then heated at 100 °C in a sealed tube for 18 h. The reaction was concentrated in vacuo. The residue was purified by column chromatography on silica gel, eluting with a gradient of 0-20 % NH₃ in MeOH in DCM to give **(1S,2S)-N-(4-(((8-bromo-6-cyclopropylimidazo[1,2-a]pyridin-2-yl)methyl)amino)pyridin-2-yl)-2-(3-chlorophenyl)cyclopropane-1-carboxamide intermediate** 679 (91 mg, 12 %).¹H NMR (400 MHz, CDCl₃) δ 9.68 - 9.68 (m, 1H), 7.82 (s, 1H), 7.67 (d, J=5.8 Hz, 1H), 7.62 (s, 1H), 7.55 - 7.52 (m, 1H), 7.21 - 7.15 (m, 3H), 7.02 (s, 1H), 7.01 - 6.95 (m, 1H), 6.18 (dd, J=2.3, 5.8 Hz, 1H), 5.20 (dd, J=5.7, 5.7 Hz, 1H), 4.54 (d, J=5.6 Hz, 2H), 2.56 - 2.50 (m, 1H), 2.28 - 2.09 (m, 2H), 2.05 - 1.98 (m, 1H), 1.90 - 1.83 (m, 2H), 1.75 - 1.66 (m, 1H), 1.32 - 1.26 (m, 1H), 1.00 - 0.94 (m, 2H), 0.69 - 0.64 (m, 2H).

**Intermediate 679** (100 mg, 0.15 mmol), 1-(2,2,2-trifluoroethyl)piperazine (100 mg, 0.60 mmol), rac-BINAP (34 mg, 0.060 mmol), and Cs₂CO₃ (190 mg, 0.60 mmol) were suspended in toluene (11 mL) and degassed with nitrogen for 5 min. Pd(OAc)₂ (6.7 mg, 0.030 mmol) was then added and the reaction was heated at 100 °C under a nitrogen atmosphere for 76 h. The reaction was cooled to room temperature and filtered through Celite^{®} and washed with DCM:MeOH (2:1, 10 mL). The reaction was concentrated in vacuo and the residue was purified by reverse phase preparative HPLC to give **(I-20)** (12 mg, 13 %) as a formic acid salt. ESI-MS (M+H)+: 624.5, δ 10.35 (s, 1H), 7.93 (s, 1H), 7.82 - 7.79 (m, 1H), 7.59 (s, 1H), 7.50 (s, 1H), 7.36 - 7.28 (m, 3H), 7.21 - 7.11 (m, 2H), 6.38 (dd, J=2.0, 5.8 Hz, 1H), 6.22 (d, J=1.3 Hz, 1H), 4.39 (d, J=5.8 Hz, 2H), 3.57 - 3.51 (m, 4H), 3.30 (q, J=10.2 Hz, 2H), 2.90 - 2.84 (m, 4H), 2.45 - 2.38 (m, 2H), 1.96 - 1.86 (m, 1H), 1.53 - 1.47 (m, 1H), 1.42 - 1.36 (m, 1H), 0.95 - 0.89 (m, 2H), 0.74 - 0.69 (m, 2H).

Using a similar procedure to that used for **(I-1),** the compounds in Table 3 were prepared from **Intermediate 679** and an appropriate coupling partner.

**Table 3**

| **EG** | **Compound** | **Coupling Partner / Analvtical Data** |
|---|---|---|
| **21** | 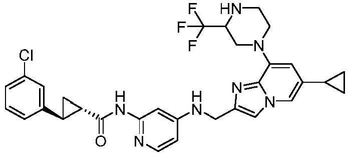 (1*S*,2*S*)-2-(3-chlorophenyl)-*N*-(4-(((6-cyclopropyl-8-(3-(trifluoro methyl)piperazin-1-yl)imidazo[1,2-*a*]pyridin-2-yl)methyl)amino) pyridin-2-yl)cyclopropane-1-carboxamide **(I-21)** | 2-(trifluoromethyl)piperazine |
| | | ESI-MS (M+H)+: 610.5, δ 10.35 (s, 1H), 8.57 (s, 1H), 7.95 (s, 1H), 7.82 - 7.79 (m, 1H), 7.60 (s, 1H), 7.49 (s, 1H), 7.39 - 7.28 (m, 3H), 7.21 - 7.11 (m, 2H), 6.39 (dd, J=2.3, 5.8 Hz, 1H), 6.30 (d, J=1.3 Hz, 1H), 4.45 - 4.37 (m, 3H), 4.08 (d, J=10.6 Hz, 1H), 3.62 (t, J=9.5 Hz, 1H), 3.12 - 3.07 (m, 1H), 3.00 - 2.96 (m, 1H), 2.87 - 2.71 (m, 2H), 2.44 - 2.38 (m, 2H), 1.96 - 1.88 (m, 1H), 1.54 - 1.36 (m, 2H), 0.95 - 0.89 (m, 2H), 0.75 - 0.69 (m, 2H). |
| **22** | 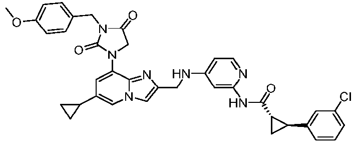 (1S,2S)-2-(3-chlorophenyl)-N-(4-(((6-cyclopropyl-8-(3-(4-methoxy benzyl)-2,4-dioxoimidazolidin-1-yl)imidazo[1,2-a]pyridin-2-yl) methyl)amino)pyridin-2-yl) cyclopropane-1-carboxamide **(I-22)** | 3-(4-methoxybenzyl)imidazolidine-2,4-dione |
| | | ESI-MS [M + H]+: 676.2; δ 10.34 (s, 1H), 8.26 (s, 1H), 7.76 - 7.72 (m, 2H), 7.35 - 7.24 (m, 7H), 7.14 (d, J = 7.3 Hz, 1H), 6.91 (d, J = 8.5 Hz, 2H), 6.35 (s, 1H), 4.99 (s, 2H), 4.61 (s, 2H), 4.37 (s, 2H), 3.73 (s, 3H), 2.40 - 2.31 (m, 2H), 1.96 - 1.92 (m, 1H), 1.47 - 1.43 (m, 1H), 1.38 - 1.34 (m, 1H), 0.95 - 0.92 (m, 2H), 0.66 - 0.62 (m, 2H). |
| **23** | 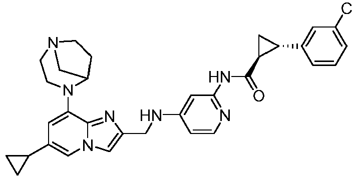 (1S,2S)-N-(4-(((8-(1,4-diazabicyclo [3.2.1]octan-4-yl)-6-cyclopropyl imidazo[1,2-a]pyridin-2-yl)methyl) amino)pyridin-2-yl)-2-(3-chloro phenyl)cyclopropane-1-carboxamide **(I-23)** | 1,4-diazabicyclo[3.2.1]octane |
| | | ESI-MS [M +H]+: 568.2.. ¹H NMR (400 MHz, MeOD) δ 7.76 (d, J = 3.4 Hz, 2H), 7.56 (s, 1H), 7.37 (s, 1H), 7.27-7.24 (m, 1H), 7.19-7.17 (m, 2H), 7.09 (d, J = 7.6 Hz, 1H), 6.41-6.39 (m, 1H), 6.33 (s, 1H), 4.48 (s, 2H), 3.44 (d, J = 11.2 Hz, 1H), 3.25-3.22 (m, 4H), 3.13-3.02 (m, 2H), 2.92 (d, J = 9.0 Hz, 1H), 2.79 (d, J = 10.5 Hz, 1H), 2.48-2.43 (m, 1H), 2.19-2.15 (m, 1H), 1.91 - 1.85 (m, 3H), 1.61-1.57 (m, 2H), 0.93 - 0.91 (m, 2H), 0.70-0.67 m, 2H). |
| **24** | 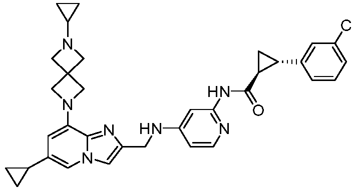 (1S,2S)-2-(3-chlorophenyl)-N-(4-(((6-cyclopropyl-8-(6-cyclopropyl-2,6-diazaspiro[3.3]heptan-2-yl) imidazo[1,2-a]pyridin-2-yl)methyl) amino)pyridin-2-yl)cyclopropane-1-carboxamide **(I-24)** | 2-cyclopropyl-2,6-diazaspiro[3.3]heptane |
| | | ESI-MS [M +H] +: 594.2. δ 10.40 (s, 1H), 7.73 (d, J = 5.9 Hz, 1H), 7.65 (s, 1H), 7.48 (s, 1H), 7.32 - 7.21 (m 2H), 7.18 - 7.10 (m, 2H), 7.18 - 7.10 (m, 2H), 6.32 (d, J = 4.8 Hz, 1H), 5.63 (s, 1H), 4.28 (d, J = 5.6 Hz, 2H), 4.17 (s, 4H), 3.68 (s, 4H), 2.38 - 2.30 (m, 3H), 1.81 - 1.74 (m, 1H), 1.45 - 1.40 (m, 1H), 1.36 - 1.31 (m, 1H), 0.83 - 0.79 (m, 2H), 0.65 - 0.56 (m, 2H), 0.52 - 0.26 (m, 4H). |
| **25** | 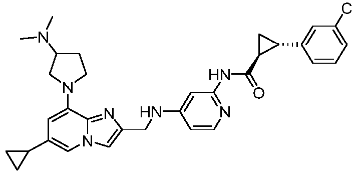 (1S,2S)-2-(3-chlorophenyl)-N-(4-(((6-cyclopropyl-8-(3-(dimethyl amino)pyrrolidin-1-yl)imidazo[1,2-a]pyridin-2-yl)methyl)amino) pyridin-2-yl)cyclopropane-1-carboxamide **(I-25)** | 3-(dimethylamino)pyrrolidine |
| | | ESI-MS [M +H] +: 570.2. δ 10.30 (s, 1H), 7.74 (d, J = 5.8 Hz, 1H), 7.62 (s, 1H), 7.50 (s, 1H), 7.44 (s, 1H), 7.33 - 7.23 (m, 3H), 7.14 (d, J = 7.6 Hz, 1H), 7.05 (t, J = 5.5 Hz, 1H), 6.33 (dd, J = 5.8, 2.1 Hz, 1H), 5.71 (s, 1H), 4.31 (d, J = 5.3 Hz, 2H), 3.94 - 3.86 (m, 2H), 3.69 - 3.62 (m, 1H), 3.40 - 3.35 (m, 1H), 2.79 - 2.71 (m, 1H), 2.39 - 2.32 (m, 2H), 2.20 (s, 6H), 2.14 - 2.08 (m, 1H), 1.83 - 1.73 (m, 2H), 1.46 - 1.42 (m, 1H), 1.36 - 1.31 (m, 1H), 0.87 - 0.81 (m, 2H), 0.65 - 0.61 (m, 2H). |
| **26** | 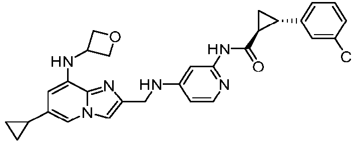 (1S,2S)-2-(3-chlorophenyl)-N-(4-(((6-cyclopropyl-8-(oxetan-3-ylamino)imidazo[1,2-a]pyridin-2-yl)methyl)amino)pyridin-2-yl) cyclopropane-1-carboxamide **(I-26)** | 3-amino-oxetane ESI-MS [M +H]+: 529.2, |
| | | δ 10.35 (s, 1H), 7.76 (d, J = 5.8 Hz, 1H), 7.62 (s, 1H), 7.52 (s, 1H), 7.42 (s, 1H), 7.33 - 7.29 (m, 1H), 7.25 (d, J = 6.7 Hz, 2H), 7.14 (d, J = 7.6 Hz, 1H), 7.09 (s, 1H), 6.57 (d, J = 6.2 Hz, 1H), 6.34 - 6.29 (m, 1H), 5.63 (s, 1H), 4.84 (t, J = 6.4 Hz, 2H), 4.75 - 4.68 (m, 1H), 4.59 (t, J = 5.9 Hz, 2H), 4.35 (d, J = 4.8 Hz, 2H), 2.40 - 2.33 (m, 2H), 1.82 - 1.76 (m, 1H), 1.47 - 1.42 (m, 1H), 1.37 - 1.33 (m, 1H), 0.86 - 0.81 (m, 2H), 0.63 - 0.59 (m, 2H). |

### Example 15: (1S,2S)-N-(6-(((R)-1-(6-cyclopropyl-8-(7-oxo-2-oxa-6-azaspiro[3.4joctan-6-yl)imidazo[1,2-alpyridin-2-yl)ethyl)amino)-2-methylpyrimidin-4-yl)-2-(4-methylpyrimidin-2-yl)cyclopropane-1-carboxamide (I-27)

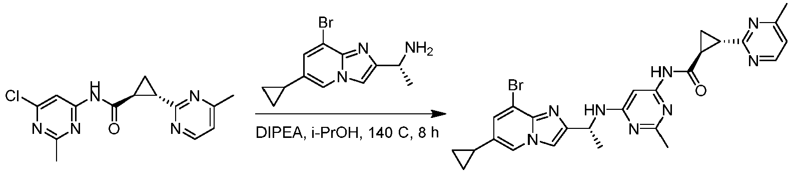

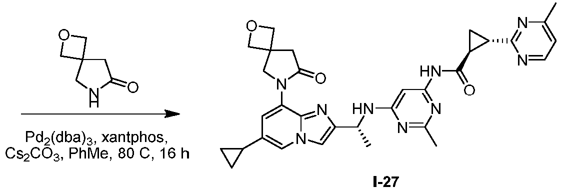

A mixture of **Intermediate 832** (200 mg, 0.66 mmol), (R)-1-(8-bromo-6-cyclopropylimidazo[1,2-a]pyridin-2-yl)ethan-1-amine (276 mg, 0.99 mmol) and DIPEA (255 mg, 1.97 mmol) in i-PrOH (2 mL) was stirred in a sealed tube. After degassing with N₂ for 1 min, the reaction mixture was irradiated in microwave at 140 °C for 8 h. The reaction was cooled to room temperature and concentrated in vacuo to give the crude, which was purified by preparative TLC (eluent: DCM/MeOH = 10/1) to give **(1S,2S)-N-(6-(((R)-1-(8-bromo-6-cyclopropylimidazo[1,2-a]pyridin-2-yl)ethyl)amino)-2-methylpyrimidin-4-yl)-2-(4-methylpyrimidin-2-yl)cyclopropane-1-carboxamide** (200 mg, 55.4%) as a yellow solid. ESI-MS [M +H]+: 547.2.

The latter intermediate (100 mg, 0.18 mmol), was used in a mixture with 2-oxa-6-azaspiro[3.4]octan-7-one (46 mg, 0.36 mmol), and Cs₂CO₃ (117 mg, 0.36 mmol) in toluene (5 mL) with added Pd₂(dba)₃ (33 mg, 0.036 mmol) and Xantphos (21 mg, 0.036 mmol). The reaction mixture was stirred at 80 °C for 16 h under N₂. After cooling to room temperature, water (20 mL) was added and the mixture was extracted with EtOAc (3 x 20 mL). The combined organic layers were washed with brine (30 mL), dried over anhydrous Na₂SO₄, and concentrated in vacuo to give the crude, which was purified by preparative TLC (eluent: DCM/MeOH = 10/1) to give **(I-27)** (33 mg, 30.9%) as a white solid. ESI-MS [M +H]⁺: 594.3. δ 10.63 (s, 1H), 8.49 (d, J = 5.1 Hz, 1H), 8.21 (s, 1H), 7.60 (s, 2H), 7.18 (d, J = 5.1 Hz, 1H), 7.10 (s, 2H), 5.40 (s, 1H), 4.63-4.62 (m, 4H), 4.44-4.43 (m, 2H), 2.87 (s, 2H), 2.59 - 2.51 (m, 2H), 2.39 (s, 3H), 2.22 (s, 3H), 1.93 - 1.83 (m, 1H), 1.47-1.45 (m, 5H), 0.89-0.87 (m, 2H), 0.60-0.59 (m, 2H).

### Intermediate 680: 3-tritylimidazolidine-2,4-dione

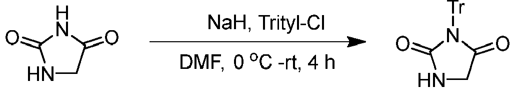

To a mixture of imidazolidine-2,4-dione (100 mg, 1.0 mmol) in DMF (4 mL) was added NaH (60 mg, 60% dispersion in mineral oil, 1.50 mmol) under N₂. The resulting reaction was stirred at rt for 0.5 h, then trityl chloride (418 mg, 1.50 mmol) in DMF (1 mL) was added. The reaction mixture was stirred at rt for 4 h. The reaction was quenched with water (20 mL) and extracted with EtOAc (30 mL x 3). The combined organic layers were washed with brine (10 mL), dried over anhydrous Na₂SO₄, and concentrated *in vacuo* to give the crude product, which was purified by column chromatography (eluent: EtOAc/PE=1/100 ~100/1) to give **intermediate 680** (150 mg, 44%) as a pale solid. ESI-MS [M +H]+: 343.2.

### Intermediate 681: 8-bromo-2-(((6-chloropyrimidin-4-yl)oxy)methyl)-6-cyclopropylimidazo[1,2-a]pyridine

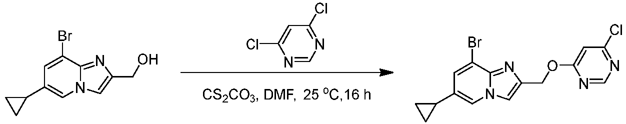

A mixture of **intermediate 453** (390 mg, 1.46 mmol), 4,6-dichloropyrimidine (434 mg, 2.91 mmol), and Cs₂CO₃ (1.4 g ,4.30 mmol) in DMF (6 ml) was stirred at 25 °C for 16 h. Water (30 mL) was added and the reaction mixture was extracted with EtOAc (3 x 50 mL). The combined organic layers were washed with brine (30 mL), dried over anhydrous Na₂SO₄, and concentrated *in vacuo* to give the crude, which was purified by column chromatography (eluent: PE/EtOAc = 2/1) to give **intermediate 681** (420 mg, 75.8%) as a yellow solid. ESI-MS [M +H]⁺: 378.9

### Intermediate 682: 1-(2-(((6-chloropyrimidin-4-yl)oxy)methyl)-6-cyclopropylimidazo[1,2-a]pyridin-8-yl)-3-methylimidazolidine-2,4-dione

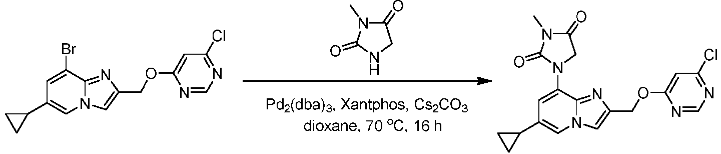

To a mixture of **intermediate 681** (200 mg, 0.53 mmol), 3-methylimidazolidine-2,4-dione (48.2 mg, 0.42 mmol), and Cs₂CO₃ (431 mg ,1.32 mmol) in dioxane (6 mL) was added Pd₂(dba)₃ (73 mg, 0.080 mmol) and XantPhos (92 mg, 0.159 mmol). The reaction mixture was stirred at 70 °C for 16 h under N₂. The reaction mixture was cooled to room temperature, filtered through Celite^{®}, and the filter cake was washed with DCM/MeOH (10/1, 50 mL). The filtrate was concentrated *in vacuo* to give the crude, which was purified by column chromatography (eluent: DCM/MeOH=50/1 ~ 20/1) to give **intermediate 682** (60 mg, 34.6%) as a yellow solid. ESI-MS [M +H]+: 413.1. The compounds in Table 4 were prepared in a similar manner to **intermediate 682** from **intermediate 681** and an appropriate coupling partner. **Table 4**

| **Compound** | **Name / Coupling partner / Analytical Data** |
|---|---|
| 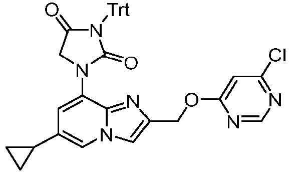 | **Intermediate 683: 1-(2-(((6-chloropyrimidin-4-yl)oxy)methyl)-6-cyclopropylimidazo [1,2-a]pyridin-8-yl)-3-tritylimidazolidine-2,4-dione** from intermediate 680 |
| | ESI-MS [M +H]⁺:641.2 |
| 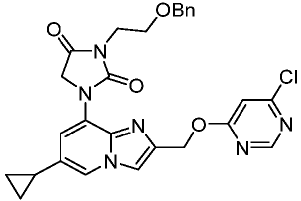 | **3-(2-(benzyloxy)ethyl)-1-(2-(((6-chloropyrimidin-4-yl)oxy)methyl)-6-cyclopropylimidazo[1,2-a]pyridin-8-yl) imidazolidine-2,4-dione** from 3-(2-(benzyloxy)ethyl) imidazolidine-2,4-dione |
| | ESI-MS [M +H]⁺: 533.2 |

### Examples 28-30

### rac-(1S*,2S*)-N-(6-((6-cyclopropyl-8-(3-methyl-2,4-dioxoimidazolidin-1-yl)imidazo[1,2-a]pyridin-2-yl)methoxy)pyrimidin-4-yl)-2-(4-methylpyrimidin-2-yl)cyclopropane-1-carboxamide (I-28)

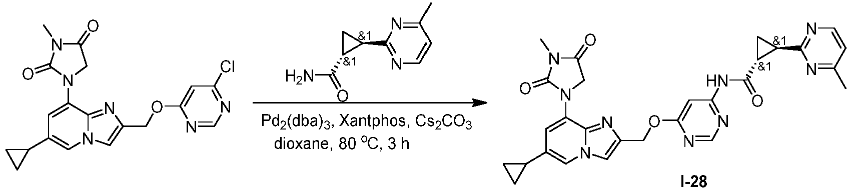

To a mixture of **intermediate 682** (50 mg, 0.12 mmol), *rac-(1S*,2S*)-2-(4-*methylpyrimidin-2-yl)cyclopropane-1-carboxamide (21 mg, 0.12 mmol), and Cs₂CO₃(79 mg ,0.24 mmol) in dioxane (4 mL) was added Pd₂(dba)₃ (22 mg, 0.024 mmol) and XantPhos (28 mg, 0.048 mmol). The reaction mixture was stirred at 80 °C for 3 h under N₂. The reaction mixture was cooled to room temperature, filtered through Celite^{®}, and the filter cake was washed with DCM/MeOH (10/1, 80 mL). The filtrate was concentrated to give the crude, which was purified by preparative TLC (eluent: DCM/MeOH = 10/1) to give **(I-28)** as a mixture of enantiomers (7 mg, 10.5%) as a white solid.

The mixture was separated using SFC (Daicel CHIRALPAK OD-H 250mm X 20 mm I.D. CO2/MeOH (0.2% NH4 ▪ OH ) = 60/40, 50 g/min, 35°C) to give two enantiomers: (1S,2S)-N-(6-((6-cyclopropyl-8-(3-methyl-2,4-dioxoimidazolidin-1-yl)imidazo[1,2-a]pyridin-2-yl)methoxy)pyrimidin-4-yl)-2-(4-methylpyrimidin-2-yl)cyclopropane-1-carboxamide and (1R,2R)-N-(6-((6-cyclopropyl-8-(3-methyl-2,4-dioxoimidazolidin-1-yl)imidazo[1,2-a]pyridin-2-yl)methoxy)pyrimidin-4-yl)-2-(4-methylpyrimidin-2-yl)cyclopropane-1-carboxamide.

First eluting isomer, **(1R,2R)-N-(6-((6-cyclopropyl-8-(3-methyl-2,4-dioxoimidazolidin-1-yl)imidazo[1,2-a]pyridin-2-yl)methoxy)pyrimidin-4-yl)-2-(4-methylpyrimidin-2-yl)cyclopropane-1-carboxamide (I-29):** δ = 11.20 (s, 1H), 8.60 - 8.46 (m, 2H), 8.28 (s, 1H), 7.95 (s, 1H), 7.49 (s, 1H), 7.38 (s, 1H), 7.22 (d, J=5.1, 1H), 5.49 (s, 2H), 4.89 (s, 2H), 2.97 (s, 3H), 2.69-2.65 (m, 1H), 2.58 - 2.55 (m, 1H), 2.42 (s, 3H), 1.96- 1.92 (m, 1H), 1.60 - 1.52 (m, 2H), 1.00 - 0.92 (m, 2H), 0.71 - 0.62 (m, 2H). RT = 1.76 min, 100.0 % e.e.

Second eluting isomer, **(1S,2S)-N-(6-((6-cyclopropyl-8-(3-methyl-2,4-dioxoimidazolidin-1-yl)imidazo[1,2-a]pyridin-2-yl)methoxy)pyrimidin-4-yl)-2-(4-methylpyrimidin-2-yl)cyclopropane-1-carboxamide (I-30):** ESI-MS (M+H)+: 554.2, ¹H NMR (400 MHz, DMSO) substantially similar to (I-29). RT = 2.33 min, 100.0 % e.e. (I-28), (I-29) & **(I-30)** data: ESI-MS [M +H]+: 554.2

### Example 31: rac-(1S*,2S*)-N-(6-((8-(3-(2-(benzyloxy)ethyl)-2,4-dioxoimidazolidin-1-yl)-6-cyclopropylimidazo[1,2-a]pyridin-2-yl)methoxy)pyrimidin-4-yl)-2-(4-methylpyrimidin-2-yl)cyclopropane-1-carboxamide (I-31)

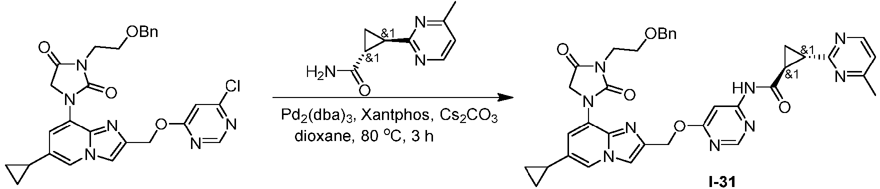

(I-31) was prepared in a similar manner to **(I-28)** from 3-(2-(benzyl oxy)ethyl)-1-(2-(((6-chloropyrimidin-4-yl)oxy)methyl)-6-cyclopropylimidazo[1,2-a]pyridin-8-yl)imidazolidine-2,4-dione and *rac*-(1S*,2S*)-2-(4-methylpyrimidin-2-yl)cyclopropane-1-carboxamide. ESI-MS (M+H)+: 674.3, δ 11.22 (s, 1H), 8.57 (d, J = 0.8 Hz, 1H), 8.53 (d, J = 5.1 Hz, 1H), 8.40 (s, 1H), 8.06 (s, 1H), 7.50 (d, J = 0.8 Hz, 1H), 7.45 (s, 1H), 7.38 - 7.29 (m, 4H), 7.29 - 7.24 (m, 1H), 7.22 (d, J = 5.1 Hz, 1H), 5.53 (s, 2H), 4.86 (s, 2H), 4.51 (s, 2H), 3.74-3.71 (m, 2H), 3.66-3.63 (2H), 2.69-2.65 (m, 1H), 2.61 - 2.53 (m, 1H), 2.42 (s, 3H), 2.01-1.98 (m, 1H), 1.60-1.52 (m, 2H), 1.09 - 0.85 (m, 2H), 0.71-0.69 (m, 2H).

### Example 32: (1S,2S)-N-(6-((6-cyclopropyl-8-(2,4-dioxoimidazolidin-1-yl)imidazo[1,2-a]pyridin-2-yl)methoxy)pyrimidin-4-yl)-2-(4-methylpyrimidin-2-yl)cyclopropane-1-carboxamide (I-32)

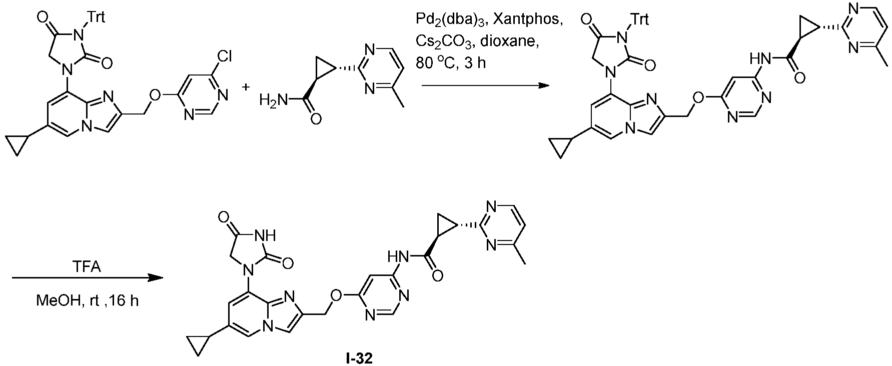

**(1S,2S)-N-(6-((6-cyclopropyl-8-(2,4-dioxo-3-tritylimidazolidin-1-yl)imidazo[1,2-a]pyridin-2-yl)methoxy)pyrimidin-4-yl)-2-(4-methylpyrimidin-2-yl)cyclopropane-1-carboxamide intermediate 684** was prepared in a similar manner to **(I-28)** from **intermediate 683** and **intermediate 55.** ESI-MS (M+H)+: 782.2

To a solution of **intermediate 684** (30 mg, 0.038 mmol) in MeOH (3 mL) was added TFA (3 mL). The resulting mixture was stirred at room temperature for 1 h. The reaction mixture was concentrated in vacuo and the residue was neutralized with NH₃ in MeOH (7M, 1 mL). The resulting solution was concentrated in vacuo and purifed by preparative TLC (eluent: MeOH/DCM=1/10) to afford **(I-32)** (10 mg, 48%) as a pale solid. ESI-MS (M+H)+: 540.2, δ 11.35 (s, 1H), 11.20 (s, 1H), 8.56 - 8.52 (m, 2H), 8.27 (s, 1H), 7.95 (s, 1H), 7.49 (d, J = 0.9 Hz, 1H), 7.39 (s, 1H), 7.22 - 7.21 (m, 1H), 5.49 (s, 2H), 4.85 (s, 2H), 2.67 - 2.65 (m, 1H), 2.58 - 2.55 (m, 1H), 2.42 (s, 3H), 1.98 - 1.94 (m, 1H), 1.59 - 1.52 (m, 2H), 0.98 - 0.93 (m, 2H), 0.69 - 0.65 (m, 2H).

### Example 33: rac-(1S*2S*)-N-(6-((6-cyclopropyl-8-(3-(2-hydroxyethyl)-2,4-dioxoimidazolidin-1-yl)imidazo[1,2-a]pyridin-2-yl)methoxy)pyrimidin-4-yl)-2-(4-methylpyrimidin-2-yl)cyclopropane-1-carboxamide (I-33)

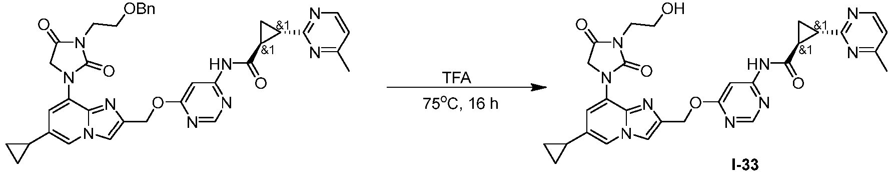

A solution of **(I-31)** (25 mg, 0.037 mmol) in TFA (3 mL) was stirred at 70 °C for 16 h. The solution was cooled to room temperature and concentrated *in vacuo,* and the residue was neutralized with NH₃ in MeOH (7M, 1 mL). The resulting mixture was concentrated *in vacuo* and purified by preparative HPLC to give **(I-33)** (10 mg, 46%) as a white solid. ESI-MS (M+H)+: 584.2, δ 11.20 (s, 1H), 8.56 (d, J = 0.9 Hz, 1H), 8.53 (d, J = 5.1 Hz, 1H), 8.27 (d, J = 0.9 Hz, 1H), 7.95 (s, 1H), 7.49 (d, J = 0.9 Hz, 1H), 7.38 (d, J = 1.4 Hz, 1H), 7.22 (d, J = 5.0 Hz, 1H), 5.49 (s, 2H), 4.90 (d, J = 10.3 Hz, 3H), 3.56 (d, J = 3.7 Hz, 4H), 2.67-2.64 (m, 1H), 2.59-2.54 (m, 1H), 2.42 (s, 3H), 2.00-1.94 (m, 1H), 1.61 - 1.50 (m, 2H), 1.06 - 0.87 (m, 2H), 0.76 - 0.60 (m, 2H).

### Intermediate 685: 8-bromo-2-(((2-chloro-6-methylpyridin-4-yl)oxy)methyl)-6-cyclopropyl imidazo[1,2-a]pyridine

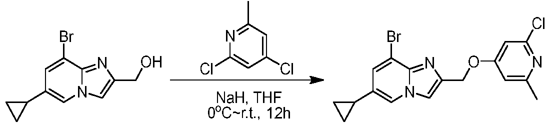

To mixture of **intermediate 453** (532 mg, 2.0 mmol) in dry THF (10.0 mL) was added NaH (120 mg, 3.0 mmol) at 0 °C . After stirring at 0 °C for 0.5 h, a solution of 2,4-dichloro-6-methylpyridine (483 mg, 3.0 mmol) was added, and the reaction mixture was stirred at room temperature for another 12h. The reaction was quenched with saturated aq. NH₄Cl (20 mL) and extracted with EtOAc (3 x 30 mL). The combined organic layers were washed with brine (30 mL), dried over anhydrous Na₂SO₄, and concentrated *in vacuo* to give the crude, which was purified by column chromatography (eluent: DCM/MeOH = 40/1) to give **intermediate 685** (160 mg, 20%) as a yellow solid. ESI-MS [M +H]⁺: 394.1.

### Intermediate 686: 1-(2-(((2-chloro-6-methylpyridin-4-yl)oxy)methyl)-6-cyclopropylimidazo[1,2-a]pyridin-8-yl)-3-methylimidazolidine-2,4-dione

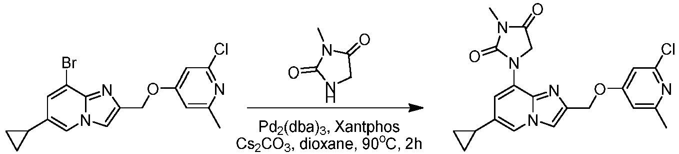

To a mixture of **intermediate 685** (50 mg, 0.13 mmol), 3-methylimidazolidine-2,4-dione (17.4 mg, 0.15 mmol), and Cs₂CO₃ (124 mg, 0.38 mmol) in dioxane (5 mL) were added Pd₂(dba)₃ (12 mg, 0.013 mmol) and Xantphos (15 mg, 0.026 mmol). After stirring at 90 °C for 2 h under N₂, the reaction mixture was filtered through Celite^{®} and the filter cake was washed with DCM/MeOH (10/1, 30 mL). The filtrate was concentrated *in vacuo* to give the crude, which was purified by preparative TLC (eluent: DCM/MeOH = 30/1) to give **intermediate 686** (30 mg, 56%) as a white solid. ESI-MS [M +H]⁺: 426.2.

The compounds in Table 5 were made in a similar manner to **intermediate 686** from appropriate starting materials. **Table 5**

| **Intermediate** | **Name / Analvtical Data** |
|---|---|
| 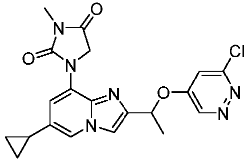 | **1-(2-(1-((6-chloropyridazin-4-yl)oxy)ethyl)-6-cyclopropylimidazo[1,2-a]pyridin-8-yl)-3-methylimidazolidine-2,4-dione** ESI-MS [M +H]⁺: 427.1. |
| 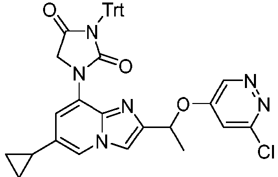 | **1-(2-(1-((6-chloropyridazin-4-yl)oxy)ethyl)-6-cyclopropylimidazo [1,2-a]pyridin-8-yl)-3-tritylimidazolidine-2,4-dione intermediate 687** ESI-MS [M +H]⁺: 655.2. |
| 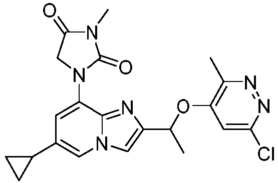 | **1-(2-(1-((6-chloro-3-methylpyridazin-4-yl)oxy)ethyl)-6-cyclopropylimidazo[1,2-a]pyridin-8-yl)-3-methylimidazolidine-2,4-dione intermediate 688** ESI-MS [M +H]⁺: 441.1. |
| 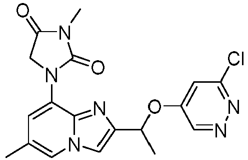 | **1-(2-(1-((6-chloropyridazin-4-yl)oxy)ethyl)-6-methylimidazo [1,2-a]pyridin-8-yl)-3-methylimidazolidine-2,4-dione** ESI-MS [M +H]⁺: 401.2. |
| 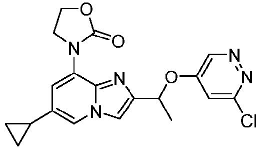 | **3-(2-(1-((6-chloropyridazin-4-yl)oxy)ethyl)-6-cyclopropylimidazo[1,2-a]pyridin-8-yl)oxazolidin-2-one intermediate 689** ESI-MS [M +H]⁺: 400.2. |
| 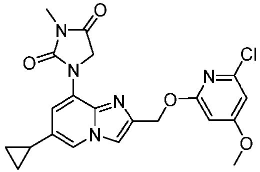 | **1-(2-(((6-chloro-4-methoxypyridin-2-yl)oxy)methyl)-6-cyclopropylimidazo[1,2-a]pyridin-8-yl)-3-methylimidazolidine-2,4-dione intermediate 690** ESI-MS [M +H]⁺: 442.1. |

### Example 34: (1S,2S)-N-(4-((6-cyclopropyl-8-(3-methyl-2,4-dioxoimidazolidin-1-yl)imidazo[1,2-a]pyridin-2-yl)methoxy)-6-methylpyridin-2-yl)-2-(4-methylpyrimidin-2-yl)cyclopropane-1-carboxamide (I-34)

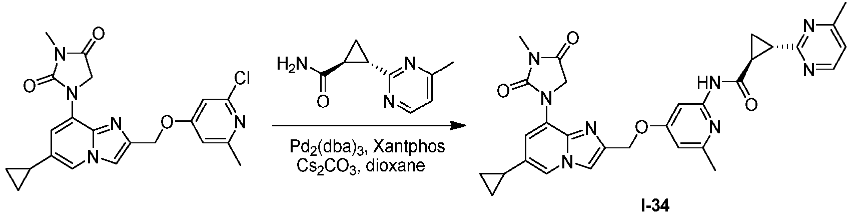

To a mixture of **intermediate 686** (40 mg, 0.094 mmol), (1S,2S)-2-(4-methyl pyrimidin-2-yl)cyclopropane-1-carboxamide (20 mg, 0.11 mmol), and Cs₂CO₃ (92 mg, 0.28 mmol) in dioxane (3 mL) were added Pd₂(dba)₃ (8.6 mg, 0.0094 mmol) and Xantphos (11 mg, 0.028 mmol). After stirring at 80 °C for 2 h under N₂, the reaction mixture was cooled to room temperature and filtered through Celite^{®}. The filter cake was washed with DCM/MeOH (10/1, 20 mL) and the filtrate was concentrated to give the crude, which was purified by preparative HPLC to give (I-34) (13 mg, 25%) as a white solid. ESI-MS [M +H]+: 567.2. δ 10.76 (s, 1H), 8.50 (dd, J = 5.1, 2.5 Hz, 1H), 8.27 (s, 1H), 7.94 (d, J = 2.3 Hz, 1H), 7.65 (s, 1H), 7.37 (s, 1H), 7.26 - 7.09 (m, 1H), 6.71 (s, 1H), 5.21 (s, 2H), 4.89 (d, J = 1.4 Hz, 2H), 2.95 (s, 3H), 2.60 - 2.58 (m, 2H), 2.39 (s, 3H), 2.31 (s, 3H), 1.99 - 1.89 (m, 1H), 1.49 - 1.47 (m, 2H), 0.98 - 0.88 (m, 2H), 0.69 - 0.60 (m, 2H)..

he compounds in Table 6 were prepared in a similar manner to **(I-34)** from **intermediate 55** and an appropriate coupling partner. **Table 6**

| **EG** | **Compound** | **Coupling Partner / Analytical Data** |
|---|---|---|
| **35** | 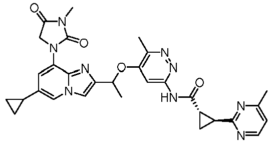 (1S,2S)-N-(5-(1-(6-cyclopropyl-8-(3-methyl-2,4-dioxo imidazolidine-1-yl)imidazo[1,2-a]pyridin-2-yl)ethoxy)-6-methyl pyridazin-3-yl)-2-(4-methyl pyrimidin-2-yl)cyclopropane-1-carboxamide (**I-35**) | **Intermediate 688** |
| | | ESI-MS [M +H]+: 582.2, δ 11.21 (s, 1H), 8.52 (t, J = 5.2 Hz, 1H), 8.22 (s, 1H), 8.16 (s, 1H), 7.92 (d, J = 3.6 Hz, 1H), 7.41 (d, J = 10.2 Hz, 1H), 7.20 (t, J = 4.8 Hz, 1H), 5.78 - 5.59 (m, 1H), 5.10 - 4.92 (m, 2H), 2.99 (d, J = 8.2 Hz, 3H), 2.62 - 2.60 (m, 2H), 2.47 - 2.36 (m, 6H), 1.99 - 1.91 (m, 1H), 1.75 (d, J = 6.4 Hz, 3H), 1.58 - 1.45 (m, 2H), 1.01 - 0.89 (m, 2H), 0.73 - 0.58 (m, 2H). |
| **36** | 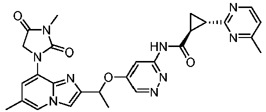 (1S,2S)-N-(5-(1-(6-methyl-8-(3-methyl-2,4-dioxoimidazolidin-1-yl)imidazo[1,2-a]pyridin-2-yl)ethoxy)pyridazin-3-yl)-2-(4-methylpyrimidin-2-yl)cyclopropane-1-carboxamide (I-36) | 1-(2-(1-((6-chloropyridazin-4-yl)oxy)ethyl)-6-methylimidazo[1,2-a]pyridin-8-yl)-3-methylimidazolidine-2,4-dione |
| | | ESI-MS [M + H]+: 542.2, δ 11.32 (s, 1H), 8.73 - 8.72 (m, 1H), 8.54 - 8.52 (m, 1H), 8.21 - 8.16 (m, 2H), 7.99 (d, J = 2.1 Hz, 1H), 7.50 - 7.48 (m, 1H), 7.22 - 7.20 (m, 1H), 5.82 - 5.77 (m, 1H), 5.02 - 4.96 (m, 2H), 2.99 (d, J = 7.1 Hz, 3H), 2.56 - 2.55 (m, 1H), 2.52 - 2.52 (m, 1H), 2.42 (d, J = 3.8 Hz, 3H), 2.27 (s, 3H), 1.74 (d, J = 6.4 Hz, 3H), 1.57 - 1.51 (m, 2H). |
| **37** | 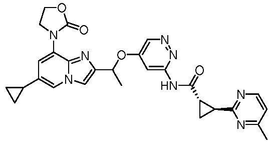 (1S,2S)-N-(5-(1-(6-cyclo propyl-8-(2-oxooxazolidin-3-yl) imidazo[1,2-a]pyridin-2-yl) ethoxy)pyridazin-3-yl)-2-(4-methylpyrimidin-2-yl)cyclo propane-1-carboxamide **(I-37)** | **Intermediate 689** |
| | | ESI-MS [M + H]+: 541.2, δ 11.34 (s, 1H), 8.75 - 8.74(m, 1H), 8.53 (d, J = 5.1 Hz, 1H), 8.24 - 8.23 (m, 1H), 8.10 - 8.08 (m, 1H), 7.92 (d, J = 2.9 Hz, 1H), 7.29 - 7.28 (m, 1H), 7.22 (d, J = 5.1 Hz, 1H), 5.84 - 5.79 (m, 1H), 4.53 - 4.43 (m, 4H), 2.56 - 2.55 (m, 1H), 2.52 - 2.52 (m, 1H), 2.42 (s, 3H), 1.97 - 1.93 (m, 1H), 1.73 (d, J = 6.4 Hz, 3H), 1.59 - 1.51 (m, 2H), 0.96 - 0.91 (m, 2H), 0.67 - 0.63 (m, 2H). |
| **38** | 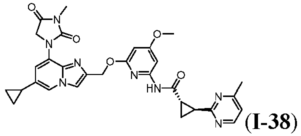 (1S,2S)-N-(6-((6-cyclo propyl-8-(3-methyl-2,4-dioxoimidazolidin -1-yl)imidazo[1,2-a]pyridin-2-yl)methoxy)-4-methoxy pyridin-2-yl)-2-(4-methyl pyrimidin-2-yl)cyclopropane-1-carboxamide | **Intermediate 690** |
| | | ESI-MS [M +H]+: 583.2. δ 10.65 (s, 1H), 8.53 (d, J = 5.1 Hz, 1H), 8.27 (s, 1H), 7.93 (s, 1H), 7.36 (d, J = 1.5 Hz, 2H), 7.21 (d, J = 5.2 Hz, 1H), 6.12 (d, J = 1.9 Hz, 1H), 5.39 (s, 2H), 4.88 (s, 2H), 3.78 (s, 3H), 2.97 (s, 3H), 2.52-2.51 (m, 2H), 2.42 (s, 3H), 1.98-1.92 (m, 1H), 1.67 - 1.50 (m, 2H), 0.97-0.92 (m, 2H), 0.73 - 0.60 (m, 2H). |

### Example 39: (1S,2S)-N-(5-(1-(6-cyclopropyl-8-(2,4-dioxoimidazolidin-1-yl)imidazo[1,2-a]pyridin-2-yl)ethoxy)pyridazin-3-yl)-2-(4-methylpyrimidin-2-yl)cyclopropane-1-carboxamide (I-39)

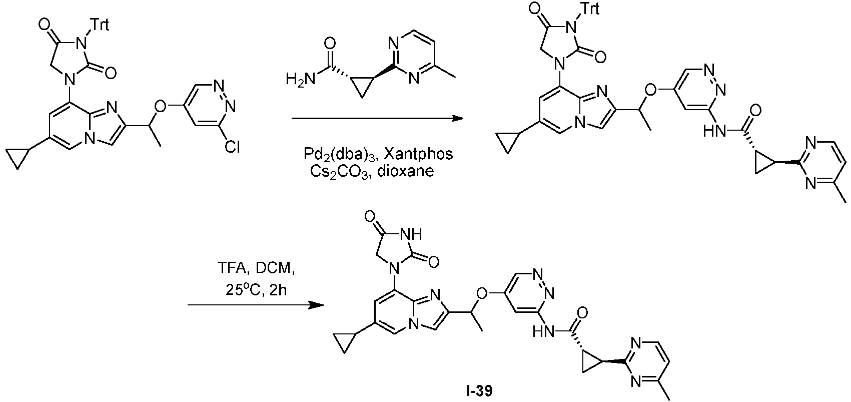

**Intermediate 691: *(1S,2S)-N-(5-(1-(6-cyclopropyl-8-(2,4-dioxo-3-tritylimidazolidin-1-yl)imidazo[1,2-a]pyridin-2-yl)ethoxy)pyridazin-3-yl)-2-(4-methylpyrimidin-2-yl)cyclopropane-1-carboxamide*** was prepared in a similar manner to **(I-34)** from **intermediate 55** and **intermediate 687.**

To a mixture of **intermediate 691** (100 mg, 0.12 mmol) in DCM (2 mL) was added TFA (1 mL). After stirring the reaction mixture at 25 °C for 2 h, the reaction mixture was diluted with DCM (30 mL) and washed with saturated aq. NaHCO₃ (10 mL), H₂O (10 mL), and brine (10 mL). The organic extract was dried over anhydrous Na₂SO₄ and concentrated to give crude (I-39), which was purified by preparative TLC (eluent: DCM/MeOH=10/1) to give **(I-39)** (30 mg, 43%) as a yellow solid. ESI-MS [M +H]+: 554.2, δ 11.40 - 11.27 (m, 2H), 8.75 - 8.54 (m, 1H), 8.54-8.52 (m, 1H), 8.22-8.21 (m, 1H), 8.16-8.07 (m, 1H), 7.92 (d, J = 2.4 Hz, 1H), 7.41-7.32 (m, 1H), 7.22-7.20 (m, 1H), 5.81-5.80 (m, 1H), 4.91 (d, J = 7.0 Hz, 2H), 2.65-2.62 (m, 1H), 2.58-2.56 (m, 1H), 2.42 (d, J = 3.2 Hz, 3H), 1.99 - 1.91 (m, 1H), 1.74-1.72 (m, 3H), 1.60 - 1.51 (m, 2H), 0.94-0.93 (m, 2H), 0.66-0.65 (m, 2H).

### Examples 40-42

### (1S,2S)-N-(5-(1-(6-cyclopropyl-8-(3-methyl-2,4-dioxoimidazolidin-1-yl)imidazo[1,2-a]pyridin-2-yl)ethoxy)pyridazin-3-yl)-2-(4-methylpyrimidin-2-yl)cyclopropane-1-carboxamide (I-40)

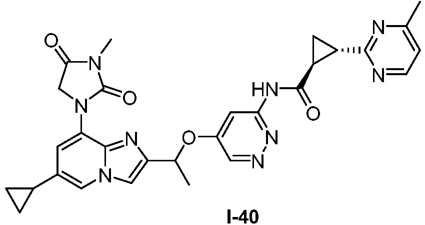

**(I-40)** ◆ was prepared in a similar manner to **(I-34)** as a mixture of diastereomers. ESI-MS [M+H]+: 568.3. δ 11.32 (s, 1H), 8.73 (d, J = 2.6 Hz, 1H), 8.56 - 8.50 (m, 1H), 8.26 - 8.21 (m, 1H), 8.18 - 8.14 (m, 1H), 7.94 (d, J = 3.1 Hz, 1H), 7.43 - 7.37 (m, 1H), 7.24 - 7.19 (m, 1H), 5.85 - 5.73 (m, 1H), 5.06 - 4.91 (m, 2H), 2.99 (d, J = 7.2 Hz, 3H), 2.58 - 2.56 (m, 2H), 2.42 (d, J = 3.7 Hz, 3H), 2.00 - 1.91 (m, 1H), 1.73 (d, J = 6.4 Hz, 3H), 1.58 - 1.50 (m, 2H), 0.98 - 0.92 (m, 2H), 0.68 - 0.61 (m, 2H).

The mixture was separated using SFC 80 (Daicel CHIRALPAK AS-H 20 x 250 mm, 5.0 µm, CO2/MeOH (0.2% NH3 (7M in MeOH)) = 65/35, 50 g/min, 35°C) to give two diastereomers: **(I-41)** & **(I-42)**

First eluting isomer, **(1S,2S)-N-(5-((S)-1-(6-cyclopropyl-8-(3-methyl-2,4-dioxo imidazolidin-1-yl)imidazo[1,2-a]pyridin-2-yl)ethoxy)pyridazin-3-yl)-2-(4-methylpyrimidin-2-yl)cyclopropane-1-carboxamide (I-41):** ESI-MS [M +H]+: 568.2, δ 11.32 (s, 1H), 8.73 (d, J = 2.7 Hz, 1H), 8.52 (d, J = 5.1 Hz, 1H), 8.25 - 8.22 (m, 1H), 8.16 (d, J = 2.7 Hz, 1H), 7.94 (s, 1H), 7.41 (d, J = 1.4 Hz, 1H), 7.21 (d, J = 5.1 Hz, 1H), 5.83 - 5.75 (m, 1H), 5.06 - 4.90 (m, 2H), 3.00 (s, 3H), 2.53 - 2.51 (m, 2H), 2.41 (s, 3H), 1.99 - 1.92 (m, 1H), 1.73 (d, J = 6.4 Hz, 3H), 1.57 - 1.51 (m, 2H), 0.97 - 0.92 (m, 2H), 0.67 - 0.63 (m, 2H). RT = 2.21 min, 100.0 % excess.

Second eluting isomer, **(1S,2S)-N-(5-((R)-1-(6-cyclopropyl-8-(3-methyl-2,4-dioxo imidazolidin-1-yl)imidazo[1,2-a]pyridin-2-yl)ethoxy)pyridazin-3-yl)-2-(4-methylpyrimidin-2-yl)cyclopropane-1-carboxamide (I-42):** ESI-MS [M +H]+: 568.3, δ 11.32 (s, 1H), 8.73 (d, J = 2.7 Hz, 1H), 8.53 (d, J = 5.1 Hz, 1H), 8.25 - 8.21 (m, 1H), 8.16 (d, J = 2.6 Hz, 1H), 7.94 (s, 1H), 7.39 (d, J = 1.4 Hz, 1H), 7.22 (d, J = 5.1 Hz, 1H), 5.82 - 5.77 (m, 1H), 4.95 (s, 2H), 2.98 (s, 3H), 2.55 - 2.53 (m, 2H), 2.42 (s, 3H), 1.98 - 1.92 (m, 1H), 1.73 (d, J = 6.4 Hz, 3H), 1.56 - 1.50 (m, 2H), 0.98 - 0.91 (m, 2H), 0.67 - 0.60 (m, 2H). RT = 3.33 min, 100.0 % excess.

### Intermediate 692: rac-(1S*,2S*)-2-(3-chloro-2-fluorophenyl)-N-(6-chloropyrimidin-4-yl)cyclopropane-1-carboxamide

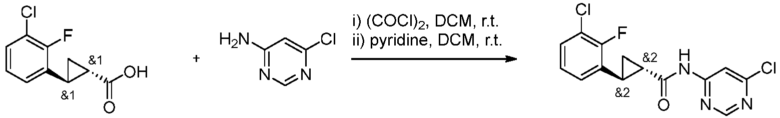

Oxalyl chloride (0.31 mL, 3.6 mmol) and DMF (2 drops) were added to a solution of *rac-*(1S*,2S*)-2-(3-chloro-2-fluorophenyl)cyclopropane-1-carboxylic acid (0.26 g, 1.2 mmol) in DCM (5 mL). The mixture was stirred at room temperature for 2 h then concentrated *in vacuo.* Residual oxalyl chloride was removed by azeotroping with toluene. The residue was redissolved in DCM (10 mL) and cooled to 0 °C. A suspension of **intermediate 846** (0.16 g, 1.2 mmol) and DIPEA (0.63 mL, 3.6 mmol) in DCM (15 mL) was added portionwise to the mixture. The mixture was allowed to warm to room temperature and stirred for 18 h. The mixture was washed with a solution of K₂CO₃ (sat. aq., 15 mL), dried (MgSO₄), filtered, and concentrated *in vacuo.* The residue was purified by column chromatography on silica gel, eluting with 5 % EtOAc in DCM to give **intermediate 692** (0.15 g, 40 %) as an orange-brown sticky residue. ESI-MS (M+H)+: 326, ¹H NMR (400 MHz, CDCl₃) δ 8.64 (s, 1H), 8.37 (s, 1H), 8.25 (s, 1H), 7.30 - 7.25 (m, 1H), 7.03 (m, 1H), 6.97 - 6.90 (m, 1H), 2.79 - 2.72 (m, 1H), 1.92 - 1.86 (m, 1H), 1.80 - 1.75 (m, 1H), 1.56 - 1.48 (m, 1H). Using a similar procedure to that used for the synthesis of **intermediate 692,** the compounds in Table 7 were prepared from **intermediate 846** and an appropriate acid coupling partner. **Table 7**

| **Structure** | **Name / Coupling Partner / Analytical Data** |
|---|---|
| 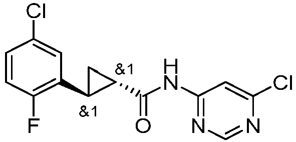 ***rac*-(1*S**,2*S**)-2-(5-chloro-2-fluoro phenyl)-*N*-(6-chloropyrimidin-4-yl)cyclopropane-1-carboxamide** | ***rac*-(1*S**,2*S**)-2-(5-chloro-2-fluorophenyl)cyclo propane-1-carboxylic acid** |
| | ESI-MS (M+H)+: 326 |
| | ¹H NMR (400 MHz, CDCl₃) δ 8.64 (s, 1H), 8.25 (s, 2H), 7.21 - 7.15 (m, 1H), 7.02 - 6.95 (m, 2H), 2.76 - 2.69 (m, 1H), 1.90 - 1.74 (m, 2H), 1.54 - 1.48 (m, 1H). |
| 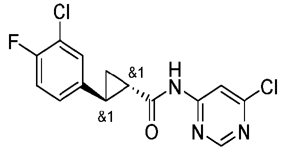 | ***rac*-(1*S**,2*S**)-2-(3-chloro-4-fluorophenyl)-*N*-(6-chloropyrimidin-4-yl)cyclopropane-1-carboxamide** from rac-(1*S**,2*S**)-2-(3-chloro-4-fluorophenyl)cyclopropane-1-carboxylic acid ESI-MS (M+H)+: 326 |
| 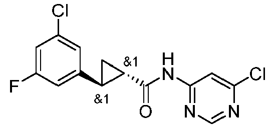 | ***rac*-(1*S**,2*S**)-2-(3-chloro-5-fluorophenyl)-*N*-(6-chloropyrimidin-4-yl)cyclopropane-1-carboxamide intermediate 693** from *rac*-(1*S**,2*S**)-2-(3-chloro-5-fluorophenyl)cyclopropane-1-carboxylic acid ESI-MS (M+H)+: 326 |
| 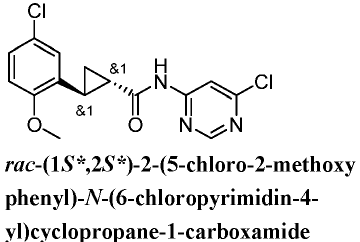 | **Intermeidate 46** |
| | ESI-MS (M+H)+: 337.9, ¹H NMR (400 MHz, CDCl₃) δ 8.63 (s, 1H), 8.33 (s, 1H), 8.26 (s, 1H), 7.21 - 7.15 (m, 1H), 6.94 - 6.91 (m, 1H), 6.79 (d, J=8.6 Hz, 1H), 3.85 (s, 3H), 2.75 - 2.67 (m, 1H), 1.74 - 1.69 (m, 2H), 1.50 - 1.45 (m, 1H). |
| 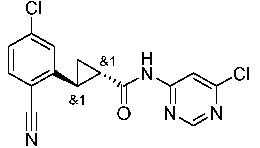 | **Intermediate 40: rac-(1*S**,2*S**)-2-(5-chloro-2-cyano phenyl)-*N*-(6-chloropyrimidin-4-yl)cyclopropane-1-carboxamide** ESI-MS (M+H)+: 331.0, δ 11.61 (s, 1H), 8.79 (d, J=1.0 Hz, 1H), 8.16 (d, J=1.0 Hz, 1H), 7.89 (d, J=8.4 Hz, 1H), 7.56 (dd, J=2.1, 8.3 Hz, 1H), 7.46 (d, J=2.1 Hz, 1H), 2.72 - 2.65 (m, 1H), 1.76 - 1.62 (m, 2H). 1H missing, presumed under DMSO peak. |
| 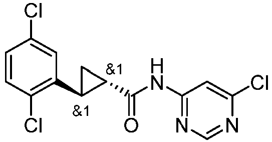 | **Intermediate** 694: *rac-*(1*S**,2*S**)-N-(6-chloro pyrimidin-4-yl)-2-(2,5-dichlorophenyl)cyclo propane-1-carboxamide from intermediate 35 ESI-MS (M+H)+: 342 |
| 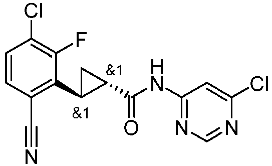 | **Intermediate** 695: rac-(1S*,2S*)-2-(3-chloro-6-cyano-2-fluorophenyl)-N-(6-chloropyrimidin-4-yl)cyclopropane-1-carboxamide from *rac*-(1*S**,2*S**)-2-(3-chloro-6-cyano-2-fluorophenyl)cyclopropane-1-carboxylic acid ESI-MS (M+H)+: 349.0 |
| 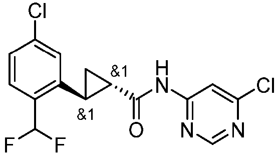 | **Intermediate 41: *rac*-(1*S**,2*S**)-2-(5-chloro-2-(difluoro methyl)phenyl)-*N*-(6-chloro pyrimidin-4-yl)cyclopropane-1-carboxamide** ESI-MS (M+H)+: 356.0, δ 11.62 - 11.59 (m, 1H), 8.83 - 8.81 (m, 1H), 8.20 (s, 1H), 7.66 - 7.61 (m, 4H), 7.54 - 7.40 (m, 4H), 7.66 - 7.19 (m, 4H), 7.34 - 7.32 (m, 4H), 2.72 - 2.67 (m, 1H), 1.69 - 1.59 (m, 2H). 1H missing, presumed under DMSO peak. |
| 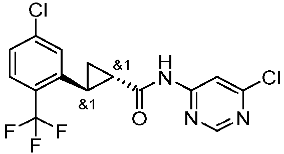 | ***rac*-(1*S**,2*S**)-2-(5-chloro-2-(trifluoromethyl) phenyl)-*N*-(6-chloropyrimidin-4-yl)cyclopropane-1-carboxamide** from *rac*-(1*S**,2*S**)-2-(5-chloro-2-(trifluoromethyl)phenyl) cyclopropane-1-carboxylic acid ESI-MS (M+H)+: 374 |
| 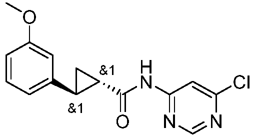 | ***rac*-(1*S**,2*S**)-N-(6-chloropyrimidin-4-yl)-2-(3-methoxyphenyl)cyclopropane-1-carboxamide** from *rac*-(1*S**,2*S**)-2-(3-methoxyphenyl)cyclopropane-1-carboxylic acid ESI-MS (M+H)+: 304.1 |
| 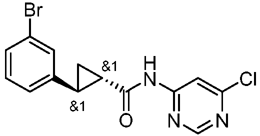 | ***rac*-(1*S**,2*S**)-2-(3-bromophenyl)-*N*-(6-chloro pyrimidin-4-yl)cyclopropane-1-carboxamide intermediate 696** from *rac*-(1*S**,2*S**)-2-(3-bromo phenyl)cyclopropane-1-carboxylic acid ESI-MS (M+H)+: 351.9, 353.9 |

### Example 43: rac-(1S*,2S*)-2-(3-chloro-2-fluorophenyl)-N-(6-(((6-cyclopropylimidazo[1,2-a]pyridin-2-yl)methyl)amino)pyrimidin-4-yl)cyclopropane-1-carboxamide (I-43)

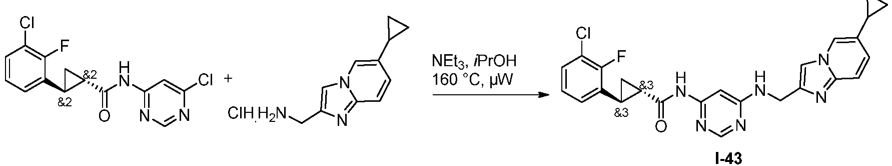

A mixture of **intermediate 692** (0.062 g, 0.19 mmol), intermediate 251 (0.036 g, 0.19 mmol), and DIPEA (0.099 mL, 0.57 mmol) in iPrOH (2.0 mL) was stirred at 140 °C in a microwave for 1.5 h. The mixture was concentrated *in vacuo.* The residue was redissolved in DCM and washed with a solution of Na₂CO₃ (sat. aq.). The organic layer was concentrated *in vacuo* and the residue was purified by reverse phase preparative HPLC to give **(I-43)** (0.035 g, 38 %) as a beige solid. ESI-MS (M+H)+: 477.3, δ 10.67 (s, 1H), 8.31 (s, 1H), 8.22 (s, 1H), 7.88 (br s, 1H), 7.62 (s, 1H), 7.49 - 7.44 (m, 1H), 7.38 (d, J=9.3 Hz, 1H), 7.29 (s, 1H), 7.22 - 7.12 (m, 2H), 6.97 (dd, J=1.8, 9.3 Hz, 1H), 4.58 (br s, 2H), 2.52 - 2.46 (m, 1H), 2.42 - 2.35 (m, 1H), 1.96 - 1.88 (m, 1H), 1.53 - 1.43 (m, 2H), 0.95 - 0.89 (m, 2H), 0.70 - 0.65 (m, 2H).
Using a similar procedure to that used **for(I-43),** the compounds in Table 8 were prepared from **intermediate 251** and and appropriate coupling partner. **Table 8**

| **EG** | **Compound** | **Coupling Partner / Analytical Data** |
|---|---|---|
| **44** | 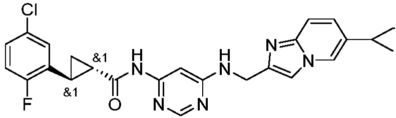 *rac*-(1*S*,*2*S**)-2-(5-chloro-2-fluorophenyl)-*N*-(6-(((6-cyclopropylimidazo[1,2-a]pyridin-2-yl)methyl)amino)pyrimidin-4-yl)cyclopropane-1-carboxamide **(I-44)** | *rac*-(1*S**,2*S**)-2-(5-chloro-2-fluorophenyl)-*N*-(6-chloropyrimidin-4-yl)cyclo propane-1-carboxamide |
| | | ESI-MS (M+H)+: 477.3, δ 10.64 (s, 1H), 8.31 (s, 1H), 8.22 (s, 1H), 7.88 (br s, 1H), 7.62 (s, 1H), 7.40 - 7.31 (m, 2H), 7.31 - 7.22 (m, 3H), 6.97 (dd, J=1.8, 9.3 Hz, 1H), 4.58 - 4.58 (br s, 2H), 2.47 - 2.38 (m, 2H), 1.96 - 1.88 (m, 1H), 1.53 - 1.44 (m, 2H), 0.95 - 0.89 (m, 2H), 0.70 - 0.65 (m, 2H). |
| **45** | 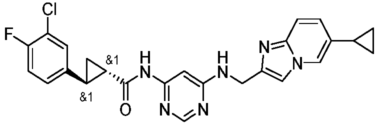 *rac-*(1*S*,*2*S**)-2-(3-chloro-4-fluoro phenyl)-*N*-(6-(((6-cyclopropyl imidazo[1,2-*a*]pyridin-2-yl)methyl) amino)pyrimidin-4-yl)cyclopropane-1-carboxamide **(I-45)** | *rac*-(1*S**,2*S**)-2-(3-chloro-4-fluorophenyl) -N-(6-chloropyrimidin-4-yl)cyclo propane-1-carboxamide ESI-MS (M+H)+: 477.3, δ 10.61 (s, 1H), 8.34 (s, 1H), 8.21 (s, 1H), 7.95 - 7.95 (m, 1H), 7.74 (s, 1H), 7.36 - 7.30 (m, 1H), 7.28 - 7.25 (m, 3H), 7.21 - 7.14 (m, 2H), 4.60 (br s, 2H), 2.46 - 2.36 (m, 2H), 1.98 - 1.90 (m, 1H), 1.52 - 1.38 (m, 2H), 0.96 - 0.90 (m, 2H), 0.74 - 0.69 (m, 2H). |
| **46** | 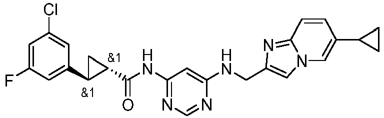 *rac*-(1*S**,2*S**)-2-(3-chloro-5-fluorophenyl)-*N*-(6-(((6-cyclopropyl imidazo[1,2-*a*]pyridin-2-yl)methyl) amino)pyrimidin-4-yl)cyclopropane-1-carboxamide **(I-46)** | **Intermediate 693** ESI-MS (M+H)+: 477.2, δ 10.59 (s, 1H), 8.31 (s, 1H), 8.21 (s, 1H), 7.87 (br s, 1H), 7.62 (s, 1H), 7.38 (d, J=9.3 Hz, 1H), 7.29 - 7.23 (m, 2H), 7.17 (s, 1H), 7.09 (d, J=9.7 Hz, 1H), 6.97 (dd, J=1.8, 9.3 Hz, 1H), 4.57 (br s, 2H), 2.49 - 2.34 (m, 2H), 1.96 - 1.88 (m, 1H), 1.53 - 1.44 (m, 2H), 0.95 - 0.89 (m, 2H), 0.70-0.65 (m,2H). |
| **47** | 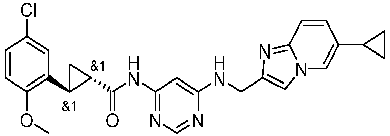 *rac-*(1*S**,2*S**)-2-(5-chloro-2-methoxyphenyl)-*N*-(6-(((6-cyclopropylimidazo[1,2-*a*]pyridin-2-yl)methyl)amino)pyrimidin-4-yl)cyclopropane-1-carboxamide **(I-47)** | *rac*-(1*S**,2*S**)-2-(5-chloro-2-methoxy phenyl)-*N*-(6-chloropyrimidin-4-yl)cyclo propane-1-carboxamide ESI-MS (M+H)+: 489.4, δ 10.57 (s, 1H), 8.31 (s, 1H), 8.21 (s, 1H), 7.86 (br s, 1H), 7.62 (s, 1H), 7.38 (d, J=9.3 Hz, 1H), 7.31 - 7.23 (m, 2H), 7.03 - 6.95 (m, 3H), 4.58 (br s, 2H), 3.81 (s, 3H), 2.53 - 2.47 (m, 1H), 2.35 - 2.28 (m, 1H), 1.96 - 1.88 (m, 1H), 1.44 - 1.34 (m, 2H), 0.95 - 0.89 (m, 2H), 0.70 - 0.65 (m, 2H). |
| **48** | 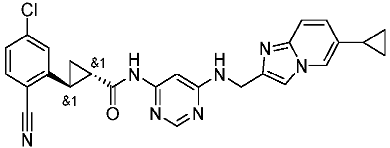 *rac-*(1*S**,2*S**)-2-(5-chloro-2-cyano phenyl)-*N*-(6-(((6-cyclopropylimidazo [1,2-*a*]pyridin-2-yl)methyl)amino) pyrimidin-4-yl)cyclopropane-1-carboxamide, formic acid salt **(I-48)** | *rac*-(1*S**,2*S**)-2-(5-chloro-2-cyanophenyl)-*N*-(6-chloropyrimidin-4-yl)cyclopropane-1-carboxamide ESI-MS (M+H)+: 484.3, δ 10.67 - 10.62 (m, 1H), 8.33 - 8.28 (m, 1H), 8.21 (s, 1H), 7.86 (d, J=8.1 Hz, 2H), 7.61 (s, 1H), 7.52 (dd, J=1.4, 8.2 Hz, 1H), 7.40 (s, 1H), 7.36 (d, J=9.3 Hz, 1H), 7.28 (s, 1H), 6.95 (dd, J=1.0, 9.3 Hz, 1H), 4.57 (br s, 2H), 2.62 - 2.56 (m, 1H), 1.95 - 1.86 (m, 1H), 1.61 - 1.53 (m, 2H), 0.94 - 0.87 (m, 2H), 0.69 - 0.62 (m, 2H). One CH of cyclopropyl group obscured by DMSO peak. |
| **49** | 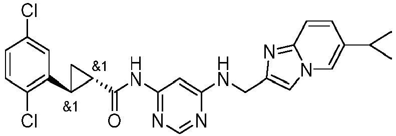 *rac*-(1*S**,2*S**)-*N*-(6-(((6-cyclopropyl imidazo[1,2-*a*]pyridin-2-yl)methyl) amino) pyrimidin-4-yl)-2-(2,5-dichlorophenyl)cyclopropane-1-carboxamide, formic acid salt **(I-49)** | **Intermediate 694** ESI-MS (M+H)+: 493.4, δ 10.69 (s, 1H), 8.51 (s, 1H), 8.35 (s, 1H), 8.26 (s, 1H), 7.93 - 7.88 (m, 1H), 7.67 (s, 1H), 7.55 (d, J=8.6 Hz, 1H), 7.41 - 7.35 (m, 4H), 7.01 (d, J=9.3 Hz, 1H), 4.63 (br s, 2H), 2.41 - 2.36 (m, 1H), 2.00 - 1.93 (m, 1H), 1.53 (t, J=6.3 Hz, 2H), 0.99 - 0.94 (m, 2H), 0.74 - 0.68 (m, 2H). |
| **50** | 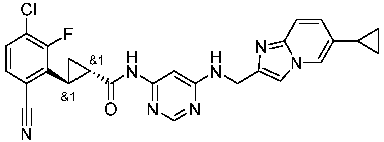 *rac-*(1*S**,2*S**)-2-(3-chloro-6-cyano-2-fluorophenyl)-*N*-(6-(((6-cyclopropyl imidazo[1,2-*a*]pyridin-2-yl)methyl) amino)pyrimidin-4-yl)cyclopropane-1-carboxamide, formic acid salt **(I-50)** | **Intermediate 695** ESI-MS (M+H)+: 502.3, δ 10.75 (s, 1H), 8.31 - 8.20 (m, 3H), 7.87 - 7.85 (m, 1H), 7.75 - 7.73 (m, 2H), 7.61 (s, 1H), 7.37 (d, J=9.3 Hz, 1H), 7.30 - 7.29 (m, 1H), 6.97 - 6.94 (m, 1H), 4.63 - 4.52 (m, 2H), 1.94 - 1.86 (m, 1H), 1.58 - 1.52 (m, 2H), 0.94 - 0.87 (m, 2H), 0.69 - 0.63 (m, 2H). Two CH of cyclopropyl group obscured by DMSO peak. |
| **51** | 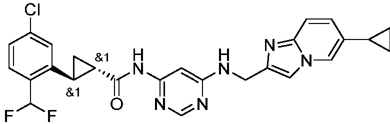 *rac*-(1*S**,2*S**)-2-(5-chloro-2-(di fluoro methyl)phenyl)-*N*-(6-(((6-cyclo propylimidazo[1,2-*a*]pyridin-2- yl)methyl)amino)pyrimidin-4- yl)cyclopropane-1-carboxamide **(I-51)** | *rac*-(1*S**,2*S**)-2-(5-chloro-2-(difluoro methyl) phenyl)-*N*-(6-chloropyrimidin-4- yl)cyclopropane-1-carboxamide |
| | | ESI-MS (M+H)+: 509.3, δ 10.63 - 10.60 (m, 1H), 8.30 - 8.28 (m, 1H), 8.20 (d, J=0.7 Hz, 1H), 7.86 (br s, 1H), 7.61 (s, 1H), 7.57 (d, J=8.4 Hz, 1H), 7.45 (q, J=3.3 Hz, 1H), 7.41 - 7.12 (m, 4H), 6.95 (dd, J=1.8, 9.3 Hz, 1H), 4.57 (br s, 2H), 2.58 - 2.54 (m, 1H), 2.45 - 2.38 (m, 1H), 1.94 - 1.87 (m, 1H), 1.51 - 1.43 (m, 2H), 0.93 - 0.87 (m, 2H), 0.68 - 0.63 (m, 2H). |
| **52** | 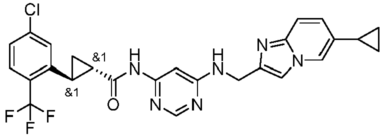 *rac-*(1*S*,*2*S**)-2-(5-chloro-2-(trifluoro methyl)phenyl)-N-(6-(((6-cyclopropyl imidazo[1,2-*a*]pyridin-2-yl)methyl) amino)pyrimidin-4-yl)cyclopropane-1- carboxamide **(I-52)** | rac-(1*S**,2*S**)-2-(5-chloro-2-(trifluoro methyl)phenyl)-N-(6-chloropyrimidin-4- yl)cyclopropane-1-carboxamide |
| | | ESI-MS (M+H)+: 527.4, δ 10.68 (s, 1H), 8.35 (s, 1H), 8.26 (s, 1H), 7.92 - 7.89 (m, 1H), 7.79 (d, J=8.1 Hz, 1H), 7.67 (s, 1H), 7.59 (d, J=8.3 Hz, 1H), 7.50 (s, 1H), 7.42 (d, J=9.6 Hz, 1H), 7.34 (s, 1H), 7.01 (d, J=9.3 Hz, 1H), 4.63 (br s, 2H), 2.49 - 2.46 (m, 1H), 1.99 - 1.92 (m, 1H), 1.71 - 1.66 (m, 1H), 1.56 - 1.50 (m, 1H), 0.99 - 0.94 (m, 2H), 0.74 - 0.68 (m, 2H). |
| 53 | 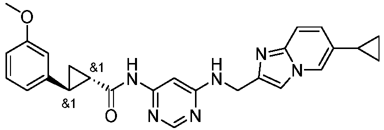 *rac-*(1*S**,2*S**)-*N*-(6-(((6-cyclopropyl imidazo[1,2-a]pyridin-2-yl)methyl) amino)pyrimidin-4-yl)-2-(3-methoxy phenyl)cyclopropane-1-carboxamide **(I-53)** | *rac*-(1*S**,2*S**)-*N*-(6-chloropyrimidin-4-yl)- 2-(3-methoxyphenyl)cyclopropane-1- carboxamide ESI-MS (M+H)+: 455.3, δ 10.64 (s, 1H), 8.35 (s, 1H), 8.24 (s, 1H), 7.89 (s, 1H), 7.66 (s, 1H), 7.42 (d, J=9.3 Hz, 1H), 7.32 (s, 1H), 7.25 (t, J=8.1 Hz, 1H), 7.01 (dd, J=1.5, 9.3 Hz, 1H), 6.84 - 6.75 (m, 3H), 4.59 (m, 2H), 3.80 - 3.78 (m, 3H), 2.44 - 2.37 (m, 2H), 2.00 - 1.92 (m, 1H), 1.53 - 1.36 (m, 2H), 1.00 - 0.93 (m, 2H), 0.74 - 0.68 (m, 2H). |
| **54** | 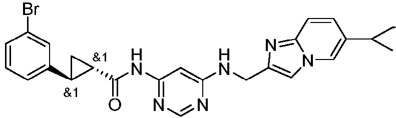 *rac*-(1*S**,*2S*)*-2-(3-bromophenyl)-*N*-(⁶- (((6-cyclopropylimidazo[1,2-*a*]pyridin-2-yl)methyl)amino)pyrimidin-4-yl)cyclopropane-1-carboxamide **(I-54)** | **Intermediate 696** ESI-MS (M+H)+: |
| | | 503.2, 505.2, δ 10.60 (s, 1H), 8.32 - 8.30 (m, 1H), 8.23 - 8.20 (m, 1H), 7.91 - 7.82 (m, 1H), 7.62 (s, 1H), 7.42 - 7.37 (m, 3H), 7.30 - 7.24 (m, 2H), 7.22 - 7.18 (m, 1H), 6.99 - 6.95 (m, 1H), 4.62 - 4.52 (m, 2H), 2.45 - 2.35 (m, 2H), 1.95 - 1.89 (m, 1H), 1.52 - 1.45 (m, 1H), 1.44 - 1.38 (m, 1H), 0.95 - 0.89 (m, 2H), 0.70 - 0.65 (m, 2H). |

### Examples 55-57

### rac-(1S*,2S*)-2-(3-chlorophenyl)-N-(6-(((6-cyclopropylimidazo[1,2-a]pyridin-2-yl)methyl)amino) pyrimidin-4-yl)cyclopropane-1-carboxamide (I-55)

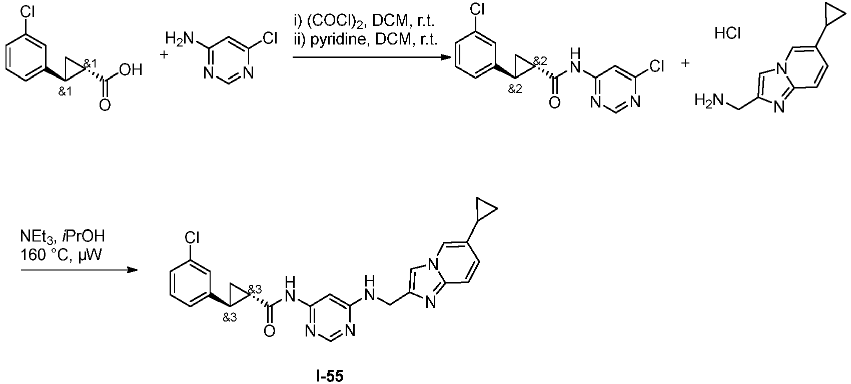

Oxalyl chloride (0.53 mL, 6.10 mmol) was added to a stirred solution of *intermediate 1149* (0.40 g, 2.0 mmol) and DMF (2 drops) in DCM (8.0 mL). The mixture was stirred at room temperature for 1 h then concentrated *in vacuo.* The residue was re-dissolved in DCM (20 mL), and **intermediate 846** (0.27 g, 2.1 mmol) and pyridine (0.25 mL, 3.1 mmol) were added. The mixture was stirred at room temperature under a nitrogen atmosphere for 18 h. The mixture was diluted with DCM (100 mL), washed with water (50 mL), dried over MgSO₄, filtered, and concentrated *in vacuo* to give the title compound (0.61 g, 96 %) as a yellow solid. ESI-MS (M+H)+: 308.1, δ 11.57 (s, 1H), 8.80 (s, 1H), 8.19 (s, 1H), 7.39 (m, 1H), 7.35 - 7.30 (m, 2H), 7.23 (d, J=7.3 Hz, 1H), 2.49 - 2.44 (m, 1H), 1.64 - 1.54 (m, 2H), 1H hidden under solvent peaks.

NEt₃ (0.063 mL, 0.13 mmol) was added to a suspension of rac-(1S*,2S*)-2-(3-chloro phenyl)-N-(6-chloropyrimidin-4-yl)cyclopropane-1-carboxamide (0.040 g, 0.13 mmol) and (6-cyclo propylimidazo[1,2-a]pyridin-2-yl)methanamine hydrochloride (0.029 g, 0.13 mmol) in iPrOH (1.0 mL). The mixture was stirred at 160°C in a microwave reactor for 1.5 h. The mixture was concentrated in vacuo. The residue was re-dissolved in DCM and washed with water. The aqueous layer was extracted with DCM (2 ×) and the combined organic layers were passed through a hydrophobic frit and concentrated in vacuo. The residue was purified by reverse phase preparative HPLC to give **(I-55)** (0.036 g, 61 %) as a beige solid (mixture of enantiomers). ESI-MS (M+H): 459.3 H NMR (400 MHz, DMSO) δ 10.60 (s, 1H), 8.29 (d, J=9.8 Hz, 1H), 8.21 (s, 1H), 7.86 (br s, 1H), 7.62 (s, 1H), 7.40 - 7.30 (m, 2H), 7.28 - 7.25 (m, 3H), 7.16 (d, J=7.7 Hz, 1H), 6.97 (dd, J=1.8, 9.3 Hz, 1H), 4.57 (br s, 2H), 2.45 - 2.34 (m, 2H), 1.96 - 1.88 (m, 1H), 1.52 - 1.38 (m, 2H), 0.95 - 0.89 (m, 2H), 0.70 - 0.65 (m, 2H).

The mixture was separated using SFC (LUX Cellulose-4 20x250 mm, 5 µm 55/45 MeOH (0.1 % DEA) / CO₂, 70 mL/min, 120 bar, 40°C) to give two enantiomers.

First eluting isomer, **(1*R*,2*R*)-2-(3-chlorophenyl)-*N-*(6-(((6-cyclopropylimidazo[1,2-*a*]pyridin-2-yl)methyl)amino)pyrimidin-4-yl)cyclopropane-1-carboxamide (I-56):** ESI-MS (M+H): 459.3, δ 10.58 (s, 1H), 8.29 (s, 1H), 8.19 (s, 1H), 7.85 - 7.84 (m, 1H), 7.60 (s, 1H), 7.39 - 7.28 (m, 2H), 7.26 (d, J=5.1 Hz, 3H), 7.15 (d, J=7.6 Hz, 1H), 6.98 - 6.94 (m, 1H), 4.56 - 4.55 (m, 2H), 2.41 - 2.35 (m, 2H), 1.95 - 1.86 (m, 1H), 1.50 - 1.36 (m, 2H), 0.94 - 0.87 (m, 2H), 0.69 - 0.62 (m, 2H). RT = 1.44 min, 99.3 % e.e.

Second eluting isomer, **(*1*S*,*2*S*)-2-(3-chlorophenyl)-*N*-(6-(((6-cyclopropylimidazo[1,2-*a*]pyridin-2-yl)methyl)amino)pyrimidin-4-yl)cyclopropane-1-carboxamide (I-57):** ESI-MS (M+H): 459.3 ¹H NMR (400 MHz, DMSO) δ 10.60 (s, 1H), 8.31 (s, 1H), 8.21 (s, 1H), 7.86 (br s, 1H), 7.62 (s, 1H), 7.40 - 7.30 (m, 2H), 7.28 - 7.25 (m, 3H), 7.16 (d, J=7.7 Hz, 1H), 6.97 (dd, J=1.7, 9.3 Hz, 1H), 4.57 (br s, 2H), 2.45 - 2.36 (m, 2H), 1.96 - 1.88 (m, 1H), 1.51 - 1.38 (m, 2H), 0.95 - 0.89 (m, 2H), 0.70 - 0.65 (m, 2H). RT = 3.93 min, 98.2 % e.e.

Using a similar procedure to that used for **(I-55),** the compounds in Table 9 were prepared from **intermediate 697** and an appropriate coupling partner. **Table** 9

| **EG** | **Compound** | **Coupling Partner /Analytical Data** |
|---|---|---|
| **58** | 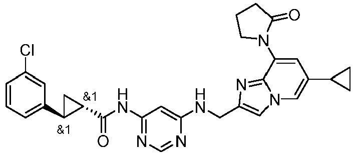 *rac*-(1*S**,2*S**)-2-(3-chlorophenyl)-*N*-(6-(((6-cyclopropyl-8-(2-oxopyrrolidin-1-yl)imidazo[1,2-*a*]pyridin-2-yl)methyl) amino)pyrimidin-4-yl)cyclopropane-1-carboxamide **(I-58)** | **Intermediate 315** ESI-MS (M+H): 542.3, δ 10.62 (s, 1H), 8.26 (s, 1H), 8.22 (s, 1H), 7.90 (s, 1H), 7.67 (s, 1H), 7.36 - 7.24 (m, 4H), 7.18 (d, J=7.6 Hz, 1H), 7.14 (s, 1H), 4.58 (br s, 2H), 4.17 (m, 2H), 2.51 - 2.39 (m, 3H), 2.20 - 2.09 (m, 2H), 1.98 - 1.90 (m, 1H), 1.52 - 1.39 (m, 2H), 0.98 - 0.91 (m, 2H), 0.67 (m, 2H). |
| **59** | 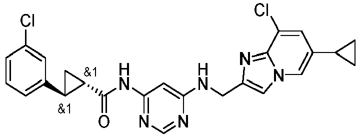 *rac*-(1*S**,2*S**)-*N*-(6-(((8-chloro-6-cyclo propylimidazo[1,2-*a*]pyridin-2-yl) methyl)amino)pyrimidin-4-yl)-2-(3-chlorophenyl)cyclopropane-1-carboxamide **(I-59)** | (8-chloro-6-cyclopropylimidazo[1,2*-a*] pyridin-2-yl)methanamine |
| | | ESI-MS (M+H): 493.3, δ 10.61 (s, 1H), 8.34 (s, 1H), 8.21 (s, 1H), 7.95 (m, 1H), 7.74 (s, 1H), 7.36 - 7.30 (m, 1H), 7.28 - 7.25 (m, 3H), 7.21 - 7.14 (m, 2H), 4.60 (br s, 2H), 2.46 - 2.36 (m, 2H), 1.98 - 1.90 (m, 1H), 1.52 - 1.38 (m, 2H), 0.96 - 0.90 (m, 2H), 0.74 - 0.69 (m, 2H). |
| **60** | 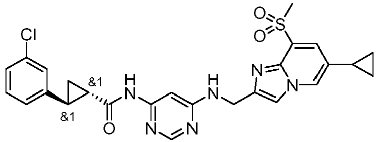 *rac*-(1*S**,2*S**)-2-(3-chlorophenyl)-*N*-(6-(((6-cyclopropyl-8-(methylsulfonyl) imidazo[1,2-*a*]pyridin-2-yl)methyl) amino)pyrimidin-4-yl)cyclopropane-1-carboxamide **(I-60)** | (6-cyclopropyl-8-(methylsulfonyl)imidazo [1,2-*a*]pyridin-2-yl)methanamine |
| | | ESI-MS (M+H): 537.2, δ10.66 (s, 1H), 8.68 (s, 1H), 8.25 (s, 1H), 8.03 (s, 1H), 7.86 (s, 1H), 7.62 (s, 1H), 7.40 - 7.18 (m, 5H), 4.70 (s, 2H), 3.59 (s, 3H), 2.48 - 2.40 (m, 2H), 2.16 - 2.08 (m, 1H), 1.56 - 1.43 (m, 2H), 1.06 - 0.99 (m, 2H), 0.79 - 0.73 (m, 2H). |
| **61** | 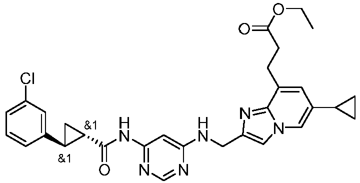 *rac*-ethyl 3-(2-(((6-((1*S**,2*S**)-2-(3-chlorophenyl)cyclopropane-1-carb oxamido)pyrimidin-4-yl)amino) methyl)-6-cyclopropylimidazo[1,2-*a*]pyridin-8-yl)propanoate **(I-61)** | **Intermediate 273** ESI-MS (M+H): 559.4, δ 10.60 (s, 1H), 8.18 (d, J=8.3 Hz, 2H), 7.90 (br s, 1H), 7.58 (s, 1H), 7.34 - 7.24 (m, 4H), 7.15 (d, J=6.8 Hz, 1H), 6.78 (s, 1H), 4.57 (br s, 2H), 4.06 (q, J=7.1 Hz, 2H), 3.10 - 3.05 (m, 2H), 2.83 - 2.77 (m, 2H), 2.43 - 2.35 (m, 2H), 1.89 - 1.82 (m, 1H), 1.48 - 1.37 (m, 2H), 1.16 (t, J=7.1 Hz, 3H), 0.91 - 0.86 (m, 2H), 0.66 - 0.61 (m, 2H). |
| **62** | 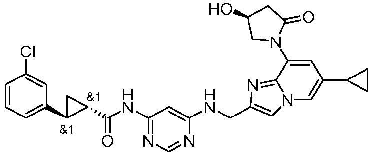 *rac-*(1*S**,2*S**)-2-(3-chlorophenyl)-*N*-(6-(((6-cyclopropyl-8-((*S*)-4-hydroxy-2-oxopyrrolidin-1-yl)imidazo[1,2-*a*]pyridin-2-yl)methyl)amino)pyrimidin-4-yl)cyclopropane-1-carboxamide **(I-62)** | (*S*)-1-(2-(aminomethyl)-6-cyclopropyl imidazo[1,2-*a*]pyridin-8-yl)-4-hydroxy pyrrolidin-2-one ESI-MS (M+H): 558.4, δ 10.61 (s, 1H), 8.23 (d, J=1.4 Hz, 1H), 8.21 (s, 1H), 7.90 (s, 1H), 7.66 (s, 1H), 7.35 - 7.15 (m, 6H), 5.37 (s, 1H), 4.58 (s, 2H), 4.49 - 4.43 (m, 2H), 4.00 - 3.96 (m, 1H), 2.86 - 2.78 (m, 1H), 2.45 - 2.37 (m, 2H), 2.34 - 2.28 (m, 1H), 1.97 - 1.90 (m, 1H), 1.49 (t, J=9.0 Hz, 1H), 1.45 - 1.38 (m, 1H), 0.96 - 0.91 (m, 2H), 0.68 - 0.62 (m, 2H) |
| **63** | 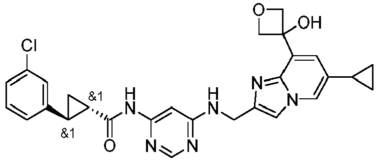 *rac*-(1*S**,2*S**)-2-(3-chlorophenyl)-*N*-(6-(((6-cyclopropyl-8-(3-hydroxyoxetan-3-yl)imidazo[1,2-*a*]pyridin-2-yl)methyl) amino)pyrimidin-4-yl)cyclopropane-1-carboxamide **(I-63)** | (6-cyclopropyl-8-(3-hydroxyoxetan-3-yl)imidazo[1,2-a]pyridin-2-yl)methanamine |
| | | ESI-MS (M+H): 3.3, δ 10.62 (s, 1H), 8.28 (d, J=1.6 Hz, 1H), 8.21 (s, 1H), 7.89 (s, 1H), 7.65 (s, 1H), 7.36 - 7.25 (m, 4H), 7.16 (d, J=8.0 Hz, 1H), 7.05 (d, J=1.8 Hz, 1H), 6.49 (s, 1H), 5.25 (d, J=6.6 Hz, 2H), 4.68 (d, J=6.6 Hz, 2H), 4.59 (s, 2H), 2.45 - 2.35 (m, 2H), 1.99 - 1.91 (m, 1H), 1.52 - 1.45 (m, 1H), 1.45 - 1.39 (m, 1H), 0.96 - 0.90 (m, 2H), 0.72 - 0.67 (m, 2H). |
| **64** | 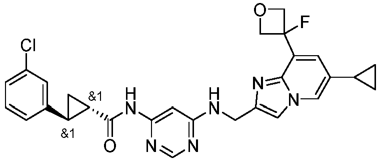 *rac*-(1*S**,2*S**)-2-(3-chlorophenyl)-*N*-(6-(((6-cyclopropyl-8-(3-fluorooxetan-3-yl)imidazo[1,2-*a*]pyridin-2-yl)methyl) amino)pyrimidin-4-yl)cyclo propane-1- carboxamide **(I-64)** | (6-cyclopropyl-8-(3-fluorooxetan-3-yl)imidazo[1,2-*a*]pyridin-2-yl)methanamine |
| | | ESI-MS (M+H): 533.4, δ 10.65 (s, 1H), 8.44 (s, 1H), 8.25 (s, 1H), 7.93 (br s, 1H), 7.73 (s, 1H), 7.38 - 7.29 (m, 4H), 7.23 - 7.15 (m, 2H), 5.40 (dd, J=8.8, 26.3 Hz, 2H), 5.03 (dd, J=8.7, 22.4 Hz, 2H), 4.63 (br s, 2H), 2.45 - 2.38 (m, 2H), 2.04 - 1.96 (m, 1H), 1.54 - 1.43 (m, 2H), 0.98 (m, 2H), 0.80 - 0.74 (m, 2H). |
| 65 | 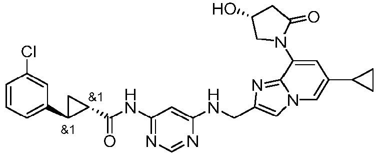 *rac*-(1*S**,2*S**)-2-(3-chlorophenyl)-N-(6- (((6-cyclopropyl-8-((*R*)-4-hydroxy-2- oxopyrrolidin-1-yl)imidazo[1,2-*a*] pyridin-2-yl)methyl)amino)pyrimidin-4- yl)cyclopropane-1-carboxamide **(I-65)** | (*R*)-1-(2-(aminomethyl)-6-cyclopropyl imidazo[1,2-*a*]pyridin-8-yl)-4-hydroxy pyrrolidin-2-one ESI-MS (M+H): 558.4, δ 10.65 (s, 1H), 8.27 (d, J=7.9 Hz, 2H), 7.94 (s, 1H), 7.70 (s, 1H), 7.38 - 7.20 (m, 6H), 5.40 (d, J=2.5 Hz, 1H), 4.62 (s, 2H), 4.52 - 4.47 (m, 2H), 4.01 (d, J=8.4 Hz, 1H), 2.90 - 2.83 (m, 1H), 2.48 - 2.33 (m, 3H), 2.00 - 1.94 (m, 1H), 1.55 - 1.43 (m, 2H), 1.01-0.95 (m,2H), 0.72-0.67 (m,2H). |
| **66** | 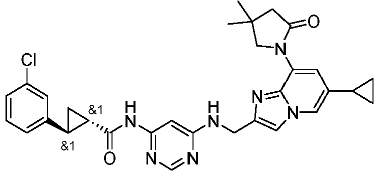 *rac*-(1*S**,2*S**)-2-(3-chlorophenyl)-N-(6- (((6-cyclopropyl-8-(4,4- dimethyl-2- oxopyrrolidin-1-yl)imidazo[1,2-*a*] pyridin-2-yl)methyl)amino)pyrimidin-4- yl)cyclopropane-1-carboxamide **(I-66)** | 1-(2-(aminomethyl)-6-cyclopropyl imidazo[1,2-*a*]pyridin-8-yl)-4,4-dimethyl pyrrolidin-2-one ESI-MS (M+H): 570.5, δ 10.61 (s, 1H), 8.26 - 8.20 (m, 2H), 7.88 (s, 1H), 7.65 (s, 1H), 7.36 - 7.26 (m, 4H), 7.17 - 7.14 (m, 2H), 4.57 (s, 2H), 3.90 (s, 2H), 2.45 - 2.37 (m, 2H), 2.34 (s, 2H), 1.96 - 1.89 (m, 1H), 1.51 - 1.37 (m, 2H), 1.22 (s, 6H), 0.96 - 0.90 (m, 2H), 0.68 - 0.63 (m, 2H). |
| **67** | 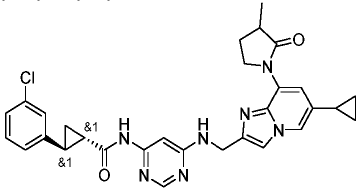 *rac*-(1*S**,2*S**)-2-(3-chlorophenyl)-N-(6- (((6-cyclopropyl-8-(3-methyl-2-oxo pyrrolidin-1-yl)imidazo[1,2-*a*]pyridin-2- yl)methyl)amino)pyrimidin-4-yl)cyclo propane-1-carboxamide **(I-67)** | 1-(2-(aminomethyl)-6-cyclopropyl imidazo [1,2-*a*]pyridin-8-yl)-3-methyl pyrrolidin-2-one ESI-MS (M+H): 556.3, δ 10.59 (s, 1H), 8.23 (s, 1H), 8.19 (s, 1H), 7.64 (s, 1H), 7.34 - 7.24 (m, 4H), 7.15 (d, J=8.3 Hz, 2H), 4.55 (br s, 2H), 4.17 - 4.01 (m, 2H), 2.68 - 2.57 (m, 1H), 2.44 - 2.31 (m, 3H), 1.95 - 1.87 (m, 1H), 1.80 - 1.68 (m, 1H), 1.50 - 1.37 (m, 2H), 1.18 (d, J=7.1 Hz, 3H), 0.95 - 0.89 (m, 2H), 0.66 - 0.61 (m, 2H). |
| **68** | 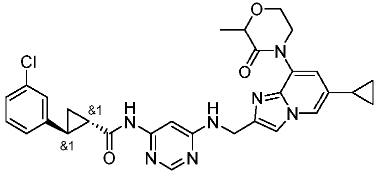 *rac*-(1*S**,2*S**)-2-(3-chlorophenyl)-N-(6- (((6-cyclopropyl-8-(2-methyl-3-oxo morpholino)imidazo[1,2-*a*]pyridin-2-yl)methyl)amino)pyrimidin-4-yl)cyclo propane-1-carboxamide, formic acid salt **(I-68)** | 4-(2-(aminomethyl)-6-cyclopropylimidazo [1,2-*a*]pyridin-8-yl)-2-methyl morpholin-3- one ESI-MS (M+H): 572.4, δ 10.60 (s, 1H), 8.40 (s, 0.2H), 8.31 - 8.29 (m, 1H), 8.20 - 8.18 (m, 1H), 7.90 (br s, 1H), 7.65 (s, 1H), 7.34 - 7.24 (m, 4H), 7.17 - 7.13 (m, 1H), 6.99 (d, J=1.5 Hz, 1H), 4.56 (br s, 2H), 4.36 (q, J=6.8 Hz, 1H), 4.10 - 4.05 (m, 1H), 3.99 - 3.95 (m, 2H), 3.74 - 3.72 (m, 1H), 2.43 - 2.34 (m, 2H), 1.96 - 1.88 (m, 1H), 1.49 - 1.44 (m, 1H), 1.41 - 1.38 (m, 4H), 0.95 - 0.89 (m, 2H), 0.69 - 0.64 (m, 2H). |
| **69** | 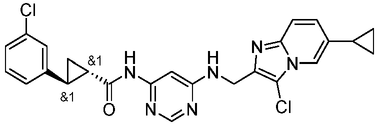 *rac-*(1*S*,2S**)-N-(6-(((3-chloro-6-cyclopropylimidazo[1,2-*a*]pyridin-2-yl)methyl)amino)pyrimidin-4-yl)-2-(3-chlorophenyl)cyclopropane-1-carboxamide (I-69) | (3-chloro-6-cyclopropylimidazo[1,2-*a*] pyridin-2-yl)methanamine |
| | | ESI-MS (M+H): 493.3, δ 10.51 (s, 1H), 8.14 (s, 1H), 8.03 - 8.03 (m, 1H), 7.77 (s, 1H), 7.43 (d, J=9.3 Hz, 1H), 7.26 - 7.18 (m, 4H), 7.09 (td, J=1.3, 7.6 Hz, 1H), 6.98 (dd, J=1.7, 9.4 Hz, 1H), 4.55 (s, 2H), 2.38 - 2.28 (m, 2H), 2.06 - 1.98 (m, 1H), 1.44 - 1.30 (m, 2H), 0.93 - 0.87 (m, 2H), 0.72 - 0.67 (m, 2H). |
| **70** | 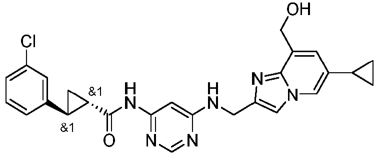 *rac*-(1*S**,2*S**)-2-(3-chlorophenyl)-N-(6-(((6-cyclopropyl-8-(hydroxymethyl) imidazo[1,2-*a*]pyridin-2-yl)methyl) amino)pyrimidin-4-yl)cyclopropane-1-carboxamide, formic acid salt **(I-70)** | **Intermediate 266** |
| | | ESI-MS (M+H): 489.3, δ 10.59 (s, 1H), 8.44 (s, 0.4H), 8.19 - 8.18 (m, 2H), 7.86 (br s, 1H), 7.60 (s, 1H), 7.34 - 7.29 (m, 1H), 7.26 - 7.24 (m, 3H), 7.17 - 7.13 (m, 1H), 6.97 - 6.96 (m, 1H), 5.31 (br s, 1H), 4.77 (s, 2H), 4.56 - 4.55 (m, 2H), 2.44 - 2.32 (m, 2H), 1.96 - 1.88 (m, 1H), 1.50 - 1.43 (m, 1H), 1.43 - 1.37 (m, 1H), 0.94 - 0.88 (m, 2H), 0.67 - 0.62 (m, 2H). |
| **71** | 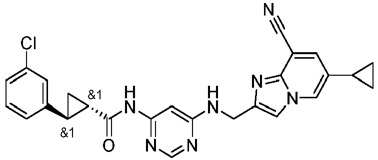 *rac*-(1*S**,2*S**)-2-(3-chlorophenyl)-N-(6-(((8-cyano-6-cyclopropylimidazo[1,2-*a*]pyridin-2-yl)methyl)amino)pyrimidin-4-yl)cyclopropane-1-carboxamide **(I-71)** | 2-(aminomethyl)-6-cyclopropylimidazo[1,2-*a*]pyridine-8-carbonitrile |
| | | ESI-MS (M+H): 484.3, δ 10.66 (s, 1H), 8.70 (s, 1H), 8.25 (s, 1H), 8.06 - 8.00 (m, 1H), 7.86 - 7.77 (m, 2H), 7.39 - 7.29 (m, 4H), 7.20 (d, J=7.3 Hz, 1H), 4.67 - 4.67 (m, 2H), 2.47 - 2.38 (m, 2H), 2.05 - 1.98 (m, 1H), 1.55 - 1.42 (m, 2H), 1.03 - 0.96 (m, 2H), 0.83 - 0.77 (m, 2H). |

### Example 72: rac-3-(2-(((6-((1S*,2S*)-2-(3-chlorophenyl)cyclopropane-1-carboxamido)pyrimidin- 4-yl)amino)methyl)-6-cyclopropylimidazo[1,2-a]pyridin-8-yl)propanoic acid (I-72)

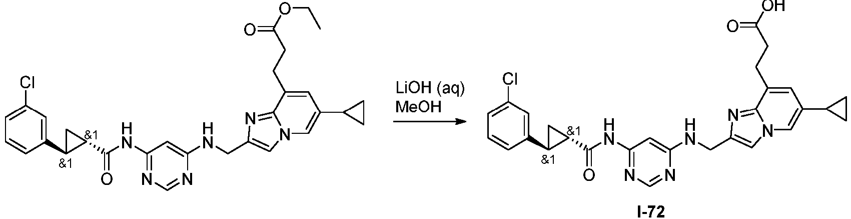

LiOH (1M, aq., 0.070 mL) was added to a solution of rac-ethyl 3-(2-(((6-((1S*,2S*)-2-(3-chlorophenyl)cyclopropane-1-carboxamido)pyrimidin-4-yl)amino)methyl)-6-cyclopropylimidazo[1,2-a]pyridin-8-yl)propanoate (26 mg, 0.046 mmol) in MeOH (1.0 mL). The mixture was stirred for 18 h, then LiOH (1M, aq., 0.070 mL) was added. The mixture was stirred at room temperature for a further 3 h, then at 50 °C for 3 h. The mixture was cooled to room temperature, then water (25 mL) was added. The pH was adjusted to ca. 6. The mixture was extracted with DCM (25 mL), then EtOAc (2 x 25 mL). The combined organics were dried over MgSO₄, then concentrated *in vacuo.* The residue was purified by preparative HPLC to give **(I-72)** (4.6mg, 18%).ESI-MS (M+H)+: 531.3, δ 10.58 (s, 1H), 8.20 - 8.19 (m, 1H), 8.15 - 8.13 (m, 1H), 7.92 - 7.86 (m, 1H), 7.57 (s, 1H), 7.34 - 7.24 (m, 4H), 7.17 - 7.13 (m, 1H), 6.79 - 6.78 (m, 1H), 4.56 (br s, 2H), 3.04 (t, J=7.7 Hz, 2H), 2.66 - 2.61 (m, 2H), 2.44 - 2.32 (m, 2H), 1.90 - 1.82 (m, 1H), 1.50 - 1.36 (m, 2H), 0.91 - 0.85 (m, 2H), 0.66 - 0.61 (m, 2H).

### Example 73: rac-(1S*,2S*)-2-(3-chlorophenyl)-N-(6-(((6-cyclopropyl-8-(morpholinomethyl) imidazo[1,2-a]pyridin-2-yl)methyl)amino)pyrimidin-4-yl)cyclopropane-1-carboxamide (I-73)

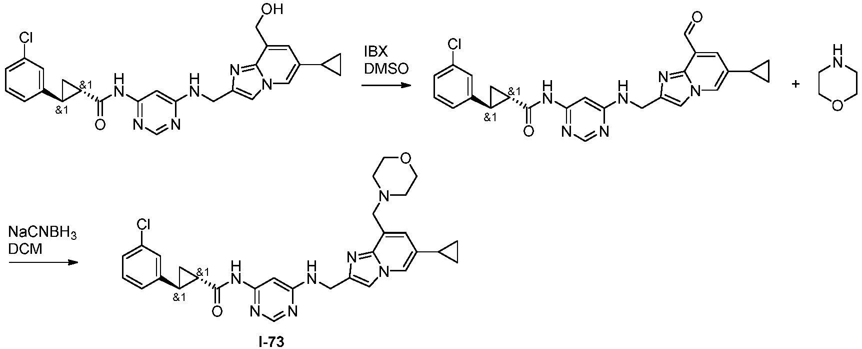

IBX (45%, 84 mg, 0.135 mmol) was added to a solution of **I-70** (55 mg, 0.11 mmol) in DMSO (1.0 mL). The mixture was stirred for 3 h, then Na₂S₂O₃ (sat. aq., 5 mL) was added. The mixture was stirred for 5 min, then NaHCO₃ (sat. aq., 5 mL) was added. The mixture was stirred for a further 5 min, then diluted with NaHCO₃ (sat. aq., 25 mL), then extracted with DCM (2 × 25 mL). The combined organics were washed with brine (25 mL), passed through a phase separator cartridge, then concentrated *in vacuo.* The residue (55 mg) was dissolved in DCM (5.0 mL), then morpholine (0.020 mL, 0.23 mmol) was added. After stirring for 30 min, NaCNBH₃ (14 mg, 0.23 mmol) was added and the mixture was stirred for 18 h. Further morpholine (0.020 mL, 0.23 mmol) was added, and after a further 2 h stirring, NaCNBH₃ (14 mg, 0.23 mmol) then MeOH (0.1 mL) were added. The mixture was stirred for 2 h then morpholine (0.020 mL, 0.23 mmol) was added. The mixture was stirred for 18 h, then diluted with NaHCO₃ (sat. aq., 15 mL). The mixture was extracted with DCM (25 mL) the EtOAc (2 × 25 mL). The combined organics were washed with brine, passed through a phase separator column then concentrated *in vacuo.* The residue was purified by preparative HPLC to give **(I-73)** as a formic acid salt. ESI-MS (M+H)+: 558.4, δ 10.59 (s, 1H), 8.19 (d, J=0.7 Hz, 2H), 7.87 (br s, 1H), 7.60 (s, 1H), 7.34 - 7.24 (m, 4H), 7.17 - 7.13 (m, 1H), 6.96 (d, J=1.3 Hz, 1H), 4.56 (s, 2H), 3.76 (s, 2H), 3.61 (t, J=4.5 Hz, 4H), 2.48 - 2.31 (m, 6H), 1.96 - 1.88 (m, 1H), 1.49 - 1.37 (m, 2H), 0.94 - 0.88 (m, 2H), 0.66 - 0.61 (m, 2H).

### Examples 74-76

### rac-(1S*,2S*)-2-(3-chlorophenyl)-N-(6-(((6-cyclopropyl-8-(3-oxomorpholino)imidazo[1,2-a]pyridin-2-yl)methyl)amino)pyrimidin-4-yl)cyclopropane-1-carboxamide (I-74)

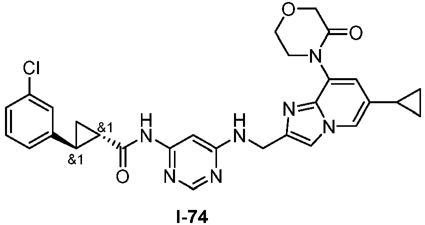

Using a similar procedure to that used for **(I-55), (I-74)** was prepared using ***rac-(1S*,2S*)-2-*(3-chlorophenyl)-N-(6-chloropyrimidin-4-yl)cyclopropane-1-carboxamide intermediate 697** (32 mg, 0.11 mmol) and 4-(2-(aminomethyl)-6-cyclopropylimidazo[1,2-a]pyridin-8-yl)morpholin-3-one (30 mg, 0.11 mmol), then purified by preparative LCMS to give **(I-74)** (23 mg, 39 %) as a mixture of enantiomers. ESI-MS (M+H)+: 558.4

The mixture was separated using SFC (YMC Amylose-C 10x250 mm, 5 µm 55/45 EtOH (0.1 % DEA) / CO₂, 15 mL/min, 120 bar, 40°C) to give two enantiomers: (1S,2S)-2-(3-chlorophenyl)*-N*-(6-(((6-cyclopropyl-8-(3-oxomorpholino)imidazo[1,2-*a*]pyridin-2-yl)methyl)amino)pyrimidin-4-yl)cyclopropane-1-carboxamide and (1*R*,2*R*)-2-(3-chlorophenyl)-*N-*(6-(((6-cyclopropyl-8-(3-oxomorpholino)imidazo[1,2-*a*]pyridin-2-yl)methyl)amino)pyrimidin-4-yl)cyclopropane-1-carboxamide.
First eluting isomer **(I-75)** (4.7 mg): ESI-MS (M+H): 558.4, δ 10.61 (s, 1H), 8.33 (s, 1H), 8.21 (s, 1H), 7.94 (s, 1H), 7.68 (s, 1H), 7.36 - 7.27 (m, 4H), 7.17 (d, J=7.7 Hz, 1H), 7.03 (d, J=1.0 Hz, 1H), 4.58 - 4.58 (m, 2H), 4.27 (s, 2H), 4.06 - 4.01 (m, 2H), 3.88 - 3.84 (m, 2H), 2.43 - 2.34 (m, 2H), 1.98 - 1.90 (m, 1H), 1.51 - 1.39 (m, 2H), 0.97 - 0.91 (m, 2H), 0.71 - 0.65 (m, 2H). RT = 2.5 min, 99.9 % e.e.
Second eluting isomer **(I-76,** 3.8 mg): ESI-MS (M+H): 558.4, ¹H NMR (400 MHz, DMSO) substantially similar to (I-75). RT = 5.1 min, 99.9 % e.e.

### Example 77: (1S,2S)-2-(3-chlorophenyl)-N-(6-(((6-cyclopropylimidazo[1,2-a]pyridin-2-yl) methyl)(2-hydroxyethyl)amino)pyrimidin-4-yl)cyclopropane-1-carboxamide (I-77)

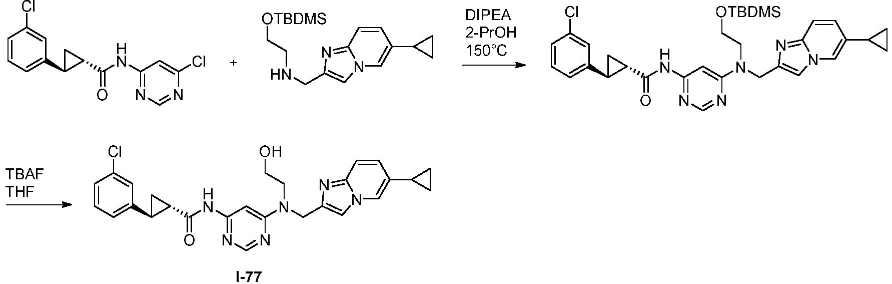

A mixture of **Intermediate 676** (0.040 g, 0.13 mmol), **intermediate 253** (0.044 g, 0.13 mmol), and DIPEA (0.068 mL, 0.39 mmol) in iPrOH (1.5 mL) was stirred in a microwave at 150 °C for 1 hour. The reaction mixture was concentrated in vacuo, then the residue was purified by column chromatography on silica gel, eluting with a gradient of 0-20 % 7N NH₃ in MeOH in DCM to give **(1S,2S)-N-(6-((2-((tert-butyldimethylsilyl)oxy)ethyl)((6-cyclopropyl imidazo[1,2-a]pyridin-2-yl)methyl)amino)pyrimidin-4-yl)-2-(3-chlorophenyl)cyclopropane-1-carboxamide** (0.070 g, 88 %), which was used without further purification to produce (I-77) using a similar procedure to that for (I-137). ESI-MS (M+H): 503.4, δ 10.72 (s, 1H), 8.36 - 8.30 (m, 2H), 7.68 (s, 1H), 7.49 - 7.30 (m, 5H), 7.22 - 7.18 (m, 1H), 7.04 (dd, J=1.5, 9.3 Hz, 1H), 5.25 (s, 1H), 4.84 (s, 2H), 3.68 - 3.67 (m, 4H), 2.49 - 2.38 (m, 2H), 2.01 - 1.93 (m, 1H), 1.56 - 1.42 (m, 2H), 0.99 - 0.93 (m, 2H), 0.74 - 0.69 (m, 2H).

### Example 78: rac-(1S*,2S*)-2-(3-chlorophenyl)-N-(4-(((6-cyclopropylimidazo[1,2-a]pyridin-2-yl)methyl)amino)pyrimidin-5-yl)cyclopropane-1-carboxamide (I-78)

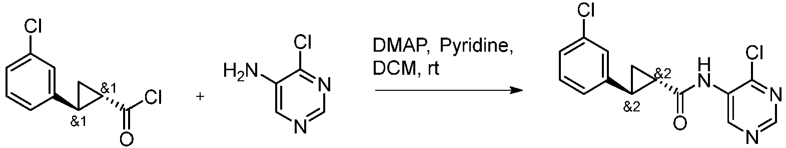

A mixture of 4-chloropyrimidin-5-amine ( 66 mg, 0.511 mmol), DMAP ( 6.2 mg, 0.0511 mmol), and pyridine (0.041 mL, 0.511 mmol) was added to a solution of rac-(1S*,2S*)-2-(3-chlorophenyl)cyclopropane-1-carbonyl chloride ( 110 mg, 0.511 mmol) in DCM ( 2 mL) at room temperature and stirred for 24 h. The mixture was diluted with DCM ( 25 mL) and washed with NH4Cl ( aq. sat. 15 mL), water ( 10 mL), NaHCO₃ ( aq. sat. 15 mL), and water ( 10 mL), then dried (MgSO₄), filtered, and concentrated *in vacuo* to give ***rac-(1S*,2S*)-2-(3-chlorophenyl)-N-(4-chloropyrimidin-5-yl)cyclopropane-1-carboxamide* intermediate 698** (131 mg, 83%). ESI-MS (M+H)+: 310.0, δ 10.26 (s, 1H), 9.22 (s, 1H), 8.82 (s, 1H), 7.38 - 7.33 (m, 1H), 7.31 - 7.28 (m, 2H), 7.23 - 7.19 (m, 1H), 2.49 - 2.46 (m, 1H), 1.59 - 1.47 (m, 2H). 1H missing, presumed under DMSO.

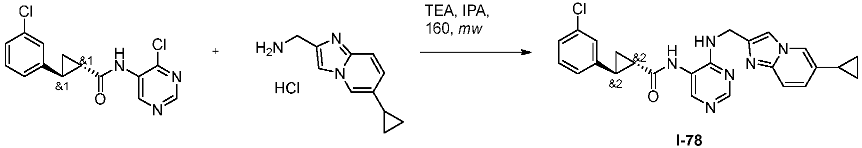

A mixture of **intermediate 698** (65 mg, 0.211 mmol), intermediate 251 hydrochloride (71 mg, 0.316 mmol), TEA (0.15 mL, 1.05 mmol), and IPA ( 1 mL) was placed in a microwave vial, sealed, and heated to 160 °C under microwave conditions for 1 h. The mixture was diluted with water (25 mL) and extracted with EtOAc ( 3x 25 mL). The organic phases were combined, dried (MgSO₄), filtered, and concentrated *in vacuo.* Purification by reverse phase preparative HPLC gave **(I-78)** (7.5 mg, 7%) as an orange solid. ESI-MS (M+H)+: 459.3, δ 9.59 (s, 1H), 8.33 - 8.28 (m, 3H), 7.65 (s, 1H), 7.40 - 7.24 (m, 6H), 7.18 (d, J=7.6 Hz, 1H), 6.96 (dd, J=1.0, 9.0 Hz, 1H), 4.66 (d, J=5.3 Hz, 2H), 2.46 - 2.39 (m, 1H), 2.17 - 2.12 (m, 1H), 1.94 - 1.86 (m, 1H), 1.54 - 1.39 (m, 2H), 0.94 - 0.87 (m, 2H), 0.68 - 0.63 (m, 2H).

### Intermediate 699: N⁴-((6-cyclopropylimidazo[1,2-a]pyridin-2-yl)methyl)-5-fluoropyridine-2,4-diamine

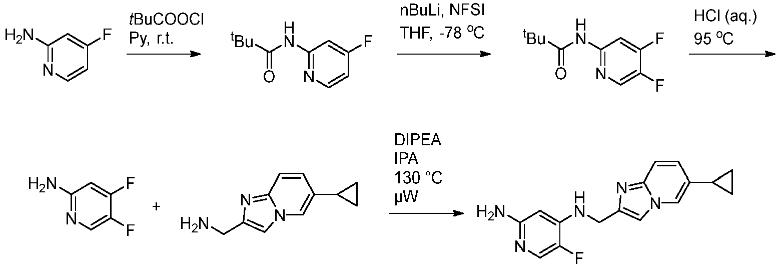

Pivaloyl chloride (1.6 mL, 13 mmol) was added dropwise to a stirred solution of 4-fluoropyridin-2-amine (1.0 g, 8.9 mmol) in pyridine (4.0 mL). The reaction was then stirred at room temperature for 18 h. The reaction was partitioned between EtOAc (20 mL) and water (30 mL), the layers were separated, and the aqueous layer was further extracted with EtOAc (2 × 20 mL). The organic layers were combined, dried (MgSO₄), and concentrated *in vacuo.* The residue was purified by column chromatography on silica gel, eluting with a gradient of 0-5 % MeOH in DCM to give N-***(4-fluoropyridin-2-yl)pivalamide* intermediate 700** (1.3 g, 75 %).ESI-MS (M+H)+: 197.1, ¹H NMR (400 MHz, CDCl₃) δ 8.38 - 8.28 (br s, 1H), 8.20 (dd, J=5.6, 8.6 Hz, 1H), 8.07 (dd, J=2.5, 11.4 Hz, 1H), 6.80 - 6.76 (m, 1H), 1.32 (s, 9H).

nBuLi (2.2 M in hexanes, 2.8 mL, 6.1 mmol) was added dropwise to a stirred solution of **intermediate 700** (500 mg, 2.6 mmol) in THF (15 mL) at -78 °C and stirred for 30 min. NFSI (1.6 g, 5.1 mmol) in THF (5.0 mL) was then added dropwise, and the reaction was stirred at -78 °C for a further 20 min and then allowed to warm to room temperature. The reaction was quenched with NH₄Cl (sat. aq., 15 mL) and extracted with EtOAc (3 × 30 mL). The organic layers were combined, dried (MgSO₄), and concentrated *in vacuo.* The residue was purified by column chromatography on silica gel, eluting with a gradient of 0-5 % MeOH in DCM to give ***N-(4,5-difluoropyridin-2-yl)pivalamide* intermediate 701** (400 mg, 73 %).ESI-MS (M+H)+: 215.1, ¹H NMR (400 MHz, CDCl₃) δ 8.14 (m, 1H), 7.68 (br s, 1H), 7.05 - 6.99 (m, 1H), 1.35 (s, 9H).

**Intermediate 701** (400 mg, 1.9 mmol) was suspended in concentrated HCl (33 % aq., 5.0 mL) and heated at 95 °C for 2 h. The reaction was cooled to room temperature and then poured over ice (25 g). The reaction was then taken to basic pH using NaOH (2.0 M aq.) and extracted with EtOAc (3 × 30 mL). The organic layers were combined, dried (MgSO₄), and concentrated *in vacuo* to give ***4,5-difluoropyridin-2-amine* intermediate 702** (150 mg, 62 %).ESI-MS (M+H)+: 131.1, ¹H NMR (400 MHz, CDCl₃) δ 7.78 (m, 1H), 6.54 - 6.49 (m, 1H), 4.77 - 4.68 (br s, 2H).

A solution of **intermediate 702** (0.120 g, 0.92 mmol), (6-cyclopropylimidazo [1,2-*a*]pyridin-2-yl)methanamine (0.31 g, 1.4 mmol) and DIPEA (0.56 mL, 3.2 mmol) in iPrOH (5.0 mL) was heated to 130 °C in a microwave for 2.5 h. The mixture was cooled to room temperature and concentrated *in vacuo.* The residue was purified by column chromatography on silica gel, eluting with a gradient of 0-5 % (7 N NH₃ in MeOH) in DCM to give intermediate 699 (0.170 g, 62 %).ESI-MS (M+H)+: 298.2, δ 8.34 (s, 1H), 7.66 (s, 1H), 7.44 - 7.38 (m, 2H), 7.01 (dd, J=1.8, 9.3 Hz, 1H), 6.61 (dd, J=5.2, 5.2 Hz, 1H), 6.11 (dd, J=5.9, 5.9 Hz, 1H), 5.61 (s, 2H), 4.45 (d, J=6.1 Hz, 2H), 1.99 - 1.91 (m, 1H), 0.98 - 0.92 (m, 2H), 0.73 - 0.68 (m, 2H).

**Intermediate 703: *1-(2-(((2-aminopyridin-4-yl)amino)methyl)-6-cyclopropylimidazo[1,2-a]pyridin-8-yl)pyrrolidin-2-one***

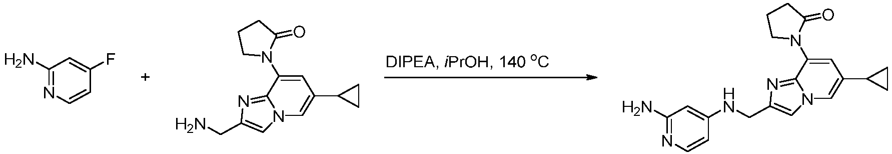

A solution of 4-fluoropyridin-2-amine (0.058 g, 0.52 mmol), **intermediate 315** (0.12 g, 0.55 mmol), and DIPEA (0.33 mL, 1.9 mmol) in iPrOH (5.0 mL) was heated to 140 °C in a microwave for 14 h. The mixture was cooled to room temperature and concentrated *in vacuo.* The residue was purified by column chromatography on silica gel, eluting with a gradient of 0-20 % (7 N NH₃ in MeOH) in DCM to give **intermediate 303** (0.070 g, 34 %).ESI-MS (M+H)+: 377, δ 8.29 (d, J=1.3 Hz, 1H), 7.65 (s, 1H), 7.46 (d, J=5.8 Hz, 1H), 6.98 (d, J=1.6 Hz, 1H), 6.62 (m, 1H), 5.93 (dd, J=2.1, 5.8 Hz, 1H), 5.77 (s, 1H), 5.62 (d, J=2.0 Hz, 1H), 5.33 (s, 2H), 4.89 - 4.83 (m, 1H), 4.31 (dd, J=1.6, 5.6 Hz, 2H), 2.50 - 2.47 (m, 3H), 1.97 - 1.89 (m, 1H), 1.76 - 1.66 (m, 1H), 0.96 - 0.92 (m, 2H), 0.69 - 0.64 (m, 2H).

The compounds in Table 10 were synthesized using a similar procedure to that used for **intermediate 703** from 4-fluropyridin-2-amine or 4-fluoropyrimidin-2-amine and an appropriate coupling partner. **Table 10**

| **Compound** | **Coupling Partner / Analytical Data** |
|---|---|
| 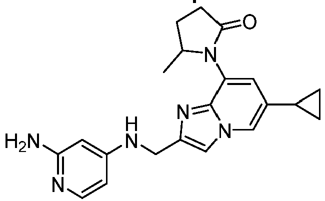 1-(2-(((2-aminopyridin-4-yl)amino) methyl)-6-cyclopropylimidazo[1,2-*a*]pyridin-8-yl)-5-methylpyrrolidin-2-one | 1-(2-(aminomethyl)-6-cyclopropylimidazo[1,2-*a*]pyridin-8-yl)-5-methylpyrrolidin-2-one |
| | ESI-MS (M+H)+: 377.4, δ 8.29 (d, J=1.3 Hz, 1H), 7.65 (s, 1H), 7.46 (d, J=5.8 Hz, 1H), 6.98 (d, J=1.6 Hz, 1H), 6.62 (dd, J=5.7, 5.7 Hz, 1H), 5.93 (dd, J=2.1, 5.8 Hz, 1H), 5.77 (s, 1H), 5.62 (d, J=2.0 Hz, 1H), 5.33 (s, 2H), 4.89 - 4.83 (m, 1H), 4.31 (dd, J=1.6, 5.6 Hz, 2H), 2.50 - 2.47 (m, 3H), 1.97 - 1.89 (m, 1H), 1.76 - 1.66 (m, 1H), 1.01 (d, 3H), 0.96 - 0.92 (m, 2H), 0.69 - 0.64 (m, 2H). |
| 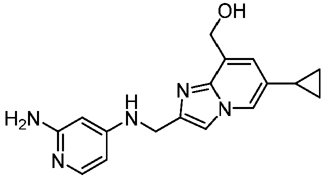 (2-(((2-aminopyridin-4-yl)amino)methyl)-6-cyclopropylimidazo[1,2-*a*]pyridin-8-yl)methanol | **Intermediate 266** |
| | δ 8.25 (s, 1H), 7.65 (s, 1H), 7.50 (d, J=5.8 Hz, 1H), 7.03 (d, J=1.5 Hz, 1H), 6.61 (m, 1H), 5.96 (dd, J=2.1, 5.9 Hz, 1H), 5.65 (d, J=2.0 Hz, 1H), 5.38 - 5.32 (m, 3H), 4.83 (d, J=5.3 Hz, 2H), 4.34 (d, J=5.8 Hz, 2H), 2.02 - 1.93 (m, 1H), 0.99 - 0.93 (m, 2H), 0.72 - 0.67 (m, 2H). |
| 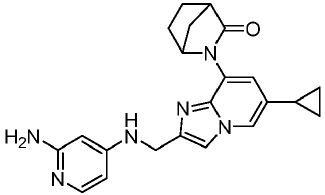 2-(2-(((2-aminopyridin-4-yl)amino)methyl) -6-cyclopropylimidazo[1,2-a]pyridin-8-yl)-2-azabicyclo[2.2.1]heptan-3-one | 2-(2-(aminomethyl)-6-cyclopropylimidazo[1,2-*a*]pyridin-8-yl)-2-azabicyclo[2.2.]heptan-3-one |
| | ESI-MS (M+H)+: 297.3, δ 8.21 (d, J=1.0 Hz, 1H), 7.68 (s, 1H), 7.51 (d, J=5.8 Hz, 1H), 7.36 (d, J=1.5 Hz, 1H), 6.62 (m, 1H), 5.98 (dd, J=2.0, 5.8 Hz, 1H), 5.66 (d, J=2.0 Hz, 1H), 5.51 (s, 1H), 5.33 (s, 2H), 4.36 (d, J=5.8 Hz, 2H), 3.22 (d, J=5.1 Hz, 1H), 2.89 (d, J=2.5 Hz, 1H), 2.08 (d, J=9.6 Hz, 1H), 2.03 - 1.79 (m, 2H), 1.71 - 1.59 (m, 1H), 0.98 - 0.92 (m, 2H), 0.70 - 0.64 (m, 2H). |
| 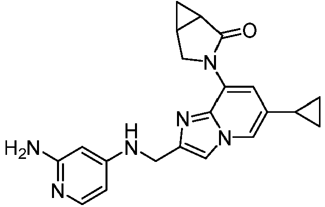 3-(2-(((2-aminopyridin-4-yl)amino) methyl)-6-cyclopropylimidazo[1,2-*a*] pyridin-8-yl)-3-azabicyclo[3.1.0]hexan-2-one | 3-(2-(aminomethyl)-6-cyclopropylimidazo [1,2-*a*]pyridin-8-yl)-3-azabicyclo[3.1.0]hexan-2-one |
| | ¹H NMR (400 MHz, CDCl₃): δ, ppm 7.72 (dd, J=0.7, 1.5 Hz, 1H), 7.69 (d, J=6.0 Hz, 1H), 7.37 (s, 1H), 7.11 (d, J=1.5 Hz, 1H), 6.04 (dd, J=2.1, 6.0 Hz, 1H), 5.73 (d, J=2.1 Hz, 1H), 4.82 (t, J=5.5 Hz, 1H), 4.49 (dd, J=5.8, 10.2 Hz, 1H), 4.44 (d, J=5.5 Hz, 2H), 4.11 (dd, J=1.5, 10.4 Hz, 1H), 2.15 - 2.03 (m, 2H), 1.91 - 1.83 (m, 1H), 1.29 - 1.23 (m, 1H), 1.00 - 0.92 (m, 3H), 0.69 - 0.65 (m, 2H) |
| 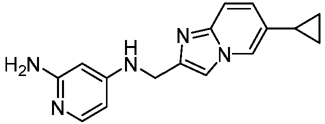 *N*⁴-((6-cyclopropylimidazo[1,2-a]pyridin-2-yl)methyl)pyridine-2,4-diamine | Intermediate 251 ESI-MS (M+H)+: 280.2, ¹H NMR (400 MHz, CDCl₃) δ 7.85 (d, J=7.3 Hz, 1H), 7.43 (dd, J=6.3, 9.3 Hz, 1H), 7.73 (d, 1H), 7.39 (s, 1H), 6.93 (m, 1H), 6.04 (d, 1H), 5.73 (s, 1H), 4.47 (d, J=5.3 Hz, 2H), 1.93 - 1.84 (m, 1H), 0.99 - 0.95 (m, 2H), 0.69 - 0.64 (m, 2H). |
| 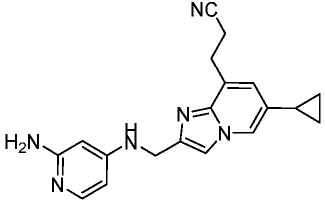 | **3-(2-(((2-aminopyridin-4-yl)amino)methyl)-6-cyclopropylimidazo[1,2-a]pyridin-8-yl) propanenitrile** from 3-(2-(aminomethyl)-6-cyclopropylimidazo[1,2-a]pyridin-8-yl)propanenitrile ESI-MS [M +H]+: 333.1 |
| 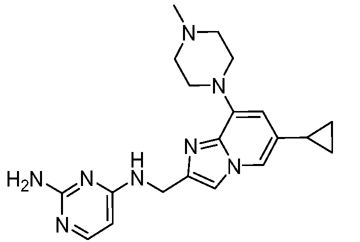 | **N4-((6-cyclopropyl-8-(4-methylpiperazin-1-yl) imidazo[1,2-a]pyridin-2-yl)methyl)pyrimidine-2,4-diamine** from intermediate 381ESI-MS (M+H)+: 379.2 |

### Examples 79-81

### rac-(1S*,2S*)-2-(5-chloro-2-cyanophenyl)-N-(4-(((6-cyclopropyl-8-(2-oxopyrrolidin-1-yl)imidazo[1,2-a]pyridin-2-yl)methyl)amino)pyridin-2-yl)cyclopropane-1-carboxamide (I-79)

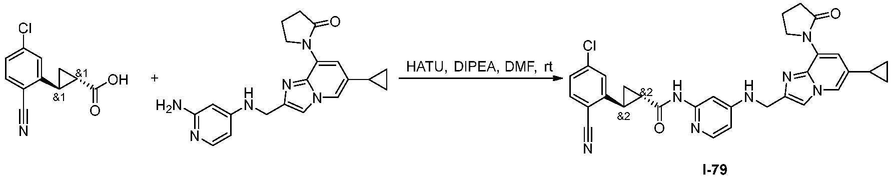

DIPEA (0.32 mL, 1.8 mmol) was added to a stirred mixture of **intermediate 703** (140 mg, 0.39 mmol), **intermediate 40** (82 mg, 0.39 mmol), and HATU (180 mg, 0.48 mmol) in DMF (4.0 mL). The mixture was then stirred at room temperature for 18 h. The mixture was diluted with EtOAc (50 mL) and washed with water (2 × 30 mL) and brine (sat. aq., 30 mL). The organic layers were dried (MgSO₄) and concentrated *in vacuo.* The residue was purified by reverse phase preparative HPLC to give **(I-79)** (19 mg, 9 %) as a formic acid salt (mixture of enantiomers). ESI-MS (M+H)+: 566.4, substantially similar to (I-80) .

The mixture was separated using SFC (YMC Amylose-C 10x250 mm, 5 µm 55/45 MeOH (0.1 % DEA) / CO₂, 15 mL/min, 120 bar, 40°C) to give two enantiomers: (1S,2S)-2-(5-chloro-2-cyanophenyl)-*N*-(4-(((6-cyclopropyl-8-(2-oxopyrrolidin-1-yl)imidazo[1,2-a]pyridin-2-yl)methyl)amino)pyridin-2-yl)cyclopropane-1-carboxamide and (1R,2R)-2-(5-chloro-2-cyanophenyl)-*N*-(4-(((6-cyclopropyl-8-(2-oxopyrrolidin-1-yl)imidazo[1,2-*a*]pyridin-2-yl)methyl)amino)pyridin-2-yl)cyclopropane-1-carboxamide.

First eluting isomer **(I-80):** ESI-MS (M+H)+: 566.4, ¹H NMR (400 MHz, CDCl₃) δ 8.50 (s, 1H), 7.81 (s, 2H), 7.61 (d, J=6.8 Hz, 1H), 7.57 - 7.52 (m, 2H), 7.30 (dd, J=1.9, 8.3 Hz, 1H), 7.19 (dd, J=1.8, 4.9 Hz, 2H), 6.38 (dd, J=2.4, 6.8 Hz, 1H), 4.55 (d, J=5.0 Hz, 2H), 4.21 - 4.14 (m, 2H), 2.95 - 2.89 (m, 1H), 2.66 - 2.61 (m, 2H), 2.42 - 2.36 (m, 1H), 2.30 - 2.21 (m, 2H), 1.94 - 1.78 (m, 2H), 1.45 (ddd, J=4.8, 6.5, 8.4 Hz, 1H), 1.00 - 0.95 (m, 2H), 0.73 - 0.68 (m, 2H). RT = 6.43 min, 99.9 % e.e. Formic acid salt.

Second eluting isomer **(I-81):** ESI-MS (M+H)+: 566.4, ¹H NMR (400 MHz, CDCl₃) δ 8.50 (s, 1H), 7.81 (s, 2H), 7.61 (d, J=6.8 Hz, 1H), 7.57 - 7.52 (m, 2H), 7.30 (dd, J=1.9, 8.3 Hz, substantially similar to (I-80) (m, 2H). RT = 8.17 min, 98.5 % e.e.

### Examples 82-84

### rac-(1S*,2S*)-2-(5-chloro-2-cyanophenyl)-N-(4-(((6-cyclopropylimidazo[1,2-a]pyridin-2-yl)methyl)amino)pyridin-2-yl)cyclopropane-1-carboxamide (I-82)

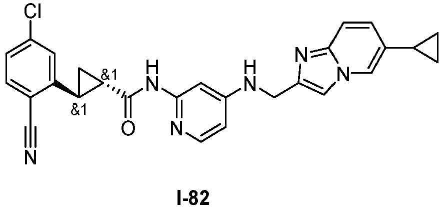

Using a similar procedure to that used for **(I-79), (I-82)** was prepared using **intermediate 40** (57 mg, 0.26 mmol) and *N*⁴-((6-cyclopropylimidazo[1,2-*a*]pyridin-2-yl)methyl)pyridine-2,4-diamine (100 mg, 0.23 mmol), then purified by preparative LCMS to give **(I-82)** (19 mg, 17 %) as a mixture of enantiomers. ESI-MS (M+H):+ 483.3, One CH of cyclopropyl group obscured by DMSO peak.

The mixture was separated using SFC (YMC Amylose-C 10x250 mm, 5 µm 55/45 MeOH (0.1 % DEA) / CO₂, 15 mL/min, 120 bar, 40°C) to give two enantiomers: **(I-83 & 1-84).**

First eluting isomer, **(1R,2R)-2-(5-chloro-2-cyanophenyl)-N-(4-(((6-cyclopropylimidazo[1,2-*a*]pyridin-2-yl)methyl)amino)pyridin-2-yl)cyclopropane-1-carboxamide (I-83):** ESI-MS (M+H)+: 483.2, δ 10.36 (s, 1H), 8.30 (s, 1H), 7.85 (d, J=8.3 Hz, 1H), 7.76 (d, J=5.8 Hz, 1H), 7.63 (s, 1H), 7.51 (dd, J=2.0, 8.3 Hz, 1H), 7.45 (s, 1H), 7.39 - 7.36 (m, 2H), 7.11 (dd, J=5.9, 5.9 Hz, 1H), 6.97 (dd, J=1.9, 9.2 Hz, 1H), 6.33 (dd, J=2.0, 5.8 Hz, 1H), 4.35 (d, J=5.8 Hz, 2H), 2.59 - 2.54 (m, 1H), 1.94 - 1.86 (m, 1H), 1.56 - 1.51 (m, 2H), 0.90 (ddd, J=4.3, 6.3, 8.3 Hz, 2H), 0.68 - 0.63 (m, 2H). One CH of cyclopropyl group obscured by DMSO peak. RT = 5.52 min, 99 % e.e.

Second eluting isomer, **(1S,2S)-2-(5-chloro-2-cyanophenyl)-N-(4-(((6-cyclopropyl imidazo[1,2-*a*]pyridin-2-yl)methyl)amino)pyridin-2-yl)cyclopropane-1-carboxamide (I-84):** ESI-MS (M+H)+: 483.2, similar to (I-83) RT = 12.87 min, 98.5 % e.e.

### Example 85: (1S,2S)-2-(3-chlorophenyl)-N-(4-(((6-cyclopropyl-8-(2-oxopyrrolidin-1-yl)imidazo[1,2-a]pyridin-2-yl)methyl)amino)pyridin-2-yl)cyclopropane-1-carboxamide (I-85)

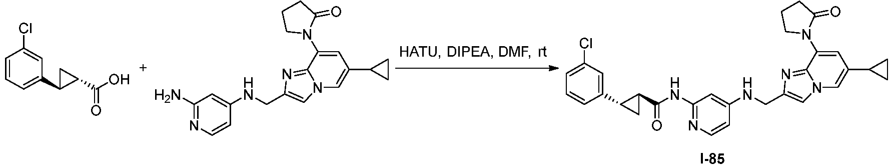

A mixture of **intermediate 703** (183 mg, 0.51 mmol), (1S,2S)-2-(3-chlorophenyl)cyclopropane-1-carboxylic acid (95 mg, 0.48 mmol), HATU (238 mg, 0.63 mmol), and DIPEA (0.42 mL, 2.40 mmol) in DMF (4.0 mL) was stirred at room temperature for 18 h. Water (50 mL) and NaHCO₃ (sat. aq., 50 mL) were added, and the mixture was extracted with DCM (3 × 50 mL). The combined organics were dried over MgSO₄, filtered, and concentrated *in vacuo.* The residue was purified by column chromatography on silica gel, eluting with 0-20 % (7N NH₃ in MeOH) in DCM to give **(I-85)** (120 mg, 46%).ESI-MS (M+H)+: 541, δ 10.36 (s, 1H), 8.30 (d, J=1.3 Hz, 1H), 7.81 (d, J=5.8 Hz, 1H), 7.72 (s, 1H), 7.52 - 7.49 (m, 1H), 7.36 (dd, J=8.0, 8.0 Hz, 1H), 7.31 - 7.28 (m, 2H), 7.21 - 7.17 (m, 3H), 6.37 (dd, J=2.0, 5.8 Hz, 1H), 4.41 (d, J=5.8 Hz, 2H), 4.21 (dd, J=7.1, 7.1 Hz, 2H), 2.54 - 2.50 (m, 2H), 2.46 - 2.37 (m, 2H), 2.23 - 2.14 (m, 2H), 2.01 - 1.93 (m, 1H), 1.53 - 1.46 (m, 1H), 1.43 - 1.37 (m, 1H), 0.97 (ddd, J=4.3, 6.3, 8.3 Hz, 2H), 0.72 - 0.67 (m, 2H).

Using a similar procedure to that used for **(I-85),** the compounds in Table 11 were prepared using *intermediate 1149* or (1S,2S)-2-(3-chlorophenyl)cyclopropane-1-carboxylic acid and an appropriate coupling partner. **Table 11**

| **EG** | **Compound** | **Coupling Partner / Analytical Data** |
|---|---|---|
| **86** | 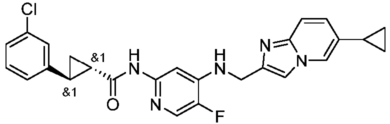 *rac*-(1S*,2S*)-2-(3-chlorophenyl)-N-(4-(((6-cyclopropylimidazo[1,2-a]pyridin-2-yl)methyl)amino)-5-fluoropyridin-2-yl) cyclopropane-1-carboxamide, formic acid salt (**I-86**) | **Intermediate 699** ESI-MS (M+H)+: 476.3, δ 10.17 (s, 1H), 8.33 (s, 1H), 7.75 (d, J=5.5 Hz, 1H), 7.68 (s, 1H), 7.42 (d, J=9.3 Hz, 1H), 7.39 - 7.34 (m, 1H), 7.31 (d, J=1.3 Hz, 2H), 7.29 - 7.29 (m, 2H), 7.20 (d, J=7.7 Hz, 1H), 7.15 (dd, J=6.1, 6.1 Hz, 1H), 7.01 (dd, J=1.8, 9.3 Hz, 1H), 6.65 (dd, J=6.0, 6.0 Hz, 1H), 4.50 (d, J=5.9 Hz, 2H), 2.58 (s, 1H), 2.44 - 2.38 (m, 1H), 2.27 - 2.22 (m, 1H), 1.98 - 1.90 (m, 1H), 1.52 - 1.41 (m, 2H), 0.97 - 0.91 (m, 2H), 0.72 - 0.67 (m, 2H). |
| **87** | 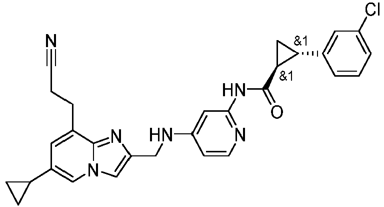 *rac*-(1S*,2S*)-2-(3-chlorophenyl)-N-(4-(((8-(2-cyanoethyl)-6-cyclopropylimidazo[1,2-a]pyridin-2-yl)methyl)amino)pyridin-2-yl)cyclopropane-1-carboxamide **(I-87)** | 3-(2-(((2-aminopyridin-4-yl)amino) methyl)-6-cyclopropylimidazo[1,2-a]pyridin-8-yl)propanenitrile ESI-MS [M +H]+: 511.2. |
| | | δ 10.31 (s, 1H), 8.24 (s, 1H), 7.75 (d, J = 5.7 Hz, 1H), 7.64 (s, 1H), 7.44 (s, 1H), 7.36 - 7.26 (m, 1H), 7.27 - 7.17 (m, 2H), 7.16 - 7.04 (m, 3H), 6.92 (s, 1H), 6.32 (d, J = 5.7 Hz, 1H), 4.37 (d, J = 5.6 Hz, 2H), 3.17 - 3.11 (m, 2H), 3.10 - 3.01 (m, 2H), 2.45 - 2.28 (m, 2H), 1.94 - 1.84 (m, 1H), 1.49 - 1.39 (m, 1H), 1.39 - 1.29 (m, 1H), 0.91 (d, J = 8.3 Hz, 2H), 0.67 (d, J = 5.1 Hz, 2H). |
| **88** | 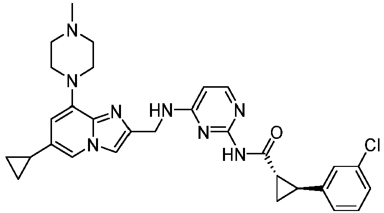 (1S,2S)-2-(3-chlorophenyl)-N-(4-(((6-cyclopropyl-8-(4-methylpiperazin-1-yl)imidazo[1,2-a]pyridin-2-yl)methyl) amino)pyrimidin-2-yl)cyclopropane-1-carboxamide **(I-88)** | N4-((6-cyclopropyl-8-(4-methyl piperazin-1-yl)imidazo[1,2-a]pyridin-2-yl)methyl) pyrimidine-2,4-diamine |
| | | ESI-MS [M +H]+: 557.2. δ 10.23 (s, 1H), 7.88 (s, 2H), 7.61 (s, 1H), 7.39 - 7.16 (m, 3H), 7.12 (d, J = 7.7 Hz, 1H), 6.24 (d, J = 5.9 Hz, 1H), 6.18 (s, 1H), 4.50 (s, 2H), 3.50 - 3.40 (m, 4H), 2.77 - 2.67 (m, 4H), 2.53 (s, 3H), 2.43 (s, 2H), 1.99 - 1.78 (m, 1H), 1.56 - 1.43 (m, 1H), 1.42 - 1.31 (m, 1H), 0.99 - 0.76 (m, 2H), 0.69 - 0.65 (m, 2H). |

### Examples 89-91

### (1S,2S)-2-(3-chlorophenyl)-N-(4-(((6-cyclopropy-8-(2-methyl-5-oxopyrrolidin-1-yl)imidazo[1,2-a]pyridin-2-yl)methyl)amino)pyridin-2-yl)cyclopropane-1-carboxamide (I-89)

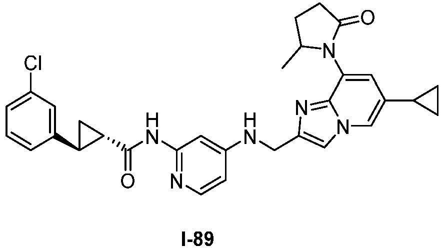

Using a similar procedure to that used for **(I-79), (I-89)** was prepared using (S,S)-2-(3-chlorophenyl)cyclopropane-1-carboxylic acid (35 mg, 0.18 mmol) and *N*⁴-((6-cyclopropylimidazo[1,2-*a*]pyridin-2-yl)methyl)pyridine-2,4-diamine (70 mg, 0.19 mmol), then purified by preparative LCMS to give **(I-89)** (20 mg, 20 %) as a mixture of diastereomers. ESI-MS (M+H)+: 555.4, δ 10.33 (s, 1H), 8.29 (d, J=1.4 Hz, 1H), 7.77 (d, J=5.8 Hz, 1H), 7.68 (s, 1H), 7.46 (s, 1H), 7.33 (dd, J=7.6, 7.6 Hz, 1H), 7.26 (d, J=7.3 Hz, 2H), 7.15 (d, J=7.7 Hz, 2H), 6.99 (d, J=1.6 Hz, 1H), 6.33 (dd, J=2.0, 5.8 Hz, 1H), 4.89 (dd, J=6.1, 12.3 Hz, 1H), 4.37 (d, J=5.8 Hz, 2H), 2.49 - 2.33 (m, 4H), 1.98 - 1.90 (m, 1H), 1.76 - 1.66 (m, 1H), 1.49 - 1.43 (m, 1H), 1.39 - 1.32 (m, 1H), 1.01 (d, J=6.4 Hz, 3H), 0.97 - 0.92 (m, 2H), 0.69 - 0.64 (m, 2H). 1 proton hidden under DMSO signal.

The mixture was separated using SFC (LUX Cellulose-4 10x250 mm, 5 µm 55/45 MeOH (0.1 % DEA) / CO₂, 15 mL/min, 120 bar, 40°C) to give two diastereomers: (1S,2S)-2-(3-chlorophenyl)-*N*-(4-(((6-cyclopropyl-8-((*S*)-2-methyl-5-oxopyrrolidin-1-yl)imidazo[1,2-*a*]pyridin-2-yl)methyl)amino)pyridin-2-yl)cyclopropane-1-carboxamide and (1*S*,2*S*)-2-(3-chlorophenyl)-N-(4-(((6-cyclopropyl-8-((*R*)-2-methyl-5-oxopyrrolidin-1-yl)imidazo[1,2-*a*]pyridin-2-yl)methyl)amino)pyridin-2-yl)cyclopropane-1-carboxamide.

First eluting isomer **(I-90):** ESI-MS (M+H)+: 555.4, δ 10.33 (s, 1H), 8.29 (d, J=1.4 Hz, 1H), 7.77 (d, J=5.8 Hz, 1H), 7.68 (s, 1H), 7.46 (s, 1H), 7.33 (dd, J=7.6, 7.6 Hz, 1H), 7.26 (d, J=7.3 Hz, 2H), 7.15 (d, J=7.7 Hz, 2H), 6.99 (d, J=1.6 Hz, 1H), 6.33 (dd, J=2.0, 5.8 Hz, 1H), 4.89 (dd, J=6.1, 12.3 Hz, 1H), 4.37 (d, J=5.8 Hz, 2H), 2.49 - 2.33 (m, 4H), 1.98 - 1.90 (m, 1H), 1.76 - 1.66 (m, 1H), 1.49 - 1.43 (m, 1H), 1.39 - 1.32 (m, 1H), 1.01 (d, J=6.4 Hz, 3H), 0.97 - 0.92 (m, 2H), 0.69 - 0.64 (m, 2H). 1 proton hidden under DMSO. RT = 14.42 min, 97.71 % excess.

Second eluting isomer (I-91): ESI-MS (M+H)+: 555.4, ¹H NMR (400 MHz, DMSO) similar to I-90 . RT = 18.25 min, 96.6 % excess.

### Examples 92-94

### (1S,2S)-2-(3-chlorophenyl)-N-(4-(((6-cyclopropyl-8-(3-oxo-2-azabicyclo[2.2.1] heptan-2-yl)imidazo[1,2-a]pyridin-2-yl)methyl)amino)pyridin-2-yl)cyclopropane-1-carboxamide (I-92)

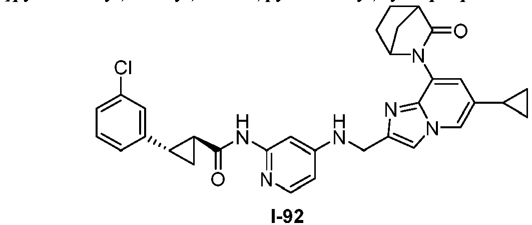

Using a similar procedure to that used for **(I-79), (I-92)** was prepared using (S,S)-2-(3-chlorophenyl)cyclopropane-1-carboxylic acid (23 mg, 0.12 mmol) and 2-(2-(((2-aminopyridin-4-yl)amino)methyl)-6-cyclopropylimidazo[1,2-*a*]pyridin-8-yl)-2-azabicyclo[2.2.1]heptan-3-one (50 mg, 0.13 mmol), then purified by preparative LCMS to give **(I-92)** (21 mg, 32 %) as a mixture of diastereomers. ESI-MS (M+H)+: 567.3, δ 10.52 - 10.52 (m, 1H), 8.17 (d, J=1.1 Hz, 1H), 7.79 (d, J=6.0 Hz, 1H), 7.69 (s, 1H), 7.36 - 7.34 (m, 3H), 7.34 - 7.30 (m, 3H), 7.27 (d, J=5.5 Hz, 2H), 7.16 (d, J=7.7 Hz, 1H), 6.44 (d, J=2.3 Hz, 1H), 5.47 (s, 1H), 4.42 (d, J=5.5 Hz, 2H), 2.84 (d, J=2.4 Hz, 1H), 2.44 - 2.32 (m, 2H), 2.03 (d, J=9.4 Hz, 1H), 1.95 - 1.74 (m, 4H), 1.66 - 1.40 (m, 4H), 0.95 - 0.88 (m, 2H), 0.66 - 0.60 (m, 2H).

The mixture was separated using SFC (YMC Amylose-C 4.6x250 mm, 5 µm 50/50 IPA (0.1 % DEA) / CO₂, 0.95 mL/min, 120 bar, 40 °C) to give two diastereomers: (1S,2S)-2-(3-chlorophenyl)-*N*-(4-(((6-cyclopropyl-8-((1*R*,4*S*)-3-oxo-2-azabicyclo[2.2.1]heptan-2-yl)imidazo[1,2-*a*]pyridin-2-yl)methyl)amino)pyridin-2-yl)cyclopropane-1-carboxamide and (1S,2S)-2-(3-chlorophenyl)-*N*-(4-(((6-cyclopropyl-8-((1*S*,4*R*)-3-oxo-2-azablcyclo[2.2.1]heptan-2-yl)imidazo[1,2-*a*]pyridin-2-yl)methyl)amino)pyridin-2-yl)cyclopropane-1-carboxamide.

First eluting isomer **(I-93,** 8.8 mg): ESI-MS (M+H)+: 567.2, δ 10.52 - 10.52 (m, 1H), 8.17 (d, J=1.1 Hz, 1H), 7.79 (d, J=6.0 Hz, 1H), 7.69 (s, 1H), 7.36 - 7.34 (m, 3H), 7.34 - 7.30 (m, 3H), 7.27 (d, J=5.5 Hz, 2H), 7.16 (d, J=7.7 Hz, 1H), 6.44 (d, J=2.3 Hz, 1H), 5.47 (s, 1H), 4.42 (d, J=5.5 Hz, 2H), 2.84 (d, J=2.4 Hz, 1H), 2.44 - 2.32 (m, 2H), 2.03 (d, J=9.4 Hz, 1H), 1.95 - 1.74 (m, 4H), 1.66 - 1.40 (m, 4H), 0.95 - 0.88 (m, 2H), 0.66 - 0.60 (m, 2H). RT = 2.02 min, 99.9 % e.e.

Second eluting isomer **(I-94,** 7.9 mg): ESI-MS (M+H)+: 567.3, ¹H NMR (400 MHz, DMSO) similar to **I-93.** RT = 2.8 min, 99.6 % e.e

### Examples 95-97

### (1S,2S)-2-(3-chlorophenyl)-N-(4-(((6-cyclopropyl-8-(2-oxo-3-azabicyclo[3.1.0]hexan-3-yl)imidazo[1,2-a]pyridin-2-yl)methyl)amino)pyridin-2-yl)cyclopropane-1-carboxamide (I-95)

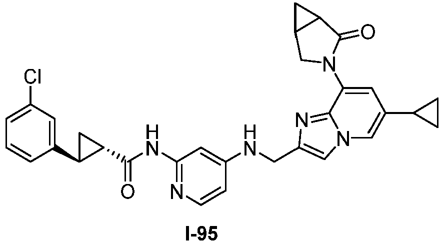

Using a similar procedure to that used for **(I-79), (I-95)** was prepared using (S,S)-2-(3-chlorophenyl)cyclopropane-1-carboxylic acid (32 mg, 0.16 mmol) and 3-(2-(((2-aminopyridin-4-yl)amino)methyl)-6-cyclopropylimidazo[1,2-a]pyridin-8-yl)-3-azabicyclo[3.1.0]hexan-2-one (60 mg, 0.16 mmol), then purified by preparative LCMS to give (I-95) (30 mg, 34 %) as a mixture of diastereomers. ESI-MS (M+H)+: 553, δ, ppm, 10.38 (1H, br s), 8.25 (1H, s), 7.80 (1H, d, J = 6.4 Hz), 7.70 (1H, s), 7.36 - 7.25 (4H, m), 7.17 (1H, d, J = 7.2 Hz), 7.06 (1H, s), 6.41 (1H, br s), 4.41 - 4.32 (3H, m), 4.00 (1H, dd, J = 5.4, 10.0 Hz), 2.42 (1H, br s), 2.32 (1H, br s), 2.10 (1H, br s), 2.01 (1H, br s), 1.95 - 1.87 (1H, m), 1.49 (1H, br s), 1.41 (1H, br s), 1.27 - 1.18 (2H, m), 0.92 (2H, dd, J = 2.0, 8.2 Hz), 0.86 (1H, br s), 0.66 - 0.62 (2H, m).

The mixture was separated using SFC (YMC Amylose-C 10x250 mm, 5 µm 55/45 MeOH (0.1 % DEA) / CO₂, 15 mL/min, 120 bar, 40°C) to give two diastereomers: (1S,2S)-2-(3-chlorophenyl)-*N*-(4-(((6-cyclopropyl-8-((1*S*,5*R*)-2-oxo-3-azabicyclo[3.1.0]hexan-3-yl)imidazo[1,2-a]pyridin-2-yl)methyl)amino)pyridin-2-yl)cyclopropane-1-carboxamide and (1S,2S)-2-(3-chlorophenyl)-*N*-(4-(((6-cyclopropyl-8-((1*R*,5*S*)-2-oxo-3-azabicyclo[3.1.0]hexan-3-yl)imidazo[1,2-*a*]pyridin-2-yl)methyl)amino)pyridin-2-yl)cyclopropane-1-carboxamide.

First eluting isomer **(I-96,** 12.68 mg): ESI-MS (M+H)+: 553, δ, ppm, 10.35 (1H, s), 8.26 (1H, dd, J = 0.5, 1.6 Hz), 7.79 (1H, d, J = 6.0 Hz), 7.68 (1H, s), 7.49 (1H, s), 7.35 (1H, ddd, J = 7.4, 7.4, 1.3 Hz), 7.30 - 7.27 (2H, m), 7.18 (1H, ddd, J = 1.3, 1.3, 7.4 Hz), 7.15 (1H, d, J = 6.0 Hz), 7.08 (1H, d, J = 1.6 Hz), 6.36 (1H, dd, J = 2.2, 5.8 Hz), 4.41 - 4.37 (3H, m), 4.03 (1H, dd, J = 1.2, 10.3 Hz), 2.41 - 2.40 (2H, m), 2.14 - 2.14 (1H, m), 2.04 - 2.04 (1H, m), 1.97 - 1.89 (1H, m), 1.51 - 1.45 (1H, m), 1.41 - 1.35 (1H, m), 1.27 - 1.21 (1H, m), 0.96 - 0.92 (2H, m), 0.88 (1H, ddd, J = 4.2, 4.2, 3.3 Hz), 0.69 - 0.65 (2H, m). RT = 8.73 min, 99.9 % e.e.

Second eluting isomer **(I-97,** 17.75 mg): ESI-MS (M+H)+: 553, ¹H NMR (400 MHz, DMSO): similar to **(I-96).** RT = 12.64 min, 98 % e.e.

### Example 98: rac-(1S*,2S*)-2-(3-chlorophenyl)-N-(4-(((6-cyclopropyl-8-(hydroxymethyl) imidazo[1,2-a]pyridin-2-yl)methyl)amino)pyridin-2-yl)cyclopropane-1-carboxamide (I-98)

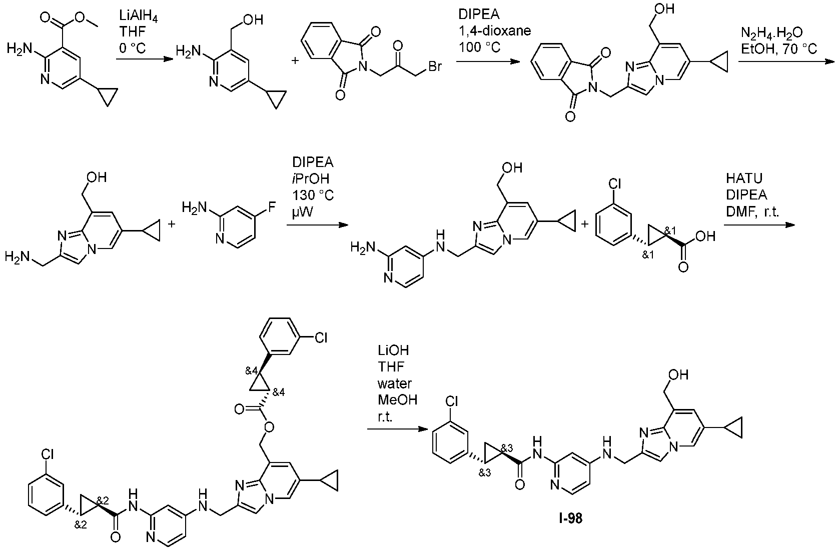

LiAlH₄ solution (1.0 M in THF, 12.5 mL, 12.5 mmol) was added dropwise to a stirred solution of methyl 2-amino-5-cyclopropylnicotinate (3.0 g, 16 mmol) in THF (60 mL) at 0 °C under a N₂ atmosphere. The mixture was stirred at 0 °C for 2 h, then further LiAlH₄ (1.0 M in THF, 2.5 mL, 2.5 mmol) was added dropwise. The mixture was stirred for 30 min, then water (0.57 mL) was added, followed by NaOH (20 % aq., 0.57 mL) and water (1.7 mL). The mixture was warmed to room temperature and diluted with Et₂O (60 mL). After 15 min MgSO₄ was added and the mixture was stirred for 75 min, then filtered through Celite^{®} with Et₂O. The filtrate was concentrated *in vacuo* to give **(2-amino-5-cyclopropylpyridin-3-yl)methanol** (2.5 g, 98 %) as a yellow oil. ESI-MS (M+H)+: 311.2, δ 7.75 (d, J=1.8 Hz, 1H), 7.12 (s, 1H), 5.47 (s, 2H), 5.18 - 5.13 (m, 1H), 4.36 (d, J=5.3 Hz, 2H), 1.85 - 1.77 (m, 1H), 0.91 - 0.84 (m, 2H), 0.60 - 0.54 (m, 2H).

A mixture of (2-amino-5-cyclopropylpyridin-3-yl)methanol (0.50 g, 3.0 mmol), 2-(3-bromo-2-oxopropyl)isoindoline-1,3-dione (1.3 g, 3.0 mmol), and DIPEA (0.53 mL, 3.0 mmol) in 1,4-dioxane (10 mL) was stirred at 100 °C for 2 h, then cooled to room temperature and allowed to stand for 18 h. The mixture was concentrated *in vacuo,* and the residue was purified by column chromatography on silica gel, eluting with 1-10 % MeOH in DCM to give **2-((6-cyclopropyl-8-(hydroxymethyl)imidazo[1,2-a]pyridin-2-yl)methyl) isoindoline-1,3-dione** (0.50 g, 47 %) as an orange gum. ESI-MS (M+H)+: 348.2, δ 8.15 (s, 1H), 7.95 - 7.87 (m, 4H), 7.73 (s, 1H), 6.98 (d, J=1.3 Hz, 1H), 5.32 - 5.28 (m, 1H), 4.87 (s, 2H), 4.73 (d, J=5.8 Hz, 2H), 1.98 - 1.89 (m, 1H), 0.95 - 0.89 (m, 2H), 0.67 - 0.61 (m, 2H).

***Alternative synthesis (2-(aminomethyl)-6-cyclopropylimidazo[1,2-a]pyridin-8-yl)methanol (intermediate 266a).*** A mixture of 2-((6-cyclopropyl-8-(hydroxymethyl)imidazo[1,2-a]pyridin-2-yl)methyl)isoindoline-1,3-dione (0.50 g, 1.4 mmol) and hydrazine monohydrate (0.18 mL, 2.9 mmol) in EtOH (14 mL) was stirred at 70 °C for 18 h. The mixture was loaded onto an SCX cartridge and washed with MeOH. The product was eluted with 7 N NH₃ in MeOH to give **intermediate 266a** (0.25 g, 80 %) as an orange gum. ESI-MS (M+H)+: 218.2, δ 8.21 (s, 1H), 7.65 (s, 1H), 6.95 (d, J=1.4 Hz, 1H), 4.77 (s, 2H), 3.79 (s, 2H), 3.18 (s, 1H), 1.99 - 1.90 (m, 1H), 0.93 (ddd, J=4.3, 6.3, 8.3 Hz, 2H), 0.69 - 0.64 (m, 2H). NH₂ not observed

A mixture of **intermediate 266a** (0.20 g, 0.92 mmol), 4-fluoropyridin-2-amine (0.10 g, 0.92 mmol), and DIPEA (0.24 mL, 1.4 mmol) in iPrOH (2.0 mL) was stirred at 130 °C in a microwave for 2 h. The mixture was concentrated *in vacuo* and the residue was purified by column chromatography on silica gel, eluting with a gradient of 1-50 % (7 N NH₃ in MeOH) in DCM to give **(2-(((2-aminopyridin-4-yl)amino)methyl)-6-cyclopropylimidazo[1,2-a]pyridin-8-yl)methanol** (0.14 g, 50 %) as an orange gum. ESI-MS (M+H)+: 310.2, δ 8.25 (s, 1H), 7.65 (s, 1H), 7.50 (d, J=5.8 Hz, 1H), 7.03 (d, J=1.5 Hz, 1H), 6.64 - 6.59 (m, 1H), 5.96 (dd, J=2.1, 5.9 Hz, 1H), 5.65 (d, J=2.0 Hz, 1H), 5.38 - 5.32 (m, 3H), 4.83 (d, J=5.3 Hz, 2H), 4.34 (d, J=5.8 Hz, 2H), 2.02 - 1.93 (m, 1H), 0.99 - 0.93 (m, 2H), 0.72 - 0.67 (m, 2H).

A mixture of (2-(((2-aminopyridin-4-yl)amino)methyl)-6-cyclopropylimidazo[1,2-a]pyridin-8-yl)methanol (0.036 g, 0.12 mmol), **intermediate 1149** (0.023 g, 0.12 mmol), HATU (0.049 g, 0.13 mmol), and DIPEA (0.041 mL, 0.23 mmol) in DMF (1.0 mL) was stirred at room temperature for 18 h. Water (10 mL) and brine (sat. aq., 15 mL) were added, and the mixture was extracted with EtOAc (3 × 25mL). The combined organics were dried (MgSO₄), filtered, and concentrated in vacuo to give **(2-(((2-((rac-(1S*,2S*))-2-(3-chlorophenyl)cyclopropane-1-carboxamido)pyridin-4-yl)amino)methyl)-6-cyclopropylimidazo[1,2-a]pyridin-8-yl)methyl (rac-(1S*,2S*))-2-(3-chlorophenyl)cyclopropane-1-carboxylate** (0.070 g, 91 %), which was used directly in the next step without further purification.]

A mixture of (2-(((2-((rac-(1S*,2S*))-2-(3-chlorophenyl)cyclopropane-1-carboxamido) pyridin-4-yl)amino)methyl)-6-cyclopropylimidazo[1,2-a]pyridin-8-yl)methyl (rac-(1S*,2S*))-2-(3-chlorophenyl)cyclopropane-1-carboxylate (0.045 g, 0.068 mmol) and LiOH (1.0 M aq., 0.034 mL, 0.034 mmol) in THF (3.0 mL) was stirred at room temperature for 3 h. MeOH (0.5 mL) was added and the mixture was stirred at room temperature for 30 min. Further LiOH (1.0 M aq., 0.10 mL, 0.10 mmol) was added and the mixture was stirred at room temperature for 1 h. The mixture was concentrated in vacuo, then dissolved in brine (sat. aq., 25 mL) and water (25 mL). The mixture was extracted with EtOAc (3 × 50 mL) and the combined organic layers were dried (MgSO₄), filtered, and concentrated in vacuo. The residue was purified by preparative HPLC to give **(I-98)** (2.3 mg, 7 %) as a white solid (formic acid salt). ESI-MS (M+H)+: 488.2, δ 10.31 (s, 1H), 8.27 - 8.26 (m, 1H), 8.20 (s, 1H), 7.75 (d, J=5.7 Hz, 1H), 7.62 (s, 1H), 7.45 - 7.42 (m, 1H), 7.34 - 7.28 (m, 1H), 7.26 - 7.23 (m, 2H), 7.16 - 7.09 (m, 2H), 6.99 - 6.97 (m, 1H), 6.31 (dd, J=2.1, 5.8 Hz, 1H), 5.31 (br s, 1H), 4.78 (s, 2H), 4.35 (d, J=5.7 Hz, 2H), 2.40 - 2.31 (m, 2H), 1.96 - 1.88 (m, 1H), 1.47 - 1.41 (m, 1H), 1.37 - 1.31 (m, 1H), 0.94 - 0.88 (m, 2H), 0.67 - 0.62 (m, 2H).

### Intermediate 704: N⁴-((8-(((tert-butyldimethylsilyl)oxy)methyl)-6-cyclopropylimidazo[1,2-a]pyridin-2-yl)methyl)pyridine-2,4-diamine

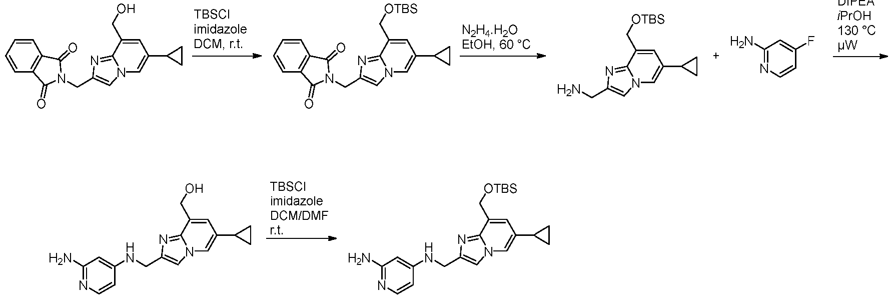

**Step 1:** TBDMS-Cl (0.65 g, 4.3 mmol) and imidazole (0.49 g, 7.2 mmol) were added to a solution of 2-((6-cyclopropyl-8-(hydroxymethyl)imidazo[1,2-a]pyridin-2-yl)methyl)isoindoline-1,3-dione (1.3 g, 3.6 mmol) in DCM (30 mL). The mixture was stirred at room temperature for 18 h. Water (100 mL) was added and the mixture was extracted with DCM (3 × 100 ml). The combined organic layers were dried over MgSO₄, filtered, and concentrated in vacuo. The residue was purified by column chromatography on silica gel, eluting with a gradient of 20-80 % EtOAc in cyclohexane to give **2-((8-(((tert-butyldimethylsilyl)oxy)methyl)-6-cyclopropylimidazo[1,2-a]pyridin-2-yl)methyl)isoindoline-1,3-dione** (0.89 g, 54 %) as an orange solid. ESI-MS (M+H)+: 462.4, δ 8.22 (s, 1H), 7.97 - 7.89 (m, 4H), 7.78 (s, 1H), 6.95 (d, J=1.1 Hz, 1H), 4.94 (s, 2H), 4.89 (s, 2H), 2.01 - 1.93 (m, 1H), 0.99 - 0.94 (m, 2H), 0.92 (s, 9H), 0.67 - 0.61 (m, 2H), 0.10 (s, 6H).

Step 2:. The product of step 1 (0.89 g, 1.9 mmol) and hydrazine monohydrate (0.24 mL, 3.9 mmol) in CHCl₃ (20 mL) was stirred at 60 °C for 18 h. The mixture was diluted with EtOAc and filtered. The filtrate was concentrated *in vacuo* to give **(8-(((tert-butyldimethylsilyl)oxy)methyl)-6-cyclopropylimidazo[1,2-a]pyridin-2-yl)methanamine** (0.82 g, quantitative) as an orange gum. δ 8.27 (s, 1H), 7.70 (s, 1H), 6.92 (d, J=1.5 Hz, 1H), 4.99 (s, 2H), 3.83 (s, 2H), 2.02 - 1.94 (m, 1H), 1.01 - 0.95 (m, 11H), 0.69 - 0.63 (m, 2H), 0.15 (s, 6H). NH₂ not observed.

**Step 3:** The product of step 2 (0.41 g, 1.2 mmol), 4-fluoropyridin-2-amine (0.14 g, 1.2 mmol), and DIPEA (0.65 mL, 3.7 mmol) in iPrOH (4.0 mL) was stirred at 130 °C in a microwave for 1.5 h. The mixture was then stirred at 130 °C in a microwave for a further 4 h. The mixture was concentrated *in vacuo* and the residue was purified by column chromatography on silica gel, eluting with 0-100 % 7 N NH₃ in MeOH in DCM to give **(2-(((2-aminopyridin-4-yl)amino)methyl)-6-cyclopropylimidazo[1,2-a]pyridin-8-yl)methanol** (0.11 g, 21 %) as a yellow gum. ESI-MS (M+H)+: 310, δ 8.21 (s, 1H), 8.09 (dd, J=3.4, 5.9 Hz, 1H), 7.89 (dd, J=3.3, 6.0 Hz, 1H), 7.62 (s, 1H), 7.46 (d, J=6.0 Hz, 1H), 6.99 (d, J=1.4 Hz, 1H), 6.73 - 6.68 (m, 1H), 5.94 (dd, J=2.1, 6.0 Hz, 1H), 5.62 (d, J=2.0 Hz, 1H), 5.46 (s, 2H), 4.79 (s, 2H), 4.31 (d, J=5.8 Hz, 2H), 3.18 (s, 1H), 1.98 - 1.90 (m, 1H), 0.96 - 0.89 (m, 2H), 0.68 - 0.63 (m, 2H).

TBDMS-Cl (0.22 g, 1.5 mmol) and imidazole (0.10 g, 1.5 mmol) were added to a solution of the product of step 3 (0.15 g, 0.49 mmol) in DCM (10 mL) and DMF (5 mL). The mixture was stirred at room temperature for 18 h. Water (50 mL) and NaHCO₃ (sat. aq., 50 mL) were added, and the mixture was extracted with DCM (3 × 50 ml). The combined organic layers were dried over MgSO₄, filtered, and concentrated in vacuo. The residue was purified by column chromatography on silica gel, eluting with 0-10 % methanolic ammonia in DCM to give **intermediate 704** (0.078 g, 37 %) as a yellow oil. δ 8.27 (s, 1H), 7.65 (s, 1H), 7.48 (d, J=5.9 Hz, 1H), 6.95 (d, J=1.5 Hz, 1H), 6.61 - 6.56 (m, 1H), 5.93 (dd, J=2.1, 5.8 Hz, 1H), 5.63 (d, J=2.0 Hz, 1H), 5.30 (s, 2H), 5.01 (s, 2H), 4.32 (d, J=5.8 Hz, 2H), 2.01 - 1.93 (m, 1H), 0.98 (s, 11H), 0.67 - 0.62 (m, 2H), 0.16 (s, 6H).

### Example 99: (1S,2S)-2-(3-chlorophenyl)-N-(4-(((6-cyclopropyl-8-(hydroxymethyl)imidazo[1,2-a]pyridin-2-yl)methyl)amino)pyridin-2-yl)cyclopropane-1-carboxamide (I-99)

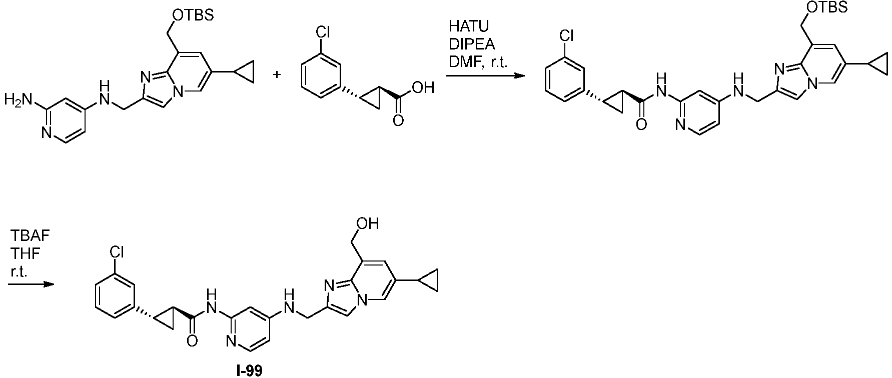

Synthesis. A mixture of **intermediate 704** (0.11 g, 0.26 mmol), (1S,2S)-2-(3-chlorophenyl)cyclopropane-1-carboxylic acid (0.050 g, 0.26 mmol), HATU (0.11 g, 0.28 mmol), and DIPEA (0.089 mL, 0.51 mmol) in DMF (3.0 mL) was stirred at room temperature for 18 h. Water (50 mL) and NaHCO₃ (sat. aq., 50 mL) were added and the mixture was extracted with DCM (3 × 50 mL). The combined organics were dried over MgSO₄, filtered, and concentrated in vacuo. The residue was purified by column chromatography on silica gel, eluting with 0-20 % 7 N NH₃ in MeOH in DCM to give of **(1S,2S)-N-(4-(((8-(((tert-butyldimethylsilyl)oxy)methyl)-6-cyclo propylimidazo [1,2-a]pyridin-2-yl)methyl)amino)pyridin-2-yl)-2-(3-chlorophenyl)cyclo propane-1-carboxamide intermediate 705** (0.076 g, 51 %) as an orange oil, which was used directly in the next step without further purification.

A mixture of **intermediate 705(0.076** g, 0.13 mmol), and TBAF (1.0 M in THF, 0.15 mL, 0.15 mmol) in THF (3 mL) was stirred at room temperature for 3 h. Water (20 mL) and brine (sat. aq., 5 mL) were added, and the mixture was extracted with EtOAc (3 × 25 mL). The combined organics were dried over MgSO₄, filtered, and concentrated in vacuo. The residue was purified by column chromatography on silica gel, eluting with 0-10 % 7 N NH₃ in MeOH in DCM to give **(I-99)** (0.011g, 17 %) as a yellow solid. ESI-MS (M+H)+: 488.3, ¹H NMR (400 MHz, DMSO) δ 10.31 (s, 1H), 8.21 - 8.20 (m, 1H), 7.75 (d, J=5.7 Hz, 1H), 7.62 (s, 1H), 7.43 (br s, 1H), 7.32 - 7.23 (m, 3H), 7.16 - 7.11 (m, 2H), 6.99 - 6.97 (m, 1H), 6.31 (dd, J=2.1, 5.7 Hz, 1H), 5.31 (t, J=5.8 Hz, 1H), 4.78 (d, J=5.6 Hz, 2H), 4.35 (d, J=5.7 Hz, 2H), 2.41 - 2.31 (m, 2H), 1.96 - 1.88 (m, 1H), 1.47 - 1.41 (m, 1H), 1.38 - 1.31 (m, 1H), 0.94 - 0.87 (m, 2H), 0.67 - 0.62 (m, 2H).

### Example 100: rac-(1S*,2S*)-2-(5-chloro-2-cyanophenyl)-N-(4-(((6-cyclopropyl-8-(hydroxy methyl)imidazo[1,2-a]pyridin-2-yl)methyl)amino)pyridin-2-yl)cyclopropane-1-carboxamide (I-100)

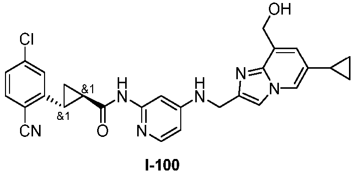

Using a similar procedure to that used for **(I-99), (I-100)** was prepared from *rac-(1S*,2S*)-*(5-chloro-2-cyanophenyl)cyclopropane-1-carboxylic acid and **intermediate 704** (3.3 mg, 6%). ESI-MS (M+H)+: 513.3, δ 10.47 - 10.47 (m, 1H), 8.21 (s, 1H), 7.86 (d, J=8.4 Hz, 1H), 7.79 - 7.76 (m, 1H), 7.66 (s, 1H), 7.55 - 7.50 (m, 1H), 7.41 (s, 1H), 6.99 (s, 1H), 5.35 - 5.31 (m, 1H), 4.78 (d, J=5.3 Hz, 2H), 4.40 - 4.39 (m, 2H), 1.95 - 1.89 (m, 1H), 1.57 - 1.57 (m, 2H), 0.96 - 0.88 (m, 2H), 0.66 - 0.64 (m, 2H). Two cyclopropyl CH signals obscured by DMSO signal. Three signals in aromatic region missing and/or extremely broad.

### Intermediate 706: N⁵-((6-cyclopropylimidazo[1,2-a]pyridin-2-yl)methyl)pyridazine-3,5-diamine

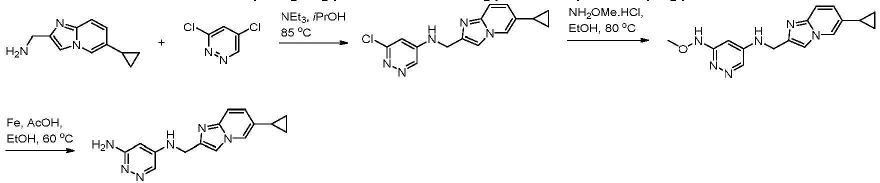

. Triethylamine (0.56 mL, 4.0 mmol) was added to a stirred mixture of intermediate 251 (300 mg, 1.3 mmol) and 3,5-dichloropyridazine (240 mg, 1.6 mmol) in iPrOH (3.0 mL). The reaction was then stirred at 85 °C for 18 h. The reaction was cooled to room temperature and diluted with EtOAc (30 mL) and water (30 mL). The layers were separated, and the aqueous layer was further extracted with EtOAc (2 × 20 mL). The organic layers were combined, dried over MgSO₄ and concentrated *in vacuo.* The residue was purified by column chromatography on silica gel, eluting with a gradient of 0-5 % MeOH in DCM to give **6-chloro-N-((6-cyclopropylimidazo[1,2-a]pyridin-2-yl)methyl)pyridazin-4-amine intermediate 707** (0.16 g, 41 %). ESI-MS (M+H)+: 300.0, 302.0, δ 8.65 (s, 1H), 8.34 (t, J=1.3 Hz, 1H), 7.93 (s, 1H), 7.77 (s, 1H), 7.42 (d, J=10.4 Hz, 1H), 7.01 (dd, J=1.8, 9.2 Hz, 1H), 6.83 (d, J=2.3 Hz, 1H), 4.47 (d, J=6.0 Hz, 2H), 1.98 - 1.90 (m, 1H), 0.96 - 0.91 (m, 2H), 0.71 - 0.67 (m, 2H).

O-Methyl hydroxylamine hydrochloride (690 mg, 8.2 mmol) was added to a stirred mixture of **intermediate 707** (160 mg, 0.55 mmol) in ethanol (9.0 mL), and the reaction was stirred at 80 °C for 24 h. The reaction was cooled to room temperature and filtered through Celite^{®}. The filtrate was concentrated *in vacuo.* The residue was suspended in NaHCO₃ (sat. aq., 30 mL) and extracted with DCM (3 × 30 mL). The organic layers were combined, dried over MgSO₄, and concentrated *in vacuo* to give **N-((6-cyclopropylimidazo[1,2-a]pyridin-2-yl)methyl)-6-(methoxyamino) pyridazin-4-amine intermediate 708** d as a crude oil (183 mg), which was carried forward to the next step without further purification. ESI-MS (M+H)+: 311.2.

Iron powder (170 mg, 3.0 mmol) and acetic acid (20 % aq., 2.8 mL, 9.8 mmol) were added to a stirred solution of **intermediate 708** (180 mg, 0.59 mol) in ethanol (19 mL). The reaction was heated at 60 °C for 2.5 h. The reaction was cooled to room temperature, diluted with EtOAc (50 mL), and filtered through Celite^{®}. The filtrate was concentrated *in vacuo,* then the residue was suspended in DCM/MeOH (1: 1, 10 mL) and filtered. The filtrate was concentrated *in vacuo.* The residue was purified by column chromatography on silica gel, eluting with a gradient of 0-5 % (7N NH₃ in MeOH) in DCM to give **intermediate 706** (0.097 g, 59 %). ESI-MS (M+H)+: 281.2, ¹H NMR (400 MHz, DMSO) δ 8.24 (s, 1H), 7.94 (d, J=2.3 Hz, 1H), 7.57 (s, 1H), 7.31 (d, J=9.3 Hz, 1H), 6.99 (dd, J=5.3, 5.3 Hz, 1H), 6.91 (dd, J=1.3, 9.3 Hz, 1H), 5.65 (s, 3H), 4.25 (d, J=5.6 Hz, 2H), 1.89 - 1.80 (m, 1H), 0.88 - 0.81 (m, 2H), 0.63 - 0.56 (m, 2H).
Using a similar procedure to that used for **I-79,** the compounds in Table 12 were prepared using *N*⁵-((6-cyclopropylimidazo[1,2-*a*]pyridin-2-yl)methyl)pyridazine-3,5-diamine and an appropriate acid and purified by preparative LCMS. **Table 12**

| **EG** | **Compound** | **Coupling Partner / Analytical Data** |
|---|---|---|
| **101** | 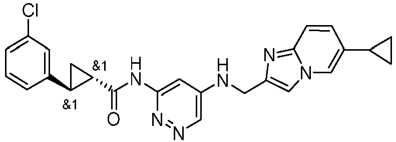 ***rac*-(1*S**,2*S**)-2-(3-chlorophenyl)-*N*-(5-(((6-cyclopropylimidazo[1,2-a]pyridin-2-yl)methyl)amino)pyridazin-3-yl)cyclopropane-1-carboxamide (I-101)** from *rac*-(1S*,2S*)-2-(3-chlorophenyl) cyclopropane-1-carboxylic acid | ESI-MS (M+H)+: 459.2, δ 10.86 (s, 1H), 8.44 (d, J=2.5 Hz, 1H), 8.33 (s, 1H), 7.70 (s, 1H), 7.56 (dd, J=5.8, 5.8 Hz, 1H), 7.50 (d, J=1.9 Hz, 1H), 7.41 (d, J=9.4 Hz, 1H), 7.36 -7.30 (m, 1H), 7.26 (dd, J=1.6, 3.5 Hz, 2H), 7.16 (d, J=7.7 Hz, 1H), 7.00 (dd, J=1.7, 9.3 Hz, 1H), 4.41 (d, J=5.6 Hz, 2H), 2.41 (dd, J=6.5, 6.5 Hz, 2H), 2.01 - 1.89 (m, 1H), 1.52 - 1.37 (m, 2H), 0.96 - 0.89 (m, 2H), 0.71 - 0.65 (m, 2H). |
| **102** | 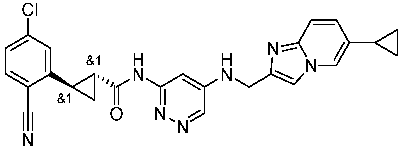 ***rac*-(1*S**,2*S**)-2-(5-chloro-2-cyanophenyl)-N-(5-(((6-cyclopropyl imidazo[1,2-*a*]pyridin-2-yl)methyl) amino)pyridazin-3-yl)cyclopropane-1-carboxamide (I-102)** | *rac-*(1*S**,2*S**)-2-(5-chloro-2-cyanophenyl) cyclopropane-1-carboxylic acid |
| | | ESI-MS (M+H)+: 484.3, δ 10.91 (s, 1H), 8.46 - 8.43 (m, 1H), 8.33 (s, 1H), 7.87 (d, J=8.6 Hz, 1H), 7.71 (s, 1H), 7.57 (s, 1H), 7.53 (d, J=9.9 Hz, 2H), 7.41 (d, J=9.1 Hz, 2H), 7.00 (d, J=9.1 Hz, 1H), 4.42 (d, J=4.5 Hz, 2H), 2.60 - 2.55 (m, 2H), 1.94 - 1.89 (m, 1H), 1.62 - 1.55 (m, 2H), 0.93 (d, J=7.1 Hz, 2H), 0.68 (d, J=4.3 Hz, 2H). |

### Example 103: rac-(1S*,2S*)-2-(3-chlorophenyl)-N-(5-((6-cyclopropylimidazo[1,2-a]pyridin-2-yl)methoxy)pyridazin-3-yl)cyclopropane-1-carboxamide (I-103)

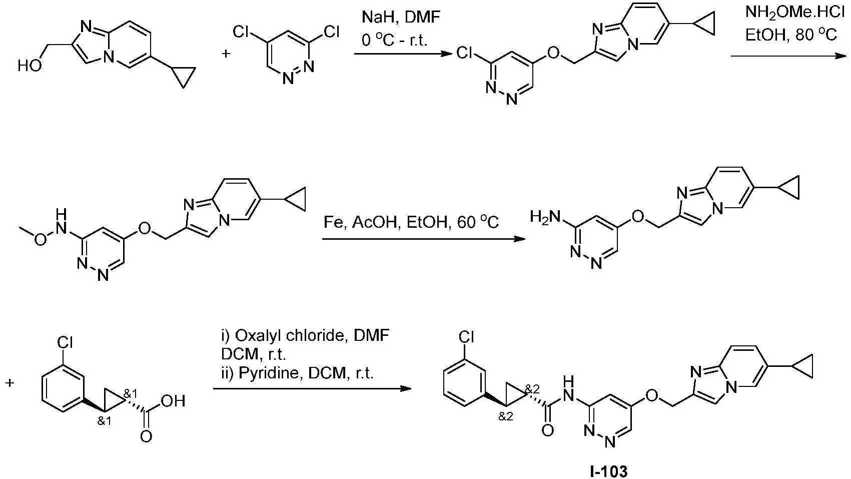

**Intermediate 827** (150 mg, 0.80 mmol) in DMF (1.0 mL) was added to a stirred mixture of NaH (60 % in mineral oil, 54 mg, 1.4 mmol) in DMF (1.0 mL) at 0 °C and stirred at 0 °C for 2 h. 3,5-Dichloropyridazine (140 mg, 0.96 mmol) in DMF (1.0 mL) was then added and the reaction was allowed to warm to room temperature and stirred for 18 h. The reaction was quenched with water (10 mL) and extracted with DCM (3 × 15 mL). The organic layers were combined, dried over MgSO₄, and concentrated *in vacuo.* The residue was purified by column chromatography on silica gel, eluting with a gradient of 0-100% EtOAc in DCM to give **2-(((6-chloropyridazin-4-yl)oxy)methyl)-6-cyclopropylimidazo[1,2-a]pyridine intermediate 709** (0.17 g, 70 %). ESI-MS (M+H)+: 301.0, ¹H NMR (400 MHz, CDCl₃) δ 8.91 (d, J=2.5 Hz, 1H), 7.89 (s, 1H), 7.57 (s, 1H), 7.49 (d, J=9.3 Hz, 1H), 7.18 (d, J=2.5 Hz, 1H), 6.99 (dd, J=1.5, 9.3 Hz, 1H), 5.34 (s, 2H), 1.94 - 1.86 (m, 1H), 1.02 - 0.95 (m, 2H), 0.72 - 0.65 (m, 2H).

O-Methyl hydroxylamine hydrochloride (830 mg, 10 mmol) was added to a stirred mixture of **intermediate 709** (200 mg, 0.67 mmol) in EtOH (25.0 mL) and the reaction was stirred at 80 °C for 24 h. The reaction was cooled to room temperature and filtered through Celite^{®}. The filtrate was concentrated *in vacuo,* and the residue was suspended in NaHCO₃ (sat. aq., 30 mL) and extracted with DCM (3 × 30 mL). The organic layers were combined, dried over MgSO₄, and concentrated *in vacuo.* The residue was purified by column chromatography on silica gel, eluting with a gradient of 0-10 % MeOH in DCM to give **N-(5-((6-cyclopropylimidazo[1,2-a]pyridin-2-yl)methoxy) pyridazin-3-yl)-O-methylhydroxylamine intermediate 710** (0.11 g, 53 %). ESI-MS (M+H)+: 312, ¹H NMR (400 MHz, CDCl₃) δ 9.29 (s, 1H), 7.88 (s, 1H), 7.54 (s, 1H), 7.47 (d, J=9.3 Hz, 1H), 7.16 (s, 1H), 6.97 (dd, J=1.6, 9.0 Hz, 1H), 5.93 (s, 1H), 5.08 (s, 2H), 3.80 (s, 3H), 1.94 - 1.86 (m, 1H), 1.01 - 0.95 (m, 2H), 0.72 - 0.66 (m, 2H).
Iron powder (110 mg, 1.9 mmol) and acetic acid (20 % aq., 0.9 mL) were added to a stirred solution of **intermediate 710** (120 mg, 0.38 mol) in EtOH (6.0 mL). The reaction was heated at 60 °C for 1 h. The reaction was cooled to room temperature, diluted with EtOAc (50 mL), and filtered through Celite^{®}. The filtrate was concentrated *in vacuo,* and the residue was suspended in DCM/MeOH (1:1, 10 mL) and filtered. The filtrate was concentrated *in vacuo.* The residue was purified by column chromatography on silica gel, eluting with a gradient of 0-5 % (7 N NH₃ in MeOH) in DCM to give **5-((6-cyclopropylimidazo[1,2-a]pyridin-2-yl)methoxy)pyridazin-3-amine intermediate 711** (0.10 g, 95 %) which was carried through to the next step without further purification. ESI-MS (M+H)+: 282.1.

. Oxalyl chloride (48 µL, 0.55 mmol) was added to a stirred solution of **intermediate 1149** (36 mg, 0.18 mmol) and DMF (3.5 µL, 0.046 mmol) in DCM (1.0 mL) and stirred at room temperature for 20 min. The reaction was concentrated in vacuo. The residue was dissolved in DCM (2.0 mL) and added to a stirred solution of **intermediate 711** (51 mg, 0.18 mmol) in DCM (1.0 mL). Pyridine (30 µL, 0.37 mmol) was then added and the reaction was stirred at room temperature for 18 h. The reaction was quenched with water (20 mL) and extracted with DCM (3 × 15 mL). The organic layers were combined, dried over MgSO₄, and concentrated in vacuo. The residue was purified by reverse phase preparative HPLC to give **(I-103)** (4.1 mg, 5 %). ESI-MS (M+H)+: 460.3, ¹H NMR (400 MHz, DMSO) δ 11.36 (s, 1H), 8.83 (d, J=2.8 Hz, 1H), 8.38 (s, 1H), 8.08 (d, J=2.8 Hz, 1H), 7.94 (s, 1H), 7.46 (d, J=9.5 Hz, 1H), 7.37 - 7.27 (m, 3H), 7.19 (d, J=7.6 Hz, 1H), 7.04 (dd, J=1.6, 9.4 Hz, 1H), 5.35 (s, 2H), 2.49 - 2.42 (m, 2H), 2.00 - 1.92 (m, 1H), 1.58 - 1.46 (m, 2H), 0.98 - 0.91 (m, 2H), 0.73 - 0.69 (m, 2H).

### Example 104: rac-(1S*,2S*)-2-(3-chlorophenyl)-N-(6-((6-cyclopropylimidazo[1,2-a]pyridin-2-yl)methoxy)pyrimidin-4-yl)cyclopropane-1-carboxamide (I-104)

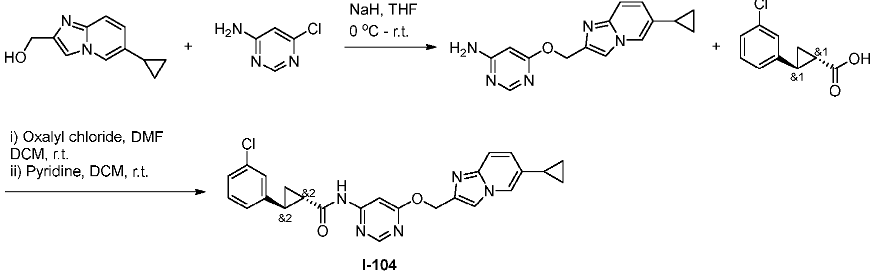

**Intermediate 827** (50 mg, 0.27 mmol) in THF (1.0 mL) was added to a stirred mixture of NaH (60 % in mineral oil, 21 mg, 0.53 mmol) in THF (1.0 mL) at room temperature and stirred for 2 h. **Intermediate 846** (41 mg, 0.32 mmol) in THF (2.0 mL) was then added, and the reaction was heated to 65 °C and stirred for 18 h. The reaction was cooled to room temperature, quenched with water (10 mL), and extracted with DCM (3 × 15 mL). The combined organic layers were combined, dried (MgSO₄), and concentrated *in vacuo* to give **6-((6-cyclopropylimidazo[1,2-a]pyridin-2-yl) methoxy)pyrimidin-4-amine intermediate 712** (0.065 g, 87 %) which was carried forward to the next step without further purification. ESI-MS (M+H)+: 282.1, ¹H NMR (400 MHz, CDCl₃) δ 8.31 (s, 1H), 7.86 (s, 1H), 7.55 (s, 1H), 7.48 (d, J=9.0 Hz, 1H), 6.96 - 6.92 (m, 1H), 5.86 (s, 1H), 5.52 (s, 2H), 4.68 (s, 2H), 1.92 - 1.85 (m, 1H), 0.99 - 0.93 (m, 2H), 0.70 - 0.64 (m, 2H).

Oxalyl chloride (60 µL, 0.69 mmol) was added to a stirred solution of **intermediate 1149** (45 mg, 0.23 mmol) and DMF (4.5 µL, 0.058 mmol) in DCM (1.0 mL) and stirred at room temperature for 20 min. The reaction was concentrated in vacuo. The residue was dissolved in DCM (2.0 mL) and added to a stirred solution of **intermediate 712** (65 mg, 0.23 mmol) in DCM (1.0 mL) at 0 °C. Pyridine (0.93 mL, 12 mmol) was then added and the reaction was stirred at 0 °C for 1 h. The reaction was quenched with water (20 mL) and extracted with DCM (3 × 15 mL). The organic layers were combined, dried over MgSO₄, and concentrated in vacuo. The residue was purified by reverse phase preparative HPLC to give **(I-104)** (39 mg, 37 %). ESI-MS (M+H)+: 460.2, δ 11.17 (s, 1H), 8.61 (d, J=0.9 Hz, 1H), 8.39 (s, 1H), 7.91 (s, 1H), 7.52 (d, J=0.9 Hz, 1H), 7.47 (d, J=9.6 Hz, 1H), 7.41 - 7.30 (m, 3H), 7.24 - 7.20 (m, 1H), 7.06 (dd, J=1.7, 9.4 Hz, 1H), 5.53 (s, 2H), 2.53 - 2.49 (m, 1H), 2.49 - 2.43 (m, 1H), 2.03 - 1.95 (m, 1H), 1.61 - 1.49 (m, 2H), 1.01 - 0.95 (m, 2H), 0.75 - 0.72 (m, 2H).

### Intermediate 713: 6-chloro-N-((6-cyclopropylimidazo[1,2-a]pyridin-2-yl)methyl)-2-(pyrrolidin-1-yl)pyrimidin-4-amine

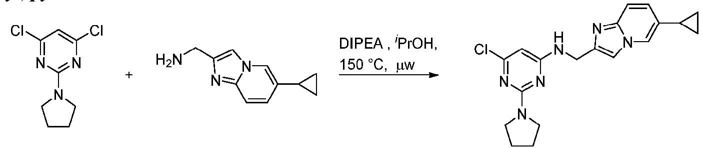

4,6-Dichloro-2-(pyrrolidin-1-yl)pyrimidine (50 mg, 0.23 mmol), intermediate 251 (47 mg, 0.25 mmol) and DIPEA (0.12 mL, 0.69 mmol) in iPrOH (2.0 mL) were heated to 150 °C in a microwave for 2.5 h. The mixture was cooled to room temperature, quenched with water (10 mL), and extracted into EtOAc (2×10 mL). The combined organics were passed through a hydrophobic frit and concentrated *in vacuo.* The residue was purified by column chromatography on silica gel, eluting with a gradient of 5-100 % EtOAc in cyclohexane to give **intermediate 713** (40 mg, 47 %). ESI-MS (M+H): 369, ¹H NMR (400 MHz, CDCl₃) δ 7.85 - 7.84 (m, 1H), 7.45 - 7.40 (m, 2H), 6.93 (dd, J=1.7, 9.2 Hz, 1H), 5.73 (s, 1H), 3.57 - 3.52 (m, 4H), 1.95 - 1.85 (m, 5H), 0.99 - 0.93 (m, 2H), 0.69 - 0.65 (m, 2H), -0.02 (s, 1H), -0.15 (s, 1H).
Using a similar procedure to that used for **intermediate 713,** the compounds in Table 13 were made from intermediate 251 and an appropriate coupling partner. **Table 13**

| **Compound** | **Coupling Partner / Analytical Data** |
|---|---|
| 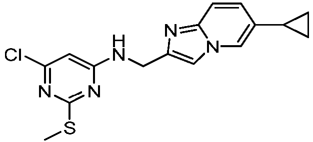 | **6-chloro-N-((6-cyclopropylimidazo[1,2-a]pyridin-2-yl) methyl)-2-(methylthio)pyrimidin-4-amine intermediate 714** from 2-(methylthio)-4,6-dichloropyrimidine |
| | ESI-MS (M+H): 346 |
| 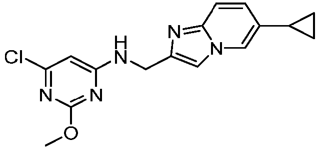 | **6-chloro-N-((6-cyclopropylimidazo[1,2-a]pyridin-2-yl)methyl)-2-methoxypyrimidin-4-amine intermediate 315** from 2-(methoxy)-4,6-dichloropyrimidine |
| | ESI-MS (M+H): 330 |
| 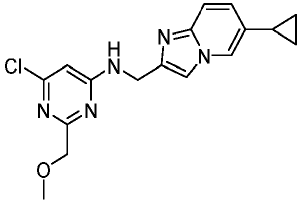 | **6-chloro-N-((6-cyclopropylimidazo[1,2-a]pyridin-2-yl)methyl)-2-(methoxymethyl)pyrimidin-4-amine intermediate 716** from 2-(methoxymethyl)-4,6-dichloropyrimidine |
| | ESI-MS (M+H): 344 |

### Example 105: rac-(1S*,2S*)-2-(3-chlorophenyl)-N-(6-(((6-cyclopropylimidazo[1,2-a]pyridin-2-yl)methyl)amino)-2-(pyrrolidin-1-yl)pyrimidin-4-yl)cyclopropane-1-carboxamide (I-105)

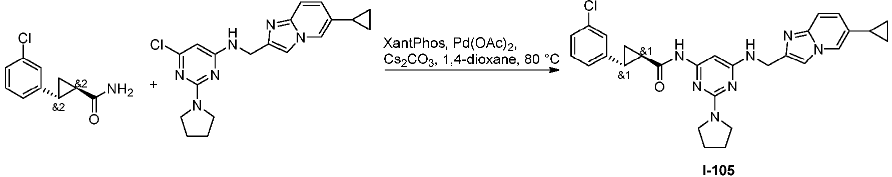

**Intermediate 1150:** rac-(1S*,2S*)-2-(3-Chlorophenyl)cyclopropane-1-carboxamide (21 mg, 0.11 mmol - prepared in a similar manner to intermediate 119), Pd(OAc)₂ (4.9 mg, 0.02 mmol), XantPhos (25 mg, 0.04 mmol), and Cs₂CO₃ (53 mg, 0.16 mmol) were placed under N₂ atmosphere. A solution of **intermediate 713** (40 mg, 0.11 mmol) in 1,4-dioxane (2.5 mL) was added, and the mixture was degassed for 15 min. The reaction mixture was stirred at 80 °C for 2 h. The reaction mixture was allowed to cool to room temperature, filtered through Celite^{®}, washed with MeOH, and concentrated *in vacuo.* The residue was purified by column chromatography on silica gel, eluting with a gradient of 5-100 % EtOAc in cyclohexane. The residue was further purified by preparative HPLC to give **(I-105)** (6.5 mg, 11 %) as a formic acid salt. ESI-MS (M+H): 528.3, δ 9.96 (s, 1H), 8.23 (s, 1H), 7.52 (s, 1H), 7.31 - 7.20 (m, 3H), 7.17 (d, J=9.1 Hz, 2H), 7.07 (d, J=7.6 Hz, 1H), 6.87 (d, J=9.3 Hz, 1H), 6.53 (s, 1H), 4.46 (s, 2H), 3.37 - 3.33 (m, 4H), 2.31 - 2.24 (m, 1H), 1.85 - 1.80 (m, 1H), 1.77 (s, 4H), 1.40 - 1.34 (m, 1H), 1.27 - 1.20 (m, 1H), 0.87 - 0.80 (m, 2H), 0.58 (q, J=5.2 Hz, 2H) 1 H missing under DMSO peak.

Using a similar procedure to that used for **(I-105),** the compounds in **Error! Reference source not found.** were prepared from **intermediate 1150** and an appropriate coupling partner.

**Table 14**

| **EG** | **Compound** | **Coupling Partner / Analytical Data** |
|---|---|---|
| **106** | 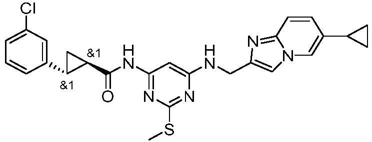 *rac*-(1S*,2S*)-2-(3-chlorophenyl)-*N*-(6-(((6-cyclopropylimidazo[1,2-*a*]pyridin-2-yl)methyl)amino)-2-(methylthio)pyrimidin-4-yl)cyclopropane-1-carboxamide **(I-106)** | **Intermediate 714** |
| | | ESI-MS (M+H): 505.4, δ 10.62 (s, 1H), 8.37 (s, 1H), 8.01 - 8.00 (m, 1H), 7.67 (s, 1H), 7.43 (d, J=9.6 Hz, 1H), 7.36 (d, J=6.8 Hz, 1H), 7.33 - 7.28 (m, 2H), 7.20 (d, J=7.3 Hz, 1H), 7.06 - 7.00 (m, 2H), 4.66 - 4.62 (s, 2H), 2.48 - 2.42 (m, 4H), 1.97 - 1.94 (m, 1H), 1.52 - 1.51 (m, 1H), 1.45 - 1.41 (m, 1H), 1.00 - 0.94 (m, 2H), 0.72 - 0.71 (m, 2H). One cyclopropyl C-H not observed - possibly under DMSO peak. |
| **107** | 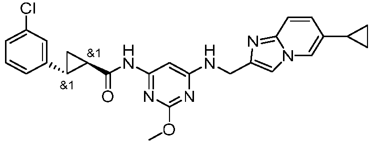 *rac*-(1*S**,2*S**)-2-(3-chlorophenyl)-N-(6-(((6-cyclopropylimidazo[1,2-a]pyridin-2-yl)methyl)amino)-2-methoxypyrimidin-4-yl)cyclopropane-1-carboxamide **(I-107)** | **Intermediate 315** |
| | | ESI-MS (M+H): 489.3, ¹) δ 10.53 (s, 1H), 8.35 (s, 1H), 7.67 (s, 1H), 7.43 (d, J=9.0 Hz, 1H), 7.36 (d, J=7.8 Hz, 1H), 7.31 (s, 1H), 7.30 (s, 1H), 7.20 (d, J=7.8 Hz, 1H), 7.03 (s, 1H), 7.01 (s, 1H), 6.60 (s, 1H), 4.61 (br s, 2H), 3.82 (s, 3H), 2.44 (t, J=7.0 Hz, 2H), 2.00 - 1.92 (m, 1H), 1.54 - 1.50 (m, 1H), 1.46 - 1.41 (m, 1H), 0.96 (q, J=6.2 Hz, 2H), 0.75 - 0.69 (m, 2H). |
| **108** | 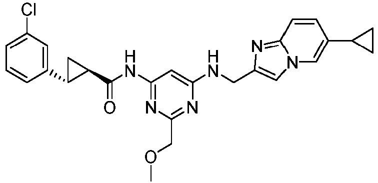 *rac*-(1S*,2S*)-2-(3-chlorophenyl)-N-(6-(((6-cyclopropylimidazo[1,2-*a*]pyridin-2-yl)methyl)amino)-2-(methoxymethyl)pyrimidin-4-yl)cyclopropane-1-carboxamide **(I-108)** | **Intermediate 716** |
| | | ESI-MS (M+H): 503.3, ¹ δ 10.70 (s, 1H), 8.30 (s, 1H), 7.82 (s, 1H), 7.62 (s, 1H), 7.37 (d, J=9.3 Hz, 1H), 7.31 (d, J=8.0 Hz, 1H), 7.26 (s, 1H), 7.25 (s, 1H), 7.19 - 7.11 (m, 2H), 6.96 (d, J=9.1 Hz, 1H), 4.57 (br s, 2H), 4.23 (s, 2H), 2.39 (t, J=7.1 Hz, 2H), 1.95 - 1.86 (m, 1H), 1.48 - 1.43 (m, 1H), 1.40 - 1.33 (m, 1H) 0.94 - 0.87 (m, 2H), 0.69 - 0.63 (m, 2H). * 3.37 (s, 3H) visible after D₂O shake. |

### Examples 109-110

### rac-(1S*,2S*)-2-(3-chlorophenyl)-N-(6-(((6-cyclopropylimidazo[1,2-a]pyridin-2-yl)methyl)amino)-2-(methylsulfinyl)pyrimidin-4-yl)cyclopropane-1-carboxamide (I-109) and rac-(1S*,2S*)-2-(3-chlorophenyl)-N-(6-(((6-cyclopropylimidazo[1,2-a]pyridin-2-yl)methyl)amino)-2-(methylsulfonyl)pyrimidin-4-yl)cyclopropane-1-carboxamide (I-110)

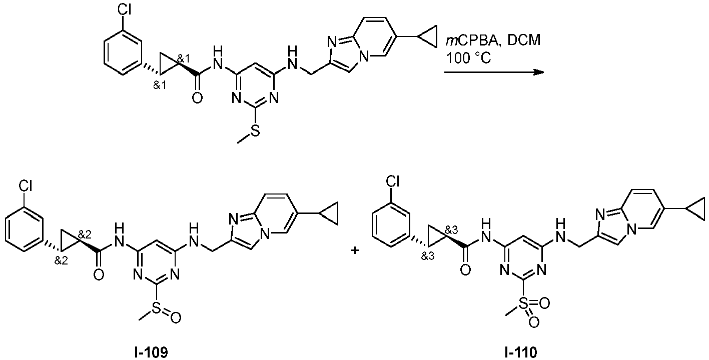

mCPBA (68 mg, 0.40 mmol) was added to a solution of **(I-106)** (100 mg, 0.20 mmol) in DCM (3.0 mL) at 0 °C. The reaction mixture was stirred for 4 h at 0 °C. The reaction mixture was allowed to warm to room temperature, quenched with water (10 mL), and extracted with DCM (3×10 mL). The combined organics were passed through a hydrophobic frit and concentrated in vacuo. The residue was purified by preparative HPLC to give **(I-109)** (11.2 mg, 11 %) and **(I-110)** (4.2 mg, 4 %).

**(I-109):** ESI-MS (M+H): 521.3, δ 11.04 (s, 1H), 8.38 (s, 1H), 8.30 (s, 1H), 7.66 (s, 1H), 7.37 (d, J=9.9 Hz, 1H), 7.35 - 7.30 (m, 2H), 7.28 - 7.23 (m, 2H), 7.15 (d, J=7.6 Hz, 1H), 6.97 (d, J=9.1 Hz, 1H), 4.62 (br s, 2H), 2.76 (s, 3H), 2.44 - 2.40 (m, 2H), 1.93 - 1.88 (m, 1H), 1.51 - 1.47 (m, 1H), 1.43 - 1.39 (m, 1H), 0.94 - 0.88 (m, 2H), 0.69 - 0.64 (m, 2H).

(I-110): ESI-MS (M+H): 537.3, δ 11.14 (s, 1H), 8.64 (s, 1H), 8.34 (s, 1H), 7.72 (s, 1H), 7.46 (s, 1H), 7.42 (d, J=8.8 Hz, 1H), 7.34 (d, J=7.1 Hz, 1H), 7.34 - 7.29 (m, 2H), 7.19 (d, J=6.6 Hz, 1H), 7.00 (d, J=9.1 Hz, 1H), 4.78 (br s, 2H), 3.29 (s, 3H), 2.50 - 2.40 (m, 2H), 1.99 - 1.92 (m, 1H), 1.58 - 1.50 (m, 1H), 1.50 - 1.41 (m, 1H), 0.99 - 0.90 (m, 2H), 0.74 - 0.67 (m, 2H).

### Example 111: rac-ethyl 4-((1S*,2S*)-2-(3-chlorophenyl)cyclopropane-1-carboxamido)-6-(((6-cyclopropylimidazo[1,2-a]pyridin-2-yl)methyl)amino)pyrimidine-2-carboxylate(I-111)

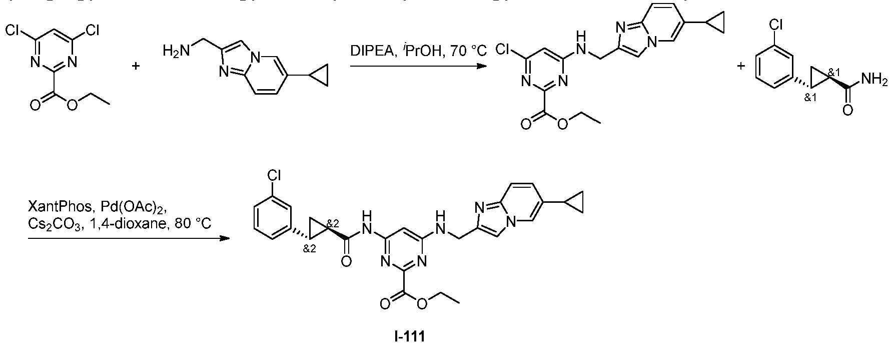

A solution of ethyl 4,6-dichloropyrimidine-2-carboxylate (200 mg, 0.91 mmol), intermediate 251 (140 mg, 0.75 mmol) and DIPEA (0.33 mL, 1.9 mmol) in iPrOH (10 mL) was heated to 70 °C for 1.5 h. The mixture was cooled to room temperature and concentrated *in vacuo.* The residue was dissolved in 5 % MeOH in DCM (60 mL) and washed with water (15 mL). The organics were passed through a hydrophobic frit and concentrated *in vacuo.* The residue was triturated with Et₂O (10 mL) to give **ethyl 4-chloro-6-(((6-cyclopropylimidazo[1,2-a]pyridin-2-yl)methyl)amino)pyrimidine-2-carboxylate intermediate 714** (190 mg, 68 %) as a beige solid. ESI-MS (M+H): 372.1, ¹H NMR (400 MHz, CDCl₃) δ 7.86 (s, 1H), 7.46 - 7.42 (m, 2H), 6.97 (dd, J=1.7, 9.3 Hz, 1H), 6.54 (s, 1H), 6.33 - 6.33 (m, 1H), 4.70 - 4.67 (m, 2H), 4.48 (q, J=7.2 Hz, 2H), 1.93 - 1.85 (m, 1H), 1.44 (dd, J=7.1, 7.1 Hz, 3H), 1.00 - 0.94 (m, 2H), 0.70 - 0.65 (m, 2H).

**Intermediate 1150** (70 mg, 0.19 mmol), **intermediate 714** (37 mg, 0.19 mmol), Pd(OAc)₂ (8.5 mg, 0.04 mmol), XantPhos (44 mg, 0.08 mmol), and Cs₂CO₃ (92 mg, 0.282 mmol) were placed under N₂ atmosphere, 1,4-dioxane (5.0 mL) was added, and the mixture was degassed for 5 min. The reaction mixture was stirred at 90 °C for 2 h. The reaction mixture was allowed to cool to room temperature, diluted with 5% MeOH in DCM (50 mL), filtered through Celite^{®}, and concentrated in vacuo. The residue was purified by column chromatography on silica gel, eluting with a gradient of 0-10 % EtOH in DCM. The residue was triturated with iPr₂O to give (I-111) (40 mg, 40 %). ESI-MS (M+H): 531.5, δ 11.00 (s, 1H), 8.31 (s, 1H), 8.22 (s, 1H), 7.66 (s, 1H), 7.42 - 7.26 (m, 5H), 7.15 (d, J=7.5 Hz, 1H), 6.98 (dd, J=1.7, 9.3 Hz, 1H), 4.64 (s, 2H), 4.30 (q, J=7.1 Hz, 2H), 2.47 - 2.39 (m, 2H), 1.97 - 1.89 (m, 1H), 1.49 - 1.37 (m, 2H), 1.30 (t, J=7.1 Hz, 3H), 0.95 - 0.89 (m, 2H), 0.70 - 0.65 (m, 2H).

### Example 112: rac-4-((1S*,2S*)-2-(3-chlorophenyl)cyclopropane-1-carboxamido)-6-(((6-cyclopropylimidazo[1,2-a]pyridin-2-yl)methyl)amino)pyrimidine-2-carboxylic acid (I-112)

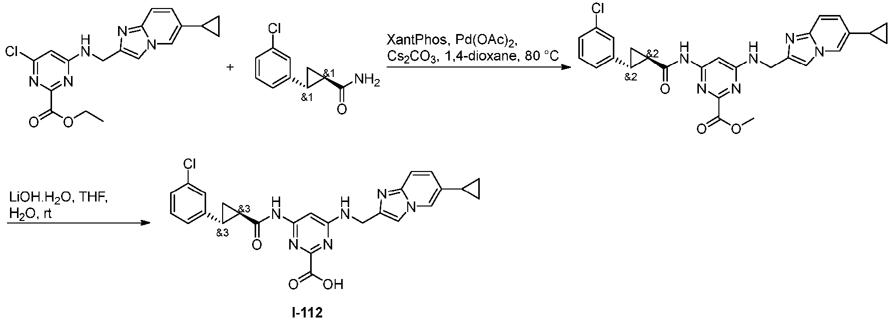

**Intermediate 1150** (70 mg, 0.19 mmol), **intermediate 714** (37 mg, 0.19 mmol), Pd(OAc)₂ (8.5 mg, 0.04 mmol), XantPhos (44 mg, 0.08 mmol), and Cs₂CO₃ (92 mg, 0.282 mmol) were placed under N₂ atmosphere, 1,4-dioxane (5.0 mL) was added, and the mixture was degassed for 5 min. The reaction mixture was stirred at 80 °C for 1.5 h. The reaction mixture was allowed to cool to room temperature, diluted with 10 % MeOH in DCM (50 mL), filtered through Celite^{®}, and concentrated in vacuo. Transesterification to the methyl ester was observed after workup. The residue was purified by column chromatography on silica gel, eluting with a gradient of 0 - 10 % MeOH in DCM to give **rac-methyl 4-((1S*,2S*)-2-(3-chlorophenyl)cyclopropane-1-carboxamido)-6-(((6-cyclopropylimidazo[1,2-a]pyridin-2-yl)methyl)amino)pyrimidine-2-carboxylate intermediate 715** (40 mg, 40 %) as a colourless oil. ESI-MS (M+H): 531.2 ¹H NMR (400 MHz, CDCl₃) δ 8.39 - 8.39 (m, 1H), 7.85 (s, 1H), 7.48 (s, 1H), 7.44 (d, J=9.3 Hz, 2H), 7.20 (dd, J=7.5, 7.5 Hz, 2H), 7.08 (s, 1H), 7.00 - 6.97 (m, 1H), 6.93 (dd, J=1.7, 9.3 Hz, 1H), 6.11 - 6.11 (m, 1H), 4.69 - 4.69 (m, 2H), 3.99 (s, 3H), 2.60 - 2.54 (m, 1H), 1.92 - 1.84 (m, 1H), 1.76 - 1.67 (m, 2H), 1.45 - 1.34 (m, 1H), 0.99 - 0.93 (m, 2H), 0.69 - 0.64 (m, 2H).

A solution of **intermediate 715**(40 mg, 0.08 mmol) and LiOH•H₂O (3.6 mg, 0.09 mmol) in THF (2.0 mL) and water (0.4 mL) was stirred at room temperature for 4 h. A solution of HCl (4 M in 1,4-dioxane, 20 µL) was added to the reaction mixture, which was stirred for 5 min, then concentrated in vacuo. The residue was purified by preparative HPLC to give **(I-112)** (12 mg, 31 %) as a formic acid salt. ESI-MS (M+H): 503.3, δ 10.96 (s, 1H), 8.32 (s, 1H), 8.11 (s, 1H), 7.66 (s, 1H), 7.39 (d, J=9.4 Hz, 1H), 7.35 - 7.30 (m, 2H), 7.28 - 7.26 (m, 2H), 7.15 (d, J=7.7 Hz, 1H), 6.98 (dd, J=1.8, 9.3 Hz, 1H), 4.65 - 4.64 (m, 2H), 2.44 - 2.38 (m, 2H), 1.96 - 1.89 (m, 1H), 1.52 - 1.38 (m, 2H), 0.95 - 0.89 (m, 2H), 0.70 - 0.66 (m, 2H)

### Intermediate 716: rac-(1S*,2S*)-N-(6-chloro-2-iodopyrimidin-4-yl)-2-(4-methylpyrimidin-2-yl)cyclopropane-1-carboxamide

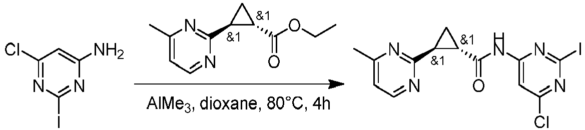

To a mixture of 6-chloro-2-iodopyrimidin-4-amine (3 g, 11.74 mmol) in dioxane (50 mL) was added AlMe3 (4.35 mL, 13.05 mmol) at 0 °C under N₂. After the reaction mixture was stirred at 30°C for 2 h, a solution of rac-ethyl (1S*,2S*)-2-(4-methylpyrimidin-2-yl)cyclopropane-1-carboxylate (900 mg, 4.36 mmol) in dioxane (5 mL) was added. The resulting mixture was stirred at 90 °C for 6 h, then quenched with water (50 mL) and extracted with EtOAc (3 x 50 mL). The combined organic layers were washed with brine (100 mL), dried over Na₂SO₄, and concentrated to give the crude, which was purified by column chromatography (eluent: PE/EtOAc = 5:1) to give **intermediate 716** as a white solid (750 mg, 41.3%). ESI-MS [M +H] +: 416

### Intermediate 717: rac-(1S*,2S*)-N-(6-chloro-2-(3-hydroxyoxetan-3-yl)pyrimidin-4-yl)-2-(4-methylpyrimidin-2-yl)cyclopropane-1-carboxamide

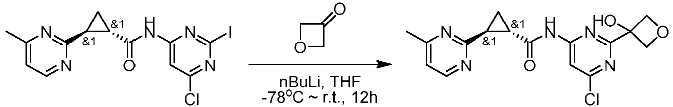

To a solution of **intermediate 716** (50 mg, 0.12 mmol) in THF (10 mL) was added nBuLi (0.23 mL, 0.58 mmol) at -78 °C for 1 h. Oxetan-3-one (173 mg, 2.40 mmol) was then added and the mixture was stirred for another 3 h. The reaction mixture was quenched with water (10 mL) and extracted with EtOAc (3 x 10 mL). The combined organic layers were washed with brine (20 mL), dried over anhydrous Na₂SO₄, and concentrated *in vacuo* to give the crude product, which was purified by preparative TLC (eluent: DCM/MeOH = 10/1) to give **intermediate 717** as a white solid (10 mg, 23%) ESI-MS [M +H] +: 362

### Examples 113-114

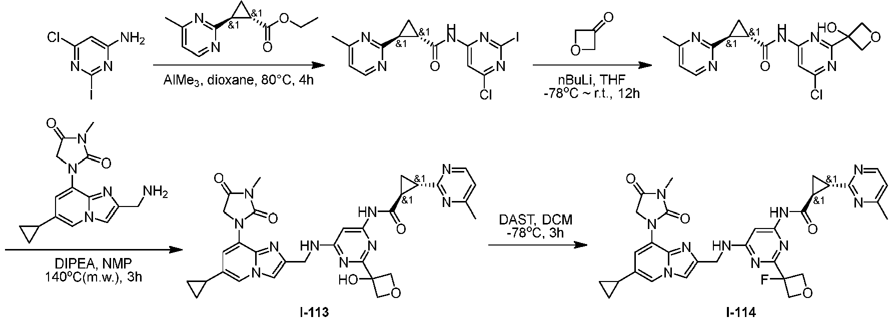

### rac-(1S*,2S*)-N-(6-(((6-cyclopropyl-8-(3-methyl-2,4-dioxoimidazolidin-1-yl)imidazo[1,2-a]pyridin-2-yl)methyl)amino)-2-(3-hydroxyoxetan-3-yl)pyrimidin-4-yl)-2-(4-methylpyrimidin-2-yl)cyclopropane-1-carboxamide (I-113)

A mixture of **intermediate 717** (20 mg, 0.055 mmol), **intermediate 365** (24 mg, 0.080 mmol), and DIPEA (34.9 mg, 0.27 mmol) in i-PrOH (5 mL) was stirred in a sealed tube. After degassing with N₂ for 1 min, the reaction was irradiated in microwave at 140 °C for 3 h. The reaction mixture was cooled to room temperature and concentrated *in vacuo* to give the crude, which was purified by preparative TLC (eluent: DCM/MeOH = 10/1) to give **(I-113)** as a white solid (20 mg, 58.2%). ESI-MS [M +H] +: 625.2, ¹H NMR (400 MHz, CD3OD) δ 8.45 (d, J = 5.2 Hz, 1H), 8.14 (s, 1H), 7.73 (s, 1H), 7.36 - 7.32 (m, 1H), 7.26 (s, 1H), 7.16 (d, J = 5.2 Hz, 1H), 5.00 (d, J = 6.2 Hz, 2H), 4.71 (d, J = 6.2 Hz, 3H), 4.66 - 4.58 (m, 3H), 3.08 (s, 3H), 2.71 - 2.67 (m, 1H), 2.53 - 2.49 (m, 1H), 2.46 (s, 3H), 1.95 - 1.92 (m, 1H), 1.68 - 1.63 (m, 2H), 0.99 - 0.94 (m, 2H), 0.74 - 0.71 (m, 2H).

### rac-(1S*,2S*)-N-(6-(((6-cyclopropyl-8-(3-methyl-2,4-dioxoimidazolidin-1-yl)imidazo[1,2-a] pyridin-2-yl)methyl)amino)-2-(3-fluorooxetan-3-yl)pyrimidin-4-yl)-2-(4-methylpyrimidin-2-yl) cyclopropane-1-carboxamide (I-114)

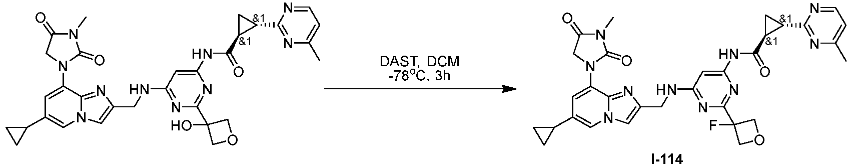

To a mixture of **(I-113)** (32 mg, 0.051 mmol) in DCM (10 mL) was added DAST (80 mg, 0.496 mmol) at -78 °C. After stirring at -78 °C for 3 h, the resulting mixture was quenched with water (10 mL) and extracted with EtOAc (3 x 10 mL). The combined organic layers were washed with brine (20 mL), dried over anhydrous Na₂SO₄, and concentrated to give the crude product, which was purified by preparative TLC (eluent: DCM/MeOH = 10/1) to give **(I-114)(17** mg, yield 53.2%) as a white solid. ESI-MS [M +H] +: 627.3, ¹H NMR (400 MHz, CD3OD) δ 8.45 (d, J = 5.2 Hz, 1H), 8.15 (s, 1H), 7.71 (s, 1H), 7.29 (d, J = 14.8 Hz, 2H), 7.15 (d, J = 5.2 Hz, 1H), 5.12 (dd, J = 7.7, 2.8 Hz, 1H), 5.07 (dd, J = 8.4, 2.0 Hz, 1H), 4.93 - 4.91 (m, 1H), 4.81 - 4.80 (m, 1H), 4.69 - 4.62 (m, 3H), 4.53 - 4.48 (m,, 1H), 3.08 (s, 3H), 2.71 - 2.67 (m, 1H), 2.53 - 2.49 (m, 1H), 2.46 (s, 3H), 1.68 - 1.62 (m, 2H), 0.98 - 0.94 (m, 2H), 0.74 - 0.70 (m, 2H).

### Intermediate 718: 2-chloro-N-((6-cyclopropylimidazo[1,2-a]pyridin-2-yl)methyl)pyridin-4-amine

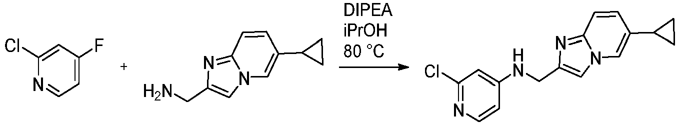

A mixture of intermediate 251 (0.30 g, 1.6 mmol), 2-chloro-4-fluoropyridine (0.14 mL, 1.6 mmol), and DIPEA (0.56 mL, 3.2 mmol) in iPrOH (16 mL) was stirred at 80 °C for 24 h. Water was added and the mixture was extracted with DCM (3 × 30 mL). The combined organic layers were dried (MgSO₄), filtered, and concentrated *in vacuo.* The residue was purified by column chromatography on silica gel, eluting with 0 - 6 % MeOH in DCM to give **intermediate 718** (0.046 g, 20 %) as a yellow oil. ¹H NMR (400 MHz, CDCl₃) δ 7.95 (d, J=5.8 Hz, 1H), 7.86 (s, 1H), 7.45 (d, J=9.3 Hz, 1H), 7.41 (s, 1H), 6.96 (dd, J=1.8, 9.3 Hz, 1H), 6.54 (d, J=2.0 Hz, 1H), 6.44 (dd, J=2.3, 5.8 Hz, 1H), 5.12 (dd, J=5.4, 5.4 Hz, 1H), 4.47 (d, J=5.3 Hz, 2H), 1.93 - 1.85 (m, 1H), 0.97 (ddd, J=4.9, 6.3, 8.4 Hz, 2H), 0.70 - 0.65 (m, 2H).

The compounds in Table **15** were synthesized using a similar procedure to that used for **intermediate 718** from 2-chloro-4-fluoropyridine and an appropriate coupling partner. **Table 15**

| **Compound** | **Coupling Partner / Analytical Data** |
|---|---|
| 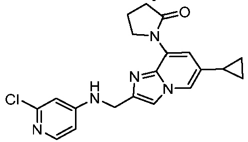 1-(2-(((2-chloropyridin-4-yl) amino) methyl)-6-cyclopropyl imidazo[1,2-*a*]pyridin-8-yl)pyrrolidin-2-one | **Intermediate 715** |
| | ESI-MS (M+H)+: 382.2, ¹H NMR (400 MHz, CDCl₃) δ 7.95 (d, J=5.8 Hz, 1H), 7.75 (s, 1H), 7.39 (s, 1H), 7.21 (d, J=1.6 Hz, 1H), 6.58 (d, J=2.1 Hz, 1H), 6.44 (dd, J=2.2, 5.8 Hz, 1H), 5.21 (dd, J=4.9, 4.9 Hz, 1H), 4.44 (d, J=5.1 Hz, 2H), 4.25 (dd, J=7.1, 7.1 Hz, 2H), 2.63 (dd, J=8.2, 8.2 Hz, 2H), 2.30 - 2.20 (m, 2H), 1.93 - 1.85 (m, 1H), 0.99 - 0.92 (m, 2H), 0.72 - 0.67 (m, 2H). |
| 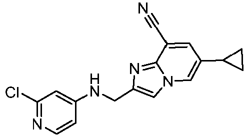 2-(((2-chloropyridin-4-yl)amino) methyl)-6-cyclopropyl imidazo [1,2-*a*]pyridine-8-carbonitrile | 2-(aminomethyl)-6-cyclopropylimidazo[1,2-*a*]pyridine-8-carbonitrile |
| | ESI-MS (M+H)+: 324 ¹H NMR (400 MHz, CDCl₃) δ 8.07 (s, 1H), 7.96 - 7.92 (m, 1H), 7.54 (s, 1H), 7.42 (d, J=1.5 Hz, 1H), 6.51 (d, J=2.0 Hz, 1H), 6.44 (dd, J=2.3, 5.8 Hz, 1H), 5.48 (dd, J=5.4, 5.4 Hz, 1H), 4.54 (d, J=5.3 Hz, 2H), 1.97 - 1.87 (m, 1H), 1.08 - 1.02 (m, 2H), 0.74 - 0.68 (m, 2H). |

### Examples 115-117

### rac-(1S*,2S*)-2-(3-chlorophenyl)-N-(4-(((6-cyclopropylimidazo[1,2-a]pyridin-2-yl)methyl)amino)pyridin-2-yl)cyclopropane-1-carboxamide (I-115)

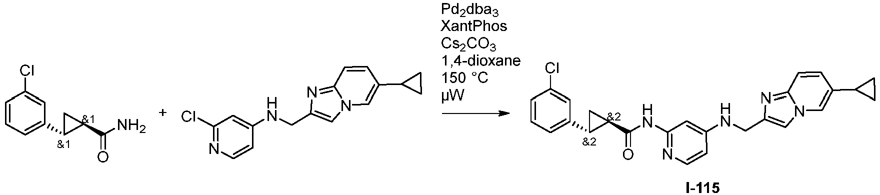

A mixture of **intermediate 718** (0.070 g, 0.23 mmol), **intermediate 1150** (0.062 g, 0.28 mmol), Pd₂(dba)₃ (0.032 g, 0.035 mmol), XantPhos (0.041 g, 0.070 mmol), and Cs₂CO₃ (0.15 g, 0.47 mmol) in 1,4-dioxane (2.5 mL) and DMF (0.5 mL) was degassed with nitrogen, then stirred at 150 °C in a microwave for 1 h. The mixture was diluted with a mixture of methanol in DCM (1:9) and filtered through Celite^{®}. The mixture was washed with water then concentrated *in vacuo.* The residue was purified by preparative HPLC to give **(I-115)** (0.010 g, 9 %, beige solid) as a mixture of enantiomers. ESI-MS (M+H)+: 458.3, δ 10.30 (s, 1H), 8.36 (s, 1H), 8.31 (s, 1H), 7.75 (d, J=5.8 Hz, 1H), 7.63 (s, 1H), 7.44 - 7.22 (m, 5H), 7.16 - 7.08 (m, 2H), 6.97 (dd, J=1.6, 9.2 Hz, 1H), 6.32 (dd, J=1.9, 5.8 Hz, 1H), 4.35 (d, J=5.8 Hz, 2H), 2.39 - 2.31 (m, 2H), 1.94 - 1.87 (m, 1H), 1.47 - 1.41 (m, 1H), 1.37 - 1.31 (m, 1H), 0.93 - 0.87 (m, 2H), 0.69-0.64 (m,2H).

The mixture was separated using SFC (YMC Cellulose-C 10x250 mm, 5 µm 55/45 MeOH (0.1 % DEA) / CO₂, 15 mL/min, 120 bar, 40°C) to give two enantiomers: (I-116) & (I-117) First eluting isomer, (1*R*,2*R*)-2-(3-chlorophenyl)-*N*-(4-(((6-cyclopropylimidazo[1,2-*a*]pyridin-2-yl)methyl)amino)pyridin-2-yl)cyclopropane-1-carboxamide **(I-116):**
ESI-MS (M+H)+: 458.2, δ 10.36 (s, 1H), 8.36 (s, 1H), 7.81 (d, J=5.8 Hz, 1H), 7.68 (s, 1H), 7.51 - 7.28 (m, 6H), 7.22 - 7.12 (m, 2H), 7.05 - 7.00 (m, 1H), 6.38 (dd, J=2.3, 5.8 Hz, 1H), 4.41 (d, J=5.8 Hz, 2H), 2.46 - 2.37 (m, 2H), 2.01 - 1.92 (m, 1H), 1.53 - 1.46 (m, 1H), 1.43 - 1.36 (m, 1H), 1.00 - 0.92 (m, 2H), 0.74 - 0.68 (m, 2H), Enantiomer RT = 3.00 min, 99.9 % e.e. Formic acid salt. Second eluting isomer, (1*S*,2*S*)-2-(3-chlorophenyl)-*N*-(4-(((6-cyclopropylimidazo[1,2-*a*]pyridin-2-yl)methyl)amino)pyridin-2-yl)cyclopropane-1-carboxamide **(I-117):**
ESI-MS (M+H)+: 458.2, δ 10.35 (s, 1H), 8.35 (s, 1H), 7.80 (d, J=5.6 Hz, 1H), 7.68 (s, 1H), 7.48 (s, 1H), 7.43 (d, J=9.3 Hz, 1H), 7.36 (dd, J=7.6, 7.6 Hz, 1H), 7.31 - 7.26 (m, 2H), 7.22 - 7.12 (m, 2H), 7.04 - 6.99 (m, 1H), 6.39 - 6.35 (m, 1H), 4.42 (d, J=4.5 Hz, 2H), 2.44 - 2.40 (m, 2H), 2.01 - 1.92 (m, 1H), 1.55 - 1.46 (m, 1H), 1.41 - 1.34 (m, 1H), 0.99 - 0.91 (m, 2H), 0.75 - 0.70 (m, 2H). Enantiomer RT = 3.91 min, 99.9 % e.e.
Using a similar procedure to that used for **(I-115),** the compounds in Table 16 were prepared from **intermediate 1150 and** the indicated coupling partner, then purified by preparative LCMS. **Table 16**

| **EG** | **Compound** | **Coupling Partner / Analytical Data** |
|---|---|---|
| **118** | 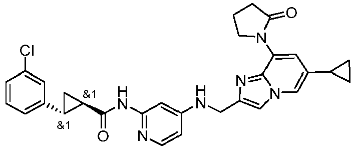 *rac-*(1*S**,2*S**)-2-(3-chlorophenyl)-*N*-(4-*((*(6-cyclopropyl-8-(2-oxopyrrolidin-1-yl)imidazo[1,2-a]pyridin-2-yl)methyl)amino)pyridin-2-yl)cyclopropane-1-carboxamide **(I-118)** | 1-(2-(((2-chloropyridin-4-yl)amino)methyl)-6-cyclopropylimidazo[1,2-a]pyridin-8-yl)pyrrolidin-2-one ESI-MS (M+H)+: 541.3, δ 10.36 (s, 1H), 8.30 (d, J=1.0 Hz, 1H), 7.81 (d, J=5.8 Hz, 1H), 7.72 (s, 1H), 7.50 (s, 1H), 7.36 (dd, J=8.0, 8.0 Hz, 1H), 7.30 (d, J=7.3 Hz, 2H), 7.21 - 7.17 (m, 3H), 6.37 (dd, J=1.8, 5.8 Hz, 1H), 4.42 (d, J=5.6 Hz, 2H), 4.21 (dd, J=7.1, 7.1 Hz, 2H), 2.45 - 2.37 (m, 2H), 2.22 - 2.13 (m, 2H), 2.01 - 1.93 (m, 1H), 1.53 - 1.47 (m, 1H), 1.42 - 1.37 (m, 1H), 1.00 - 0.94 (m, 2H), 0.72 - 0.67 (m, 2H). |
| **119** | 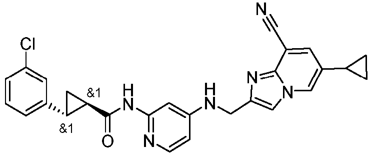 **rac-(1S*,2S*)-2-(3-chlorophenyl)-N-(4-(((8-cyano-6-cyclopropylimidazo [1,2-a]pyridin-2-yl)methyl) amino) pyridin-2-yl)cyclopropane-1-carboxamide (I-119)** from 2-(((2-chloropyridin-4-yl)amino)methyl)-6-cyclopropylimidazo [1,2-a]pyridine-8-carbonitrile | ESI-MS (M+H)+: 483.2, δ 10.37 (s, 1H), 8.72 (d, J=1.4 Hz, 1H), 7.86 (s, 1H), 7.82 (d, J=5.9 Hz, 1H), 7.80 (d, J=1.6 Hz, 1H), 7.49 (s, 1H), 7.37 - 7.26 (m, 4H), 7.19 (td, J=1.3, 7.6 Hz, 1H), 6.38 (dd, J=2.2, 5.8 Hz, 1H), 4.48 (d, J=6.0 Hz, 2H), 2.46 - 2.38 (m, 2H), 2.02 (tt, J=4.0, 9.2 Hz, 1H), 1.50 (ddd, J=4.0, 5.3, 9.1 Hz, 1H), 1.40 (ddd, J=4.0, 6.3, 8.0 Hz, 1H), 1.00 (ddd, J=4.4, 6.5, 8.2 Hz, 2H), 0.80 (td, J=4.6, 6.2 Hz, 2H). |

### Example 120: rac-(1S*,2S*)-N-(4-(((8-(aminomethyl)-6-cyclopropylimidazo[1,2-a]pyridin-2-yl)methyl)amino)pyridin-2-yl)-2-(3-chlorophenyl)cyclopropane-1-carboxamide (I-120)

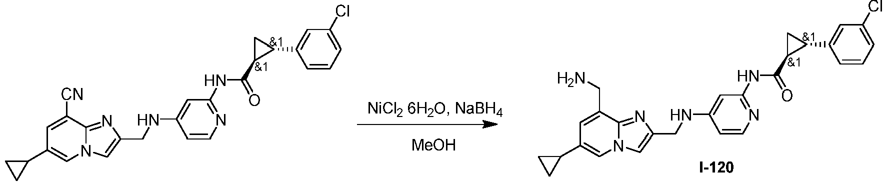

To a mixture of **(I-119)** (70 mg, 0.145 mmol), in MeOH (5.0 mL) was added NiCl₂6H₂O (34.6 mg, 0.145 mmol) at 0 °C, and then NaBH₄ (15.7 mg, 0.435 mmol) was added. After stirring at 0 °C under N₂ for 1 h, the mixture was quenched with water (1.0 mL), concentrated, and purified by preparative HPLC to give **(I-120)** (6.1 mg, 8.7%) as a white solid. ESI-MS [M +H]+: 487.2. δ 10.33 (s, 1H), 8.72 - 7.97 (m, 4H), 7.88 - 7.58 (m, 2H), 7.45 (s, 1H), 7.37 - 6.95 (m, 6H), 6.32 (s, 1H), 4.38 (s, 2H), 4.14 (s, 2H), 2.39 - 2.33 (m,, 2H), 1.93 - 1.87 (m, 1H), 1.45 - 1.41 (m, 1H), 1.36 - 1.31 (m, 1H), 0.93 - 0.88 (m,, 2H), 0.80 - 0.55 (m, 2H).

### Intermediate 719: 2-(2-(((2-bromopyridin-4-yl)amino)methyl)-6-cyclopropylimidazo[1,2-a]pyridin-8-yl)isothiazolidine 1,1-dioxide

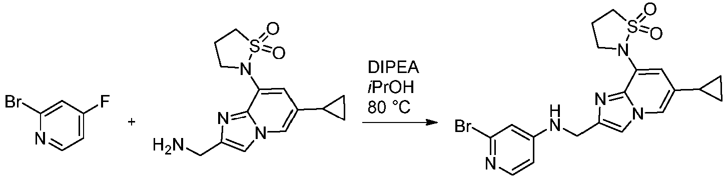

A mixture of 2-(2-(aminomethyl)-6-cyclopropylimidazo[1,2-a]pyridin-8-yl)isothiazolidine 1,1-dioxide (0.18 g, 0.59 mmol), 2-bromo-4-fluoropyridine (0.098 mL, 0.56 mmol) and DIPEA (0.36 mL, 2.1 mmol) in iPrOH (5.0 mL) was stirred at 80 °C for 18 h. The mixture was concentrated *in vacuo.* The residue was purified by column chromatography on silica gel, eluting with 0-100 % EtOAc in cyclohexane to give **intermediate 719.** ¹H NMR (400 MHz, CDCl₃) δ , 7.76 - 7.74 (m, 1H), 7.41 - 7.39 (m, 1H), 7.15 (d, J=1.5 Hz, 1H), 6.99 - 6.95 (m, 1H), 6.78 (d, J=2.1 Hz, 1H), 6.48 - 6.45 (m, 1H), 4.45 (dd, J=5.3, 8.3 Hz, 2H), 4.31 (t, J=6.8 Hz, 2H), 3.41 (t, J=7.5 Hz, 2H), 2.66 - 2.59 (m, 2H), 1.91 - 1.84 (m, 1H), 1.00 - 0.93 (m, 2H), 0.71 - 0.67 (m, 2H).

The compounds in Table 17 were synthesized using a similar procedure to that used for **intermediate 719** from 2-bromo-4-fluoropyridine and an appropriate coupling partner. **Table 17**

| **Compound** | **Coupling Partner /Analytical Data** |
|---|---|
| 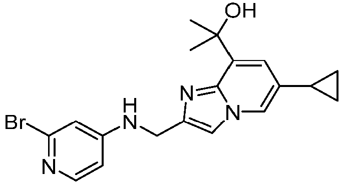 2-(2-(((2-bromopyridin-4-yl)amino)methyl)-6-cyclopropylimidazo[1,2-*a*]pyridin-8-yl)propan-2-ol **intermediate 720** | **intermediate 574¹H** NMR (400 MHz, CDCl₃) δ 7.94 (d, J=5.8 Hz, 1H), 7.74 (s, 1H), 7.36 (s, 1H), 6.85 (d, J=1.5 Hz, 1H), 6.72 (d, J=2.1 Hz, 1H), 6.48 (dd, J=2.3, 5.8 Hz, 1H), 6.31 (s, 1H), 4.95 (dd, J=4.6, 4.6 Hz, 1H), 4.46 (d, J=5.1 Hz, 2H), 1.92 - 1.85 (m, 1H), 1.70 (s, 6H), 0.99 - 0.93 (m, 2H), 0.69 - 0.63 (m, 2H). |
| 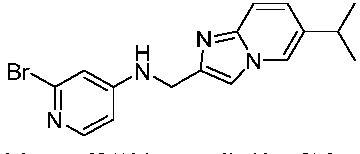 2-bromo-*N*-((6-isopropylimidazo[1,2-*a*]pyridin-2-yl)methyl)pyridin-4-amine **intermediate 721** | **Intermediate 256** ESI-MS (M+H)+: 347.0, 345.0 δ 8.35 (d, J=0.4 Hz, 1H), 7.78 (d, J=5.8 Hz, 1H), 7.74 (s, 1H), 7.46 - 7.42 (m, 2H), 7.21 (dd, J=1.8, 9.3 Hz, 1H), 6.74 (s, 1H), 6.62 (dd, J=2.1, 5.8 Hz, 1H), 4.40 (d, J=5.8 Hz, 2H), 2.94 - 2.86 (m, 1H), 1.24 (d, J=6.9 Hz, 6H). |
| 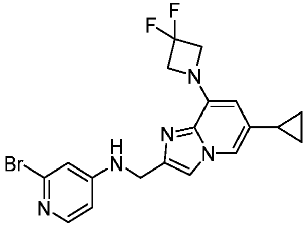 2-bromo-*N*-((6-cyclopropyl-8-(3,3-difluoro azetidin-1-yl)imidazo[1,2-*a*]pyridin-2-yl) methyl)pyridin-4-amine **intermediate 722** | (6-cyclopropyl-8-(3,3-difluoroazetidin-1-yl)imidazo[1,2-a]pyridin-2-yl)methanamine ESI-MS (M+H)+: 434.1, δ 7.88 (s, 1H), 7.83 (d, J=5.8 Hz, 1H), 7.67 (s, 1H), 7.42 - 7.37 (m, 1H), 6.87 (d, J=2.0 Hz, 1H), 6.66 (dd, J=2.0, 5.8 Hz, 1H), 5.93 (s, 1H), 4.58 (dd, J=12.4, 12.4 Hz, 4H), 4.40 (dd, J=5.8, 5.8 Hz, 2H), 1.93 - 1.85 (m, 1H), 0.96 - 0.89 (m, 2H), 0.74 - 0.68 (m, 2H). |
| 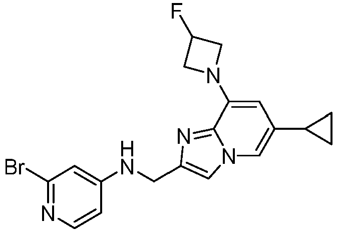 2-bromo-*N*-((6-cyclopropyl-8-(3-fluoro azetidin-1-yl)imidazo[1,2-*a*]pyridin-2-yl)methyl)pyridin-4-amine | **Intermediate 723 in table A7** |
| | ESI-MS (M+H)+: 416.1, δ 7.79 (d, J=5.7 Hz, 1H), 7.77 - 7.75 (m, 1H), 7.60 (s, 1H), 7.40 - 7.33 (m, 1H), 6.82 (d, J=2.0 Hz, 1H), 6.62 (dd, J=2.1, 5.8 Hz, 1H), 5.75 (d, J=1.5 Hz, 1H), 5.59 - 5.40 (m, 1H), 4.46 (ddd, J=5.0, 10.4, 20.8 Hz, 2H), 4.35 (d, J=5.6 Hz, 2H), 4.21 - 4.10 (m, 2H), 1.88 - 1.80 (m, 1H), 0.89 - 0.84 (m, 2H), 0.68 - 0.63 (m, 2H). |
| 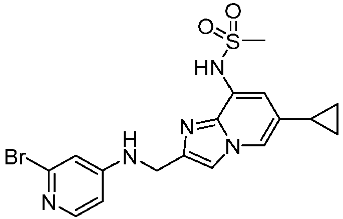 | *N*-(2-(((2-bromopyridin-4-yl)amino)methyl)-6-cyclopropylimidazo[1,2-a]pyridin-8-yl)methanesulfonamide from **Intermediate 278** ESI-MS (M+H)+: 438.3 |
| 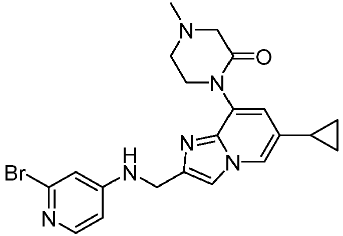 1-(2-(((2-bromopyridin-4-yl)amino)methyl)-6-cyclopropylimidazo[1,2-*a*]pyridin-8-yl)-4-methylpiperazin-2-one **intermediate 724** | **Intermediate 330** |
| | ¹H NMR (400 MHz, CDCl₃) δ 7.92 (d, J=5.8 Hz, 1H), 7.83 (s, 1H), 7.42 (s, 1H), 7.00 - 6.97 (m, 1H), 6.72 (d, J=2.1 Hz, 1H), 6.47 (dd, J=2.3, 5.8 Hz, 1H), 4.45 (d, J=5.1 Hz, 2H), 3.92 (dd, J=5.5, 5.5 Hz, 2H), 3.37 (s, 2H), 2.91 (dd, J=5.5, 5.5 Hz, 2H), 2.45 (s, 3H), 1.91 - 1.85 (m, 1H), 0.99 - 0.94 (m, 2H), 0.70 - 0.66 (m, 2H). |
| 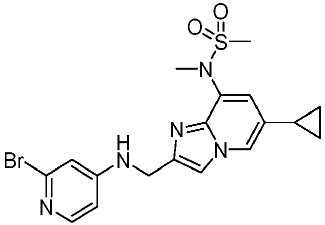 *N*-(2-(((2-bromopyridin-4-yl)amino)methyl)-6-cyclopropylimidazo[1,2-*a*]pyridin-8-yl)-N-methylmethanesulfonamide | **Intermediate 347** |
| | ESI-MS (M+H)+: 452.0, ¹H NMR (400 MHz, CDCl₃) δ 7.92 (d, J=5.6 Hz, 1H), 7.84 (s, 1H), 7.43 (s, 1H), 7.08 (d, J=1.5 Hz, 1H), 6.76 (d, J=2.3 Hz, 1H), 6.46 (dd, J=2.3, 5.8 Hz, 1H), 5.06 (dd, J=5.2, 5.2 Hz, 1H), 4.46 (d, J=5.6 Hz, 2H), 3.51 (s, 3H), 3.13 (s, 3H), 1.93 - 1.83 (m, 2H), 1.01 - 0.94 (m, 2H), 0.71 - 0.65 (m, 2H). |
| 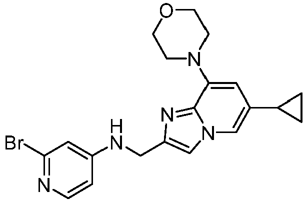 2-bromo-N-((6-cyclopropyl-8-morpholinoimidazo[1,2-*a*]pyridin-2-yl)methyl)pyridin-4-amine | (6-cyclopropyl-8-morpholinoimidazo[1,2-a]pyridin-2-yl)methanamine Table A5 |
| | ESI-MS (M+H)+: 430.1, ¹H NMR (400 MHz, CDCl₃) δ 7.91 (d, J=5.6 Hz, 1H), 7.52 (s, 1H), 7.32 (s, 1H), 6.73 (d, J=2.0 Hz, 1H), 6.46 (dd, J=2.0, 5.8 Hz, 1H), 6.24 (d, J=1.3 Hz, 1H), 5.17 (dd, J=5.1, 5.1 Hz, 1H), 4.43 (d, J=5.3 Hz, 2H), 3.99 - 3.95 (m, 4H), 3.49 (dd, J=4.7, 4.7 Hz, 4H), 1.89 - 1.81 (m, 1H), 0.96 - 0.87 (m, 2H), 0.68 - 0.63 (m, 2H). |
| 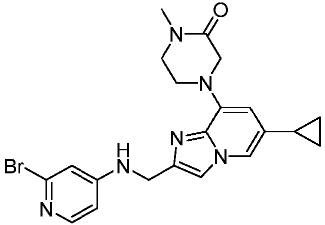 | **4-(2-(((2-bromopyridin-4-yl)amino)methyl)-6-cyclopropylimidazo[1,2-a]pyridin-8-yl)-1-methylpiperazin-2-one intermediate 275** from 4-(2-(aminomethyl)-6-cyclopropylimidazo[1,2-a]pyridin-8-yl)-1-methylpiperazin-2-one |
| | ESI-MS (M+H)+: 455.3, 457.3 |

### Example 121: (1S,2S)-2-(3-chlorophenyl)-N-(4-(((6-cyclopropylimidazo[1,2-a]pyridin-2-yl) methyl)amino)pyridin-2-yl)cyclopropane-1-carboxamide (I-121)

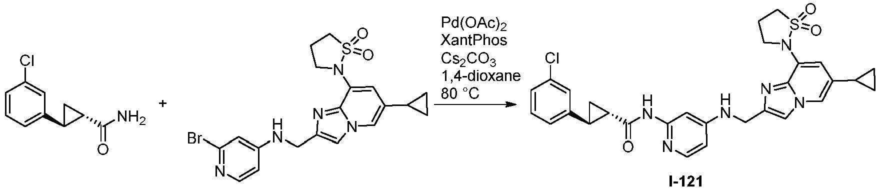

A mixture of **intermediate 719** (0.090 g, 0.20 mmol), intermediate 119 (0.042 g, 0.21 mmol), Pd(OAc)₂ (0.009 g, 0.039 mmol), XantPhos (0.045 g, 0.078 mmol), and Cs₂CO₃ (0.13 g, 0.39 mmol) in 1,4-dioxane (5.0 mL) was degassed with nitrogen then stirred at 80 °C for 18 h. The mixture was filtered through Celite^{®} and concentrated *in vacuo.* The residue was purified by preparative HPLC to give **(I-121)** (0.016 g, 14 %). ESI-MS (M+H)+: 577, δ 10.38 - 10.38 (m, 1H), 8.26 (s, 1H), 7.78 (d, J=5.9 Hz, 1H), 7.69 (s, 1H), 7.46 (s, 1H), 7.32 (dd, J=7.7, 7.7 Hz, 1H), 7.26 (d, J=7.4 Hz, 2H), 7.15 (d, J=7.9 Hz, 2H), 6.97 (s, 1H), 6.34 (d, J=3.9 Hz, 1H), 4.39 (d, J=5.3 Hz, 2H), 4.25 (dd, J=6.7, 6.7 Hz, 2H), 3.53 - 3.47 (m, 2H), 2.49 - 2.44 (m, 2H), 2.37 (d, J=6.5 Hz, 2H), 1.97 - 1.89 (m, 1H), 1.47 - 1.43 (m, 1H), 1.40 - 1.35 (m, 1H), 0.94 (q, J=6.1 Hz, 2H), 0.65 - 0.61 (m, 2H).

Using a similar procedure to that used for **(I-117),** the compounds in Table **18** were prepared from **intermediate 119** and an appropriate coupling partner, then purified by preparative LCMS. **Table 18**

| **EG** | **Compound** | **Coupling Partner / Analvtical Data** |
|---|---|---|
| **122** | 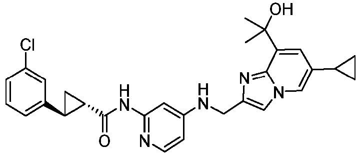 (1*S*,2*S*)-2-(3-chlorophenyl)-*N*-(4-(((6-cyclopropyl-8-(2-hydroxypropan-2-yl)imidazo[1,2-*a*]pyridin-2-yl)methyl) amino)pyridin-2-yl)cyclopropane-1-carboxamide **(I-122)** | **Intermediate 720** |
| | | ESI-MS (M+H)+: 516.4, δ 10.35 (s, 1H), 8.19 (d, J=1.4 Hz, 1H), 7.77 (d, J=5.8 Hz, 1H), 7.61 (s, 1H), 7.47 - 7.46 (m, 1H), 7.33 (dd, J=7.7, 7.7 Hz, 1H), 7.26 (d, J=8.9 Hz, 2H), 7.15 (d, J=7.7 Hz, 2H), 7.07 (d, J=1.6 Hz, 1H), 6.35 (dd, J=2.0, 5.8 Hz, 1H), 5.52 (s, 1H), 4.38 (d, J=5.8 Hz, 2H), 2.41 - 2.34 (m, 2H), 1.96 - 1.88 (m, 1H), 1.67 (s, 6H), 1.48 - 1.33 (m, 2H), 0.95 - 0.89 (m, 2H), 0.69 - 0.64 (m, 2H). |
| **123** | 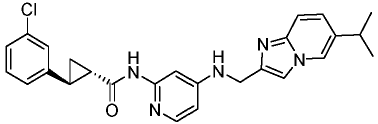 (1*S*,2*S*)-2-(3-chlorophenyl)-*N*-(4-(((6-isopropylimidazo[1,2-*a*]pyridin-2-yl)methyl)amino)pyridin-2-yl)cyclopropane-1-carboxamide **(I-123)** | **Intermediate 721** |
| | | ESI-MS (M+H)+: 460.4, ¹ δ 10.31 (s, 1H), 8.32 - 8.31 (m, 1H), 7.75 (d, J=5.8 Hz, 1H), 7.66 (s, 1H), 7.44 - 7.40 (m, 2H), 7.33 - 7.28 (m, 1H), 7.26 - 7.23 (m, 2H), 7.20 - 7.12 (m, 3H), 6.32 (dd, J=2.1, 5.8 Hz, 1H), 4.36 (d, J=5.8 Hz, 2H), 2.93 - 2.82 (m, 1H), 2.40 - 2.31 (m, 2H), 1.47 - 1.41 (m, 1H), 1.37 - 1.31 (m, 1H), 1.22 (d, J=6.9 Hz, 6H). |
| **124** | 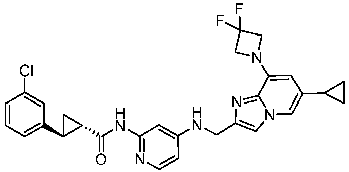 (1*S*,2*S*)-2-(3-chlorophenyl)-*N*-(4-(((6-cyclopropyl-8-(3,3-difluoroazetidin-1-yl)imidazo[1,2-*a*]pyridin-2-yl)methyl) amino)pyridin-2-yl)cyclopropane-1-carboxamide **(I-124)** | **Intermediate 722** |
| | | ESI-MS (M+H)+: 549.3, δ 10.34 (br s, 1H), 7.82 - 7.80 (m, 1H), 7.75 (d, J=5.9 Hz, 1H), 7.57 (s, 1H), 7.44 - 7.23 (m, 4H), 7.16 - 7.09 (m, 2H), 6.34 - 6.32 (m, 1H), 5.86 (d, J=1.0 Hz, 1H), 4.53 (t, J=12.3 Hz, 4H), 4.32 (d, J=5.6 Hz, 2H), 2.39 - 2.31 (m, 2H), 1.87 - 1.79 (m, 1H), 1.48 - 1.41 (m, 1H), 1.38 - 1.31 (m, 1H), 0.89 - 0.83 (m, 2H), 0.69 - 0.63 (m, 2H). |
| **125** | 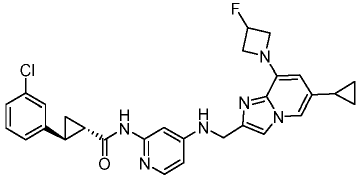 (1*S*,2*S*)-2-(3-chlorophenyl)-*N*-(4-(((6-cyclopropyl-8-(3-fluoroazetidin-1-yl) imidazo[1,2-*a*]pyridin-2-yl)methyl) amino)pyridin-2-yl)cyclopropane-1-carboxamide **(I-125)** | 2-bromo-N-((6-cyclopropyl-8-(3-fluoro azetidin-1-yl)imidazo[1,2-a]pyridin-2-yl) methyl)pyridin-4-amine |
| | | ESI-MS (M+H)+: 531.3, ¹ δ 10.30 (s, 1H), 7.76 - 7.73 (m, 2H), 7.52 (s, 1H), 7.45 (br s, 1H), 7.31 - 7.23 (m, 3H), 7.15 - 7.12 (m, 1H), 7.05 (t, J=5.8 Hz, 1H), 6.32 (dd, J=2.1, 5.8 Hz, 1H), 5.73 (d, J=1.2 Hz, 1H), 5.57 - 5.37 (m, 1H), 4.51 - 4.40 (m, 2H), 4.33 - 4.29 (m, 2H), 4.19 - 4.08 (m, 2H), 2.40 - 2.32 (m, 2H), 1.85 - 1.77 (m, 1H), 1.47 - 1.41 (m, 1H), 1.37 - 1.31 (m, 1H), 0.87 - 0.81 (m, 2H), 0.66 - 0.61 (m, 2H). |
| **126** | 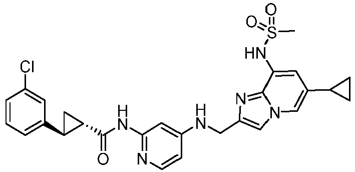 (1S,2S)-2-(3-chlorophenyl)-N-(4-(((6-cyclopropyl-8-(methylsulfonamido) imidazo[1,2-*a*]pyridin-2-yl)methyl) amino)pyridin-2-yl)cyclopropane-1-carboxamide **(I-126)** | N-(2-(((2-bromopyridin-4-yl)amino) methyl)-6-cyclopropylimidazo[1,2-a]pyridin-8-yl)methanesulfonamide |
| | | ESI-MS (M+H)+: 551, δ 10.37 (s, 1H), 8.42 (s, 1H), 8.06 (s, 1H), 7.81 (d, J=5.8 Hz, 1H), 7.67 (s, 1H), 7.50 (s, 1H), 7.37 - 7.28 (m, 3H), 7.21 - 7.11 (m, 2H), 6.85 (s, 1H), 6.37 (dd, J=2.0, 5.8 Hz, 1H), 4.42 (d, J=7.1 Hz, 2H), 3.16 (s, 3H), 2.46 - 2.37 (m, 2H), 1.97 - 1.90 (m, 1H), 1.53 - 1.47 (m, 1H), 1.43 - 1.36 (m, 1H), 0.98 - 0.92 (m, 2H), 0.70 - 0.65 (m, 2H). |
| **127** | 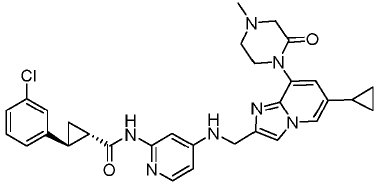 (1*S*,2*S*)-2-(3-chlorophenyl)-*N*-(4-(((6-cyclopropyl-8-(4-methyl-2-oxo piperazin-1-yl)imidazo[1,2-*a*]pyridin-2-yl)methyl)amino)pyridin-2-yl) cyclopropane-1-carboxamide **(I-127)** | **Intermediate 724** |
| | | ESI-MS (M+H)+: 570.4, δ 10.18 (s, 1H), 8.18 (d, J=1.0 Hz, 1H), 7.63 (d, J=5.8 Hz, 1H), 7.54 (s, 1H), 7.31 (s, 1H), 7.21 - 7.16 (m, 1H), 7.13 - 7.11 (m, 2H), 7.04 - 6.99 (m, 2H), 6.82 (d, J=1.8 Hz, 1H), 6.18 (dd, J=2.0, 5.8 Hz, 1H), 4.23 (d, J=5.8 Hz, 2H), 3.64 (dd, J=5.6, 5.6 Hz, 2H), 3.02 (s, 2H), 2.64 (dd, J=5.4, 5.4 Hz, 2H), 2.27 - 2.21 (m, 2H), 2.20 (s, 3H), 1.83 - 1.75 (m, 1H), 1.34 - 1.19 (m, 2H), 0.82 - 0.76 (m, 2H), 0.57 - 0.52 (m, 2H). |
| **128** | 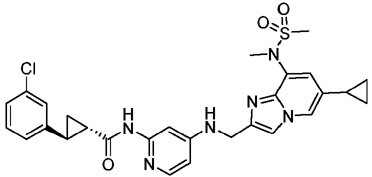 (1*S*,2*S*)-2-(3-chlorophenyl)-*N*-(4-(((6-cyclopropyl-8-(*N*-methylmethyl sulfonamido)imidazo[1,2-*a*]pyridin-2-yl)methyl)amino)pyridin-2-yl)cyclo propane-1-carboxamide, formic acid salt **(I-128)** | *N*-(2-(((2-bromopyridin-4-yl)amino) methyl)-6-cyclopropylimidazo[1,2-a]pyridin-8-yl)-*N-*methylmethanesulfonamide |
| | | ESI-MS (M+H)+: 565, δ 10.23 (s, 1H), 8.27 - 8.25 (m, 1H), 7.70 - 7.63 (m, 2H), 7.41 - 7.38 (m, 1H), 7.27 - 7.15 (m, 3H), 7.09 - 7.03 (m, 2H), 6.95 (d, J=1.5 Hz, 1H), 6.26 (dd, J=2.0, 5.8 Hz, 1H), 4.31 (d, J=5.8 Hz, 2H), 3.31 (s, 3H), 3.16 - 3.14 (m, 3H), 2.32 - 2.25 (m, 2H), 1.90 - 1.82 (m, 1H), 1.40 - 1.24 (m, 2H), 0.89 - 0.82 (m, 2H), 0.63 - 0.58 (m, 2H). |
| **129** | 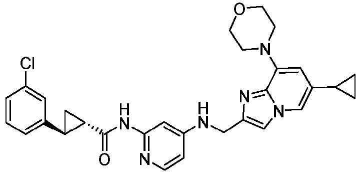 (1S,2S)-2-(3-chlorophenyl)-N-(4-(((6-cyclopropyl-8-morpholinoimidazo [1,2-*a*]pyridin-2-yl)methyl)amino) pyridin-2-yl)cyclopropane-1-carboxamide **(I-129)** | 2-bromo-N-((6-cyclopropyl-8-morpho linoimidazo[1,2-a]pyridin-2-yl)methyl) pyridin-4-amine ESI-MS (M+H)+: 543.4, |
| | | δ 10.23 (s, 1H), 7.82 (s, 1H), 7.69 (d, J=5.8 Hz, 1H), 7.49 (s, 1H), 7.37 (s, 1H), 7.25 - 7.16 (m, 3H), 7.09 - 7.00 (m, 2H), 6.25 (dd, J=2.3, 5.8 Hz, 1H), 6.11 - 6.10 (m, 1H), 4.29 - 4.25 (m, 2H), 3.75 - 3.70 (m, 4H), 3.44 - 3.38 (m, 4H), 2.34 - 2.26 (m, 2H), 1.83 - 1.75 (m, 1H), 1.41 - 1.24 (m,2H), 0.83-0.77 (m,2H), 0.62-0.57 (m,2H). |
| **130** | 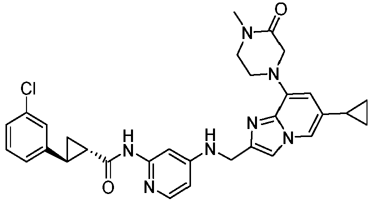 (1*S*,2*S*)-2-(3-chlorophenyl)-*N*-(4-(((6-cyclopropyl-8-(4-methyl-3-oxo piperazin-1-yl)imidazo[1,2-*a*]pyridin-2-yl)methyl)amino)pyridin-2-yl)cyclopropane-1-carboxamide **(I-130)** | **Intermediate 725** |
| | | ESI-MS (M+H)+: 570.4, δ 10.30 (s, 1H), 7.91 - 7.90 (m, 1H), 7.75 (d, J=5.8 Hz, 1H), 7.57 (s, 1H), 7.48 - 7.42 (m, 1H), 7.32 - 7.28 (m, 1H), 7.26 - 7.23 (m, 2H), 7.16 - 7.07 (m, 2H), 6.32 (dd, J=2.0, 5.8 Hz, 1H), 6.19 (d, J=1.3 Hz, 1H), 4.35 (d, J=5.8 Hz, 2H), 4.07 (s, 2H), 3.92 - 3.86 (m, 2H), 3.46 (dd, J=5.5, 5.5 Hz, 2H), 2.91 (s, 3H), 2.40 - 2.32 (m, 2H), 1.90 - 1.82 (m, 1H), 1.47 - 1.41 (m, 1H), 1.37 - 1.31 (m, 1H), 0.90 - 0.83 (m, 2H), 0.71 - 0.65 (m, 2H). |

Using a similar procedure to that used **for(I-117),** the compound in Table 19 was prepared using **intermediate 118** and an appropriate coupling partner, then purified by preparative LCMS.

**Table 19**

| **EG** | **Compound** | **Coupling Partner /Analvtical Data** |
|---|---|---|
| **131** | 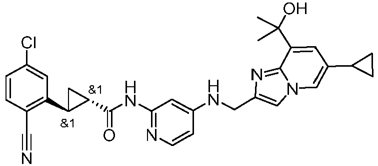 ***rac*-(1*S**,2*S**)-2-(5-chloro-2-cyano phenyl)-N-(4-(((6-cyclopropyl-8-(2-hydroxypropan-2-yl)imidazo[1,2-*a*]pyridin-2-yl)methyl) amino)pyridin-2-yl)cyclopropane-1-carboxamide (I-131)** from 2-(2-(((2-bromopyridin-4-yl)amino)methyl)-6-cyclo propylimidazo[1,2-*a*]pyridin-8-yl)propan-2-ol | ESI-MS (M+H)+: 541.3, δ 10.44 (s, 1H), 8.26 (d, J=1.3 Hz, 1H), 7.95 (d, J=8.3 Hz, 1H), 7.86 (d, J=5.8 Hz, 1H), 7.69 (s, 1H), 7.62 - 7.55 (m, 2H), 7.47 (d, J=2.0 Hz, 1H), 7.21(dd, J=5.8, 5.8 Hz, 1H), 7.14 (d, J=1.8 Hz, 1H), 6.43 (dd, J=2.0, 5.8 Hz, 1H), 5.59 (s, 1H), 4.46 (d, J=5.6 Hz, 2H), 2.69 - 2.63 (m, 1H), 2.03 - 1.95 (m, 1H), 1.74 (s, 6H), 1.65 - 1.60 (m, 2H), 0.99 (ddd, J=4.3, 6.3, 8.3 Hz, 2H), 0.76 - 0.71 (m, 2H). |

### Intermediate 726: tert-butyl 4-(2-(((2-bromopyridin-4-yl)amino)methyl)-6-cyclopropylimidazo[1,2-a]pyridin-8-yl)piperazine-1-carboxylate

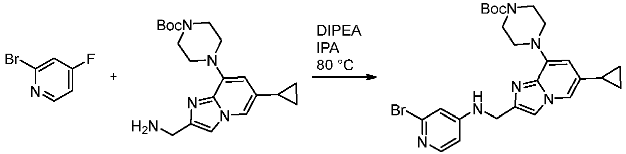

A mixture of *tert-butyl* 4-(2-(aminomethyl)-6-cyclopropylimidazo[1,2-a]pyridin-8-yl)piperazine-1-carboxylate (0.30 g, 0.80 mmol), 2-bromo-4-fluoropyridine (0.15 g, 0.84 mmol), and DIPEA (0.28 mL, 1.6 mmol) in iPrOH (5.0 mL) was stirred at 80 °C for 18 h. The mixture was concentrated *in vacuo* and the residue was purified by column chromatography on silica gel, eluting with 0-100 % ethyl acetate in cyclohexane to give **intermediate 726** (0.17 g, 40 %). ¹H NMR (400 MHz, CDCl₃) δ 7.93 (d, J=5.8 Hz, 1H), 7.53 - 7.52 (m, 1H), 7.33 - 7.32 (m, 1H), 6.73 (d, J=2.1 Hz, 1H), 6.46 (dd, J=2.3, 5.8 Hz, 1H), 6.24 - 6.23 (m, 1H), 5.03 - 5.00 (m, 1H), 4.44 (d, J=5.1 Hz, 2H), 3.72 - 3.68 (m, 4H), 3.47 - 3.41 (m, 4H), 1.88 - 1.80 (m, 1H), 1.50 (s, 9H), 0.96 - 0.90 (m, 2H), 0.67 - 0.62 (m, 2H).

Purification for intermediate 726 may be effected by column chromatography on silica gel, eluting with a gradient of 0 - 100 % EtOAc in cyclohexane (170 mg, 40 %).

The compounds in
Table **20** were synthesized using a similar procedure to that used for **intermediate 726** from 2-bromo-4-fluoropyridine and an appropriate coupling partner.

**Table 20**

| **Compound** | **Coupling Partner / Analytical Data** |
|---|---|
| 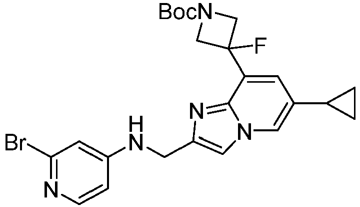 tert-butyl-3-(2-(((2-bromopyridin-4-yl)amino) methyl)-6-cyclopropylimidazo[1,2-*a*]pyridin-8-yl)-3-fluoroazetidine-1-carboxylate | tert-butyl-3-(2-(aminomethyl)-6-cyclopropyl imidazo[1,2-a]pyridin-8-yl)-3-fluoro azetidine-1-carboxylate |
| | ¹H NMR (400 MHz, CDCl₃) δ 7.93 (d, J=5.8 Hz, 1H), 7.86 (s, 1H), 7.43 (s, 1H), 7.03 (d, J=1.6 Hz, 1H), 6.75 (d, J=2.1 Hz, 1H), 6.59 (dd, J=2.2, 5.8 Hz, 1H), 5.51 (dd, J=4.8, 4.8 Hz, 1H), 4.87 - 4.87 (m, 2H), 4.40 - 4.37 (m, 4H), 4.32 (s, 4H), 1.94 - 1.87 (m, 1H), 1.45 (s, 9H), 1.01 - 0.96 (m, 2H), 0.71 - 0.66 (m, 2H). |
| 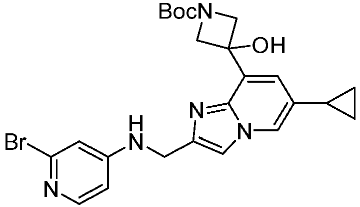 *tert*-butyl3-(2-(((2-bromopyridin-4-yl)amino) methyl)-6-cyclopropylimidazo[1,2-*a*]pyridin-8-yl)-3-hydroxyazetidine-1-carboxylate | *tert-butyl* 3-(2-(aminomethyl)-6-cyclopropyl imidazo[1,2-a]pyridin-8-yl)-3-hydroxy azetidine-1-carboxylate ¹H NMR (400 MHz, CDCl₃) δ 7.94 (d, J=5.8 Hz, 1H), 7.79 (s, 1H), 7.40 (s, 1H), 7.06 (d, J=1.5 Hz, 1H), 6.89 (s, 1H), 6.72 (d, J=2.1 Hz, 1H), 6.48 (dd, J=2.2, 5.8 Hz, 1H), 4.92 (dd, J=5.2, 5.2 Hz, 1H), 4.46 (d, J=5.4 Hz, 2H), 4.32 - 4.25 (m, 4H), 1.96 - 1.89 (m, 1H), 1.48 (s, 9H), 1.03 - 0.97 (m, 2H), 0.72 - 0.67 (m, 2H). |

### Intermediate 727: tert-butyl 4-(2-(((2-((1S,2S)-2-(3-chlorophenyl)cyclopropane-1-carboxamido)pyridin-4-yl)amino)methyl)-6-cyclopropylimidazo[1,2-a]pyridin-8-yl)piperazine-1-carboxylate

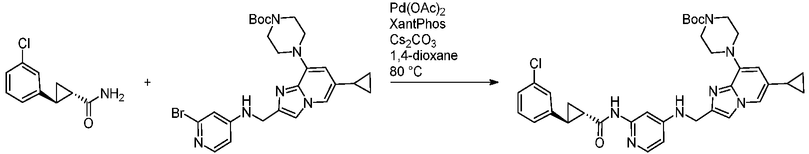

A mixture of *tert-butyl* 4-(2-(((2-bromopyridin-4-yl)amino)methyl)-6-cyclopropyl imidazo[1,2-a]pyridin-8-yl)piperazine-1-carboxylate (0.085 g, 0.16 mmol), **intermediate 119** (0.035 g, 0.18 mmol), Pd(OAc)₂ (0.007 g, 0.032 mmol), XantPhos (0.037 g, 0.065 mmol), and Cs₂CO₃ (0.11 g, 0.32 mmol) in 1,4-dioxane (5.0 mL) was degassed with nitrogen then stirred at 80 °C for 3 h. The mixture was filtered through Celite^{®} and concentrated *in vacuo.* The residue was purified by column chromatography on silica gel, eluting with 0-20 % (7 N NH₃ in MeOH) in DCM to give **intermediate 727** (0.071 g, 69 %). ¹H NMR (400 MHz, CDCl₃) δ 7.83 (d, J=5.9 Hz, 1H), 7.56 (s, 1H), 7.52 (s, 1H), 7.35 (s, 1H), 7.24 - 7.17 (m, 2H), 7.09 (s, 1H), 7.03 - 6.99 (m, 1H), 6.29 (dd, J=2.3, 5.9 Hz, 1H), 6.22 (d, J=1.3 Hz, 1H), 5.04 (s, 1H), 4.49 (d, J=5.1 Hz, 2H), 3.70 (dd, J=5.0, 5.0 Hz, 4H), 3.44 - 3.43 (m, 4H), 2.60 - 2.54 (m, 1H), 1.86 - 1.65 (m, 3H), 1.50 (s, 9H), 1.38 - 1.32 (m, 1H), 0.95 - 0.89 (m, 2H), 0.66 - 0.62 (m,2H).

Using a similar procedure to that used for **intermediate 727,** the compounds in **Table 21**
were prepared using **intermediate 119** and an appropriate coupling partner, then purified by preparative LCMS. **Table 21**

| **Compound** | **Coupling Partner / Analytical Data** |
|---|---|
| 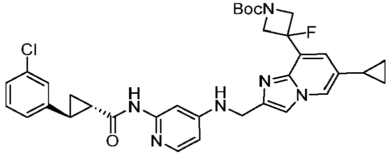 *tert*-butyl3-(2-(((2-((1*S*,2*S*)-2-(3-chloro phenyl)cyclopropane-1-carboxamido)pyridin-4-yl)amino)methyl)-6-cyclopropyl imidazo[1,2-a]pyridin-8-yl)-3-fluoroazetidine-1-carboxylate | *tert*-butyl3-(2-(((2-bromopyridin-4-yl)amino)methyl)-6-cyclopropylimidazo[1,2-*a*]pyridin-8-yl)-3-fluoroazetidine-1-carboxylate |
| | ¹H NMR (400 MHz, CDCl₃) δ 7.85 - 7.82 (m, 2H), 7.54 - 7.52 (m, 1H), 7.45 (s, 1H), 7.25 - 7.17 (m, 2H), 7.10 (s, 1H), 7.03 - 6.99 (m, 2H), 6.44 (dd, J=1.9, 5.7 Hz, 1H), 5.38 - 5.34 (m, 1H), 4.87 (s, 2H), 4.47 (s, 2H), 4.39 - 4.29 (m, 2H), 2.60 - 2.55 (m, 1H), 1.93 - 1.86 (m, 1H), 1.75 - 1.69 (m, 2H), 1.46 (s, 9H), 1.39 - 1.33 (m, 1H), 1.00 - 0.95 (m, 2H), 0.70 - 0.65 (m, 2H). |
| 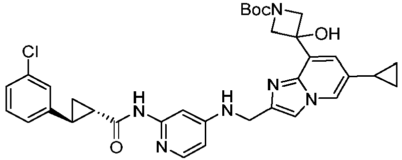 *tert*-butyl3-(2-(((2-((1*S*,2*S*)-2-(3-chloro phenyl)cyclopropane-1-carboxamido) pyridin-4-yl)amino)methyl)-6-cyclopropyl imidazo[1,2-a]pyridin-8-yl)-3-hydroxy azetidine-1-carboxylate | *tert*-butyl3-(2-(((2-bromopyridin-4-yl)amino) methyl)-6-cyclopropylimidazo[1,2-a]pyridin-8-yl) -3-hydroxyazetidine-1-carboxylate |
| | ¹H NMR (400 MHz, CDCl₃) δ 7.84 (d, J=5.9 Hz, 1H), 7.78 (s, 1H), 7.58 (s, 1H), 7.44 (s, 1H), 7.24 - 7.17 (m, 2H), 7.09 (dd, J=2.0, 2.0 Hz, 1H), 7.05 - 6.99 (m, 2H), 6.29 (dd, J=2.2, 5.8 Hz, 1H), 4.90 - 4.90 (m, 1H), 4.50 (d, J=5.6 Hz, 2H), 4.30 (d, J=9.0 Hz, 4H), 2.60 - 2.54 (m, 1H), 1.95 - 1.88 (m, 1H), 1.77 - 1.66 (m, 2H), 1.48 (s, 9H), 1.39 - 1.33 (m, 1H), 1.02 - 0.96 (m, 2H), 0.71 - 0.66 (m, 2H). |

### Example 132: (1S,2S)-2-(3-chlorophenyl)-N-(4-(((6-cyclopropyl-8-(piperazin-1-yl)imidazo[1,2-a]pyridin-2-yl)methyl)amino)pyridin-2-yl)cyclopropane-1-carboxamide (I-132)

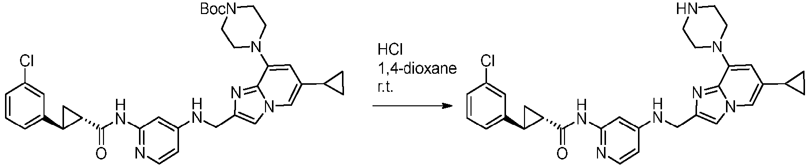

Boc protected (1S,2S)-2-(3-chlorophenyl)-N-(4-(((6-cyclopropyl-8-(piperazin-1-yl)imidazo[1,2-a]pyridin-2-yl)methyl)amino)pyridin-2-yl)cyclopropane-1-carboxamide (0.071 g, 0.11 mmol) was stirred in HCl solution (4.0 M in 1,4-dioxane, 0.28 mL, 1.1 mmol) in 1,4-dioxane (5.0 mL) at room temperature for 18 h. The mixture was diluted with methanol and loaded onto an SCX cartridge and washed with a mixture of methanol and DCM (1:4). The product was eluted with a mixture of 7 N NH₃ in MeOH in DCM (1:4) and concentrated *in vacuo.* The residue was purified by preparative HPLC to give **(I-132)** (0.036 g, 60 %). ESI-MS (M+H)+: 542.4, δ 10.34 (s, 1H), 7.91 (s, 1H), 7.80 (d, J=5.8 Hz, 1H), 7.57 (s, 1H), 7.51 - 7.47 (m, 1H), 7.38 - 7.32 (m, 1H), 7.31 - 7.28 (m, 2H), 7.18 (d, J=7.3 Hz, 1H), 7.12 (dd, J=5.2, 5.2 Hz, 1H), 6.37 (dd, J=2.1, 5.7 Hz, 1H), 6.19 (s, 1H), 4.38 (d, J=5.6 Hz, 2H), 3.48 - 3.42 (m, 4H), 2.97 - 2.96 (m, 4H), 2.41 - 2.37 (m, 2H), 1.93 - 1.85 (m, 1H), 1.52 - 1.45 (m, 1H), 1.42 - 1.36 (m, 1H), 0.94 - 0.87 (m, 2H), 0.72 - 0.67 (m, 2H).

Using a similar procedure to that used for **(I-132),** the compounds in Table 22 were prepared and purified by preparative LCMS. **Table 22**

| **EG** | **Compound** | **Prepared From / Analvtical Data** |
|---|---|---|
| 133 | 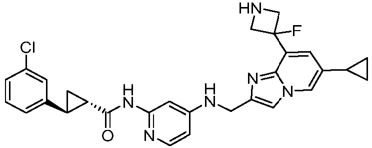 (1*S*,2*S*)-2-(3-chlorophenyl)-*N*-(4-(((6-cyclopropyl-8-(3-fluoroazetidin-3-yl)imidazo[1,2-*a*]pyridin-2-yl)methyl)amino)pyridin-2-yl)cyclo propane-1-carboxamide, formic acid salt **(I-133)** | *tert*-butyl-3-(2-(((2-((1*S*,2*S*)-2-(3-chloro phenyl)cyclopropane-1-carboxamido) pyridin-4-yl)amino)methyl)-6-cyclo propylimidazo[1,2-a]pyridin-8-yl)-3-fluoroazetidine-1-carboxylate |
| | | ESI-MS (M+H)+: 531.3, |
| | | δ 10.33 (s, 1H), 8.37 (s, 1H), 7.79 - 7.76 (m, 1H), 7.71 (s, 1H), 7.47 (s, 1H), 7.33 (dd, J=7.8, 7.8 Hz, 1H), 7.26 (d, J=7.7 Hz, 2H), 7.17 - 7.13 (m, 2H), 7.09 (s, 1H), 6.35 (dd, J=2.1, 5.8 Hz, 1H), 4.44 - 4.34 (m, 4H), 3.97 (dd, J=11.4, 21.5 Hz, 2H), 2.41 - 2.33 (m, 3H), 2.01 - 1.93 (m, 1H), 1.48 - 1.33 (m, 2H), 0.96 - 0.90 (m, 2H), 0.73 - 0.69 (m, 2H). |
| **134** | 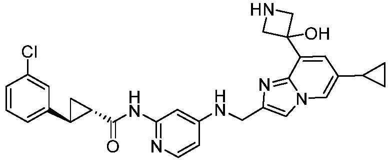 (1*S*,2*S*)-2-(3-chlorophenyl)-*N*-(4-(((6-cyclopropyl-8-(3-hydroxyazetidin-3-yl) imidazo[1,2-a]pyridin-2-yl)methyl)amino)pyridin-2-yl)cyclopropane-1-carboxamide **(I-134)** | *tert-butyl* 3-(2-(((2-((1*S*,2*S*)-2-(3-chloro phenyl)cyclopropane-1-carboxamido) pyridin-4-yl)amino)methyl)-6-cyclo propylimidazo[1,2-a]pyridin-8-yl)-3-hydroxyazetidine-1-carboxylate |
| | | ESI-MS (M+H)+: 529.3, ¹H NMR (400 MHz, MeOD) δ 8.16 (s, 1H), 7.79 (d, J=6.0 Hz, 1H), 7.71 (s, 1H), 7.47 (s, 1H), 7.29 (dd, J=7.8, 7.8 Hz, 1H), 7.23 - 7.19 (m, 3H), 7.13 (d, J=7.7 Hz, 1H), 6.44 (dd, J=2.3, 5.9 Hz, 1H), 4.56 (s, 2H), 4.41 (d, J=10.8 Hz, 2H), 4.07 (d, J=10.7 Hz, 2H), 2.51 - 2.45 (m, 1H), 2.23 - 2.17 (m, 1H), 2.02 - 1.92 (m, 1H), 1.65 - 1.59 (m, 1H), 1.39 (ddd, J=4.5, 6.4, 8.3 Hz, 1H), 1.03 - 0.97 (m, 2H), 0.78 - 0.73 (m, 2H). |

### Intermediate 728: tert-butyl 3-((2-(((2-bromopyridin-4-yl)amino)methyl)-6-cyclopropylimidazo[1,2-a]pyridin-8-yl)(hydroxy)methyl)azetidine-1-carboxylate

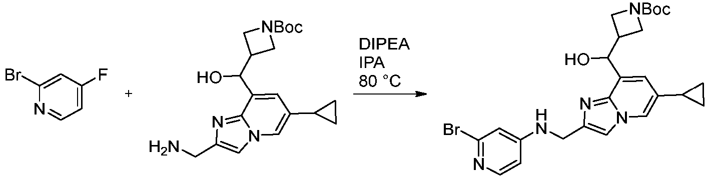

A mixture of *tert-butyl* 3-((2-(aminomethyl)-6-cyclopropylimidazo[1,2-a]pyridin-8-yl)(hydroxy)methyl)azetidine-1-carboxylate (0.082 g, 0.22 mmol), 2-bromo-4-fluoropyridine (0.039 g, 0.22 mmol), and DIPEA (0.076 mL, 0.44 mmol) in iPrOH (3.0 mL) was stirred at 80 °C for 48 h. The mixture was concentrated *in vacuo* and the residue was purified by column chromatography on silica gel, eluting with 0-20 % methanol in DCM to give **intermediate 728** (0.060 g, 52 %). ¹H NMR (400 MHz, CDCl₃) δ 7.94 (d, J=5.8 Hz, 1H), 7.78 (s, 1H), 7.39 (s, 1H), 6.81 (d, J=1.4 Hz, 1H), 6.73 (d, J=2.1 Hz, 1H), 6.48 (dd, J=2.1, 5.8 Hz, 1H), 4.46 (d, J=5.4 Hz, 2H), 4.06 (d, J=5.7 Hz, 1H), 3.90 - 3.84 (m, 2H), 3.76 - 3.71 (m, 2H), 3.18 - 3.12 (m, 1H), 1.90 - 1.85 (m, 1H), 1.45 (s, 9H), 1.01 - 0.95 (m, 2H), 0.68 - 0.63 (m, 2H).

### Intermediate 729: tert-butyl 3-((2-(((2-((1S,2S)-2-(3-chlorophenyl)cyclopropane-1-carboxamido)pyridin-4-yl)amino)methyl)-6-cyclopropylimidazo[1,2-a]pyridin-8-yl)(hydroxy)methyl)azetidine-1-carboxylate

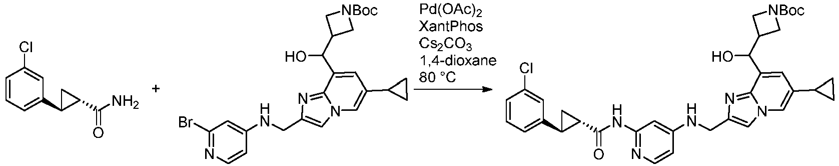

A mixture of **intermediate 728** (0.060 g, 0.11 mmol), **intermediate 119** (0.024 g, 0.13 mmol), XantPhos (0.013 g, 0.023 mmol), and Cs₂CO₃ (0.074 g, 0.23 mmol) in 1,4-dioxane (5.0 mL) was degassed with nitrogen and Pd(OAc)₂ (0.003 g, 0.011 mmol) was added. The mixture was stirred at 80°C for 3 h. The mixture was filtered through Celite^{®} and concentrated *in vacuo.* The residue was purified by column chromatography on silica gel, eluting with 0-20 % (7 N NH₃ in MeOH) in DCM to give **intermediate 729** (0.025 g, 34 %). ¹H NMR (400 MHz, CDCl₃) δ 7.85 (d, J=5.6 Hz, 1H), 7.77 (s, 1H), 7.59 (s, 1H), 7.43 (s, 1H), 7.23 - 7.19 (m, 2H), 7.10 (s, 1H), 7.03 - 6.99 (m, 1H), 6.79 (d, J=1.1 Hz, 1H), 6.30 (dd, J=2.3, 5.8 Hz, 1H), 4.50 (d, J=5.4 Hz, 2H), 4.06 (d, J=6.4 Hz, 2H), 3.86 - 3.84 (m, 1H), 3.75 - 3.71 (m, 1H), 3.18 - 3.18 (m, 1H), 2.61 - 2.55 (m, 1H), 1.87 (dd, J=8.3, 8.3 Hz, 1H), 1.74 - 1.69 (m, 2H), 1.39 - 1.34 (m, 1H), 1.00 - 0.94 (m, 2H), 0.67 - 0.63 (m, 2H), -0.03 (s, 1H).

### Example 135: (1S,2S)-N-(4-(((8-(-azetidin-3-yl(hydroxy)methyl)-6-cyclopropylimidazo[1,2-alpyridin-2-yl)methyl)amino)pyridin-2-yl)-2-(3-chlorophenyl)cyclopropane-1-carboxamide (I-135)

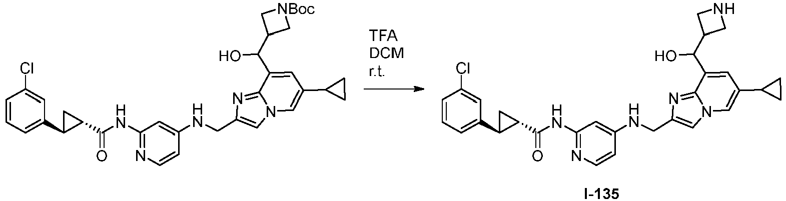

**Intermediate 729** (0.025 g, 0.039 mmol) was stirred with trifluoroacetic acid (0.10 mL, 1.3 mmol) in DCM (2.0 mL) at room temperature for 1 h. The mixture was concentrated *in vacuo* and the residue was loaded onto an SCX cartridge and washed with a mixture of methanol and DCM. The product was eluted with a mixture of 7 N NH₃ in MeOH in DCM and concentrated *in vacuo.* The residue was purified by preparative HPLC to give **(I-135)** (0.016 g, 76 %) as a trifluoroacetic acid salt. ESI-MS (M+H)+: 543.4¹H NMR (400 MHz, MeOD) δ 8.24 (s, 1H), 7.93 - 7.81 (m, 2H), 7.46 (s, 1H), 7.34 - 7.23 (m, 3H), 7.15 (d, J=7.7 Hz, 1H), 6.79 (s, 1H), 6.38 (s, 1H), 5.35 (d, J=3.1 Hz, 1H), 4.71 (s, 2H), 4.23 (dd, J=3.6, 11.2 Hz, 2H), 4.15 (dd, J=6.6, 10.6 Hz, 1H), 3.89 (dd, J=9.7, 9.7 Hz, 1H), 3.52 - 3.42 (m, 1H), 2.68 - 2.62 (m, 1H), 2.13 - 2.00 (m, 2H), 1.80 - 1.74 (m, 1H), 1.58 (ddd, J=4.6, 6.7, 8.2 Hz, 1H), 1.10 - 1.04 (m, 2H), 0.78 (q, J=5.0 Hz, 2H).

### Example 137: (1S,2S)-2-(4-chloropyridin-2-yl)-N-(4-(((6-cyclopropylimidazo[1,2-a]pyridin-2-yl)methyl)(2-hydroxyethyl)amino)pyridin-2-yl)cyclopropane-1-carboxamide (I-137)

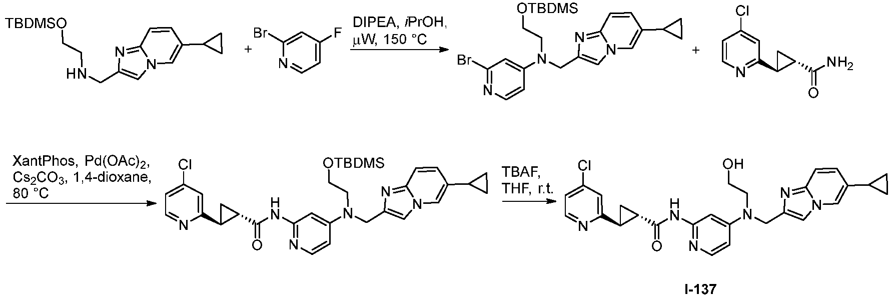

2-bromo-4-fluoropyridine (150 mg, 0.85 mmol), **intermediate 253** (290 mg, 0.85 mmol), and DIPEA (0.44 mL, 2.6 mmol) in iPrOH (4.0 mL) were heated to 150 °C in a microwave for 12 h. The mixture was cooled to room temperature and concentrated in vacuo. The residue was purified by column chromatography on silica gel, eluting with a gradient of 0-10 % (7 N NH₃ in MeOH) in DCM to give **2-bromo-N-(2-((tert-butyldimethylsilyl)oxy)ethyl)-N-((6-cyclopropylimidazo[1,2-a]pyridin-2-yl)methyl)pyridin-4-amine** (100 mg, 23 %). ESI-MS (M+H)+: 501, 503, ¹H NMR (400 MHz, CDCl₃) δ 7.86 (d, J=6.1 Hz, 1H), 7.76 (d, J=0.5 Hz, 1H), 7.41 (d, J=9.3 Hz, 1H), 7.19 (s, 1H), 6.91 (dd, J=1.7, 9.3 Hz, 1H), 6.78 (d, J=2.4 Hz, 1H), 6.53 (dd, J=2.4, 6.1 Hz, 1H), 4.69 (s, 2H), 3.82 (dd, J=5.6, 5.6 Hz, 2H), 3.60 (dd, J=5.6, 5.6 Hz, 2H), 1.88 - 1.81 (m, 1H), 0.95 - 0.88 (m, 2H), 0.84 (s, 9H), 0.65 - 0.60 (m, 2H), 0.00 (s, 6H).

(1S,2S)-2-(4-chloropyridin-2-yl)cyclopropane-1-carboxamide (86 mg, 0.44 mmol), Pd(OAc)₂ (9.0 mg, 0.04 mmol), XantPhos (46 mg, 0.08 mmol), and Cs₂CO₃ (260 mg, 0.80 mmol) were placed under N₂ atmosphere. A solution of 2-bromo-N-(2-((tert-butyldimethylsilyl)oxy)ethyl)-N-((6-cyclopropylimidazo[1,2-a]pyridin-2-yl)methyl)pyridin-4-amine (200 mg, 0.40 mmol) in 1,4-dioxane (0.5 mL) was added, and the reaction mixture was degassed for 10 min and stirred at 80 °C for 16 h. The reaction mixture was allowed to cool to room temperature, filtered through Celite^{®}, washed with MeOH, and concentrated in vacuo. The residue was purified by column chromatography on silica gel, eluting with a gradient of 0-10 % (7N NH₃ in MeOH) in DCM to give **(1S,25)-N-(4-((2-((tert-butyldimethylsilyl)oxy)ethyl)((6-cyclopropylimidazo[1,2-a]pyridin-2-yl)methyl)amino)pyridin-2-yl)-2-(4-chloropyridin-2-yl)cyclopropane-1-carboxamide** (90 mg, 37 %). ESI-MS (M+H)+: 617.4, ¹H NMR (400 MHz, CDCl₃) δ 9.88 (s, 1H), 8.18 (d, J=5.6 Hz, 1H), 7.77 (s, 1H), 7.73 (s, 1H), 7.62 (d, J=6.3 Hz, 1H), 7.42 (d, J=9.3 Hz, 1H), 7.33 (s, 1H), 7.22 (d, J=1.5 Hz, 1H), 7.02 (dd, J=2.0, 5.3 Hz, 1H), 6.89 (dd, J=1.6, 9.2 Hz, 1H), 6.22 (dd, J=2.5, 6.3 Hz, 1H), 4.75 (s, 2H), 3.86 (dd, J=5.6, 5.6 Hz, 2H), 3.71 - 3.65 (m, 2H), 2.61 - 2.55 (m, 1H), 1.88 - 1.80 (m, 1H), 1.72 - 1.66 (m, 1H), 0.95 - 0.90 (m, 2H), 0.85 (s, 9H), 0.65 - 0.59 (m, 2H), 0.00 (s, 6H).

(1S,2S)-N-(4-((2-((tert-butyldimethylsilyl)oxy)ethyl)((6-cyclopropylimidazo[1,2-a]pyridin-2-yl)methyl)amino)pyridin-2-yl)-2-(4-chloropyridin-2-yl)cyclopropane-1-carboxamide (90 mg, 0.15 mmol) was stirred with a solution of TBAF (1M in THF, 0.29 mL, 0.29 mmol) in DCM (1.0 mL) at room temperature for 4 h. The reaction mixture was concentrated in vacuo. The residue was purified by column chromatography on silica gel, eluting with a gradient of 0-10 % (7 N NH₃ in MeOH) in DCM and freeze-dried to give **(I-137)** (60 mg, 82 %). ESI-MS (M+H)+: 503.0, ¹H NMR (400 MHz, DMSO) δ 10.40 (s, 1H), 8.42 (d, J=5.3 Hz, 1H), 8.29 (s, 1H), 7.79 (d, J=5.8 Hz, 1H), 7.65 - 7.62 (m, 2H), 7.58 (s, 1H), 7.38 (d, J=9.3 Hz, 1H), 7.33 (dd, J=2.1, 5.4 Hz, 1H), 6.98 (dd, J=1.8, 9.3 Hz, 1H), 6.45 (dd, J=2.4, 6.2 Hz, 1H), 5.35 (s, 1H), 4.66 (s, 2H), 3.66 (dd, J=5.7, 5.7 Hz, 2H), 3.57 (dd, J=6.4, 6.4 Hz, 2H), 2.63 - 2.52 (m, 2H), 1.95 - 1.87 (m, 1H), 1.53 - 1.44 (m, 2H), 0.93 - 0.87 (m, 2H), 0.69 - 0.64 (m, 2H).

### Intermediate 730: 2-bromo-N-(1-(6-cyclopropylimidazo[1,2-a]pyridin-2-yl)-2,2,2-trifluoroethyl)pyridin-4-amine

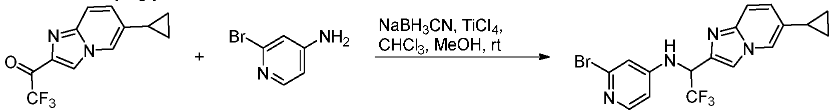

TiCl₄ (0.10 mL, 0.87 mmol) was added dropwise to a solution of **intermediate 254** (220 mg, 0.87 mmol) and 2-bromopyridin-4-amine (300 mg, 1.7 mmol) in CHCl₃ (10 mL), and the reaction mixture was stirred at room temperature for 16 h. A solution of NaCNBH₃ (110 mg, 1.7 mmol) in MeOH (3.0 mL) was added dropwise and the reaction mixture was stirred at room temperature for 5 h. The reaction mixture was quenched with NaHCO₃ (sat. aq., 20 mL) and extracted with DCM (3×40 mL). The combined organics were dried over MgSO₄ and concentrated *in vacuo.* The residue was purified by column chromatography on silica gel, eluting with a gradient of 30-40 % EtOAc in cyclohexane to give **intermediate 730** (80 mg, 22 %) at ~50 % purity. ESI-MS (M+H)+: 411, 413.

### Intermediate 731: tert-butyl 6-(2-(aminomethyl)-6-cyclopropylimidazo[1,2-a]pyridin-8-yl)-2,6-diazaspiro[3.3]heptane-2-carboxylate

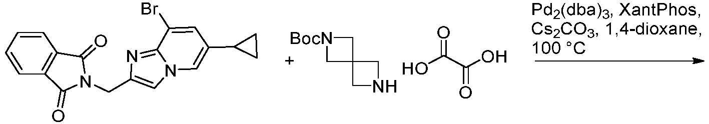

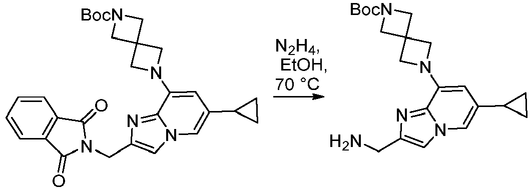

A suspension of 2-((8-bromo-6-cyclo propylimidazo[1,2-a]pyridin-2-yl)methyl)isoindoline-1,3-dione (300 mg, 0.76 mmol), tert-butyl 2,6-diazaspiro[3.3]heptane-2-carboxylate oxalate (330 mg, 1.1 mmol), XantPhos (88 mg, 0.15 mmol), and Cs₂CO₃ (740 mg, 2.3 mmol) in 1,4-dioxane (6.0 mL) was degassed for 10 min. Pd₂(dba)₃ (69 mg, 0.08 mmol) was added and the reaction mixture was stirred at 100 °C for 3 h. The reaction mixture was allowed to cool to room temperature, filtered through Celite^{®} and concentrated in vacuo. The residue was purified by column chromatography on silica gel, eluting with a gradient of 0-10 % EtOAc in cyclohexane to give **tert-butyl 6-(6-cyclopropyl-2-((1,3-dioxoisoindolin-2-yl)methyl)imidazo[1,2-a]pyridin-8-yl)-2,6-diazaspiro[3.3]heptane-2-carboxylate intermediate 732** (190 mg, 49 %). ESI-MS (M+H)+: 514.2

Using a similar procedure to that for **intermediate 278, intermediate 731** was synthesized from **intermediate 732** using hydrazine hydrate and used without further purification. ESI-MS (M+H)+: 384.3, ¹H NMR (400 MHz, CDCl₃) δ 7.36 (s, 1H), 7.28 (s, 1H), 5.67 (d, J=1.4 Hz, 1H), 4.29 (s, 4H), 4.11 (s, 4H), 3.94 (s, 2H), 1.84 - 1.78 (m, 1H), 1.45 (s, 9H), 0.92 - 0.86 (m, 2H), 0.65 - 0.60 (m, 2H).

### Intermediate 733: N-(2-(((2-bromopyridin-4-yl)amino)methyl)-6-cyclopropylimidazo[1,2-a]pyridin-8-yl)-N-methylacetamide

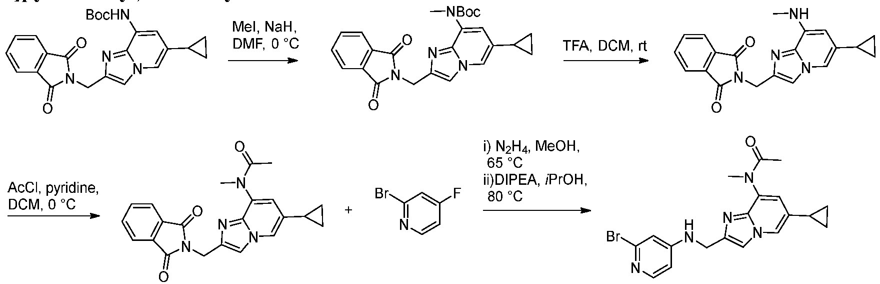

A solution of **intermediate 338** (500 mg, 1.2 mmol) in DMF (10 mL) was cooled to 0 °C. NaH (60 % in mineral oil, 56 mg, 1.4 mmol) was added and the reaction mixture was stirred at 0 °C for 30 min. MeI (86 µL, 1.4 mmol) was added and the reaction mixture was stirred at 0 °C for 2 h. The reaction mixture was allowed to warm to room temperature, quenched with water (10 mL), and extracted with EtOAc (3×20 mL). The combined organics were passed through a hydrophobic frit and concentrated *in vacuo.* The residue was purified by column chromatography on silica gel, eluting with a gradient of 0-100 % EtOAc in cyclohexane to give ***tert-butyl* (6-cyclopropyl-2-((1,3-dioxoisoindolin-2-yl)methyl)imidazo[1,2-a]pyridin-8-yl)(methyl)carbamate intermediate 734** (195 mg, 38 %). ¹H NMR (400 MHz, CDCl₃) δ 7.87 (dd, J=3.0, 5.6 Hz, 2H), 7.72 (dd, J=3.0, 5.6 Hz, 2H), 7.68 (s, 1H), 7.41 (s, 1H), 6.81 (s, 1H), 5.05 (s, 2H), 3.34 (s, 3H), 1.88 - 1.80 (m, 1H), 1.37 (s, 9H), 0.96 - 0.89 (m, 2H), 0.65 - 0.59 (m, 2H).

Trifluoroacetic acid (0.14 mL, 1.8 mmol) was added to a solution of **intermediate 734** (195 mg, 0.45 mmol) in DCM (10 mL) and the reaction mixture was stirred at room temperature for 5 h. The reaction mixture was quenched with NaHCO₃ (sat. aq., 35 mL) and extracted with DCM (2×50 mL). The combined organics were passed through a hydrophobic frit and concentrated *in vacuo* to give **2-((6-cyclopropyl-8-(methylamino)imidazo [1,2-a]pyridin-2-yl)methyl) isoindoline-1,3-dione intermediate 735** (106 mg, 71 %). ESI-MS (M+H)+: 347.2

. Acetyl chloride (21 µL, 0.29 mmol) was added to a solution of **intermediate 735** (100 mg, 0.29 mmol) and pyridine (0.07 mL, 0.87 mmol) in DCM (1.0 mL) at 0 °C, and the reaction mixture was stirred at 0 °C for 3 h. The reaction mixture was allowed to warm to room temperature, quenched with water (5 mL), and extracted with DCM (3×15 mL). The combined organics were dried over a hydrophobic frit and concentrated *in vacuo.* The residue was purified by column chromatography on silica gel, eluting with a gradient of 0-100 % EtOAc in cyclohexane to give **N-(6-cyclopropyl-2-((1,3-dioxoisoindolin-2-yl)methyl)imidazo[1,2-a]pyridin-8-yl)-N-methylacetamide intermediate 736** (87 mg, 78 %). ESI-MS (M+H)+: 384.2, ¹H NMR (400 MHz, CDCl₃) δ 7.88 (dd, J=3.0, 5.6 Hz, 2H), 7.79 (s, 1H), 7.74 (dd, J=3.0, 5.3 Hz, 2H), 7.47 (s, 1H), 6.78 (s, 1H), 5.06 (s, 2H), 3.33 (s, 3H), 1.88 - 1.81 (m, 1H), 1.26 (s, 3H), 1.26 (t, J=7.2 Hz, 3H), 0.97 (d, J=8.3 Hz, 2H), 0.66 - 0.60 (m, 2H).

Using a similar procedure to that for **intermediate 278,** *N*-(2-(aminomethyl)-6-cyclopropylimidazo[1,2-*a*]pyridin-8-yl)-*N*-methylacetamide (44 mg, 0.17 mmol) was synthesized from **intermediate 736** using hydrazine hydrate. Without further purification. this was dissolved in iPrOH (5.0 mL), then DIPEA (0.09 mL, 0.51 mmol) and 2-bromo-4-fluoropyridine (30 mg, 0.17 mmol) were added. The reaction mixture was stirred at 80 °C for 16 h. The reaction mixture was allowed to cool to room temperature, water (10 mL) was added, and the reaction mixture was extracted with EtOAc (3×20 mL). The combined organics were dried over a hydrophobic frit and concentrated *in vacuo.* The residue was purified by column chromatography on silica gel, eluting with a gradient of 0-100 % EtOAc in cyclohexane followed by 0-20 % (7 N NH₃ in MeOH) in EtOAc to give **intermediate 733** (45 mg, 63 %). ESI-MS (M+H)+: 416.1, ¹H NMR (400 MHz, CDCl₃) δ 7.91 (d, J=5.8 Hz, 1H), 7.87 (s, 1H), 7.48 (s, 1H), 6.85 (s, 1H), 6.70 (d, J=2.3 Hz, 1H), 6.47 (dd, J=2.0, 5.8 Hz, 1H), 5.40 (dd, J=5.6, 5.6 Hz, 1H), 4.48 (d, J=5.6 Hz, 2H), 3.36 (s, 3H), 1.94 (br s, 4H), 1.05 - 0.97 (m, 2H), 0.72 - 0.64 (m, 2H).

### N-(2-(((2-bromopyridin-4-yl)amino)methyl)-6-cyclopropylimidazo[1,2-a]pyridin-8-yl)acetamide

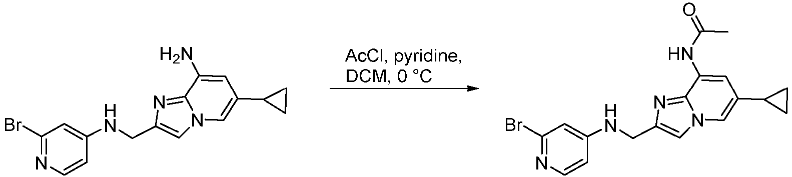

Acetyl chloride (12 µL, 0.17 mmol) was added to a solution of 2-(((2-bromopyridin-4-yl)amino)methyl)-6-cyclopropylimidazo[1,2-a]pyridin-8-amine (60 mg, 0.17 mmol) and pyridine (0.04 mL, 0.48 mmol) in DCM (0.3 mL) at 0 °C and the reaction mixture was stirred at 0 °C for 2 h. The reaction mixture was allowed to warm to room temperature, quenched with water (3 mL), and extracted with EtOAc (3×10 mL). The combined organics were dried over a hydrophobic frit and concentrated *in vacuo.* The residue was purified by column chromatography on silica gel, eluting with a gradient of 0 - 10 % (7N NH₃ in MeOH) in DCM to give the title compound (75 mg, quant). ESI-MS (M+H)+: 400.1, 402.1

Using a similar procedure to that used for **intermediate 726,** the compound in Table **23** was prepared from **intermediate 731** and 2-bromo-4-fluoropyridine. **Table 23**

| **Compound** | **Coupling Partner / Analvtical Data** |
|---|---|
| 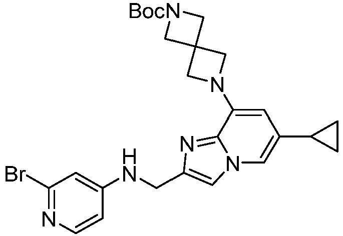 *tert*-butyl6-(2-(((2-bromopyridin-4-yl)amino) methyl)-6-cyclopropylimidazo[1,2-a]pyridin-8-yl)-2,6-diazaspiro[3.3]heptane-2-carboxylate | tert-butyl6-(2-(aminomethyl)-6-cyclo propylimidazo[1,2-a]pyridin-8-yl)-2,6-diazaspiro[3.3]heptane-2-carboxylate |
| | ESI-MS (M+H)+: 539.1, 541.0, ¹H NMR (400 MHz, CDCl₃) δ 7.92 (d, J=5.8 Hz, 1H), 7.36 (s, 1H), 7.30 (s, 1H), 6.75 (d, J=2.1 Hz, 1H), 6.47 (dd, J=2.2, 5.8 Hz, 1H), 5.72 (s, 1H), 5.08 (s, 1H), 4.41 (d, J=5.1 Hz, 2H), 4.31 (s, 4H), 4.13 (s, 4H), 1.85 - 1.77 (m, 1H), 1.46 (s, 9H), 0.93 - 0.86 (m, 2H), 0.66 - 0.61 (m, 2H). |

### Intermediate 737: 2-bromo-N-((6-cyclopropyl-8-(4-methylpiperazin-1-yl)imidazo[1,2-a]pyridin-2-yl)methyl)pyridin-4-amine intermediate 737

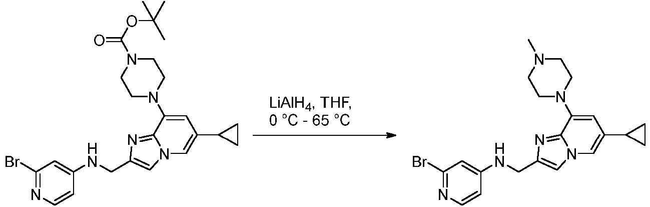

A solution of **intermediate 276** (85 mg, 0.16 mmol) in THF (5.0 mL) was cooled to 0 °C, and LiAlH₄ (1 M in THF, 0.48 mL, 0.48 mmol) was added dropwise. The reaction mixture was stirred at 65 °C for 3 h. The reaction mixture was allowed to cool to room temperature, quenched with NH₄Cl (sat. aq., 10 mL), and extracted with DCM (3 × 20 mL). The combined organics were dried over MgSO₄ and concentrated *in vacuo* to give **intermediate 737** which was used without further purification. ESI-MS (M+H)+: 441.1, 443.1 ¹H NMR (400 MHz, CDCl₃): δ ppm, 8.20 (ddd, J=1.3, 1.3, 6.2 Hz, 1H), 7.50 (ddd, J=0.8, 1.3, 4.8 Hz, 1H), 7.32 - 7.30 (m, 1H), 6.53 (ddd, J=1.3, 1.3, 6.2 Hz, 1H), 6.23 (dd, J=1.3, 4.8 Hz, 1H), 4.45 (dd, J=5.2, 13.3 Hz, 2H), 3.53 (br s, 4H), 2.70 (t, J=4.7 Hz, 4H), 2.40 (s, 3H), 1.88 - 1.80 (m, 1H), 0.95 - 0.89 (m, 2H), 0.67 - 0.63 (m, 2H), aromatic CH obscured by CDCl₃ peak.

### Intermediate 738: 2-bromo-N-((6-cyclopropyl-8-(6-methyl-2,6-diazaspiro[3.3]heptan-2-yl)imidazo[1,2-a]pyridin-2-yl)methyl)pyridin-4-amine

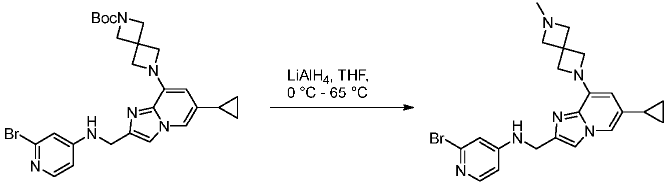

Using a similar procedure to that used for **intermediate** 737, **intermediate 738** was prepared from *tert-butyl* 6-(2-(((2-bromopyridin-4-yl)amino)methyl)-6-cyclopropylimidazo[1,2-a]pyridin-8-yl)-2,6-diazaspiro[3.3]heptane-2-carboxylate and LiAlH₄. ESI-MS (M+H)+: 441.1, 443.1, ¹H NMR (400 MHz, CDCl₃) δ 7.92 (d, J=5.8 Hz, 1H), 7.34 (s, 1H), 7.28 (s, 1H), 6.76 (d, J=2.0 Hz, 1H), 6.47 (dd, J=2.3, 5.8 Hz, 1H), 5.70 (d, J=1.3 Hz, 1H), 5.11 (t, J=4.6 Hz, 1H), 4.39 (d, J=5.0 Hz, 2H), 4.28 (s, 4H), 3.52 (s, 4H), 2.39 (s, 3H), 1.83 - 1.76 (m, 1H), 0.93 - 0.86 (m, 2H), 0.65 - 0.60 (m, 2H).

### Example 138: (1S,2S)-2-(3-chlorophenyl)-N-(4-(((6-cyclopropyl-8-(4-methylpiperazin-1-yl) imidazo[1,2-a]pyridin-2-yl)methyl)amino)pyridin-2-yl)cyclopropane-1-carboxamide (I-138)

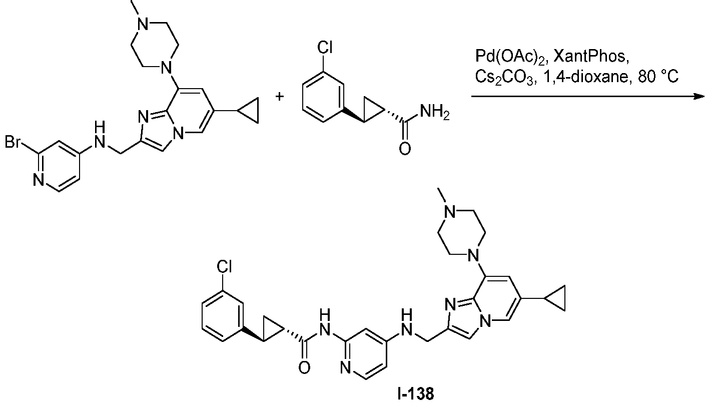

**Intermediate 737** (69 mg, 0.16 mmol), **intermediate 119** (31 mg, 0.16 mmol), XantPhos (18 mg, 0.03 mmol), Cs₂CO₃ (100 mg, 0.31 mmol), and Pd(OAc)₂ (3.5 mg, 0.015 mmol) were placed under a N₂ atmosphere. 1,4-Dioxane (1.5 mL) was added and the reaction mixture was stirred at 80 °C for 16 h. The reaction mixture was cooled to room temperature, filtered through Celite^{®}, washed with EtOAc (30 mL), and concentrated in vacuo. The residue was purified by column chromatography on silica gel, eluting with a gradient of 0 - 20 % 7 N NH₃ in MeOH in DCM. The residue was further purified by column chromatography on silica gel, eluting with 4 % 7 N NH₃ in MeOH in DCM. The residue was further purified by preparative HPLC to give of **(I-138)** (10 mg, 11 %) as a formic acid salt. ESI-MS (M+H)+: 556.3, δ 8.24 (s, 1H), 7.88 (d, J=0.9 Hz, 1H), 7.77 (d, J=5.8 Hz, 1H), 7.54 (s, 1H), 7.46 (s, 1H), 7.32 (ddd, J=7.4, 7.4, 1.3 Hz, 1H), 7.28 - 7.24 (m, 2H), 7.15 (ddd, J=1.3, 1.3, 7.6 Hz, 1H), 7.10 (t, J=5.8 Hz, 1H), 6.33 (dd, J=2.2, 5.8 Hz, 1H), 6.17 (d, J=1.3 Hz, 1H), 4.35 (d, J=5.7 Hz, 2H), 3.49 (s, 4H), 2.41 - 2.36 (m, 2H), 2.26 (s, 3H), 1.90 - 1.83 (m, 1H), 1.48 - 1.43 (m, 1H), 1.39 - 1.33 (m, 1H), 0.90 - 0.85 (m, 2H), 0.69 - 0.64 (m, 2H).

Using a similar procedure to that used for (1S,2S)-2-(3-chlorophenyl)-N-(4-(((6-cyclopropyl-8-(4-methylpiperazin-1-yl)imidazo[1,2-a]pyridin-2-yl)methyl)amino)pyridin-2-yl)cyclopropane-1-carboxamide, the compounds in Table **24** were prepared from (1S,2S)-2-(3-chlorophenyl)cyclo propane-1-carboxamide and an appropriate coupling partner. **Table 24**

| **EG** | **Compound** | **Coupling Partner / Analytical Data** |
|---|---|---|
| **139** | 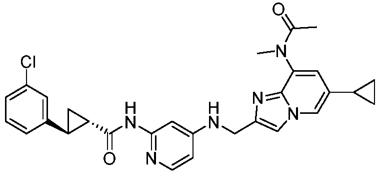 (1S,2S)-2-(3-chlorophenyl)-N-(4-(((6-cyclopropyl-8-(N-methylacetamido)imidazo[1,2-a]pyridin-2-yl)methyl)amino)pyridin-2-yl)cyclopropane-1-carboxamide **(I-139)** | **Intermediate 733**ESI-MS (M+H)+: 529, δ 10.35 (s, 1H), 8.40 (s, 1H), 7.82 - 7.76 (m, 2H), 7.50 (s, 1H), 7.39 - 7.28 (m, 3H), 7.23 - 7.18 (m, 2H), 7.11 (s, 1H), 6.38 (dd, J=2.3, 5.8 Hz, 1H), 4.43 (d, J=5.8 Hz, 2H), 3.26 (s, 3H), 2.45 - 2.37 (m, 2H), 2.02 - 1.94 (m, 1H), 1.84 (s, 3H), 1.52 - 1.47 (m, 1H), 1.42 - 1.37 (m, 1H), 1.01 - 0.95 (m, 2H), 0.77 (q, J=5.1 Hz, 2H). |
| 140 | 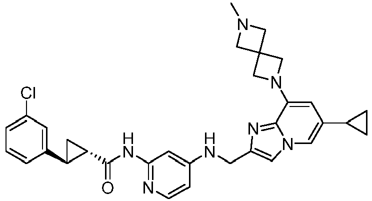 (1S,2S)-2-(3-chlorophenyl)-N-(4-(((6-cyclopropyl-8-(6-methyl-2,6-diazaspiro[3.3]heptan-2-yl)imidazo[1,2-a]pyridin-2-yl)methyl)amino)pyridin-2-yl)cyclopropane-1-carboxamide **(I-140)** | **Intermediate 738** |
| | | ESI-MS (M+H)+: 568.4, δ 10.32 (s, 1H), 7.77 (d, J=5.8 Hz, 1H), 7.68 (s, 1H), 7.51 (s, 1H), 7.45 (s, 1H), 7.35 - 7.30 (m, 1H), 7.26 (d, J=7.3 Hz, 2H), 7.15 (d, J=7.8 Hz, 1H), 7.05 (dd, J=5.6, 5.6 Hz, 1H), 6.33 (dd, J=2.1, 5.8 Hz, 1H), 5.66 (d, J=1.1 Hz, 1H), 4.31 (d, J=5.6 Hz, 2H), 4.16 (s, 4H), 3.30 (s, 2H), 2.42 - 2.34 (m, 2H), 2.22 (s, 3H), 1.85 - 1.77 (m, 1H), 1.49 - 1.43 (m, 1H), 1.39 - 1.33 (m, 1H), 0.88 - 0.82 (m, 2H), 0.65 - 0.60 (m, 2H). |
| **141** | 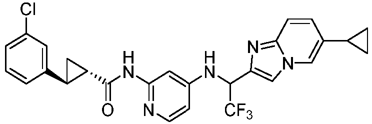 (1S,2S)-2-(3-chlorophenyl)-N-(4-((1-(6-cyclopropylimidazo[1,2-a]pyridin-2-yl)-2,2,2-trifluoroethyl)amino)pyridin-2-yl)cyclopropane-1-carboxamide, formic acid salt **(I-141)** | **Intermediate 730** ESI-MS (M+H)+: 526.3, δ 10.42 (s, 1H), 8.43 (s, 1H), 7.97 (s, 1H), 7.86 (d, J=5.8 Hz, 1H), 7.66 (s, 1H), 7.48 (d, J=9.3 Hz, 1H), 7.42 (dd, J=2.7, 9.5 Hz, 1H), 7.36 - 7.31 (m, 1H), 7.27 (d, J=5.8 Hz, 2H), 7.17 (d, J=7.8 Hz, 1H), 7.08 (dd, J=1.8, 9.4 Hz, 1H), 6.65 (dd, J=2.2, 5.8 Hz, 1H), 5.64 (dd, J=8.1, 8.1 Hz, 1H), 2.42 - 2.34 (m, 3H), 1.99 - 1.92 (m, 1H), 1.51 - 1.36 (m, 2H), 0.97 - 0.92 (m, 2H), 0.73 - 0.68 (m, 2H). |
| 142 | 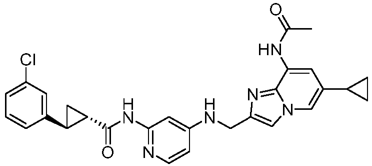 (1*S*,2*S*)-*N*-(4-(((8-acetamido-6-cyclopropylimidazo[1,2-a]pyridin-2-yl)methyl)amino)pyridin-2-yl)-2-(3-chlorophenyl)cyclopropane-1-carboxamide, formic acid salt **(I-142)** | *N*-(2-(((2-bromopyridin-4-yl)amino) methyl)-6-cyclopropylimidazo[1,2*-a*] pyridine-8-yl)acetamide |
| | | ESI-MS (M+H)+: 515.4, δ 10.38 (s, 1H), 9.93 (s, 1H), 8.08 (s, 1H), 7.84 - 7.80 (m, 2H), 7.69 (s, 1H), 7.50 (s, 1H), 7.37 - 7.28 (m, 3H), 7.21 - 7.12 (m, 2H), 6.36 (dd, J=1.6, 5.9 Hz, 1H), 4.45 (d, J=5.6 Hz, 2H), 2.45 - 2.39 (m, 2H), 2.25 (s, 3H), 1.98 - 1.91 (m, 1H), 1.53 - 1.46 (m, 1H), 1.43 - 1.38 (m, 1H), 0.98 - 0.92 (m, 2H), 0.69 - 0.64 (m, 2H). |

### Example 143: rac-(1S*2S*)-2-(5-chloro-2-cyanophenyl)-N-(4-(((6-cyclopropyl-8-(4-methyl piperazin-1-yl)imidazo[1,2-a]pyridin-2-yl)methyl)amino)pyridin-2-yl)cyclopropane-1-carboxamide (I-143)

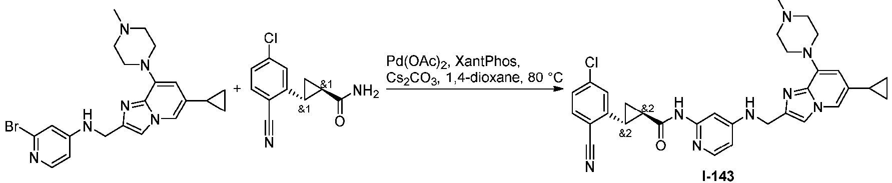

A suspension of **intermediate 737** (56 mg, 0.13 mmol), **intermediate 118** (31 mg, 0.14 mmol), XantPhos (15 mg, 0.03 mmol), and Cs₂CO₃ (83 mg, 0.25 mmol) in 1,4-dioxane (3.0 mL) was degassed for 5 min. Pd(OAc)₂ (2.8 mg, 0.01 mmol) was added and the reaction mixture was stirred at 80 °C for 16 h. The reaction mixture was cooled to room temperature, filtered through Celite^{®}, and concentrated *in vacuo.* The residue was purified by column chromatography on silica gel, eluting with a gradient of 0-20 % (7N NH₃ in MeOH) in DCM. The residue was further purified by column chromatography on silica gel, eluting with 2 - 2.5 % (7N NH₃ in MeOH) in DCM. The residue was further purified by preparative HPLC to give **(I-143)** (10 mg, 14 %). ESI-MS (M+H)+: 581.5, δ 10.38 (s, 1H), 7.89 - 7.86 (m, 2H), 7.78 (d, J=5.8 Hz, 1H), 7.56 - 7.51 (m, 2H), 7.48 (s, 1H), 7.40 (d, J=2.0 Hz, 1H), 7.13 (dd, J=5.6, 5.6 Hz, 1H), 6.34 (dd, J=2.1, 5.8 Hz, 1H), 6.16 (s, 1H), 4.35 (d, J=5.8 Hz, 2H), 3.49 - 3.49 (m, 4H), 2.61 - 2.55 (m, 2H), 2.25 (s, 3H), 1.90 - 1.82 (m, 1H), 1.55 (dd, J=7.3, 7.3 Hz, 2H), 0.90 - 0.84 (m, 2H), 0.69 - 0.64 (m, 2H).

### Intermediate 739: 1-(2-(((6-chloropyrimidin-4-yl)amino)methyl)-6-cyclopropylimidazo[1,2-a]pyridin-8-yl)pyrrolidin-2-one

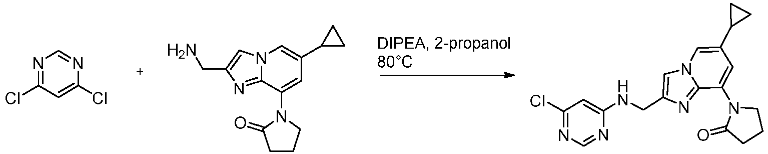

A solution of 2,4-dichloropyrimidine (0.12 g, 0.84 mmol), 1-(2-(aminomethyl)-6-cyclo propylimidazo[1,2-a]pyridin-8-yl)pyrrolidin-2-one (0.25 g, 0.92 mmol) and DIPEA (0.44 mL, 2.52 mmol) in 2-propanol (5 mL) was heated to 70°C for 1 hour. The mixture was cooled to RT, diluted with H2O (20 mL), then extracted with DCM (3 x 10 mL). The combined organics were dried over MgSO₄, then concentrated *in vacuo.* The residue was triturated with diethyl ether to give the crude product which was used without further purification (0.27g, 84 %). ESI-MS (M+H)+: 383, δ 8.35 (s, 1H), 8.30 (d, J=1.0 Hz, 1H), 8.25 (s, 1H), 7.77 (s, 1H), 7.18 (s, 1H), 6.69 (s, 1H), 4.67 - 4.66 (m, 2H), 4.19 (dd, J=7.1, 7.1 Hz, 2H), 2.02 - 1.93 (m, 1H), 1.32 (dd, J=7.2, 7.2 Hz, 4H), 0.98 (ddd, J=4.4, 6.4, 8.4 Hz, 2H), 0.72 - 0.67 (m, 2H).

The compounds in Table **25** were synthesized using a similar procedure to that used for **intermediate** 739 from a suitable di-halo-pyrimidine or pyridine and an appropriate coupling partner. **Table 25**

| **Structure** | **Name/Coupling Partner / Analvtical Data** |
|---|---|
| 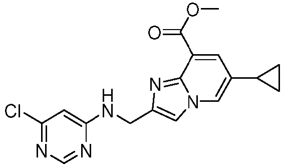 | **methyl 2-(((6-chloropyrimidin-4-yl)amino)methyl)-6-cyclopropylimidazo[1,2-a]pyridine-8-carboxylate** from methyl-2-(((6-chloropyrimidin-4-yl)amino)methyl)-6-cyclopropylimidazo[1,2-*a*]pyridine-8-carboxylate |
| | ESI-MS (M+H)+: 358.2 δ 8.30 - 8.28 (m, 1H), 8.14 (s, 1H), 7.75 (s, 1H), 7.59 (s, 1H), 6.65 - 6.61 (m, 1H), 5.78 - 5.74 (m, 1H), 5.58 - 5.38 (m, 1H), 4.58 - 4.58 (m, 1H), 4.51 - 4.35 (m, 2H), 4.19 - 4.09 (m, 2H), 1.87 - 1.80 (m, 1H), 0.90 - 0.84 (m, 2H), 0.68 - 0.62 (m, 2H). |
| 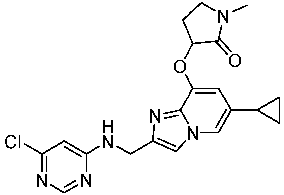 | **3-((2-(((6-chloropyrimidin-4-yl)amino)methyl)-6-cyclopropylimidazo[1,2-a]pyridin-8-yl)oxy)-1-methylpyrrolidin-2-one from intermediate 475** |
| | ESI-MS (M+H)+: 413.2 |
| 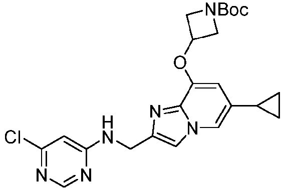 | ***tert*-butyl 3-((2-(((6-chloropyrimidin-4-yl)amino)methyl)-6-cyclopropylimidazo[1,2-a]pyridin-8-yl)oxy)azetidine-1-carboxylate** from *tert-butyl* 3-((2-(((6-chloropyrimidin-4-yl)amino)methyl)-6-cyclopropylimidazo[1,2-a]pyridin-8-yl)oxy)azetidine-1-carboxylate ¹H NMR (400 MHz, CDCl₃). δ, ppm, 8.39 (1H, s), 8.04 (dd, J = 0.7, 1.8 Hz, 1H), 7.75 (d, J = 1.8 Hz, 1H), 7.51 (s, 1H), 6.43 (d, J = 0.9 Hz, 1H), 6.14 (br s, 1H), 4.75 (br s, 2H), 4.03 (s, 3H), 1.97 - 1.90 (m, 1H), 1.05 - 1.00 (m, 2H), 0.75 - 0.69 (m, 2H). |
| 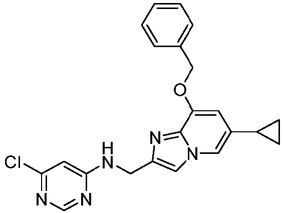 | ***N*-((8-(benzyloxy)-6-cyclopropylimidazo[1,2-a]pyridin-2-yl)methyl)-6-chloropyrimidin-4-amine intermediate 744** from intermediate 471 |
| | ESI-MS (M+H)+: 406.3 ¹H NMR (400 MHz, CDCl₃) δ 8.38 (s, 1H), 7.55 (s, 1H), 7.39 (s, 1H), 6.75 (s, 1H), 6.42 (s, 1H), 6.04 - 6.03 (m, 1H), 5.24 - 5.19 (m, 1H), 4.65 (s, 2H), 3.63 - 3.56 (m, 1H), 3.44 - 3.37 (m, 1H), 2.93 (s, 3H), 2.65 - 2.55 (m, 1H), 2.47 - 2.38 (m, 1H), 1.90 - 1.83 (m, 1H), 0.96 - 0.91 (m, 2H), 0.70 - 0.66 (m, 2H). |
| 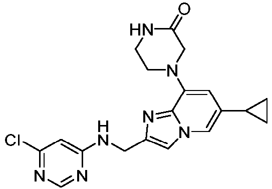 | **Intermediate 745: 4-(2-(((6-chloropyrimidin-4-yl)amino)methyl)-6-cyclopropylimidazo [1,2-*a*]pyridin-8-yl)piperazin-2-one** from intermediate 323ESI-MS (M+H)+: 398.3 δ 8.30 (s, 1H), 8.21 (s, 1H), 7.97 (s, 1H), 7.69 (s, 1H), 6.63 (s, 1H), 6.16 (s, 1H), 4.60 - 4.60 (m, 2H), 4.36 (dd, J=7.2, 7.2 Hz, 2H), 3.86 (dd, J=2.6, 9.3 Hz, 2H), 1.41 (s, 9H), 0.94 - 0.87 (m, 2H), 0.71 - 0.66 (m, 2H). |
| 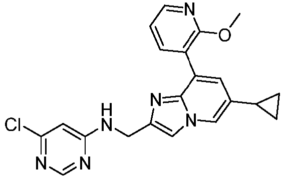 | **4-(2-(((6-chloropyrimidin-4-yl)amino)methyl)-6-cyclopropylimidazo[1,2-*a*]pyridin-8-yl)piperazin-2-one** from intermediate 478 |
| | ESI-MS (M+H)+: 406.3 |
| 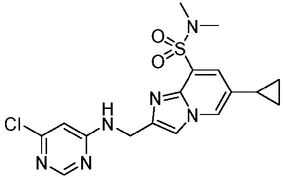 | **2-(((6-chloropyrimidin-4-yl)amino)methyl)-6-cyclopropyl-*N,N-*dimethylimidazo[1,2-*a*]pyridine-8-sulfonamide** from **intermediate 567** |
| | δ 8.34 (s, 1H), 8.24 (s, 1H), 7.99 (s, 1H), 7.72 (s, 1H), 7.57 - 7.53 (m, 2H), 7.50 - 7.41 (m, 3H), 6.67 (s, 1H), 6.52 (d, J=1.0 Hz, 1H), 5.30 (s, 2H), 4.63 - 4.62 (m, 2H), 1.97 - 1.89 (m, 1H), 0.97 - 0.91 (m, 2H), 0.75 - 0.69 (m, 2H). |
| 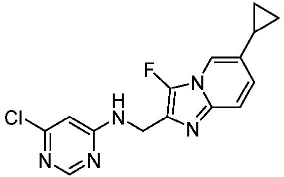 | **Intermediate 746: 6-chloro-*N*-((6-cyclopropyl-3-fluoroimidazo[1,2-*a*]pyridin-2-yl)methyl)pyrimidin-4-amine** from (6-cyclopropyl-3-fluoroimidazo[1,2-a]pyridin-2-yl)methanamine |
| | ESI-MS (M+H)+: 318.4 |
| 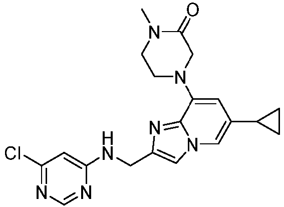 | **Intermediate 747: 4-(2-(((6-chloropyrimidin-4-yl)amino)methyl)-6-cyclopropylimidazo [1,2-*a*]pyridin-8-yl)-1-methylpiperazin-2-one** from 4 4-(2-(aminomethyl)-6-cyclopropylimidazo[1,2-a]pyridin-8-yl)-1-methylpiperazin-2-one |
| | ESI-MS (M+H)+: 412.4 |
| 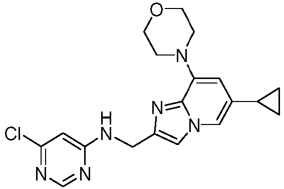 | **Intermediate 748: 6-chloro-*N*-((6-cyclopropyl-8-morpholinoimidazo[1,2-*a*]pyridin-2-yl)methyl)pyrimidin-4-amine** from (6-cyclopropyl-8-morpholinoimidazo[1,2-*a*]pyridin-2-yl)methanamine |
| | ESI-MS (M+H)+: 385.1 |
| 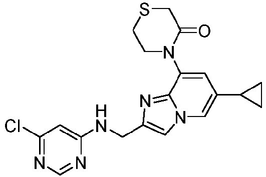 | **Intermediate 749: 4-(2-(((6-chloropyrimidin-4-yl)amino)methyl)-6-cyclopropylimidazo[1,2-*a*]pyridin-8-yl)thiomorpholin-3-one** from 4-(2-(aminomethyl)-6-cyclopropylimidazo[1,2-*a*]pyridin-8-yl)thiomorpholin-3-one Table A7 |
| | ESI-MS (M+H)+: 415 |
| 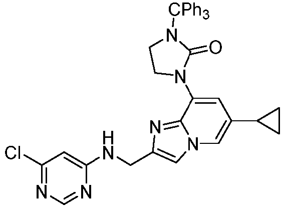 | **Intermediate 787: 1-(2-(((6-chloropyrimidin-4-yl)amino)methyl)-6-cyclo propylimidazo[1,2-*a*]pyridin-8-yl)-3-tritylimidazolidin-2-one from intermediate 326** |
| | ¹H NMR (400 MHz, CDCl₃) δ 8.37 (d, J=9.2 Hz, 1H), 8.22 (dd, J=1.9, 5.0 Hz, 1H), 8.06 (dd, J=1.9, 7.4 Hz, 1H), 7.87 - 7.86 (m, 1H), 7.48 (s, 1H), 7.15 (d, J=1.5 Hz, 1H), 7.05 - 6.99 (m, 1H), 6.41 (s, 1H), 5.97 - 5.97 (m, 1H), 4.63 - 4.63 (m, 2H), 3.95 (s, 3H), 1.97 - 1.89 (m, 1H), 1.02 - 0.96 (m, 2H), 0.73 - 0.68 (m, 2H). |
| 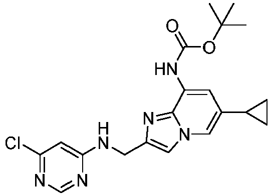 | **Intermediate 740: *tert*-butyl (2-(((6-chloropyrimidin-4-yl)amino)methyl)-6-cyclopropylimidazo[1,2-a]pyridin-8-yl)carbamate** from *tert-*butyl (2-(aminomethyl)-6-cyclopropylimidazo[1,2-*a*]pyridin-8-yl)carbamate |
| | ESI-MS (M+H)+: 414.3 |
| 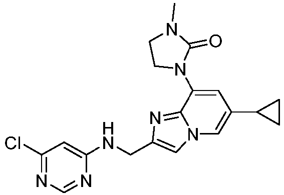 | **Intermediate 750: 1-(2-(((6-chloropyrimidin-4-yl)amino)methyl)-6-cyclo propylimidazo[1,2-*a*]pyridin-8-yl)-3-methylimidazolidin-2-one** from *tert-*butyl (2-(aminomethyl)-6-cyclopropylimidazo[1,2-*a*]pyridin-8-yl)carbamate δ 8.57 (s, 1H), 8.28 (dd, J=6.3, 6.3 Hz, 2H), 7.84 (s, 1H), 7.51 (s, 1H), 6.63 (s, 1H), 4.68 - 4.68 (m, 2H), 2.84 (s, 6H), 2.09 - 2.01 (m, 1H), 1.01 - 0.95 (m, 2H), 0.75 - 0.69 (m, 2H). |
| 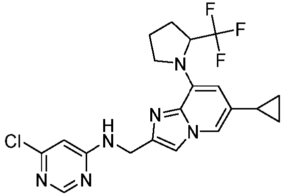 | **Intermediate 751: 6-chloro-*N*-((6-cyclopropyl-8-(2-(trifluoromethyl) pyrrolidin-1-yl)imidazo[1,2-*a*]pyridin-2-yl)methyl) pyrimidin-4-amine** from (6-cyclopropyl-8-(2-(trifluoro methyl)pyrrolidin-1-yl)imidazo[1,2-*a*]pyridin-2-yl) methanamine |
| | ESI-MS (M+H)+: 436.2 |
| 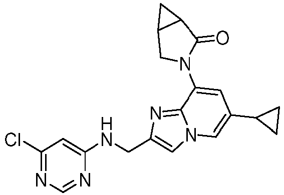 | **Intermediate 809: 3-(2-(((6-chloropyrimidin-4-yl)amino)methyl)-6-cyclo propylimidazo[1,2-*a*]pyridin-8-yl)-3-azabicyclo [3.1.0]hexan-2-one** from 3-(2-(aminomethyl)-6-cyclopropyl imidazo[1,2-*a*]pyridin-8-yl)-3-azabicyclo[3.1.0]hexan-2-one Table A7 |
| | ¹H NMR (400 MHz, CDCl₃) δ 8.40 (s, 1H), 7.66 (s, 1H), 7.35 (d, J=9.6 Hz, 1H), 6.93 (dd, J=1.6, 9.5 Hz, 1H), 6.47 (s, 1H), 5.86 - 5.83 (m, 1H), 4.68 - 4.67 (m, 2H), 1.95 - 1.88 (m, 1H), 1.02 - 0.97 (m, 2H), 0.73 - 0.68 (m, 2H). |
| 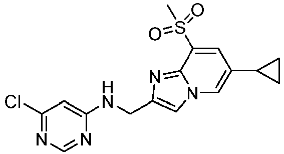 | **Intermediate 741: 6-chloro-*N*-((6-cyclopropyl-8-(methylsulfonyl)imidazo[1,2-*a*]pyridin-2-yl)methyl)pyrimidin-4-amine from intermediate 487** |
| | ESI-MS (M+H)+: 378.3 |
| 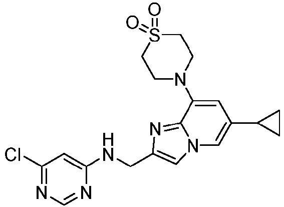 | **4-(2-(((6-chloropyrimidin-4-yl)amino)methyl)-6-cyclopropylimidazo[1,2-*a*]pyridin-8-yl)thiomorpholine 1,1-dioxide** from 4-(2-(aminomethyl)-6-cyclopropylimidazo[1,2-*a*]pyridin-8-yl)thiomorpholine 1,1-dioxide |
| | ESI-MS (M+H)+: 433.3 |
| | Unnumbered list at the beginning of the table |
| 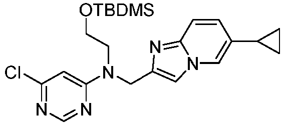 | **Intermediate 811: *N*-(2-((*tert*-butyldimethylsilyl)oxy)ethyl)-6-chloro-*N*-((6-cyclopropylimidazo[1,2-*a*]pyridin-2-yl)methyl)pyrimidin-4-amine** from **intermediate 253** |
| | ESI-MS (M+H)+: 458.4 |
| 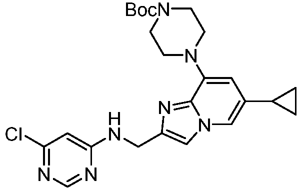 | ***tert*-butyl 4-(2-(((6-chloropyrimidin-4-yl)amino)methyl)-6-cyclopropylimidazo[1,2-*a*]pyridin-8-yl)piperazine-1-carboxylate** from *tert*-butyl4-(2-(aminomethyl)-6-cyclopropylimidazo[1,2-*a*]pyridin-8-yl)piperazine-1-carboxylate |
| | ESI-MS (M+H)+: 484.2 |
| 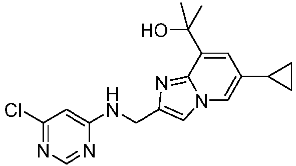 | **Intermediate 752: 2-(2-(((6-chloropyrimidin-4-yl)amino)methyl)-6-cyclopropylimidazo [1,2-*a*]pyridin-8-yl)propan-2-ol** from **intermediate 574** |
| | ESI-MS (M+H)+: 358 |
| 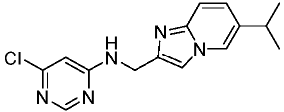 | **6-chloro-*N*-((6-isopropylimidazo[1,2-*a*]pyridin-2-yl)methyl)pyrimidin-4-amine** from **intermediate 256** |
| | ESI-MS (M+H)+: 302.1 |
| 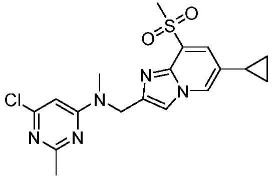 | **Intermediate 753: 6-chloro-N-((6-cyclopropyl-8-(methylsulfonyl)imidazo[1,2-a]pyridin-2-yl)methyl)-N,2-dimethylpyrimidin-4-amine from intermediate 492** |
| | ESI-MS [M +H]⁺:406.2 |
| 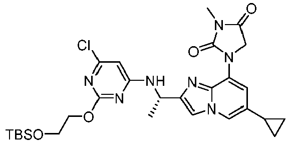 | **(R)-1-(2-(1-((2-(2-((*tert*-butyldimethylsilyl)oxy)ethoxy)-6-chloropyrimidin-4-yl)amino)ethyl)-6-cyclopropyl imidazo [1,2-a]pyridin-8-yl)-3-methylimidazolidine-2,4-dione** from (S)-1-(2-(1-aminoethyl)-6-cyclopropylimidazo[1,2-a]pyridin-8-yl)-3-methylimidazolidine-2,4-dione |
| | ESI-MS [M +H]⁺:600.2 |
| 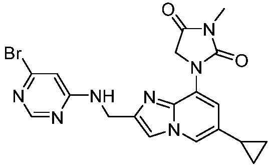 | **Intermediate 754: 1-(2-(((6-bromopyrimidin-4-yl)amino)methyl)-6-cyclopropylimidazo[1,2-a]pyridin-8-yl)-3-methylimidazolidine-2,4-dione** from intermediate 365ESI-MS [M +H]⁺:456.2 |
| 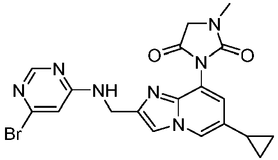 | **Intermediate 755: 3-(2-(((6-bromopyrimidin-4-yl)amino)methyl)-6-cyclopropylimidazo [1,2-a]pyridin-8-yl)-1-methylimidazolidine-2,4-dione** from 3-(2-(aminomethyl)-6-cyclopropylimidazo[1,2-a]pyridin-8-yl)-1-methylimidazolidine-2,4-dione Table A8ESI-MS [M +H]⁺:456.2 |
| 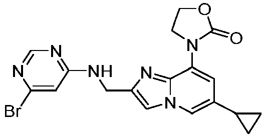 | **Intermediate 756: 3-(2-(((6-bromopyrimidin-4-yl)amino)methyl)-6-cyclo propylimidazo[1,2-a]pyridin-8-yl)oxazolidin-2-one** from 3-(2-(aminomethyl)-6-cyclopropylimidazo[1,2-a]pyridin-8-yl) oxazolidin-2-one ESI-MS [M +H]+:429.2. δ 8.25-8.24 (m, 3H), 7.75 (s, 1H), 7.23 (s, 1H), 6.82 (s, 1H), 4.66 - 4.51 (m, 2H), 4.52 - 4.41 (m, 4H), 1.97 - 1.89 (m, 1H), 0.98 - 0.90 (m, 2H), 0.68 - 0.62 (m, 2H). |
| 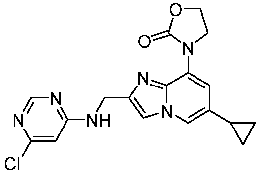 | **Intermediate 757: 3-(2-(((6-chloropyrimidin-4-yl)amino)methyl)-6-cyclopropylimidazo[1,2-a]pyridin-8-yl)oxazolidin-2-one** from 3-(2-(aminomethyl)-6-cyclopropylimidazo[1,2-a] pyridin-8-yl)oxazolidin-2-one |
| | ESI-MS [M +H]⁺:385.2 |
| 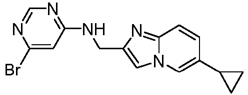 | **Intermediate 758: 6-bromo-N-((6-cyclopropylimidazo[1,2-a]pyridin-2-yl)methyl)pyrimidin-4-amine** from intermediate 251 ESI-MS [M +H]⁺:344.2 |
| 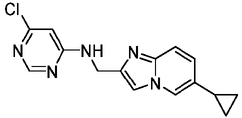 | **6-chloro-N-((6-cyclopropylimidazo[1,2-a]pyridin-2-yl)methyl)pyrimidin-4-amine intermediate 742** from intermediate 251 ESI-MS [M +H]⁺:300.2 |
| 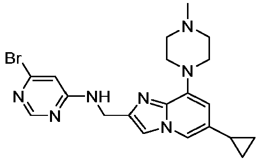 | **6-bromo-N-((6-cyclopropyl-8-(4-methylpiperazin-1-yl)imidazo[1,2-a]pyridin-2-yl)methyl)pyrimidin-4-amine intermediate 743** from (6-cyclopropyl-8-(4-methylpiperazin-1-yl)imidazo[1,2-a]pyridin-2-yl)methanamine |
| | ESI-MS [M +H]⁺:442.2 |
| 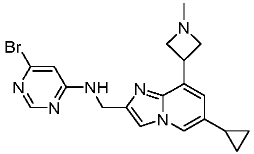 | **Intermediate 759: 6-bromo-N-((6-cyclopropyl-8-(1-methylazetidin-3-yl)imidazo[1,2-a]pyridin-2-yl)methyl) pyrimidin-4-amine** from (6-cyclopropyl-8-(1-methylazetidin-3-yl)imidazo[1,2-a]pyridin-2-yl)methanamine |
| | ESI-MS [M +H]⁺:413.2 |
| 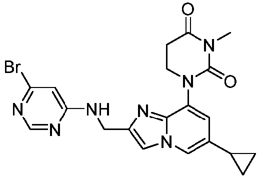 | **Intermediate 760: 1-(2-(((6-bromopyrimidin-4-yl)amino)methyl)-6-cyclo propylimidazo[1,2-a]pyridin-8-yl)-3-methyldihydro pyrimidine-2,4(1H,3H)-dione** from 1-(2-(aminomethyl)-6-cyclopropylimidazo[1,2-a]pyridin-8-yl)-3-methyl dihydropyrimidine-2,4(1H,3H)-dione ESI-MS [M +H]⁺:470.2 |
| 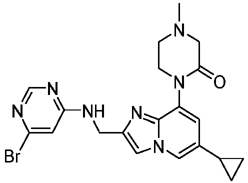 | **Intermediate 761: 1-(2-(((6-bromopyrimidin-4-yl)amino)methyl)-6-cyclopropylimidazo[1,2-a]pyridin-8-yl)-4-methylpiperazin-2-one** from intermediate 330 |
| | ESI-MS [M +H]⁺:456.2 |
| 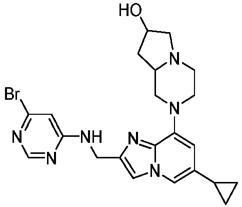 | **Intermediate 762: 2-(2-(((6-bromopyrimidin-4-yl)amino)methyl)-6-cyclopropylimidazo[1,2-a]pyridin-8-yl)octahydropyrrolo[1,2-a]pyrazin-7-ol** from 2-(2-(aminomethyl)-6-cyclopropylimidazo[1,2-a]pyridin-8-yl)octahydropyrrolo[1,2-a]pyrazin-7-ol |
| | ESI-MS [M +H]⁺:484.2 |
| 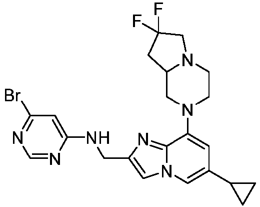 | **Intermediate 763: 6-bromo-N-((6-cyclopropyl-8-(7,7-difluorohexahydro pyrrolo[1,2-a]pyrazin-2(1H)-yl)imidazo[1,2-a]pyridin-2-yl)methyl)pyrimidin-4-amine** from (6-cyclopropyl-8-(7,7-difluorohexahydropyrrolo[1,2-a]pyrazin-2(1H)-yl)imidazo[1,2-a]pyridin-2-yl)methanamine |
| | ESI-MS [M +H]⁺:504.2 |
| 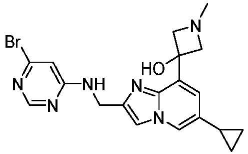 | **Intermediate 764: 3-(2-(((6-bromopyrimidin-4-yl)amino)methyl)-6-cyclopropylimidazo[1,2-a]pyridin-8-yl)-1-methylazetidin-3-ol** from 3-(2-(aminomethyl)-6-cyclopropylimidazo[1,2-a]pyridin-8-yl)-1-methylazetidin-3-ol |
| | ESI-MS [M +H]⁺:429.2 |
| 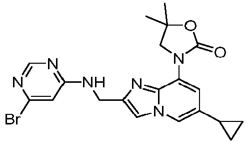 | **Intermediate 765**: **3-(2-(((6-bromopyrimidin-4-yl)amino)methyl)-6-cyclo propylimidazo[1,2-a]pyridin-8-yl)-5,5-dimethyloxazolidin-2-one** from 3-(2-(aminomethyl)-6-cyclopropylimidazo[1,2-a]pyridin-8-yl)-5,5-dimethyloxazolidin-2-one |
| | ESI-MS [M +H]⁺:457.2 |
| 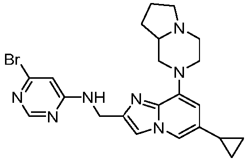 | **Intermediate 766**: **6-bromo-N-((6-cyclopropyl-8-(hexahydropyrrolo[1,2-a]pyrazin-2(1H)-yl)imidazo[1,2-a]pyridin-2-yl)methyl) pyrimidin-4-amine** from (6-cyclopropyl-8-(hexahydro pyrrolo[1,2-a]pyrazin-2(1H)-yl)imidazo[1,2-a]pyridin-2-yl)methanamine ESI-MS [M +H]⁺:468.2 |
| 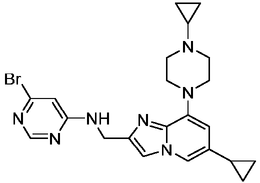 | **Intermediate 767: 6-bromo-N-((6-cyclopropyl-8-(4-cyclopropylpiperazin-1-yl)imidazo[1,2-a]pyridin-2-yl)methyl)pyrimidin-4-amine** from (6-cyclopropyl-8-(4-cyclopropylpiperazin-1-yl) imidazo[1,2-a]pyridin-2-yl)methanamine ESI-MS [M +H]⁺:468.2 |
| 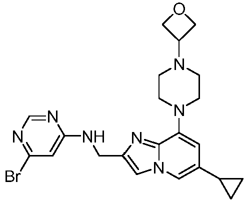 | **Intermediate 768: 6-bromo-N-((6-cyclopropyl-8-(4-(oxetan-3-yl)piperazin-1-yl)imidazo[1,2-a]pyridin-2-yl)methyl)pyrimidin-4-amine** from (6-cyclopropyl-8-(4-(oxetan-3-yl)piperazin-1-yl)imidazo[1,2-a]pyridin-2-yl)methanamine ESI-MS [M +H]⁺:484.2 |
| 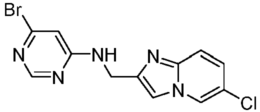 | **Intermediate 769: 6-bromo-N-((6-chloroimidazo[1,2-a]pyridin-2-yl)methyl) pyrimidin-4-amine** from (6-chloroimidazo[1,2-a]pyridin-2-yl)methanamine ESI-MS [M +H]⁺:338.2 |
| 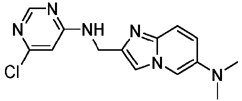 | **2-(((6-chloropyrimidin-4-yl)amino)methyl)-N,N-dimethyl imidazo[1,2-a]pyridin-6-amine** from (6-(N,N-dimethyl amino)-imidazo[1,2-a]pyridin-2-yl)methanamine |
| | ESI-MS [M +H]⁺:303.2 |
| 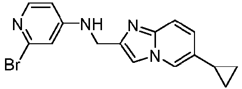 | **Intermediate 770: 2-bromo-N-((6-cyclopropylimidazol1,2-a]pyridin-2-yl)methyl)pyridin-4-amine** from intermediate 251 ESI-MS [M +H]⁺:343.2 |
| 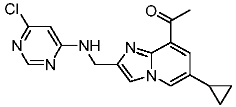 | **1-(2-(((6-chloropyrimidin-4-yl)amino)methyl)-6-cyclopropylimidazo[1,2-a]pyridin-8-yl)ethan-1-one** from 1-(2-(aminomethyl)-6-cyclopropylimidazo[1,2-a]pyridin-8-yl)ethan-1-one ESI-MS [M +H]⁺:342.2 |
| 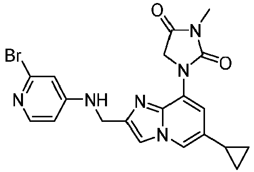 | **Intermediate 772: 1-(2-(((2-bromopyridin-4-yl)amino)methyl)-6-cyclo propylimidazo[1,2-a]pyridin-8-yl)-3-methyl imidazolidine-2,4-dione** from 1-(2-(aminomethyl)-6-cyclopropylimidazo[1,2-a]pyridin-8-yl)-3-methylimidazolidine-2,4-dione ESI-MS [M +H]⁺:455.2 |
| 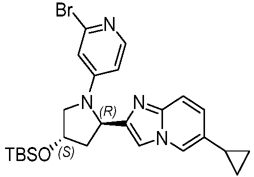 | **2-((2R,4S)-1-(2-bromopyridin-4-yl)-4-((*tert*-butyl dimethylsilyl)oxy)pyrrolidin-2-yl)-6-cyclo propylimidazo[1,2-a]pyridine** from 2-((2R,4S)-4-((*tert*butyldimethylsilyl)oxy)pyrrolidin-2-yl)-6-cyclopropyl imidazo[1,2-a]pyridine ESI-MS [M +H]⁺:513.1 |
| 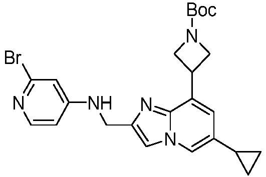 | ***tert*-butyl 3-(2-(((2-bromopyridin-4-yl)amino)methyl)-6-cyclopropylimidazo[1,2-a]pyridin-8-yl)azetidine-1-carboxylate** from *tert-*butyl 3-(2-(aminomethyl)-6-cyclopropylimidazo[1,2-a]pyridin-8-yl)azetidine-1-carboxylate ESI-MS [M +H]⁺:498.1 |
| 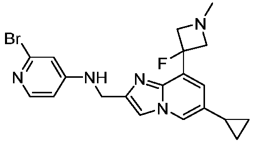 | **Intermediate 773: 2-bromo-N-((6-cyclopropyl-8-(3-fluoro-1-methylazetidin-3-yl)imidazo[1,2-a]pyridin-2-yl)methyl)pyridin-4-amine** from (6-cyclopropyl-8-(3-fluoro-1-methylazetidin-3-yl)imidazo[1,2-a]pyridin-2-yl)methanamine ESI-MS [M +H]⁺:430.1 |
| 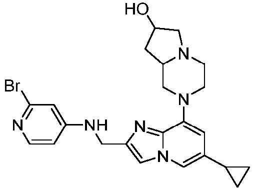 | **Intermediate 774: 2-(2-(((2-bromopyridin-4-yl)amino)methyl)-6-cyclo propylimidazo[1,2-a]pyridin-8-yl)octahydropyrrolo[1,2-a]pyrazin-7-ol** from 2-(2-(aminomethyl)-6-cyclopropylimidazo[1,2-a]pyridin-8-yl)octahydropyrrolo [1,2-a]pyrazin-7-ol ESI-MS [M +H]⁺:483.2 |
| 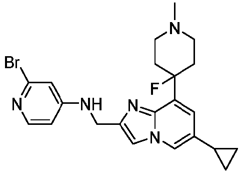 | **Intermediate 775: 2-bromo-N-((6-cyclopropyl-8-(4-fluoro-1-methylpiperidin-4-yl)imidazo[1,2-a]pyridin-2-yl)methyl)pyridin-4-amine** from (6-cyclopropyl-8-(4-fluoro-1-methylpiperidin-4-yl)imidazo[1,2-a]pyridin-2-yl)methanamine ESI-MS [M +H]⁺:458.2 |
| 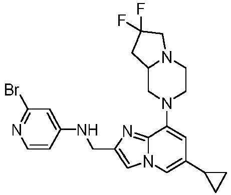 | **Intermediate 776: 2-bromo-N-((6-cyclopropyl-8-(7,7-difluorohexahydro pyrrolo[1,2-a]pyrazin-2(1H)-yl)imidazo[1,2-a]pyridin-2-yl)methyl)pyridin-4-amine** from (6-cyclopropyl-8-(7,7-difluorohexahydropyrrolo[1,2-a]pyrazin-2(1H)-yl)imidazo[1,2-a]pyridin-2-yl)methanamine ESI-MS [M +H]⁺:503.1 |
| 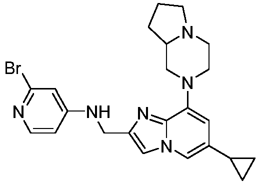 | **Intermediate 777: 2-bromo-N-((6-cyclopropyl-8-(hexahydropyrrolo[1,2-a]pyrazin-2(1H)-yl)imidazo[1,2-a]pyridin-2-yl)methyl)pyridin-4-amine** from (6-cyclopropyl-8-(hexahydropyrrolo[1,2-a]pyrazin-2(1H)-yl)imidazo[1,2-a]pyridin-2-yl)methanamine |
| | ESI-MS [M +H]⁺:467.1 |
| 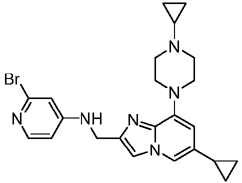 | **Intermediate 778: 2-bromo-N-((6-cyclopropyl-8-(4-cyclopropylpiperazin-1-yl)imidazo[1,2-a]pyridin-2-yl)methyl)pyridin-4-amine** from (6-cyclopropyl-8-(4-cyclopropylpiperazin-1-yl)imidazo[1,2-a]pyridin-2-yl)methanamine ESI-MS [M +H]⁺:467.1 |
| 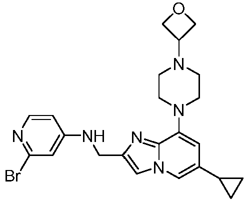 | **Intermediate 779: 2-bromo-N-((6-cyclopropyl-8-(4-(oxetan-3-yl)piperazin-1-yl)imidazo[1,2-a]pyridin-2-yl)methyl)pyridin-4-amine** from (6-cyclopropyl-8-(4-(oxetan-3-yl)piperazin-1-yl)imidazo[1,2-a]pyridin-2-yl)methanamine |
| | ESI-MS [M +H]⁺:483.2 |
| 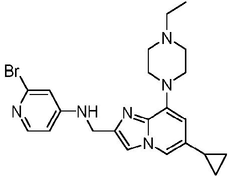 | **Intermediate 780: 2-bromo-N-((6-cyclopropyl-8-(4-ethylpiperazin-1-yl)imidazo[1,2-a]pyridin-2-yl)methyl)pyridin-4-amine** from (6-cyclopropyl-8-(4-ethylpiperazin-1-yl)imidazo[1,2-a]pyridin-2-yl)methanamine ESI-MS [M +H]⁺:455.3 |
| 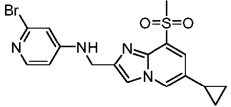 | **Intermediate 781: 2-bromo-N-((6-cyclopropyl-8-(methylsulfonyl)imidazo [1,2-a]pyridin-2-yl)methyl)pyridin-4-amine** from (6-cyclo propyl-8-(methylsulfonyl)imidazo[1,2-a]pyridin-2-yl)methanamine |
| | ESI-MS [M +H]⁺:421.2 |
| 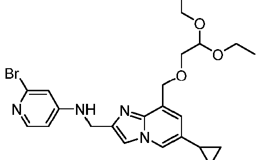 | **2-bromo-N-((6-cyclopropyl-8-((2,2-diethoxyethoxy) methyl)imidazo[1,2-a]pyridin-2-yl)methyl)pyridin-4-amine** from (6-cyclopropyl-8-((2,2-diethoxyethoxy)methyl) imidazo[1,2-a]pyridin-2-yl)methanamine |
| | ESI-MS [M +H]⁺:489.1 |
| 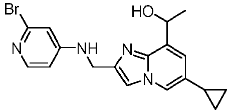 | **Intermediate 782: 1-(2-(((2-bromopyridin-4-yl)amino)methyl)-6-cyclo propylimidazo[1,2-a]pyridin-8-yl)ethan-1-ol** from 1-(2-(aminomethyl)-6-cyclopropylimidazo[1,2-a]pyridin-8-yl)ethan-1-ol ESI-MS [M +H]⁺:387.1 |
| 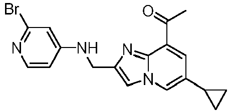 | **Intermediate 771: 1-(2-(((2-bromopyridin-4-yl)amino)methyl)-6-cyclopropylimidazo[1,2-a]pyridin-8-yl)ethan-1-one** from 1-(2-(aminomethyl)-6-cyclopropylimidazo[1,2-a]pyridin-8-yl)ethan-1-one ESI-MS [M +H]⁺:385.1 |
| 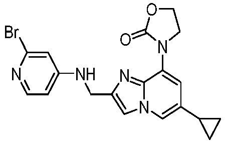 | **Intermediate 783: 3-(2-(((2-bromopyridin-4-yl)amino)methyl)-6-cyclopropylimidazo[1,2-a]pyridin-8-yl)oxazolidin-2-one** from 3-(2-(aminomethyl)-6-cyclopropylimidazo[1,2-a]pyridin-8-yl)oxazolidin-2-one |
| | ESI-MS [M +H]⁺:428.2 |
| 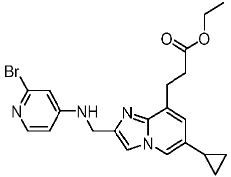 | **Intermediate 784: ethyl 3-(2-(((2-bromopyridin-4-yl)amino)methyl)-6-cyclopropylimidazo[1,2-a]pyridin-8-yl)propanoate** from **Intermediate 273** ESI-MS [M +H]⁺:443.2 |
| 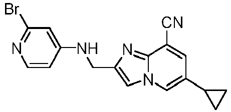 | **2-(((2-bromopyridin-4-yl)amino)methyl)-6-cyclo propylimidazo[1,2-a]pyridine-8-carbonitrile** from 2-(aminomethyl)-6-cyclopropylimidazo[1,2-a]pyridine-8-carbonitrile ESI-MS [M +H]⁺:368.2 |
| 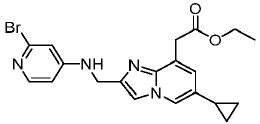 | **Intermediate 785: ethyl 2-(2-(((2-bromopyridin-4-yl)amino)methyl)-6-cyclopropylimidazo[1,2-a]pyridin-8-yl)acetate** from ethyl 2-(2-(aminomethyl)-6-cyclopropylimidazo[1,2-a]pyridin-8-yl)acetate ESI-MS [M +H]⁺:429.1 |
| 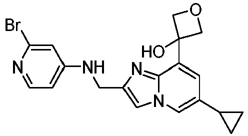 | **3-(2-(((2-bromopyridin-4-yl)amino)methyl)-6-cyclo propylimidazo[1,2-a]pyridin-8-yl)oxetan-3-ol** from **intermediate 278** ESI-MS [M +H]⁺:415.1 |
| 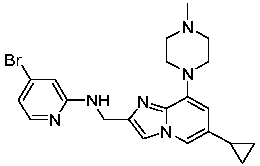 | **Intermediate 786: 4-bromo-N-((6-cyclopropyl-8-(4-methylpiperazin-1-yl)imidazo[1,2-a]pyridin-2-yl) methyl)pyridin-2-amine** from (6-cyclopropyl-8-(4-methylpiperazin-1-yl)imidazo[1,2-a]pyridin-2-yl) methanamine ESI-MS [M +H]⁺:441.2 |
| 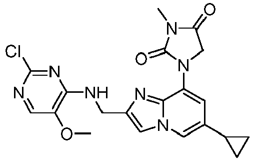 | **1-(2-(((2-chloro-5-methoxypyrimidin-4-yl)amino) methyl)-6-cyclopropylimidazo[1,2-a]pyridin-8-yl)-3-methylimidazolidine-2,4-dione** from intermediate 365 ESI-MS [M +H]⁺:442.2 |
| 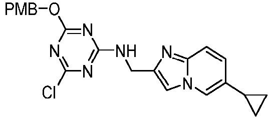 | **4-chloro-N-((6-cyclopropylimidazo[1,2-a]pyridin-2-yl)methyl)-6-((4-methoxybenzyl)oxy)-1,3,5-triazin-2-amine** from intermediate 251 ESI-MS [M +H]⁺:437.2 |
| 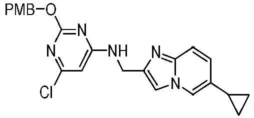 | **6-chloro-N-((6-cyclopropylimidazo[1,2-a]pyridin-2-yl)methyl)-2-((4-methoxybenzyl)oxy)pyrimidin-4-amine** from intermediate 251 ESI-MS [M +H]⁺:436.2 |
| 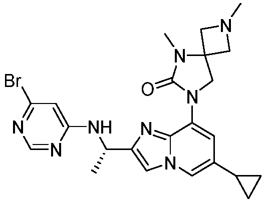 | **(R)-7-(2-(1-((6-bromopyrimidin-4-yl)amino)ethyl)-6-cyclopropylimidazo[1,2-a]pyridin-8-yl)-2,5-dimethyl-2,5,7-triazaspiro[3.4]octan-6-one** from (S)-7-(2-(1-aminoethyl)-6-cyclopropylimidazo[1,2-a]pyridin-8-yl)-2,5-dimethyl-2,5,7-triazaspiro[3.4]octan-6-one |
| | ESI-MS [M +H]⁺:511.1 |
| | Unnumbered list at the beginning of the table |
| 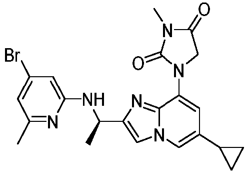 | **(R)-1-(2-(1-((4-bromo-6-methylpyridin-2-yl) amino) ethyl)-6-cyclopropylimidazo[1,2-a] pyridin-8-yl)-3-methylimidazolidine-2,4-dione** from (R)-1-(2-(1-aminoethyl)-6-cyclopropylimidazo[1,2-a]pyridin-8-yl)-3-methyl imidazolidine-2,4-dione ESI-MS [M +H]⁺:483.3 |
| 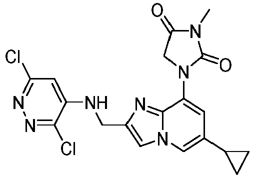 | **Intermediate 788: 1-(6-cyclopropyl-2-(((3,6-dichloropyridazin-4-yl)amino)methyl)imidazo [1,2-a]pyridin-8-yl)-3-methylimidazolidine-2,4-dione** from intermediate 365ESI-MS [M +H]⁺:446.2 |
| 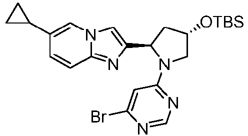 | **Intermediate 789: 2-((2R,4S)-1-(6-bromopyrimidin-4-*yl)-4-((tert-butyl* dimethylsilyl)oxy)pyrrolidin-2-yl)-6-cyclopropyl imidazo[1,2-a]pyridine** from 2-((2R,4S)-4-((*tert*-butyldimethylsilyl)oxy)pyrrolidin-2-yl)-6-cyclopropylimidazo[1,2-a]pyridine ESI-MS [M +H]⁺:514.2 |
| 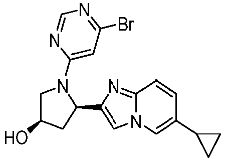 | **Intermediate 790: (3R,5R)-1-(6-bromopyrimidin-4-yl)-5-(6-cyclopropyl imidazo[1,2-a]pyridin-2-yl)pyrrolidin-3-ol** from (3R,5R)-5-(6-cyclopropylimidazo[1,2-a]pyridin-2-yl)pyrrolidin-3-ol ESI-MS [M +H]⁺:400.2 |
| 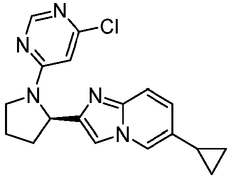 | **Intermediate 791: (R)-2-(1-(6-chloropyrimidin-4-yl)pyrrolidin-2-yl)-6-cyclopropylimidazo[1,2-a]pyridine** from (R)-6-cyclopropyl-2-(pyrrolidin-2-yl)imidazo[1,2-a]pyridine ESI-MS [M +H]⁺:340.2 |
| 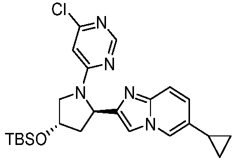 | **2-((2R,4S)-4-((*tert*-butyldimethylsilyl)oxy)-1-(6-chloro pyrimidin-4-yl)pyrrolidin-2-yl)-6-cyclopropyl imidazo[1,2-a]pyridine** from 2-((2R,4S)-4-((*tert-*butyldimethylsilyl)oxy)pyrrolidin-2-yl)-6-cyclopropyl imidazo[1,2-a]pyridine ESI-MS [M +H]⁺:470.2 |
| 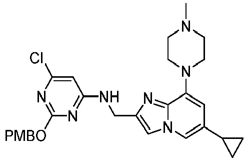 | **6-chloro-N-((6-cyclopropyl-8-(4-methylpip-erazin-1-yl)imidazo[1,2-a]pyridin-2-yl)methyl)-2-((4-methoxybenzyl)oxy)pyrimidin-4-amine** from intermediate 381 ESI-MS [M +H]⁺:534.3 |
| 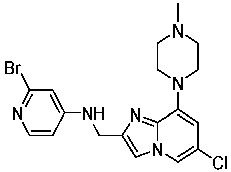 | **2-bromo-N-((6-chloro-8-(4-methylpiperazin-1-yl) imidazo[1,2-a]pyridin-2-yl)methyl)pyridin-4-amine** from intermediate 511 ESI-MS [M +H]⁺:435.2 |
| 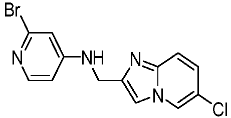 | **2-bromo-N-((6-chloroimidazo[1,2-a]pyridin-2-yl) methyl)pyridin-4-amine** from (6-chloroimidazo[1,2-a]pyridin-2-yl)methanamine ESI-MS [M +H]⁺:337.2 |
| 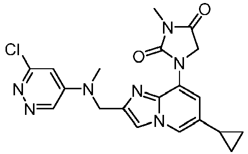 | **1-(2-(((6-chloropyridazin-4-yl)(methyl)amino) methyl)-6-cyclopropylimidazo[1,2-a]pyridin-8-yl)-3-methyl imidazolidine-2,4-dione** from 1-(6-cyclopropyl-2-((methyl amino)methyl)imidazo[1,2-a]pyridin-8-yl)-3-methylimidazolidine-2,4-dione ESI-MS [M +H]⁺:426.2 |
| 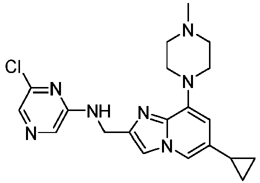 | **Intermediate 793: 6-chloro-N-((6-cyclopropyl-8-(4-methylpiperazin-1-yl)imidazo[1,2-a]pyridin-2-yl)methyl)pyrazin-2-amine** from (6-cyclopropyl-8-(4-methylpiperazin-1-yl)imidazo [1,2-a]pyridin-2-yl)methanamine ESI-MS [M +H]⁺:398.2 |

### Example 144: (1S,2S)-2-(3-chlorophenyl)-N-(6-(((6-cyclopropyl-8-(2-oxopyrrolidin-1-yl) imidazo[1,2-a]pyridin-2-yl)methyl)amino)pyrimidin-4-yl)cyclopropane-1-carboxamide (I-144)

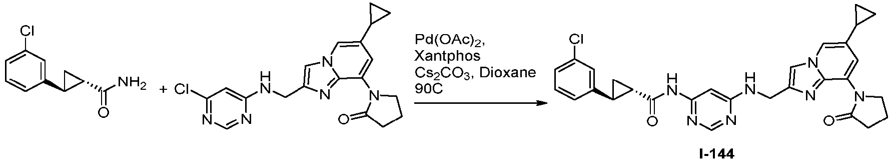

A suspension of **intermediate 739** (0.16 g, 0.70 mmol), intermediate 119 (0.16 g, 0.83 mmol), Pd(OAc)₂ (0.037 g, 0.17 mmol), Xantphos (0.19 g, 0.34 mmol), and Cs₂CO₃ (0.54 g, 1.66 mmol) in 1,4-dioxane (10 mL) was degassed with N₂ for 10 min, then heated to 80 °C for 3 h. The mixture was cooled to room temperature, diluted with water (50 mL), then extracted with EtOAc (3 x 20 mL), then extracted with 10 % MeOH in DCM (3 x 20 mL). The combined organics were dried over MgSO₄, then concentrated *in vacuo.* The residue was purified by silica gel chromatography, eluting with a gradient of DCM - 12 % (7N NH₃ in MeOH) in DCM. This crude product was triturated with diethyl ether to give **(I-144)** (0.15 g, 38 %). ESI-MS (M+H)+: 542.4, ¹H NMR (400 MHz, DMSO) δ 10.65 (s, 1H), 8.29 (s, 1H), 8.25 (s, 1H), 7.93 (s, 1H), 7.70 (s, 1H), 7.40 - 7.29 (m, 4H), 7.23 - 7.16 (m, 2H), 4.62 - 4.61 (m, 2H), 4.20 (dd, J=6.9, 6.9 Hz, 2H), 2.56 (dd, J=1.6, 1.6 Hz, 9H), 2.54 - 2.50 (m, 2H), 2.49 - 2.40 (m, 2H), 2.22 - 2.13 (m, 2H), 2.01 - 1.93 (m, 1H), 1.56 - 1.42 (m, 2H), 1.01 - 0.94 (m, 2H), 0.72 - 0.66 (m, 2H).

Using a similar procedure to that used for ((**I-44**), the compounds in Table **26** were prepared using a cyclopropyl carboxamide and an appropriate coupling partner, then purified by preparative LCMS. **Table 26**

| | **Compound** | **Coupling Partner / Analytical Data** |
|---|---|---|
| **145** | 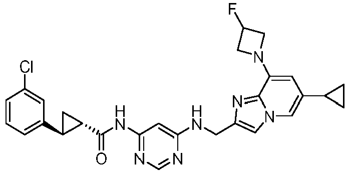 *(*1*S*,2*S)*-2-(3-chlorophenyl)-*N*-(6-(((6-cyclopropyl-8-(3-fluoroazetidin-1-yl)imidazo[1,2-*a*]pyridin-2-yl)methyl)amino)pyrimidin-4-yl)cyclopropane-1-carboxamide **(I-145)** | **Intermediate 792** |
| | | ESI-MS (M+H)+: 532.4, δ 10.59 (s, 1H), 8.19 (s, 1H), 7.80 (br s, 1H), 7.72 (d, J=0.9 Hz, 1H), 7.50 (s, 1H), 7.34 - 7.25 (m, 4H), 7.17 - 7.12 (m, 1H), 5.73 - 5.71 (m, 1H), 5.57 - 5.37 (m, 1H), 4.52 - 4.39 (m, 4H), 4.19 - 4.07 (m, 2H), 2.44 - 2.32 (m, 2H), 1.85 - 1.77 (m, 1H), 1.50 - 1.36 (m, 2H), 0.88 - 0.81 (m, 2H), 0.66 - 0.60 (m, 2H). |
| **146** | 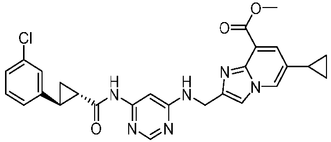 methyl 2-(((6-(*(*1*S*,2*S)*-2-(3-chlorophenyl)cyclopropane-1-carboxamido)pyrimidin-4-yl)amino)methyl)-6-cyclopropylimidazo[1,2-*a*]pyridine-8-carboxylate **(I-146)** | Methyl-2-(((6-chloropyrimidin-4-yl)amino) methyl)-6-cyclopropylimidazo[1,2*-a*] pyridine-8-carboxylate |
| | | ESI-MS (M+H)+: 517.3, δ 10.62 (s, 1H), 8.57 (d, J=1.3 Hz, 1H), 8.21 (s, 1H), 7.96 (br s, 1H), 7.73 (s, 1H), 7.60 (d, J=1.4 Hz, 1H), 7.36 - 7.26 (m, 4H), 7.17 (d, J=8.2 Hz, 1H), 4.62 (br s, 2H), 3.91 (s, 3H), 2.41 - 2.40 (m, 2H), 2.01 - 2.01 (m, 1H), 1.49 - 1.48 (m, 1H), 1.42 - 1.42 (m, 1H), 0.97 - 0.96 (m, 2H), 0.71 - 0.71 (m, 2H). |
| **147** | 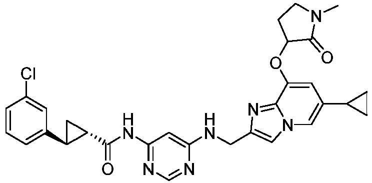 *(*1*S*,2*S)*-2-(3-chlorophenyl)-*N*-(6-(((6-cyclopropyl-8-((1-methyl-2-oxo pyrrolidin-3-yl)oxy)imidazo[1,2-a]pyridin-2-yl)methyl)amino) pyrimidin-4-yl)cyclopropane-1-carboxamide **(I-147)** | 3-((2-(((6-chloropyrimidin-4-yl)amino)methyl)-6-cyclopropylimidazo[1,2-*a*]pyridin-8-yl)oxy)-1-methylpyrrolidin-2-one |
| | | ESI-MS (M+H)+: 572.4, δ 10.59 (s, 1H), 8.21 (s, 1H), 7.98 (s, 1H), 7.89 (s, 1H), 7.61 (s, 1H), 7.35 - 7.30 (m, 1H), 7.27 (dd, J=1.8, 3.6 Hz, 3H), 7.16 (d, J=7.5 Hz, 1H), 6.54 (s, 1H), 5.35 (dd, J=7.3, 7.3 Hz, 1H), 4.55 - 4.55 (m, 2H), 3.46 (ddd, J=2.9, 8.3, 10.3 Hz, 1H), 3.40 - 3.35 (m, 1H), 2.83 (s, 3H), 2.63 - 2.54 (m, 1H), 2.45 - 2.35 (m, 2H), 2.06 - 1.85 (m, 2H), 1.51 - 1.38 (m, 2H), 0.93 - 0.89 (m, 2H), 0.70 - 0.67 (m, 2H). |
| **148** | 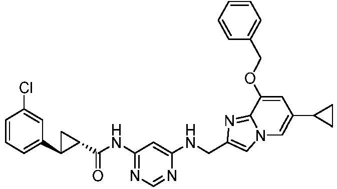 *(*1*S*,2*S)*-*N*-(6-(((8-(benzyloxy)-6-cyclopropylimidazo[1,2-*a*]pyridin-2-yl)methyl)amino)pyrimidin-4-yl)-2-(3-chlorophenyl)cyclopropane-1-carboxamide **(I-148)** | **Intermediate 744 Table 25** |
| | | ESI-MS (M+H)+: 565.6, δ 10.59 (s, 1H), 8.21 - 8.19 (m, 1H), 7.96 - 7.94 (m, 1H), 7.88 (br s, 1H), 7.60 (s, 1H), 7.53 - 7.50 (m, 2H), 7.46 - 7.30 (m, 4H), 7.28 - 7.26 (m, 3H), 7.18 - 7.14 (m, 1H), 6.46 (d, J=1.1 Hz, 1H), 5.27 (s, 2H), 4.54 (br s, 2H), 2.45 - 2.35 (m, 2H), 1.92 - 1.85 (m, 1H), 1.51 - 1.38 (m, 2H), 0.93 - 0.87 (m, 2H), 0.71 - 0.66 (m, 2H). |
| **149** | 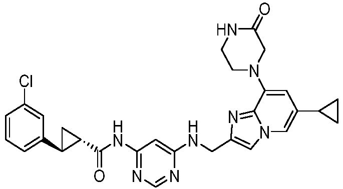 *(*1*S*,2*S)*-2-(3-chlorophenyl)-*N*-(6-(((6-cyclopropyl-8-(3-oxopiperazin-1-yl)imidazo[1,2-*a*]pyridin-2-yl)methyl) amino)pyrimidin-4-yl)cyclopropane-1-carboxamide **(I-149)** | 4-(2-(((6-chloropyrimidin-4-yl)amino)methyl)-6-cyclopropylimidazo[1,2-*a*]pyridin-8-yl)piperazin-2-**oneIntermediate 745 table 25** |
| | | ESI-MS (M+H)+: 557.4, δ 10.58 (s, 1H), 8.19 (s, 1H), 8.02 - 7.99 (m, 1H), 7.90 - 7.85 (m, 2H), 7.54 (s, 1H), 7.34 - 7.23 (m, 4H), 7.17 - 7.13 (m, 1H), 6.17 (s, 1H), 4.54 (br s, 2H), 4.00 (s, 2H), 3.83 (t, J=4.9 Hz, 2H), 2.43 - 2.32 (m, 2H), 1.91 - 1.82 (m, 1H), 1.50 - 1.36 (m, 2H), 0.90 - 0.84 (m, 2H), 0.70 - 0.64 (m, 2H). One piperazinone CH2 signal obscured by H2O signal. |
| **150** | 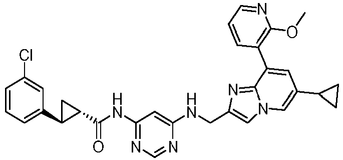 *(*1S,2*S)*-2-(3-chlorophenyl)-*N*-(6-(((6-cyclopropyl-8-(2-methoxypyridin-3-yl)imidazo[1,2-*a*]pyridin-2-yl)methyl) amino)pyrimidin-4-yl)cyclopropane-1-carboxamide **(I-150)** | 6-chloro-*N*-((6-cyclopropyl-8-(2-methoxy pyridin-3-yl)imidazo[1,2-*a*]pyridin-2-yl)methyl)pyrimidin-4-amine |
| | | ESI-MS (M+H)+: 566.4, δ 10.60 (s, 1H), 8.33 (d, J=1.5 Hz, 1H), 8.24 (dd, J=1.9, 5.0 Hz, 1H), 8.20 (s, 1H), 8.05 (d, J=6.1 Hz, 1H), 7.88 (s, 1H), 7.68 (s, 1H), 7.36 - 7.31 (m, 1H), 7.27 (d, J=5.3 Hz, 3H), 7.18 - 7.11 (m, 2H), 7.09 (s, 1H), 4.54 - 4.53 (m, 2H), 3.85 (s, 3H), 2.44 - 2.34 (m, 3H), 1.99 - 1.92 (m, 1H), 1.50 - 1.39 (m, 2H), 0.97 - 0.92 (m, 2H), 0.73 - 0.68 (m, 2H). |
| **151** | 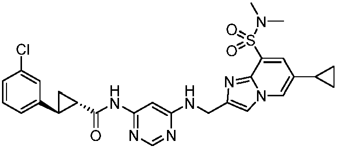 (1*S*,2*S*)-2-(3-chlorophenyl)-*N*-(6-(((6-cyclopropyl-8-(*N,N*-dimethyl sulfamoyl)imidazol[1,2-*a*]pyridin-2-yl)methyl)amino)pyrimidin-4-yl)cyclopropane-1-carboxamide (**I-151)** | **Intermediate 567** ESI-MS (M+H)+: 566.3, δ 10.64 (s, 1H), 8.57 - 8.55 (m, 1H), 8.21 - 8.19 (m, 1H), 7.98 (br s , 1H), 7.77 (s, 1H), 7.51 - 7.50(m, 1H), 7.35 - 7.24 (m, 4H), 7.17 - 7.13(m, 1H), 4.62 (br s, 2H), 2.84 (s, 6H), 2.42 - 2.32 (m, 2H), 2.08 -2.00 (m, 1H), 1.48 - 1.38 (m, 2H), 2.08 - 2.00 (m, 1h), 1.48 - 1.38 (m, 2H), 0.99 - 0.93 (m, 2H), 0.73 - 0.68 (m, 2H). |
| **152** | 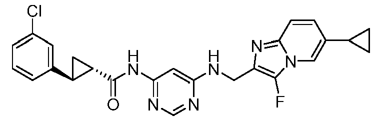 (1*S*,2*S*)-2-(3-chlorophenyl)-*N*-(6-(((6-cyclopropyl-3-fluoroimidazol[1,2-*a*]pyridin-2-yl)methyl)amino)pyrimidin-4-yl)cyclopropane-1-carboxamide **(I-152)** | **Intermediate 746 table 25** ESI-MS (M+H)+: 477.3 δ 10.63 (s, 1H), 8.25 (s, 1H), 8.08 (s, 1H), 7.94 - 7.88 (m, 1H), 7.40 - 7.29 (m, 5H), 7.20 (d, J=7.6 Hz, 1H), 7.00 (dd, J=1.7, 9.4 Hz, 1H), 4.63 (s, 2H), 2.47 - 2.38 (m, 2H), 2.08-2.01 (m, 1H), 1.55 - 1.42 (m, 2H), 1.01 - 0.95 (m, 2H), 0.81 - 0.76 (m, 2H). |
| **153** | 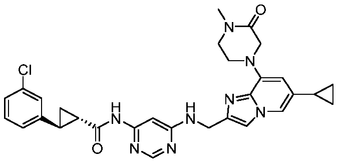 (1*S*,2*S*)-2-(3-chlorophenyl)-*N*-(6-(((6-cyclopropyl-8-(4-methyl-3-oxo piperazin-1-yl)imidazol[1,2-*a*]pyridin-2-yl)methyl)amino)pyrimidin-4-yl)cyclopropane-1-carboxamide **(I-153)** | **Intermediate 747** ESI-MS (M+H)+: 571.4, δ 10.59 (s, 1H), 8.19 (s, 1H), 7.91 - 7.85 (m, 2H), 7.55 (s, 1H), 7.34 - 7.24 (m, 4H), 7.17 - 7.13 (m, 1H), 6.18 (d, J=1.1 Hz, 1h), 4.55 (br s, 2H), 4.07 (s, 2H), 3.89 (dd, J=5.1, 5.1 Hz, 2H), 3.45 (dd, J=5.3, 5.3 Hz, 2H), 2.91 - 2.90 (m, 3H), 2.43 - 2.32 (m, 2H), 1.90 - 1.82 (m, 1H), 1.50 - 1.37 (m, 2H), 0.90 - 0.84 (m, 2H), 0.70 - 0.65 (m, 2H). |
| **154** | 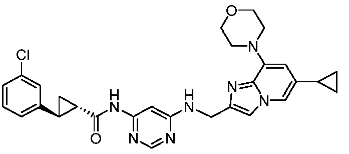 (1*S*,2*S*)-2-(3-chlorophenyl)-*N*-(6-(((6-cyclopropyl-8-morpholinoimidazo[1,2-*a*]pyridin-2-yl)methyl)amino)pyrimidin-4-yl)cyclopropane-1-carboxamide **(I-154)** | **Intermediate 748**table 25 ESI-MS (M+H)+: 544, δ 10.63 (s, 1H), 8.24 (s, 1h), 7.95 - 7.88 (m, 2H), 7.58 (s, 1H), 7.38-7.29 (m, 4H), 7.20 (d, J=7.1 Hz, 1H), 6.21 (s, 1H), 4.58 (s, 2H), 3.87 - 3.84 (m, 4h), 3.55 - 3.49 (m, 4H), 2.47 - 2.43 (m, 2H), 1.93 - 1.87 (m, 1H), 1.54 - 1.44 (m, 2H), 0.94 - 0.90 (m, 2H), 0.74 - 0.68 (m, 2H). |
| **155** | 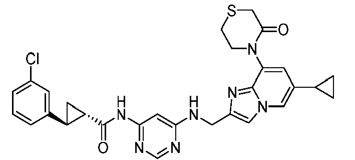 *(*1*S*,2*S)*-2-(3-chlorophenyl)-*N*-(6-(((6-cyclopropyl-8-(3-oxothiomorpholino) imidazo[1,2-*a*]pyridin-2-yl)methyl) amino)pyrimidin-4-yl)cyclopropane-1-carboxamide **(I-155)** | **Intermediate 749 table 25** ESI-MS (M+H)_+: 574.5, δ 10.59 (s, 1H), 8.27 (d, J=1.0 Hz, 1H), 8.19 (s, 1H), 7.90 (br s, 1H), 7.66 (s, 1H), 7.34 - 7.24 (m, 4H), 7.15 (d, J=7.8 Hz, 1H), 6.95 (d, J=1.5 Hz, 1H), 4.56 (br s, 2H), 4.01 (m 2H), 3.46 (s, 2H), 3.16 (m 2H), 2.43 - 2.34 (m, 2H), 1.96 - 1.88 (m, 1H), 1.50 - 1.44 (m, 1H), 1.43 - 1.37 (m, 1H), 0.95 - 0.89 (m, 2H), 0.68 - 0.63 (m, 2H). |
| **156** | 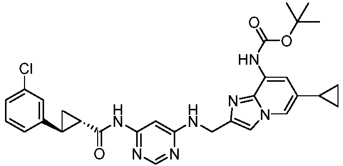 *tert-butyl* (2-(((6-((1*S*,2*S*)-2-(3-chloro phenyl)cyclopropane-1-carboxamido) pyrimidin-4-yl)amino)methyl)-6-cyclo propylimidazo[1,2-*a*]pyridin-8-yl) carbamate **(I-156)** | **Intermediate 740** |
| | | ESI-MS (M+H)+: 574.4, δ 10.65 (s, 1H), 8.29 (s, 1H), 8.25 (s, 1H), 8.04 (s, 1H), 7.89 (s, 1H), 7.68 (s, 1H), 7.40 - 7.18 (m, 7H), 4.62 (br s, 2H), 2.47 - 2.40 (m, 2H), 2.00 - 1.89 (m, 1H), 1.55 (s, 9H), 1.48 - 1.36 (m, 2H), 0.99 - 0.93 (m, 2H), 0.70 - 0.65 (m, 2H). |
| **157** | 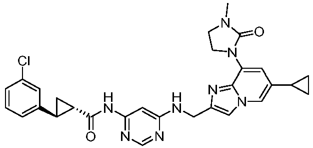 (1*S*,2*S*)-2-(3-chlorophenyl)-*N*-(6-(((6-cyclopropyl-8-(3-methyl-2-oxo imidazolidin-1-yl)imidazo[1,2-*a*]pyridin-2-yl)methyl)amino) pyrimidin-4-yl)cyclopropane-1-carboxamide (**I-157**) | **Intermediate 750 table 25** |
| | | ESI-MS (M+H)+: 557.4, δ 10.60 (s, 1H), 8.19 (s, 1H), 8.13 - 8.11 (m, 1H), 7.85 (br s, 1H), 7.62 (s, 1H), 7.34 - 7.21 (m, 5H), 7.17 - 7.13 (m, 1H), 4.55 (br s, 2H), 4.32 - 4.25 (m, 2H), 3.49 - 3.44 (m, 2H), 2.78 (s, 3H), 2.44 - 2.34 (m, 2H), 1.92 - 1.84 (m, 1H), 1.50 - 1.37 (m, 2H), 0.93 - 0.87 (m, 2H), 0.64 - 0.58 (m, 2H). |
| **158** | 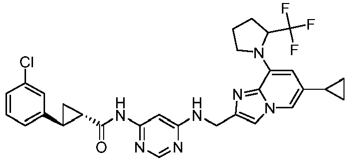 (1*S*,2*S*)-2-(3-chlorophenyl)-*N*-(6-(((6-cyclopropyl-8-(2-(trifluoromethyl) pyrrolidin-1-yl)imidazo[1,2-*a*]pyridin-2-yl)methyl)amino)pyrimidin-4-yl) cyclopropane-1-carboxamide **(I-158)** | **Intermediate 751 table 25**ESI-MS (M+H)+: 596.3, δ 10.59 (s, 1H), 8.18 (s, 1H), 7.79 - 7.77 (m, 2H), 7.52 (s, 1H), 7.33 - 7.29 (m, 2H), 7.28 - 7.24 (m, 2H), 7.15 (d, J=7.6 Hz, 1H), 6.32 - 6.23 (m, 1H), 6.08 (s, 1H), 4.53 - 4.53 (m, 2H), 3.91 - 3.83 (m, 1H), 3.41 - 3.34 (m, 1H), 2.42 - 2.34 (m, 2H), 2.28 - 2.18 (m, 1H), 2.11 - 2.00 (m, 3H), 1.87 - 1.79 (m, 1H), 1.50 - 1.37 (m, 2H), 0.88 - 0.83 (m, 2H), 0.70 - 0.60 (m, 2H). |
| **159** | 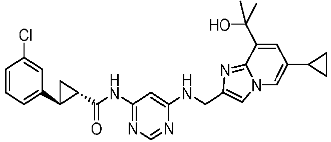 (1*S*,2*S*)-2-(3-chlorophenyl)-*N*-(6-(((6-cyclopropyl-8-(2-hydroxypropan-2-yl) imidazo[1,2-*a*]pyridin-2-yl)methyl) amino)pyrimidin-4-yl)cyclopropane-1-carboxamide **(I-159)** | **Intermediate 752 table 25** |
| | | ESI-MS (M+H)+: 517.4, ¹ δ 10.63 (s, 1H), 8.19 (d, J=9.9 Hz, 2H), 7.89 - 7.89 (m, 1H), 7.58 (s, 1H), 7.36 - 7.30 (m, 2H), 7.28 (d, J=6.4 Hz, 2H), 7.16 (d, J=7.8 Hz, 1H), 7.06 (d, J=1.5 Hz, 1H), 5.53 (s, 1H), 4.59 - 4.59 (m, 2H), 2.45 - 2.34 (m, 2H), 1.96 - 1.88 (m, 1H), 1.66 (s, 6H), 1.50 - 1.39 (m, 2H), 0.95 - 0.89 (m, 2H), 0.69 - 0.63 (m, 2H). |
| **160** | 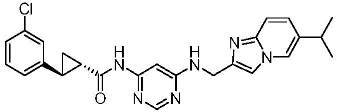 (1S,2S)-2-(3-chlorophenyl)-N-(6-(((6-isopropylimidazo[1,2-*a*]pyridin-2-yl)methyl)amino)pyrimidin-4-yl)cyclopropane-1-carboxamide **(I-160)** | 6-chloro-*N*-((6-isopropylimidazo[1,2-a]pyridin-2-yl)methyl)pyrimidin-4-amine ESI-MS (M+H)+: 461.3, δ 10.58 (s, 1H), 8.32 - 8.30 (m, 1H), 8.20 - 8.18 (m, 1H), 7.85 (br s, 1H), 7.64 (s, 1H), 7.40 (d, J=9.3 Hz, 1H), 7.35 - 7.23 (m, 4H), 7.19 - 7.13 (m, 2H), 4.56 (br s, 2H), 2.93 - 2.82 (m, 1H), 2.44 - 2.34 (m, 2H), 1.50 - 1.37 (m, 2H), 1.22 (d, J=6.8 Hz, 6H). |
| **161** | 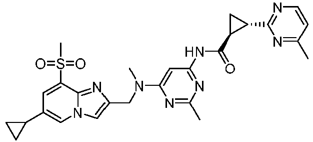 (1S,2S)-N-(6-(((6-cyclopropyl-8-(methylsulfonyl)imidazo[1,2-a] pyridin-2-yl)methyl)(methyl)amino)-2-methylpyrimidin-4-yl)-2-(4-methylpyrimidin-2-yl)cyclopropane-1-carboxamide **(I-161)** | **Intermediate 753 table 25** |
| | | ESI-MS (M+H)+: 547.1, δ 10.80 (s, 1H), 8.60 (d, J = 1.1 Hz, 1H), 8.52 (d, J = 5.1 Hz, 1H), 7.76 (s, 1H), 7.58 (d, J = 1.6 Hz, 1H), 7.28 (s, 1H), 7.20 (d, J = 5.1 Hz, 1H), 4.90 (s, 2H), 3.54 (s, 3H), 3.12 (s, 3H), 2.62 - 2.58 (m, 2H), 2.41 (s, 3H), 2.31 (s, 3H), 2.09 - 2.05 (m, 1H), 1.55 - 1.47 (m, 2H), 1.01 - 0.92 (m, 2H), 0.75 - 0.65 (m, 2H). |
| **162** | 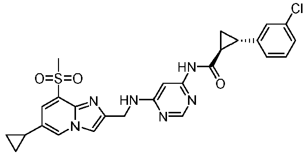 (1S,2S)-2-(3-chlorophenyl)-N-(6-(((6-cyclopropyl-8-(methylsulfonyl) imidazo[1,2-a]pyridin-2-yl)methyl) amino)pyrimidin-4-yl)cyclopropane-1-carboxamide **(I-162)** | **Intermediate 741** |
| | | ESI-MS (M+H)+: 537.1, δ 10.62 (s, 1H), 8.63 (d, J = 1.2 Hz, 1H), 8.20 (s, 1H), 7.99 (s, 1H), 7.81 (s, 1H), 7.56 (d, J = 1.6 Hz, 1H), 7.34 - 7.30 (m, 2H), 7.27 - 7.25 (m, 2H), 7.15 (d, J = 7.6 Hz, 1H), 4.65 (s, 2H), 3.54 (s, 3H), 2.43 - 2.36 (m, 2H), 2.09 - 2.05 (m, 1H), 1.49 - 1.45 (m, 1H), 1.43 - 1.38 (m, 1H), 1.00 - 0.95 (m, 2H), 0.73 - 0.69 (m, 2H). |
| | | ESI-MS [M +H]+: 537.1. |
| **163** | 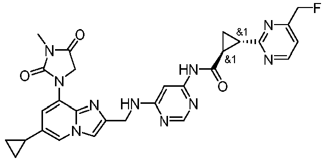 *rac-*(1S**,*2S*)-N-(6-(((6-cyclopropyl-8-(3-methyl-2,4-dioxoimidazolidin-1-yl)imidazo[1,2-a]pyridin-2-yl)methyl) amino)pyrimidin-4-yl)-2-(4-(fluoro methyl)pyrimidin-2-yl)cyclopropane-1-carboxamide **(I-163)** | **Intermediate 754 table 25** |
| | | ESI-MS (M+H)+: 571.2, δ 10.69 (s, 1H), 8.76 (d, J = 5.0 Hz, 1H), 8.21 (d, J = 22.2 Hz, 2H), 7.87 (s, 1H), 7.69 (s, 1H), 7.38 (d, J = 5.0 Hz, 1H), 7.32 (d, J = 11.9 Hz, 1H), 5.53 (s, 1H), 5.42 (s, 1H), 4.91 (s, 2H), 4.57 (s, 2H), 2.97 (s, 3H), 2.68-2.62 (m, 2H), 1.95-1.92 (m, 1H), 1.57-1.53 (m, 2H), 0.96-0.91 (m, 2H), 0.68-0.61 (m, 2H). |
| **164** | 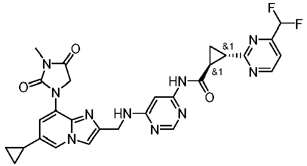 *rac-*(1S**,2*S*)-N-(6-(((6-cyclopropyl-8-(3-methyl-2,4-dioxoimidazolidin-1-yl)imidazo[1,2-a]pyridin-2-yl)methyl) amino)pyrimidin-4-yl)-2-(4-(difluoro methyl)pyrimidin-2-yl)cyclopropane-1-carboxamide **(I-164)** | **Intermediate 754 table 25** |
| | | ESI-MS (M+H)+: 589.2, δ 10.74 (s, 1H), 8.95 (d, J = 5.0 Hz, 1H), 8.22 (d, J = 23.1 Hz, 2H), 7.90 (s, 1H), 7.69 (s, 1H), 7.62 (d, J = 5.0 Hz, 1H), 7.32 (d, J = 8.0 Hz, 2H), 7.08 (s, 0.29H), 6.95 (s, 0.48H), 6.81 (s, 0.27H), 4.91 (s, 2H), 4.58 (s, 2H), 2.97 (s, 3H), 2.76 - 2.60 (m, 2H), 1.98-1.89(m, 1H), 1.66 - 1.48 (m, 2H), 1.05 - 0.84 (m, 2H), 0.77 - 0.50 (m, 2H). |
| **165** | 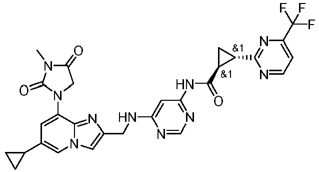 *rac-*(1S*,2S*)-N-(6-(((6-cyclopropyl-8-(3-methyl-2,4-dioxoimidazolidin-1-yl)imidazo[1,2-a]pyridin-2-yl)methyl) amino)pyrimidin-4-yl)-2-(4-(trifluoro methyl)pyrimidin-2-yl)cyclopropane-1-carboxamide **(I-165)** | **Intermediate 754 table 25** |
| | | ESI-MS (M+H)+: 607.2, δ 10.74 (s, 1H), 9.08 (d, J = 5.0 Hz, 1H), 8.24 - 8.19 (m, 2H), 7.90 (s, 1H), 7.86 (d, J = 5.1 Hz, 1H), 7.69 (s, 1H), 7.33 - 7.31 (m, 2H), 4.91 (s, 2H), 4.58 (s, 2H), 2.97 (s, 3H), 2.77 - 2.73 (m, 1H), 2.70 - 2.65 (m, 1H), 1.97 - 1.90 (m, 1H), 1.65 - 1.58 (m, 2H), 0.96 - 0.91 (m, 2H), 0.66 - 0.62 (m, 2H). |
| **166** | 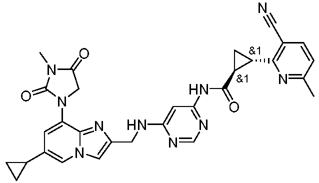 *rac-*(1S***,2S*)-2-(3-cyano-6-methyl *p*yridin-2-yl)-N-(6-(((6-cyclopropyl-8-(3-methyl-2,4-dioxoimidazolidin-1-yl) imidazo[1,2-a]pyridin-2-yl)methyl) amino)pyrimidin-4-yl)cyclopropane-1-carboxamide **(I-166)** | **Intermediate 754 table 25** |
| | | ESI-MS (M+H)+: 577.2, δ 10.71 (s, 1H), 8.24 (d, J = 1.0 Hz, 1H), 8.19 (s, 1H), 8.12 (d, J = 8.0 Hz, 1H), 7.90 (s, 1H), 7.69 (s, 1H), 7.33 - 7.29 (m, 3H), 4.91 (s, 2H), 4.58 (s, 2H), 2.97 (s, 3H), 2.81 - 2.76 (m, 1H), 2.74 - 2.67 (m, 1H), 2.51 (s, 3H), 1.97 - 1.90 (m, 1H), 1.63 - 1.55 (m, 2H), 0.96 - 0.91 (m, 2H), 0.66 - 0.62 (m, 2H). |
| **167** | 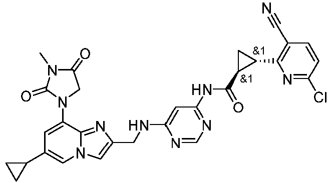 *rac-*(1S*,2S*)-2-(6-chloro-3-cyano pyridin-2-yl)-N-(6-(((6-cyclopropyl-8-(3-methyl-2,4-dioxoimidazolidin- 1-yl)imidazo[1,2-a]pyridin-2-yl)methyl) amino)pyrimidin-4-yl)cyclopropane-1-carboxamide **(I-167)** | **Intermediate 754 table 25** |
| | | ESI-MS (M+H)+: 597.2, δ 10.73 (s, 1H), 8.32 (d, J = 8.3 Hz, 1H), 8.24 - 8.19 (m, 2H), 7.90 (s, 1H), 7.69 (s, 1H), 7.59 (d, J = 8.3 Hz, 1H), 7.33 (d, J = 3.8 Hz, 2H), 4.90 (s, 2H), 4.58 (s, 2H), 2.97 (s, 3H), 2.77 - 2.74 (m, 2H), 1.93 - 1.90 (m, 1H), 1.66 - 1.58 (m, 2H), 0.94 - 0.91 (m, 2H), 0.66 - 0.62 (m, 2H). ESI-MS [M +H]+: 597.1 |
| **168** | 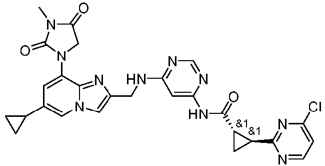 *rac-*(1S**,*2S*)-2-(4-chloropyrimidin-2-yl)-N-(6-(((6-cyclopropyl-8-(3-methyl-2,4-dioxoimidazolidin-1-yl)imidazo[1,2-a]pyridin-2-yl)methyl) amino)pyrimidin-4-yl)cyclopropane-1-carboxamide **(I-168)** | **Intermediate 754 table 25** |
| | | ESI-MS (M+H)+: 573.2, δ 10.71 (s, 1H), 8.68 (d, J = 5.4 Hz, 1H), 8.35 - 8.09 (m, 2H), 7.89 (s, 1H), 7.69 (s, 1H), 7.55 (d, J = 5.3 Hz, 1H), 7.42 - 7.14 (m, 2H), 4.91 (s, 2H), 4.58 (s, 2H), 2.96 (d, J = 10.1 Hz, 3H), 2.77 - 2.62 (m, 1H), 2.62 - 2.53 (m, 1H), 2.03 - 1.78 (m, 1H), 1.67 - 1.38 (m, 2H), 1.06 - 0.77 (m, 2H), 0.76 - 0.51 (m, 2H). |
| 169 | 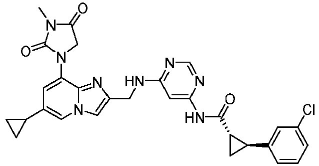 (1S,2S)-2-(3-chlorophenyl)-N-(6-(((6-cyclopropyl-8-(3-methyl-2,4-di oxoimidazolidin-1-yl)imidazo[1,2-a]pyridin-2-yl)methyl)amino) pyrimidin-4-yl)cyclopropane-1-carboxamide **(I-169)** | **Intermediate 754 table 25**ESI-MS (M+H)+: 571.1, |
| | | δ 10.67 (s, 1H), 8.31 (d, J = 1.0 Hz, 1H), 8.25 (s, 1H), 7.96 (s, 1H), 7.75 (s, 1H), 7.39 - 7.31 (m, 5H), 7.21 (d, J = 7.6 Hz, 1H), 4.97 (s, 2H), 4.64 (s, 2H), 3.04 (s, 3H), 2.48 - 2.39 (m, 2H), 2.03 - 1.97 (m, 1H), 1.54 - 1.50 (m, 1H), 1.50-1.45 (d, J = 7.6 Hz, 1H), 1.03 - 0.98 (m, 2H), 0.72 - 0.68 (m, 1H). |
| **170** | 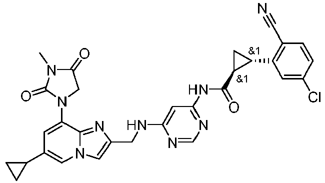 *rac-*(1S**,*2S*)-2-(5-chloro-2-cyano phenyl)-N-(6-(((6-cyclopropyl-8-(3-methyl-2,4-dioxoimidazolidin-1-yl)imidazo[1,2-a]pyridin-2-yl)methyl) amino)pyrimidin-4-yl)cyclopropane-1-carboxamide **(I-170)** | **Intermediate 754 table 25** ESI-MS (M+H)+: 596.1, |
| | | δ 10.67 (s, 1H), 8.24 (d, J = 1.1 Hz, 1H), 8.20 (s, 1H), 7.91 (s, 1H), 7.86 (d, J = 8.3 Hz, 1H), 7.69 (s, 1H), 7.54 - 7.51 (m, 1H), 7.41 (d, J = 1.9 Hz, 1H), 7.33 (d, J = 2.8 Hz, 2H), 4.91 (s, 2H), 4.58 (s, 2H), 2.97 (s, 3H), 2.62 - 2.57 (m, 1H), 2.49 - 2.45 (m, 1H), 1.97 - 1.90 (m, 1H), 1.62 - 1.53 (m, 2H), 0.96 - 0.91 (m, 2H), 0.66 - 0.62 (m, 2H). |
| **171** | 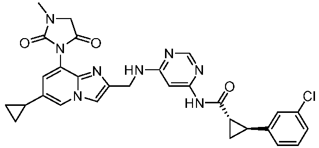 (1S,25)-2-(3-chlorophenyl)-N-(6-(((6-cyclopropyl-8-(3-methyl-2,5-di oxoimidazolidin-1-yl)imidazo[1,2-a]pyridin-2-yl)methyl)amino) pyrimidin-4-yl)cyclopropane-1-carboxamide **(I-171)** | **Intermediate 755 table 25**ESI-MS (M+H)+: 571.3, |
| | | δ 10.57 (s, 1H), 8.16 (s, 1H), 7.79 (s, 1H), 7.48 (d, J = 21.6 Hz, 2H), 7.33 - 7.17 (m, 4H), 7.12 (d, J = 7.6 Hz, 1H), 5.92 (s, 1H), 5.47 (d, J = 8.5 Hz, 1H), 4.51 (s, 2H), 3.09-3.07 (m, 1H), 2.78 - 2.59 (m, 1H), 2.53(s, 3H), 2.40 - 2.27 (m, 1H), 1.47-1.42 (m, 1H), 1.41 - 1.34 (m, 1H), 0.83-0.81 (m, 2H), 0.66 - 0.52 (m, 2H). |
| **172** | 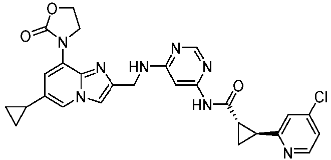 (1S,2S)-2-(4-chloropyridin-2-yl)-N-(6-(((6-cyclopropyl-8-(2-oxooxazolidin-3-yl)imidazo[1,2-a]pyridin-2-yl) methyl)amino)pyrimidin-4-yl)cyclo propane-1-carboxamide **(I-172)** | **Intermediate 756 table 25** |
| | | ESI-MS (M+H)+: 545.2, ¹H NMR (400 MHz, MeOD) δ 8.35 (d, J = 5.4 Hz, 1H), 8.16 (dd, J = 6.5, 0.8 Hz, 2H), 7.70 (s, 1H), 7.44 (d, J = 1.8 Hz, 1H), 7.34 (d, J = 0.9 Hz, 1H), 7.26 (dd, J = 5.4, 2.0 Hz, 1H), 7.21 (d, J = 1.5 Hz, 1H), 4.69 - 4.59 (m, 4H), 4.32-4.28 (m, 2H), 2.63 - 2.58 (m, 1H), 2.47 - 2.43 (m, 1H), 1.97 - 1.93 (m, 1H), 1.64 - 1.59 (m, 2H), 1.00 - 0.94 (m, 2H), 0.75 - 0.71 (m, 2H). |
| **173** | 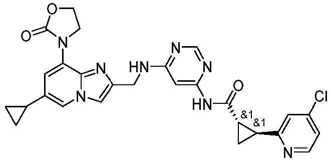 *rac-*(1S*,2S*)-2-(4-chloropyridin-2-yl)-N-(6-(((6-cyclopropyl-8-(2-oxo oxazolidin-3-yl)imidazo[1,2-a]pyridin-2-yl)methyl)amino)pyrimidin-4-yl) cyclopropane-1-carboxamide **(I-173)** | **Intermediate 756 table 25**ESI-MS (M+H)+: 545.2, ¹H NMR (400 MHz, MeOD) δ 8.35 (d, J = 5.4 Hz, 1H), 8.16 (dd, J = 6.5, 0.8 Hz, 2H), 7.70 (s, 1H), 7.44 (d, J = 1.8 Hz, 1H), 7.34 (d, J = 0.9 Hz, 1H), 7.26 (dd, J = 5.4, 2.0 Hz, 1H), 7.21 (d, J = 1.5 Hz, 1H), 4.69 -4.59 (m, 4H), 4.32-4.28 (m, 2H), 2.63 - 2.58 (m, 1H), 2.47 - 2.43 (m, 1H), 1.97 - 1.93 (m, 1H), 1.64 - 1.59 (m, 2H), 1.00 - 0.94 (m, 2H), 0.75 - 0.71 (m, 2H). |
| **174** | 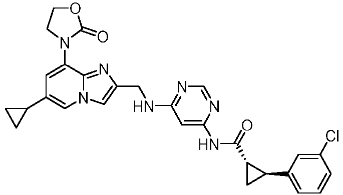 (1S,2S)-2-(3-chlorophenyl)-N-(6-(((6-cyclopropyl-8-(2-oxooxazolidin-3-yl) imidazo[1,2-a]pyridin-2-yl)methyl) amino)pyrimidin-4-yl)cyclopropane-1-carboxamide **(I-174)** | **Intermediate 757 table 25** |
| | | ESI-MS (M+H)+: 544.2, δ 10.61 (s, 1H), 8.24 (d, J = 1.2 Hz, 1H), 8.19 (s, 1H), 7.89 (s, 1H), 7.67 (s, 1H), 7.41 - 6.94 (m, 6H), 4.57 - 4.44 (m, 6H), 2.46 - 2.32 (m, 2H), 1.93 - 1.89 (m, 1H), 1.52 - 1.44 (m, 1H), 1.44 - 1.35 (m, 1H), 0.98 - 0.86 (m, 2H), 0.71 - 0.52 (m, 2H). |
| **175** | 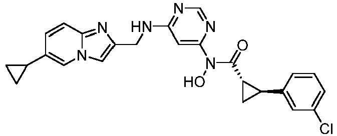 (1S,2S)-2-(3-chlorophenyl)-N-(6-(((6-cyclopropylimidazo[1,2-a]pyridin-2-yl)methyl)amino)pyrimidin-4-yl)-N-hydroxycyclopropane-1-carboxamide **(I-175)** | **Intermediate 758 table 25** |
| | | ESI-MS (M+H)+: 475.1, δ 10.39 (s, 1H), 8.28 (s, 1H), 8.04-7.96 (m, 1H), 7.61 (s, 2H), 7.44 - 7.06 (m, 5H), 6.97-6.92(m, 1H), 5.71 (s, 1H), 4.60-4.49 (m, 2H), 2.60-2.55 (m, 1H), 2.48 - 2.38 (m, 1H), 1.91-1.87 (m, 1H), 1.61-1.42 (m, 2H), 0.92-0.89 (m, 2H), 0.69-0.61 (m, 2H). |
| **176** | 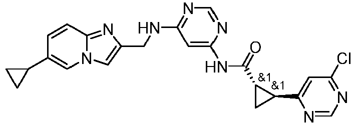 *rac-*(1S*,2S*)-2-(6-chloropyrimidin-4-yl)-N-(6-(((6-cyclopropylimidazo[1,2-a]pyridin-2-yl)methyl)amino) pyrimidin-4-yl)cyclopropane-1-carboxamide **(I-176)** | **Intermediate 758 table 25** |
| | | ESI-MS (M+H)+: 461.1, δ 10.70 (s, 1H), 8.89 (s, 1H), 8.29 (s, 1H), 8.19 (s, 1H), 7.87 (s, 2H), 7.60 (s, 1H), 7.36 (d, J = 9.3 Hz, 1H), 7.26 (s, 1H), 6.97-6.94 (m, 1H), 4.55 (s, 2H), 2.76 - 2.68 (m, 1H), 2.67 - 2.59 (m, 1H), 1.96 - 1.85 (m, 1H), 1.64 - 1.52 (m, 2H), 0.95 - 0.86 (m, 2H), 0.69 - 0.61 (m, 2H). |
| **177** | 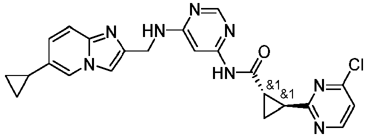 *rac-*(1S*,2S*)-2-(4-chloropyrimidin-2-yl)-N-(6-(((6-cyclopropylimidazo[1,2-a]pyridin-2-yl)methyl)amino) pyrimidin-4-yl)cyclopropane-1-carboxamide **(I-177)** | **Intermediate 758 table 25** |
| | | ESI-MS (M+H)+: 461.1, δ 10.70 (s, 1H), 8.68 (d, J = 5.3 Hz, 1H), 8.30 (s, 1H), 8.19 (s, 1H), 7.87 (s, 1H), 7.61 (s, 1H), 7.55 (d, J = 5.3 Hz, 1H), 7.37 (d, J = 9.3 Hz, 1H), 7.27 (s, 1H), 6.95 (dd, J = 9.3, 1.6 Hz, 1H), 4.57 (s, 2H), 2.80 - 2.63 (m, 1H), 2.64 - 2.50 (m, 11H), 1.90 - 1.87 (m, 1H), 1.59 - 1.54 (m, 2H), 0.99 - 0.79 (m, 2H), 0.77 - 0.51 (m, 2H). |
| **178** | 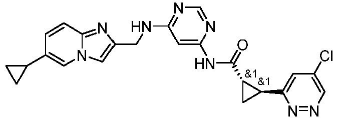 *rac-*(1S*,2S*)-2-(5-chloropyridazin-3-yl)-N-(6-(((6-cyclopropylimidazo[1,2-a]pyridin-2-yl)methyl)amino) pyrimidin-4-yl)cyclopropane-1-carboxamide **(I-178)** | **Intermediate 758 table 25** ESI-MS (M+H)+: 461.1, δ 10.69 (s, 1H), 9.24 (d, J = 2.3 Hz, 1H), 8.30 (s, 1H), 8.20 (s, 1H), 8.04 (d, J = 2.2 Hz, 1H), 7.88 (s, 1H), 7.61 (s, 1H), 7.37 (d, J = 9.3 Hz, 1H), 7.28 (s, 1H), 6.95 (d, J = 9.3 Hz, 1H), 4.57 (s, 2H), 2.78 - 2.64 (m, 2H),1.96 - 1.85 (m, 1H), 1.75 - 1.65 (m, 1H), 1.64 - 1.56 (m, 1H), 0.97 - 0.84 (m, 2H), 0.71 - 0.58 (m, 2H). |
| **179** | 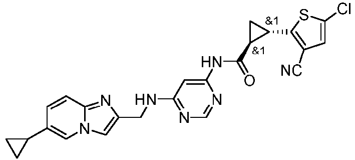 *rac-*(1S*,2S*)-2-(5-chloro-3-cyano thiophen-2-yl)-N-(6-(((6-cyclo propylimidazo[1,2-a]pyridin-2-yl) methyl)amino)pyrimidin-4-yl)cyclo propane-1-carboxamide **(I-179)** | **Intermediate 758 table 25** ESI-MS (M+H)+: 490.1, δ 10.79 (s, 1H), 8.30 (s, 1H), 8.22 (s, 1H), 7.91 (s, 1H), 7.61 (s, 1H), 7.52 (s, 1H), 7.37 (d, J = 9.3 Hz, 1H), 7.27 (s, 1H), 7.01 - 6.93 (m, 1H), 4.57 (s, 2H), 2.73 - 2.65 (m, 2H), 1.94-1.87 (m, 1H), 1.65-1.62 (m, 1H), 1.49-1.44 (m, 1H), 0.96 -0.86 (m, 2H), 0.72 - 0.59 (m, 2H). |
| **180** | 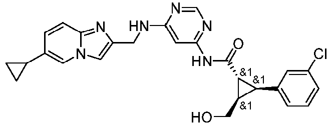 *rac*-(1R*,2S*,3R*)-2-(3-chloro phenyl)-N-(6-(((6-cyclopropyl imidazo[1,2-a]pyridin-2-yl)methyl) amino)pyrimidin-4-yl)-3-(hydroxy methyl)cyclopropane-1-carboxamide **(I-180)** | **Intermediate 758 table 25** ESI-MS (M+H)+: 489.2, δ 10.64 (s, 1H), 8.29 (s, 1H), 8.22 (s, 1H), 7.86 (s, 1H), 7.61 (s, 1H), 7.46 - 7.22 (m, 6H), 7.01-6.92 (m, 1H), 4.65-4.60 (m, 1H), 4.57 (s, 2H), 3.28 - 3.14 (m, 1H), 3.14 - 3.02 (m, 1H), 2.68-2.60 (m, 1H), 2.59-2.50 (m, 1H), 2.00 - 1.78 (m, 2H), 1.02 - 0.80 (m, 2H), 0.70 - 0.60 (m, 2H). |
| **181** | 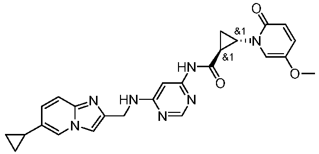 *rac*-(1S*,2S*)-N-(6-(((6-cyclopropyl imidazo[1,2-a]pyridin-2-yl)methyl) amino)pyrimidin-4-yl)-2-(5-methoxy-2-oxopyridin-1(2H)-yl)cyclopropane-1-carboxamide **(I-181)** | **Intermediate 758 table 25** ESI-MS (M+H)+: 472.2, δ 10.70 (s, 1H), 8.30 (s, 1H), 8.22 (s, 1H), 7.90 (s, 1H), 7.61 (s, 1H), 7.38 - 7.29 (m, 3H), 7.07 (d, J = 2.6 Hz, 1H), 6.95 (d, J = 9.1 Hz, 1H), 6.37 (d, J = 9.9 Hz, 1H), 4.58 (s, 2H), 3.67 (s, 3H), 3.54 - 3.49 (m, 1H), 2.35 - 2.29 (m, 1H), 1.94 - 1.87 (m, 1H), 1.76 - 1.71 (m, 1H), 1.50 - 1.45 (m, 1H), 0.93-0.88 (m, 2H), 0.67-0.64 (m,2H). |
| **182** | 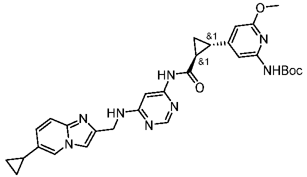 *rac-tert-butyl* (4-((1S*,2S*)-2-((6-(((6-cyclopropylimidazo[1,2-a]pyridin-2-yl)methyl)amino)pyrimidin-4-yl) carbamoyl)cyclopropyl)-6-methoxy pyridin-2-yl)carbamate **(I-182)** | **Intermediate 758 table 25** ESI-MS (M+H)+: 571.3, δ 10.66 (s, 1H), 9.50 (s, 1H), 8.29 (s, 1H), 8.19 (s, 1H), 7.86 (s, 1H), 7.60 (s, 1H ), 7.37 (d, J = 9.3 Hz, 1H), 7.27 (s, 1H), 7.12 (s, 1H), 6.95 (dd, J = 9.3, 1.7 Hz, 1H), 6.29 (d, J = 0.7 Hz, 1H), 4.56 (s, 2H), 3.77 (s, 3H), 2.48 - 2.42 (m, 1H), 2.35 - 2.29 (m, 1H), 1.94 - 1.86 (m, 1H), 1.46 (s, 9H), 1.46 - 1.41 (m, 1H), 1.37 - 1.30 (m, 1H), 0.94 - 0.87 (m, 2H), 0.68 - 0.63 (m, 2H). |
| **183** | 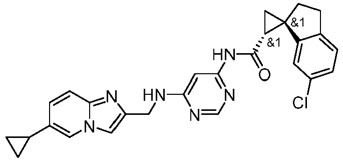 *rac*-(1R*,2R*)-6'-chloro-N-(6-(((6-cyclopropylimidazo[1,2-a]pyridin-2-yl)methyl)amino)pyrimidin-4-yl)-2',3'-dihydrospiro [cyclopropane-1,1'-indene]-2-carboxamide **(I-183)** | **Intermediate 758 table 25** ESI-MS (M+H)+: 485.3, δ 10.49 (s, 1H), 8.30 (s, 1H), 8.17 (s, 1H), 7.84 (s, 1H), 7.61 (s, 1H), 7.37 (d, J = 9.3 Hz, 1H), 7.31 (s, 1H), 7.26 - 7.14 (m, 2H), 6.96 (dd, J = 9.3, 1.6 Hz, 1H), 6.90 (d, J = 1.6 Hz, 1H), 4.57 (s, 2H), 3.05 - 2.72 (m, 2H), 2.48 - 2.41 (m, 1H), 2.15 - 2.10 (m, 2H), 1.90 - 1.85 (m, 1H), 1.56 - 1.51 (m, 2H), 1.04 - 0.83 (m, 2H), 0.67 - 0.64 (m, 2H). |
| **184** | 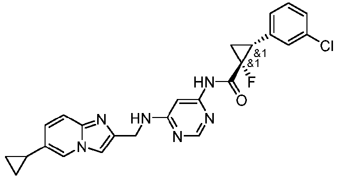 *rac-*(1S*,2S*)-2-(3-chlorophenyl)-N-(6-(((6-cyclopropylimidazo[1,2-a] pyridin-2-yl)methyl)amino)pyrimidin-4-yl)-1-fluorocyclopropane-1-carboxamide **(I-184)** | **Intermediate 758 table 25** ESI-MS (M+H)+: 477.1, δ 10.12 (s, 1H), 8.28 (d, J = 21.0 Hz, 2H), 8.01 (s, 1H), 7.63 (s, 1H), 7.45 (s, 1H), 7.38 - 7.24 (m, 5H), 6.96 (d, J = 9.1 Hz, 1H), 4.60 (s, 2H), 2.95-2.86 (m, 1H), 2.23 - 2.04 (m, 1H), 1.99 - 1.81 (m, 2H), 1.00 - 0.82 (m, 2H), 0.70-0.61 (m, 2H). |
| **185** | 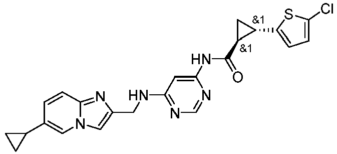 *rac-*(1S*,2S*)-2-(5-chlorothiophen-2-yl)-N-(6-(((6-cyclopropylimidazo[1,2-a]pyridin-2-yl)methyl)amino) pyrimidin-4-yl)cyclopropane-1-carboxamide **(I-185)** | **Intermediate 758 table 25** ESI-MS (M+H)+: 465.2, δ = 10.12 (s, 1H), 8.31 (s, 1H), 8.25 (s, 1H), 8.01 (s, 1H), 7.63 (s, 1H), 7.45 (s, 1H), 7.39-7.30(m, 3H), 6.97 (d, J=9.1, 1H), 4.60 (s, 2H), 3.31 (s, 1H), 2.99-2.84(m, 1H), 2.23 - 2.05 (m, 1H), 2.03 - 1.76 (m, 2H), 0.99 - 0.80 (m, 2H), 0.72-0.58 (m, 2H). |
| **186** | 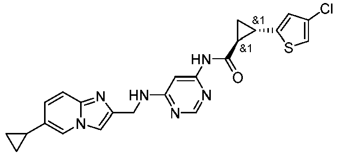 *rac-*(1S**,*2S*)-2-(4-chlorothiophen-2-yl)-N-(6-(((6-cyclopropylimidazo[1,2-a]pyridin-2-yl)methyl)amino) pyrimidin-4-yl)cyclopropane-1-carboxamide **(I-186)** | **Intermediate 758 table 25** ESI-MS (M+H)+: 465.1, δ = 10.68 (s, 1H), 8.30 (s, 1H), 8.20 (s, 1H), 7.88 (s, 1H), 7.60 (s, 1H), 7.41-7.32 (m, 2H), 7.26 (s, 1H), 7.02 - 6.88 (m, 2H), 4.57 (s, 2H), 3.31 - 3.30 (m, 1H), 2.41 - 2.30 (m, 1H), 1.94-1.86 (m, 1H), 1.52-1.45 (m, 1H), 1.38-1.30 (m, 1H), 0.96 - 0.85 (m, 2H), 0.69-0.61 (m, 2H). |
| **187** | 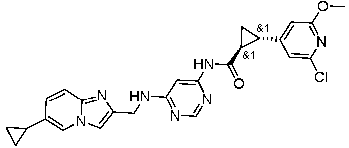 *rac-*(1S*,2S*)-2-(2-chloro-6-methoxypyridin-4-yl)-N-(6-(((6-cyclo propylimidazo[1,2-a]pyridin-2-yl) methyl)amino)pyrimidin-4-yl)cyclo propane-1-carboxamide **(I-187)** | **Intermediate 758 table 25** ESI-MS (M+H)+: 490.2, δ 10.61 (s, 1H), 8.30 (s, 1H), 8.20 (s, 1H), 7.88 (s, 1H), 7.61 (s, 1H), 7.37 (d, J = 9.2 Hz, 1H), 7.25 (s, 1H), 6.99 - 6.91 (m, 2H), 6.68 (s, 1H), 4.57 (s, 2H), 3.82 (s, 3H), 2.48 - 2.44 (m, 1H), 2.42 - 2.37 (m, 1H), 1.96 - 1.86 (m, 1H), 1.55 - 1.47 (m, 2H), 0.95-0.86 (m, 2H), 0.69-0.63 (m,2H). |
| **188** | 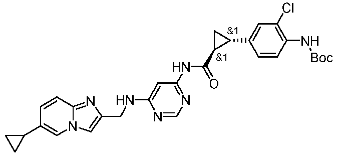 *rac-tert-*butyl (2-chloro-4-((1S*,2S*)-2-((6-(((6-cyclopropylimidazo[1,2-a] pyridin-2-yl)methyl)amino)pyrimidin-4-yl)carbamoyl)cyclopropyl)phenyl) carbamate **(I-188)** | **Intermediate 742** ESI-MS (M+H)+: 574.2, ¹H NMR (400 MHz, DMSO-d6) δ 10.61 (s, 1H), 8.61 (s, 1H), 8.30 (s, 1H), 8.18 (s, 1H), 7.87 (s, 1H), 7.60 (s, 1H), 7.43 (d, J = 8.4 Hz, 1H), 7.37 (d, J = 9.2 Hz, 1H), 7.27 (d, J = 1.9 Hz, 2H), 7.11 - 7.06 (m, 1H), 6.99 - 6.93 (m, 1H), 4.56 (s, 2H), 2.39 - 2.29 (m, 2H), 1.94 - 1.86 (m, 1H), 1.44 (s, 9H), 1.43 - 1.40 (m, 1H), 1.38 - 1.33 (m, 1H), 0.93 - 0.87 (m, 2H), 0.68 - 0.62 (m, 2H). |
| **189** | 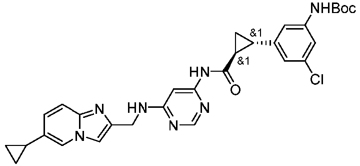 *rac-tert-butyl* (3-chloro-5-((1S*,2S*)-2-((6-(((6-cyclopropylimidazo[1,2-a] pyridin-2-yl)methyl)amino)pyrimidin-4-yl)carbamoyl)cyclopropyl)phenyl) carbamate **(I-189)** | **Intermediate 742** |
| | | ESI-MS (M+H)+: 574.3, δ 10.62 (s, 1H), 9.53 (s, 1H), 8.29 (s, 1H), 8.19 (s, 1H), 7.85 (s, 1H), 7.60 (s, 1H), 7.39 - 7.36 (m, 2H), 7.26 (s, 1H), 7.21 (s, 1H), 6.97-6.94 (m, 1H), 6.84 (s, 1H), 4.56 (s, 2H), 3.32 (s, 11H), 2.51- 2.50 (m, 13H), 2.35-2.30 (m, 2H), 1.93 - 1.88 (m, 1H), 1.47 (s, 9H), 1.45-1.40 (m, 1H), 1.33 - 1.28 (m, 1H), 0.93-0.88 (m, 2H), 0.68 - 0.64 (m, 2H). |
| **190** | 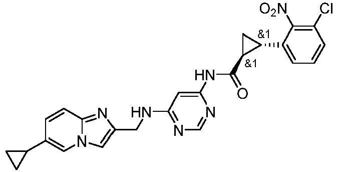 *rac-*(1*S*,2S**)*-*2-(3-chloro-2-nitro phenyl)-N-(6-(((6-cyclopropylimidazo [1,2-a]pyridin-2-yl)methyl)amino) pyrimidin-4-yl)cyclopropane-1-carboxamide **(I-190)** | **Intermediate 742** |
| | | ESI-MS (M+H)+: 504.1, δ 10.68 (s, 1H), 8.31 (s, 1H), 8.21 (s, 1H), 7.89 (s, 1H), 7.71 - 7.57 (m, 3H), 7.43 - 7.33 (m, 2H), 7.26 (s, 1H), 7.00 (d, J = 9.3 Hz, 1H), 4.58 (s, 2H), 2.43 - 2.35 (m, 1H), 2.25 - 2.17 (m, 1H), 1.96 - 1.86 (m, 1H), 1.55 - 1.40 (m, 2H), 0.95 - 0.88 (m, 2H), 0.70 - 0.63 (m, 2H) |
| **191** | 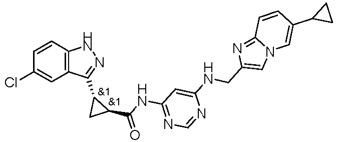 *rac-*(1S**,*2S*)*-*2-(5-chloro-1H-indazol -3-yl)-N-(6-(((6-cyclopropylimidazo [1,2-a]pyridin-2-yl)methyl)amino) pyrimidin-4-yl)cyclopropane-1-carboxamide **(I-191)** | **Intermediate 742** |
| | | ESI-MS (M+H)+: 499.1, δ 8.74 (d, J = 9.1 Hz, 1H), 8.46 (s, 1H), 8.30 (s, 1H), 8.10 (s, 1H), 8.04 (s, 1H), 7.72 (s, 1H), 7.67 (s, 1H), 7.58 (d, J = 8.8 Hz, 1H), 7.38 (d, J = 9.2 Hz, 1H), 7.04 (s, 1H), 7.02 (s, 1H), 6.96 (d, J = 9.3 Hz, 1H), 4.66 (s, 2H), 2.73 - 2.70 (m, 1H), 2.27 - 2.23 (m, 1H), 1.93 - 1.89 (m, 1H), 1.51 - 1.49 (m, 2H), 0.93 - 0.88 (m, 2H), 0.66 - 0.64 (m, 2H). |
| **192** | 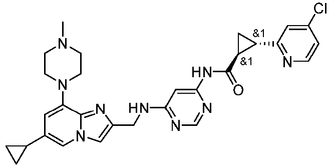 *rac*-(1S*,2S*)-2-(4-chloropyridin-2-yl)-N-(6-(((6-cyclopropyl-8-(4-methyl piperazin-1-yl)imidazo[1,2-a]pyridin-2-yl)methyl)amino)pyrimidin-4-yl)cyclopropane-1-carboxamide **(I-192)** | **Intermediate 743** |
| | | ESI-MS (M+H)+: 558.2, δ 10.63 (s, 1H), 8.42 (d, J = 5.3 Hz, 1H), 8.18 (s, 1H), 7.86 (s, 2H), 7.64 (s, 1H), 7.51 (s, 1H), 7.38 - 7.32 (m, 1H), 7.28 (s, 1H), 6.14 (s, 1H), 4.52 (s, 2H), 3.47 (s, 4H), 3.31 (s, 4H), 2.59-2.58 (m, 2H), 2.23 (s, 3H), 1.88 - 1.81 (m, 1H), 1.52 - 1.47 (m, 2H), 0.88-0.83 (m, 2H), 0.65-0.64 (m, 2H). |
| **193** | 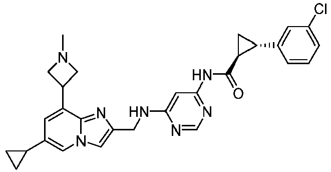 (1S,2S)-2-(3-chlorophenyl)-N-(6-(((6-cyclopropyl-8-(1-methylazetidin-3-yl) imidazo[1,2-a]pyridin-2-yl)methyl) amino)pyrimidin-4-yl)cyclopropane-1-carboxamide **(I-193)** | **Intermediate 759 table 25** |
| | | ESI-MS (M+H)+: 528.2, δ 10.60 (s, 1H), 8.19 (s, 2H), 7.87 (s, 1H), 7.61 (s, 1H), 7.34 - 7.25 (m, 4H), 7.16 - 7.14 (m, 1H), 6.93 (s, 1H), 4.57 (s, 2H), 4.09 - 4.01 (m, 1H), 3.90 - 3.86 (m, 2H), 3.60 - 3.56 (m, 2H), 2.46 (s, 3H), 2.43 - 2.36 (m, 2H), 1.93 - 1.88 (m, 1H), 1.49 - 1.44 (m, 1H), 1.43 - 1.38 (m, 1H), 0.93 - 0.89 (m, 2H), 0.70 - 0.66 (m, 2H). |
| **194** | 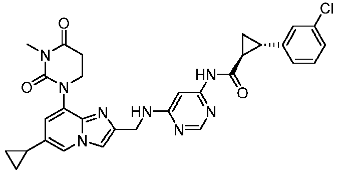 (1S,25)-2-(3-chlorophenyl)-N-(6-(((6-cyclopropyl-8-(3-methyl-2,4-dioxo tetrahydropyrimidin-1(2H)-yl)imidazo [1,2-a]pyridin-2-yl)methyl)amino) pyrimidin-4-yl)cyclopropane-1-carboxamide **(I-194)** | **Intermediate 760 table** 25ESI-MS (M+H)+: 585.2, δ 10.60 (s, 1H), 8.31 (s, 1H), 8.19 (s, 1H), 7.90 (s, 1H), 7.67 (s, 1H), 7.33 - 7.25 (m, 4H), 7.15 (d, J = 7.6 Hz, 1H), 7.03 (s, 1H), 4.56 (s, 2H), 3.83 (t, J = 6.6 Hz, 2H), 3.07 (s, 3H), 2.88 (t, J = 6.6 Hz, 2H), 2.42 - 7.35 (m, 2H), 1.96 - 1.89 (m, 1H), 1.49 - 1.38 (m, 2H), 0.95 - 0.90 (m, 2H), 0.68 - 0.64 (m, 2H). |
| **195** | 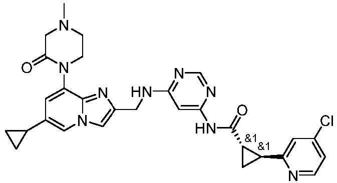 *rac-*(1S*,2S*)-2-(4-chloropyridin-2-yl)-N-(6-(((6-cyclopropyl-8-(4-methyl -2-oxopiperazin-1-yl)imidazo[1,2-a]pyridin-2-yl)methyl)amino) pyrimidin-4-yl)cyclopropane-1-carboxamide **(I-195)** | **Intermediate 761 table 25** |
| | | ESI-MS (M+H)+: 572.2, δ 10.65 (s, 1H), 8.42 (d, J = 5.4 Hz, 1H), 8.29 (s, 1H), 8.18 (s, 1H), 7.92 (s, 1H), 7.64 (s,2H), 7.32-7.26 (m, 1H), 7.28 (s, 1H), 6.94 (d, J = 1.2 Hz, 1H), 4.55 (s, 2H), 3.79-3.70 (m, 2H), 3.15 (s, 2H), 2.79-2.73 ( m, 2H), 2.61-2.52 (m, 2H), 2.32 (s, 3H), 1.98-1.86 (m, 1H), 1.58 - 1.41 (m, 2H), 0.96 - 0.86 (m, 2H), 0.72 - 0.61 (m, 2H). |
| **196** | 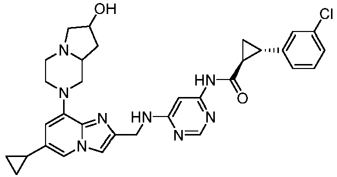 (1S,2S)-2-(3-chlorophenyl)-N-(6-(((6-cyclopropyl-8-(7-hydroxyhexa hydropyrrolo[1,2-a]pyrazin-2(1H)-yl)imidazo[1,2-a]pyridin-2-yl)methyl) amino)pyrimidin-4-yl)cyclopropane-1-carboxamide **(I-196)** | **Intermediate 762 table 25** |
| | | ESI-MS (M+H)+: 599.2, |
| | | ¹H NMR (400 MHz, MeOD) δ 8.28 (s, 1H), 8.10 (s, 1H), 7.86 (s, 1H), 7.29 - 7.04 (m, 6H), 4.81 (s, 2H), 4.76 - 4.69 (m, 1H), 4.16 - 3.71 (m, 4H), 3.69 - 3.41 (m, 4H), 3.29 - 3.10 (m, 2H), 2.74 (s, 1H), 2.53 - 2.48 (m, 1H), 2.20 - 2.16 (m, 1H), 2.05 - 1.98 (m, 2H), 1.66 - 1.61 (m, 1H), 1.46 - 1.36 (m, 1H), 1.07 - 1.02 (m, 2H), 0.82-0.78 (m,2H). |
| **197** | 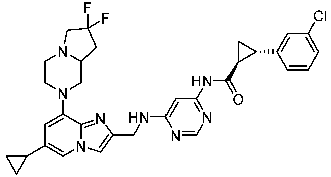 (1S,2S)-2-(3-chlorophenyl)-N-(6-(((6-cyclopropyl-8-(7,7-difluorohexahydro pyrrolo[1,2-a]pyrazin-2(1H)-yl) imidazo[1,2-a]pyridin-2-yl)methyl) amino)pyrimidin-4-yl)cyclopropane-1-carboxamide **(I-197)** | **Intermediate 763 table 25** |
| | | ESI-MS (M+H)+: 619.2, δ 10.60 (s, 1H), 8.19 (s, 1H), 7.88 (s, 1H), 7.85 (s, 1H), 7.52 (s, 1H), 7.29 - 7.25 (m, 4H), 7.19 - 7.14 (m, 1H), 6.18 (s, 1H), 4.54 (s, 2H), 4.41 - 4.33 (m, 2H), 3.46 - 3.41 (m, 2H), 3.03 (d, J = 10.9 Hz, 1H), 2.80 (t, J = 10.7 Hz, 1H), 2.62 - 2.55 (m, 3H), 2.45 - 2.32 (m, 3H), 1.95 - 1.89 (m, 1H), 1.86 - 1.82 (m, 1H), 1.48 - 1.43 (m, 1H), 1.41 - 1.38 (m, 1H), 0.87 - 0.84 (m, 2H), 0.67 - 0.63 (m, 2H). |
| **198** | 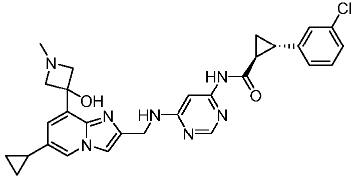 (1S,2S)-2-(3-chlorophenyl)-N-(6-(((6-cyclopropyl-8-(3-hydroxy-1-methyl azetidin-3-yl)imidazo[1,2-a]pyridin-2-yl)methyl)amino)pyrimidin-4-yl) cyclopropane-1-carboxamide **(I-198)** | **Intermediate 764 table 25** |
| | | ESI-MS (M+H)+: 544.2, δ 10.61 (s, 1H), 8.24 (d, J = 1.1 Hz, 1H), 8.19 (s, 1H), 7.88 (s, 1H), 7.63 (s, 1H), 7.43 - 7.35 (m, 1H), 7.35 - 7.23 (m, 3H), 7.15 (d, J = 7.6 Hz, 1H), 7.09 (d, J = 1.6 Hz, 1H), 6.46 (s, 1H), 4.58 (s, 2H), 3.95 (d, J = 7.2 Hz, 2H), 3.50 - 3.38 (m, 2H), 2.45 - 2.28 (m, 5H), 2.00 - 1.91 (m, 1H), 1.55 - 1.43 (m, 1H), 1.43 - 1.36 (m, 1H), 0.98 - 0.87 (m, 2H), 0.69 - 0.64 (m, 2H). |
| **199** | 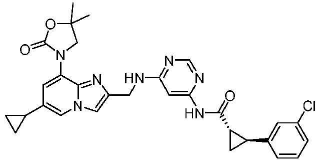 (1S,2S)-2-(3-chlorophenyl)-N-(6-(((6-cyclopropyl-8-(5,5-dimethyl-2-oxo oxazolidin-3-yl)imidazo[1,2-a]pyridin-2-yl)methyl)amino)pyrimidin-4-yl)cyclopropane-1-carboxamide **(I-199)** | **Intermediate 765 table 25** |
| | | ESI-MS (M+H)+: 572.2, δ 10.61 (s, 1H), 8.33 - 8.13 (m, 2H), 7.88 (s, 1H), 7.66 (s, 1H), 7.39 - 7.22 (m, 5H), 7.15 (d, J = 7.6 Hz, 1H), 4.57 (s, 2H), 4.23 (s, 2H), 2.44 - 2.33 (m, 2H), 2.01 - 1.85 (m, 1H), 1.51 (s, 6H), 1.49 - 1.37 (m, 2H), 0.98 - 0.86 (m, 2H), 0.72 - 0.55 (m, 2H). |
| **200** | 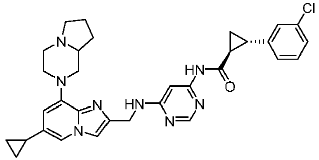 (1S,2S)-2-(3-chlorophenyl)-N-(6-(((6-cyclopropyl-8-(hexahydropyrrolo[1,2-a]pyrazin-2(1H)-yl)imidazo[1,2-a]pyridin-2-yl)methyl)amino) pyrimidin-4-yl)cyclopropane-1-carboxamide **(I-200)** | **Intermediate 766 table 25** |
| | | ESI-MS (M+H)+: 583.1, ¹H NMR (400 MHz, cd3od) δ 8.17 (s, 1H), 7.78 (s, 1H), 7.55 (s, 1H), 7.33 (s, 1H), 7.26 - 7.16 (m, 3H), 7.08 (d, J = 7.6 Hz, 1H), 6.41 (s, 1H), 4.68 (S, J = 36.7 Hz, 2H), 3.01 - 2.82 (m, 3H), 2.70 - 2.43 (m, 3H), 2.20 - 1.85 (m, 6H), 1.65 - 1.56 (m, 2H), 1.39 - 1.25 (m, 4H), 0.94 - 0.87 (m, 2H), 0.70 - 0.66 (m, 2H). |
| **201** | 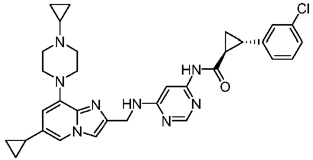 (1S,2S)-2-(3-chlorophenyl)-N-(6-(((6-cyclopropyl-8-(4-cyclopropylpipera zin-1-yl)imidazo[1,2-a]pyridin-2-yl)methyl)amino)pyrimidin-4-yl)cyclo propane-1-carboxamide **(I-201)** | **Intermediate 767 table 25** |
| | | ESI-MS (M+H)+: 583.3, δ 10.58 (s, 1H), 8.17 (s, 1H), 7.85 (s, 2H), 7.50 (s, 1H), 7.31 - 7.23 (m, 4H), 7.12 (d, J = 7.6 Hz, 1H), 6.12 (s, 1H), 4.52 (s, 2H), 3.39 (s, 4H), 2.67 (s, 4H), 2.41 - 2.31 (m, 2H), 1.84 - 1.80 (m, 1H), 1.66 (s, 1H), 1.47 - 1.40 (m, 1H), 1.40 - 1.36 (m, 1H), 0.86 - 0.81 (m, 2H), 0.66 - 0.62 (m, 2H), 0.43 - 0.32 (m, 4H). |
| **202** | 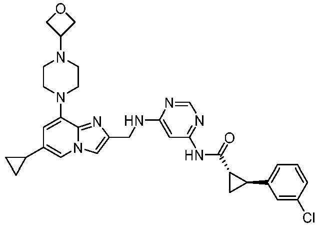 (1S,2S)-2-(3-chlorophenyl)-N-(6-(((6-cyclopropyl-8-(4-(oxetan-3-yl) piperazin-1-yl)imidazo[1,2-a]pyridin-2-yl)methyl)amino)pyrimidin-4-yl) cyclopropane-1-carboxamide **(I-202)** | **Intermediate 768 table 25** |
| | | ESI-MS (M+H)+: 599.2, δ 10.59 (s, 1H), 8.18 (s, 1H), 7.88 - 7.86 (m, 2H), 7.51 (s,1H), 7.34 - 7.23 (m, 4H), 7.15 (d, J = 7.6 Hz, 1H), 6.15 (s, 1H), 4.59-4.46 (m, 6H), 3.50-3.42 (m, 5H), 2.40-2.32 (m, 6H), 1.88 - 1.80 (m, 1H), 1.46-1.42 (m, 2H), 0.91 - 0.82 (m, 2H), 0.68 - 0.59 (m, 2H). |
| **203** | 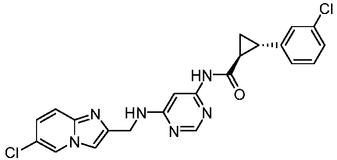 (1S,2S)-N-(6-(((6-chloroimidazo[1,2-a]pyridin-2-yl)methyl)amino) pyrimidin-4-yl)-2-(3-chlorophenyl) cyclopropane-1-carboxamide **(I-203)** | **Intermediate 769 table 25** |
| | | ESI-MS (M+H)+: 453.1, δ 10.61 (s, 1H), 8.77 (d, J = 1.4 Hz, 1H), 8.20 (s, 1H), 7.92 (s, 1H), 7.73 (s, 1H), 7.53 (d, J = 9.6 Hz, 1H), 7.34 - 7.24 (m, 5H), 7.15 (d, J = 7.6 Hz, 1H), 4.60 (s, 2H), 2.43 - 2.36 (m, 2H), 1.49 - 1.45 (m, 1H), 1.43 - 1.39 (m, 1H). |
| **204** | 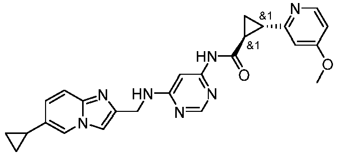 *rac*-(1S*,2S*)-N-(6-(((6-cyclo propyl imidazo[1,2-a]pyridin-2-yl) methyl) amino)pyrimidin-4-yl)-2-(4-methoxy pyridin-2-yl)cyclopropane-1-carboxamide **(I-204)** | **Intermediate 758 table 25**ESI-MS (M+H)+: 456.2, ¹H NMR (400 MHz, CDCl₃) δ 8.31 - 8.24 (m, 2H), 8.06 (s, 1H), 7.88 (s, 1H), 7.51-7.46 (m, 2H), 7.33 (s, 1H), 6.99 (d, J = 10.7 Hz, 1H), 6.81 (d, J = 2.4 Hz, 1H), 6.69-6.64 (m, 1H), 5.80 (s, 1H), 4.71 (s, 2H), 3.85 (s, 3H), 2.63 - 2.56 (m, 1H), 2.25 -2.18 (m, 1H), 1.92-1.89 (m, 2H), 1.30-1.22 (m, 1H), 1.03 -0.94 (m, 2H), 0.72 - 0.65 (m, 2H). |
| **205** | 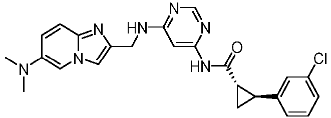 (1S,2S)-2-(3-chlorophenyl)-N-(6-(((6-(dimethylamino)imidazo[1,2-a]pyridin-2-yl)methyl)amino) pyrimidin-4-yl)cyclopropane-1-carboxamide **(I-205)** | 2-(((6-chloropyrimidin-4-yl)amino)methyl)-N,N-dimethylimidazo[1,2-a]pyridin-6-amine |
| | | ESI-MS (M+H)+: 462.1, ¹H NMR (400 MHz, DMSO) δ 10.58 (s, 1H), 8.19 (s, 1H), 7.87 - 7.76 (m, 2H), 7.57 (s, 1H), 7.35 - 7.22 (m, 5H), 7.16 - 7.13 (m, 2H), 4.53 (s, 2H), 2.78 (s, 6H), 2.43 - 2.36 (m, 2H), 1.48 - 1.43 (m, 1H), 1.42 - 1.36 (m, 1H). |
| | | ESI-MS [M +H]+:: 462.1 |
| **206** | 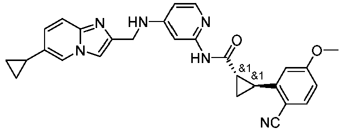 *rac*-(1S*,2S*)-2-(2-cyano-5-methoxy phenyl)-N-(4-(((6-cyclopropylimidazo [1,2-a]pyridin-2-yl)methyl)amino) pyridin-2-yl)cyclopropane-1-carboxamide **(I-206)** | **Intermediate 770 table 25** |
| | | ESI-MS (M+H)+: 480.2, δ 10.66 (s, 1H), 8.29 (s, 1H), 8.20 (s, 1H), 7.88 (s, 1H), 7.74 (d, J = 8.6 Hz, 1H), 7.61 (s, 1H), 7.37 (d, J = 9.3 Hz, 1H), 7.29 (s, 1H), 6.94-6.99 (m, 2H), 6.76 (d, J = 2.4 Hz, 1H), 4.58 (s, 2H), 3.84 (s, 3H), 2.60 - 2.53 (m, 1H), 2.49-2.40 (m, 1H), 1.94-1.87 (m, 1H), 1.56-1.51 (m, 2H), 0.98 - 0.87 (m, 2H), 0.66-0.60 (m, 2H). |
| **207** | 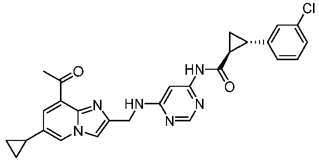 (1S,2S)-N-(6-(((8-acetyl-6-cyclo propylimidazo[1,2-a]pyridin-2-yl) methyl)amino)pyrimidin-4-yl)-2-(3-chlorophenyl)cyclopropane-1-carboxamide **(I-207)** | **Intermediate 771 table 25** |
| | | ESI-MS (M+H)+: 501.2, ¹H NMR (400 MHz, CDCl₃) δ 8.29 (s, 1H),δ 8.25 (s, 1H), 8.04 (s, 1H), 7.62 (s, 1H), 7.53 (s, 1H), 7.36 (s, 1H), 7.24 - 7.21 (m, 2H), 7.08 (s, 1H), 7.00 (d, J = 6.1 Hz, 1H), 5.85 (s, 1H), 4.72 (s, 2H), 3.03 (s, 3H), 2.61 - 2.56 (m, 1H), 1.94 - 1.89 (m, 1H), 1.77 - 1.71 (m, 2H), 1.42 - 1.38 (m, 1H), 1.02 - 0.99 (m, 2H), 0.74 - 0.71 (m, 2H). |
| | | ESI-MS [M +H]+: 501.2. |
| **208** | 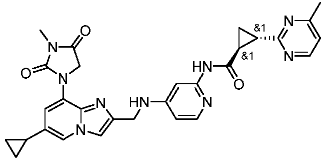 *rac-*(1S*,2S*)-N-(4-(((6-cyclopropyl-8-(3-methyl-2,4-dioxoimidazolidin-1-yl) imidazo[1,2-a]pyridin-2-yl)methyl) amino)pyridin-2-yl)-2-(4-methyl pyrimidin-2-yl)cyclopropane-1-carboxamide **(I-208)** | **Intermediate 772 table 25** |
| | | ESI-MS [M +H]+: 552.2. δ 10.39 (s, 1H), 8.52 (d, J = 5.1 Hz, 1H), 8.29 - 8.26 (m, 2H), 7.75 (d, J = 5.8 Hz, 1H), 7.71 (s, 1H), 7.46 (s, 1H), 7.34 (d, J = 1.4 Hz, 1H), 7.20 (d, J = 5.1 Hz, 1H), 7.15 (t, J = 5.7 Hz, 1H), 6.32 (dd, J = 5.7, 2.0 Hz, 1H), 4.93 (s, 2H), 4.37 (d, J = 5.6 Hz, 2H), 2.98 (s, 3H), 2.62 - 2.52 (m, 2H), 2.41 (s, 3H), 1.97 - 1.90 (m, 1H), 1.52 - 1.44 (m, 2H), 1.02 - 0.85 (m, 2H), 0.71 - 0.53 (m, 2H). |
| **209** | 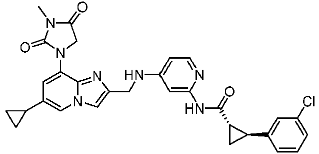 (1S,2S)-2-(3-chlorophenyl)-N-(4-(((6-cyclopropyl-8-(3-methyl-2,4-dioxo imidazolidin-1-yl)imidazo[1,2-a] pyridin-2-yl)methyl)amino)pyridin-2-yl)cyclopropane-1-carboxamide **(I-209)** | **Intermediate 772 table 25**ESI-MS [M +H] +: 570.2. |
| | | δ 10.31 (s, 1H), 8.23 (d, J = 1.1 Hz, 1H), 7.73 (d, J = 5.8 Hz, 1H), 7.68 (s, 1H), 7.43 (s, 1H), 7.36 - 7.16 (m, 5H), 7.11 (d, J = 7.7 Hz, 1H), 6.30 (d, J = 4.2 Hz, 1H), 4.90 (s, 2H), 4.35 (d, J = 5.6 Hz, 2H), 2.95 (s, 3H), 2.37 - 2.28 (m, 2H), 1.94 - 1.88 (m, 1H), 1.45 - 1.38 (m, 1H), 1.35 - 1.28 (m, 1H), 0.95 - 0.87 (m, 2H), 0.66 - 0.59 (m, 2H). |
| **210** | 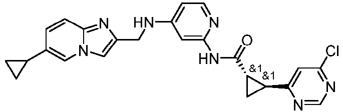 *rac*-(1S*,2S*)-2-(6-chloropyrimidin-4-yl)-N-(4-(((6-cyclopropylimidazo[1,2-a]pyridin-2-yl)methyl)amino)pyridin-2-yl)cyclopropane-1-carboxamide **(I-210)** | **Intermediate 770 table 25** |
| | | ESI-MS [M +H]+: 460.2., δ 10.42 (s, 1H), 8.88 (s, 1H), 8.31 (s, 1H), 7.85 (s, 1H), 7.75 (d, J = 5.4 Hz, 1H), 7.62 (s, 1H), 7.41 (s, 1H), 7.38 (d, J = 9.6 Hz, 1H), 7.12 (s, 1H), 6.97 (d, J = 8.9 Hz, 1H), 6.33 (d, J = 4.4 Hz, 1H), 4.34 (d, J = 5.6 Hz, 2H), 2.72 - 2.64 (m, 2H), 2.04 - 1.95 (m, 1H), 1.94 - 1.87 (m, 1H), 1.60 - 1.51 (m, 1H), 0.95 - 0.86 (m, 2H), 0.69 - 0.62 (m, 2H). |
| **211** | 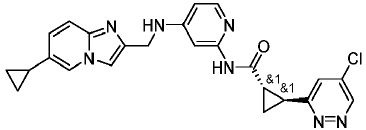 *rac*-(1S*,2S*)-2-(5-chloropyridazin-3-yl)-N-(4-(((6-cyclopropylimidazo[1,2-a]pyridin-2-yl)methyl)amino)pyridin-2-yl)cyclopropane-1-carboxamide **(I-211)** | **Intermediate 770 table 25** |
| | | ESI-MS [M +H]+: 460.2, δ 10.41 (s, 1H), 9.23 (d, J = 2.1 Hz, 1H), 8.31 (s, 1H), 8.02 (d, J = 2.2 Hz, 1H), 7.76 (d, J = 5.7 Hz, 1H), 7.63 (s, 1H), 7.43 (s, 1H), 7.38 (d, J = 9.3 Hz, 1H), 7.13 (t, J = 2.2 Hz , 1H), 7.01 - 6.96 (m, 1H), 6.39 - 6.33 (m, 1H), 4.35 (d, J = 5.6 Hz, 2H), 2.76 - 2.62 (m, 2H), 1.96-1.85 (m, 1H), 1.70-1.64 (m, 1H), 1.62 - 1.49 (m, 1H), 0.91 - 0.85 (m, 2H), 0.68 - 0.64 (m, 2H). |
| **212** | 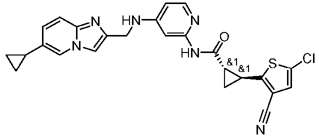 *rac-*(1S*,2S*)-2-(5-chloro-3-cyano thiophen-2-yl)-N-(4-(((6-cyclopropyl imidazo[1,2-a]pyridin-2-yl)methyl) amino)pyridin-2-yl)cyclopropane-1-carboxamide **(I-212)** | **Intermediate 770 table 25** |
| | | ESI-MS [M +H]+: 489.1, δ 10.52 (s, 1H), 8.30 (s, 1H), 7.77 (d, J = 5.8 Hz, 1H), 7.63 (s, 1H), 7.51 (s, 1H), 7.42 (s, 1H), 7.38 (d, J = 9.3 Hz, 1H), 7.15 (s, 1H), 6.98-6.95 (m, 1H), 6.35-6.33 (m, 1H), 4.35 (d, J = 5.7 Hz, 2H), 2.70 -2.63 (m, 2H), 1.94-1.87 (m, 1H), 1.65-1.60 (m, 1H), 1.44 - 1.37 (m, 1H), 0.93-0.88 (m, 2H), 0.68-0.64 (m, 2H). |
| **213** | 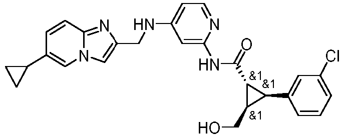 *rac*-(1R* ,2S* ,3R*)-2-(3-chlorophenyl) -N-(4-(((6-cyclopropylimidazo[1,2-a] pyridin-2-yl)methyl)amino)pyridin-2-yl)-3-(hydroxymethyl)cyclopropane-1-carboxamide **(I-213)** | **Intermediate 770 table 25** |
| | | ESI-MS [M +H]+: 488.2, δ 10.64 (s, 1H), 8.29 (s, 1H), 8.22 (s, 1H), 7.86 (s, 1H), 7.61 (s, 11H), 7.46 - 7.27 (m, 6H), 6.97-6.94 (m, 1H), 4.65-4.56 (m, 1H), 4.57 (s, 2H), 3.22 - 3.15 (m, 1H), 3.14 - 3.02 (m, 1H), 2.63-2.60 (m, 1H), 2.57-2.50 (m, 1H), 1.90 - 1.85 (m, 2H), 0.92 - 0.85 (m, 2H), 0.67 - 0.64(m, 2H). |
| **214** | 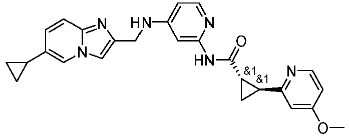 *rac*-(1S*,2S*)-N-(4-(((6-cyclopropyl imidazo[1,2-a]pyridin-2-yl)methyl) amino)pyridin-2-yl)-2-(4-methoxy pyridin-2-yl)cyclopropane-1-carboxamide **(I-214)** | **Intermediate 770 table 25** |
| | | ESI-MS [M +H]+: 555.1, ¹H NMR (400 MHz, CDCl₃) δ 8.58 (s, 1H), 8.25 (d, J = 5.7 Hz, 1H), 7.86 (s, 1H), 7.79 (d, J = 5.9 Hz, 1H), 7.60 (s, 1H), 7.48 - 7.41 (m, 2H), 6.94 (dd, J = 9.3, 1.7 Hz, 1H), 6.79 (d, J = 2.4 Hz, 1H), 6.64 (dd, J = 5.7, 2.5 Hz, 1H), 6.27 (dd, J = 5.9, 2.2 Hz, 1H), 5.18 (s, 1H), 4.52 (d, J = 5.2 Hz, 2H), 3.84 (s, 3H), 2.63 - 2.55 (m, 1H), 2.26 - 2.18 (m, 1H), 1.92 - 1.86 (m, 1H), 1.69 - 1.61 (m, 2H), 0.96 - 0.94 (m, 2H), 0.71 - 0.64 (m, 2H). |
| | | NO EG 215 |
| **216** | 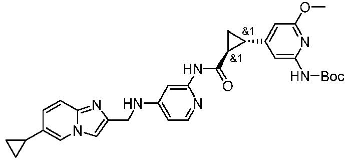 *rac-tert-butyl* (4-((1S*,2S*)-2-((4-(((6-cyclopropylimidazo[1,2-a]pyridin-2-yl)methyl)amino)pyridin-2-yl)carbamoyl)cyclopropyl)-6-methoxypyridin-2-yl)carbamate **(I-216)** | **Intermediate 770 table 25** |
| | | ESI-MS [M + H]+: 570.2. ¹H NMR (400 MHz, MeOD) δ 8.17 (s, 1H), 7.78 (d, J = 6.1 Hz, 1H), 7.65 (s, 1H), 7.40 (d, J = 9.4 Hz, 1H), 7.32 (s, 1H), 7.21 (s, 1H), 7.09 (dd, J = 9.4, 1.7 Hz, 1H), 6.42 (dd, J = 6.1, 2.2 Hz, 1H), 6.26 (s, 1H), 4.51 (s, 2H), 3.84 (s, 3H), 2.44 - 2.39 (m, 1H), 2.30 - 2.24 (m, 1H), 1.98 - 1.92 (m, 1H), 1.66 - 1.61 (m, 1H), 1.54 (s, 9H), 1.44 - 1.39 (m, 1H), 1.01 - 0.95 (m, 2H), 0.75 - 0.70 (m, 2H). |
| **217** | 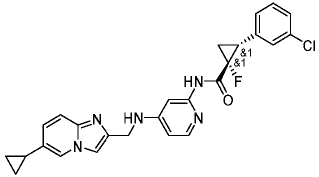 **(I-217)** name is wrong not replaced | **Intermediate 770 table 25** |
| | | ESI-MS [M +H]+:476.2, δ 9.69 (s, 1H), 8.32 (s, 1H), 7.81 (d, J = 5.8 Hz, 1H), 7.65 (s, 1H), 7.44 (s, 1H), 7.42 - 7.27 (m, 5H), 7.29-7.21 (m, 1H), 6.98 - 6.96 (m, 1H), 6.43-6.41 (m, 1H), 4.38 (d, J = 5.5 Hz, 2H), 2.90 - 2.85(m, 1H), 2.16-2.02 (m, 1H), 1.93 - 1.82 (m, 2H), 0.92 - 0.89(m, 2H), 0.72 - 0.64 (m, 2H). |
| **218** | 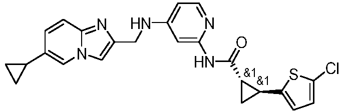 *rac-*(1S**,*2S*)-2-(5-chlorothiophen-2-yl)-N-(4-(((6-cyclopropylimidazo[1,2-a]pyridin-2-yl)methyl)amino)pyridin-2-yl)cyclopropane-1-carboxamide **(I-218)** | **Intermediate 770 table 25** |
| | | ESI-MS [M +H] +: 464.2, δ = 9.69 (s, 1H), 8.32 (s, 1H), 7.81 (d, J=5.8, 1H), 7.65 (s, 1H), 7.42 (d, J=16.4, 1H), 7.38 - 7.31 (m, 3H), 7.29 - 7.20 (m, 1H), 7.03 - 6.90 (m, 1H), 6.48 - 6.36 (m, 1H), 4.38 (d, J=5.5, 2H), 3.31 (s, 1H), 2.97 - 2.81 (m, 1H), 2.17-2.03 (m, 1H), 1.96 - 1.76 (m, 2H), 1.01 - 0.83 (m, 2H), 0.73 - 0.55 (m, 2H). |
| **219** | 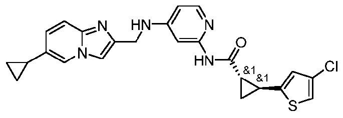 *rac-*(1S**,*2S***)-2-(4-chlorothiophen-2-yl)-N-(4-(((6-cyclopropylimidazo[1,2-a]pyridin-2-yl)methyl)amino)pyridin-2-yl)cyclopropane-1-carboxamide **(I-219)** | **Intermediate 770 table 25** |
| | | ESI-MS [M +H]+: 464.1, δ = 10.40 (s, 1H), 8.31 (s, 1H), 7.76 (d, J=5.8, 1H), 7.62 (s, 1H), 7.45 - 7.31 (m, 3H), 7.12 (s, 1H), 7.00-6.91 (m, 2H), 6.36-6.30 (m, 1H), 4.35 (d, J=5.4, 2H), 2.52 - 2.50 (m, 1H), 2.35 - 2.33 (m, 1H), 1.94 - 1.83 (m, 1H), 1.50 - 1.40 (m, 1H), 1.33 - 1.25 (m, 1H), 0.94 - 0.85 (m, 2H), 0.72 - 0.62 (m, 2H). |
| **220** | 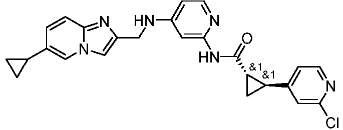 *rac-*(1S*,2S*)-2-(2-chloropyridin-4-yl)-N-(4-(((6-cyclopropylimidazo[1,2-a]pyridin-2-yl)methyl)amino)pyridin-2-yl)cyclopropane-1-carboxamide **(I-220)** | **Intermediate 770 table 25** |
| | | ESI-MS [M +H]+: 459.2, δ 10.37 (s, 0.62H), 10.03 (s, 0.32H), 8.31 - 8.22 (m, 2H), 7.76-7.74 (m, 1H), 7.61 (d, J = 8.1 Hz, 1H), 7.47 - 7.33 (m, 3H), 7.26 - 7.07 (m, 2H), 7.01-6.90 (m, 1H), 6.35 - 6.31 (m, 1H), 4.36-4.32 (m , 2H), 2.49 - 2.35 (m, 1H), 2.05 - 1.84 (m, 1H), 1.50- 1.33 (m, 2H), 1.07 - 0.88 (m, 2H), 0.67 - 0.63 (m, 2H). |
| **221** | 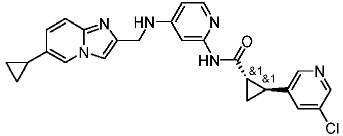 *rac-*(1S*,2S*)-2-(5-chloropyridin-3-yl)-N-(4-(((6-cyclopropylimidazo[1,2-a]pyridin-2-yl)methyl)amino)pyridin-2-yl)cyclopropane-1-carboxamide **(I-221)** | **Intermediate 770 table 25** |
| | | ESI-MS [M +H]+: 459.2, δ 10.33 (s, 1H), 8.47-8.41 (m, 2H), 8.31 (s, 1H), 7.76 (d, J = 5.8 Hz, 1H), 7.75-7.69 (m, 1H), 7.63 (s, 1H), 7.41 (s, 1H), 7.38 (d, J = 9.3 Hz, 1H), 7.13 (s, 1H), 6.98-6.95 (m, 1H), 6.34-6.32( m, 1H), 4.35 (d, J = 5.7 Hz, 2H), 2.47-2.38 (m, 2H), 1.96-1.85(m, 1H), 1.54 - 1.42 (m, 2H), 0.94 **-** 0.82 (m, 2H), 0.72 - 0.60 (m, 2H). |
| **222** | 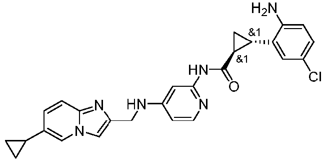 *rac-*(1S*,2S*)-2-(2-amino-5-chlorophenyl)-N-(4-(((6-cyclopropylimidazo[1,2-a]pyridin-2-yl)methyl)amino)pyridin-2-yl)cyclopropane-1-carboxamide **(I-222)** | **Intermediate 770 table 25** |
| | | ESI-MS [M + H] +: 473.3. δ 10.40 (s, 1H), 8.30 (s, 1H), 7.77 (d, J = 5.8 Hz, 1H), 7.63 (s, 1H), 7.45 (s, 1H), 7.38 (d, J = 9.3 Hz, 1H), 7.15 - 7.06 (m, 1H), 6.95 - 6.91 (m, 3H), 6.66- 6.60 (m, 1H), 6.33 (dd, J = 5.8, 2.1 Hz, 1H), 5.12 - 5.05 (m, 2H), 4.35 (d, J = 5.5 Hz, 2H), 2.29 - 2.24 (m, 1H), 2.14 - 2.09 (m, 1H), 1.94 - 1.87 (m, 1H), 1.44 - 1.36 (m, 1H), 1.05 - 0.97 (m, 1H), 0.92 - 0.85 (m, 2H), 0.68 - 0.64 (m, 2H). |
| **223** | 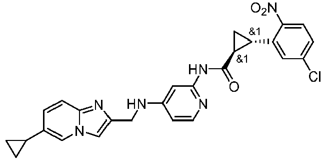 *rac-*(1S**,*2S*)-2-(5-chloro-2-nitro phenyl)-N-(4-(((6-cyclopropylimidazo [1,2-a]pyridin-2-yl)methyl)amino) pyridin-2-yl)cyclopropane-1-carboxamide **(I-223)** | **Intermediate 770 table 25** |
| | | ESI-MS [M + H] +: 503.1, δ 10.36 (s, 1H), 8.31 (s, 1H), 8.02 (d, J = 8.7 Hz, 1H), 7.77 (d, J = 5.8 Hz, 1H), 7.64 (s, 1H), 7.59 (dd, J = 8.7, 2.2 Hz, 1H), 7.55 - 7.54 (m, 1H), 7.44 (s, 1H), 7.38 (d, J = 9.3 Hz, 1H), 7.15 (s, 1H), 6.97 (dd, J = 9.3, 1.8 Hz, 1H), 6.33 (dd, J = 5.8, 2.0 Hz, 1H), 4.36 (d, J = 5.7 Hz, 2H), 2.69 - 2.64 (m, 1H), 2.39 - 2.33 (m, 1H), 1.90 - 1.87 (m, 1H), 1.48 - 1.42 (m, 2H), 0.89 - 0.88 (m, 2H), 0.68 - 0.64 (m, 2H). |
| **224** | 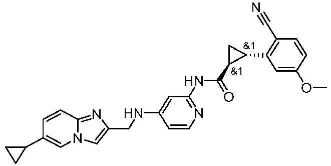 *rac*-(1S*,2S*)-2-(2-cyano-5-methoxy phenyl)-N-(4-(((6-cyclopropylimidazo [1,2-a]pyridin-2-yl)methyl)amino) pyridin-2-yl)cyclopropane-1-carboxamide **(I-224)** | **Intermediate 770 table 25** |
| | | ESI-MS [M +H]+: 479.2, δ 10.39 (s, 1H), 8.30 (s, 1H), 7.76-7.72 (m, 2H), 7.64 (s, 1H), 7.45 (s, 1H), 7.37 (d, J = 9.3 Hz, 1H), 7.14 (s, 1H), 6.97-6.94 (m, 2H), 6.74 (d, J = 2.4 Hz, 1H), 6.34-6.32 (m, 1H), 4.36 (d, J = 5.7 Hz, 2H), 3.84 (s, 3H), 2.60 - 2.52 (m, 1H), 2.53-2.47 (m, 1H), 1.97 - 1.82 (m, 1H),1.52- 1.49 (m, 2H), 0.93-0.84 (m,2H), 0.68-0.64 (m,2H). |
| **225** | 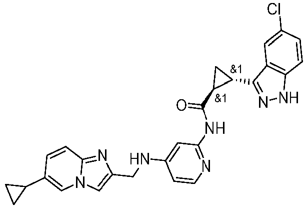 *rac-*(1S*,2S*)-2-(5-chloro-1H-*i*ndazol-3-yl)-N-(4-(((6-cyclopropyl imidazo[1,2-a]pyridin-2-yl)methyl) amino)pyridin-2-yl)cyclopropane-1-carboxamide **(I-225)** | **Intermediate 770 table 25** |
| | | ESI-MS [M +H] +: 498.1, δ 8.69 (d, J = 9.1 Hz, 1H), 8.32 (s, 1H), 7.99 (d, J = 5.8 Hz, 1H), 7.97 (d, J = 1.7 Hz, 1H), 7.72 (s, 1H), 7.71 (s, 1H), 7.50 (dd, J = 9.0 Hz, 1.9 Hz, 1H), 7.40 (d, J = 9.0 Hz, 2H), 7.14 (s, 1H), 7.03 (s, 1H), 6.98 (dd, J = 9.4 Hz, 1.5 Hz, 1H), 6.56 - 6.54 (m, 1H), 4.46 (d, J = 5.5 Hz, 2H), 2.71 - 2.67 (m, 1H), 2.25 - 2.21 (m, 1H), 1.93 - 1.88 (m, 1H), 1.52 - 1.45 (m, 2H), 0.92 - 0.88 (m, 2H), 0.68 - 0.64 (m, 2H). |
| **226** | 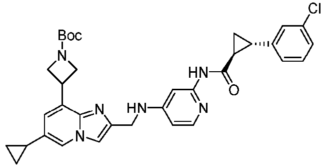 *tert-*butyl 3-(2-(((2-((1S,2S)-2-(3-chlorophenyl)cyclopropane-1-carbox amido)pyridin-4-yl)amino)methyl)-6-cyclopropylimidazo[1,2-a]pyridin-8-yl)azetidine-1-carboxylate **(I-226)** | **Intermediate 770 table 25** |
| | | ESI-MS [M +H]+: 613.3, δ 10.31 (s, 1H), 8.21 (s, 1H), 7.76 (d, J = 5.7 Hz, 1H), 7.62 (s, 1H), 7.43 (s, 1H), 7.34 - 7.21 (m, 3H), 7.14-7.09 (m, 2H), 6.95 (s, 1H), 6.35-6.30 (m, 1H), 4.35 (d, J = 5.6 Hz, 2H), 4.25-4.41 (m, 5H), 2.38 - 2.32 (m, 2H), 1.96 - 1.88 (m, 1H), 1.60 - 1.27 (m, 11H), 0.96 - 0.85 (m, 2H), 0.75 - 0.64 (m, 2H). |
| **227** | 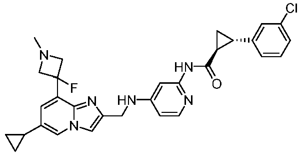 (1S,2S)-2-(3-chlorophenyl)-N-(4-(((6-cyclopropyl-8-(3-fluoro-1-methyl azetidin-3-yl)imidazo[1,2-a]pyridin-2-yl)methyl)amino)pyridin-2-yl)cyclo propane-1-carboxamide **(I-227)** | **Intermediate 773 table 25** |
| | | ESI-MS [M +H]+: 545.2, δ 10.34 (s, 1H), 8.37 (s, 1H), 7.77 (d, J = 5.8 Hz, 1H), 7.69 (s, 1H), 7.45 (s, 1H), 7.34 - 7.23 (m, 3H), 7.21 - 7.06 (m, 3H), 6.36-6.32 (m, 1H), 4.39 (d, J = 5.7 Hz, 2H), 4.20-4.12 (m, 2H), 3.89-3.80 (m, 2H), 2.49 (s, 3H), 2.41 - 2.31 (m, 2H), 1.98-1.92 (m, 1H), 1.47 - 1.40 (m, 1H), 1.36-1.32 (m, 1H), 0.97 - 0.87 (m, 2H), 0.74 - 0.66 (m, 2H). |
| **228** | 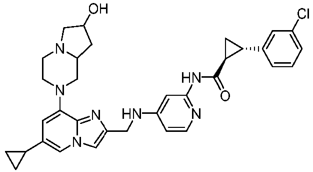 (1S,2S)-2-(3-chlorophenyl)-N-(4-(((6-cyclopropyl-8-(7-hydroxyhexahydro pyrrolo[1,2-a]pyrazin-2(1H)-yl) imidazo[1,2-a]pyridin-2-yl)methyl) amino)pyridin-2-yl)cyclopropane-1-carboxamide **(I-228)** | **Intermediate 774 table 25** |
| | | ESI-MS (M+H)+: 598.2, ¹H NMR (400 MHz, MeOD) δ 7.75 (d, J = 5.7 Hz, 2H), 7.54 (s, 1H), 7.37 (s, 1H), 7.25 (t, J = 7.7 Hz, 1H), 7.19 - 7.17 (m, 2H), 7.09 (d, J = 7.6 Hz, 1H), 6.40 - 6.38 (m, 2H), 4.48 (s, 2H), 4.42 - 4.39 (m, 1H), 4.10 (d, J = 10.9 Hz, 1H), 3.99 (d, J = 11.9 Hz, 1H), 3.11 (d, J = 11.0 Hz, 1H), 3.03 (d, J = 10.2 Hz, 1H), 2.95 - 2.89 (m, 1H), 2.64 - 2.51 (m, 2H), 2.48 - 2.36 (m, 4H), 2.18 - 2.14 (m, 1H), 1.90 - 1.85 (m, 1H), 1.60 - 1.57 (m, 1H), 1.48 - 1.42 (m, 1H), 1.37 - 1.34 (m, 1H), 0.94 - 0.89 (m, 2H), 0.72 - 0.66 (m, 2H). |
| **229** | 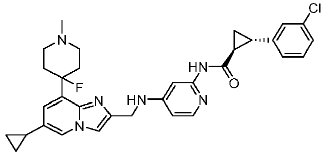 (1S,2S)-2-(3-chlorophenyl)-N-(4-(((6-cyclopropyl-8-(4-fluoro-1-methyl piperidin-4-yl)imidazo[1,2-a]pyridin-2-yl)methyl)amino)pyridin-2-yl)cyclo propane-1-carboxamide **(I-229)** | **Intermediate 775 table 25** |
| | | ESI-MS (M+H)+: 573.3, δ 10.32 (s, 1H), 8.27 (s, 1H), 7.75 (d, J = 5.8 Hz, 1H), 7.63 (s, 1H), 7.43 (s, 1H), 7.32 - 7.28 (m, 1H), 7.25 - 7.23 (m, 2H), 7.17 - 7.12 (m, 2H), 7.00 (s, 1H), 6.33 - 6.31 (m, 1H), 4.37 (d, J = 5.7 Hz, 2H), 3.17 - 3.09 (m, 1H), 3.06 - 2.98 (m, 1H), 2.81 - 2.78 (m, 2H), 2.38 - 2.32 (m, 4H), 2.30 (s, 3H), 1.97 - 1.90 (s, 1H), 1.80 - 1.74 (m, 2H), 1.45 - 1.41 (m, 1H), 1.36 - 1.32 (m, 1H), 0.92-0.88 (m,2H), 0.67-0.63 (m,2H). |
| **230** | 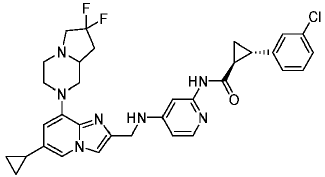 (1S,2S)-2-(3-chlorophenyl)-N-(4-(((6-cyclopropyl-8-(7,7-difluorohexahydro pyrrolo[1,2-a]pyrazin-2(1H)-yl) imidazo[1,2-a]pyridin-2-yl)methyl) amino)pyridin-2-yl)cyclopropane-1-carboxamide **(I-230)** | **Intermediate 776 table 25** |
| | | ESI-MS (M+H)+: 618.3, δ 10.35 (s, 1H), 7.89 (s, 1H), 7.75 (d, J = 5.8 Hz, 1H), 7.54 (s, 1H), 7.41 (s, 1H), 7.31 - 7.28 (m, 1H), 7.24 - 7.22 (m, 2H), 7.19 - 7.10 (m, 2H), 6.34 (d, J = 5.2 Hz, 1H), 6.19 (s, 1H), 4.40 (d, J = 12.0 Hz, 2H), 4.34 (d, J = 5.7 Hz, 2H), 3.51 - 3.41 (m, 2H), 3.03 (d, J = 10.7 Hz, 1H), 2.80 (t, J = 10.7 Hz, 1H), 2.62 - 2.57 (m, 3H), 2.40 - 2.33 (m, 3H), 2.09 - 1.92 (m, 1H), 1.87 - 1.82 (m, 1H), 1.47 - 1.42 (m, 1H), 1.37 - 1.35 (m, 1H), 0.89 - 0.84 (m, 2H), 0.68 - 0.64 (m, 2H). |
| **231** | 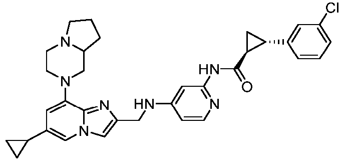 (1S,2S)-2-(3-chlorophenyl)-N-(4-(((6-cyclopropyl-8-(hexahydropyrrolo[1,2-a]pyrazin-2(1H)-yl)imidazo[1,2-a]pyridin-2-yl)methyl)amino)pyridin-2-yl)cyclopropane-1-carboxamide **(I-231)** | **Intermediate 777 table 25** |
| | | ESI-MS [M +H]+: 582.3, δ 10.29 (s, 1H), 7.84 (s, 1H), 7.73 (d, J = 5.8 Hz, 1H), 7.51 (s, 1H), 7.42 (s, 1H), 7.29 (t, J = 7.6 Hz, 1H), 7.24 - 7.22 (m, 2H), 7.13 - 7.06 (m, 2H), 6.32 - 6.30 (m,1H), 6.16 (s, 1H), 4.32 - 4.31 (m, 2H), 3.05 - 3.00 (m, 2H), 2.74 - 2.65 (m, 2H), 2.34 - 2.31 (m, 2H), 2.10 - 2.05 (m, 2H), 1.89 - 1.80 (m, 2H), 1.71 - 1.69 (m, 2H), 1.45 - 1.32 (m, 4H), 1.21 - 1.21 (m, 2H), 0.86 - 0.82 (m, 2H), 0.65 - 0.61 (m, 2H). |
| **232** | 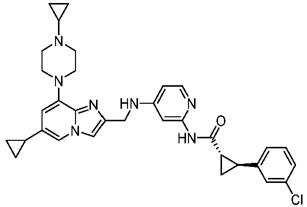 (1S,2S)-2-(3-chlorophenyl)-N-(4-(((6-cyclopropyl-8-(4-cyclopropyl piperazin-1-yl)imidazo[1,2-a]pyridin-2-yl)methyl)amino)pyridin-2-yl)cyclo propane-1-carboxamide **(I-232)** | **Intermediate 778 table 25** |
| | | ESI-MS [M +H]+: 582.3, δ 10.36 (s, 1H), 7.86 (s, 1H), 7.75 (d, J = 5.8 Hz, 1H), 7.53 (s, 1H), 7.42 (s, 1H), 7.34 - 7.28 (m, 1H), 7.28 - 7.22 (m, 2H), 7.19 - 7.09 (m, 2H), 6.34 (d, J = 4.0 Hz, 1H), 6.14 (s, 1H), 4.34 (d, J = 5.6 Hz, 2H), 3.43 (s, 4H), 2.72 (s, 4H), 2.44 - 2.30 (m, 2H), 1.90 - 1.78 (m, 1H), 1.70 (s, 1H), 1.50 - 1.41 (m, 1H), 1.40 - 1.30 (m, 1H), 0.91 - 0.82 (m, 2H), 0.69 - 0.62 (m, 2H), 0.49 - 0.41 (m, 2H), 0.39 - 0.31 (m, 2H). |
| **233** | 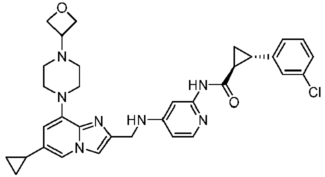 (1S,2S)-2-(3-chlorophenyl)-N-(4-(((6-cyclopropyl-8-(4-(oxetan-3-yl) pipera zin-1-yl)imidazo[1,2-a]pyridin-2-yl) methyl)amino)pyridin-2-yl)cyclo propane-1-carboxamide **(I-233)** | **Intermediate 779 table 25** |
| | | ESI-MS [M +H]+: 598.2, δ 10.68 (s, 1H), 7.87 (d, J = 5.7 Hz, 1H), 7.76 (t, J = 5.8 Hz, 1H), 7.56 (d, J = 5.6 Hz, 2H), 7.43 - 6.92 (m, 5H), 6.43 (s, 1H), 6.17 (d, J = 5.7 Hz, 1H), 4.77 - 4.17 (m, 6H), 3.56-3.48 (m, 4H), 2.46 - 2.37 (m, 4H), 2.30-2.26 (m, 2H), 1.86-1.82 (m, 1H), 1.53 - 1.33 (m, 2H), 0.93 - 0.76 (m, 2H), 0.68-0.62 (m, 2H). |
| **234** | 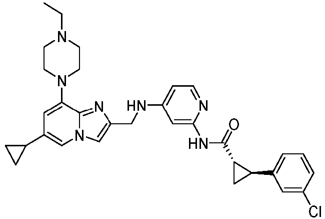 (1S,2S)-2-(3-chlorophenyl)-N-(4-(((6-cyclopropyl-8-(4-ethylpiperazin-1-yl)imidazo[1,2-a]pyridin-2-yl)methyl) amino)pyridin-2-yl)cyclopropane-1-carboxamide **(I-234)** | **Intermediate 780 table 25** |
| | | ESI-MS [M +H]+: 570.3, δ 10.32 (s, 1H), 7.87 (s, 1H), 7.75 (d, J = 5.7 Hz, 1H), 7.53 (s, 1H), 7.45 (s, 1H), 7.38 - 7.20 (m, 3H), 7.20 - 7.04 (m, 2H), 6.32 (d, J = 4.1 Hz, 1H), 6.15 (s, 1H), 4.34 (d, J = 5.5 Hz, 2H), 3.48 (s, 4H), 2.55 (s, 4H), 2.46 - 2.31 (m, 2H), 1.90 - 1.78 (m, 1H), 1.86 - 1.82 (m, 2H), 1.53 - 1.39 (m, 1H), 1.39 - 1.31 (m, 1H), 1.05 (t, J = 7.1 Hz, 3H), 0.93 - 0.80 (m, 2H), 0.73 - 0.59 (m, 2H). |
| **235** | 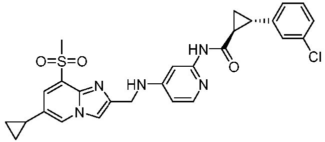 (1S,25)-2-(3-chlorophenyl)-N-(4-(((6-cyclopropyl-8-(methylsulfonyl) imidazo[1,2-a]pyridin-2-yl)methyl) amino)pyridin-2-yl)cyclopropane-1-carboxamide **(I-235)** | **Intermediate 781 table 25** |
| | | ESI-MS [M +H]+: 536.1, δ 10.33 (s, 1H), 8.65 (d, J = 1.2 Hz, 1H), 7.83 (s, 1H), 7.77 (d, J = 5.8 Hz, 1H), 7.57 (d, J = 1.7 Hz, 1H), 7.45 (s, 1H), 7.33 - 7.29 (m, 1H), 7.26 - 7.23 (m, 3H), 7.15 - 7.12 (m, 1H), 6.34 (dd, J = 5.8, 2.1 Hz, 1H), 4.45 (d, J = 5.6 Hz, 2H), 3.54 (s, 3H), 2.38 - 2.32 (m, 2H), 2.10 - 2.05 (m, 1H), 1.46 - 1.42 (m, 1H), 1.37 - 1.33 (m, 1H), 0.99 - 0.95 (m, 2H), 0.73 - 0.69 (m, 2H). |
| **236** | 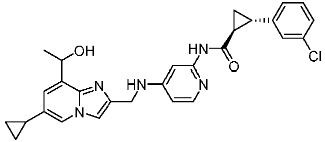 (1S,2S)-2-(3-chlorophenyl)-N-(4-(((6-cyclopropyl-8-(1-hydroxyethyl) imidazo[1,2-a]pyridin-2-yl)methyl) amino)pyridin-2-yl)cyclopropane-1-carboxamide **(I-236)** | **Intermediate 782 table 25** |
| | | ESI-MS [M +H]+: 502.2, δ 10.38 (s, 1H), 8.18 (s, 1H), 7.76 (d, J = 5.8 Hz, 1H), 7.62 (s, 1H), 7.39 (s, 1H), 7.33 - 7.29 (m, 1H), 7.25 (d, J = 6.8 Hz, 2H), 7.14 (d, J = 7.5 Hz, 1H), 7.01 (s, 1H), 6.34 (d, J = 4.4 Hz, 1H), 5.30 (d, J = 4.7 Hz, 1H), 5.20 - 5.17 (m, 1H), 4.37 (d, J = 5.7 Hz, 2H), 2.40 - 2.34 (m, 2H), 1.95 - 1.88 (m, 1H), 1.47 - 1.45 (m, 4H), 1.38 - 1.33 (m, 6.2 Hz, 1H), 0.93 - 0.89 (m, 2H), 0.66 - 0.62 (m, 2H). |
| **237** | 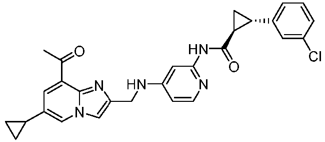 (1S,2S)-N-(4-(((8-acetyl-6-cyclo propylimidazo[1,2-a]pyridin-2-yl) methyl)amino)pyridin-2-yl)-2-(3-chlorophenyl)cyclopropane-1-carboxamide **(I-237)** | **Intermediate 771 table 25** |
| | | ESI-MS [M +H]+: 500.2, ¹H NMR (400 MHz, CDCl₃) δ 8.73 (s, 1H), 8.04 (s, 1H), 7.80 (d, J = 5.8 Hz, 1H), 7.61 (s, 2H), 7.53 (s, 1H), 7.22 - 7.17 (m, 2H), 7.08 (s, 1H), 7.01 (d, J = 6.9 Hz, 1H), 6.30 - 6.29 (m, 1H), 5.18 - 5.15 (m, 1H), 4.56 (d, J = 5.3 Hz, 2H), 3.02 (s, 3H), 2.59 - 2.54 (m, 1H), 1.93 - 1.90 (m, 1H), 1.82 - 1.79 (m, 2H), 1.37 - 1.33 (m, 1H), 1.03 - 0.98 (m, 2H), 0.74 - 0.71 (m, 2H). |
| **238** | 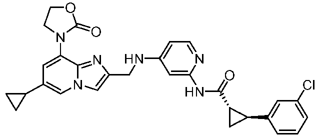 (1S,2S)-2-(3-chlorophenyl)-N-(4-(((6-cyclopropyl-8-(2-oxooxazolidin-3-yl) imidazo[1,2-a]pyridin-2-yl)methyl) amino)pyridin-2-yl)cyclopropane-1-carboxamide **(I-238)** | **Intermediate 783 table 25** |
| | | ESI-MS [M +H]+: 543.2, δ 10.32 (s, 1H), 8.26 (s, 1H), 8.15 (s, 1H), 7.76 (d, J = 5.8 Hz, 1H), 7.69 (s, 1H), 7.45 (s, 1H), 7.29 - 7.13 (m, 6H), 6.32 (d, J = 4.0 Hz, 1H), 4.59 - 4.46 (m, 4H), 4.38 - 4.36 (m, 2H), 2.40 - 2.29 (m, 2H), 1.99 - 1.84 (m, 1H), 1.49 - 1.38 (m, 1H), 1.38 - 1.24 (m, 1H), 0.99 - 0.83 (m, 2H), 0.68 - 0.58 (m, 2H). |
| **239** | 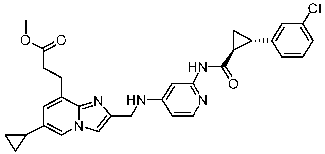 methyl 3-(2-(((2-((1S,2S)-2-(3-chloro phenyl)cyclopropane-1-carboxamido) pyridin-4-yl)amino)methyl)-6-cyclo propylimidazo[1,2-a]pyridin-8-yl)propanoate **(I-239)** | Methyl3-(2-(((2-bromopyridin-4-yl)amino)methyl)-6-cyclopropylimidazo[1,2-a]pyridin-8-yl)propanoate |
| | | ESI-MS [M +H]+: 544.3, δ 10.31 (s, 1H), 8.18 (s, 1H), 7.75 (d, J = 5.7 Hz, 1H), 7.61 - 7.58 (m, 1H), 7.44 (s, 1H), 7.37 - 7.18 (m, 3H), 7.15 - 7.10 (m, 2H), 6.80 (s, 1H), 6.32 (d, J = 5.8 Hz, 1H), 4.36 (d, J = 5.5 Hz, 2H), 3.60 (s, 3H), 3.09 (t, J = 7.6 Hz, 2H), 2.82 (t, J = 7.6 Hz, 2H), 2.36 - 2.35 (m, 2H), 1.87 - 1.85 (m, 1H), 1.52 - 1.37 (m, 1H), 1.34 - 1.33 (m, 1H), 0.90 - 0.88 (m, 2H), 0.66 - 0.63 (m, 2H). |
| **240** | 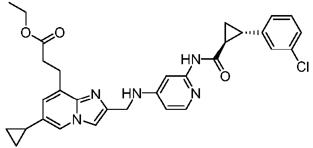 ethyl 3-(2-(((2-((1S,2S)-2-(3-chloro phenyl)cyclopropane-1-carboxamido) pyridin-4-yl)amino)methyl)-6-cyclo propylimidazo[1,2-a]pyridin-8-yl)propanoate **(I-240)** | **Intermediate 784 table 25** |
| | | ESI-MS [M +H]+: 558.2. δ 10.30 (s, 1H), 8.38 (s, 1H), 8.18 (d, J = 1.3 Hz, 1H), 7.75 (d, J = 5.8 Hz, 1H), 7.61 (s, 1H), 7.43 (s, 1H), 7.34 - 7.28 (m, 1H), 7.26 - 7.22 (m, 2H), 7.15-7.12 (m, 2H), 6.80 (s, 1H), 6.33-6.31 (m, 1H), 4.36 (d, J = 5.7 Hz, 2H), 4.09-4.03 (m, 2H), 3.12 - 3.06 (m, 2H), 2.82-1.78 (m, 2H), 2.38 - 2.32 (m, 2H), 1.90 - 1.83 (m, 1H), 1.48 - 1.41 (m, 1H), 1.36-1.32 (m, 1H), 1.18-1.15 (m, 3H),0.95 - 0.85 (m, 2H), 0.64 (m, 2H). |
| **241** | 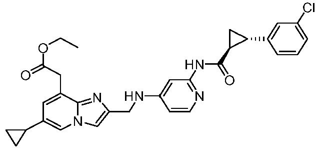 ethyl 2-(2-(((2-((1S,2S)-2-(3-chloro phenyl)cyclopropane-1-carboxamido) pyridin-4-yl)amino)methyl)-6-cyclo propylimidazo[1,2-a]pyridin-8-yl) acetate **(I-241)** | **Intermediate 785 table 25** |
| | | ESI-MS [M +H]+: 544.2. δ 8.25 (s, 1H), 7.79 (d, J = 6.3 Hz, 1H), 7.69 (s, 1H), 7.34 - 7.26 (m, 4H), 7.18 - 7.16 (m, 1H), 6.93 (s, 1H), 6.49 (s, 1H), 4.43 - 4.42 (m, 2H), 4.11 - 4.06 (m, 2H), 3.90 (s, 2H), 2.51 - 2.49 (m, 1H), 2.30 - 2.27 (m, 1H), 1.94 - 1.88 (m, 1H), 1.52 - 1.45 (m, 2H), 1.18 (t, J = 7.1 Hz, 3H), 0.94 - 0.90 (m, 2H), 0.67 - 0.63 (m, 2H). |
| **242** | 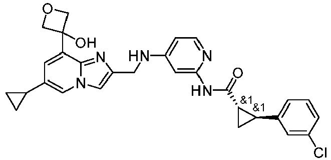 *rac-*(1S*,2S*)-2-(3-chlorophenyl)-N-(4-(((6-cyclopropyl-8-(3-hydroxy oxetan-3-yl)imidazo[1,2-a]pyridin-2-yl)methyl)amino)pyridin-2-yl) cyclopropane-1-carboxamide **(I-242)** | 3-(2-(((2-aminopyridin-4-yl)amino) methyl)-6-cyclopropylimidazo[1,2-a] pyridin-8-yl)oxetan-3-ol |
| | | ESI-MS [M +H]+: 530.2., δ 10.31 (s, 1H), 8.28 (s, 1H), 7.76 (d, J = 5.8 Hz, 1H), 7.65 (s, 1H), 7.45 (s, 1H), 7.33-7.22 (m, 3H), 7.15-7.11 (m, 2H), 7.05 (s, 1H), 6.46 (s, 1H), 6.33 (d, J = 5.7 Hz, 1H), 5.25 (d, J = 6.4 Hz, 2H), 4.67 (d, J = 6.4 Hz, 2H), 4.38 (d, J = 5.8 Hz, 2H), 2.39-2.32 (m, 2H), 1.97 - 1.89 (m, 1H), 1.47-1.41 (m, 1H), 1.35-1.31 (m, 1H), 0.95 - 0.87 (m, 2H), 0.72-0.66 (m, 2H). |
| **243** | 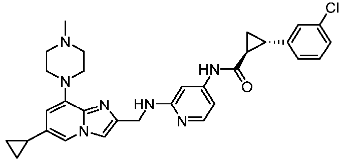 (1S,2S)-2-(3-chlorophenyl)-N-(2-(((6-cyclopropyl-8-(4-methylpiperazin-1-yl)imidazo[1,2-a]pyridin-2-yl) methyl)amino)pyridin-4-yl)cyclo propane-1-carboxamide **(I-243)** | **Intermediate 786 table 25** |
| | | ESI-MS [M +H]+: 556.3. δ 10.26 (s, 1H), 7.97 - 7.73 (m, 2H), 7.47 (s, 1H), 7.41 - 7.23 (m, 3H), 7.17 (d, J = 7.6 Hz, 1H), 6.96 - 6.81 (m, 2H), 6.67 (d, J = 5.7 Hz, 1H), 6.13 (s, 1H), 4.46 (d, J = 5.6 Hz, 2H), 3.47 (s, 4H), 2.67 (s, 4H), 2.39 - 2.36 (m, 1H), 2.23 (s, 3H), 2.16 - 2.09 (m, 1H), 1.88 - 1.77 (m, 1H), 1.51 - 1.42 (m, 2H), 0.94 - 0.76 (m, 2H), 0.73 - 0.49 (m, 2H). |
| **244** | 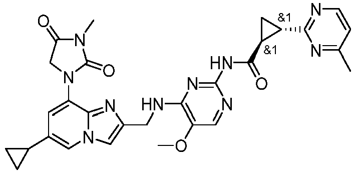 *rac-*(1S*,2S*)-N-(4-(((6-cyclopropyl-8-(3-methyl-2,4-dioxoimidazolidin-1-yl)imidazo[1,2-a]pyridin-2-yl)methyl) amino)-5-methoxypyrimidin-2-yl)-2-(4-methylpyrimidin-2-yl)cyclo propane-1-carboxamide **(I-244)** | **Intermediate 994** in eg 626 |
| | | ESI-MS [M +H]+: 583.2, δ 10.16 (s, 1H), 8.46 (d, J = 5.0 Hz, 1H), 8.31 (s, 1H), 8.20 (s, 1H), 7.70 (d, J = 21.9 Hz, 2H), 7.50 (s, 1H), 7.31 (s, 1H), 7.14 (d, J = 5.0 Hz, 1H), 4.88 (s, 2H), 4.58 (d, J = 5.1 Hz, 2H), 3.81 (s, 3H), 2.97 (s, 3H), 2.67 - 2.59 (m,, 2H), 2.38 (s, 3H), 1.93 - 1.90 (m, 1H), 1.50 - 1.46 (m, 2H), 1.03 - 0.80 (m, 2H), 0.65 - 0.61 (m, 2H). |
| **245** | 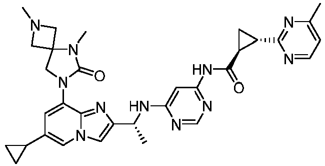 (1S,25)-N-(6-(((R)-1-(6-cyclopropyl-8-(2,5-dimethyl-6-oxo-2,5,7-tri azaspiro[3.4]octan-7-yl)imidazo[1,2-a]pyridin-2-yl)ethyl)amino)pyrimidin-4-yl)-2-(4-methylpyrimidin-2-yl)cyclo propane-1-carboxamide **(I-245)** | (R)-7-(2-(1-((6-bromopyrimidin-4-yl)amino)ethyl)-6-cyclopropylimidazo[1,2-a]pyridin-8-yl)-2,5-dimethyl-2,5,7-triazaspiro [3.4]octan-6-one |
| | | ESI-MS [M+H]+: 608.4;. δ 10.65 (s, 1H), 8.52 (d, J = 5.1 Hz, 1H), 8.15 (s, 1H), 7.80 - 7.58 (m, 2H), 7.47 (s, 1H), 7.29 (s, 1H), 7.21 (d, J = 5.1 Hz, 1H), 6.98 (s, 1H), 5.28 (s, 1H), 4.41 - 4.31 (m, 2H), 4.18 - 4.06 (m, 2H), 3.58 (s, 2H), 2.81 (s, 3H), 2.67 (s, 3H), 2.64 - 2.58 (m, 1H), 2.56 - 2.53 (m, 1H), 2.41 (s, 3H), 1.86 - 1.76 (m, 1H), 1.55 - 1.44 (m, 5H), 0.87 - 0.77 (m, 2H), 0.64 - 0.55 (m, 2H). |
| **246** | 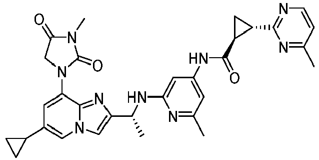 (1S,25)-N-(2-(((R)-1-(6-cyclopropyl-8-(3-methyl-2,4-dioxoimidazolidin-1-yl)imidazo[1,2-a]pyridin-2-yl)ethyl) amino)-6-methylpyridin-4-yl)-2-(4-methylpyrimidin-2-yl)cyclopropane-1-carboxamide **(I-246)** | (*R*)-1-(2-(1-((4-bromo-6-methylpyridin-2-yl)amino)ethyl)-6-cyclopropylimidazo[1,2-a]pyridin-8-yl)-3-methylimidazolidine-2,4-dione |
| | | ESI-MS [M +H]+: 580.2, δ 10.24 (s, 1H), 8.52 (d, J = 5.1 Hz, 1H), 8.30 (s, 1H), 8.23 (d, J = 1.1 Hz, 1H), 7.61 (s, 1H), 7.31 (d, J = 1.5 Hz, 1H), 7.21 (d, J = 5.1 Hz, 1H), 6.69 (d, J = 8.7 Hz, 2H), 6.52 (s, 1H), 5.19 - 5.04 (m, 1H), 4.94 (d, J = 2.0 Hz, 2H), 2.97 (s, 3H), 2.55-2.51 (m,2H), 2.41 (s,3H), 2.17 (s,3H), 1.99 - 1.86 (m, 1H), 1.54 - 1.41 (m, 5H), 0.98 - 0.88 (m, 2H), 0.68 - 0.57 (m, 2H). |
| **247** | 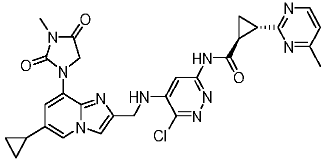 (1S,2S)-N-(6-chloro-5-(((6-cyclo propyl-8-(3-methyl-2,4-dioxo imidazolidin-1-yl)imidazo[1,2-a] pyridin-2-yl)methyl)amino) pyridazin-3-yl)-2-(4-methylpyrimidin-2-yl)cyclo propane-1-carboxamide **(I-247)** | **Intermediate 788 table 25** ESI-MS [M +H]+: 588.2, |
| | | δ 10.58 (s, 1H), 8.54 (d, J = 5.1 Hz, 1H), 8.29 (s, 1H), 7.74 (s, 1H), 7.37 (s, 1H), 7.32 (s, 1H), 7.22 (d, J = 5.1 Hz, 1H), 6.95 (s, 1H), 4.90 (d, J = 2.7 Hz, 2H), 4.50 (d, J = 5.7 Hz, 2H), 2.97 (s, 3H), 2.65-2.55 (m, 2H), 2.42 (s, 3H), 1.98-1.88 (m, 1H), 1.57-1.50 (m, 2H), 0.96-0.90 (m, 2H), 0.69-0.62 (m, 2H). |
| **248** | 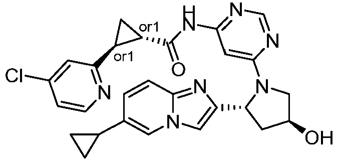 (1S*,2S*)-2-(4-chloropyridin-2-yl)-N-(6-((2R,4S)-2-(6-cyclopropyl imidazo[1,2-a]pyridin-2-yl)-4-hydroxypyrrolidin-1-yl)pyrimidin-4-yl)cyclopropane-1-carboxamide, **isomer 1 (I-248)** | **Intermediate 789 table 25** |
| | | ESI-MS [M +H]+: 516.2, ¹H NMR (400 MHz, cd3od) δ 8.32 (d, J=5.0 Hz, 1H), 8.25-8.04 (m, 2H), 7.63 (s, 1H), 7.35 (d, J=9.3Hz, 2H), 7.31 - 7.23 (m, 2H), 7.05 (d, J=9.3Hz, 1H), 5.30-5.01 (m, 1H), 4.90 (s, 1H), 4.60 (s, 1H), 4.12-3.88 (m, 1H), 2.55-2.30 (m, 4H), 1.99-1.87 (m, 1H), 1.62 - 1.57 (m, 2H), 0.97-0.92 (m, 2H), 0.76-0.64 (m, 2H). |
| **249** | 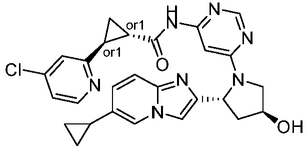 (1S*,2S*)-2-(4-chloropyridin-2-yl)-N-(6-((2R,4S)-2-(6-cyclopropyl imidazo [1,2-a]pyridin-2-yl)-4-hydroxy pyrrolidin-1-yl)pyrimidin-4-yl)cyclo propane-1-carboxamide, **isomer 2 (I-249)** | **Intermediate 789 table 25** |
| | | ESI-MS [M +H]+: 516.2, ¹H NMR (400 MHz, MeOD) δ 8.33 (d, J=5.1 Hz, 1H), 8.20-8.07 (m, 2H), 7.62 (s, 1H), 7.43 (s, 1H), 7.34 (d, J=9.3 Hz, 1H), 7.25 (d, J=5.2 Hz, 2H), 7.05 (d, J=9.5 Hz, 1H), 5.24-5.04 (m, 1H), 4.90 (s, 1H), 4.59 (s, 1H), 4.07-3.86 (m, 1H), 2.58 (s, 1H), 2.48-2.34 (m, 3H), 1.95-1.89 (m, 1H), 1.56 (s, 2H), 0.99-0.93 (m, 2H), 0.73-0.65 (m, 2H). |
| **250** | 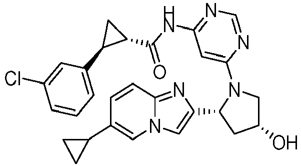 (1S,2S)-2-(3-chlorophenyl)-N-(6-((2R,4R)-2-(6-cyclopropylimidazo [1,2-a]pyridin-2-yl)-4-hydroxy pyrrolidin-1-yl)pyrimidin-4-yl) cyclopropane-1-carboxamide **(I-250)** | **Intermediate 790 table 25** |
| | | ESI-MS [M +H]+: 515.2. ¹H NMR (400 MHz, MeOD) δ 8.24-8.14 (m, 2H), 7.66-7.58 (m, 1H), 7.35 (d, J = 9.4 Hz, 1H), 7.30 - 6.89 (m, 6H), 5.18-5.52 (m, 1H), 4.89 (s, 1H), 4.59 (d, J = 4.1 Hz, 1H), 3.98-3.94 (m, 2H), 2.46-2.32 (m, 3H), 2.18-2.14 (m, 1H), 1.96-1.88 (m, 1H), 1.57 (s, 1H), 1.36 (s, 1H), 1.03-0.86 (m,2H), 0.79-0.58 (m,2H). |
| **251** | 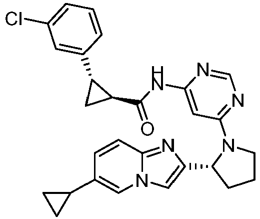 (1S,25)-2-(3-chlorophenyl)-N-(6-((R)-2-(6-cyclopropylimidazo[1,2-a] pyridin-2-yl)pyrrolidin-1-yl) pyrimidin-4-yl)cyclopropane-1-carboxamide **(I-251)** | **Intermediate 791 table 25** |
| | | ESI-MS [M +H]+: 499.2, δ 10.65 (d, J = 46.2 Hz, 1H), 8.34 - 8.09 (m, 2H), 7.57 - 6.83 (m, 8H), 5.55 - 5.29 (m, 1H), 3.90 - 3.69 (m, 1H), 3.68 - 3.49 (m, 1H), 2.46 - 2.24 (m, 2H), 2.21 - 2.09 (m, 1H), 2.06 - 1.85 (m, 3H), 1.58 - 1.35 (m, 3H), 0.99 - 0.80 (m, 2H), 0.74 - 0.52 (m, 2H). |
| **252** | 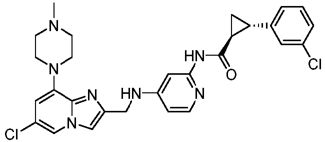 (1S,2S)-N-(4-(((6-chloro-8-(4-methylpiperazin-1-yl)imidazo[1,2-a]pyridin-2-yl)methyl)amino)pyridin-2-yl)-2-(3-chlorophenyl)cyclopropane-1-carboxamide **(I-252)** | 2-bromo-N-((6-chloro-8-(4-methylpiperazin-1-yl)imidazo[1,2-a]pyridin-2-yl)methyl)pyridin-4-amine |
| | | ESI-MS [M +H]+: 550.2, ¹H NMR (400 MHz, DMSO) δ 10.32 (s, 1H), 8.31 (d, J = 1.6 Hz, 1H), 7.76 (d, J = 5.8 Hz, 1H), 7.65 (s, 1H), 7.45 (s, 1H), 7.31 (t, J = 7.9 Hz, 1H), 7.25 (d, J = 7.5 Hz, 2H), 7.14 (d, J = 7.4 Hz, 2H), 6.40 (s, 1H), 6.34-6.31 (m, 1H), 4.37 (d, J = 5.3 Hz, 2H), 3.57 (s, 4H), 3.30 (s, 4H), 2.39 - 2.33 (m, 2H), 2.26 (s, 3H), 1.48 - 1.41 (m, 1H), 1.37-1.32 (m, 1H). |
| **253** | 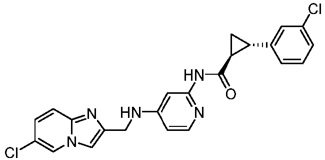 (1S,2S)-N-(4-(((6-chloroimidazo[1,2-a]pyridin-2-yl)methyl)amino)pyridin-2-yl)-2-(3-chlorophenyl)cyclopropane-1-carboxamide **(I-253)** | 2-bromo-N-((6-chloroimidazo[1,2-a]pyridin-2-yl)methyl)pyridin-4-amine |
| | | ESI-MS [M + H ]+: 452.1, ¹H NMR (400 MHz, DMSO) δ 10.33 (s, 1H), 8.80 (dd, J = 2.0, 0.7 Hz, 1H), 7.77 - 7.75 (m, 2H), 7.54 (d, J = 9.6 Hz, 1H), 7.43 (s, 1H), 7.33 - 7.24 (m, 4H), 7.17 - 7.13 (m, 2H), 6.33 (dd, J = 5.8, 2.0 Hz, 1H), 4.39 (d, J = 5.6 Hz, 2H), 2.38 - 2.33 (m, 2H), 1.46 - 1.42 (m, 1H), 1.37 - 1.34 (m, 1H). |
| **254** | 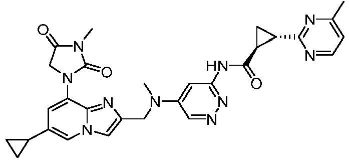 (1S,2S)-N-(5-(((6-cyclopropyl-8-(3-methyl-2,4-dioxoimidazolidin-1-yl) imidazo[1,2-a]pyridin-2-yl)methyl) (methyl)amino)pyridazin-3-yl)-2-(4-methylpyrimidin-2-yl)cyclopropane-1-carboxamide **(I-254)** | **Intermediate 995** in eg 641 |
| | | ESI-MS [M +H]+: 567.3, δ 10.97 (s, 1H), 8.67 (d, J = 2.8 Hz, 1H), 8.52 (d, J = 5.1 Hz, 1H), 8.35 (s, 1H), 8.22 (d, J = 1.1 Hz, 1H), 7.76 (s, 1H), 7.65 (d, J = 2.4 Hz, 1H), 7.38 (d, J = 1.5 Hz, 1H), 7.21 (d, J = 5.1 Hz, 1H), 4.88 (s, 2H), 4.72 (s, 2H), 3.13 (s, 3H), 2.96 (s, 3H), 2.71 - 2.64 (m, 1H), 2.41 (s, 3H), 2.37 - 2.30 (m, 1H), 1.98 - 1.90 (m, 1H), 1.56 - 1.48 (m, 2H), 0.98 - 0.89 (m, 2H), 0.68 - 0.60 (m, 2H). |
| **255** | 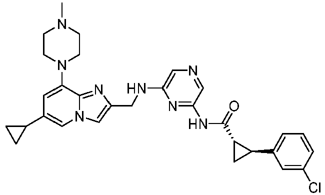 (1S,2S)-2-(3-chlorophenyl)-N-(6-(((6-cyclopropyl-8-(4-methylpiperazin-1-yl)imidazo[1,2-a]pyridin-2-yl)methyl) amino)pyrazin-2-yl)cyclopropane-1-carboxamide **(I-255)** | **Intermediate 793 table 25** |
| | | ESI-MS [M+H]+: 557.2. ¹H NMR (400 MHz, MeOD) δ 8.48 (s, 1H), 7.76 (s, 1H), 7.67 - 7.61 (m, 1H), 7.56 (s, 1H), 7.27 - 7.22 (m, 1H), 7.19 (d, J = 7.5 Hz, 2H), 7.10 (d, J = 7.6 Hz, 1H), 6.37 (s, 1H), 4.64 (s, 2H), 3.35 (d, J = 17.0 Hz, 4H), 2.48 - 2.46 (m, 2H), 2.39 (s, 3H), 2.20 - 2.19 (m, 2H), 2.02 - 2.01 (m, 1H), 1.93 - 1.82 (m, 2H), 1.65 - 1.56 (m, 2H), 0.93 - 0.89 (m, 2H), 0.67 (dt, J = 6.4, 4.7 Hz, 2H). |

### Example 256-258

### rac-(1S*,2S*)-2-(3-amino-5-chlorophenyl)-N-(6-(((6-cyclopropylimidazo[1,2-a]pyridin-2-yl)methyl)amino)pyrimidin-4-yl)cyclopropane-1-carboxamide (I-256)

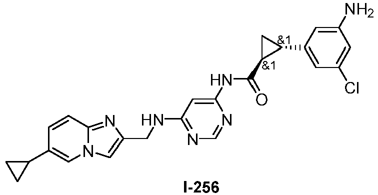

**I-256** was prepared in a similar manner to **(I-144)** and obtained as a mixture of enantiomers. ESI-MS (M+H)+: 474.2, ¹H NMR (400 MHz, DMSO) substantially similar to (I-257)

The mixture was separated using SFC (Daicel CHIRALPAK OZ-H 20 x 250 mm, 5.0 µm, 50/50 MeOH (0.2% NH₄OH) / CO₂, 45 g/min, 35°C) to give two enantiomers: (1S,2S)-2-(3-amino-5-chlorophenyl)-N-(6-(((6-cyclopropylimidazo[1,2-a]pyridin-2-yl)methyl)amino)pyrimidin-4-yl)cyclopropane-1-carboxamide and (1R,2R)-2-(3-amino-5-chlorophenyl)-N-(6-(((6-cyclopropylimidazo[1,2-a]pyridin-2-yl)methyl)amino)pyrimidin-4-yl)cyclopropane-1-carboxamide.

First eluting isomer **(I-257):** ESI-MS (M+H)+: 474.2, δ 10.60 (s, 1H), 8.29 (s, 1H), 8.19 (s, 1H), 7.85 (s, 1H), 7.61 (s, 1H), 7.37 (d, J = 9.3 Hz, 1H), 7.26 (s, 1H), 6.97-6.95 (m, 1H), 6.42-6.41 (m, 1H), 6.29 (d, J = 10.3 Hz, 2H), 5.37 (s, 2H), 4.56 (s, 2H), 2.32 - 2.24 (m, 1H), 2.23 - 2.16 (m, 1H), 1.94 - 1.88 (m, 1H), 1.41 - 1.37 (m, 1H), 1.28 - 1.20 (m, 1H), 0.92 - 0.90 (m, 2H), 0.69 - 0.63 (m, 2H). RT = 3.138 min, 100.0% e.e.

Second eluting isomer **(I-258):** ESI-MS (M+H)+: 474.2, ¹H NMR (400 MHz, DMSO) substantially similar to (I-257). RT = 4.193 min, 100.0% e.e.

### Examples 259-261

### rac-(1S*,2S*)-N-(6-(((6-cyclopropyl-8-(3-methyl-2,4-dioxoimidazolidin-1-yl)imidazo[1,2-a]pyridin-2-yl)methyl)amino)pyrimidin-4-yl)-2-(4-methylpyrimidin-2-yl)cyclopropane-1-carboxamide (I-259)

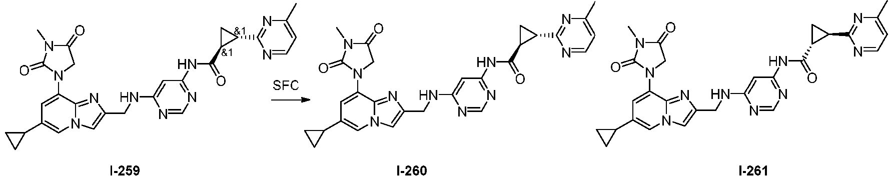

**I-259** was prepared in a similar manner to (**I-144**) from *rac*-(1R,2R)-2-(4-methylpyrimidin-2-yl)cyclopropane-1-carboxamide and **intermediate 365** and obtained as a mixture of enantiomers. ESI-MS [M +H]+: 553.2, ^{1 1}H NMR (400 MHz, DMSO) substantially same as (I-260).

The mixture was separated using SFC (Daicel CHIRALPAK OJ-H 250mm x 20 mm I.D., 5µm. CO2/MeOH (0.1%DEA) = 50/50, 45 g/min, 35°C) to give two enantiomers: (1S,2S)-N-(6-(((6-cyclopropyl-8-(3-methyl-2,4-dioxoimidazolidin-1-yl)imidazo[1,2-a]pyridin-2-yl)methyl)amino)pyrimidin-4-yl)-2-(4-methylpyrimidin-2-yl)cyclopropane-1-carboxamide **(I-260)** and (1R,2R)-N-(6-(((6-cyclopropyl-8-(3-methyl-2,4-dioxoimidazolidin-1-yl)imidazo[1,2-a]pyridin-2-yl)methyl)amino)pyrimidin-4-yl)-2-(4-methylpyrimidin-2-yl)cyclopropane-1-carboxamide **(I-261).**

First eluting isomer **(I-260):**
ESI-MS [M +H]+: 553.3, δ 10.68 (s, 1H), 8.53 (d, J = 5.1 Hz, 1H), 8.24 - 8.18 (m, 2H), 7.88 (s, 1H), 7.69 (s, 1H), 7.32 (d, J = 9.4 Hz, 2H), 7.21 (d, J = 5.1 Hz, 1H), 4.91 (s, 2H), 4.58 (s, 2H), 2.97 (s, 3H), 2.64 - 2.60 (m, 1H), 2.57 - 2.53 (m, 1H), 2.41 (s, 3H), 1.98 - 1.90 (m, 1H), 1.53 - 1.48 (m, 2H), 0.96 - 0.91 (m, 2H), 0.68 - 0.62 (m, 2H). RT = 3.21 min, 100% e.e.

Second eluting isomer **(I-261):**
ESI-MS [M +H]+: 553.3, ¹H NMR (400 MHz, DMSO) substantially same as (I-260) . RT = 6.47min, 100 % e.e.

### Examples 262-264

### rac-(1S*,2S*)-2-(4-chloropyridin-2-yl)-N-(6-(((6-cyclopropyl-8-(3-methyl-2,4-dioxoimidazolidin-1-yl)imidazo[1,2-a]pyridin-2-yl)methyl)amino)pyrimidin-4-yl)cyclopropane-1-carboxamide (I-262)

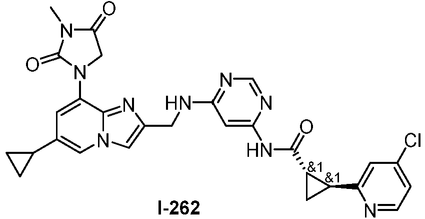

**I-262** was prepared in a similar manner to **(I-144)** from *rac*-(1R,2R)-2-(4-chloropyridin-2-yl)cyclopropane-1-carboxamide and **intermediate 365** and obtained as a mixture of enantiomers. ESI-MS [M +H] +: 572.1, ¹H NMR (400 MHz, DMSO) substantially same as (I-263).

The mixture was separated using SFC (Daicel CHIRALPAK AD-H 250mm × 20 mm I.D. 5µm, CO2/EtOH = 74/26, 50 g/min, 35°C) to give two enantiomers: (1S,2S)-2-(4-chloropyridin-2-yl)-N-(6-(((6-cyclopropyl-8-(3-methyl-2,4-dioxoimidazolidin-1-yl)imidazo[1,2-a]pyridin-2-yl)methyl)amino)pyrimidin-4-yl)cyclopropane-1-carboxamide **(I-264)** and (1R,2R)-2-(4-chloropyridin-2-yl)-N-(6-(((6-cyclopropyl-8-(3-methyl-2,4-dioxoimidazolidin-1-yl)imidazo[1,2-a]pyridin-2-yl)methyl)amino)pyrimidin-4-yl)cyclopropane-1-carboxamide**(I-263)** .

First eluting isomer **(I-263):** ESI-MS [M +H] +: 572.2, δ 10.65 (s, 1H), 8.42 (d, J = 5.3 Hz, 1H), 8.24 (s, 1H), 8.18 (s, 1H), 7.89 (s, 1H), 7.69 (s, 1H), 7.64 (s, 1H), 7.39 - 7.28 (m, 3H), 4.91 (s, 2H), 4.57 (s, 2H), 2.98 (s, 3H), 2.60 - 2.55 (m, 2H), 1.99-1.88 (m, 1H), 1.55 - 1.45 (m, 2H), 0.98-0.90 (m, 2H), 0.68 - 0.59 (m, 2H). RT = 0.815min, 100% e.e.

Second eluting isomer ( **(I-264):** ESI-MS [M +H] +: 572.2, ¹H NMR (400 MHz, DMSOsubstantially same as (I-263). RT = 1.620min, 100% e.e.

### Examples 265-267

### rac-(1S*,2S*)-2-(4-chloropyridin-2-yl)-N-(6-(((6-cyclopropylimidazo[1,2-a]pyridin-2-yl)methyl)amino)pyrimidin-4-yl)cyclopropane-1-carboxamide (I-265)

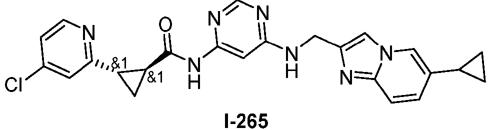

**I-265** was prepared in a similar manner to **(I-144)** and obtained as a mixture of enantiomers. ESI-MS [M +H] +: 460.1, δ = 10.64 (s, 1H), 8.42 (d, J=5.4, 1H), 8.30 (s, 1H), 8.19 (s, 1H), 7.90-7.80 (m, 1H), 7.65 (d, J=1.7, 1H), 7.61 (s, 1H), 7.41-7.32(m, 2H), 7.27 (s, 1H), 6.98-6.93 (m, 1H), 4.56 (s, 2H), 2.63-2.55(m, 2H), 1.94-1.88(m, 1H), 1.58 - 1.41 (m, 2H), 0.94-0.87 (m, 2H), 0.71-0.59 (m, 2H).

The mixture was separated using SFC (Daicel CHIRALPAK OD-H 250mm × 20 mm I.D 5µm, CO2/MeOH (0.2% NH4 ▪ OH ) = 50/50 , 45 g/min, 35°C) to give two enantiomers: (1S,2S)-2-(4-chloropyridin-2-yl)-N-(6-(((6-cyclopropylimidazo[1,2-a]pyridin-2-yl)methyl)amino)pyrimidin-4-yl)cyclopropane-1-carboxamide and (1R,2R)-2-(4-chloropyridin-2-yl)-N-(6-(((6-cyclopropylimidazo[1,2-a]pyridin-2-yl)methyl)amino)pyrimidin-4-yl)cyclopropane-1-carboxamide.

First eluting isomer **(I-266):** ESI-MS [M +H]+: 460.1, δ 10.64 (s, 1H), 8.42 (d, J = 5.4 Hz, 1H), 8.30 (s, 1H), 8.18 (s, 1H), 7.86 (s, 1H), 7.67 - 7.56 (m, 2H), 7.38 - 7.32 (m, 2H), 7.27 (s, 1H), 6.98-6.93 (m, 1H), 4.56 (s, 2H), 2.58-2.51 (m, 2H), 1.94 - 1.87 (m, 1H), 1.55-1.50 (m, 1H), 1.49-1.46 (m, 1H), 0.91-0.88 (m, 2H), 0.68 - 0.63 (m, 2H). RT = 2.462min, 100% e.e.

Second eluting isomer **(I-267):** ESI-MS [M +H]+: 460.1, δ 10.64 (s, 1H), 8.42 (d, J = 5.3 Hz, 1H), 8.30 (s, 1H), 8.18 (s, 1H), 7.86 (s, 1H), 7.65 - 7.61 (m, 2H), 7.36 - 7.34 (m, 2H), 7.27 (s, 1H), 6.98-6.93 (m, 1H), 4.56 (s, 2H), 2.61 - 2.55 (m, 2H), 1.93 - 1.89 (m, 1H), 1.55 - 1.46 (m, 2H), 0.92 - 0.89 (m, 2H), 0.67 - 0.65 (m, 2H). RT = 3.034min, 100 % e.e.

### Examples 268-270

### rac-(1S*,2S*)-2-(4-chloropyridin-2-yl)-N-(4-(((6-cyclopropylimidazo[1,2-a]pyridin-2-yl)methyl)amino)pyridin-2-yl)cyclopropane-1-carboxamide (Example 1-268)

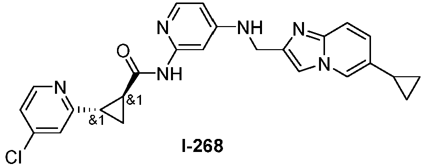

**I-268** was prepared in a similar manner to **(I-144)** and obtained as a mixture of enantiomers. ESI-MS [M +H]+: 459.2, δ 10.34 (s, 1H), 8.41 (d, J = 5.3 Hz, 1H), 8.31 (s, 1H), 7.75 (d, J = 5.7 Hz, 1H), 7.63 (s, 2H), 7.42 (s, 1H), 7.38 (d, J = 9.3 Hz, 1H), 7.35-7.31 (m, 1H), 7.09 (t, J = 5.8 Hz, 1H), 6.99-6.94 (m, 1H), 6.34-6.30 (m, 1H), 4.35 (d, J = 5.5 Hz, 2H), 2.60-2.53 (m, 2H), 1.94-1.86 (m, 1H), 1.51 - 1.44 (m, 2H), 0.95 - 0.87 (m, 2H), 0.71 - 0.61 (m, 2H).

The mixture was separated using SFC(Daicel CHIRALPAK OD-H 250mm × 20 mm I.D. 5 um, CO2/MeOH (0.1% NH4OH) = 50/50. 45 g/min, 35 °C) to give two enantiomers: (1S,2S)-2-(4-chloropyridin-2-yl)-N-(4-(((6-cyclopropylimidazo[1,2-a]pyridin-2-yl)methyl)amino)pyridin-2-yl)cyclopropane-1-carboxamide and (1R,2R)-2-(4-chloropyridin-2-yl)-N-(4-(((6-cyclopropylimidazo[1,2-a]pyridin-2-yl)methyl)amino)pyridin-2-yl)cyclopropane-1-carboxamide.

First eluting isomer **(I-269):** ESI-MS [M +H]+: 459.1. δ 10.35 (s, 1H), 8.41 (d, J=5.3, 1H), 8.31 (s, 1H), 7.75 (d, J=5.8, 1H), 7.63 (d, J=1.9, 2H), 7.45 - 7.29 (m, 3H), 7.12-7.08 (m, 1H), 6.97 (dd, J=9.3, 1.7, 1H), 6.32 (dd, J=5.8, 2.1, 1H), 4.35 (d, J=5.7, 2H), 2.60 - 2.53 (m, 2H), 1.94-1.87 (m, 1H), 1.50-1.42 (m, 2H), 0.94 - 0.87 (m, 2H), 0.69 - 0.62 (m, 2H). RT = 2.20 min, 100.0 % e.e

Second eluting isomer **(I-270):** ESI-MS [M +H]+: 459.2. δ 10.36 (s, 1H), 8.41 (d, J=5.4, 1H), 8.31 (s, 1H), 7.75 (d, J=5.8, 1H), 7.66-7.59 (m, 2H), 7.44 - 7.36 (m, 2H), 7.33 (dd, J=5.3, 2.0, 1H), 7.11 (s, 1H), 6.97 (dd, J=9.3, 1.7, 1H), 6.33 (d, J=3.9, 1H), 4.35 (d, J=5.5, 2H), 2.59 - 2.53 (m, 2H), 1.95 - 1.85 (m, 1H), 1.52 - 1.42 (m, 2H), 0.94 - 0.87 (m, 2H), 0.70 - 0.62 (m, 2H). RT = 3.29 min, 100.0 % e.e

### Examples 271-273

### rac-(1S*,2S*)-6'-chloro-N-(4-(((6-cyclopropylimidazo[1,2-a]pyridin-2-yl)methyl) amino)pyridin-2-yl)-2',3'-dihydrospiro[cyclopropane-1,1'-indene]-2-carboxamide (I-271)

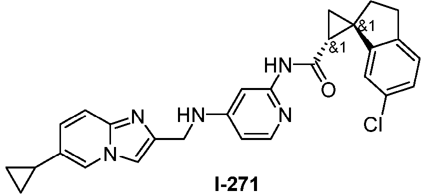

**I-271** was prepared in a similar manner to (I-144) and obtained as a mixture of enantiomers. ESI-MS [M +H]+: 484.2. δ 10.23 (s, 1H), 8.30 (s, 1H), 7.73 (d, J = 5.8 Hz, 1H), 7.64 (s, 1H), 7.44 (s, 1H), 7.38 (d, J = 9.3 Hz, 1H), 7.28 - 7.05 (m, 3H), 6.97 (dd, J = 9.3, 1.7 Hz, 1H), 6.88 (d, J = 1.8 Hz, 1H), 6.31 (dd, J = 5.8, 2.0 Hz, 1H), 4.36 (d, J = 5.6 Hz, 2H), 3.03 - 2.78 (m, 2H), 2.44 - 2.42 (m, 1H), 2.24 - 2.11 (m, 2H), 1.98 - 1.84 (m, 1H), 1.60 - 1.40 (m, 2H), 0.98 - 0.85 (m, 2H), 0.75 - 0.59 (m, 2H).

The mixture was separated using SFC (Daicel CHIRALPAK OD-H, 250mm × 20 mm I.D. CO₂/MeOH (0.1% NH4OH) = 50/50. 50 g/min, 35 °C) to give two enantiomers: (1S,2S)-6'-chloro-N-(4-(((6-cyclopropylimidazo[1,2-a]pyridin-2-yl)methyl)amino)pyridin-2-yl)-2',3'-dihydrospiro[cyclopropane-1,1'-indene]-2-carboxamide and (1R,2R)-6'-chloro-N-(4-(((6-cyclo propylimidazo[1,2-a]pyridin-2-yl)methyl)amino)pyridin-2-yl)-2',3'-dihydrospiro[cyclopropane-1,1'-indene]-2-carboxamide. RT = 2.15 min, 100.0 % e.e

First eluting isomer **(I-272):** ESI-MS [M +H]+: 484.1, δ 10.22 (s, 1H), 8.30 (s, 1H), 7.73 (d, J = 5.8 Hz, 1H), 7.64 (s, 1H), 7.44 (s, 1H), 7.38 (d, J = 9.3 Hz, 1H), 7.25 - 7.08 (m, 3H), 6.97 (dd, J = 9.3, 1.7 Hz, 1H), 6.88 (d, J = 1.8 Hz, 1H), 6.31 (d, J = 3.9 Hz, 1H), 4.35 (d, J = 5.7 Hz, 2H), 3.00 - 2.79 (m, 2H), 2.48 - 2.40 (m, 1H), 2.23 - 2.09 (m, 2H), 1.95 - 1.86 (m, 1H), 1.58 - 1.42 (m, 2H), 0.97 - 0.86 (m, 2H), 0.72 - 0.60 (m, 2H). RT = 2.15 min, 100.0 % e.e

Second eluting isomer **(I-273):** ESI-MS [M +H]+: 484.1, NMR (400 MHz, DMSO) substantially same as (I-272).. RT = 3.52 min, 100.0% e.e

### Example 274: (1S,2S)-2-(3-chlorophenyl)-N-(6-(((6-cyclopropyl-8-(N-methylacetamido)imidazo [1,2-a]pyridin-2-yl)methyl)amino)pyrimidin-4-yl)cyclopropane-1-carboxamide (I-274)

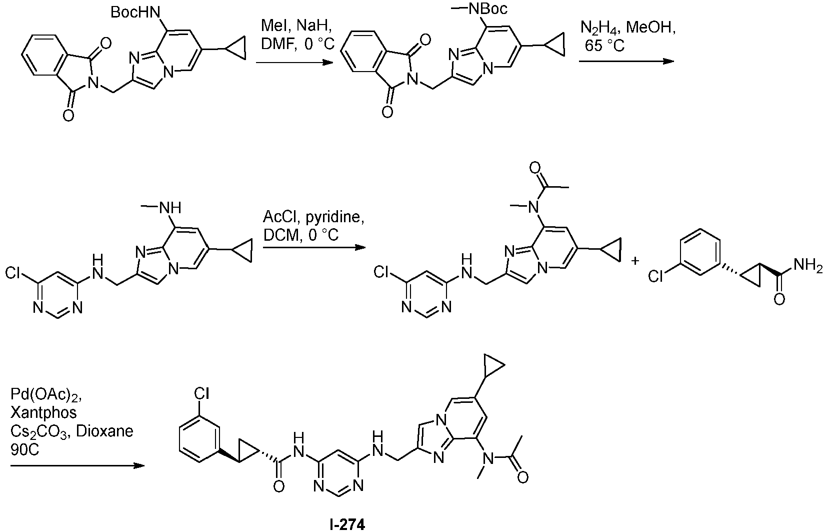

DUPLICATE OF INTERMEDIATE 734**Intermediate 794:** *tert-**butyl (2-(aminomethyl)-6-cyclopropylimidazo[1,2-a]pyridin-8-yl)(methyl)carbamate**.* Using a similar procedure to that for **intermediate 278,** the title compound (260 mg, quant.) was synthesized from intermediate **734** using hydrazine hydrate. ESI-MS (M+H)+: 317.4

**Intermediate 795:** *tert**-butyl (2-(((6-chloropyrimidin-4-yl)amino)methyl)-6-cyclopropylimidazo[1,2-a]pyridin-8-yl)(methyl)carbamate.*** Using a similar procedure to that used for **intermediate 739,** the title compound (160 mg, 40%) was made from 2,4-dichloropyrimidine and **intermediate 794.** ESI-MS (M+H)+: 429.3

**Intermediate 796: *2-(((6-chloropyrimidin-4-yl)amino)methyl)-6-cyclopropyl-N-methylimidazo[1,2-a]pyridin-8-amine.*** Trifluoroacetic acid (0.11 mL, 1.49 mmol) was added to a solution of **intermediate 795** (160 mg, 0.37 mmol) in DCM (5.0 mL), and the reaction mixture was stirred at room temperature for 2 h. The reaction mixture was quenched with NaHCO₃ (sat. aq., 35 mL) and extracted with DCM (2 × 50 mL). The combined organics were passed through a hydrophobic frit and concentrated *in vacuo* to give the title compound (126 mg, quant %). ESI-MS (M+H)+: 329.3.

**Intermediate 797: *N-(2-(((6-chloropyrimidin-4-yl)amino)methyl)-6-cyclopropylimidazo[1,2-a]pyridin-8-yl)-N-methylacetamide.*** Acetyl chloride (26 µL, 0.36 mmol) was added to a solution of **intermediate 796** (120 mg, 0.36 mmol) and pyridine (0.088 mL, 1.09 mmol) in DCM (1.0 mL) at 0 °C, and the reaction mixture was stirred at 0 °C for 2 h. The reaction mixture was allowed to warm to room temperature, quenched with water (5 mL), and extracted with DCM (3×15 mL). The combined organics were passed through a hydrophobic frit and concentrated *in vacuo.* The residue was purified by column chromatography on silica gel, eluting with a gradient of 0-10 % (7N NH₃ in MeOH) in DCM to give the title compound which was used directly in the next reaction. ESI-MS (M+H)+: 371.3.

**(1S,2S)-2-(3-chlorophenyl)-N-(6-(((6-cyclopropyl-8-(N-methylacetamido)imidazo[1,2-a]pyridin-2-yl)methyl)amino)pyrimidin-4-yl)cyclopropane-1-carboxamide (I-274).** Using a similar procedure to that used for **(I-144),** the title compound was prepared using intermediate 119 and **intermediate 797** then purified by column chromatography on silica gel, eluting with a gradient of 0-10 % (7N NH₃ in MeOH) in DCM (0.63 mg). ESI-MS (M+H)+:530.4, δ 10.64 (s, 1H), 8.38 (s, 1H), 8.25 (s, 1H), 7.99 - 7.92 (m, 1H), 7.74 (s, 1H), 7.40 - 7.29 (m, 4H), 7.22 - 7.18 (m, 1H), 7.10 (s, 1H), 4.63 - 4.61 (m, 2H), 3.25 (br s, 3H), 2.47 - 2.37 (m, 2H), 2.02 - 1.94 (m, 1H), 1.84 (br s, 3H), 1.54 - 1.42 (m, 2H), 1.01 - 0.95 (m, 2H), 0.79 - 0.73 (m, 2H).

### Example 275: (1S,2S)-N-(6-(((8-acetamido-6-cyclopropylimidazo[1,2-a]pyridin-2-yl)methyl) amino)pyrimidin-4-yl)-2-(3-chlorophenyl)cyclopropane-1-carboxamide (I-275)

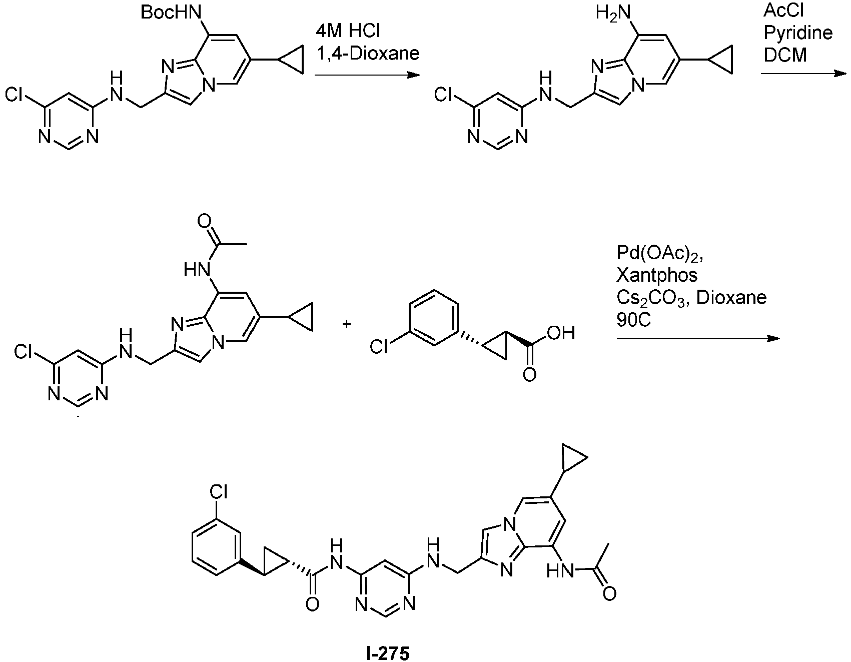

A solution of HCl (4M in 1,4-Dioxane, 0.16 mL) was added to a solution of **intermediate 740** (175 mg, 0.42 mmol) in 1,4-dioxane (5.0 mL), followed by a few drops of water. The resulting solution was stirred for 2 h. Further HCl (4M in 1,4-Dioxane, 0.5 mL) was added, then then mixture was stirred for 18 h at room temperature, then for 2 h at reflux. NaHCO₃ (sat. aq., 10 mL) was added, and then the mixture was extracted with EtOAc (3 x 10 mL). The combined aqueous layers were passed through a hydrophobic frit, then concentrated *in vacuo.* The residue was purified by column chromatography on silica gel, eluting with a gradient of 0-10 % (7N NH₃ in MeOH) in DCM to give **2-(((6-chloropyrimidin-4-yl)amino)methyl)-6-cyclopropylimidazo[1,2-a]pyridin-8-amine intermediate 798** (61 mg, 46 %). ESI-MS (M+H)+: 315.1

Using a similar procedure to that used for the synthesis of **intermediate 797, N-(2-(((6-chloropyrimidin-4-yl)amino)methyl)-6-cyclopropylimidazo[1,2-a]pyridin-8-yl)acetamide intermediate 799** was prepared from **intermediate 798** (35 mg, 55%). ESI-MS (M+H)+: 357.1.

### (1S,2S)-N-(6-(((8-acetamido-6-cyclopropylimidazo[1,2-a]pyridin-2-yl)methyl)amino)pyrimidin-4-yl)-2-(3-chlorophenyl)cyclopropane-1-carboxamide (I-275).

Using a similar procedure to that used for **(I-144),** the title compound was prepared using intermediate 119 and **intermediate 799** and purified by column chromatography on silica gel, eluting with a gradient of 0-20 % (7N NH₃ in MeOH) in DCM followed by preparative HPLC (7.1 mg, 14%). ESI-MS (M+H)+:516.2, δ 10.63 (s, 1H), 9.89 (s, 1H), 8.23 - 8.23 (m, 1H), 8.05 (s, 1H), 7.91 - 7.86 (m, 1H), 7.79 - 7.76 (m, 1H), 7.65 (s, 1H), 7.37 - 7.28 (m, 4H), 7.18 (d, J=7.6 Hz, 1H), 4.63 - 4.63 (m, 2H), 2.47 - 2.37 (m, 2H), 2.22 (s, 3H), 1.96 - 1.88 (m, 1H), 1.53 - 1.39 (m, 2H), 0.96 - 0.90 (m, 2H), 0.66 - 0.60 (m, 2H).

### Example 276: (1S,2S)-N-(6-(((R)-1-(6-cyclopropyl-8-(3-methyl-2,4-dioxoimidazolidin-1-yl)imidazo[1,2-a]pyridin-2-yl)ethyl)amino)-2-(2-(dimethylamino)ethoxy)pyrimidin-4-yl)-2-(4-methylpyrimidin-2-yl)cyclopropane-1-carboxamide (I-276)

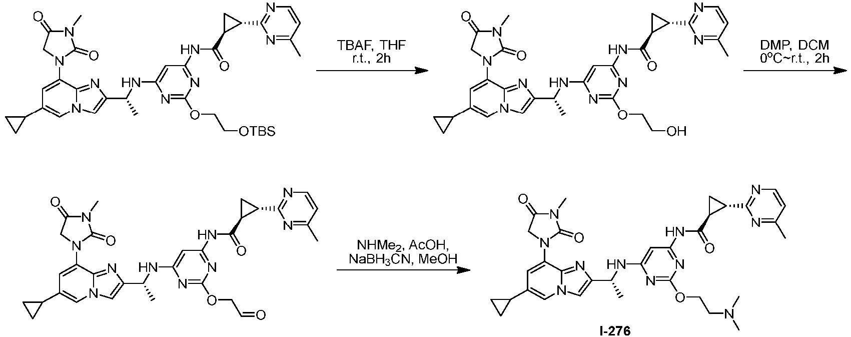

To a solution of *(1*S, *2S*)-*N*-(2-(2-((tert-butyldimethylsilyl)oxy)ethoxy)-6-(((R)-1-(6-cyclopropyl-8-(3-methyl-2,4-dioxoimidazolidin-1-yl)imidazo[1,2-a]pyridin-2-yl)ethyl)amino)pyrimidin-4-yl)-2-(4-methylpyrimidin-2-yl)cyclopropane-1-carboxamide (170 mg, 0.23 mmol) in THF (10 mL) was added TBAF (0.7 mL, 1M solution in THF, 0.7 mmol). The resulting reaction was stirred at room temperature for 2 h. The reaction was concentrated in vacuo to give the crude, which was purified with preparative TLC (eluent: DCM / MeOH = 10/1) to give ***(1*S*,2*S*)-N*-(6-(((*R*)-1-(6-cyclopropyl-8-(3-methyl-2,4-dioxoimidazolidin-1-yl)imidazo[1,2-a]pyridin-2-yl)ethyl)amino)-2-(2-hydroxyethoxy)pyrimidin-4-yl)-2-(4-methylpyrimidin-2-yl)cyclopropane-1-carboxamide intermediate 800** (80 mg, 56%) as a yellow solid. ESI-MS [M +H]+: 627.2.

To a solution of **intermediate 800** (80 mg, 0.128 mmol) in DCM (10 mL) was added Dess-Martin Periodinane (108 mg, 0.255 mmol). The resulting reaction was stirred at room temperature for 2 h. The reaction was quenched with saturated aqueous NaHCO₃ (30 mL) and extracted with DCM (3 x 30 mL). The combined organic layers were washed with brine (30 mL), dried over Na2SO4, and concentrated in vacuo to give the crude, which was purified with preparative TLC (eluent: DCM / MeOH = 15/1) to give **(1S,2S)-N-(6-(((R)-1-(6-cyclopropyl-8-(3-methyl-2,4-dioxoimidazolidin-1-yl)imidazo[1,2-a]pyridin-2-yl)ethyl)amino)-2-(2-oxoethoxy)pyrimidin-4-yl)-2-(4-methylpyrimidin-2-yl)cyclopropane-1-carboxamide intermediate 801** (30 mg, 37.5%) as a yellow solid. ESI-MS [M +H]+: 625.2.

A solution of **intermediate 801** (30 mg, 0.048 mmol), NHMe₂ (22 mg, 0.48 mmol), and AcOH (1 drop) in MeOH (3 mL) was stirred at room temperature for 30 min. Then NaBH₃CN (10 mg, 0.144 mmol) was added to the mixture, which was then stirred at room temperature for 3 h. The reaction was quenched with saturated aqueous NaHCO₃ (20 mL) and extracted with DCM (3 x 20 mL). The combined organic layers were washed with brine (30 mL), dried over Na₂SO₄, and concentrated in vacuo to give the crude, which was purified with preparative TLC (eluent: DCM / MeOH =10/1) to give **(I-276)** (12 mg, 38.8%) as a white solid. ESI-MS (M+H)+: 654.3, δ 10.52 (s, 1H), 8.52 (d, J = 5.1 Hz, 1H), 8.23 (d, J = 1.1 Hz, 1H), 7.83 (s, 1H), 7.64 (s, 1H), 7.33 (d, J = 1.3 Hz, 1H), 7.21 (d, J = 5.1 Hz, 1H), 6.98 (s, 1H), 5.31 (s, 1H), 4.93 (s, 2H), 4.26 - 4.17 (m, 2H), 2.98 (s, 3H), 2.64 - 2.58 (m, 1H), 2.56 - 2.51 (m, 2H), 2.48 - 2.45 (m, 1H), 2.41 (s, 3H), 2.15 (s, 6H), 1.96 - 1.89 (m, 1H), 1.56 - 1.46 (m, 5H), 0.96 - 0.91 (m, 2H), 0.67 - 0.61 (m, 2H)..

### Example 277: rac-(1S*,2S*)-2-(2-amino-6-methoxypyridin-4-yl)-N-(6-(((6-cyclo propylimidazo[1,2-a]pyridin-2-yl)methyl)amino)pyrimidin-4-yl)cyclopropane-1-carboxamide (I-277)

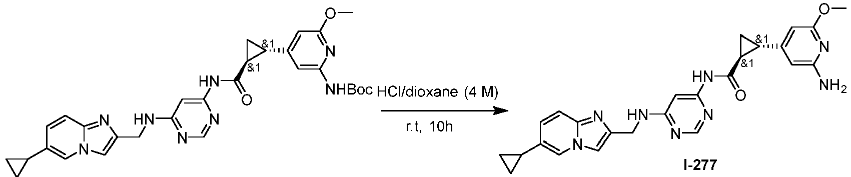

To a solution of *rac-tert*-butyl (4-((1S*,2S*)-2-((6-(((6-cyclopropylimidazo[1,2-a]pyridin-2-yl)methyl)amino)pyrimidin-4-yl)carbamoyl)cyclopropyl)-6-methoxypyridin-2-yl)carbamate (78 mg, 0.137 mmol) in MeOH (2 mL) was added HCl (4 M solution in dioxane, 2 mL). The resulting mixture was stirred at room temperature for 10 h. The reaction mixture was diluted in water (40 mL), adjusted to pH = 9~10 with NaHCO₃ aqueous (10 mL), and extracted with DCM (2 x 30 ml). The combined organics were washed with brine (60 mL), dried over Na₂SO₄, concentrated, and dried *in vacuo* to give **(I-277)** as a mixture of enantiomers. ESI-MS (M+H)+: 471.3, ¹H NMR (400 MHz, MeOD) δ 8.20 - 8.14 (m, 2H), 7.61 (s, 1H), 7.37 (d, J = 9.3 Hz, 1H), 7.32 (d, J = 0.8 Hz, 1H), 7.06 (dd, J = 9.4, 1.7 Hz, 1H), 5.93 (d, J = 0.9 Hz, 1H), 5.79 (d, J = 0.9 Hz, 1H), 4.64 (s, 2H), 3.77 (s, 3H), 2.32 - 2.26 (m, 1H), 2.23 - 2.17 (m, 1H), 1.96 - 1.89 (m, 1H), 1.58 - 1.52 (m, 1H), 1.40 - 1.33 (m, 1H), 0.99 - 0.94 (m, 2H), 0.73 - 0.67 (m, 2H).

### Example 278: rac-(1S*,2S*)-2-(4-amino-3-chlorophenyl)-N-(6-(((6-cyclopropylimidazo[1,2-a]pyridin-2-yl)methyl)amino)pyrimidin-4-yl)cyclopropane-1-carboxamide (I-278)

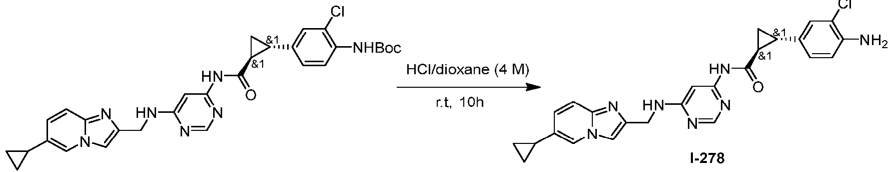

**(I-278)** was prepared in a similar manner to **(I-277).** ESI-MS (M+H)+: 474.2, δ 10.55 (s, 1H), 8.31 - 8.27 (m, 1H), 8.18 (d, J = 3.6 Hz, 1H), 7.83 (s, 1H), 7.61 (s, 1H), 7.37 (d, J = 9.3 Hz, 1H), 7.26 (s, 1H), 7.00 (d, J = 1.9 Hz, 1H), 6.97 (dd, J = 9.3, 1.7 Hz, 1H), 6.85 (dd, J = 8.3, 1.9 Hz, 1H), 6.71 (d, J = 8.3 Hz, 1H), 5.20 (s, 2H), 4.56 (s, 2H), 2.26 - 2.15 (m, 2H), 1.95 - 1.87 (m, 1H), 1.39 - 1.32 (m, 1H), 1.27 - 1.21 (m, 1H), 0.94 - 0.88 (m, 2H), 0.69 - 0.63 (m, 2H).

### Example 279: rac-(18% 28^{%})-2-(2-amino-5-chlorophenyl)-N-(6-(((6-cyclopropylimidazof1,2-a]pyridin-2 yl)methyl)amino)pyrimidin-4yl)cyclopropane-1-carboxamide (I-279)

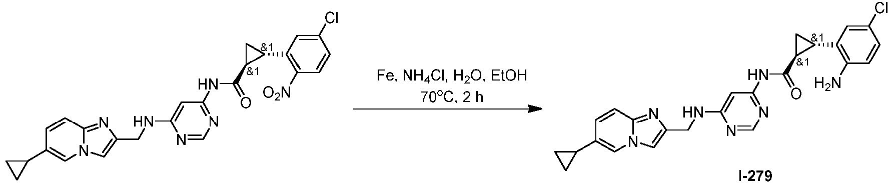

A mixture of **intermediate 802** from eg 316 (70 mg, 0.14 mmol), NH₄Cl (60 mg, 1.12 mmol), and Fe (39 mg, 0.7 mmol) in water (0.5 ml) and EtOH (2 ml) were stirred at 70 °C for 2 h. After the reaction was completed, water (10 ml) was added, and the mixture was extracted with DCM (3 x 10 ml), washed with brine (2 x 10 ml), dried over Na₂SO₄, and concentrated to give the crude, which was purified by preparative TLC (DCM:MeOH = 15:1) to afford **(I-279)** (30 mg, 45%) as a yellow solid. ESI-MS (M+H)+:474.2, δ 10.67 (s, 1H), 8.32 (s, 1H), 8.21 (s, 1H), 7.87 (s, 1H), 7.63 (s, 1H), 7.39 (d, J = 9.3 Hz, 1H), 7.29 (s, 1H), 7.02 - 6.94 (m, 3H), 6.65 - 6.62 (m, 1H), 5.11 (s, 2H), 4.58 (s, 2H), 2.32 - 2.26 (m, 1H), 2.18 - 2.10 (m, 1H), 1.97 - 1.86 (m, 1H), 1.46 - 1.38 (m, 1H), 1.11 - 1.02 (m, 1H), 0.95 - 0.87 (m, 2H), 0.70 - 0.62 (m, 2H).

### Example 280: rac-(1S*,2S*)-2-(2-amino-3-chlorophenyl)-N-(6-(((6-cyclopropylimidazo[1,2-a]pyridin-2-yl)methyl)amino)pyrimidin-4-yl)cyclopropane-1-carboxamide (I-280)

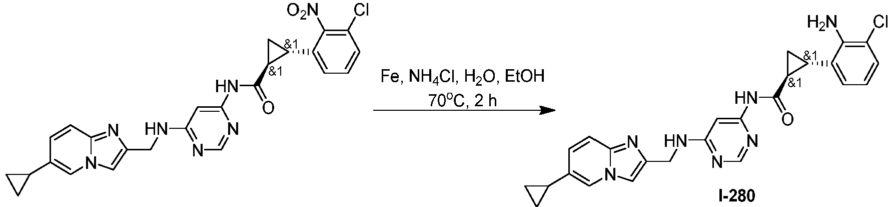

**(I-280)** was prepared in a similar manner to **(I-279).** ESI-MS (M+H)+: 474.2, δ 10.67 (s, 1H), 8.32 (s, 1H), 8.21 (s, 1H), 7.87 (s, 1H), 7.64 (s, 1H), 7.40 (d, J = 9.3 Hz, 1H), 7.31 (s, 1H), 7.16 - 7.11 (m, 1H), 7.04 - 6.90 (m, 2H), 6.55 (t, J = 7.8 Hz, 1H), 5.04 (s, 2H), 4.58 (s, 2H), 2.30 - 2.20 (m, 2H), 1.97 - 1.86 (m, 1H), 1.50 - 1.39 (m, 1H), 1.19 - 1.07 (m, 1H), 0.97 - 0.87 (m, 2H), 0.71 - 0.61 (m, 2H).

### Example 281: (1S,2S)-2-(3-chlorophenyl)-N-(6-(((6-cyclopropyl-8-(1-hydroxyethyl)imidazo[1,2-a]pyridin-2yl)methyl)amino)pyrimidin-4yl)cyclopropane-1-carboxamide (I-281)

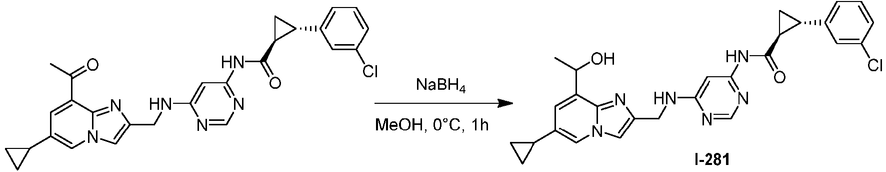

To a solution of (1S,2S)-N-(6-(((8-acetyl-6-cyclopropylimidazo[1,2-a]pyridin-2-yl)methyl)amino)pyrimidin-4-yl)-2-(3-chlorophenyl)cyclopropane-1-carboxamide (25 mg, 0.05 mmol) in MeOH (5 mL) was added NaBH₄ (0.95 mg, 0.025 mmol) slowly under stirring at 0 °C. The mixture was further stirred for 1 h at 0 °C, and then stirred at room temperature for another 1 h. The mixture was concentrated *in vacuo* and purified by preparative HPLC to give **(I-281)**(4 mg, 16 %) as a white solid. ESI-MS (M+H)+: 503.2, δ 10.60 (s, 1H), 8.40 (s, 1H), 8.18 (d, J = 9.6 Hz, 2H), 7.88 (s, 1H), 7.59 (s, 1H), 7.32 - 7.25 (m, 4H), 7.16 - 7.14 (m, 1H), 7.00 (s, 1H), 5.33 (s, 1H), 5.20 - 5.16 (m, 1H), 4.57 (s, 2H), 2.42 - 2.38 (m, 2H), 1.94 - 1.90 (m, 1H), 1.46 - 1.41 (m, 5H), 0.93 - 0.90 (m, 2H), 0.67 - 0.63 (m, 2H).

### Example 282: (1S,2S)-2-(3-chlorophenyl)-N-(4-((2R,4S)-2-(6-cyclopropylimidazo[1,2-a]pyridin-2-yl)-4-hydroxypyrrolidin-1-yl)pyridin-2-yl)cyclopropane-1-carboxamide (I-282)

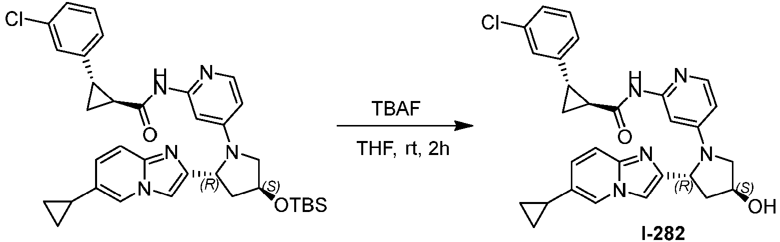

To a solution of (1S,2S)-N-(4-((2R,4S)-4-((*tert*-butyldimethylsilyl)oxy)-2-(6-cyclopropylimidazo[1,2-a]pyridin-2-yl)pyrrolidin-1-yl)pyridin-2-yl)-2-(3-chlorophenyl)cyclopropane-1-carboxamide (10 mg, 0.016 mmol) in THF (4mL) was added TBAF (1.0M, 0.03 mL). After stirring the mixture at room temperature for 2 h, water (10 mL) was added, and the mixture was then extracted with EtOAc (3 x 30 mL). The combined organic layers were washed with brine, dried over Na₂SO₄, filtered, and concentrated *in vacuo* to give the crude, which was purified by preparative TLC to give **(I-282)** (4.7mg, 57%) as a white solid. ESI-MS [M +H]+:514.2., ¹H NMR (400 MHz, MeOD) δ 8.10 (s, 1H), 7.73 (d, J = 6.0 Hz, 1H), 7.55 (s, 1H), 7.35 - 7.31 (m, 2H), 7.29-7.22 (m, 1H), 7.21-7.16 (m, 2H), 7.13-7.03 (m, 2H), 6.28 (d, J = 4.8 Hz, 1H), 5.14 - 5.10 (m, 1H), 4.92 - 4.87 (m, 1H), 4.63 - 4.60 (m, 1H), 4.00 - 3.92 (m, 2H), 3.50 - 3.45 (m, 1H),2.48 - 2.40 (m, 2H), 2.39 - 2.30 (m, 1H), 2.24 - 2.17 (m, 1H), 1.99 - 1.87 (m, 1H), 1.64 - 1.55 (m, 1H), 1.44 - 1.37 (m, 1H), 1.06 - 0.85 (m, 2H), 0.78 - 0.67 (m, 2H)..

### Example 283: (1S,2S)-2-(3-chlorophenyl)-N-(6-((2R,4S)-2-(6-cyclopropylimidazo[1,2-a]pyridin-2-yl)-4-hydroxypyrrolidin-1-yl)pyrimidin-4-yl)cyclopropane-1-carboxamide (I-283)

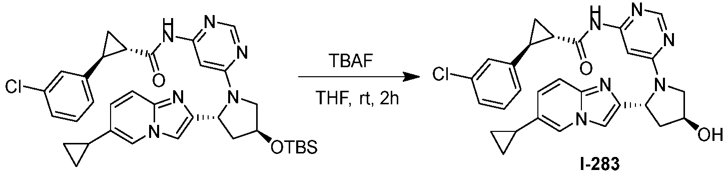

**(I-283)** was prepared in a similar manner to **(I-282).** ESI-MS [M +H]+:515.2, ¹H NMR (400 MHz, MeOD) δ 8.14 (d, J = 16.8 Hz, 2H), 7.63 (s, 1H), 7.34 (d, J = 9.3 Hz, 1H), 7.30-7.15 ( m, 4H), 7.12-7.00 (m, 2H), 5.15 (s, 1H), 4.93 (s, 1H), 4.60 - 4.56 (m, 1H), 4.07-3.93 (m, 1H), 2.53-2.34 (m, 3H), 2.12 (s, 1H), 1.97-1.86 (m, 1H), 1.51 (s, 1H), 1.33 (d, J = 32.6 Hz, 1H), 1.01 - 0.91 (m, 2H), 0.78 - 0.63 (m, 2H).

### Example 284: (1S,2S)-N-(4-(((8-(azetidin-3-yl)-6-cyclopropylimidazo[1,2-a]pyridin-2-yl)methyl)amino)pyridin-2-yl)-2-(3-chlorophenyl)cyclopropane-1-carboxamide (I-284)

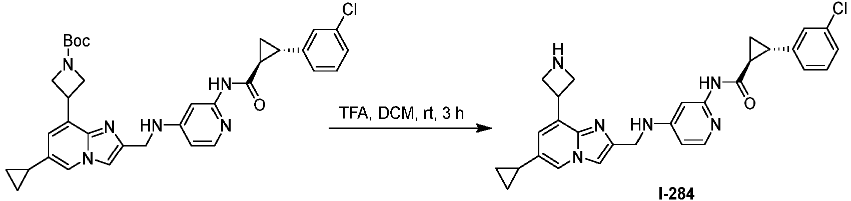

To a solution of *tert*-butyl 3-(2-(((2-((1S,2S)-2-(3-chlorophenyl)cyclopropane-1-carboxamido)pyridin-4-yl)amino)methyl)-6-cyclopropylimidazo[1,2-a]pyridin-8-yl)azetidine-1-carboxylate (30 mg, 0.049 mmol) in DCM (10.0 mL) was added TFA (1 mL) at 0 °C. After stirring at room temperature for 3 h, the mixture was concentrated *in vacuo* to give the crude, which was neutralized with a solution of ammonia in methanol, then concentrated *in vacuo* and purified by Pre-TLC (DCM/MeOH= 10/1) to give the product **(I-284)** (6 mg, 24%) as a white solid. ESI-MS [M +H]+: 513.2. δ 10.31 (s, 1H), 8.21 (s, 1H), 7.76 (d, J = 5.6 Hz, 1H), 7.63 (s, 1H), 7.44 (s, 1H), 7.35 - 7.20 (m, 3H), 7.18 - 7.06 (m, 2H), 6.96 (s, 1H), 6.32 (d, J = 5.5 Hz, 1H), 4.36 (d, J = 5.1 Hz, 2H), 4.32 - 4.08 (m, 2H), 3.93 (d, J = 8.1 Hz, 3H), 2.36-2.32 (m, 2H), 1.94-1.91 (m, 1H), 1.45-1.42 (m, 1H), 1.37-1.32 (m, 1H), 0.93-0.90 (m, 2H), 0.71-0.38 (m, 2H).

### Example 285: (1S,2S)-2-(3-chlorophenyl)-N-(6-(((6-cyclopropyl-8-(4-methylpiperazin-1-yl)imidazo[1,2-a]pyridin-2-yl)methyl)amino)-2-hydroxypyrimidin-4-yl)cyclopropane-1-carboxamide (I-285)

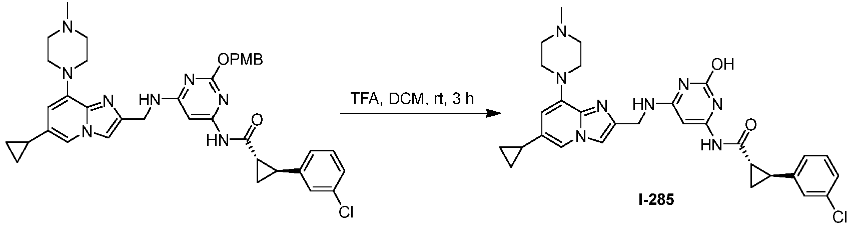

**(I-285)** was prepared in a similar manner to **(I-285).** ESI-MS [M +H]+: 573.2, δ 10.89 (s, 1H), 10.63- 10.18 (m, 1H), 8.10 (s, 1H), 7.89 (s, 1H), 7.58 (s, 1H), 7.33 - 7.25 (m, 3H), 7.17-7.15 (m, 1H), 6.16 (s, 1H), 5.77 (s, 1H), 4.48 (s, 2H), 3.48 (s, 4H), ), 2.50- 2.49 (m, 4H), 2.46- 2.44 (m, 2H), 2.24 (s, 3H), 1.89-1.82 (m, 1H), 1.54 - 1.43 (m, 2H), 0.89 - 0.84 (m, 2H), 0.67 - 0.64 (m, 2H).

### Example 286: rac-(1S*2S*)-2-(2-amino-6-methoxypyridin-4yl)-N-(4(((6-cyclopropylimidazo[1,2-1]pyridin-2-yl)methyl)amino)pyridin-2-yl)cyclopropane-1-carboxamide (I-286)

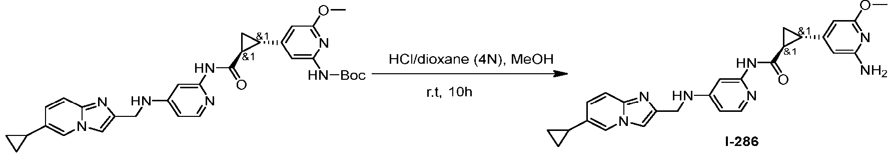

To a solution of **(I-216)** (85 mg, 0.149 mmol) in MeOH (2 mL) was added HCl (4 M in dioxane, 3 mL). After stirring at room temperature for 10 h, the reaction mixture was diluted in water (20 mL), basified to pH = 9~10 with saturated aq. NaHCO₃ (10 mL), and extracted with DCM (2 x 30 ml). The combined organics were washed with brine (60 mL), dried over anhydrous Na₂SO₄, and concentrated and dried *in vacuo* to give (**I-286**)(33 mg, 47%) as a white solid. ESI-MS [M + H]+: 470.1, ¹H NMR (400 MHz, MeOD) δ 8.13 (s, 1H), 7.75 (d, J = 6.0 Hz, 1H), 7.62 (s, 1H), 7.40 - 7.31 (m, 2H), 7.06 (dd, J = 9.4, 1.7 Hz, 1H), 6.38 (dd, J = 6.0, 2.2 Hz, 1H), 5.92 (d, J = 0.9 Hz, 1H), 5.79 (d, J = 1.0 Hz, 1H), 4.48 (s, 2H), 3.77 (s, 3H), 2.31 - 2.25 (m, 1H), 2.20 - 2.15 (m, 1H), 1.96 - 1.88 (m, 1H), 1.57 - 1.52 (m, 1H), 1.36 - 1.31 (m, 1H), 0.98 - 0.93 (m, 2H), 0.73 - 0.66 (m, 2H).

### Example 287: rac-(1S*,2S*)-2-(5-chloro-2-(1H-tetrazol-1-yl)phenyl)-N-(4-(((6-cyclopropylimidazo[1,2-a]pyridin-2-yl)methyl)amino)pyridin-2-yl)cyclopropane-1-carboxamide (I-287)

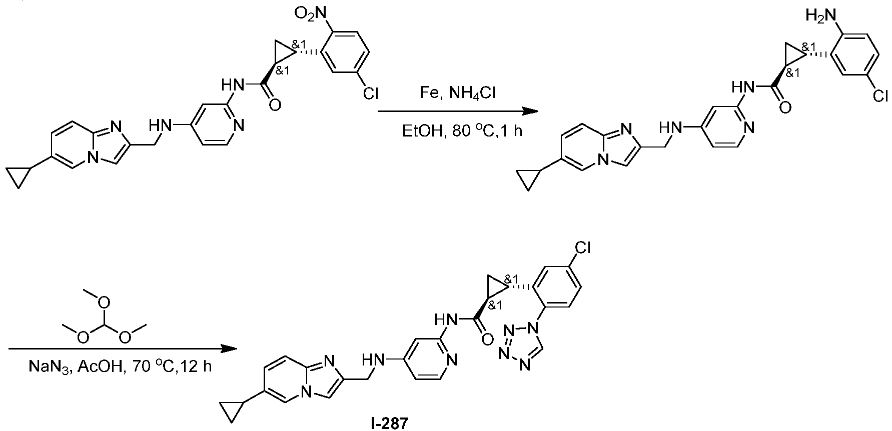

To a solution of rac-(1S*,25*)-2-(5-chloro-2-nitrophenyl)-N-(4-(((6-cyclopropylimidazo [1,2-a]pyridin-2-yl)methyl)amino)pyridin-2-yl)cyclopropane-1-carboxamide (90 mg, 0.18 mmol) in EtOH (4 mL) were added Fe (50 mg, 0.90 mmol), NH₄Cl (77 mg, 1.43 mmol), and water (1 mL). After stirring at 80 °C for 1 h, the resulting mixture was cooled to room temperature and filtered, and the residue was washed with MeOH/DCM (2/1, 5 x 6 mL). The organic layers were concentrated in vacuo and purified by Pre-TLC (eluent: DCM/MeOH = 15/1) to give rac-(1S*,2S*)-**2-(2-amino-5-chlorophenyl)-N-(4-(((6-cyclopropylimidazo[1,2-a]pyridin-2-yl)methyl)amino) pyridin-2-yl)cyclopropane-1-carboxamide (I-222)** (60 mg, 71%) as a pale yellow solid. ESI-MS [M + H]+: 473.2.

To a solution of **(I-222)** (50 mg, 0.11 mmol) in trimethoxymethane (2 mL) was added NaN₃ (21 mg, 0.32 mmol) at 0 °C . After stirring at 0 °C for 40 min, AcOH (4 mL) was added and the resulting mixture was stirred at 70 °C for 12 h. The reaction mixture was cooled to room temperature, then quenched by adding water (20 mL) and extracted with EtOAc (4 x 20mL). The organic layers were concentrated in vacuo and purified by preparative TLC (eluent: MeOH/DCM = 15/1) to give **(I-287)** (5.7 mg, 10%) as a white solid. ESI-MS [M + H] +: 526.1, δ 10.26 (s, 1H), 9.84 (s, 1H), 8.31 (s, 1H), 7.76 (d, J = 5.8 Hz, 1H), 7.63 - 7.59 (m, 3H), 7.54 (s, 1H), 7.39 - 7.36 (m, 2H), 7.11 (t, J = 5.3 Hz, 1H), 6.96 (dd, J = 9.3, 1.4 Hz, 1H), 6.31 (dd, J = 5.7, 1.7 Hz, 1H), 4.33 (d, J = 5.5 Hz, 2H), 2.36 - 2.28 (m, 1H), 2.05 - 2.00 (m, 1H), 1.94 - 1.87 (m, 1H), 1.33 - 1.26 (m, 2H), 0.92 - 0.88 (m, 2H), 0.68 - 0.64 (m, 2H)..

### Example 288: (1S,2S)-2-(3-chlorophenyl)-N-(4-(((6-cyclopropyl-8-((2-hydroxyethoxy)methyl) imidazo[1,2-a]pyridin-2-yl)methyl)amino)pyridin-2-yl)cyclopropane-1-carboxamide (I-288)

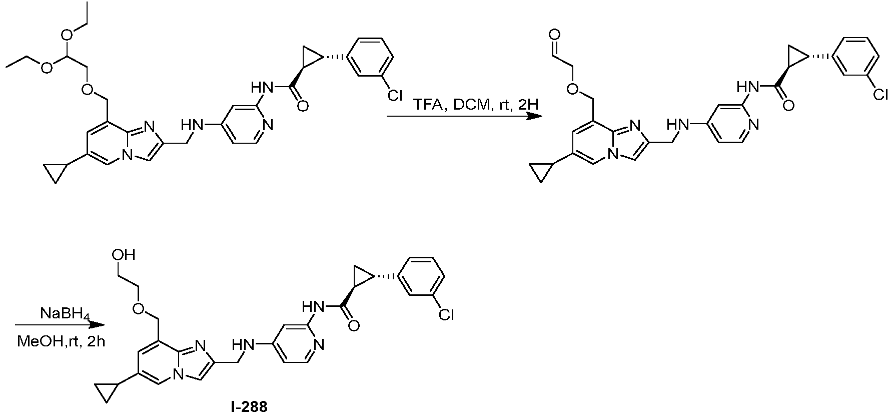

A mixture of (1S,2S)-2-(3-chlorophenyl)-N-(4-(((6-cyclopropyl-8-((2,2-diethoxyethoxy)methyl)-imidazo[1,2-a]pyridin-2-yl)methyl)amino)pyridin-2-yl)cyclopropane-1-carboxamide (100 mg, 0.16 mmol),TFA(0.4 ml), and DCM (4 mL) was stirred at room temperature for 2 h. Water (20 mL) was added to the mixture, which was then extracted with DCM (3 x 20 mL). The combined organic layers were washed with brine, dried over Na₂SO₄, filtered, and concentrated in vacuo to give **(1S,2S)-2-(3-chlorophenyl)-N-(4-(((6-cyclopropyl-8-((2-oxoethoxy)methyl) imidazo[1,2-a]pyridin-2-yl)methyl)amino)pyridin-2-yl)cyclopropane-1-carboxamide intermediate 803** (100 mg, crude) as a yellow oil. ESI-MS [M +H]+: 530.2

A mixture of **intermediate 803** (100 mg, crude), NaBH₄ (21.3 mg, 0.57 mmol), and MeOH (5 mL) was stirred at room temperature for 2 h. Water (20 mL) was added to the mixture, which was then extracted with EtOAc (3 x 20 mL). The combined organic layers were washed with brine, dried over Na₂SO₄, filtered, and concentrated in vacuo to give the crude, which was purified by silica gel chromatography (DCM/MeOH = 20/1) to give **(I-288)** (45 mg, 35%) as a white solid. ESI-MS [M +H]+:532.2, δ 10.33 (s, 1H), 8.24 (s, 1H), 7.75 (d, J = 5.8 Hz, 1H), 7.64 (s, 1H), 7.43 (s, 1H), 7.35 - 7.29 (m, 1H), 7.27 -7.21(m, 2H), 7.14 (d, J = 7.6 Hz, 2H), 7.02 (s, 1H), 6.33-6.31 (m, 1H), 4.91 (s, 1H), 4.78 (s, 2H), 4.36 (d, J = 5.6 Hz, 2H), 3.59 (s, 4H), 2.50-2.33(m, 2H), 1.96-1.88 (m, 1H), 1.48-1.41 (m, 1H), 1.38-1.31 (m, 1H), 0.95 - 0.86(m, 2H), 0.67 - 064 (m, 2H)..

### Example 289: rac-(1S*,2S*)-2-(5-chloro-2-cyanophenyl)-N-(4-(((6-cyclopropyl-8-((2-hydroxyethoxy)methyl)imidazo[1,2-a]pyridin-2-yl)methyl)amino)pyridin-2-yl)cyclopropane-1-carboxamide (I-289)

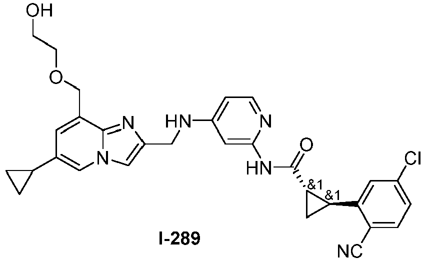

(I-289) was prepared in a similar manner to **(I-288).** ESI-MS [M +H] +: 557.1. δ 10.39 (s, 1H), 8.24 (s, 1H), 7.86 (d, J = 8.3 Hz, 1H), 7.77 (d, J = 5.8 Hz, 1H), 7.65 (s, 1H), 7.52 (dd, J = 8.3, 2.0 Hz, 1H), 7.46 (s, 1H), 7.39 (d, J = 1.8 Hz, 1H), 7.17 (t, J = 5.1 Hz, 1H), 7.01 (s, 1H), 6.31 (dd, J = 5.8, 1.9 Hz, 1H), 4.95 (s, 1H), 4.78 (s, 2H), 4.36 (d, J = 5.7 Hz, 2H), 3.59 (s, 4H), 2.60 - 2.53 (m, 2H), 1.95 - 1.88 (m, 1H), 1.56 - 1.45 (m, 2H), 0.93 - 0.83 (m, 2H), 0.77 - 0.64 (m, 2H).

### Example 290: 3-(2-(((2-((1S,2S)-2-(3-chlorophenyl)cyclopropane-1-carboxamido)pyridin-4-yl)amino)methyl)-6-cyclopropylimidazo[1,2-a]pyridin-8-yl)propanoic acid (I-290)

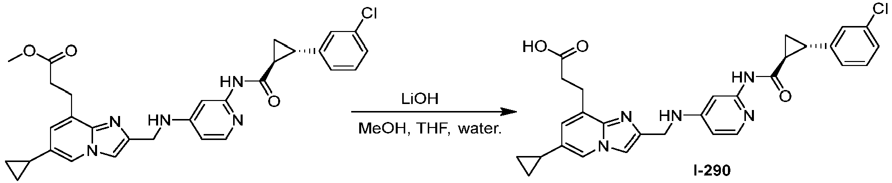

A mixture of methyl 3-(2-(((2-((1S,2S)-2-(3-chlorophenyl)cyclopropane-1-carboxamido)pyridin-4-yl)amino)methyl)-6-cyclopropylimidazo[1,2-a]pyridin-8-yl)propanoate (40 mg, 0.074 mmol) and LiOH.H₂O (15.5 mg, 0.37 mmol) in MeOH/THF/water (1.0 mL/1.0 mL/1.0 mL ) was stirred at room temperature for 1 h. The mixture was concentrated and purified by preparative HPLC (FA) to give (**I-290**)(21 mg, 54 %) as a white solid. ESI-MS [M +H]+: 530.2, δ 12.94 (s, 1H), 10.31 (s, 1H), 8.19 (s, 1H), 7.61 (s, 2H), 7.27 - 7.24 (m, 6H), 6.81 (s, 1H), 6.38 (s, 1H), 4.33 (s, 2H), 3.07 (s, 2H), 2.85 - 2.61 (m, 2H), 2.33 (s, 2H), 1.87 - 1.86 (m, 1H), 1.45 - 1.35 (m, 2H), 0.90 - 0.86 (m, 2H), 0.65 - 0.64 (m, 2H).

### Example 291: (1S,2S)-2-(3-chlorophenyl)-N-(4-(((6-cyclopropyl-8-(3-hydroxypropyl)imidazo[1,2-a]pyridin-2-yl)methyl)amino)pyridin-2-yl)cyclopropane-1-carhoxamide (I-291)

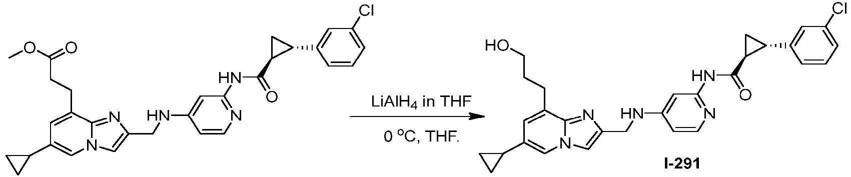

To a mixture of methyl 3-(2-(((2-((1S,2S)-2-(3-chlorophenyl)cyclopropane-1-carboxamido)pyridin-4-yl)amino)methyl)-6-cyclopropylimidazo[1,2-a]pyridin-8-yl)propanoate (70 mg, 0.129 mmol) in THF (5.0 mL) was added LiAlH₄ (0.19 ml, 1M, 0.19 mmol). After stirring at 0 °C under N₂ for 1 h, the mixture was quenched with water (1.0 mL), concentrated *in vacuo,* and purified by preparative HPLC (FA) to give the desired product (**I-291**) (26 mg, 39 %) as a white solid. ESI-MS [M +H]+: 516.2, δ 10.33 (s, 1H), 8.15 - 8.13 (m, 1H), 7.81 - 7.12 (m, 9H), 6.55 - 6.50 (m, 1H), 4.35 (s, 2H), 2.86 - 2.84 (m, 2H), 2.36 - 2.30 (m, 4H), 1.86 - 1.83 (m, 3H), 1.39 - 1.37 (m, 2H), 0.89 - 0.86 (m, 2H), 0.65 - 0.62 (m, 2H)..

### Example 292: (1S,2S)-2-(3-chlorophenyl)-N-(4-(((6-cyclopropyl-8-(2-hydroxyethyl)imidazo[1,2-a]pyridin-2-yl)methyl)amino)pyridin-2-yl)cyclopropane-1-carboxamide (I-292)

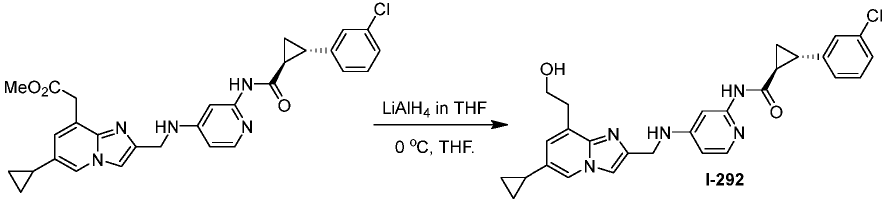

(**I-292**) was prepared in a similar manner to (**I-292**). ESI-MS [M +H]+: 502.1. ¹H NMR (400 MHz, CDCl₃) δ 7.75 - 7.74 (m, 2H), 7.58 (s, 1H), 7.49 (s, 1H), 7.21 - 7.19 (m, 2H), 7.09 (s, 1H), 7.01 (d, J = 6.7 Hz, 1H), 6.76 (s, 1H), 6.31 - 6.28 (m, 1H), 5.27 (s, 1H), 4.52 (d, J = 5.6 Hz, 2H), 3.97 - 3.93 (m, 2H), 3.16 - 3.14 (m, 2H), 2.57 (s, 1H), 1.88 - 1.84 (m, 2H), 1.72 - 1.70 (m, 1H), 1.37 - 1.31 (m, 2H), 0.95 (d, J = 8.2 Hz, 2H), 0.66 (d, J = 5.1 Hz, 2H).

### Example 293: (1S,2S)-N-(4-(((8-(aminomethyl)-6-cyclopropylimidazo[1,2-a]pyridin-2-yl)methyl)amino)pyridin-2-yl)-2-(3-chlorophenyl)cyclopropane-1-carboxamide (I-293)

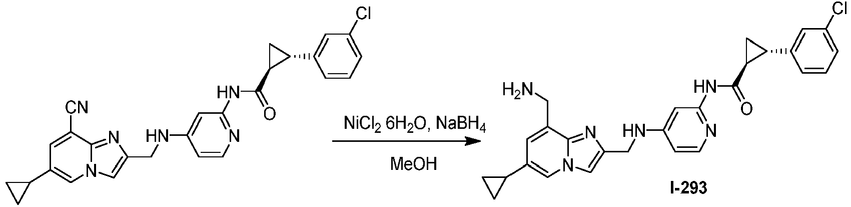

To a mixture of (1S,2S)-2-(3-chlorophenyl)-N-(4-(((8-cyano-6-cyclopropylimidazo[1,2-a]pyridin-2-yl)methyl)amino)pyridin-2-yl)cyclopropane-1-carboxamide (70 mg, 0.145 mmol) in MeOH (5.0 mL) was added NiCl₂.6H₂O (34.6 mg, 0.145 mmol) at 0 °C, and then NaBH₄ (15.7 mg, 0.435 mmol) was added. After stirring at 0 °C under N₂ for 1 h, the mixture was quenched with water (1.0 mL), concentrated *in vacuo,* and purified by preparative HPLC (FA) to give (**I-293**)(6.1 mg, 8.7 %) as a white solid. ESI-MS [M +H]+: 487.2. δ 10.33 (s, 1H), 8.65 - 8.05 (m, 4H), 7.82 - 7.59 (m, 2H), 7.45 (s, 1H), 7.37 - 7.20 (m, 3H), 7.16 - 7.14 (m, 2H), 7.03 (s, 1H), 6.32 (s, 1H), 4.38 (s, 2H), 4.14 (s, 2H), 2.39 - 2.36 (m, 2H), 1.97 - 1.76 (m, 1H), 1.50 - 1.40 (m, 1H), 1.38 - 1.30 (m, 1H), 0.93 - 0.92 (m, 2H), 0.69 - 0.67 (m, 2H)..

### Example 294: rac-(1S*,2S*)-2-(3-chlorophenyl)-N-(4-(((6-cyclopropyl-8-(3-fluorooxetan-3-yl)imidazo[1,2-a]pyridin-2-yl)methyl)amino)pyridin-2-yl)cyclopropane-1-carboxamide (I-294)

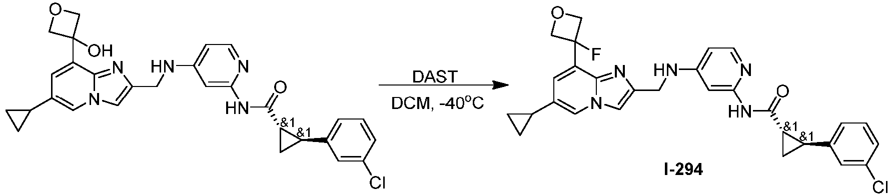

To a mixture of *rac*-(1S*,2S*)-2-(3-chlorophenyl)-N-(4-(((6-cyclopropyl-8-(3-hydroxyoxetan-3-yl)imidazo[1,2-a]pyridin-2-yl)methyl)amino)pyridin-2-yl)cyclopropane-1-carboxamide (20 mg, 0.038 mmol) in DCM ( 5ml ) was added DAST (30 mg, 0.19 mmol). The mixture was stirred at -40 °C for 3 h under N₂. Water (10 mL) was added to the reaction, which was then extracted with EtOAc (3 x 10 mL). The combined organic layers were washed with brine, dried over Na₂SO₄, and concentrated *in vacuo* to give the crude, which was purified with silica gel chromatography (DCM/MeOH = 10/1) to give (**I-294**)(7 mg, 35%) as a white solid. ESI-MS [M +H]+: 532.2, δ 10.31 (s, 1H), 8.40 (s, 1H), 7.76 (d, J = 5.7 Hz, 1H), 7.69 (s, 1H), 7.45 (s, 1H), 7.35 - 7.20 (m, 3H), 7.18-7.11 (m, 3H), 6.35-6.31 (m, 1H), 5.39 (d, J = 8.6 Hz, 1H), 5.32 (d, J = 8.6 Hz, 1H), 5.01 (d, J = 8.6 Hz, 1H), 4.95 (d, J = 8.6 Hz, 1H), 4.38 (d, J = 5.8 Hz, 2H), 2.38-2.32 (m, 2H), 2.00 - 1.89 (m, 1H), 1.48-1.42 (m, 1H), 1.36-1.32 (m, 1H), 0.97 - 0.86 (m, 2H), 0.77 - 0.63 (m, 2H).

### Example 295: (1S,2S)-2-(3-chlorophenyl)-N-(4-(((6-cyclopropylimidazo[1,2-a]pyridin-2-yl)methyl)amino)-6-hydroxy-1,3,5-triazin-2-yl)cyclopropane-1-carboxamide (I-295)

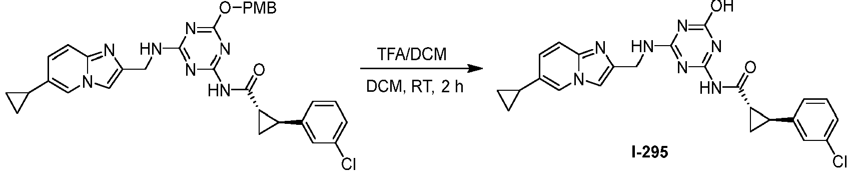

A mixture of (1S,2S)-2-(3-chlorophenyl)-N-(4-(((6-cyclopropylimidazo[1,2-a]pyridin-2-yl)methyl)amino)-6-((4-methoxybenzyl)oxy)-1,3,5-triazin-2-yl)cyclopropane-1-carboxamide (45 mg, 0.1 mmol) in TFA/DCM (1 mL/3 mL) was stirred at room temperature for 2 h. The reaction mixture was neutralized with saturated NaHCO₃ solution (20 mL) to pH~8 and extracted with DCM (5 x 30 mL). The combined organic layers were washed with brine (30 mL), dried over Na₂SO₄, and concentrated *in vacuo.* The residue was purified by preparative TLC (eluent: MeOH/DCM = 1/8) to afford (**I-295**) (10 mg, 26% yield) as a white solid. ESI-MS [M +H]+: 476.2. ¹H NMR (400 MHz, CDCl₃) δ 14.12 (s, 1H), 11.45 (s, 1H), 8.64 (s, 1H), 7.78 (s, 1H), 7.61 (s, 1H), 7.10 - 6.98 (m, 2H), 6.74 (d, J = 7.4 Hz, 1H), 6.67-6.60 (m, 2H), 6.36 (d, J = 8.9 Hz, 1H)., 5.15 (s, 1H), 4.37 (d, J = 13.9 Hz, 1H), 2.60 (s, 1H), 2.20 (s, 1H), 2.02 (s, 1H), 1.81 - 1.75 (m, 2H), 0.98-0.90 (m, 2H), 0.62 - 0.55 (m, 2H)..

### Example 296: (1S,2S)-2-(3-chlorophenyl)-N-(6-(((6-cyclopropylimidazo[1,2-a]pyridin-2-yl)methyl)amino)-2-oxo-2,3-dihydropyrimidin-4-yl)cyclopropane-1-carboxamide (I-296)

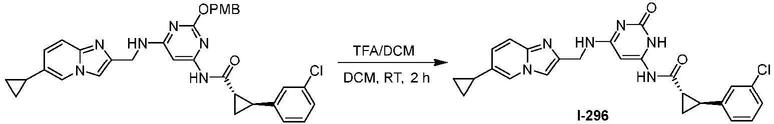

**(I-296)** was prepared in a similar manner to **(I-295).** ESI-MS [M+H]+: 475.1,. δ = 10.91 (s, 1H), 10.45 (s, 1H), 8.33 (s, 1H), 8.00 (s, 1H), 7.67 (s, 1H), 7.44 - 7.37 (m,1H), 7.35 - 7.22 (m, 3H), 7.20 - 7.10 (m,1H), 6.98 (d, J=9.2, 1H), 5.76 (s, 1H), 4.50 (s, 2H), 2.46 - 2.35 (m, 1H), 2.25-2.06 (m, 1H), 1.97-1.87 (m, 1H), 1.56-1.38 (m, 2H), 1.00 - 0.80 (m, 2H), 0.74 - 0.47 (m, 2H).

### Example 297: (1S,2S)-N-(6-((2R,4S)-4-amino-2-(6-cyclopropylimidazo[1,2-a]pyridin-2-yl)pyrrolidin-1-yl)pyrimidin-4-yl)-2-(3-chlorophenyl)cyclopropane-1-carboxamide (I-297)

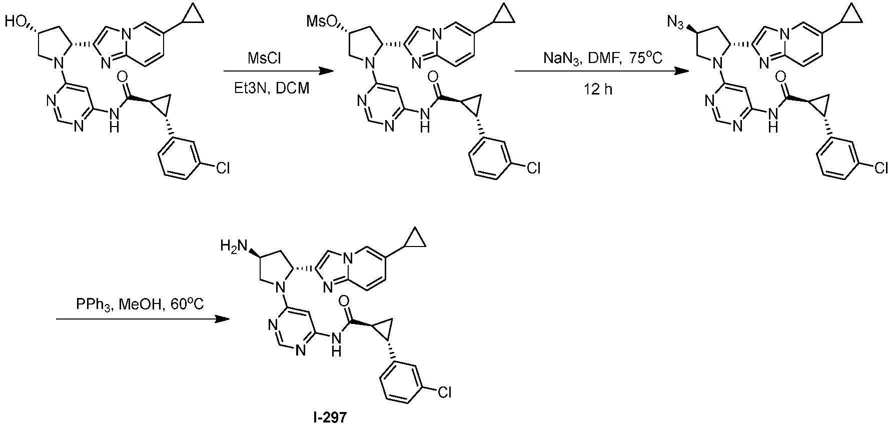

To a solution of (1S,2S)-2-(3-chlorophenyl)-N-(6-((2R,4R)-2-(6-cyclopropylimidazo[1,2-a]pyridin-2-yl)-4-hydroxypyrrolidin-1-yl)pyrimidin-4-yl)cyclopropane-1-carboxamide (60 mg, 0.12 mmol) in DCM ( 5 ml) was added Et₃N (36 mg, 0.36 mmol) and MsCl ( 41 mg, 0.36 mmol) at 0 °C. After stirring at room temperature for 3 h, the mixture was quenched with water (20 mL) and extracted with DCM (3 x 30 mL). The organic layer was concentrated to give **(3R,5R)-1-(6-((1S,2S)-2-(3-chlorophenyl)cyclopropane-1-carboxamido)pyrimidin-4-yl)-5-(6-cyclopropylimidazo[1,2-a]pyridin-2-yl)pyrrolidin-3-yl methanesulfonate intermediate 804** (60 mg, crude) as a yellow oil. ESI-MS [M + H]⁺: 593.2.

A solution of **intermediate 804**(60 mg, crude) and NaN₃ (10 mg, 0.15 mmol) in DMF ( 10 mL) was stirred at 75 °C for 12 h. After cooling to room temperature, water (30 mL) was added, and the mixture was extracted with EtOAc (3 x 30 mL). The combined organic layers were washed with brine, dried over Na₂SO₄, filtered. and concentrated in vacuo to give the crude, which was purified by preparative TLC to give **(1S,2S)-N-(6-((2R,4S)-4-azido-2-(6-cyclopropylimidazo[1,2-a]pyridin-2-yl)pyrrolidin-1-yl)pyrimidin-4-yl)-2-(3-chlorophenyl)cyclopropane-1-carboxamide intermediate 805** (40 mg, 74 %) as a yellow oil. ESI-MS [M +H]⁺: 540.2.

A solution of **intermediate 805** (40 mg, 0.07 mmol) and PPh₃ (55 mg, 0.21 mmol) in MeOH (5 mL) was stirred at 60 °C for 2 h under N₂. After cooling to room temperature, water (15 mL) was added, and the mixture was extracted with EtOAc (3 x 15 mL). The combined organic layers were washed with brine, dried over Na₂SO₄, filtered, and concentrated in vacuo to give the crude, which was purified by preparative TLC to give (**I-297**)(20 mg, 56 %) as a yellow solid. ESI-MS [M +H]+: 514.2. ¹H NMR (400 MHz, MeOD) δ 8.19 (s, 2H), 7.79 (s, 1H), 7.44 - 7.33 (m, 2H), 7.28 - 7.12 (m, 3H), 7.09 - 6.98 (m, 2H), 5.36-5.15 (m, 1H), 4.15 - 3.72 (m, 3H), 2.96 - 2.77 (m, 1H), 2.46-2.42 (m, 1H), 2.28-2.21 (m, 1H), 2.16-2.12 (m, 1H), 1.96-1.88 (m, 1H), 1.64-1.58 (m, 1H), 1.43 - 1.34 (m, 1H), 0.98-0.91 (m, 2H), 0.73-0.68 (m, 2H).

### Example 298: 2-(((6-((1S,2S)-2-(3-chlorophenyl)cyclopropane-1-carboxamido)pyrimidin-4-yl)amino)methyl)-6-cyclopropylimidazo[1,2-a]pyridine-8-carboxylic acid (I-298)

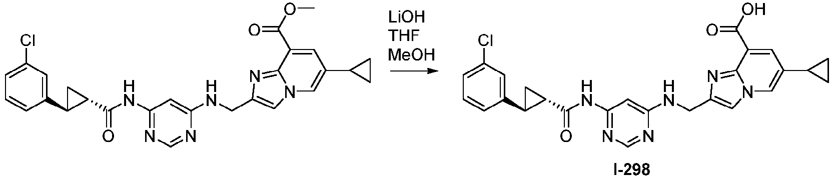

**(I-146)** (160 mg, 0.31 mmol) was dissolved in THF (10 mL) and MeOH (0.5 mL), and lithium hydroxide was added (8.2 mg, 0.34 mmol). The resulting reaction mixture was stirred at room temperature for 16 h. The solvent was removed under reduced pressure to give the title compound as a lithium salt (160 mg). 140 mg was used without further purification, however 20 mg was purified by preparative HPLC to give **(I-298)** (6 mg). ESI-MS (M+H)+:503.3, δ 10.64 (s, 1H), 8.58 (s, 1H), 8.23 (s, 1H), 8.02 (br s, 1H), 7.80 (s, 1H), 7.69 (s, 1H), 7.36 - 7.26 (m, 4H), 7.17 (ddd, J=1.4, 1.4, 7.7 Hz, 1H), 4.67 (br s, 2H), 2.41 - 2.40 (m, 2H), 2.05 - 2.05 (m, 1H), 1.49 - 1.48 (m, 1H), 1.43 - 1.42 (m, 1H), 0.98 - 0.98 (m, 2H), 0.74 - 0.74 (m, 2H).

### Example 299: 2-(((6-((1S,2S)-2-(3-chlorophenyl)cyclopropane-1-carboxamido)pyrimidin-4-yl)amino)methyl)-6-cyclopropyl-N,N-dimethylimidazo[1,2-a]pyridine-8-carboxamide (I-299)

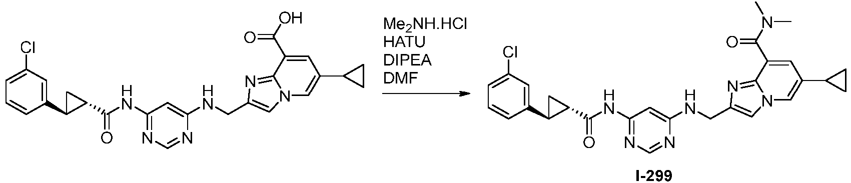

**(I-298)** (70 mg, 0.14 mmol) was dissolved in DMF (1 mL), and HATU (58 mg, 1.10 mmol) and DIPEA (0.048 mL, 0.28 mmol) were added. The resulting mixture was stirred at room temperature, and then dimethylamine hydrochloride (11 mg, 0.139 mmol) was added. The reaction mixture was stirred at room temperature for 16 h. The reaction mixture was diluted with water (20 mL) and brine solution (20 mL) and extracted with EtOAc (2 × 20 mL). The combined organics were washed with brine (20 mL) and passed through a hydrophobic frit. The solvent was removed *in vacuo* and the residue was purified by silica gel chromatography (eluting with a gradient of 0-20 % (7N NH₃ in MeOH) in DCM to give **(I-299)** (33 mg, 45 %) as a formic acid salt. ESI-MS (M+H)+: 530.4,: δ 10.61 (s, 1H), 8.37 (d, J=1.6 Hz, 1H), 8.21 (d, J=1.0 Hz, 1H), 7.92 (br s, 1H), 7.68 (s, 1H), 7.36 - 7.26 (m, 4H), 7.16 (ddd, J=1.3, 1.3, 7.6 Hz, 1H), 6.95 (d, J=1.7 Hz, 1H), 4.58 (s, 2H), 3.05 (s, 3H), 2.81 (s, 3H), 2.44 - 2.38 (m, 2H), 1.98 - 1.91 (m, 1H), 1.51 - 1.46 (m, 1H), 1.44 - 1.39 (m, 1H), 0.96 - 0.90 (m, 2H), 0.72 - 0.67 (m, 2H).

Using a procedure similar to that for **(I-299),** the compounds in Table **27** were synthesized from 2-(((6-((1S,2S)-2-(3-chlorophenyl) cyclopropane-1-carboxamido)pyrimidin-4-yl)amino)methyl)-6-cyclopropylimidazo[1,2-*a*]pyridine-8-carboxylic acid and an appropriate coupling partner. **Table 27**

| **EG** | **Compound** | **Coupling Partner /Analvtical Data** |
|---|---|---|
| **300** | 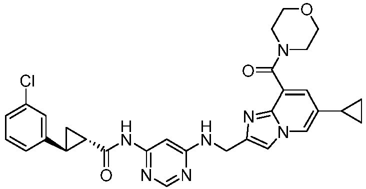 (1*S*,2*S*)-2-(3-chlorophenyl)-*N*-(6-(((6-cyclo propyl-8-(morpholine-4-carbonyl) imidazo [1,2-a]pyridin-2-yl)methyl)amino) pyrimidin-4-yl)cyclopropane-1-carboxamide **(I-300)** | Morpholine |
| | | ESI-MS (M+H)+: 572.3, δ 10.59 (s, 1H), 8.37 - 8.35 (m, 1H), 8.19 (s, 1H), 7.87 (br s, 1H), 7.67 (s, 1H), 7.34 - 7.24 (m, 4H), 7.16 - 7.13 (m, 1H), 7.00 (d, J=1.5 Hz, 1H), 4.56 (br s, 2H), 3.67 (s, 4H), 3.55 - 3.50 (m, 2H), 3.16 - 3.12 (m, 2H), 2.42 - 2.34 (m, 2H), 1.97 - 1.90 (m, 1H), 1.49 - 1.36 (m, 2H), 0.95 - 0.89 (m, 2H), 0.71 - 0.66 (m, 2H). |
| **301** | 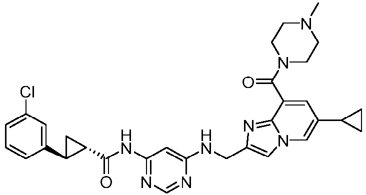 (1*S*,2*S*)-2-(3-chlorophenyl)-*N*-(6-(((6-cyclopropyl-8-(4-methylpiperazine-1-carbonyl)imidazo[1,2-*a*]pyridin-2-yl) methyl)amino)pyrimidin-4-yl)cyclo propane-1-carboxamide **(I-301)** | 1-methylpiperazine |
| | | ESI-MS (M+H)+: 585.3, δ 10.61 (s, 1H), 8.37 (d, J=1.4 Hz, 1H), 8.20 (d, J=0.8 Hz, 1H), 7.90 (br s, 1H), 7.68 (s, 1H), 7.36 - 7.25 (m, 4H), 7.16 (ddd, J=1.3, 1.3, 7.6 Hz, 1H), 6.97 (d, J=1.6 Hz, 1H), 4.58 (br s, 2H), 3.67 (t, J=4.5 Hz, 2H), 3.13 (t, J=4.7 Hz, 2H), 2.41 - 2.36 (m, 4H), 2.25 (br s, 2H), 1.98 - 1.91 (m, 1H), 1.50 - 1.46 (m, 2H), 1.44 - 1.39 (m, 1H), 0.97 - 0.91 (m, 2H), 0.72 - 0.67 (m, 2H). |
| **302** | 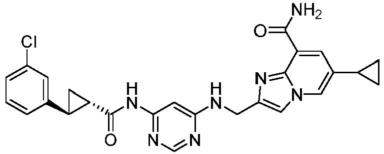 2-(((6-((1*S*,2*S*)-2-(3-chlorophenyl) cyclo propane-1-carboxamido)pyrimidin-4-yl) amino)methyl)-6-cyclopropyl imidazo[1,2-*a*]pyridine-8-carboxamide **(I-302)** | Ammonium carbonate |
| | | ESI-MS (M+H)+: 502.4, δ 10.62 (s, 1H), 9.49 (s, 1H), 8.55 (d, J=1.4 Hz, 1H), 8.22 (d, J=0.7 Hz, 1H), 8.00 - 7.96 (m, 2H), 7.78 (s, 1H), 7.73 (d, J=1.8 Hz, 1H), 7.34 - 7.26 (m, 4H), 7.17 (ddd, J=1.3, 1.3, 7.6 Hz, 1H), 4.65 (br s, 2H), 2.45 - 2.38 (m, 2H), 2.06 - 1.99 (m, 1H), 1.51 - 1.40 (m, 2H), 1.00 - 0.95 (m, 2H), 0.73 - 0.69 (m, 2H). |
| **303** | 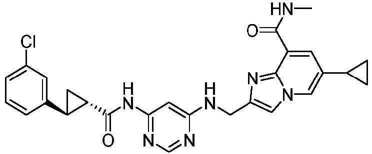 2-(((6-((1*S*,2*S*)-2-(3-chlorophenyl)cyclo propane-1-carboxamido)pyrimidin-4-yl) amino)methyl)-6-cyclopropyl-N-methyl imidazo[1,2-*a*]pyridine-8-carboxamide **(I-303)** | Methylamine hydrochloride |
| | | ESI-MS (M+H)+: 516.3, δ 10.64 (s, 1H), 9.94 (d, J=4.5 Hz, 1H), 8.53 (d, J=1.5 Hz, 1H), 8.22 (s, 1H), 7.96 (br s, 1H), 7.78 (s, 1H), 7.75 (d, J=1.7 Hz, 1H), 7.36 - 7.26 (m, 4H), 7.17 (ddd, J=1.4, 1.4, 7.5 Hz, 1H), 4.65 (br s, 2H), 2.97 (d, J=4.8 Hz, 3H), 2.45 - 2.37 (m, 2H), 2.07 - 2.00 (m, 1H), 1.51 - 1.40 (m, 2H), 1.01 - 0.95 (m, 2H), 0.73 - 0.68 (m, 2H). |

### Example 304: (1S,2S)-2-(3-chlorophenyl)-N-(6-(((6-cyclopropyl-8-(piperazine-1-carbonyl) imidazo[1,2-a]pyridin-2-yl)methyl)amino)pyrimidin-4-yl)cyclopropane-1-carboxamide (I-304)

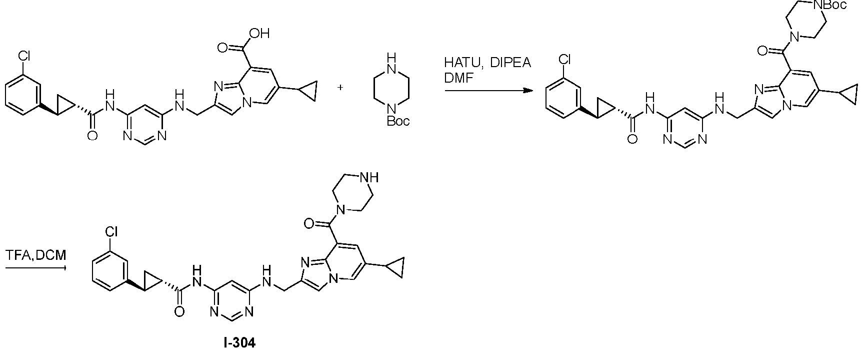

Using a procedure similar to that for **(I-299), tert-butyl 4-(2-(((6-((1S,2S)-2-(3-chloro phenyl)cyclopropane-1-carboxamido)pyrimidin-4-yl)amino)methyl)-6-cyclopropylimidazo[1,2-a]pyridine-8-carbonyl)piperazine-1-carboxylate intermediate 806 was synthesized from (I-298)** (0.23 mmol, 116 mg) and tert-butyl piperazine-1-carboxylate (0.23 mmol, 43 mg) and purified by silica gel column chromatography, eluting with a gradient of 0-20 % MeOH in DCM (45 mg, 29 %). ESI-MS (M+H)+: 671, ¹H NMR (400 MHz, CDCl₃) δ 8.28 (s, 1H), 8.13 - 8.09 (m, 1H), 7.90 - 7.89 (m, 1H), 7.49 (s, 1H), 7.29 - 7.27 (m, 1H), 7.23 - 7.19 (m, 2H), 7.10 - 7.07 (m, 1H), 7.03 (d, J=1.5 Hz, 1H), 7.02 - 6.98 (m, 1H), 5.73 - 5.72 (m, 1H), 4.68 - 4.65 (m, 2H), 3.85 - 3.82 (m, 2H), 3.60 - 3.60 (m, 2H), 3.44 - 3.42 (m, 2H), 3.30 - 3.29 (m, 2H), 2.62 - 2.54 (m, 1H), 1.93 - 1.85 (m, 1H), 1.80 - 1.69 (m, 2H), 1.47 (s, 9H), 1.41 - 1.35 (m, 1H), 1.01 - 0.95 (m, 2H), 0.71 - 0.66 (m, 2H).

A solution of **intermediate 806** (0.067 mmol, 45 mg) and trifluoroacetic acid (0.18 mL) in DCM (3.4 mL) was stirred at room temperature for 6 h. The mixture was concentrated in vacuo, then applied to an SCX column (eluting with MeOH then NH₃ (2M) in DCM/MeOH. The crude product was then purified by preparative HPLC to give **(I-304)** (8.7 mg, 23 %). ESI-MS (M+H)+: 571.3, δ 10.59 (s, 1H), 8.35 - 8.34 (m, 1H), 8.20 - 8.17 (m, 1H), 7.88 (br s, 1H), 7.66 (s, 1H), 7.34 - 7.24 (m, 4H), 7.17 - 7.13 (m, 1H), 6.95 (d, J=1.5 Hz, 1H), 4.56 (br s, 2H), 3.63 - 3.58 (m, 2H), 3.10 - 3.07 (m, 2H), 2.81 - 2.77 (m, 2H), 2.68 - 2.65 (m, 2H), 2.43 - 2.34 (m, 2H), 1.97 - 1.89 (m, 1H), 1.50 - 1.37 (m, 2H), 0.95 - 0.89 (m, 2H), 0.71 - 0.65 (m, 2H). Exchangeable NH signal not visible.

### Example 305: (1S,2S)-2-(3-chlorophenyl)-N-(6-(((6-cyclopropyl-8-(2-oxo-1,2-dihydropyridin-3-yl)imidazo[1,2-a]pyridin-2-yl)methyl)amino)pyrimidin-4-yl)cyclopropane-1-carboxamide (I-305)

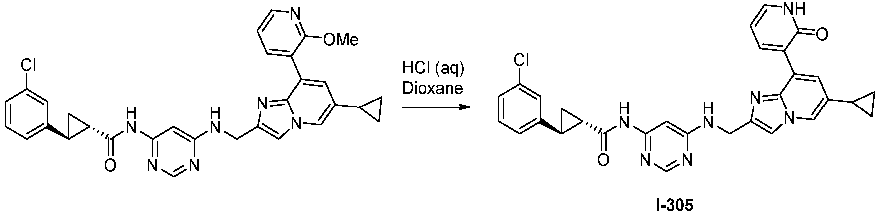

A mixture of ((1*S*,2*S*)-2-(3-chlorophenyl)-*N*-(6-(((6-cyclopropyl-8-(2-methoxypyridin-3-yl)imidazo[1,2-*a*]pyridin-2-yl)methyl)amino)pyrimidin-4-yl)cyclopropane-1-carboxamide) (0.008 mmol, 0.065 g), aqueous HCl (2M, 2.30 mmol, 1.1 mL), and 1,4-dioxane (3 mL) were heated at 80 °C for 2 h. The mixture was cooled to room temperature and concentrated *in vacuo.* The residue was applied to a SCX column (eluting with 25 % MeOH in DCM then 25 % (7N NH3 in MeOH) in DCM). The product-containing fractions were concentrated and the residue purified by preparative HPLC to give **(I-305)** (4.7 mg, 7 %). ESI-MS (M+H)+: 522.3, δ 11.86 - 11.85 (m, 1H), 10.61 (s, 1H), 8.69 (s, 1H), 8.27 (d, J=1.5 Hz, 1H), 8.21 (s, 1H), 7.90 - 7.87 (m, 2H), 7.66 (s, 1H), 7.45 (d, J=5.8 Hz, 1H), 7.36 - 7.26 (m, 4H), 7.16 (d, J=7.7 Hz, 1H), 6.39 (dd, J=6.7, 6.7 Hz, 1H), 5.77 (s, 1H), 2.42 - 2.34 (m, 2H), 1.96 - 1.90 (m, 1H), 1.49 - 1.39 (m, 2H), 0.97 - 0.91 (m, 2H), 0.70 - 0.65 (m, 2H).

### Example 306: (1S,2S)-N-(6-(((8-(azetidin-3-yloxy)-6-cyclopropylimidazo[1,2-a]pyridin-2-yl)methyl)amino)pyrimidin-4-yl)-2-(3-chlorophenyl)cyclopropane-1-carboxamide (I-306)

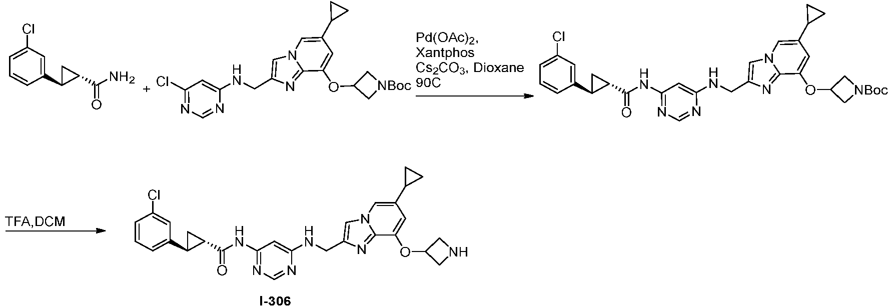

Using a similar procedure to that used for **(I-144), tert-butyl 3-((2-(((6-((1S,2S)-2-(3-chlorophenyl)cyclopropane-1-carboxamido)pyrimidin-4-yl)amino)methyl)-6-cyclopropylimidazo[1,2-a]pyridin-8-yl)oxy)azetidine-1-carboxylate intermediate 807** was prepared using intermediate 119 (0.23 mmol, 0.046 g) and tert-butyl 3-((2-(((6-chloropyrimidin-4-yl)amino)methyl)-6-cyclopropylimidazo[1,2-a]pyridin-8-yl)methyl)azetidine-1-carboxylate (0.21 mmol; 0.1 g) then purified by silica gel chromatography, eluting with a gradient of 1-20 % (7N NH₃ in MeOH) in DCM (0.012g, 9 %).

. A solution of (0.019 mmol, 0.012 g) and trifluoroacetic acid (0.5 mL) in DCM (1 mL) was stirred for 30 min, then concentrated in vacuo. The residue was purified by preparative HPLC to give **(I-306)** (4.2 mg, 42 %). ESI-MS (M+H)+: 530.3, ¹ δ 10.59 (s, 1H), 8.36 (s, 1H), 8.19 (s, 1H), 7.93 - 7.90 (m, 2H), 7.59 (s, 1H), 7.34 - 7.25 (m, 4H), 7.17 - 7.12 (m, 1H), 6.08 (s, 1H), 5.22 - 5.16 (m, 1H), 4.54 - 4.54 (m, 2H), 3.90 - 3.83 (m, 2H), 3.62 (dd, J=5.8, 8.5 Hz, 2H), 2.43 - 2.32 (m, 2H), 1.90 - 1.82 (m, 1H), 1.50 - 1.37 (m, 2H), 0.91 - 0.85 (m, 2H), 0.67 - 0.62 (m, 2H).

### Example 307: (1S,2S)-2-(3-chlorophenyl)-N-(6-(((6-cyclopropyl-8-(piperazin-1-yl)imidazo[1,2-a]pyridin-2-yl)methyl)amino)pyrimidin-4-yl)cyclopropane-1-carboxamide (I-307)

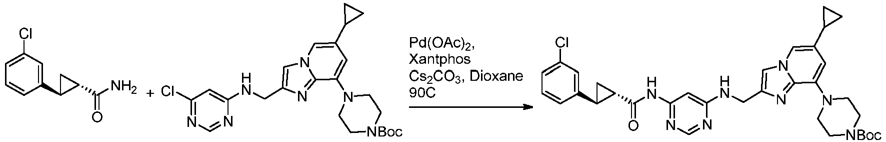

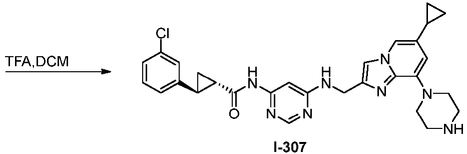

Using a procedure similar to that used for **(I-306), (I-307)was** synthesized from **intermediate 119** and *tert*-butyl 4-(2-(aminomethyl)-6-cyclopropylimidazo[1,2-a]pyridin-8-yl)piperazine-1-carboxylate, then purified by preparative HPLC (22 mg, 37%) (formic acid salt). ESI-MS (M+H)+: 543.3, δ 10.59 (s, 1H), 8.27 (0.s, 5H), 8.20 - 8.18 (m, 1H), 7.87 - 7.83 (m, 2H), 7.51 (s, 1H), 7.34 - 7.25 (m, 4H), 7.16 - 7.13 (m, 1H), 6.14 (d, J=1.1 Hz, 1H), 4.53 (br s, 2H), 3.46 - 3.41 (m, 4H), 2.96 - 2.90 (m, 4H), 2.43 - 2.32 (m, 2H), 1.91 - 1.81 (m, 1H), 1.50 - 1.37 (m, 2H), 0.89 - 0.83 (m, 2H), 0.67 - 0.62 (m, 2H). Piperazine NH exchangeable not observed.

### Example 308: (1S,2S)-N-(6-(((8-amino-6-cyclopropylimidazo[1,2-a]pyridin-2-yl)methyl)amino)pyrimidin-4-yl)-2-(3-chlorophenyl)cyclopropane-1-carboxamide (I-308)

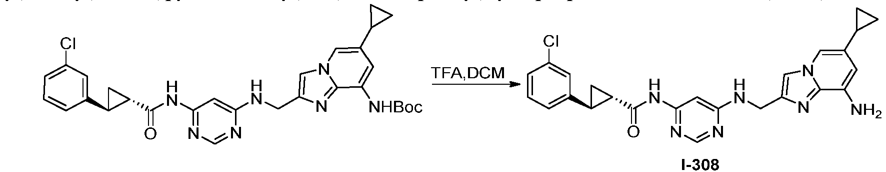

Using a procedure similar to that for **(I-306), (I-308)** was synthesized from tert-butyl(2-(((6-((1*S*,2*S*)-2-(3-chlorophenyl)cyclopropane-1-carboxamido)pyrimidin-4-yl)amino)methyl)-6-cyclopropylimidazo[1,2-*a*]pyridin-8-yl)carbamate using trifluoroacetic acid (12 mg, 60%). ESI-MS (M+H)+: 474.3, δ 10.61 (s, 1H), 8.22 (s, 1H), 7.82 - 7.81 (m, 1H), 7.60 (d, J=1.0 Hz, 1H), 7.50 (s, 1H), 7.37 - 7.26 (m, 4H), 7.17 (d, J=7.6 Hz, 1H), 5.98 (d, J=1.3 Hz, 1H), 5.50 - 5.45 (m, 2H), 4.56 - 4.56 (m, 2H), 2.47 - 2.36 (m, 2H), 1.84 - 1.76 (m, 1H), 1.53 - 1.39 (m, 2H), 0.89 - 0.83 (m, 2H), 0.61 - 0.55 (m, 2H).

### Example 309: (1S,2S)-2-(3-chlorophenyl)-N-(6-(((6-cyclopropyl-8-(2-oxoimidazolidin-1-yl)midazo[1,2-a]pyridin-2-yl)methyl)amino)pyrimidin-4-yl)cyclopropane-1-carboxamide (I-309)

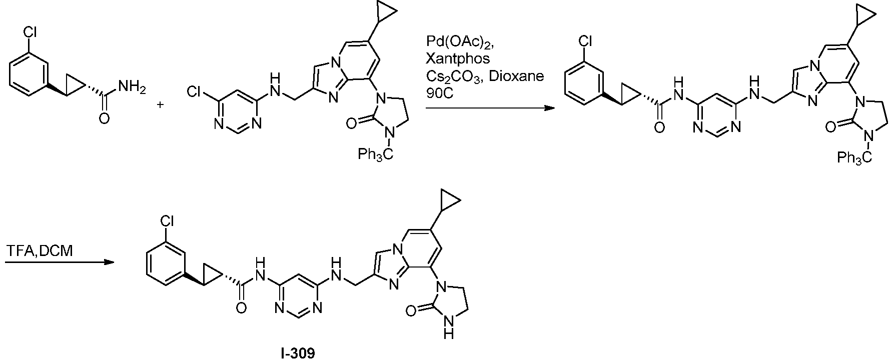

Employing a similar procedure to that used for **(I-144),** using **intermediate 119** (0.19 mmol, 0.04 g) and **intermediate 787** table 25 (0.17 mmol; 0.11 g) then purified by silica gel chromatography (eluting with a gradient of 1-10 % (7N NH₃ in MeOH) in DCM) (0.117 g, 85 % **(1S,2S)-2-(3-chlorophenyl)-N-(6-(((6-cyclopropyl-8-(2-oxo-3-trityl imidazolidin-1-yl)imidazo[1,2-a]pyridin-2-yl)methyl)amino) pyrimidin-4-yl)cyclopropane-1-carboxamide** was prepared.

A solution of the same (0.15 mmol; 0.12 g) and trifluoroacetic acid (1 mL) in DCM (5 mL) was stirred for 1 h the concentrated in vacuo. The residue was passed through an SCX column (eluting with 1:1 MeOH:DCM then 7N NH₃ in MeOH) then purified by silica gel chromatography, eluting with a gradient of 0-100 % (7N NH₃ in MeOH) in DCM (0.026g, 32 %) to give **(I-309).** ESI-MS (M+H)+: 543.3, δ 10.59 (s, 1H), 8.19 (s, 1H), 8.11 - 8.09 (m, 1H), 7.85 (br s, 1H), 7.61 (s, 1H), 7.34 - 7.23 (m, 5H), 7.17 - 7.13 (m, 1H), 6.98 - 6.95 (m, 1H), 4.55 (br s, 2H), 4.38 - 4.32 (m, 2H), 3.46 - 3.40 (m, 2H), 2.44 - 2.32 (m, 2H), 1.92 - 1.84 (m, 1H), 1.50 - 1.43 (m, 1H), 1.43 - 1.36 (m, 1H), 0.93 - 0.87 (m, 2H), 0.64 - 0.59 (m, 2H).

Using a similar procedure to that used for **(I-144),** the compounds in Table **28** were prepared from **intermediate 742** and an appropriate coupling partner. **Table 28**

| **EG** | **Compound** | **Coupling Partner / Analvtical Data** |
|---|---|---|
| **310** | 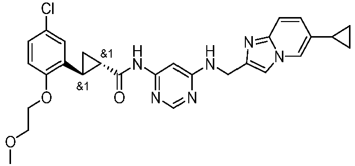 *rac*-(1*S**,2*S**)-2-(5-chloro-2-(2-methoxyethoxy)phenyl)-*N*-(6-(((6-cyclopropylimidazo[1,2-*a*]pyridin-2-yl)methyl)amino)pyrimidin-4-yl)cyclo propane-1-carboxamide, formic acid salt **(I-310)** | *rac*-(1*S**,2*S**)-2-(5-chloro-2-(2-methoxy ethoxy)phenyl)cyclopropane-1-carboxamide |
| | | ESI-MS (M+H)+: 533.3, δ, ppm 10.57 (1H, s), 8.31 (1H, t, J=0.8 Hz), 8.21 (1H, d, J=0.7 Hz), 8.15 (1H, s), 7.85 (1H, br s), 7.62 (1H, s), 7.38 (1H, d, J=9.4 Hz), 7.29 (1H, br s), 7.23 (1H, dd, J=2.6, 8.7 Hz), 7.03 (1H, d, J=2.6 Hz), 7.01 (1H, d, J=8.8 Hz), 6.97 (1H, dd, J=1.8, 9.3 Hz), 4.58 (2H, s), 4.11 (2H, t, J=4.4 Hz), 3.64 (2H, t, J=4.4 Hz), 3.23 (3H, s), 2.28 (1H, td, J=4.9, 8.0 Hz), 1.92 (1H, ddd, J=5.0, 8.4, 13.4 Hz), 1.40 (2H, ddd, J=4.1, 9.8, 13.8 Hz), 0.92 (2H, ddd, J=4.3, 6.3, 8.4 Hz), 0.67 (2H, ddd, J=3.2, 4.6, 6.7 Hz); CH₂ of cyclopropyl obscured by DMSO |
| **311** | 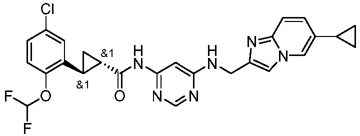 *rac*-(1*S**,2*S**)-2-(5-chloro-2-(difluoro methoxy)phenyl)-N-(6-(((6-cyclo propylimidazo[1,2-a]pyridin-2-yl)methyl)amino)pyrimidin-4-yl)cyclo propane-1-carboxamide **(I-311)** | rac-(1S*,2S*)-2-(5-chloro-2-(difluoro methoxy)phenyl)cyclopropane-1-carboxamide |
| | | ESI-MS (M+H)+: 525.4, ¹ δ 10.60 (s, 1H), 8.32 (s, 1H), 8.27 - 8.21 (m, 2H), 7.92 - 7.83 (m, 1H), 7.64 (s, 1H), 7.39 (d, J=9.3 Hz, 2H), 7.30 - 7.21 (m, 3H), 7.05 - 6.96 (m, 1H), 4.60 - 4.59 (m, 2H), 2.40 - 2.36 (m, 1H), 1.95 - 1.89 (m, 1H), 1.51 - 1.44 (m, 2H), 0.96 - 0.92 (m, 2H), 0.71 - 0.66 (m, 2H). 1H missing, presumed under DMSO peak. |
| **312** | 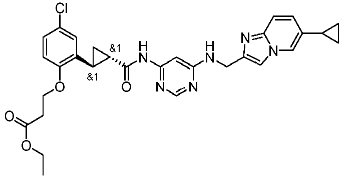 *rac*-ethyl 3-(4-chloro-2-((1*S**,2*S**)-2-((6-(((6-cyclopropylimidazo[1,2*-a*] pyridin-2-yl)methyl)amino)pyrimidin-4-yl)carbamoyl)cyclopropyl)phenoxy) propanoate **(I-312)** | rac-ethyl3-(2-((1S*,2S*))-2-carbamoyl cyclopropyl)-4-chlorophenoxy)propanoate |
| | | ESI-MS (M+H)+: 561.3, ¹H NMR (400 MHz, CDCl₃) δ 8.48 - 8.42 (m, 1H), 8.30 (s, 1H), 7.93 (s, 1H), 7.61 - 7.54 (m, 2H), 7.40 (s, 1H), 7.19 - 7.05 (m, 3H), 6.71 - 6.68 (m, 1H), 6.04 - 5.99 (m, 0H), 4.79 - 4.66 (m, 4H), 4.35 - 4.22 (m, 2H), 2.67 - 2.61 (m, 1H), 1.98 - 1.84 (m, 2H), 1.76 - 1.70 (m, 1H), 1.46 - 1.40 (m, 1H), 1.31 (t, J=7.1 Hz, 3H), 1.07 - 1.01 (m, 2H), 0.75 - 0.70 (m, 2H). |
| **313** | 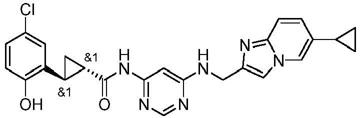 *rac-*(1*S**,2*S**)-2-(5-chloro-2-hydroxyphenyl)-N-(6-(((6-cyclopropyl imidazo[1,2-*a*]pyridin-2-yl)methyl) amino)pyrimidin-4-yl)cyclopropane-1-carboxamide **(I-313)** | rac-(1S*,2S*)-2-(5-chloro-2-hydroxyphenyl) cyclopropane-1-carboxamide |
| | | ESI-MS (M+H)+: 475.3, δ 10.59 (s, 1H), 9.91 (br s, 1H), 8.35 (s, 1H), 8.25 (s, 1H), 7.89 (br s, 1H), 7.66 (s, 1H), 7.42 (d, J=9.1 Hz, 1H), 7.33 (s, 1H), 7.09 (dd, J=1.9, 8.5 Hz, 1H), 7.01 (d, J=9.1 Hz, 1H), 6.95 (s, 1H), 6.84 (d, J=8.6 Hz, 1H), 4.62 (br s, 2H), 2.34 - 2.28 (m, 1H), 2.00 - 1.92 (m, 1H), 1.45 - 1.37 (m, 2H), 0.99 - 0.93 (m, 2H), 0.73 - 0.69 (m, 2H). |
| **314** | 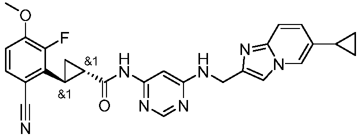 *rac-*(1*S**,2*S**)-2-(6-cyano-2-fluoro-3-methoxyphenyl)-N-(6-(((6-cyclo propylimidazo[1,2-*a*]pyridin-2-yl) methyl)amino)pyrimidin-4-yl)cyclo propane-1-carboxamide, formic acid salt **(I-314)** | *rac*-(1*S**,2*S**)-2-(6-cyano-2-fluoro-3-methoxyphenyl)cyclopropane-1-carboxamide |
| | | ESI-MS (M+H)+: 498.4, δ 10.78 (s, 1H), 8.50 (s, 1H), 8.35 (s, 1H), 8.26 (s, 1H), 7.91 - 7.91 (m, 1H), 7.75 - 7.65 (m, 2H), 7.45 - 7.27 (m, 3H), 7.04 - 7.00 (m, 1H), 4.63 - 4.58 (m, 2H), 3.97 (s, 3H), 1.99 - 1.92 (m, 1H), 1.60 - 1.53 (m, 2H), 0.99 - 0.93 (m, 2H), 0.74 - 0.68 (m, 2H). 2H missing, presumed under DMSO peak. |

### Example 315: rac-3-(4-chloro-2-((1S*,2S*)-2-((6-(((6-cyclopropylimidazo[1,2-a]pyridin-2-yl)methyl)amino)pyrimidin-4-yl)carbamoyl)cyclopropyl)phenoxy)acetic acid (I-315)

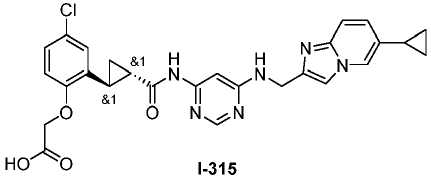

Using a similar procedure to that used for the synthesis of **(I-298), (I-315)** was prepared from rac-ethyl 3-(4-chloro-2-((1*S**,2*S**)-2-((6-(((6-cyclopropylimidazo[1,2-*a*]pyridin-2-yl)methyl)amino)pyrimidin-4-yl)carbamoyl)cyclopropyl)phenoxy)acetate (8.8 mg, 68%)(formic acid salt). ESI-MS (M+H)+: 533.3, ¹ δ 10.78 (s, 1H), 8.50 (s, 1H), 8.35 (s, 1H), 8.26 (s, 1H), 7.91 - 7.91 (m, 1H), 7.75 - 7.65 (m, 2H), 7.45 - 7.27 (m, 3H), 7.04 - 7.00 (m, 1H), 4.63 - 4.58 (m, 2H), 3.97 (s, 3H), 1.99 - 1.92 (m, 1H), 1.60 - 1.53 (m, 2H), 0.99 - 0.93 (m, 2H), 0.74 - 0.68 (m, 2H). 2H missing, presumed under DMSO peak.

### Example 316: rac-(1S*,2S*)-2-(5-chloro-2-(1H-tetrazol-1-yl)phenyl)-N-(6-(((6-cyclopropyl imidazo[1,2-a]pyridin-2-yl)methyl)amino)pyrimidin-4-yl)cyclopropane-1-carboxamide (I-316)

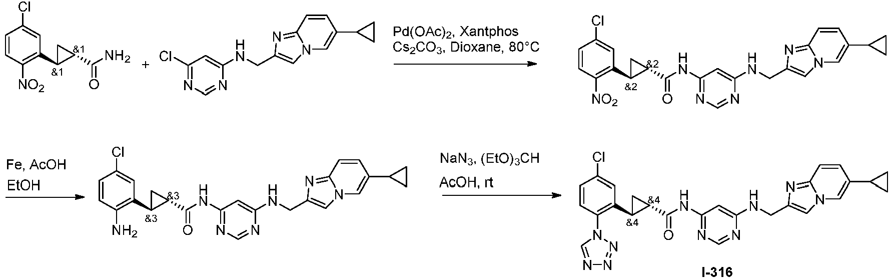

Using a similar procedure to that used for **(I-144), rac-(1S*,2S*)-2-(5-chloro-2-nitro phenyl)-N-(6-(((6-cyclopropylimidazo[1,2-a]pyridin-2-yl)methyl)amino)pyrimidin-4-yl)cyclopropane-1-carboxamide intermediate 802** was prepared from intermediate 742 and rac-(1S*,2S*)-2-(5-chloro-2-nitrophenyl)cyclopropane-1-carboxamide, then purified by column chromatography on silica using 0-10% (7N NH₃ in MeOH) in DCM (35 mg, ~50% purity). Used without further purification.

. A mixture of **intermediate 802** (~50% purity, 35 mg), iron (9.7 mg, 0.17 mmol), ethanol (2 mL), and acetic acid (0.5 mL) were stirred overnight at 70 °C. The mixture was cooled to room temperature, filtered through Celite^{®}, and concentrated in vacuo to give **(I-279)** (20 mg, (~50% purity) which was used without further purification.

**(I-279)** (~50% purity, 20 mg, 0.042 mmol) was dissolved in acetic acid (0.24 mL). Sodium azide (3.4 mg, 0.053 mmol) and triethyl orthoformate (0.022 mL) were added. The mixture was stirred at room temperature overnight. Water (1 mL) was added and product extracted into EtOAc (3 x 2 mL). The combined organics were passed through a hydrophobic frit and concentrated in vacuo. The residue was purified by preparative HPLC to give **(I-316)** (0.8 mg) as a formic acid salt. ESI-MS (M+H)+: 527.3, δ 8.50 (s, 2H), 8.32 (s, 1H), 8.23 (s, 1H), 7.69 - 7.57 (m, 4H), 7.41 - 7.37 (m, 1H), 7.21 (s, 1H), 7.00 - 6.96 (m, 1H), 4.59 - 4.59 (m, 2H), 2.13 - 2.07 (m, 1H), 1.97 - 1.89 (m, 1H), 1.40 - 1.35 (m, 1H), 1.30 - 1.23 (m, 1H), 0.96 - 0.90 (m, 2H), 0.71 - 0.66 (m, 2H). 2H missing, 1H assumed under DMSO and 1H exchangable.

### Examples 317-321

### rac-(1S*,2S*)-N-(6-(((6-cyclopropyl-8-(2-oxo-3-azabicyclo[3.1.0]hexan-3-yl)imidazo[1,2-a]pyridin-2-yl)methyl)amino)pyrimidin-4-yl)-2-(4-methylpyrimidin-2-yl)cyclopropane-1-carboxamide (I-317)

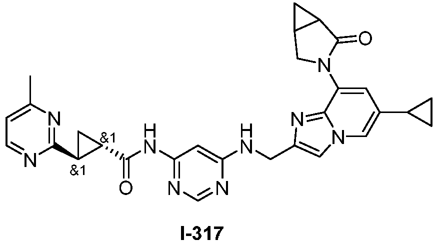

Using a similar procedure to that used for **(I-144), (I-317)** was prepared using 2-(4-methylpyrimidin-2-yl)cyclopropane-1-carboxamide (35 mg, 0.20 mmol) and **intermediate 809 table 25** (71 mg, 0.18 mmol), then purified by preparative LCMS to give (40 mg, 42 %) as a mixture of stereoisomers. ESI-MS (M+H)+: 536, δ 10.72 (s, 1H), 8.58 (d, J=5.1 Hz, 1H), 8.26 (d, J=1.3 Hz, 1H), 8.24 (s, 1H), 7.92 (s, 1H), 7.69 (s, 1H), 7.35 (s, 1H), 7.26 (d, J=5.3 Hz, 1H), 7.08 (d, J=1.3 Hz, 1H), 4.61 (s, 2H), 4.41 - 4.36 (m, 1H), 4.05 (d, J=10.4 Hz, 1H), 2.71 - 2.65 (m, 1H), 2.62 - 2.57 (m, 1H), 2.47 (s, 3H), 2.19 - 2.11 (m, 1H), 2.08 - 2.02 (m, 1H), 1.98 - 1.91 (m, 1H), 1.63 - 1.53 (m, 2H), 1.31 - 1.23 (m, 2H), 0.98 - 0.93 (m, 2H), 0.92 - 0.87 (m, 1H), 0.71 - 0.64 (m, 2H).

The mixture was resolved using SFC (YMC Cellulose-C 4.6x250 mm, 5 µm 40/60 IPA (0.1 % DEA) / CO₂, 5.0 mL/min, 120 bar, 40°C) to give four stereoisomers: (1S,2S)-N-(6-(((6-cyclo propyl-8-((1*R*,5*S*)-2-oxo-3-azabicyclo[3.1.0]hexan-3-yl)imidazo[1,2-*a*]pyridin-2-yl)methyl) amino)pyrimidin-4-yl)-2-(4-methylpyrimidin-2-yl)cyclopropane-1-carboxamide, (1*R*,2*R*)-*N*-(6-(((6-cyclopropyl-8-((1*R*,5*S*)-2-oxo-3-azabicyclo[3.1.0]hexan-3-yl)imidazo[1,2-*a*]pyridin-2-yl)methyl) amino)pyrimidin-4-yl)-2-(4-methylpyrimidin-2-yl)cyclopropane-1-carboxamide, (1*S*,2*S*)-*N*-(6-(((6-cyclopropyl-8-((1*S*,5*R*)-2-oxo-3-azabicyclo[3.1.0]hexan-3-yl)imidazo[1,2-*a*]pyridin-2-yl)methyl) amino)pyrimidin-4-yl)-2-(4-methylpyrimidin-2-yl)cyclopropane-1-carboxamide, and (1*R*,2*R)-N-*(6-(((6-cyclopropyl-8-((1*S*,5*R*)-2-oxo-3-azabicyclo[3.1.0]hexan-3-yl)imidazo[1,2-*a*] pyridin-2-yl)methyl) amino)pyrimidin-4-yl)-2-(4-methylpyrimidin-2-yl)cyclopropane-1-carboxamide.

First eluting isomer **(I-318),** 4.94 mg: ESI-MS (M+H)+: 536, δ 10.68 (s, 1H), 8.54 (d, J=5.1 Hz, 1H), 8.23 (d, J=1.1 Hz, 1H), 8.20 (s, 1H), 7.88 (s, 1H), 7.65 (s, 1H), 7.31 (s, 1H), 7.22 (d, J=5.4 Hz, 1H), 7.04 (d, J=1.3 Hz, 1H), 4.57 (s, 2H), 4.35 (dd, J=5.9, 10.4 Hz, 1H), 4.01 - 3.98 (m, 1H), 2.64 (ddd, J=3.7, 5.2, 8.7 Hz, 1H), 2.55 (q, J=2.8 Hz, 1H), 2.43 (s, 3H), 2.15 - 2.08 (m, 1H), 2.05 - 1.98 (m, 1H), 1.94 - 1.87 (m, 1H), 1.58 - 1.49 (m, 2H), 1.24 - 1.18 (m, 1H), 0.94 - 0.84 (m, 3H), 0.66 - 0.63 (m, 2H). RT = 5.99 min, 76.6 % e.e.

Second eluting isomer **(I-319,** 4.59 mg): ESI-MS (M+H)+: 536, ¹H NMR (400 MHz, DMSO) substantially similar to (I-318). RT = 8.32 min, 78.0 % e.e.

The third and fourth eluting isomers were not resolved under the above conditions. Further SFC resolution (LUX Cellulose-4 4.6x250mm, 5um 55/45 IPA (0.1 % DEA) / CO2, 5.0ml/min, 120bar, 40°C) gave:

Third eluting isomer **(I-320,** 2.97 mg): ESI-MS (M+H)+: 536, δ 10.69 (s, 1H), 8.54 (d, J=5.1 Hz, 1H), 8.23 (d, J=1.1 Hz, 1H), 8.20 (s, 1H), 7.89 (s, 1H), 7.65 (s, 1H), 7.32 (s, 1H), 7.22 (d, J=5.1 Hz, 1H), 7.04 (d, J=1.5 Hz, 1H), 4.57 (s, 2H), 4.35 (dd, J=5.8, 10.3 Hz, 1H), 4.00 (d, J=10.8 Hz, 1H), 2.64 (ddd, J=3.6, 5.2, 8.5 Hz, 1H), 2.58 - 2.54 (m, 1H), 2.43 (s, 3H), 2.15 - 2.08 (m, 1H), 2.04 - 1.99 (m, 1H), 1.94 - 1.87 (m, 1H), 1.58 - 1.49 (m, 2H), 1.26 - 1.18 (m, 2H), 0.95 - 0.84 (m, 3H), 0.66 - 0.63 (m, 2H). RT = 8.36 min, 99.9 % e.e.
Fourth eluting isomer **(I-321,** 5.99 mg): ESI-MS (M+H)+: 536, ¹H NMR (400 MHz, DMSO) substantially similar to (I-320). RT = 11.82 min, 94.4 % e.e.

### Examples 322-324

### rac-(1S*,2S*)-N-(6-(((6-cyclopropyl-8-(methylsulfonyl)imidazo[1,2-a]pyridin-2-yl)methyl)amino) pyrimidin-4-yl)-2-(4-methylpyrimidin-2-yl)cyclopropane-1-carboxamide (I-322)

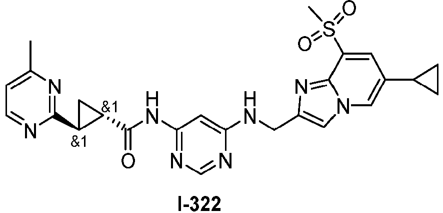

Using a similar procedure to that used for **(I-144), (I-322)** was prepared using 2-(4-methylpyrimidin-2-yl)cyclopropane-1-carboxamide (35 mg, 0.198) and **intermediate 741** (68 mg, 0.180 mmol) , then purified by preparative LCMS (26 mg, 28 %) as a mixture of enantiomers. ESI-MS (M+H)+: 519, δ 10.73 (s, 1H), 8.69 (d, J=1.3 Hz, 1H), 8.58 (d, J=5.1 Hz, 1H), 8.25 (s, 1H), 8.02 (s, 1H), 7.86 (s, 1H), 7.62 (d, J=1.8 Hz, 1H), 7.36 (s, 1H), 7.26 (d, J=5.1 Hz, 1H), 4.70 (s, 2H), 3.59 (s, 3H), 2.71 - 2.65 (m, 1H), 2.61 - 2.58 (m, 1H), 2.47 (s, 3H), 2.16 - 2.08 (m, 1H), 1.63 - 1.53 (m, 2H), 1.06 - 1.00 (m, 2H), 0.79 - 0.74 (m, 2H).

The mixture was resolved using SFC (YMC Cellulose-C 20x250 mm, 5 µm 35/65 MeOH (0.1 % DEA) / CO₂, 100 mL/min, 120 bar, 40°C) to give two enantiomers: (1S,2S)-N-(6-(((6-cyclopropyl-8-(methylsulfonyl)imidazo[1,2-*a*]pyridin-2-yl)methyl)amino)pyrimidin-4-yl)-2-(4-methylpyrimidin-2-yl)cyclopropane-1-carboxamide **(I-324)** and (1*R*,2*R*)-*N*-(6-(((6-cyclopropyl-8-(methylsulfonyl)imidazo[1,2-a]pyridin-2-yl)methyl)amino)pyrimidin-4-yl)-2-(4-methylpyrimidin-2-yl)cyclopropane-1-carboxamide **(I-323).**

First eluting isomer, **(I-323):** ESI-MS (M+H)+: 519, δ 10.71 (s, 1H), 8.65 (d, J=1.3 Hz, 1H), 8.54 (d, J=5.1 Hz, 1H), 8.21 (s, 1H), 8.00 (s, 1H), 7.82 (s, 1H), 7.58 (d, J=1.8 Hz, 1H), 7.33 (s, 1H), 7.22 (d, J=5.0 Hz, 1H), 4.66 (s, 2H), 3.55 (s, 3H), 2.67 - 2.61 (m, 1H), 2.58 - 2.53 (m, 1H), 2.43 (s, 3H), 2.12 - 2.04 (m, 1H), 1.58 - 1.49 (m, 2H), 1.01 - 0.96 (m, 2H), 0.75 - 0.70 (m, 2H). RT = 2.37 min, 98.2 % e.e.

Second eluting isomer, ( **(I-324):** ESI-MS (M+H)+: 519, ¹H NMR (400 MHz, DMSO) substantially similar to **(I-324).** RT = 3.45 min, 99.2 % e.e.

Using a similar procedure to that used for **(I-144),** the compounds in Table **29** were prepared using (1*S*,2*S*)-2-(4-chloropyridin-2-yl)cyclopropane-1-carboxamide and an appropriate coupling partner, then purified by preparative LCMS. **Table 29**

| **EG** | **Compound** | **Coupling Partner / Analvtical Data** |
|---|---|---|
| **325** | 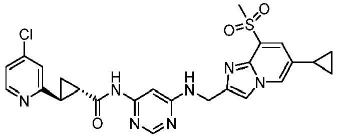 (1*S*,2*S*)-2-(4-chloropyridin-2-yl)-*N*-(6-(((6-cyclopropyl-8-(methylsulfonyl) imidazo[1,2-*a*]pyridin-2-yl)methyl) amino)pyrimidin-4-yl)cyclopropane-1-carboxamide **(I-325)** | **Intermediate 741** |
| | | ESI-MS (M+H)+: 538.4δ 10.68 (s, 1H), 8.64 (s, 1H), 8.44 (d, J=5.3 Hz, 1H), 8.21 (s, 1H), 8.00 (s, 1H), 7.82 (s, 1H), 7.66 - 7.65 (m, 1H), 7.57 (d, J=1.4 Hz, 1H), 7.36 (dd, J=2.0, 5.3 Hz, 1H), 7.32 (s, 1H), 4.70 - 4.63 (m, 2H), 3.56 (s, 3H), 2.64 - 2.57 (m, 1H), 2.12 - 2.06 (m, 1H), 1.57 - 1.47 (m, 3H), 1.02 - 0.96 (m, 2H), 0.75 - 0.70 (m, 2H). |
| **326** | 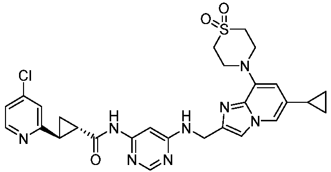 (1*S*,2S)-2-(4-chloropyridin-2-yl)-*N*-(6-(((6-cyclopropyl-8-(1,1-dioxidothio morpholino)imidazo[1,2-*a*]pyridin-2-yl)methyl)amino)pyrimidin-4-yl)cyclopropane-1-carboxamide **(I-326)** | 4-(2-(((6-chloropyrimidin-4-yl)amino)methyl)-6-cyclopropylimidazo[1,2-a]pyridin-8-yl)thio morpholine 1,1-dioxide ESI-MS (M+H)+: 593.4, δ 10.66 (s, 1H), 8.44 (d, J=5.3 Hz, 1H), 8.20 (s, 1H), 7.93 (s, 1H), 7.83 (s, 1H), 7.66 (d, J=1.9 Hz, 1H), 7.57 (s, 1H), 7.36 (dd, J=2.1, 5.3 Hz, 1H), 7.30 (s, 1H), 6.31 (s, 1H), 4.55 - 4.55 (m, 2H), 4.13 - 4.13 (m, 4H), 3.24 (dd, J=4.8, 4.8 Hz, 4H), 2.64-2.57 (m, 2H), 1.93- .84 (m, 1H), 1.57-1.46 (m, 2H), 0.91-0.86 (m, 2H), 0.73-0.68 (m, 2H). |
| **327** | 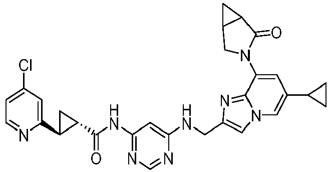 (1*S*,2*S*)-2-(4-chloropyridin-2-yl)-*N*-(6-(((6-cyclopropyl-8-(2-oxo-3-azabicyclo [3.1.0]hexan-3-yl)imidazo[1,2-*a*] pyridin-2-yl)methyl)amino)pyrimidin-4-yl)cyclopropane-1-carboxamide **(I-327)** | **Intermediate 809 table 25** |
| | | ESI-MS (M+H)+: 555.3, δ 10.67 (s, 1H), 8.23 (d, J=1.2 Hz, 1H), 8.20 (d, J=0.6 Hz, 1H), 7.89 (s, 1H), 7.66 (d, J=1.7 Hz, 1H), 7.64 (s, 1H), 7.36 (dd, J=2.0, 5.4 Hz, 1H), 7.31 (s, 1H), 7.04 (d, J=1.3 Hz, 1H), 4.35 (ddd, J=3.4, 5.9, 10.3 Hz, 1H), 4.00 (d, J=10.0 Hz, 1H), 2.65 - 2.57 (m, 2H), 2.15 - 2.07 (m, 1H), 2.04 - 1.98 (m, 1H), 1.91 (tdd, J=3.8, 9.2, 9.2 Hz, 1H), 1.57 - 1.47 (m, 2H), 1.21 (ddd, J=4.0, 4.0, 12.0 Hz, 1H), 0.95 - 0.90 (m, 2H), 0.86 (ddd, J=1.3, 4.2, 7.3 Hz, 1H), 0.65 (ddd, J=1.9, 4.3, 7.1 Hz, 2H). |

### Example 328: (1S,2S)-2-(4-chloropyridin-2-yl)-N-(6-(((6-cyclopropylimidazo[1,2-a]pyridin-2-yl)methyl)(2-hydroxyethyl)amino)pyrimidin-4-yl)cyclopropane-1-carboxamide (I-328)

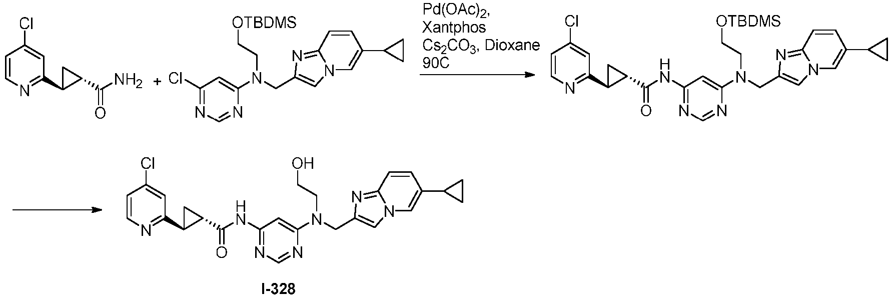

Using a similar procedure to that used for **(I-144), (1S,2S)-N-(6-((2-((tert-butyldimethyl silyl)oxy)ethyl)((6-cyclopropylimidazo[1,2-a]pyridin-2-yl)methyl)amino)pyrimidin-4-yl)-2-(4-chloropyridin-2-yl)cyclopropane-1-carboxamide intermediate 810** was prepared using (1S,2S)-2-(4-chloropyridin-2-yl)cyclopropane-1-carboxamide (0.14 mmol, 0.028 g) and **intermediate 811 table 25** (0.13 mmol; 0.060 g), then purified by silica gel chromatography (eluting with a gradient of 1-20 % (7N NH₃ in MeOH) in DCM) (0.030g, 37 %). ESI-MS (M+H)+: 618.4

Using a similar procedure to that for **(I-137), (I-328)** was synthesized from **intermediate 810** e (8 mg, 33 %). ESI-MS (M+H)+: 502.4, δ 10.75 - 10.72 (m, 1H), 8.43 (d, J=5.4 Hz, 1H), 8.33 - 8.30 (m, 1H), 8.27 - 8.25 (m, 1H), 7.66 - 7.63 (m, 2H), 7.45 - 7.34 (m, 3H), 7.02 - 6.99 (m, 1H), 5.21 - 5.20 (m, 1H), 4.95 - 4.69 (m, 2H), 3.80 - 3.55 (m, 4H), 2.62 - 2.55 (m, 2H), 1.97 - 1.89 (m, 1H), 1.56 - 1.48 (m, 2H), 0.95 - 0.90 (m, 2H), 0.71 - 0.65 (m, 2H).

### Examples 329-333

### rac-2-(3-chlorophenyl)-N-(6-(((6-cyclopropyl-8-(3-methyl-2,4-dioxoimidazolidin-1-yl)imidazo[1,2-a]pyridin-2-yl)methyl)amino)pyrimidin-4-yl)-2-fluorocyclopropane-1-carboxamide (I-329)

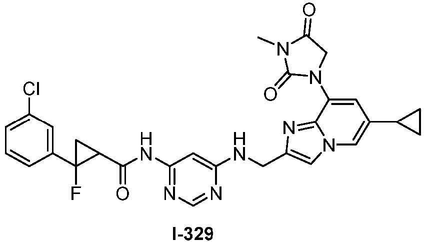

Using a similar procedure to that used for **(I-137), (I-329)** was prepared, as a mixture of stereoisomers, using 2-(3-chloro phenyl)-2-fluorocyclopropane-1-carboxamide (67 mg, 0.31 mmol) and 1-(2-(((6-chloropyrimidin-4-yl)amino) methyl)-6-cyclopropylimidazo[1,2-*a*]pyridin-8-yl)-3-methylimidazolidine-2,4-dione (118 mg, 0.29 mmol). The mixture was further purified by preparative LCMS to give two mixtures of enantiomers: (1*R*,2*R*)-2-(3-chlorophenyl)-*N*-(6-(((6-cyclopropyl-8-(3-methyl-2,4-dioxoimidazolidin-1-yl)imidazo[1,2-*a*]pyridin-2-yl)methyl)amino)pyrimidin-4-yl)-2-fluorocyclopropane-1-carboxamide, (1*S*,2*S*)-2-(3-chlorophenyl)-*N*-(6-(((6-cyclopropyl-8-(3-methyl-2,4-dioxoimidazolidin-1-yl)imidazo[1,2-*a*]pyridin-2-yl)methyl)amino)pyrimidin-4-yl)-2-fluorocyclopropane-1-carboxamide, (1*R*,2*S*)-2-(3-chlorophenyl)-*N*-(6-(((6-cyclopropyl-8-(3-methyl-2,4-dioxo imidazolidin-1-yl)imidazo[1,2-*a*]pyridin-2-yl)methyl)amino)pyrimidin-4-yl)-2-fluorocyclopropane-1-carboxamide, and (1*S*,2*R*)-2-(3-chlorophenyl)-*N*-(6-(((6-cyclopropyl-8-(3-methyl-2,4-dioxo imidazolidin-1-yl)imidazo[1,2-*a*]pyridin-2-yl)methyl)amino)pyrimidin-4-yl)-2-fluorocyclopropane-1-carboxamide. First eluting mixture of enantiomers, **(I-330):** ESI-MS (M+H)+: 589.4, δ 10.79 (s, 1H), 8.24 - 8.19 (m, 2H), 7.84 - 7.79 (m, 1H), 7.66 (s, 1H), 7.52 (s, 1H), 7.46 - 7.40 (m, 3H), 7.34 (s, 1H), 7.04 (s, 1H), 4.89 - 4.86 (m, 2H), 4.59 - 4.51 (m, 2H), 2.99 - 2.98 (m, 4H), 2.09 - 1.85 (m, 3H), 0.98 - 0.92 (m, 2H), 0.68 - 0.62 (m, 2H).
Second eluting mixture of enantiomers **(I-331):** ESI-MS (M+H)+: 589.3, ¹H NMR (400 MHz, DMSO) substantially similar to **(I-330).**
The second eluting mixture was then further separated by SFC (YMC Cellulose-C 4.6x250 mm, 5 µm 50/50 MeOH (0.1 % DEA) / CO₂, 5.0 mL/min, 120 bar, 40°C) to give two enantiomers:
First eluting enantiomer **(I-332):** ESI-MS (M+H)+: 589.3, δ 10.79 (s, 1H), 8.30 - 8.24 (m, 2H), 7.98 - 7.90 (m, 1H), 7.75 (s, 1H), 7.54 - 7.50 (m, 3H), 7.44 - 7.38 (m, 3H), 4.98 - 4.96 (m, 2H), 4.64 - 4.61 (m, 2H), 3.03 (s, 3H), 2.81 - 2.72 (m, 1H), 2.29 - 2.18 (m, 1H), 2.03 - 1.95 (m, 1H), 1.89 - 1.80 (m, 1H), 1.04 - 0.96 (m, 2H), 0.72 - 0.67 (m, 2H), RT =4.18 min, 99.9 %ee.
Second eluting enantiomer **(I-333):** ESI-MS (M+H)+: 589.3, ¹H NMR (400 MHz, DMSO) substantially similar to (I-332) RT = 5.57 min, 99.2 % e.e.

### Examples 334-336

### rac-(1S*,2S*))-1-(2-(((6-chloropyrimidin-4-yl)amino)methyl)-6-cyclopropylimidazo[1,2-a] pyridin-8-yl)pyrrolidin-2-one (I-334)

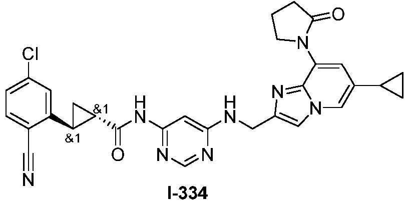

Using a similar procedure to that used for **(I-137),** the following compound was prepared using (*rac-*(1*S**,2*S**))-2-(5-chloro-2-cyanophenyl)cyclopropane-1-carboxamide (89 mg, 0.40 mmol) and **intermediate 739** (140 mg, 0.37 mmol), then purified by preparative LCMS to give **(I-334)** (12 mg, 6 %) as a mixture of enantiomers. ESI-MS (M+H)+: 567.2, δ 10.69 (s, 1H), 8.26 (s, 1H), 8.22 (s, 1H), 7.96 (s, 1H), 7.88 (d, J=8.3 Hz, 1H), 7.67 (s, 1H), 7.54 (dd, J=1.9, 8.3 Hz, 1H), 7.43 (s, 1H), 7.32 (s, 1H), 7.13 (s, 1H), 4.59 - 4.59 (m, 2H), 4.16 (dd, J=7.0, 7.0 Hz, 2H), 2.62 - 2.55 (m, 1H), 2.50 - 2.46 (m, 2H), 2.18 - 2.09 (m, 2H), 1.97 - 1.88 (m, 1H), 1.65 - 1.54 (m, 2H), 0.96 - 0.90 (m, 2H), 0.68 - 0.62 (m, 2H).

The mixture was then separated by SFC (YMC Cellulose-C 10x250 mm, 5 µm 55/45 MeOH (0.1 % DEA) / CO₂, 15ml/min, 120bar, 40°C) to give two enantiomers: (1S,2S)-2-(5-chloro-2-cyanophenyl)-*N*-(6-(((6-cyclopropyl-8-(2-oxopyrrolidin-1-yl)imidazo[1,2-*a*]pyridin-2-yl)methyl)amino)pyrimidin-4-yl)cyclopropane-1-carboxamide and (1*R*,2*R*)-2-(5-chloro-2-cyanophenyl)-*N*-(6-(((6-cyclopropyl-8-(2-oxopyrrolidin-1-yl)imidazo[1,2-*a*]pyridin-2-yl)methyl)amino)pyrimidin-4-yl)cyclopropane-1-carboxamide.
First eluting isomer **(I-335):** ESI-MS (M+H)+: 567.3δ 10.69 (s, 1H), 8.26 (s, 1H), 8.22 (s, 1H), 7.96 (s, 1H), 7.88 (d, J=8.3 Hz, 1H), 7.67 (s, 1H), 7.54 (dd, J=1.9, 8.3 Hz, 1H), 7.43 (s, 1H), 7.32 (s, 1H), 7.13 (s, 1H), 4.59 - 4.59 (m, 2H), 4.16 (dd, J=7.0, 7.0 Hz, 2H), 2.62 - 2.55 (m, 1H), 2.50 - 2.46 (m, 2H), 2.18 - 2.09 (m, 2H), 1.97 - 1.88 (m, 1H), 1.65 - 1.54 (m, 2H), 0.96 - 0.90 (m, 2H), 0.68 - 0.62 (m, 2H). 1.65 min, RT = 1.65 min, 99.9 % e.e
Second eluting isomer **(I-336):** ESI-MS (M+H)+: 567.2, ¹H NMR (400 MHz, DMSO) substantially similary to (I-335). 3.86 min, RT = 3.86 min, 99.9 % e.e.

### Example 337: rac-(1S*,2S*)-2-(5-chloro-2-cyanophenyl)-N-(6-(((6-cyclopropyl-8-(2-hydroxy propan-2-yl)imidazo[1,2-a]yridin-2-yl)methyl)amino)pyrimidin-4-yl)cyclopropane-1-carboxamide (I-337)

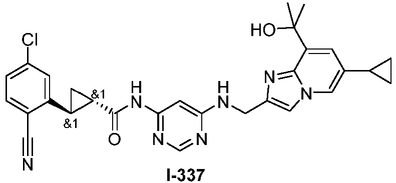

Using a similar procedure to that used for **(I-137), (I-337)** was prepared using (*rac-*(1*S**,2*S**))-2-(5-chloro-2-cyanophenyl)cyclopropane-1-carboxamide (92 mg, 0.41 mmol) and **2intermediate 574** (135 mg, 0.38 mmol), then purified by silica gel chromatography, eluting with a gradient of 0-10% MeOH in DCM (36 mg, 18 %) as a mixture of enantiomers. ESI-MS (M+H)+: 542.3, δ 10.67 (s, 1H), 8.22 (s, 1H), 8.18 (d, J=1.3 Hz, 1H), 7.91 - 7.87 (m, 2H), 7.59 (s, 1H), 7.54 (dd, J=2.1, 8.3 Hz, 1H), 7.42 (d, J=1.9 Hz, 1H), 7.33 (s, 1H), 7.06 (d, J=1.8 Hz, 1H), 5.52 (s, 1H), 4.59 - 4.59 (m, 2H), 2.64 - 2.57 (m, 1H), 2.50 - 2.47 (m, 1H), 1.96 - 1.88 (m, 1H), 1.66 (s, 6H), 1.64 - 1.54 (m, 2H), 0.94 - 0.89 (m, 2H), 0.68 - 0.63 (m, 2H).

### Example 338: (1S,2S)-2-(3-chlorophenyl)-N-(4-(((6-cyclopropylimidazo[1,2-a]pyridin-2-yl) methyl)amino)-6-methylpyridin-2-yl)cyclopropane-1-carboxamide (I-338)

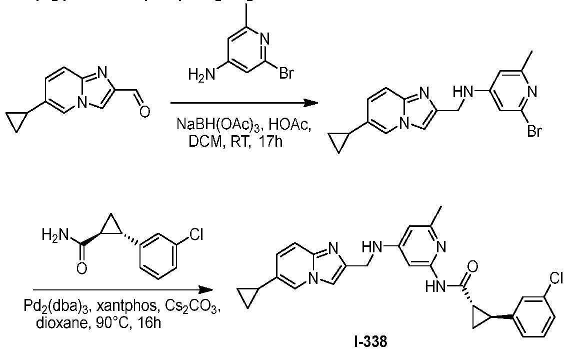

A mixture of **intermediate 828** (200 mg, 1.07 mmol), 2-bromo-6-methylpyridin-4-amine (187 mg, 1.0 mmol), and HOAc (10 drops) in DCM (5 mL) was stirred at room temperature for 1 h under N₂. Then the NaBH(OAc)₃ (318 mg, 1.5 mmol) was added to the mixture and stirred for 16 h. The mixture was diluted with DCM (20 mL) and then extracted with water (3 x 5 mL). The organic layer was concentrated *in vacuo* and purified by Prep - TLC (DCM/MeOH = 15/1) to give **2-bromo-N-((6-cyclopropylimidazo[1,2-a]pyridin-2-yl)methyl)-6-methylpyridin-4-amine intermediate 812** as a light yellow oil (140 mg, 36 %). ESI-MS [M +H]⁺: 357.1. **(1S,2S)-2-(3-chlorophenyl)-N-(4-(((6-cyclopropylimidazo[1,2-a]pyridin-2-yl)methyl)amino)-6-methylpyridin-2-yl)cyclopropane-1-carboxamide (I-338).** A mixture of **intermediate 812** (45 mg, 0.12 mmol), **intermediate 119** (23.4 mg, 0.12 mmol), Pd₂(dba)₃ (11 mg, 0.012 mmol), xantphos (7 mg, 0.012 mmol), and Cs₂CO₃ (98 mg, 0.23 mmol) in 1,4-dioxane ( 5 mL) was stirred at 90 °C for 16 h under N₂. The mixture was cooled to room temperature and concentrated in vacuo. The residue was purified by Prep-HPLC to give **((I-338)** (6 mg, 11 %) as a light yellow solid. ESI-MS [M +H]+: 472.2, δ 10.32 (s, 1H), 8.31 (s, 1H), 7.61 (s, 1H), 7.38 (d, J = 9.4 Hz, 1H), 7.31 - 7.23 (m, 4H), 7.13 (d, J = 7.7 Hz, 1H), 7.00 - 6.94 (m, 2H), 6.20 (s, 1H), 4.34 (d, J = 5.7 Hz, 2H), 2.37 - 2.33 (m, 2H), 2.17 (s, 3H), 1.94 - 1.89 (m, 1H), 1.45 - 1.40 (m, 1H), 1.34 - 1.30 (m, 1H), 0.93 - 0.88 (m, 2H), 0.68 - 0.64 (m, 2H).

The compounds in **Error! Reference source not found.** were prepared in a similar manner to **intermediate 812** from 6-cyclopropyl imidazo[1,2-a]pyridine-2-carbaldehyde and an appropriate aminopyridine.

**Table 30**

| **Structure** | **Analvtical Data** |
|---|---|
| 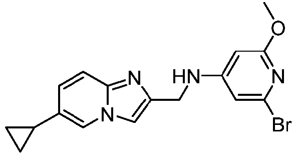 | **Intermediate 813:** 2-bromo-N-((6-cyclopropylimidazo[1,2-a]pyridin-2-yl)methyl)-6-methoxypyridin-4-amine |
| | ESI-MS [M +H]+: 373.2. |
| 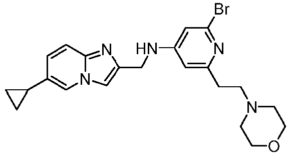 | **Intermediate 814:** 2-bromo-N-((6-cyclopropylimidazo[1,2-a]pyridin-2-yl)methyl)-6-(2-morpholinoethyl)pyridin-4-amine ESI-MS [M +H]+: 456.1 |

The compounds in Table 31 were prepared in a similar manner to I-338 from a cyclopropyl amide and an appropriate coupling partner.

**Table 31**

| **EG** | **Structure** | **Coupling Partner /Analvtical Data** |
|---|---|---|
| **339** | 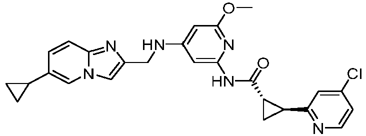 (1S,2S)-2-(4-chloropyridin-2-yl)-N-(4-(((6-cyclopropylimidazo[1,2-a]pyridin-2-yl)methyl)amino)-6-methoxypyridin-2-yl)cyclopropane-1-carboxamide **(I-339)** | **Intermediate 813**ESI-MS [M +H]+: 489.2. δ 10.17 (s, 1H), 8.42 (d, J = 5.3 Hz, 1H), 8.31 (s, 1H), 7.64 - 7.60 (m, 2H), 7.40 - 7.31 (m, 2H), 7.14 (s, 1H), 7.09 - 7.02 (m, 1H), 6.96 (dd, J = 9.3, 1.6 Hz, 1H), 5.63 (s, 1H), 4.32 (d, J = 5.7 Hz, 2H), 3.68 (s, 3H), 2.62 - 2.57 (m, 1H), 2.56 - 2.52 (m, 1H), 1.96 - 1.85 (m, 1H), 1.53 - 1.40 (m, 2H), 0.95 - 0.86 (m, 2H), 0.72 - 0.60 (m, 2H). |
| **340** | 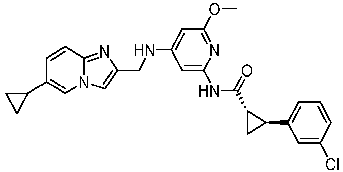 (1S,25)-2-(3-chlorophenyl)-N-(4-(((6-cyclopropylimidazo [1,2-a]pyridin-2-yl)methyl)amino)-6-methoxypyridin-2-yl)cyclopropane-1-carboxamide**(I-340)** | **Intermediate 813**ESI-MS [M +H]+: 488.2. δ 10.10 (s, 1H), 8.31 (s, 1H), 8.18 (s, 1H), 7.61 (s, 1H), 7.48 - 7.21 (m, 4H), 7.15 (d, J = 6.1 Hz, 2H), 7.06 (s, 1H), 6.98 - 6.95 (m, 1H), 5.64 (s, 1H), 4.32 (d, J = 5.6 Hz, 2H), 3.69 (s, 3H), 2.46 - 2.30 (m, 2H), 1.98 - 1.81 (m, 1H), 1.55 - 1.40 (m, 1H), 1.35-1.30 (m, 1H), 0.99 - 0.80 (m, 2H), 0.76 - 0.54 (m, 2H). |
| **341** | 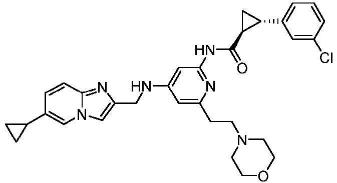 (1S,2S)-2-(3-chlorophenyl)-N-(4-(((6-cyclopropylimidazo[1,2-a]pyridin-2-yl)methyl)amino)-6-(2-morpholino ethyl)pyridin-2-yl)cyclopropane-1-carboxamide **(I-341)** | **Intermediate 814** |
| | | ESI-MS [M +H]+: 571.2, δ 10.32 (s, 1H), 8.31 (s, 1H), 7.62 (s, 1H), 7.37 (d, J = 9.3 Hz, 1H), 7.33 - 7.23 (m, 4H), 7.14 - 7.07 (m, 2H), 6.98 - 6.95 (m, 1H), 6.26 (s, 1H), 4.35 (d, J = 5.6 Hz, 2H), 3.56 - 3.51 (m, 4H), 2.67 - 2.62 (m, 4H), 2.45 - 2.32 (m, 6H), 1.94 - 1.87 (m, 1H), 1.46 - 1.41 (m, 1H), 1.34 - 1.30 (m, 1H), 0.93 - 0.88 (m, 2H), 0.68 - 0.64 (m, 2H). |
| **342** | 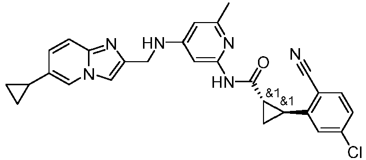 *rac-*(1S**,*2S*)-2-(5-chloro-2-cyanophenyl)-N-(4-(((6-cyclopropyl imidazo[1,2-a]pyridin-2-yl)methyl) amino)-6-methylpyridin-2-yl)cyclo propane-1-carboxamide **(I-342)** | **Intermediate 812** ESI-MS [M +H]+: 497.2, δ 10.36 (s, 1H), 8.30 (s, 1H), 8.23 (s, 1H), 7.85 (d, J = 8.3 Hz, 1H), 7.62 (s, 1H), 7.53 - 7.50 (m, 2.0 Hz, 1H), 7.39 - 7.37 (m, 2H), 7.30 (s, 1H), 7.03 - 7.00 (m, 1H), 6.98 - 6.95 (m, 1.7 Hz, 1H), 6.20 (s, 1H), 4.35 (d, J = 5.7 Hz, 2H), 2.57 - 2.54 (m, 2H), 2.18 (s, 3H), 1.94 - 1.87 (m, 1H), 1.51 - 1.49 (m, 2H), 0.93 - 0.88 (m, 2H), 0.68 - 0.64 (m, 2H). |

### Example343: (1S,2S)-2-(4-chloropyridin-2-yl)-N-(4-(((6-cyclopropylimidazo[1,2-a]pyridin-2-yl)methyl)amino)-6-oxo-1,6-dihydropyridin-2-yl)cyclopropane-1-carboxamide (I-343)

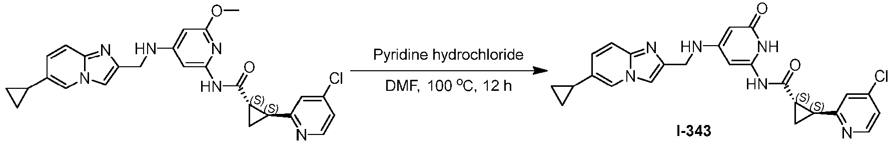

A mixture of **(I-339)** (70 mg, 0.14 mmol) and pyridine hydrochloride (800 mg, 6.9 mmol) in DMF (15 mL) was stirred at 100 °C for 12 h. After cooling to room temperature, the mixture was diluted with water (100 mL) and extracted with EtOAc (3 x 50 mL). The combined extract was washed with brine (50 mL), dried over Na₂SO₄, and concentrated to give the crude product. The crude product was purified by preparative TLC (DCM : MeOH = 10 : 1) to give **(I-343)** (3.7 mg, 5.4% yield) as a white solid. ESI-MS [M+H]+: 475.1. δ 11.10 - 10.67 (m, 2H), 10.64 - 10.17 (m, 2H), 8.41 (d, J = 5.3 Hz, 1H), 8.31 (s, 1H), 7.64 (d, J = 6.4 Hz, 2H), 7.43 - 7.30 (m, 2H), 6.97 (dd, J = 9.3, 1.7 Hz, 1H), 4.99 (s, 1H), 4.26 (d, J = 5.5 Hz, 2H), 2.64 - 2.56 (m, 1H), 2.45 - 2.36 (m, 1H), 1.96 - 1.86 (m, 1H), 1.56 - 1.41 (m, 2H), 0.96 - 0.86 (m, 2H), 0.71 - 0.60 (m, 2H).

### Example 344: rac-(1S*,2S*)-2-(3-chlorophenyl)-N-(4-(((6-cyclopropylimidazo[1,2-a]pyridin-2-yl)methyl)amino)-1,3,5-triazin-2-yl)cyclopropane-1-carboxamide (I-344)

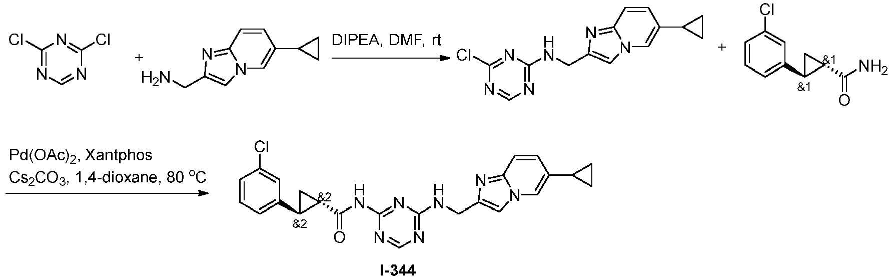

DIPEA (0.52 mL, 3.0 mmol) was added to a stirred solution of 2,4-dichloro-1,3,5-triazine (150 mg, 1.0 mmol) and **intermediate 251** (190 mg, 1.0 mmol) in DMF (2.0 mL) and stirred at room temperature for 1 h. The reaction was diluted with water (25 mL) and brine (25 mL) and extracted with EtOAc (3 x 50 mL). The organics were combined, dried over MgSO₄, and concentrated *in vacuo.* The residue was purified by silica gel chromatography, eluting with a gradient of 0-5 % MeOH in DCM to give **4-chloro-N-((6-cyclopropylimidazo[1,2-a]pyridin-2-yl)methyl)-1,3,5-triazin-2-amine intermediate 815** (91 mg, 30 %). ESI-MS (M+H)+: 301.1, δ 9.18 - 9.11 (m, 1H), 8.49 (s, 0.5H), 8.43 (s, 0.5H), 8.33 - 8.29 (m, 1H), 7.68 (s, 1H), 7.42 - 7.37 (m, 1H), 7.01 - 6.97 (m, 1H), 4.64 - 4.57 (m, 2H), 1.96 - 1.89 (m, 1H), 0.95 - 0.90 (m, 2H), 0.71 - 0.65 (m, 2H), 1:1 mixture of rotamers.

**Intermediate 815** (85 mg, 0.283 mmol), (rac-(1S*,2S*))-2-(3-chlorophenyl)cyclopropane-1-carboxamide (55 mg, 0.28 mmol), Xantphos (65 mg, 0.11 mmol), Cs₂CO₃ (140 mg, 0.42 mmol), and Pd(OAc)₂ (13 mg, 0.057 mmol) were suspended in 1,4-dioxane (2.0 mL) and degassed with N₂ for 5 min. The reaction was then heated to 80 °C under a N₂ environment for 1.5 h. The reaction was cooled to room temperature and diluted with water (25 mL) and brine (25 mL) and extracted with EtOAc (3 x 20 mL). The organics were combined, dried over MgSO₄ and concentrated in vacuo. The residue was purified by reverse phase preparative HPLC to give **(I-344)** (13.7 mg, 11 %). ESI-MS (M+H)+: 460.2, δ 10.62 (s 0.4H), 10.61 (s, 0.6H), 8.40 (t, J=6.1 Hz, 0.6H), 8.37 (s, 0.4H), 8.30 (s, 0.6H), 8.29 - 8.26 (m, 1.4H), 7.65 (s, 0.6H), 7.60 (s, 0.4H), 7.36 (d, J=9.3 Hz, 1H), 7.32 - 7.20 (m, 3H), 7.18 - 7.14 (m, 0.4H), 7.12 - 7.08 (m, 0.6H), 6.96 - 6.93 (m, 1H), 4.58 (d, J=6.0 Hz, 0.8H), 4.52 (d, J=6.1 Hz, 1.2H), 2.78 - 2.75 (m, 0.6H), 2.64 - 2.61 (m, 0.4H), 2.47 - 2.39 (m, 1H), 1.95 - 1.87 (m, 1H), 1.52 - 1.45 (m, 1H), 1.43 - 1.37 (m, 1H), 0.94 - 0.88 (m, 2H), 0.69 - 0.64 (m, 2H), 6:4 mixture of rotamers.

### Example 345: rac-(1S*,2S*)-2-(3-chlorophenyl)-N-(6-(((6-cyclopropylimidazo[1,2-a]pyridin-2-yl)methyl)amino)pyrazin-2-yl)cyclopropane-1-carboxamide (I-345)

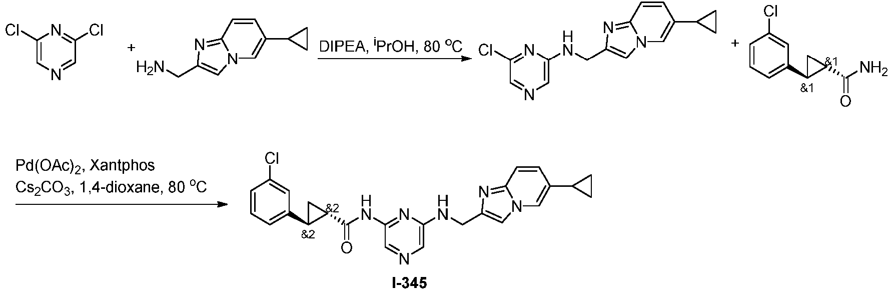

DIPEA (0.26 mL, 1.51 mmol) was added to a stirred solution of 2,6-dichloropyrazine (75 mg, 0.50 mmol) and **intermediate 251** (100 mg, 0.55 mmol) in isopropanol (1.0 mL) and stirred at 80 °C for 18 h. The reaction was diluted with water (40 mL) and extracted with EtOAc (3 x 30 mL). The organics were combined, dried over MgSO₄, and concentrated *in vacuo.* The residue was purified by silica gel chromatography, eluting with a gradient of 0-5 % MeOH in DCM to give **6-chloro-N-((6-cyclopropylimidazo[1,2-a]pyridin-2-yl)methyl)pyrazin-2-amine intermediate 816** (54 mg, 36 %) as a formic acid salt. ESI-MS (M+H)+: 300.2

**Intermediate 816** (50 mg, 0.17 mmol), rac-(1S*,2S*)-2-(3-chlorophenyl)cyclopropane-1-carboxamide (33 mg, 0.14 mmol), XantPhos (19 mg, 0.033 mmol), and Cs₂CO₃ (82 mg, 0.25 mmol), and Pd(OAc)₂ (15 mg, 0.067 mmol) were suspended in 1,4-dioxane (2.0 mL) and degassed with N₂ for 5 min. The reaction mixture was heated to 80 °C under a N₂ environment for 3 h. The reaction was cooled to room temperature and diluted with water (30 mL) and saturated brine solution (30 mL). The solution was extracted with DCM (3 × 20 mL) and the organics were combined, dried over MgSO₄ and concentrated *in vacuo.* The residue was purified by reverse phase preparative HPLC to give **(I-345)** (6.7 mg, 5 %). ESI-MS (M+H)+: 459.2, δ, ppm 10.45 (1H, s), 8.46 (1H, s), 8.31 (1H, t, J=0.8 Hz), 7.74 (1H, s), 7.68 (1H, s), 7.50 (1H, t, J=5.8 Hz), 7.38 (1H, d, J=9.3 Hz), 7.35 - 7.30 (1H, m), 7.29 - 7.25 (2H, m), 7.17 (1H, td, J=1.3, 7.6 Hz), 6.97 (1H, dd, J=1.8, 9.3 Hz), 4.56 (2H, d, J=5.7 Hz), 2.48 - 2.39 (2H, m), 1.92 (1H, tdd, J=3.8, 9.3, 9.3 Hz), 1.50 (1H, ddd, J=4.1, 5.2, 9.2 Hz), 1.40 (1H, ddd, J=4.2, 6.4, 8.1 Hz), 0.92 (2H, ddd, J=4.3, 6.3, 8.4 Hz), 0.67 (2H, ddd, J=3.2, 4.6, 6.7 Hz).

### Example 346: rac-(1S*,2S*)-2-(3-chlorophenyl)-N-(4-(((6-cyclopropylimidazo[1,2-a]pyridin-2-yl)methyl)amino)pyrimidin-2-yl)cyclopropane-1-carboxamide (I-346)

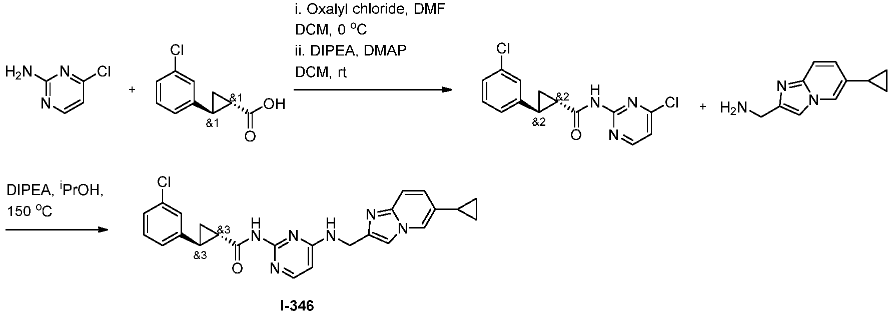

Oxalyl chloride (0.33 mL, 3.81 mmol) was added to a stirred solution of **intermediate 1149** (250 mg, 1.27 mmol) and DMF (0.02mL) in DCM (4 mL) and stirred at room temperature for 20 min. The reaction was concentrated *in vacuo.* The residue was dissolved in DCM (3 mL) and added to a stirred solution of 4-chloropyrimidin-2-amine (165 mg, 1.27 mmol) and DMAP (16 mg, 0.13 mmol) in DCM (1 mL). DIPEA (0.66 mL, 3.81 mmol) was then added and the reaction was stirred at room temperature for 18 h. The reaction was quenched with water (20 mL) and extracted with DCM (3 x 15 mL). The organics were combined, dried (MgSO₄), and concentrated *in vacuo.* The residue was purified by silica gel chromatography, eluting with a gradient of 0-100 % EtOAc in cyclohexane to give **rac-(1S*,2S*)-2-(3-chloro phenyl)-N-(4-chloropyrimidin-2-yl)cyclopropane-1-carboxamide intermediate 817** (90 mg, 23 %). ¹H NMR (400 MHz, CDCl₃) δ 8.45 (d, J=5.3 Hz, 1H), 8.41 (s, 1H), 7.23 - 7.14 (m, 3H), 7.10 - 7.02 (m, 2H), 2.86 - 2.78 (m, 1H), 2.69 - 2.61 (m, 1H), 1.86 - 1.80 (m, 1H), 1.21 - 1.15 (m, 1H).

**Intermediate 817** (30 mg, 0.097 mmol), intermediate 251 (22 mg, 0.12 mmol) and DIPEA (51 µL, 0.29 mmol) were dissolved in isopropanol (2.5 mL) and heated at 150 °C in the microwave for 1 h. The reaction was cooled to room temperature and concentrated in vacuo. The residue was purified by reverse phase preparative HPLC to give **(I-346)** (3.1 mg, 7 %) as a formic acid salt. ESI-MS (M+H)+: 459.3,) δ 10.22 (s, 1H), 8.35 (s, 1H), 8.29 (s, 1H), 7.94 (s, 1H), 7.86 (s, 1H), 7.73 (s, 1H), 7.43 (d, J=9.3 Hz, 1H), 7.33 - 7.30 (m, 3H), 7.17 (d, J=7.8 Hz, 1H), 7.01 (dd, J=1.4, 9.2 Hz, 1H), 6.30 (d, J=5.8 Hz, 1H), 4.59 (br s, 2H), 2.49 - 2.38 (m, 1H), 2.01 - 1.93 (m, 1H), 1.56 - 1.49 (m, 1H), 1.43 - 1.37 (m, 1H), 1.00 - 0.93 (m, 2H), 0.75-0.69 (m,2H).

### Example 347: rac-(1S*,2S*)-2-(3-chlorophenyl)-N-(2-(((6-cyclopropylimidazo[1,2-a]pyridin-2-yl)methyl)amino)pyrimidin-4-yl)cyclopropane-1-carboxamide (I-347)

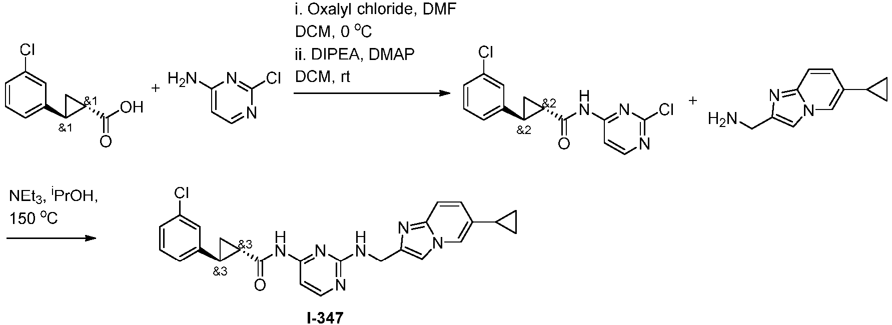

Oxalyl chloride (0.27 mL, 3.05 mmol) was added to a stirred solution of (*rac*-(1*S**,2*S**))-2-(3-chlorophenyl)cyclopropane-1-carboxylic acid (200 mg, 1.02 mmol) and DMF (0.02mL) in DCM (4 mL) and stirred at room temperature for 20 min. The reaction was concentrated *in vacuo.* The residue was dissolved in DCM (3.0 mL) and added to a stirred solution of 2-chloropyrimidin-4-amine (130 mg, 1.02 mmol) and DMAP (25 mg, 0.20 mmol) in DCM (1.0 mL). DIPEA (0.27 mL, 1.53 mmol) was then added and the reaction was stirred at rt for 18 h. The reaction was quenched with water (20 mL) and extracted with DCM (3 x 15 mL). The organics were combined, dried over MgSO₄, and concentrated *in vacuo.* The residue was purified by silica gel chromatography, eluting with a gradient of 0-100 % EtOAc in cyclohexane to give rac-(**1S*,2S*)-2-(3-chlorophenyl)-N-(2-chloropyrimidin-4-yl)cyclopropane-1-carboxamide intermediate 818** (145 mg, 46 %). ESI-MS (M+H)+: 308.1, δ 11.62 (s, 1H), 8.60 (d, J=5.8 Hz, 1H), 8.07 (d, J=5.8 Hz, 1H), 7.34 - 7.28 (m, 3H), 7.18 (d, J=7.5 Hz, 1H), 2.41 - 2.35 (m, 1H), 1.59 - 1.50 (m, 2H), one proton hidden under DMSO peak.

**Intermediate 818** (75 mg, 0.24 mmol), intermediate 251 (46 mg, 0.24 mmol), and triethylamine (0.1 mL, 0.73 mmol) were dissolved in isopropanol (2.5 mL) and heated at 150 °C in the microwave for 30 min. The reaction was cooled to room temperature and concentrated in vacuo. The residue was purified by reverse phase preparative HPLC to give **(I-347)** (23 mg, 21 %) as a formic acid salt. ESI-MS (M+H)+: 459.3, δ 10.61 (s, 1H), 8.28 (s, 1H), 8.16 (d, J=5.7 Hz, 1H), 7.58 (s, 1H), 7.37 - 7.29 (m, 2H), 7.28 - 7.25 (m, 3H), 7.15 (d, J=7.7 Hz, 1H), 6.93 (dd, J=1.8, 9.3 Hz, 1H), 4.56 (d, J=6.0 Hz, 2H), 2.47 - 2.38 (m, 2H), 1.93 - 1.85 (m, 1H), 1.52 - 1.37 (m, 2H), 0.92 - 0.87 (m, 2H), 0.67 - 0.62 (m, 2H).

### Example 348: rac-(1S*,2S*)-2-(3-chlorophenyl)-N-(6-(((6-cyclopropylimidazo[1,2-a]pyridin-2-yl)methyl)amino)pyridazin-4-yl)cyclopropane-1-carboxamide (I-348)

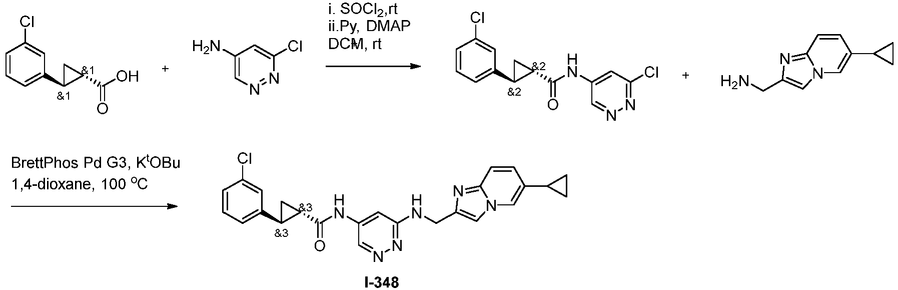

**Intermediate 1149** (180 mg, 0.91 mmol) was dissolved in thionyl chloride (0.33 mL, 4.57 mmol) and stirred at rt for 40 min. The reaction was concentrated *in vacuo.* The residue was dissolved in DCM (5.0 mL) and added to a stirred solution of 6-chloropyridazin-4-amine (118 mg, 0.913 mmol) in DCM (5.0 mL). Pyridine (0.11 mL, 1.37 mmol) was then added and the reaction was stirred at room temperature for 18 h. The reaction was quenched with water (20 mL) and extracted with DCM (3 x 15 mL), the organics were combined, dried (MgSO₄), and concentrated *in vacuo.* The residue was purified by silica gel chromatography, eluting with a gradient of 0-10 % MeOH in DCM to give **rac-(1S*,2S*)-2-(3-chlorophenyl)-N-(6-chloropyridazin-4-yl)cyclo propane-1-carboxamide intermediate 819** (100 mg, 36 %). ESI-MS (M+H)+: 309.1, ¹H NMR (400 MHz, CDCl₃) δ 9.05 (d, J=2.3 Hz, 1H), 8.95 (s, 1H), 8.34 (d, J=2.1 Hz, 1H), 7.25 - 7.18 (m, 2H), 7.08 (s, 1H), 7.05 - 7.02 (m, 1H), 2.65 - 2.58 (m, 1H), 2.14 - 2.08 (m, 1H), 1.82 - 1.76 (m, 1H), 1.50 - 1.43 (m, 1H).

**Intermediate 819** (69 mg, 0.22 mmol), **intermediate 251** (62 mg, 0.33 mmol), and K^{t}OBu (75 mg, 0.67 mmol) were suspended in 1,4-dioxane (2.7 mL) and degassed with N₂ for 5 min, BrettPhos Pd G3 (20 mg, 0.022 mmol) was then added and the reaction was heated at 100 °C under a N₂ environment for 18 h. The reaction was cooled to room temperature and filtered through Celite^{®}. The filtrate was diluted with EtOAc (40 mL) and water (40 mL). The layers were separated and the aqueous layer was then further extracted with EtOAc (2 x 20 mL). The organics were combined, dried (MgSO₄), and concentrated in vacuo. The residue was purified by reverse phase preparative HPLC to give **(I-348)** (8.4 mg, 8 %). ESI-MS (M+H)+: 459.3, δ 10.59 (s, 1H), 8.49 (d, J=2.1 Hz, 1H), 8.31 (s, 1H), 7.65 (s, 1H), 7.41 - 7.26 (m, 6H), 7.20 (d, J=7.5 Hz, 1H), 6.97 (dd, J=1.8, 9.3 Hz, 1H), 4.61 (d, J=5.6 Hz, 2H), 2.48 - 2.41 (m, 1H), 2.17 - 2.11 (m, 1H), 1.96 - 1.88 (m, 1H), 1.58 - 1.46 (m, 2H), 0.95 - 0.89 (m, 2H), 0.70 - 0.65 (m, 2H).

### Example 349: 2-(3-chlorophenyl)-2-cyano-N-(6-(((6-cyclopropylimidazo[1,2-a]pyridin-2-yl)methyl)amino)pyrimidin-4-yl)cyclopropane-1-carboxamide (I-349)

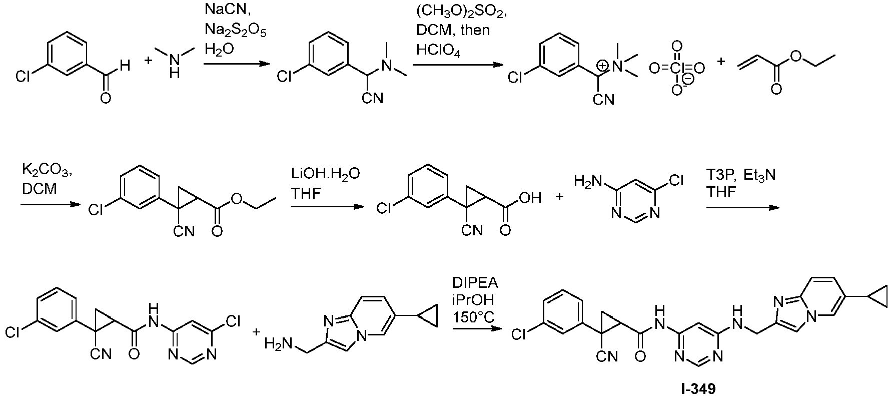

3-chlorobenzaldehyde (2.0 g, 14.23 mmol) was added dropwise to a solution of Na₂S₂O₅ (2.7 g, 14.23 mmol) in water (7 mL), followed by dimethylamine (40 % aq., 1.8 mL, 14.23 mmol) at room temperature. After 30 min the mixture was cooled to 0 °C and a solution of NaCN (0.7 g, 14.23 mmol) in water (3 mL) was added. The mixture was allowed to warm to room temperature and stirred for 18 h. The reaction mixture was diluted with water and extracted with DCM (3 x 10 mL) The organic phases were combined and passed through a phase separator cartridge and concentrated *in vacuo* to give **2-(3-chlorophenyl)-2-(dimethylamino)acetonitrile intermediate 820** as a residue that was taken on to the next stage without further purification.

Dimethyl sulfate (1.60 mL, 14.23 mmol) was added to a cooled solution of **intermediate 820** (2.77 g, 14.23 mmol) in DCM (10 mL) at 0 °C. After 2 h the mixture was allowed to warm to room temperature and stirred for 18 h. The mixture was concentrated *in vacuo* and the residue was dissolved in water (10 mL) then extracted with DCM (3 x 10 mL). The aqueous phase was treated with HClO₄ (70 %, aq. 0.9 mL). The precipitate formed was collected, washed with Et₂O, dried to give **1-(3-chlorophenyl)-1-cyano-N,N,N-trimethylmethammonium perchlorate intermediate 821** (1.37 g, 31 % as a perchlorate salt).

. Potassium carbonate (2.24 g, 16.17 mmol) was added to a vigorously stirred mixture of **intermediate 821** (0.50 g, 1.52 mmol) followed by a solution of ethyl acrylate (0.88 mL, 8.09 mmol) in DCM (20 mL). The mixture was stirred at room temperature for 18 h. The mixture was diluted with water (20 mL) and extracted with DCM (3 x 20 mL). The organic phases were combined and passed through a phase separator cartridge and concentrated *in vacuo.* Purification by silica gel column chromatography, eluting with a gradient of 0-40 % ethyl acetate in hexane to give **ethyl 2-(3-chlorophenyl)-2-cyanocyclopropane-1-carboxylate intermediate 822** (0.17 g, 43 %) as a clear oil (mixture of E:Z isomers). ESI-MS: (M+H)+: 250.1, **Isomer 1:** ¹H NMR (400 MHz, CDCl₃) δ 7.39 - 7.27 (m, 4H), 4.30 (q, J=7.0 Hz, 2H), 2.37 (dd, J=7.7, 7.7 Hz, 1H), 2.30 - 2.26 (m, 1H), 1.84 (dd, J=5.8, 8.3 Hz, 1H), 1.34 (dd, J=7.1, 7.1 Hz, 3H). **Isomer 2:** ¹H NMR (400 MHz, CDCl₃) δ 7.39 - 7.27 (m, 4H), 3.99 - 3.91 (m, 2H), 2.73 (dd, J=6.9, 8.7 Hz, 1H), 2.23 - 2.17 (m, 1H), 1.95 (dd, J=5.8, 8.6 Hz, 1H), 1.05 (dd, J=7.1, 7.1 Hz, 3H).

Lithium hydroxide (1M, aq, 0.99 mL, 0.99 mmol) was added dropwise to a cooled solution of **intermediate 822** (124 mg, 0.50 mmol) at 0 °C in THF (5 mL ), and the resulting mixture was stirred for 2 h. The mixture was diluted with water, acidified with HCl (1 M. aq, 2 mL), and extracted with EtOAc (3 x 10 mL). The organic phases were combined, dried over MgSO₄, filtered, and concentrated *in vacuo* to give **2-(3-chlorophenyl)-2-cyanocyclopropane-1-carboxylic acid intermediate 823** as a clear oil (105 mg, 95 %) which was used without further purification. **Isomer 1:** ¹H NMR (400 MHz, CDCl₃) δ 7.45 - 7.28 (m, 4H), 2.38 (dd, J=7.7, 7.7 Hz, 1H), 2.28 - 2.24 (m, 1H), 1.95 - 1.85 (m, 1H). **Isomer 2:** ¹H NMR (400 MHz, CDCl₃) δ 7.45 - 7.28 (m, 4H), 2.79 - 2.73 (m, 1H), 2.22 - 2.17 (m, 1H), 2.05 - 2.01 (m, 1H).

T3P (1.53 mL, 2.58 mmol) was added dropwise to a solution of **intermediate 823** (228 mg, 1.03 mmol), 4-amino-6-chloro-pyrimidine (120 mg, 0.93 mmol) and triethylamine (0.50 mL, 3.61 mmol) in THF (5 mL). The mixture was stirred for 60 h, then concentrated *in vacuo.* The residue was partitioned between EtOAc (10 mL) and NaHCO₃ solution (sat. aq., 10 mL). The aqueous layer was extracted with EtOAc (2 x 10 mL). The combined organics were dried over MgSO₄ then concentrated *in vacuo.* The residue was purified by silica gel column chromatography, eluting with a gradient of 0-50 % ethyl acetate in cyclohexane to give **2-(3-chlorophenyl)-N-(6-chloropyrimidin-4-yl)-2-cyanocyclopropane-1-carboxamide intermediate 824** (88 mg, 26 %) as a pink solid which was used without further purification.

Using similar procedure to that for **I-55, (I-349)** was made from **intermediate 824** and **intermediate 251** hydrochloride (5 mg, 7 %). ESI-MS (M+H): 484.4, δ 10.99 - 10.94 (m, 1H), 8.31 (s, 1H), 8.25 (s, 1H), 7.83 - 7.82 (m, 1H), 7.63 - 7.59 (m, 1H), 7.56 - 7.49 (m, 1H), 7.45 - 7.36 (m, 3H), 7.01 - 6.97 (m, 2H), 6.78 - 6.72 (m, 1H), 4.64 - 4.50 (m, 2H), 2.32 - 2.28 (m, 1H), 2.15 - 2.09 (m, 1H), 1.99 - 1.91 (m, 1H), 0.99 - 0.92 (m, 2H), 0.73 - 0.67 (m, 2H). 1H missing, assumed under water peak

### Example 350: (1S,2S)-2-(3-chlorophenyl)-N-(6-(((6-cyclopropylimidazo[1,2-a]pyridin-2-yl)methyl)amino)pyrimidin-4-yl)-N-methylcyclopropane-1-carboxamide (I-350)

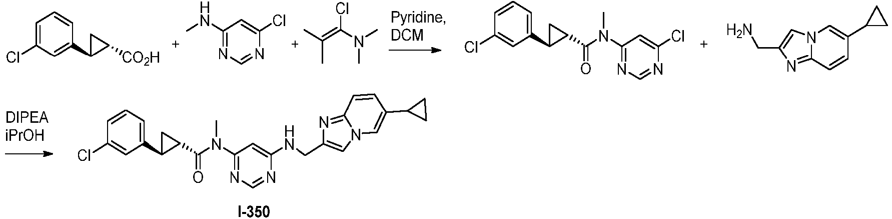

1-chloro-*N*,*N*.2-trimethylprop-1-en-1-amine (0.081 mL, 0.61 mmol) was added to a N₂ purged solution of (1*S*,2*S*)-2-(3-chlorophenyl)cyclopropane-1-carboxylic acid (100 mg, 0.509 mmol) in DCM (2.0 mL) and the mixture was stirred at room temperature. After 2 h, 6-chloro-N-methylpyrimidin-4-amine (80 mg, 0.559 mmol) was added, followed by pyridine (0.062 mL, 0.763 mmol), and the mixture was stirred at room temperature for 18 h. The mixture was diluted with water (5.0 mL), then extracted with DCM (3 x 5 mL). The organics were concentrated *in vacuo* onto silica gel and purified by silica gel column chromatography, eluting with a gradient of 0-100 % ethyl acetate in hexane to give **(1S,2S)-2-(3-chlorophenyl)-N-(6-chloropyrimidin-4-yl)-N-methylcyclopropane-1-carboxamide intermediate 825** (75 mg, 45 %). ESI-MS (M+H)+: 322.1, ¹H NMR (400 MHz, CDCl₃) δ 8.74 (d, J=0.8 Hz, 1H), 7.81 (s, 1H), 7.25 - 7.20 (m, 2H), 7.12 (d, J=2.0 Hz, 1H), 7.05 - 7.01 (m, 1H), 3.60 (s, 3H), 2.62 (ddd, J=4.2, 6.4, 9.1 Hz, 1H), 2.23 (ddd, J=4.2, 5.4, 8.1 Hz, 1H), 1.88 - 1.82 (m, 1H), 1.49 - 1.42 (m, 1H).

Using similar procedure to that for **(I-55), (I-350)** was made from **intermediate 825** and **intermediate 251** (25 mg, 23 %). ESI-MS (M+H): 473.5, δ 8.35 - 8.34 (m, 2H), 7.93 (t, J=5.8 Hz, 1H), 7.69 (s, 1H), 7.42 (d, J=9.3 Hz, 1H), 7.32 - 7.25 (m, 4H), 7.01 (dd, J=1.8, 9.3 Hz, 1H), 6.65 - 6.61 (m, 1H), 4.66 - 4.59 (m, 2H), 2.53 - 2.49 (m, 1H), 2.32 (s, 1H), 2.00 - 1.92 (m, 1H), 1.59 - 1.53 (m, 1H), 1.34 - 1.28 (m, 1H), 0.99 - 0.93 (m, 2H), 0.74 - 0.68 (m, 2H). 3H missing, assumed under DMSO or water peak.

### Example 351: rac-(1S*,2S*)-N-(6-(((6-cyclopropylimidazo[1,2-a]pyridin-2 yl)methyl)amino)pyrimidin-4-yl)-2-(3-cyclopropylphenyl)cyclopropane-1-carboxamide (I-351)

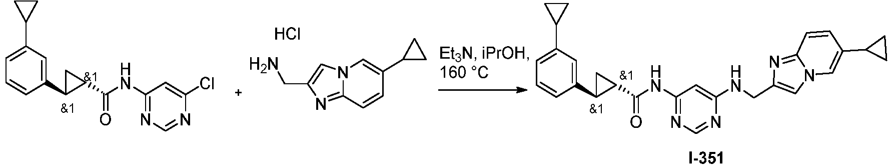

To a suspension of **intermediate 251** hydrochloride (140 mg, 0.45 mmol) and rac-(1S*,2S*)-N-(6-chloropyrimidin-4-yl)-2-(3-cyclopropylphenyl)cyclopropane-1-carboxamide (130 mg, 0.58 mmol) in 2-propanol (4.0 mL) was added triethylamine (0.25 mL, 1.8 mmol) and the reaction mixture was stirred at 80 °C until dissolution, then stirred in a microwave at 160 °C for 4.5 h. The reaction mixture was allowed to cool to room temperature and concentrated *in vacuo.* The residue was dissolved in DCM (25 mL) and washed with water (2×25 mL). The organics were passed through a hydrophobic frit and concentrated in vacuo. The residue was purified by preparative HPLC to give **(I-351)** (47 mg, 22 %) as an off white solid. ESI-MS (M+H): 465.4, δ 10.62 (s, 1H), 8.35 (s, 1H), 8.24 (s, 1H), 7.89 - 7.89 (m, 1H), 7.66 (s, 1H), 7.42 (d, J=9.3 Hz, 1H), 7.35 - 7.30 (m, 1H), 7.23 - 7.17 (m, 1H), 7.04 - 6.98 (m, 1H), 6.98 - 6.93 (m, 2H), 6.92 - 6.89 (m, 1H), 4.62 - 4.61 (m, 2H), 2.41 - 2.35 (m, 2H), 2.01 - 1.88 (m, 2H), 1.52 - 1.45 (m, 1H), 1.42 - 1.35 (m, 1H), 1.01 - 0.92 (m, 4H), 0.74 - 0.67 (m, 4H).

### Example 352: rac-(1S*,2S*)-2-(5-chloro-2-cyanophenyl)-N-(6-((6-cyclopropyl imidazo[1,2-a]pyridin-2-yl)(hydroxy)methyl)pyrimidin-4-yl)cyclopropane-1-carboxamide (I-352)

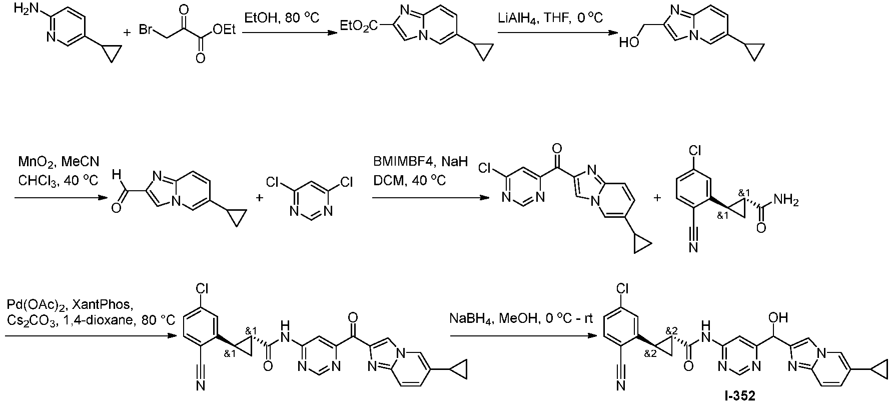

5-Cyclopropylpyridin-2-amine (1.5 g, 11 mmol) and ethyl bromopyruvate (1.7 mL, 13 mmol) were dissolved in EtOH (164 mL) and heated to 80 °C for 1 h. The reaction was cooled to room temperature and concentrated *in vacuo.* The residue was diluted with DCM (100 mL) and washed with NaHCO₃ (sat. aq., 3 x 50 mL). The combined organics were dried over MgSO₄, then concentrated *in vacuo.* The residue was purified by column chromatography on silica gel, eluting with 0-100% EtOAc in cyclohexane to give **ethyl 6-cyclopropylimidazo[1,2-a]pyridine-2-carboxylate intermediate 826** (1.76 g, 68%). ESI-MS (M+H)+: 231, ¹H NMR (400 MHz, CDCl₃) δ 8.09 (s, 1H), 7.90 (s, 1H), 7.57 (d, J=9.5 Hz, 1H), 6.99 (dd, J=1.8, 9.4 Hz, 1H), 4.45 (q, J=7.2 Hz, 2H), 1.94 - 1.86 (m, 1H), 1.44 (t, J=7.0 Hz, 3H), 1.03 - 0.97 (m, 2H), 0.73 - 0.68 (m, 2H).

LiAlH4 (1.0 M in THF, 17 mL, 17 mmol) was added dropwise to a stirred solution of intermediate 826 (2.0 g, 8.7 mmol) in THF (25 mL) at 0 °C. The reaction was stirred at 0 °C for 1 h. The reaction was quenched by pouring into NH₄Cl (sat. aq., 50 mL). The mixture was diluted with DCM, filtered through Celite^{®}, and washed with DCM. The layers were separated and the aqueous phase was further extracted with DCM (2 x 20 mL). The combined organics were dried over MgSO₄, then concentrated in vacuo to give (6-cyclopropylimidazo[1,2-a]pyridin-2-yl)methanol intermediate 827 (1.3 g, 80%) which was carried forward without further purification. ESI-MS (M+H)+: 189, ¹H NMR (400 MHz, CDCl₃) δ 7.87 (d, J=0.4 Hz, 1H), 7.44 (d, J=8.0 Hz, 2H), 6.93 (dd, J=1.8, 9.4 Hz, 1H), 4.82 (s, 2H), 1.92 - 1.84 (m, 1H), 0.99 - 0.93 (m, 2H), 0.70 - 0.65 (m, 2H).

MnO₂ (3.1 g, 36 mmol) was added to a stirred solution of **intermediate 827** (670 mg, 3.6 mmol) in MeCN (10 mL) and CHCl₃ (10 mL). The reaction was heated at 40 °C for 1 h. The reaction was cooled to room temperature and filtered through Celite^{®}. The Celite^{®} was washed with DCM and the filtrate was concentrated *in vacuo* to give **6-cyclopropylimidazo[1,2-a]pyridine-2-carbaldehyde intermediate 828** (520 mg, 78%) which was carried forward without further purification. ESI-MS (M+H)+: 187, ¹H NMR (400 MHz, CDCl₃) δ 10.13 (s, 1H), 8.07 (s, 1H), 7.93 (d, J=0.4 Hz, 1H), 7.58 (d, J=9.5 Hz, 1H), 7.03 (dd, J=1.7, 9.5 Hz, 1H), 1.95 - 1.87 (m, 1H), 1.05 - 0.99 (m, 2H), 0.75 - 0.70 (m, 2H).

1-Butyl-3-methylimidazolium tetrafluoroborate (0.17 mL, 0.91 mmol) was added to a stirred degassed solution of 4,6-dichloropyrimidine (320 mg, 2.1 mmol) and **intermediate 828** (520 mg, 2.8 mmol) in DCM (50 mL), followed by the addition of NaH (60% in mineral oil, 110 mg, 2.8 mmol). The reaction was then heated at 40 °C for 1 h and cooled to room temperature. The reaction was diluted with DCM (30 mL) and washed with water (2 x 30 mL) and brine (sat. aq., 30 mL). The combined organics were dried over MgSO₄ then concentrated *in vacuo.* The residue was purified by column chromatography on silica gel, eluting with 0-100% EtOAc in cyclohexane to give **6-chloropyrimidin-4-yl)(6-cyclopropylimidazo[1,2-a]pyridin-2-yl)methanone intermediate 829** (320 mg, 50%). ESI-MS (M+H)+: 299, ¹H NMR (400 MHz, CDCl₃) δ 9.20 (s, 1H), 8.88 (s, 1H), 8.24 (s, 1H), 7.96 (d, J=0.4 Hz, 1H), 7.61 (d, J=9.4 Hz, 1H), 7.04 (dd, J=1.8, 9.5 Hz, 1H), 1.96 - 1.88 (m, 1H), 1.06 - 1.00 (m, 2H), 0.76 - 0.71 (m, 2H).

A suspension of **intermediate 829** (41 mg, 0.14 mmol), **intermediate 118** (3 mg, 0.14 mmol), XantPhos (15 mg, 0.03 mmol), and Cs₂CO₃ (66 mg, 0.20 mmol) in 1,4-dioxane (2.0 mL) was degassed for 5 min. Pd(OAc)₂ (6.1 mg, 0.03 mmol) was added and the reaction mixture was stirred at 80 °C for 16 h. The reaction mixture was cooled to room temperature, filtered through Celite^{®}, and concentrated in vacuo. The residue was purified by column chromatography on silica gel, eluting with a gradient of 0-10% MeOH in DCM to give **rac-(1S*,2S*)-2-(5-chloro-2-cyanophenyl)-N-(6-(6-cyclopropylimidazo[1,2-a]pyridine-2-carbonyl)pyrimidin-4-yl)cyclo propane-1-carboxamide intermediate 830** of crude compound (21 mg) which was used in the next step without further purification.

NaBH₄ (1.8 mg, 0.05 mmol) was added portion-wise to a stirred solution of **intermediate 830** (21 mg, 0.04 mmol) in MeOH (2.0 mL) at 0 °C and stirred at 0 °C for 5 min. The reaction was then allowed to warm to room temperature and stirred for 30 min. The reaction was quenched with NH₄Cl (sat. aq., 10 mL) and extracted with EtOAc (3 x 15 mL). The combined organics were dried over MgSO₄ then concentrated in vacuo. The residue was purified by preparative HPLC to give **(I-352)** (5 mg, 23%). ESI-MS (M+H+): 485.3, δ 11.24 (s, 1H), 8.76 (d, J=1.0 Hz, 1H), 8.38 (dd, J=1.8, 1.8 Hz, 1H), 8.33 (dd, J=0.8, 0.8 Hz, 1H), 7.89 (dd, J=1.0, 8.3 Hz, 1H), 7.72 (d, J=2.3 Hz, 1H), 7.55 (ddd, J=0.8, 2.0, 8.3 Hz, 1H), 7.46 (s, 1H), 7.36 (dd, J=2.3, 9.4 Hz, 1H), 6.97 (ddd, J=1.8, 1.8, 9.4 Hz, 1H), 6.16 (dd, J=3.5, 4.8 Hz, 1H), 5.71 (d, J=4.8 Hz, 1H), 2.67 - 2.64 (m, 1H), 1.97 - 1.89 (m, 1H), 1.71 - 1.66 (m, 1H), 1.65 - 1.60 (m, 1H), 0.95 - 0.90 (m, 2H), 0.70 - 0.65 (m, 2H) (one CH of cyclopropyl obscured by DMSO).

### Intermediate 831 rac-(1S*,2S*)-N-(6-chloro-2-(fluoromethyl)pyrimidin-4-yl)-2-(4-methylpyrimidin-2-yl)cyclopropane-1-carboxamide

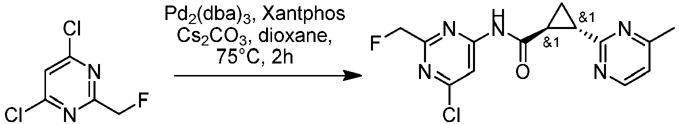

To a solution of 4,6-dichloro-2-(fluoromethyl)pyrimidine (50 mg, 0.28 mmol) in dioxane (3 mL) were added rac-(1S*,2S*)-2-(4-methylpyrimidin-2-yl)cyclopropane-1-carboxamide (50 mg, 0.28 mmol), Pd₂(dba)₃ (26 mg, 0.028 mmol), Xantphos (16 mg, 0.028 mmol), and Cs₂CO₃ (274 mg, 0.84 mmol). Then the mixture was stirred for 2 h at 75 °C under N₂. Water (10 mL) was added and extracted with EtOAc (3 x 10 mL). The organic layers were concentrated to give the crude, which was purified by preparative TLC (DCM/MeOH=20/1) to give **intermediate 831** as a white solid (20 mg, 15%). ESI-MS [M +H]⁺: 322.1.

The compounds in Table **32** were prepared in a similar manner to **intermediate 831** from appropriately substituted cyclopropyl amide and a di-halo-pyrimidine. **Table 32**

| **Compound** | **Name/Analvtical Data** |
|---|---|
| 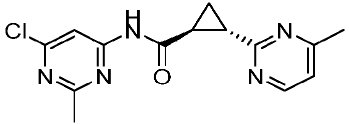 **(1S,2S)-N-(6-chloro-2-methyl pyrimidin-4-yl)-2-(4-methyl pyrimidin-2-yl)cyclopropane-1-carboxamide intermediate 832** | ESI-MS [M +H]+: 304.2. δ 11.57 (s, 1H), 8.54 (d, J = 5.1 Hz, 1H), 7.95 (s, 1H), 7.22 (d, J = 5.1 Hz, 1H), 2.69 - 2.65 (m, 1H), 2.62- 2.57 (m, 1H), 2.51 (s, 3H), 2.42 (s, 3H), 1.62 - 1.54 (m, 2H). |
| 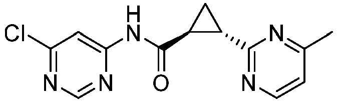 **(1S,2S)-N-(6-chloropyrimidin-4-yl)-2-(4-methyl pyrimidin-2-yl)cyclopropane-1-carboxamide intermediate 833** | ESI-MS [M +H]+: 290.2. δ 11.60 (s, 1H), 8.75 (s, 1H), 8.53 (t, J = 6.9 Hz, 1H), 8.13 (s, 1H), 7.23 (d, J = 5.1 Hz, 1H), 2.72 - 2.68 (m, 1H), 2.63 - 2.57 (m, 1H), 2.42 (s, 3H), 1.63 - 156 (m, 2H). |
| 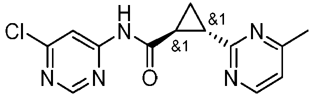 | **Intermediate 834: *rac*-(1S*,2S*)-N-(6-chloropyrimidin-4-yl)-2-(4-methylpyrimidin-2-yl)cyclopropane-1-carboxamide** ESI-MS [M +H]⁺: 290.2. |
| 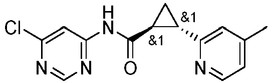 | **Intermediate 835: *rac-*(1S*,2S*)-N-(6-chloropyrimidin-4-yl)-2-(4-methylpyridin-2-yl)cyclopropane-1-carboxamide** ESI-MS [M +H]⁺: 289.2. |
| 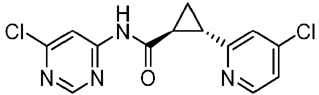 **(1S,2S)-2-(4-chloropyridin-2-yl)-N-(6- chloropyrimidin-4-yl)cyclopropane-1- carboxamide intermediate 836** | ESI-MS [M +H]+: 309.2. δ 11.58 (s, 1H), 8.75 (d, J = 0.9 Hz, 1H), 8.44 (d, J = 5.4 Hz, 1H), 8.13 (d, J = 0.9 Hz, 1H), 7.68 (d, J = 1.8 Hz, 1H), 7.37 (dd, J = 5.4, 2.0 Hz, 1H), 2.72 - 2.63 (m, 2H), 1.63 - 1.53 (m, 2H). |
| 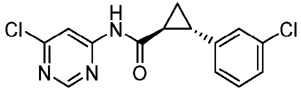 **(1S,2S)-2-(3-chlorophenyl)-N-(6-chloropyrimidin-4-yl)cyclopropane-1-carboxamide intermediate 676** | ESI-MS [M +H]+: 308.2. δ 11.44 (s, 1H), 8.75 (d, J = 0.9 Hz, 1H), 8.14 (d, J = 0.9 Hz, 1H), 7.35- 7.30 (m, 3H), 7.18 (d, J = 7.5 Hz, 1H), 2.50 - 2.47 (m, 1H), 2.43 - 2.38 (m, 1H), 1.57 - 1.51 (m, 2H). |
| 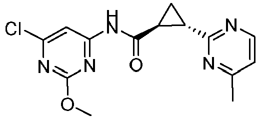 **(1S,2S)-N-(6-chloro-2-methoxypyrimidin-4-yl)-2-(4-methylpyrimidin-2-yl)cyclopropane-1-carboxamide intermediate 837** | ESI-MS [M + H]+: 320.2. δ 11.52 (s, 1H), 8.54 (d, J = 5.1 Hz, 1H), 7.77 (s, 1H), 7.23 (d, J = 5.1 Hz, 1H), 3.89 (s, 3H), 2.71 - 2.67 (m, 1H), 2.60 - 2.56 (m, 1H), 2.42 (s, 3H), 1.62 - 1.55 (m, 2H). |
| 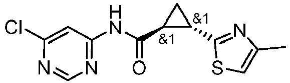 | ***rac*-(1S*,2S*)-N-(6-chloropyrimidin-4-yl)-2-(4-methylthiazol-2-yl)cyclopropane-1-carboxamide** ESI-MS [M +H]+: 295.1. |
| 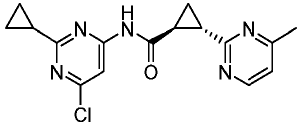 | **Intermediate 838: (1S,2S)-N-(6-chloro-2-cyclopropylpyrimidin-4-yl)-2-(4-methylpyrimidin-2-yl)cyclopropane-1-carboxamide** ESI-MS [M +H]+: 330.3. |
| 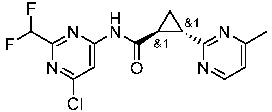 | **Intermediate 839: *rac-*(1S*,2S*)-N-(6-chloro-2-(difluoromethyl)pyrimidin-4-yl)-2-(4-methylpyrimidin-2-yl)cyclopropane-1-carboxamide** ESI-MS [M +H]+: 340.2. |
| 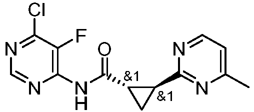 | **Intermediate 840: *rac*-(1S*,2S*)-N-(6-chloro-5-fluoropyrimidin-4-yl)-2-(4-methylpyrimidin-2-yl)cyclopropane-1-carboxamide** ESI-MS [M +H]⁺: 308.2 |
| 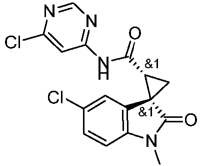 | **Intermediate 841 *rac-*(1R**,*2*S**)-5*'-*chloro-N-(6-chloropyrimidin-4-yl)-1'-methyl-2'-oxospiro[cyclopropane-1,3'-indoline]-2-carboxamide** ESI-MS [M +H]⁺: 363.2 |
| 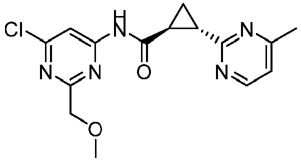 | **Intermediate 842: (1S,2S)-N-(6-chloro-2-(methoxymethyl)pyrimidin-4-yl)-2-(4-methylpyrimidin-2-yl)cyclopropane-1-carboxamide** ESI-MS [M +H]+: 334.2 |

### Example 353: rac-(1S*,2S*)-N-(6-(((6-cyclopropyl-8-(3-methyl-2,4-dioxoimidazolidin-1-yl) imidazo[1,2-a]pyridin-2-yl)methyl)amino)-2-(fluoromethyl)pyrimidin-4-yl)-2-(4-methylpyrimidin-2-yl)cyclopropane-1-carboxamide (I-353)

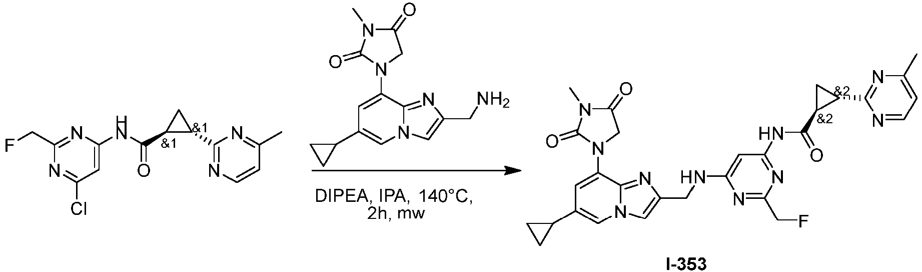

To a solution of **intermediate 365** (19 mg, 0.062 mmol) in IPA (3 mL) was added *rac-*(1S*,2S*)-N-(6-chloro-2-(fluoromethyl)pyrimidin-4-yl)-2-(4-methylpyrimidin-2-yl)cyclopropane-1-carboxamide (20 mg, 0.062 mmol) and DIPEA (24 mg, 0.19 mmol). Then the mixture was stirred in a sealed tube, then after degassing with N₂ for 1 min, the reaction was irradiated in a microwave reactor at 140 °C for 2 h. The mixture was cooled to room temperature, concentrated *in vacuo* and purified by preparative TLC (eluent: DCM/MeOH=20/1) to give **(I-353)** as a white solid (13 mg, 36%). ESI-MS [M +H]+: 585.2, δ 10.89 (s, 1H), 8.53 (d, J = 5.1 Hz, 1H), 8.26 (s, 1H), 8.03 (s, 1H), 7.72 (s, 1H), 7.35 (s, 1H), 7.28 (s, 1H), 7.22 (d, J = 5.1 Hz, 1H), 5.18 (d, J = 46.9 Hz, 2H), 4.93 (s, 2H), 4.62 (s, 2H), 2.99 (s, 3H), 2.68 - 2.64 (m, 2H), 2.42 (s, 3H), 1.96 - 1.92 (m, 1H), 1.55 - 1.49 (m, 2H), 1.01 - 0.93 (m, 2H), 0.68 - 0.64 (m, 2H).

The compounds in Table 33 were prepared in a similar manner to **(I-353)** from a suitable amine and the indicated coupling partner.

**Table 33**

| **EG** | **Structure** | **Coupling Partner / Analytical Data** |
|---|---|---|
| **354** | 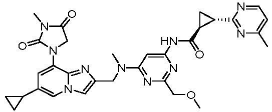 (1S,2S)-N-(6-(((6-cyclopropyl-8-(3-methyl-2,4-dioxoimidazolidin-1-yl)imidazo[1,2-a]pyridin-2-yl) methyl)(methyl)amino)-2-(methoxymethyl)pyrimidin-4-yl)-2-(4-methylpyrimidin-2-yl)cyclo propane-1-carboxamide **(I-354)** | **Intermediate 842** |
| | | ESI-MS (M+H)+: 611.2, δ 10.91 (s, 1H), 8.52 (d, J = 5.1 Hz, 1H), 8.21 (s, 1H), 7.68 (s, 1H), 7.36 (s, 2H), 7.20 (d, J = 5.1 Hz, 1H), 4.92-4.80 (m, 4H), 4.26 (s, 2H), 3.33 (s, 3H), 3.13 (s, 3H), 2.97 (s, 3H), 2.67-2.61 (m, 1H), 2.56-2.50 (m, 1H), 2.41 (s, 3H), 1.98-1.89 (m, 1H), 1.56-1.48 (m, 2H), 0.97-0.90 (m, 2H), 0.67-0.60 (m, 2H). |
| **355** | 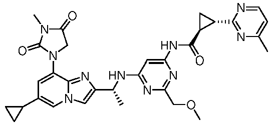 (1S,2S)-N-(6-(((R)-1-(6-cyclopropyl-8-(3-methyl-2,4-dioxoimidazolidin-1-yl)imidazo[1,2-a]pyridin-2-yl) ethyl)amino)-2-(methoxymethyl) pyrimidin-4-yl)-2-(4-methyl pyrimidin-2-yl)cyclopropane-1-carboxamide **(I-355)** | **Intermediate 842** |
| | | ESI-MS (M+H)+: 611.2, δ 10.78 (s, 1H), 8.52 (d, J = 4.9 Hz, 1H), 8.23 (s, 1H), 7.78 (d, J = 7.8 Hz, 1H), 7.67 (s, 1H), 7.34 (s, 1H), 7.24-7.16 (m, 2H), 5.42 (s, 1H), 4.94 (s, 2H), 4.20 (s, 2H), 3.30 (s, 3H), 2.97 (s, 3H), 2.68-2.60 (m, 2H), 2.41 (s, 3H), 1.93 (s, 1H), 1.55-1.40 (m, 5H), 0.97-0.90 (m, 2H), 0.67-0.60 (m, 2H). |
| **356** | 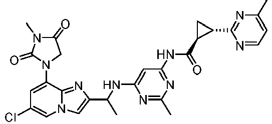 (1S,2S)-N-(6-((1-(6-chloro-8-(3-methyl-2,4-dioxoimidazolidin-1-yl)imidazo[1,2-a]pyridin-2-yl)ethyl)amino)-2-methyl pyrimidin-4-yl)-2-(4-methylpyrimidin-2-yl)cyclopropane-1-carboxamide **(I-356)** | **Intermediate 832** |
| | | ESI-MS (M+H)+: 575.2, δ 10.66 (s, 1H), 8.67 (s, 1H), 8.52 (d, J = 5.1 Hz, 1H), 7.79 (s, 1H), 7.71 - 7.67 (m, 2H), 7.20 (d, J = 5.1 Hz, 1H), 7.14 (s, 1H), 5.65 - 5.09 (m, 1H), 5.03 (s, 2H), 2.98 (s, 3H), 2.61 - 2.56 (m, 2H), 2.41 (s, 3H), 2.25 (s, 3H), 1.53 - 1.46 (m, 5H). |
| **357** | 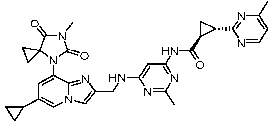 (1S,2S)-N-(6-(((6-cyclopropyl-8-(6-methyl-5,7-dioxo-4,6-diazaspiro[2.4]heptan-4-yl)imidazo[1,2-a]pyridin-2-yl)methyl) amino)-2-methylpyrimidin-4-yl)-2-(4-methylpyrimidin-2-yl)cyclopropane-1-carboxamide **(I-357)** | **Intermediate 832** ESI-MS (M+H)+: 593.3, δ 10.67 (s, 1H), 8.52 (d, *J =* 5.1 Hz, 1H), 8.41 (d, *J =* 1.3 Hz, 1H), 7.76 (s, 1H), 7.68 (s, 1H), 7.20 (d, *J =* 5.1 Hz, 1H), 7.15 - 7.02 (m, 2H), 4.63 - 4.44 (m, 2H), 3.06 (s, 3H), 2.63 - 2.52 (m, 2H), 2.41 (s, 3H), 2.26 (s, 3H), 1.96 - 1.89 (m, 1H), 1.54 - 1.44 (m, 2H), 1.32 - 1.24 (m, 2H), 1.19 - 1.10 (m, 2H), 0.97 - 0.89 (m, 2H), 0.73 - 0.65 (m, 2H). |
| **358** | 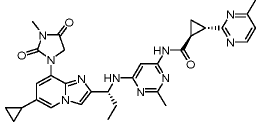 (1S,2S)-N-(6-(((R)-1-(6-cyclopropyl-8-(3-methyl-2,4-dioxoimidazolidin-1-yl)imidazo[1,2-a]pyridin-2-yl) propyl)amino)-2-methylpyrimidin-4-yl)-2-(4-methylpyrimidin-2-yl)cyclo propane-1-carboxamide **(I-358)** | **Intermediate 832** ESI-MS (M+H)+: 595.2, δ 10.64 (s, 1H), 8.52 (d, J = 5.1 Hz, 1H), 8.23 (d, J = 1.1 Hz, 1H), 7.65 -7.60 (m, 2H), 7.34 (d, J = 1.3 Hz, 1H), 7.20 - 7.17 (m, 2H), 5.30 - 5.24 (m, 1H), 4.96 - 4.95 (m, 2H), 2.98 (s, 3H), 2.59 - 2.57 (m, 1H), 2.54 - 2.52 (m, 1H), 2.41 (s, 3H), 2.24 (s, 3H), 2.02 - 1.91 (m, 2H), 1.83 - 1.77 (m, 1H), 1.52 - 1.47 (m, 2H), 0.95 - 0.89 (m, 5H), 0.65 - 0.62 (m, 2H). |
| **359** | 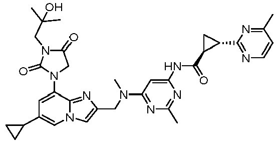 (1S,2S)-N-(6-(((6-cyclopropyl-8-(3-(2-hydroxy-2-methylpropyl)-2,4-dioxo imidazolidin-1-yl)imidazo[1,2-a]pyridin-2-yl)methyl)(methyl)amino)-2-methyl pyrimidin-4-yl)-2-(4-methylpyrimidin-2-yl)cyclo propane-1-carboxamide (I-359) | **Intermediate 832** ESI-MS (M+H)+: 639.3, δ 10.75 (s, 1H), 8.49 (d, J = 5.1 Hz, 1H), 8.17 (s, 1H), 7.63 (s, 1H), 7.35 (d, J = 1.4 Hz, 1H), 7.26 (s, 1H), 7.17 (d, J = 5.1 Hz, 1H), 4.90 (s, 2H), 4.78 (s, 2H), 4.56 (s, 1H), 3.41 (s, 2H), 3.08 (s, 3H), 2.60 - 2.55 (m, 1H), 2.52 - 2.49 (m, 1H), 2.38 (s, 3H), 2.27 (s, 3H), 1.94 - 1.88 (m, 1H), 1.51 - 1.44 (m, 2H), 1.10 (s, 6H), 0.92 - 0.88 (m, 2H), 0.62 - 0.58 (m, 2H) |
| **360** | 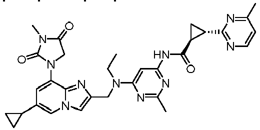 (1S,2S)-N-(6-(((6-cyclopropyl-8-(3-methyl-2,4-dioxoimidazolidin-1-yl) imidazo[1,2-a]pyridin-2-yl)methyl) (ethyl)amino)-2-methyl pyrimidin-4-yl)-2-(4-methyl pyrimidin-2-yl)cyclo propane-1-carboxamide (I-360) | **Intermediate 832** ESI-MS (M+H)+: 595.2, δ = 10.74 (s, 1H), 8.51 (d, J=5.1, 1H), 8.22 (d, J=1.1, 1H), 7.68 (s, 1H), 7.36 (d, J=1.4, 1H), 7.29 (s, 1H), 7.20 (d, J=5.1, 1H), 4.91 (s, 2H), 4.73 (s, 2H), 3.59 (s, 2H), 2.97 (s, 3H), 2.61-2.56 (m, 1H), 2.54-2.51 (m, 1H), 2.41 (s, 3H), 2.30 (s, 3H), 1.97-1.90 (m, 1H), 1.55-1.45 (m, 2H), 1.16-1.12 (m, 3H), 0.98-0.89 (m, 2H), 0.68 - 0.59 (m, 2H). |
| **361** | 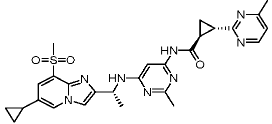 (1S,2S)-N-(6-(((R)-1-(6-cyclopropyl-8-(methylsulfonyl)imidazo[1,2-a]pyridin-2-yl)ethyl)amino)-2-methyl pyrimidin-4-yl)-2-(4-methyl pyrimidin-2-yl)cyclo propane-1-carboxamide **(I-361)** | **Intermediate 832** ESI-MS (M+H)+: 547.2, δ 10.67 (s, 1H), 8.64 (s, 1H), 8.52 (d, J = 5.1 Hz, 1H), 7.84 - 7.67 (m, 2H), 7.56 (s, 1H), 7.20 (d, J = 5.1 Hz, 1H), 7.14 (s, 1H), 5.47 (s, 1H), 3.55 (s, 3H), 2.64 - 2.56 (m, 2H), 2.41 (s, 3H), 2.24 (s, 3H), 2.10 - 2.03 (m, 1H), 1.54 - 1.44 (m, 5H), 1.04 - 0.90 (m, 2H), 0.72 - 0.69 (m, 2H). |
| **362** | 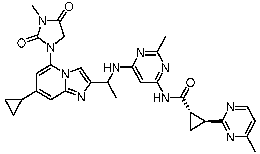 (1S,25)-N-(6-((1-(7-cyclopropyl-5-(3-methyl-2,4-dioxoimidazolidin-1-yl) imidazo[1,2-a]pyridin-2-yl)ethyl) amino)-2-methylpyrimidin-4-yl)-2-(4-methyl pyrimidin-2-yl)cyclo propane-1-carboxamide **(I-362)** | **Intermediate 832** ESI-MS (M+H)+: 581.2, ¹H NMR (400 MHz, cd3od) δ 8.47 - 8.45 (m, 1H), 7.68 (s, 1H), 7.25 (s, 1H), 7.16 - 7.15 (m, 1H), 7.10 (d, J = 4.9 Hz, 1H), 6.79 - 6.78 (m, 1H), 4.60 (s, 2H), 4.53 - 4.49 (m, 1H), 3.09 (s, 3H), 2.70 - 2.65 (m, 1H), 2.51 - 2.46 (m, 4H), 2.30 (s, 3H), 2.05 - 2.02 (m, 1H), 1.66 - 1.60 (m, 5H), 1.09 - 1.07 (m, 2H), 0.84 - 0.82 (m, 2H). |
| **363** | 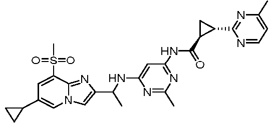 (1S,2S)-N-(6-((1-(6-cyclopropyl-8-(methylsulfonyl)imidazo[1,2-a]pyridin-2-yl)ethyl)amino)-2-methylpyrimidin-4-yl)-2-(4-methyl pyrimidin-2-yl)cyclopropane-1-carboxamide **(I-363)** | **Intermediate 832** ESI-MS (M+H)+: 547.2, δ 10.67 (s, 1H), 8.64 (s, 1H), 8.52 (d, J = 5.1 Hz, 1H), 8.39 (s, 1H), 7.79 - 7.73 (m, 2H), 7.56 (s, 1H), 7.20 (d, J = 5.1 Hz, 1H), 7.14 (s, 1H), 5.54 - 5.46 (m, 1H), 3.55 (s, 3H), 2.58 - 2.56 (m, 2H), 2.41 (s, 3H), 2.24 (s, 3H), 2.13 - 2.01 (m, 1H), 1.51 (m, 5H), 0.98 - 0.96 (m, 2H), 0.71 - 0.70 (m, 2H). |
| **364** | 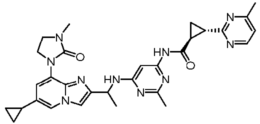 (1S,2S)-N-(6-((1-(6-cyclopropyl-8-(3-methyl-2-oxoimidazolidin-1-yl)imidazo [1,2-a]pyridin-2-yl)ethyl)amino)-2-methylpyrimidin-4-yl)-2-(4-methyl pyrimidin-2-yl)cyclopropane-1-carboxamide **(I-364)** | **Intermediate 832** ESI-MS (M+H)+: 567.2, δ 10.65 (s, 1H), 8.52 (d, *J =* 5.1 Hz, 1H), 8.11 - 8.07 (m, 1H), 7.65 - 7.57 (m, 2H), 7.24 - 7.18 (m, 2H), 7.13 (s, 1H), 5.55 - 5.30 (m, 1H), 4.34 - 4.28 (m, 2H), 3.50 - 3.44 (m, 2H), 2.79 (s, 3H), 2.63 - 2.53 (m, 2H), 2.41 (s, 3H), 2.24 (s, 3H), 1.91 - 1.86 (m, 1H), 1.57 - 1.42 (m, 5H), 0.93 - 0.87 (m, 2H), 0.64 - 0.57 (m, 2H). |
| **365** | 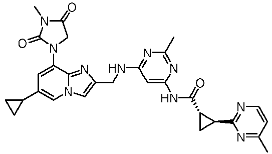 (1S,2S)-N-(6-(((6-cyclopropyl-8-(3-methyl-2,4-dioxoimidazolidin-1-yl) imidazo[1,2-a]pyridin-2-yl)methyl) amino)-2-methylpyrimidin-4-yl)-2-(4-methylpyrimidin-2-yl)cyclopropane-1-carboxamide **(I-365)** | **Intermediate 832** ESI-MS (M+H)+: 567.2, δ 10.65 (s, 1H), 8.51 (d, J = 5.1 Hz, 1H), 8.25 (d, J = 0.7 Hz, 1H), 7.71 (s, 1H), 7.69 (s, 1H), 7.33 (d, J = 1.0 Hz, 1H), 7.20 (d, J = 5.1 Hz, 1H), 7.13 (s, 1H), 4.91 (s, 2H), 4.56 (s, 2H), 2.98 (s, 3H), 2.62-2.57 (m, 1H), 2.55-2.50 (m, 1H),2.41 (s, 3H), 2.27 (s, 3H), 1.97-1.90 (m, 1H), 1.54 - 1.46 (m, 2H), 0.96- 0.91 (m, 2H), 0.66- 0.62 (m, 2H). |
| **366** | 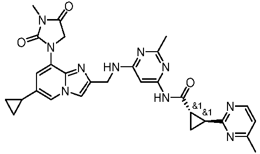 *rac*-(1S*,2S*)-N-(6-(((6-cyclopropyl-8-(3-methyl-2,4-dioxoimidazolidin-1-yl)imidazo[1,2-a]pyridin-2-yl)methyl) amino)-2-methylpyrimidin-4-yl)-2-(4-methylpyrimidin-2-yl)cyclopropane-1-carboxamide **(I-366)** | **Intermediate 832** ESI-MS (M+H)+: 567.2, δ 10.67 (s, 1H), 8.52 (d, J = 5.1 Hz, 1H), 8.25 (d, J = 1.0 Hz, 1H), 7.73 (s, 1H), 7.69 (s, 1H), 7.33 (d, J = 1.2 Hz, 1H), 7.20 (d, J = 5.1 Hz, 1H), 7.14 (s, 1H), 4.92 (s, 2H), 4.57 (s, 2H), 2.98 (s, 3H), 2.61 - 2.57 (m, 1H), 2.54 - 2.51 (m, 1H), 2.41 (s, 3H), 2.27 (s, 3H), 1.97 - 1.90 (m, 1H), 1.54 - 1.45 (m, 2H), 0.96 - 0.91 (m, 2H), 0.66 - 0.62 (m, 2H). |
| **367** | 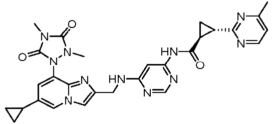 (1S,2S)-N-(6-(((6-cyclopropyl-8-(2,4-dimethyl-3,5-dioxo-1,2,4-triazolidin-1-yl)imidazo[1,2-a]pyridin-2-yl)methyl) amino)pyrimidin-4-yl)-2-(4-methyl pyrimidin-2-yl)cyclopropane-1-carboxamide **(I-367)** | **Intermediate 833** ESI-MS (M+H)+: 568.2, δ 10.69 (s, 1H), 8.52 (d, J = 5.1 Hz, 1H), 8.45 (s, 1H), 8.18 (s, 1H), 7.94 (s, 1H), 7.70 (s, 1H), 7.28 (s, 1H), 7.27 - 7.16 (m, 2H), 4.55 (s, 2H), 3.04 (s, 3H), 2.95 (s, 3H), 2.66 - 2.56 (m, 2H), 2.41 (s, 3H), 2.02 - 1.92 (m, 1H), 1.56 - 1.49 (m, 2H), 0.99 - 0.89 (m, 2H), 0.75 - 0.67 (m, 2H). |
| **368** | 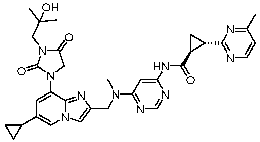 (1S,2S)-N-(6-(((6-cyclopropyl-8-(3-(2-hydroxy-2-methylpropyl)-2,4-dioxo imidazolidin-1-yl)imidazo[1,2-a]pyridin-2-yl)methyl)(methyl) amino)pyrimidin-4-yl)-2-(4-methypyrimidin-2-yl)cyclo propane-1-carboxamide **(I-368)** | **Intermediate 833** ESI-MS (M+H)+: 625.3, δ 10.75 (s, 1H), 8.50 (d, J = 5.1 Hz, 1H), 8.24 (d, J = 0.8 Hz, 1H), 8.18 (s, 1H), 7.65 (s, 1H), 7.43 (s, 1H), 7.36 (d, J = 1.4 Hz, 1H), 7.18 (d, J = 5.1 Hz, 1H), 4.91 (s, 2H), 4.80 (s, 2H), 4.55 (s, 1H), 3.42 (s, 2H), 3.11 (s, 3H), 2.64 - 2.59 (m, 1H), 2.53 - 2.52 (m, 1H), 2.39 (s, 3H), 1.94 - 1.89 (m, 1H), 1.53 - 1.48 (m, 2H), 1.11 (s, 6H), 0.93 - 0.89 (m, 2H), 0.63 - 0.59 (m, 2H). |
| **369** | 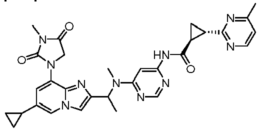 (1S,2S)-N-(6-((1-(6-cyclopropyl-8-(3-methyl-2,4-dioxoimidazolidin-1-yl)imidazo[1,2-a]pyridin-2-yl)ethyl) (methyl)amino)pyrimidin-4-yl)-2-(4-methylpyrimidin-2-yl)cyclopropane-1-carboxamide **(I-369)** | **Intermediate 833** ESI-MS (M+H)+: 581.3, δ 10.80 (s, 1H), 8.52 (d, J = 5.1 Hz, 1H), 8.28 (d, J = 0.7 Hz, 1H), 8.20 (s, 1H), 7.76 (s, 1H), 7.35 (s, 2H), 7.20 (d, J = 5.1 Hz, 1H), 4.87 (s, 2H), 3.88 (d, J = 0.9 Hz, 1H), 2.95 (d, J = 1.2 Hz, 3H), 2.80 (s, 3H), 2.67 - 2.62 (m, 1H), 2.56 - 2.53 (m, 1H), 2.41 (s, 3H), 1.98 - 1.91 (m, 1H), 1.55 - 1.49 (m, 5H), 0.96 - 0.91 (m, 2H), 0.65 - 0.61 (m, 2H). |
| **370** | 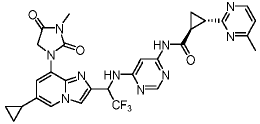 (1S,2S)-N-(6-((1-(6-cyclopropyl-8-(3-methyl-2,4-dioxoimidazolidin-1-yl) imidazo[1,2-a]pyridin-2-yl)-2,2,2-tri fluoroethyl)amino)pyrimidin-4-yl)-2-(4-methylpyrimidin-2-yl)cyclopropane-1-carboxamide **(I-370)** | **Intermediate 833** ESI-MS (M+H)+: 621.2, δ 10.82 (s, 1H), 8.54 - 8.46 (m, 2H), 8.33 - 8.30 (m, 2H), 8.06 (s, 1H), 7.54 (s, 1H), 7.44 (s, 1H), 7.22 - 7.20 (m, 1H), 6.38 - 6.34 (m, 1H), 4.98 (s, 2H), 2.98 (s, 3H), 2.65 - 2.63 (m, 1H), 2.59 - 2.55 (m, 1H), 2.42 - 2.41(m, 3H), 1.99 - 1.95 (m, 1H), 1.57 - 1.51 (m, 2H), 0.99 - 0.94 (m, 2H), 0.69 - 0.65 (m, 2H). |
| **371** | 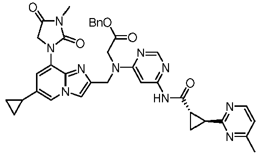 **Benzyl-N-((6-cyclopropyl-8-(3-methyl-**2,4-dioxoimidazolidin-1-yl)imidazo[1,2-a]pyridin-2-yl) methyl)-N-(6-((1S,2S)-2-(4-methyl pyrimidin-2-yl) cyclopropane-1-carboxamido)pyrimidin-4-yl) glycinate **(I-371)** | **Intermediate 833** ESI-MS (M+H)+: 701.2, δ 10.82 (s, 1H), 8.52 (d, J = 4.7 Hz, 1H), 8.31 - 8.17 (m, 2H), 7.92 - 7.66 (m, 2H), 7.43 - 7.28 (m, 6H), 7.20 (d, J = 4.7 Hz, 1H), 5.14 (s, 2H), 4.93 (s, 2H), 4.78 - 4.45 (m, 4H), 2.97 (s, 3H), 2.66 - 2.58 (m, 1H), 2.57 - 2.50 (s, 1H), 2.41 (s, 3H), 2.00 - 1.85 (m, 1H), 1.60 - 1.44 (m, 2H), 1.02 - 0.87 (m, 2H), 0.73 - 0.59 (m, 2H). |
| **372** | 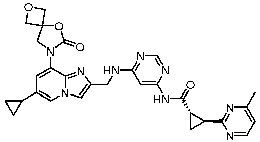 (1S,2S)-N-(6-(((6-cyclopropyl-8-(6-oxo-2,5-dioxa-7-azaspiro[3.4]octan-7-yl)imidazo[1,2-a]pyridin-2-yl)methyl) amino)pyrimidin-4-yl)-2-(4-methyl pyrimidin-2-yl)cyclopropane-1-carboxamide **(I-372)** | **Intermediate 833** ESI-MS (M+H)+: 568.2, δ 10.69 (s, 1H), 8.52 (d, J = 5.1 Hz, 1H), 8.25 (s, 1H), 8.19 (s, 1H), 7.90 (s, 1H), 7.69 (s, 1H), 7.31 (s, 1H), 7.26 - 7.18 (m, 2H), 4.87-4.78 (m, 4H), 4.72 (s, 2H), 4.59 (s, 2H), 2.65 - 2.59 (m, 1H), 2.56-2.51 (m, 1H), 2.41 (s, 3H), 1.94 - 1.90 (m, 1H), 1.57 - 1.47 (m, 2H), 0.96 - 0.89 (m, 2H), 0.67 - 0.59 (m, 2H). |
| **373** | 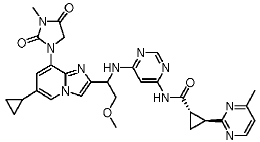 (1S,2S)-N-(6-((1-(6-cyclopropyl-8-(3-methyl-2,4-dioxoimidazolidin-1-yl)imidazo[1,2-a]pyridin-2-yl)-2-methoxyethyl)amino)pyrimidin-4-yl)-2-(4-methylpyrimidin-2-yl)cyclo propane-1-carboxamide **(I-373)** | **Intermediate 833** ESI-MS (M+H)+: 597.2, δ 10.66 (s, 1H), 8.52 (d, J = 5.1 Hz, 1H), 8.24 (s, 1H), 8.16 (s, 1H), 7.86 (s, 1H), 7.71 (d, J = 2.0 Hz, 1H), 7.35 (s, 2H), 7.21 (d, J = 5.1 Hz, 1H), 5.51 (s, 1H), 4.95 (s, 2H), 3.79 - 3.73 (m, 1H), 3.71 - 3.65 (m, 1H), 3.28 (s, 3H), 2.98 (s, 3H), 2.64 - 2.60 (m, 1H), 2.57 - 2.53 (m, 1H), 2.41 (s, 3H), 1.97 - 1.90 (m, 1H), 1.57 - 1.48 (m, 2H), 0.96 - 0.90 (m, 2H), 0.66 - 0.61 (m, 2H). |
| **374** | 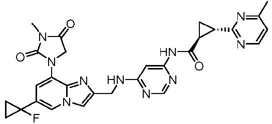 (1S,2S)-N-(6-(((6-(1-fluorocyclo propyl)-8-(3-methyl-2,4-dioxoimidazolidin-1-yl)imidazo[1,2-a]pyridin-2-yl)methyl) amino)pyrimidin-4-yl)-2-(4-methyl pyrimidin-2-yl)cyclopropane-1-carboxamide **(I-374)** | **Intermediate 833** ESI-MS (M+H)+: 571.2, δ 10.69 (s, 1H), 8.60 - 8.46 (m, 2H), 8.19 (s, 1H), 7.94 (s, 1H), 7.79 (s, 1H), 7.59 (s, 1H), 7.32 (s, 1H), 7.21 (d, J = 5.1 Hz, 1H), 4.95 (s, 2H), 4.61 (s, 2H), 2.98 (s, 3H), 2.69 - 2.58 (m, 2H), 2.41 (s, 3H), 1.61 - 1.39 (m, 4H), 1.16-1.08 (m, 2H). |
| **375** | 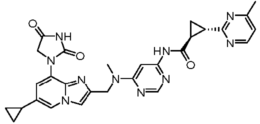 (1S,2S)-N-(6-(((6-cyclopropyl-8-(2,4-dioxoimidazolidin-1-yl)imidazo[1,2-a]pyridin-2-yl)methyl)(methyl)amino) pyrimidin-4-yl)-2-(4-methylpyrimidin-2-yl)cyclopropane-1-carboxamide **(I-375)** | **Intermediate 833** ESI-MS (M+H)+: 553.3, δ 11.32 (s, 1H), 10.77 (s, 1H), 8.52 (d, J = 5.1 Hz, 1H), 8.26 (s, 1H), 8.18 (s, 1H), 7.65 (s, 1H), 7.45 (s, 1H), 7.35 (d, J = 1.1 Hz, 1H), 7.20 (d, J = 5.1 Hz, 1H), 4.84 (d, J = 19.7 Hz, 4H), 3.13 (s, 3H), 2.64 (s, 1H), 2.57 - 2.53 (m, 1H), 2.41 (s, 3H), 1.95-1.89 (m, 1H), 1.54-1.51 (m, 2H), 0.95-0.90 (m, 2H), 0.67 - 0.58 (m, 2H). |
| **376** | 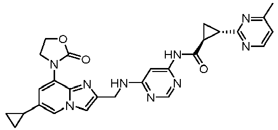 (1S,2S)-N-(6-(((6-cyclopropyl-8-(2-oxooxazolidin-3-yl)imidazo[1,2-a] pyridin-2-yl)methyl)amino)pyrimidin-4-yl)-2-(4-methylpyrimidin-2-yl)cyclo propane-1-carboxamide **(I-376)** | **Intermediate 833** ESI-MS (M+H)+: 526.2, δ 10.65 (s, 1H), 8.50 (d, J = 5.1 Hz, 1H), 8.22 (s, 1H), 8.16 (s, 1H), 7.86 (s, 1H), 7.65 (s, 1H), 7.27 (s, 1H), 7.18 (d, J = 5.4 Hz, 2H), 4.55 - 4.42 (m, 6H), 2.62 - 2.57 (m, 1H), 2.54 - 2.52 (m, 1H), 2.39 (s, 3H), 1.93 - 1.87 (m, 1H), 1.53 - 1.45 (m, 2H), 0.92 - 0.87 (m, 2H), 0.64 - 0.60 (m, 2H). |
| **377** | 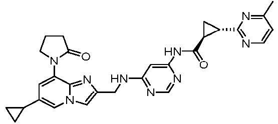 (1S,2S)-N-(6-(((6-cyclopropyl-8-(2-oxopyrrolidin-1-yl)imidazo[1,2-a]pyridin-2-yl)methyl)amino) pyrimidin-4-yl)-2-(4-methyl pyrimidin-2-yl)cyclopropane-1-carboxamide **(I-377)** | **Intermediate 833** ESI-MS (M+H)+: 524.1, δ 10.64 (s, 1H), 8.50 (d, J = 5.1 Hz, 1H), 8.21 (d, J = 1.2 Hz, 1H), 8.16 (s, 1H), 7.85 (s, 1H), 7.63 (s, 1H), 7.27 (s, 1H), 7.18 (d, J = 5.1 Hz, 1H), 7.09 (d, J = 1.2 Hz, 1H), 4.54 (s, 1H), 4.12 (t, J = 7.1 Hz, 2H), 2.62 - 2.58 (m, 1H), 2.53 - 2.51 (m, 1H), 2.45 (s, 1H), 2.43 (s, 1H), 2.39 (s, 3H), 2.13 - 2.06 (m, 2H), 1.92 - 1.86 (m, 1H), 1.54 - 1.45 (m, 2H), 0.92 - 0.87 (m, 2H), 0.63 - 0.59 (m, 2H). |
| **378** | 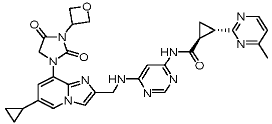 (1S,2S)-N-(6-(((6-cyclopropyl-8-(3-(oxetan-3-yl)-2,4-dioxoimidazolidin-1-yl)imidazo[1,2-a]pyridin-2-yl)methyl) amino)pyrimidin-4-yl)-2-(4-methyl pyrimidin-2-yl)cyclopropane-1-carboxamide **(I-378)** | **Intermediate 833** ESI-MS (M+H)+: 595.3, δ 10.65 (s, 1H), 8.50 (d, J = 5.1 Hz, 1H), 8.23 (d, J = 1.1 Hz, 1H), 8.16 (s, 1H), 7.86 (s, 1H), 7.67 (s, 1H), 7.30 (d, J = 9.0 Hz, 2H), 7.19 (d, J = 5.1 Hz, 1H), 5.19 - 5.13 (m, 1H), 5.11 - 5.08 (m, 2H), 4.89 (s, 2H), 4.71 - 4.67 (m, 2H), 4.55 (s, 2H), 2.61 - 2.57 (m, 1H), 2.54 - 2.53 (m, 1H), 2.39 (s, 3H), 1.95 - 1.88 (m, 1H), 1.53 - 1.46 (m, 2H), 0.94 - 0.89 (m, 2H), 0.64 - 0.60 (m, 2H). |
| **379** | 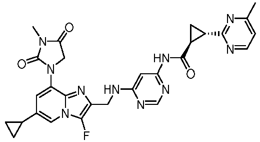 (1S,2S)-N-(6-(((6-cyclopropyl-3-fluoro-8-(3-methyl-2,4-dioxoimidazolidin-1-yl)imidazo[1,2-a]pyridin-2-yl)methyl) amino)pyrimidin-4-yl)-2-(4-methyl pyrimidin-2-yl)cyclopropane-1-carboxamide **(I-379)** | **Intermediate 833** ESI-MS (M+H)+: 571.2, δ 10.66 (s, 1H), 8.52 (d, J = 5.1 Hz, 1H), 8.20 - 8.19 (m, 1H), 7.96 (d, J = 1.2 Hz, 1H), 7.89 (s, 1H), 7.34 (d, J = 1.3 Hz, 1H), 7.30 (s, 1H), 7.20 (d, J = 5.1 Hz, 1H), 4.90 (s, J = 8.7 Hz, 2H), 4.58 (s, 2H), 2.97 (s, 3H), 2.68 - 2.54 (m, 2H), 2.41 (s, 3H), 2.04 - 1.98 (m, 1H), 1.55 - 1.47 (m, 2H), 0.98 - 0.93 (m, 2H), 0.73 - 0.69 (m, 2H). |
| **380** | 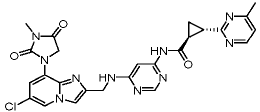 (1S,2S)-N-(6-(((6-chloro-8-(3-methyl-2,4-dioxoimidazolidin-1-yl)imidazo[1,2-a]pyridin-2-yl)methyl)amino)pyrimidin-4-yl)-2-(4-methylpyrimidin-2-yl)cyclo propane-1-carboxamide **(I-380)** | **Intermediate 833** |
| | | ESI-MS (M+H)+: 547.2, ¹H NMR (400 MHz, cd3od) δ 8.49 - 8.44 (m, 2H), 8.17 (d, J = 4.8 Hz, 1H), 7.78 (s, 1H), 7.68 (d, J = 1.6 Hz, 1H), 7.39 (s, 1H), 7.17 (d, J = 5.2 Hz, 1H), 4.87 (s, 2H), 4.67 (s, 2H), 3.10 (s, 3H), 2.72 - 2.65 (m, 1H), 2.64 - 2.56 (m, 1H), 2.47 (s, 3H), 1.70 - 1.62 (m, 2H). |
| **381** | 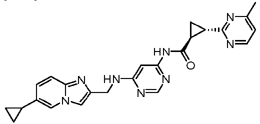 (1S,25)-N-(6-(((6-cyclopropyl imidazo[1,2-a]pyridin-2-yl)methyl) amino)pyrimidin-4-yl)-2-(4-methyl pyrimidin-2-yl)cyclopropane-1-carboxamide **(I-381)** | **Intermediate 833** ESI-MS (M+H)+: 441.1, ¹H NMR (400 MHz, MeOD) δ 8.47 (d, J = 5.2 Hz, 2H), 8.31 (s, 1H), 7.90 (s, 1H), 7.82 (s, 1H), 7.42 (s, 1H), 7.29 (d, J = 10.0 Hz, 1H), 7.17 (d, J = 5.2 Hz, 1H), 4.75 (s, 2H), 2.72 - 2.67 (m, 1H), 2.57 - 2.52 (m, 1H), 2.47 (s, 3H), 2.03 - 1.95 (m, 1H), 1.71 - 1.62 (m, 2H), 1.05 - 0.97 (m, 2H), 0.80 - 0.71 (m, 2H). |
| **382** | 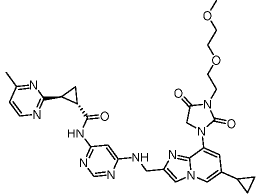 (1S,2S)-N-(6-(((6-cyclopropyl-8-(3-(2-(2-methoxyethoxy)ethyl)-2,4-dioxo imidazolidin-1-yl)imidazo[1,2-a]pyridin-2-yl)methyl)amino)pyrimidin-4-yl)-2-(4-methylpyrimidin-2-yl)cyclopropane-1-carboxamide **(I-382)** | **Intermediate 833** ESI-MS (M+H)+: 641.3, δ 10.69 (s, 1H), 8.52 (d, J = 5.1 Hz, 1H), 8.42 (s, 1H), 8.25 (d, J = 1.1 Hz, 1H), 8.19 (s, 1H), 7.91 (s, 1H), 7.69 (s, 1H), 7.31 (s, 2H), 7.21 (d, J = 5.2 Hz, 1H), 4.95 (s, 2H), 4.58 (s, 2H), 3.60 - 3.60 (m, 4H), 3.56 - 3.54 (m, 2H), 3.42 - 3.41 (m, 2H), 3.23 (s, 3H), 2.64 - 2.60 (m, 1H), 2.55 - 2.53 (m, 1H), 2.41 (s, 3H), 1.97 - 1.91 (m, 1H), 1.56 - 1.48 (m, 2H), 0.96 - 0.92 (m, 2H), 0.66 - 0.62 (m, 2H). |
| **383** | 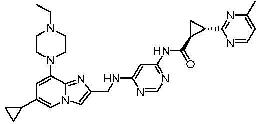 (1S,2S)-N-(6-(((6-cyclopropyl-8-(4-ethyl piperazin-1-yl)imidazo[1,2-a]pyridin-2-yl)methyl)amino)pyrimidin-4-yl)-2-(4-methylpyrimidin-2-yl)cyclopropane-1-carboxamide **(I-383)** | **Intermediate 833** ESI-MS (M+H)+: 553.3, δ 10.68 (s, 1H), 8.52 (d, J = 5.1 Hz, 1H), 8.20 - 8.16 (m, 1H), 7.89 (s, 2H), 7.54 (s, 1H), 7.27 (s, 1H), 7.21 (d, J = 5.1 Hz, 1H), 6.14 (s, 1H), 4.54 (s, 2H), 3.48 (s, 4H), 2.67 - 2.60 (m, 2H), 2.55 - 2.53 (m, 4H), 2.41 (s, 3H), 2.39 - 2.33 (m, 2H), 1.88 - 1.81 (m, 1H), 1.53 - 1.48 (m, 2H), 1.04 (t, J = 7.1 Hz, 3H), 0.88 - 0.84 (m, 2H), 0.67 - 0.58 (m, 2H). |
| **384** | 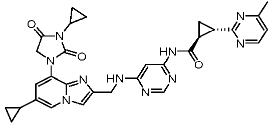 (1S,2S)-N-(6-(((6-cyclopropyl-8-(3-cyclo propyl-2,4-dioxoimidazolidin-1-yl) imidazo[1,2-a]pyridin-2-yl)methyl) amino)pyrimidin-4-yl)-2-(4-methyl pyrimidin-2-yl)cyclopropane-1-carboxamide **(I-384)** | **Intermediate 833** ESI-MS (M+H)+: 579.2, δ 10.69 (s, 1H), 8.52 (d, *J =* 5.1 Hz, 1H), 8.22 (d, *J* = 23.2 Hz, 2H), 7.90 (s, 1H), 7.69 (s, 1H), 7.31 (d, *J =* 9.4 Hz, 2H), 7.21 (d, *J* = 5.1 Hz, 1H), 4.82 (s, 2H), 4.58 (s, 2H), 2.65 - 2.59 (m, 2H), 2.55 - 2.53 (m, 1H), 2.41 (s, 3H), 1.97 - 1.90 (m, 1H), 1.56 - 1.48 (m, 2H), 0.96 - 0.85 (m, 6H), 0.66 - 0.62 (m, 2H). |
| **385** | 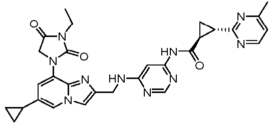 (1S,25)-N-(6-(((6-cyclopropyl-8-(3-ethyl-2,4-dioxoimidazolidin-1-yl)imidazo[1,2-a]pyridin-2-yl) methyl)amino)pyrimidin-4-yl)-2-(4-methylpyrimidin-2-yl)cyclo propane-1-carboxamide **(I-385)** | **Intermediate 833** ESI-MS (M+H)+: 567.3, δ 10.70 (s, 1H), 8.55 (d, J = 5.1 Hz, 1H), 8.27 (s, 1H), 8.21 (s, 1H), 7.90 (s, 1H), 7.71 (s, 1H), 7.35 (d, J = 13.9 Hz, 2H), 7.23 (d, J = 5.1 Hz, 1H), 4.95 (s, 2H), 4.60 (s, 2H), 3.58 - 3.53 (m, 2H), 2.70 - 2.57 (m, 2H), 2.44 (s, 3H), 2.00 - 1.93 (m, 1H), 1.56 - 1.51 (m, 2H), 1.20 (t, J = 7.2 Hz, 3H), 0.99 - 0.94 (m, 2H), 0.69 - 0.65 (m, 2H). |
| **386** | 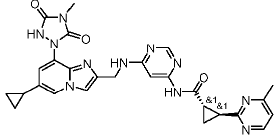 *rac*-(1S*,2S*)-N-(6-(((6-cyclopropyl-8-(4-methyl-3,5-dioxo-1,2,4-triazolidin-1-yl)imidazo[1,2-a]pyridin-2-yl)methyl) amino)pyrimidin-4-yl)-2-(4-methyl pyrimidin-2-yl)cyclopropane-1-carboxamide **(I-386)** | **Intermediate 834** ESI-MS (M+H)+: 554.2, δ 10.84 - 10.32 (m, 2H), 8.65 - 8.46 (m, 1H), 8.32 - 8.18 (m, 1H), 8.11 - 7.66 (m, 3H), 7.34 - 7.16 (m, 2H), 6.30 - 6.12 (m, 1H), 4.62 (d, J = 29.0 Hz, 2H), 2.94 (d, J = 33.4 Hz, 3H), 2.70 - 2.57 (m, 1H), 2.51 (s, 1H), 2.43 (d, J = 16.8 Hz, 3H), 1.95 (s, 1H), 1.84 - 1.42 (m, 2H), 1.09 - 0.44 (m, 4H). |
| **387** | 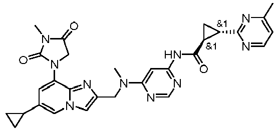 *rac*-(1S*,2S*)-N-(6-(((6-cyclo propyl-8-(3-methyl-2,4-dioxo imidazolidin-1-yl)imidazo[1,2-a]pyridin-2-yl)methyl) (methyl) amino)pyrimidin-4-yl)-2-(4-methyl pyrimidin-2-yl)cyclopropane-1-carboxamide **(I-387)** | **Intermediate 834** ESI-MS (M+H)+: 567.3, δ 10.78 (s, 1H), 8.52 (d, J = 5.1 Hz, 1H), 8.26 (s, 1H), 8.20 (s, 1H), 7.68 (s, 1H), 7.46 (s, 1H), 7.38 - 7.34 (m, 1H), 7.21 (d, J = 5.1 Hz, 1H), 4.90 (s, 2H), 4.81 (s, 2H), 3.13 (s, 3H), 2.97 (s, 3H), 2.64 - 2.61 (m, 1H), 2.56 - 2.53 (m, 1H), 2.41 (s, 3H), 1.97 - 1.90 (m, 1H), 1.55 - 1.50 (m, 2H), 0.96 - 0.91 (m, 2H), 0.65 - 0.61 (m, 2H) |
| **388** | 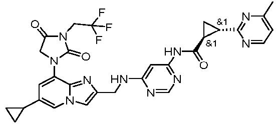 *rac*-(1S*,2S*)-N-(6-(((6-cyclo propyl-8-(2,4-dioxo-3-(2,2,2-trifluoroethyl)imidazolidin-1-yl) imidazo[1,2-a]pyridin-2-yl)methyl) amino)pyrimidin-4-yl)-2-(4-methylpyrimidin-2-yl)cyclopropane-1-carboxamide **(I-388)** | **Intermediate 834** ESI-MS (M+H)+: 621.2, δ 10.68 (s, 1H), 8.52 (d, J = 5.1 Hz, 1H), 8.28 (s, 1H), 8.19 (s, 1H), 7.88 (s, 1H), 7.71 (s, 1H), 7.32 (d, J = 5.0 Hz, 2H), 7.21 (d, J = 5.1 Hz, 1H), 5.05 (s, 2H), 4.58 (s, 2H), 4.38 - 4.31 (m, 2H), 2.64 - 2.60 (m, 1H), 2.57 - 2.52 (m, 1H), 2.41 (s, 3H), 1.99 - 1.92 (m, 1H), 1.56 - 1.48 (m, 2H), 0.97 - 0.90 (m, 2H), 0.67 - 0.61 (m, 2H). |
| **389** | 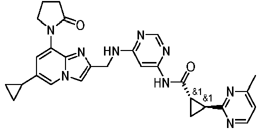 *rac*-(1S*,2S*)-N-(6-(((6-cyclopropyl-8-(2-oxopyrrolidin-1-yl)imidazo[1,2-a] pyridin-2-yl) methyl)amino)pyrimidin-4-yl)-2-(4-methylpyrimidin-2-yl)cyclo propane-1-carboxamide **(I-389)** | **Intermediate 834** ESI-MS (M+H)+: 524.2, δ 10.66 (s, 1H), 8.50 (d, J = 5.1 Hz, 1H), 8.22 (d, J = 1.2 Hz, 1H), 8.17 (s, 1H), 7.87 (s, 1H), 7.63 (s, 1H), 7.28 (s, 1H), 7.19 (d, J = 5.1 Hz, 1H), 7.09 (d, J = 1.3 Hz, 1H), 4.55 (s, 2H), 4.13 (t, J = 7.1 Hz, 2H), 2.62 - 2.58 (m, 1H), 2.53 - 2.52 (m, 1H), 2.46 (s, 1H), 2.44 (s, 1H), 2.40 (s, 3H), 2.14 - 2.06 (m, 2H), 1.92 - 1.87 (m, 1H), 1.54 - 1.46 (m, 2H), 0.92 - 0.87 (m, 2H), 0.64 - 0.60 (m, 2H). |
| **390** | 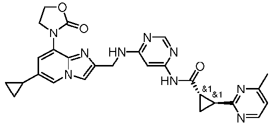 *rac*-(1S*,2S*)-N-(6-(((6-cyclo propyl-8-(2-oxooxazolidin-3-yl)imidazo[1,2-a] pyridin-2-yl) methyl) amino)pyrimidin-4-yl)-2-(4-methylpyrimidin-2-yl)cyclo propane-1-carboxamide **(I-390)** | **Intermediate 834** ESI-MS (M+H)+: 526.2, δ 10.66 (s, 1H), 8.50 (d, J = 5.1 Hz, 1H), 8.23 (d, J = 1.2 Hz, 1H), 8.17 (s, 1H), 7.88 (s, 1H), 7.66 (s, 1H), 7.28 (s, 1H), 7.20 - 7.18 (m, 2H), 4.59 - 4.40 (m, 6H), 2.62 - 2.58 (m, 1H), 2.53 - 2.52 (m, 1H), 2.39 (s, 3H), 1.94 - 1.87 (m, 1H), 1.54 - 1.46 (m, 2H), 0.93 - 0.88 (m, 2H), 0.64 - 0.60 (m, 2H). |
| **391** | 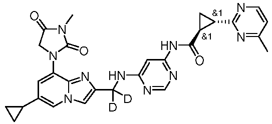 *rac*-(1S*,2S*)-N-(6-(((6-cyclo propyl-8-(3-methyl-2,4-dioxoimidazolidin-1-yl) imidazo[1,2-a]pyridin-2-yl)methyl-d2)amino)pyrimidin-4-yl)-2-(4-methyl pyrimidin-2-yl)cyclopropane-1-carboxamide **(I-391)** | **Intermediate 834** ESI-MS (M+H)+: 555.2, δ 10.67 (s, 1H), 8.52 (d, J = 5.1 Hz, 1H), 8.24 (s, 1H), 8.17 (s, 1H), 7.86 (s, 1H), 7.69 (s, 1H), 7.32 (d, J = 11.3 Hz, 2H), 7.21 (d, J = 5.1 Hz, 1H), 4.91 (s, 2H), 2.98 (s, 3H), 2.64 - 2.60 (m, 1H), 2.55 - 2.52 (m, 1H), 2.41 (s, 3H), 1.97 - 1.90 (m, 1H), 1.58 - 1.48 (m, 2H), 0.96 - 0.91 (m, 2H), 0.66 - 0.62 (m, 2H). |
| **392** | 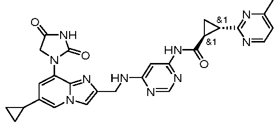 *rac*-(1S*,2S*)-N-(6-(((6-cyclopropyl-8-(2,4-dioxoimidazolidin-1-yl)imidazo[1,2-a]pyridin-2-yl) methyl)amino)pyrimidin-4-yl)-2-(4-methylpyrimidin-2-yl)cyclo propane-1-carboxamide **(I-392)** | **Intermediate 834** ESI-MS (M+H)+: 539.2, δ 11.33 (s, 1H), 10.68 (s, 1H), 8.52 (d, J = 5.1 Hz, 1H), 8.21 (d, J = 16.3 Hz, 2H), 7.90 (s, 1H), 7.68 (s, 1H), 7.31 (d, J = 8.4 Hz, 2H), 7.21 (d, J = 5.1 Hz, 1H), 4.87 (s, 2H), 4.58 (s, 2H), 2.72 - 2.52 (m, 2H), 2.41 (s, 3H), 2.01 - 1.85 (m, 1H), 1.55 - 1.48 (m, 2H), 1.10 - 0.84 (m, 2H), 0.79 - 0.47 (m, 2H). |
| **393** | 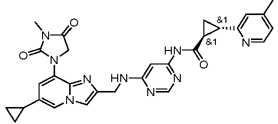 *rac*-(1S*,2S*)-N-(6-(((6-cyclo propyl-8-(3-methyl-2,4-dioxo imidazolidin-1-yl)imidazo[1,2-a]pyridin-2-yl)methyl) amino)pyrimidin-4-yl)-2-(4-methyl pyridin-2-yl)cyclopropane-1-carboxamide **(I-393)** | **Intermediate 835** ESI-MS (M+H)+: 552.2, δ 10.63 (s, 1H), 8.29 (d, J = 5.0 Hz, 1H), 8.25 (d, J = 1.0 Hz, 1H), 8.18 (s, 1H), 7.89 (s, 1H), 7.69 (s, 1H), 7.33 - 7.31 (m, 2H), 7.25 (s, 1H), 7.03 (d, J = 4.6 Hz, 1H), 4.91 (s, 2H), 4.58 (s, 2H), 2.98 (s, 3H), 2.47 - 2.43 (m, 2H), 2.27 (s, 3H), 1.97 - 1.90 (m, 1H), 1.52 - 1.47 (m, 1H), 1.44 - 1.40 (m, 1H), 0.96 - 0.92 (m, 2H), 0.66 - 0.62 (m, 2H). |
| **394** | 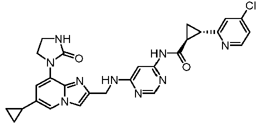 (1S,2S)-2-(4-chloropyridin-2-yl)-N-(6-(((6-cyclopropyl-8-(2-oxo imidazolidin-1-yl)imidazo[1,2-a]pyridin-2-yl)methyl) amino)pyrimidin-4-yl)cyclopropane-1-carboxamide (**I-394**) | **Intermediate 836** ESI-MS (M+H)+: 544.2, δ 10.63 (s, 1H), 8.41 (d, J = 5.3 Hz, 1H), 8.17 (s, 1H), 8.09 (d, J = 1.1 Hz, 1H), 7.85 (s, 1H), 7.63 (d, J = 1.7 Hz, 1H), 7.60 (s, 1H), 7.33 (dd, J = 5.3, 2.0 Hz, 1H), 7.28 (s, 1H), 7.22 (s, 1H), 6.97 (s, 1H), 4.54 (s, 2H), 4.34 (t, J = 8.1 Hz, 2H), 3.42 (t, J = 8.1 Hz, 2H), 2.60 - 2.55 (m, 2H), 1.90 - 1.85 (m, 1H), 1.53 - 1.45 (m, 2H), 0.91 - 0.87 (m, 2H), 0.62 - 0.58 (m, 2H). |
| **395** | 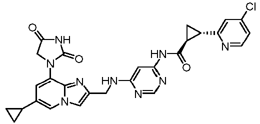 (1S,2S)-2-(4-chloropyridin-2-yl)-N-(6-(((6-cyclopropyl-8-(2,4-dioxo imidazolidin-1-yl)imidazo[1,2-a]pyridin-2-yl)methyl)amino)pyrimidin-4-yl)cyclo propane-1-carboxamide (I-395) | **Intermediate 836** |
| | | ESI-MS (M+H)+: 558.1, δ 11.34 (s, 1H), 10.65 (s, 1H), 8.42 (d, J = 5.4 Hz, 1H), 8.23 - 8.18 (m, 2H), 7.89 (s, 2H), 7.68 - 7.64 (m, 2H), 7.36 - 7.31 (m, 2H), 4.88 (s, 2H), 4.57 (s, 2H), 2.62 - 2.56 (m, 2H), 1.95 - 1.91 (m, 1H), 1.53 - 1.46 (m, 2H), 0.95 - 0.91 (m, 2H), 0.66 - 0.62 (m, 2H). |
| **396** | 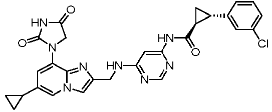 (1S,2S)-2-(3-chlorophenyl)-N-(6-(((6-cyclopropyl-8-(2,4-dioxo imidazolidin-1-yl)imidazo[1,2-a]pyridin-2-yl)methyl) amino)pyrimidin-4-yl)cyclopropane-1-carboxamide (**I-396**) | **Intermediate 676** |
| | | ESI-MS (M+H)+: 557.2, ¹H NMR (400 MHz, MeOD) δ 8.17 (s, 1H), 8.15 (s, 1H), 7.70 (s, 1H), 7.36 (s, 1H), 7.27 - 7.24 (m, 2H), 7.20 - 7.18 (m, 2H), 7.10 (d, J = 7.6 Hz, 1H), 4.70 (s, 2H), 4.64 (s, 2H), 2.49 - 2.47 (m, 1H), 2.19 - 2.17 (m, 1H), 1.96 - 1.93 (m, 1H), 1.62 - 1.58 (m, 2H), 0.98 - 0.96 (m, 2H), 0.75 - 0.71 (m, 2H). ESI-MS [M +H]⁺: 557.2. |
| **397** | 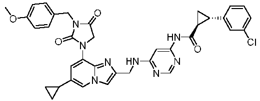 (1S,2S)-2-(3-chlorophenyl)-N-(6-(((6-cyclopropyl-8-(3-(4-methoxy benzyl)-2,4-dioxoimidazolidin-1-yl)imidazo[1,2-a] pyridin-2-yl) methyl)amino)pyrimidin-4-yl)cyclo propane-1-carboxamide (**I-397**) | **Intermediate 676** |
| | | ESI-MS (M+H)+: 677.2, δ 10.60 (s, 1H), 8.24 (s, 1H), 8.19 (s, 1H), 7.88 (s, 1H), 7.69 (s, 1H), 7.31 - 7.25 (m, 7H), 7.15 (d, J = 7.5 Hz, 1H), 6.91 (d, J = 8.7 Hz, 2H), 4.98 (s, 2H), 4.61 - 4.55 (m, 4H), 3.73 (s, 3H), 2.42 - 2.38 (m, 2H), 1.96 - 1.92 (m, 1H), 1.48 - 1.44 (m, 1H), 1.42 - 1.39 (m, 1H), 0.95 - 0.91 (m, 2H), 0.65 - 0.62 (m, 2H). |
| **398** | 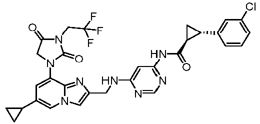 (1S,2S)-2-(3-chlorophenyl)-N-(6-(((6-cyclopropyl-8-(2,4-dioxo-3-(2,2,2-tri fluoroethyl)imidazolidin-1-yl)imidazo [1,2-a]pyridin-2-yl)methyl)amino) pyrimidin-4-yl)cyclopropane-1-carboxamide (**I-398**) | **Intermediate 676** |
| | | ESI-MS (M+H)+: 639.2, δ 10.59 (s, 1H), 8.26 (s, 1H), 8.17 (s, 1H), 7.88 (s, 1H), 7.69 (s, 1H), 7.32 - 7.28 (m, 3H), 7.24 - 7.23 (m, 2H), 7.13 (d, J = 7.5 Hz, 1H), 5.03 (s, 2H), 4.56 (s, 2H), 4.36 - 4.29 (m, 2H), 2.41 - 2.34 (m, 2H), 1.99 - 1.90 (m, 1H), 1.48 - 1.43 (m, 1H), 1.41 - 1.36 (m, 1H), 0.95 - 0.90 (m, 2H), 0.65 - 0.61 (m, 2H). |
| **399** | 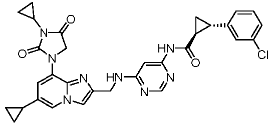 (1S,2S)-2-(3-chlorophenyl)-N-(6-(((6-cyclopropyl-8-(3-cyclopropyl-2,4-dioxoimidazolidin-1-yl)imidazo[1,2-a]pyridin-2-yl)methyl)amino) pyrimidin-4-yl)cyclopropane-1-carboxamide (**I-399**) | **Intermediate 676** |
| | | ESI-MS (M+H)+: 597.2, ¹H NMR (400 MHz, MeOD) δ 8.16 (s, 1H), 8.15 (d, J = 0.9 Hz, 1H), 7.69 (s, 1H), 7.36 (s, 1H), 7.27 - 7.23 (m, 2H), 7.20 - 7.18 (m, 2H), 7.10 - 7.09 (m, 1H), 4.63 (s, 2H), 4.59 (s, 2H), 2.70 - 2.64 (m, 1H), 2.50 - 2.45 (m, 1H), 2.20-2.17 (m, 1H), 1.98 - 1.91 (m, 1H), 1.62 - 1.58 (m, 1H), 1.39 - 1.35 (m, 1H), 1.02 - 0.94 (m, 6H), 0.74- 0.70 (m, 2H). ESI-MS [M +H]+: 597.2. |
| **400** | 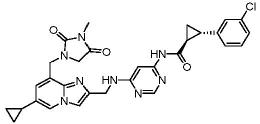 (1S,2S)-2-(3-chlorophenyl)-N-(6-(((6-cyclopropyl-8-((3-methyl-2,4-dioxoimidazolidin-1-yl)methyl) imidazo[1,2-a]pyridin-2-yl)methyl) amino)pyrimidin-4-yl)cyclopropane-1-carboxamide (**I-400**) | **Intermediate 676** |
| | | ESI-MS (M+H)+: 585.2, ¹H NMR (400 MHz, CDCl₃) δ 8.44 (s, 1H), 8.28 (s, 1H), 7.80 (s, 1H), 7.47 (s, 1H), 7.35 (s, 1H), 7.22 - 7.18 (m, 2H), 7.10 (s, 1H), 7.01 (d, J = 6.8 Hz, 1H), 6.95 (s, 1H), 5.82 (s, 1H), 4.86 (s, 2H), 4.66 (s, 2H), 4.11 (s, 2H), 3.04 (s, 3H), 2.60 - 2.55 (m, 1H), 1.91 - 1.83 (m, 2H), 1.42 - 1.37 (m, 2H), 0.99 - 0.94 (m, 2H), 0.68 - 0.64 (m, 2H). |
| **401** | 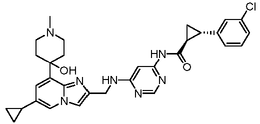 (1S,2S)-2-(3-chlorophenyl)-N-(6-(((6-cyclopropyl-8-(4-hydroxy-1-methyl piperidin-4-yl)imidazo[1,2-a]pyridin-2-yl)methyl)amino)pyrimidin-4-yl)cyclo propane-1-carboxamide (**I-401**) | **Intermediate 676** |
| | | ESI-MS (M+H)+: 572.2, δ 10.58 (s, 1H), 8.17 (s, 2H), 7.86 (s, 1H), 7.55 (s, 1H), 7.31 - 7.22 (m, 4H), 7.12 (d, J = 7.8 Hz, 1H), 7.07 - 7.06 (m, 1H), 5.37 (s, 1H), 4.54 (s, 2H), 2.87 - 2.80 (m, 2H), 2.71 (s, 2H), 2.32 - 2.30 (m, 7H), 1.92 - 1.85 (m, 1H), 1.51 - 1.43 (m, 4H), 0.92 - 0.86 (m, 2H), 0.65 - 0.60 (m, 2H). |
| **402** | 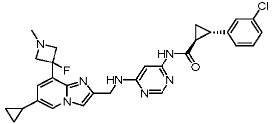 (1S,2S)-2-(3-chlorophenyl)-N-(6-(((6-cyclopropyl-8-(3-fluoro-1-methyl azetidin-3-yl)imidazo[1,2-a]pyridin-2-yl)methyl)amino)pyrimidin-4-yl)cyclo propane-1-carboxamide (**I-402**) | **Interedite 676** |
| | | ESI-MS (M+H)+: 546.2, δ 10.61 (s, 1H), 8.34 (s, 1H), 8.19 (s, 1H), 7.89 (s, 1H), 7.65 (s, 1H), 7.37 - 7.25 (m, 4H), 7.15 (d, J = 7.7 Hz, 1H), 7.07 (s, 1H), 4.61 (d, J = 21.0 Hz, 2H), 4.00 (dd, J = 20.7, 10.1 Hz, 2H), 3.67 (dd, J = 20.6, 10.1 Hz, 3H), 2.39 - 2.30 (m, 5H), 1.98 - 1.91 (m, 1H), 1.48 - 1.43 (m, 1H), 1.43 - 1.38 (m, 1H), 0.94 - 0.89 (m, 2H), 0.71 - 0.67 (m, 2H). |
| **403** | 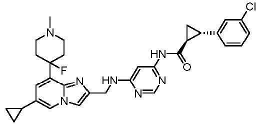 (1S,2S)-2-(3-chlorophenyl)-N-(6-(((6-cyclopropyl-8-(4-fluoro-1-methyl piperidin-4-yl)imidazo[1,2-a]pyridin-2-yl)methyl)amino)pyrimidin-4-yl)cyclo propane-1-carboxamide (**I-403**) | **Intermediate 676** |
| | | ESI-MS (M+H)+: 574.2, δ 10.61 (s, 1H), 8.26 - 8.19 (m, 2H), 7.90 (s, 1H), 7.61 (s, 1H), 7.34 - 7.25 (m, 4H), 7.15 (d, J = 7.6 Hz, 1H), 7.00 (s, 1H), 4.57 (s, 2H), 3.16 - 3.08 (m, 1H), 3.05 - 2.97 (m, 1H), 2.77 (d, J = 10.0 Hz, 2H), 2.41 - 2.28 (m, 7H), 1.97 - 1.90 (m, 1H), 1.78 - 1.72 (m, 2H), 1.49 - 1.38 (m, 2H), 0.93 - 0.89 (m, 2H), 0.67 - 0.63 (m, 2H). |
| **404** | 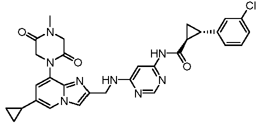 (1S,2S)-2-(3-chlorophenyl)-N-(6-(((6-cyclopropyl-8-(4-methyl-2,5-dioxo piperazin-1-yl)imidazo[1,2-a]pyridin-2-yl)methyl)amino)pyrimidin-4-yl)cyclo propane-1-carboxamide (**I-404**) | **Intermediate 676** |
| | | ESI-MS (M+H)+: 585.2, δ 10.60 (s, 1H), 8.33 (s, 1H), 8.19 (s, 1H), 7.92 (s, 1H), 7.67 (s, 1H), 7.39 - 7.20 (m, 4H), 7.15 (d, J = 7.6 Hz, 1H), 7.04 (d, J = 1.0 Hz, 1H), 4.56 (s, 2H), 4.42 (s, 2H), 4.20 (s, 2H), 2.93 (s, 3H), 2.47 - 2.30 (m, 2H), 1.98 - 1.87 (m, 1H), 1.51 - 1.44 (m, 1H), 1.43 - 1.37 (m, 1H), 0.98 - 0.88 (m, 2H), 0.71 - 0.61 (m, 2H). |
| **405** | 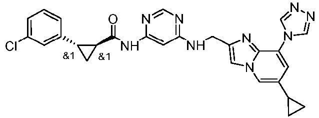 rac-(1S*,2S*)-2-(3-chlorophenyl)-N-(6-(((6-cyclopropyl-8-(4H-1,2,4-triazol-4-yl)imidazo[1,2-a]pyridin-2-yl)methyl) amino)pyrimidin-4-yl)cyclopropane-1-carboxamide (**I-405**) | **Intermediate 676** |
| | | ESI-MS (M+H)+: 526.1, δ 10.62 (s, 1H), 9.46 (s, 2H), 8.44 (s, 1H), 8.20 (s, 1H), 7.93 (s, 1H), 7.82 (s, 1H), 7.41 (s, 1H), 7.36 - 7.23 (m, 4H), 7.15 (d, J = 7.6 Hz, 1H), 4.62 (s, 2H), 2.42 - 2.37 (m,,2H), 2.12 - 1.82 (m, 1H), 1.49 - 1.41 (m, 2H), 0.99 - 0.96 (m, 2H), 0.83 - 0.80 (m, 2H). ESI-MS [M +H]⁺: 526.1. |
| **406** | 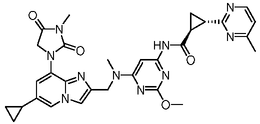 (1S,2S)-N-(6-(((6-cyclopropyl-8-(3-methyl-2,4-dioxoimidazolidin-1-yl) imidazo[1,2-a]pyridin-2-yl)methyl) (methyl)amino)-2-methoxypyrimidin-4-yl)-2-(4-methylpyrimidin-2-yl)cyclo propane-1-carboxamide (**I-406**) | **Intermediate 837** |
| | | ESI-MS (M+H)+: 597.3, δ 10.71 (s, 1H), 8.52 (d, *J =* 5.1 Hz, 1H), 8.22 (d, *J =* 1.0 Hz, 1H), 7.69 (s, 1H), 7.36 (d, *J* = 1.4 Hz, 1H), 7.20 (d, *J =* 5.1 Hz, 1H), 7.14 (s, 1H), 4.91 (s, 2H), 4.80 (s, 2H), 3.78 (s, 3H), 3.12 (s, 3H), 2.97 (s, 3H), 2.65 - 2.54 (m, 2H), 2.41 (s, 3H), 1.97 - 1.89 (m, 1H), 1.55 - 1.47 (m, 2H), 0.97 - 0.90 (m, 2H), 0.67 - 0.60 (m, 2H). |
| **407** | 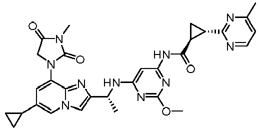 (1S,2S)-N-(6-(((R)-1-(6-cyclopropyl-8-(3-methyl-2,4-dioxoimidazolidin-1-yl)imidazo[1,2-a]pyridin-2-yl)ethyl) amino)-2-methoxypyrimidin-4-yl)-2-(4-methylpyrimidin-2-yl)cyclo propane-1-carboxamide (**I-407**) | **Intermediate 837** ESI-MS (M+H)+: 597.2, δ 10.56 (s, 1H), 8.52 (d, *J* = 5.1 Hz, 1H), 8.23 (d, *J =* 1.1 Hz, 1H), 7.82 (s, 1H), 7.66 (s, 1H), 7.33 (d, *J =* 1.4 Hz, 1H), 7.20 (d, *J* = 5.1 Hz, 1H), 6.98 (s, 1H), 5.34 (s, 1H), 4.94 (s, 2H), 3.73 (s, 3H), 2.97 (s, 3H), 2.64 - 2.60 (m, 1H), 2.54 - 2.52 (m, 1H), 2.41 (s, 3H), 1.95 - 1.91 m, 1H), 1.51 - 1.49 (m, 5H), 0.96 - 0.91 (m, 2H), 0.65 - 0.61 (m, 2H). |
| **408** | 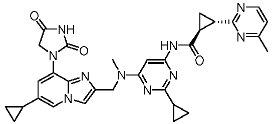 (1S,2S)-N-(2-cyclopropyl-6-(((6-cyclo propyl-8-(2,4-dioxoimidazolidin-1-yl)imidazo[1,2-a]pyridin-2-yl)methyl) (methyl)amino)pyrimidin-4-yl)-2-(4-methylpyrimidin-2-yl)cyclopropane-1-carboxamide (**I-408**) | **Intermediate 838** ESI-MS (M+H)+: 593.3, δ 11.34 (s, 1H), 10.67 (s, 1H), 8.53 (d, J = 5.3 Hz, 1H), 8.22 (s, 1H), 7.63 (s, 1H), 7.36 (s, 1H), 7.22 - 7.20 (m, 2H), 4.86 (s, 2H), 4.78 (s, 2H), 3.13 (s, 3H), 2.67 - 2.64 (m, 2H), 2.41 (s, 3H), 1.95 - 1.90 (m, 1H), 1.88 - 1.82 (m, 1H), 1.53 - 1.49 (m, 2H), 1.28 - 1.23 (m, 4H), 0.86 - 0.83 (m, 2H), 0.66 - 0.62 (m, 2H). |
| **409** | 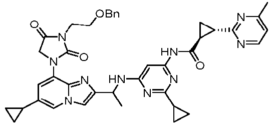 (1S,2S)-N-(6-((1-(8-(3-(2-(benzyl oxy) ethyl)-2,4-dioxoimidazolidin-1-yl)-6-cyclopropylimidazo[1,2-a]pyridin-2-yl)ethyl)amino)-2-cyclopropylpyrimidin-4-yl)-2-(4-methylpyrimidin-2-yl)cyclo propane-1-carboxamide (**I-409**) | **Intermediate 838** ESI-MS (M+H)+: 727.3, δ 10.53 (s, 1H), 8.52 (d, J = 5.1 Hz, 1H), 8.26 (s, 1H), 7.70 - 7.55 (m, 2H), 7.35 - 7.27 (m, 6H), 7.20 (d, J = 5.1 Hz, 1H), 7.07 (s, 1H), 5.56 - 5.12 (m, 1H), 4.97 (s, 2H), 4.51 (s, 2H), 3.74 - 3.70 (m, 2H), 3.67 - 3.63 (m, 2H), 2.64 - 2.58 (m, 2H), 2.41 (s, 3H), 1.97 - 1.91 (m, 1H), 1.82 - 1.75 (m, 1H), 1.53 - 1.45 (m, 5H), 0.97 - 0.92 (m, 2H), 0.91 - 0.83 (m, 2H), 0.81 - 0.76 (m, 2H), 0.67 - 0.61 (m,2H). |
| **410** | 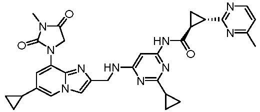 (1S,2S)-N-(2-cyclopropyl-6-(((6-cyclo propyl-8-(3-methyl-2,4-dioxo imidazolidin-1-yl)imidazo[1,2-a]pyridin-2-yl)methyl)amino) pyrimidin-4-yl)-2-(4-methylpyrimidin-2-yl)cyclopropane-1-carboxamide (**I-410**) | **Intermediate 838** ESI-MS (M+H)+: 593.3, δ 10.55 (s, 1H), 8.52 (d, J = 5.1 Hz, 1H), 8.26 (s, 1H), 7.66 (s, 2H), 7.33 (s, 1H), 7.20 (d, J = 5.1 Hz, 1H), 7.08 (s, 1H), 4.92 (s, 2H), 4.54 (s, 2H), 2.98 (s, 3H), 2.68 - 2.56 (m, 2H), 2.41 (s, 3H), 1.94 - 1.90 (m, 1H), 1.89 - 1.71 (m, 1H), 1.60 - 1.41 (m, 2H), 1.01 - 0.75 (m, 6H), 0.73 - 0.53 (m, 2H). |
| **411** | 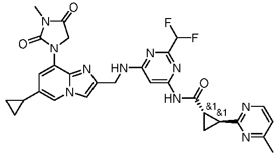 rac-(1S*,2S*)-N-(6-(((6-cyclopropyl-8-(3-methyl-2,4-dioxoimidazolidin-1-yl)imidazo[1,2-a]pyridin-2-yl)methyl)amino)-2-(di fluoromethyl)pyrimidin-4-yl)-2-(4-methylpyrimidin-2-yl)cyclopropane-1-carboxamide (**I-411**) | **Intermediate 839** ESI-MS (M+H)+: 603.2, δ 11.03 (s, 1H), 8.52 (d, J = 5.1 Hz, 1H), 8.25 (d, J = 1.1 Hz, 2H), 7.73 (s, 1H), 7.40 (s, 1H), 7.34 (s, 1H), 7.20 (d, J = 5.1 Hz, 1H), 6.54 (t, J = 54.6 Hz, 1H), 4.91 (s, 2H), 4.69 - 4.31 (m, 2H), 2.98 (s, 3H), 2.67 - 2.60 (m, 2H), 2.41 (s, 3H), 1.98 - 1.91 (m, 1H), 1.53 - 1.51 (m, 2H), 0.96 - 0.92 (m, 2H), 0.67 - 0.63 (m, 2H). |
| **412** | 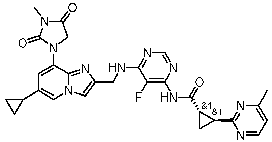 *rac-*(1S*,2S*)-N-(6-(((6-cyclopropyl-8-(3-methyl-2,4-dioxoimidazolidin-1-ylimidazo[1,2-a]pyridin-2-yl)methyl) amino)-5-fluoropyrimidin-4-yl)-2-(4-methylpyrimidin-2-yl)cyclopropane-1-carboxamide (**I-412**) | **Intermediate 840** |
| | | ESI-MS (M+H)+: 571.2, ¹H NMR (400 MHz, MeOD) δ 10.57 (s, 1H), 8.54 (d, J = 5.1 Hz, 1H), 8.26-8.20 (m, 2H), 8.09 (s, 1H), 7.70 (s, 1H), 7.33 (d, J = 1.5 Hz, 1H), 7.22 (d, J = 5.1 Hz, 1H), 4.89 (s, 2H), 4.67 (d, J = 5.8 Hz, 2H), 2.97 (d, J = 2.4 Hz, 3H), 2.54 - 2.51 (m, 2H), 2.43 (s, 3H), 1.97 - 1.85 (m, 1H), 1.65 - 1.47 (m, 2H), 0.96 - 0.89 (m, 2H), 0.69 - 0.59 (m, 2H). |
| **413** | 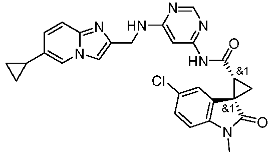 *rac*-(1R*,2S*)-5'-chloro-N-(6-(((6-cyclopropylimidazo[1,2-a]pyridin-2-yl)methyl)amino)pyrimidin-4-yl)-1'-methyl-2'-oxospiro[cyclopropane-1,3'-indoline]-2-carboxamide (**I-413**) | **Intermediate 841** ESI-MS (M+H)+: 514.2, δ 10.58 (s, 1H), 8.30 (s, 1H), 8.14 (s, 1H), 7.87 (s, 1H), 7.61 (s, 1H), 7.40 - 7.31 (m, 3H), 7.27 (s, 1H), 7.21 (d, J = 2.1 Hz, 1H), 7.07 (d, J = 8.3 Hz, 1H), 6.97 - 6.94 (m, 1H), 4.57 (s, 2H), 3.15 (s, 3H), 3.10 (t, J = 8.5 Hz, 1H), 2.12 - 2.07 (m, 1H), 2.00 - 1.86 (m, 3H). |
| **414** | 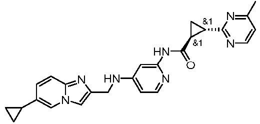 *rac*-(1S*,2S*)-N-(4-(((6-cyclo propylimidazo[1,2-a]pyridin-2-yl) methyl)amino)pyridin-2-yl)-2-(4-methylpyrimidin-2-yl)cyclopropane-1-carboxamide (**I-414**) | *rac-*(1S*,2S*)-N-(4-fluoropyridin-2-yl)-2-(4-methylpyrimidin-2-yl)cyclopropane-1-carboxamide ESI-MS (M+H)+: 440.2, |
| | | δ 10.47 (s, 1H), 8.51 - 8.49 (m, 1H), 8.30 (s, 1H), 7.75 - 7.74 (m, 1H), 7.63 (s, 1H), 7.38 - 7.35 (m, 2H), 7.20 - 7.18 (m, 2H), 6.98 - 6.95 (m, 1H), 6.35 - 6.33 (m, 1H), 4.36 - 4.34 (m, 2H), 2.59 - 2.51 (m, 2H), 2.40 (s, 3H), 1.92 - 1.87 (m, 1H), 1.51 - 1.46 (m, 2H), 0.92 - 0.87 (m, 2H), 0.67 - 0.63 (m, 2H). |
| **415** | 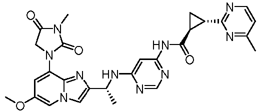 (1S,2S)-N-(6-(((R)-1-(6-methoxy-8-(3-methyl-2,4-dioxoimidazolidin-1-yl)imidazo[1,2-a]pyridin-2-yl)ethyl) amino)pyrimidin-4-yl)-2-(4-methyl pyrimidin-2-yl)cyclopropane-1-carboxamide (**I-415**) | **Intermediate 833** |
| | | ESI-MS [M +H]+: 557.3, δ 10.64 (s, 1H), 8.52 (d, J = 5.1 Hz, 1H), 8.16 (s, 1H), 8.13 (d, J = 2.1 Hz, 1H), 7.76 (s, 1H), 7.68 (s, 1H), 7.40 (d, J = 2.1 Hz, 1H), 7.31 (s, 1H), 7.20 (d, J = 5.1 Hz, 1H), 5.33 (s, 1H), 5.00 (s, 2H), 3.75 (s, 3H), 2.97 (s, 3H), 2.64 - 2.60 (m, 1H), 2.55 - 2.52 (m, 1H), 2.41 (s, 3H), 1.58 - 1.46 (m, 5H). |
| **416** | 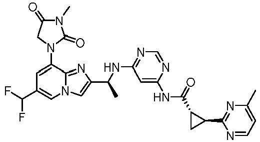 (1S,25)-N-(6-(((S)-1-(6-(difluoro methyl)-8-(3-methyl-2,4-dioxo imidazolidin-1-yl)imidazo[1,2-a]pyridin-2-yl)ethyl)amino) pyrimidin-4-yl)-2-(4-methyl pyrimidin-2-yl)cyclopropane-1-carboxamide (**I-416**) | **Intermediate 833**ESI-MS [M +H]+: 577.1. δ 10.65 (s, 1H), 8.74 (s, 1H), 8.52 (d, J = 5.1 Hz, 1H), 8.16 (s, 1H), 7.91 (s, 1H), 7.82 (s, 1H), 7.77 (s, 1H), 7.32 (s, 1H), 7.26-6.99 (m, 2H), 5.47 - 5.34 (m, 1H), 5.06-4.96 (m, 2H), 2.98 (s, 3H), 2.64 - 2.60 (m, 2H), 2.41 (s, 3H), 1.58 - 1.47 (m, 5H). |
| **417** | 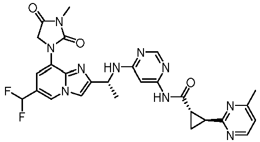 (1S,25)-N-(6-(((R)-1-(6-(difluoro methyl)-8-(3-methyl-2,4-dioxo imidazolidin-1-yl)imidazo[1,2-a]pyridin-2-yl)ethyl)amino) pyrimidin-4-yl)-2-(4-methyl pyrimidin-2-yl)cyclopropane-1-carboxamide (**I-417**) | **Intermediate 833**ESI-MS [M +H]+: 577.3, δ 10.66 (s, 1H), 8.74 (s, 1H), 8.52 (d, J = 5.1 Hz, 1H), 8.16 (s, 1H), 7.91 (s, 1H), 7.83 (s, 1H), 7.77 (s, 1H), 7.32 (s, 1H), 7.26-6.98 (m, 2H) ,5.45 - 5.35 (m, 1H), 5.05-4.96 (m, 2H), 2.98 (s, 3H), 2.64 - 2.60 (m, 2H), 2.41 (s, 3H), 1.57 - 1.46 (m, 5H). |
| **418** | 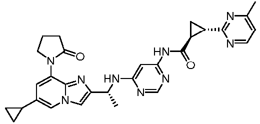 (1S,2S)-N-(6-(((R)-1-(6-cyclopropyl-8-(2-oxopyrrolidin-1-yl)imidazo[1,2-a]pyridin-2-yl)ethyl)amino) pyrimidin-4-yl)-2-(4-methyl pyrimidin-2-yl)cyclopropane-1-carboxamide (**I-418**) | **Intermediate 833** ESI-MS [M +H]⁺: 538.0, δ 10.65 (s, 1H), 8.52 (d, J = 5.1 Hz, 1H), 8.22 (d, J = 1.2 Hz, 1H), 8.15 (s, 1H), 7.78 (s, 1H), 7.62 (s, 1H), 7.30 (s, 1H), 7.21 (d, J = 5.1 Hz, 1H), 7.12 (d, J = 1.4 Hz, 1H), 5.32 (s, 1H), 4.17 (t, J = 7.1 Hz, 2H), 2.64 - 2.60 (m, 1H), 2.56 - 2.53 (m, 1H), 2.48 (s, 1H), 2.46 (s, 1H), 2.41 (s, 3H), 2.14 - 2.10 (m, 2H), 1.93 - 1.89 (m, 1H), 1.53 - 1.49 (m, 5H), 0.92 - 0.90 (m,2H), 0.65-0.61 (m,2H). |
| **419** | 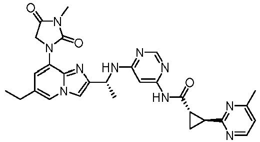 (1S,2S)-N-(6-(((R)-1-(6-ethyl-8-(3-methyl-2,4-dioxoimidazolidin-1-yl)imidazo[1,2-a]pyridin-2-yl)ethyl) amino)pyrimidin-4-yl)-2-(4-methyl pyrimidin-2-yl)cyclopropane-1-carboxamide (**I-419**) | **Intermediate 833** |
| | | ESI-MS [M +H]+: 555.2, δ 10.64 (s, 1H), 8.52 (d, J = 5.1 Hz, 1H), 8.23 (s, 1H), 8.16 (d, J = 0.7 Hz, 1H), 7.77 (s, 1H), 7.69 (s, 1H), 7.46 (d, J = 1.4 Hz, 1H), 7.30 (s, 1H), 7.20 (d, J = 5.1 Hz, 1H), 5.33 (s, 1H), 4.95 (d, J = 1.2 Hz, 2H), 2.98 (s, 3H), 2.64 - 2.52 (m, 4H), 2.41 (s, 3H), 1.57 - 1.46 (m, 5H), 1.19 (t, J = 7.5 Hz, 3H). |
| **420** | 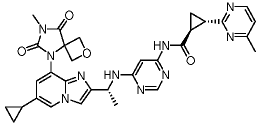 (1S,2S)-N-(6-(((R)-1-(6-cyclopropyl-8-(7-methyl-6,8-dioxo-2-oxa-5,7-di azaspiro[3.4]octan-5-yl)imidazo[1,2-a]pyridin-2-yl)ethyl)amino) pyrimidin-4-yl)-2-(4-methyl pyrimidin-2-yl)cyclopropane-1-carboxamide (**I-420**) | **Intermediate 833** ESI-MS [M +H]⁺: 609.2, δ 10.64 (s, 1H), 8.52 (d, J = 5.1 Hz, 1H), 8.48 (s, 1H), 8.14 (s, 1H), 7.78 (s, 1H), 7.73 (d, J = 2.2 Hz, 1H), 7.28 (s, 1H), 7.25 - 7.17 (m, 2H), 5.32 (s, 1H), 4.86 - 4.73 (m, 4H), 2.98 (s, 3H), 2.60 (d, J = 8.6 Hz, 2H), 2.41 (s, 3H), 2.04 - 1.83 (m, 1H), 1.52 - 1.46 (m, 5H), 0.97 - 0.93 (m, 2H), 0.69 - 0.65 (m, 2H). |
| **421** | 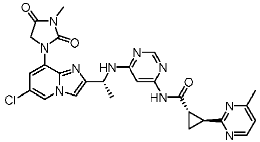 (1S,2S)-N-(6-(((R)-1-(6-chloro-8-(3-methyl-2,4-dioxoimidazolidin-1-yl)imidazo[1,2-a]pyridin-2-yl)ethyl) amino)pyrimidin-4-yl)-2-(4-methyl pyrimidin-2-yl)cyclopropane-1-carboxamide (**I-421**) | **Intermediate 833**ESI-MS [M +H]+: 561.1, δ 10.67 (s, 1H), 8.66 (d, J = 1.9 Hz, 1H), 8.52 (d, J = 5.1 Hz, 1H), 8.16 (s, 1H), 7.84 (s, 1H), 7.77 (s, 1H), 7.67 (d, J = 1.9 Hz, 1H), 7.32 (s, 1H), 7.21 (d, J = 5.1 Hz, 1H), 5.38 (s, 1H), 5.01 (s, 1H), 2.98 (s, 3H), 2.63 - 2.54 (m, 2H), 2.41 (s, 3H), 1.52 (d, J = 6.8 Hz, 5H). |
| **422** | 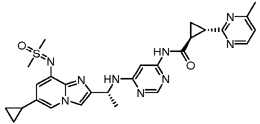 (1S,2S)-N-(6-(((R)-1-(6-cyclopropyl-8-((dimethyl(oxo)-l6-sulfanylidene) amino)imidazo[1,2-a]pyridin-2-yl) ethyl)amino)pyrimidin-4-yl)-2-(4-methylpyrimidin-2-yl)cyclopropane-1-carboxamide (**I-422**) | **Intermediate 833** ESI-MS [M +H]⁺: 546.2, δ 10.63 (s, 1H), 8.52 (d, J = 5.1 Hz, 1H), 8.15 (s, 1H), 7.84 (s, 1H), 7.74 (s, 1H), 7.49 (s, 1H), 7.26 (s, 1H), 7.21 (d, J = 5.1 Hz, 1H), 6.43 (s, 1H), 5.31 (s, 1H), 3.36 (s, 6H), 2.63 - 2.59 (m, 2H), 2.41 (s, 3H), 1.86 - 1.80 (m, 1H), 1.53 - 1.48 (m, 5H), 0.88 - 0.83 (m, 2H), 0.61 - 0.57 (m, 2H). |
| **423** | 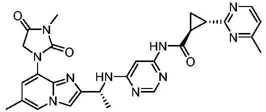 (1S,2S)-N-(6-(((R)-1-(6-methyl-8-(3-methyl-2,4-dioxoimidazolidin-1-yl) imidazo[1,2-a]pyridin-2-yl)ethyl) amino)pyrimidin-4-yl)-2-(4-methyl pyrimidin-2-yl)cyclopropane-1-carboxamide (**I-423**) | **Intermediate 833**ESI-MS [M +H]⁺: 541.3, δ 10.64 (s, 1H), 8.52 (d, J = 5.1 Hz, 1H), 8.21 (s, 1H), 8.16 (s, 1H), 7.76 (s, 1H), 7.69 (s, 1H), 7.42 (d, J = 1.2 Hz, 1H), 7.30 (s, 1H), 7.21 (d, J = 5.1 Hz, 1H), 5.34 (s, 1H), 4.95 (d, J = 1.1 Hz, 2H), 2.98 (s, 3H), 2.64 - 2.60 (m, 1H), 2.55 - 2.54 (m, 1H), 2.41 (s, 3H), 2.26 (s, 3H), 1.55 - 1.49 (m, 5H). |
| **424** | 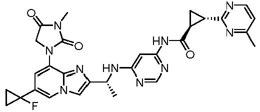 (1S,2S)-N-(6-(((R)-1-(6-(1-fluoro cyclopropyl)-8-(3-methyl-2,4-dioxo imidazolidin-1-yl)imidazo[1,2-a] pyridin-2-yl)ethyl)amino) pyrimidin-4-yl)-2-(4-methyl pyrimidin-2-yl) cyclopropane-1-carboxamide (**I-424**) | **Intermediate 833** ESI-MS [M +H]+: 585.2, δ 10.66 (s, 1H), 8.52 (d, J = 5.0 Hz, 2H), 8.16 (s, 1H), 7.81 (s, 1H), 7.75 (d, J = 2.3 Hz, 1H), 7.60 (d, J = 1.4 Hz, 1H), 7.31 (s, 1H), 7.21 (d, J = 5.1 Hz, 1H), 5.37 (s, 1H), 5.02 - 4.93 (m, 2H), 2.98 (s, 3H), 2.63 - 2.60 (m, 1H), 2.57 - 2.55 (m, 1H), 2.41 (s, 3H), 1.53 - 1.42 (m, 7H), 1.14 -1.08 (m,2H). |
| **425** | 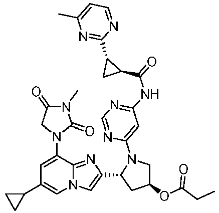 (3S,5R)-5-(6-cyclopropyl-8-(3-methyl-2,4-dioxoimidazolidin-1-yl)I midazo[1,2-a]pyridin-2-yl)-1-(6-((1S,2S)-2-(4-methyl pyrimidin-2-yl)cyclopropane-1-carbox amido) pyrimidin-4-yl)pyrrolidin-3-yl propionate (**I-425**) | **Intermediate 833** ESI-MS [M +H]⁺: 665.3, δ 10.70 (s, 1H), 8.52 (d, J = 5.1 Hz, 1H), 8.21 (d, J = 9.4 Hz, 2H), 7.76 (s, 1H), 7.37 (s, 1H), 7.29 (s, 1H), 7.20 (d, J = 5.1 Hz, 1H), 5.44 (s, 1H), 4.94 (s, 2H), 3.97 (s, 1H), 3.40-3.39 (m, 1H), 3.30 - 3.26 (m, 1H), 2.97 (s, 3H), 2.53-2.51 (m, 4H), 2.41 (s, 3H), 2.34 - 2.28 (m, 2H), 1.94-1.93 (m, 1H), 1.50-1.48 (m, 2H), 1.02 (t, J = 7.5 Hz, 3H), 0.93-0.92 (m, 2H), 0.64-0.63 (m, 2H). |
| **426** | 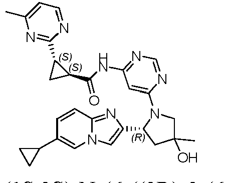 (1S,2S)-N-(6-((2R)-2-(6-cyclo propyl imidazo[1,2-a]pyridin-2-yl)-4-hydroxy-4-methylpyrrolidin-1-yl) pyrimidin-4-yl)-2-(4-methyl pyrimidin-2-yl)cyclopropane-1-carboxamide (**I-426**) | **Intermediate 833** ESI-MS [M + H ]⁺: 511.2. |
| | | δ 10.66 (s, 1H), 8.50 (d, J = 5.1 Hz, 1H), 8.38 - 8.30 (m, 2H), 8.19 - 8.15 (m, 1H), 7.71 (s, 1H), 7.36 (d, J = 9.2 Hz, 1H), 7.19 (d, J = 5.1 Hz, 1H), 7.01 - 6.98 (m, 1H), 5.47 (s, 1H), 5.01 - 4.96 (m, 1H), 3.79 - 3.61 (m, 2H), 2.65 - 2.59 (m, 2H), 2.39 (s, 3H), 2.32 - 2.30 (m, 1H), 2.12 (d, J = 13.4 Hz,1H), 1.94-1.88 (m,1H), 1.54-1.47 (m,2H), 1.34 (s,3H), 0.92-0.87 (m,2H), 0.66-0.63 (m,2H) |
| **427** | 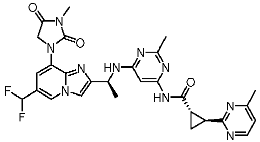 (1S,2S)-N-(6-(((S)-1-(6-(difluoro methyl)-8-(3-methyl-2,4-dioxo imidazolidin-1-yl)imidazo[1,2-a] pyridin-2-yl)ethyl)amino)-2-methyl pyrimidin-4-yl)-2-(4-methyl pyrimidin-2-yl)cyclo propane-1-carboxamide (**I-427**) | **Intermediate 832**ESI-MS [M +H]+: 591.1. δ 10.65 (s, 1H), 8.75 (s, 1H), 8.51 (d, J = 5.1 Hz, 1H), 7.92 (s, 1H), 7.77 (s, 1H), 7.70 (s, 1H), 7.26-6.99 (m, 3H), 5.52-5.38 (m, 1H), 5.07-4.99 (m, 2H), 2.99 (s, 3H), 2.57-2.25 (m, 2H), 2.41 (s, 3H), 2.25 (s, 3H), 1.58 - 1.38 (m, 5H). |
| **428** | 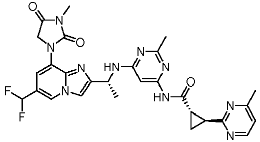 (1S,2S)-N-(6-(((R)-1-(6-(difluoromethyl)-8-(3-methyl-2,4-dioxoimidazolidin-1-yl) imidazo[1,2-a]pyridin-2-yl)ethyl)amino)-2-methylpyrimidin-4-yl)-2-(4-methyl pyrimidin-2-yl)cyclopropane-1-carboxamide (**I-428**) | **Intermediate 832** |
| | | ESI-MS [M +H]+: 591.1. δ 10.64 (s, 1H), 8.75 (s, 1H), 8.51 (d, J = 5.1 Hz, 1H), 7.91 (s, 1H), 7.76 (s, 1H), 7.69 (s, 1H), 7.26-6.98 (m, 3H), 5.59 - 5.34 (m, 1H), 5.05-4.96 (m, 2H), 2.98 (s, 3H), 2.62 - 2.56 (m, 2H), 2.40 (s, 3H), 2.24 (s, 3H), 1.56 - 1.39 (m, 5H). |
| **429** | 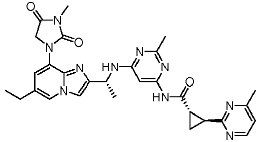 (1S,2S)-N-(6-(((R)-1-(6-ethyl-8-(3-methyl-2,4-dioxoimidazolidin-1-yl) imidazo[1,2-a]pyridin-2-yl)ethyl)amino)-2-methylpyrimidin-4-yl)-2-(4-methyl pyrimidin-2-yl)cyclopropane-1-carboxamide (**I-429**) | **Intermediate 832** ESI-MS [M +H]+: 569.2, δ 10.64 (s, 1H), 8.51 (d, J = 5.1 Hz, 1H), 8.24 (s, 1H), 7.70 (s, 1H), 7.64 (d, J = 7.4 Hz, 1H), 7.47 (d, J = 1.4 Hz, 1H), 7.20 (d, J = 5.1 Hz, 1H), 7.13 (s, 1H), 5.41 (s, 1H), 5.01 - 4.91 (m, 2H), 2.98 (s, 3H), 2.61 - 2.59 (m, 4H), 2.41 (s, 3H), 2.24 (s, 3H), 1.55 - 1.46 (m, 5H), 1.19 (t, J = 7.5 Hz, 3H). |
| **430** | 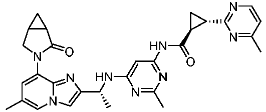 (1S,2S)-N-(2-methyl-6-(((1R)-1-(6-methyl-8-(2-oxo-3-azabicyclo[3.1.0] hexan-3-yl)imidazo[1,2-a]pyridin-2-yl) ethyl)amino)pyrimidin-4-yl)-2-(4-methyl pyrimidin-2-yl)cyclopropane-1-carboxamide (**I-430**) | **Intermediate 832**ESI-MS [M +H]⁺: 538.3, δ 10.65 (s, 1H), 8.52 (d, J = 5.1 Hz, 1H), 8.21 (s, 1H), 7.64 (s, 2H), 7.20 (d, J = 5.1 Hz, 1H), 7.11 (d, J = 9.5 Hz, 2H), 5.40 (s, 1H), 4.40-4.35 (m, 1H), 3.99 (d, J = 10.1 Hz, 1H), 2.65 - 2.53 (m, 2H), 2.41 (s, 3H), 2.23 (d, J = 10.1 Hz, 6H), 2.16 - 2.03 (m, 1H), 2.03-2.01 (m, 1H), 1.50-1.47 (m, 5H), 1.33 - 1.12 (m, 1H), 0.85-1.82 (m, 1H). |
| **431** | 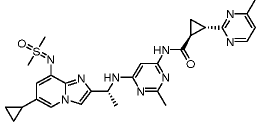 (1S,2S)-N-(6-(((R)-1-(6-cyclopropyl-8-((dimethyl(oxo)-16-sulfaneylidene) amino)imidazo[1,2-a]pyridin-2-yl) ethyl) amino)-2-methylpyrimidin-4-yl)-2-(4-methylpyrimidin-2-yl)cyclo propane-1-carboxamide (**I-431**) | **Intermediate 832** (1S,2S)-N-(6-chloro-2-methylpyrimidin-4-yl)-2-(4-methylpyrimidin-2-yl)cyclopropane-1-carboxamideESI-MS [M +H]⁺: 560.3, ESI-MS [M +H]⁺: 560.3, ¹H NMR (400 MHz, DMSO) δ 10.63 (s, 1H), 8.52 (d, J = 5.1 Hz, 1H), 7.85 (s, 1H), 7.60 (s, 1H), 7.48 (s, 1H), 7.20 (d, J = 5.1 Hz, 1H), 7.07 (s, 1H), 6.43 (s, 1H), 5.39 (s, 1H), 3.36 (s, 6H), 2.60 - 2.54 (m, 2H), 2.41 (s, 3H), 2.24 (s, 3H), 1.86 - 1.80 (m, 1H), 1.49 (t, J = 9.8 Hz, 5H), 0.88 - 0.81 (m, 2H), 0.59 (q, J = 5.4 Hz, 2H). |
| **432** | 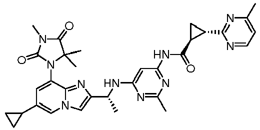 (1S,2S)-N-(6-(((R)-1-(6-cyclopropyl-8-(3,5,5-trimethyl-2,4-dioxo imidazolidin-1-yl)imidazo[1,2-a] pyridin-2-yl)ethyl) amino)-2-methyl pyrimidin-4-yl)-2-(4-methyl pyrimidin-2-yl)cyclopropane-1-carboxamide (**I-432**) | **Intermediate 832** ESI-MS [M + H]⁺: 609.3, δ 10.64 (s, 1H), 8.52 (d, *J* = 5.1 Hz, 1H), 8.40 (d, *J =* 1.2 Hz, 1H), 7.68 - 7.59 (m, 2H), 7.20 (d, *J =* 5.1 Hz, 1H), 7.10 (s, 1H), 7.01 (d, *J =* 1.4 Hz, 1H), 5.47 - 5.27 (m, 1H), 3.00 (s, 3H), 2.63 - 2.51 (m, 2H), 2.41 (s, 3H), 2.23 (s, 3H), 1.98 - 1.91 (m, 1H), 1.57 - 1.43 (m, 5H), 1.38 - 1.35 (m, 6H), 0.95 - 0.90 (m, 2H), 0.71 - 0.64 (m, 2H). |
| **433** | 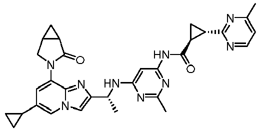 (1S,2S)-N-(6-(((1R)-1-(6-cyclo propyl-8-(2-oxo-3-azabicyclo [3.1.0]hexan-3-yl)imidazo[1,2-a] pyridin-2-yl)ethyl) amino)-2-methylpyrimidin-4-yl)-2-(4-methylpyrimidin-2-yl)cyclopropane-1-carboxamide (**I-433**) | **Intermediate 832** ESI-MS [M +H]⁺: 564.2. δ 10.65 (s, 1H), 8.52 (d, J = 5.1 Hz, 1H), 8.21 (d, J = 1.1 Hz, 1H), 7.70 - 7.47 (m, 2H), 7.20 (d, J = 5.2 Hz, 1H), 7.12 (s, 1H), 7.02 (s, 1H), 5.38 (s, 1H), 4.43-4.33 (m, 1H), 3.98 (d, J = 10.1 Hz, 1H), 2.65 - 2.53 (m, 2H), 2.42 (s, 3H), 2.24 (s, 3H), 2.12-2.07 (m, 1H), 2.05-1.98 (m, 1H), 1.93-1.84 (m, 1H), 1.55-1.45(m, 5H), 1.29 - 1.08 (m, 1H), 0.93-0.83 (m, 3H),0.65-0.58 (m, 2H). |
| **434** | 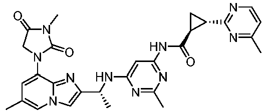 (1S,25)-N-(2-methyl-6-(((R)-1-(6-methyl-8-(3-methyl-2,4-dioxo imidazolidin-1-yl)imidazo[1,2-a]pyridin-2-yl)ethyl)amino)pyrimidin-4-yl)-2-(4-methylpyrimidin-2-yl)cyclopropane-1-carboxamide (**I-434**) | **Intermediate 832** |
| | | ESI-MS [M +H]⁺: 555.2, δ 10.65 (s, 1H), 8.52 (d, J = 5.1 Hz, 1H), 8.22 (s, 1H), 7.69 (s, 1H), 7.65 - 7.63 (m, 1H), 7.42 (s, 1H), 7.20 (d, J = 5.1 Hz, 1H), 7.13 (s, 1H), 5.43 (s, 1H), 5.01 - 4.91 (m, 2H), 2.98 (s, 3H), 2.61 - 2.57 (m, 1H), 2.55 - 2.53 (m, 1H), 2.41 (s, 3H), 2.26 - 2.25 (m, 6H), 1.51 - 1.46 (m, 5H). |
| **435** | 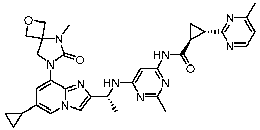 (1S,2S)-N-(6-(((R)-1-(6-cyclopropyl-8-(5-methyl-6-oxo-2-oxa-5,7-diazaspiro [3.4]octan-7-yl) imidazo[1,2-a]pyridin-2-yl)ethyl) amino)-2-methylpyrimidin-4-yl)-2-(4-methylpyrimidin-2-yl)cyclo propane-1-carboxamide (**I-435**) | **Intermediate 832** ESI-MS [M +H]+: 609.3, δ 10.65 (s, 1H), 8.52 (d, J=5.1, 1H), 8.12 (s, 1H), 7.59-7.52 (m, 2H), 7.23-7.18 (m, 2H), 7.13 (s, 1H), 5.41(s, 1H), 4.98 (d, J=7.4, 2H), 4.68 - 4.59 (m, 4H), 3.03 (s, 3H), 2.62 - 2.56 (m, 2H), 2.41 (s, 3H), 2.25 (s, 3H), 1.90-1.85 (m, 1H), 1.55-1.44 (m, 5H), 0.92-0.87 (m, 2H), 0.62-0.58 (m, 2H). |
| **436** | 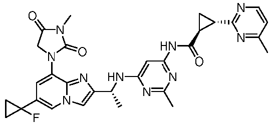 (1S,2S)-N-(6-(((R)-1-(6-(1-fluoro cyclo propyl)-8-(3-methyl-2,4-dioxo imidazolidin-1-yl)imidazo[1,2-a] pyridin-2-yl)ethyl)amino)-2-methyl pyrimidin-4-yl)-2-(4-methyl pyrimidin-2-yl)cyclo propane-1-carboxamide (**I-436**) | **Intermediate 832** ESI-MS [M +H]+: 599.2, δ 10.66 (s, 1H), 8.54 - 8.51 (m, 2H), 7.76 (d, J = 2.6 Hz, 1H), 7.69 (d, J = 7.6 Hz, 1H), 7.60 (d, J = 1.5 Hz, 1H), 7.20 (d, J = 5.1 Hz, 1H), 7.13 (s, 1H), 5.44 (s, 1H), 5.03 - 4.93 (m, 2H), 2.98 (s, 3H), 2.64 - 2.54 (m, 2H), 2.41 (s, 3H), 2.25 (s, 3H), 1.52 - 1.43 (m, 7H), 1.14 - 1.09 (m, 2H). |
| **437** | 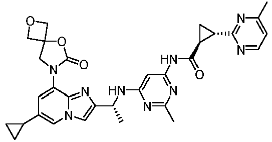 (1S,2S)-N-(6-(((R)-1-(6-cyclopropyl-8-(6-oxo-2,5-dioxa-7-azaspiro [3.4]octan-7-yl)imidazo[1,2-a] pyridin-2-yl)ethyl) amino)-2-methylpyrimidin-4-yl)-2-(4-methyl pyrimidin-2-yl)cyclopropane-1-carboxamide (**I-437**) | **Intermediate 832** ESI-MS [M +H]⁺: 596.2, δ 10.67 (s, 1H), 8.52 (d, J = 5.1 Hz, 1H), 8.25 (s, 1H), 7.66 (s, 2H), 7.23 - 7.13 (m, 3H), 5.48 - 5.33 (m, 1H), 4.86 - 4.75 (m, 6H), 2.61 - 2.57 (m, 2H), 2.41 (s, 3H), 2.25 (s, 3H), 1.94 - 1.91 (m, 1H), 1.52 - 1.47 (m, 5H), 0.93 - 0.91 (m, 2H), 0.64 - 0.61 (m, 2H). |
| **438** | 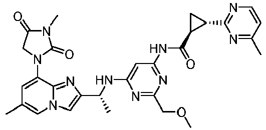 (1S,2S)-N-(2-(methoxymethyl)-6-(((R)-1-(6-methyl-8-(3-methyl-2,4-dioxo imidazolidin-1-yl)imidazo[1,2-a]pyridin-2-yl)ethyl)amino)pyrimidin-4-yl)-2-(4-methylpyrimidin-2-yl)cyclopropane-1-carboxamide (**I-438**) | **Intermediate 832**ESI-MS [M +H]⁺: 585.2 δ 10.77 (s, 1H), 8.52 (d, J = 5.1 Hz, 1H), 8.22 (s, 1H), 7.77 (d, J = 8.1 Hz, 1H), 7.71 (s, 1H), 7.42 (d, J = 1.2 Hz, 1H), 7.20 (d, J = 5.0 Hz, 2H), 5.43 (s, 1H), 4.96 (d, J = 1.4 Hz, 2H), 4.21 (s, 2H), 3.30 (s, 3H), 2.98 (s, 3H), 2.64 - 2.59 (m, 1H), 2.56 - 2.52 (m, 1H), 2.41 (s, 3H), 2.26 (s, 3H), 1.52 - 1.48 (m, 5H). |
| **439** | 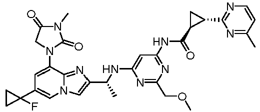 (1S,2S)-N-(6-(((R)-1-(6-(1-fluoro cyclo propyl)-8-(3-methyl-2,4-dioxo imidazolidin-1-yl)imidazo[1,2-a] pyridin-2-yl)ethyl)amino)-2-(methoxymethyl) pyrimidin-4-yl)-2-(4-methylpyrimidin-2-yl)cyclo propane-1-carboxamide (**I-439**) | **Intermediate 832** ESI-MS [M +H]+: 629.2, δ 10.79 (s, 1H), 8.54 - 8.51 (m, 2H), 7.82 (t, J = 6.9 Hz, 1H), 7.77 (s, 1H), 7.60 (d, J = 1.1 Hz, 1H), 7.20 (d, J = 5.1 Hz, 2H), 5.44 (s, 1H), 5.03 - 4.93 (m, 2H), 4.21 (s, 2H), 3.29 (s, 3H), 2.98 (s, 3H), 2.65 - 2.59 (m, 1H), 2.58 - 2.54 (m, 1H), 2.41 (s, 3H), 1.55 - 1.42 (m, 7H), 1.14 -1.09 (m,2H). |
| **440** | 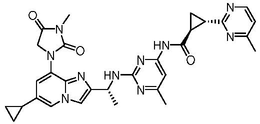 (1S,2S)-N-(2-(((R)-1-(6-cyclopropyl-8-(3-methyl-2,4-dioxoimidazolidin-1-yl)imidazo[1,2-a]pyridin-2-yl)ethyl) amino)-6-methylpyrimidin-4-yl)-2-(4-methylpyrimidin-2-yl) cyclopropane-1-carboxamide (**I-440**) | (1S,2S)-N-(2-chloro-6-methylpyrimidin-4-yl)-2-(4-methylpyrimidin-2-yl)cyclopropane-1-carboxamide ESI-MS [M +H]+: 581.3, |
| | | δ 10.57 (s, 1H), 8.51 (d, J = 5.1 Hz, 1H), 8.21 (s, 1H), 7.65 (s, 1H), 7.31 (d, J = 1.3 Hz, 1H), 7.19 (d, J = 5.1 Hz, 1H), 7.15 (s, 1H), 5.30 - 5.27 (m, 1H), 4.91 (s, 2H), 2.96 (s, 3H), 2.67 - 2.62 (m, 1H), 2.52 - 2.52 (m, 1H), 2.40 (s, 3H), 2.21 (s, 3H), 1.93 - 1.89 (m, 1H), 1.55 - 1.47 (m, 5H), 0.94 - 0.89 (m, 2H), 0.64 - 0.60 (m, 2H). |
| **441** | 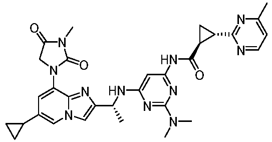 (1S,2S)-N-(6-(((R)-1-(6-cyclopropyl-8-(3-methyl-2,4-dioxoimidazolidin-1-yl)imidazo[1,2-a]pyridin-2-yl)ethyl) amino)-2-(dimethylamino) pyrimidin-4-yl)-2-(4-methyl pyrimidin-2-yl)cyclo propane-1-carboxamide (**I-441**) | (1S,2S)-N-(6-chloro-2-(dimethylamino) pyrimidin-4-yl)-2-(4-methylpyrimidin-2-yl) cyclopropane-1-carboxamide |
| | | ESI-MS [M +H]⁺: 610.3, δ 10.13 (s, 1H), 8.52 (d, J = 5.1 Hz, 1H), 8.24 (s, 1H), 7.64 (s, 1H), 7.32 - 7.19 (m, 3H), 6.60 (s, 1H), 5.36 - 5.29 (m, 1H), 4.93 (s, 2H), 2.98 - 2.97 (m, 9H), 2.65 - 2.59 (m, 2H), 2.41 (s, 3H), 1.95 - 1.91 (m, 1H), 1.50 - 1.45 (m, 5H), 0.95 - 0.91 (m, 2H), 0.65 - 0.62 (m, 2H). |
| **442** | 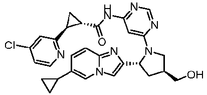 (1S,2S)-2-(4-chloropyridin-2-yl)-N-(6-((2R,4S)-2-(6-cyclopropyl imidazo[1,2-a]pyridin-2-yl)-4-(hydroxymethyl) pyrrolidin-1-yl) pyrimidin-4-yl)cyclo propane-1-carboxamide (**I-442**) | **Intermediate 836** ESI-MS [M + H ]⁺: 530.2, δ 10.61 (s, 1H), 8.37 (d, J = 5.1 Hz, 1H), 8.21 (s, 2H), 7.57 (s, 1H), 7.43 (s, 1H), 7.35 - 7.29 (m, 3H), 6.92 (d, J = 9.2 Hz, 1H), 5.39 - 5.18 (m, 1H), 5.05 - 4.80 (m, 1H), 4.62 (s, 1H), 4.17 - 3.93 (m, 1H), 3.64 - 3.33 (m, 3H), 2.70 - 2.51 (m, 2H), 2.37 - 2.21 (m, 1H), 2.03 - 1.97 (m, 1H), 1.90 - 1.83 (m, 1H), 1.51 - 1.35 (m, 2H), 0.87 - 0.85 (m, 2H), 0.62 - 0.58 (m, 2H). |

### Examples 443-445

### (1S,2S)-N-(6-(((R)-1-(6-cyclopropy1-8-((1R*,5S*)-2-oxo-3-azabicyclo[3.1.0]hexan-3-yl)imidazo[1,2-a]pyridin-2-yl)ethyl)amino)pyrimidin-4-yl)-2-(4-methylpyrimidin-2-yl)cyclopropane-1-carboxamide (I-443)

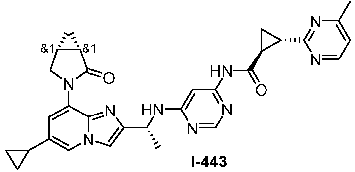

(**I-443**) was prepared in a similar manner to (**I-353**) and obtained as a mixture of diastereomers. ESI-MS [M +H]+: 550.2,

The mixture was separated using chiral column separation [CHIRALPAK IA, 0.46 cm I.D × 15 cm L MEOH/ACN=80/20, 1 mL/min, 35°C) to give two diastereomers: (1S,2S)-N-(6-(((R)-1-(6-cyclopropyl-8-((1S,5R)-2-oxo-3-azabicyclo[3.1.0]hexan-3-yl)imidazo[1,2-a]pyridin-2-yl)ethyl) amino)pyrimidin-4-yl)-2-(4-methylpyrimidin-2-yl)cyclopropane-1-carboxamide and (1S,2S)-N-(6-(((R)-1-(6-cyclopropyl-8-((1R,5S)-2-oxo-3-azabicyclo[3.1.0]hexan-3-yl)imidazo[1,2-a]pyridin-2-yl)ethyl)amino)pyrimidin-4-yl)-2-(4-methylpyrimidin-2-yl)cyclopropane-1-carboxamide.
First eluting isomer (**I-444**): ESI-MS: [M + H]+, 550.3. δ 10.65 (s, 1H), 8.52 (d, J = 5.1 Hz, 1H), 8.20 (d, J = 1.2 Hz, 1H), 8.15 (d, J = 0.7 Hz, 1H), 7.77 (s, 1H), 7.59 (s, 1H), 7.30 (s, 1H), 7.21 (d, J = 5.1 Hz, 1H), 7.02 (d, J = 1.3 Hz, 1H), 5.32 (s, 1H), 4.39 - 4.35 (m, 1H), 3.97 (d, J = 10.5 Hz, 1H), 2.64 - 2.60 (m, 1H), 2.55 - 2.53 (m, 1H), 2.41 (s, 3H), 2.13 - 2.07 (m, 1H), 2.02 - 1.98 (m, 1H), 1.91 - 1.85 (m, 1H), 1.55 - 1.49 (m, 5H), 1.22 - 1.18 (m, 1H), 0.91 - 0.85 (m, 3H), 0.65 - 0.60 (m, 2H). RT = 3.885 min, 97.4% e.e.
Second eluting isomer (**I-445**): ESI-MS: [M + H]+, 550.3. ¹H NMR (400 MHz, DMSO) was substantially similar to (**I-444**). RT = 5.552 min, 99.3% e.e.

### Examples 446-448

### rac-(1S*,2S*)-N-(6-(((6-cyclopropyl-8-(3-methyl-2,4-dioxoimidazolidin-1-yl)imidazo[1,2-a]pyridin-2-yl)methyl)amino)pyrimidin-4-yl)-2-(4-methylthiazol-2-yl)cyclopropane-1-carboxamide (I-446)

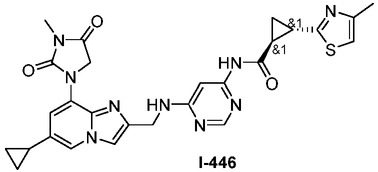

(**I-446**) was prepared in a similar manner to (**I-353**) and obtained as a mixture of enantiomers. ESI-MS (M+H)+: 558.1, δ 10.69 (s, 1H), 8.22 (s, 1H), 8.17 (s, 1H), 7.90 (s, 1H), 7.66 (s, 1H), ), 7.31 (s, 1H), 7.29 (s, 1H), 7.05 (s, 1H), 4.88 (s, 2H), 4.56 (s, 2H), 2.95 (s, 3H), 2.77 - 2.69 (m, 1H), 2.60 - 2.56 (m, 1H), 2.27 (s, 3H), 1.94 - 1.87 (m, 1H), 1.53 - 1.49 (m, 2H), 0.94 - 0.83 (m, 2H), 0.63 - 0.59 (m, 2H).

The mixture was separated using SFC (Daicel CHIRALPAK OZ-H 20 x 250 mm, 5.0 µm, 46/54 MeOH (0.2% NH₄OH) / CO₂, 45 g/min, 35°C) to give two enantiomers: (1S,2S)-N-(6-(((6-cyclopropyl-8-(3-methyl-2,4-dioxoimidazolidin-1-yl)imidazo[1,2-a]pyridin-2-yl)methyl)amino)pyrimidin-4-yl)-2-(4-methylthiazol-2-yl)cyclopropane-1-carboxamide and (1R,2R)-N-(6-(((6-cyclopropyl-8-(3-methyl-2,4-dioxoimidazolidin-1-yl)imidazo[1,2-a]pyridin-2-yl)methyl)amino)pyrimidin-4-yl)-2-(4-methylthiazol-2-yl)cyclopropane-1-carboxamide.

First eluting isomer (**I-447**): ESI-MS (M+H)+: 558.2, ¹ δ 10.68 (s, 1H), 8.22 (s, 1H), 8.17 (s, 1H), 7.87 (s, 1H), 7.67 (s, 1H), 7.31 (s, 1H), 7.29 (s, 1H), 7.05 (s, 1H), 4.89 (s, 2H), 4.55 (s, 2H), 2.95 (s, 3H), 2.74 - 2.70 (m, 1H), 2.61 - 2.57 (m, 1H), 2.28 (s, 3H), 1.93 - 1.88 (m, 1H), 1.54 - 1.47 (m, 2H), 0.94 - 0.89 (m, 2H), 0.64 - 0.60 (m, 2H). RT = 2.771 min, 100.0 % e.e.

Second eluting isomer (**I-448**): ESI-MS (M+H)+: 558.2, ¹H NMR (400 MHz, DMSO) substantially similar to (I-447)RT = 5.332 min, 100.0 % e.e.

### Examples 449-453

### rac-(1S*,2S*)-N-(6-((1-(6-cyclopropyl-8-(3-methyl-2,4-dioxoimidazolidin-1-yl)imidazo[1,2-a]pyridin-2-yl)ethyl)amino)pyrimidin-4-yl)-2-(4-methylpyrimidin-2-yl)cyclopropane-1-carboxamide (I-449)

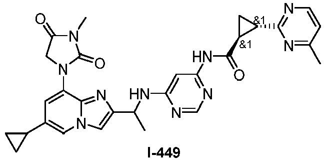

(**I-449**) was prepared in a similar manner to that described for (**I-353**) from **intermediate 834** and 1-(2-(1-aminoethyl)-6-cyclopropylimidazo[1,2-a]pyridin-8-yl)-3-methylimidazolidine-2,4-dione and obtained as a mixture of stereoisomers. ESI-MS (M + H)+: 567.2, ¹H NMR (400 MHz, DMSO) substantially similar to (I-450)

The mixture was separated using SFC (Daicel CHIRALPAK AS-H 250mm x 20 mm I.D. 5µm. CO2/MeOH (0.2% NH4OH ) = 52/48, 45 g/min, 35°C) to give two separated isomers and a mixture of another two isomers. The remaining mixture was further separated using SFC (Daicel CHIRALPAK OD-H 250mm x 20 mm I.D., 5µm. CO2/MeOH (0.2% NH4 ▪ OH) = 66/34, 50 g/min, 35°C): (1S*,2S*)-N-(6-(((R*)-1-(6-cyclopropyl-8-(3-methyl-2,4-dioxoimidazolidin-1-yl)imidazo[1,2-a]pyridin-2-yl)ethyl)amino)pyrimidin-4-yl)-2-(4-methylpyrimidin-2-yl)cyclopropane-1-carboxamide, isomer 1-4 (i.e., (1S,2S)-N-(6-(((R)-1-(6-cyclopropyl-8-(3-methyl-2,4-dioxoimidazolidin-1-yl)imidazo[1,2-a]pyridin-2-yl)ethyl)amino)pyrimidin-4-yl)-2-(4-methylpyrimidin-2-yl)cyclopropane-1-carboxamide, (1R,2R)-N-(6-(((R)-1-(6-cyclopropyl-8-(3-methyl-2,4-dioxoimidazolidin-1-yl)imidazo[1,2-a]pyridin-2-yl)ethyl)amino)pyrimidin-4-yl)-2-(4-methylpyrimidin-2-yl)cyclopropane-1-carboxamide, (1S,2S)-N-(6-(((S)-1-(6-cyclopropyl-8-(3-methyl-2,4-dioxoimidazolidin-1-yl)imidazo[1,2-a]pyridin-2-yl)ethyl)amino)pyrimidin-4-yl)-2-(4-methylpyrimidin-2-yl)cyclopropane-1-carboxamide, and (1R,2R)-N-(6-(((S)-1-(6-cyclopropyl-8-(3-methyl-2,4-dioxoimidazolidin-1-yl)imidazo[1,2-a]pyridin-2-yl)ethyl)amino)pyrimidin-4-yl)-2-(4-methylpyrimidin-2-yl)cyclopropane-1-carboxamide).
First eluting isomer (**I-450**): ESI-MS [M +H]+: 567.2, δ 10.65 (s, 1H), 8.52 (d, J = 5.1 Hz, 1H), 8.23 (s, 1H), 8.16 (s, 1H), 7.78 (s, 1H), 7.66 (s, 1H), 7.34 - 7.30 (m, 2H), 7.21 (d, J = 5.1 Hz, 1H), 5.33 - 5.31 (m, 1H), 4.94 (s, 2H), 2.98 (s, 3H), 2.64 - 2.59 (m, 1H), 2.57 - 2.54 (m, 1H), 2.41 (s, 3H), 1.94 - 1.91 (m, 1H), 1.54 - 1.49 (m, 5H), 0.95 - 0.92 (m, 2H), 0.65 - 0.62 (m, 2H). RT = 2.28 min, 100% e.e.
Second eluting isomer (**I-451**): (1S,2S)-N-(6-(((R)-1-(6-cyclopropyl-8-(3-methyl-2,4-dioxoimidazolidin-1-yl)imidazo[1,2-a]pyridin-2-yl)ethyl)amino)pyrimidin-4-yl)-2-(4-methylpyrimidin-2-yl)cyclopropane-1-carboxamide. ESI-MS [M +H]+: 567.2, δ 10.65 (s, 1H), 8.52 (d, J = 5.1 Hz, 1H), 8.22 (s, 1H), 8.16 (s, 1H), 7.77 (s, 1H), 7.65 (s, 1H), 7.34 - 7.30 (m, 2H), 7.21 (d, J = 5.1 Hz, 1H), 5.34 - 5.31 (m, 1H), 4.94 (s, 2H), 2.97 (s, 3H), 2.63 - 2.61 (m, 1H), 2.56 - 2.55 (m, 1H), 2.41 (s, 3H), 1.94 - 1.91(m, 1H), 1.53 - 1.50 (m, 5H), 0.94 - 0.92 (m, 2H), 0.64 - 0.61 (m, 2H). RT = 3.18 min, 100% e.e.
Third eluting isomer (**I-452**): (1S,2S)-N-(6-(((S)-1-(6-cyclopropyl-8-(3-methyl-2,4-dioxoimidazolidin-1-yl)imidazo[1,2-a]pyridin-2-yl)ethyl)amino)pyrimidin-4-yl)-2-(4-methylpyrimidin-2-yl)cyclopropane-1-carboxamide ESI-MS [M +H]+: 567.2, δ 10.65 (s, 1H), 8.52 (d, J = 5.1 Hz, 1H), 8.23 (s, 1H), 8.16 (s, 1H), 7.78 (s, 1H), 7.66 (s, 1H), 7.34 - 7.30 (m, 2H), 7.21 (d, J = 5.1 Hz, 1H), 5.33 - 5.31 (m, 1H), 4.94 (s, 2H), 2.98 (s, 3H), 2.63 - 2.60 (m, 1H), 2.57 - 2.55 (m, 1H), 2.41 (s, 3H), 1.95 - 1.90 (m, 1H), 1.54 - 1.49 (m, 5H), 0.95 - 0.91 (m, 2H), 0.65 - 0.63 (m, 2H). RT = 3.8 min, 100% e.e.
Fourth eluting isomer (**I-453**): ESI-MS [M +H]+: 567.2, δ 10.66 (s, 1H), 8.52 (d, J = 5.1 Hz, 1H), 8.22 (s, 1H), 8.15 (s, 1H), 7.78 (s, 1H), 7.65 (s, 1H), 7.34 - 7.30 (m, 2H), 7.21 (d, J = 5.2 Hz, 1H), 5.34 - 5.31 (m, 1H), 4.94 (s, 2H), 2.97 (s, 3H), 2.62 - 2.60 (m, 1H), 2.56 - 2.54 (m, 1H), 2.41 (s, 3H), 1.95 - 1.91 (m, 1H), 1.54 - 1.50 (m, 5H), 0.95 - 0.92 (m, 2H), 0.65 - 0.61 (m, 2H). RT = 5.16 min, 100% e.e.

### Example 454-456

### (1S,2S)-N-(6-((1-(6-cyclopropyl-8-(3-methyl-2,4-dioxoimidazolidin-1-yl)imidazo[1,2-a]pyridin-2-yl)ethyl)amino)-2-methylpyrimidin-4-yl)-2-(4-methylpyrimidin-2-yl)cyclopropane-1-carboxamide (I-454)

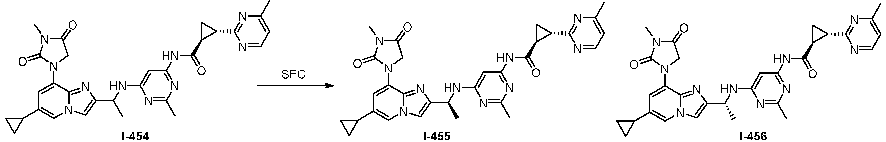

(I-454) was prepared in a similar manner to that described for (**I-353**) from *rac*-(1S*,2S*)-N-(6-chloro-2-methyl-pyrimidin-4-yl)-2-(4-methylpyrimidin-2-yl)cyclopropane-1-carboxamide and 1-(2-(1-aminoethyl)-6-cyclopropylimidazo[1,2-a]pyridin-8-yl)-3-methylimidazolidine-2,4-dione and obtained as a mixture of diastereomers. ESI-MS (M+H)+: 581.2, δ 10.65 (s, 1H), 8.51 (d, J = 5.1 Hz, 1H), 8.23 (s, 1H), 7.65 - 7.63 (m, 2H), 7.33 (s, 1H), 7.20 (d, J = 5.1 Hz, 1H), 7.12 (s, 1H), 5.38 (s, 1H), 4.95 - 4.92 (m, 2H), 2.97 (s, 3H), 2.60 - 2.57 (m, 2H), 2.40 (s, 3H), 2.24 (s, 3H), 1.95 - 1.91 (m, 1H), 1.50 - 1.48 (m, 5H), 0.95 - 0.92 (m, 2H), 0.64 - 0.63 (m, 2H).ESI-MS [M +H] +: 581.2.

The mixture was separated using SFC (Daicel CHIRALPAK IC-H 250mm x 20 mm I.D, 5µm, CO₂ /EtOH ((0.2% NH₄▪OH ) = 85/15, 40g/min, UV 214 nm, 35 °C) to give two diastereomers.
First eluting isomer (**I-455**): (1S,2S)-N-(6-(((S-((1-(6-cyclopropyl-8-(3-methyl-2,4-dioxoimidazolidin-1-yl)imidazo[1,2-a]pyridin-2-yl)ethyl)amino)-2-methylpyrimidin-4-yl)-2-(4-methylpyrimidin-2-yl)cyclopropane-1-carboxamide: ESI-MS (M+H)+:581.2, δ 10.66 (s, 1H), 8.52 (d, J = 5.1 Hz, 1H), 8.25 (s, 1H), 7.67 - 7.60 (m, 2H), 7.35 (s, 1H), 7.21 (d, J = 5.1 Hz, 1H), 7.13 (s, 1H), 5.40 (s, 1H), 4.96 (d, J = 4.1 Hz, 2H), 2.98 (s, 3H), 2.67 - 2.58 (m, 2H), 2.41 (s, 3H), 2.25 (s, 3H), 1.96 - 1.92 (m, 1H), 1.55 - 1.47 (m, 5H), 0.97 - 0.92 (m, 2H), 0.66 - 0.63 (m, 2H). RT=5.439min, 100%, e.e;
Second eluting isomer (**I-456**): (1S,2S)-N-(6-((R-((1-(6-cyclopropyl-8-(3-methyl-2,4-dioxoimidazolidin-1-yl)imidazo[1,2-a]pyridin-2-yl)ethyl)amino)-2-methylpyrimidin-4-yl)-2-(4-methylpyrimidin-2-yl)cyclopropane-1-carboxamide ESI-MS (M+H)+:581.2, δ 10.66 (s, 1H), 8.53 (d, J = 5.1 Hz, 1H), 8.25 (d, J = 1.2 Hz, 1H), 7.67 - 7.63 (m, 2H), 7.35 (s, 1H), 7.22 (s, 1H), 7.14 (s, 1H), 5.41 (s, 1H), 4.96 (d, J = 2.7 Hz, 2H), 2.99 (s, 3H), 2.69 - 2.58 (m, 2H), 2.43 (s, 3H), 2.26 (s, 3H), 1.94 - 1.94 (m, 1H), 1.52 - 1.51 (m, 5H), 0.96 - 0.94 (m, 2H), 0.67 - 0.63 (m, 2H). RT=6.889min, 100% e.e.

### Examples 457-459

### rac-(1S,2S)-2-(3-chlorophenyl)-N(6(((6cyclopropyl8-((1R*5S*)-2-oxo-3-azabicyclo[3.1.0]hexan-3-yl)imidazo[1,2-a]pyridin-2-yl)methyl)amino)pyrimidin-4-yl)cyclopropane-1-carboxamide (I-457)

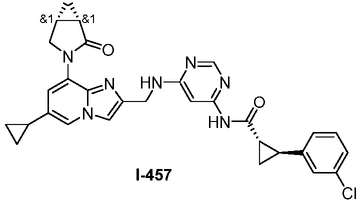

(**I-457**) ◆was prepared in a similar manner to (**I-353**) and obtained as a mixture of diastereomers. ESI-MS (M+H)+: 554.2, δ 10.60 (s, 1H), 8.20 (d, J = 8.7 Hz, 2H), 7.87 (s, 1H), 7.63 (s, 1H), 7.41 - 7.19 (m, 4H), 7.15 (d, J = 7.6 Hz, 1H), 7.03 (s, 1H), 4.55 (s, 2H), 4.35-4.29 (m, 1H), 3.98 (d, J = 10.3 Hz, 1H), 2.41-2.36 (m, 2H), 2.11-2.06 (m, 1H), 2.05-1.98(m, 1H), 1.91-1.87 (m, 1H), 1.50-1.38(m, 2H), 1.22-1.08 (m, 1H), 0.93-0.82 (m, 3H), 0.65-0.57 (m, 2H).

The mixture was separated using chiral column separation [CHIRALPAK IC, 2.5 cm I.D × 25 cm L MEOH=100, 30 mL/min, 35°C) to give two diastereomers: (1S,2S)-2-(3-chlorophenyl)-N-(6-(((6-cyclopropyl-8-((1R,5S)-2-oxo-3-azabicyclo[3.1.0]hexan-3-yl)imidazo[1,2-a]pyridin-2-yl)methyl)amino)pyrimidin-4-yl)cyclopropane-1-carboxamide and (1S,2S)-N-(5-((6-cyclopropyl-8-((1S,5R)-2-oxo-3-azabicyclo[3.1.0]hexan-3-yl)imidazo[1,2-a]pyridin-2-yl)methoxy)pyridazin-3-yl)-2-(4-methylpyrimidin-2-yl)cyclopropane-1-carboxamide
First eluting isomer (**I-458**) ESI-MS (M+H)+: 554.2, δ 10.61(s, 1H), 8.20 (d, J = 8.7 Hz, 2H), 7.89 (s, 1H), 7.63 (s, 1H), 7.41 - 7.23 (m, 4H), 7.15 (d, J = 7.6 Hz, 1H), 7.03 (s, 1H), 4.56 (s, 2H), 4.35-4.29 (m, 1H), 3.98 (d, J = 10.3 Hz, 1H), 2.32-2.39 (m, 2H), 2.12-2.06 (m, 1H), 2.05-1.98(m, 1H), 1.94-1.85 (m, 1H), 1.50-1.38(m, 2H), 1.23-1.17 (m, 1H), 0.92-0.83 (m, 3H), 0.64-0.62 (m, 2H). RT = 6.328 min, 100.0% e.e.
Second eluting isomer (**I-459**): ESI-MS (M+H)+: 554.2, δ 10.60 (s, 1H), 8.20 (d, J = 8.7 Hz, 2H), 7.87 (s, 1H), 7.68 (s, 1H), 7.41 - 7.19 (m, 4H), 7.15 (d, J = 7.6 Hz, 1H), 7.03 (s, 1H), 4.55 (s, 2H), 4.36-4.29 (m, 1H), 3.98 (d, J = 10.3 Hz, 1H), 2.41-2.36 (m, 2H), 2.11-2.06 (m, 1H), 2.05-1.98(m, 1H), 1.91-1.87 (m, 1H), 1.50-1.38(m, 2H), 1.22-1.10 (m, 1H), 0.93-0.82 (m, 3H), 0.65-0.57 (m, 2H). RT = 7.635 min, 100.0% e.e.

### Example 460: (1S,2S)-N-(2-cyclopropyl-6-((1-(6-cyclopropyl-8-(3-(2-hydroxyethyl)-2,4-dioxoimidazolidin-1-yl)imidazo[1,2-a]pyridin-2-yl)ethyl)amino)pyrimidin-4-yl)-2-(4-methylpyrimidin-2-yl)cyclopropane-1-carboxamide (I-460)

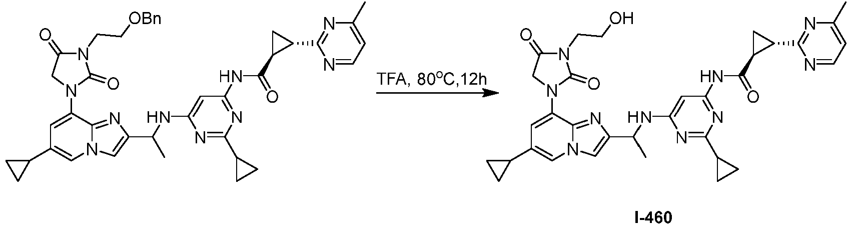

A solution of (1S,2S)-N-(6-((1-(8-(3-(2-(benzyloxy)ethyl)-2,4-dioxoimidazolidin-1-yl)-6-cyclopropylimidazo[1,2-a]pyridin-2-yl)ethyl)amino)-2-cyclopropylpyrimidin-4-yl)-2-(4-methylpyrimidin-2-yl)cyclopropane-1-carboxamide (40 mg, 0.06 mmol) in TFA (3 mL) was stirred at 80 °C for 12 h. The reaction mixture was concentrated *in vacuo,* diluted with NH₃/MeOH (7M, 2 mL) and stirred at room temperature for 10 min. Removal of the solvents gave the crude product, which was purified by preparative TLC (eluted with DCM: MeOH=20:1) to give (**I-460**) ◆as a white solid. (22 mg, 58%). ESI-MS (M+H)+: 637.3, δ 10.53 (s, 1H), 8.52 (d, J = 5.1 Hz, 1H), 8.24 (s, 1H), 7.69 - 7.55 (m, 2H), 7.33 (s, 1H), 7.20 (d, J = 5.1 Hz, 1H), 7.07 (s, 1H), 5.53 - 5.21 (m, 1H), 4.96 (s, 2H), 4.92 - 4.87 (m, 1H), 3.60 - 3.54 (m, J = 9.5 Hz, 4H), 2.63 - 2.60 (m, 1H), 2.55 - 2.53 (m, 1H), 2.41 (s, 3H), 1.97 - 1.91 (m, 1H), 1.82 - 1.75 (m, 1H), 1.53 - 1.45 (m, 5H), 0.96 - 0.92 (m, 2H), 0.91 - 0.84 (m, 2H), 0.82 - 0.78 (m, 2H), 0.66 - 0.60 (m, 2H).

The compounds in Table **34** were prepared in a similar manner to (**I-460**) from the corresponding benzyl esters or ethers, which were in turn prepared in a similar manner to (**I-353**).

**Table 34**

| **EG** | **Structure** | **Analytical Data** |
|---|---|---|
| **461** | 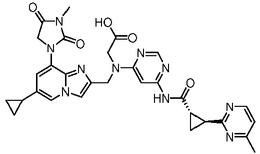 N-((6-cyclopropyl-8-(3-methyl-2,4-dioxo imidazolidin-1-yl)imidazo[1,2-a]pyridin-2-yl)methyl)-N-(6-((1S,2S)-2-(4-methyl pyrimidin-2-yl)cyclopropane-1-carbox amido)pyrimidin-4-yl)glycine (**I-461**) | ESI-MS (M+H)+: 611.2, δ 10.77 (s, 1H), 8.52 (d, J = 5.0 Hz, 1H), 8.25 (s, 2H), 7.87 - 7.60 (m, 2H), 7.36 (s, 1H), 7.20 (d, J = 5.0 Hz, 1H), 4.92 (s, 2H), 4.64 (s, 2H), 4.37 (s, 2H), 2.98 (s, 3H), 2.69 - 2.57 (m, 2H), 2.41 (s, 3H), 2.00 - 1.83 (m, 1H), 1.61 - 1.46 (m, 2H), 1.00 - 0.88 (m, 2H), 0.76 - 0.51 (m, 2H). |
| **462** | 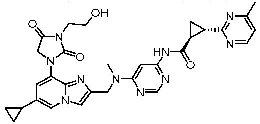 (1S,25)-N-(6-(((6-cyclopropyl-8-(3-(2-hydroxyethyl)-2,4-dioxoimidazolidin-1-yl)imidazo[1,2-a]pyridin-2-yl)methyl) (methyl)amino)pyrimidin-4-yl)-2-(4-methylpyrimidin-2-yl)cyclopropane-1-carboxamide (**I-462**) | ESI-MS (M+H)+: 597.2, δ 10.76 (s, 1H), 8.52 (d, J = 5.1 Hz, 1H), 8.26 (d, J = 0.6 Hz, 1H), 8.20 (s, 1H), 7.68 (s, 1H), 7.46 (s, 1H), 7.37 (d, J = 1.0 Hz, 1H), 7.20 (d, J = 5.1 Hz, 1H), 4.91 - 4.81 (m, 5H), 3.59 - 3.56 (m, 4H), 3.13 (s, 3H), 2.67 - 2.62 (m, 1H), 2.56 - 2.54 (m, 1H), 2.41 (s, 3H), 1.97 - 1.91 (m, 1H), 1.56 - 1.50 (m, 2H), 0.96 - 0.91 (m, 2H), 0.65 - 0.62 (m, 2H). |
| **463** | 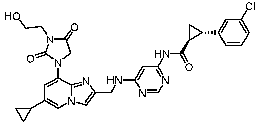 (1S,2S)-2-(3-chlorophenyl)-N-(6-(((6-cyclopropyl-8-(3-(2-hydroxyethyl)-2,4-dioxoimidazolidin-1-yl)imidazo[1,2-a] pyridin-2-yl)methyl)amino)pyrimidin-4-yl)cyclopropane-1-carboxamide (**I-463**) | ESI-MS (M+H)+: 601.2, ¹H NMR (400 MHz, MeOD) δ 8.17 (s, 2H), 7.71 (s, 1H), 7.36 (s, 1H), 7.32 - 7.23 (m, 2H), 7.22 - 7.16 (m,, 2H), 7.10 (d, *J* = 7.6 Hz, 1H), 4.69 (s, 2H), 4.64 (s, 2H), 3.83 - 3.70 (m, 4H), 2.51 - 2.45 (m, 1H), 2.21 - 2.15 (m, 1H), 1.98 - 1.93 (m, 1H), 1.64 - 1.57 (m, 1H), 1.41 - 1.35 (m, 1H), 1.02 - 0.95 (m, 2H), 0.78 - 0.70 (m, 2H). |
| **464** | 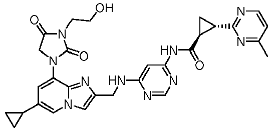 (1S,2S)-N-(6-(((6-cyclopropyl-8-(3-(2-hydroxyethyl)-2,4-dioxoimidazolidin-1-yl)imidazo[1,2-a]pyridin-2-yl)methyl) amino)pyrimidin-4-yl)-2-(4-methyl pyrimidin-2-yl)cyclopropane-1-carboxamide (**I-464**) | ESI-MS (M+H)+: 583.2, δ 10.67 (s, 1H), 8.52 (d, J = 5.1 Hz, 1H), 8.24 (d, J = 1.0 Hz, 1H), 8.18 (s, 1H), 7.89 (s, 1H), 7.69 (s, 1H), 7.31 (d, J = 10.6 Hz, 2H), 7.20 (d, J = 5.1 Hz, 1H), 4.93 (s, 2H), 4.89 (s, 1H), 4.57 (s, 2H), 3.61 - 3.53 (m, 4H), 2.64 - 2.59 (m, 1H), 2.53 - 2.51 (m, 1H), 2.41 (s, 3H), 1.97 - 1.90 (m, 1H), 1.55 - 1.47 (m, 2H), 0.96-0.91 (m,2H), 0.66-0.62 (m,2H). |

### Example 465: 2-(3-(6-cyclopropyl-2-(((6-((1S,2S)-2-(4-methylpyrimidin-2-yl)cyclopropane-1-carboxamido)pyrimidin-4-yl)amino)methyl)imidazo[1,2-a]pyridin-8-yl)-2,5-dioxoimidazolidin-1-yl)acetic acid. (I-465)

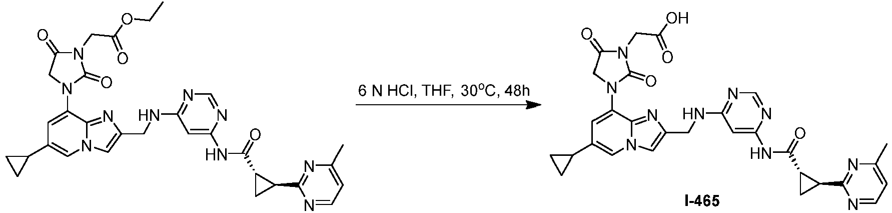

To a solution of ethyl 2-(3-(6-cyclopropyl-2-(((6-((1S,2S)-2-(4-methylpyrimidin-2-yl)cyclopropane-1-carboxamido)pyrimidin-4-yl)amino)methyl)imidazo[1,2-a]pyridin-8-yl)-2,5-dioxoimidazolidin-1-yl)acetate (30 mg, 0.05 mmol) in THF (1 mL) was added 6 N HCl (0.5 mL) at room temperature. The reaction mixture was stirred at 35 °C for 48 h and then concentrated *in vacuo* to provide crude product which was purified by preparative HPLC to afford (**I-465**) (2 mg, 7 % yield) as a white solid. ESI-MS (M+H)+: 597.2, δ 10.67 (s, 1H), 8.52 (d, J = 5.0 Hz, 1H), 8.24 (s, 1H), 8.19 (s, 1H), 7.93 (s, 1H), 7.69 (s, 1H), 7.30 (d, J = 5.9 Hz, 1H), 7.21 (d, J = 5.0 Hz, 1H), 6.69 (s, 1H), 4.97 (s, 2H), 4.59 (s, 2H), 3.86 (s, 2H), 2.59 (d, J = 14.8 Hz, 2H), 2.41 (s, 3H), 1.94 (s, 1H), 1.51 (s, 2H), 0.93 (d, J = 7.0 Hz, 2H), 0.64 (d, J = 5.5 Hz, 2H).

### Example 466: (3-(6-cyclopropyl-2-(((6-((1S,2S)-2-(4-methylpyrimidin-2-yl)cyclopropane-1-carboxamido)pyrimidin-4-yl)amino)methyl)imidazo[1,2-a]pyridin-8-yl)-2,5-dioxoimidazolidin-1-yl)methyl dihydrogen phosphate (I-466)

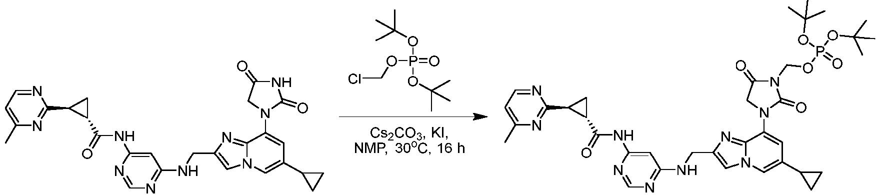

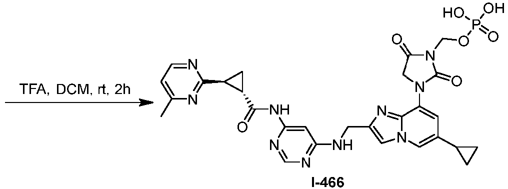

To a mixture of (1S,2S)-N-(6-(((6-cyclopropyl-8-(2,4-dioxoimidazolidin-1-yl)imidazo[1,2-a]pyridin-2-yl)methyl)amino)pyrimidin-4-yl)-2-(4-methylpyrimidin-2-yl)cyclopropane-1-carboxamide (220 mg, 0.41 mmol), KI (136 mg, 0.82 mmol), and Cs₂CO₃ (401 mg, 1.23 mmol) in NMP (5 mL) was added di-tert-butyl (chloromethyl) phosphate (159 mg, 0.62 mmol). The reaction was stirred at room temperature for 16 h, then diluted with water (20 mL). The mixture was extracted with EtOAc (3 x 20 mL). The combined organic layers were washed with brine, dried over Na₂SO₄, and concentrated in vacuo to give the crude, which was purified with preparative TLC (eluent: DCM/MeOH = 15:1) to give **di-tert-butyl ((3-(6-cyclopropyl-2-(((6-((1S,2S)-2-(4-methylpyrimidin-2-yl)cyclopropane-1-carboxamido)pyrimidin-4-yl)amino)methyl)imidazo [1,2-a]pyridin-8-yl)-2,5-dioxoimidazolidin-1-yl)methyl) phosphate intermediate 843** (50 mg, 16%) as yellow solid. ESI-MS [M +H]⁺: 761.3.

To a solution of **intermediate 843** (50 mg, 0.066 mol) in DCM (10 mL) was added TFA (1 mL). After stirring at room temperature for 2 h, the reaction was neutralized with sat. aqueous NaHCO₃ (10 mL) and extracted with DCM (3 x 20 mL). The combined organic layers were washed with brine, dried over Na₂SO₄, and concentrated in vacuo to give the crude, which was purified with preparative TLC (eluent: DCM/MeOH = 10:1) to afford (**I-466**) (21.8 mg, 51 %) as white solid. ESI-MS [M +H]+: 649.2 δ 10.94 (s, 1H), 8.54 - 8.51 (m, 2H), 8.28 (s, 2H), 7.94 (s, 1H), 7.59 (s, 1H), 7.30 (s, 1H), 7.22 (d, J = 5.1 Hz, 1H), 5.27 (d, J = 7.3 Hz, 2H), 4.82 (s, 2H), 4.69 (s, 2H), 2.64 - 2.61 (m, 1H), 2.58 - 2.54 (m, 1H), 2.42 (s, 3H), 2.07 - 2.00 (m, 1H), 1.58 - 1.51 (m, 2H), 1.04 - 1.00 (m, 2H), 0.76 - 0.72 (m, 2H).

### Example 467: 1-((((6-cyclopropyl-8-(3-methyl-2,4-dioxoimidazolidin-1-yl)imidazo[1,2-a]pyridin-2-yl)methyl)(6-((1S,2S)-2-(4-methylpyrimidin-2-yl)cyclopropane-1-carboxamido)pyrimidin-4-yl)carbamoyl)oxy)ethyl isobutyrate (I-467)

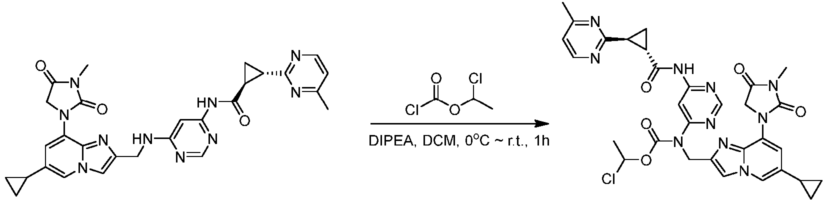

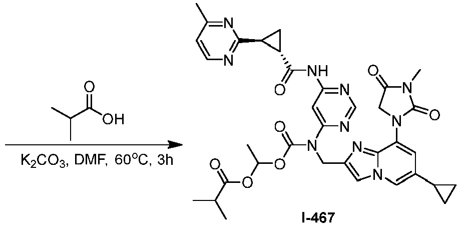

To a solution of (**I-260**) (200 mg, 0.36 mmol) and DIPEA (140 mg, 1.08 mmol) in DCM (20 mL) was added 1-chloroethyl carbonochloridate (102 mg, 0.72 mmol) at 0 °C. After stirring at 0 °C for 1 h, the reaction was concentrated in vacuo to give the crude product of **1-chloroethyl ((6-cyclopropyl-8-(3-methyl-2,4-dioxoimidazolidin-1-yl)imidazo[1,2-a]pyridin-2-yl)methyl)(6-((1S,2S)-2-(4-methylpyrimidin-2-yl)cyclopropane-1-carboxamido)pyrimidin-4-yl)carbamate intermediate 844** as a yellow solid. (236 mg, crude). ESI-MS [M +H]+: 659.2

A mixture of **intermediate 844** (118 mg, 0.18 mmol), DIPEA (70 mg, 0.54 mmol), and isobutyric acid (48 mg, 0.54 mmol) in DMF (6 mL) was stirred at 55 °C for 3 h. The reaction was concentrated in vacuo to give the crude product, which was purified by preparative HPLC to give (**I-467**) as a white solid (26 mg, 10%). ESI-MS (M+H)+: 711.3, δ 11.24 (s, 1H), 8.69 (s, 1H), 8.64 (s, 1H), 8.54 (d, J = 5.1 Hz, 1H), 8.19 (s, 1H), 7.61 (s, 1H), 7.34 (d, J = 1.2 Hz, 1H), 7.22 (d, J = 5.1 Hz, 1H), 6.87 - 6.75 (m, 1H), 5.30 (s, 2H), 4.81 (s, 2H), 2.96 (s, 3H), 2.73 - 2.65 (m, 1H), 2.61 - 2.52 (m, 2H), 2.42 (s, 3H), 1.98 - 1.84 (m, 1H), 1.64 - 1.51 (m, 2H), 1.47 (d, J = 5.3 Hz, 3H), 1.05 - 0.98 (m, 3H), 0.96 - 0.86 (m, 5H), 0.67 - 0.54 (m, 2H).

### Example 468: (1S,2S)-N-(6-(((R)-1-(6-cyclopropyl-8-(3-methyl-2,4-dioxoimidazolidin-1-yl) imidazo[1,2-a]pyridin-2-yl)ethyl)amino)-2-(2-hydroxyethyl)pyrimidin-4-yl)-2-(4-methylpyrimidin-2-yl)cyclopropane-1-carboxamide (I-468)

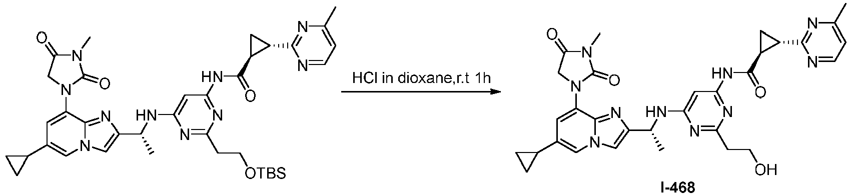

To a solution of (1S,2S)-N-(2-(2-((*tert*-butyldimethylsilyl)oxy)ethyl)-6-(((R)-1-(6-cyclopropyl-8-(3-methyl-2,4-dioxoimidazolidin-1-yl)imidazo[1,2-a]pyridin-2-yl)ethyl)amino) pyrimidin-4-yl)-2-(4-methylpyrimidin-2-yl)cyclopropane-1-carboxamide (50 mg, 0.07 mmol) in MeOH (3 mL) was added HCl (4M solution in dioxane, 3 mL). After stirring at room temperature for 1 h, the reaction mixture was concentrated *in vacuo* to give the crude product, which was purified by Pre-TLC (eluent:DCM/MeOH = 15/1 ) to give (**I-468**) as a white solid (20 mg, 47%). ESI-MS [M +H]+: 611.3, δ 10.64 (s, 1H), 8.52 (d, J = 5.1 Hz, 1H), 8.23 (d, J = 1.1 Hz, 1H), 7.67 (s, 2H), 7.33 (d, J = 1.2 Hz, 1H), 7.20 (d, J = 5.1 Hz, 1H), 7.13 (s, 1H), 5.36 (d, J = 29.4 Hz, 1H), 4.94 (d, J = 1.6 Hz, 2H), 4.63 - 4.55 (m, 1H), 3.79 - 3.66 (m, 2H), 2.97 (s, 3H), 2.73 - 2.56 (m, 4H), 2.41 (s, 3H), 1.97 - 1.88 (m, 1H), 1.59 - 1.43 (m, 5H), 1.00 - 0.86 (m, 2H), 0.70 - 0.57 (m, 2H).

The compounds in Table 35 were prepared in a similar manner to (**I-468**) from the corresponding silyl protected ethers, which were in turn prepared in a similar manner to (**I-353**).

**Table 35**

| **EG** | **Structure** | **Analytical Data** |
|---|---|---|
| **469** | 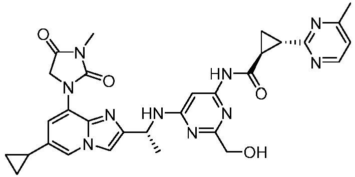 (1S,2S)-N-(6-(((R)-1-(6-cyclopropyl-8-(3-methyl-2,4-dioxoimidazolidin-1-yl)imidazo [1,2-a]pyridin-2-yl)ethyl)amino)-2-(hydroxy methyl)pyrimidin-4-yl)-2-(4-methylpyrimidin-2-yl)cyclopropane-1-carboxamide (**I-469**) | ESI-MS [M +H]⁺: 597.2, δ 10.74 (s, 1H), 8.52 (d, J = 5.1 Hz, 1H), 8.23 (s, 1H), 7.80 (s, 1H), 7.67 (s, 1H), 7.34 (s, 1H), 7.23 - 7.16 (m, 2H), 4.94 (s, 2H), 4.64 (s, 1H), 4.25 (d, J = 5.8 Hz, 2H), 2.97 (s, 3H), 2.62 (s, 2H), 2.41 (s, 3H), 1.97 - 1.90 (m, 1H), 1.51 (d, J = 6.4 Hz, 5H), 0.97 - 0.89 (m, 2H), 0.64-0.58 (m, 2H). |
| **470** | 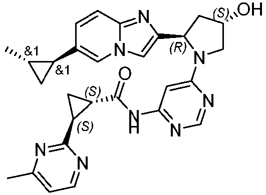 (1S,2S)-N-(6-((2R,4S)-4-hydroxy-2-(6-((1S*,2S*)-2-methylcyclopropyl)imidazo[1,2-a]pyridin-2-yl)pyrrolidin-1-yl)pyrimidin-4-yl)-2-(4-methylpyrimidin-2-yl)cyclopropane-1-carboxamide (**I-470**) | ESI-MS [M +H]+: 511.3, δ 10.77 - 10.63 (m, 1H), 8.51 (d, J = 4.9 Hz, 1H), 8.31 - 8.09 (m, 2H), 7.59 - 7.47 (m, 1H), 7.40 - 7.34 (m, 1H), 7.20 - 7.08 (m, 2H), 6.93 (d, J = 9.1 Hz, 1H), 5.28 - 4.98 (m, 2H), 4.57 - 4.46 (m, 1H), 3.82 - 3.66 (m, 2H), 2.62 - 2.54 (m, 2H), 2.41 (s, 3H), 2.33 - 2.30 (m, 2H), 1.61 - 1.44 (m, 3H), 1.23 - 1.22 (m, 1H), 1.14 (d, J = 5.9 Hz, 3H), 1.05 - 0.97 (m, 1H), 0.71 - 0.66 (m, 1H). |
| **471** | 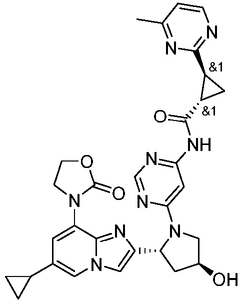 *rac-* (1S*,2S*)-N-(6-((2R,4S)-2-(6-cyclo propyl-8-(2-oxooxazolidin-3-yl)imidazo[1,2-a] pyridin-2-yl)-4-hydroxypyrrolidin-1-yl) pyrimidin-4-yl)-2-(4-methylpyrimidin-2-yl) cyclopropane-1-carboxamide (**I-471**) | ESI-MS: [M +H]+, 582.2, δ 10.79-10.64 (m, 1H), 8.51 (d, J = 4.8 Hz, 1H), 8.22 - 8.16 (m, 2H), 7.67 - 7.54 (m, 1H), 7.23 - 7.19 (m, 3H), 5.54 - 5.31 (m, 1H), 5.20 - 4.98 (m, 2H), 4.57 - 4.46 (m, 4H), 3.89 - 3.62 (m, 2H), 2.69 - 2.64 (m, 1H), 2.54 - 2.53 (m, 1H), 2.40 (s, 3H), 2.33 - 2.31 (m, 2H), 1.95 - 1.87 (m, 1H), 1.58 - 1.42 (m, 2H), 0.92 - 0.90 (m, 2H), 0.66 - 0.58 (m, 2H) |
| **472** | 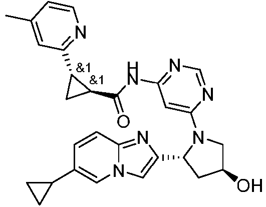 *rac-*(1S**,*2S*)-N-(6-((2R,4S)-2-(6-cyclo propylimidazo[1,2-a]pyridin-2-yl)-4-hydroxy pyrrolidin-1-yl)pyrimidin-4-yl)-2-(4-methyl pyridin-2-yl)cyclopropane-1-carboxamide (**I-472**) | ESI-MS [M +H]+: 496.2, δ 10.70 - 10.58 (m, 1H), 8.27 - 8.13 (m, 3H), 7.64 - 7.51 (m, 1H), 7.39 - 7.37 (m, 1H), 7.28 - 7.12 (m, 2H), 7.02 - 6.95 (m, 2H), 5.19 - 4.98 (m, 2H), 4.60 - 4.46 (m, 1H), 3.84 - 3.64 (m, 2H), 2.45 - 2.17 (m, 7H), 1.95 - 1.87 (m, 1H), 1.56 - 1.39 (s, 2H), 0.93 - 0.88 (m, 2H), 0.69 - 0.62 (m, 2H). |
| **473** | 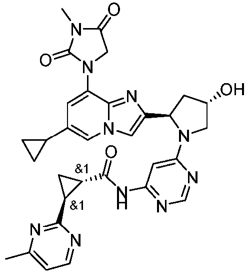 *rac-* (1S*,2S*)-N-(6-((2R,4S)-2-(6-cyclo propyl-8-(3-methyl-2,4-dioxo imidazolidin-1-yl)imidazo[1,2-a]pyridin-2-yl)-4-hydroxy pyrrolidin-1-yl)pyrimidin-4-yl)-2-(4-methyl pyrimidin-2-yl)cyclopropane-1-carboxamide (**I-473**) | ESI-MS: [M +H]+, 609.2, δ 11.01 (s, 1H), 8.53 (d, J = 5.0 Hz, 1H), 8.43 - 8.19 (m, 2H), 7.95 - 7.90 (m, 1H), 7.62 (s, 1H), 7.22 - 7.16 (m, 2H), 5.51 - 5.15 (m, 2H), 4.83 - 4.66 (m, 2H), 4.53 - 4.52 (m, 1H), 3.88 - 3.84 (m, 1H), 2.99 - 2.96 (m, 3H), 2.67 - 2.56 (m, 2H), 2.41 (s, 4H), 2.33 - 2.27 (m, 1H), 2.07 - 1.99 (m, 1H), 1.60 - 1.49 (m, 2H), 1.05 - 0.96 (m, 2H), 0.78 - 0.69 (m, 2H). |
| **474** | 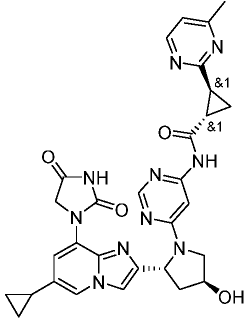 *rac-* (1S*,2S*)-N-(6-((2R,4S)-2-(6-cyclo propyl-8-(2,4-dioxoimidazolidin-1-yl) imidazo [1,2-a]pyridin-2-yl)-4-hydroxy pyrrolidin-1-yl)pyrimidin-4-yl)-2-(4-methyl pyrimidin-2-yl)cyclopropane-1-carboxamide (**I-474**) | ESI-MS: [M +H]+, 595.2 δ 11.30 (s, 1H), 10.75-10.60 (m, 1H), 8.49 (d, J = 5.0 Hz, 1H), 8.20 - 8.12 (m, 2H), 7.68 - 7.54 (m, 1H), 7.32 (s, 1H), 7.22 - 7.17 (m, 2H), 5.44- 5.09 (m, 2H), 4.93 - 4.85 (m, 2H), 4.56 - 4.50 (m, 1H), 3.77- 3.70 (m, 2H), 2.64 - 2.53 (m, 1H), 2.39 (s, 3H), 2.30 - 2.17 (m, 3H), 1.93 - 1.88 (m, 1H), 1.50 - 1.45 (m, 2H), 0.91 - 0.89 (m, 2H), 0.65 - 0.56 (m, 2H). |
| **475** | 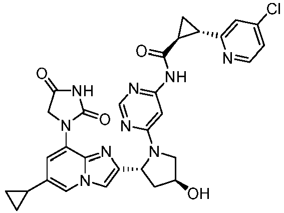 (1S,2S)-2-(4-chloropyridin-2-yl)-N-(6-((2R,4S)-2-(6-cyclopropyl-8-(2,4-dioxo imidazolidin-1-yl)imidazo[1,2-a]pyridin-2-yl)-4-hydroxypyrrolidin-1-yl)pyrimidin-4-yl) cyclopropane-1-carboxamide (**I-475**) | ESI-MS: [M +H]+, 614.1 δ 11.37 (s, 1H), 10.75 - 10.61 (m, 1H), 8.41 (d, J = 5.2 Hz, 1H), 8.22- 8.14 (m, 2H), 7.69 - 7.56 (m, 2H), 7.35 - 7.25 (m, 3H), 5.48 (s, 1H), 5.22 - 5.12 (m, 1H), 4.91 - 4.86(m, 2H), 4.63 -4.51 (m, 1H), 3.80 - 3.72 (m, 2H), 2.67- 2.59 (m, 2H), 2.33 - 2.21 (m, 2H), 1.97 - 1.88 (m, 1H), 1.59 - 1.38 (m, 2H), 0.92 - 0.90 (m 2H), 0.63 - 0.62 (m, 2H). |
| **476** | 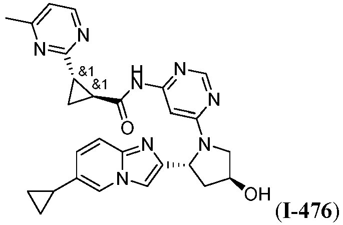 *rac-* (1S*,2S*)-N-(6-((2R,4S)-2-(6-cyclo propylimidazo[1,2-a]pyridin-2-yl)-4-hydroxy pyrrolidin-1-yl)pyrimidin-4-yl)-2-(4-methyl pyrimidin-2-yl)cyclopropane-1-carboxamide | ESI-MS [M +H]+: 497.2, ¹H NMR (400 MHz, cd3od) δ 8.45 (t, J = 5.2 Hz, 1H), 8.27 (s, 1H), 8.16 (s, 1H), 7.77 (s, 1H), 7.47 (d, J = 8.7 Hz, 1H), 7.28 (s, 2H), 7.16 (t, J = 4.6 Hz, 1H), 5.26 (s, 1H), 4.62 - 4.60 (m, 1H), 3.99 - 3.80 (m, 2H), 2.67 - 2.61 (m, 1H), 2.47 (s, 3H), 2.46 (s, 2H), 2.42 - 2.36 (m, 1H), 2.03 - 1.96 (m, 1H), 1.68 - 1.59 (m, 2H), 1.04 - 0.99 (m, 2H), 0.78 - 0.73 (m, 2H). |
| **477** | 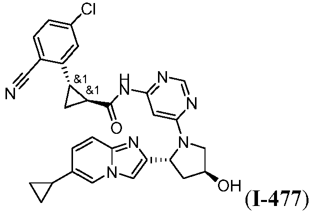 *rac-*(1S*,2S*)-2-(5-chloro-2-cyanophenyl)-N-(6-((2R,4S)-2-(6-cyclopropylimidazo[1,2-a] pyridin-2-yl)-4-hydroxypyrrolidin-1-yl) pyrimidin-4-yl)cyclopropane-1-carboxamide | ESI-MS [M +H] ⁺: 540.1, ¹H NMR (400 MHz, MeOD) δ 8.21 - 8.12 (m, 2H), 7.68 (t, J = 8.0 Hz, 2H), 7.43 - 7.40 (m, 1H), 7.35 - 7.28 (m, 3H), 7.06 - 7.02 (m, 1H), 5.54 - 5.15 (m, 1H), 4.60 (d, J = 3.5 Hz, 1H), 3.97 (s, 2H), 2.74 - 2.65 (m, 1H), 2.44 (d, J = 5.0 Hz, 2H), 2.24 (s, 1H), 1.95 - 1.88 (m, 1H), 1.68 - 1.62 (m, 1H), 1.48 (s, 1H), 0.98-0.90 (m,2H), 0.70-0.66 (m,2H). |
| **478** | 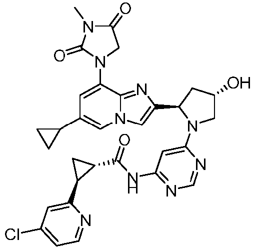 (1S,2S)-2-(4-chloropyridin-2-yl)-N-(6-((2R,4S)-2-(6-cyclopropyl-8-(3-methyl-2,4-dioxoimidazolidin-1-yl)imidazo[1,2-a]pyridin-2-yl)-4-hydroxypyrrolidin-1-yl)pyrimidin-4-yl)cyclopropane-1-carboxamide (**I-478**) | ESI-MS [M +H]+: 628.2, ¹H NMR (400 MHz, cd3od) δ 8.33 (d, J = 5.4 Hz, 1H), 8.15 (s, 1H), 8.11 (s, 1H), 7.69 (s, 1H), 7.47 (d, J = 1.8 Hz, 1H), 7.29 - 7.24 (m, 3H), 5.12 (s, 1H), 4.94 - 4.89 (m, 1H), 4.65 - 4.61 (m, 2H), 3.97 - 3.81 (m, 2H), 3.10 (s, 3H), 2.62 - 2.57 (m, 1H), 2.51 - 2.38 (m, 3H), 1.97 - 1.90 (m, 1H), 1.55 (s, 2H), 0.99 - 0.94 (m, 2H), 0.74 - 0.70 (m, 2H). |
| **479** | 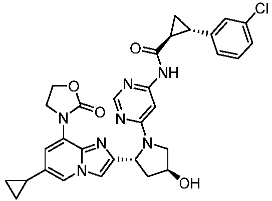 (1S,2S)-2-(3-chlorophenyl)-N-(6-((2R,4S)-2-(6-cyclopropyl-8-(2-oxooxazolidin-3-yl) imidazo [1,2-a]pyridin-2-yl)-4-hydroxy pyrrolidin-1-yl)pyrimidin-4-yl)cyclopropane-1-carboxamide (**I-479**) | ESI-MS [M +H]+: 600.2, ¹H NMR (400 MHz, cd3od) δ 8.36 - 8.11 (m, 2H), 7.68 (s, 1H), 7.27 - 7.07 (m, 6H), 5.16 (s, 1H), 4.62 - 4.56 (m, 3H), 4.39 (s, 1H), 4.28-4.18 (m, 1H), 4.06 - 3.80 (m, 2H), 2.46 (s, 3H), 2.24 - 2.03 (m, 1H), 1.97 - 1.91 (m, 1H), 1.51 (s, 1H), 1.32 (s, 1H), 0.99 - 0.94 (m, 2H), 0.74 - 0.70 (m, 2H). |
| **480** | 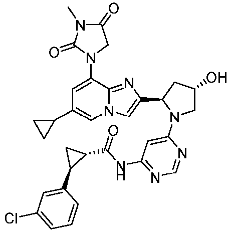 (1S,2S)-2-(3-chlorophenyl)-N-(6-((2R,4S)-2-(6-cyclopropyl-8-(3-methyl-2,4-dioxo imidazolidin-1-yl)imidazo[1,2-a]pyridin-2-yl)-4-hydroxypyrrolidin-1-yl)pyrimidin-4-yl) cyclopropane-1-carboxamide (**I-480**) | ESI-MS [M +H] ⁺: 627.1, ¹H NMR (400 MHz, MeOD) δ 8.14 (d, J = 19.9 Hz, 2H), 7.69 (s, 1H), 7.29 - 7.23 (m, 3H), 7.18 (d, J = 9.5 Hz, 2H), 7.09 (d, J = 7.5 Hz, 1H), 5.12 (s, 1H), 4.95 (s, 1H), 4.66 - 4.59 (m, 2H), 3.95 (s, 2H), 3.10 (s, 3H), 2.45 (d, J = 5.5 Hz, 3H), 2.12 (s, 1H), 1.97 - 1.91 (m, 1H), 1.51 (s, 1H), 1.38 - 1.31 (m, 1H), 1.00 - 0.95 (m, 2H), 0.74 - 0.72 (m, 2H). |
| **481** | 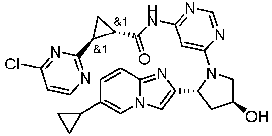 *rac-*(1S*,2S*)-2-(4-chloropyrimidin-2-yl)-N-(6-((2R,4S)-2-(6-cyclopropylimidazo[1,2-a] pyridin-2-yl)-4-hydroxypyrrolidin-1-yl) pyrimidin-4-yl)cyclopropane-1-carboxamide (**I-481**) | ESI-MS [M +H]+: 517.2, ¹H NMR (400 MHz, MeOD) δ 8.55-8.47 (m, 1H), 8.14 (s, 2H), 7.65 (s, 1H), 7.40-7.30 (m, 2H), 7.25 (s, 1H), 7.05 (d, J=8.1, 1H), 5.13 (s, 1H), 4.88 (s, 1H), 4.63-4.57 (m, 1H), 4.05-3.85 (m, 1H), 2.50 - 2.38 (m, 3H), 2.21 - 1.99 (m, 1H), 1.95-1.90 (m, 1H), 1.69 - 1.53 (m, 2H), 0.99 - 0.92 (m, 2H), 0.75 - 0.66 (m, 2H). |

### Example 482: rac-(1S*,2S*)-N-(6-((2R,4S)-2-(6-cyclopropyl-8-(2-oxo-3-azabicyclo[3.1.0]hexan-3-yl)imidazo[1,2-a]pyridin-2-yl)-4-hydroxypyrrolidin-1-yl)pyrimidin-4-yl)-2-(4-methylpyrimidin-2-yl)cyclopropane-1-carboxamide (I-482)

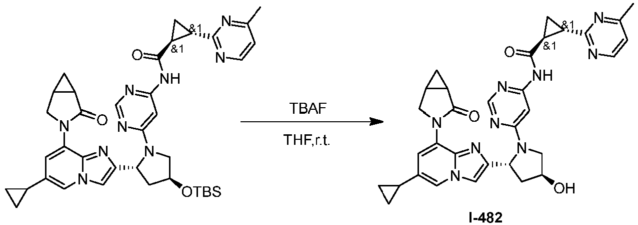

rac-(1S*,2S*)-N-(6-((2R,4S)-4-((tert-butyldimethylsilyl)oxy)-2-(6-cyclopropyl-8-(2-oxo-3-azabicyclo[3.1.0]hexan-3-yl)imidazo[1,2-a]pyridin-2-yl)pyrrolidin-1-yl)pyrimidin-4-yl)-2-(4-methylpyrimidin-2-yl)cyclopropane-1-carboxamide) (100 mg,0.14 mmol) and TBAF (74 mg,0.28 mmol) in THF (5 mL) was stirred at room temperature for 2 h. The reaction was then quenched with water (20 mL) and extracted with DCM (3 x 25 mL). The combined organic layer was concentrated *in vacuo* to give the crude product, which was purified by preparative HPLC to afford (**I-482**) (10 mg,12 %) as a white solid. ESI-MS [M +H]+: 592.3, ¹H NMR (400 MHz, MeOD) δ 8.46 (d, J = 4.9 Hz, 1H), 8.17 (s, 1H), 8.09 (s, 1H), 7.65 (s, 1H), 7.25 (s, 1H), 7.16 (d, J = 4.5 Hz, 1H), 7.01 (d, J = 12.8 Hz, 1H), 5.18 (s, 1H), 4.64 (s, 1H), 4.36 (s, 1H), 4.13 (d, J = 40.8 Hz, 1H), 3.96 (s, 2H), 2.67 (s, 1H), 2.46 (d, J = 3.7 Hz, 5H), 2.08 (s, 1H), 2.06 (s, 1H), 1.92 (d, J = 5.2 Hz, 1H), 1.63 (s, 2H), 1.29 (s, 2H), 1.13 (d, J = 33.5 Hz, 1H), 0.95 (d, J = 6.8 Hz, 2H), 0.71 (s, 2H).

The compounds in Table **36** were prepared in a similar manner to (**I-468**) from the corresponding silyl protected ethers (or acetylene for (1S,2S)-N-(6-((2R,4S)-2-(6-cyclopropyl imidazo[1,2-a]pyridin-2-yl)-4-hydroxypyrrolidin-1-yl)pyrimidin-4-yl)-2-(3-ethynylphenyl)cyclo propane-1-carboxamide), which were in turn prepared in a similar manner to (**I-353**). **Table 36**

| **EG** | **Structure** | **Analytical Data** |
|---|---|---|
| **483** | 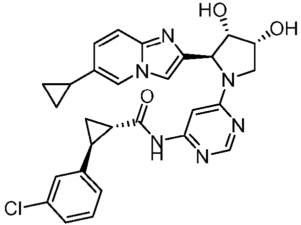 (1S,2S)-2-(3-chlorophenyl)-N-(6-((2S,3 S,4R)-2-(6-cyclopropyl imidazo[1,2-a]pyridin-2-yl)-3,4-di hydroxypyrrolidin-1-yl)pyrimidin-4-yl) cyclopropane-1-carboxamide (**I-483**) | ESI-MS [M +H]⁺: 531.2, ¹H NMR (400 MHz, CDCl₃) δ 9.73 (s, 1H), 8.21-8.13 (m, 2H), 7.79 (s, 1H), 7.44 (d, J = 9.2 Hz, 1H), 7.33 (s, 1H),7.23 - 7.14 (m, 2H), 7.11 (s, 1H), 7.03 - 6.93 (m, 2H), 5.55 (s, 1H), 5.05 (s, 1H), 4.64 (s, 2H), 4.00 (s, 2H), 3.54 (s, 1H), 2.55 (s, 1H), 1.96-1.80 (m, 1H), 1.67 (s, 1H), 1.34 (s, 1H), 1.02 - 0.87 (m, 2H), 0.69-0.59 (m, 2H). |
| **484** | 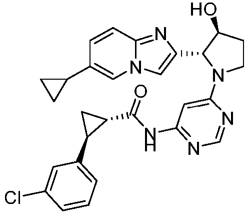 (1S,2S)-2-(3-chlorophenyl)-N-(6-((2R,3S)-2-(6-cyclopropylimidazo[1,2-a] pyridin-2-yl)-3-hydroxypyrrolidin-1-yl) pyrimidin-4-yl)cyclopropane-1-carboxamide (**I-484**) | ESI-MS [M + H ]+: 515.2, δ 10.64 (d, J = 44.0 Hz, 1H), 8.27 - 8.10 (m, 2H), 7.52 - 7.18 (m, 6H), 7.08 - 6.97 (m, 2H), 5.37 - 5.25 (m, 2H), 4.35 (s, 1H), 3.84 - 3.71 (m, 1H), 3.59 - 3.46 (m, 1H), 2.38 - 2.28 (m, 2H), 2.17 - 2.05 (m, 1H), 1.89 - 1.79 (m, 2H), 1.51 - 1.34 (m, 2H), 0.89 - 0.87 (m, 2H), 0.66 - 0.58 (m, 2H). |
| **485** | 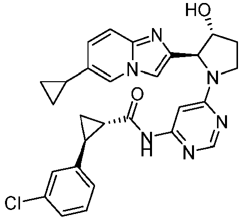 (1S,2S)-2-(3-chlorophenyl)-N-(6-((2S,3R)-2-(6-cyclopropylimidazo[1,2-a]pyridin-2-yl)-3-hydroxypyrrolidin-1-yl)pyrimidin-4-yl)cyclopropane-1-carboxamide (**I-485**) | ESI-MS [M +H] ⁺: 515.2, δ 10.66 (d, J = 45.6 Hz, 1H), 8.30 - 8.11 (m, 2H), 7.54 - 7.23 (m, 5H), 7.17 - 6.94(m, 3H), 5.39 - 5.27(m, 2H), 4.37 (d, J = 3.3 Hz, 1H), 3.86 - 3.72(m, 1H), 3.58 - 3.51 (m 1H), 2.44 - 2.30 (m, 2H), 2.13 - 2.08 (m, 1H), 1.94 - 1.83 (m, 2H), 1.52 - 1.33 (m, 2H), 0.90 - 0.85 (m, 2H), 0.64 (s, 2H). |
| **486** | 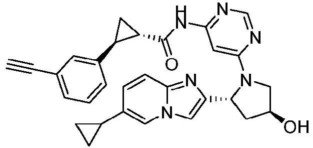 (1S,2S)-N-(6-((2R,4S)-2-(6-cyclo propylimidazo[1,2-a]pyridin-2-yl)-4-hydroxypyrrolidin-1-yl)pyrimidin-4-yl)-2-(3-ethynylphenyl)cyclopropane-1-carboxamide (**I-486**) | ESI-MS [M +H]+: 505.2, ¹H NMR (400 MHz, MeOD) δ 8.21-8.07 (m, 2H), 7.65 (s, 1H), 7.35 (d, J=9.2, 1H), 7.31 - 7.19 (m, 4H), 7.17 (s, 1H), 7.05 (d, J=7.7, 1H), 5.19 (s, 1H), 4.89 (s, 1H), 4.61-4.58 (m, 1H), 4.03 - 3.84(m, 1H), 3.46 (s, 1H), 2.51-2.38 (m, 3H), 2.12 (s, 1H), 1.95-1.89 (m, 1H), 1.50 (s, 1H), 1.34-1.28 (m, 1H), 0.99-0.91 (m, 2H), 0.74-0.66 (m, 2H). |

### Example 487: (1S,2S)-2-(4-chloropyridin-2-yl)-N-(6-6-cyclopropyl-8-(2,4-dioxoimidazolidin-1-yl)imidazo[1,2-a]pyridin-2-yl)methyl)amino)-2-hydroxypyrimidin-4-yl)cyclopropane-1-carboxamide (I-487)

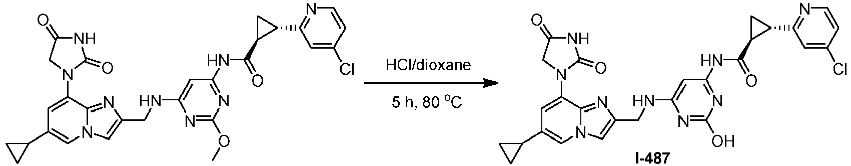

To a solution of (1S,2S)-2-(4-chloropyridin-2-yl)-N-(6-(((6-cyclopropyl-8-(2,4-dioxo imidazolidin-1-yl)imidazo[1,2-a]pyridin-2-yl)methyl)amino)-2-methoxypyrimidin-4-yl)cyclo propane-1-carboxamide (prepared in a similar manner to (**I-353**) (27 mg, 0.046 mmol) in dioxane (2 mL) was added HCl/dioxane (1 mL, 4 M, 8.0 mmol) at room temperature. The mixture was stirred at 80 °C for 5 h. After cooling to room temperature, the mixture was treated with a solution of ammonia in methanol (5 mL). The resulting solution was concentrated *in vacuo* to give the crude product, which was purified by preparative TLC (eluent: DCM/MeOH = 14/1) to give (**I-487**) (14 mg, 53%) as a white solid. ESI-MS [M +H]⁺: 574.1, δ 12.28 (s, 1H), 8.38 (d, J = 5.0 Hz, 1H), 8.13 (s, 1H), 7.78-7.60 (m, 3H), 7.34 (d, J = 1.1 Hz, 1H), 7.29 (dd, J = 5.3Hz, 1.7 Hz, 1H), 7.24 - 7.11 (m, 1H), 6.26 (s, 1H), 4.72 (s, 2H), 4.45 (s, 2H), 2.57 - 2.54 (m, 1H), 2.43 - 2.42 (m, 1H), 1.91 - 1.84 (m, 1H), 1.50 - 1.40 (m, 2H), 0.91 - 0.86 (m, 2H), 0.62 - 0.58 (m, 2H).

### Example 488: (1S,2S)-2-(3-chlorophenyl)-N-(4-(((6-cyclopropyl-8-(4-hydroxy-1-methylpiperidin-4-yl)imidazo[1,2-alpyridin-2-yl)methyl)amino)pyridin-2-yl)cyclopropane-1-carboxamide (I-488)

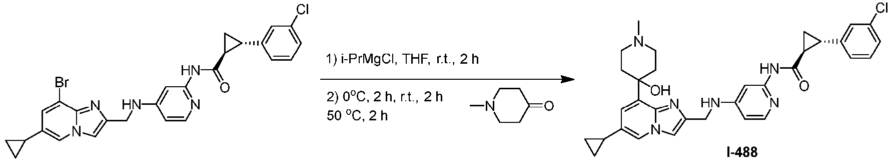

Isopropylmagnesium chloride (0.33 mL, 0.66 mmol) was added to a solution of **Intermediate 679** (45 mg, 0.084 mmol) in THF (1.5 mL) at 0 °C and then stirred at room temperature for 2 h under N₂. Then a solution of 1-methylpiperidin-4-one (95 mg, 0.84 mmol) in THF (0.5 mL) was added and the mixture was stirred at room temperature for 2 h and then stirred at 50 °C for additional 2 h. Water (10 mL) was added and the mixture was extracted with EtOAc (3 x 20 mL), the combined organic layers were concentrated *in vacuo* and purified by preparative TLC (DCM:MeOH = 10:1) to afford (**I-488**) (5 mg, 10%) as a white solid. ESI-MS [M +H]+: 571.3, ¹H NMR (400 MHz, MeOD) ? 8.03 (s, 1H), 7.75 (d, J = 5.9 Hz, 1H), 7.59 (s, 1H), 7.43 (s, 1H), 7.26 (t, J = 8.0 Hz, 1H), 7.20 - 7.18 (m, 2H), 7.10 (d, J = 7.6 Hz, 1H), 7.02 (d, J = 1.4 Hz, 1H), 6.41 (dd, J = 6.0, 2.2 Hz, 1H), 4.47 (s, 2H), 2.78 (d, J = 10.6 Hz, 2H), 2.65 (t, J = 11.2 Hz, 2H), 2.56 - 2.51 (m, 2H), 2.35 (s, 3H), 2.21 - 2.17 (m, 2H), 2.03 - 2.00 (m, 1H), 1.96 - 1.92 (m, 2H), 1.63 - 1.58 (m, 2H), 0.97 - 0.93 (m, 2H), 0.71 - 0.63 (m, 2H).

### Intermediate 845 rac-(1S*,2S*)-N-(6-chloropyrimidin-4-yl)-2-(5-fluoro-4-methylpyrimidin-2-yl) cyclopropane-1-carboxamide

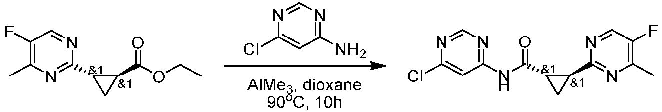

To a solution of **intermediate 846** (344 mg, 2.67 mmol) in dioxane (7 mL) was added AlMe₃ (1.6 M solution in toluene, 2.2 mL, 3.56 mmol) at 25 °C. After stirring at 30 °C for 2 h under N₂, a solution of *rac*-ethyl (1S*,2S*)-2-(5-fluoro-4-methylpyrimidin-2-yl)cyclopropane-1-carboxylate (200 mg, 0.89 mmol) in dioxane (3 mL) was added dropwise. The resulting reaction mixture was stirred at 90 °C for another 10 h. After cooling to room temperature, the reaction was quenched with water (30 mL) and extracted with EtOAc (3 x 40 mL). The combined organic layers were washed with brine, dried over anhydrous Na₂SO₄, and concentrated *in vacuo* to give the crude, which was purified by silica gel chromatography (eluent: DCM/MeOH = 20/1) to give **intermediate 845** (60 mg, 22%) as a yellow solid. ESI-MS [M +H]+: 308.1, δ 11.61 (s, 1H), 8.75 (d, J = 0.7 Hz, 1H), 8.63 (d, J = 1.7 Hz, 1H), 8.13 (d, J = 0.7 Hz, 1H), 2.74 - 2.63 (m, 2H), 2.44 (s, 3H), 1.63 - 1.53 (m, 2H).
The compounds in Table **37** were prepared in a similar fashion to **intermediate 845** from a substituted cyclopropyl ester and an appropriate heterocyclic amine. **Table 37**

| **Compound** | **Name/Analytical Data** |
|---|---|
| 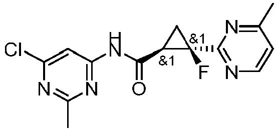 | ***rac*-(1S*,2S*)-N-(6-chloro-2-methylpyrimidin-4-yl)-2-fluoro-2-(4-methylpyrimidin-2-yl)cyclopropane-1-carboxamide intermediate 847** |
| | ESI-MS [M +H]+: 322.3 |
| 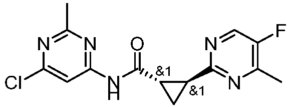 | ***rac*-(1S*,2S*)-N-(6-chloro-2-methylpyrimidin-4-yl)-2-(5-fluoro-4-methylpyrimidin-2-yl)cyclopropane-1-carboxamide intermediate 848** |
| | ESI-MS [M +H]⁺: 322.3 |
| 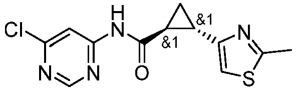 | ***rac*-(1S*,2S*)-N-(6-chloropyrimidin-4-yl)-2-(2-methylthiazol-4-yl)cyclopropane-1-carboxamide intermediate 849** ESI-MS [M +H]⁺: 295.2 |
| 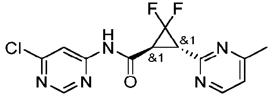 | ***rac*-(1S*,2S*)-N-(6-chloropyrimidin-4-yl)-2,2-difluoro-3-(4-methylpyrimidin-2-yl)cyclopropane-1-carboxamide** ESI-MS [M +H] +: 326.2 |
| 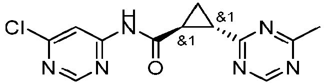 | ***rac*-(1S*,2S*)-N-(6-chloropyrimidin-4-yl)-2-(4-methyl-1,3,5-triazin-2-yl)cyclopropane-1-carboxamide intermediate 850** ESI-MS [M +H] +: 291.2 |
| 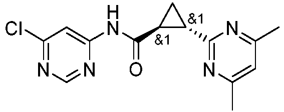 | ***rac-*(1S*,2S*)-N-(6-chloropyrimidin-4-yl)-2-(4,6-dimethylpyrimidin-2-yl)cyclopropane-1-carboxamide intermediate 851** ESI-MS [M +H]+: 304.2. |
| 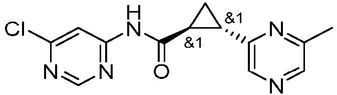 | ***rac*-(1S*,2S*)-N-(6-chloropyrimidin-4-yl)-2-(6-methylpyrazin-2-yl)cyclopropane-1-carboxamide intermediate 852** ESI-MS [M +H]⁺: 290.2. |
| 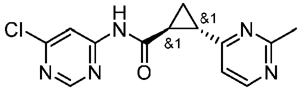 | ***rac*-(1S*,2S*)-N-(6-chloropyrimidin-4-yl)-2-(2-methylpyrimidin-4-yl)cyclopropane-1-carboxamide intermediate 853** ESI-MS [M +H] +: 290.2 |
| 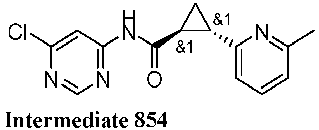 | ***rac*-(1S*,2S*)-N-(6-chloropyrimidin-4-yl)-2-(6-methylpyridin-2-yl)cyclopropane-1-carboxamide** ESI-MS [M +H]+: 289.3. δ 11.52 (s, 1H), 8.74 (s, 1H), 8.14 (s, 1H), 7.57 (t, J = 7.6 Hz, 1H), 7.22 (d, J = 7.6 Hz, 1H), 7.07 (d, J = 7.5 Hz, 1H), 2.63 - 2.57 (m, 2H), 2.41 (s, 3H), 1.60 - 1.56 (m, 1H), 1.53 - 1.48 (m, 1H). |
| 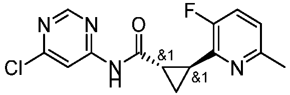 | ***rac*-(1S*,2S*)-N-(6-chloropyrimidin-4-yl)-2-(3-fluoro-6-methylpyridin-2-yl)cyclopropane-1-carboxamide intermediate 855** ESI-MS [M +H]⁺: 307.2 |
| 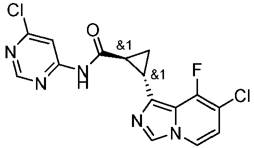 | ***rac*-(1S*,2S*)-2-(7-chloro-8-fluoroimidazo[1,5-a]pyridin-1-yl)-N-(6-chloropyrimidin-4-yl)cyclopropane-1-carboxamide intermediate 856** ESI-MS [M +H]⁺: 366.2 |
| 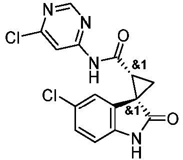 | ***rac*-(1R*,2S*)-5'-chloro-N-(6-chloropyrimidin-4-yl)-2'-oxospiro[cyclopropane-1,3'-indoline]-2-carboxamide intermediate 857** ESI-MS [M +H] +: 349.3 |
| 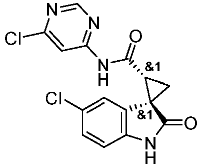 | ***rac*-(1R*,2R*)-5'-chloro-N-(6-chloropyrimidin-4-yl)-2'-oxospiro[cyclopropane-1,3'-indoline]-2-carboxamide intermediate 858** ESI-MS [M +H]⁺: 349.1 |
| 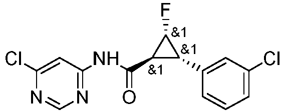 | ***rac*-(1R*,2R*,3S*)-2-(3-chlorophenyl)-N-(6-chloropyrimidin-4-yl)-3-fluorocyclopropane-1-carboxamide intermediate 859** ESI-MS [M +H]+: 326.1. |
| 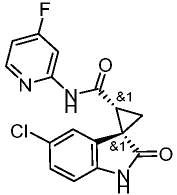 | ***rac*-(1R*,2S*)-5'-chloro-N-(4-fluoropyridin-2-yl)-2'-oxospiro[cyclopropane-1,3'-indoline]-2-carboxamide intermediate 860** ESI-MS [M +H]+: 332.1. |
| 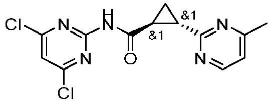 | ***rac*-(1S*,2S*)-N-(4,6-dichloropyrimidin-2-yl)-2-(4-methylpyrimidin-2-yl)cyclopropane-1-carboxamide** ESI-MS [M +H]⁺: 324.1. |
| 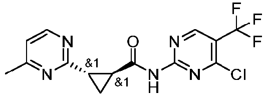 | ***rac*-(1S*,2S*)-N-(4-chloro-5-(trifluoromethyl)pyrimidin-2-yl)-2-(4-methylpyrimidin-2-yl)cyclopropane-1-carboxamide intermediate 861** see eg 628 ESI-MS [M +H]⁺: 358.1. |
| 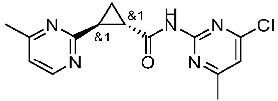 | ***rac*-(1S*,2S*)-N-(4-chloro-6-methylpyrimidin-2-yl)-2-(4-methylpyrimidin-2-yl)cyclopropane-1-carboxamide intermediate 862** ESI-MS [M +H]+: 304.1. |
| 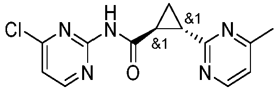 | ***rac*-(1S*,2S*)-N-(4-chloropyrimidin-2-yl)-2-(4-methylpyrimidin-2-yl)cyclopropane-1-carboxamide intermediate 863** ESI-MS [M +H]+: 290.1. |
| 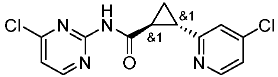 | ***rac*-(1S*,2S*)-2-(4-chloropyridin-2-yl)-N-(4-chloropyrimidin-2-yl)cyclopropane-1-carboxamide intermediate 864** ESI-MS [M +H]+: 309.1. |
| 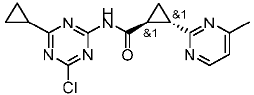 | ***rac*-(1S*,2S*)-N-(4-chloro-6-cyclopropyl-1,3,5-triazin-2-yl)-2-(4-methylpyrimidin-2-yl)cyclopropane-1-carboxamide intermediate 865** ESI-MS [M +H]+: 331.2. |
| 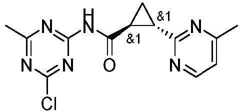 | ***rac*-(1S*,2S*)-N-(4-chloro-6-methyl-1,3,5-triazin-2-yl)-2-(4-methylpyrimidin-2-yl)cyclopropane-1-carboxamide intermediate 866** ESI-MS [M +H]+: 305.2. |
| 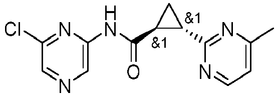 | ***rac*-(1S*,2S*)-N-(6-chloropyrazin-2-yl)-2-(4-methylpyrimidin-2-yl)cyclopropane-1-carboxamide intermediate 867** ESI-MS [M +H]+: 290.1. |
| 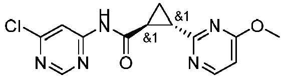 | ***rac*-(1R,2R)-N-(6-chloropyrimidin-4-yl)-2-(4-methoxypyrimidin-2-yl)cyclopropane-1-carboxamide intermediate 868** ESI-MS [M +H]+: 306.2 |
| 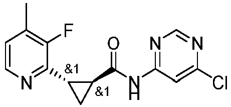 | ***rac*-(1S*,2S*)-N-(6-chloropyrimidin-4-yl)-2-(3-fluoro-4-methylpyridin-2-yl)cyclopropane-1-carboxamide** ESI-MS [M +H]+: 307.1. |
| 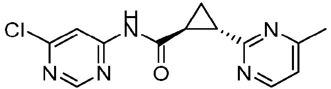 | **Intermediate 833** |
| | ESI-MS [M +H]+: 290.2. |
| 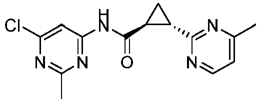 | **Intermediate 832** |
| | ESI-MS [M +H]+: 304.2. |
| 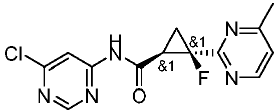 | ***rac*-(1S*,2S*)-N-(6-chloropyrimidin-4-yl)-2-fluoro-2-(4-methylpyrimidin-2-yl)cyclopropane-1-carboxamide intermediate 869** ESI-MS [M +H]⁺: 308.2 |
| 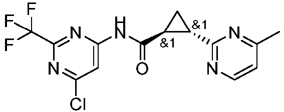 | ***rac*-(1S*,2S*)-N-(6-chloro-2-(trifluoromethyl)pyrimidin-4-yl)-2-(4-methylpyrimidin-2-yl)cyclopropane-1-carboxamide intermediate 870** ESI-MS [M +H]⁺: 358.3. |
| 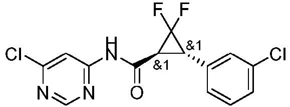 | ***rac*-(1S*,3S*)-3-(3-chlorophenyl)-N-(6-chloropyrimidin-4-yl)-2,2-difluorocyclopropane-1-carboxamide intermediate 871** ESI-MS [M +H] +: 344.2 |
| 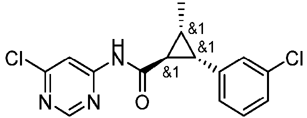 | ***rac-*(1R*,2R*,3S*)-2-(3-chlorophenyl)-N-(6-chloropyrimidin-4-yl)-3-methylcyclopropane-1-carboxamide intermediat 872** ESI-MS [M +H] +: 322.2 |
| 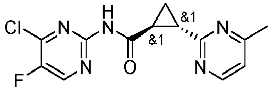 | ***rac*-(1S*,2S*)-N-(4-chloro-5-fluoropyrimidin-2-yl)-2-(4-methylpyrimidin-2-yl)cyclopropane-1-carboxamide intermediate 874** ESI-MS [M +H]+: 308.1. |
| 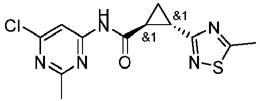 | ***rac*-(1S*,2S*)-N-(6-chloro-2-methylpyrimidin-4-yl)-2-(5-methyl-1,2,4-thiadiazol-3-yl)cyclopropane-1-carboxamide intermediate 875** ESI-MS [M +H]+: 310.2. |
| 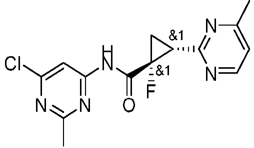 | ***rac*-(1S*,2S*)-N-(6-chloro-2-methylpyrimidin-4-yl)-1-fluoro-2-(4-methylpyrimidin-2-yl)cyclopropane-1-carboxamide intermediate 876** |
| | ESI-MS [M +H]+: 322.1. |

### Example 489: rac-(1S*,2S*)-N-(6-(((6-cyclopropyl-8-(3-methyl-2,4-dioxoimidazolidin-1-yl)imidazo[1,2-a]pyridin-2-yl)methyl)amino)pyrimidin-4-yl)-2-(5-fluoro-4-methylpyrimidin-2-yl)cyclopropane-1-carboxamide (I-489)

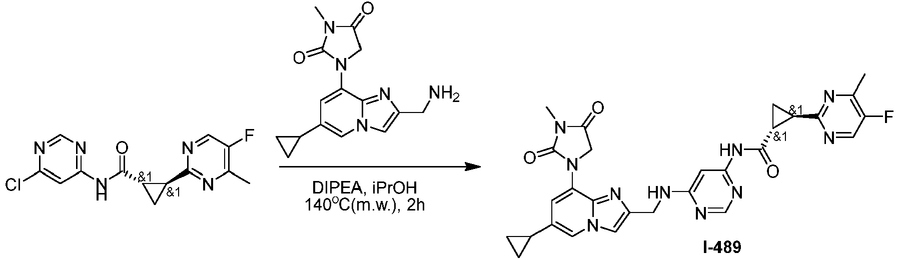

A mixture of **intermediate 845** (30 mg, 0.098 mmol), **intermediate 365** (29 mg, 0.098 mmol), and DIPEA (37 mg, 0.29 mmol) in i-PrOH (2 mL) was stirred in a sealed tube. After degassing with N₂ for 1 min, the reaction mixture was irradiated in a microwave reactor at 140 °C for 2 h. The reaction was then cooled to room temperature and concentrated *in vacuo* to give the crude, which was purified by preparative TLC (eluent: DCM/MeOH=15/1) to give (**I-489**) (20.1 mg, 36%) as a white solid. ESI-MS [M +H]+: 571.3., δ 10.69 (s, 1H), 8.62 (s, 1H), 8.22 (d, J = 22.4 Hz, 2H), 7.89 (s, 1H), 7.69 (s, 1H), 7.32 (d, J = 11.4 Hz, 2H), 4.91 (s, 2H), 4.58 (s, 2H), 2.97 (s, 3H), 2.67-2.66 (m, 1H), 2.46 - 2.45 (m, 1H), 2.43 (s, 3H), 1.93-1.92 (m, 1H), 1.50-1.49 (m, 2H), 0.94-0.93 (m, 2H), 0.64-0.63 (m, 2H). ESI-MS [M +H]+: 621.3.

The compounds in Table 38 were prepared in a similar manner to (**I-489**) from a suitable amine and the indicated coupling partner.

**Table 38**

| **EG** | **Structure** | **Coupling Partner / Analytical Data** |
|---|---|---|
| **490** | 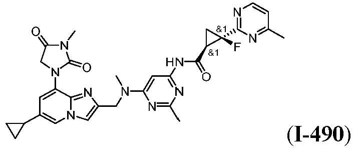 *rac*-(1S***,2S*)-N-(6-(((6-cyclopropyl-8-(3-methyl-2,4-dioxoimidazolidin-1-yl) imidazo[1,2-a]pyridin-2-yl)methyl) (methyl)amino)-2-methylpyrimidin-4-yl)-2-fluoro-2-(4-methylpyrimidin-2-yl)cyclopropane-1-carboxamide | **Intermediate 847** |
| | | ESI-MS (M+H)+: 599.2, δ 10.77 (s, 1H), 8.66 (d, J = 5.1 Hz, 1H), 8.21 (s, 1H), 7.67 (s, 1H), 7.36-7.31 (m, 3H), 4.91 - 4.82 (m, 4H), 3.12 (s, 3H), 2.97 (s, 3H), 2.91 (s, 1H), 2.48 (s, 3H), 2.29 (s, 3H), 2.22 - 2.16 (m, 1H), 1.97 - 1.85 (m, 2H), 0.96 - 0.91 (m, 2H), 0.66 - 0.62 (m, 2H). |
| **491** | 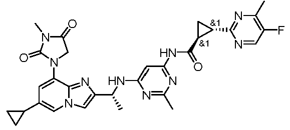 *rac*-((1S*2S*)-N-(6-((R)-1-(6-cyclo propyl-8-(3-methyl-2,4-dioxo imidazolidin-1-yl)imidazo[1,2-a]pyridin-2-yl)ethyl)amino)-2-methylpyrimidin-4-yl)-2-(5-fluoro-4-methylpyrimidin-2-yl)cyclopropane-1-carboxamide (**I-491**) | **Intermediate 848** |
| | | ESI-MS (M+H)+: 599.2, δ 10.66 (s, 1H), 8.61 (d, J = 1.5 Hz, 1H), 8.24 (s, 1H), 7.68 - 7.64 (m, 2H), 7.34 (d, J = 1.6 Hz, 1H), 7.12 (s, 1H), 5.40 (s, 1H), 4.95 (s, 2H), 2.98 (s, 3H), 2.51-2.49 (m, 2H), 2.43 (d, J = 2.2 Hz, 3H), 2.25 (s, 3H), 1.97 - 1.90 (m, 1H), 1.50-1.49 (m, 5H), 0.94-0.92 (m, 2H), 0.64-0.63 (m, 2H). |
| **492** | 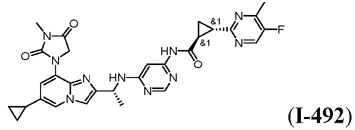 *rac-*((1S*,2S*)-N-(6-(((R)-1-(6-cyclopropyl-8-(3-methyl-2,4-dioxo imidazolidin-1-yl)imidazo[1,2-a]pyridin-2-yl)ethyl)amino)pyrimidin-4-yl)-2-(5-fluoro-4-methylpyrimidin-2-yl)cyclo propane-1-carboxamide | **Intermediate 845** ESI-MS (M+H)+: 585.2, δ 10.66 (s, 1H), 8.62 (d, J = 1.5 Hz, 1H), 8.25-8.16 (m, 2H), 7.78 (s, 1H), 7.65 (d, J = 2.1 Hz, 1H), 7.34-7.30 (m, 2H), 5.33 (s, 1H), 4.93 (s, 2H), 2.97 (s, 3H), 2.55-2.51 (m, 2H), 2.43 (s, 3H), 1.93-1.92 (m, 1H), 1.59 - 1.46 (m, 5H), 0.96-0.92 (m, 2H), 0.63-0.62 (m, 2H). |
| **493** | 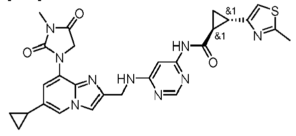 *rac*-(1S*,2S*)-N-(6-(((6-cyclopropyl-8-(3-methyl-2,4-dioxoimidazolidin-1-yl)imidazo[1,2-a]pyridin-2-yl)methyl) amino)pyrimidin-4-yl)-2-(2-methyl thiazol-4-yl)cyclopropane-1-carboxamide (**I-493**) | **Intermediate 849** ESI-MS (M+H)+: 558.2, δ 10.60 (s, 1H), 8.24 (s, 1H), 8.18 (s, 1H), 7.87 (s, 1H), 7.68 (s, 1H), 7.33 (s, 1H), 7.30 (s, 1H), 7.25 (s, 1H), 4.91 (s, 2H), 4.57 (s, 2H), 2.97 (s, 3H), 2.60 (s, 3H), 2.54 - 2.52 (m, 1H), 2.46 - 2.40 (m, 1H), 1.98 - 1.89 (m, 1H), 1.45 - 1.37 (m, 2H), 0.97 - 0.90 (m, 2H), 0.68 - 0.58 (m, 2H). |
| **494** | 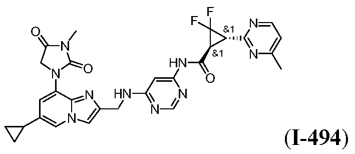 *rac-*(1S*,3S*)-N-(6-(((6-cyclopropyl-8-(3-methyl-2,4-dioxoimidazolidin-1-yl)imidazo[1,2-a]pyridin-2-yl)methyl) amino)pyrimidin-4-yl)-2,2-difluoro-3-(4-methylpyrimidin-2-yl)cyclopropane-1-carboxamide | *rac-*(1*S*,*3S*)-N-(6-chloropyrimidin-4-yl)-2,2-difluoro-3-(4-methylpyrimidin-2-yl)cyclo propane-1-carboxamide |
| | | ESI-MS (M+H)+: 589.2, δ 11.02 (s, 1H), 8.66 (d, J = 5.1 Hz, 1H), 8.24 (s, 2H), 7.97 (s, 1H), 7.70 (s, 1H), 7.37 (d, J = 5.1 Hz, 1H), 7.34 - 7.29 (m, 2H), 4.91 (s, 2H), 4.60 (s, 2H), 3.97 - 3.91 (m, 1H), 3.66 - 3.60 (m, 1H), 2.48 (s, 3H) 2.97 (s, 3H), 1.95 - 1.91 (m, 1H), 0.96 - 0.91 (m, 2H), 0.65 - 0.62 (m, 2H). |
| **495** | 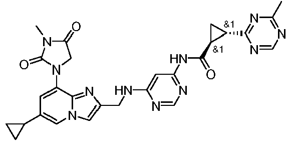 *rac*-(1S*,2S*)-N-(6-(((6-cyclopropyl-8-(3-methyl-2,4-dioxoimidazolidin-1-yl) imidazo[1,2-a]pyridin-2-yl)methyl) amino)pyrimidin-4-yl)-2-(4-methyl-1,3,5-triazin-2-yl)cyclopropane-1-carboxamide (**I-495**) | **Intermediate 850** |
| | | ESI-MS (M+H)+: 554.2, δ 10.74 (s, 1H), 9.00 (s, 1H), 8.24 (d, J = 1.0 Hz, 1H), 8.19 (s, 1H), 7.91 (s, 1H), 7.69 (s, 1H), 7.33 (d, J = 1.0 Hz, 1H), 7.30 (d, J = 0.6 Hz, 1H), 4.91 (s, 2H), 4.58 (s, 2H), 2.97 (s, 3H), 2.80 - 2.72 (m, 1H), 2.54 (s, 3H), 2.48 - 2.42 (m, 1H), 1.99-1.89 (m, 1H), 1.65 - 1.56 (m, 2H), 0.98-0.89 (m, 2H), 0.68 - 0.60 (m, 2H). |
| **496** | 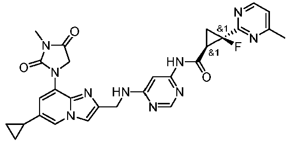 *rac*-(1S*,2S*)-N-(6-(((6-cyclopropyl-8-(3-methyl-2,4-dioxoimidazolidin-1-yl) imidazo [1,2-a]pyridin-2-yl)methyl) amino)pyrimidin-4-yl)-2-fluoro-2-(4-methylpyrimidin-2-yl)cyclo propane-1-carboxamide (**I-496**) | **Intermediate 869** ESI-MS (M+H)+: 571.2, δ 10.70 (s, 1H), 8.67 (d, J = 5.1 Hz, 1H), 8.28 - 8.16 (m, 2H), 7.93 (s, 1H), 7.69 (s, 1H), 7.36 - 7.33 (m, 3H), 4.91 (s, 2H), 4.59 (s, 2H), 2.97 (s, 3H), 2.92 (t, J = 7.3 Hz, 1H), 2.51 (s, 3H), 2.26 - 2.17 (m, 1H), 1.97 - 1.87 (m, 2H), 0.96 - 0.91 (m, 2H), 0.65 - 0.63 (m, 2H). |
| **497** | 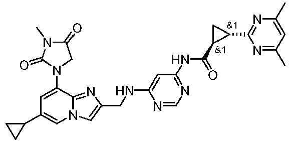 *rac*-(1S*,2S*)-N-(6-(((6-cyclopropyl-8-(3-methyl-2,4-dioxoimidazolidin-1-yl) imidazo [1,2-a]pyridin-2-yl)methyl) amino)pyrimidin-4-yl)-2-(4,6-dimethyl pyrimidin-2-yl) cyclo propane-1-carboxamide (**I-497**) | **Intermediate 851** ESI-MS (M+H)+: 567.2, δ 10.63 (s, 1H), 8.21 (d, J = 21.5 Hz, 2H), 7.86 (s, 1H), 7.69 (s, 1H), 7.32 (d, J = 11.0 Hz, 2H), 7.06 (s, 1H), 4.90 (d, J = 6.0 Hz, 2H), 4.57 (s, 2H), 2.97 (s, 3H), 2.60 - 2.57 (m, 1H), 2.47 - 2.44 (m, 1H), 2.35 (s, 6H), 1.96 - 1.90 (m, 1H), 1.54 - 1.45 (m,2H), 0.976- 0.91 (m,2H), 0.66 -0.62 (m,2H). |
| **498** | 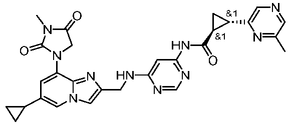 *rac*-(1S*,2S*)-N-(6-(((6-cyclopropyl-8-(3-methyl-2,4-dioxoimidazolidin-1-yl) imidazo[1,2-a]pyridin-2-yl) methyl) amino)pyrimidin-4-yl)-2-(6-methyl pyrazin-2-yl)cyclo propane-1-carboxamide (**I-498**) | **Intermediate 852** |
| | | ESI-MS (M+H)+: 553.3, δ 10.64 (s, 1H), 8.51 (s, 1H), 8.35 (s, 1H), 8.24 (s, 1H), 8.18 (s, 1H), 7.89 (s, 1H), 7.68 (s, 1H), 7.32 (d, J = 6.4 Hz, 2H), 4.91 (s, 2H), 4.57 (s, 2H), 2.97 (s, 3H), 2.64 - 2.55 (m, 2H), 2.43 (s, 3H), 1.97 - 1.90 (m, 1H), 1.52 - 1.48 (m, 2H), 0.96 - 0.91 (m, 2H), 0.66 - 0.62 (m, 2H). |
| **499** | 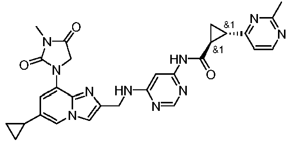 *rac*-(1S*,2S*)-N-(6-(((6-cyclopropyl-8-(3-methyl-2,4-dioxoimidazolidin-1-yl) imidazo[1,2-a]pyridin-2-yl)methyl) amino)pyrimidin-4-yl)-2-(2-methyl pyrimidin-4-yl)cyclopropane-1-carboxamide (**I-499**) | **Intermediate 853** ESI-MS (M+H)+: 553.2, δ 10.87 (s, 1H), 8.49 (d, J = 5.1 Hz, 1H), 8.37 (d, J = 2.5 Hz, 1H), 8.23 (s, 1H), 7.73 (s, 1H), 7.52 - 7.50 (m, 2H), 7.35 - 7.31 (m, 1H), 7.17 (d, J = 5.1 Hz, 1H), 4.90 (s, 2H), 4.38 (d, J = 5.5 Hz, 2H), 2.95 (s, 3H), 2.64-2.57 (m, 2H), 2.38 (s, 3H), 1.95 - 1.88 (m, J = 13.3 Hz, 1H), 1.50 - 1.44 (m, J = 13.1 Hz, 2H), 0.93 - 0.89 (m, 2H), 0.64 -0.60 (m, J = 4.7 Hz, 2H). |
| **500** | 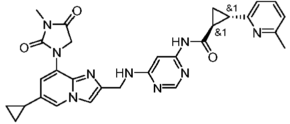 *rac*-(1S*,2S*)-N-(6-(((6-cyclopropyl-8-(3-methyl-2,4-dioxoimidazolidin-1-yl) imidazo[1,2-a]pyridin-2-yl)methyl) amino)pyrimidin-4-yl)-2-(6-methyl pyridin-2-yl)cyclopropane-1-carboxamide (**I-500**) | **Intermediate 854** ESI-MS (M+H)+: 552.3, δ 10.60 (s, 1H), 8.25 (s, 1H), 8.18 (s, 1H), 7.87 (s, 1H), 7.69 (s, 1H), 7.58 - 7.54 (m, 1H), 7.34 - 7.32 (m, 2H), 7.19 - 7.18 (m, 1H), 7.06 - 7.04 (m, 1H), 4.91 (s, 2H), 4.58 (s, 2H), 2.98 (s, 3H), 2.61 - 2.54 (m, 2H), 2.40 (s, 3H), 1.97 - 1.90 (m, 1H), 1.52 - 1.48 (m, 1H), 1.45 - 1.41 (m, 1H), 0.96 - 0.92 (m, 2H), 0.66 - 0.62 (m, 2H). |
| **501** | 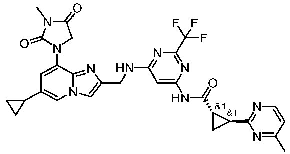 *rac*-(1S*,2S*)-N-(6-(((6-cyclopropyl-8-(3-methyl-2,4-dioxoimidazolidin-1-yl) imidazo[1,2-a]pyridin-2-yl)methyl) amino)-2-(trifluoromethyl )pyrimidin-4-yl)-2-(4-methylpyrimidin-2-yl)cyclo propane-1-carboxamide (**I-501**) | **Intermediate 870** ESI-MS (M+H)+: 621.3, δ 11.14 (s, 1H), 8.57 - 8.47 (m, 2H), 8.26 (s, 1H), 7.74 (s, 1H), 7.49 (s, 1H), 7.34 (s, 1H), 7.21 (d, J = 5.0 Hz, 1H), 4.93 (d, J = 17.6 Hz, 2H), 4.63-4.44 (m, 2H), 2.98 (s, 3H), 2.67-2.66 (m, 1H), 2.41 (s, 3H), 2.00 - 1.90 (m, 1H), 1.52-1.51 (m, 2H), 0.97 - 0.92 (m, 2H), 0.67 - 0.62 (m, 2H). |
| **502** | 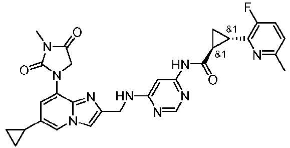 *rac*-(1S*,2S*)-N-(6-(((6-cyclopropyl-8-(3-methyl-2,4-dioxoimidazolidin-1-yl) imidazo[1,2-a]pyridin-2-yl)methyl) amino)pyrimidin-4-yl)-2-(3-fluoro-6-methylpyridin-2-yl)cyclopropane-1-carboxamide (**I-502**) | **Intermediate 855** ESI-MS (M+H)+: 570.2, δ 10.64 (s, 1H), 8.21 (d, J = 22.7 Hz, 2H), 7.87 (s, 1H), 7.69 (s, 1H), 7.59 - 7.48 (m, 1H), 7.33 (d, J = 8.8 Hz, 2H), 7.15-7.10 (m, 1H), 4.91 (s, 2H), 4.57 (s, 2H), 2.97 (s, 3H), 2.69-2.64 (m, 1H), 2.62-2.58 (m, 1H), 2.40 (s, 3H), 1.97-1.90 (m, 1H), 1.56-1.46 (m,2H), 1.00-0.86 (m,2H), 0.68-0.56 (m,2H). |
| **503** | 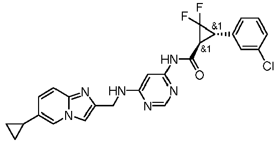 *rac*-(1S*,3S*)-3-(3-chlorophenyl)-N-(6-(((6-cyclopropylimidazo[1,2-a] pyridin-2-yl)methyl)amino)pyrimidin-4-yl)-2,2-di fluorocyclopropane-1-carboxamide (**I-503**) | **Intermediate 871** ESI-MS (M+H)+: 495.1, δ 10.79 (s, 1H), 8.30 (s, 1H), 8.24 (s, 1H), 7.96 (s, 1H), 7.62 (s, 1H), 7.46 - 7.40 (m, 3H), 7.37 (d, J = 9.3 Hz, 1H), 7.33 - 7.22 (m, 2H), 6.98-6.93(m, 1H), 4.59 (s, 2H), 3.71 - 3.63 (m, 1H), 3.55-3.47 (m, 1H), 1.95-1.86 (m, 1H), 0.95 - 0.87 (m, 2H), 0.69 - 0.62 (m, 2H). |
| **504** | 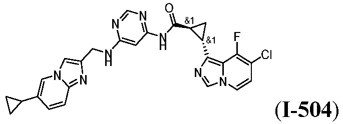 *rac-*(1S*,2S*)-2-(7-chloro-8-fluoro imidazo[1,5-a]pyridin-1-yl)-N-(6-(((6-cyclopropylimidazo[1,2-a] pyridin-2-yl)methyl)amino)pyrimidin-4-yl)cyclo propane-1-carboxamide | **Intermediate 856** ESI-MS (M+H)+: 517.1, δ 10.63 (s, 1H), 8.39 (d, J = 2.3 Hz, 1H), 8.30 (s, 1H), 8.23 - 8.12 (m, 2H), 7.85 (s, 1H), 7.61 (s, 1H), 7.37 (d, J = 9.3 Hz, 1H), 7.29 (s, 1H), 6.96 (dd, J = 9.3, 1.6 Hz, 1H), 6.76 - 6.69 (m, 1H), 4.57 (s, 2H), 2.67-2.66 (m, 1H), 2.49-2.47 (m, 1H), 1.94 - 1.87 (m, 1H), 1.55 - 1.45 (m, 2H), 0.92-0.89 (m, 2H), 0.67-0.65 (m, 2H). |
| **505** | 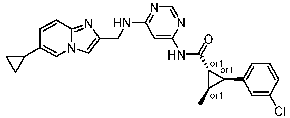 (1R*,2R*,3S*)-2-(3-chlorophenyl)-N-(6-(((6-cyclopropylimidazo[1,2-a] pyridin-2-yl)methyl)amino)pyrimidin-4-yl)-3-methylcyclopropane-1-carboxamide, isomer 2 (**I-505**) | **Intermediate 872**, isomer 1 - 4 |
| | | ESI-MS (M+H)+: 473.2, δ 10.62 (s, 1H), 8.30 (s, 1H), 8.22 (s, 1H), 7.86 (s, 1H), 7.61 (s, 1H), 7.39-7.34 (m, 3H), 7.33 - 7.21 (m, 3H), 6.98-6.93 (m, 1H), 4.57 (s, 2H), 2.62-2.56 (m, 1H), 2.42 (t, J = 4.5 Hz, 1H), 1.95-1.86 (m, 1H), 1.76 - 1.67 (m, 1H), 0.94 - 0.88 (m, 2H), 0.83 (d, J = 6.3 Hz, 3H), 0.69 - 0.62 (m, 2H). |
| **506** | (**I-506**) is isomer 3 in (**I-505**) family | See (**I-505**) |
| **507** | (**I-507**) is isomer 1 in (**I-505**) &(**I-506**) family | See (**I-505**) |
| **508** | (**I-508**) is isomer 4 in (**I-505**) to (**I-507**) family | See (**I-505**) |
| **509** | 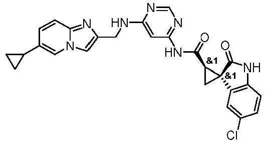 *rac-*(1R*,2S*)-5'-chloro-N-(6-(((6-cyclopropylimidazo[1,2-a]pyridin-2-yl) methyl)amino)pyrimidin-4-yl)-2-oxospiro[cyclopropane-1,3'-indoline]-2-carboxamide (**I-509**) | **Intermediate 857** ESI-MS (M+H)+: 500.2, δ 10.63 (d, J = 6.7 Hz, 2H), 8.29 (s, 1H), 8.14 (s, 1H), 7.88 (s, 1H), 7.60 (s, 1H), 7.37 (d, J = 9.3 Hz, 1H), 7.29 - 7.17 (m, 2H), 7.14 (d, J = 1.7 Hz, 1H), 6.95 (d, J = 9.3 Hz, 1H), 6.87 (d, J = 8.2 Hz, 1H), 4.57 (s, 2H), 3.03 (t, J = 8.4 Hz, 1H), 2.10 - 2.00 (m, 1H), 1.97 - 1.80 (m, 2H), 0.93 - 0.86 (m, 2H), 0.72 - 0.55 (m, 2H). |
| **510** | 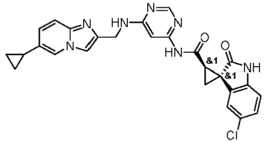 *rac*-(1R*,2R*)-5'-chloro-N-(6-(((6-cyclopropylimidazo[1,2-a]pyridin-2-yl) methyl)amino)pyrimidin-4-yl)-2'-oxospiro[cyclopropane-1,3'-indoline]-2-carboxamide (**I-510**) | **Intermediate 858** ESI-MS (M+H)+: 500.1, δ 10.87 (s, 1H), 10.76 (s, 1H), 8.48 (s, 1H), 8.28 (s, 1H), 8.16 (s, 1H), 7.97 (s, 1H), 7.61 (s, 1H), 7.37 - 7.35 (m,1H), 7.28 - 7.21 (m, 3H), 6.96 - 6.90 (m, 2H), 4.56 (s, 2H), 2.98 (t, J = 8.0 Hz, 1H), 2.14 - 2.11 (m, 1H), 1.93 - 1.87(m, 1H), 1.85 - 1.82 (m,1H), 0.93 - 0.88 (m, 2H), 0.67-0.63 (m,2H). |
| **511** | 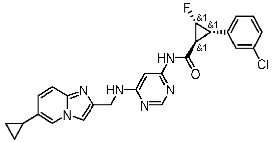 *rac*-(1R*,2R*,3S*)-2-(3-chloro phenyl)-N-(6-(((6-cyclopropyl imidazo[1,2-a] pyridin-2-yl)methyl) amino)pyrimidin-4-yl)-3-fluorocyclo propane-1-carboxamide (**I-511**) | **Intermediate 859** ESI-MS (M+H)+: 477.1, δ 10.75 (s, 1H), 8.29 (s, 1H), 8.23 (s, 1H), 7.92 (s, 1H), 7.61 (s, 1H), 7.43-7.33 (m, 4H), 7.30 (d, J = 7.2 Hz, 1H), 7.23 (s, 1H), 6.95 (d, J = 9.3 Hz, 1H), 5.23-5.7 (m, 1H), 4.57 (s, 2H), 3.14-3.06 (m, 1H), 2.84-2.77 (m, 1H), 1.94 - 1.87 (m, 1H), 0.95 - 0.87 (m, 2H), 0.69-0.62 (m, 2H). |
| **512** | 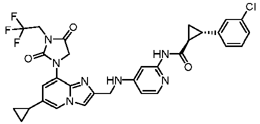 (1S,2S)-2-(3-chlorophenyl)-N-(4-(((6-cyclopropyl-8-(2,4-dioxo-3-(2,2,2-trifluoroethyl)imidazolidin-1-yl) imidazo[1,2-a]pyridin-2-yl)methyl) amino)pyridin-2-yl)cyclopropane-1-carboxamide (**I-512**) | **Intermediate 873** eg 20 |
| | | ESI-MS [M +H] ⁺: 638.1, ¹H NMR (400 MHz, cd3od) δ 8.17 (s, 1H), 7.75 (d, J = 6.0 Hz, 1H), 7.71 (s, 1H), 7.35 (s, 1H), 7.31 (d, J = 0.9 Hz, 1H), 7.27 - 7.23(m, 1H), 7.18 (d, J = 7.1 Hz, 2H), 7.09 (d, J = 7.6 Hz, 1H), 6.39 (dd, J = 6.0, 2.1 Hz, 1H), 4.80 (s, 2H), 4.49 (s, 2H), 4.31 (q, J = 8.9 Hz, 2H), 2.49 - 2.44 (m, 1H), 2.18 - 2.13 (m, 1H), 1.99 - 1.92 (m, 1H), 1.62 - 1.57 (m, 2H), 1.00 - 0.95 (m, 2H), 0.75 - 0.71 (m,, 2H). |
| **513** | 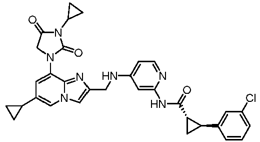 (1S,2S)-2-(3-chlorophenyl)-N-(4-(((6-cyclopropyl-8-(3-cyclopropyl-2,4-dioxo imidazolidin-1-yl)imidazo[1,2-a]pyridin-2-yl)methyl)amino)pyridin-2-yl)cyclo propane-1-carboxamide **I-513**) | **Intermediate 873** ESI-MS [M +H]+: 596.2, ¹H NMR (400 MHz, MeOD) δ 8.15 (d, J = 0.9 Hz, 1H), 7.75 (d, J = 6.0 Hz, 1H), 7.70 (s, 1H), 7.38 (s, 1H), 7.27 - 7.23 (m, 2H), 7.20 - 7.17 (m, 2H), 7.10 (d, J = 7.6 Hz, 1H), 6.38 (dd, J = 6.0, 2.2 Hz, 1H), 4.59 (s, 2H), 4.48 (s, 2H), 2.70 - 2.65 (m, 1H), 2.49 - 2.44 (m, 1H), 2.19 - 2.13 (m, 1H), 1.98 - 1.91 (m, 1H), 1.62 - 1.57 (m, 1H), 1.36 - 1.34 (m, 1H), 1.03 - 0.93 (m, 6H), 0.74 - 0.70 (m, 2H). |
| **514** | 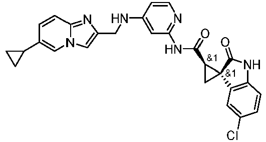 *rac*-(1R*,2S*)-5'-chloro-N-(4-(((6-cyclopropylimidazo[1,2-a]pyridin-2-yl) methyl)amino)pyridin-2-yl)-2'-oxo spiro[cyclopropane-1,3'-indoline]-2-carboxamide (**I-514**) | **Intermediate 860** ESI-MS [M +H] ⁺: 499.1. |
| | | δ 10.85 (s, 1H), 10.48 (s, 1H), 8.28 (s, 1H), 7.72 (d, J = 5.7 Hz, 1H), 7.63 (s, 1H), 7.41 - 7.37 (m, 2H), 7.28 (d, J = 1.7 Hz, 1H), 7.22 - 7.14 (m, 2H), 6.97 (dd, J = 9.3, 1.6 Hz, 1H), 6.90 (d, J = 8.3 Hz, 1H), 6.30 (dd, J = 5.7, 1.9 Hz, 1H), 5.32 (t, J = 4.7 Hz, 1H), 4.35 (d, J = 5.7 Hz, 2H), 2.98 - 2.94 (m, 1H), 2.14 - 2.11 (m, 1H), 1.92 - 1.87 (m, 1H), 1.82-1.79 (m,1H), 0.93-0.89 (m,2H), 0.68-0.64 (m,2H). |
| **515** | 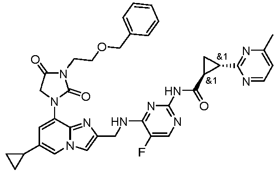 *rac*-(1S*,2S*)-N-(4-(((8-(3-(2-(benzyl oxy)ethyl)-2,4-dioxoimidazolidin- 1-yl)-6-cyclopropylimidazo[1,2-a]pyridin-2-yl) methyl)amino)-5-fluoropyrimidin-2-yl)-2-(4-methylpyrimidin-2-yl)cyclopropane-1-carboxamide (**I-515**) | **Intermediate 874** ESI-MS [M +H]+: 691.3. |
| | | δ 10.42 (s, 1H), 8.46 (d, J = 5.1 Hz, 1H), 8.22 (d, J = 4.9 Hz, 2H), 8.00 (d, J = 3.4 Hz, 1H), 7.79 (s, 1H), 7.32 (s, 6H), 7.14 (d, J = 5.1 Hz, 1H), 4.92 (s, 2H), 4.61 (d, J = 5.6 Hz, 2H), 4.51 (s, 2H), 3.72 (t, J = 5.3 Hz, 2H), 3.64 (t, J = 5.1 Hz, 2H), 2.87-2.86 (m, 1H), 2.51-2.50 (m, 1H), 2.37 (s, 3H), 1.97 - 1.90 (m, 1H), 1.52-1.49 (m, 2H), 0.95-0.93 (m, 2H), 0.65-0.63 (m, 2H). |
| **516** | 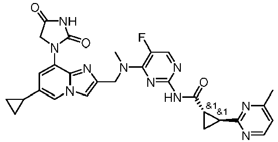 *rac*-(1S*,2S*)-N-(4-(((6-cyclopropyl-8-(2,4-dioxoimidazolidin-1-yl) imidazo[1,2-a]pyridin-2-yl)methyl) (methyl)amino)-5-fluoropyrimidin-2-yl)-2-(4-methyl pyrimidin-2-yl)cyclopropane-1-carboxamide (I-516) | **Intermediate 874** ESI-MS [M+H]+: 571.2. |
| | | δ 11.33 (s, 1H), 10.50 (s, 1H), 8.48 (d, J = 5.1 Hz, 1H), 8.19 (s, 1H), 8.06 (d, J = 6.6 Hz, 1H), 7.77 (s, 1H), 7.36 (d, J = 1.1 Hz, 1H), 7.17 (d, J = 5.1 Hz, 1H), 4.83 (s, 4H), 3.21 (s, 3H), 2.83 - 2.82 (m, 1H), 2.54-2.53 (m, 1H), 2.39 (s, 3H), 1.94-1.92 (m, 1H), 1.54 - 1.48 (m, 2H), 0.95 - 0.91 (m, 2H), 0.66 - 0.61 (m, 2H). |
| **517** | 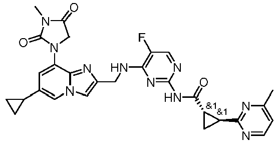 *rac*-(1S*,2S*)-N-(4-(((6-cyclopropyl-8-(3-methyl-2,4-dioxoimidazolidin-1-yl)imidazo[1,2-a]pyridin-2-yl)methyl) amino)-5-fluoropyrimidin-2-yl)-2-(4-methylpyrimidin-2-yl)cyclopropane-1-carboxamide (**I-517**) | **Intermediate 874** ESI-MS [M+H]+: 571.2. |
| | | δ 10.42 (s, 1H), 8.46 (d, J = 5.1 Hz, 1H), 8.20 (d, J = 6.1 Hz, 2H), 8.01 (d, J = 3.4 Hz, 1H), 7.79 (s, 1H), 7.32 (d, J = 1.5 Hz, 1H), 7.14 (d, J = 5.1 Hz, 1H), 4.88 (s, 2H), 4.61 (d, J = 5.9 Hz, 2H), 2.97 (s, 3H), 2.86 (s, 2H), 2.37 (s, 3H), 1.94 - 1.91 (m, 1H), 1.55 - 1.46 (m, 2H), 0.97 - 0.88 (m, 2H), 0.64 - 0.60 (m,2H) |
| **518** | 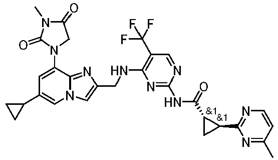 *rac*-(1S*,2S*)-N-(4-(((6-cyclopropyl-8-(3-methyl-2,4-dioxoimidazolidin-1-yl)imidazo[1,2-a]pyridin-2-yl)methyl) amino)-5-(trifluoromethyl) pyrimidin-2-yl)-2-(4-methylpyrimidin-2-yl)cyclo propane-1-carboxamide (**I-518**) | **Intermediate 861** ESI-MS [M+H]+: 621.2. δ 10.73 (s, 1H), 8.46 (d, J = 5.1 Hz, 1H), 8.35 (s, 1H), 8.23 (s, 1H), 7.87-7.84 (m, 1H), 7.71 (s, 1H), 7.34 (s, 1H), 7.14 (d, J = 5.1 Hz, 1H), 4.87 (s, 2H), 4.70 (d, J = 5.4 Hz, 2H), 2.97 (s, 3H), 2.58-2.55 (m, 2H), 2.38 (s, 3H), 1.99-1.90 (m, 1H), 1.53 (s, 2H), 0.96-0.91 (m, 2H), 0.66-0.64 (m, 2H). |
| **519** | 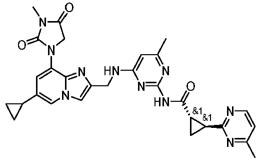 *rac*-(1S*,2S*)-N-(4-(((6-cyclopropyl-8-(3-methyl-2,4-dioxoimidazolidin-1-yl) imidazo[1,2-a]pyridin-2-yl)methyl) amino)-6-methylpyrimidin-2-yl)-2-(4-methylpyrimidin-2-yl)cyclopropane-1-carboxamide (**I-519**) | **Intermediate 862** ESI-MS [M+H]+: 567.2. |
| | | δ 10.17 (s, 1H), 8.46 (d, J = 4.9 Hz, 1H), 8.24 (d, J = 1.0 Hz, 1H), 7.73 (s, 2H), 7.32 (d, J = 1.2 Hz, 1H), 7.12 (d, J = 4.4 Hz, 1H), 6.09 (s, 1H), 4.89 (s, 2H), 4.50 (s, 2H), 2.97 (s, 3H), 2.52-2.51 (m, 2H), 2.39 - 2.33 (m, 3H), 2.09 (s, 3H), 1.96 - 1.91 (m, 1H), 1.55 - 1.48 (m, 2H), 0.99-0.91 (m, 2H), 0.67 - 0.62 (m, 2H). |
| **520** | 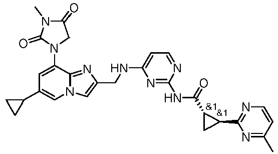 *rac*-(1S*,2S*)-N-(4-(((6-cyclopropyl-8-(3-methyl-2,4-dioxoimidazolidin-1-yl) imidazo[1,2-a]pyridin-2-yl)methyl) amino)pyrimidin-2-yl)-2-(4-methyl pyrimidin-2-yl)cyclopropane-1-carboxamide (**I-520**) | **Intermediate 863** ESI-MS [M+H]+: 553.2. |
| | | δ 10.23 (s, 1H), 8.50-8.46 (m, 1H), 8.26-8.22 (m, 2H), 7.89 (s, 1H), 7.76 (s, 1H), 7.33 (s, 1H), 7.18 - 7.09 (m, 1H), 6.24 (d, J = 5.9 Hz, 1H), 4.89 (s, 2H), 4.51 (s, 2H), 2.97 (d, J = 4.0 Hz, 3H), 2.54 (s, 2H), 2.38 (d, J = 11.7 Hz, 3H), 1.94 (s, 1H), 1.52 (s, 2H), 0.97 - 0.90 (m, 2H), 0.65-0.63 (m, 2H). |
| **521** | 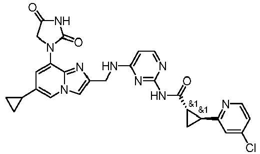 *rac*-(1S*,2S*)-2-(4-chloropyridin-2-yl)-N-(4-(((6-cyclopropyl-8-(2,4-dioxo imidazolidin-1-yl)imidazo[1,2-a]pyridin-2-yl)methyl)amino)pyrimidin-2-yl)cyclopropane-1-carboxamide (**I-521**) | **Intermediate 864** ESI-MS [M+H]+: 558.1. |
| | | δ 10.21 (s, 1H), 8.36 (d, J = 5.5 Hz, 1H), 8.22 (s, 1H), 7.95 - 7.81 (m, 3H), 7.74 (s, 1H), 7.63 (d, J = 1.8 Hz, 1H), 7.31 (s, 1H), 7.30 - 7.26 (m, 1H), 6.23 (d, J = 5.9 Hz, 1H), 5.32 (s, 1H), 4.86 (s, 2H), 4.51 (s, 2H), 2.69 - 2.66 (m, 1H), 2.64 - 2.58 (m, 1H), 2.34 - 2.31 (m, 1H), 1.99 - 1.92 (m, 2H), 0.96 - 0.91 (m, 2H), 0.67 - 0.62 (m, 2H). |
| **522** | 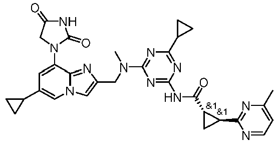 *rac-*(1S**,2*S*)-N-(4-cyclopropy1-6-(((6-cyclopropyl-8-(2,4-dioxo imidazolidin-1-yl)imidazo[1,2-a]pyridin-2-yl)methyl) (methyl)amino)-1,3,5-triazin-2-yl)-2-(4-methylpyrimidin-2-yl)cyclopropane-1-carboxamide (**I-522**) | **Intermediate 865** ESI-MS [M +H]+:594.2 δ 11.35 (s, 1H), 10.50 (s, 1H), 8.52-8.41 (m, 1H), 8.19 (d, J = 13.1 Hz, 1H), 7.68 (d, J = 15.2 Hz, 1H), 7.34 (s, 1H), 7.35-7.10 (m, 1H), 4.85 - 4.72 (m, 4H), 3.10 (d, J = 35.5 Hz, 3H), 2.53 (d, J = 8.1 Hz, 2H), 2.37 (d, J = 17.1 Hz, 3H), 1.95-1.92 (m, 1H), 1.79-1.77 (m, 1H), 1.64 - 1.46 (m, 2H), 0.99-0.90(m, 6H), 0.65-0.62 (m, 2H). |
| **523** | 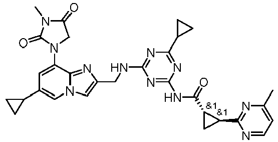 *rac-*(1S*,2S*)-N-(4-cyclopropyl-6-(((6-cyclopropyl-8-(3-methyl-2,4-dioxo imidazolidin-1-yl)imidazo[1,2-a] pyridin-2-yl)methyl)amino)-1,3,5-triazin-2-yl)-2-(4-methylpyrimidin-2-yl) cyclopropane-1-carboxamide (**I-523**) | **Intermediate 865** ESI-MS [M +H]+: 594.2. |
| | | δ 10.46 (d, J = 22.1 Hz, 1H), 8.52-8.41 (m, 1H), 8.29 - 7.97 (m, 2H), 7.69 (d, J = 22.8 Hz, 1H), 7.32 (d, J = 1.4 Hz, 1H), 7.22-7.10 (m, 1H), 4.89 (d, J = 3.3 Hz, 2H), 4.58-4.46 (m, 2H), 2.97 (s, 3H), 2.60 - 2.50 (m, 2H), 2.39 (d, J = 20.3 Hz, 3H), 2.01 - 1.84 (m, 1H), 1.76-1.72 (m, 1H), 1.58-1.52 (m, 2H), 0.98-0.87 (m, 6H), 0.67-0.61 (m, 2H). |
| **524** | 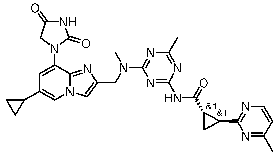 *rac-*(1S*,2S*)-N-(4-(((6-cyclopropyl-8-(2,4-dioxoimidazolidin-1-yl) imidazo[1,2-a]pyridin-2-yl)methyl) (methyl)amino)-6-methyl-1,3,5-triazin-2-yl)-2-(4-methyl pyrimidin-2-yl)cyclopropane-1-carboxamide (**I-524**) | **Intermediate 866** ESI-MS [M +H]+:568.2) δ 11.33 (s, 1H), 10.62 (d, J = 6.6 Hz, 1H), 8.52-8.40 (m, 1H), 8.18 (s, 1H), 7.69 (d, J = 2.8 Hz, 1H), 7.34 (s, 1H), 7.20-7.06(m, 1H), 4.94 - 4.63 (m, 4H), 3.10 (d, J = 43.9 Hz, 3H), 2.53-2.50 (m, 2H), 2.36 (d, J = 20.2 Hz, 3H), 2.24 (s, 3H), 1.95-1.90 (m, 1H), 1.58-1.53 (m, 2H), 0.98 - 0.87 (m, 2H), 0.69 - 0.55 (m, 2H). |
| **525** | 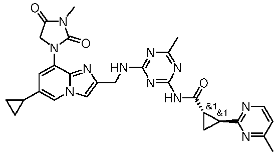 *rac*-(1S*,2S*)-N-(4-(((6-cyclopropyl-8-(3-methyl-2,4-dioxoimidazolidin-1-yl) imidazo[1,2-a]pyridin-2-yl)methyl) amino)-6-methyl-1,3,5-triazin-2-yl)-2-(4-methylpyrimidin-2-yl)cyclopropane-1-carboxamide (**I-525**) | **Intermediate 866** ESI-MS [M+H]+: 568.2. δ 10.61 (d, J = 30.0 Hz, 1H), 8.54 -8.49(m, 1H), 8.35 - 8.14 (m, 2H), 7.70 (d, J = 11.0 Hz, 1H), 7.32 (s, 1H), 7.25 -7.08(m, 1H), 4.86 (d, J = 18.1 Hz, 2H), 4.62 -4.47(m, 1H), 2.97 (s, 3H), 2.58-2.55 (m, 1H), 2.38 (d, J = 24.9 Hz, 3H), 2.20 (d, J = 4.8 Hz, 3H), 1.97 - 1.88 (m, 1H), 1.59 - 1.47 (m, 2H), 0.98 - 0.88 (m, 2H), 0.69 - 0.59 (m, 2H). |
| **526** | 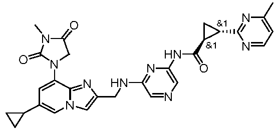 *rac*-(1S*,2S*)-N-(6-(((6-cyclopropyl-8-(3-methyl-2,4-dioxoimidazolidin-1-yl) imidazo[1,2-a]pyridin-2-yl)methyl) amino)pyrazin-2-yl)-2-(4-methyl pyrimidin-2-yl)cyclopropane-1-carboxamide (**I-526**) | **Intermediate 867** ESI-MS [M +H]+: 553.2. |
| | | δ 10.55 (s, 1H), 8.52 (d, J = 5.1 Hz, 1H), 8.44 (s, 1H), 8.24 (d, J = 1.1 Hz, 1H), 7.73 (d, J = 8.9 Hz, 2H), 7.55-7.52 (m, 1H), 7.32 (d, J = 1.5 Hz, 1H), 7.20 (d, J = 5.1 Hz, 1H), 4.88 (s, 2H), 4.55 (d, J = 5.8 Hz, 2H), 2.97 (s, 3H), 2.78 - 2.60 (m, 1H), 2.51 (s, 1H), 2.41 (s, 3H), 1.96-1.90 (m, 1H), 1.55-1.50 (m, 2H), 0.94 - 0.92 (m, 2H), 0.64-0.63 (m, 2H). |
| **527** | 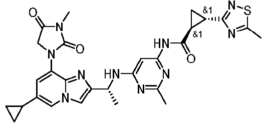 *rac-*(1S*,2S*)-N-(6-(((R)-1-(6-cyclopropyl-8-(3-methyl-2,4-dioxo imidazolidin-1-yl)imidazo[1,2-a]pyridin-2-yl)ethyl)amino)-2-methyl pyrimidin-4-yl)-2-(5-methyl-1,2,4-thiadiazol-3-yl)cyclopropane-1-carboxamide (**I-527**) | **Intermediate 875** ESI-MS: [M +H]+, 587.2, δ 10.69 (s, 1H), 8.24 (s, 1H), 7.66 (d, J = 2.6 Hz, 2H), 7.34 (s, 1H), 7.12 (s, 1H), 5.40 (s, 1H), 4.95 - 4.94 (m, 2H), 2.97 (d, J = 0.8 Hz, 3H), 2.75 (s, 3H), 2.64 - 2.61 (m, 2H), 2.25 (s, 3H), 1.95 - 1.90 (m, 1H), 1.49 (d, J = 6.7 Hz, 5H), 0.96 - 0.91 (m, 2H), 0.65 - 0.61 (m, 2H). |
| **528** | 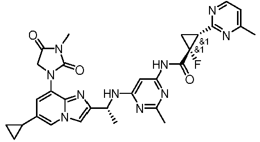 *rac-*(1S***,2S*)-N-(6-(((R)-1-(6-cyclo propyl-8-(3-methyl-2,4-dioxo imidazolidin-1-yl)imidazo[1,2-a] pyridin-2-yl)ethyl)amino)-2-methyl pyrimidin-4-yl)-1-fluoro-2-(4-methyl pyrimidin-2-yl)cyclopropane-1-carboxamide (**I-528**) | **Intermediate 876** ESI-MS [M +H]⁺: 599.1, δ 10.06 (s, 1H), 8.62 (d, J = 5.0 Hz, 1H), 8.24 (s, 1H), 7.79 (s, 1H), 7.68 (s, 1H), 7.34 (s, 1H), 7.31 (d, J = 5.1 Hz, 1H), 7.13 (d, J = 14.1 Hz, 1H), 5.45 (s, 1H), 4.95 (s, 2H), 3.09 - 3.01 (m, 1H), 2.97 (s, 3H), 2.46 (s, 3H), 2.28 (d, J = 3.1 Hz, 3H), 2.04 - 1.81 (m, 1H), 1.54-1.50 (m, 2H), 1.23 (s, 3H), 1.01 - 0.88 (m, 2H), 0.67-0.61 (m, 2H). |
| **529** | 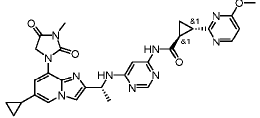 *rac-*(1S*,2S*)-N-(6-(((R)-1-(6-cyclo propyl-8-(3-methyl-2,4-dioxo imidazolidin-1-yl)imidazo[1,2-a] pyridin-2-yl)ethyl)amino)pyrimidin-4-yl)-2-(4-methoxypyrimidin-2-yl)cyclo propane-1-carboxamide (**I-529**) | **Intermediate 868** ESI-MS [M +H]+: 583.2, δ 10.68 (s, 1H), 8.38 (d, J=5.8, 1H), 8.23 (s, 1H), 8.16 (s, 1H), 7.78 (s, 1H), 7.65 (d, J=2.1, 1H), 7.34 (s, 1H), 7.30 (s, 1H), 6.75 (d, J=5.8, 1H), 5.35 (s, 1H), 4.94 (s, 2H), 3.90 (s, 3H), 2.98 (d, J=1.0, 3H), 2.72-2.68 (m, 1H), 2.54-2.52 (m, 1H), 1.94 - 1.92 (m, 1H), 1.55-1.50 (m, 5H), 0.96-0.91 (m, 2H), 0.65-0.63 (m, 2H). |
| **530** | 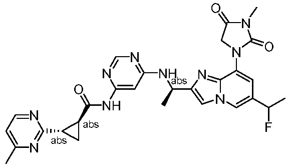 (1S,2S)-N-(6-(((1R)-1-(6-(1-fluoroethyl)-8-(3-methyl-2,4-dioxoimidazolidin-1-yl)imidazo[1,2-a]pyridin-2-yl)ethyl) amino)pyrimidin-4-yl)-2-(4-methylpyrimidin-2-yl)cyclopropane-1-carboxamide (**I-530**) | **Intermediate 833** |
| | | ESI-MS [M +H]⁺: 573.3. δ 10.66 (s, 1H), 8.53 - 8.51 (m, 2H), 8.16 (s, 1H), 7.80 (s, 2H), 7.66 (s, 1H), 7.32 (s, 1H), 7.21 (d, J = 5.1 Hz, 1H), 5.83 - 5.66 (m, 1H), 5.39 (s, 1H), 5.03 - 4.93 (m, 2H), 2.98 (s, 3H), 2.63 - 2.60 (m, 1H), 2.6 - 2.54 (m, 1H), 2.41 (s, 3H), 1.65 - 1.57 (m, 3H), 1.52 (d, J = 6.7 Hz, 5H). |
| **531** | 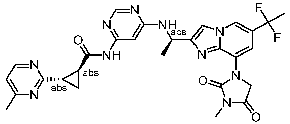 (1S,2S)-N-(6-(((R)-1-(6-(1,1-difluoroethyl)-8-(3-methyl-2,4-dioxoimidazolidin-1-yl)imidazo[1,2-a]pyridin-2-yl)ethyl)amino)pyrimidin-4-yl)-2-(4-methylpyrimidin-2-yl)cyclo propane-1-carboxamide (**I-531**) | **Intermediate 833** ESI-MS [M +H]+: 591.3, δ 10.67 (s, 1H), 8.76 (d, J = 1.5 Hz, 1H), 8.52 (d, J = 5.1 Hz, 1H), 8.16 (d, J = 0.7 Hz, 1H), 7.89 - 7.80 (m, 2H), 7.75 (d, J = 1.5 Hz, 1H), 7.32 (s, 1H), 7.21 (d, J = 5.2 Hz, 1H), 5.37 (s, 1H), 4.99 (d, J = 1.5 Hz, 2H), 2.98 (s, 3H), 2.64 - 2.60 (m, 1H), 2.55 - 2.52 (m, 1H), 2.41 (s, 3H), 2.01 (t, J = 18.8 Hz, 3H), 1.54 - 1.50 (m, 5H). |
| **532** | 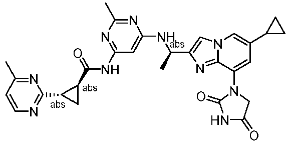 (1S,25)-N-(6-(((R)-1-(6-cyclopropyl-8-(2,4-dioxoimidazolidin-1-yl)imidazo [1,2-a]pyridin-2-yl)ethyl)amino)-2-methyl pyrimidin-4-yl)-2-(4-methyl pyrimidin-2-yl)cyclopropane-1-carboxamide (**I-532**) | (1S,2S)-N-(6-chloro-2-methyl-pyrimidin-4-yl)-2-(4-methylpyrimidin-2-yl)cyclopropane-1-carboxamide ESI-MS [M +H]⁺: 567.2. |
| | | δ 11.34 (s, 1H), 10.65 (s, 1H), 8.52 (d, J = 5.1 Hz, 1H), 8.22 (d, J = 1.1 Hz, 1H), 7.67 - 7.64 (m, 2H), 7.32 (d, J = 1.4 Hz, 1H), 7.20 (d, J = 5.1 Hz, 1H), 7.12 (s, 1H), 5.38 (s, 1H), 4.91 (d, J = 17.9 Hz 2H), 2.55-2.52 (m, 2H), 2.41 (s, 3H), 2.24 (s, 3H), 1.95-1.89 (m, 1H), 1.51-1.48 (m, 5H), 0.95 - 0.91 (m, 2H), 0.65-0.60 (m, 2H). |
| **533** | 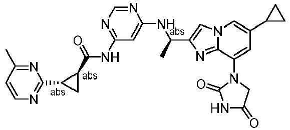 (1S,2S)-N-(6-(((R)-1-(6-cyclopropyl-8-(2,4-dioxoimidazolidin-1-yl)imidazo[1,2-a]pyridin-2-yl)ethyl)amino)pyrimidin-4-yl)-2-(4-methylpyrimidin-2-yl)cyclo propane-1-carboxamide (**I-533**) | **Intermediate 833** ESI-MS [M +H]⁺: 553.2. |
| | | δ 11.36 (s, 1H), 10.68 (s, 1H), 8.52 (d, J = 5.1 Hz, 1H), 8.17 (s, 1H), 8.16 (s, 1H), 7.83 (s, 1H), 7.66 (s, 1H), 7.33 (d, J = 15.5 Hz, 2H), 7.20 (d, J = 4.8 Hz, 1H), 5.36 (s, 1H), 4.89 (d, J = 2.9 Hz, 2H), 2.61-2.55 (m, 2H), 2.41 (s, 3H), 1.95-1.90 (m, 1H), 1.52(s, 3H), 1.24-1.20 (m,2H), 0.97-0.90 (m,2H), 0.66-0.60 (m,2H). |
| **534** | 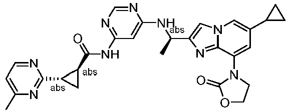 (1S,2S)-N-(6-(((R)-1-(6-cyclopropyl-8-(2-oxooxazolidin-3-yl)imidazo[1,2-a] pyridin-2-yl)ethyl)amino)pyrimidin-4-yl)-2-(4-methylpyrimidin-2-yl)cyclo propane-1-carboxamide (**I-534**) | **Intermediate 833** ESI-MS [M +H] +: 540.2, δ 10.65 (s, 1H), 8.52 (d, *J=* 5.1 Hz, 1H), 8.23 (s, 1H), 8.16 (s, 1H), 7.77 (s, 1H), 7.64 (s, 1H), 7.30 (s, 1H), 7.23 - 7.19 (m, 2H), 5.33 (s, 1H), 4.54 - 4.44 (m, 4H), 2.63 - 2.60 (m, 1H), 2.56 - 2.54 (m, 1H), 2.42 (s, 3H),1.96-1.90 (m, 1H), 1.58-1.45 (m, 5H), 0.95-0.90 (m, 2H), 0.64-0.63 (m, 2H). |
| **535** | 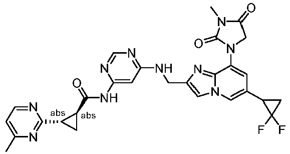 (1S,2S)-N-(6-(((6-(2,2-difluorocyclo propyl)-8-(3-methyl-2,4-dioxo imidazolidin-1-yl)imidazo[1,2-a]pyridin-2-yl)methyl)amino) pyrimidin-4-yl)-2-(4-methylpyrimidin-2-yl)cyclopropane-1-carboxamide (**I-535**) | **Intermediate 833** (ESI-MS [M +H] +: 589.2, δ 10.68 (s, 1H), 8.52 (d, J = 5.1 Hz, 1H), 8.41 (s, 1H), 8.19 (s, 1H), 7.90 (s, 1H), 7.76 (s, 1H), 7.54 (s, 1H), 7.32 (s, 1H), 7.21 (d, J = 5.1 Hz, 1H), 4.93 (d, J = 18.5 Hz, 2H), 4.60 (s, 2H), 3.12 - 3.01 (m, 1H), 2.98 (s, 3H), 2.66 - 2.52 (m, 2H), 2.41 (s, 3H), 2.10 - 1.97 (m, 1H), 1.90 - 1.80 (m, 1H), 1.55 - 1.46 (m, 2H). |
| **536** | 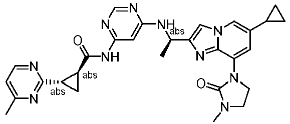 (1S,2S)-N-(6-(((R)-1-(6-cyclopropyl-8-(3-methyl-2-oxoimidazolidin-1-yl) imidazo[1,2-a]pyridin-2-yl)ethyl) amino)pyrimidin-4-yl)-2-(4-methyl pyrimidin-2-yl)cyclopropane-1-carboxamide (**I-536**) | **Intermediate 833** ESI-MS [M +H] +: 553.7, δ 10.65 (s, 1H), 8.52 (d, *J* = 5.1 Hz, 1H), 8.16 (d, *J =* 0.8 Hz, 1H), 8.10 (d, *J* = 1.1 Hz, 1H), 7.75 (s, 1H), 7.59 (s, 1H), 7.30 (s, 1H), 7.22 - 7.20 (m, 2H), 5.31 (s, 1H), 4.32 - 4.28 (m, 2H), 3.49 - 3.45 (m, 2H), 2.79 (s, 3H), 2.64 - 2.60 (m, 1H), 2.53 - 2.52 (m, 1H), 2.41 (s, 3H), 1.90 - 1.84 (m, 1H), 1.55 - 1.49 (m, 5H), 0.92 - 0.87 (m, 2H), 0.62 - 0.59 (m, 2H). |
| **537** | 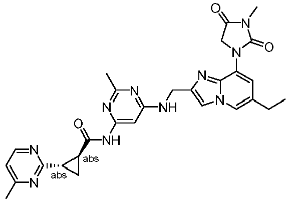 (1S,2S)-N-(6-(((6-ethyl-8-(3-methyl-2,4-dioxoimidazolidin-1-yl)imidazo[1,2-a]pyridin-2-yl)methyl)amino)-2-methyl pyrimidin-4-yl)-2-(4-methylpyrimidin-2-yl)cyclopropane-1-carboxamide (**I-537**) | (1S,2S)-N-(6-chloro-2-methyl-pyrimidin-4-yl)-2-(4-methylpyrimidin-2-yl)cyclopropane-1-carboxamide |
| | | ESI-MS [M +H]+: 555.3 δ 10.68 (s, 1H), 8.52 (d, J = 5.1 Hz, 1H), 8.26 (s, 1H), 7.74 (s, 2H), 7.47 (s, 1H), 7.20 (d, J = 5.1 Hz, 1H), 7.14 (s, 1H), 4.93 (s, 2H), 4.58 (s, 2H), 2.98 (s, 3H), 2.60-2.56 (m, J = 11.1 Hz, 4H), 2.41 (s, 3H), 2.27 (s, 3H), 1.54- 1.426(m, 2H), 1.20 (t, J = 7.5 Hz, 3H). |
| **538** | 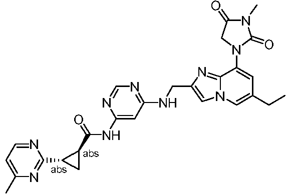 (1S,2S)-N-(6-(((6-ethyl-8-(3-methyl-2,4-dioxoimidazolidin-1-yl)imidazo[1,2-a] pyridin-2-yl) methyl)amino)pyrimidin-4-yl)-2-(4-methylpyrimidin-2-yl)cyclo propane-1-carboxamide (**I-538**) | **Intermediate 833** |
| | | ESI-MS [M +H]+: 541.3, δ 10.67 (s, 1H), 8.52 (d, J = 5.1 Hz, 1H), 8.25 (d, J = 1.0 Hz, 1H), 8.18 (s, 1H), 7.88 (s, 1H), 7.73 (s, 1H), 7.47 (d, J = 1.1 Hz, 1H), 7.31 (d, J = 0.7 Hz, 1H), 7.20 (d, J = 5.1 Hz, 1H), 4.93 (s, 2H), 4.58 (s, 2H), 2.98 (s, 3H), 2.64 - 2.56 (m, 3H), 2.55 - 2.52 (m, 1H), 2.41 (s, 3H), 1.55- 1.47 (m, 2H), 1.20 (t, J = 7.5 Hz, 3H). |

### Example 539: rac-(1S*,2S*)-N-(4-cyclopropyl-6-(((6-cyclopropyl-8-(3-methyl-2,4-dioxoimidazolidin-1-yl)imidazo[1,2-a]pyridin-2-yl)methyl)amino)pyrimidin-2-yl)-2-(4-methylpyrimidin-2-yl)cyclopropane-1-carboxamide (I-539)

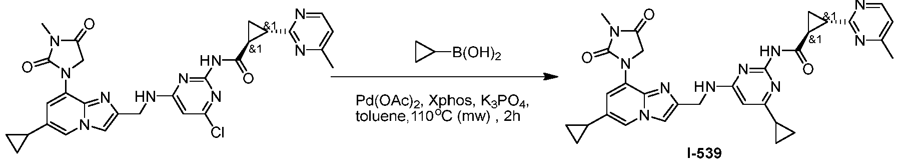

To a mixture of N-(4-chloro-6-(((6-cyclopropyl-8-(3-methyl-2,4-dioxoimidazolidin-1-yl)imidazo[1,2-a]pyridin-2-yl)methyl)amino)pyrimidin-2-yl)-2-(4-methylpyrimidin-2-yl)cyclopropane-1-carboxamide (120 mg, 0.2 mmol), cyclopropylboronic acid (51.6 mg, 0.6 mmol), and Cs₂CO₃ (195.5 mg, 0.6 mmol) in dioxane (5 mL) were added Pd(OAc)₂ (8.9 g, 0.04 mmol) and Xphos (28.5 mg, 0.06 mmol). After degassing with N₂ for 1 min, the reaction was irradiated in microwave at 110 °C for 2 h. The reaction mixture was filtered through Celite and the filter cake was washed with DCM/MeOH (10/1, 20 mL). The filtrate was concentrated *in vacuo* to give the crude, which was purified by preparative TLC (eluent: DCM/MeOH = 20/1) to give (**I-539**) (3.3 mg, 2.8%) as a white solid. ESI-MS [M +H]+:593.2, δ 9.95 (s, 1H), 8.47 (d, J = 4.9 Hz, 1H), 8.23 (s, 1H), 7.74 (s, 1H), 7.67 (s, 1H), 7.32 (s, 1H), 7.15 (d, J = 5.0 Hz, 1H), 6.16 (s, 1H), 4.89 (s, 2H), 4.50 (s, 2H), 2.97 (s, 3H), 2.54-2.52 (m, 2H), 2.38 (s, 3H), 2.01- 1.98 (m, 1H), 1.77-1.71 (m, 1H), 1.58 - 1.45 (m, 2H), 0.96-0.90 (m, 2H), 0.89 - 0.72 (m, 4H), 0.66-0.63 (m, 2H).

### Example 540: rac-(1S*,2S*)-N-(4-(((6-cyclopropyl-8-(3-(2-hydroxyethyl)-2,4-dioxoimidazolidin-1-yl)imidazo[1,2-a]pyridin-2-yl)methyl)amino)-5-fluoropyrimidin-2-yl)-2-(4-methylpyrimidin-2-yl)cyclopropane-1-carboxamide (I-540)

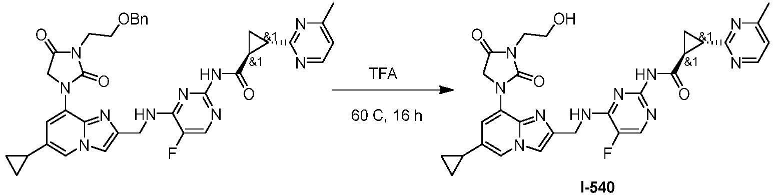

A solution of *rac-*(1S*, 2S*)-N-(4-(((8-(3-(2-(benzyloxy)ethyl)-2,4-dioxoimidazolidin-1-yl)-6-cyclopropylimidazo[1,2-a]pyridin-2-yl)methyl)amino)-5-fluoropyrimidin-2-yl)-2-(4-methylpyrimidin-2-yl)cyclopropane-1-carboxamide (50 mg, 0.072 mmol) in TFA (2 mL) was stirred at 60 °C for 16 h. After cooling to room temperature, the mixture was basified to pH~8 with a solution of saturated aq. NaHCO₃ (15 mL) and then extracted with DCM (2 x 20 mL). The combined organic layers were washed with brine (30 mL), dried over anhydrous Na₂SO₄, and concentrated *in vacuo* to give the crude, which was purified by preparative TLC (eluent: DCM/MeOH=10:1) to give (**I-540**) (27 mg, 62%) as a yellow solid. ESI-MS [M +H]+: 601.2. δ 10.42 (s, 1H), 8.47 (d, J = 4.6 Hz, 1H), 8.30 - 8.15 (m, 2H), 8.01 (s, 1H), 7.75 (d, J = 34.0 Hz, 1H), 7.32 (s, 1H), 7.15 (s, 1H), 4.97 - 4.85 (m, 3H), 4.62 (s, 2H), 3.61 - 3.52 (m, 4H), 2.87-2.86 (m, 1H), 2.58-2.56 (m, 1H), 2.41 (s, 3H), 2.03 - 1.88 (m, 1H), 1.57 - 1.40 (m, 2H), 0.99 - 0.89 (m, 2H), 0.69 - 0.58 (m, 2H).

### Example 541: rac-(1S*,2S*)-N-(4-((2R,4S)-2-(6-cyclopropyl-8-(3-methyl-2,4-dioxoimidazolidin-1-yl)imidazo[1,2-a]pyridin-2-yl)-4-hydroxypyrrolidin-1-yl)pyrimidin-2-yl)-2-(4-methylpyrimidin-2-yl)cyclopropane-1-carboxamide (I-541)

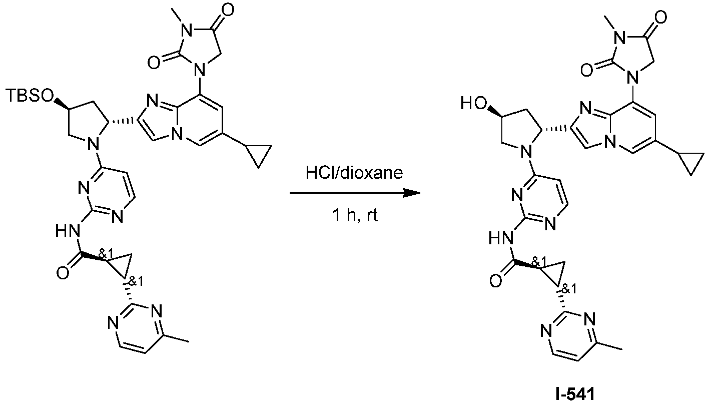

To a solution of rac-(1S*,2S*)-N-(4-((2R,4S)-4-((tert-butyldimethylsilyl)oxy)-2-(6-cyclo propyl-8-(3-methyl-2,4-dioxoimidazolidin-1-yl)imidazo[1,2-a]pyridin-2-yl)pyrrolidin-1-yl) pyrimidin-2-yl)-2-(4-methylpyrimidin-2-yl)cyclopropane-1-carboxamide (45 mg, 0.06 mmol) in MeOH (5 mL) was added HCl (4 M solution in dioxane, 1 mL). The resulting mixture was stirred at room temperature for 1 h, then ammonia in methanol (2 mL, 7M) was added. The reaction mixture was concentrated *in vacuo* and purified by preparative TLC to give (**I-541**) (11.3 mg, 31%) as a yellow solid. ESI-MS [M+H] +: 609.2. δ 10.19 (s, 1H), 8.50 - 8.48 (d, J = 5.1 Hz, 1H), 8.31 (s, 2H), 8.18 - 8.14 (m, 1H), 7.73 - 7.72 (m, 1H), 7.32 (s, 1H), 7.17 (d, J = 5.0 Hz, 1H), 5.08 - 5.01 (m, 1H), 4.93 -4.87 (m, 2H), 4.83 - 4.79 (m, 1H), 4.47 - 4.42 (m, 1H), 3.67 - 3.63 (m, 2H), 2.93 (d, J = 1.7 Hz, 3H), 2.65- 2.63 (m, 2H), 2.39 (m, 3H), 2.30 - 2.29 (m, 2H), 1.92 -1.88 (m, 1H), 1.55 -1.47 (m, 2H), 0.91 -0.89 (m, 2H), 0.60 - 0.55 (m, 2H).

The compound in Table **39** was prepared in a similar manner to (**I-541**) from the corresponding silyl ether. **Table 39**

| **EG** | **Structure** | **Analytical Data** |
|---|---|---|
| **542** | 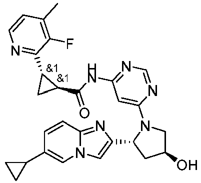 *rac-(*1S***,2S*)-N-(6-((2R,4S)-2-(6-cyclo propylimidazo[1,2-a]pyridin-2-yl)-4-hydroxypyrrolidin-1-yl)pyrimidin-4-yl)-2-(3-fluoro-4-methylpyridin-2-yl)cyclo propane-1-carboxamide (**I-542**) | ESI-MS [M +H]⁺: 514.2, δ 10.77-10.63 (m, 1H), 8.24-8.15 (m, 3H), 7.62-7.51 (m, 1H), 7.38 (d, J = 8.9 Hz, 1H), 7.18-7.17 (m, 2H), 6.96 (d, J = 8.8 Hz, 1H), 5.46-4.99 (m, 2H), 4.57-4.44 (m, 1H), 3.76 (s, 1.5H), 3.40 - 3.38 (m, 1H), 3.20 (s, 0.5H), 2.75 - 2.61 (m, 1H), 2.31 - 2.23 (m, 4H), 1.96 - 1.85 (m, 1H), 1.61 - 1.40 (m, 2H), 0.94 - 0.87 (m, 2H), 0.70 - 0.60 (m, 2H). |

### Examples 543-545

### rac-(1S*,3S*)-3-(3-chlorophenyl)-N-(6-(((6-cyclopropyl-8-(3-methyl-2,4-dioxo imidazolidin-1-yl)imidazo[1,2-a]pyridin-2-yl)methyl)amino)pyrimidin-4-yl)-2,2-difluorocyclopropane-1-carboxamide (I-543)

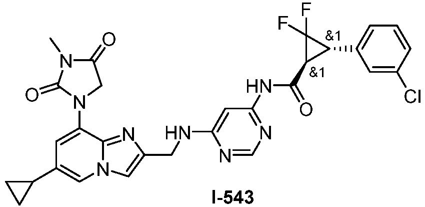

(**I-543**) was prepared in a similar manner to (**I-489**) as a mixture of enantiomers. ESI-MS [M +H] +: 607.2, δ 10.80 (s, 1H), 8.24 (s, 2H), 7.98 (s, 1H), 7.70 (s, 1H), 7.48 - 7.39 (m, 3H), 7.37 - 7.26 (m, 3H), 4.91 (s, 2H), 4.60 (s, 2H), 3.72 - 3.63 (m, 1H), 3.55 - 3.47 (m, 1H), 2.96 (s, 3H), 1.99-1.88 (m, 1H), 0.97 - 0.90 (m, 2H), 0.67 - 0.60 (m, 2H).

The mixture was separated using SFC (Daicel CHIRALPAK OJ-H 250mm × 20 mm I.D., 5µm, CO₂/EtOH = 54/46, 50 g/min, 35 °C) to give two enantiomers: (1S,3S)-3-(3-chlorophenyl)-N-(6-(((6-cyclopropyl-8-(3-methyl-2,4-dioxoimidazolidin-1-yl)imidazo[1,2-a]pyridin-2-yl)methyl)amino)pyrimidin-4-yl)-2,2-difluorocyclopropane-1-carboxamide and (1R,3R)-3-(3-chlorophenyl)-N-(6-(((6-cyclopropyl-8-(3-methyl-2,4-dioxoimidazolidin-1-yl)imidazo[1,2-a]pyridin-2-yl)methyl)amino)pyrimidin-4-yl)-2,2-difluorocyclopropane-1-carboxamide. First eluting isomer (**I-544**): ESI-MS [M +H] +: 607.2, δ 10.80 (s, 1H), 8.24 (s, 2H), 7.98 (s, 1H), 7.70 (s, 1H), 7.47 - 7.39 (m, 3H), 7.36 - 7.26 (m, 3H), 4.91 (s, 2H), 4.60 (s, 2H), 3.72-3.62 (m, 1H), 3.55-3.46 (m, 1H), 2.96 (s, 3H), 1.96 - 1.90 (m, 1H), 0.97 - 0.89 (m, 2H), 0.67 - 0.60 (m, 2H). RT = 4.314min, 100% e.e.
Second eluting isomer (**I-545**): ESI-MS [M +H] +: 607.2, ¹H NMR (400 MHz, DMSO) substantially similar to (I-544). RT = 7.144min, 100% e.e.

### Examples 546-548

### rac-(1S*,2S*)-N-(6-(((R)-1-(6-cyclopropyl-8-(3-methyl-2,4-dioxoimidazolidin-1-yl)imidazo[1,2-a]pyridin-2-yl)ethyl)amino)-2-methylpyrimidin-4-yl)-2-fluoro-2-(4-methylpyrimidin-2-yl)cyclopropane-1-carboxamide (I-546)

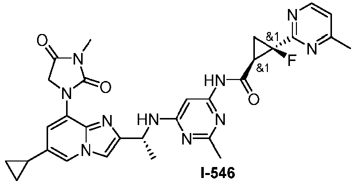

The title compound was prepared in a similar manner to (**I-489**) as a mixture of diastereomerss. ESI-MS (M+H)+: 599.3, δ 10.64 (s, 1H), 8.66 (d, J = 5.0 Hz, 1H), 8.24 (s, 1H), 7.66 (d, J = 7.2 Hz, 2H), 7.34 (d, J = 4.3 Hz, 2H), 7.15 - 7.06 (m, 1H), 5.41- 5.32 (m, 1H), 4.95 (s, 2H), 2.98 (s, 3H), 2.93 - 2.82 (m, 1H), 2.49 (s, 3H), 2.23 (s, 3H), 1.94 - 1.86 (m, 2H), 1.50 (d, J = 6.7 Hz, 3H), 1.00 - 0.88 (m, 2H), 0.72 - 0.57 (m, 2H).

The mixture was separated using SFC 80 (Daicel CHIRALPAK OJ-H 20 x 250 mm, 5.0 µm, CO2/MeOH (0.2% NH₃ (7M in MeOH)) = 75/25, 60 g/min, 35°C) to give two diastereomers: (1S,2S)-N-(6-(((R)-1-(6-cyclopropyl-8-(3-methyl-2,4-dioxoimidazolidin-1-yl)imidazo[1,2-a]pyridin-2-yl)ethyl)amino)-2-methylpyrimidin-4-yl)-2-fluoro-2-(4-methylpyrimidin-2-yl)cyclopropane-1-carboxamide and (1R,2R)-N-(6-(((R)-1-(6-cyclopropyl-8-(3-methyl-2,4-dioxoimidazolidin-1-yl)imidazo[1,2-a]pyridin-2-yl)ethyl)amino)-2-methylpyrimidin-4-yl)-2-fluoro-2-(4-methylpyrimidin-2-yl)cyclopropane-1-carboxamide.
First eluting isomer (**I-547**): ESI-MS [M +H]+: 599.3, δ 10.64 (s, 1H), 8.66 (d, J = 5.1 Hz, 1H), 8.24 (d, J = 1.0 Hz, 1H), 7.68-7.64 (m, 2H), 7.34 (d, J = 5.2 Hz, 2H), 7.13 (s, 1H), 5.42 (s, 1H), 4.95 (s, 2H), 2.97 (s, 3H), 2.93 - 2.82 (m, 1H), 2.49 (s, 3H), 2.26 - 2.13 (m, 4H), 1.95-1.87 (m, 2H), 1.50 (d, J = 6.7 Hz, 3H), 0.98 - 0.90 (m, 2H), 0.66 - 0.60 (m, 2H). RT = 2.52 min, 100.0 % e.e.
Second eluting isomer (**I-548**): ESI-MS [M +H]+: 599.3, ¹H NMR (400 MHz, DMSO substantially similar to (I-547). RT = 4.12 min, 100.0 % e.e.

### Intermediate 877 1-(2-(1-((6-chloropyrimidin-4-yl)oxy)ethyl)-6-cyclopropylimidazo[1,2-a]pyridin-8-yl)-3-methylimidazolidine-2,4-dione

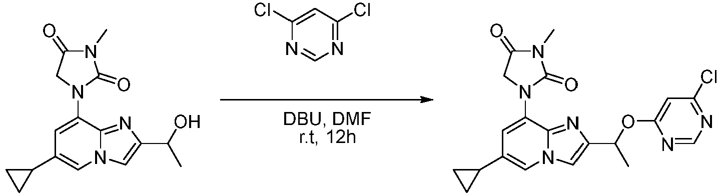

To a solution of 1-(6-cyclopropyl-2-(1-hydroxyethyl)imidazo[1,2-a]pyridin-8-yl)-3-methylimidazolidine-2,4-dione (100 mg, 0.32 mmol) in DMF (3 mL) was added 4,6-dichloropyrimidine (284 mg, 1.92 mmol) andDBU (145 mg, 0.96 mmol), and then the mixture was stirred at 45 °C for 48 h. The reaction mixture was quenched by water (20 mL) and extracted with EtOAc (3 x 30 mL). The combined organic layers were concentrated to dryness. The residue was purified by preparative TLC (eluted with PE: EtOAc =3:1) to give **intermediate 877** as a yellow oil (30 mg, yield 22%). ESI-MS [M +H]⁺: 427.1

The compounds in Table **40** were prepared in a similar manner to **intermediate 877** from a di-halo-pyrimidine and and appropriate alcohol. **Table 40**

| **Structure** | **Analytical Data** |
|---|---|
| 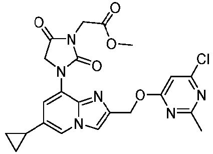 | **methyl 2-(3-(2-(((6-chloro-2-methylpyrimidin-4-yl)oxy)methyl)-6-cyclopropylimidazo[1,2-a]pyridin-8-yl)-2,5-dioxoimidazolidin-1-yl)acetate intermediate 878** ESI-MS [M +H]⁺:485.2 |
| 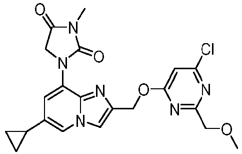 | **1-(2-(((6-chloro-2-(methoxymethyl)pyrimidin-4-yl)oxy)methyl)-6-cyclopropylimidazo [1,2-a]pyridin-8-yl)-3-methylimidazolidine-2,4-dione intermediate 879** |
| | ESI-MS [M +H]⁺:457.2 |
| 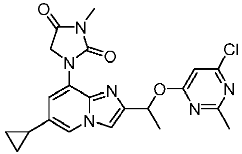 | **1-(2-(1-((6-chloro-2-methylpyrimidin-4-yl)oxy)ethyl)-6-cyclopropylimidazo[1,2-a]pyridin-8-yl)-3-methylimidazolidine-2,4-dione intermediate 880** ESI-MS [M +H]⁺:441.2 |
| 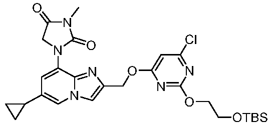 | **1-(2-(((2-(2-((*tert*-butyldimethylsilyl)oxy)ethoxy)-6-chloropyrimidin-4-yl)oxy)methyl)-6-cyclopropylimidazo[1,2-a]pyridin-8-yl)-3-methylimidazolidine-2,4-dione intermediate 881** ESI-MS [M +H]⁺:587.2 |
| 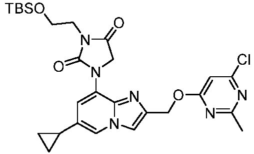 | **3-(2-((*tert*-butyldimethylsilyl)oxy)ethyl)-1-(2-(((6-chloro-2-methylpyrimidin-4-yl)oxy)methyl)-6-cyclopropylimidazo[1,2-a]pyridin-8-yl)imidazolidine-2,4-dione intermediate 882** ESI-MS [M +H]⁺:571.2 |
| 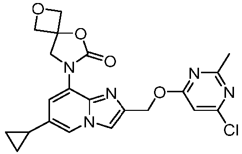 | **7-(2-(((6-chloro-2-methylpyrimidin-4-yl)oxy)methyl)-6-cyclopropylimidazo[1,2-a]pyridin-8-yl)-2,5-dioxa-7-azaspiro[3.4]octan-6-one intermediate 883** ESI-MS [M +H]⁺:442.2 |
| 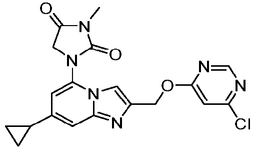 | **1-(2-(((6-chloropyrimidin-4-yl)oxy)methyl)-7-cyclopropylimidazo[1,2-a]pyridin-5-yl)-3-methylimidazolidine-2,4-dione intermediate 884** ESI-MS [M +H]⁺:413.2 |
| 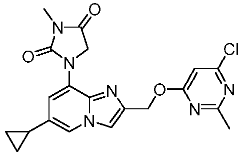 | **1-(2-(((6-chloro-2-methylpyrimidin-4-yl)oxy)methyl)-6-cyclopropylimidazo[1,2-a]pyridin-8-yl)-3-methylimidazolidine-2,4-dione intermediate 885** ESI-MS [M +H]+:427.2. δ 8.29 (d, J = 1.2 Hz, 1H), 7.98 (s, 1H), 7.39 (d, J = 1.4 Hz, 1H), 7.04 (s, 1H), 5.51 (s, 2H), 4.88 (s, 2H), 2.97 (s, 3H), 2.55 (s, 3H), 2.00 - 1.93 (m, 1H), 0.98 - 0.94 (m, 2H), 0.68 - 0.64 (m, 2H). |
| 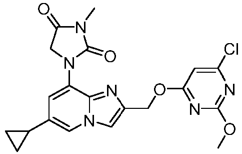 | **1-(2-(((6-chloro-2-methoxypyrimidin-4-yl)oxy)methyl)-6-cyclopropylimidazo[1,2-a]pyridin-8-yl)-3-methylimidazolidine-2,4-dione intermediate 886** |
| | ESI-MS [M +H]⁺:443.2 |
| 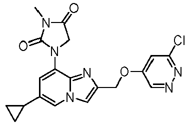 | **1-(2-(((6-chloropyridazin-4-yl)oxy)methyl)-6-cyclopropylimidazol[1,2-a]pyridin-8-yl)-3-methylimidazolidine-2,4-dione intermediate 887** ESI-MS (M+H)+: 413.3 |
| 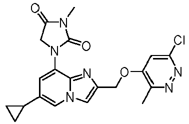 | **1-(2-(((6-chloro-3-methylpyridazin-4-yl)oxy)methyl)-6-cyclopropylimidazo[1,2-a]pyridin-8-yl)-3-methylimidazolidine-2,4-dione intermediate 888** ESI-MS (M+H)+: 427.2 |
| 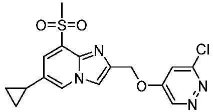 | **2-(((6-chloropyridazin-4-yl)oxy)methyl)-6-cyclopropyl-8-(methylsulfonyl)imidazo[1,2-a]pyridine intermediate 889** ESI-MS (M+H)+: 379.1 |

### Example 549: (1S,2S)-N-(6-(1-(6-cyclopropyl-8-(3-methyl-2,4-dioxoimidazolidin-1-yl)imidazo [1,2-a]pyridin-2-yl)ethoxy)pyrimidin-4-yl)-2-(4-methylpyrimidin-2-yl)cyclopropane-1-carboxamide (I-549)

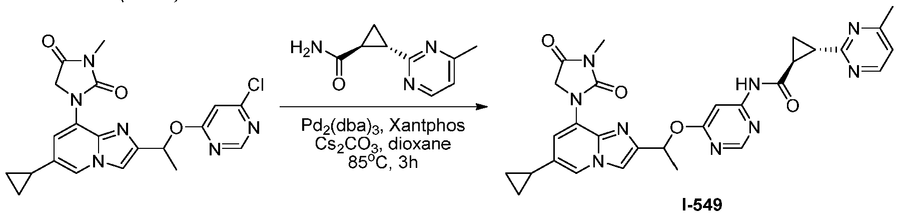

To a solution of 1-(2-(1-((6-chloropyrimidin-4-yl)oxy)ethyl)-6-cyclopropylimidazo[1,2-a]pyridin-8-yl)-3-methylimidazolidine-2,4-dione (30 mg, 0.07 mmol) in dioxane (3 mL) was added intermediate 55 (12 mg, 0.07 mmol), Pd₂(dba)₃ (9 mg, 0.01 mmol), Xantphos (6 mg, 0.01 mmol), and Cs₂CO₃ (69 mg, 0.21 mmol). The mixture was degassed with N₂ three times and the mixture was stirred at 85 °C for 6 hr. After cooling to room temperature, the mixture was diluted with water (10 mL) and extracted with EtOAc (3 x 30). The combined organic layers were washed with brine, dried over anhydrous Na₂SO₄, filtered, and concentrated *in vacuo.* The residue was purified by preparative TLC (eluted with DCM: MeOH=20:1) to give (1S,2S)-N-(6-(1-(6-cyclopropyl-8-(3-methyl-2,4-dioxoimidazolidin-1-yl)imidazo[1,2-a]pyridin-2-yl)ethoxy)pyrimidin-4-yl)-2-(4-methylpyrimidin-2-yl)cyclopropane-1-carboxamide as white solid (12 mg, 30%). ESI-MS (M+H)+: 568.2, δ 11.17 (s, 1H), 8.52-8.49 (m, 2H), 8.26 (s, 1H), 7.85 (d, J = 2.0 Hz, 1H), 7.48 (s, 1H), 7.40 - 7.35 (m, 1H), 7.22 (d, J = 5.0 Hz, 1H), 6.39 - 6.32 (m, 1H), 4.91 (d, J = 1.7 Hz, 2H), 2.97 (d, J = 1.5 Hz, 3H), 2.68 - 2.64 (m, 1H), 2.58 - 2.55 (m, 1H), 2.44 - 2.39 (m, 3H), 2.00 - 1.91 (m, 1H), 1.68 (d, J = 6.5 Hz, 3H), 1.60 - 1.51 (m, 2H), 0.98 - 0.91 (m, 2H), 0.67 - 0.61 (m, 2H).

The compounds in Table 41 were prepared in a similar manner to (1S,2S)-N-(6-(1-(6-cyclopropyl-8-(3-methyl-2,4-dioxoimidazolidin-1-yl)imidazo[1,2-a]pyridin-2-yl)ethoxy)pyrimidin-4-yl)-2-(4-methylpyrimidin-2-yl)cyclopropane-1-carboxamide from the appropriate cyclopropyl amide and the indicated coupling partner. **Table 41**

| **EG** | **Structure** | **Coupling Partner** / **Analytical Data** |
|---|---|---|
| **550** | 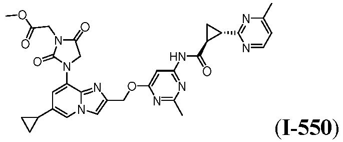 methyl 2-(3-(6-cyclopropyl-2-(((2-methyl-6-((1S,2S)-2-(4-methyl pyrimidin-2-yl)cyclopropane-1-carboxamido)pyrimidin-4-yl)oxy) methyl)imidazo[1,2-a]pyridin-8-yl)-2,5-dioxoimidazolidin-1-yl)acetate | **Intermediate 878** ESI-MS (M+H)+: 626.3, ¹H NMR (400 MHz, MeOD) δ 8.46 (d, J = 5.2 Hz, 1H), 8.22 (d, J = 0.9 Hz, 1H), 7.89 (s, 1H), 7.41 (s, 1H), 7.36 (d, J = 1.5 Hz, 1H), 7.17 (d, J = 5.2 Hz, 1H), 5.51 (s, 2H), 4.82 (s, 2H), 4.40 (s, 2H), 3.79 (s, 3H), 2.73 - 2.68 (m, 1H), 2.56 - 2.52 (m, 1H), 2.47 (d, J = 1.5 Hz, 6H), 1.99 - 1.94 (m, 1H), 1.69 - 1.65 (m, 2H), 1.01 - 0.97 (m, 2H), 0.77 - 0.73 (m, 2H). |
| **551** | 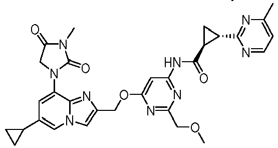 (1S,2S)-N-(6-((6-cyclopropyl-8-(3-methyl-2,4-dioxoimidazolidin-1-yl)imidazo[1,2-a]pyridin-2-yl) methoxy)-2-(methoxymethyl) pyrimidin-4-yl)-2-(4-methyl pyrimidin-2-yl)cyclopropane-1-carboxamide **(I-551)** | **Intermediate 879** |
| | | ESI-MS (M+H)+: 598.1, δ 11.31 (s, 1H), 8.53 (d, J = 5.1 Hz, 1H), 8.28 (s, 1H), 7.96 (s, 1H), 7.39 (d, J = 4.3 Hz, 2H), 7.21 (d, J = 5.1 Hz, 1H), 5.48 (s, 2H), 4.89 (s, 2H), 4.43 (s, 2H), 3.37 (s, 3H), 2.97 (s, 3H), 2.69-2.65(m, 1H), 2.54-2.52 (m, 1H), 2.41 (s, 3H), 1.99-1.95 (m, 1H), 1.56 - 1.53(m, 2H), 0.97-0.93 (m, 2H), 0.68-0.65 (m, 2H). |
| **552** | 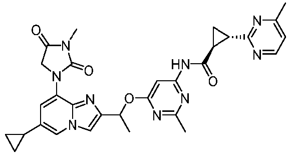 (1S,2S)-N-(6-(1-(6-cyclopropyl-8-(3-methyl-2,4-dioxoimidazolidin-1-yl)imidazo[1,2-a]pyridin-2-yl) ethoxy)-2-methylpyrimidin-4-yl)-2-(4-methylpyrimidin-2-yl)cyclo propane-1-carboxamide (**I-552**) | **Intermediate 880** ESI-MS (M+H)+: 582.2, δ 11.14 (s, 1H), 8.53 (d, J = 5.1 Hz, 1H), 8.23 (s, 1H), 7.86 (d, J = 2.2 Hz, 1H), 7.38 (s, 1H), 7.29 (s, 1H), 7.21 (d, J = 5.0 Hz, 1H), 6.38 (d, J = 6.6 Hz, 1H), 4.92 (s, 2H), 2.97 (d, J = 1.2 Hz, 3H), 2.66 - 2.59 (m, 1H), 2.56-2.54 (m, 1H), 2.43 (s, 3H), 2.41 (s, 3H), 2.02 - 1.89 (m, 1H), 1.67 (d, J = 6.5 Hz, 3H), 1.58-1.50 (m, 2H), 0.97-0.94 (m, 2H), 0.66-0.63 (m, 2H). |
| **553** | 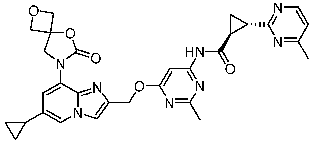 (1S,2S)-N-(6-((6-cyclopropyl-8-(6-oxo-2,5-dioxa-7-azaspiro[3.4]octan-7-yl)imidazo[1,2-a]pyridin-2-yl) methoxy)-2-methylpyrimidin-4-yl)-2-(4-methylpyrimidin-2-yl)cyclo propane-1-carboxamide **(I-553)** | **Intermediate 883** ESI-MS (M+H)+: 583.2, δ 11.18 (s, 1H), 8.53 (d, J = 5.1 Hz, 1H), 8.28 (d, J = 1.1 Hz, 1H), 7.95 (s, 1H), 7.33 - 7.27 (m, 2H), 7.21 (d, J = 5.1 Hz, 1H), 5.47 (s, 2H), 4.89 - 4.78 (m, 4H), 4.73 (s, 2H), 2.69 - 2.61 (m, 1H), 2.58 - 2.53 (m, 1H), 2.47 (s, 3H), 2.41 (s, 3H), 1.98-1.93 (m, 1H), 1.58-1.50 (m, 2H), 0.98 - 0.90 (m, 2H), 0.69 - 0.61 (m, 2H). |
| **554** | 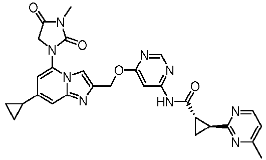 (1S,2S)-N-(6-((7-cyclopropyl-5-(3-methyl-2,4-dioxoimidazolidin-1-yl)imidazo[1,2-a]pyridin-2-yl) methoxy)pyrimidin-4-yl)-2-(4-methylpyrimidin-2-yl)cyclopropane-1-carboxamide (**I-554**) | **Intermediate 884** |
| | | ESI-MS (M+H)+: 554.1, δ 11.19 (s, 1H), 8.55 (d, J = 0.7 Hz, 1H), 8.53 (d, J = 5.1 Hz, 1H), 7.99 (s, 1H), 7.48 (d, J = 0.7 Hz, 1H), 7.34 (s, 1H), 7.22 (d, J = 5.1 Hz, 1H), 6.82 (d, J = 1.4 Hz, 1H), 5.46 (s, 2H), 4.51 (s, 2H), 2.97 (s, 3H), 2.69 - 2.63 (m, 1H), 2.59 - 2.54 (m, 1H), 2.42 (s, 3H), 2.07-1.97 (m, 1H), 1.60 - 1.50 (m, 2H), 1.07-0.98 (m, 2H), 0.83 - 0.73 (m, 2H). |
| **555** | 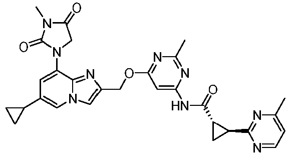 (1S,2S)-N-(6-((6-cyclopropyl-8-(3-methyl-2,4-dioxoimidazolidin-1-yl)imidazo[1,2-a]pyridin-2-yl) methoxy)-2-methylpyrimidin-4-yl)-2-(4-methylpyrimidin-2-yl)cyclo propane-1-carboxamide **(I-555)** | 1-(2-(((6-chloro-2-methylpyrimidin-4-yl)oxy) methyl)-6-cyclopropylimidazo[1,2-a]pyridin-8-yl)-3-methylimidazolidine-2,4-dione |
| | | ESI-MS (M+H)+: 568.2, δ 11.17 (s, 1H), 8.53 (d, J = 5.1 Hz, 1H), 8.28 (d, J = 1.1 Hz, 1H), 7.95 (s, 1H), 7.38 (d, J = 1.4 Hz, 1H), 7.31 (s, 1H), 7.21 (d, J = 5.1 Hz, 1H), 5.46 (s, 2H), 4.90 (s, 2H), 2.97 (s, 3H), 2.66 - 2.61 (m, 1H), 2.57 - 2.54 (m, 1H), 2.46 (s, 3H), 2.41 (s, 3H), 1.98 - 1.94 (m, 1H), 1.58 - 1.51 (m, 2H), 0.98 - 0.93 (m, 2H), 0.68 - 0.64 (m, 2H). |
| 556 | 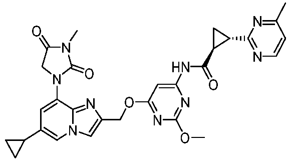 (1S,2S)-N-(6-((6-cyclopropyl-8-(3-methyl-2,4-dioxoimidazolidin-1-yl)imidazo[1,2-a]pyridin-2-yl) methoxy)-2-methoxypyrimidin-4-yl)-2-(4-methylpyrimidin-2-yl)cyclo propane-1-carboxamide (**I-556**) | 1-(2-(((6-chloro-2-methoxypyrimidin-4-yl)oxy) methyl)-6-cyclopropylimidazo[1,2-a]pyridin-8-yl)-3-methylimidazolidine-2,4-dione |
| | | ESI-MS (M+H)+: 584.2, δ 11.09 (s, 1H), 8.53 (d, J = 5.1 Hz, 1H), 8.29 (s, 1H), 7.95 (s, 1H), 7.38 (d, J = 1.5 Hz, 1H), 7.21 (d, J = 5.1 Hz, 1H), 7.14 (s, 1H), 5.46 (s, 2H), 4.89 (s, 2H), 3.89 (s, 3H), 2.97 (s, 3H), 2.70 - 2.64 (m, 2H), 2.41 (s, 3H), 1.98 - 1.95 (m, 1H), 1.58 - 1.52 (m, 2H), 0.98 - 0.93 (m, 2H), 0.70 - 0.64 (m, 2H). ESI-MS [M +H] +: 581, |
| **557** | 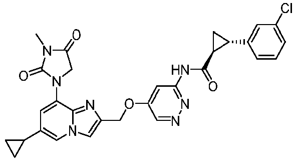 (1S,2S)-2-(3-chlorophenyl)-N-(5-((6-cyclopropyl-8-(3-methyl-2,4-dioxo imidazolidin-1-yl)imidazo[1,2-a] pyridin-2-yl)methoxy)pyridazin-3-yl) cyclopropane-1-carboxamide (**I-557**) | **Intermediate 887** |
| | | ESI-MS [M+H]+: 572.2, δ 11.33 (s, 1H), 8.77 (d, J = 2.6 Hz, 1H), 8.45 (s, 1H), 8.30 (s, 1H), 8.26 (d, J = 2.4 Hz, 1H), 8.02 (s, 1H), 7.41 (s, 1H), 7.32 (d, J = 7.9 Hz, 1H), 7.28 (s, 1H), 7.17 (d, J = 7.5 Hz, 1H), 5.37 (s, 2H), 4.93 (s, 2H), 2.98 (s, 3H), 2.45 - 2.38 (m, 2H), 1.98-1.97 (m, 1H), 1.57 - 1.49 (m, 1H), 1.48-1.47 (m, 1H), 0.97-0.95 (m, 2H), 0.67-0.66 (m, 2H). |
| **558** | 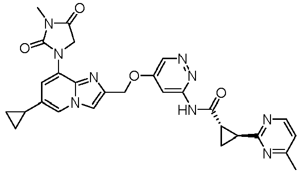 (1S,2S)-N-(5-((6-cyclopropyl-8-(3-methyl-2,4-dioxoimidazolidin-1-yl)imidazo[1,2-a]pyridin-2-yl) methoxy)pyridazin-3-yl)-2-(4-methyl pyrimidin-2-yl)cyclopropane-1-carboxamide (**I-558**) | **Intermediate 887** |
| | | ESI-MS [M +H]+: 554.2 δ 11.39 (s, 1H), 8.77 (d, J = 2.7 Hz, 1H), 8.54 (d, J = 5.1 Hz, 1H), 8.30 (d, J = 1.3 Hz, 1H), 8.21 (d, J = 2.7 Hz, 1H), 8.02 (s, 1H), 7.41 (d, J = 1.5 Hz, 1H), 7.22 (d, J = 5.1 Hz, 1H), 5.37 (s, 2H), 4.93 (d, J = 4.4 Hz, 2H), 2.98 (s, 3H), 2.72 - 2.66 (m, 1H), 2.60 - 2.55 (m, 1H), 2.42 (s, 3H), 2.00 - 1.95 (m, 1H), 1.61 - 1.52 (m, 2H), 1.01 - 0.92 (m, 2H), 0.71 - 0.63 (m, 2H). |
| **559** | 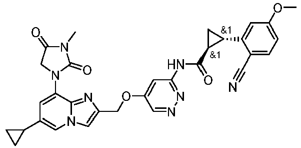 *rac*-(1S*,2S*)-2-(2-cyano-5-methoxy phenyl)-N-(5-((6-cyclopropyl-8-(3-methyl-2,4-dioxoimidazolidin-1-yl) imidazo[1,2-a]pyridin-2-yl)methoxy) pyridazin-3-yl)cyclopropane-1-carboxamide (**I-559**) | **Intermediate 887**ESI-MS [M +H]⁺: 593.2. δ 11.41 (s, 1H), 8.78 (d, J = 2.7 Hz, 1H), 8.30 (s, 1H), 8.26 (d, J = 2.5 Hz, 1H), 8.03 (s, 1H), 7.75 (d, J = 8.6 Hz, 1H), 7.42 (d, J = 1.3 Hz, 1H), 6.99 (dd, J = 8.7, 2.4 Hz, 1H), 6.80 (d, J = 2.4 Hz, 1H), 5.38 (s, 2H), 4.92 (s, 2H), 3.86 (s, 3H), 2.96 (s, 3H), 2.64 - 2.62 (m, 2H), 2.03 - 1.89 (m, 1H), 1.59 (t, J = 7.2 Hz, 2H), 1.01 - 0.89 (m, 2H), 0.75 - 0.60 (m, 2H). |
| **560** | 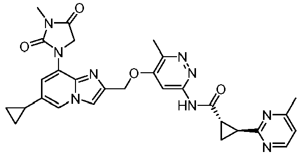 (1S,2S)-N-(5-((6-cyclopropyl-8-(3-methyl-2,4-dioxoimidazolidin-1-yl)imidazo[1,2-a]pyridin-2-yl) methoxy)-6-methylpyridazin-3-yl)-2-(4-methylpyrimidin-2-yl)cyclo propane-1-carboxamide **(I-560)** | **Intermediate 888** |
| | | ESI-MS [M +H] ⁺: 568.2. δ 11.26 (s, 1H), 8.51 (d, J = 5.1 Hz, 1H), 8.27 (s, 1H), 8.20 (s, 1H), 7.99 (s, 1H), 7.39 (d, J = 1.5 Hz, 1H), 7.19 (d, J = 5.1 Hz, 1H), 5.33 (s, 2H), 4.97 - 4.87 (m, 2H), 2.96 (s, 3H), 2.65 - 2.63 (m, 1H), 2.52 - 2.51 (m, 1H), 2.39 (d, J = 2.5 Hz, 6H), 1.97 - 1.92 (m, 1H), 1.53 (t, J = 9.9 Hz, 2H), 0.94 (t, J = 7.4 Hz, 2H), 0.67 - 0.61 (m, 2H). |
| **561** | 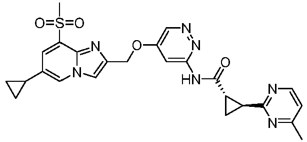 (1S,2S)-N-(5-((6-cyclopropyl-8-(methylsulfonyl)imidazo[1,2-a] pyridin-2-yl)methoxy)pyridazin-3-yl)-2-(4-methylpyrimidin-2-yl)cyclo propane-1-carboxamide **(I-561)** | **Intermediate 889** ESI-MS [M +H]+: 520.2. δ 11.42 (s, 1H), 8.83 (d, J = 2.7 Hz, 1H), 8.71 (d, J = 1.2 Hz, 1H), 8.54 (d, J = 5.1 Hz, 1H), 8.18 - 8.09 (m, 2H), 7.63 (d, J = 1.7 Hz, 1H), 7.23 (d, J = 5.1 Hz, 1H), 5.44 (s, 2H), 3.53 (s, 3H), 2.73 - 2.67 (m, 1H), 2.62 - 2.56 (m, 2H), 2.43 (s, 3H), 2.14 - 2.06 (m, 1H), 1.63 - 1.56 (m, 2H), 1.03 - 0.95 (m, 2H), 0.78 - 0.72 (m, 2H). |

### Example 562: (1S,2S)-N-(6-((6-cyclopropyl-8-(3-(2-hydroxy-2-methylpropyl)-2,4-dioxoimidazolidin-1-yl)imidazo[1,2-a]pyridin-2-yl)methoxy)-2-methylpyrimidin-4-yl)-2-(4-methylpyrimidin-2-yl)cyclopropane-1-carboxamide (I-562)

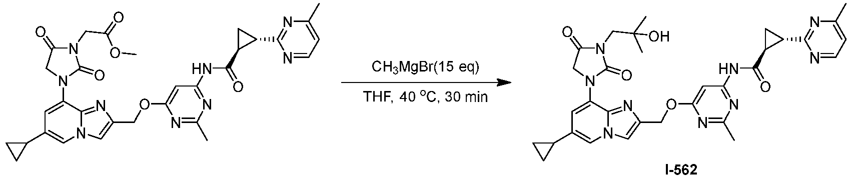

To a mixture of methyl 2-(3-(6-cyclopropyl-2-(((2-methyl-6-((1S,2S)-2-(4-methylpyrimidin-2-yl)cyclopropane-1-carboxamido)pyrimidin-4-yl)oxy)methyl)imidazo[1,2-a]pyridin-8-yl)-2,5-dioxoimidazolidin-1-yl)acetate (105 mg, 0.17 mmol) in THF (3 mL) was added CH₃MgBr (0.85 mL, 2.55 mmol) at 0 °C. The mixture was stirred at 40 °C for 30 min. The mixture was quenched with sat. NH₄Cl (10 mL) and extracted with EtOAc (3 x 20 mL). Then the combined organic layers were concentrated *in vacuo* to give the crude product, which was purified by preparative TLC (eluent: DCM/MeOH = 12/1) to give **(I-562)** (3.4 mg, 3%) as a white solid. ESI-MS (M+H)+: 626.3, ¹H NMR (400 MHz, MeOD) δ 8.46 (d, J = 5.2 Hz, 1H), 8.23 (s, 1H), 7.90 (s, 1H), 7.39 (s, 1H), 7.33 (d, J = 1.4 Hz, 1H), 7.17 (d, J = 5.2 Hz, 1H), 5.51 (s, 2H), 4.69 (s, 2H), 3.63 (s, 2H), 2.73 - 2.68 (m, 1H), 2.56 - 2.51 (m, 1H), 2.47 (d, J = 1.5 Hz, 6H), 2.03 - 1.94 (m, 2H), 1.67 - 1.63 (m, 1H), 1.27 (s, 6H), 1.02 - 0.97 (m, 2H), 0.77 - 0.73 (m, 2H)..

### Example 563: (1S,2S)-N-(6-((6-cyclopropyl-8-(3-methyl-2,4-dioxoimidazolidin-1-yl)imidazo[1,2-a]pyridin-2-yl)methoxy)-2-(2-(dimethylamino)ethoxy)pyrimidin-4-yl)-2-(4-methylpyrimidin-2-yl)cyclopropane-1-carboxamide (I-563)

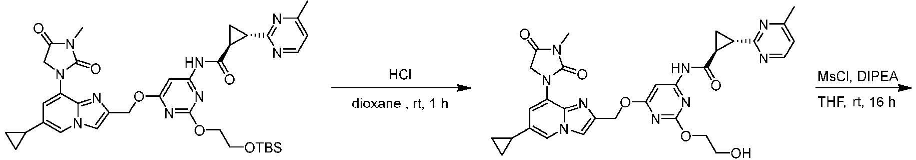

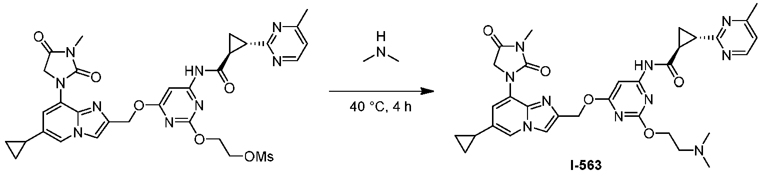

To the mixture of (1S,25)-N-(2-(2-((tert-butyldimethylsilyl)oxy)ethoxy)-6-((6-cyclopropyl-8-(3-meth yl-2,4-dioxoimidazolidin-1-yl)imidazo[1,2-a]pyridin-2-yl)methoxy)pyrimidin-4-yl)-2-(4-methylpyri midin-2-yl)cyclopropane-1-carboxamide (90 mg, 0.13 mmol) (prepared in a similar manner to methyl 2-(3-(6-cyclopropyl-2-(((2-methyl-6-((1S,2S)-2-(4-methylpyrimidin-2-yl)cyclopropane-1-carboxamido)pyrimidin-4-yl)oxy)methyl)imidazo[1,2-a]pyridin-8-yl)-2,5-dioxoimidazolidin-1-yl)acetate) in dioxane (5 mL) was added HCl/dioxane (4M solution in dioxane, 3 mL) . The reaction mixture was stirred at room temperature for 1 h, concentrated in vacuo, then diluted in DCM/MeOH (30 mL, 10/1) and adjusted to pH = 9~10 by aqueous NaHCO₃ (10 mL). The organics were washed with brine (30 mL), dried over Na₂SO₄, and concentrated in vacuo to give the crude, which was purified by silica gel chromatography (eluent: DCM/MeOH = 10/1) to afford **(1S,2S)-N-(6-((6-cyclopropyl-8-(3-methyl-2,4-dioxoimidazolidin-1-yl)imidazo[1,2-a]pyridin-2-yl)methoxy)-2-(2-hydroxyethoxy) pyrimidin-4-yl)-2-(pyrimidin-2-yl)cyclopropane-1-carboxamide intermediate 890 (60** mg, 79 %) as yellow solid. ESI-MS [M +H]+: 614.2.

To a mixture of **intermediate 890** (30 mg, 0.049 mmol) in THF (2 mL) was added DIPEA (19 mg, 0.147 mmol) and MsCl (14 mg, 0.122 mmol) at 0°C . The reaction mixture was stirred at room temperature for 16 h. The reaction was then quenched with water (10 mL) and extracted with EtOAc (3 x 10 mL). The combined organic layers were washed with brine (10 mL), dried over anhydrous Na₂SO₄, and concentrated in vacuo to afford **2-((4-((6-cyclopropyl-8-(3-methyl-2,4-dioxoimida zolidin-1-yl)imidazo[1,2-a]pyridin-2-yl)methoxy)-6-((1S,2S)-2-(4-methylpyrimidin-2-yl)cyclopropane-1-carboxamido)pyrimidin-2-yl)oxy)ethylmethanesulfonate intermediate 891** (30 mg, 87% ) as a yellow solid, which was used in the next step without purification. ESI-MS [M +H]+: 692.2.

A mixture of **intermediate 891** (30 mg, 0.043 mmol) in dimethylamine (1.5 mL) was stirred at 40 °C for 4 h. The reaction mixture was concentrated in vacuo and purified by preparative TLC(eluent: DCM/MeOH=15/1) to afford **(I-563)** (8 mg, 30%) as a pale solid. ESI-MS (M+H)+: 641.2, δ 11.03 (s, 1H), 8.53 (d, J = 5.1 Hz, 1H), 8.28 (d, J = 1.4 Hz, 1H), 7.93 (s, 1H), 7.38 (d, J = 1.5 Hz, 1H), 7.21 (d, J = 5.1 Hz, 1H), 7.13 (s, 1H), 5.45 (s, 2H), 4.89 (s, 2H), 4.35 (t, J = 5.9 Hz, 2H), 2.97 (s, 3H), 2.67 - 2.63 (m, 1H), 2.59 - 2.54 (m, 3H), 2.42 (s, 3H), 2.17 (s, 6H), 1.98 - 1.95 (m, 1H), 1.57 - 1.52 (m, 2H), 0.98 - 0.93 (m, 2H), 0.68 - 0.64 (m, 2H).
The compound in Table 42 was prepared in a similar manner to **(I-563). Table 42**

| **EG** | **Structure** | **Analvtical Data** |
|---|---|---|
| **564** | 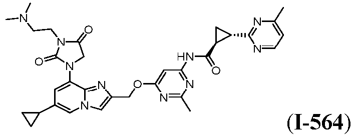 (1S,2S)-N-(6-((6-cyclopropyl-8-(3-(2-(dimethylamino)ethyl)-2,4-dioxo imidazolidin-1-yl)imidazo[1,2-a] pyridin-2-yl)methoxy)-2-methyl pyrimidin-4-yl)-2-(4-methylpyrimidin-2-yl)cyclopropane-1-carboxamide | ESI-MS (M+H)+: 625.3, δ 11.17 (s, 1H), 8.53 (d, *J* = 5.1 Hz, 1H), 8.28 (d, *J* = 1.5 Hz, 1H), 7.95 (s, 1H), 7.37 (d*, J* = 1.5 Hz, 1H), 7.31 (s, 1H), 7.21 (d, *J* = 5.1 Hz, 1H), 5.47 (s, 2H), 4.93 (s, 2H), 3.60 - 3.57 (m, 2H), 2.67 - 2.62 (m, 1H), 2.57 - 2.54 (m, 1H), 2.47 - 2.45 (m, 5H), 2.41 (s, 3H), 2.18 (s, 6H), 1.99 - 1.95 (m, 1H), 1.58 - 1.51 (m, 2H), 0.98 - 0.93 (m, 2H), 0.68 - 0.65 (m, 2H). |

### Intermediate 892:2-(((2-bromopyridin-4-yl)oxy)methyl)-5-cyclopropyl-7-(4-methylpiperazin-1-yl)pyrazolo[1,5-a]pyridine

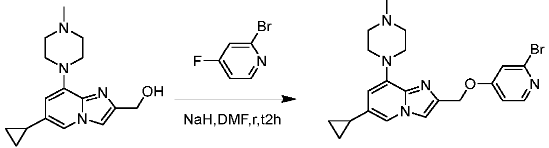

A mixture of (6-cyclopropyl-8-(4-methylpiperazin-1-yl)imidazo[1,2-a]pyridin-2-yl)methanol (80 mg, 0.28 mmol), 2-bromo-4-fluoropyridine (54 mg,0.31 mmol), NaH (33.5 mg,1.4 mmol) and DMF(4 mL) was stirred at room temperature for 2 h. Water (20 mL) was added to the mixture, which was then extracted with EtOAc (3 x 20 mL). The combined organic layers were washed with brine, dried over Na₂SO₄, filtered, and concentrated *in vacuo* to give the crude, which was purified by silica gel chromatography (DCM/MeOH = 20/1) to give **intermediate 892** (80 mg, 65%) as a gray solid. ESI-MS [M +H]+: 442.2.
The compounds in Table 43 were prepared in a similar manner to **intermediate 892. Table 43**

| **Structure** | **Analytical Data** |
|---|---|
| 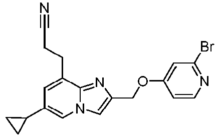 | **3-(2-(((2-bromopyridin-4-yl)oxy)methyl)-6-cyclopropylimidazo[1,2-a]pyridin-8-yl)propanenitrile intermediate 893** ESI-MS [M +H]+: 397.2. |
| 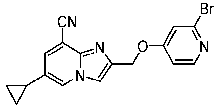 | **2-(((2-bromopyridin-4-yl)oxy)methyl)-6-cyclopropylimidazo[1,2-a]pyridine-8-carbonitrile intermediate 894** ESI-MS [M +H]+: 369.1. |
| 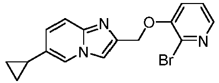 | **2-(((2-bromopyridin-3-yl)oxy)methyl)-6-cyclopropylimidazo[1,2-a]pyridine intermediate 895** ESI-MS [M +H]⁺: 344.2. |
| 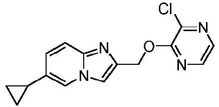 | **2-(((3-chloropyrazin-2-yl)oxy)methyl)-6-cyclopropylimidazo[1,2-a]pyridine intermediate 896** ESI-MS [M +H]+: 301.2. |
| 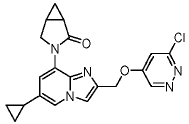 | **3-(2-(((6-chloropyridazin-4-yl)oxy)methyl)-6-cyclopropylimidazo[1,2-a]pyridin-8-yl)-3-azabicyclo[3.1.0]hexan-2-one intermediate 897** ESI-MS [M +H]⁺: 396.2. |

### Example 565: (1S,2S)-2-(3-chlorophenyl)-N-(6-(((5-cyclopropyl-7-(4-methylpiperazin-1-yl)pyrazolo[1,5-a]pyridin-2-yl)methyl)amino)pyrimidin-4-yl)cyclopropane-1-carboxamide (I-565)

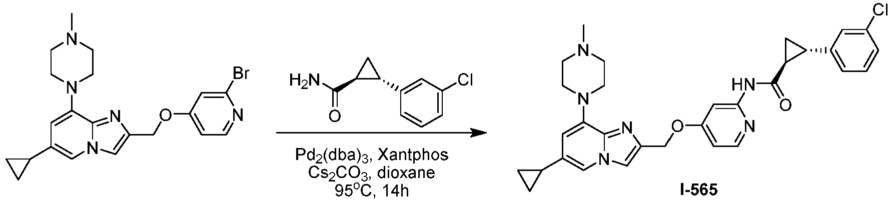

To a mixture of **intermediate 892** (60 mg, 0.135 mmol), (1S,2S)-2-(3-chlorophenyl) cyclopropane-carboxamide (26.3 mg,0.135 mmol), and Cs₂CO₃ (131.9 mg,0.405 mmol) in 1,4-dioxane (5 mL) was added Pd₂(dba)₃ (18.3 mg,0.02 mmol) and xantphos (15.6 mg,0.027 mmol). After stirring at 95 °C for 6 h under N₂, the reaction mixture was filtered through Celite^{®} and the filter cake was washed with DCM/MeOH (10/1, 20 mL). The filtrate was concentrated *in vacuo* to give the crude, which was purified by preparative TLC (DCM/MeOH = 20/1) to give (**I-565**) (20 mg, 27%) as a white solid. ESI-MS [M +H]+: 557.2, δ 10.74 (s, 1H), 8.12 (d, J = 5.8 Hz, 1H), 7.91 (s, 1H), 7.83 (s, 1H), 7.80 (s, 1H), 7.36-7.29 (m, 1H), 7.29 - 7.22 (m, 2H), 7.16 (d, J = 7.6 Hz, 1H), 6.84-6.82 (m, 1H), 6.18 (s, 1H), 5.22 (s, 2H), 2.45-2.40 (m, 4H), 2.53-2.50 (m, 4H), 2.47 - 2.35 (m, 2H), 2.23 (s, 3H),1.90-1.84 (m, 1H), 1.56 -1.47 (m, 1H), 1.43-1.39 (m, 1H), 0.94 - 0.84 (m, 2H), 0.74 - 0.66 (m, 2H).

The compounds in Table **44** were prepared in a similar manner to **(I-565)** from an appropriate cyclopropyl amide and the indicated coupling partner. **Table 44**

| **EG** | **Structure** | **Coupling Partner / Analytical Data** |
|---|---|---|
| **566** | 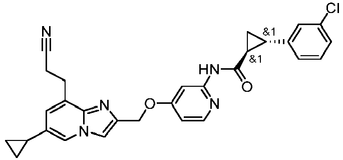 *rac-*(1S*,2S*)-2-(3-chlorophenyl)-N-(4-((8-(2-cyanoethyl)-6-cyclo propyl imidazo[1,2-a]pyridin-2-yl)methoxy) pyridin-2-yl)cyclopropane-1-carboxamide (**I-566**) | **Intermediate 893** ESI-MS [M +H]+: 512.2, δ 10.75 (s, 1H), 8.29 (s, 1H), 8.12 (d, J = 5.8 Hz, 1H), 7.91 (s, 1H), 7.84 (d, J = 1.8 Hz, 1H), 7.38 - 7.28 (m, 1H), 7.29 - 7.23 (m, 2H), 7.16 (d, J = 7.7 Hz, 1H), 6.97 (s, 1H), 6.83 - 6.82 (m, 1H), 5.25 (s, 2H), 3.16 (t, J = 7.0 Hz, 2H), 3.06 (t, J = 6.8 Hz, 2H), 2.45 - 2.35 (m, 2H), 1.96 - 1.91 (m, 1H), 1.53 - 1.46 (m, 1H), 1.45 - 1.35 (m, 1H), 0.99 - 0.91 (m, 2H), 0.73 - 0.68 (m, 2H). |
| 567 | 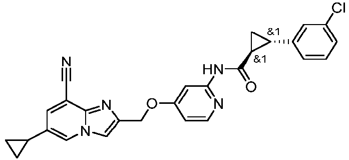 *rac-*(1S*,2S*)-2-(3-chlorophenyl)-N-(4-((8-cyano-6-cyclopropyl imidazo[1,2-a]pyridin-2-yl)methoxy) pyridin-2-yl)cyclopropane-1-carboxamide (**I-567**) | **Intermediate 894** ESI-MS [M +H]⁺: 484.2. δ 10.76 (s, 1H), 8.71 (s, 1H), 8.14 (d, J = 5.7 Hz, 1H), 8.10 (d, J = 27.8 Hz, 1H), 7.81 (d, J = 9.0 Hz, 2H), 7.37 - 7.22 (m, 3H), 7.16 (d, J = 7.6 Hz, 1H), 6.84 (d, J = 7.6 Hz, 1H), 5.30 (s, 2H), 2.42 - 2.40 (m, 2H), 2.07 - 1.90 (m, 1H), 1.59 - 1.47 (m, 1H), 1.43 - 1.37 (m, 1H), 0.99 - 0.94 (m, 2H), 0.80 - 0.76 (m, 2H). |
| **568** | 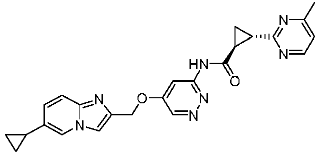 (1S,2S)-N-(5-((6-cyclopropylimidazo [1,2-a]pyridin-2-yl)methoxy) pyridazin-3-yl)-2-(4-methylpyrimidin-2-yl)cyclopropane-1-carboxamide (**I-568**) | **Intermediate 709** ESI-MS [M +H]+: 442.2 δ 11.41 (s, 1H), 8.81 (d, J = 2.7 Hz, 1H), 8.54 (d, J = 5.1 Hz, 1H), 8.37 (s, 1H), 8.05 (d, J = 2.7 Hz, 1H), 7.93 (s, 1H), 7.44 (d, J = 9.4 Hz, 1H), 7.22 (d, J = 5.1 Hz, 1H), 7.02 (dd, J = 9.4, 1.7 Hz, 1H), 5.34 (s, 2H), 2.73 - 2.66 (m, 1H), 2.64 - 2.56 (m, 1H), 2.43 (s, 3H), 2.00 - 1.90 (m, 1H), 1.63 - 1.53 (m, 2H), 0.96 - 0.89 (m, 2H), 0.74 - 0.63 (m, 2H). |
| **569** | 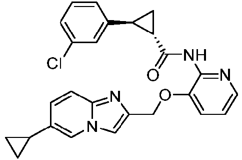 (1S,2S)-2-(3-chlorophenyl)-N-(3-((6-cyclopropylimidazo[1,2-a]pyridin-2-yl)methoxy)pyridin-2-yl)cyclo propane-1-carboxamide **(I-569)** | **Intermediate 895** |
| | | ESI-MS [M +H] +: 459.1. δ 9.98 (s, 1H), 8.33 (s, 1H), 7.94 (d, J = 3.9 Hz, 1H), 7.84 (s, 1H), 7.65 (d, J = 7.5 Hz, 1H), 7.41 (d, J = 9.3 Hz, 1H), 7.34 - 7.15 (m, 4H), 7.08 (d, J = 7.3 Hz, 1H), 6.99 (dd, J = 9.4 Hz, 1.4 Hz, 1H), 5.24 (s, 2H), 2.38 - 2.27 (m, 2H), 1.95 - 1.91 (m, 1H), 1.48 - 1.44 (m, 1H), 1.39 - 1.35 (m, 1H), 0.95 - 0.90 (m, 2H), 0.70 - 0.66 (m, 2H). |
| **570** | 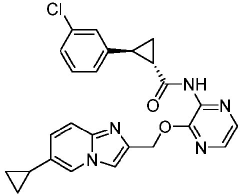 (1S,2S)-2-(3-chlorophenyl)-N-(3-((6-cyclopropylimidazo[1,2-a]pyridin-2-yl)methoxy)pyrazin-2-yl)cyclo propane-1-carboxamide **(I-570)** | **Intermediate 896** ESI-MS [M +H]⁺: 460.1. ¹H NMR (400 MHz, DMSO-d6) δ 10.30 (s, 1H), 8.33 (s, 1H), 8.02 (dd, *J* = 8.4, 2.8 Hz, 2H), 7.84 (s, 1H), 7.41 (d, *J =* 9.3 Hz, 1H), 7.31 - 7.22 (m, 2H), 7.22 - 7.20 (m, 1H), 7.10 (dd, *J* = 7.3, 1.5 Hz, 1H), 6.99 (dd, *J* = 9.4, 1.7 Hz, 1H), 5.49 (s, 2H), 2.40 - 2.30 (m, 2H), 1.97 - 1.89 (m, 1H), 1.51 - 1.45 (m, 1H), 1.43 - 1.37 (m, 1H), 0.95 - 0.89 (m, 2H), 0.71 - 0.65 (m, 2H). |
| **571** | 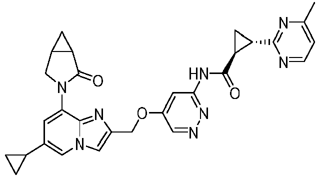 (1S,2S)-N-(5-((6-cyclopropyl-8-(2-oxo-3-azabicyclo[3.1.0]hexan-3-yl) imidazo[1,2-a]pyridin-2-yl)methoxy) pyridazin-3-yl)-2-(4-methylpyrimidin-2-yl)cyclopropane-1-carboxamide (**I-571**) | **Intermediate 897** ESI-MS [M +H]⁺: 537.3, δ 11.41 (s, 1H), 8.79 (d, J = 2.6 Hz, 1H), 8.54 (d, J = 5.1 Hz, 1H), 8.28 (s, 1H), 8.18-8.15 (m, 1H), 7.97 (s, 1H), 7.22 (d, J = 5.1 Hz, 1H), 7.11 (s, 1H), 5.35 (s, 2H), 4.35-4.29 (m, 1H), 4.02 (d, J = 9.8 Hz, 1H), 2.74 - 2.66 (m, 1H), 2.65 - 2.58 (m, 1H), 2.43 (s, 3H), 2.12-2.05 (m, 1H), 2.03-1.97 (m, 1H), 1.95-1.89 (m, 1H), 1.60-1.54 (m, 2H), 1.22-1.14 (m, 1H), 0.97 - 0.89 (m, 2H), 0.86-0.80 (m, 1H), 0.68-0.65 (m, 2H). |

### Example 572: rac-(1S*,2S*)-N-(4-((8-(aminomethyl)-6-cyclopropylimidazo[1,2-a]pyridin-2-yl)methoxy)pyridin-2-yl)-2-(3-chlorophenyl)cyclopropane-1-carboxamide (I-572)

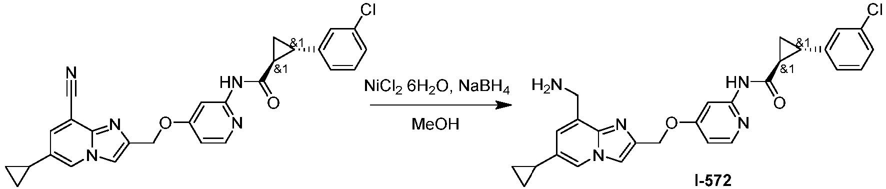

(**I-572**) was prepared from rac-(1S*,2S*)-2-(3-chlorophenyl)-N-(4-((8-cyano-6-cyclopropylimidazo[1,2-a]pyridin-2-yl)methoxy)pyridin-2-yl)cyclopropane-1-carboxamide using a similar method to that used for the synthesis of (**I-293**). ESI-MS [M +H]+: 488.2, δ 10.76 (s, 1H), 8.32 (s, 1H), 8.13 (d, J = 5.6 Hz, 1H), 7.93 (s, 1H), 7.84 (s, 1H), 7.34 - 7.27 (m, 3H), 7.16 (d, J = 7.5 Hz, 1H), 7.06 (s, 1H), 6.82 (d, J = 4.0 Hz, 1H), 5.25 (s, 2H), 4.13 (s, 2H), 2.47 - 2.33 (m, 2H), 2.00 - 1.83 (m, 1H), 1.58 - 1.46 (m, 1H), 1.45 - 1.36 (m, 1H), 1.05 - 0.82 (m, 2H), 0.82 - 0.50 (m, 2H).

### Example 573: (1S,2S)-N-(4-((6-cyclopropyl-8-(3-methyl-2,4-dioxoimidazolidin-1-yl)imidazo[1,2-a]pyridin-2-yl)methoxy)-6-methy1-1,3,5-triazin-2-yl)-2-(4-methylpyrimidin-2-yl)cyclopropane-1-carboxamide (I-573)

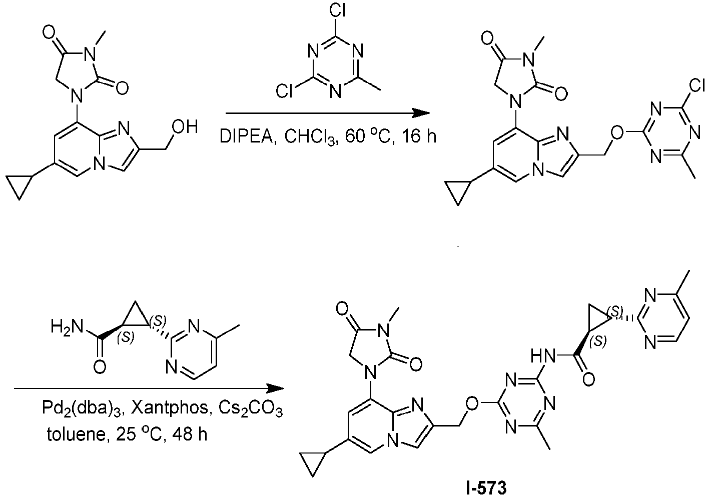

To a mixture of **intermediate 454** (200 mg, 0.66 mmol) and 2,4-dichloro-6-methyl-1,3,5-triazine (538 mg, 3.3 mmol) in CHCl₃ (10 mL) was added DIPEA (256 mg, 1.98 mmol). The mixture was stirred at 60 °C for 16 h. Water (20 mL) was added, and the reaction mixture was extracted with DCM (3 x 20 mL). The combined organic layers were washed with brine, dried over Na₂SO₄, filtered, and concentrated in vacuo to give the crude, which was purified by silica gel chromatography (DCM/MeOH = 30/1) to afford **1-(2-(((4-chloro-6-methyl-1,3,5-triazin-2-yl) oxy)methyl)-6-cyclopropylimidazo[1,2-a]pyridin-8-yl)-3-methylimidazolidine-2,4-dione intermediate 898** (176 mg, yield 62%) as pale yellow solid. ESI-MS [M +H]+: 428.1.

(**I-573**) was prepared in a similar manner to **(I-565)** from **intermediate 898** and **intermediate 55.** ESI-MS [M +H]+: 569.2. δ 11.21 (s, 1H), 8.49 (d, J = 5.1 Hz, 1H), 8.28 (d, J = 1.2 Hz, 1H), 7.98 (s, 1H), 7.38 (d, J = 1.5 Hz, 1H), 7.16 (d, J = 5.1 Hz, 1H), 5.44 (s, 2H), 4.88 (s, 2H), 3.02 - 2.95 (m, 4H), 2.59 - 2.56 (m, 1H), 2.38 - 2.37 (m, 6H), 1.99 - 1.95 (m, 1H), 1.60 - 1.57 (m, 2H), 0.98 - 0.93 (m, 2H), 0.69 - 0.65 (m, 2H).

### Intermediate 900: rac-(1S*,2S*)-N-(5-aminopyridin-3-yl)-2-(3-chlorophenyl)cyclopropane-1-carboxamide

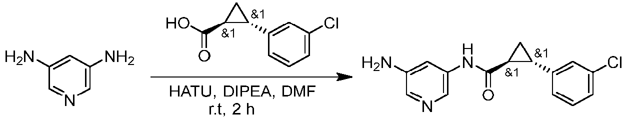

A mixture of pyridine-3,5-diamine (30 mg, 0.275 mmol), *intermediate 1149* (54 mg, 0.275 mmol), HATU (125 mg, 0.33 mmol), and DIPEA (178 mg, 1.38 mmol) in DMF (2 mL) was stirred at room temperature for 2 h. The reaction mixture was poured into water (30 mL) and extracted with EtOAc (2 x 30 mL). The combined organics were washed with water (3 x 60 mL) and brine (60 mL), dried over anhydrous Na₂SO₄, then concentrated *in vacuo* to give the crude product, which was purified by silica gel chromatography (eluent: EtOAc) to give **intermediate 900** (50 mg, 63%) as a yellow solid. ESI-MS [M + H]⁺: 288.1.

The compound in Table 45 was made in a similar manner to **intermediate 900. Table 45**

| **Intermediate** | **Name/Analvtical Data** |
|---|---|
| 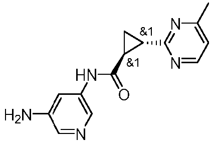 | ***rac-*(1S*,2S*)-N-(5-aminopyridin-3-yl)-2-(4-methylpyrimidin-2-yl)cyclopropane-1-carboxamide intermediate 901** ESI-MS [M + H]+: 270.2. |

### Example 574: rac-(1S*,2S*)-2-(3-chlorophenyl)-N-(5-(((6-cyclopropylimidazo[1,2-a]pyridin-2-yl)methyl)amino)pyridin-3-yl)cyclopropane-1-carboxamide (I-574)

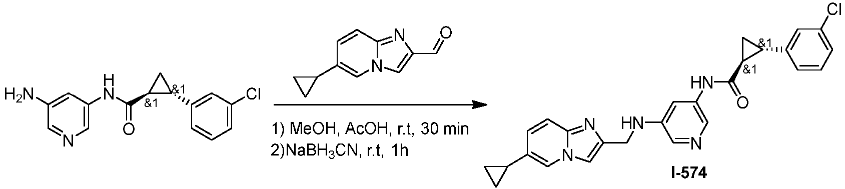

A mixture of **intermediate 900** (50 mg, 0.174 mmol), **intermediate 828** (32 mg, 0.174 mmol) and AcOH (0.2 mL) in MeOH (5 mL) was stirred at room temperature for 30 min. NaBH₃CN (55 mg, 0.87 mmol) was added to the mixture, which was stirred at room temperature for 1 h. The reaction mixture was poured into water (30 mL), basified to pH = 9~10 with saturated aq. NaHCO₃, and extracted with EtOAc (2 x 30 mL). The combined organics were washed with brine (60 mL), dried over anhydrous Na₂SO₄, and concentrated *in vacuo* to give the crude product, which was purified by preparative HPLC to give (I-574) (27 mg, 34%) as a pale solid. ESI-MS [M + H]+: 458.2. δ 10.16 (s, 1H), 8.28 (s, 1H), 7.97 (d, J = 1.5 Hz, 1H), 7.71 (d, J = 2.2 Hz, 1H), 7.61 (s, 1H), 7.35 (d, J = 9.3 Hz, 1H), 7.32 - 7.20 (m, 4H), 7.14 (d, J = 7.6 Hz, 1H), 6.94 (dd, J = 9.3, 1.3 Hz, 1H), 6.47 (t, J = 5.8 Hz, 1H), 4.30 (d, J = 5.7 Hz, 2H), 2.39 - 2.33 (m, 1H), 2.10 - 2.04 (m, 1H), 1.92 - 1.83 (m, 1H), 1.49 - 1.35 (m, 2H), 0.92 - 0.85 (m, 2H), 0.66 - 0.61 (m, 2H).

The compound in Table 46 was prepared in a similar manner to **(I-574). Table 46**

| **EG** | **Structure** | **Coupling Partner / Analytical Data** |
|---|---|---|
| **575** | 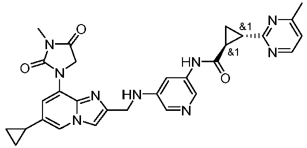 *rac-*(1S*,2S*)-N-(5-(((6-cyclopropyl-8-(3-methyl-2,4-dioxoimidazolidin-1-yl)imidazo[1,2-a]pyridin-2-yl)methyl)amino)pyridin-3-yl)-2-(4-methylpyrimidin-2-yl)cyclopropane-1-carboxamide **(I-575)** | **Intermediate 901** ESI-MS [M +H]+: 552.2. δ 10.25 (s, 1H), 8.50 (d, J = 5.1 Hz, 1H), 8.23 (d, J = 1.0 Hz, 1H), 7.92 (d, J = 8.2 Hz, 1H), 7.71 - 7.69 (m, 2H), 7.37 - 7.32 (m, 2H), 7.19 (d, J = 5.1 Hz, 1H), 6.50 (t, J = 5.9 Hz, 1H), 4.90 (s, 2H), 4.33 (d, J = 5.8 Hz, 2H), 2.96 (s, 3H), 2.53 - 2.49 (m, 2H), 2.40 (s, 3H), 1.95 - 1.88 (m, 1H), 1.51 - 1.44 (m, 2H), 0.94 - 0.85 (m, 2H), 0.68 - 0.61 (m, 2H). |

### Intermediate 902: 1-(2-((4-chloro-2H-pyrazolo[4,3-c]pyridin-2-yl)methyl)-6-cyclopropylimidazo[1,2-a]pyridin-8-yl)-3-methylimidazolidine-2,4-dione

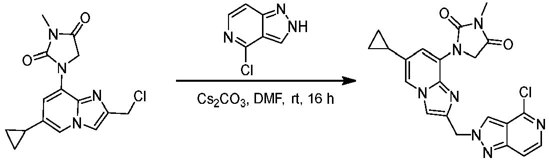

A mixture of **intermediate 451** (420 mg, 1.32 mmol), 4-chloro-2H-pyrazolo[4,3-c]pyridine (200 mg,1.32 mmol) and Cs₂CO₃ (1.3 g, 3.96 mmol) in DMF (8 mL) was stirred at 25 °C for 16 h. Water (50 mL) was added, then the reaction was extracted with EtOAc (3 x 100 mL). The combined organic layers were washed with brine (30 mL), dried over anhydrous Na₂SO₄, and concentrated *in vacuo* to give the crude, which was purified by preparative HPLC to give **intermediate 902** (90 mg, 16%) as a yellow solid. ESI-MS [M +H]+: 436.1.
The compounds in Table 47 were prepared in a similar manner to **intermediate 902. Table 47**

| **Intermediate** | **Analytical Data** |
|---|---|
| 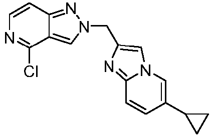 | **4-chloro-2-((6-cyclopropylimidazo[1,2-a]pyridin-2-yl)methyl)-2H-pyrazolo[4,3-c]pyridine intermediate 903** ESI-MS [M +H]+: 324.2 |
| 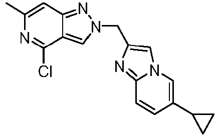 | **4-chloro-2-((6-cyclopropylimidazo[1,2-a]pyridin-2-yl)methyl)-6-methyl-2H-pyrazolo[4,3-c]pyridine intermediate 904** ESI-MS [M +H]+: 338.2 |
| 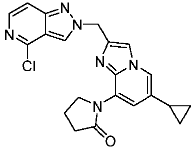 | **1-(2-((4-chloro-2H-pyrazolo[4,3-c]pyridin-2-yl)methyl)-6-cyclopropylimidazo[1,2-a]pyridin-8-yl)pyrrolidin-2-one intermediate 905** ESI-MS [M +H]+: 407.1 |
| 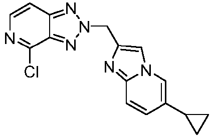 | **4-chloro-2-((6-cyclopropylimidazo[1,2-a]pyridin-2-yl)methyl)-2H-[1,2,3]triazolo[4,5-c]pyridine intermediate 906** ESI-MS [M +H]+: 325.2 |
| 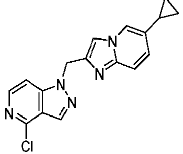 | **4-chloro-1-((6-cyclopropylimidazo[1,2-a]pyridin-2-yl)methyl)-1H-pyrazolo[4,3-c]pyridine intermediate 907** ESI-MS [M +H]+: 324.1 |

### Example 576

### rac-(1S*,2S*)-N-(2-((6-cyclopropyl-8-(3-methyl-2,4-dioxoimidazolidin-1-yl)imidazo[1,2-a]pyridin-2-yl)methyl)-2H-pyrazolo[4,3-c]pyridin-4-yl)-2-(4-methylpyrimidin-2-yl)cyclopropane-1-carboxamide (I-576)

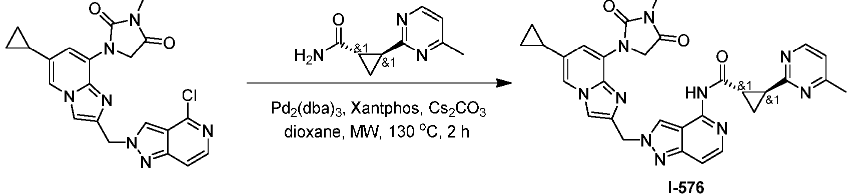

To a mixture of **intermediate 902** (25 mg, 0.05 mmol), *rac-(1S*,2S*)-2-(4-*methylpyrimidin-2-yl)cyclopropane-1-carboxamide (10.2 mg, 0.05 mmol), and Cs₂CO₃ ( 56 mg , 0.15 mmol) in dioxane (3 mL) were added Pd₂(dba)₃ (15.7 mg, 0.015 mmol) and xantphos (20 mg, 0.03 mmol). The mixture was stirred at 90 °C for 2 h under N₂, then cooled to room temperature. The reaction mixture was filtered through Celite and the filter cake was washed with DCM/MeOH (10/1, 50 mL). The filtrate was concentrated *in vacuo* to give the crude, which was purified by preparative TLC (eluent: DCM/MeOH = 10/1) to give **(I-576)** (5 mg, 33%) as a white solid. ESI-MS [M +H]+: 577.2. δ 11.04 (s, 1H), 8.85 (s, 1H), 8.54 (d, J=5.1, 1H), 8.46 (s, 1H), 8.29 (s, 1H), 7.89 (s, 1H), 7.86 (d, J=5.8, 1H), 7.39 (d, J=1.4, 1H), 7.25 (d, J=5.9, 1H), 7.22 (d, J=5.1, 1H), 5.77 (s, 2H), 4.88 (s, 2H), 2.96 (s, 3H), 2.76-2.65 (m, 2H), 2.43 (s, 3H), 1.98-1.93 (m, 1H), 1.63-1.57 (m, 2H), 0.98-0.92 (m, 2H), 0.67 - 0.63 (m, 2H).

The compounds in Table **48** were prepared in a similar manner to **(I-576)** using the indicated coupling partner. **Table 48**

| **EG** | **Structure** | **Coupling Partner / Analytical Data** |
|---|---|---|
| **577** | 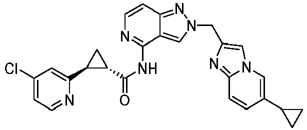 (1S,2S)-2-(4-chloropyridin-2-yl)-N-(2-((6-cyclopropylimidazo[1,2-a]pyridin-2-yl)methyl)-2H-pyrazolo[4,3-c]pyridin-4-yl)cyclopropane-1-carboxamide (**I-577**) | **Intermediate 903** ESI-MS [M +H]+: 484.1. δ 11.02 (s, 1H), 8.78 (s, 1H), 8.44 (d, J=5.4, 1H), 8.35 (s, 1H), 7.89 - 7.83 (m, 1H), 7.83 (s, 1H), 7.67 (d, J=1.8, 1H), 7.41-7.38 (m, 1H), 7.37-7.35 (m, 1H), 7.27-7.20 (m, 1H), 7.02-6.98 (m, 1H), 5.74 (s, 2H), 2.70- 2.66 (m, 2H), 1.94-1.89 (m, 1H), 1.60-1.54 (m, 2H), 0.94 - 0.89 (m, 2H), 0.69 - 0.65 (m, 2H). |
| **578** | 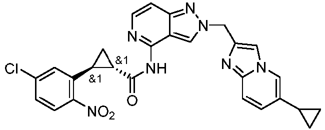 *rac*-(1S*,2S*)-2-(5-chloro-2-nitrophenyl)-N-(2-((6-cyclopropyl imidazo[1,2-a]pyridin-2-yl)methyl)-2H-pyrazolo[4,3-c]pyridin-4-yl)cyclo propane-1-carboxamide **(I-578)** | **Intermediate 903** ESI-MS [M + H]+: 528.2, δ 11.02 (s, 1H), 8.82 (s, 1H), 8.35 (s, 1H), 8.05 (d, J = 9.2 Hz, 1H), 7.91 - 7.80 (m, 2H), 7.62-7.60 (m, 2H), 7.39 (d, J = 9.3 Hz, 1H), 7.25 (d, J = 6.1 Hz, 1H), 7.01 - 6.98 (m, 1H), 5.75 (s, 2H), 2.81 - 2.76 (m, 1H), 2.51 - 2.44 (m, 1H), 1.97 - 1.87 (m, 1H), 1.63 - 1.50 (m, 2H), 0.96 - 0.82 (m, 2H), 0.69 - 0.65 (m, 2H). |
| **579** | 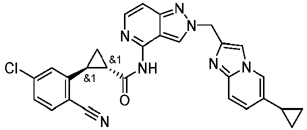 *rac-*(1S*,2S*)-2-(5-chloro-2-cyano phenyl)-N-(2-((6-cyclopropylimidazo [1,2-a]pyridin-2-yl)methyl)-2H-pyrazolo[4,3-c]pyridin-4-yl)cyclo propane-1-carboxamide **(I-579)** | **Intermediate 903** ESI-MS [M + H] ⁺: 508.2. δ 11.04 (s, 1H), 8.81 (s, 1H), 8.34 (s, 1H), 7.88 - 7.82 (m, 3H), 7.53 (dd, J = 8.3, 1.9 Hz, 1H), 7.45 (s, 1H), 7.39 (d, J = 9.3 Hz, 1H), 7.25 (d, J = 6.2 Hz, 1H), 7.00 (dd, J = 9.4, 1.8 Hz, 1H), 5.75 (s, 2H), 2.70 - 2.65 (m, 2H), 1.95 - 1.88 (m, 1H), 1.66 - 1.62 (m, 2H), 0.94 - 0.89 (m, 2H), 0.69 - 0.65 (m, 2H). |
| **580** | 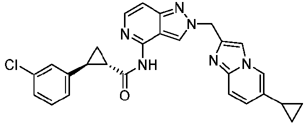 (1S,2S)-2-(3-chlorophenyl)-N-(2-((6-cyclopropylimidazo[1,2-a]pyridin-2-yl) methyl)-2H-pyrazolo[4,3-c]pyridin-4-yl)cyclopropane-1-carboxamide (**I-580**) | **Intermediate 903** ESI-MS [M+ H]⁺: 483.1. δ 10.98 (s, 1H), 8.80 (s, 1H), 8.35 (s, 1H), 7.86 - 7.83 (m, 2H), 7.41 - 7.17 (m, 6H), 7.00 (dd, J = 9.4, 1.7 Hz, 1H), 5.74 (s, 2H), 2.52 - 2.51 (m, 1H), 2.47 - 2.44 (m, 1H), 1.92 - 1.89 (m, 1H), 1.58 - 1.44 (m, 2H), 0.94 - 0.89 (m, 2H), 0.69 - 0.65 (m, 2H). |
| **581** | 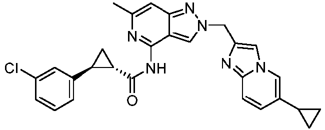 (1S,2S)-2-(3-chlorophenyl)-N-(2-((6-cyclopropylimidazo[1,2-a]pyridin-2-yl) methyl)-6-methyl-2H-pyrazolo[4,3-c] pyridin-4-yl)cyclopropane-1-carboxamide **(I-581)** | **Intermediate 904** |
| | | ESI-MS [M +H]⁺: 497.2. δ 10.98 (s, 1H), 8.73 (s, 1H), 8.35 (s, 1H), 7.80 (s, 1H), 7.40 - 7.28 (m, 4H), 7.19 - 7.16 (m, 1H), 7.04 - 6.98 (m, 2H), 5.69 (s, 2H), 2.49 - 2.44 (m, 2H), 2.38 (s, 3H), 1.95 - 1.90 (m, 1H), 1.56 - 1.51 (m, 1H), 1.46 - 1.41 (m, 1H), 0.94 - 0.89 (m, 2H), 0.69 - 0.65 (m, 2H). |
| **582** | 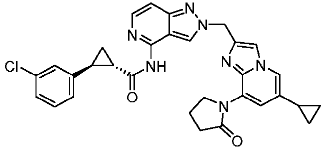 (1S,2S)-N-(5-((6-cyclopropyl-8-(3-methyl-2,4-dioxoimidazolidin-1-yl) imidazo[1,2-a]pyridin-2-yl)methoxy) pyridazin-3-yl)-2-(4-methylpyrimidin-2-yl)cyclopropane-1-carboxamide (**I-582**) | **Intermediate 905** ESI-MS [M + H] ⁺: 566.1. δ 10.98 (s, 1H), 8.86 (s, 1H), 8.28 (d, J = 1.2 Hz, 1H), 7.867 - 7.85 (m, 2H), 7.35 - 7.17 (m, 6H), 5.76 (s, 2H), 4.14 (t, J = 7.1 Hz, 2H), 2.47 - 2.42 (m, 4H), 2.15 - 2.08 (m, 2H), 1.96 - 1.90 (m, 1H), 1.56 - 1.44 (m, 2H), 0.95 - 0.91 (m, 2H), 0.67 - 0.63 (m, 2H). |
| **583** | 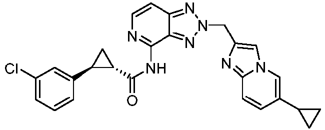 (1S,2S)-2-(3-chlorophenyl)-N-(2-((6-cyclopropylimidazo[1,2-a]pyridin-2-yl) methyl)-2H-[1,2,3]triazolo[4,5-c] pyridin-4-yl)cyclopropane-1-carboxamide (**I-583**) | **Intermediate 906** ESI-MS (M+H)+: 484.1, δ 11.04 (s, 1H), 8.40 (s, 1H), 8.33 (s, 1H), 8.10 (s, 1H), 7.89 (s, 1H), 7.61 (s, 1H), 7.35 - 7.15 (m, 5H), 6.98 (d, J = 9.4 Hz, 1H), 6.07 (s, 2H), 2.44 - 2.36 (m, 2H), 1.95 - 1.86 (m, 1H), 1.57 - 1.48 (m, 1H), 1.44 - 1.40 (m, 1H), 0.95 - 0.85 (m, 2H), 0.69 - 0.60 (m, 2H). |
| **584** | 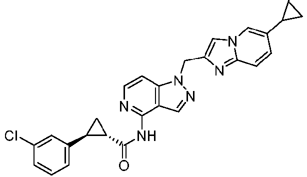 (1S,2S)-2-(3-chlorophenyl)-N-(1-((6-cyclopropylimidazo[1,2-a]pyridin-2-yl) methyl)-1H-pyrazolo[4,3-c]pyridin-4-yl)cyclopropane-1-carboxamide (**I-584**) | **Intermediate 907** ESI-MS [M + H]⁺: 483.1. δ 10.99 (s, 1H), 8.31 (d, J = 0.8 Hz, 1H), 8.26 - 8.26 (m, 1H), 8.04 (d, J = 6.0 Hz, 1H), 7.62 (s, 1H), 7.50-7.48 (m, 1H), 7.34 - 7.23 (m, 4H), 7.18 - 7.16 (m, 1H), 6.96-6.93 (m, 1H), 5.68 (s, 2H), 1.99 - 1.99 (m, 1H), 1.91 - 1.83 (m, 1H), 1.57 - 1.53 (m, 1H), 1.47 - 1.42 (m, 2H), 0.90 - 0.85 (m, 2H), 0.64 - 0.60 (m, 2H). |

### Examples 585-586

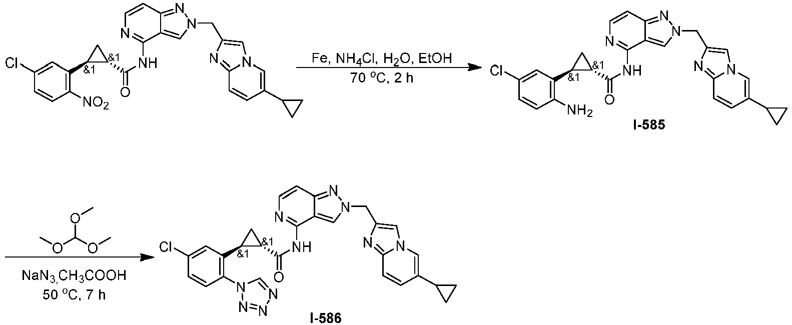

rac-**(1S*,2S*)-2-(2-amino-5-chlorophenyl)-N-(2-((6-cyclopropylimidazo[1,2-a]pyridin-2-yl)methyl)-2H-pyrazolo[4,3-c]pyridin-4-yl)cyclopropane-1-carboxamide (I-585)** was prepared in a similar manner to (I-222) from rac-(1S*,2S*)-2-(5-chloro-2-nitrophenyl)-N-(2-((6-cyclopropylimidazo[1,2-a]pyridin-2-yl)methyl)-2H-pyrazolo[4,3-c]pyridin-4-yl)cyclopropane-1-carboxamide. ESI-MS [M+H]⁺: 498.2; δ 11.07 (s, 1H), 8.83 (s, 1H), 8.35 (s, 1H), 7.88 - 7.83 (m, 2H), 7.39 (d, J = 9.3 Hz, 1H), 7.25 (d, J = 6.0 Hz, 1H), 7.05 - 6.90 (m, 3H), 6.65 (d, J = 8.5 Hz, 1H), 5.74 (s, 2H), 5.14 (s, 2H), 2.41 - 2.32 (m, 1H), 2.26 - 2.21 (m, 1H), 1.97 - 1.87 (m, 1H), 1.55 - 1.46 (m, 1H), 1.19 - 1.11 (m, 1H), 0.97 - 0.81 (m, 2H), 0.72 - 0.62 (m, 2H).
rac**-(1S*,2S*)-2-(5-chloro-2-(1H-tetrazol-1-yl)phenyl)-N-(2-((6-cyclopropylimidazo[1,2-a]pyridin-2-yl)methyl)-2H-pyrazolo[4,3-c]pyridin-4-yl)cyclopropane-1-carboxamide (I-586)** was prepared in a similar manner to (**I-287**) **(I-585).** ESI-MS [M+H]⁺: 551.3, ¹H NMR (400 MHz, MeOD) δ 9.54 (s, 1H), 8.67 (s, 1H), 8.21 (s, 1H), 7.84 (s, 2H), 7.55 - 7.52 (m, 3H), 7.40 (d, J = 9.3 Hz, 1H), 7.26 (d, J = 6.1 Hz, 1H), 7.14 - 7.07 (m, 1H), 5.78 (s, 2H), 2.37 - 2.30 (m, 1H), 2.16 - 2.10 (m, 1H), 1.96 - 1.92 (m, 1H), 1.53 - 1.44 (m, 2H), 1.00 - 0.94 (m, 2H), 0.73 - 0.69 (m, 2H).

### Example 587: rac-(1S*,2S*)-2-(3-chlorophenyl)-N-(6-((6-cyclopropylimidazo[1,2-a]pyridin-2-yl)methyl)pyrimidin-4-yl)cyclopropane-1-carboxamide (I-587)

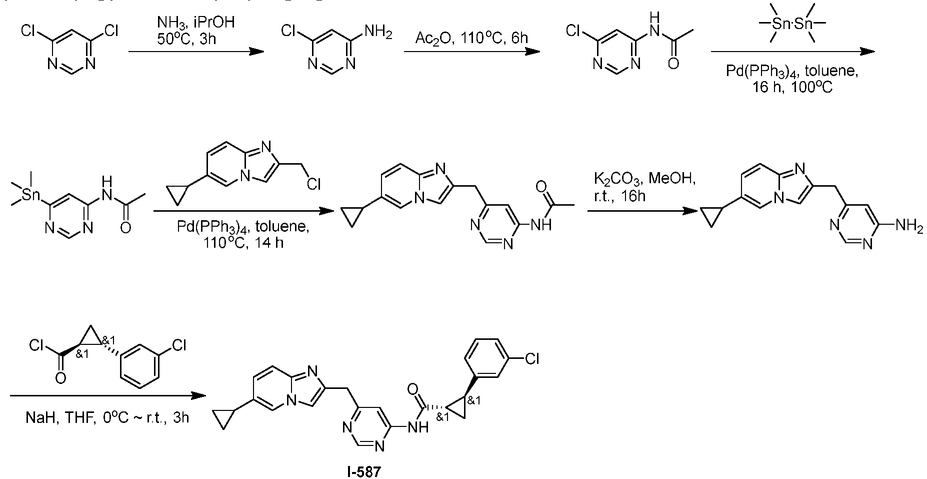

A mixture of 4,6-dichloropyrimidine (3.2 g, 21 mmol) and NH₄OH (15 mL) in i-PrOH (15 mL) was stirred at 50 °C for 3 h in a sealed tube. The reaction mixture was concentrated *in vacuo* to give **6-chloropyrimidin-4-amine intermediate 846** (2.8 g, quant.) as a white solid. ESI-MS [M + H]⁺: 130.1.

A mixture of **intermediate 846(2.8** g, 21 mmol) in acetic anhydride (20 mL) was stirred at 110 °C for 6 h. The reaction mixture was cooled to room temperature and concentrated *in vacuo* to give the residue, which was diluted in EtOAc (150 mL) and washed with saturated aq. NaHCO₃ (60 mL). The organic layer was washed with brine (60 mL), dried over Na₂SO₄, and concentrated *in vacuo* to give the crude product, which was purified by silica gel column chromatography (elue nt: PE/EtOAc = 2/1) to give **N-(6-chloropyrimidin-4-yl)acetamide intermediate 908** (2.9 g, 79%) as a yellow solid. ESI-MS [M + H]⁺: 172.0.

A mixture of **intermediate 908** (2.0 g, 12 mmol), 1,1,1,2,2,2-hexamethyldistannane (7.6 g, 23 mmol) and Pd(PPh₃)₄ (1.4 g, 1.2 mmol) in toluene (100 mL) was stirred at 100 °C for 6 h. After cooling to room temperature, the reaction mixture was filtered and the filtrate was concentrated *in vacuo* to give the crude product, which was purified by column chromatography on neutral Al₂O₃ (eluent: PE/EtOAc = 2/1) to give **N-(6-(trimethylstannyl)pyrimidin-4-yl)acetamide intermediate 909** (3.2 g, 91%) as a white solid. ESI-MS [M + H]⁺: 302.0.

A mixture of **intermediate 909** (2.4 g, 8.0 mmol), 2-(chloromethyl)-6-cyclopropylimidazo[1,2-a]pyridine (1.7 g, 8.0 mmol) and Pd(PPh₃)₄ (0.92 g, 0.80 mmol) in toluene (100 mL) was stirred at 110 °C for 14 h. The reaction mixture was cooled to room temperature and concentrated *in vacuo* to give the crude product, which was purified by column chromatography on silica gel column chromatography (eluent: DCM/MeOH = 20/1) to give **N-(6-((6-cyclopropylimidazo[1,2-a]pyridin-2-yl)methyl)pyrimidin-4-yl)acetamide intermediate 910** (0.20 g, 8%) as a yellow solid. ESI-MS [M + H]⁺: 308.2.

To a stirred solution of **intermediate 910** (0.20 g, 0.65 mmol) in MeOH (6.0 mL) was added K₂CO₃ (0.18 g, 1.3 mmol) and the mixture was stirred at room temperature for 16 h. The reaction mixture was concentrated *in vacuo* to give the crude, which was diluted in water (15 mL), extracted with DCM/MeOH (10/1, 4 x 30 mL). The combined organics was washed with brine (30 mL), dried over Na₂SO₄ and concentrated *in vacuo* to give the crude product, which was purified by silica gel column chromatography (eluent: DCM/MeOH = 15/1) to afford **6-((6-cyclopropylimidazo[1,2-a]pyridin-2-yl)methyl)pyrimidin-4-amine intermediate 911** (120 mg, 70%) as a yellow solid. ESI-MS [M + H]⁺: 266.2.

To a stirred solution of **intermediate 911** (70 mg, 0.26 mmol) in THF (4.0 mL) was added NaH (21 mg, 0.53 mmol, 60% dispersion in mineral oil) in one portion at 0 °C. After 10 min, a solution of rac-(1S*,2S*)-2-(3-chlorophenyl)cyclopropane-1-carbonyl chloride (110 mg, 0.53 mmol) in THF (2 mL) was added dropwise. After stirring at room temperature for 3 h, the reaction mixture was quenched with water (20 mL) and extracted with EtOAc (2 x 50 mL). The combined organics was washed with brine (40 mL), dried over Na₂SO₄, and concentrated in vacuo to give the crude product, which was purified by preparative HPLC to give (I-587)(7.0 mg, 6%) as a white solid.
ESI-MS [M + H]⁺: 444.1 δ 11.12 (s, 1H), 8.76 (s, 1H), 8.32 (s, 1H), 7.98 (s, 1H), 7.67 (s, 1H), 7.35 - 7.25 (m, 4H), 7.14 (d, *J* = 7.6 Hz, 1H), 6.96 (d, *J* = 9.3 Hz, 1H), 4.10 (s, 2H), 2.44 - 2.33 (m, 2H), 1.96 - 1.89 (m, 1H), 1.52 - 1.41 (m, 2H), 0.97 - 0.86 (m, 2H), 0.70 - 0.66 (m, 2H).

### Example 588: rac-(1S*,2S*)-2-(3-chlorophenyl)-N-((1-((6-cyclopropylimidazo[1,2-a]pyridin-2-yl)methyl)-1H-1,2,3-triazol-4-yl)methyl)cyclopropane-1-carboxamide (I-588)

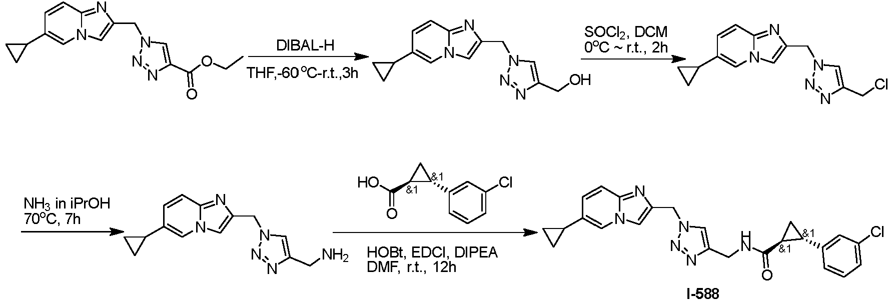

To a mixture of ethyl 1-((6-cyclopropylimidazo[1,2-a]pyridin-2-yl)methyl)-1H-1,2,3-triazole-4-carboxylate (815 mg, 2.62 mmol) in THF (10 mL) was added DIBALH (5.3 mL, 5.3 mmol, 1M in THF) at -60 °C. The mixture was warmed to room temperature and stirred for 3 h. The mixture was quenched with NH₄Cl (sat. aq., 20 mL), filtered through Celite^{®}, and the filter cake was washed with EtOAc (30 mL x 3). The aqueous filtrate was extracted with EtOAc (20 mL x 3). The combined organic layers were washed with brine (50 mL), dried over Na₂SO₄, and concentrated *in vacuo.* The residue was purified by column chromatography (eluent: EtOAc/PE from 0 to 30%) to afford **(1-((6-cyclopropylimidazo[1,2-a]pyridin-2-yl)methyl)-1H-1,2,3-triazol-4-yl)methanol intermediate 912** (425 mg, 60% yield) as a white solid. ESI-MS [M +H] +: 270.1

To a solution of **intermediate 912** (300 mg, 1.1 mmol) in DCM (20 mL) was added SOCl₂ (2 mL) at 0 °C. The resulting reaction was stirred at room temperature for 2 h and concentrated *in vacuo* to give crude **2-((4-(chloromethyl)-1H-1,2,3-triazol-1-yl)methyl)-6-cyclopropylimidazo[1,2-a]pyridine intermediate** 913 (320 mg crude), which was used into next step without further purification. ESI-MS [M +H]+: 288.1.

A solution of **intermediate 913** (320 mg crude from previous step) in NH₃/iPrOH (7M, 70 mL) was stirred in a sealed tube at 70 °C for 7h. The reaction was cooled to room temperature and concentrated *in vacuo* to give the crude, which was purified with silica gel column chromatography (eluent: DCM/MeOH = 10/1) to give **(1-((6-cyclopropylimidazo[1,2-a]pyridin-2-yl)methyl)-1H-1,2,3-triazol-4-yl)methanamine intermediate 914** (130 mg, 44% over 2 steps). ESI-MS [M +H]+: 269.2.

A mixture of **intermediate 914** (100 mg, 0.73 mmol), intermediate 1149 (143 mg, 0.73 mmol), HOBt (148.5 mg, 1.1 mmol), EDCI (211 mg, 1.1 mmol), and DIPEA (282.5 mg, 2.19 mmol) in DMF (5 mL) was stirred at room temperature for 12 h. The resulting mixture was diluted with water (30 mL) and extracted with EtOAc (3 x 30 mL). The combined organic layers were washed with brine (30 mL), dried over Na₂SO₄, and concentrated in vacuo to give the crude, which was purified with preparative TLC (eluent: DCM/MeOH = 10/1) to give **(I-588)** (25 mg, 8%) as a white solid. ESI-MS [M +H]+: 447.2.. δ 8.59 (t, J = 5.0 Hz, 1H), 8.35 (s, 1H), 7.92 (s, 1H), 7.82 (s, 1H), 7.40 (d, J = 9.3 Hz, 1H), 7.28 (t, J = 7.7 Hz, 1H), 7.22 (d, J = 8.0 Hz, 1H), 7.18 (s, 1H), 7.09 (d, J = 7.5 Hz, 1H), 7.01 (d, J = 9.4 Hz, 1H), 5.63 (s, 2H), 4.32 (d, J = 5.4 Hz, 2H), 2.31-2.24 (m, 1H), 1.95-1.88 (m, 2H), 1.39-1.31(m, 1H), 1.25-1.23 (m, 1H), 0.96-0.88 (m, 2H), 0.71-0.64 (m, 2H).

### Examples 589-590

### rac-(1S*,2S*)-2-(3-chlorophenyl)-N-(1-(1-((6-cyclopropylimidazo[1,2-a]pyridin-2-yl)methyl)-1H-1,2,3-triazol-4-yl)ethyl)cyclopropane-1-carboxamide

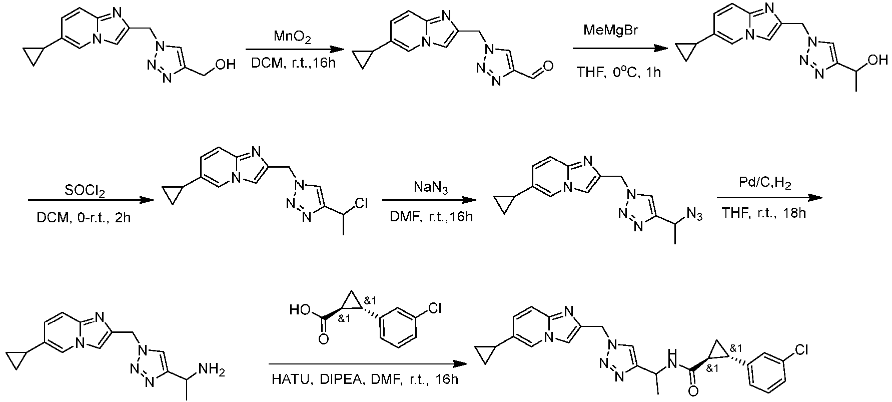

A mixture of **intermediate 912** (3.0 g, 11.1 mmol) and MnO₂ (9.7 g, 111 mmol) in DCM (50 mL) was stirred at room temperature for 16 h. The reaction mixture was filtered through Celite^{®} and the filter cake was washed with DCM/MeOH (10/1, 100 mL). The filtrate was concentrated *in vacuo* to give the crude, which was purified by column chromatography (eluent: EtOAc/PE from 0 to 30%) to afford **1-((6-cyclopropylimidazo[1,2-a]pyridin-2-yl)methyl)-1H-1,2,3-triazole-4-carbaldehyde intermediate 915** (2.2 g, 73% yield) as a white solid. ESI-MS [M +H] +: 268.2

To a mixture of **intermediate 915** (2.0 g, 7.5 mmol) in THF (100 mL) was added methylmagnesium bromide (5.0 mL, 15.0 mmol, 3M in THF) at 0 °C, and the mixture was stirred at 0 °C for 1 h. The mixture was quenched with NH₄Cl (sat. aq., 100 mL) and extracted with EtOAc (50 mLx 3). The combined organic layers were washed with brine (50 mL), dried over Na₂SO₄, and concentrated. The residue was purified by column chromatography (eluent: EtOAc/PE from 0 to 50%) to afford **1-(1-((6-cyclopropylimidazo[1,2-a]pyridin-2-yl)methyl)-1H-1,2,3-triazol-4-yl)ethan-1-ol intermediate 916** (1.5 g, 71% yield) as a white solid. ESI-MS [M +H] +: 284.1

To a mixture of **intermediate 916** (500 mg, 1.77 mmol) in DCM (10 mL) was added SOCl₂ (1 mL) at 0 °C and the mixture was stirred at room temperature for 2 h. The mixture was concentrated *in vacuo* to afford **2-((4-(1-chloroethyl)-1H-1,2,3-triazol-1-yl)methyl)-6-cyclopropylimidazo[1,2-a]pyridine intermediate 917** (600 mg, crude) as a yellow oil which was used in the next step. ESI-MS [M +H] +: 302.1

A mixture of **intermediate 917** (600 mg, 1.77 mmol), NaN₃ (230 mg, 3.54 mmol) in DMF (10 mL) was stirred at room temperature for 16 h. The mixture was quenched with water (100 mL) and extracted with EtOAc (30 mL x 3). The combined organic layers were washed with brine (30 mL), dried over Na₂SO₄ and concentrated *in vacuo.* The residue was purified by flash column chromatography (eluent: EtOAc/PE from 0 to 10%) to afford **2-((4-(1-azidoethyl)-1H-1,2,3-triazol-1-yl)methyl)-6-cyclopropylimidazo[1,2-a]pyridine intermediate 918** (500 mg, 92% yield, for 2 steps) as a yellow oil. ESI-MS [M +H] +: 309.1

A mixture of **intermediate 918** (250 mg, 0.81 mmol) and Pd/C (50 mg) in THF (10 mL) was stirred at room temperature for 18 h. The mixture was filtered and the filtrate was concentrated *in vacuo* to give **1-(1-((6-cyclopropylimidazo[1,2-a]pyridin-2-yl)methyl)-1H-1,2,3-triazol-4-yl)ethan-1-amine intermediate 919** (150 mg, crude) as a brown oil which was used in the next step directly. ESI-MS [M +H] +: 283.2

**Synthesis of rac-(1S*,2S*)-2-(3-chlorophenyl)-N-(1-(1-((6-cyclopropylimidazo[1,2-a]pyridin-2-yl)methyl)-1H-1,2,3-triazol-4-yl)ethyl)cyclopropane-1-carboxamide.** To a mixture of **intermediate 919** (60 mg, 0.21 mmol), intermediate 1149 (42 mg, 0.21 mmol), DIPEA (54 mg, 0.42 mmol) in DMF (5 mL) was added HATU (96 mg, 0.25 mmol) and the mixture was stirred at room temperature for 18 h. The mixture was quenched with water (50 mL) and extracted with EtOAc (30 mL x 3). The combined organic layers were washed with brine (30 mL), dried over Na₂SO₄ and concentrated *in vacuo.* The residue was purified by preparative TLC (eluent: EtOAc/PE= 25%) to afford isomer 1 (12 mg, 12 % yield) and isomer 2 (13 mg, 13 % yield) as white solids. ESI-MS [M +H] +: 461.2
Isomer 1(I-589):ESI-MS [M +H]+: 461.2.. δ 8.53 (d, J = 8.1 Hz, 1H), 8.35 (d, J = 8.4 Hz, 1H), 7.90 (d, J = 2.3 Hz, 1H), 7.83 (d, J = 8.4 Hz, 1H), 7.40 (dd, J = 12.6, 9.3 Hz, 1H), 7.31 - 7.26 (m, 1H), 7.25 - 7.21 (m, 1H), 7.18-7.14 (m, 1H), 7.19 - 7.06 (m, 1H), 7.04 - 6.98 (m, 1H), 5.62 (d, J = 7.3 Hz, 2H), 5.12 - 5.02 (m, 1H), 2.31 - 2.22 (m, 1H), 2.01 - 1.94 (m, 2H), 1.50-1.35 (m, 4H), 1.38-1.25 (m, 1H), 0.95 - 0.87 (m, 2H), 0.71 - 0.63 (m, 2H).
Isomer 2 (I-590): ESI-MS [M +H]+: 461.2, δ 8.56 - 8.52 (m, 2H), 8.05 - 7.94 (m, 2H), 7.59 - 7.51 (m, 1H), 7.37 - 7.06 (m, 5H), 5.79 - 5.75 (m, 2H), 5.11 - 5.04 (m, 1H), 2.31 - 2.24 (m, 1H), 2.04 - 1.91 (m, 2H), 1.44 - 1.31 (m, 4H), 1.26 - 1.20 (m, 1H), 1.02 - 0.95 (m, 2H), 0.76 - 0.68 (m, 2H).

### Example 591: (1S,2S)-2-(3-chlorophenyl)-N-((1-((6-cyclopropyl-8-(4H-1,2,4-triazol-4-yl)imidazo[1,2-a]pyridin-2-yl)methyl)-1H-1,2,3-triazol-4-yl)methyl)cyclopropane-1-carboxamide (I-591)

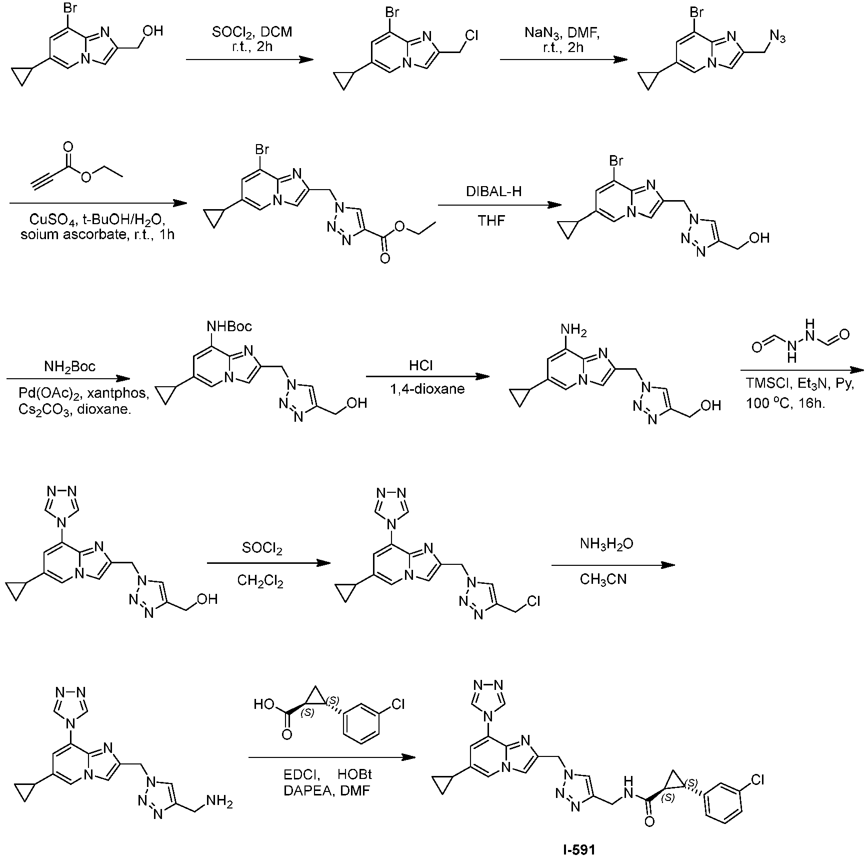

A solution of **intermediate 453** (2 g, 7.5 mmol) and COCl₂ (2 mL) in DCM (25 mL) was stirred at room temperature for 2 h. The reaction was concentrated *in vacuo* to give 8-bromo-2-**(chloromethyl)-6-cyclopropylimidazo[1,2-a]pyridine intermediate 920** (2.2 g, crude) as yellow oil, which was used without further purification. ESI-MS [M +H]+: 285.2

To a solution of intermediate **920** (2.2 g, crude) in DMF (20 mL) was added NaN₃ (975 mg, 15 mmol). The resulting reaction mixture was stirred at room temperature for 2 h. The reaction was diluted with H₂O (200 mL) and extracted with EtOAc (100 mL x 3). The combined organic layers were washed with brine (100 mL), dried over anhydrous Na₂SO₄ and concentrated *in vacuo* to give crude, which was purified by column chromatography (eluent: EtOAc/PE from 0 to 40%) to give 2-**(azidomethyl)-8-bromo-6-cyclopropylimidazo[1,2-a]pyridine** intermediate 921 (1.8 g, 82% yield over 2 steps) ESI-MS [M +H]+: 292.2

To a mixture of intermediate 921 (1.8 g, 6.2 mmol) in t-BuOH/H₂O (20 mL / 20 mL) was added CuSO₄ (192 mg, 1.2 mmol) and sodium ascorbate ( 238 mg, 1.2 mmol). The resulting reaction was stirred at room temperature for 1 h. The reaction was diluted with H₂O (50 mL) and extracted with EtOAc (50 mL x 3). The combined organic layers were washed with brine (50 mL), dried over anhydrous Na₂SO₄, and concentrated *in vacuo* to give the crude, which was purified by column chromatography (eluent: EtOAc/PE from 0 to 50%) to give ethyl 1-((8-bromo-6-**cyclopropylimidazo[1,2-a]pyridin-2-yl)methyl)-1H-1,2,3-triazole-4-carboxylate** intermediate 922 (2.1 g, 88%) as a yellow solid. ESI-MS [M +H]+: 390.2

To a solution of intermediate 922 (828 mg, 2.13 mmol) in dry THF ( 20 mL) at - 60 °C was added DIBAL-H (6.39 mL, 6.39 mmol, 1M in THF) under N₂, The mixture was stirred at - 60 °C for 1 h and then warmed to room temperature for 2 h. The mixture was quenched with NH₄Cl (sat. aq., 50 mL), filtered through Celite^{®}, and the filter cake was washed with EtOAc (50 mL x 3). The filtrate was extracted with EtOAc (50 mL x 3). The combined organic layers were washed with brine (50 mL), dried over Na₂SO₄ and concentrated *in vacuo.* The residue was purified by column chromatography (eluent: DCM/MeOH = 0 - 5%) to give **(1-((8-bromo-6-cyclopropylimidazo[1,2-**a]pyridin-2-yl)methyl)-1H-1,2,3-triazol-4-yl)methanol intermediate 923 as a white solid (600 mg, 81%). ESI-MS [M +H]+: 348.1.

To a mixture of **intermediate 923** (450 mg, 1.30 mmol) in dioxane (10 mL) was added tert-butyl carbamate (228 mg, 1.95 mmol), Pd(OAc)₂ (29 mg, 0.13 mmol), Xantphos(75 mg, 0.13 mmol), and Cs₂CO₃ (1.27 g, 3.9 mmol), and the mixture was stirred at 95 °C for 16 h under N₂. After cooling to room temperature, the reaction mixture was filtered through Celite^{®} and the filter cake was washed with DCM/MeOH (10/1, 50 mL). The filtrate was concentrated in vacuo to give the crude, which was purified by column chromatography (eluent: DCM/MeOH = 0- 3%) to give **tert-butyl(6-cyclopropyl-2-((4-(hydroxymethyl)-1H-1,2,3-triazol-1-yl)methyl)imidazo[1,2-a]pyridin-8-yl)carbamate intermediate 924** as a white solid (90 mg, 18%). ESI-MS [M +H]+: 385.2.

A solution of **intermediate 924** (90 mg, 0.23 mmol) in HCl (5.0 mL, 4M in dioxane) was stirred at room temperature for 1 h. The mixture was concentrated to give **(1-((8-amino-6-cyclopropylimidazo[1,2-a]pyridin-2-yl)methyl)-1H-1,2,3-triazol-4-yl)methanol intermediate** 925 (66 mg, crude) as a yellow solid which was used for the next step directly without purification. ESI-MS [M +H]+: 285.1.

To a mixture of N'-formylformohydrazide (280 mg, 3.18 mmol) and TEA (749 mg, 7.42 mmol) and **intermediate 925** (300 mg, 1.06 mmol) in pyridine (10 mL) was added TMSCl (1.72 g, 15.9 mmol) at 0 °C under N₂. The resulting mixture was stirred at 100 °C for 18 h. After cooling to room temperature, the reaction mixture was quenched with water (100 mL) and extracted with EtOAc (50 mL x 3). The combined organic layers were washed with brine (50 mL), dried over anhydrous Na₂SO₄ and concentrated *in vacuo* to give the crude product, which was purified by column chromatography (eluent: DCM/MeOH = 20/1 ~ 10/1) to give **(1-((6-cyclopropyl-8-(4H-1,2,4-triazol-4-yl)imidazo[1,2-a]pyridin-2-yl)methyl)-1H-1,2,3-triazol-4-yl)methanol intermediate 926** (150 mg, 42%) as a white solid. ESI-MS [M +H]+: 337.1.

A mixture of **intermediate 926** (33 mg, 0.10 mmol) in SOCl₂ / CH₂Cl₂ (0.5 mL / 2.5 mL) was stirred at 0 °C for 1 h, then at 35 °C for 1.5 h. The mixture was cooled then concentrated *in vacuo* to give **2-((4-(chloromethyl)-1H-1,2,3-triazol-1-yl)methyl)-6-cyclopropyl-8-(4H-1,2,4-triazol-4-yl)imidazo[1,2-a]pyridine intermediate 927** (30 mg, crude) as a yellow solid, which was used without purification. ESI-MS [M +H]+: 355.1.

A mixture of **intermediate 927** (36 mg, 0.10 mmol) in NH₄OH / CH₃CN (2.5 mL / 0.5 mL) was stirred in a sealed in tube at room temperature for 16 h. Then, the mixture was concentrated *in vacuo* to give intermediate **(1-((6-cyclopropyl-8-(4H-1,2,4-triazol-4-yl)imidazo[1,2-a]-pyridin-2-yl)methyl)-1H-1,2,3-triazol-4-yl)methanamine** (30 mg, crude) as a yellow solid, which was used without purification. ESI-MS [M +H]+: 336.1.

(I-591). To a solution of the latter intermediate (30 mg, 0.1 mmol) in DMF (2.0 mL) were added (1S,2S)-2-(3-chlorophenyl)cyclopropane-1-carboxylic acid (20 mg. 0.1 mmol), HOBt (20 mg, 0.15 mmol), and DIPEA (39 mg, 0.3 mmol) at room temperature. After stirring at room temperature for 16 h, the reaction was diluted with water (20 mL) and extracted with EtOAc (3 x 40 mL). The combined organic layers were washed with brine (30 mL), dried over Na₂SO₄ and concentrated in vacuo to give the crude, which was purified by preparative HPLC to give **(I-591)(6** mg, 11%) as a white solid. ESI-MS [M +H]+: 514.2., δ 9.40 (s, 2H), 8.58 (t, J = 5.5 Hz, 1H), 8.48 (s, 1H), 8.01 (d, J = 3.3 Hz, 2H), 7.45 (s, 1H), 7.31 - 7.20 (m, 2H), 7.17 (s, 1H), 7.08 (d, J = 7.6 Hz, 1H), 5.71 (s, 2H), 4.36 - 4.28 (m, 2H), 2.33 - 2.24 (m, 1H), 2.02 - 1.96 (m, 1H), 1.95 - 1.86 (m, 1H), 1.38 - 1.33 (m, 1H), 1.26-1.20 (m, 1H), 1.05 - 0.93 (m, 2H), 0.89 - 0.76 (m, 2H).

### Example 592: rac-(1S*,2S*)-2-(3-chlorophenyl)-N-(2-((6-cyclopropylimidazo[1,2-a]pyridin-2-yl)methyl)-2H-pyrazolo[3,4-c]pyridin-7-yl)cyclopropane-1-carboxamide (I-592)

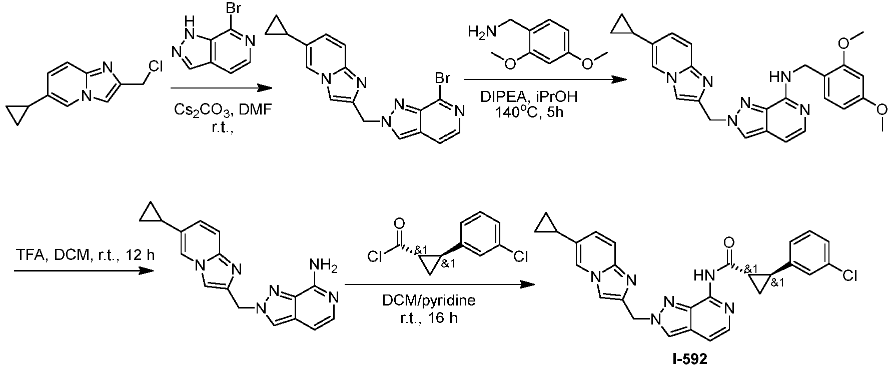

To a solution of 2-(chloromethyl)-6-cyclopropylimidazo[1,2-a]pyridine (380 mg, 1.84 mmol) in DMF (15 mL) was added Cs₂CO₃ (1.79 g, 5.5 mmol) and 7-bromo-1H-pyrazolo[3,4-c]pyridine (302 mg, 1.52 mmol). The mixture was stirred at rt for 16 h. Water (30 mL) was added and extracted by EtOAc (30 mL × 3). The combined organic layers were washed with brine, dried over Na₂SO₄, filtered, then concentrated *in vacuo.* The residue was purified by preparative TLC (EA:PE=1:1) to give the product **7-bromo-2-((6-cyclopropylimidazo[1,2-a]pyridin-2-yl)methyl)-2H-pyrazolo[3,4-c]pyridine intermediate 928** (300 mg, 44%) as a white solid. ESI-MS [M +H]+: 369.1.

A mixture of **intermediate 928** (100 mg, 0.27 mmol), (2,4-dimethoxyphenyl)methanamine (68 mg, 0.41 mmol), and DIPEA (104 mg, 0.81 mmol) in iPrOH (2 mL) was degassed for 1 min and stirred in a sealed tube. The reaction mixture was irradiated in microwave at 140 °C for 5 h and then concentrated *in vacuo* to give the crude product, which was purified by preparative TLC (eluent: DCM/MeOH = 10/1) to give **2-((6-cyclopropylimidazo[1,2-a]pyridin-2-yl)methyl)-N-(2,4-dimethoxybenzyl)-2H-pyrazolo[3,4-c]pyridin-7-amine intermediate 929** (100 mg, 81%) as a yellow oil. ESI-MS [M +H]⁺: 455.3.

To a mixture of **intermediate 929** (100 mg, 0.26 mmol) in DCM (2 mL) was added TFA (1 mL). After stirring at room temperature for 12 h, the reaction solution was diluted with DCM (20 mL) and washed with NaHCO₃ (10 mL). The organic layer was dried over anhydrous Na₂SO₄ then concentrated *in vacuo* to give the crude product, which was purified by preparative TLC (eluent: DCM/MeOH = 10/1, 1% of NH₃ as the additive) to give **2-((6-cyclopropylimidazo[1,2-a]pyridin-2-yl)methyl)-2H-pyrazolo[3,4-c]pyridin-7-amine intermediate 930** (70 mg, 88%) as a yellow oil ESI-MS [M +H]+: 305.2.

To a mixture of **intermediate 930** (50 mg, 0.16 mmol) in DCM (2 mL) was added pyridine (1 mL) and DMAP (2 mg, 0.016 mmol). After stirring the mixture at room temperature for 20 min, a solution of rac-(1S*,2S*)-2-(3-chlorophenyl)cyclopropane-1-carbonyl chloride (39 mg, 0.18 mmol) in DCM (1 mL) was added dropwise and stirred at room temperature for 16 h. The reaction was diluted with DCM (20 mL) and washed sequentially with water (2 x 10 mL) and brine (10 mL). The organic extract was dried over anhydrous Na₂SO₄ and concentrated in vacuo to give the crude product, which was purified by preparative TLC (eluent: DCM/MeOH = 10/1) to give (**I-592**) (11 mg, 14%) as a white solid. ESI-MS [M +H]+: 483.2., δ 10.54 (s, 1H), 8.53 (s, 1H), 8.33 (s, 1H), 8.24 (s, 1H), 7.86 - 7.72 (m, 2H), 7.40-7.38 (m, 2H), 7.34-7.30 (m, 1H), 7.27-7.25 (m, 2H), 7.19-7.17 (m, 1H), 7.01-6.98 (m, 1H), 5.78 (s, 2H), 2.44 - 2.41 (m, 2H), 1.92-1.91 (m, 1H), 1.51-1.50 (m, 1H), 1.38-1.37 (m, 1H), 0.92-0.90 (m, 2H), 0.67-0.65 (m, 2H).

### Example 593: rac-(1S*,2S*)-2-(3-chlorophenyl)-N-(1-((6-cyclopropylimidazo[1,2-a]pyridin-2-yl)methyl)-1H-1,2,3-triazol-4-yl)cyclopropane-1-carboxamide (I-593)

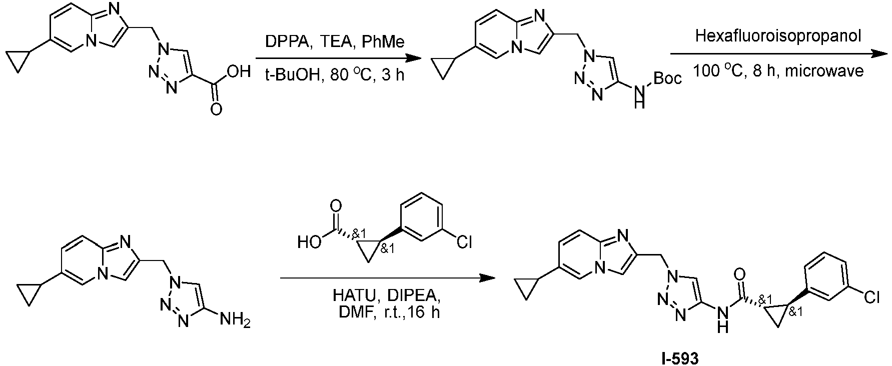

A mixture of 1-((6-cyclopropylimidazo[1,2-a]pyridin-2-yl)methyl)-1H-1,2,3-triazole-4-carboxylic acid (1 g, 3.53 mmol), TEA (400 mg, 3.88 mmol), and DPPA (1.04 g, 3.88 mmol) in toluene (10 mL) was stirred at 80 °C for 2 h. Then tBuOH (3 mL) was added and the resulting reaction was stirred at 80 °C for another 3 h. After cooling to room temperature, the mixture was washed with saturated aq. NaCl (25 mL) and extracted with EtOAc (3 x 30 mL). The combined organic layers were dried over Na₂SO₄, concentrated *in vacuo,* and the residue was purified by column chromatography (eluent: MeOH/DCM = 1/50) to give ***tert*-butyl(1-((6-cyclopropyl imidazo[1,2-a]pyridin-2-yl)methyl)-1H-1,2,3-triazol-4-yl)carbamate intermediate 931** (140 mg, 11%) as a white solid. ESI-MS [M +H]+: 355.2

A mixture of **intermediate 931** (140 mg, 0.395 mmol) in hexafluoroisopropanol (6 mL) was degassed with N₂ for 1 min, then stirred in a sealed tube. After heating in microwave at 100 °C for 8 h, the mixture was concentrated *in vacuo* to give the crude product, which was purified by column chromatography (eluent: MeOH/DCM = 1/20) to give **1-((6-cyclopropylimidazo[1,2-a]pyridin-2-yl)methyl)-1H-1,2,3-triazol-4-amine intermediate 932** (70 mg, 71%) as a yellow solid. ESI-MS [M +H]⁺: 255.1

A mixture of **intermediate 932** (70 mg, 0.27 mmol), rac-(1S*, 2S*)-2-(3-chlorophenyl) cyclopropane-1-carboxylic acid (63 mg, 0.22 mmol), HATU (132 mg,0.33 mmol), and DIPEA (120 mg, 0.89 mmol) ) in DMF (5 mL) was stirred at room temperature for 16 h. The reaction was diluted with water (20 mL) and extracted with EtOAc (3 x 30 mL). The combined organic layers were washed with brine (30 mL), dried over anhydrous Na₂SO₄, then concentrated in vacuo to give the crude, which was purified by column chromatography (eluent: MeOH/DCM = 1/20) to give (**I-593**) (6.6 mg, 5%) as a white solid. ESI-MS [M +H]+: 433.1., δ = 11.15 (s, 1H), 8.35 (s, 1H), 8.11 (s, 1H), 7.83 (s, 1H), 7.42 (d, J=9.3, 1H), 7.34-7.28 (m, 1H), 7.28-7.21 (m, 2H), 7.14 (d, J=7.6, 1H), 7.02 (d, J=9.5, 1H), 5.63 (s, 2H), 2.42 - 2.32 (m, 1H), 2.23-2.18 (m, 1H), 1.96 -1.88 (m,1H), 1.49-1.37 (m, 2H), 0.97-0.88 (m,2H), 0.72-0.64 (m, 2H).

### Example 594: rac-(1S*,2S*)-2-(3-chlorophenyl)-N-(1-((6-cyclopropylimidazo[1,2-a]pyridin-2-yl)methyl)-1H-imidazo[4,5-c]pyridin-6-yl)cyclopropane-1-carboxamide (I-594)

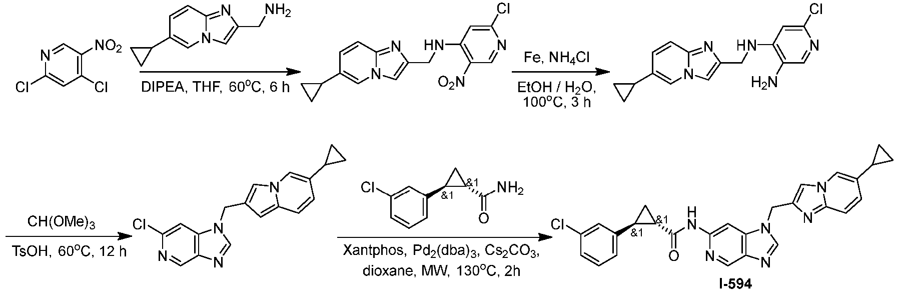

To a solution of 2,4-dichloro-5-nitropyridine (1.0 g, 5.2 mmol) in THF (30 mL) were added intermediate 251 (2.0 g, 8.9 mmol) and DIPEA (2.7 g, 20.8 mmol). The resulting mixture was heated to 60 °C and stirred for 6 h. The reaction was quenched with water (100 ml) and extracted with EtOAc (3 x 100 mL). The combined organic layers were washed with brine, dried over Na₂SO₄ and concentrated *in vacuo* to give **2-chloro-N-((6-cyclopropylimidazo[1,2-a]pyridin-2-yl)methyl)-5-nitropyridin-4-amine intermediate 933** the crude product, which was purified by silica gel column chromatography (eluent: MeOH/DCM = 1/20) to give the product (1.6 g, 90%) as a yellow solid. LC-MS: (M+H)⁺ 344.0.

A solution of **intermediate 933** (500 mg, 1.45 mmol) in ethanol (20 mL) was heated at 100 °C , then sequentially were added iron (820 mg, 14.5 mmol) and a solution of NH₄Cl (780 mg, 14.5 mmol) in water (6 ml). The resulting suspension mixture was stirred at 100 °C for 3 h. The hot reaction mixture was filtered through a Celite^{®} pad and the filtrate was concentrated *in vacuo.* The residue was dissolved in EtOAc (30 mL) and washed with water (30 mL). The aqueous phase was further extracted with i-PrOH:CHCl3 (1:3, 3 x 50 mL). The combined organic layers were washed with brine, dried over Na₂SO₄ and concentrated *in vacuo* to give **6-chloro-N4-((6-cyclopropylimidazo[1,2-a]pyridin-2-yl)methyl)pyridine-3,4-diamine intermediate 934** (450 mg, 99%) as a yellow solid. LC-MS: (M+H)⁺ 314.0

***Synthesis of.*** To a solution of **intermediate 934** (450 mg, 1.44 mmol) in CH(OMe)₃ (10 mL) was added p-MeC₆H₄SO₃H (49.5 mg, 1.45 mmol). After stirring for 2 h at 60 °C, the reaction was quenched with saturated aq. NaHCO₃ (50 mL) and extracted with EtOAc (3 x 50 mL). The combined organic layers were washed with brine, dried over Na₂SO₄ and concentrated *in vacuo* to give **6-chloro-1-((6-cyclopropylimidazo[1,2-a]pyridin-2-yl)methyl)-1H-imidazo[4,5-c]pyridine intermediate 935 ,** which was purified by silica gel column chromatography (eluent: MeOH/DCM =1/10) to give the product (320 mg, 34%) as a yellow solid. LC-MS: (M+H)⁺ 324.0

To a solution of **intermediate 935** (50 mg, 0.155 mmol) in 1,4-dioxane (2 mL) was added **intermediate 935** (30.2 mg, 0.155 mmol), Cs₂CO₃ (150 mg, 0.465 mmol), xantphos (9 mg, 0.0155 mmol), and Pd₂(dba)₃ (25.2 mg, 0.0155 mmol). The reaction was degassed with N₂ for 3 min, then irradiated in a microwave reactor at 130 °C for 2 h. The reaction was concentrated in vacuo to give **(I-594),** which was purified with preparative HPLC to give the product as a white solid (30 mg, 40%). ESI-MS [M +H]+: 483.2. δ 10.77 (s, 1H), 8.68 (s, 1H), 8.41 (s, 1H), 8.33 (s, 1H), 8.29 (s, 1H), 7.71 (s, 1H), 7.38 (d, J = 9.3 Hz, 1H), 7.34-7.30 (m, 1H), 7.26-7.25 (m, 2H), 7.15 (d, J = 7.6 Hz, 1H), 7.00-6.98 (m, 1H), 5.54 (s, 2H), 2.41-2.38 (m, 2H), 1.93-1.88 (m, 1H), 1.50-1.37 (m, 2H), 0.93-0.88 (m, 2H), 0.68-0.64 (m, 2H)

### Example 595: (1S,2S)-2-(3-chlorophenyl)-N-(4-(((6-cyclopropyl-8-((methylamino)methyl) imidazo[1,2-a]pyridin-2-yl)methyl)amino)pyridin-2-yl)cyclopropane-1-carboxamide (I-595)

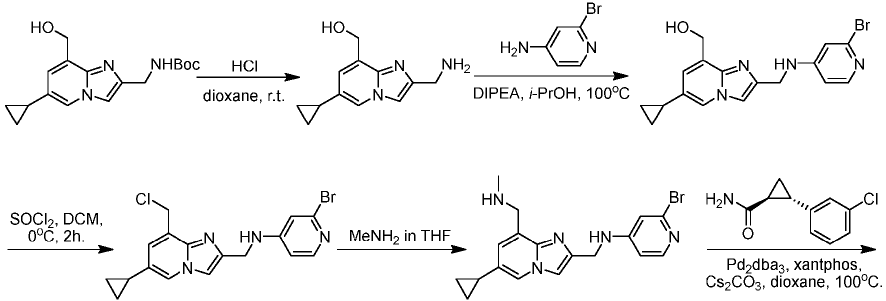

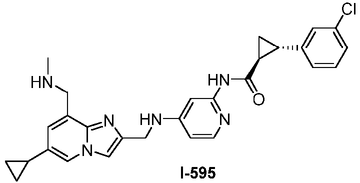

Alternative synthesis of ***(2-(aminomethyl)-6-cyclopropylimidazo[1,2-a]pyridin-8-yl)** methanol* **intermediate 266b.** A mixture of *tert*-butyl ((6-cyclopropyl-8-(hydroxymethyl)imidazo[1,2-a]pyridin-2-yl)methyl)-carbamate (317 mg, 1.0 mmol) in HCl (4M in dioxane, 5.0 mL) was stirred at room temperature for 2 h. The mixture was concentrated *in vacuo* to give (2-(aminomethyl)-6-cyclopropylimidazo-[1,2-a]pyridin-8-yl)methanol (220 mg, crude) as a yellow solid, which was used into the next step directly. ESI-MS [M +H]⁺: 218.2.

To a mixture of **intermediate 266b** (217 mg, 1.0 mmol), 2-bromopyridin-4-amine (262.5 mg, 1.5 mmol), and DIPEA (387 mg, 3.0 mml) in i-PrOH (8 mL) was stirred at 100 °C for 16 h. The mixture was concentrated *in vacuo* and purified by preparative TLC (DCM/MeOH = 15/1) to give the product **(2-(((2-bromopyridin-4-yl)amino)methyl)-6-cyclopropylimidazo[1,2-a]pyridin-8-**yl)methanol **intermediate 936** (400 mg, 82 %) as yellow oil. ESI-MS [M +H]⁺: 373.2.

To a mixture of **intermediate 936** (300 mg, 0.81 mmol) in DCM (5.0 mL) was added SOCl₂ (1.0 mL) at 0 °C. The reaction mixture was stirred at 0 °C for 2 h. The mixture was concentrated *in vacuo* to give the product **2-bromo-N-((8-(chloromethyl)-6-cyclopropylimidazo[1,2-a]pyridin-2-yl)methyl)pyridin-4-amine intermediate 937** (300 mg, crude) as a yellow solid, which was used without further purification. ESI-MS [M +H]⁺: 391.2.

A mixture of **intermediate 937** (200 mg, 0.51 mmol) in MeNH₂ (2.0 M in THF, 10 mL) was stirred at 70 °C in a sealed tube for 48 h. The mixture was cooled to room temperature, then concentrated *in vacuo* and purified by preparative TLC (DCM/MeOH = 10/1) to give the **2-bromo-N-((6-cyclopropyl-8-((methylamino)methyl)imidazo-[1,2-a]pyridin-2-yl)methyl)pyridin-4-amine intermediate 938** (30 mg, 15 %) as a yellow oil. ESI-MS [M +H]⁺: 387.2.

A mixture of **intermediate 938** (19.3 mg, 0.05 mmol), **intermediate 119** (9.8 mg, 0.05 mmol), Pd₂(dba)₃ (4.6 mg, 0.005 mmol), xantphos (2.9 mg, 0.005 mmol), and Cs₂CO₃ (48.9 mg, 0.15 mmol) in 1,4-dioxane (2.5 mL) was stirred at 100 °C under N₂ for 16 h. The mixture was cooled to room temperature, concentrated in vacuo and purified by preparative TLC (DCM/MeOH = 7/1) to give **(I-595)** (5.2 mg, 21 %) as a light yellow solid. ESI-MS [M +H]⁺: 501.2., δ 10.32 (s, 1H), 8.26 (s, 1H), 7.76 (d, J = 5.7 Hz, 1H), 7.65 (s, 1H), 7.44 (s, 1H), 7.30 (d, J = 7.6 Hz, 1H), 7.26 -7.24 (m, 2H), 7.13 (d, J = 6.9 Hz, 2H), 6.98 (s, 1H), 6.32 (d, J = 5.7 Hz, 1H), 4.37 (d, J = 5.3 Hz, 2H), 4.04 (s, 2H), 2.41 (s, 3H), 2.39 - 2.23 (m, 2H), 1.97 - 1.81 (m, 1H), 1.51 - 1.40 (m, 1H), 1.39 - 1.29 (m, 1H), 0.97 - 0.87 (m, 2H), 0.68 - 0.65 (m, 2H).

### Example 596: (1S,2S)-2-(3-chlorophenyl)-N-(5-((6-cyclopropylimidazo[1,2-a]pyridin-2-yl)methoxy)pyrimidin-4-yl)cyclopropane-1-carboxamide (I-596)

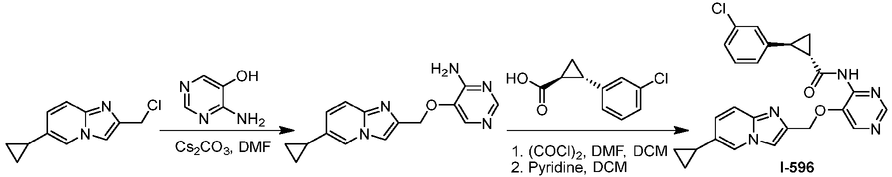

A solution of 2-(chloromethyl)-6-cyclopropylimidazo[1,2-a]pyridine (50 mg, 0.45 mmol), 4-aminopyrimidin-5-ol (102 mg, 0.50 mmol), and Cs₂CO₃ (293 mg, 0.9 mmol) in DMF (10 mL) was stirred at 60 °C for 16 h. The reaction was quenched with water (30 mL) and extracted with EtOAc (3 x 30 mL). The combined organic layers were washed with brine, dried over Na₂SO₄, and concentrated *in vacuo* to give the crude, which was purified by silica gel chromatography (eluent: DCM/MeOH = 10/1) to give the **5-((6-cyclopropylimidazo[1,2-a]pyridin-2-yl)methoxy)pyrimidin-4-amine intermediate 939** as a yellow solid. (50 mg, 40%), ESI-MS [M +H]+: 282.2

To a solution of (1S,2S)-2-(3-chlorophenyl)cyclopropane-1-carboxylic acid (50 mg, 0.6 mmol) in DCM (5 mL) was added oxalyl dichloride ( 65 mg, 0.52 mmol ) and DMF (3 drops ) at 0 °C under nitrogen. After stirring at 0 °C for 3 h, the mixture was evaporated to give (1S,2S)-2-(3-chlorophenyl)cyclopropane-1-carbonyl chloride as light-yellow oil (46 mg, crude), which was used without further purification.

To a solution of intermediate 939 (30 mg, 0.11 mmol) in pyridine (1 mL) in DCM (3 mL) was added a solution of (1S,2S)-2-(3-chlorophenyl)cyclopropane-1-carbonyl chloride (46 mg, 0.22 mmol) in DCM (2 mL). After stirring at room temperature for 12 h, the reaction was quenched with water (10 mL) and extracted with EtOAc (3 x 10 mL). The combined organic layers were washed with brine, dried over Na₂SO₄, concentrated *in vacuo* to give the crude, which was purified with preparative TLC (eluent: DCM / MeOH = 10 / 1) to give **(I-596)** as a white solid. (15 mg, 29 %). ESI-MS [M +H]⁺: 460.1., δ 10.32 (s, 1H), 8.66 (s, 1H), 8.51 (s, 1H), 8.36 (s, 1H), 7.92 (s, 1H), 7.45 - 7.37 (m, 1H), 7.36 - 7.20 (m, 3H), 7.14 (d, J = 7.5 Hz, 1H), 7.06 - 6.95 (m, 1H), 5.38 (s, 2H), 2.58-2.56 (m, 1H), 2.45 - 2.38 (m, 1H), 1.97 - 1.91 (m, 1H), 1.54 - 1.47 (m, 1H), 1.45 - 1.38 (m, 1H), 0.96 - 0.90 (m, 2H), 0.72 - 0.66 (m, 2H).

### Example 597: (1S,2S)-2-(3-chlorophenyl)-N-(1-((6-cyclopropylimidazo[1,2-a]pyridin-2-yl)methyl)-2,3-dihydro-1H-pyrido[3,4-b][1,4]oxazin-7-yl)cyclopropane-1-carboxamide (I-597)

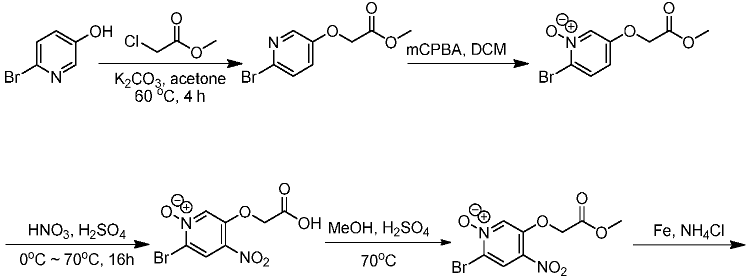

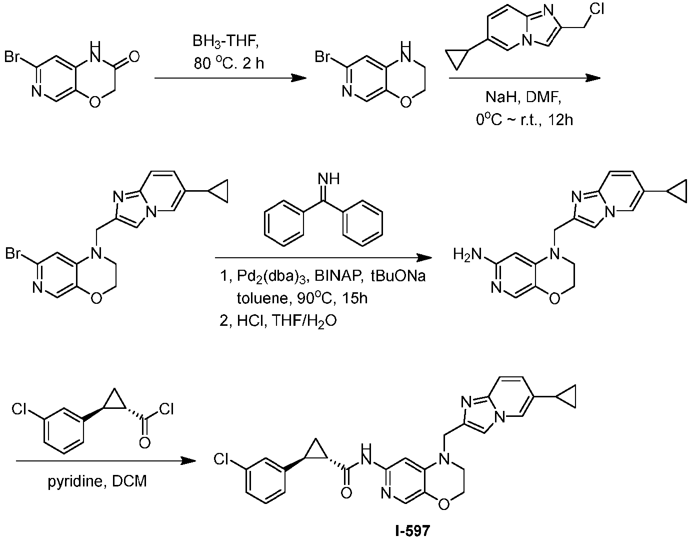

To a mixture of 6-bromopyridin-3-ol (1.74 g, 10 mmol) and potassium carbonate (2.76 g, 20 mmol) in acetone (30 mL) was added dropwise chloroacetic methyl ester (1.08 g, 10 mmol). After stirring at 60 °C for 15 hours, the mixture was filtered, and the filtrate was concentrated *in vacuo* to afford the crude product, which was purified by flash silica gel chromatography (PE / EtOAc = 1/1) to give **methyl 2-((6-bromopyridin-3-yl)oxy)acetate intermediate 940** (1.23 g, 50%) as a yellow solid. ESI-MS [M +H]+: 246.1.

To a mixture of methyl 2-(6-bromopyridin-3-yloxy)acetate (1.2 g, 5 mmol) in dichloromethane (20 mL) was added m-chloroperbenzoic acid (1.72 g, 10 mmol). After stirring at room temperature for 18 hours, reaction was quenched with saturated aq. sodium sulfite solution (50 mL) and extracted by DCM (3 x 40 mL). The combined organic layers were washed with brine, dried over Na₂SO₄ then concentrated *in vacuo* to give crude **2-bromo-5-(2-methoxy-2-oxoethoxy)pyridine 1-oxide intermediate 941** (0.9 g, crude) as a yellow solid, which was used without further purification. ESI-MS [M +H]+: 262.1.

**Intermediate 941** (1 g, 3.8 mmol) was dissolved in sulfuric acid (4 mL) at 0 °C, and then nitric acid (2 mL) was added slowly over several minutes. The reaction mixture was then placed in an oil bath heated to 40 °C , then raised to 75 °C over 1 hour. After stirring at 75 °C for another 2 hours, the reaction mixture was slowly poured into the ice water and basified (pH=9) with saturated aq. NaHCO₃. The mixture was concentrated *in vacuo* to give crude **2-bromo-5-(carboxymethoxy)-4-nitropyridine 1-oxide intermediate 942(1.5** g, crude) as a white solid, which was used without further purification. ESI-MS [M +H]+: 294.1.

To a mixture of **intermediate 942** (1.5 g, crude from previous step) in MeOH (100 mL) was added H₂SO₄ (3 mL). After stirring at 70 °C for 16 h, then mixture was filtered and washed with MeOH (100 mL). The filtrate was concentrated *in vacuo* to give the residue, which was re-dissolved in EtOAc (100 mL) and washed with saturated aq. NaHCO₃ (50 mL). The organic layer was washed with brine, dried over Na₂SO₄, and concentrated *in vacuo* to give crude, which was purified with

silica gel chromatography (eluent: PE / EtOAc = 1/1) to give **2-bromo-5-(2-methoxy-2-oxoethoxy)-4-nitropyridine 1-oxide intermediate 943** (600 mg, 51% over two steps) as a yellow solid. ESI-MS [M +H]+: 307.1.

To a solution of **intermediate 943** (307 mg, 1 mmol) in MeOH (15 mL) was added Fe power (560 mg, 10 mmol) and NH₄Cl (540 mg, 10 mmol) in water (3 mL). After stirring at 70 °C for 2 h, the hot reaction mixture was filtered and washed with MeOH (50 mL). The filtrate was concentrated to give the residue, which was washed with saturated aq. NaHCO₃ (30 mL) and extracted by EtOAc (3 x 40 mL). The combined organic layers were washed with brine, dried over Na₂SO₄, and concentrated *in vacuo* to give the crude, which was purified with silica gel chromatography (eluent: PE / EtOAc = 1/1) to give **intermediate 944** (140 mg, 61%) as a white solid. ESI-MS [M +H]+: 229.1.

To a solution of **intermediate 944** (160 mg, 0.7 mmol) in THF (10 mL) was added BH₃-THF (1.68 mL, 1.68 mmol) at 0 °C. The resulting mixture was stirred at 80 °C for 2 h and cooled to room temperature. The reaction was quenched with methanol (10 mL), washed with saturated aq. NaHCO₃ (20 mL), and extracted by EtOAc (3 x 30 mL). The combined organic layers was washed with brine, dried over Na₂SO₄, concentrated *in vacuo,* and purified by flash silica gel chromatography (eluent: PE/EtOAc = 2/1) to give **7-bromo-2,3-dihydro-1H-pyrido[3,4-b] [1,4]oxazine intermediate 945** (80 mg, 50%) as a yellow solid. ESI-MS [M +H]+: 215.1.

To a solution of **intermediate 945** (65 mg, 0.30mmol) in DMF (5 mL) was added NaH (60 mg, 1.51 mmol, 60% dispersion in mineral oil) at 0 °C and stirred at room temperature for 1 h. Then 2-(chloromethyl)-6-cyclopropylimidazo[1,2-a]pyridine (74 mg, 0.36 mmol) was added and the resulting mixture was stirred at room temperature for another 16 h. The reaction was quenched with saturated aq.NH₄Cl (20 mL) and extracted by EtOAc (3 x 20 mL). The combined organic layers were washed with brine, dried over Na₂SO₄, and concentrated *in vacuo* to give **7-bromo-1-((6-cyclopropylimidazo[1,2-a]pyridin-2-yl)methyl)-2,3-dihydro-1H-pyrido[3,4-b][1,4]oxazine intermediate 946** the crude, which was purified by silica gel chromatography (eluent: 100% of EtOAc) to give the product (90 mg, 77%) as a white solid. ESI-MS [M +H]+: 385.1.

Step 1: A mixture of **intermediate 946** (115 mg, 0.3 mmol), diphenylmethanimine (66 mg, 0.36 mmol), Pd₂(dba)₃ (57 mg, 0.06 mmol), BINAP(75 mg, 0.12 mmol) and t-BuOK (45 mg, 0.45 mmol) in toluene (4 mL) was stirred at 80 °C for 16 h. The reaction was washed with water (10 mL) and extracted by EtOAc (3 x 20 mL). The combined organic layers were washed with brine, dried over Na₂SO₄, and concentrated *in vacuo* to give crude N-(1-((6-cyclopropylimidazo[1,2-a]pyridin-2-yl)methyl)-2,3-dihydro-1H-pyrido[3,4-b][1,4]oxazin-7-yl)-1,1-diphenylmethanim, which was purified by preparative TLC (DCM / MeOH=10/1) to give product (120 mg, 82%). ESI-MS [M +H]+: 486.1.

Step 2: To a solution ofN-(1-((6-cyclopropylimidazo[1,2-a]pyridin-2-yl)methyl)-2,3-dihydro-1H-pyrido[3,4-b][1,4]oxazin-7-yl)-1,1-diphenylmethanimine (100 mg, 0.206 mmol) in THF (6 mL) was added 1 N HCl (3mL). After stirring at room temperature for 3 h, the pH of the reaction was adjusted to 8 by saturated aq. NaHCO₃ and extracted by EtOAc (3 x 30 mL). The combined organic layers were washed with brine, dried over Na₂SO₄, and concentrated *in vacuo* to give **crude1-((6-cyclopropylimidazo[1,2-a]pyridin-2-yl)methyl)-2,3-dihydro-1H-pyrido[3,4-b][1,4]oxazin-7-amine intermediate 947,** which was purified by preparative TLC (DCM/MeOH=10/1) to give product (50 mg, 75.7%) as a brown solid. ESI-MS [M +H]+: 322.1.

To a solution of **intermediate 947** (50 mg, 0.16 mmol) in pyridine / DCM (1 mL / 3 mL) was added a solution of (1S,2S)-2-(3-chlorophenyl)cyclopropane-1-carbonyl chloride (49 mg, 0.23 mmol) in DCM (1 mL) at 0 °C. After stirring at room temperature for 5 h, the reaction was washed with water (10 mL) and extracted by DCM (3 x 20 mL). The combined organic layers were washed with brine, dried over Na₂SO₄, then concentrated in vacuo to give the crude, which was purified by preparative TLC (DCM / MeOH=10 / 1) to give (**I-597**) (10 mg, 13%) as a white solid. ESI-MS [M +H]+: 500.2., δ 10.28 (s, 1H), 8.29 (s, 1H), 7.66 (s, 1H), 7.61 (s, 1H), 7.54 (s, 1H), 7.40 (d, J = 9.3 Hz, 1H), 7.30 (t, J = 7.7 Hz, 1H), 7.23 (dd, J = 9.1, 1.2 Hz, 2H), 7.12 (d, J = 7.6 Hz, 1H), 6.98 (dd, J = 9.3, 1.7 Hz, 1H), 4.57 (s, 2H), 4.27 - 4.10 (m, 2H), 3.62 - 3.50 (m, 2H), 2.40 - 2.22 (m, 2H), 1.95 -1.88 (m, 1H), 1.48 - 1.39 (m, 1H), 1.35 - 1.30 (m, 1H), 0.95 - 0.84 (m, 2H), 0.71 - 0.62 (m, 2H).

### Example 598: (1S,2S)-2-(3-chlorophenyl)-N-(4-(((6-cyclopropyl-8-(2-(dimethylamino) ethyl)imidazo[1,2-alpyridin-2-yl)methyl)amino)pyridin-2-yl)cyclopropane-1-carboxamide (I-598)

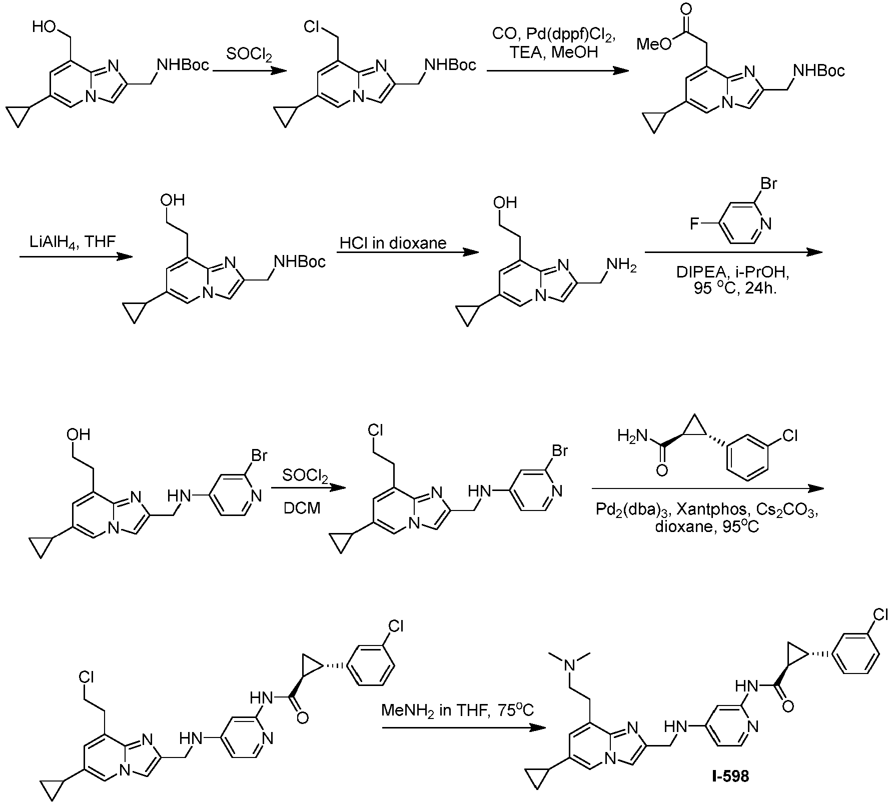

To a mixture of *tert*-butyl ((6-cyclopropyl-8-(hydroxymethyl)imidazo[1,2-a] pyridin-2-yl)methyl)-carbamate (317 mg, 1.0 mmol) in DCM (5.0 mL) was added SOCl₂ (178.5 mg, 1.5 mmol) at 0 °C. After stirring at 0 °C for 1 h, the reaction mixture was concentrated *in vacuo* to give ***tert*-butyl ((8-(chloromethyl)-6-cyclopropylimidazo[1,2-a]pyridin-2-yl)methyl)carbamate intermediate 948** (335 mg, crude) as a yellow solid, which was used without further purification. ESI-MS [M +H]⁺: 336.2.

A mixture of **intermediate 948** (335 mg, 1.0 mmol) and Pd(dppf)Cl₂ (41 mg, 0.05 mmol) in TEA/MeOH (2.0 mL/10.0 mL) was stirred at 70 °C under CO atmosphere for 16 h. The mixture was concentrated *in vacuo* and purified by preparative TLC ( DCM/MeOH = 15/1) to give **methyl 2-(2-(((*tert*-butoxycarbonyl)amino)-methyl)-6-cyclopropylimidazo[1,2-a]pyridin-8-yl)acetate intermediate 949** (160 mg, 45 %) as a yellow solid. ESI-MS [M +H]⁺: 360.2.

To a mixture of **intermediate 949** (160 mg, 0.45 mmol) in THF (10.0 mL) was added a solution of LiAlH₄ (1.34 mL, 1.34 mmol, 1M solution in THF) at 0 °C under N₂. After stirring at 0 °C for 1 h, the mixture was quenched with water (0.2 mL), 15 % NaOH solution (0.2 mL), and water (0.6 mL). The mixture was filtered and the filtrate concentrated *in vacuo* to give ***tert*-butyl** ((6-**cyclopropyl-8-(2-hydroxyethyl)imidazo[1,2-a]pyridin-2-yl)methyl)carbamate intermediate 950** (130 mg, crude) as a yellow solid, which was used without further purification. ESI-MS [M +H]⁺: 332.2.

A mixture of **intermediate 950** (130 mg, 0.39 mmol) in HCl (4M in 1,4-dioxane, 2.0 mL) was stirred at room temperature for 1 h. The mixture was concentrated *in vacuo* to give **2-(2-(aminomethyl)-6-cyclopropylimidazo[1,2-a]pyridin-8-yl)ethan-1-ol intermediate 951** (100 mg, crude) as a yellow solid, which was used without further purification. ESI-MS [M +H]⁺: 232.2.

A mixture of **intermediate 951** (100 mg, crude from previous step), 2-bromo-4-fluoropyridine (150.0 mg, 0.86 mmol), and DIPEA (166.4 mg, 1.29 mmol) in *i*-PrOH (5 mL) was stirred at 100 °C for 16 h. The mixture was concentrated *in vacuo* and purified by preparative TLC (DCM/MeOH = 15/1) to give **2-(2-(((2-bromopyridin-4-yl)amino)methyl)-6-cyclopropylimidazo[1,2-a]pyridin-8-yl)ethan-1-ol intermediate 952** (80 mg, 48 % over 2 steps) as yellow oil. ESI-MS [M +H]⁺: 387.2.

To a mixture of **intermediate 952** (600 mg, 1.55 mmol) in DCM (10.0 mL) was added SOCl₂ (1.0 mL) at 0 °C. After stirring at 0 °C for 4 h, the mixture was concentrated *in vacuo* to give crude **2-bromo-N-((8-(2-chloroethyl)-6-cyclopropylimidazo[1,2-a]pyridin-2-yl)methyl)pyridin-4-amine intermediate 953** (600 mg, crude) as a yellow solid, which was used without further purification. ESI-MS [M +H]⁺: 405.2.

A mixture of **intermediate 953(100.0** mg, 0.25 mmol), intermediate 119 (48 mg, 0.25 mmol), Pd₂(dba)₃ (23 mg, 0.025 mmol), Xantphos (14 mg, 0.025 mmol), and Cs₂CO₃ (202 mg, 0.62 mmol) in 1,4-dioxane (5.0 mL) was stirred at 100 °C under N₂ for 3 h. The mixture was concentrated in vacuo and purified by preparative TLC (DCM/MeOH = 10/1) to give **(1S,2S)-N-(4-(((8-(2-chloroethyl)-6-cyclopropylimidazo[1,2-a]pyridin-2-yl)methyl)amino)pyridin-2-yl)-2-(3-chlorophenyl)cyclopropane-1-carboxamide intermediate 954** (50.0 mg, 39 %) as a light yellow solid.

A mixture of **intermediate 954** (52 mg, 0.10 mmol) in MeNH₂ (2.0 M in THF, 3.0 mL) was stirred at 80 °C in a sealed tube for 48 h. The mixture was concentrated in vacuo and purified by preparative TLC (DCM/MeOH = 10/1) to give **(I-598)** (6.0 mg, 11 %) as a light-yellow solid. ESI-MS [M +H]⁺: 529.2., δ 10.32 (s, 1H), 8.16 (s, 1H), 7.75 (d, J = 5.7 Hz, 1H), 7.60 (s, 1H), 7.44 (s, 1H), 7.37 - 7.20 (m, 3H), 7.14 (d, J = 6.1 Hz, 2H), 6.84 (s, 1H), 6.34 - 6.32 (m, 1H), 4.36 (d, J = 5.7 Hz, 2H), 3.00 (t, J = 7.4 Hz, 2H), 2.79 - 2.63 (m, 2H), 2.41 - 2.31 (m, 2H), 2.26 (s, 6H), 1.93 - 1.79 (m, 1H), 1.54 - 1.41 (m, 1H), 1.37 - 1.32 (m, 1H), 0.98 - 0.79 (m, 2H), 0.76 - 0.52 (m, 2H).

### Example 599: (1S,2S)-2-(3-chlorophenyl)-N-(4-(((6-cyclopropyl-8-(4-(2-methoxyethyl)piperazin-1-yl)imidazo[1,2-a]pyridin-2-yl)methyl)amino)pyridin-2-yl)cyclopropane-1-carboxamide (I-599)

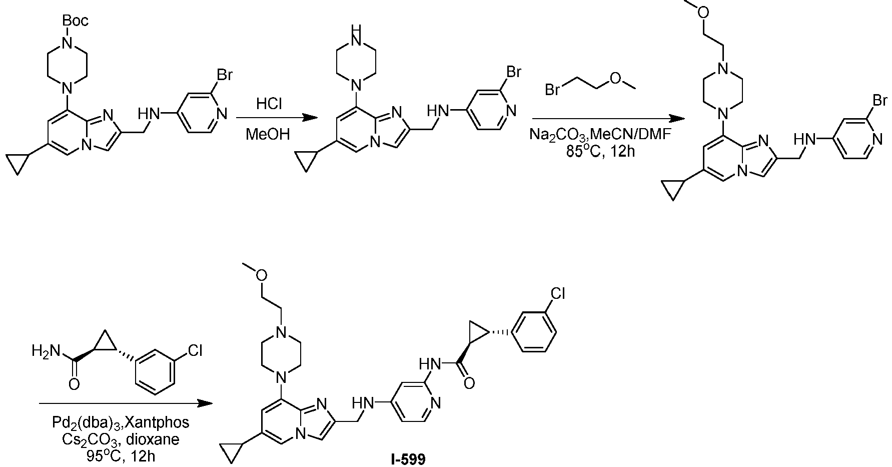

To a solution of **intermediate 276** (500 mg, 0.95 mmol) in MeOH (10 ml) was added HCl (4 M solution in MeOH, 1.19 ml, 4.75 mmol). After stirring at 0 °C for 5 h, the mixture was concentrated *in vacuo* to give **2-bromo-N-((6-cyclopropyl-8-(piperazin-1-yl)imidazo[1,2-a]pyridin-2-yl)methyl)pyridin-4-amine intermediate 955** as a light-yellow oil (300 mg, crude). ESI-MS [M +H]+: 427.2

To a solution of intermediate 955(50 mg, 0.12 mmol) and Na₂CO₃ (38 mg, 0.36 mmol) in MeCN /DMF (10 mL/1 mL), 1-bromo-2-methoxyethane (13 mg, 0.096 mmol) was added. After stirring at 85 °C for 12 h, the reaction was quenched with water (30 mL) and extracted with EtOAc (3 x 30 mL). The combined organic layers were washed with brine (50 mL), dried over Na₂SO₄ then concentrated *in vacuo* to give the crude, which was purified by preparative TLC (DCM/MeOH= 10/1) to give **2-bromo-N-((6-cyclopropyl-8-(4-(2-methoxyethyl)piperazin-1-yl)imidazo[1,2-a] pyridin-2-yl)methyl)pyridin-4-amine intermediate 956** (30 mg, 52 %) as a white solid. ESI-MS [M +H]+: 485.2

To a solution of **intermediate 956** (30 mg, 0.062 mmol), **intermediate 119** (18 mg, 0.092 mmol), Pd₂(dba)₃ (6 mg, 0.0062 mmol), Xantphos (4 mg, 0.0062 mmol), and Cs₂CO₃ (61 mg, 0.186 mmol) in 1,4-dioxane (10 mL) was stirred at 95 °C for 12 h under N₂. After quenching with water (20 mL), the aqueous phase was extracted with EtOAc (3 x 20 mL). The combined organic layers were washed with brine (50 mL), dried over Na₂SO₄ then concentrated in vacuo to give the crude, which was purified by preparative TLC (DCM/MeOH= 10/1) to give (**I-599**) ((20 mg, 54%) as a white solid. ESI-MS [M +H]+: 600.3. δ 10.32 (s, 1H), 7.87 (s, 1H), 7.75 (d, J = 5.8 Hz, 1H), 7.53 (s, 1H), 7.44 (s, 1H), 7.33 - 7.20 (m, 3H), 7.19 - 7.10 (m, 2H), 6.32 (dd, J = 5.8, 2.1 Hz, 1H), 6.14 (s, 1H), 4.33 (d, J = 5.7 Hz, 2H), 3.49 - 3.46 (m, 6H), 3.25 (s, 3H), 2.67 - 2.55 (m, J = 25.8 Hz, 4H), 2.54 - 2.51 (m, 2H), 2.40 - 2.33 (m, 2H), 1.88 - 1.81 (m, J = 13.4, 8.4, 5.1 Hz, 1H), 1.46 - 1.42 (m, 1H), 1.37 - 1.32 (m, 1H), 0.88 - 0.83 (m, 2H), 0.67 - 0.63 (m, 2H).

### Example 600: Ethyl2-(4-(2-(((2-((1S,2S)-2-(3-chlorophenyl)cyclopropane-1-carboxamido) pyridin-4-yl)amino)methyl)-6-cyclopropylimidazo[1,2-a]pyridin-8-yl)piperazin-1-yl)acetate (I-600)

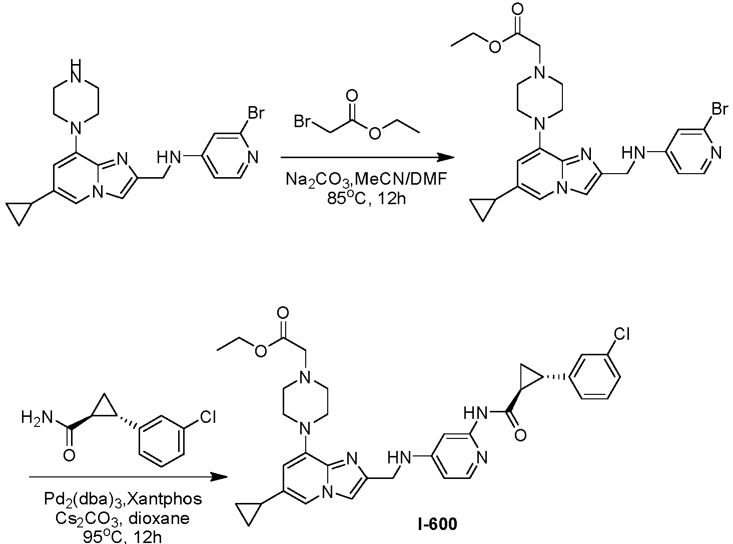

To a solution of **intermediate 955** (90 mg, 0.21 mmol) and Na₂CO₃ (67 mg, 0.63 mmol) in MeCN /DMF (10 mL/1 mL), ethyl 2-bromoacetate (35 mg, 0.21 mmol) was added. After stirring at 85 °C for 12 h, the reaction was quenched with water (30 mL) and extracted with EtOAc (3 x 20 mL). The combined organic layers were washed with brine (50 mL), dried over Na₂SO₄, and concentrated *in vacuo* to give the crude, which was purified by preparative TLC (DCM/MeOH= 10/1) to give the product ethyl 2-(4-(2-(((2-bromopyridin-4-yl)amino)methyl)-6-**cyclopropylimidazo[1,2-a]pyridin-8-yl)piperazin-1-yl)acetate** intermediate 957 (100 mg, 90 %) as a white solid. ESI-MS [M +H]+: 513.2

A solution of intermediate 957 (100 mg, 0.19 mmol), intermediate 119 (57 mg, 0.29 mmol), Pd₂(dba)₃ (17 mg, 0.019 mmol), Xantphos (11 mg, 0.019 mmol), and Cs₂CO₃ (186 mg, 0.57 mmol) in 1,4-dioxane (10 mL) was stirred at 95 °C for 12 h under N₂. The reaction was quenched with water (20 mL) and the aqueous phase was extracted with EtOAc (3 x 20 mL). The combined organic layers were washed with brine (50 mL), dried over Na₂SO₄ then concentrated in vacuo to give the crude, which was purified by preparative TLC (DCM/MeOH= 10/1) to give (**I-600**) (90 mg, 75%) as a white solid.

ESI-MS [M +H]+: 628.4., δ 10.32 (s, 1H), 7.87(s, 1H), 7.80 - 7.71 (m, 1H), 7.53 (s, 1H), 7.43 (s, 1H), 7.36 - 7.07 (m, 5H), 6.33 (d, J = 5.2 Hz, 1H), 6.16 (s, 1H), 4.39 - 4.29 (m, 2H), 4.20 - 4.06 (m, 2H), 3.52 - 3.46 (m, 4H), 2.76 - 2.68 (m, 4H), 2.41 - 2.29 (m, 2H), 1.87-1.82 (m, 1H), 1.47-1.42 (m, 1H), 1.37-1.32 (m, 1H), 1.24 - 1.17 (m, 3H), 0.89 - 0.83 (m, 2H), 0.69 - 0.60 (m, 2H).

### Example 601: (1S,2S)-2-(3-chlorophenyl)-N-(4-(((6-cyclopropyl-8-(4-(cyclopropylmethyl) piperazin-1-yl)imidazo[1,2-a]pyridin-2-yl)methyl)amino)pyridin-2-yl)cyclopropane-1-carboxamide (I-601)

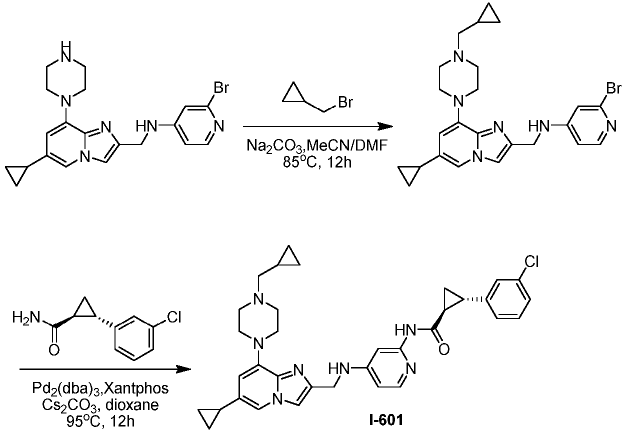

To a solution of **intermediate 955** (80 mg, 0.19 mmol) and Na₂CO₃ (60 mg, 0.56 mmol) in MeCN /DMF (15 mL/1 mL) was added (bromomethyl)cyclopropane (25 mg, 0.19 mmol). After stirring at 85 °C for 12 h, the reaction was quenched with water (30 mL) and extracted with EtOAc (3 x 30 mL). The combined organic layers were washed with brine (30 mL), dried over Na₂SO₄ then concentrated *in vacuo* to give the crude, which was purified by Pre-TLC (eluent: DCM/MeOH= 10/1) to give **2-bromo-N-((6-cyclopropyl-8-(4-(cyclopropylmethyl)piperazin-1-yl)imidazo[1,2-a]pyridin-2-yl)methyl)pyridin-4-amine intermediate 958** (60 mg, 66 %) as a white solid. ESI-MS [M +H]+: 481.1

A mixture of **intermediate 958** (60 mg, 0.13 mmol), **intermediate 119** (27 mg, 0.13 mmol), Pd₂(dba)₃ (23 mg, 0.025 mmol), Xantphos (15 mg, 0.025 mmol), and Cs₂CO₃ (122 mg, 0.38 mmol) in 1,4-dioxane (15 mL) was stirred at 95 °C for 12 h. The reaction was quenched with water (30 mL) and extracted with EtOAc (3 x 30 mL). The combined organic layers were washed with brine (50 mL), dried over Na₂SO₄ then concentrated in vacuo to give the crude, which was purified by Pre-TLC (eluent: DCM/MeOH= 10/1) to give **(I-601)** (20 mg, 27%) as a white solid. ESI-MS [M +H]+: 596.3., δ 10.31 (s, 1H), 7.86 (s, 1H), 7.75 (d, J = 5.8 Hz, 1H), 7.53 (s, 1H), 7.44 (s, 1H), 7.35 - 7.28 (m, 1H), 7.27 - 7.22 (m, 2H), 7.16 - 7.08 (m, 2H), 6.32 (dd, J = 5.8, 2.1 Hz, 1H), 6.16 (s, 1H), 4.33 (d, J = 5.5 Hz, 2H), 3.49 (s, 4H), 2.64 (s, 4H), 2.43 - 2.30 (m, 2H), 2.25 (d, J = 6.4 Hz, 2H), 1.92 - 1.80 (m, 1H), 1.49 - 1.40 (m, 1H), 1.39 - 1.30 (m, 1H), 0.90 - 0.82 (m, 3H), 0.70 - 0.62 (m, 2H), 0.52 - 0.45 (m, 2H), 0.15 - 0.08 (m, 2H).

### Example 602: 2-(4-(2-(((2-((1S,2S)-2-(3-chlorophenyl)cyclopropane-1-carboxamido)pyridin-4-yl)amino)methyl)-6-cyclopropylimidazo[1,2-a]pyridin-8-yl)piperazin-1-yl)acetic acid (I-602)

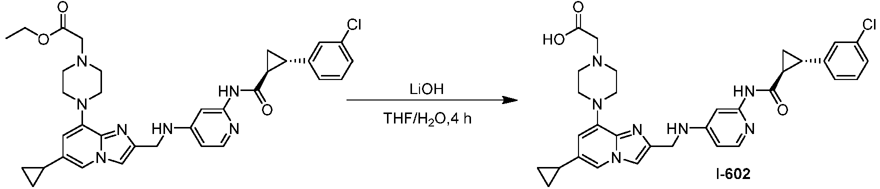

To a solution of **(I-600)** (30 mg, 0.048 mmol) in THF (3 mL) and water (3 mL) was added LiOH-H₂O (4 mg, 0.095 mmol). The mixture was stirred at room temperature for 4 h. The organic layer was evaporated, and the aqueous phase was acidified with 1 N HCl to pH=3 and extracted with EtOAc (3 x 10 mL). The combined organic layers were washed with brine (10 mL), dried over Na₂SO₄ and concentrated *in vacuo.* The residue was purified by column chromatography (eluent: DCM/MeOH=7/1) to give **(I-602)** (12 mg, 42% yield). ESI-MS [M+H] ⁺: 600.2., δ 10.44 (s, 1H), 7.89 (s, 1H), 7.76 (d, J = 5.8 Hz, 1H), 7.55 (s, 1H), 7.39 (s, 1H), 7.34 - 7.21 (m, 4H), 7.14 (d, J = 7.7 Hz, 1H), 6.36 (d, J = 4.9 Hz, 1H), 6.19 (s, 1H), 4.34 (d, J = 5.5 Hz, 2H), 3.54 (s, 4H), 3.26 (s, 2H), 2.83 (s, 4H), 2.39 - 2.32 (m, 2H), 1.88-1.82 (m, 1H), 1.50 - 1.42 (m, 1H), 1.38-1.32 (m, 1H), 0.88 - 0.82 (m, 2H), 0.68-0.62 (m, 2H).

### Example 603: (1S,2S)-2-(3-chlorophenyl)-N-(4-(((6-cyclopropyl-8-(4-(2-hydroxyethyl)piperazin-1-yl)imidazo[1,2-a]pyridin-2-yl)methyl)amino)pyridin-2-yl)cyclopropane-1-carboxamide (I-603)

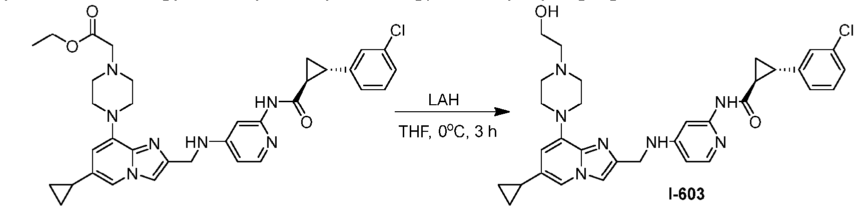

To a solution of **(I-600)** (15 mg, 0.024 mmol) in THF (5 mL) was added LAH (2 mg, 0.048 mmol) at 0 °C. After stirring at 0 °C for 3 h, the reaction was quenched with water (15 mL) and extracted with EtOAc (3 x 10 mL). The combined organic layers were washed with brine (15 mL), dried over Na₂SO₄ then concentrated *in vacuo* to give the crude, which was purified by column chromatography (eluent: DCM/MeOH=10/1) to give the (**I-603**) as a white solid. (10 mg, 69% yield). ESI-MS [M+H] ⁺: 586.3., δ 10.31 (s, 1H), 7.87 (s, 1H), 7.75 (d, J = 5.8 Hz, 1H), 7.53 (s, 1H), 7.44 (s, 1H), 7.34 - 7.22 (m, 3H), 7.17 - 7.04 (m, 2H), 6.33-6.31 (m, 1H), 6.15 (s, 1H), 4.44 (s, 1H), 4.33 (d, J = 5.6 Hz, 2H), 3.56-3.47 (m, 6H), 2.68-2.62 (m, 6H), 2.38-2.34 (m, 2H), 1.89 - 1.78 (m, 1H), 1.48 - 1.40 (m, 1H), 1.38-1.32 (m, 1H), 0.88-0.82 (m, 2H), 0.71 - 0.61 (m, 2H).

### Example 604: (1S,2S)-2-(3-chlorophenyl)-N-(4-(((6-cyclopropylimidazo[1,2-a]pyridin-2-yl)methyl)(2-hydroxyethyl)amino)pyridin-2-yl)cyclopropane-1-carboxamide (I-604)

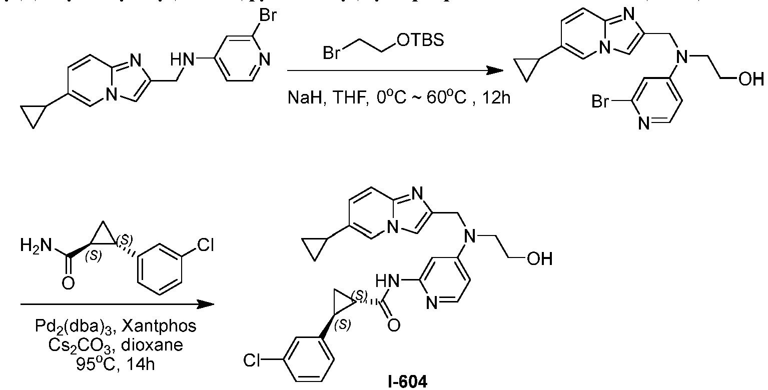

To a mixture of **Intermediate 770 table 25** (300 mg, 0.88 mmol) in DMF(5 mL) was added NaH (126 mg, 5.26 mmol) at 0 °C. After stirring at room temperature for 1 h, (2-bromoethoxy)(tert-butyl)dimethylsilane (315 mg, 1.32 mmol) was added to the mixture and stirred at 60 °C for 12 h. Water (30 mL) was added and the mixture was extracted with EtOAc (3 x 30 mL). The combined organic layers were washed with brine (20 mL), dried over Na₂SO₄ and concentrated *in vacuo* to give the crude, which was purified by column chromatography (eluent: DCM/MeOH = 20/1) to **2-((2-bromopyridin-4-yl)((6-cyclopropylimidazo[1,2-a]pyridin-2-yl)methyl)amino)ethan-1-ol intermediate 959** (100 mg, 28%) as a yellow solid. ESI-MS [M +H]+: 387.2.

To a mixture of **intermediate 959** (50 mg, 0.13 mmol), (1S,2S)-2-(3-chlorophenyl)cyclopropane-carboxamide(25 mg,0.13 mmol), and Cs₂CO₃ (126 mg,0.39 mmol) in 1,4-dioxane (5 mL) were added Pd₂(dba)₃ (17 mg,0.019mmol) and Xantphos (15.0 mg,0.026 mmol). The mixture was stirred at 95 °C for 14 h under N₂ and then filtered through Celite^{®}. The filter cake was washed with DCM/MeOH (10/1, 30 mL) and the filtrate was concentrated in vacuo to give the crude, which was purified by preparative TLC (DCM/MeOH = 20/1) to give (**I-604**) (30 mg, 47%) as a yellow solid. ESI-MS [M +H]+: 502.2, δ 10.38 (s, 1H), 8.30 (s, 1H), 7.79 (d, J = 6.0 Hz, 1H), 7.65 (s, 1H), 7.57 (s, 1H), 7.38 (d, J = 9.3 Hz, 1H), 7.26 - 7.25 (m, 1H), 7.24 (d, J = 7.1 Hz, 2H), 7.14 (d, J = 7.6 Hz, 1H), 7.00-6.97 (m, 1H), 6.46 - 6.45 (m, 1H), 5.33 (s, 1H), 4.66 (s, 2H), 3.66 - 3.51 (m, 4H), 2.42 - 2.33 (m, 2H), 1.93 - 1.89 (m, 1H), 1.47 - 1.41 (m, 1H), 1.38 - 1.33 (m, 1H), 0.91-1.85 (m, 2H), 0.67 - 0.64 (m, 2H).

### Example 605: (1S,2S)-2-(3-chlorophenyl)-N-(4-(((6-cyclopropyl-8-(4-methylpiperazin-1-yl) imidazo[1,2-a]pyridin-2-yl)methyl)amino)-6-methylpyridin-2-yl)cyclopropane-1-carboxamide (I-605)

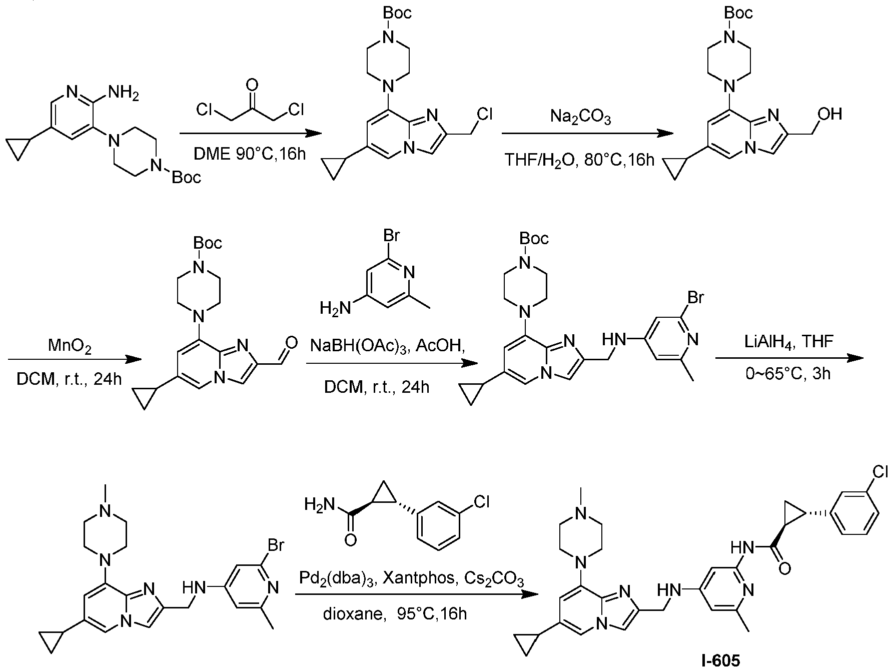

A mixture of *tert*-butyl 4-(2-amino-5-cyclopropylpyridin-3-yl)piperazine-1-carboxylate (800 mg, 2.52 mmol), 1,3-dichloropropan-2-one (639 mg, 5.03 mmol), and DIPEA (649 mg, 5.03 mmol) in DME (10 mL) was stirred at 90 °C for 16 h. The mixture was quenched with saturated aq. NaHCO₃ (50 mL) and extracted with EtOAc (3 x 50 mL). The combined organic layers were washed with brine (50 mL), dried over Na₂SO₄ and concentrated *in vacuo* to give ***tert*-butyl 4-(2-(chloromethyl)-6-cyclopropylimidazo[1,2-a]pyridin-8-yl)piperazine-1-carboxylate intermediate 960** as a yellow solid (700 mg, crude), which was used without purification. ESI-MS [M +H]⁺: 391.2.

A mixture of **intermediate 960** (700 mg, 1.79 mmol, crude) and Na₂CO₃ (951 mg, 8.97 mmol) in THF / H₂O (45 mL / 45 mL) was stirred at 80 °C for 16 h. The mixture was cooled to room temperature and extracted with EtOAc (3 x 50 mL). The organic layers were washed with brine (50 mL), dried over Na₂SO₄ and concentrated *in vacuo.* The residue was purified by column chromatography (eluent: DCM/MeOH = 0 ~ 15%) to give ***tert-*butyl 4-(6-cyclopropyl-2-(hydroxymethyl)imidazo[1,2-a]pyridin-8-yl)piperazine-1-carboxylate intermediate 961** as a red solid (470 mg, 70%). ESI-MS [M +H]⁺: 373.2.

A mixture of *tert*-butyl 4-(6-cyclopropyl-2-(hydroxymethyl)imidazo[1,2-a]pyridin-8-yl)-piperazine-1-carboxylate (370 mg, 1.0 mmol) and MnO₂ (435 mg, 5.0 mmol) in DCM (10 mL) was stirred at room temperature for 16 h. Further MnO₂ (435 mg, 5.0 mmol) was added into the mixture and stirring was continued for 8 h. The mixture was filtered and the filtrate was concentrated *in vacuo* to give the ***tert*-butyl 4-(6-cyclopropyl-2-formylimidazo[1,2-a]pyridin-8-yl)-piperazine-1-carboxylate intermediate 962** as a yellow solid (170 mg, crude) which was used without purification. ESI-MS [M +H]⁺: 371.2

A mixture of **intermediate 962**(370 mg, 1.0 mmol), 2-bromo-6-methylpyridin-4-amine (186 mg, 1.0 mmol), and AcOH (2 drops) in DCM (5 mL) was stirred at room temperature for 8 h. Then NaBH(OAc)₃ (318 mg, 1.5 mmol) was added and the mixture was stirred at room temperature for 16 h. The mixture was diluted with water (15 mL) and extracted with EtOAc (3 x 15 mL). The organic layers were washed with brine (15 mL), dried over Na₂SO₄ and concentrated in vacuo. The residue was purified by silica gel column chromatography (eluent: DCM/MeOH = 20 : 1) to give ***tert*-butyl 4-(2-(((2-bromo-6-methylpyridin-4-yl)amino)methyl)-6-cyclopropylimidazo[1,2-a]pyridin-8-yl)piperazine-1-carboxylate intermediate 963** as a yellow solid (75 mg, 20%). ESI-MS [M +H]⁺: 541.3.

To a mixture of **intermediate 963** (60 mg, 0.11 mmol) in THF (2 mL) was added LiAlH₄ (0.33 mL, 0.33 mmol) at 0 °C under N₂. The mixture was stirred at 65 °C for 16 h under N₂. The reaction was quenched with saturated aq. KOH (5 mL) and extracted with DCM (3 x 15 mL). The organic layers were washed with brine (15 mL), dried over Na₂SO₄ and concentrated *in vacuo.* The residue was purified by preparative TLC (eluent: DCM / MeOH=20 / 1) to give **2-bromo-N-((6-cyclopropyl-8-(4-methylpiperazin-1-yl)imidazo[1,2-a]pyridin-2-yl)methyl)-6-methylpyridin-4-amine intermediate 964** as a yellow solid (30 mg, 59%). ESI-MS [M +H]⁺: 455.1.

A mixture of (30 mg, 0.066 mmol), **intermediate 119** (13 mg, 0.066 mmol), Pd₂(dba)₃ (6 mg, 0.0066 mmol), Xantphos (4 mg, 0.0066 mmol), and Cs₂CO₃ (65 mg, 0.2 mmol) in dioxane (3 mL) was stirred at 95 °C for 16 h under N₂. The mixture was filtered through Celite^{®} and the filter cake was washed with DCM/MeOH (10/1, 30 mL). The filtrate was concentrated in vacuo to give the crude, which was purified by preparative TLC (DCM /MeOH=10 / 1) to give (**I-605**) as a light yellow solid (16.6 mg, 45%). ESI-MS [M +H]+: 570.2. δ 10.30 (s, 1H), 7.86 (s, 1H), 7.50 (s, 1H), 7.31 - 7.26 (m, 2H), 7.24 - 7.22 (m, 1H), 7.21 - 7.21 (m, 1H), 7.11 - 7.09 (m, 1H), 6.99 - 6.96 (m, 1H), 6.20 (d, J = 1.6 Hz, 1H), 6.15 (d, 1H), 4.30 (d, J = 5.8 Hz, 2H), 3.50 (s, 4H), 2.66 - 2.65 (m, 3H), 2.33 - 2.31 (m, 4H), 2.16 (s, 3H), 1.86 - 1.79 (m, 2H), 1.41 - 1.39 (m, 1H), 1.31 - 1.28 (m, 1H), 1.23 - 1.20 (m, 1H), 0.85 - 0.83 (m, 2H), 0.65 - 0.63 (m, 2H).

### Example 606: (1S,2S)-2-(3-chlorophenyl)-N-(5-(((6-cyclopropylimidazo[1,2-a]pyridin-2-yl)methyl)amino)-1,3,4-thiadiazol-2-yl)cyclopropane-1-carboxamide (I-606)

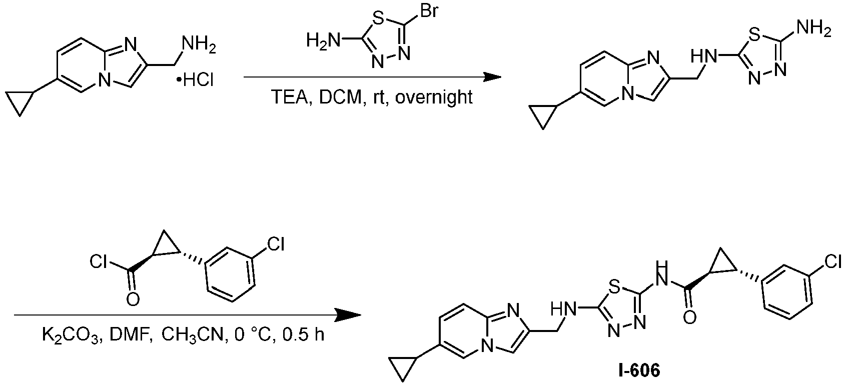

To a solution of intermediate 251 hydrochloride (600 mg, 2.68 mmol) in DCM (7 mL) was added TEA (1.12 mL, 8.05 mmol) and 5-bromo-1,3,4-thiadiazol-2-amine (724 mg, 4.02 mmol). After stirring at room temperature overnight, the reaction was quenched with saturated aq. NaHCO₃ (50 mL) and extracted with EtOAc (3 x 50 mL). The combined organic layers were washed with brine (30 mL), dried over Na₂SO₄ and concentrated *in vacuo.* The residue was purified by silica gel column chromatography (eluent: MeOH/DCM = 0 - 10%) to afford **N²-((6-cyclopropylimidazo[1,2-a]pyridin-2-yl)methyl)-1,3,4-thiadiazole-2,5-diamine intermediate 965** (206 mg, yield 27%) as off-white solid. ESI-MS [M +H]+: 287.1.

To a solution of **intermediate 965** (100 mg, 0.349 mmol) in DMF (0.7 mL) and CH₃CN (0.7 mL) was added K₂CO₃ (145 mg, 1.05 mmol). A solution of (1S,2S)-2-(3-chlorophenyl)cyclopropane-1-carbonyl chloride (80 mg, 0.347 mmol) in DCM (0.5 mL) was then added at 0 °C and the mixture stirred at 0 °C for 0.5 h. The mixture was diluted with water (30 mL) and extracted with DCM (3 x 20 mL). The combined organic layers were washed with brine (20 mL), dried over Na₂SO₄ and concentrated in vacuo. The residue was purified by preparative TLC (eluent: DCM: MeOH = 7: 1) to afford **(I-606)** e (45 mg, yield 28%) as white solid. ESI-MS [M +H]+: 465.1. δ 12.19 (s, 1H), 8.33 (s, 1H), 7.79 - 7.76 (m, 1H), 7.71 (s, 1H), 7.38 (d, J = 9.3 Hz, 1H), 7.34 - 7.25 (m, 3H), 7.15 (d, J = 7.6 Hz, 1H), 6.97 (dd, J = 9.3, 1.7 Hz, 1H), 4.50 (d, J = 5.5 Hz, 2H), 2.47 - 2.45 (m, 1H), 2.19 - 2.17 (m, 1H), 1.95 - 1.88 (m, 1H), 1.53 - 1.49 (m, 2H), 0.94 - 0.89 (m, 2H), 0.67 - 0.65 (m, 2H).

### Example 607 (1S,2S)-2-(3-chlorophenyl)-N-(4-(((6-cyclopropyl-8-(2-(methylamino)ethyl)imidazo[1,2-a]pyridin-2-yl)methyl)amino)pyridin-2-yl)cyclopropane-1-carboxamide (I-607)

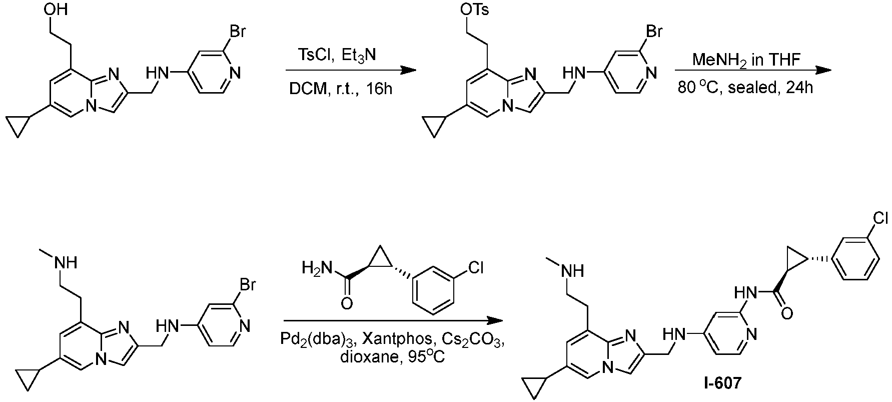

To a mixture of 2-(2-(((2-bromopyridin-4-yl)amino)methyl)-6-cyclopropylimidazo-[1,2-a]pyridin-8-yl)ethan-1-ol (38.6 mg, 0.1 mmol) and TEA (30.3 mg, 0.3 mmol) in DCM (5 mL) was added TsCl (28.5 mg, 0.15 mmol) at room temperature. The mixture was stirred at room temperature for 16 h. The reaction was quenched with water (30 mL) and extracted with EtOAc (3 x 30 mL). The combined organic layers were washed with brine (30 mL), dried over Na₂SO₄ and concentrated *in vacuo.* The residue was by preparative TLC (eluent: DCM/MeOH = 10/1) to give **2-(2-(((2-bromopyridin-4-yl)amino)methyl)-6-cyclopropylimidazo[1,2-a]pyridin-8-yl)ethyl 4-methylbenzenesulfonate intermediate 966** (35 mg, 65 %) as a yellow solid. ESI-MS [M +H]⁺: 541.2.

A mixture of **intermediate 966** (35 mg, 0.065 mmol) in MeNH₂/THF (2.0 M in THF, 3.0 mL) was stirred at 80 °C in a sealed tube for 24 h. The mixture was concentrated *in vacuo* and purified by preparative TLC (eluent: DCM/MeOH = 10/1) to give **2-bromo-N-((6-cyclopropyl-8-(2-(methylamino)ethyl)imidazo[1,2-a]pyridin-2-yl)methyl)pyridin-4-amine intermediate 967** (15.0 mg, 58 %) as a light yellow solid. ESI-MS [M +H]⁺: 401.1.

A mixture of **intermediate 967** (15.0 mg, 0.038 mmol), **intermediate** 119 (7.3 mg, 0.038 mmol), Pd₂(dba)₃ (3.4 mg, 0.0038 mmol), Xantphos (2.2 mg, 0.0038 mmol), and Cs₂CO₃ (36.8 mg, 0.11 mmol) in dioxane (2.0 mL) was stirred at 100 °C under N₂ for 5 h. The mixture was quenched with water (30 mL) and extracted with EtOAc (3 x 30 mL). The combined organic layers were washed with brine (30 mL), dried over Na₂SO₄ and concentrated in vacuo. The residue was purified by preparative TLC (eluent: DCM/MeOH = 8/1) to give (I-607) (4.2 mg, 22 %) as a white yellow solid. ESI-MS [M +H]⁺: 515.2. δ 10.31 (s, 1H), 8.16 (s, 1H), 7.75 (d, J = 5.7 Hz, 1H), 7.60 (s, 1H), 7.44 (s, 1H), 7.39 - 7.19 (m, 3H), 7.14 (d, J = 7.0 Hz, 2H), 6.81 (s, 1H), 6.32 (d, J = 4.0 Hz, 1H), 4.36 (d, J = 5.1 Hz, 2H), 3.03 - 2.94 (m, 2H), 2.91 - 2.87 (m,, 2H), 2.41 - 2.30 (m, 5H), 1.92 - 1.80 (m, 1H), 1.48 - 1.40 (m, 1H), 1.40 - 1.29 (m, 1H), 0.95 - 0.83 (m, 2H), 0.70 - 0.59 (m, 2H).

### Examples 608-609

### (1S,2S)-2-(3-chlorophenyl)-N-(4-(((6-cyclopropyl-8-(3-hydroxy-1-methylazetidin-3-ylimidazo[1,2-a]pyridin-2-yl)methyl)amino)pyridin-2-yl)cyclopropane-1-carboxamide (I-608) & (1S,2S)-2-(3-chlorophenyl)-N-(4-(((6-cyclopropyl-8-(1-methylazetidin-3-yl)imidazo[1,2-a]pyridin-2-yl)methyl)amino)pyridin-2-yl)cyclopropane-1-carboxamide (I-609)

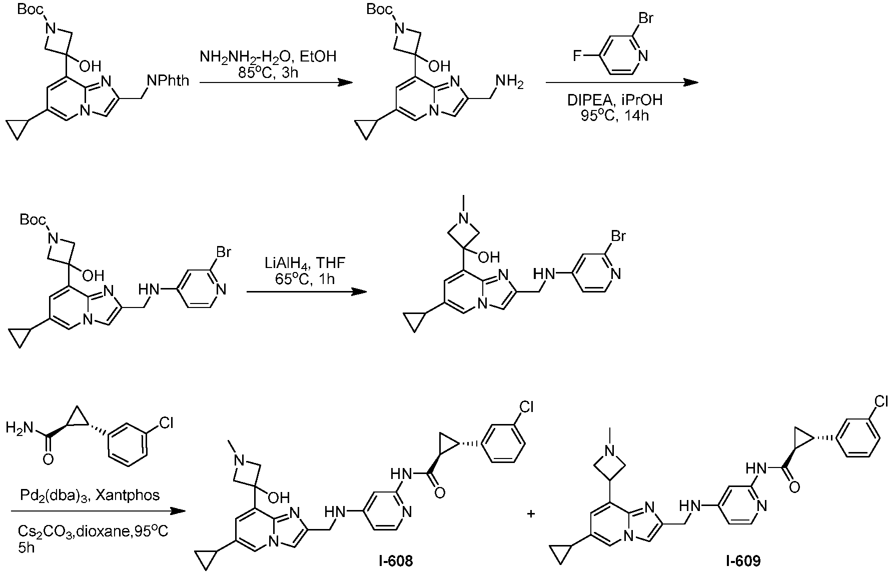

To a solution of *tert-butyl* 3-(6-cyclopropyl-2-((1,3-dioxoisoindolin-2-yl)methyl) imidazo[1,2-a]pyridin-8-yl)-3-hydroxyazetidine-1-carboxylate (1 g, 2.0 mmol)) in EtOH (30 mL) was added hydrazine hydrate (1 g, 20.0 mmol). The mixture was stirred at 85 °C for 3 h and cooled to room temperature. The mixture was filtered and the filtrate was concentrated *in vacuo* to **give *tert*-butyl 3-(2-(aminomethyl)-6-cyclopropylimidazo[1,2-a]pyridin-8-yl)-3-hydroxyazetidine-1-carboxylate intermediate 968** (730 mg, crude) as a yellow solid. ESI-MS [M +H]+: 359.2

To a solution of **intermediate 968** (700 mg, crude) and 2-bromo-4-fluoropyridine (704 mg, 4.0 mmol) in IPA (30 mL) was added DIPEA (1.29 g, 10.0 mmol). The mixture was stirred at 95 °C for 12 h under N₂. The reaction was quenched with water (50 mL) and extracted with EtOAc (3 x 50 mL). The combined organic layers were washed with brine (50 mL), dried over Na₂SO₄ and concentrated in vacuo. The residue was purified by column chromatography (eluent: DCM/MeOH= 15/1) to give **tert-butyl 3-(2-(((2-bromopyridin-4-yl)amino)methyl)-6-cyclopropylimidazo[1,2-a]pyridin-8-yl)-3-hydroxyazetidine-1-carboxylate intermediate 969** (530 mg, 52% yield, for two steps) as a pink solid. ESI-MS [M +H]+: 514.1

To a solution of **intermediate 969** (330 mg, 0.64 mmol) in THF (25 mL) was added LiAlH₄ (3.84 mL, 3.84 mmol, 1M in THF) at 0 °C. After stirring at 70 °C for 1 h under N₂, the reaction was quenched with saturated aq. NH₄Cl (50 mL). The aqueous phase was extracted with EtOAc (3 x 30 mL). The combined organic layers were washed with brine (30 mL), dried over Na₂SO₄, then concentrated *in vacuo* to give the crude, which was purified by Pre-TLC (eluent: DCM/MeOH= 10/1) to give the product **3-(2-(((2-bromopyridin-4-yl)amino)methyl)-6-cyclopropylimidazo[1,2-a]pyridin-8-yl)-1-methylazetidin-3-ol intermediate 970** (130 mg, 47%) as a yellow solid. ESI-MS [M +H]+: 428.1

A mixture of **intermediate 970** (130 mg, 0.30 mmol), **intermediate 119** (66 mg, 0.33 mmol), Pd₂(dba)₃ (56 mg, 0.068 mmol), Xantphos (35 mg, 0.068 mmol), and Cs₂CO₃ (298 mg, 0.91 mmol) in dioxane (15 mL) was stirred at 95 °C for 5 h. The reaction was quenched with water (50 mL) and extracted with EtOAc (3 x 30 mL). The combined organic layers were washed with brine (30 mL), dried over Na₂SO₄, and concentrated in vacuo to give the crude, which was purified by Pre-TLC (eluent: DCM/MeOH= 10/1) to give the product **(I-608)** (10 mg, 12 %) as a white solid. ESI-MS [M +H]+: 543.2. δ 10.32 (s, 1H), 8.25 (d, J = 1.2 Hz, 1H), 7.80 - 7.72 (m, 1H), 7.65 (s, 1H), 7.45 (s, 1H), 7.31 (t, J = 7.9 Hz, 1H), 7.26 - 7.23 (m, 2H), 7.15 - 7.10 (m, 3H), 6.43 (s, 1H), 6.34 - 6.32 (m, 1H), 4.43 - 4.35 (m, 2H), 3.97 (d, J = 6.3 Hz, 2H), 3.37 - 3.35 (m, 2H), 2.42 (s, 3H), 2.34 - 2.32 (m, 2H), 2.03 - 1.91 (m, 1H), 1.49 - 1.40 (m, 1H), 1.38 - 1.31 (m, 1H), 0.94 - 0.89 (m, 2H), 0.70 - 0.65 (m, 2H).

Also isolated was **(I-609)** (10 mg, 12 %) as a white solid. ESI-MS [M +H]+: 527.2., ¹H NMR (400 MHz, DMSO) substantially similar to (I-608).

### Example 610: (1S,2S)-2-(3-chlorophenyl)-N-(5-(((6-cyclopropylimidazo[1,2-a]pyridin-2-yl)methyl)amino)-1,2,4-thiadiazol-3-yl)cyclopropane-1-carboxamide (I-610)

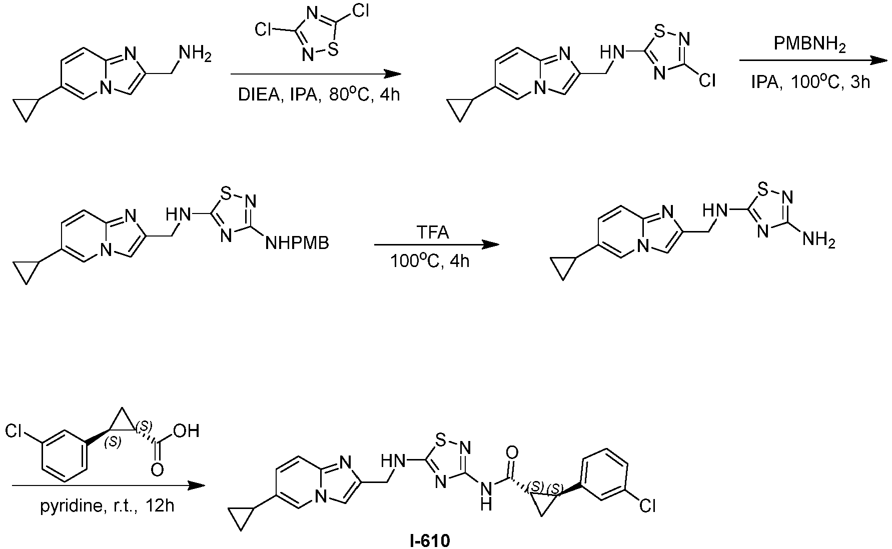

A solution of **intermediate 251** (0.5 g, 2.67 mmol), 3,5-dichloro-1,2,4-thiadiazole (494mg, 3.21mmol), and DIPEA (1.03 g, 8.01 mmol) in IPA (20 mL) was stirred at 80 °C for 4 h. The reaction was quenched with water (30 mL) and extracted with EtOAc (3 x 30 mL). The combined organic layers were washed with brine (30 mL), dried over Na₂SO₄ and concentrated *in vacuo* to give the crude, which was purified by silica gel column chromatography (eluent: DCM/MeOH = 10/1) to give the **3-chloro-N-((6-cyclopropylimidazo[1,2-a]pyridin-2-yl)methyl)-1,2,4-thiadiazol-5-amine intermediate 971** as a yellow solid. (400 mg, 49%), ESI-MS [M +H]+: 306.2

A solution of **intermediate 971** (500 mg, 1.64 mmol) and (4-methoxyphenyl)methanamine (1.12 g, 8.20 mmol) in DMSO (10 mL) was stirred at 100 °C for 3 h. The reaction was quenched with water (100 mL) and extracted with EtOAc (3 x 30 mL). The combined organic layers were washed with brine (30 mL), dried over Na₂SO₄ then concentrated *in vacuo* to give **N5-((6-cyclopropylimidazo[1,2-a]pyridin-2-yl)methyl)-N3-(4-methoxybenzyl)-1,2,4-thiadiazole-3,5-diamine intermediate 972** as a white solid (350 mg, crude) which was used without further purification. ESI-MS [M +H]+: 407.2.

A solution of **intermediate 972** (400 mg, 0.99 mmol) in TFA (5 mL) was stirred at 100 °C for 4 h. The reaction was quenched with saturated aq. NaHCO3 (100 mL) and extracted with EtOAc (3 x 30 mL). The combined organic layers were washed with brine (30 mL), dried over Na₂SO₄, concentrated *in vacuo* and purified by silica gel column chromatography (eluent: DCM/MeOH=10/1) to give **N5-((6-cyclopropylimidazo[1,2-a]pyridin-2-yl)methyl)-1,2,4-thiadiazole-3,5-diamine intermediate 973** (200 mg, 71% yield) as a white solid. ESI-MS [M +H]+: 287.2.

To a solution of (1S,2S)-2-(3-chlorophenyl)cyclopropane-1-carboxylic acid (33 mg, 0.17mmol) in DCM (5 ml ) was added oxalyl dichloride ( 43 mg, 0.34 mmol ) and DMF (3 drops) at 0 °C under N2. The mixture was stirred at 0 °C for 3 h and evaporated to give the product as a light-yellow oil (36 mg, crude). DCM (3 mL) was added, followed by **intermediate 973** (50 mg, 0.17 mmol) and pyridine (1 mL). The mixture was stirred at room temperature for 12 h, then quenched with saturated aq. NaHCO₃ (50 mL) and extracted with EtOAc (3 x 10 mL). The combined organic layers were washed with brine (30 mL), dried over Na₂SO₄ and concentrated in vacuo to give the crude, which was purified by silica gel column chromatography (eluent: DCM/MeOH = 10/1) to give **(I-610)** as a white solid. (20 mg, 25 %). ESI-MS [M +H]⁺: 465.0., δ 10.89 (s, 1H), 8.87 (s, 1H), 8.32 (s, 1H), 7.72 (s, 1H), 7.40 (d, J = 9.3 Hz, 1H), 7.35 - 7.27 (m, 1H), 7.27 - 7.21 (m, 2H), 7.13 (d, J = 7.6 Hz, 1H), 7.01-6.98 (m, 1H), 4.54 (d, J = 4.7 Hz, 2H), 2.38-2.32 (m, 2H), 1.97 - 1.88 (m, 1H), 1.48-1.43 (m, 1H), 1.37-1.33 (m, 1H), 0.95 - 0.89 (m, 2H), 0.70 - 0.62 (m, 2H).

### Example 611: (1S,2S)-2-(3-chlorophenyl)-N-(4-(((6-cyclopropylimidazo[1,2-a]pyridin-2-yl)methyl)amino)-6-hydroxypyridin-2-yl)cyclopropane-1-carboxamide (I-611)

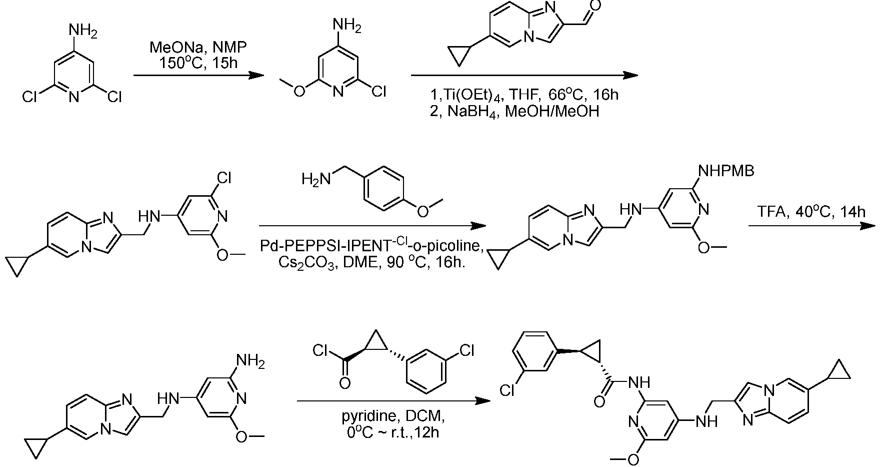

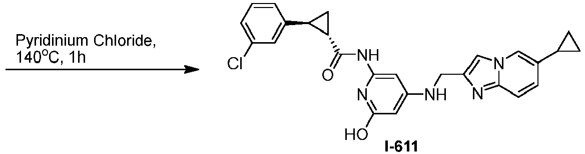

A mixture of 2,6-dichloropyridin-4-amine (1 g, 6.13 mmol) and NaOMe (1 g, 19.39 mmol) in NMP (40 mL) was stirred at 150 °C for 15h. After cooling to room temperature. the reaction was quenched with water (80 mL) and extracted with EtOAc (3 x 80 mL). The combined organic layers were washed with brine (50 mL), dried over Na₂SO₄, and concentrated *in vacuo* to givethe crude, which was purified by silica gel column chromatography (eluent: PE/EtAOc = 3/1) to give **2-chloro-6-methoxypyridin-4-amine intermediate 974** (400 mg, 40.8%) as yellow oil. ESI-MS [M +H]+: 159.2

A mixture of **intermediate 974** (400 mg, 2.51 mmol), **intermediate 828** (466.9 mg, 2.51 mmol) and Ti(OEt)₄ (1.14 g, 5.02 mmol) in THF (20 mL) was stirred at 66 °C for 16 h. The reaction was cooled to room temperature, and then MeOH (10 mL) was added, followed by NaBH₄ (286 mg, 7.53 mmol). The resulting reaction mixture was stirred at room temperature for another 2 h. The reaction was quenched with saturated aq. NH₄Cl (30 mL) and extracted with DCM (3 x 50 mL). The combined organic layers were washed with brine (30 mL), dried over Na₂SO₄, and concentrated *in vacuo* to give the crude, which was purified with silica gel column chromatography (eluent: DCM/MeOH = 15/1) to give **2-chloro-N-((6-cyclopropylimidazo[1,2-a]pyridin-2-yl)methyl)-6-methoxypyridin-4-amine intermediate 975** (350 mg, 43%) as a yellow solid. ESI-MS [M +H]+: 329.2

A mixture of **2intermediate 975** (350 mg, 1.1 mmol), (4-methoxyphenyl)methanamine (370 mg, 2.7 mmol), Pd-PEPPSI-IPENT⁻Cl-o-picoline (92.4 mg, 0.11 mmol), and Cs₂CO₃ (1.1 g, 3.3 mmol) in DME (25 mL) was stirred at 90 °C for 16 h. The reaction was cooled to room temperature and filtered. The filtrate was concentrated *in vacuo* to give the crude, which was purified with preparative TLC (eluent: DCM /MeOH = 15/1) to give **N4-((6-cyclopropylimidazo[1,2-a]pyridin-2-yl)methyl)-6-methoxy-N2-(4-methoxybenzyl)pyridine-2,4-diamine intermediate 976** (265 mg, 56%) as a yellow solid. ESI-MS [M +H]+: 430.2

A solution of **intermediate 976** (265 mg, 0.62 mmol) in TFA (10 mL) was stirred at 40 °C for 14 h. The reaction was concentrated *in vacuo* to give the residue, which was washed with saturated aq. NaHCO₃ (30 mL) and extracted with EtOAc (3 x 50 mL). The combined organic layers were washed with brine (40 mL), dried over Na₂SO₄, and concentrated *in vacuo* to give the crude, which was purified with preparative TLC (eluent: DCM / MeOH = 10/1) to give **N4-((6-cyclopropylimidazo[1,2-a]pyridin-2-yl)methyl)-6-methoxypyridine-2,4-diamine intermediate 977** (120 mg, 63%) as a yellow solid. ESI-MS [M +H]+: 310.2

To a solution of **intermediate 977** (120 mg, 0.39 mmol) in pyridine/DCM (2mL / 10 mL) was added (1S,2S)-2-(3-chlorophenyl)cyclopropane-1-carbonyl chloride (167.7 mg, 0.78 mmol) at 0 °C. The resulting reaction mixture was stirred at room temperature for 12 h. The reaction was quenched with saturated aq. NH₄Cl (20 mL) and extracted with DCM (3 x 30 mL). The combined organic layers were washed with brine (30 mL), dried over Na₂SO₄, and concentrated in vacuo to give crude, which was purified with preparative TLC (eluent: DCM/MeOH = 10/1) to give **(1S,2S)-2-(3-chlorophenyl)-N-(4-(((6-cyclopropylimidazo[1,2-a]pyridin-2-yl)methyl)amino)-6-methoxypyridin-2-yl)cyclopropane-1-carboxamide intermediate 978** (75 mg, 39%) as a yellow solid. ESI-MS [M +H]+: 488.2

A mixture of **intermediate 978** (75 mg, 0.15 mmol) and pyridinium chloride (345 mg, 3 mmol) was stirred at 140 °C for 1 h. The reaction was washed with water (30 mL) and extracted with EtOAc (3 x 40 mL). The combined organic layers were washed with brine (40 mL), dried over Na₂SO₄, and concentrated in vacuo to give the crude, which was purified with silica gel column chromatography (eluent: DCM / MeOH = 10/1) to give (**(I-611)** (15 mg, 21%) as a white solid. ESI-MS [M +H]+: 474.2., δ 10.67 (s, 1H), 10.45 (s, 1H), 8.32 (s, 1H), 7.65 (s, 1H), 7.38 (d, J = 9.3 Hz, 1H), 7.33 - 7.25 (m, 3H), 7.17 (d, J = 7.6 Hz, 1H), 7.10 (t, J = 3.8 Hz, 1H), 6.98 - 6.96 (m, 1H), 5.69 (s, 1H), 5.05 (s, 1H), 4.27 (d, J = 8.0 Hz, 2H), 2.47 - 2.42 (m, 2H), 1.94 - 1.88 (m, 1H), 1.54 - 1.50 (m, 1H), 1.48 - 1.43 (m, 1H), 0.93 - 0.87 (m, 2H), 0.71 - 0.65 (m, 2H).

### Example 612: (1S,2S)-2-(3-chlorophenyl)-N-(5-(((6-cyclopropyl-8-(4-methylpiperazin-1-ylimidazo[1,2-a]pyridin-2-yl)methyl)amino)pyridazin-3-yl)cyclopropane-1-carboxamide (I-612)

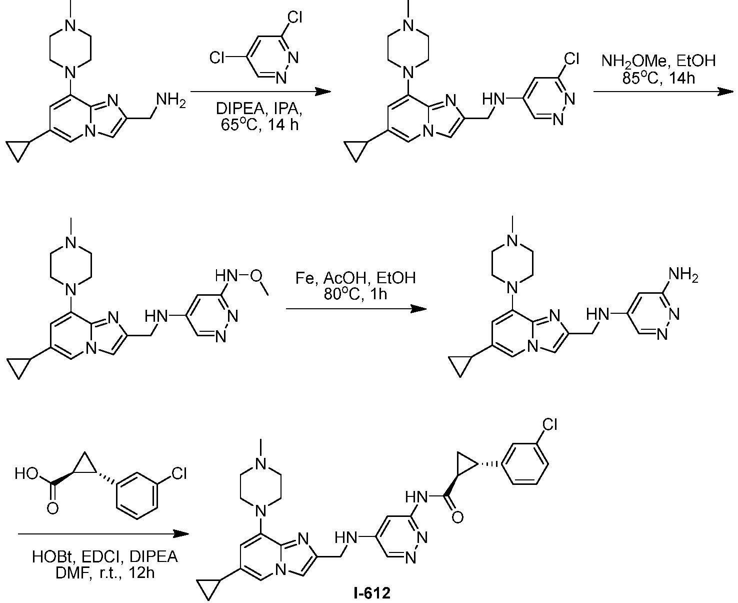

A mixture of **intermediate 381** (570 mg, 2 mmol), 3,5-dichloropyridazine (298 mg, 2 mmol), and DIPEA (1.42 mL, 18 mmol) in IPA (15 mL) was stirred at 65 °C for 14 h. The mixture was quenched with saturated aq. NaHCO₃ (50 mL) and extracted with EtOAc (3 x 50 mL). The combined organic layers were washed with brine (30 mL), dried over Na₂SO₄ and concentrated *in vacuo.* The residue was purified by column chromatography (eluent: DCM / MeOH = 15/1) to give **6-chloro-N-((6-cyclopropyl-8-(4-methylpiperazin-1-yl)imidazo[1,2-a]pyridin-2-yl)methyl) pyridazin-4-amine intermediate 979** (350 mg, 46%) as a yellow solid. ESI-MS [M +H]+: 398.2

A mixture of **intermediate 979** (200 mg, 0.5 mmol) and NH₂OMe (236 mg, 5 mmol) in EtOH (10 mL) was stirred at 85 °C for 14h. The reaction was quenched with saturated aq. NaHCO₃ (50 mL) and extracted with EtOAc (3 x 50 mL). The combined organic layers were washed with brine (30 mL), dried over Na₂SO₄ and concentrated *in vacuo.* The residue was purified by preparative TLC (eluent: DCM / MeOH = 15/1) to give **N-((6-cyclopropyl-8-(4-methylpiperazin-1-yl)imidazo[1,2-a]pyridin-2-yl)methyl)-6-(methoxyamino)pyridazin-4-amine intermediate** 980 (160 mg, 78%) as a yellow solid. ESI-MS [M +H]+: 409.2

A mixture of **intermediate 980** (160 mg, 0.39 mmol) and Fe (109 mg, 1.95 mmol) in AcOH/EtOH (2 mL / 10 mL) was stirred at 80 °C for 1 h. The reaction mixture was filtered and washed with MeOH (20 mL). The combined filtrate was concentrated *in vacuo* to give **N5-((6-cyclopropyl-8-(4-methylpiperazin-1-yl)imidazo[1,2-a]pyridin-2-yl)methyl)pyridazine-3,5-diamine 981** (100 mg, 68%) as a yellow solid which was used in next step directly without further purification. ESI-MS [M +H]+: 379.2

A mixture of **intermediate 981** (60 mg, 0.16 mmol), (1S,2S)-2-(3-chlorophenyl)cyclo propane-1-carboxylic acid (37 mg, 0.19 mmol), HOBt (33 mg, 0.24 mmol), EDCI (46 mg, 0.24 mmol), and DIPEA (83 mg, 0.64 mmol) in DMF (3 mL) was stirred at room temperature for 12 h. The reaction was quenched with water (30 mL) and extracted with EtOAc (4 x 25 mL). The combined organic layers were washed with brine (30 mL), dried over Na₂SO₄, and concentrated in vacuo to give the crude, which was purified with preparative TLC (eluent: DCM / MeOH = 15/1) to give **(I-612)** (10 mg, 11%) as a white solid. ESI-MS [M +H]+: 557.2, δ 10.84 (s, 1H), 8.40 (d, J = 1.4 Hz, 1H), 7.87 (s, 1H), 7.66 - 7.53 (m, 3H), 7.33 - 7.25 (m, 3H), 7.15 (d, J = 7.5 Hz, 1H), 6.16 (s, 1H), 4.37 (d, J = 5.4 Hz, 2H), 3.55 - 3.45 (m, 4H), 2.61 - 2.53 (m, 4H), 2.41 - 2.38 (m, 2H), 2.26 (s, 3H), 1.88 - 1.82 (m, 1H), 1.47 - 1.44 (m, 1H), 1.42 - 1.39 (m, 1H), 0.89 - 0.84 (m, 2H), 0.69-0.63 (m, 2H).

### Example 613: (1S,2S)-2-(3-chlorophenyl)-N-(2-(((6-cyclopropy-8-(4-methylpiperazin-1-yl)imidazo[1,2-a]pyridin-2-yl)methyl)amino)pyrimidin-4-yl)cyclopropane-1-carboxamide (I-613)

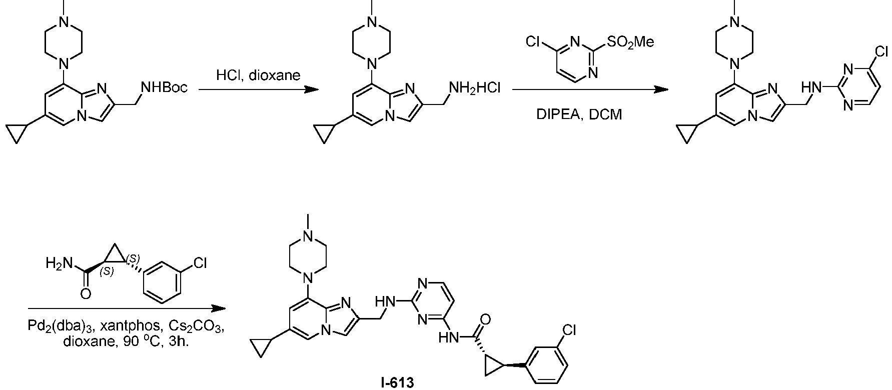

HCl (4M solution in dioxane, 5 mL) was added to a solution of *tert*-butyl ((6-cyclopropyl-8-(4-methylpiperazin-1-yl)imidazo[1,2-a]pyridin-2-yl)-methyl)carbamate (385 mg, 1.0 mmol) in MeOH (5 mL). The mixture was stirred at room temperature for 1 h and concentrated *in vacuo* to give **(6-cyclopropyl-8-(4-methylpiperazin-1-yl)imidazo[1,2-a]pyridin-2-yl)methanamine as the hydrochloric acid salt intermediate 381** (300 mg, 94%) as a yellow solid which was used in next step directly without further purification. ESI-MS [M +H]+: 286.1A mixture of the latter intermediate (200 mg, 0.63 mmol), 4-chloro-2-(methylsulfonyl)pyrimidine (121.0 mg, 0.63 mmol), and DIPEA (162.5 mg, 1.26 mmol) in dry DCM (20 mL) was stirred at room temperature for 1 h. The mixture was quenched with saturated aq. NaHCO₃ (50 mL) and extracted with EtOAc (3 x 50 mL). The combined organic layers were washed with brine (30 mL), dried over Na₂SO₄ and concentrated *in vacuo.* The residue was purified by preparative TLC (eluent: DCM/MeOH = 10/1) to give **4-chloro-N-((6-cyclopropyl-8-(4-methylpiperazin-1-yl)imidazo[1,2-a]pyridin-2-yl) methyl)pyrimidin-2-amine intermediate 982** (30 mg, 12 %) as a yellow solid. ESI-MS [M +H]⁺: 398.2.

To a mixture of **intermediate 982** (30 mg, 0.076 mmol), **intermediate 119** (14.7 mg, 0.076 mmol), and Cs₂CO₃ (74.3 mg, 0.228 mmol) in 1,4-dioxane (5 mL) were added Pd₂(dba)₃ (7.0 mg, 0.0076 mmol) and Xantphos (8.8 mg, 0.015 mmol). After stirring at 90 °C for 3 h under N₂, the reaction mixture was filtered through Celite^{®} and the filter cake was washed with DCM/MeOH (10/1, 20 mL). The filtrate was concentrated in vacuo to give the crude, which was purified by preparative HPLC (eluent: DCM/MeOH = 10/1) to give (**I-613**) (13.1 mg, 31%) as a white solid. ESI-MS [M +H]⁺: 557.2., ¹H NMR (400 MHz, MeOD) δ 8.15 (d, J = 5.9 Hz, 1H), 7.95 (s, 1H), 7.71 (s, 1H), 7.43 (d, J = 5.9 Hz, 1H), 7.26 - 7.20 (m, 1H), 7.23 - 7.14 (m, 2H), 7.11 (d, J = 7.6 Hz, 1H), 6.69 (s, 1H), 4.72 (s, 2H), 3.71 - 3.39 (m, 4H), 3.31 - 3.30 (m, 4H), 3.00 (s, 3H), 2.57 - 2.45 (m, 1H), 2.27 - 2.13 (m, 1H), 1.99 - 1.91 (m, 1H), 1.70 - 1.59 (m, 1H), 1.47 - 1.35 (m, 1H), 1.05 - 0.91 (m, 2H), 0.80 - 0.65 (m, 2H).

### Example 614: rac-(1S*,2S*)-2-(3-chloropyridinyl)-N-(5-(((6-cyclopropylimidazo[1,2-a]pyridin-2-yl)methyl)amino)-1,2,4-thiadiazol-3-yl)cyclopropane-1-carboxamide (I-614)

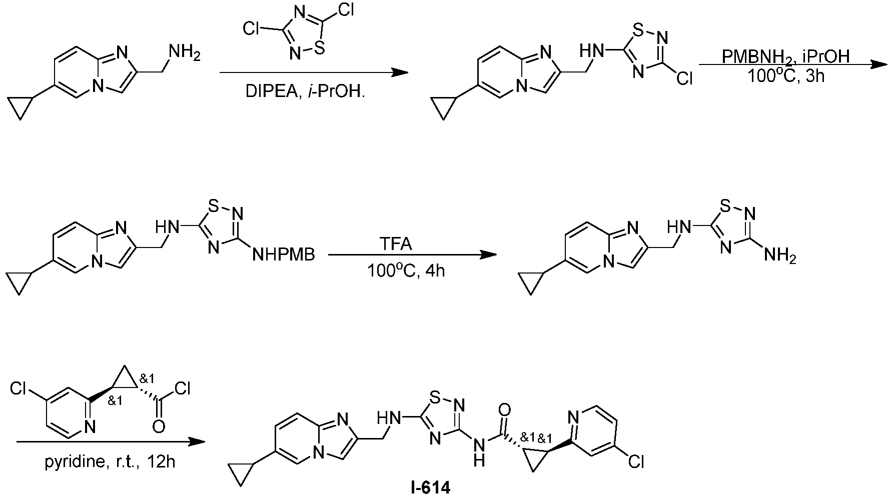

A solution of **intermediate 972** (400 mg, 0.99 mmol) in TFA (5 mL) was stirred at 100 °C for 4 h. The reaction was quenched with NaHCO₃ (50 mL) and extracted with EtOAc (3 x 30 mL). The combined organic layers were washed with brine, dried over Na₂SO₄, and then concentrated *in vacuo* to give the crude, which was purified by silica gel column chromatography (eluent: DCM/MeOH=10/1) to give **N5-((6-cyclopropylimidazo[1,2-a]pyridin-2-yl)methyl)-1,2,4-thiadiazole-3,5-diamine intermediate 983** (200 mg, 71%) as a white solid. ESI-MS [M +H]+: 287.2.

To a solution of **intermediate 1149** (82 mg, 0.42 mmol) in DCM (5 ml) was added oxalyl dichloride ( 53 mg, 0.34 mmol) and DMF (3 drops ) at 0 °C under N₂. After stirring at 0 °C for 3 h, the mixture was evaporated to give **rac-(1S*,2S*)-2-(3-chlorophenyl)cyclopropane-1-carboxylic acid** as a light yellow oil (80 mg, crude). This was dissolved in DCM (3 mL) then **intermediate 983** (80 mg, 0.28 mmol) and pyridine (1 mL) were added. The mixture was stirred at room temperature for 12 h. The reaction mixture was quenched with water (10 mL) and extracted with EtOAc (3 x 10 mL). The combined organic layers were washed with brine, dried over Na₂SO₄, and concentrated in vacuo to give the crude, which was purified by silica gel column chromatography (eluent: DCM/MeOH = 10/1) to give(**I-614)** (20 mg, 15%) as a white solid. ESI-MS [M +H]+: 466.1, δ 10.90 (s, 1H), 8.87 (s, 1H), 8.40 (d, J = 5.3 Hz, 1H), 8.32 (s, 1H), 7.72 (s, 1H), 7.64 (s, 1H), 7.39 (d, J = 9.3 Hz, 1H), 7.33 (d, J = 5.2 Hz, 1H), 6.98 (d, J = 9.3 Hz, 1H), 4.53 (d, J = 3.9 Hz, 2H), 2.58-2.55 (m, 2H), 1.96 - 1.86 (m, 1H), 1.48-1.42 (m, 2H), 0.94 - 0.88 (m, 2H), 0.72 - 0.60 (m, 2H).

### Example 615: (1S,2S)-2-(3-chlorophenyl)-N-(5-(((6-cyclopropylimidazo[1,2-a]pyridin-2-yl) methyl)amino)-1,2,4-oxadiazol-3-yl)cyclopropane-1-carboxamide (I-615)

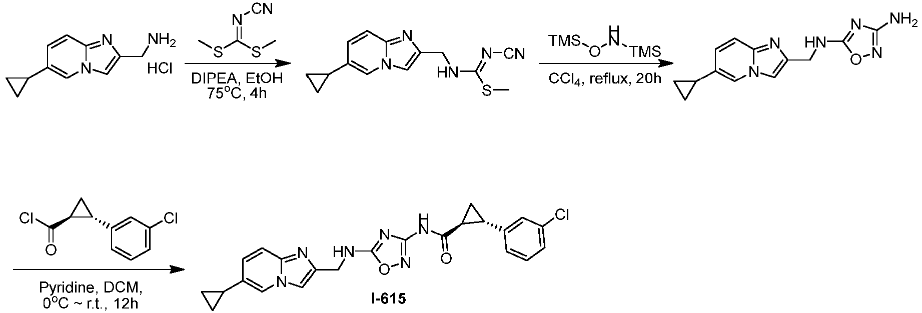

To a mixture of **intermediate 251** hydrochloride (450 mg, 2 mmol) and dimethyl cyanocarbonimidodithioate (438 mg, 3 mmol) in EtOH (20 mL) was added DIPEA (774 mg, 6 mmol) at room temperature and then was stirred at 75 °C for 4 h. The reaction was cooled to room temperature and concentrated *in vacuo* to give the crude, which was purified by silica gel column chromatography (eluent: DCM/MeOH = 30/1) to give the ***methyl (Z)-N'-cyano-N-((6-cyclopropylimidazo[1,2-a]pyridin-2-yl)methyl)carbamimidothioate* intermediate 984** (450 mg, 9%) as a yellow solid. ESI-MS [M +H]+: 286.1.

To a mixture of **intermediate 984** (250 mg, 0.88 mmol) in CC14 (4 mL) and 1,4-dioxane (4 mL) was added N,O-bis(trimethylsilyl)hydroxylamine (779 mg, 4.4 mmol). After stirring at 90 °C for 20 h, the reaction mixture was then cooled to room temperature and concentrated *in vacuo* to give the crude, which was purified by silica gel column chromatography (eluent: DCM/MeOH = 30/1) to give the **N⁵-((6-cyclopropylimidazo[1,2-a]pyridin-2-yl)methyl)-1,2,4-oxadiazole-3,5-diamine intermediate 985** (150 mg, 63%) as a yellow oil. ESI-MS [M +H]+: 271.1.

To a stirred solution of **intermediate 985** (90 mg, 0.33 mmol) in DCM (6 mL) was added pyridine (1 mL) and (1S,2S)-2-(3-chlorophenyl)cyclopropane-1-carbonyl chloride (140 mg, 0.66 mmol ) at 0 °C. After stirring at room temperature for 12 h, the reaction mixture was concentrated in vacuo to give the crude, which was purified with preparative TLC (eluent: DCM/MeOH = 20/1) to give (I-615) (7.3 mg, 5 %) as a white solid. ESI-MS [M +H]⁺: 449.1. δ 10.08 (s, 1H), 8.33 (s, 1H), 7.68 (s, 1H), 7.59 (t, J = 9.4 Hz, 1H), 7.41 - 7.34 (m, 2H), 7.33 - 7.28 (m, 2H), 7.24 - 7.21 (m, 1H), 7.01 - 6.92 (m, 1H), 4.42 (d, J = 5.2 Hz, 2H), 2.75 - 2.64 (m, 2H), 1.99 - 1.89 (m, 1H), 1.81 - 1.73 (m, 2H), 0.94 - 0.89 (m, 2H), 0.69 - 0.65 (m, 2H).

### Example 616: (1S,2S)-2-(3-chlorophenyl)-N-(6-((2R,4S)-2-(6-cyclopropylimidazo[1,2-a]pyridin-2-yl)-4-methoxypyrrolidin-1-yl)pyrimidin-4-yl)cyclopropane-1-carboxamide (I-616)

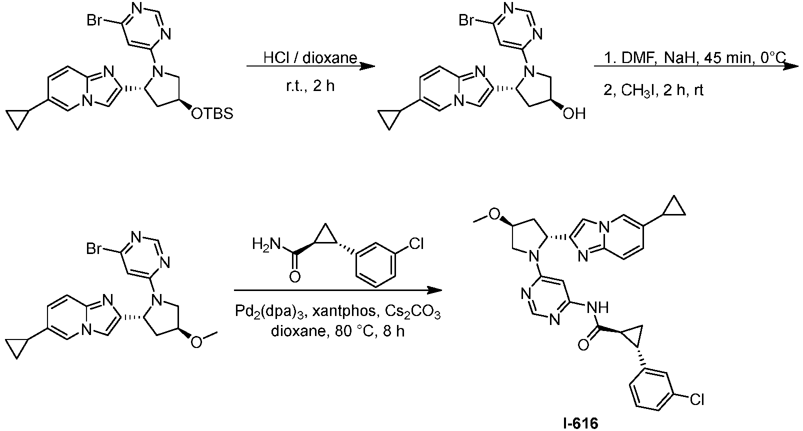

A mixture of **Intermediate 789** table 25 (200 mg, 0.39 mmol) in HCl (4M solution in dioxane, 5 mL) was stirred at room temperature for 1 h. The reaction mixture was concentrated *in vacuo* to give the crude, which was purified by preparative HPLC to give **(3S,5R)-1-(6-bromopyrimidin-4-yl)-5-(6-cyclopropylimidazo[1,2-a]pyridin-2-yl)pyrrolidin-3-ol intermediate** 986 (170 mg, 43%) as a white solid. ESI-MS [M +H]⁺: 400.1.

To mixture of **intermediate 986** (170 mg, 0.425 mmol) in dry DMF (8.0 mL) was added NaH (21 mg, 0.51 mmol, 60% dispersion in mineral oil) at 0 °C. After stirring at 0 °C for 45 min, a solution of MeI (125 mg, 0.85 mmol) in dry THF (1.7 mL) was added and the resulting mixture was stirred at room temperature for another 2 h. The reaction was quenched with saturated aq. NH₄Cl (20 mL) and extracted with EtOAc (3 x 30 mL). The combined organic layers were washed with brine (30 mL), dried over anhydrous Na₂SO₄, and concentrated *in vacuo* to give the crude, which was purified by silica gel column chromatography (eluent: DCM/MeOH = 20/1) to give **2-((2R,4S)-1-(6-bromopyrimidin-4-yl)-4-methoxypyrrolidin-2-yl)-6-cyclopropylimidazo[1,2-a]pyridine intermediat 987** (100 mg, 57%) as a yellow solid. ESI-MS [M +H]⁺: 414.1.

To a mixture of **intermediate 987** (80 mg, 0.19 mmol), **intermediate 119** (38 mg, 0.19 mmol) and Cs₂CO₃ (186 mg, 0.57 mmol) in 1,4-dioxane (5 mL) was added Pd₂(dba)₃ (18 mg, 0.019 mmol) and Xantphos (23 mg, 0.038 mmol). The reaction mixture was stirred at 80 °C for 1 h under N₂. The reaction mixture was filtered through Celite^{®} and the filter cake was washed with DCM/MeOH (10/1, 30 mL). The filtrate was concentrated in vacuo to give the crude, which was purified by preparative TLC (eluent: DCM/MeOH = 20/1) to give (**I-616**) (33 mg, 33%) as a yellow solid. ESI-MS: [M +H]+, 529.2. δ 10.68 - 10.59 (m, 1H), 8.25 (s, 2H), 7.59 (s, 1H), 7.37 (d, J = 9.2 Hz, 1H), 7.32 - 7.28 (m, 1H), 7.26-7.21 (m, 2H), 7.17 - 7.12 (m, 2H), 6.95 (d, J = 9.2 Hz, 1H), 4.96 (s, 1H), 4.19 (s, 1H), 3.84 - 3.79 (m, 2H), 3.26 (s, 3H), 2.40-2.31 (m, 4H), 1.91 - 1.88 (m, 1H), 1.39 - 1.33 (m, 2H), 0.91 - 0.87 (m, 2H), 0.65 - 0.63 (m, 2H).

### Example 617: rac-(1S*,2S*)-N-(5-(((6-cyclopropyl-8-(3-methyl-2,4-dioxoimidazolidin-1-yl)imidazo[1,2-a]pyridin-2-yl)methyl)amino)pyridazin-3-yl)-2-(4-methylpyrimidin-2-yl)cyclopropane-1-carboxamide (I-617)

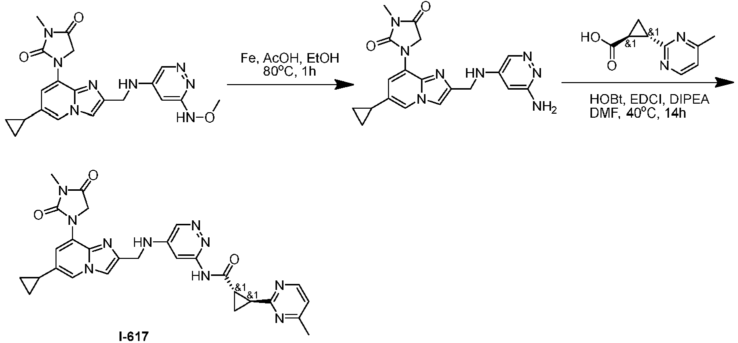

To a mixture of 1-(6-cyclopropyl-2-(((6-(methoxyamino)pyridazin-4-yl)amino) methyl)imidazo[1,2-a]pyridin-8-yl)-3-methylimidazolidine-2,4-dione (800 mg, 1.92 mmol) in EtOH (5 mL) was added Fe (528 mg, 9.6 mmol) and AcOH (4 mL) at room temperature. After stirring at 80 °C for 1 h, the reaction mixture was poured into water (50 mL) and extracted with EtOAc (3 x 50 mL). The combined organics were washed with brine (50 mL) then dried over Na₂SO₄. The reaction mixture was concentrated in vacuo and purified by silica gel column chromatography (eluent: PE/EtOAc = 5/1) to give **1-(2-(((6-aminopyridazin-4-yl)amino)methyl)-6-cyclopropyl imidazo[1,2-a]pyridin-8-yl)-3-methylimidazolidine-2,4-dione intermediate 988** (600 mg, 80%) as a colorless oil. ESI-MS [M +H]+: 393.1

To a stirred solution of **intermediate 988** (100 mg, 0.26 mmol) in DMF (5mL) was added rac-(1S*,2S*)-2-(4-methylpyrimidin-2-yl)cyclopropane-1-carboxylic acid (44 mg, 0.26 mmol), EDCI (146 mg, 0.52 mmol), HOBt (102mg, 0.52 mmol), and DIPEA (164.6 mg, 2.6 mmol). The mixture was stirred at 40 °C for 14 h. The reaction was concentrated in vacuo to give the crude product, which was purified by preparative HPLC to give (**I-617**) (43.4 mg, 30%) as a white solid. ESI-MS [M +H] +: 553.1. δ 10.87 (s, 1H), 8.49 (d, J = 5.1 Hz, 1H), 8.37 (d, J = 2.5 Hz, 1H), 8.23 (s, 1H), 7.73 (s, 1H), 7.52 - 7.50 (m, 2H), 7.35 - 7.31 (m, 1H), 7.17 (d, J = 5.1 Hz, 1H), 4.90 (s, 2H), 4.38 (d, J = 5.5 Hz, 2H), 2.95 (s, 3H), 2.64- 2.57 (m, 2H), 2.38 (s, 3H), 1.95 - 1.88 (m, J = 13.3 Hz, 1H), 1.50 - 1.44 (m, J = 13.1 Hz, 2H), 0.93 - 0.89 (m, 2H), 0.64 -0.60 (m, J = 4.7 Hz, 2H).

### Example 618: rac-(1S*,2S*)-N-(4-(((6-cyclopropyl-8-(3-methyl-2,4-dioxoimidazolidin-1-yl)imidazo[1,2-a]pyridin-2-yl)methyl)amino)-6-(trifluoromethyl)pyridin-2-yl)-2-(4-methylpyrimidin-2-yl)cyclopropane-1-carboxamide (I-618)

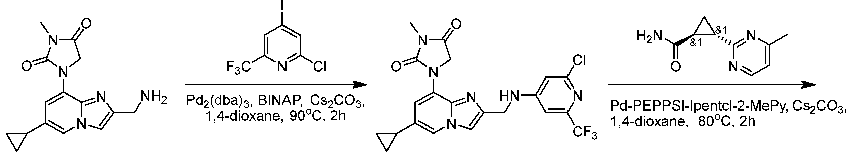

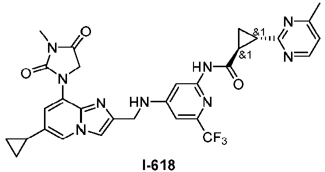

To a mixture of 2-chloro-4-iodo-6-(trifluoromethyl)pyridine (384 mg, 1.25 mmol), **intermediate 365** (299.0 mg, 1.0 mmol) and Cs₂CO₃ (978 mg, 3.0 mmol) in 1,4-dioxane (20 mL) were added Pd₂(dba)₃ (45.8 mg, 0.05 mmol) and BINAP (62.3 mg, 0.1 mmol). The reaction mixture was stirred at 90 °C for 2 h under N₂ and cooled to room temperature. Then the reaction mixture was filtered through Celite^{®} and the filter cake was washed with DCM/MeOH (10/1, 30 mL). The filtrate was concentrated in vacuo to give the crude, which was purified by preparative TLC (eluent: DCM/MeOH = 20/1) to give **1-(2-(((2-chloro-6-(trifluoromethyl)pyridin-4-yl)amino)methyl)-6-cyclopropylimidazo[1,2-a]pyridin-8-yl)-3-methylimidazolidine-2,4-dione intermediate 989** (200 mg, 42%) as a yellow solid. ESI-MS [M +H]⁺: 479.2.

To a mixture of **intermediate 989** (65 mg, 0.14 mmol), rac-(1S*,2S*)-2-(4-methyl pyrimidin-2-yl)cyclopropane-1-carboxamide (24 mg, 0.14 mmol) and Cs₂CO₃ (137 mg, 0.42 mmol) in 1,4-dioxane (6 mL) was added Pd-PEPPSI-IpentCl-2-MePy (12 mg, 0.014 mmol). The reaction mixture was stirred at 80 °C for 2 h under N₂ and cooled to room temperature. The reaction mixture was filtered through Celite^{®} and the filter cake was washed with DCM/MeOH (10/1, 20 mL). The filtrate was concentrated in vacuo to give the crude, which was purified by preparative TLC (eluent: DCM/MeOH = 15/1) to give (**I-618**) (40 mg, 46%) as a yellow solid. ESI-MS [M +H]⁺: 620.2. δ 10.82 (s, 1H), 8.52 (d, J = 5.1 Hz, 1H), 8.27 (s, 1H), 7.76 (s, 1H), 7.69 - 7.65 (m, 2H), 7.36 (d, J = 1.4 Hz, 1H), 7.20 (d, J = 5.1 Hz, 1H), 6.84 (d, J = 1.7 Hz, 1H), 4.92 (s, 2H), 4.44 (d, J = 5.6 Hz, 2H), 2.98 (s, 3H), 2.62 -2.60 (m, 2H), 2.41 (s, 3H), 1.96 - 1.91 (m, 1H), 1.60 - 1.39 (m, 2H), 1.04 - 0.83 (m, 2H), 0.74 - 0.52 (m, 2H).

### Example 619: rac-(1S*,2S*)-N-(5-(((6-cyclopropylimidazo[1,2-a]pyridin-2-yl)methyl)amino)-2-(methylsulfonyl)phenyl)-2-(4-methylpyrimidin-2-yl)cyclopropane-1-carboxamide (I-619)

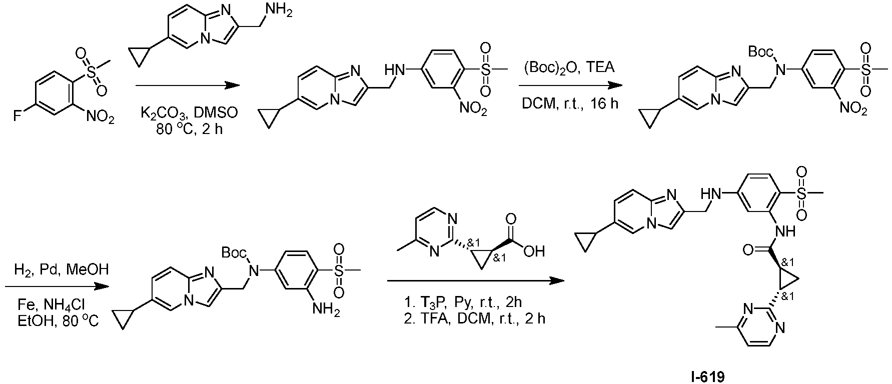

To a mixture of 4-fluoro-1-(methylsulfonyl)-2-nitrobenzene (430 mg, 1.96 mmol) and intermediate 251 (366 mg, 2.16 mmol) in DMSO (10 mL) was added K₂CO₃ (676 mg, 4.9 mmol) and the mixture was stirred at 80 °C for 2 h under N₂. After cooling to 25 °C, water (50 mL) was added and the mixture was extracted with EtOAc (3 x 30 mL). The combined organic layers were washed with brine (60 mL), dried over anhydrous Na₂SO₄, and concentrated *in vacuo* to give **N-((6-cyclopropylimidazo[1,2-a]pyridin-2-yl)methyl)-4-(methylsulfonyl)-3-nitroaniline intermediate 990** (740 mg, crude) as a yellow oil. ESI-MS [M +H]+: 387.1.

To a mixture of **intermediate 990** (740 mg, 1.9 mmol) in DCM (30 mL) were added di*-tert-*butyl dicarbonate (828 mg, 3.8 mmol), DMAP (23 mg, 0.19 mmol), and TEA (576 mg, 5.7 mmol). After stirring at 25 °C for 16 h under N₂, water (50 mL) was added to the mixture and extracted with EtOAc (3 x 30 mL). The combined organic layers were washed with brine (60 mL), dried over anhydrous Na₂SO₄, and concentrated *in vacuo* to give ***tert*-butyl ((6-cyclopropylimidazo[1,2-a]pyridin-2-yl)methyl)(4-(methylsulfonyl)-3-nitrophenyl)carbamate intermediate 991** (900 mg, 60%) as a yellow solid. ESI-MS [M +H]⁺: 487.2.

To a mixture of **intermediate 991** (900 mg, 1.85 mmol) in MeOH (10 mL) and THF (2 mL) was added Pd/C (200 mg) and the mixture was stirred at 25 °C for 2 h under H₂. The reaction mixture was filtered through Celite^{®} and the filter cake was washed with DCM/MeOH (10/1, 20 mL). The filtrate was concentrated *in vacuo* and the resulting residue was mixed with EtOH (20 mL) and NH₄Cl (1.08 g, 20 mmol). The mixture was then heated to 80 °C and Fe powder (311 mg, 5.55 mmol) was added. The mixture was stirred at 80 °C for 20 min under N₂, then filtered through Celite^{®} and the filter cake was washed with DCM/MeOH (10/1, 20 mL). The filtrate was concentrated *in vacuo* to give the crude, which was purified by silica gel column chromatography (eluent: DCM/MeOH=50/1) to give ***tert*-butyl (3-amino-4-(methylsulfonyl)phenyl)((6-cyclo propylimidazo[1,2-a]pyridin-2-yl)methyl)carbamate intermediate 992** (450 mg, 53%) as a yellow solid. ESI-MS [M +H]+: 457.2.

To a mixture of **intermediate 992** (200 mg, 0.44 mmol) and rac-(1S*,2S*)-2-(4-methylpyrimidin-2-yl)cyclopropane-1-carboxylic acid (78 mg, 0.44 mmol) in pyridine (5 mL) was added T₃P (2.1 g, 6.6 mmol) dropwise at 25 °C under N₂ for 3 h under N₂. Water (20 mL) was added, and the mixture was extracted with EtOAc (3 x 20 mL). The combined organic layers were washed with brine (10 mL), dried over anhydrous Na₂SO₄ and concentrated to give the crude product, which was purified by preparative TLC (eluent: DCM/MeOH=15/1) to give ***rac*-tert-butyl ((6-cyclopropylimidazo[1,2-a]pyridin-2-yl)methyl)(3-((1S*,2S*)-2-(4-methylpyrimidin-2-yl) cyclopropane-1-carboxamido)-4-(methylsulfonyl)phenyl)carbamate intermediate 993** (200 mg, 74%) as a yellow solid. ESI-MS [M +H]+: 617.3.

To a solution of give **intermediate 993** (50 mg, 0.081 mmol) in DCM (3 mL) was added TFA (1 mL). The mixture was stirred at room temperature for 2 h. The reaction mixture was concentrated and neutralized by adding NH3 (5.0 mL, 4 N in MeOH, 20 mmol) , then concentrated to give the crude product, which was purified by Prep-TLC (eluent: DCM/MeOH=10/1) to give (**I-619**) (25 mg, 59.7%) as a yellow solid. ESI-MS [M +H]+: 517.2. δ 9.84 (s, 1H), 8.54 (d, J = 5.1 Hz, 1H), 8.32 (s, 1H), 7.67 (s, 1H), 7.52 (d, J = 8.9 Hz, 1H), 7.39 (d, J = 9.3 Hz, 1H), 7.36 - 7.29 (m, 2H), 7.22 (d, J = 5.1 Hz, 1H), 6.97 (dd, J = 9.3, 1.6 Hz, 1H), 6.55 (dd, J = 8.8, 2.1 Hz, 1H), 4.39 (d, J = 5.7 Hz, 2H), 3.09 (s, 3H), 2.65 - 2.57 (m, 1H), 2.43 (s, 3H), 2.34-2.33 (m, 1H), 1.95-1.88 (m, 1H), 1.56 - 1.50 (m, 2H), 0.94 - 0.88 (m, 2H), 0.69 - 0.64 (m, 2H).

### Example 620: rac-(1S*,2S*)-N-(6-((6-cyclopropyl-8-(3-methyl-2,4-dioxoimidazolidin-1-yl)imidazo[1,2-a]pyridin-2-yl)methyl)pyrimidin-4-yl)-2-(4-methylpyrimidin-2-yl)cyclopropane-1-carboxamide (I-620)

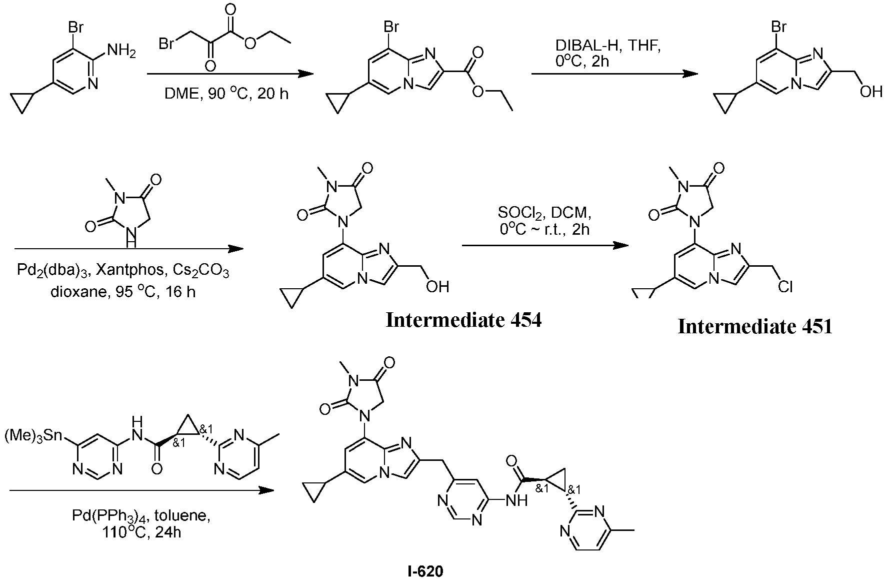

A mixture of **Intermediate 834** (200 mg, 0.69 mmol), 1,1,1,2,2,2-hexamethyldistannane (227 mg, 104 mmol), and Pd(PPh₃)₄ (81 mg, 0.07 mmol) in toluene (10 mL) was stirred at 110 °C for 16h. The reaction mixture was cooled to room temperature and filtered. The filtrate was concentrated *in vacuo* to ***rac*-(*1S**,*2S**)-2-(4-methylpyrimidin-2-yl)-N-(6-(trimethylstannyl)pyrimidin-4-yl)cyclopropane-1-carboxamide intermediate 899** (350 mg crude), which was used into next step without any purification. ESI-MS [M +H]+:402.0

A mixture of **intermediate 899** (150 mg crude), **intermediate 451** (114 mg, 0.36 mmol), and Pd (PPh₃)₄ (46 mg, 0.04 mmol) in toluene (10 mL) was stirred at 110 °C for 24 h. The reaction mixture was cooled to room temperature and filtered. The filtrate was concentrated in vacuo to provide crude product, which was purified by preparative HPLC to give **(I-620)** ( 17 mg, 9 %). ESI-MS [M +H]+:538.1, δ 11.20 (s, 1H), 8.76 (d, J = 1.1 Hz, 1H), 8.52 (d, J = 5.1 Hz, 1H), 8.26 (d, J = 1.1 Hz, 1H), 8.16 (s, 1H), 7.75 (s, 1H), 7.34 (d, J = 1.5 Hz, 1H), 7.21 (d, J = 5.1 Hz, 1H), 4.88 (d, J = 1.4 Hz, 2H), 4.14 (s, 2H), 2.97 (s, 3H), 2.64 (dd, J = 6.9, 4.6 Hz, 1H), 2.58 - 2.55 (m, 1H), 2.41 (s, 3H), 1.96 (ddd, J = 13.4, 8.3, 5.0 Hz, 1H), 1.58 - 1.50 (m, 2H), 0.98 - 0.92 (m, 2H), 0.66 (dt, J = 6.4, 4.5 Hz, 2H)

### Example 621: rac-(1S*,2S*)-N-(4-(((6-cyclopropyl-8-(3-methyl-2,4-dioxoimidazolidin-1-yl) imidazo[1,2-a]pyridin-2-yl)methyl)amino)-5-(methoxymethyl)pyrimidin-2-yl)-2-(4-methyl pyrimidin-2-yl)cyclopropane-1-carboxamide (I-621)

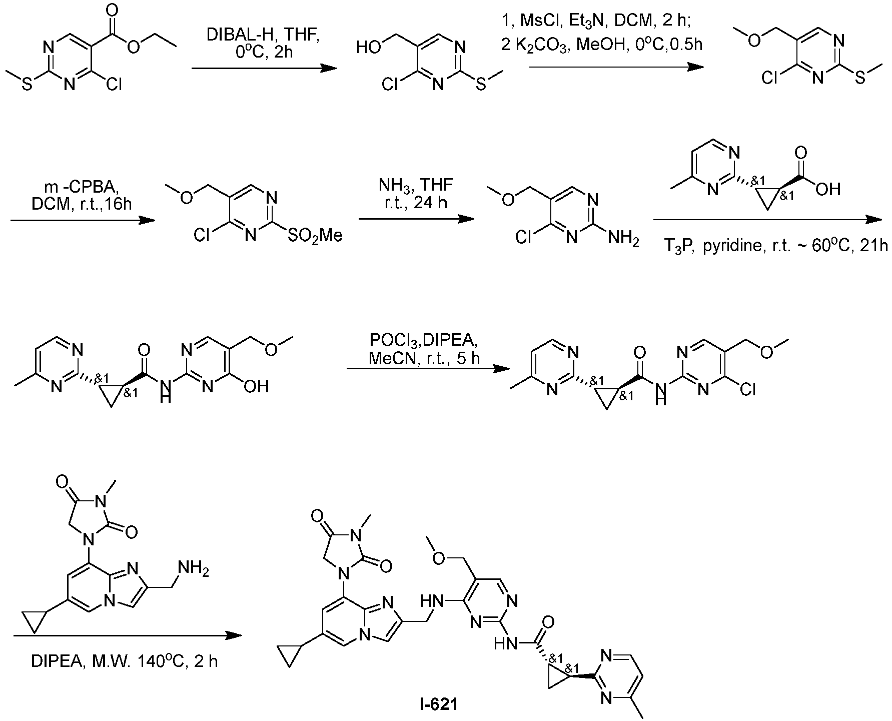

To a solution of ethyl 4-chloro-2-(methylthio)pyrimidine-5-carboxylate (3 g, 13 mmol) in THF (30 mL) was added DIBAL-H (39 mL, 39 mmol) at 0 °C, then the reaction mixture was stirred at 0 °C for 2 h under N₂. The reaction was quenched with water (30 mL) and extracted with EtOAc (3 x 30 mL). The combined organic layers were washed with brine (30 mL), dried over anhydrous Na₂SO₄, and concentrated *in vacuo* to give the crude product, which was purified by silica gel column chromatography (eluent:PE/ EtOAc =1/1) to give **(4-chloro-2-(methylthio)pyrimidin-5-yl)methanol intermediate 996** (1.5 g, 61%) as a white solid. ESI-MS [M +H]+: 191.1.

To a solution of **intermediate 996** (500 mg, 2.63 mmol) in DCM (15 mL) was added Et₃N (797 mg, 7.89 mmol) and methanesulfonyl chloride (449 mg, 3.94 mmol) at 0 °C, then the solution was stirred at room temperature for 2 h. Water (50 mL) was added and the mixture was extracted with EtOAc (3 x 30 mL). The combined organic layers were washed with brine (60 mL), dried over anhydrous Na₂SO₄, and concentrated *in vacuo* to give the crude **(4-chloro-2-(methylthio) pyrimidin-5-yl)methyl methanesulfonate** (500 mg, crude), which was used in the next step without purification (500 mg, 0.26 mmol) in MeOH (10 mL). K₂CO₃ (258 mg, 1.87 mmol) was added at 0 °C, then the solution was stirred at 0 °C for 30 min. Water (50 mL) was added and the mixture was extracted with EtOAc (3 x 30 mL). The combined organic layers were washed with brine (60 mL), dried over anhydrous Na₂SO₄, and concentrated *in vacuo* to give the crude, which was purified by column chromatography (eluent:PE/ EtOAc =4/1) to give **4-chloro-5-(methoxy methyl)-2-(methylthio)pyrimidine intermediate 997** (300 mg, 56% ) as a colorless oil. ESI-MS [M +H]+: 205.1.

To a solution of **intermediate 997** (400 mg, 1.96 mmol) in DCM (20 mL) was added m-CPBA (1.01 g, 5.88 mmol) and the reaction mixture was stirred at room temperature for 16 h. The reaction was quenched with saturated aq. Na₂S₂O₃ (30 mL) and saturated aq. NaHCO₃ (30 mL), then extracted with DCM (3 x 20 ml). The combined organic layers were washed with brine (30 mL), dried over anhydrous Na₂SO₄, and concentrated *in vacuo* to give the crude, which was purified by column chromatography (eluent:PE: EtOAc =1:1) to give **4-chloro-5-(methoxymethyl)-2-(methyl sulfonyl)pyrimidine intermediate 998** (380 mg, 82%) as a white solid. ESI-MS [M +H]+: 237.1.

A mixture of **intermediate 998** (320 mg, 1.35 mmol) in ammonia solution (2M in THF, 40 mL) was stirred at room temperature for 24 h. The mixture was concentrated *in vacuo* to give the crude, which was purified by column chromatography (eluent: EtOAc /PE=8/1) to give **4-chloro-5-(methoxymethyl)pyrimidin-2-amine intermediate 999** (170 mg, 73%) as a white solid. ESI-MS [M +H]+: 174.1.

To a solution of **intermediate 999** (50 mg, 0.289 mmol) in pyridine (10 mL) was added *rac-*(1S*,2S*)-2-(4-methylpyrimidin-2-yl)cyclopropane-1-carboxylic acid (51 mg, 0.289 mmol) and T3P (1.84 g, 2.89 mmol, 50% in EtOAc). After stirring at 60 °C for 21 h under N₂, water (10 mL) was added and the mixture was extracted with EtOAc (3 x 20 mL). The combined organic layers were washed with brine (30 mL), dried over anhydrous Na₂SO₄, and concentrated *in vacuo* to give the crude, which was purified by column chromatography (eluent: DCM/MeOH=20/1) to give *rac-***(1S*,2S*)-N-(4-hydroxy-5-(methoxymethyl)pyrimidin-2-yl)-2-(4-methylpyrimidin-2-yl)cyclopropane-1-carboxamide intermediate 1000** (50 mg, 55%) as a white solid. ESI-MS [M +H]+: 316.1

To a solution of **intermediate 1000** (50 mg, 0.158 mmol) in MeCN (5 mL) was added slowly POCl₃ (73 mg, 0.476 mmol) at 0 °C, followed by DIPEA (62 mg, 0.476 mmol). After stirring at room temperature for 5 h, water (10 mL) was added and the mixture was extracted with EtOAc (3 x 20 mL). The combined organic layers were washed with brine (30 mL), dried over anhydrous Na₂SO₄, and concentrated *in vacuo* to give the crude, which was purified by preparative TLC (eluent: DCM/MeOH=20/1) to give ***rac*-(1S*,2S*)-N-(4-chloro-5-(methoxymethyl)pyrimidin-2-yl)-2-(4-methylpyrimidin-2-yl)cyclopropane-1-carboxamide intermediate 1001** (20 mg, 38%) as a white solid. ESI-MS [M +H]+: 334.1

To a solution of intermediate 1001 (10 mg, 0.03 mmol) in i-PrOH (1 mL) were added **intermediate 365** (9 mg, 0.03 mmol) and DIPEA (12 mg, 0.09 mmol). After degassing with N₂ for 1 min, the reaction was irradiated in a microwave reactor at 140 °C for 2 h. The mixture was then concentrated in vacuo to get the crude, which was purified by preparative TLC (eluent: DCM/MeOH=10/1) to give the product **(I-621)** (10 mg. 56%) as a white solid. ESI-MS [M +H]+: 597.3. δ 10.32 (s, 1H), 8.46 (d, J = 5.1 Hz, 1H), 8.23 (s, 1H), 7.91 (s, 1H), 7.74 (s, 1H), 7.40 - 7.23 (m, 2H), 7.13 (d, J = 5.0 Hz, 1H), 4.90 (s, 2H), 4.61 (d, J = 5.3 Hz, 2H), 4.29 (s, 2H), 3.26 (s, 3H), 2.98 (s, 3H), 2.59 - 2.52 (m, 2H), 2.37 (s, 3H), 1.96-1.91 (m, 1H), 1.54-1.50 (m, 2H), 1.03 - 0.87 (m, 2H), 0.70 - 0.57 (m, 2H).

### Examples 622-623

### rac-(1S*,2S*)-N-(4-chloro-6-(((6-cyclopropyl-8-(3-methyl-2,4-dioxoimidazolidin-1-yl)imidazo[1,2-a]pyridin-2-yl)methyl)amino)-1,3,5-triazin-2-yl)-2-(4-methylpyrimidin-2-yl)cyclo propane-1-carboxamide (I-622), and

### rac-(1S*,2S*)-N-(4-(((6-cyclopropyl-8-(3-methyl-2,4-dioxo imidazolidin-1-yl)imidazo[1,2-a]pyridin-2-yl)methyl)amino)-1,3,5-triazin-2-yl)-2-(4-methylpyrimidin-2-yl)cyclopropane-1-carboxamide (I-623)

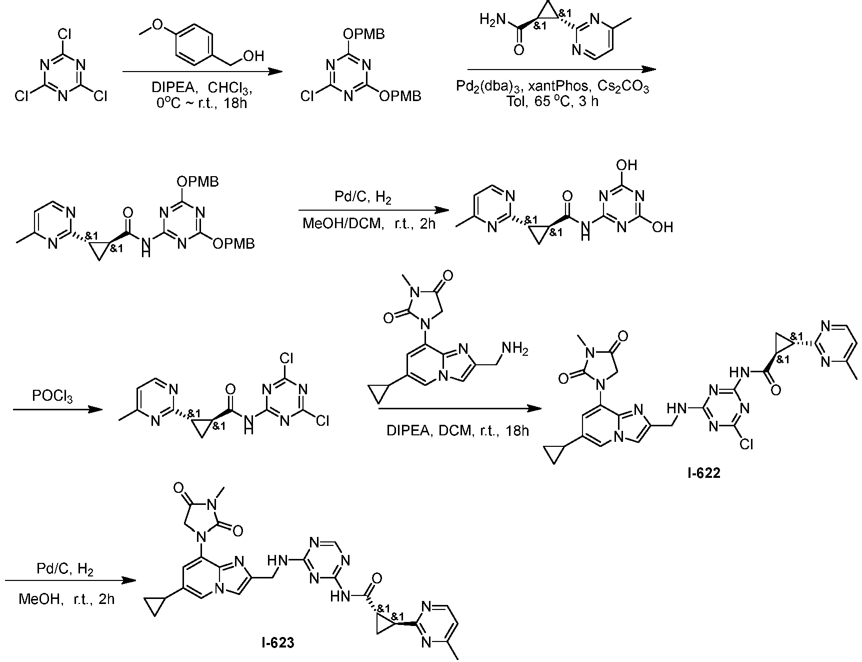

To a mixture of 2,4,6-trichloro-1,3,5-triazine (2 g, 10.9 mol) and (4-methoxyphenyl)methanol (3.0 g, 21.8 mol) in CHCl₃ (50 mL) was added DIPEA (4.2 g, 32.7 mol) at 0 °C. After stirring at room temperature for 18 h, the reaction was quenched with saturated aq. NaHCO₃ (150 mL) and extracted with EtOAc (3 x 80 mL). The combined organic layers were washed with brine (80 mL), dried over Na₂SO₄ and concentrated *in vacuo* to get the crude, which was purified by column chromatography (eluent: EtOAc/PE=1/10) to give **2-chloro-4,6-bis((4-methoxybenzyl)oxy)-1,3,5-triazine intermediate 1002** (1.6 g, 38%) as a white solid. ESI-MS [M +H] +: 388.1.

To a mixture of **intermediate 1002** (400 mg, 1.03 mmol), rac-(1S*,2S*)-2-(4-methylpyrimidin-2-yl)cyclopropane-1-carboxamide (182 mg, 1.03 mmol), and Cs₂CO₃ (674 mg, 2.07 mmol) in toluene (20 mL) were added Pd₂(dba)₃ (146 mg, 0.16 mmol) and XantPhos (179mg, 0.31 mmol). The mixture was degassed with N₂ for three times and stirred at 6 5°C in for 3 h. The mixture was diluted with H2O (100 mL) and extracted with EtOAc (3 x 50 mL). The combined organic layers were washed with brine (50 mL), dried over Na₂SO₄ and concentrated in vacuo. The residue was purified by silica gel column chromatography (eluent: MeOH/DCM=1/20) to give **rac-(1S*,2S*)-N-(4,6-bis ((4-methoxybenzyl)oxy)-1,3,5-triazin-2-yl)-2-(4-methylpyrimidin-2-yl)cyclopropane-1-carboxamide intermediate 1003** (180 mg, 33%) as a white solid. ESI-MS [M +H] +: 529.1, δ 11.16 (s, 1H), 8.49 (d, J = 5.1 Hz, 1H), 7.44-7.33 (m, 4H), 7.16 (d, J = 5.1 Hz, 1H), 6.97-6.89 (m, 4H), 5.34 - 5.21 (m, 4H), 3.03 (s, 1H), 2.62-2.55 (m, 1H), 2.36 (s, 3H), 1.65 - 1.55 (m, 2H).

A mixture of **intermediate 1003** (280 mg, 0.53 mmol) and Pd/C (100 mg) in MeOH/DCM (30mL/30 mL) was degassed and stirred under H₂ at room temperature for 3 h. The mixture was filtered and concentrated *in vacuo* to give ***rac*-(1S*,2S*)-N-(4,6-dihydroxy-1,3,5-triazin-2-yl)-2-(4-methylpyrimidin-2-yl)cyclopropane-1-carboxamide intermediate 1004** (140 mg, crude) as a white solid which was used in the next step directly. ESI-MS [M +H] +: 289.1

A mixture of **intermediate 1004** (140 mg, 0.48 mmol) and PhNEt₂ (107 mg, 0.72 mmol) in POCl₃ (5 mL) was degassed with N₂ and stirred at 120 °C for 3 h. The mixture was cooled to room temperature and concentrated *in vacuo* to give ***rac*-(1S*,2S*)-N-(4,6-dichloro-1,3,5-triazin-2-yl)-2-(4-methylpyrimidin-2-yl)cyclopropane-1-carboxamide intermediate 1005** (250 mg, crude) as a brown oil which was used in the next step. ESI-MS [M +H] +: 325.1

A mixture of **intermediate 1005** (250 mg, 0.48 mmol), **intermediate 365** (144 mg, 0.48 mmol), and DIPEA (186 mg, 1.45 mmol) in DCM (10 mL) was stirred at room temperature for 18 h. The reaction was quenched with saturated aq. NaHCO₃ (50 mL) and extracted with EtOAc (3 x 50 mL). The combined organic layers were washed with brine (50 mL), dried over Na₂SO₄ and concentrated in vacuo. The residue was purified by preparative TLC (eluent: MeOH/DCM = 1/15) to afford **(I-622)** (75 mg, 27 %) as a white solid. ESI-MS [M+H]+: 588.2. δ 11.06-11.00 (m, , 1H), 8.94-8.85 (m, 1H), 8.54-8.40 (m, 1H), 8.25-8.20 (m, 1H), 7.77-7.71 (m, 1H), 7.35-7.32 (m, 1H), 7.21-7.08 (m, 1H), 4.88 (s, 2H), 4.58-4.50 (m, 2H), 2.97 (s, 3H), 2.61 - 2.52 (m, 2H), 2.42-2.33 (s, 3H), 1.97-1.91 (m, 1H), 1.59-1.50 (m, 2H), 0.99 - 0.90 (m, 2H), 0.68-0.61 (m, 2H).

. A mixture of rac-(1S*,2S*)-N-(4-chloro-6-(((6-cyclopropyl-8-(3-methyl-2,4-dioxoimidazolidin-1-yl)imidazo[1,2-a]pyridin-2-yl)methyl)amino)-1,3,5-triazin-2-yl)-2-(4-methylpyrimidin-2-yl)cyclopropane-1-carboxamide (45 mg, 0.077 mmol), and Pd/C (25 mg) in MeOH/DCM (5 mL/5 mL) was degassed and stirred under H₂ at RT for 2 h. The mixture was filtered, concentrated in vacuo to get the crude, & purified by preparative TLC (eluent: MeOH/DCM = 1/15) to afford **(I-623)** (20 mg, 48%) as a white solid. ESI-MS [M +H]+: 554.2. δ 10.75-10.66 (m, 1H), 8.54 - 8.29 (m,3H), 8.23 (s,1H), 7.76-7.66 (m,1H), 7.33 (s,1H), 7.22-7.08(m,1H), 4.92-4.86 (m,2H), 4.60-4.47 (m,2H), 2.98-2.96 (m,3H), 2.59-2.54 (m,2H), 2.42-2.33 (m,3H), 1.98 - 1.89 (m,1H), 1.58-1.48 (m,2H), 0.97 - 0.89 (m,2H), 0.68 - 0.60 (m, 2H).

### Example 624: rac-(1S*,2S*)-N-(7-(((6-cyclopropyl-8-(3-methyl-2,4-dioxoimidazolidin-1-yl) imidazo[1,2-a]pyridin-2-yl)methyl)amino)-[1,2,4]triazolo[1,5-a]pyrimidin-5-yl)-2-(4-methyl pyrimidin-2-yl)cyclopropane-1-carboxamide (I-624)

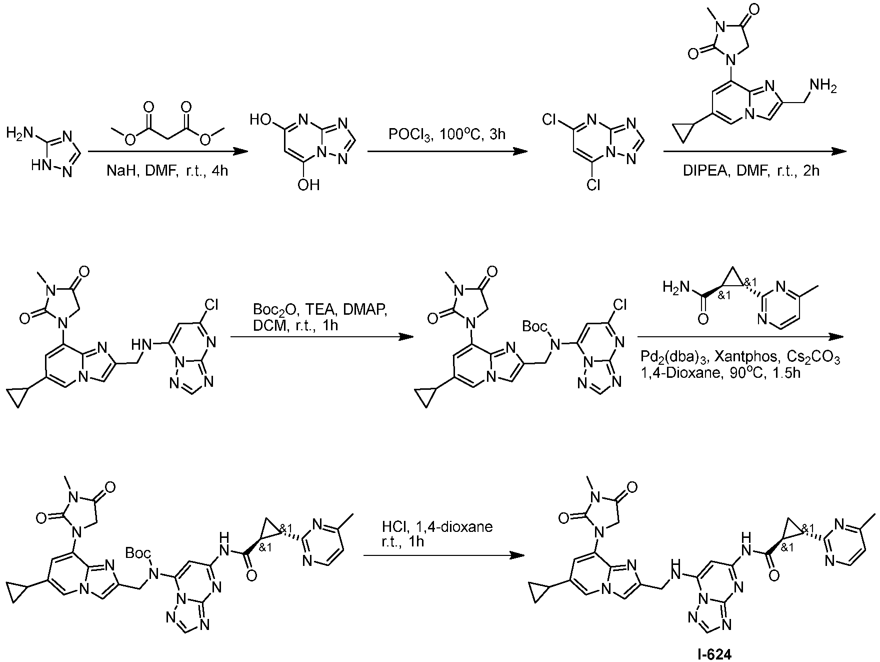

A mixture of [1,2,4]triazolo[1,5-a] pyrimidine-5,7-diol (5 g, 59.5 mmol), dimethyl malonate (11.5 g, 71.4 mmol), and NaH (2.8g, 119mmol, 60% dispersion in mineral oil) in DMF (25 mL) was stirred at RT for 4 h. The reaction was quenched with H2O (100 mL) and extracted with EtOAc (3 x 100 mL). The combined organic layers were washed with brine (60 mL), dried over Na₂SO₄, filtered and concentrated *in vacuo* to give the crude, purified by silica gel column chromatography (DCM/MeOH = 10/1) to give **[1,2,4] triazolo[1,5-a]pyrimidine-5,7-diol intermediate 1006** (2.6 g, 28 %) as a white solid. ESI-MS [M +H]+: 153.2.

A mixture of **intermediate 1006** (1.5 g, 9.9 mmol) and POCl₃ (15 mL) was stirred at 100 °C for 3 h. The reaction mixture was cooled to RT and concentrated *in vacuo* to give the crude, which was washed with saturated aq. NaHCO₃ (60 mL) and extracted with EtOAc (4 x 60 mL). The combined organic layers were washed with brine (60 mL), dried over Na₂SO₄ then concentrated *in vacuo* to give the crude. The residue was purified by silica gel column chromatography (PE/EtOAc = 5/1) to give **5,7-dichloro-[1,2,4]triazolo[1,5-a]pyrimidine intermediate 1007** (400 mg, 22 %) as a white solid. ESI-MS [M +H]+: 188.9.

A mixture of **intermediate 1007** (400 mg, 2.1 mmol), 1-(2-(aminomethyl)-6-cyclo propylimidazo [1,2-a]pyridin-8-yl)-3-methylimidazolidine-2,4-dione hydrochloride ( 703 mg, 2.1 mmol), DIPEA (774 mg, 6 mmol ), and DMF (20 mL) was stirred at room temperature for 2 h. The reaction was washed with water (30 mL) and extracted with EtOAc (3 x 50 mL). The combined organic layers were washed with brine (40 mL), dried over Na₂SO₄, filtered, and concentrated in vacuo to give the crude, which was purified by preparative TLC (DCM/MeOH = 30/1) to give **1-(2-(((5-chloro-[1,2,4]triazolo[1,5-a]pyrimidin-7-yl)amino)methyl)-6-cyclopropylimidazo[1,2-a]pyridin-8-yl)-3-methylimidazolidine-2,4-dione intermediate 1008** (300 mg, 32%) as a white solid. ESI-MS [M +H]+: 452.2.

A mixture of **intermediate 1008** (300 mg, 0.66 mmol), di-tert-butyl dicarbonate (216 mg, 0.99 mmol), TEA (200 mg, 1.98 mmol), and DMAP (80.5 mg, 0.66 mmol) in DCM (20 ml) was stirred at room temperature for 1 h. The reaction was washed with water (50 mL) and extracted with DCM (3 x 40 mL). The combined organic layers were washed with brine (40 mL), dried over Na₂SO₄, filtered, and concentrated in vacuo to give the crude, which was purified by silica gel column chromatography (DCM/MeOH = 20/1) to give **tert-butyl (5-chloro-[1,2,4]triazolo [1,5-a]pyrimidin-7-yl)((6-cyclopropyl-8-(3-methyl-2,4-dioxoimidazolidin-1-yl)imidazo[1,2-a] pyridin-2-yl)methyl)carbamate intermediate 1009** (300 mg, 82%) as a yellow solid. ESI-MS [M +H]+: 552.2.

To a mixture **intermediate 1009** (140 mg,0.25 mmol), rac-(1S*,2S*)-2-(4-methyl pyrimidin-2-yl) cyclopropane-1-carboxamide (49 mg, 0.26 mmol), & Cs₂CO₃ (234.6 mg, 0.72mmol) in 1,4-dioxane (5 mL) were added Pd₂(dba)₃ (33 mg, 0.038 mmol) and Xantphos (27.8 mg, 0.048 mmol). After stirring at 90 °C for 1.5 h, the reaction was washed with H2O (30mL) and extracted with EtOAc (3 x 40 mL). The combined organic layers were washed with brine (30mL), dried over Na₂SO₄, filtered, concentrated in vacuo to give the crude, & purified by preparative TLC (DCM/MeOH = 20/1) to give **rac-tert-butyl((6-cyclopropyl-8-(3-methyl-2,4-dioxo imidazolidin-1-yl)imi-dazo[1,2-a]pyridin-2-yl)methyl)(5-((1S*,2S*)-2-(4-methylpyrimidin-2-yl) cyclopropane-1-carbox amido)-[1,2,4] triazolo[1,5-a]pyrimidin-7-yl)carbamate intermediate 1010** (60 mg, 36 %) as a yellow solid. ESI-MS [M +H]+: 693.2.

A mixture of **intermediate 1010** (60mg, 0.086 mmol) in HCl (3mL, 4M solution in 1,4-dioxane) was stirred at RT for 1h. The reaction was concentrated in vacuo to give the crude, which was purified by preparative TLC (DCM/MeOH = 20/1) to give **(I-624)** (30 mg, 59%) as a white solid. ESI-MS [M +H]⁺:593.2. δ 11.04 (s, 1H), 8.80 (t, J = 6.1 Hz, 1H), 8.52 (d, J = 5.1 Hz, 1H), 8.34 (s, 1H), 8.23 (s, 1H), 7.80 (s, 1H), 7.46 (d, J = 21.5 Hz, 1H), 7.38 (s, 1H), 7.20 (d, J = 5.1 Hz, 1H), 4.98 (d, J = 4.4 Hz, 2H), 4.65 (d, J = 5.9 Hz, 2H), 2.99 (s, 3H), 2.69-2.54 (m, 2H), 2.41 (s, 3H), 1.98-1.90 (m, 1H), 1.55-1.52 (m, 2H), 0.99 - 0.85 (m, 2H), 0.70 - 0.58 (m, 2H).

### Example 625: rac-(1S*,2S*)-N-(6-(2-(6-cyclopropyl-8-(3-methyl-2,4-dioxoimidazolidin-1-yl) imidazo[1,2-a]pyridin-2-yl)ethyl)pyrimidin-4-yl)-2-(4-methylpyrimidin-2-yl)cyclopropane-1-carboxamide (I-625)

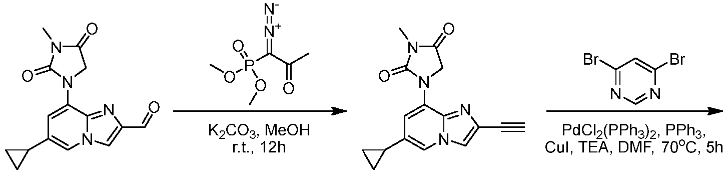

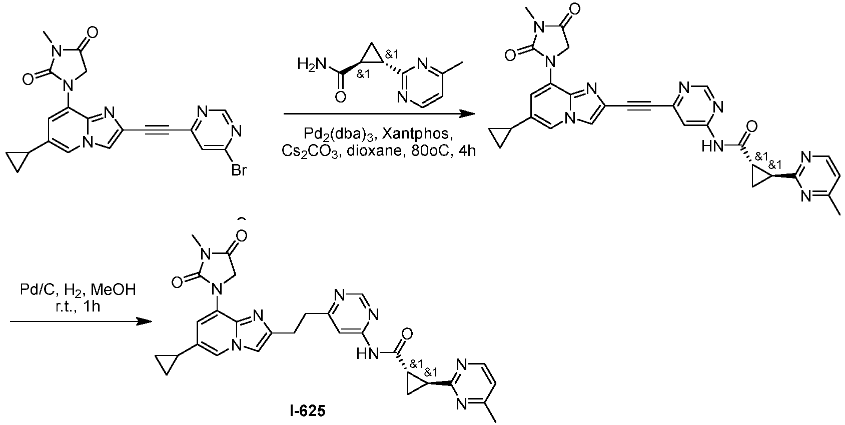

To a solution of **intermediate 1053** (700 mg, 2.35 mmol) in MeOH (30 mL) were added dimethyl (1-diazo-2-oxopropyl)phosphonate ( 2.25 g, 11.75 mmol) and K₂CO₃ (1.62 g, 11.75 mmol). The reaction mixture was stirred at room temperature for 12 h. The reaction was diluted with water (60 mL) and extracted with EtOAc (3 x 50 mL). The combined organic layers were washed with brine (40 mL), dried over Na₂SO₄, filtered and concentrated *in vacuo* to give the crude, which was purified by silica gel column chromatography (eluent: DCM/ MeOH = 30/1) to give **1-(6-cyclopropyl-2-ethynylimidazo[1,2-a]pyridin-8-yl)-3-methylimidazolidine-2,4-dione intermediate 1011** (250 mg, 36%) as a light yellow oil. ESI-MS [M +H]+: 295.2

To a solution of **intermediate 1011** (250 mg, 0.85 mmol ), 4,6-dibromopyrimidine (300 mg, 1.28 mmol ) PdCl₂(PPh₃)₂ (60 mg, 0.085mmol), CuI (16 mg, 0.085mmol), and TEA (1 mL) in DMF (20 mL) was stirred at 70 °C for 5 h under N₂. After cooling to room temperature, the mixture washed with water (50 mL) and extracted with EtOAc (3 x 30 mL). The combined organic layers were washed with brine (40 mL), dried over Na₂SO₄, filtered, and concentrated *in vacuo* to give the crude, which was purified by silica gel column chromatography (eluent: DCM/ MeOH = 20/1) to give the **1-(2-((6-bromopyrimidin-4-yl)ethynyl)-6-cyclopropylimidazo[1,2-a]pyridin-8-yl)-3-methylimidazolidine-2,4-dione intermediate 1012** (100 mg, 17%) as a yellow solid. ESI-MS [M +H]+: 451.2.

A mixture of **intermediate 1012** (100 mg, 0.22 mmol), rac-(1S*,2S*)-2-(4-methyl pyrimidin-2-yl)cyclopropane-1-carboxamide (58 mg, 0.33 mmol), Pd₂(dba)₃ (20 mg, 0.022 mmol), Xantphos (13 mg, 0.022 mmol), and Cs₂CO₃ (215 mg, 0.66 mmol) in 1,4-dioxane (10 mL) was stirred at 80 °C for 4 h under nitrogen. The reaction was washed with water (30 mL) and extracted with EtOAc (3 x 30 mL). The combined organic layers were washed with brine (30 mL), dried over Na₂SO₄ then concentrated in vacuo to give the crude, which was purified by silica gel column chromatography (eluent: DCM/ MeOH = 20/1) to give **rac-(1S*,2S*)-N-(6-((6-cyclo propyl-8-(3-methyl-2,4-dioxoimidazolidin-1-yl)imidazo[1,2-a]pyridin-2-yl)ethynyl)pyrimidin-4-yl)-2-(4-methylpyrimidin-2-yl)cyclopropane-1-carboxamide intermediate 1013** (40 mg, 33%) as a light yellow solid. ESI-MS [M +H]+: 548.2.

To a solution of **intermediate 1013** (40 mg, 0.073 mmol ) and Pd/C (10 mg) in MeOH (15 mL) was stirred at room temperature for 1 h under H₂. The reaction was filtered and concentrated in vacuo to give the crude, which was purified by preparative TLC (DCM/MeOH= 15/1) to give **(I-625)** (10 mg, 25%) as a white solid. ESI-MS [M +H]+: 555.2. ¹H NMR (400 MHz, MeOD) δ 8.68 (s, 1H), 8.47 (d, J = 5.1 Hz, 1H), 8.12 (s, 1H), 7.95 (s, 1H), 7.53 (s, 1H), 7.25 (s, 1H), 7.17 (d, J = 5.1 Hz, 1H), 4.64 (s, 2H), 3.16 (s, 4H), 3.10 (s, 3H), 2.68-2.62 (m, 1H), 2.56 - 2.50 (m, 1H), 2.47 (s, 3H), 1.98-1.92 (m, 1H), 1.70 - 1.56 (m, 2H), 1.00 - 0.93 (m, 2H), 0.75-0.71 (m, 2H).

### Example 626: rac-(1S*,2S*)-N-(3-((6-cyclopropyl-8-(3-methyl-2,4-dioxoimidazolidin-1-yl) imidazo[1,2-a]pyridin-2-yl)methyl)-2-oxo-2,3-dihydrooxazolo[4,5-d]pyrimidin-5-yl)-2-(4-methylpyrimidin-2-yl)cyclopropane-1-carboxamide (I-626)

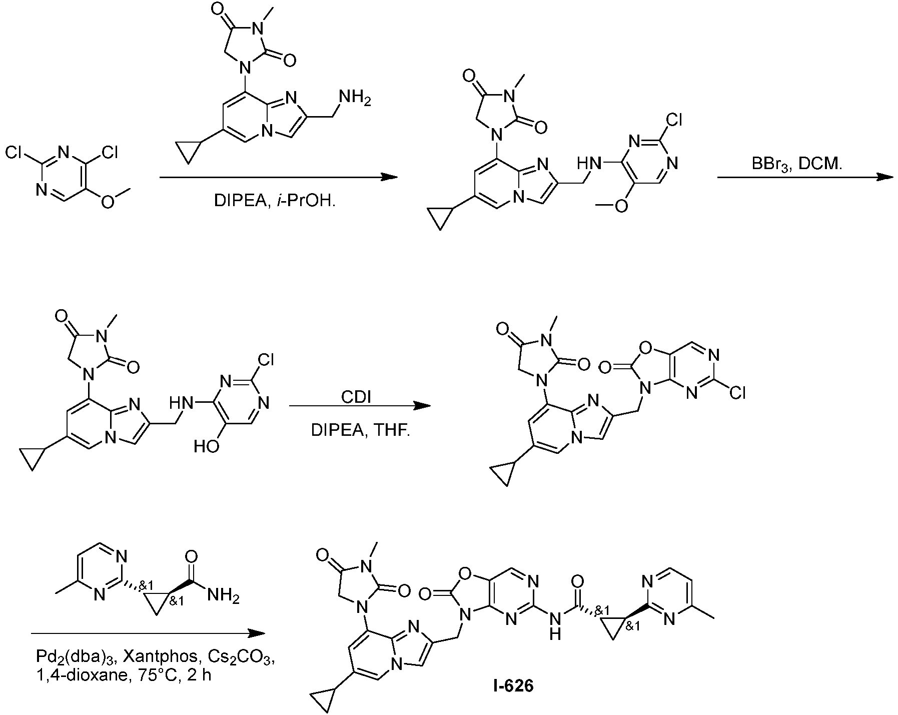

A mixture of 2,4-dichloro-5-methoxypyrimidine (300 mg, 1.0 mmol), **intermediate 365** (356 mg, 2.0 mmol), and DIPEA (645 mg, 5.0 mmol) in i-PrOH (10 mL) was stirred at 60 °C for 16 h under N₂. The mixture was concentrated in vacuo to give the crude, which was purified by Prep-TLC (eluent: DCM/MeOH =10/1) to give **1-(2-(((2-chloro-5-methoxypyrimidin-4-yl)amino) methyl)-6-cyclopropylimidazo[1,2-a]pyridin-8-yl)-3-methylimidazolidine-2,4-dione intermediate 994** as a white solid (260 mg, 59%). ESI-MS [M +H]⁺: 442.0.

To a mixture of **intermediate 994** (260 mg, 0.59 mmol) in DCM (10 mL) was added BBr₃ (731 mg, 2.95 mmol) at 0 °C under N₂. After stirring for 36 h at room temperature under N₂, the reaction was quenched with saturated aq. NaHCO₃ (15 mL) and extracted with EtOAc (3 x 10 mL). The combined organic layers were washed with brine (20 mL), dried over anhydrous Na₂SO₄, and concentrated in vacuo to give the crude product, which was purified by Prep- TLC (eluent: DCM/MeOH=10/1) to give **1-(2-(((2-chloro-5-hydroxypyrimidin-4-yl)amino)methyl)-6-cyclopropylimidazo[1,2-a]pyridin-8-yl)-3-methylimidazolidine-2,4-dione intermediate 1014** (110 mg, 44%).as a white solid. ESI-MS [M +H]⁺: 428.1.

A mixture of **intermediate 1014** (80 mg, 0.19 mmol) and CDI (152 mg, 0.94 mmol) in THF (5 mL) was stirred for 2 h at 60 °C under N₂. The mixture was concentrated in vacuo to give the crude product, which was purified by Prep- TLC (eluent: DCM/MeOH=20/1) to give **1-(2-((5-chloro-2-oxooxazolo[4,5-d]pyrimidin-3(2H)-yl)methyl)-6-cyclopropylimidazo[1,2-a]pyridin-8-yl)-3-methylimidazolidine-2,4-dione intermediate 1015** (70 mg, 81%) as a white solid. ESI-MS [M +H]⁺: 454.1.

A mixture of **intermediate 1015** (20 mg, 0.044 mmol), rac-(1S*,2S*)-2-(4-methylpyrimidin-2-yl)cyclopropane-1-carboxamide (7.8 mg, 0.044 mmol), Pd₂(dba)₃ (4 mg, 0.0044 mmol), Xantphos (3 mg, 0.0044 mmol), and Cs₂CO₃ (43 mg, 0.13 mmol) in 1,4-dioxane (3 mL) was stirred for 2 h at 75 °C under N₂. The reaction mixture was filtered through Celite^{®} and the filter cake was washed with DCM/MeOH (10/1, 10 mL). The filtrate was concentrated in vacuo to give the crude, which was purified by preparative HPLC to give **(I-626)** (4 mg, 15%) as a white solid. ESI-MS [M +H]⁺: 595.2. δ 10.99 (s, 1H), 8.49 - 8.47 (m, 2H), 8.23 - 8.19 (m, 1H), 7.94 (s, 1H), 7.37 (d, J = 1.3 Hz, 1H), 7.16 (d, J = 5.1 Hz, 1H), 5.02 (s, 2H), 4.80 (s, 2H), 2.95 (s, 3H), 2.76 - 2.67 (m, 2H), 2.40 (s, 3H), 1.99 - 1.92 (m, 1H), 1.54 - 1.50 (m, 2H), 0.96 - 0.85 (m, 2H), 0.70 - 0.62 (m, 2H).

### Example 627: rac-(1S*,2S*)-N-(4-(((6-cyclopropyl-8-(3-methyl-2,4-dioxoimidazolidin-1-yl)imidazo[1,2-a]pyridin-2-yl)methyl)amino)-5-(hydroxymethyl)pyrimidin-2-yl)-2-(4-methylpyrimidin-2-yl)cyclopropane-1-carboxamide (I-627)

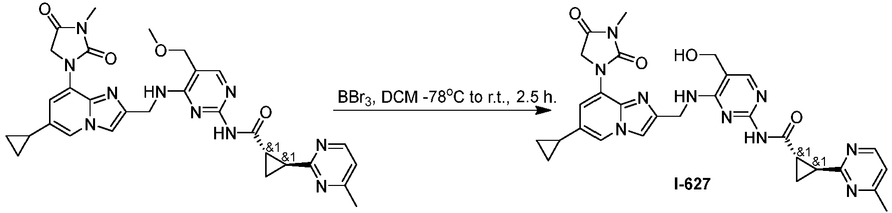

To a solution of **(I-621)** (100 mg, 0.167 mmol) in DCM (8 mL) was added BBr₃ (251 mg, 1.0 mmol) at -78 °C. The reaction was allowed warm to room temperature and it was stirred at room temperature for 2.5 h. Then the mixture was concentrated *in vacuo* to get the crude, which was purified by preparative HPLC to give **(I-627)** (15 mg, 15%) as a white solid. ESI-MS [M +H]+: 583.2. δ 10.26 (s, 1H), 8.46 (d, J = 5.1 Hz, 1H), 8.38 (s, 1H), 8.22 (d, J = 1.0 Hz, 1H), 7.88 (s, 1H), 7.76 (s, 1H), 7.33 (d, J = 1.4 Hz, 1H), 7.22-7.27 (m, 1H), 7.13 (d, J = 5.1 Hz, 1H), 5.16 (s, 1H), 4.89 (s, 2H), 4.61 (d, J = 5.4 Hz, 2H), 4.35 (s, 2H), 2.98 (s, 3H), 2.52-2.56 (m, 2H), 2.37 (s, 3H), 1.97-1.91 (m, 1H), 1.53-1.50 (m, 2H), 1.02 - 0.87 (m, 2H), 0.71 - 0.55 (m, 2H).

### Example 628: rac-(1S*,2S*)-N-(4-((6-cyclopropyl-8-(3-methyl-2,4-dioxoimidazolidin-1-yl)imidazo[1,2-a]pyridin-2-yl)methoxy)-5-(trifluoromethyl)pyrimidin-2-yl)-2-(4-methylpyrimidin-2-yl)cyclopropane-1-carboxamide (I-628)

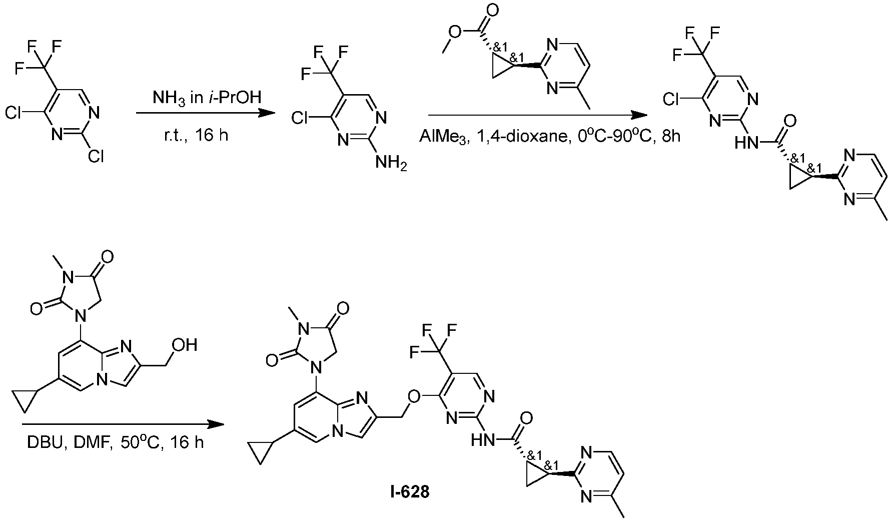

A mixture of 2,4-dichloro-5-(trifluoromethyl)pyrimidine (1 g, 4.6 mmol) in ammonia solution (2M in i-PrOH, 40 mL) was stirred at room temperature for 16 h. The reaction mixture was concentrated *in vacuo* to give the crude, which was purified by silica gel column chromatography (eluent: EtOAc/PE = 1/8) to give **4-chloro-5-(trifluoromethyl)pyrimidin-2-amine intermediate 1016** (350 mg, 39%) as a white solid. ESI-MS [M + H]⁺: 198.0.

To a solution of **intermediate 1016** (350 mg, 1.78 mmol) in 1,4-dioxane (10 mL) was added AlMe₃ (2M in 1,4-dioxane, 1.2 mL, 2.4 mmol) at 0 °C under N₂ and stirred at 30 °C for 2 h. To this resulting mixture was added a solution of *rac*-methyl (1S*,2S*)-2-(4-methylpyrimidin-2-yl)cyclopropane-1-carboxylate (386 mg, 1.78 mmol) in 1,4-dioxane (10 mL). After stirring at 90 °C for 8 h under N₂, the reaction was quenched with water (30 mL) and extracted with EtOAc (3 x 30 mL). The combined organic layers were washed with brine (20 mL), dried over anhydrous Na₂SO₄, and concentrated *in vacuo* to give the crude product, which was purified by silica gel column chromatography (eluent: EtOAc/PE = 1/1) to give ***rac*-(1S*,2S*)-N-(4-chloro-5-(trifluoromethyl)pyrimidin-2-yl)-2-(4-methylpyrimidin-2-yl)cyclopropane-1-carboxamide intermediate 861** (270 mg, 43%) as a white solid. ESI-MS [M + H]⁺: 358.1.

A mixture of **intermediate 861** (60 mg, 0.168 mmol), **intermediate 454** e (50 mg, 0.168 mmol), and DBU (25.5 mg, 0.168 mmol) in DMF (5 mL) was stirred at 50 °C for 16 h. The reaction was diluted with water (20 mL) and extracted with EtOAc (3 x 20 mL). The organic layer was concentrated in vacuo and purified by preparative HPLC to give **(I-628)** (10 mg, 10%) as a white solid. ESI-MS [M+H]+: 622.2. δ 11.34 (s, 1H), 8.74 (s, 1H), 8.50 (d, J = 5.1 Hz, 1H), 8.30 (s, 1H), 8.00 (s, 1H), 7.40 (s, 1H), 7.17 (d, J = 5.1 Hz, 1H), 5.60 (s, 2H), 4.87 (s, 2H), 2.97 (s, 3H), 2.68 - 2.58 (m, 1H), 2.54 (s, 1H), 2.39 (s, 3H), 2.05 - 1.88 (m, 1H), 1.63-1.60 (m, 2H), 1.02 - 0.90 (m, 2H), 0.69-0.65 (m, 2H).

### Example 629: (1S,2S)-N-(2-((6-cyclopropyl-8-(3-methyl-2,4-dioxoimidazolidin-1-yl)imidazo[1,2-a]pyridin-2-yl)methyl)oxazolo[5,4-c]pyridin-4-yl)-2-(4-methylpyrimidin-2-yl)cyclopropane-1-carboxamide (I-629)

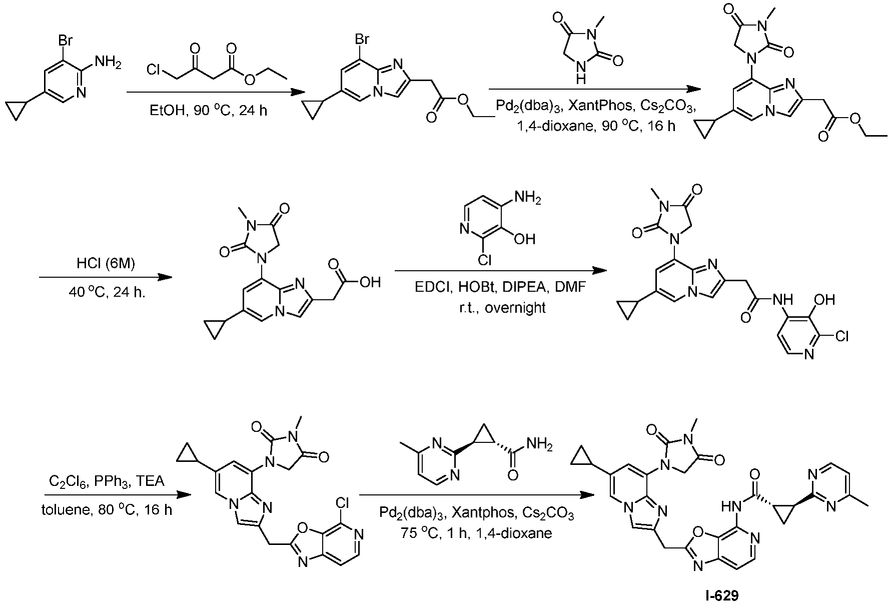

A mixture of ethyl 4-chloro-3-oxobutanoate (3.0 g, 18 mmol) and 3-bromo-5-cyclopropylpyridin-2-amine (2.5 g, 12 mmol) in EtOH (50 mL) was stirred at 90 °C for 24 h. The reaction mixture was concentrated *in vacuo* to give the crude, which was purified by column chromatography (eluent: PE/EtOAc = 5/1) to give **ethyl 2-(8-bromo-6-cyclopropylimidazo[1,2-a]pyridin-2-yl)acetate intermediate 1017** (3.0 g, 78%) as a white solid. ESI-MS [M +H]+: 323.2.

To a solution of **intermediate 1017** (3.0 g, 9.3 mmol), 3-methylimidazolidine-2,4-dione (3.2 g, 27.9 mmol), and Cs₂CO₃ (9.1 g, 27.9 mmol) in 1,4-dioxane (50 mL) was added Pd₂(dba)₃ (431 mg, 0.47 mmol) and Xantphos (538 mg, 0.93 mmol). The reaction mixture was stirred at 90 °C for 16 h under N₂. The reaction mixture was filtered through Celite^{®} and the filter cake was washed with DCM/MeOH (10/1, 100 mL). The filtrate was concentrated *in vacuo* to give the crude, which was purified by column chromatography (eluent: DCM/MeOH = 40/1) to give **ethyl 2-(6-cyclo propyl-8-(3-methyl-2,4-dioxoimidazolidin-1-yl)imidazo[1,2-a]pyridin-2-yl)acetate intermediate 1018** (2.8 g, 85%) as a yellow solid. ESI-MS [M +H]+: 357.2.

A solution of **intermediate 1018** (2.8 g, 7.9 mmol) in HCl (6M solution in water, 50 mL) was stirred at 40 °C for 24 h. The reaction mixture was concentrated *in vacuo* to give **2-(6-cyclo propyl-8-(3-methyl-2,4-dioxoimidazolidin-1-yl)imidazo[1,2-a]pyridin-2-yl)acetic acid intermediate 1019** (2.4 g, 93%) as a yellow solid, which was used in the next step without further purification. ESI-MS [M +H]+: 329.2.

A mixture of 2-(6-cyclopropyl-8-(3-methyl-2,4-dioxoimidazolidin-1-yl)imidazo[1,2-a] pyridin-2-yl) acetic acid (0.40 g, 1.2 mmol), 4-amino-2-chloropyridin-3-ol (0.18 g, 1.3 mmol), EDCI (0.35 g, 1.8 mmol), HOBt (0.25 g, 1.9 mmol), and DIPEA (0.47 g, 3.6 mmol) in dry DMF (10 mL) was stirred at room temperature for 16 h. The reaction was quenched with water (50 mL) and extracted with EtOAc (3 x 30 mL). The combined organic layers were washed with brine (sat. aq., 20 mL), dried over anhydrous Na₂SO₄, and concentrated in vacuo to give the crude product, which was purified by preparative TLC (eluent: DCM/MeOH = 15/1) to give **N-(2-chloro-3-hydroxy pyridin-4-yl) -2-(6-cyclopropyl-8-(3-methyl-2,4-dioxoimidazolidin-1-yl)imidazo[1,2-a]pyridin-2-yl)acetamide intermediate 1020** (0.22 g, 40 %) as a light yellow solid. ESI-MS [M+H]⁺: 455.1.

A mixture of **intermediate 1020** (0.22 g, 0.48 mmol), perchloroethane (0.34 g, 1.4 mmol), TEA (0.51 mL, 3.7 mmol), and PPh₃ (0.30 g, 1.1 mmol) in toluene (10 mL) was stirred at 80 °C for 16 h under N₂. The reaction mixture was filtered through Celite^{®} and the filter cake was washed with DCM/MeOH (10/1, 30 mL). The filtrate was concentrated in vacuo to give the crude, which was purified by preparative HPLC to give **1-(2-((4-chlorooxazolo[5,4-c]pyridin-2-yl)methyl)-6-cyclopropylimidazo[1,2-a]pyridin-8-yl)-3-methylimidazolidine-2,4-dione intermediate 1021** (0.11 g, 52%) as a white solid. ESI-MS [M +H]⁺: 437.2.

To a mixture of **intermediate 1021** (44 mg, 0.10 mmol), (1S,2S)-2-(4-methylpyrimidin-2-yl) cyclopropane-1-carboxamide (18 mg, 0.10 mmol), and Cs₂CO₃ (98 mg, 0.30 mmol) in dioxane (6.0 mL) were added Pd₂(dba)₃ (9.0 mg, 0.0098 mmol) and Xant-phos (12 mg, 0.021 mmol). After stirring at 75 °C for 1 h under N₂, the mixture was filtered through Celite^{®} and the filter cake was washed with DCM/MeOH (10/1, 20 mL). The filtrate was concentrated in vacuo to give the crude, which was purified by preparative HPLC to give **(I-629)** (2.0 mg, 3%) as a yellow solid. ESI-MS [M +H]⁺: 578.2. δ 11.04 (s, 1H), 8.54 (d, J = 5.1 Hz, 1H), 8.40 - 8.10 (m, 2H), 7.92 (s, 1H), 7.60 (d, J = 5.4 Hz, 1H), 7.37 (s, 1H), 7.23 (d, J = 5.1 Hz, 1H), 4.85 (s, 2H), 4.52 (s, 2H), 2.98 (s, 3H), 2.62 - 2.55 (m, 2H), 2.43 (s, 3H), 1.99 - 1.93 (m, 1H), 1.60 - 1.55 (m, 2H), 0.99 - 0.91 (m, 2H), 0.71 - 0.62 (m, 2H).

### Example 630: (1S,2S)-N-(3-((6-cyclopropyl-8-(3-methyl-2,4-dioxoimidazolidin-1-yl)imidazo[1,2-a]pyridin-2-yl)methyl)-[1,2,4]triazolo[4,3-a]pyrazin-6-yl)-2-(4-methylpyrimidin-2-yl)cyclo propane-1-carboxamide (I-630)

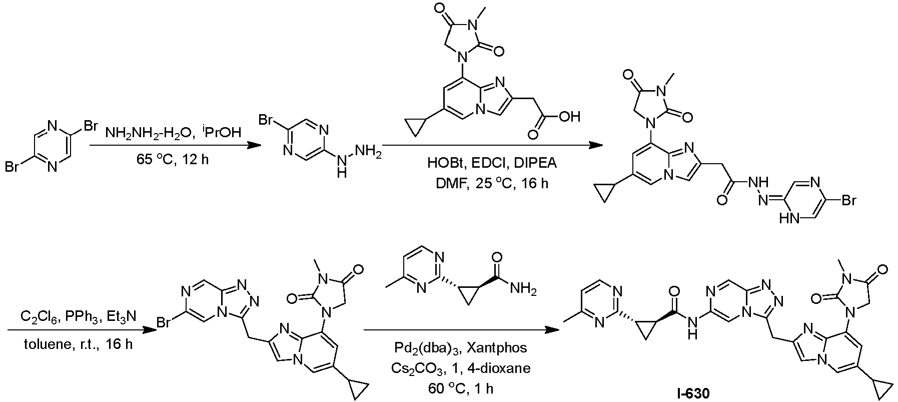

A mixture of 2,5-dibromopyrazine (1.0 g, 4.2 mmol) and NH₂NH₂-H₂O (4 g, purity: 80%, 64 mmol) in ⁱPrOH (20 mL) was stirred at 65 °C for 12 h. The reaction mixture was poured into water (50 mL) and extracted with EtOAc (3 x 50 mL). The combined organic layers were washed with brine (30 mL), dried over anhydrous Na₂SO₄, and concentrated *in vacuo* to give the crude, which was purified by column chromatography on silica gel (eluent: PE/EtOAc = 5/1) to give **2-bromo-5-hydrazineylpyrazine intermediate** 1022 (0.70 g, 88%) as a yellow oil. ESI-MS [M +H]+: 188.1

A mixture of **intermediate 1022** (0.20 g, 1.1 mmol), **intermediate 1019** (0.35 g, 1.1 mmol), EDCI (0.40 g, 2.1mmol), HOBt (0.29 g, 2.1 mmol), and DIPEA (0.19 g, 1.5 mmol) in dioxane (8.0 mL) was stirred at 25 °C for 16 h. The mixture was diluted with EtOAc (3 x 30 mL) and washed with saturated brine (60 mL), dried over anhydrous Na₂SO₄, and concentrated in vacuo to give the crude, which was purified by column chromatography on silica gel (eluent: PE/EtOAc = 10/1) to give **(E)-N'-(5-bromopyrazin-2(1H)-ylidene)-2-(6-cyclopropyl-8-(3-methyl-2,4-dioxoimidazolidin-1-yl)imidazo[1,2-a]pyridin-2-yl)acetohydrazide intermediate 1023** (0.10 g, yield 18 %) as a yellow oil.. ESI-MS [M+H]+:499.1

A mixture **intermediate 1023** (25 mg, 0.050 mmol), C₂Cl₆ (30 mg, 0.13 mmol), PPh₃ (33 mg, 0.13 mmol), and Et₃N (0.050 mL, 0.36 mmol) in toluene (8 mL) was stirred at room temperature for 16 h. The combined organic layers were washed with brine (sat. aq., 60 mL), dried over anhydrous Na₂SO₄, and concentrated in vacuo to give the crude, which was purified by column chromatography on silica gel (eluent: PE/EtOAc = 15/1) to give **1-(2-((6-bromo-[1,2,4]triazolo[4,3-a]pyrazin-3-yl)methyl)-6-cyclopropylimidazo[1,2-a]pyridin-8-yl)-3-methylimidazolidine-2,4-dione intermediate 1024** (10 mg, 42%) as a yellow oil. ESI-MS [M +H]⁺:481.1

To a mixture of **intermediate 1024** (0.10 g, 0.21 mmol), **intermediate 55** (46 mg, 0.26 mmol), and Cs₂CO₃ (0.20 g, 0.61 mmol) in dioxane (8.0 mL) were added Pd₂(dba)₃ (20 mg, 0.022 mmol) and Xant-phos (1.6 g, 2.8 mmol). The reaction mixture was stirred at 60 °C for 1 h under N₂. The mixture was filtered through Celite^{®} and the filter cake was washed with DCM/MeOH (10/1, 0.10 mL). The filtrate was concentrated in vacuo to give the crude, which was purified by silica gel chromatography (eluent: DCM/MeOH=50/1 ~ 20/1) to give **(I-630)** (50 mg, 41%) as a yellow solid. ESI-MS [M +H] +: 578.2. δ 11.07 (s, 1H), 9.42 (s, 1H), 9.26 (s, 1H), 8.53 (d, J = 5.1 Hz, 1H), 8.22 (s, 1H), 7.83 (s, 1H), 7.38 (d, J = 1.4 Hz, 1H), 7.21 (d, J = 5.1 Hz, 1H), 4.91 (d, J = 3.4 Hz, 2H), 4.67 (s, 2H), 3.00 (s, 3H), 2.56 - 2.55 (m, 2H), 2.42 (s, 3H), 2.02 - 1.99 (m, 1H), 1.50 - 1.47 (m, 2H), 0.95 - 0.93 (m, 2H), 0.64 - 0.63 (m, 2H).

### Example 631: (1S,2S)-2-(4-chloropyrimidin-2-yl)-N-(8-((6-cyclopropyl-8-(3-methyl-2,4-dioxoimidazolidin-1-yl)imidazo[1,2-a]pyridin-2-yl)methyl)-7,8-dihydro-6H-pyrimido[5,4-b][1,4]oxazin-2-yl)cyclopropane-1-carboxamide (I-631)

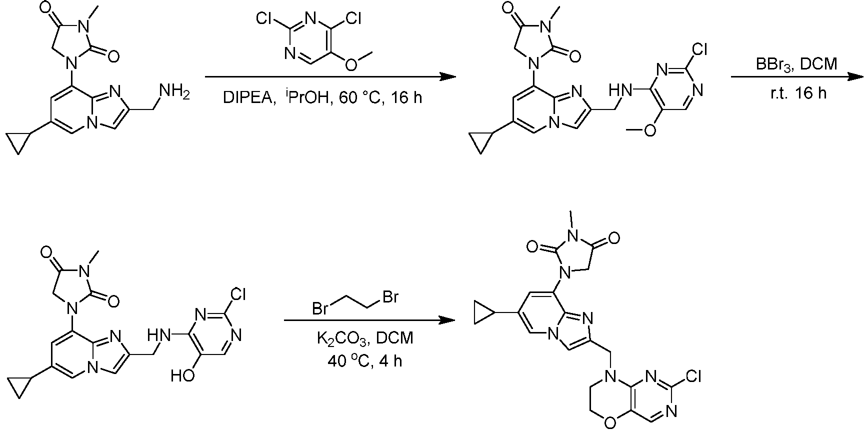

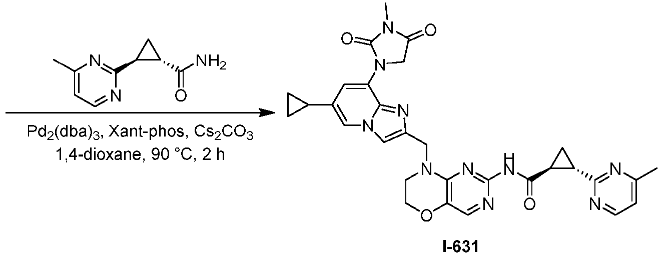

A mixture of 2,4-dichloro-5-methoxypyrimidine (0.36 g, 2.0 mmol), **intermediate 365** (0.30 g, 1.0 mmol), and DIPEA (0.39 g, 3.0 mmol) in ⁱPrOH (10 mL) was stirred at 60 °C for 16 h. The mixture was concentrated in vacuo to get a crude, which was purified by preparative TLC (DCM/MeOH = 20/1) to give **1-(2-(((2-chloro-5-methoxypyrimidin-4-yl)amino)methyl)-6-cyclo propylimidazo [1,2-a]pyridin-8-yl)-3-methylimidazolidine-2,4-dione intermediate 1025** (0.35 g, 79 %) as a white solid. ESI-MS [M+H]⁺: 442.2.

To mixture of **intermediate 1025** (0.22 g, 0.50 mmol) in dry DCM (10 mL) was added BBr₃ (1.3 g, 5.2 mmol) at 0 °C . Then the mixture was stirred at room temperature for 16 h. The reaction mixture was quenched with saturated aq. NH₄Cl (20 mL) and extracted with EtOAc (3 x 30 mL). The combined organic layers were washed with saturated brine (30 mL), dried over anhydrous Na₂SO₄, and concentrated in vacuo to give the crude, which was purified by column chromatography on silica gel (eluent: DCM/MeOH = 20/1) to give **1-(2-(((2-chloro-5-hydroxy pyrimidin-4-yl)amino)methyl)-6-cyclopropylimidazo[1,2-a]pyridin-8-yl)-3-methyl imidazolidine-2,4-dione intermediate 1026** (0.10 g, 47%) as a yellow solid. ESI-MS [M +H]⁺: 428.1.

A mixture of **intermediate 1026**(0.10 g, 0.23 mmol), 1,2-dibromoethane (65 mg, 0.35 mmol), and K₂CO₃ (95 mg, 0.69 mmol) in DCM (10.0 mL) was stirred at 40 °C for 4 h. The mixture was concentrated in vacuo to get a crude, which was purified by preparative TLC (DCM/MeOH = 20/1) to give **1-(2-((2-chloro-6,7-dihydro-8H-pyrimido[5,4-b][1,4]oxazin-8-yl)methyl)-6-cyclopropyl imidazo[1,2-a]pyridin-8-yl)-3-methylimidazolidine-2,4-dione intermediate 1027** (40 mg, 38 %) as a white solid. ESI-MS [M +H]⁺: 454.2.

To a mixture of **intermediate 1027**(40 mg, 0.088 mmol), 2-(4-methylpyrimidin-2-yl)cyclopropane-1-carboxamide (16 mg, 0.090 mmol), and Cs₂CO₃ (86 mg, 0.26 mmol) in dioxane (5.0 mL) were added Pd₂(dba)₃ (8.0 mg, 0.0087 mmol) and Xant-phos (9.5 mg, 0.02 mmol). After stirring at 90 °C for 2 h under N₂, the reaction mixture was filtered through Celite^{®} and the filter cake was washed with DCM/MeOH (10/1, 30 mL). The filtrate was concentrated in vacuo to give the crude, which was purified by preparative TLC (eluent: DCM/MeOH=15/1) to give **(I-631)** (22 mg, 42%) as a white solid. ESI-MS [M +H]⁺: 595.2, δ 10.26 (s, 1H), 8.46 (d, J = 5.1 Hz, 1H), 8.20 (s, 1H), 7.80 (s, 1H), 7.64 (s, 1H), 7.35 (d, J = 1.3 Hz, 1H), 7.14 (d, J = 5.0 Hz, 1H), 4.88 (s, 2H), 4.85 - 4.75 (m, 2H), 4.14 (t, J = 4.3 Hz, 2H), 3.60 (s, 2H), 2.53 - 2.50 (m, 2H), 2.37 (s, 3H), 1.98 - 1.91 (m, 1H), 1.57 - 1.42 (m, 2H), 1.00 - 0.84 (m, 2H), 0.74 - 0.54 (m, 2H) ppm.

### Examples 632-634

### rac-(1S*,2S*)-N-(2-((6-cyclopropyl-8-(3-methyl-2,4-dioxoimidazolidin-1-yl)imidazo[1,2-a]pyridin-2-yl)methyl)-2H-pyrazolo[3,4-d]pyrimidin-4-yl)-2-(4-methylpyrimidin-2-yl)cyclopropane-1-carboxamide (I-632)

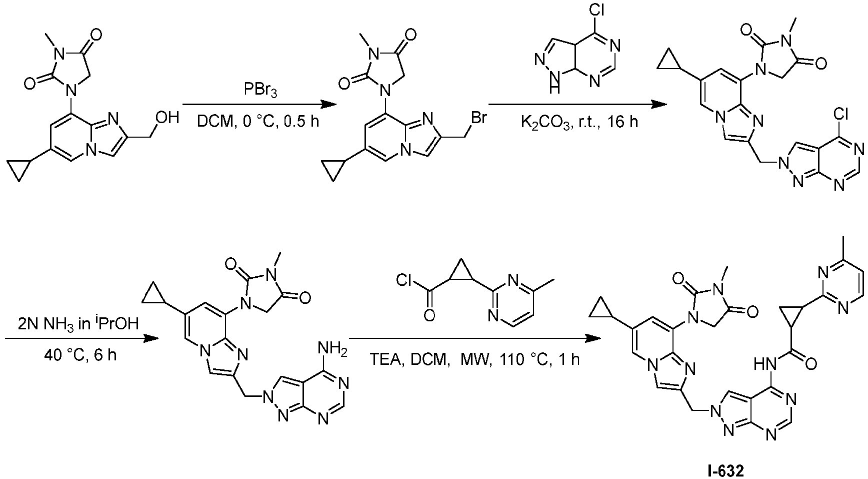

To a mixture of **intermediate 454** (0.50 g, 1.7 mmol) in DCM (10 mL) was added PBr₃ (1.0 mL). After stirring at 0 °C for 0.5 h, the reaction mixture was quenched by saturated aq. NaHCO₃ (10 mL) and extracted by DCM (3 x 20 mL). The combined organic layers were concentrated *in vacuo* to afford **1-(2-(bromomethyl)-6-cyclopropylimidazo[1,2-a]pyridin-8-yl)-3-methyl imidazolidine-2,4-dione intermediate 1028** (0.32 g, 52% yield ) as a yellow oil. ESI-MS [M+H]+: 363.1 .

A mixture of **intermediate 1028**(0.32 g, 0.88 mmol), 4-chloro-3a,7a-dihydro-1H-pyrazolo[3,4-d]pyrimidine (0.14 g, 0.89 mmol), and K₂CO₃ (0.37 g, 2.7 mmol) in DMF (10 mL) was stirred at room temperature for 16 h under N₂. The reaction mixture was quenched with water (30 mL) and extracted with EtOAc (3 x 30 mL). The combined organic layers were washed with saturated brine (20 mL), dried over anhydrous Na₂SO₄, and concentrated in vacuo to give the crude product, which was purified by prep-HPLC to afford **1-(2-((4-chloro-2H-pyrazolo[3,4-d] pyrimidin-2-yl)methyl)-6-cyclopropylimidazo[1,2-a]pyridin-8-yl)-3-methylimidazolidine-2,4-dione intermediate 1029** (50 mg, 13% yield) as yellow solid. ESI-MS [M+H]+: 437.2 .

A solution of **intermediate 1029** (0.34 g, 0.78 mmol) in NH₃-ⁱPrOH (2 N, 10 mL) was stirred at 40 °C for 6 h under N₂. The reaction mixture was concentrated in vacuo to give a crude, which was purified by preparative TLC (eluent: DCM/MeOH = 10/1) to afford **1-(2-((4-amino-2H-pyrazolo[3,4-d]pyrimidin-2-yl)methyl)-6-cyclopropylimidazo[1,2-a]pyridin-8-yl)-3-methyl imidazolid ine-2,4-dione intermediate 1030** (40 mg, 12% yield) as yellow solid. ESI-MS [M+H]+: 418.2 .

A mixture of **intermediate 1030**(50 mg, 0.12 mmol), rac-(1S*,2S*)-2-(4-methylpyrimidin-2-yl)cyclopropane-1-carbonyl chloride (26 mg, 0.13 mmol) and TEA (13 mg, 0.13 mmol) in DCM (0.5 mL) was stirred in a sealed tube. After degassing with N₂ for 1 min, the mixture was irradiated in a microwave reactor at 110 °C for 1 h. The reaction mixture was concentrated to give a crude, which was purified by preparative HPLC to afford **(I-632)** (16 mg , 23% yield ) as a pale solid (mixture of enantiomers). ESI-MS [M+H]+: 578.2, δ 11.56 (s, 1H), 9.01 (s, 1H), 8.57 - 8.54 (m, 2H), 8.30 (d, J = 1.3 Hz, 1H), 7.94 (s, 1H), 7.39 (d, J = 1.5 Hz, 1H), 7.23 (d, J = 5.1 Hz, 1H), 5.79 (s, 2H), 4.88 (s, 2H), 2.96 (s, 3H), 2.82 - 2.78 (m, 1H), 2.69 - 2.65 (m, 1H), 2.43 (s, 3H), 1.98 - 1.94 (m, 1H), 1.68 - 1.61 (m, 2H), 0.98 - 0.93 (m, 2H), 0.68 - 0.64 (m, 2H).

The mixture was separated using SFC 80 (Daicel CHIRALPAK OD-H 250mm × 20 mm I.D. CO₂/[MeOH/ACN=1/1 (0.2% MA ) ]= 55/45. 50 g/min, 35 °C) to give two enantiomers: (1S,2S)-N-(2-((6-cyclopropyl-8-(3-methyl-2,4-dioxoimidazolidin-1-yl)imidazo[1,2-a]pyridin-2-yl)methyl)-2H-pyrazolo[3,4-d]pyrimidin-4-yl)-2-(4-methylpyrimidin-2-yl)cyclopropane-1-carboxamide and (1R,2R)-N-(2-((6-cyclopropyl-8-(3-methyl-2,4-dioxoimidazolidin-1-yl)imidazo[1,2-a]pyridin-2-yl)methyl)-2H-pyrazolo[3,4-d]pyrimidin-4-yl)-2-(4-methylpyrimidin-2-yl)cyclopropane-1-carboxamide
First eluting isomer **(I-633):** ESI-MS [M +H]+: 578.2 . ¹H NMR (400 MHz, MeOD) δ 9.03 (s, 1H), 8.54 (s, 1H), 8.48 - 8.47(m, 1H), 8.19 (d, J = 0.9 Hz, 1H), 7.92 (s, 1H), 7.38 (d, J = 1.4 Hz, 1H), 7.18 (d, J = 1.5 Hz, 1H), 5.76 (s, 2H), 4.76 (d, J = 1.9 Hz, 2H), 3.08 (s, 3H), 2.81 - 2.77 (m, 1H), 2.70 - 2.66 (m, 1H), 2.48 (s, 3H), 1.99 - 1.94 (m, 1H), 1.80 - 1.72 (m, 2H), 1.02 - 0.97 (m, 2H), 0.76 - 0.72 (m, 2H). RT = 1.74 min, 100.0 % e.e.
Second eluting isomer **(I-634):** ESI-MS [M +H]+: 578.2. δ 11.56 (s, 1H), 9.00 (s, 1H), 8.57 - 8.54 (m, 2H), 8.30 (d, J = 1.5 Hz, 1H), 7.94 (s, 1H), 7.39 (d, J = 1.5 Hz, 1H), 7.23 (d, J = 1.5 Hz, 1H), 5.79 (s, 2H), 4.88 (s, 2H), 2.96 (s, 3H), 2.82 - 2.78 (m, 1H), 2.69 - 2.64 (m, 1H), 2.43 (s, 3H), 1.98 - 1.94 (m, 1H), 1.68-1.61 (m, 2H), 0.98-0.93 (m, 2H), 0.68-0.64 (m, 2H). RT = 2.87 min, 100.0 % e.e.

### Example 635: (1S,2S)-N-(1-((6-cyclopropyl-8-(3-methyl-2,4-dioxoimidazolidin-1-yl)imidazo[1,2-a]pyridin-2-yl)methyl)-1H-[1,2,3]triazolo[4,5-c]pyridin-6-yl)-2-(4-methylpyrimidin-2-yl)cyclo propane-1-carboxamide (I-635)

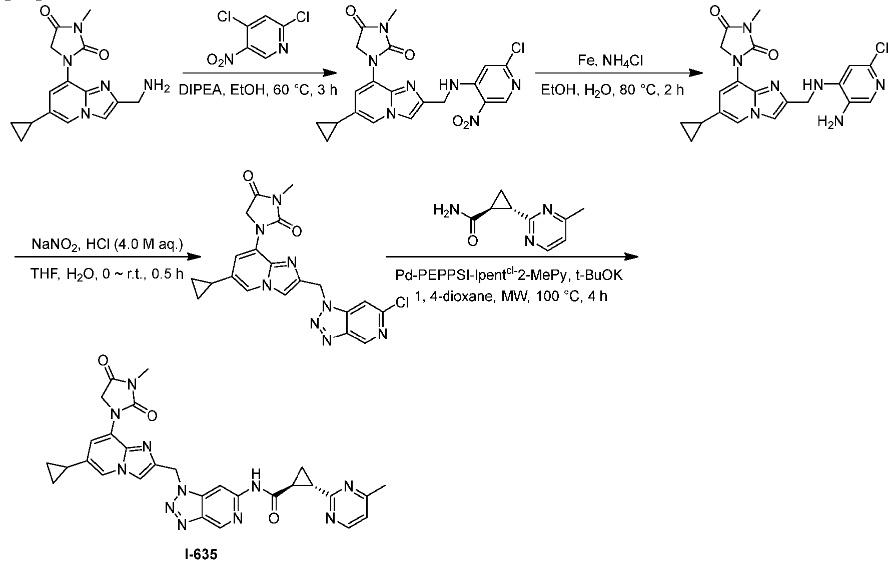

To a mixture of intermediate 365 (0.80 g, 2.7 mmol) and 2,4-dichloro-5-nitropyridine (0.62 g, 3.2 mmol) in EtOH (10 mL) was added DIPEA (1.0 g, 8.0 mmol). After stirring at 60 °C for 3 h, the reaction mixture was concentrated in vacuo to give the crude, which was purified by column chromatography on silica gel (eluent: EtOAc/PE = 0 ~ 100%, then, MeOH/DCM = 0 ~20%) to afford **1-(2-(((2-chloro-5-nitropyridin-4-yl)amino)methyl)-6-cyclopropylimidazo[1,2-a]pyridin-8-yl)-3-methylimidazolidine-2,4-dione intermediate 1031** (1.0 g, yield 81%) as yellow solid. ESI-MS [M +H]+: 456.2.

To a mixture of **1intermediate 1031** (1.0 g, 2.2 mmol) and Fe powder (0.49 g, 8.8 mmol) in EtOH (18 mL) was added a solution of NH₄Cl (0.47 g, 8.8 mmol) in water (2.0 mL). After stirring at 80 °C for 2 h, the reaction mixture was filtered by Celite^{®}. The filtered cake was washed with DCM/MeOH (v/v = 3/1, 10 mL x 3). The filtrate was concentrated in vacuo to afford **1-(2-(((5-amino-2-chloropyridin-4-yl)amino)methyl)-6-cyclopropylimidazo[1,2-a]pyridin-8-yl)-3-methylimidazolidine-2,4-dione intermediate 1032** (0.80 g, yield 85%) as yellow solid, which was used without further purification. ESI-MS [M +H]+: 426.2.

To a mixture of **intermediate 1032** (0.80 g, 1.9 mmol) and HCl (2.4 mL, 4 N HCl in dioxane, 9.6 mmol) in THF (10 mL) was added of a solution of NaNO₂ (0.39 g, 5.7 mmol) in water (5.0 mL) at 0 °C. After stirring at room temperature for 0.5 h, the reaction mixture was neutralized with NH₄OH (5.0 mL), and then extracted with DCM (3 x 10 mL). The combined organic phase was washed with saturated brine (15 mL), dried over Na₂SO₄, and concentrated in vacuo to get a residue, which was purified by column chromatography on silica gel (eluent: EtOAc/PE = 0 ~ 75%) to afford **1-(2-((6-chloro-1H-[1,2,3]triazolo[4,5-c]pyridin-1-yl)methyl)-6-cyclopropylimidazo[1,2-a]pyridin-8-yl)-3-methylimidazolidine-2,4-dione intermediate 1033** (0.20 g, yield 24%) as brown solid. ESI-MS [M +H]+: 437.2.

To a mixture of **intermediate 1033** (90 mg, 0.21 mmol) and **intermediate 55** (40 mg, 0.23 mmol) in dioxane (10 mL) was added Pd-PEPPSI-IpentCl-2-MePy (69 mg, 0.082 mmol) and t-BuOK (92 mg, 0.82 mmol). After stirring at 100 °C for 4 h in a microwave reactor, the mixture was diluted with DCM (20 mL) and filtered through Celite^{®}. The filtered cake was washed with DCM/MeOH (v/v = 3/1, 3 x 10 mL). The filtrates were concentrated in vacuo and purified by column chromatography on silica gel (eluent: EtOAc/PE = 0 ~ 100%) to afford crude product, which was purified by preparative TLC again (eluent: PE:EA = 1: 3) to afford **(I-635)** (33 mg, yield 27%) as white solid. ESI-MS [M +H]+: 578.3. δ 11.14 (s, 1H), 9.19 (d, J = 1.0 Hz, 1H), 8.69 (d, J = 0.9 Hz, 1H), 8.53 (d, J = 5.1 Hz, 1H), 8.25 (d, J = 1.0 Hz, 1H), 7.94 (s, 1H), 7.40 (d, J = 1.4 Hz, 1H), 7.21 (d, J = 5.1 Hz, 1H), 6.03 (s, 2H), 4.93 - 4.83 (m, 2H), 2.99 (s, 3H), 2.71 - 2.67 (m, 1H), 2.56 - 2.53 (m, 1H), 2.42 (s, 3H), 1.96 - 1.91 (m, 1H), 1.56 - 1.49 (m, 2H), 0.96 - 0.91 (m, 2H), 0.66 - 0.62 (m, 2H).

### Examples 636-637

### (1S,2S)-N-(4-(((8-(3-(2-(benzyloxy)ethyl)-2,4-dioxoimidazolidin-1-yl)-6-cyclopropylimidazo[1,2-a]pyridin-2-yl)methyl)(methyl)amino)-6-cyclopropylpyridin-2-yl)-2-(4-methylpyrimidin-2-yl)cyclopropane-1-carboxamide (I-636) and (1S,2S)-N-(6-cyclopropyl-4-(((6-cyclopropyl-8-(3-(2-hydroxyethyl)-2,4-dioxoimidazolidin-1-yl)imidazo[1,2-a]pyridin-2-yl)methyl) (methyl)amino)pyridin-2-yl)-2-(4-methylpyrimidin-2-yl)cyclopropane-1-carboxamide (I-637)

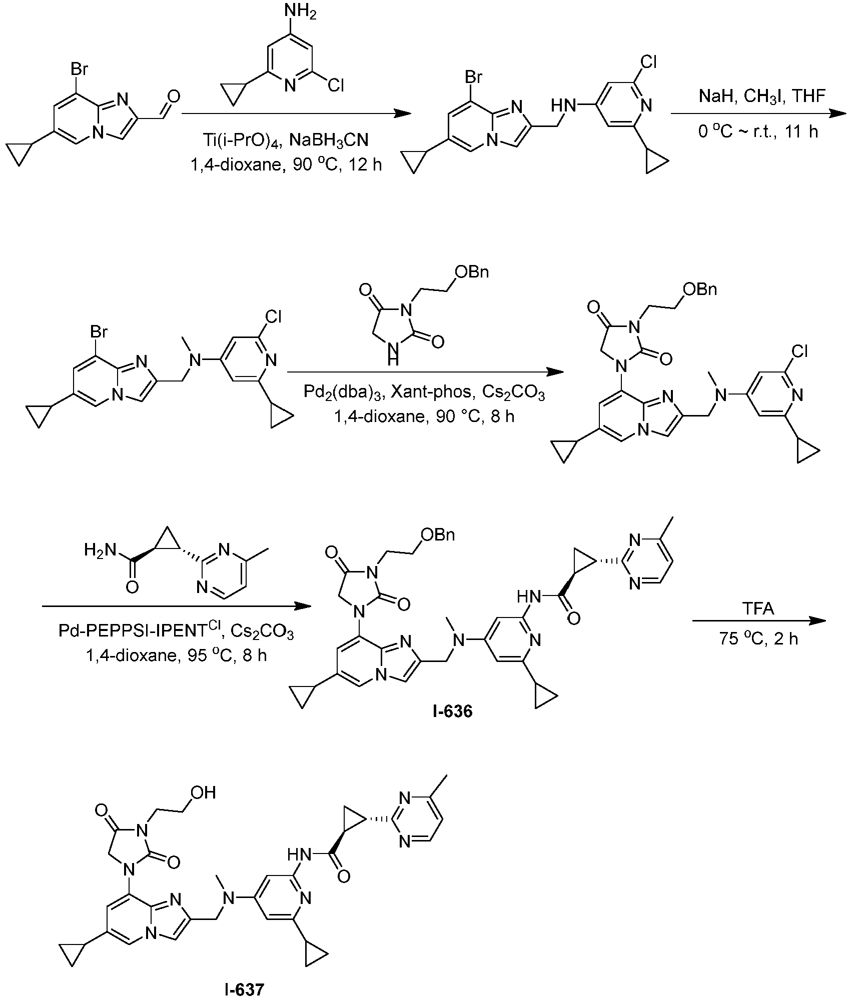

A mixture of 8-bromo-6-cyclopropylimidazo[1,2-a]pyridine-2-carbaldehyde (0.26 g, 0.98 mmol), 2-chloro-6-cyclopropylpyridin-4-amine (0.17 g, 1.0 mmol) and Ti(i-PrO)₄ (1.4 g, 4.9 mmol) in dioxane (6.0 mL) was stirred at 90 °C for 10 h under N₂. After cooling to room temperature, the mixture was diluted with MeOH (0.50 mL) and NaBH₃CN (0.32 g, 5.1 mmol) was added. Then the mixture was stirred for another 1 h at room temperature. The resulting mixture was quenched with water (50 mL) and extracted with EtOAc (3 x 15 mL). The combined organic layers were concentrated *in vacuo* and purified by column chromatography on silica gel (eluent: DCM/MeOH = 0 ~ 5%) to give **N-((8-bromo-6-cyclopropylimidazo[1,2-a]pyridin-2-yl)methyl)-2-chloro-6-cyclopropylpyridin-4-amine intermediate 1034** (0.20 g, 49%) as a light yellow solid. ESI-MS [M +H]⁺: 416.9.

To a solution of **intermediate 1034** (0.20 g, 0.48 mmol) in THF (2.0 mL) was added NaH (20 mg, 0.50 mmol) at 0 °C. After stirring at room temperature for 1 h under N₂, CH₃I (124 mg, 0.87 mmol) was added and continued stirring for another 10 h at room temperature. The reaction mixture was quenched with water and then concentrated to give the crude product, which was purified by preparative TLC (eluent: DCM/MeOH = 20:1) to give **N-((8-bromo-6-cyclopropylimidazo[1,2-a]pyridin-2-yl)methyl)-2-chloro-6-cyclopropyl-N-methylpyridin-4-amine intermediate 1035** (0.10 g, 48%) as a white solid. ESI-MS [M +H]+: 431.0.

A mixture of **intermediate 1035** (0.10 g, 0.23 mmol), **intermediate 373** (0.11 g, 0.47 mmol), Pd₂(dba)₃ (21 mg, 0.023 mmol), Xant-phos (13 mg, 0.022 mmol), and Cs₂CO₃ (0.12 g, 0.37 mmol) in dioxane (3.0 mL) was stirred at 90 °C for 8 h under N₂. The reaction mixture was concentrated in vacuo and purified by preparative TLC (eluent: DCM/MeOH = 20:1) to give **3-(2-(benzyloxy)ethyl)-1-(2-(((2-chloro-6-cyclopropylpyridin-4-yl)(methyl)amino)methyl)-6-cyclopropylimidazo[1,2-a]pyridin-8-yl)imidazolidine-2,4-dione intermediate 1036** (80 mg, 59%) as a light yellow solid. ESI-MS [M +H]+: 585.2.

A mixture **intermediate 1036** (80 mg, 0.14 mmol), **intermediate 55** (29 mg, 0.16 mmol), Pd-PEPPSI-IPENTCl (13 mg, 0.015 mmol), and Cs₂CO₃ (0.16 g, 0.49 mmol) in dioxane (3.0 mL) was stirred at 95 °C for 8 h. The reaction mixture was concentrated in vacuo and purified by preparative TLC (eluent: DCM/MeOH = 15:1) to give **(I-636)** (43 mg, 42%) as a light yellow solid. ESI-MS [M +H]+: 726.3. δ 10.18 (s, 1H), 8.47 (d, J = 5.1 Hz, 1H), 8.20 (s, 1H), 7.61 (s, 1H), 7.34 - 7.20 (m, 7H), 7.15 (d, J = 5.1 Hz, 1H), 6.41 (d, J = 2.0 Hz, 1H), 4.91 (s, 2H), 4.60 (s, 2H), 4.47 (s, 2H), 3.68 (t, J = 5.4 Hz, 2H), 3.61 (t, J = 5.3 Hz, 2H), 3.03 (s, 3H), 2.63 - 2.50 (m, 2H), 2.37 (s, 3H), 1.93 - 1.86 (m, 1H), 1.81 - 1.78 (m, 1H), 1.45 - 1.42 (m, 2H), 0.92 - 0.87 (m, 2H), 0.77 - 0.72 (m, 4H), 0.62 - 0.58 (m, 2H).

A solution of **(I-636)** ((43 mg, 0.059 mmol) in TFA (3.0 mL) was stirred at 75 °C for 2 h. The reaction mixture was concentrated in vacuo and purified by preparative TLC (eluent: DCM/MeOH= 10:1) to give **(I-637)** (20 mg, 53%) as a light yellow solid. ESI-MS [M +H]+: 636.2. δ 10.19 (s, 1H), 8.48 (d, J = 5.1 Hz, 1H), 8.19 (d, J = 0.9 Hz, 1H), 7.61 (s, 1H), 7.35 - 7.33 (m, 2H), 7.16 (d, J = 5.1 Hz, 1H), 6.42 (d, J = 2.0 Hz, 1H), 4.90 (s, 2H), 4.87 - 4.83 (m, 1H), 4.61 (s, 2H), 3.56 - 3.52 (m, 4H), 3.05 (s, 3H), 2.64 - 2.55 (m, 2H), 2.38 (s, 3H), 1.93 - 1.88 (m, 1H), 1.82 - 1.78 (m, 1H), 1.46 - 1.43 (m, 2H), 0.91 - 0.89 (m, 2H), 0.78 - 0.73 (m, 4H), 0.62 - 0.60 (m, 2H).

### Example 638: (1S,2S)-N-(6-(((6-cyclopropyl-8-(3-methyl-2,4-dioxoimidazolidin-1-yl)imidazo[1,2-a]pyridin-2-yl)methyl)thio)-2-methylpyrimidin-4-yl)-2-(4-methylpyrimidin-2-yl)cyclopropane-1-carboxamide (I-638)

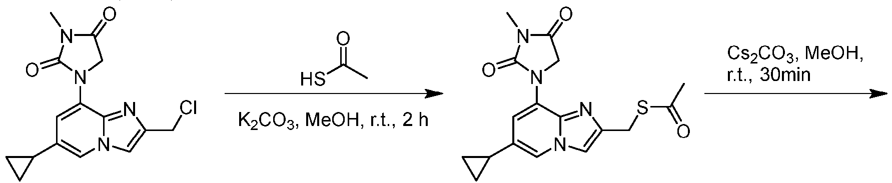

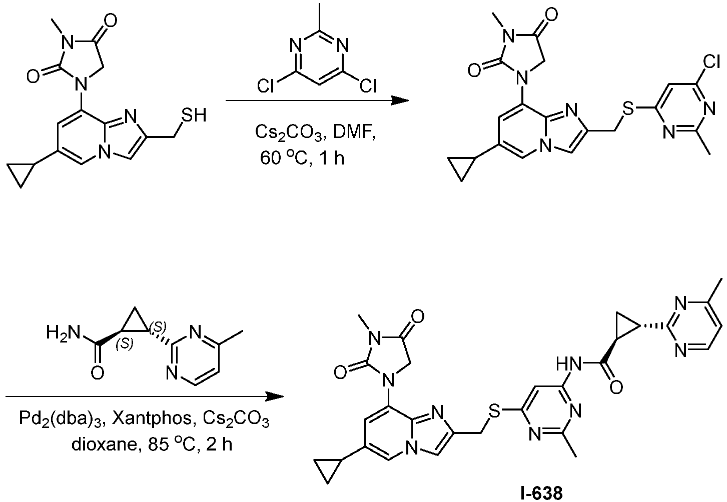

A mixture of **intermediate 451** (0.41 g, 1.3 mmol), ethanethioic S-acid (0.19 g, 2.5 mmol) and K₂CO₃ (0.35 g, 2.5 mmol) in MeOH (15 mL) was stirred at room temperature for 2 h. The reaction mixture was concentrated *in vacuo,* diluted in water (40 mL), and extracted with EtOAc (3 x 40 ml). The combined organics were washed with brine (40 mL), dried over Na₂SO₄, and concentrated *in vacuo* to give the crude product, which was purified by silica gel column chromatography (elute: DCM/MeOH = 25/1) to give **S-((6-cyclopropyl-8-(3-methyl-2,4-dioxoimidazolidin-1-yl)imidazo[1,2-a]pyridin-2-yl)methyl) ethanethioate intermediate 1037** (250 mg, 52%) as a yellow solid. ESI-MS [M +H]⁺: 359.1.

To a stirred solution of **intermediate 1037** (230 mg, 0.64 mmol) in MeOH (10 mL) was added Cs₂CO₃ (0.42 g, 1.3 mmol). After stirring at room temperature for 30 min, the reaction mixture was concentrated *in vacuo* , diluted in water (30 mL), and extracted with EtOAc (2 x 30 mL). The combined organics were washed with brine (40 mL), dried over Na₂SO₄, concentrated, and dried *in vacuo* to give **1-(6-cyclopropyl-2-(mercaptomethyl)imidazo[1,2-a]pyridin-8-yl)-3-methylimidazolidine-2,4-dione intermediate 1038** (220 mg, quant.) as a yellow solid. ESI-MS [M + H]⁺: 317.1.

A mixture of **intermediate 1038** (220 mg, 0.70 mmol), 4,6-dichloro-2-methylpyrimidine (230 mg, 1.4 mmol), and Cs₂CO₃ (450 mg, 1.4 mmol) in DMF (10 mL) was stirred at 60 °C for 1 h. The reaction mixture was poured into water (40 mL) and extracted with EtOAc (2 x 40 mL). The combined organics were washed with brine (40 mL), dried over Na₂SO₄, concentrated in vacuo, and purified by silica gel column chromatography (elute: PE/EtOAc = 2/1) to give **1-(2-(((6-chloro-2-methylpyrimidin-4-yl)thio)methyl)-6-cyclopropylimidazo[1,2-a]pyridin-8-yl)-3-methylimidazolidine-2,4-dione intermediate 1039** (250 mg, 81%) as a yellow solid. ESI-MS [M + H]⁺: 443.1.

A mixture of **intermediate 1039** (60 mg, 0.14 mmol), **intermediate 55** (29 mg, 0.16 mmol), Pd₂(dba)₃ (12 mg, 0.014 mmol), Xantphos (16 mg, 0.027 mmol), and Cs₂CO₃ (0.13 g, 0.41 mmol) in 1,4-dioxane (5.0 mL) was stirred at 85 °C for 2 h under N₂. The reaction mixture was diluted in DCM/MeOH (20 mL, 10/1), filtered, and the filtrate was concentrated in vacuo and purified by preparative TLC (DCM/MeOH = 15/1) to give **(I-638)** (35 mg, 44%) as a white solid. ESI-MS [M + H]⁺: 584.2. δ 11.20 (s, 1H), 8.53 (d, J = 5.1 Hz, 1H), 8.24 (d, J = 1.2 Hz, 1H), 7.91 (s, 1H), 7.84 (s, 1H), 7.37 (d, J = 1.5 Hz, 1H), 7.21 (d, J = 5.1 Hz, 1H), 4.89 (s, 2H), 4.48 (s, 2H), 2.97 (s, 3H), 2.65 - 2.60 (m, 1H), 2.57 - 2.55 (m, 1H), 2.49 (s, 3H), 2.41 (s, 3H), 1.99 - 1.91 (m, 1H), 1.58 - 1.49 (m, 2H), 0.98 - 0.90 (m, 2H), 0.69 - 0.61 (m, 2H).

### Example 639: (1S,2S)-N-(2-(((6-cyclopropyl-8-(3-methyl-2,4-dioxoimidazolidin-1-yl)imidazo[1,2-a]pyridin-2-yl)methyl)amino)-6-methylpyridin-4-yl)-2-(4-methylpyrimidin-2-yl)cyclopropane-1-carboxamide (I-639)

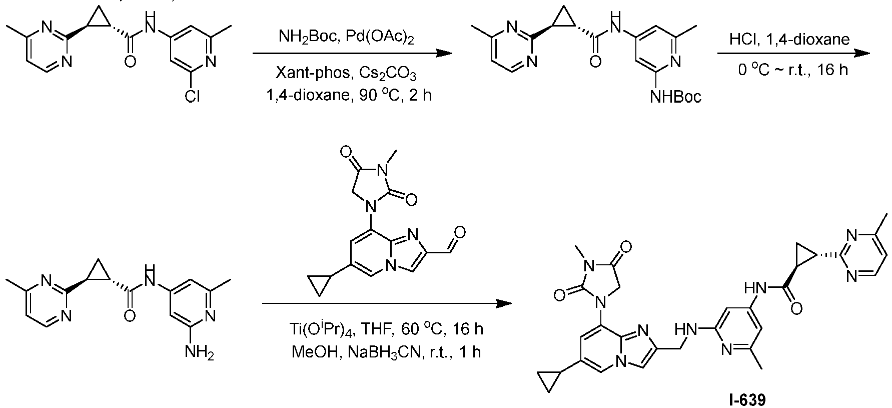

To a solution of (1S,2S)-N-(2-chloro-6-methylpyridin-4-yl)-2-(4-methylpyrimidin-2-yl)cyclopropane-1-carboxamide (0.15 g, 0.50 mmol) in dioxane (5.0 mL) was added NH₂Boc (0.12 g, 1.0 mmol), Cs₂CO₃ (0.49 g, 1.5 mmol), Xant-phos (48 mg, 0.083 mmol), and Pd(OAc)₂ (22 mg, 0.098 mmol) under N₂. After stirring at 90 °C for 2 h, the reaction mixture was concentrated *in vacuo* to give the crude, which was purified by column chromatography on silica gel (eluent: EtOAc/PE = 1/1) to give ***tert*-butyl (6-methyl-4-((1S,2S)-2-(4-methylpyrimidin-2-yl)cyclopropane-1-carboxamido)pyridin-2-yl)carbamate intermediate 1040** (0.11 g, 57%) as a white solid. ESI-MS [M + H]⁺: 384.2.

To a solution of **intermediate 1040** (0.11 g, 0.29 mmol) in dioxane (3.0 mL) was added HCl (2.0 mL, 4 N HCl in 1, 4-dioxane, 8.0 mmol). After stirring at room temperature for 16 h, the reaction mixture was neutralized by adding NH₃ in MeOH (5.0 mL, 4 N, 20 mmol). The mixture was concentrated *in vacuo* and purified by preparative HPLC (eluent: MeOH/DCM = 1/10) to give **(1S,2S)-N-(2-amino-6-methylpyridin-4-yl)-2-(4-methylpyrimidin-2-yl)cyclopropane-1-carboxamide intermediate 1041** (60 mg, 73%) as a yellow solid. ESI-MS [M + H ]⁺: 284.2.

To a solution of **intermediate 1041(60** mg, 0.21 mmol) in THF (5.0 mL) was added **intermediate 1053** (63 mg, 0.21 mmol) and Ti(OⁱPr)₄ (0.30 g, 1.1 mmol) at room temperature under N₂. After stirring at 60 °C for 16 h, NaBH₃CN (40 mg, 0.64 mmol) and MeOH (1.0 mL) were added and the resulting mixture was stirred at room temperature for another 1 h. The reaction was quenched with saturated aq. NH₄Cl (5.0 mL) and extracted with EtOAc/MeOH (10/1, 3 x 50 mL). The combined organic layers were concentrated and purified by preparative HPLC (eluent: MeOH/DCM = 1/10) to give **(I-639)** (50 mg, 42%) as a white solid. ESI-MS [M+H]+: 566.2. δ 10.34 (s, 1H), 8.52 (d, J = 5.1 Hz, 1H), 8.25 (d, J = 1.1 Hz, 1H), 7.68 (s, 1H), 7.32 (d, J = 1.4 Hz, 1H), 7.21 (d, J = 5.1 Hz, 1H), 6.96 (s, 1H), 6.80 (s, 1H), 6.56 (s, 1H), 4.91 (s, 2H), 4.50 (d, J = 5.7 Hz, 2H), 2.97 (s, 3H), 2.52 (s, 2H), 2.41 (s, 3H), 2.22 (s, 3H), 1.96-1.90 (m, 1H), 1.51 - 1.48 (m, 2H), 0.96-0.91 (m, 2H), 0.67 - 0.61 (m, 2H).

### Example 640: (1S,2S)-N-(5-(((6-cyclopropyl-8-(3-methyl-2,4-dioxoimidazolidin-1-yl)midazo[1,2-a]pyridin-2-yl)methyl)amino)-6-(trifluoromethyl)pyridazin-3-yl)-2-(4-methylpyrimidin-2-yl)cyclopropane-1-carboxamide (I-640)

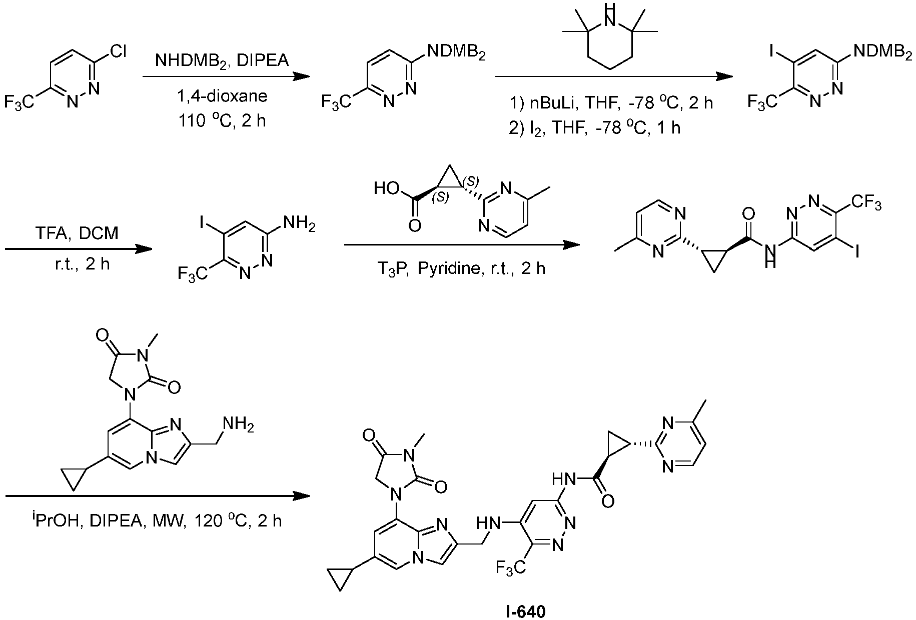

A mixture of 3-chloro-6-(trifluoromethyl)pyridazine (2.0 g, 11 mmol), bis(3,4-dimethoxybenzyl) amine (5.2 g, 16 mmol), and DIPEA (4.3 g, 33 mmol) in dioxane (30 mL) was stirred under N₂ at 110 °C for 2 h. After cooling to room temperature, the reaction was quenched with saturated aq. NaHCO₃ (100 mL) and extracted with EtOAc (3 x 50 mL). The combined organic layers were washed with saturated brine (50 mL), dried over Na₂SO₄ and concentrated *in vacuo* to get a residue, which was purified by column chromatography on silica gel (eluent: EtOAc/PE = 0 ~ 20%) to afford **N,N-bis(2,4-dimethoxybenzyl)-6-(trifluoromethyl)pyridazin-3-amine intermediate 1042** (2.0 g, 39 %) as an off-white solid.

To a mixture of 2,2,6,6-tetramethylpiperidine (0.15 g, 1.1 mmol) in THF (6 mL) was added nBuLi (0.44 mL, 1.1 mmol) at -78 °C. The mixture was stirred at -78 °C for 1 h, then was added a solution of **intermediate 1042** (0.40 g, 0.86 mmol) in THF (5.0 mL) was added and the mixture was stirred at -78 °C for 1 h. Then a solution of I₂ (0.26 g, 1.0 mmol) in THF (4 mL) was added and stirring was continued for another 1 h at -78 °C. The reaction mixture was quenched with saturated aq. NH₄Cl (100 mL) and extracted with EtOAc (3 x 50 mL). The combined organic layers were washed with saturated brine (50 mL), dried over Na₂SO₄ and concentrated *in vacuo* to get a residue. The residue was purified by column chromatography on silica gel (eluent: EtOAc/PE = 0 ~ 20%) to afford **N,N-bis(2,4-dimethoxybenzyl)-5-iodo-6-(trifluoromethyl)pyridazin-3-amine intermediate 1043** (0.35 g, 69 %) as a yellow solid.

A mixture of **intermediate 1043** (0.35 g, 0.59 mmol) in TFA (5.0 mL) and DCM (5.0 mL) was stirred at room temperature for 2 h. Then the reaction was quenched with saturated aq. NaHCO₃ (0.10 L) and extracted with EtOAc (3 x 50 mL). The combined organic layers were washed with saturated brine (50 mL), dried over Na₂SO₄ and concentrated *in vacuo* to get a residue. The residue was purified by column chromatography on silica gel (eluent: EtOAc /PE = 0 ~ 50 %) to afford **5-iodo-6-(trifluoromethyl)pyridazin-3-amine intermediate 1044** (93 mg, 55%) as a light-yellow solid.

To a mixture of **intermediate 1044** (50 mg, 0.17 mmol), (1S,2S)-2-(4-methylpyrimidin-2-yl)cyclopropane-1-carboxylic acid (46 mg, 0.26 mmol), and pyridine (0.20 mL) was added T₃P ( 1.1 g, 50% in EtOAc, 1.7 mmol). After stirring at room temperature for 2 h, the reaction was quenched with water (50 mL) and extracted with EtOAc (3 x 30 mL). The combined organic layers were washed with saturated brine (30 mL) and concentrated *in vacuo.* The residue was purified by preparative TLC (eluent: DCM/MeOH = 15/1) to afford **(1S,2S)-N-(5-iodo-6-(trifluoromethyl)pyridazin-3-yl)-2-(4-methylpyrimidin-2-yl)cyclopropane-1-carboxamide intermediate 1045** (50 mg, 65%) as an off-white solid.

A mixture of **intermediate 1045** (40 mg, 0.089 mmol), 1-(2-(aminomethyl)-6-cyclopropylimidazo-[1,2-a]pyridin-8-yl)-3-methylimidazolidine-2,4-dione (40 mg, 0.13 mmol), and DIPEA (34 mg, 0.26 mmol) in ⁱPrOH (0.50 mL) was stirred in a sealed tube. After degassing with N₂ for 1 min, the reaction was irradiated in a microwave reactor at 120 °C for 2 h. The reaction mixture was quenched with saturated aq. NaHCO₃ (50 mL) and extracted with EtOAc (3 x 30 mL). The combined organic layers were washed with saturated brine (30 mL) and concentrated *in vacuo.* The residue was purified by preparative TLC (eluent: MeOH/DCM = 10%) to afford **(I-640)** (30 mg, 54 %) as a white solid. ESI-MS [M +H] +: 621.1, δ 11.34 (s, 1H), 8.52 (d, J = 5.0 Hz, 1H), 8.25 (s, 1H), 7.95 (s, 1H), 7.73 (s, 1H), 7.45-7.39 (m, 2H), 7.20 (d, J = 5.0 Hz, 1H), 5.03 - 4.88 (m, 2H), 4.53 (d, J = 5.1 Hz, 2H), 2.99 (s, 3H), 2.69-2.62 (m, 2H), 2.41 (s, 3H), 1.99 - 1.88 (m, 1H), 1.53 (s, 2H), 0.98-0.90 (m, 2H), 0.68-0.61 (m, 2H).

### Example 641: (1S,2S)-2-(3-chlorophenyl)-N-(5-(((6-cyclopropyl-8-(3-methyl-2,4-dioxoimidazolidin-1-yl)imidazo[1,2-a]pyridin-2-yl)methyl)(methyl)amino)pyridazin-3-yl)cyclopropane-1-carboxamide (I-641)

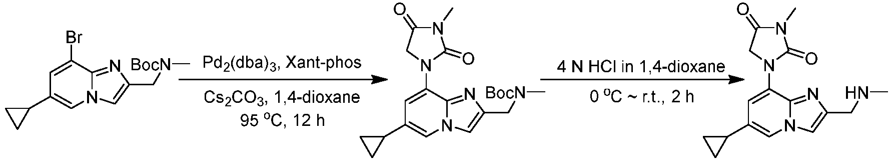

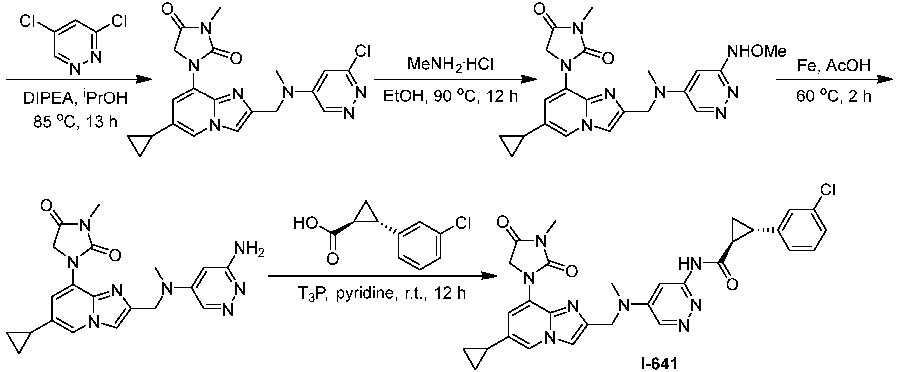

To a mixture of **intermediate 1120** (0.40 g, 1.1 mmol), 3-methylimidazolidine-2,4-dione (0.36 g, 3.2 mmol), and Cs₂CO₃ (1.0 g, 3.1 mmol) in dioxane (20 mL) were added Pd₂(dba)₃ (91 mg, 0.099 mmol) and Xant-phos (58 mg, 0.10 mmol). After stirring at 95 °C for 12 h under N₂, the reaction mixture was filtered through Celite^{®} and the filter cake was washed with DCM/MeOH (10/1, 100 mL). The filtrate was concentrated in vacuo to give the crude, which was purified by column chromatography on silica gel (eluent: PE/ EtOAc = 10/1 ~ 1/1) to give ***tert*-butyl ((6-cyclopropyl-8-(3-methyl-2,4-dioxoimidazolidin-1-yl)imidazo[1,2-a]pyridin-2-yl)methyl) (methyl)carbamate intermediate 1046** (350 mg, 77 %) as a yellow solid. ESI-MS [M +H]+: 414.2.

To a solution of **intermediate 1046** (0.35 g, 0.85 mmol) in MeOH (10 mL) was added HCl (10 mL, 4N HCl in dioxane, 40 mmol). The resulting mixture was stirred at room temperature for 2 h and concentrated *in vacuo* to give **1-(6-cyclopropyl-2-((methylamino)methyl)imidazo[1,2-a]pyridin-8-yl)-3-methylimidazolidine-2,4-dione intermediate 1047** as the hydrochloric acid salt (0.27 g, quant) as a yellow solid. ESI-MS [M +H]+: 314.1.

To a solution of **intermediate 1047** (0.27 g, 0.86 mmol) and 3,5-dichloropyridazine (0.25 g, 1.7 mmol) in i-PrOH (10 mL) was added DIPEA (0.55 mg, 4.3 mmol). The mixture was stirred at 85 °C for 13 h and then concentrated in vacuo to give the crude, which was purified with preparative TLC (eluent: DCM/MeOH = 12/1) to give **1-(2-(((6-chloropyridazin-4-yl)(methyl)amino)methyl)-6-cyclopropylimidazo[1,2-a]pyridin-8-yl)-3-methylimidazolidine-2,4-dione intermediate 995** (0.25 g, 68%) as a white solid. ESI-MS [M +H]+: 426.2.

To a solution of **intermediate 995** (0.22 g, 0.52 mmol) in EtOH (15 mL) was added MeNH₂.HCl (0.63 g, 9.3 mmol). The mixture was stirred at 90 °C for 12 h and then concentrated in vacuo. The residue was washed with saturated aq. NaHCO₃ (20 mL) and extracted with DCM (3 x 30 mL). The combined organic layers were washed with saturated brine (50 mL), dried over Na₂SO₄, and concentrated in vacuo to give the crude **1-(6-cyclopropyl-2-(((6-(methoxyamino)pyridazin-4-yl)(methyl)amino)methyl)imidazo[1,2-a]pyridin-8-yl)-3-methylimidazolidine-2,4-dione intermediate 1048** (0.22 g, 97%) as a yellow solid. ESI-MS [M +H]+: 437.2.

To a solution of **intermediate 1048** (0.22 g, 0.50 mmol) in EtOH (15 mL) were added AcOH (2.0 mL, 20% H₂O, v/v) and Fe powder (0.14 g, 2.5 mmol). After stirring at 60 °C for 2 h, the reaction mixture was filtered through Celite^{®} and the filtrate was concentrated in vacuo. The residue was added saturated aq. NaHCO₃ (10 mL) and extracted with DCM (3 x 30 mL). The combined organic layers were washed with saturated brine (30 mL), dried over Na₂SO₄, and concentrated in vacuo to give the crude, which was purified by preparative TLC (eluent: DCM/ MeOH = 10/1) to give **1-(2-(((6-aminopyridazin-4-yl)(methyl)amino)methyl)-6-cyclopropylimidazo[1,2-a]pyridin-8-yl)-3-methylimidazolidine-2,4-dione intermediate 1049** (0.12 g, 59%) as a white solid. ESI-MS [M +H]+: 407.1.

A mixture of **intermediate 1049** (50 mg, 0.12 mmol), (1S,2S)-2-(3-chlorophenyl)cyclopropane-1-carboxylic acid (36 mg, 0.18 mg), pyridine (0.25 mL), and T₃P (0.53 g, 1.7 mmol) was stirred at room temperature for 12 h. The reaction mixture was quenched with saturated aq. NaHCO₃ (10 mL) and extracted with DCM (3 x 20 mL). The combined organic layers were washed with saturated brine (30 mL), dried over Na₂SO₄, concentrated in vacuo to give the crude, which was purified with Pre-TLC (eluent: DCM/MeOH = 20/1) to give the product **(I-641)** (48 mg, 68%) as a white solid. ESI-MS [M +H]+: 585.2, δ 10.91 (s, 1H), 8.67 (d, J = 2.8 Hz, 1H), 8.22 (d, J = 1.0 Hz, 1H), 7.77 (s, 1H), 7.71 (s, 1H), 7.41 - 7.24 (m, 4H), 7.15 (d, J = 7.6 Hz, 1H), 4.88 (s, 2H), 4.72 (s, 2H), 3.13 (s, 3H), 2.96 (s, 3H), 2.71 - 2.63 (m, 1H), 2.35 - 2.28 (m, 1H), 2.00 - 1.88 (m, 1H), 1.52 - 1.43 (m, 1H), 1.45 - 1.35 (m, 1H), 1.01 - 0.87 (m, 2H), 0.70 - 0.54 (m, 2H)

### Example 642: (1S,2S)-N-(4-(((6-cyclopropyl-8-(3-methyl-2,4-dioxoimidazolidin-1-yl)imidazo-[1,2-a]pyridin-2-yl)methyl)amino)-6-methylpyridin-2-yl)-2-(4-methylpyrimidin-2-yl)cyclopropane-1-carboxamide (I-642)

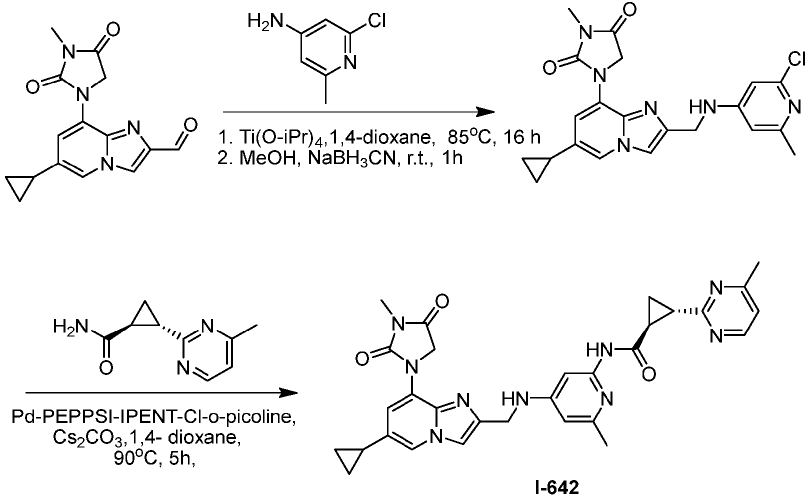

A mixture of **intermediate 1053** (149 mg, 0.5 mmol), 2-chloro-6-methylpyridin-4-amine (78.0 mg, 0.55 mmol), and Ti(O-iPr)₄ (710 mg, 2.5 mmol) in 1,4-dioxane (6.0 mL) was stirred at 85 °C for 16 h. Then the reaction mixture was cooled to 0 °C and then added MeOH (3 mL), followed by NaBH₃CN (94.5 mg, 1.5 mmol). The reaction mixture was stirred at room temperature for 1 h. The reaction was quenched with water (10 mL) and extracted with EtOAc (3 x 20 mL). The combined organic layers were washed with brine (20 mL), dried over anhydrous Na₂SO₄, and concentrated in vacuo to give the crude product, which was purified by preparative TLC (eluent: DCM/MeOH = 15/1) to give **1-(2-(((2-chloro-6-methylpyridin-4-yl)amino)methyl)-6-cyclopropylimidazo[1,2-a]pyridin-8-yl)-3-methylimidazolidine-2,4-dione intermediate 1050** (100 mg, 48 %) as a light yellow solid. ESI-MS [M +H]⁺: 425.2.

To a mixture of **intermediate 1050** (80 mg, 0.19 mmol), **intermediate 55** (34 mg, 0.19 mmol), and Cs₂CO₃ (185 mg, 0.57 mmol) in 1,4-dioxane (10 mL) was added Pd-PEPPSI-IPENT-Cl-o-picoline (16 mg, 0.02 mmol). After stirring at 90 °C for 5h under N₂, the reaction mixture was filtered through Celite^{®} and the filter cake was washed with DCM/MeOH (10/1, 20 mL). The filtrate was concentrated in vacuo to give the crude, which was purified by preparative HPLC to give **(I-642)** (22 mg, 21%) as a white solid. ESI-MS [M +H]⁺: 566.3. δ 10.39 (s, 1H), 8.51 (d, J = 5.1 Hz, 1H), 8.26 (d, J = 1.1 Hz, 1H), 7.70 (s, 1H), 7.34 (d, J = 1.5 Hz, 1H), 7.30 (s, 1H), 7.19 (d, J = 5.1 Hz, 1H), 7.01 (t, J = 5.9 Hz, 1H), 6.22 (d, J = 1.7 Hz, 1H), 4.93 (s, 2H), 4.36 (d, J = 5.9 Hz, 2H), 2.98 (s, 3H), 2.54 - 2.52 (m, 2H), 2.41 (s, 3H), 2.17 (s, 3H), 1.99 - 1.86 (m, 1H), 1.54 - 1.39 (m, 2H), 0.99 - 0.86 (m, 2H), 0.71 - 0.54 (m, 2H).

### Example 643: (1S,2S)-N-(3-((6-cyclopropyl-8-(3-methyl-2,4-dioxoimidazolidin-1-yl)imidazo[1,2-a]pyridin-2-yl)methoxy)phenyl)-2-(4-methylpyrimidin-2-yl)cyclopropane-1-carboxamide (I-643)

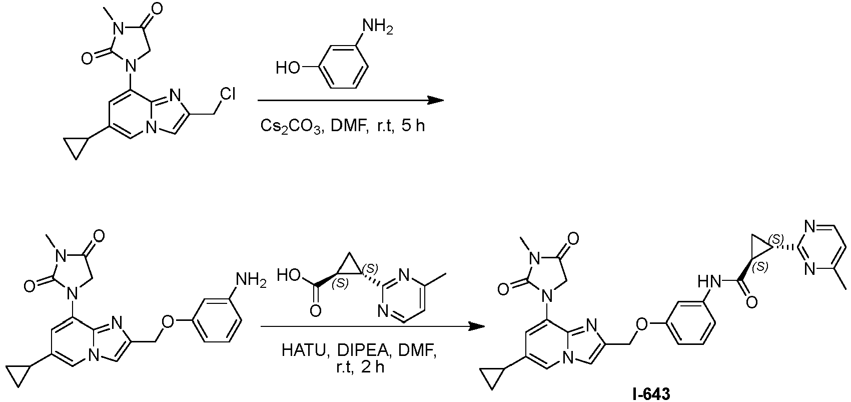

A mixture of **intermediate 451** (0.38 g, 1.2 mmol), 3-aminophenol (0.14 g, 1.3 mmol), and Cs₂CO₃ (0.78 g, 2.4 mmol) in DMF (10 mL) was stirred at room temperature for 5 h. The reaction mixture was poured into water (100 mL) and extracted with EtOAc (3 x 40 mL). The combined organics were washed and brine (3 x 30 mL), dried over Na₂SO₄ then concentrated *in vacuo* to give the crude product, which was purified by column chromatography on silica gel (eluent: EtOAc/PE from 0 to 60%) to give **1-(2-((3-aminophenoxy)methyl)-6-cyclopropylimidazo[1,2-a]pyridin-8-yl)-3-methylimidazolidine-2,4-dione intermediate 1051** (0.15 g, 32%) as a yellow solid. ESI-MS [M + H]⁺: 392.1.

To a stirred solution of **intermediate 1051** (50 mg, 0.13 mmol) and (1S,2S)-2-(4-methylpyrimidin-2-yl)cyclopropane-1-carboxylic acid (23 mg, 0.13 mmol) in DMF (3.0 mL) was added HATU (58 mg, 015 mmol) and DIPEA (50 mg, 0.38 mmol). The resulting mixture was stirred at room temperature for 2 h. The reaction mixture was poured into water (30 mL) and extracted with EtOAc (3 x 30 mL). The combined organics were washed with brine (2 x 30 mL), dried over Na₂SO₄, concentrated in vacuo to give the crude product, which was purified by column chromatography (eluent: EtOAc/PE from 0 to 60%) to give **(I-643)** (45 mg, 53%) as a white solid. ESI-MS [M + H]⁺: 552.2. δ 10.32 (s, 1H), 8.53 (d, J = 5.1 Hz, 1H), 8.29 (d, J = 1.1 Hz, 1H), 7.93 (s, 1H), 7.43 - 7.40 (m, 1H), 7.38 (d, J = 1.4 Hz, 1H), 7.23 - 7.16 (m, 2H), 7.15 - 7.09 (m, 1H), 6.75 (dd, J = 8.1, 1.8 Hz, 1H), 5.15 (s, 2H), 4.90 (s, 2H), 2.97 (s, 3H), 2.57 - 2.52 (m, 1H), 2.45 - 2.38 (m, 1H), 2.42 (s, 3H), 1.99 - 1.93 (m, 1H), 1.56 - 1.45 (m, 2H), 1.00 - 0.91 (m, 2H), 0.71 - 0.62 (m, 2H).

### Example 644: (1S,2S)-N-(3-(((6-cyclopropyl-8-(3-methyl-2,4-dioxoimidazolidin-1-yl)imidazo[1,2-a]pyridin-2-yl)methyl)amino)phenyl)-2-(4-methylpyrimidin-2-yl)cyclopropane-1-carboxamide (I-644)

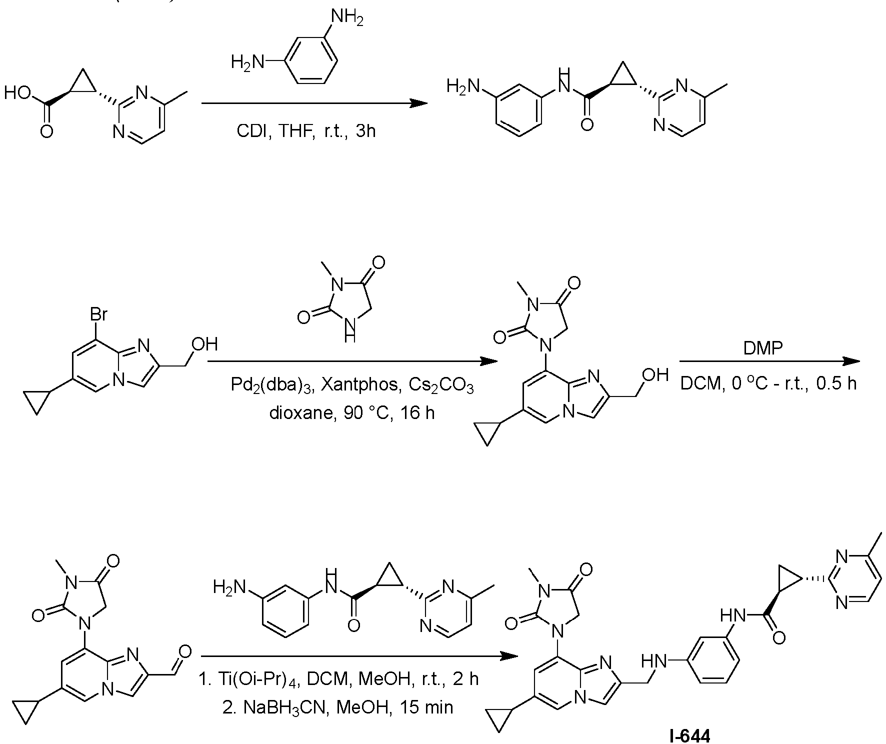

To a mixture of (1S,2S)-2-(4-methylpyrimidin-2-yl)cyclopropane-1-carboxylic acid (200 mg, 1.29 mmol) in dry THF (10 mL) was added benzene-1,3-diamine (266.4 mg, 2.464 mmol). The mixture was stirred at room temperature for 45 min and then was added CDI in dry THF (2 mL). After stirring at room temperature for 2 h, the reaction was quenched with water (30 mL) and extracted with EtOAc (3 x 30 mL). The combined organic layers were washed with saturated brine (30 mL), dried over anhydrous Na₂SO₄, and concentrated *in vacuo.* The residue was purified by column chromatography (eluent: EtOAc/PE= 0 ~ 40%) to afford **(1S,2S)-N-(3-aminophenyl)-2-(4-methylpyrimidin-2-yl)cyclopropane-1-carboxamide intermediate 1052** (216 mg, 81%) as brown solid. ESI-MS [M +H]+: 179.1.

To a mixture of **intermediate 454** (140 mg, 0.47 mmol) in DCM (3.6 mL) was added Dess Martin periodinane (493 mg, 1.16 mmol). The reaction mixture was stirred at room temperature for 0.5 h. The resulting solution was washed with saturated aq. NaHCO3 (20 mL) and saturated aq. Na₂S₂O₃ (20 mL) and then extracted with EtOAc (3 x 30 mL). The combined organic layers were washed with brine (30 mL), dried over Na2SO4 and concentrated *in vacuo.* The residue was purified by silica gel column chromatography (eluent: MeOH/DCM= 0 ~ 5%) to afford **6-cyclopropyl-8-(3-methyl-2,4-dioxoimidazolidin-1-yl)imidazo[1,2-a]pyridine-2-carbaldehyde intermediate 1053** (67 mg, 47%) as pale solid. ESI-MS [M +H]+: 299.1.

To a mixture of **intermediate 1052** (34.7 mg, 0.13 mmol) in DCM (1 mL) and MeOH (1 mL) was added **intermediate 1053** (35 mg, 0.12 mol) and Ti (Oi-Pr)₄ (168 mg, 0.59 mmol). The mixture was stirred at 50 °C for 2 h, then NaBH₃CN (62.84 mg, 0.413 mmol) was added. After stirring at room temperature for 15 min, the reaction was quenched with water (20 mL) and extracted with EtOAc (3 x 30 mL). The combined organic phases were washed with brine (30 mL), dried over anhydrous Na₂SO₄ and concentrated in vacuo to give the crude product, which was purified by pre-TLC (eluent: DCM: MeOH=20:1) to give **(I-644)** (27.2 mg, 77%) as pale solid. ESI-MS [M +H]+: 551.2. δ 10.04 (s, 1H), 8.52 (d, J = 5.1 Hz, 1H), 8.24 (d, J = 1.1 Hz, 1H), 7.67 (s, 1H), 7.32 (d, J = 1.4 Hz, 1H), 7.20 (d, J = 5.1 Hz, 1H), 6.97 - 6.92 (m, 2H), 6.78 (d, J = 8.0 Hz, 1H), 6.33 (d, J = 7.8 Hz, 1H), 6.19 (t, J = 6.0 Hz, 1H), 4.91 (s, 2H), 4.30 (d, J = 5.8 Hz, 2H), 2.97 (s, 3H), 2.47 - 2.45 (m, 1H), 2.41 - 2.37 (m, 4H), 1.96 - 1.89 (m, 1H), 1.50 - 1.43 (m, 2H), 0.95 - 0.90 (m, 2H), 0.66 - 0.62 (m, 2H).

### Examples 645-646

### (1S,2S)-N-(3-(((6-cyclopropyl-8-(3-methyl-2,4-dioxoimidazolidin-1-yl)imidazo[1,2-a]pyridin-2-yl)methyl)thio)phenyl)-2-(4-methylpyrimidin-2-yl)cyclopropane-1-carboxamide (I-645) and (1S,2S)-N-(3-(((6-cyclopropyl-8-(3-methyl-2,4-dioxoimidazolidin-1-yl)imidazo[1,2-a] pyridin-2-yl)methyl)sulfonyl)phenyl)-2-(4-methylpyrimidin-2-yl)cyclopropane-1-carboxamide (I-646)

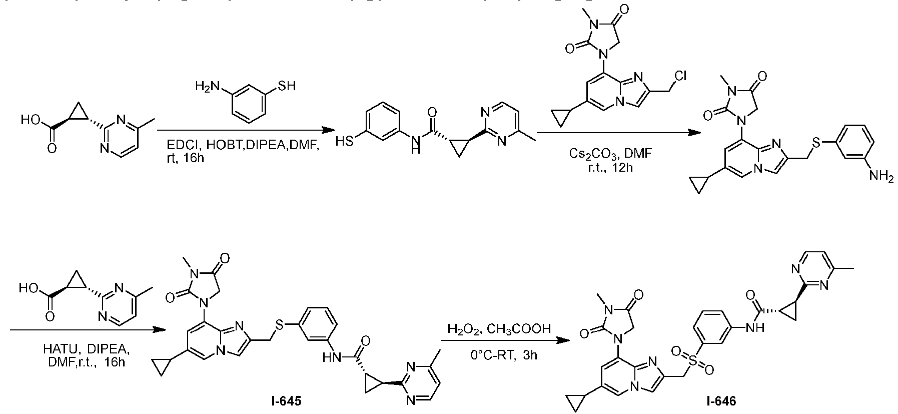

To a mixture of (1S,2S)-2-(4-methylpyrimidin-2-yl)cyclopropane-1-carboxylic acid (200 mg, 1.12 mmol) in DMF(4 mL) was added 3-aminobenzenethiol (421 mg, 3.36 mmol), EDCI (428 mg, 2.24 mmol), HOBt (303 mg, 2.24 mmol), and DIPEA (1.08 g, 8.4 mmol), and the mixture was stirred at room temperature for 16 h. The reaction mixture was quenched with water (50 mL) and extracted with EtOAc (30 mLx3). The combined organic layers were washed with brine (30 mL), dried over anhydrous Na₂SO₄, and concentrated *in vacuo* to give the crude, which was purified by column chromatography (eluent: EtOAc/PE = 0 - 10%) to give **(1S,2S)-N-(3-mercaptophenyl)-2-(4-methylpyrimidin-2-yl)cyclopropane-1-carboxamide intermediate 1054** (150 mg, yield 47 %) as a yellow oil.. ESI-MS [M+H]+:286.1

To a mixture of **intermediate 1054** (100 mg, 0.35 mmol) in DMF (5 mL) was added **intermediate 451** (112 mg, 0.35 mmol) and CsCO3 (342 mg, 1.05 mmol), and the mixture was stirred at room temperature for 12 h. The reaction mixture was quenched with water (50 mL) and extracted with EtOAc (30 mLx3). The combined organic layers were washed with brine (30 mL), dried over anhydrous Na₂SO₄, and concentrated *in vacuo* to give the crude, which was purified by column chromatography (eluent: MeOH/DCM = 0- 8%) to give **1-(2-(((3-**aminophenyl)thio)methyl)-6-cyclopropylimidazo|[1,2-a]pyridin-8-yl)-3-methylimidazolidine-**2,4-dione intermediate 1055 (50 mg, 35%) as a yellow oil. ESI-MS [M +H]+: 408.1**

**A mixture of intermediate 1055** (50 mg, 0.1 mmol), (1S,2S)-2-(4-methylpyrimidin-2-yl)cyclopropane-1-carboxylic acid (45 mg, 0.2 mmol), HATU (114 mg, 0.3 mmol), and DIPEA (39 mg, 0.3 mmol) in DMF (2 mL) was stirred at room temperature for 16 h under N₂. The reaction mixture was quenched with water (50 mL) and extracted with EtOAc (3 x 50 mL). The combined organics were washed with brine (50 mL), dried over Na₂SO₄ and concentrated in vacuo . The residue was purified by flash chromatography (eluent: EtOAc/PE = 0- 20%) to give **(I-645)** (60 mg, yield 60%) as a colorless oily liquid ESI-MS: [M +H]+,568.1 . δ 10.35 (s, 1H), 8.53 (d, J = 5.1 Hz, 1H), 8.23 (d, J = 1.0 Hz, 1H), 7.77 (s, 1H), 7.70 (s, 1H), 7.37 - 7.33 (m, 2H), 7.23 - 7.19 (m, 2H), 7.08 (d, J = 8.3 Hz, 1H), 4.86 (s, 2H), 4.28 (s, 2H), 2.96 (s, 3H), 2.56 - 2.54 (m, 1H), 2.46 - 2.45 (m, 1H), 2.42 (s, 3H), 1.95 - 1.91 (m, 1H), 1.53 -1.43 (m, 2H), 0.96 - 0.91 (m, 2H), 0.66 - 0.62 (m, 2H).

To a mixture of **(I-645)** (60 mg, 0.11 mmol) in AcOH (5 mL) was added H₂O₂ (0.08 mL, 0.11 mmol) and the mixture was stirred for 3 h at room temperature under N₂. The reaction mixture was concentrated in vacuo and the residue was purified by preparative TLC (eluent: DCM/MeOH=10/1) to afford **(I-646)** (27 mg, 41%) as white solid. ESI-MS: [M +H]+,600.1 . δ 10.68 (s, 1H), 8.53 (d, J = 5.1 Hz, 1H), 8.29 (s, 1H), 8.06 (s, 1H), 7.84 (s, 1H), 7.76 (d, J = 7.7 Hz, 1H), 7.53 - 7.45 (m, 2H), 7.34 (d, J = 1.3 Hz, 1H), 7.21 (d, J = 5.1 Hz, 1H), 4.76 (s, 2H), 4.52 (s, 2H), 2.93 (s, 3H), 2.55 - 2.54 (m, 1H), 2.46 - 2.45 (m, 1H), 2.42 (s, 3H), 1.97 - 1.92 (m, 1H), 1.51 - 1.47 (m, 2H), 0.97 - 0.92 (m, 2H), 0.67 - 0.64 (m, 2H).

### Example 647: (1S,2S)-2-(3-chlorophenyl)-N-(6-(((6-cyclopropyl-8-(2-oxopiperazin-1-yl)imidazo[1,2-a]pyridin-2-yl)methyl)amino)pyrimidin-4-yl)cyclopropane-1-carboxamide (I-647)

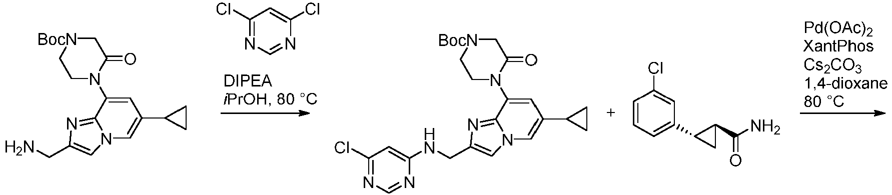

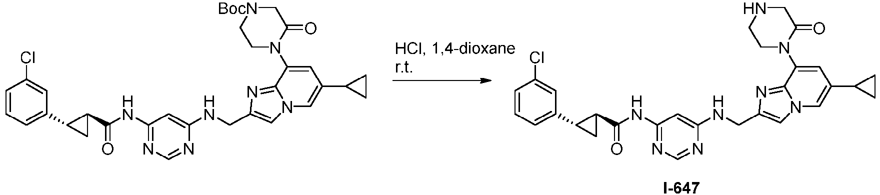

A mixture of tert-butyl 4-(2-(aminomethyl)-6-cyclopropylimidazo[1,2-a]pyridin-8-yl)-3-oxopiperazine-1-carboxylate (0.13 g, 0.32 mmol), 4,6-dichloropyrimidine (0.048 g, 0.32 mmol), and DIPEA (0.56 mL, 3.2 mmol) in iPrOH (1.8 mL) was stirred at 80 °C for 18 h. Further 4,6-dichloropyrimidine (0.048 g, 0.32 mmol) was added and the mixture was stirred at 80 °C for 1 h. The mixture was concentrated in vacuo and purified by column chromatography on silica gel, eluting with 0-20 % (7 N NH₃ in MeOH) in DCM to give tert-butyl **4-(2-(((6-chloropyrimidin-4-yl)amino)methyl)-6-cyclopropylimidazo[1,2-a]pyridin-8-yl)-3-oxopiperazine-1-carboxylate intermediate 1056** (0.14 g, 85 %) as a yellow gum. ESI-MS (M+H)+: 498.

A mixture of **intermediate 1056** (0.14 g, 0.28 mmol), **intermediate 119** (0.075 g, 0.39 mmol), Pd(OAc)₂ (0.012 g, 0.055 mmol), XantPhos (0.064 g, 0.11 mmol), and Cs₂CO₃ (0.27 g, 0.83 mmol) in 1,4-dioxane (3.0 mL) was degassed with nitrogen and stirred at 80 °C for 18 h. Further Pd(OAc)₂ (0.012 g, 0.055 mmol), XantPhos (0.064 g, 0.11 mmol), and 1,4-dioxane (1.0 mL) were added and the mixture was degassed with nitrogen. The mixture was stirred at 100 °C for 2 h, then concentrated in vacuo. The residue was purified by column chromatography on silica gel (25 g), eluting with 0-20 % (7 N NH₃ in MeOH) in DCM to give ***tert*-butyl 4-(2-(((6-((1S,2S)-2-(3-chlorophenyl)cyclopropane-1-carbox amido)pyrimidin-4-yl)amino)methyl)-6-cyclopropylimidazo[1,2-a]pyridin-8-yl)-3-oxopiperazine-1-carboxylate intermediate 1057** (0.057 g, 31 %) as a brown gum, which was used directly in the next step without further purification. ESI-MS (M+H)+: 657.

HCl (4.0 M in 1,4-dioxane, 1.0 mL, 4.0 mmol) was added to a solution of **intermediate 1057** (0.054 g, 0.082 mmol) in 1,4-dioxane (3.0 mL). The mixture was stirred at room temperature for 3.5 h then concentrated in vacuo. The residue was loaded onto an SCX cartridge and washed with a mixture of methanol and DCM then with MeOH. The product was eluted with 7 N NH₃ in MeOH and concentrated in vacuo. The residue was purified by preparative HPLC to give **(I-647)** (0.0094 g, 20 %) as an off-white solid. ESI-MS (M+H)+: 557.7, δ 10.63 (s, 1H), 8.32 (s, 1H), 8.21 (s, 1H), 7.93 (s, 1H), 7.67 (s, 1H), 7.36 - 7.31 (m, 1H), 7.28 (d, J=6.4 Hz, 3H), 7.16 (d, J=7.7 Hz, 1H), 6.95 (s, 1H), 4.58 - 4.58 (m, 2H), 3.79 (dd, J=5.1, 5.1 Hz, 2H), 3.58 (s, 2H), 3.19 (dd, J=5.1, 5.1 Hz, 2H), 2.45 - 2.34 (m, 2H), 1.98 - 1.90 (m, 1H), 1.51 - 1.39 (m, 2H), 0.97 - 0.91 (m, 2H), 0.69 - 0.64 (m, 2H). One NH proton not visible.

### Example 648: (1S,2S)-N-(6-((6-cyclopropyl-8-(3-methyl-2,4-dioxoimidazolidin-1-yl)imidazo[1,2-a]pyridin-2-yl)methoxy)pyridazin-4-yl)-2-(4-methylpyrimidin-2-yl)cyclopropane-1-carboxamide (I-648)

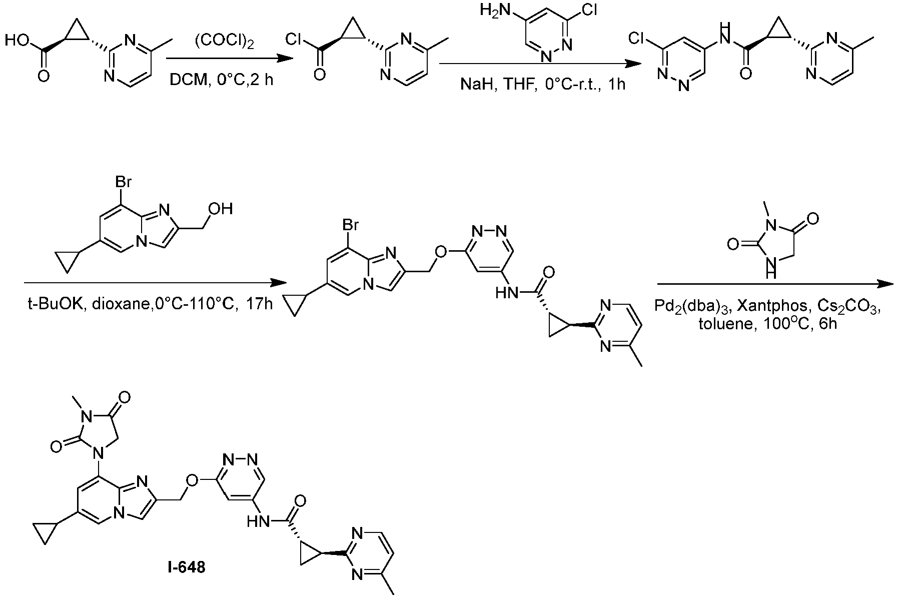

To a solution of (1S,2S)-2-(4-methylpyrimidin-2-yl)cyclopropane-1-carboxylic acid (1.0 g, 5.62 mol) in DCM (20 mL) was added (COCl)₂ (1 mL) at 0 °C, and the mixture was stirred at 0 °C for 2 h. The mixture was concentrated *in vacuo* to give **(1S,2S)-2-(4-methylpyrimidin-2-yl)cyclo propane-1-carbonyl chloride intermediate 1058** (1.1 g, 99%, crude) as a yellow solid which was used directly in the next step. ESI-MS [M +H-35+31]⁺: 193.0.

To a solution of 6-chloropyridazin-4-amine (1.1 g, 8.42 mmol) in THF (15 mL) was added NaH (1.1 g, 28.0 mmol), and the mixture was stirred at 0 °C for 30 min. Then was added **intermediate 1058** (1.1 g, 5.61 mmol) and then the mixture was stirred at 25 °C for 30 min. The reaction was quenched by saturated aq. NH4Cl (100 mL) and extracted with EtOAc (3 x 50 mL). The combined organic layers were washed with brine (50 mL), dried over Na₂SO₄ and concentrated *in vacuo.* The residue was purified by silica gel column chromatography (eluent: DCM: MeOH = 0 ~ 20:1) to give **(1S,2S)-N-(6-chloropyridazin-4-yl)-2-(4-methylpyrimidin-2-yl)cyclopropane-1-carboxamide intermediate 1059** (560 mg, 35%) as yellow solid. ESI-MS [M +H]⁺: 290.1

To a mixture of t-BuOK (651 mg, 5.81 mmol) in 1,4-dioxane (20 mL) was added **intermediate 453** (928 mg, 3.49 mmol) at 0 °C, and the mixture was stirred at room temperature for 1 h. **Intermediate 1059** (560 mg, 1.94 mmol) was added and then stirred at 110 °C for 16 h. After cooling to room temperature, the mixture was quenched by ice water and extracted with EtOAc (3 x 50 mL). The combined organic layers were washed with brine (50 mL), dried over Na₂SO₄ and concentrated in vacuo. The residue was purified by silica gel column chromatography (eluent: DCM: MeOH = 10 : 1) to give **(1S,2S)-N-(6-((8-bromo-6-cyclopropylimidazo[1,2-a]pyridin-2-yl)methoxy)pyridazin-4-yl)-2-(4-methylpyrimidin-2-yl)cyclopropane-1-carboxamide intermediate 1060** (205 mg, yield 20%) as yellow solid. ESI-MS [M +H]⁺: 520.1

To a solution of **intermediate 1060** (100 mg, 0.19 mmol) in toluene (20 mL) was added 3-methylimidazolidine-2,4-dione (110 mg, 0.96 mmol), Pd₂(dba)₃ (35 mg, 0.039 mmol), Cs₂CO₃ (188 mg, 0.58 mmol), and Xantphos (45 mg, 0.077 mmol), and the mixture was stirred at 100 °C for 6 h under N₂. The mixture was quenched with water (50 mL) and extracted with EtOAc (3 x 50 mL). The combined organic layers were washed with brine (50 mL), dried over Na₂SO₄ and concentrated in vacuo. The residue was purified by preparative HPLC to give **(I-648)** (15 mg, 14%) as a white solid. ESI-MS [M +H]+: 554.2.. δ 11.05 (s, 1H), 8.89 (d, J = 2.0 Hz, 1H), 8.54 (d, J = 5.1 Hz, 1H), 8.31 - 8.26 (m, 1H), 7.97 (s, 1H), 7.49 (d, J = 2.0 Hz, 1H), 7.38 (d, J = 1.5 Hz, 1H), 7.23 (d, J = 5.1 Hz, 1H), 5.56 (s, 2H), 4.89 (s, 2H), 2.97 (s, 3H), 2.60 - 2.55 (m, 2H), 2.42 (s, 3H), 2.00 - 1.92 (m, 1H), 1.63 - 1.53 (m, 2H), 0.98 - 0.93 (m, 2H), 0.69 - 0.64 (m, 2H).

### Example 649: (1S,2S)-N-(2-((6-cyclopropyl-8-(3-methyl-2,4-dioxoimidazolidin-1-yl)imidazo[1,2-a]pyridin-2-yl)methyl)pyrazolo[1,5-a]pyrazin-4-yl)-2-(4-methylpyrimidin-2-yl)cyclopropane-1-carboxamide (I-649)

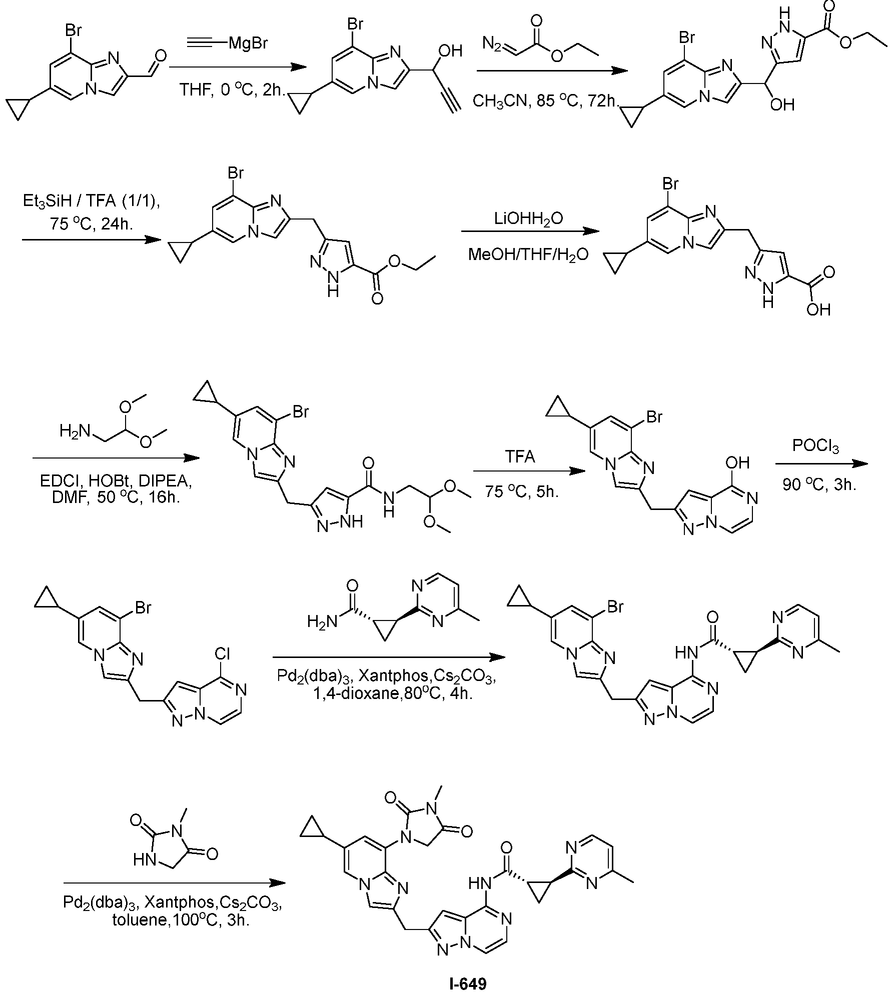

To a mixture of 8-bromo-6-cyclopropylimidazo[1,2-a]pyridine-2-carbaldehyde (1.0 g, 3.78 mmol) in THF (30.0 mL) was added ethynylmagnesium bromide (22.8 mL, 11.4 mmol, 0.5 M in THF) at 0 °C and the mixture was stirred at 0 °C for 2 h under N₂. The reaction was quenched with saturated aq.NH₄Cl (50 mL) and extracted with EtOAc (3 x 50 mL). The combined organic layers were washed with brine (50 mL), dried over anhydrous Na₂SO₄, and concentrated *in vacuo* to give **1-(8-bromo-6-cyclopropylimidazo[1,2-a]pyridin-2-yl)prop-2-yn-1-ol intermediate 1061** (1.1 g, crude) as a yellow solid which was used for the next step directly. ESI-MS [M +H]⁺: 291.2.

A mixture of **intermediate 1061** (1.0 g, 3.45 mmol) and ethyl 2-diazoacetate (1.18 g, 10.34 mmol) in CH₃CN (20 mL) was stirred at 85 °C for 72 h under N₂. The reaction mixture was quenched with water (50 mL) and extracted with EtOAc (3 x 50 mL). The combined organic layers were washed with brine (50 mL), dried over anhydrous Na₂SO₄ and concentrated *in vacuo.* The residue was purified by column chromatography (eluent: DCM/MeOH = 30/1) to give **ethyl** 3-((8-**bromo-6-cyclopropylimidazo[1,2-a]-pyridin-2-yl)(hydroxy)methyl)-1H-pyrazole-5-carboxylate intermediate 1062** (700 mg, 50 %) as a yellow solid. ESI-MS [M +H]⁺: 405.2.

A mixture of **intermediate 1062** (300 mg, 0.74) in Et₃SiH/TFA (5 mL/5 mL) was stirred at 75 °C for 24 h. The reaction was quenched with saturated aq. NaHCO₃ (100 mL) and extracted with EtOAc (3 x 50 mL). The combined organic layers were washed with brine (50 mL), dried over anhydrous Na₂SO₄ and concentrated *in vacuo.* The residue was purified by column chromatography (eluent: DCM/MeOH = 20/1) to give **ethyl 3-((8-bromo-6-cyclopropylimidazo[1,2-a]-pyridin-2-yl)methyl)-*1H*-pyrazole-5-carboxylate intermediate 1063** (190 mg, 66%) as a yellow oil. ESI-MS [M +H]⁺: 389.2.

A mixture of **intermediate 1063** (190 mg, 0.5 mmol) in a solution of MeOH/THF/H2O (5 mL/5 mL/1 mL) was stirred at 60 °C for 5 h. The mixture was acidified to pH=3 with HCl (1N) and concentrated *in vacuo* to give **3-((8-bromo-6-cyclopropylimidazo[1,2-a]pyridin-2-yl)methyl)-1H-pyrazole-5-carboxylic acid intermediate 1064** (200 mg, crude) as a yellow solid which was used for the next step directly. ESI-MS [M +H]⁺: 361.2.

A mixture of **intermediate 1064** (200 mg, 0.5 mmol, crude), 2,2-dimethoxyethan-1-amine (263 mg, 2.5 mmol), EDCI (384 mg, 2.0 mmol), HOBt (270 mg, 2.0 mmol), and DIPEA (387 mg, 3.0 mmol) in DMF (10 mL) was stirred at 50 °C for 16 h. The reaction was quenched with water (100 mL) and extracted with EtOAc (3 x 30 mL). The combined organic layers were washed with brine (30 mL), dried over anhydrous Na₂SO₄ and concentrated *in vacuo* to give the crude, which was purified by column chromatography (eluent: DCM/MeOH = 15/1) to give **3-((8-bromo-6-cyclopropylimidazo[1,2-a]pyridin-2-yl)methyl)-N-(2,2-dimethoxy ethyl)-1H-pyrazole-5-carboxamide intermediate 1065** (140 mg, 63%) as a yellow solid. ESI-MS [M +H]⁺: 448.2.

A mixture of **intermediate 1065** (140 mg, 0.31 mmol) in TFA (5.0 mL) was stirred at 75 °C for 5 h. The reaction was concentrated *in vacuo* to give **2-((8-bromo-6-cyclopropylimidazo[1,2-a]pyridin-2-yl)methyl)-pyrazolo[1,5-a]pyrazin-4-ol intermediate 1066** (110 mg, crude) as a yellow solid which was used for the next step directly. ESI-MS [M +H]⁺: 384.2.

A mixture of **2intermediate 1066** (110 mg, 0.29 mmol, crude) in POCl₃ (5.0 mL) was stirred at 90 °C for 3 h. The reaction was concentrated *in vacuo* to give the crude, which was diluted with EtOAc (50 mL) and quenched with saturated aq. NaHCO₃ (100 mL). The mixture was extracted with EtOAc (3 x 30 mL). The combined organic layers were washed with brine (30 mL), dried over anhydrous Na₂SO₄, concentrated *in vacuo* to give the crude, & purified by preparative TLC (eluent: DCM/MeOH = 15/1) to give **2-((8-bromo-6-cyclopropylimidazo-[1,2-a]pyridin-2-yl)methyl)-4-chloropyrazolo[1,5-a]pyrazine intermediate 1067** (85 mg, 74%) as a white solid. ESI-MS [M +H]⁺: 402.1.

To a mixture of **intermediate 1067** (85 mg, 0.21 mmol), (1S,2S)-2-(4-methylpyrimidin-2-yl)-cyclopropane-1-carboxamide (41 mg, 0.23 mmol) and Cs₂CO₃ (205 mg, 0.63 mmol) in 1,4-dioxane (10 mL) were added Pd₂(dba)₃ (19 mg, 0.021 mmol) and Xantphos (24 mg, 0.042 mmol). After stirring at 80 °C for 4 h under N₂, the reaction mixture was filtered through Celite^{®} and the filter cake was washed with DCM/MeOH (10/1, 30 mL). The filtrate was concentrated to give the crude, which was purified by preparative TLC (eluent: DCM/MeOH = 15/1) to give **(1S,2S)-N-(2-**((8-bromo-6-cyclopropylimidazo[1,2-a]pyridin-2-yl)methyl)pyrazolo[1,5-a]pyrazin-4-yl)-2-(4-**methylpyrimidin-2-yl)cyclopropane-1-carboxamide intermediate 1068** (55 mg, 48%) as a yellow solid. ESI-MS [M +H]⁺: 545.2.

To a mixture of **(intermediate 1068** (54 mg, 0.1 mmol), 3-methylimidazolidine-2,4-dione (57 mg, 0.5 mmol), and Cs₂CO₃ (98 mg, 0.3 mmol) in 1,4-dioxane (5 mL) were added Pd₂(dba)₃ (18 mg, 0.02 mmol) and Xantphos (23 mg, 0.04 mmol). After stirring at 100 °C for 3 h under N₂, the reaction mixture was filtered through Celite^{®} and the filter cake was washed with DCM/MeOH (10/1, 30 mL). The filtrate was concentrated in vacuo to give the crude, which was purified by preparative HPLC to give **(I-649)** (5 mg, 9%) as a white solid. ESI-MS [M +H]⁺: 577.2. δ 10.96 (s, 1H), 8.53 (d, J = 5.1 Hz, 1H), 8.49 (d, J = 4.8 Hz, 1H), 8.23 (s, 1H), 7.69 (s, 1H), 7.57 (d, J = 4.5 Hz, 1H), 7.33 (s, 1H), 7.22 (d, J = 4.9 Hz, 1H), 6.80 (s, 1H), 4.89 (s, 2H), 4.25 (s, 2H), 2.96 (s, 3H), 2.71 - 2.67 (m, 2H), 2.42 (s, 3H), 1.99 - 1.88 (m, 1H), 1.59 - 1.55 (m, 2H), 0.99 - 0.87 (m, 2H), 0.66 - 0.62 (m, 2H).

### Example 650: (1S,2S)-N-(6-((1-(6-cyclopropyl-8-(3-methyl-2,4-dioxoimidazolidin-1-yl)imidazo [1,2-a]pyridin-2-yl)-2-hydroxyethyl)amino)-2-methylpyrimidin-4-yl)-2-(4-methylpyrimidin-2-yl)cyclopropane-1-carboxamide (I-650)

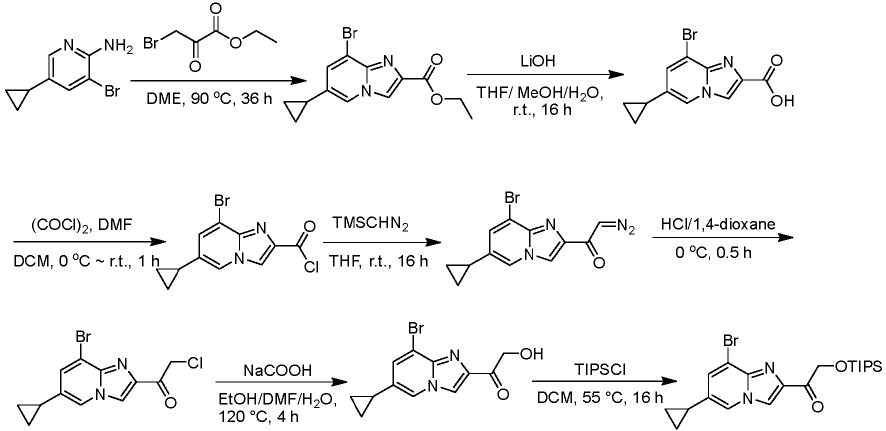

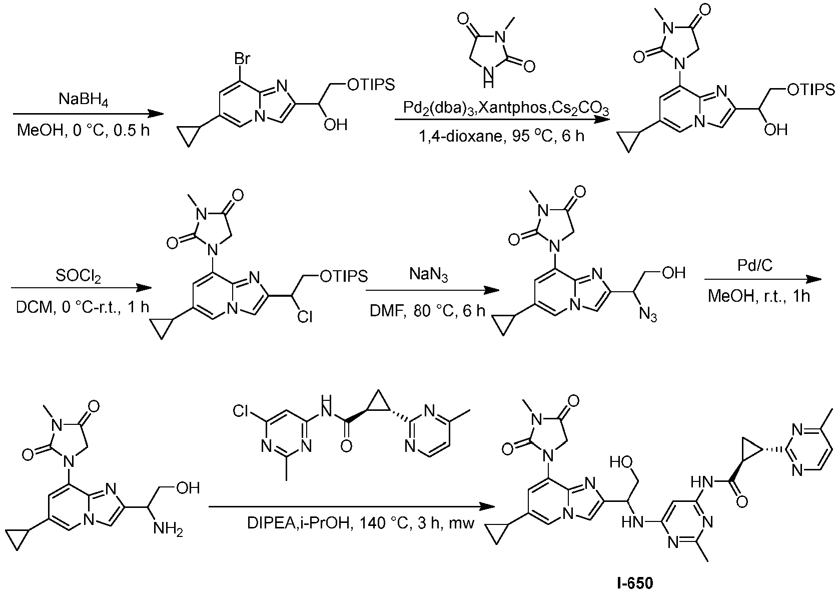

A mixture of **intermediate 452** (4 g, 12.9 mmol) and LiOH-H₂O (1.09 g, 25.8 mmol) in THF /MeOH/H₂O (40 mL/40 mL/16 mL) was stirred at room temperature for 16 h under N₂ . The reaction mixture was diluted with water (50 mL) and extracted with EtOAc (3 x 50 mL). The aqueous phase was adjusted to pH=4 with HCl (1N) and filtered. The solid was dried to afford **8-bromo-6-cyclopropylimidazo[1,2-a]pyridine-2-carboxylic acid intermediate 1069** (3.0 g, 83% ) as yellow solid. ESI-MS [M +H]+:281.0. pH~3

To a mixture of **intermediate 1069** (2.0 g, 7.1 mmol) in DCM (50 mL) was added oxalyl chloride (1.1 g, 8.5 mmol) and DMF (2 drops) at 0 °C. The mixture was stirred at room temperature for 1 h and then concentrated to afford **8-bromo-6-cyclopropylimidazo[1,2-a]pyridine-2-carbonyl chloride intermediate 1070** (2.1 g, quant ) as yellow oil which was used in next step directly. ESI-MS [M +H]+:295.0.

To a mixture of **intermediate 1070** (2.1 g, 7.1 mmol) in THF (50 mL) was added TMSCHN₂ (7.1 mL, 14 mmol) at 0 °C .The mixture was stirred at room temperature for 16 h and then concentrated *in vacuo* to afford **1-(8-bromo-6-cyclopropylimidazo[1,2-a]pyridin-2-yl)-2-diazoethan-1-one intermediate 1071** (2.2 g, quant) as yellow oil which was used in next step directly. ESI-MS [M +H]+:305.0.

To a mixture of **intermediate 1071** (2.1 g, 7.1 mmol) in THF (50 mL) was added HCl (2.1 mL, 4.0 M) at 0 °C .The mixture was stirred at 0 °C for 0.5 h . The reaction mixture was quenched with water (100 mL) and extracted with EtOAc (3 x 50 mL). The combined organic layers were washed with brine (50 mL), dried over Na₂SO₄ and concentrated. The residue was purified by column chromatography (eluent: EtOAc/PE, 0-50%) to afford **1-(8-bromo-6-cyclopropylimidazo[1,2-a]pyridin-2-yl)-2-chloroethan-1-one intermediate 1072** (1.0 g, 47%, for three steps) as yellow solid. ESI-MS [M +H]+:313.0.

A mixture of **intermediate 1072** (1.1 g, 3.5 mmol) and HCOONa (0.72 g, 11 mmol) in EtOH/DMF/water (28 mL/10 mL/8 mL) was stirred at 120 °C for 4 h under N₂. After cooling to room temperature, water (40 mL) was added and the mixture was extracted by EtOAc (3 x 40 mL). The combined organic layers were washed with brine (50 mL), dried over Na₂SO₄ and concentrated *in vacuo.* The residue was purified by column chromatography (eluent: EtOAc /PE = 0 - 50%) to afford **1-(8-bromo-6-cyclopropylimidazo[1,2-a]pyridin-2-yl)-2-hydroxyethan-1-one intermediate 1073** (0.54 g, 52 %) as pale solid. ESI-MS [M +H]+:295.1.

To a mixture of **intermediate 1073** (0.54 g, 1.8 mmol) and imidazole (0.87 g, 13 mmol) in DCM (30 mL) was added TIPSCl (1.8 g, 9.2 mmol) . After stirring at 55 °C for 16 h under N₂, the reaction mixture was quenched with H2O (100 mL) and extracted with DCM (3 x 30 mL). The combined organic layers were washed with brine (100 mL), dried over Na₂SO₄ and concentrated *in vacuo.* The residue was purified by column chromatography (eluent: EtOAc/PE from 0 to 50%) to afford **1-(8-bromo-6-cyclopropylimidazo[1,2-a]pyridin-2-yl)-2-((triisopropylsilyl)oxy)ethan-1-one intermediate 1074** (0.69 g, 84 %) as pale solid. ESI-MS [M +H]+:451.1.

To a mixture of **intermediate 1074** (0.69g, 1.5mmol) in MeOH (20 mL) was added NaBH₄ (0.29mg, 7.7 mmol) at 0°C. After stirring at 0 °C for 0.5 h under N₂, the reaction mixture was quenched by H2O (30mL) and extracted with DCM (3x30mL). The combined organic layers were washed with brine (30mL), dried over Na₂SO₄ and concentrated *in vacuo.* The residue was purified by column chromatography (eluent: EtOAc/PE from 0-50%) to afford **1-(8-bromo-6-cyclopropyl imidazo[1,2-a]pyridin-2-yl)-2-((triisopropylsilyl)oxy)ethan-1-ol intermediate 1075** (0.65g, 94%) as pale solid. ESI-MS [M+H]+:453.1.

To a mixture of **intermediate 1075** (0.65 g, 1.4 mmol) , 3-methyl imidazolidine-2,4-dione (0.49 g, 4.3 mmol), and Cs₂CO₃ (1.4 g, 4.3 mmol) in 1,4-dioxane (40mL) were added Pd₂(dba)₃ (0.26g, 0.29mmol) and Xantphos (0.55 mg, 0.57 mmol). After stirring at 95°C for 6 h under N₂, the reaction mixture was filtered through Celite^{®} and the filter cake was washed with DCM/MeOH (10/1, 100 mL). The filtrate was concentrated in vacuo to give the crude which was purified by column chromatography (eluent: EtOAc/PE from 0 to 50%) to afford **1-(6-cyclo propyl-2-(1-hydroxy-2-((triisopropylsilyl) oxy)ethyl)imidazo[1,2-a]pyridin-8-yl)-3-methyl imidazolidine-2,4-dione intermediate 1076** (0.82 g, quant) as yellow oil. ESI-MS [M +H]+:487.3.

To a mixture of **intermediate 1076** (0.82 g, 1.7 mmol) in DCM (20 mL) was added SOCl₂ (0.40 g, 3.4 mmol) at 0 °C. The mixture was stirred at room temperature for 1 h under N₂ and then concentrated in vacuo to afford **1-(2-(1-chloro-2-((triisopropylsilyl)oxy)ethyl)-6-cyclopropylimidazo[1,2-a]pyridin-8-yl)-3-methylimidazolidine-2,4-dione intermediate 1077** (0.85 g, quant) as yellow oil which was used in next step directly. ESI-MS [M +H]+: 505.2.

To a mixture of **intermediate 1077** (0.85 g, 1.7 mmol) in DMF (20 mL) was added NaN₃ (0.55 g, 8.4 mmol). After stirring at 80 °C for 6 h under N₂, the reaction mixture was quenched with water (200 mL) and extracted with EtOAc (3 x 60 mL). The combined organic layers were washed with brine (60 mL), dried over Na₂SO₄ and concentrated *in vacuo.* The residue was purified by column chromatography (eluent: EtOAc/PE from 0 to 50%) to afford **1-(2-(1-azido-2-hydroxyethyl)-6-cyclopropylimidazo[1,2-a]pyridin-8-yl)-3-methylimidazolidine-2,4-dione intermediate 1078** (0.18 g, 24% for two steps) as yellow solid. ESI-MS [M +H]+:356.2.

A mixture of **intermediate 1078** (0.21 g, 0.59 mmol) and Pd/C (0.21 g) in MeOH (10 mL) was stirred at RT for 1 h. The reaction mixture was filtered through Celite^{®} and the filter cake was washed with MeOH (10 mL). The filtrate was concentrated *in vacuo* to afford **1-(2-(1-amino-2-hydroxyethyl)-6-cyclopropylimidazo[1,2-a]pyridin-8-yl)-3-methylimidazolidine-2,4-dione intermediate 1079** (170 mg, crude) as yellow oil which was used in next step directly. ESI-MS [M +H]+:330.1.

A mixture of **intermediate 1079** (88mg, 0.27mmol), (1S,2S)-N-(6-chloro-2-methylpyrimidin-4-yl)-2-(4-methylpyrimidin-2-yl)cyclopropane-1-carbox amide (81mg, 0.27mmol), and DIPEA (0.10g, 0.81mmol) in IPA (3 mL) in a sealed tube was degassed with N₂ for 1 min. After heating in a microwave reactor at 140 °C for 3 h, the mixture quenched with H2O (50 mL) and extracted with EtOAc (3x50 mL). The combined organic layers were washed with brine (50mL), dried over Na₂SO₄ and concentrated in vacuo. The residue was purified by preparative TLC (eluent: DCM:MeOH = 20:1) to afford **(I-650)** (21mg, 13%) as pale solid. ESI-MS [M+H]+ :597.3. δ 10.64 (s, 1H), 8.52 (d, J = 5.1 Hz, 1H), 8.24 (s,1H), 7.69 (d, J = 2.7 Hz,1H), 7.61 (s,1H), 7.34 (s,1H), 7.21 - 7.16 (m,2H), 5.39 (s,1H), 4.96 - 4.93 (m,2H), 4.82 (s, 1H), 3.86 - 3.81 (m, 1H), 3.74 - 3.68 (m, 1H), 2.98 (d, J = 1.2 Hz,3H), 2.57 - 2.56 (m,2H), 2.41 (s,3H), 2.24 (s,3H), 1.95 - 1.92 (m,1H), 1.53 - 1.46 (m,2H), 0.94 - 0.91 (m,2H), 0.66 - 0.62 (m, H).

### Example 651: (1S,2S)-N-(5-(((6-cyclopropyl-8-(3-methyl-2,4-dioxoimidazolidin-1-yl)imidazo[1,2-a] pyridin-2-yl)methyl)amino)-1,2,4-triazin-3-yl)-2-(4-methylpyrimidin-2-yl)cyclopropane-1-carboxamide (I-651)

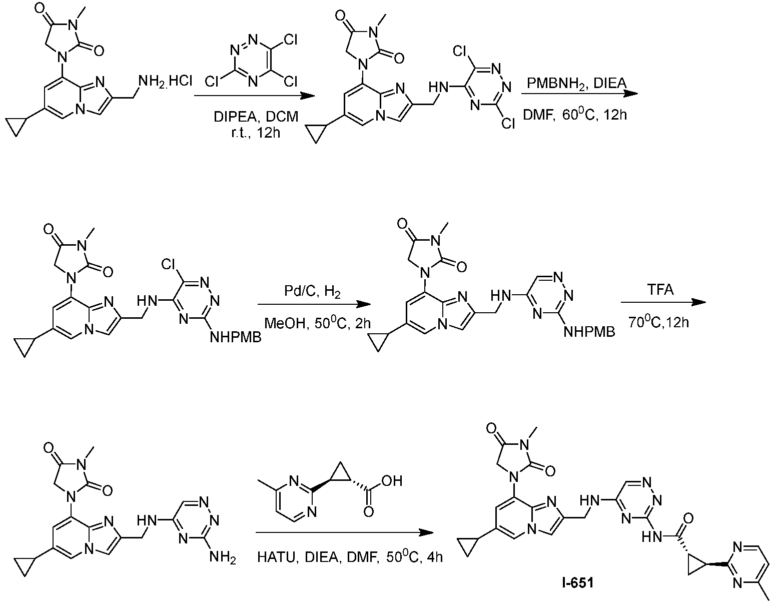

A mixture of **intermediate 365** hydrochloride (300 mg, 0.89 mmol), 3,5,6-trichloro-1,2,4-triazine (197 mg, 1.07 mmol), and DIPEA (344 mg, 2.67 mmol) in DCM (6 mL) was stirred at room temperature for 12 h. The mixture quenched with saturated aq. NaHCO₃ (100 mL) and extracted with EtOAc (3 x 50 mL). The combined organic layers were washed with brine (50 mL), dried over Na₂SO₄ and concentrated in vacuo. The residue was purified by preparative TLC (eluent: DCM/MeOH = 20/1) to give **1-(6-cyclopropyl-2-(((3,6-dichloro-1,2,4-triazin-5-yl)amino)methyl)imidazo[1,2-a]pyridin-8-yl)-3-methylimidazolidine-2,4-dione intermediate** 1080 (300 mg, 70%) as a yellow solid. ESI-MS [M +H]+: 447.2.

To a mixture of **intermediate 1080** (300 mg, 0.67 mmol), DIPEA (258 mg, 2.0 mmol) in DMF (5 mL) was added PMBNH₂(275.7 mg, 2.0 mmol). The reaction mixture was stirred at 60 °C for 12 h under N₂. Water (50 mL) was added and the mixture was extracted with EtOAc (30 mL x 3). The combined organic layers were washed with brine (30 mL), dried over Na₂SO₄ and concentrated in vacuo to give the crude, which was purified by preparative TLC(eluent: DCM/MeOH=20/1) to give **1-(2-(((6-chloro-3-((4-methoxybenzyl)amino)-1,2,4-triazin-5-yl)amino)methyl)-6-cyclopropylimidazo[1,2-a]pyridin-8-yl)-3-methylimidazolidine-2,4-dione intermediate 1081** (300 mg, 82%) as a yellow solid. ESI-MS [M +H]+: 584.2

A mixture of **intermediate 1081** (200 mg, 0.36 mmol) and Pd/C(100 mg) in MeOH (5 mL) was stirred at 50 °C for 2 h under H₂. The reaction mixture was filtered through Celite^{®} and the filter cake was washed with DCM/MeOH (10/1, 50 mL). The filtrate was concentrated to give the crude, which was purified by preparative TLC (eluent: DCM / MeOH = 20 / 1) to give **1-(6-cyclopropyl-2-(((3-((4-methoxybenzyl)amino)-1,2,4-triazin-5-yl)amino)methyl)imidazo[1,2-a]pyridin-8-yl)-3-methylimidazolidine-2,4-dione intermediate 1082** (150 mg, 80%) as a yellow solid. ESI-MS [M +H]+: 514.2

A mixture of **intermediate 1082** (300 mg, 0.58 mmol) in TFA (4 ml) was stirred at 70 ⁰C for 12 h. The mixture was quenched with saturated aq. NaHCO₃ (50 mL) and extracted with EtOAc (3 x 30 mL). The combined organic layers were washed with brine (30 mL), dried over anhydrous Na₂SO₄, and concentrated in vacuo to give the crude product, which was purified by preparative TLC (eluent: DCM/MeOH = 10/1) to give **1-(2-(((3-amino-1,2,4-triazin-5-yl)amino)methyl)-6-cyclopropylimidazo[1,2-a]pyridin-8-yl)-3-methylimidazolidine-2,4-dione intermediate 1083** (140 mg, 61%) as a white solid. ESI-MS [M +H]+: 394.2.

A mixture of **intermediate 1083** (140 mg, 0.35 mmol), (1S,2S)-2-(4-methylpyrimidin-2-yl)cyclopropane-1-carboxylic acid (70 mg, 0.39 mmol), HATU (200 mg, 0.52 mmol), and DIPEA (135 mg, 1.05 mmol) in DMF (4 mL) was stirred at 50 °C for 4 h. Water (50 mL) was added and extracted with EtOAc (3 x 30 mL). The combined organic layers were washed with brine (30 mL), dried over Na₂SO₄, filtered and concentrated in vacuo to give the crude, which was purified by preparative TLC(eluent: DCM / MeOH = 20 / 1 ) to give **(I-561)** (95 mg, 50%) as a yellow solid. ESI-MS [M +H]+: 554.2 δ 10.63 (s, 1H), 8.65 (s, 1H), 8.48 (d, J = 5.1 Hz, 1H), 8.33 (s, 1H), 8.23 (d, J = 8.6 Hz, 1H), 7.83 (s, 1H), 7.34 (s, 1H), 7.15 (d, J = 5.1 Hz, 1H), 4.89 (s, 2H), 4.54 (d, J = 5.2 Hz, 2H), 2.97 (s, 3H), 2.69 - 2.59 (m, 2H), 2.38 (s, 3H), 1.98-1.90 (m, 1H), 1.59 - 1.47 (m, 2H), 0.98-0.90 (m, 2H), 0.70 - 0.60 (m, 2H).

### Example 652: (1S,2S)-N-(2-((6-cyclopropyl-8-(3-methyl-2,4-dioxoimidazolidin-1-yl)imidazo[1,2-a]pyridin-2-yl)methoxy)-6-methylpyrimidin-4-yl)-2-(4-methylpyrimidin-2-yl)cyclopropane-1-carboxamide (I-652)

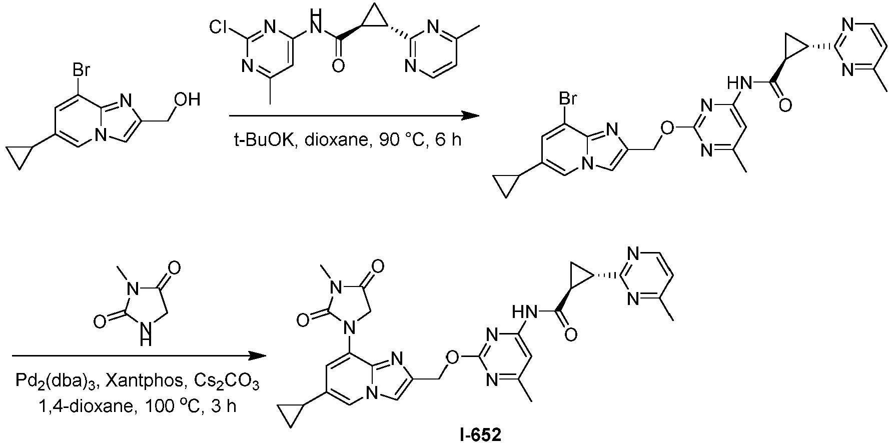

To a mixture of intermediate 453 (0.12 g, 0.44 mmol) and (1S,2S)-N-(2-chloro-6-methylpyrimidin-4-yl)-2-(4-methylpyrimidin-2-yl)cyclopropane-1-carboxamide (0.15 g, 0.51 mmol) in 1,4-dioxane (4.0 mL) was added t-BuOK (0.88 mL, 0.88 mmol, 1M in THF). The mixture was stirred at 90 °C for 6 h and cooled to room temperature. The reaction was quenched with saturated aq. NH₄Cl (50 mL) and extracted with EtOAc (3 x 50 mL). The combined organic layers were washed with brine (50 mL), dried over Na₂SO₄ and concentrated in vacuo. The residue was purified by flash column chromatography (eluent: MeOH/DCM from 0 to 5%) to afford **(1S,2S)-N-(2-((8-bromo-6-cyclopropylimidazo[1,2-a]pyridin-2-yl)methoxy)-6-methylpyrimidin-4-yl)-2-(4-methylpyrimidin-2-yl)cyclopropane-1-carboxamide intermediate 1084** (0.12 mg, yield 49%) as white solid. ESI-MS [M +H]+: 534.1

A mixture of **(intermediate 1084** (60 mg, 0.11 mmol), 3-methylimidazolidine-2,4-dione (64 mg, 0.56 mmol), Pd₂(dba)₃ (31 mg, 0.034 mmol), Xantphos (39 mg, 0.067 mmol), and Cs₂CO₃ (0.11 g, 0.34 mmol) in 1,4-dioxane (4.0 mL) was stirred at 100 °C for 3 h under N₂. The mixture was filtered through Celite^{®} and the filter cake was washed with DCM/MeOH (10/1, 100 mL). The filtrate was concentrated in vacuo to give the crude, which was purified by preparative TLC (eluent: 100% EtOAc) to afford **(I-652)** (21.2 mg, yield 33%) as white solid. ESI-MS [M +H]+: 568.2. δ 11.11 (s, 1H), 8.53 (d, J = 5.1 Hz, 1H), 8.28 (d, J = 1.3 Hz, 1H), 7.92 (s, 1H), 7.63 (s, 1H), 7.37 (d, J = 1.4 Hz, 1H), 7.21 (d, J = 5.1 Hz, 1H), 5.42 (s, 2H), 4.88 (s, 2H), 2.96 (s, 3H), 2.59 - 2.54 (m, 2H), 2.42 (s, 3H), 2.37 (s, 3H), 1.97 - 1.93 (m, 1H), 1.59 - 1.53 (m, 2H), 0.96 - 0.92 (m, 2H), 0.67 - 0.64 (m, 2H).

### Example 653: (1S,2S)-N-(4-(2-(6-cyclopropyl-8-(3-methyl-2,4-dioxoimidazolidin-1-yl)imidazo[1,2-a]pyridin-2-yl)ethyl)pyridin-2-yl)-2-(4-methylpyrimidin-2-yl)cyclopropane-1-carboxamide (I-653)

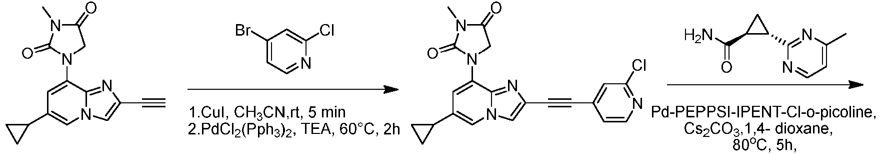

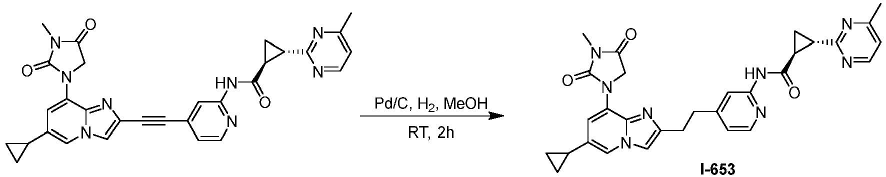

To a mixture of **intermediate 1011** (100 mg, 0.3 mmol) in MeCN (20 mL) was added 4-bromo-2-chloropyridine (98 mg, 0.45 mmol) andCuI (10 mg, 0.9 mmol) at room temperature for 5 min, followed by PdCl₂(PPh3)2 (40 mg, 0.06 mmol), TEA (1mL). After stirring at 60 °C for 2 h, the reaction mixture was filtered through Celite^{®} and the filter cake was washed with DCM/MeOH (10/1, 30 mL). The filtrate was concentrated *in vacuo* to give the crude, which was purified by preparative TLC (eluent: DCM/MeOH = 20/1) to **1-(2-((2-chloropyridin-4-yl)ethynyl)-6-cyclo propylimidazo[1,2-a]pyridin-8-yl)-3-methylimidazolidine-2,4-dione intermediate 1085** (80 mg, 65%) as a yellow solid. ESI-MS [M +H]+: 406.2.

To a mixture of **intermediate 1085** (80 mg, 0.19 mmol), **intermediate 55** (34 mg, 0.19 mmol), and Cs₂CO₃ (185 mg, 0.57 mmol) in 1,4-dioxane (10 mL) was added Pd-PEPPSI-IPENT-Cl-o-picoline (16 mg, 0.02 mmol). The reaction mixture was stirred at 80 °C for 5h under N₂. The reaction mixture was filtered through Celite^{®} and the filter cake was washed with DCM/MeOH (10/1, 50 mL). The filtrate was concentrated in vacuo to give the crude, which was purified by preparative HPLC to give **(1S,2S)-N-(4-((6-cyclopropyl-8-(3-methyl-2,4-dioxoimidazolidin-1-yl)imidazo[1,2-a]pyridin-2-yl)ethynyl)pyridin-2-yl)-2-(4-methylpyrimidin-2-yl)cyclopropane-1-carboxamide intermediate 1086** (22 mg, 21%) as a white solid. ESI-MS [M +H]⁺: 547.1.

A mixture of **intermediate 1086**(22 mg, 0.04 mmol) and Pd/C (20 mg) in MeOH(5 mL) was stirred at room temperature for 2 h under H₂. The reaction mixture was filtered through Celite^{®} and the filter cake was washed with DCM/MeOH (10/1, 20 mL). The filtrate was concentrated in vacuo to give the crude, which was purified by preparative HPLC to give **(I-653)** (18 mg, 81%) as a white solid. ESI-MS: [M +H]+, 551.2. δ 10.75 (s, 1H), 8.53 (d, J = 5.1 Hz, 1H), 8.20 (d, J = 1.1 Hz, 1H), 8.15 (d, J = 5.0 Hz, 1H), 8.01 (s, 1H), 7.62 (s, 1H), 7.31 (d, J = 1.5 Hz, 1H), 7.21 (d, J = 5.1 Hz, 1H), 6.99 (dd, J = 5.1, 1.4 Hz, 1H), 4.92 (s, 2H), 3.00 (s, 4H), 2.98 (s, 3H), 2.68 - 2.61 (m, 1H), 2.56 - 2.53 (m, 1H), 2.42 (s, 3H), 1.97 - 1.92(m, 1H), 1.53 - 1.47 (m, 2H), 0.96 - 0.91 (m, 2H), 0.67 - 0.63 (m, 2H).

### Example 654: Ethyl 4-((6-cyclopropyl-8-(3-methyl-2,4-dioxoimidazolidin-1-yl)imidazo[1,2-a]pyridin-2-yl)methoxy)-2-((1S,2S)-2-(4-methylpyrimidin-2-yl)cyclopropane-1-carboxamido) pyrimidine-5-carboxylate (I-654)

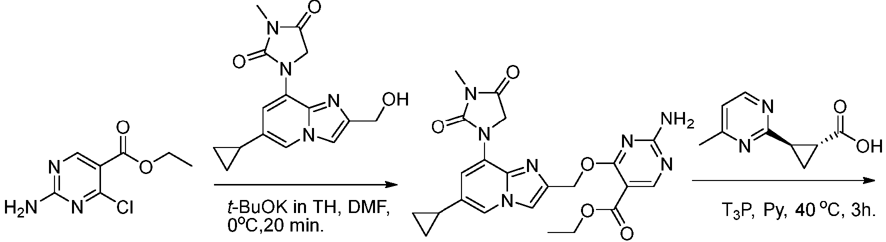

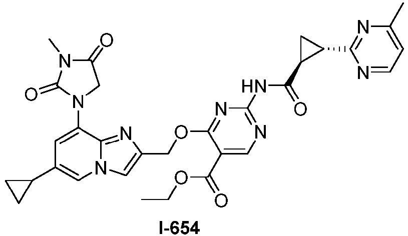

To a solution of **intermediate 454** (140 mg, 0.47 mmol) and ethyl 2-amino-4-chloropyrimidine-5-carboxylate (95 mg, 0.47 mmol) in DMF (10 mL) was added t-BuOK (1M in THF, 0.94 mL) at 0 °C. After stirring at 0 °C for 20 min, the mixture was quenched with water (10 mL) and extracted with EtOAc (3 x 20 mL). The combined organic layers were washed with brine (30 mL), dried over Na₂SO₄, and concentrated in vacuo to give the crude product, which was purified with Pre-TLC (eluent: DCM /MeOH = 15/1) to give **ethyl 2-amino-4-((6-cyclopropyl-8-(3-methyl-2,4-dioxoimidazolidin-1-yl)imidazo[1,2-a]pyridin-2-yl)methoxy)pyrimidine-5-carboxylate 1087** (40 mg, 18%) as a white solid. ESI-MS [M +H]+: 466.1

To a mixture of **intermediate 1087** (30 mg, 0.06 mmol) and (1R,2R)-2-(4-methylpyrimidin-2-yl)cyclopropane-1-carboxylic acid (12 mg, 0.066 mg) in pyridine (1.5 mL) was added T₃P (305 mg, 0.48 mmol). After stirring at 40 °C for 3 h, the mixture was quenched with saturated aq. NaHCO₃ solution (10 mL) and extracted with EtOAc (3 x 20 mL). The combined organic layers were washed with brine (30 mL), dried over Na₂SO₄, and concentrated in vacuo to give the crude product, which was purified with Pre-TLC (DCM/MeOH=20/1) to give **(I-654)** (10 mg, 26%) as a white solid. ESI-MS [M +H]+: 626.2 δ 11.27 (s, 1H), 8.83 (s, 1H), 8.50 (d, J = 5.1 Hz, 1H), 8.31 (d, J = 1.2 Hz, 1H), 8.00 (s, 1H), 7.38 (d, J = 1.4 Hz, 1H), 7.17 (d, J = 5.1 Hz, 1H), 5.55 (s, 2H), 4.88 (s, 2H), 4.27 - 4.16 (m, 2H), 2.97 (s, 3H), 2.65 - 2.60 (m, 1H), 2.39 (s, 3H), 2.02 - 1.93 (m, 1H), 1.61 (t, J = 7.4 Hz, 2H), 1.29 - 1.21 (m, 4H), 1.00 - 0.92 (m, 2H), 0.71 - 0.63 (m, 2H).

### Intermediate 1088: 3,5-diaminobenzonitrile

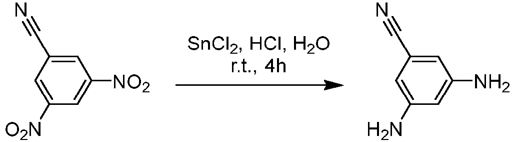

A mixture of 3,5-dinitrobenzonitrile (2 g, 10.4 mmol) and SnCl₂ (1.89 g, 10.4 mmol) in HCl (2M in water, 10 mL) was stirred at room temperature for 4 h under N₂. The reaction was quenched with water (100 mL) and extracted with EtOAc (3 x 100 mL). The combined organic layers were washed with brine (100 mL), dried over anhydrous Na₂SO₄, and concentrated *in vacuo* to give the crude product, which was purified by column chromatography (eluent: DCM/MeOH = 20/1) to give **intermediate 1088** (280 mg, 20%) as a yellow solid. ESI-MS [M +H]⁺: 134.2.

### Intermediate 1089: (1S,2S)-N-(3-amino-5-cyanophenyl)-2-(4-methylpyrimidin-2-yl)cyclopropane-1-carboxamide

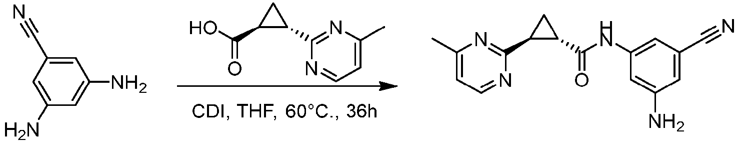

A mixture of **intermediate 1088** (280 mg, 2.1 mmol), (1S,2S)-2-(4-methylpyrimidin-2-yl)cyclopropane-1-carboxylic acid (269 mg, 2.1 mmol) and CDI (340 mg, 2.1 mmol) in THF (10 mL) was stirred at 60 °C for 36 h under N₂. The reaction was quenched with water (50 mL) and extracted with EtOAc (3 x 50 mL). The combined organic layers were washed with brine (50 mL), dried over anhydrous Na₂SO₄, and concentrated *in vacuo* to give the crude product, which was purified by preparative TLC (eluent: DCM/MeOH = 20/1) to give **intermediate 1089** (300 mg, 49%) as a yellow solid. ESI-MS [M +H]⁺: 294.2.

### Example 655: (1S,2S)-N-(3-cyano-5-(((6-cyclopropyl-8-(3-methyl-2,4-dioxoimidazolidin-1-yl) imidazo[1,2-a]pyridin-2-yl)methyl)amino)phenyl)-2-(4-methylpyrimidin-2-yl)cyclopropane-1-carboxamide (I-655)

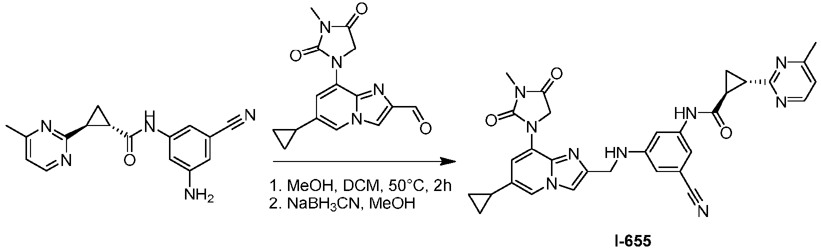

A mixture of **intermediate 1089** (70 mg, 0.24 mmol), **intermediate 1053** (72.0 mg, 0.24mmol), and Ti(O-*i*Pr)₄ (710 mg, 2.5 mmol) in a solution of MeOH/DCM (2.0 mL/10.0 mL) was stirred at 50 °C for 2 h under N₂. After cooling to 0 °C, NaBH₃CN (47 mg, 0.72 mmol) in MeOH (3.0 mL) was added to the mixture which was then stirred at room temperature for 1 h. The reaction was quenched with water (10 mL) and extracted with EtOAc (3 x 10 mL). The combined organic layers were washed with brine (20 mL), dried over anhydrous Na₂SO₄, then concentrated *in vacuo* to give the crude product, which was purified by preparative TLC (eluent: DCM/MeOH = 15/1) to give **(I-655)** (40 mg, 29%) as a light yellow solid. ESI-MS [M +H]+: 576.2. δ 10.39 (s, 1H), 8.52 (d, J = 5.1 Hz, 1H), 8.25 (s, 1H), 7.71 (s, 1H), 7.33 (d, J = 1.3 Hz, 1H), 7.21 - 7.20 (m, 2H), 7.16 (s, 1H), 6.82 - 6.79 (m, 1H), 6.69 (s, 1H), 4.90 (s, 2H), 4.35 (d, J = 5.8 Hz, 2H), 2.97 (s, 3H), 2.55 - 2.52 (m, 2H), 2.41 (s, 3H), 1.95 - 1.91 (m, 1H), 1.52 - 1.46 (m, 2H), 0.96 - 0.91 (m, 2H), 0.66 - 0.62 (m, 2H).

### Example 656: (1S,2S)-N-(3-(((6-cyclopropyl-8-(3-methyl-2,4-dioxoimidazolidin-1-yl)imidazo[1,2-a]pyridin-2-yl)methyl)amino)-4-fluorophenyl)-2-(4-methylpyrimidin-2-yl)cyclopropane-1-carboxamide (I-656)

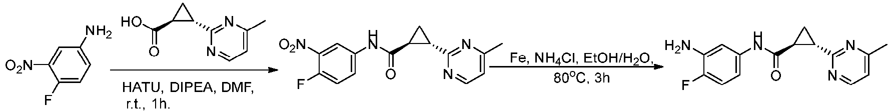

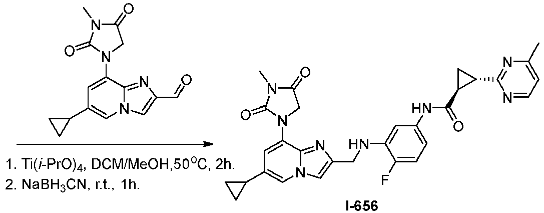

A mixture of 4-fluoro-3-nitroaniline (262 mg, 1.68 mmol), (1S,2S)-2-(4-methylpyrimidin-2-yl)cyclopropane-1-carboxylic acid (300 mg, 1.68 mmol), HATU (958 mg, 2.52 mmol), and DIPEA (720 mg, 5.58 mmol) in DMF (15 mL) was stirred at room temperature for 1 h. The mixture was quenched with water (50 mL) and extracted with EtOAc (3 x 50 mL). The combined organic layers were washed with brine (50 mL), dried over Na₂SO₄ and concentrated *in vacuo* to give the crude product, which was purified by column chromatography (eluent: PE/EA = 1/2) to give the product **(1S,2S)-N-(4-fluoro-3-nitrophenyl)-2-(4-methylpyrimidin-2-yl)cyclopropane-1-carboxamide intermediate 1090** (500 mg, 93%) as a yellow solid. ESI-MS [M +H]+: 317.1

To a mixture of **intermediate 1090** (316 mg, 1.0 mmol) and NH₄Cl (64 mg, 1.2 mmol) in EtOH/H2O (10 mL/1.0 mL) was added Fe powder (336 mg, 6 mmol). The mixture was stirred at 80 °C for 3h and then concentrated *in vacuo* to give the crude product, which was purified with preparative TLC (eluent: DCM/MeOH =15/1) to give the product **(1S,2S)-N-(3-amino-4-fluorophenyl)-2-(4-methylpyrimidin-2-yl)cyclopropane-1-carboxamide intermediate 1091** (125 mg, 44%) as a yellow solid. ESI-MS [M +H]+: 287.1

To a solution of **intermediate 1091** (86 mg, 0.3 mmol) and **intermediate 1053** (90 mg, 0.3 mmol) in DCM/MeOH (4 mL/4mL) was added Ti(i-PrO)₄ (426 mg, 1.5 mmol). The mixture was stirred at 50 °C for 2 h, then after cooling to room temperature, NaBH₃CN (57 mg, 0.9 mmol) was added. Then the mixture was stirred at room temperature for 1 h. The reaction was quenched with water (20 mL) and the aqueous phase was extracted with DCM (3 x 30 mL). The combined organic layers were washed with brine (50 mL), dried over Na₂SO₄, concentrated in vacuo to give the crude product, which was purified by preparative HPLC to give the product **(I-656)** (66 mg, 39%) as a yellow solid. ESI-MS [M +H]+: 569.2 δ 10.08 (s, 1H), 8.50 (d, J = 5.1 Hz, 1H), 8.23 (d, J = 1.0 Hz, 1H), 7.66 (s, 1H), 7.33 (d, J = 1.4 Hz, 1H), 7.18 (d, J = 5.1 Hz, 1H), 7.00 (dd, J = 8.2, 2.2 Hz, 1H), 6.98 - 6.87 (m, 1H), 6.86 - 6.77 (m, 1H), 6.07 (t, J = 5.1 Hz, 1H), 4.92 (s, 2H), 4.37 (d, J = 5.9 Hz, 2H), 2.97 (s, 3H), 2.46 - 2.42 (m, 1H), 2.40 (s, 3H), 2.35 - 2.30 (m, 1H), 1.96 - 1.89 (m, 1H), 1.48 - 1.40 (m, 2H), 0.95 - 0.88 (m, 2H), 0.68 - 0.59 (m, 2H).

### Example 657: (1S,2S)-N-(3-((6-cyclopropyl-8-(3-methyl-2,4-dioxoimidazolidin-1-yl)imidazo[1,2-a]pyridin-2-yl)methoxy)-4-fluorophenyl)-2-(4-methylpyrimidin-2-yl)cyclopropane-1-carboxamide (I-657)

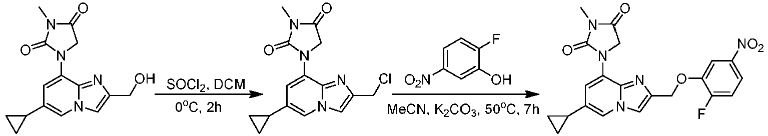

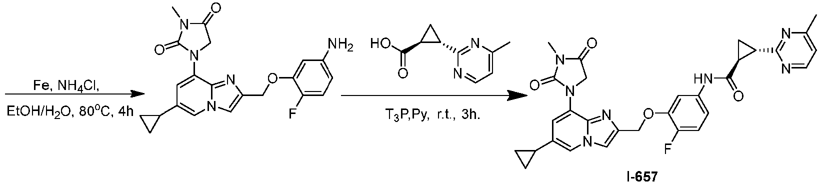

A mixture of **intermediate 451** (100 mg, 0.32 mmol), 2-fluoro-5-nitrophenol (101 mg, 0.64 mmol), and K₂CO₃ (443 mg, 3.20 mmol) in MeCN (5 mL) was stirred at 50 °C under N₂ for 7 h. The reaction was quenched with water (10 mL) and extracted with EtOAc (3 x 10 mL). The combined organic layers were washed with brine (20 mL), dried over anhydrous Na₂SO₄, and concentrated *in vacuo* to give the crude product, which was purified by preparative TLC (eluent: PE/EA = 2/1) to give **1-(6-cyclopropyl-2-((2-fluoro-5-nitrophenoxy)methyl)imidazo[1,2-a]pyridin-8-yl)-3-methylimidazolidine-2,4-dione intermediate 1092** (105 mg, 75%) as yellow solid. ESI-MS [M +H]+: 440.1.

A mixture of **intermediate 1092** (24 mg, 0.06 mmol), NH₄Cl (3.5 mg, 0.07 mmol), and Fe powder (18.4 mg, 0.33 mmol) in EtOH (2 mL) and H2O (0.2 mL) was stirred at 80 °C for 4 h under N₂. The reaction mixture was filtered and the filtrate was concentrated *in vacuo* to give the crude, which was purified by preparative TLC (eluent: DCM/ MeOH = 20/1) to give **1-(2-((5-amino-2-fluorophenoxy)methyl)-6-cyclopropylimidazo[1,2-a]pyridin-8-yl)-3-methylimidazolidine-2,4-dione intermediate 1093** (13 mg, 53%) as yellow solid. ESI-MS [M +H]+: 410.1.

To a mixture of **intermediate 1093** (62 mg, 0.15 mmol) and (1S,2S)-2-(4-methylpyrimidin-2-yl)cyclopropane-1-carboxylic acid (62 mg, 0.15 mmol) ) in pyridine (0.5 mL) was added T₃P (734 mg, 15 mmol). After stirring at room temperature for 3 h, the reaction was quenched with water (10 mL) and extracted with EtOAc (3 x 10 mL). The combined organic layers were washed with brine (20 mL), dried over anhydrous Na₂SO₄, and concentrated in vacuo to give the crude product, which was purified by preparative TLC (eluent: DCM/MeOH = 20/1) to give **(I-657** (70 mg, 82%) as a yellow solid. ESI-MS [M +H]+: 570.2, δ 10.36 (s, 1H), 8.53 (d, J = 5.1 Hz, 1H), 8.30 (d, J = 1.1 Hz, 1H), 7.96 (s, 1H), 7.75-7.73 (m, 1H), 7.39 (d, J = 1.5 Hz, 1H), 7.21 (d, J = 5.1 Hz, 1H), 7.16 - 7.10 (m, 1H), 7.09-7.05 (m, 1H), 5.21 (s, 2H), 4.86 (s, 2H), 2.97 (s, 3H), 2.54 (s, 1H), 2.42 (s, 3H), 2.39-2.37 (m,1H), 2.00-1.93 (m, 1H), 1.54-1.46 (m, 2H), 1.00 - 0.90 (m, 2H), 0.71 - 0.60 (m, 2H).

### Example 658: (1S,2S)-N-(4-chloro-3-(((6-cyclopropyl-8-(3-methyl-2,4-dioxoimidazolidin-1-yl)imidazo[1,2-a]pyridin-2-yl)methyl)amino)-5-fluorophenyl)-2-(4-methylpyrimidin-2-yl)cyclopropane-1-carboxamide (I-658)

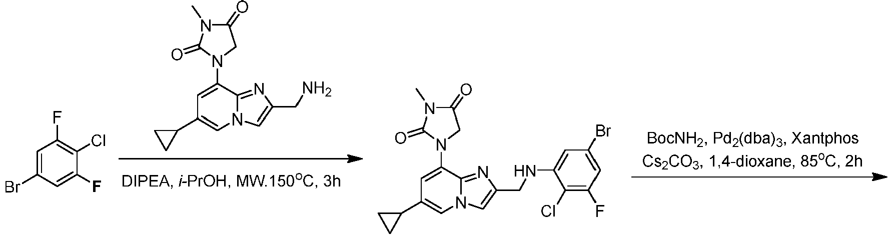

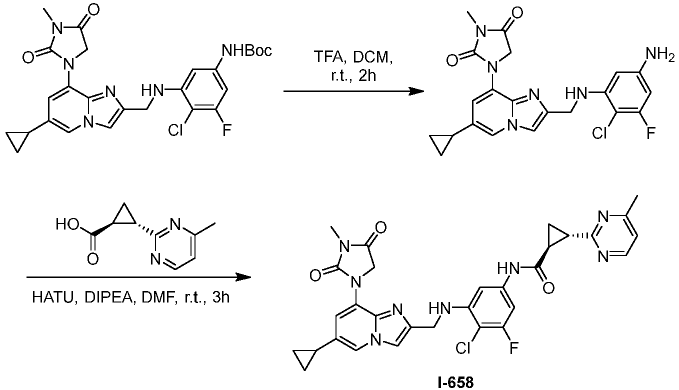

A solution of 5-bromo-2-chloro-1,3-difluorobenzene (648 mg, 2.34 mmol), intermediate 365 (200 mg, 0.67 mmol) and DIPEA (260 mg, 0.21 mmol) in i-PrOH (2 mL) was stirred in a sealed tube. After degassing with N₂ for 1min, the reaction was irradiated in a microwave reactor at 150 °C for 3 h. The reaction was concentrated in vacuo to give the crude product, which was purified by preparative TLC (eluent: DCM/MeOH = 15/1) to give **1-(2-(((5-bromo-2-chloro-3-fluoro phenyl)amino)methyl)-6-cyclopropylimidazo[1,2-a]pyridin-8-yl)-3-methylimidazolidine-2,4-dione intermediate 1094** (170 mg, 50%) as a yellow oil. ESI-MS [M +H]+: 506.0

To a solution of **intermediate 1094**(170 mg, 0.34 mmol), tert-butyl carbamate (48 mg, 0.41 mmol), and Cs₂CO₃ (222 mg, 0.68 mmol) in toluene (10 mL) were added Pd₂(dba)₃ (62 mg, 0.068 mmol) and Xantphos (59 mg, 0.10 mmol). The reaction mixture was stirred at 85 °C for 2 h under N₂. The reaction mixture was filtered through Celite^{®} and the filter cake was washed with DCM/MeOH (20/1, 30 mL). The filtrate was concentrated in vacuo to give the crude, which was purified by preparative TLC (eluent: PE/EtOAc = 2/1) to give **tert-butyl (4-chloro-3-(((6-cyclo propyl-8-(3-methyl-2,4-dioxoimidazolidin-1-yl)imidazo[1,2-a]pyridin-2-yl)methyl)amino)-5-fluorophenyl)carbamate intermediate 1095** (160 mg, 87%) as a yellow solid. ESI-MS [M +H]+: 543.2

To a mixture of **intermediate 1095** (160 mg, 0.30 mmol) in DCM (3 mL) was added TFA (1 ml) at room temperature. The resulting reaction was stirred at room temperature for 2 h. The reaction was diluted with saturated aq. NaHCO₃ solution (5 mL) and extracted with DCM/MeOH (20/1, 3 x 30 mL). The combined organic layers were washed with brine (20 mL), dried over anhydrous Na₂SO₄, and concentrated in vacuo to give the crude product, which was purified by preparative TLC (eluent: DCM/MeOH = 15/1) to give **1-(2-(((5-amino-2-chloro-3-fluorophenyl)amino)methyl)-6-cyclopropylimidazo[1,2-a]pyridin-8-yl)-3-methylimidazolidine-2,4-dione intermediate 1096** (130 mg, quant) as a yellow solid. ESI-MS [M +H]+: 443.1.

A mixture of **intermediate 1096** (130 mg, 0.29 mmol), (1S,2S)-2-(4-methylpyrimidin-2-yl)cyclopropane-1-carboxylic acid (52 mg, 0.29 mmol), HATU (165 mg, 0.44 mmol) and DIPEA (187 mg, 1.45 mmol) in DMF (2 mL) was stirred at room temperature for 3 h. Water (20 mL) was added and the mixture was extracted with DCM/MeOH (20/1, 3 x 30 mL). The combined organic layers were washed with brine (20 mL), dried over Na₂SO₄, filtered and concentrated in vacuo to give the crude, which was purified by preparative TLC (eluent: DCM/MeOH= 20/1) to give **(I-658)** (35 mg, 20%) as a white solid. ESI-MS [M +H]⁺: 603.2. δ 10.34 (s, 1H), 8.50 (d, J = 5.1 Hz, 1H), 8.23 (s, 1H), 7.64 (s, 1H), 7.34 (s, 1H), 7.19 (d, J = 5.1 Hz, 1H), 7.04 (d, J = 10.1 Hz, 1H), 6.73 (s, 1H), 6.38 (t, J = 5.8 Hz, 1H), 4.91 (s, 2H), 4.44 (d, J = 5.6 Hz, 2H), 2.97 (s, 3H), 2.49 - 2.43 (m, 1H), 2.39 (s, 3H), 2.34 (m,1H), 1.97 - 1.88 (m, 1H), 1.50-1.40 (m, 2H), 0.97 - 0.88 (m, 2H), 0.67-0.60 (m, 2H).

### Example 659: (1S,2S)-N-(5-(((6-cyclopropyl-8-(3-methyl-2,4-dioxoimidazolidin-1-yl)imidazo[1,2-a]pyridin-2-yl)methyl)amino)-2,4-difluorophenyl)-2-(4-methylpyrimidin-2-yl)cyclopropane-1-carboxamide (I-659)

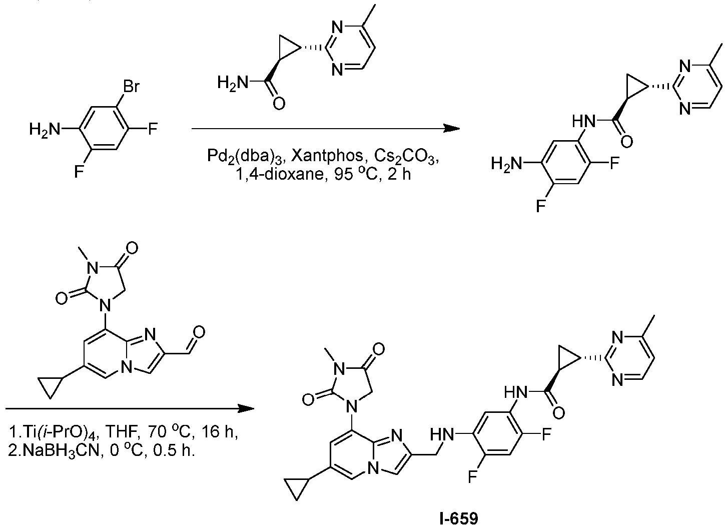

To a mixture of 5-bromo-2,4-difluoroaniline (141.2 mg, 0.68 mmol), **intermediate 55** (60 mg, 0.68 mmol) and Cs₂CO₃ (665 mg, 2.04 mmol) in 1,4-dioxane (14 mL) were added Pd₂(dba)₃ (124.5 mg, 0.136 mmol) and XantPhos (157.4 mg, 0.272 mmol) at 25 °C. After stirring at 95 °C for 2 h under N₂, the reaction mixture was filtered through Celite^{®} and the filter cake was washed with DCM/MeOH (10/1, 50 mL). The filtrate was concentrated *in vacuo* to give the crude, which was purified by column chromatography (eluent: DCM/MeOH=30/1 ~ 10/1) to give **(1S,2S)-N-(5-amino-2,4-difluorophenyl)-2-(4-methylpyrimidin-2-yl)cyclopropane-1-carboxamide intermediate 1097** (20 mg, 10%) as a yellow solid. ESI-MS [M +H] +: 305.1

To a mixture of **intermediate 1097** (20 mg, 0.065 mmol) and **intermediate 1053** (30 mg, 0.098 mmol) in THF (3 mL) was added Ti(i-PrO)₄ (93 mg, 0.325 mmol). The resulting mixture was stirred at 70 °C for 16 h under N₂. After cooling to 25 °C, NaBH₃CN (14.5 mg, 0.23 mmol) was added and the mixture was stirred at 25 °C for 0.5 h. Then the mixture was quenched with water (20 mL) and extracted with EtOAc (3 x 10 mL). The organic were concentrated in vacuo to give the crude, which was purified by column chromatography (eluent: DCM/MeOH=30/1 ~ 10/1) to give (I-659) (20 mg, 53%) as a white solid. ESI-MS [M +H]⁺: 587.2.. δ 9.86 (s, 1H), 8.51 (d, J = 5.1 Hz, 1H), 8.23 (d, J = 1.2 Hz, 1H), 7.69 (s, 1H), 7.34 (d, J = 1.5 Hz, 1H), 7.23 - 7.11 (m, 3H), 5.93 - 5.91 (m, 1H), 4.92 (s, 2H), 4.35 (d, J = 5.9 Hz, 2H), 2.97 (s, 3H), 2.47 - 2.43 (m, 2H), 2.40 (s, 3H), 1.97 - 1.90 (m, 1H), 1.47 - 1.44 (m, 2H), 0.96 - 0.91 (m, 2H), 0.66 - 0.62 (m, 2H).

### Example 660: (1S,2S)-N-(4-(benzyloxy)-6-((6-cyclopropyl-8-(3-methyl-2,4-dioxoimidazolidin-1-yl)imidazo[1,2-a]pyridin-2-yl)methoxy)pyridin-2-yl)-2-(4-methylpyrimidin-2-yl)cyclopropane-1-carboxamide (I-660)

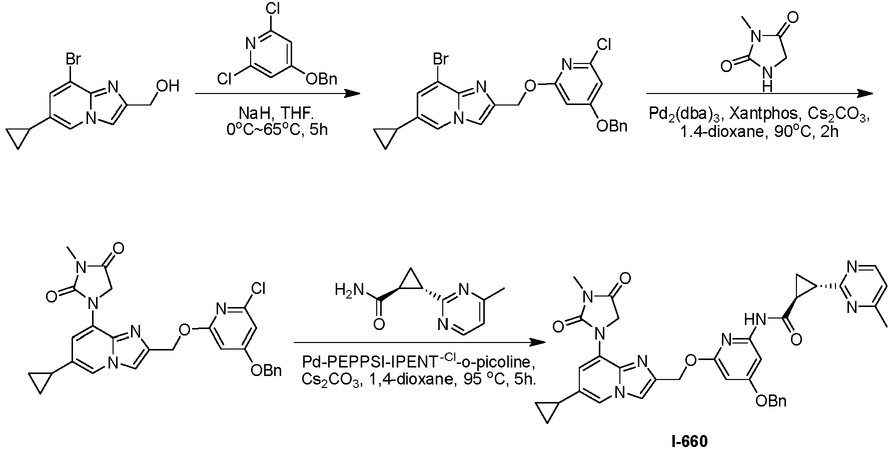

To a mixture of intermediate 453 (100 mg, 0.38 mmol) in dry THF (10.0 mL) was added NaH (60 mg, 1.5 mmol, 60% dispersion in mineral oil) at 0 °C . After stirring the mixture at 0 °C for 0.5 h, a solution of 4-(benzyloxy)-2,6-dichloropyridine (95 mg, 1.2 mmol) in dry THF (2.0 mL) was added and stirred at 65 °C for another 5 h. The reaction was quenched with saturated aq. NH₄Cl (20 mL) and extracted with EtOAc (3 x 50 mL). The combined organic layers were washed with brine (50 mL), dried over anhydrous Na₂SO₄, and concentrated *in vacuo* to give the crude, which was purified by silica gel column chromatography (eluent: DCM/MeOH = 50/1) to give 2-(((4-(benzyl **oxy)-6-chloropyridin-2-yl)oxy)methyl)-8-bromo-6-cyclopropylimidazo[1,2-a]pyridine intermediate 1098** (110 mg, 60%) as a yellow solid. ESI-MS [M +H]⁺: 486.2.

A mixture of **intermediate 1098** (110 mg, 0.23 mmol), 3-methylimidazolidine-2,4-dione (78 mg, 0.25 mmol), and Cs₂CO₃ (222 mg, 0.68 mmol) in 1,4-dioxane (8 mL) were added Pd₂(dba)₃ (21 mg, 0.023 mmol) and Xantphos (26 mg, 0.045 mmol). After stirring at 85 °C for 8 h under N₂, the reaction mixture was filtered through Celite^{®} and the filter cake was washed with DCM/MeOH (10/1, 30 mL). The filtrate was concentrated in vacuo to give the crude, which was purified by preparative TLC (eluent: DCM/MeOH = 30/1) to give **1-(2-(((4-(benzyloxy)-6-chloropyridin-2-yl)oxy)methyl)-6-cyclopropylimidazo[1,2-a]pyridin-8-yl)-3-methylimidazolidine-2,4-dione intermediate 1099** (60 mg, 51%) as a yellow solid. ESI-MS [M +H]⁺: 518.2.

To a mixture of **intermediate 1099** (60 mg, 0.12 mmol), **intermediate 55** (23 mg, 0.13 mmol), and Cs₂CO₃ (113 mg, 0.35 mmol) in 1,4-dioxane (5 mL) was added Pd-PEPPSI-IPENT-Cl-o-picoline (10 mg, 0.012 mmol). The reaction mixture was stirred at 90 °C for 2 h under N₂. The reaction mixture was filtered through Celite^{®} and the filter cake was washed with DCM/MeOH (10/1, 20 mL). The filtrate was concentrated in vacuo to give the crude, which was purified by preparative TLC (eluent: DCM/MeOH = 20/1) to give **(I-660)** (42 mg, 55%) as a white solid. ESI-MS [M +H]⁺: 659.2. δ 10.66 (s, 1H), 8.53 (d, J = 5.1 Hz, 1H), 8.27 (s, 1H), 7.92 (s, 1H), 7.52 - 7.27 (m, 7H), 7.21 (d, J = 5.1 Hz, 1H), 6.20 (d, J = 1.8 Hz, 1H), 5.38 (s, 2H), 5.14 (s, 2H), 4.88 (s, 2H), 2.97 (s, 3H), 2.56 - 2.52 (m, 2H), 2.42 (s, 3H), 1.98 - 1.92 (m, 1H), 1.55 - 1.52 (m, 2H), 0.97 - 0.92 (m, 2H), 0.67 - 0.64 (m, 2H).

### Example 661: (1S,2S)-N-(6-((6-cyclopropyl-8-(3-methyl-2,4-dioxoimidazolidin-1-yl)imidazo[1,2-alpyridin-2-yl)methoxy)-4-hydroxypyridin-2-yl)-2-(4-methylpyrimidin-2-yl)cyclopropane-1-carboxamide (I-661)

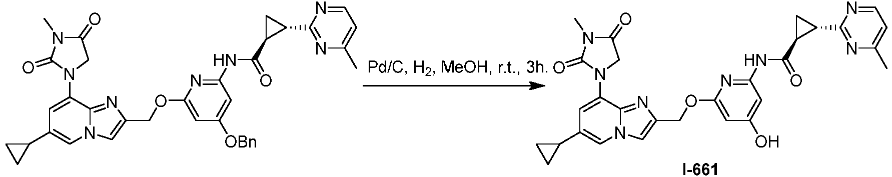

A mixture of **(I-660)** (100 mg, 0.15 mmol) and Pd/C (10%, 20 mg) in MeOH (5 mL) was stirred at room temperature for 3 h under H₂. The reaction mixture was filtered through Celite^{®} and the filter cake was washed with DCM/MeOH (10/1, 20 mL). The filtrate was concentrated *in vacuo* to give the crude, which was purified by preparative TLC (eluent: DCM/MeOH = 15/1) to give **(I-661)** (75 mg, 88%) as a white solid. ESI-MS [M +H]⁺: 569.2. δ 10.52 (d, J = 6.6 Hz, 2H), 8.53 (d, J = 4.7 Hz, 1H), 8.26 (s, 1H), 7.90 (s, 1H), 7.35 (s, 1H), 7.27 (s, 1H), 7.21 (d, J = 4.9 Hz, 1H), 5.87 (s, 1H), 5.34 (s, 2H), 4.88 (s, 2H), 2.97 (s, 3H), 2.56 - 2.52 (m, 2H), 2.42 (s, 3H), 2.00 - 1.87 (m, 1H), 1.55 - 1.50 (m, 2H), 1.04 - 0.85 (m, 2H), 0.67 - 0.63 (m, 2H).

### Example 662: (1S,2S)-N-(6-((6-cyclopropyl-8-(3-methyl-2,4-dioxoimidazolidin-1-yl)imidazo[1,2-alpyridin-2-yl)methoxy)-2-(1-hydroxyethyl)pyrimidin-4-yl)-2-(4-methylpyrimidin-2-yl)cyclo propane-1-carboxamide (I-662)

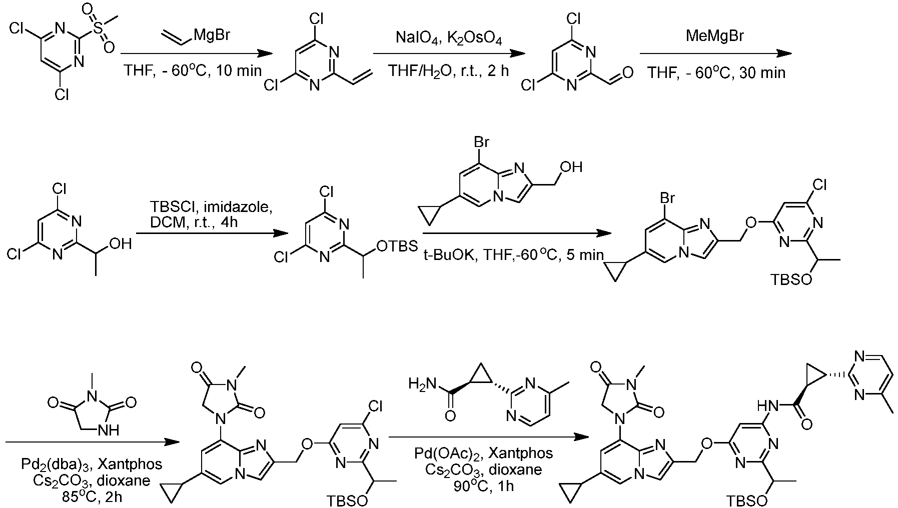

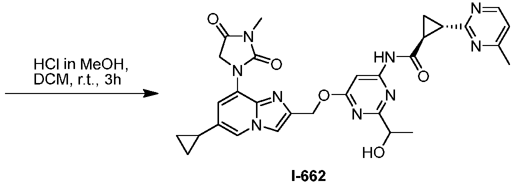

To a mixture of 4,6-dichloro-2-(methylsulfonyl)pyrimidine (50 g, 221 mmol) in THF (300 mL) was added vinylmagnesium bromide in THF (1M solution in THF, 332 mL, 332 mmol) dropwise at -60 °C under N₂. After stirring at -60 °C for 10min, the reaction was quenched with saturated aq. NH₄Cl (100 mL) and extracted with EtOAc (2 x 200 mL). The combined organic layers were washed with brine (80 mL), dried over anhydrous Na₂SO₄, and concentrated *in vacuo* to give the crude, which was purified by silica gel column chromatography (eluent: PE/ EtOAc = 15/1) to give **4,6-dichloro-2-vinylpyrimidine intermediate 1100** (35 g, 91%) as yellow oil. ESI-MS [M +H]+: No MS.

To a mixture of **intermediate 1100** (15 g, 86.2 mmol) in THF (200 mL) and water (100 mL) were added NaIO₄ (55.3 g, 258.6 mmol) and K₂O_{S}O₄·2H₂O (1.59 g, 4.3 mmol) at 0 °C. After stirring at room temperature for 2 h, the reaction mixture was filtered through Celite^{®} and the filter cake was washed with EtOAc (200 mL). The filtrate was concentrated *in vacuo* to give the crude, which was purified with silica gel column chromatography (eluent: PE/EtOAc = 10/1) to give **4,6-dichloro pyrimidine-2-carbaldehyde intermediate 1101** (12 g, 79%) as yellow oil. ESI-MS [M +H]+: 177.0.

To a solution of **intermediate 1101** (5 g, 28.4 mmol) in THF (50 mL) was added MeMgBr in THF (3M solution in THF, 19 mL, 56.8 mmol) at -60 °C dropwise. After stirring at -60 °C for 30 min under N₂, the reaction was quenched with saturated aqueous NH₄Cl (50 mL), extracted with EtOAc (3 x 50 mL). The combined organic layers were washed with brine (60 mL), dried over anhydrous Na₂SO₄, and concentrated *in vacuo* to give the crude, which was purified by silica gel column chromatography (eluent: PE/ EtOAc = 5/1) to give **1-(4,6-dichloropyrimidin-2-yl)ethan-1-of intermediate 1102** (500 mg, 9%) as a yellow oil. ESI-MS [M +H]+: 193.0.

To a mixture of **intermediate 1102** (500 mg, 2.6 mmol) and imidazole (265 mg, 3.9 mmol) in DCM (10 mL) was added TBSCl (465 mg, 3.1 mmol). After stirring at room temperature for 4 h under N₂, the reaction was diluted with DCM (50 mL) and washed with water (40 mL). The organic layer was washed with brine (40 mL), dried over **anhydrous** Na₂SO₄, and concentrated *in vacuo* to give the crude, which was purified by preparative TLC (eluent: PE/ EtOAc = 50/1) give **2-(1-((tertbutyldimethylsilyl)oxy)ethyl)-4,6-dichloropyrimidine intermediate 1103** (300 mg, 38%) as a yellow oil. ESI-MS [M +H]+: 307.0.

To a mixture of **intermediate 453** (230 mg, 0.88 mmol), **intermediate 1103** (270 mg, 0.88 mmol) in THF (5 mL) was added t-BuOK (1M in THF, 0.88 mL, 0.88 mmol) dropwise at -60 °C. After stirring at -60 °C for 5 min under N₂, the reaction was quenched with saturated aq. NH₄Cl (30 mL) and extracted with EtOAc (3 x 30 mL). The combined organic layers were washed with brine (30 mL), dried over anhydrous Na₂SO₄, and concentrated in vacuo to give the crude product, which was purified by preparative TLC (eluent: PE/EtOAc = 4/1) to give **8-bromo-2-(((2-(1-((tertbutyldimethylsilyl)oxy)ethyl)-6-chloropyrimidin-4-yl)oxy)methyl)-6-cyclopropylimidazo[1,2-a]pyridine intermediate 1104** (230 mg, 50%) as a yellow oil. ESI-MS [M +H]+: 537.1.

To a mixture of **intermediate 1104** (230 mg, 0.43 mmol), 3-methylimidazolidine-2,4-dione (49 mg, 0.43 mmol), and Cs₂CO₃ (421 mg, 1.29 mmol) in 1, 4-dioxane (20 mL) were added Pd₂(dba)₃ (39 mg, 0.043 mmol) and Xantphos (50 mg, 0.086 mmol). The resulting reaction mixture was stirred at 85 °C for 2 h under N₂. The reaction mixture was filtered through Celite^{®} and the filter cake was washed with DCM/MeOH (10/1, 50 mL). The filtrate was concentrated in vacuo to give the crude, which was purified by silica gel column chromatography (eluent: PE/EtOAc=2/1) to give **1-(2-(((2-(1-((tert-butyldimethylsilyl)oxy)ethyl)-6-chloropyrimidin-4-yl)oxy)methyl)-6-cyclopropylimidazo[1,2-a]pyridin-8-yl)-3-methylimidazolidine-2,4-dione intermediate 1105** (80 mg, 33%) as a yellow solid. ESI-MS [M +H]+: 571.2.

To a mixture of **intermediate 1105** (80 mg, 0.14 mmol), **intermediate 55** (37 mg, 0.21 mmol), and Cs₂CO₃ (137 mg, 0.42 mmol) in 1,4-dioxane (5 mL) were added Pd(OAc)₂ (6 mg, 0.028 mmol) and Xantphos (32 mg, 0.056 mmol). After stirring at 90 °C for 1 h under N₂, the reaction mixture was filtered through Celite^{®} and the filter cake was washed with DCM/MeOH (10/1, 50 mL). The filtrate was concentrated in vacuo to give the crude, which was purified by silica gel column chromatography (eluent: DCM/MeOH=20/1) to give **(1S,2S)-N-(2-(1-((tertbutyldimethylsilyl)oxy)ethyl)-6-((6-cyclopropyl-8-(3-methyl-2,4-dioxoimidazolidin-1-yl)imidazo[1,2-a]pyridin-2-yl)methoxy)pyrimidin-4-yl)-2-(4-methylpyrimidin-2-yl)cyclopropane-1-carboxamide intermediate 1106** (50 mg, 50%) as a yellow solid. ESI-MS [M +H]+: 712.3

To a solution of **intermediate 1106** (50 mg, 0.07 mmol) in DCM (5 mL) was added HCl (4M solution in MeOH, 1 mL). After stirring at room temperature for 3 h, the reaction was concentrated in vacuo to give the crude, which was purified by preparative TLC (eluent: DCM/MeOH=15/1) to give **(I-662)** (30 mg, 71%) as a yellow solid. ESI-MS [M +H]⁺: 598.3. δ 11.25 (d, J = 1.7 Hz, 1H), 8.53 (d, J = 5.1 Hz, 1H), 8.28 (d, J = 1.3 Hz, 1H), 7.98 (s, 1H), 7.40 - 7.33 (m, 2H), 7.21 (d, J = 5.1 Hz, 1H), 5.52 (s, 2H), 5.09 (m, 1H), 4.89 (s, 2H), 4.65 - 4.58 (m, 1H), 2.97 (s, 3H), 2.69 - 2.65 (m, 1H), 2.55-2.49 (m, 1H), 2.41 (s, 3H), 1.99-1.95 (m, 1H), 1.58 - 1.50 (m, 2H), 1.39 (d, J = 6.6 Hz, 3H), 1.00 - 0.93 (m, 2H), 0.69 - 0.64 (m, 2H).

### Example 663: (1S,2S)-N-(4-((6-cyclopropyl-8-(3-methyl-2,4-dioxoimidazolidin-1-yl)imidazo[1,2-a]pyridin-2-yl)methoxy)-5-(methoxymethyl)pyrimidin-2-yl)-2-(4-methylpyrimidin-2-yl)cyclo propane-1-carboxamide (I-663)

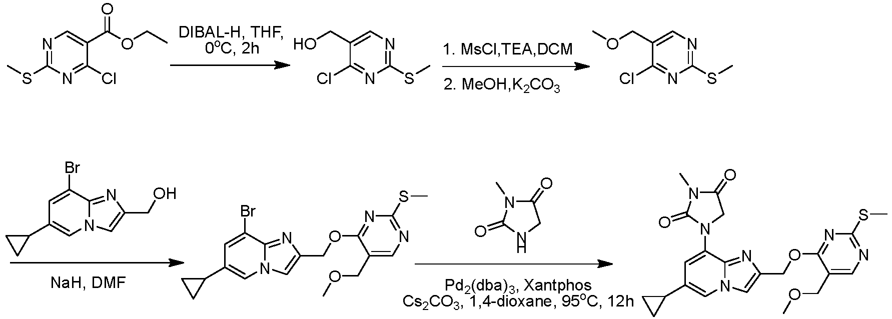

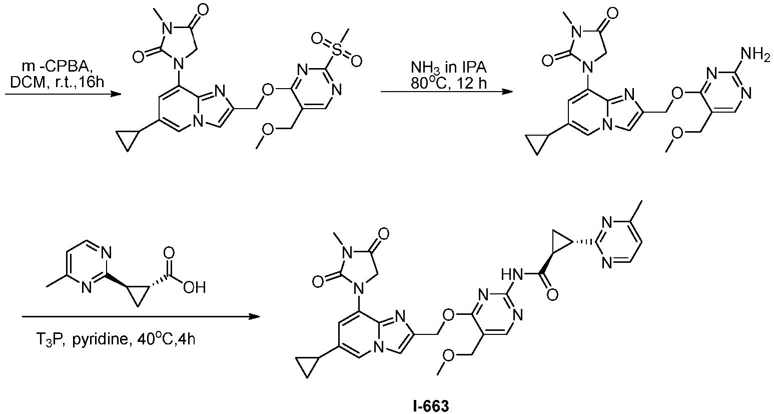

To a solution of **intermediate 453** (4.0 g, 15.2 mmol) in THF (50 mL) was added NaH (1.2 g, 30.4 mmol) at 0°C and the mixture was stirred at 0 °C for 1 h. Then a solution of **intermediate 997** (3.1 g, 15.2 mmol) in DMF (1 mL) was added and stirred at room temperature for 2 h. The mixture was quenched with water (50 mL) and extracted with EtOAc (3 x 50 mL). The combined organic layers were washed with brine (50 mL), dried over Na₂SO₄, and concentrated *in vacuo* to give the crude product, which was purified by column chromatography (eluent: PE/EA = 1/1) to give **8-bromo-6-cyclopropyl-2-(((5-(methoxymethyl)-2-(methylthio)pyrimidin-4-yl)oxy) methyl)imidazo[1,2-a]pyridine intermediate 1107** (5.6 g, 85%) as a white solid. ESI-MS [M +H]+: 435.1

To a mixture of **intermediate 1107** (1.5 g, 3.46 mmol), 3-methylimidazolidine-2,4-dione (1.18 g, 10.38 mmol), and Cs₂CO₃ (3.38 g, 10.38 mmol) in 1,4-dioxane (50 mL) were added Pd₂(dba)₃ (316.6 mg, 0.346 mmol) and Xantphos (400 mg, 0.692 mmol). After stirring at 95 °C for 12 h under N₂, the reaction mixture was filtered through Celite^{®} and the filter cake was washed with DCM/MeOH (10/1, 100 mL). The filtrate was concentrated in vacuo to give the crude, which was purified by column chromatography (eluent: PE/EA=10/1 ~ 1/2) to give ***1*-(6-cyclopropyl-2-(((5-(methoxymethyl)-2-(methylthio)pyrimidin-4-yl)oxy)methyl)imidazo[1,2-a]pyridin-8-yl)-3-methylimidazolidine-2,4-dione intermediate 1108** the product (1.5 g, 92.6%) as a yellow solid. ESI-MS [M +H]+: 469.2

To a solution of **intermediate 1108** (1.5 g, 3.2 mmol) in DCM (40 mL) was added m-CPBA (2.2 g, 12.8 mmol) at 0 °C. After stirring at room temperature for 16 h, the reaction was quenched with water (50 mL) and extracted with DCM (3 x 50 mL). The combined organic layers were washed with brine (50 mL), dried over Na₂SO₄, and concentrated in vacuo to give the crude product, which was purified with column chromatography (eluent: DCM/MeOH = 10/1) to give the product **1-(6-cyclopropyl-2-(((5-(methoxymethyl)-2-(methylsulfonyl)pyrimidin-4-yl)oxy)methyl)imidazo[1,2-a]pyridin-8-yl)-3-methylimidazolidine-2,4-dione intermediate 1109** as a red solid. (430 mg, 27%) ESI-MS [M +H]+: 501.2

A mixture of **intermediate 1109** (430 mg, 0.86 mmol) in NH₃ (2M in IPA, 30 mL) was stirred at 80 °C for 12 h. The mixture was concentrated in vacuo to give the crude product, which was purified by column chromatography (eluent: PE/EA = 1/1) to give the product 1-(2-(((2-amino-**5-(methoxymethyl)pyrimidin-4-yl)oxy)methyl)-6-cyclopropylimidazo[1,2-a]pyridin-8-yl)-3-methylimidazolidine-2,4-dione intermediate 1110** (200 mg, 53%) as a brown solid. ESI-MS [M +H]+: 438.2

To a mixture of **intermediate 1110** (200 mg, 0.46 mmol) and (1R,2R)-2-(4-methyl pyrimidin-2-yl)cyclopropane-1-carboxylic acid (91 mg, 0.51 mg) in pyridine (1.0 mL) was added T₃P (2.3 g, 3.68 mmol). After stirring at 40 °C for 4 h, the reaction was quenched with saturated aq. NaHCO₃ solution (10 mL) and extracted with EtOAc (20 mL x 3). The combined organic layers were washed with brine (30 mL), dried over Na₂SO₄ and concentrated in vacuo to give the crude product, which was purified by preparative TLC (eluent: DCM/MeOH=15/1) to give **(I-663)** (100 mg, 36%) as a yellow solid. ESI-MS [M +H]+: 598.2 δ 10.88 (s, 1H), 8.49 (d, J = 5.1 Hz, 1H), 8.32 (s, 1H), 8.27 (d, J = 1.2 Hz, 1H), 8.01 (s, 1H), 7.38 (d, J = 1.5 Hz, 1H), 7.16 (d, J = 5.1 Hz, 1H), 5.48 (s, 2H), 4.88 (s, 2H), 4.30 (s, 2H), 3.26 (s, 3H), 2.97 (s, 3H), 2.63 - 2.57 (m, 1H), 2.55 - 2.51 (m, 1H), 2.39 (s, 3H), 2.01 - 1.91 (m, 1H), 1.61 - 1.54 (m, 2H), 0.98 - 0.90 (m, 2H), 0.72-0.63 (m, 2H).

### Example 664: (1S,2S)-N-(4-((6-cyclopropyl-8-(3-methyl-2,4-dioxoimidazolidin-1-yl)imidazo[1,2-a]pyridin-2-yl)methoxy)-5-(hydroxymethyl)pyrimidin-2-yl)-2-(4-methylpyrimidin-2-yl)cyclo propane-1-carboxamide (I-664)

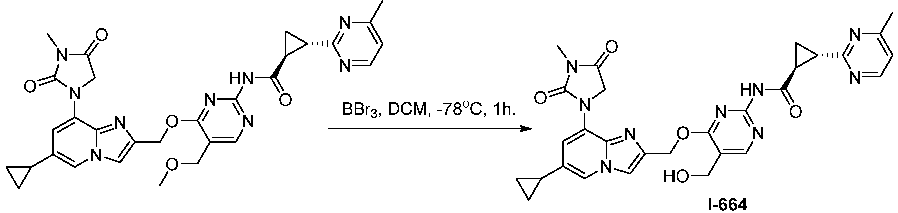

To a solution of (**I-663**) (50 mg, 0.08 mmol) in DCM (5 mL) was added BBr₃ ( 100 mg ,0.40 mmol) at -78 °C. After stirring at -78 °C for 1 h, the reaction was quenched with MeOH (1 mL). Then the mixture was concentrated *in vacuo* to give the crude, which was purified by preparative TLC (eluent: DCM/MeOH= 10/1) to give (**I-664**)as a white solid. (7 mg, 15%). ESI-MS [M +H]+: 584.3 δ 10.82 (s, 1H), 8.49 (d, J = 5.1 Hz, 1H), 8.31 (s, 1H), 8.27 (s, 1H), 8.02 (s, 1H), 7.38 (d, J = 1.4 Hz, 1H), 7.16 (d, J = 5.1 Hz, 1H), 5.47 (s, 2H), 5.15 (t, J = 5.5 Hz, 1H), 4.88 (s, 2H), 4.39 (d, J = 5.4 Hz, 2H), 2.97 (s, 3H), 2.61 - 2.57 (m, 1H), 2.55 - 2.52 (m, 1H), 2.39 (s, 3H), 2.00 - 1.94 (m, 1H), 1.60 - 1.55 (m, 2H), 1.01 - 0.88 (m, 2H), 0.69 - 0.65 (m, 2H).ESI-MS [M +H]+: 584.3

### Example 665: (1S,2S)-N-(5-(((R)-1-(6-cyclopropyl-8-(3-methyl-2,4-dioxoimidazolidin-1-yl)imidazo[1,2-a]pyridin-2-yl)ethyl)amino)-1,2,4-triazin-3-yl)-2-(4-methylpyrimidin-2-yl)cyclo propane-1-carboxamide (I-665)

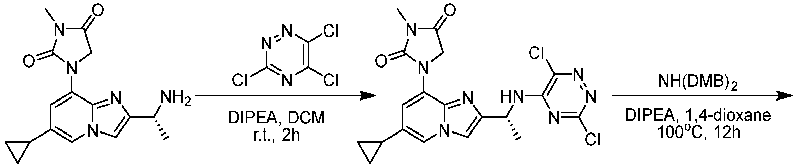

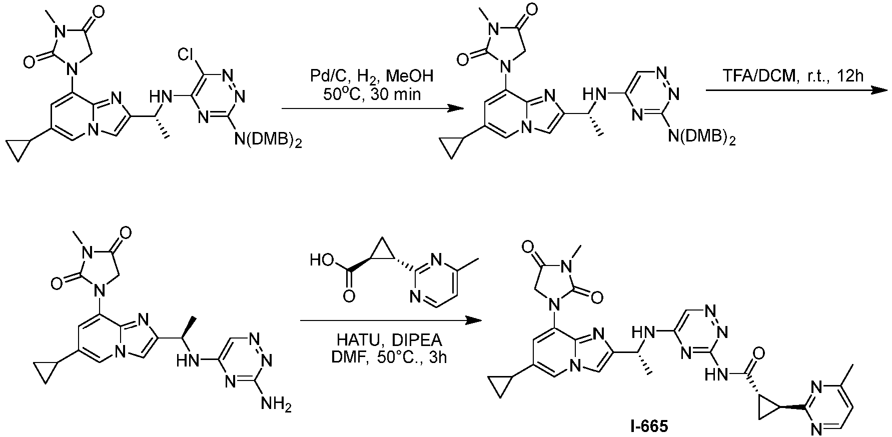

A mixture of (R)-1-(2-(1-aminoethyl)-6-cyclopropylimidazo[1,2-a]pyridin-8-yl)-3-methylimidazolidine-2,4-dione (600 mg, 1.91 mmol), 3,5,6-trichloro-1,2,4-triazine (527 mg, 2.87 mmol) and DIPEA (740 mg, 5.73 mmol) in DCM (12 mL) was stirred in a sealed tube. After degassing with N₂ for 1 min, the mixture was stirred at room temperature for 2 h. The mixture was concentrated in vacuo to give the crude product, which was purified by preparative TLC (eluent: DCM/MeOH = 20/1) to give **(R)-1-(6-cyclopropyl-2-(1-((3,6-dichloro-1,2,4-triazin-5-yl)amino) ethyl)imidazo[1,2-a]pyridin-8-yl)-3-methylimidazolidine-2,4-dione intermediate 1111** (600 mg, 68%) as a yellow solid. ESI-MS [M +H]+: 461.2.

A mixture of **intermediate 1111** (600 mg, 1.3 mmol), NH(DMB)₂ (658 mg, 2.6 mmol) and DIPEA (500 mg, 3.9 mmol) in DMF (10 mL) was stirred at 100 °C for 12 h under N₂. Water (20 mL) was added and the mixture was extracted with DCM (3 x 20 mL). The combined organic layers were washed with brine, dried over Na₂SO₄, filtered, and concentrated in vacuo to give the crude, which was purified by preparative TLC (eluent: DCM/MeOH=20/1) to give **(R)-1-(2-(1-((3-(bis(3,4-dimethylbenzyl)amino)-6-chloro-1,2,4-triazin-5-yl)amino)ethyl)-6-cyclopropylimidazo[1,2-a] pyridin-8-yl)-3-methylimidazolidine-2,4-dione intermediate 1112** (500 mg, 58%) as a yellow solid. ESI-MS [M +H]+: 678.2

A mixture of **intermediate 1112** (500 mg, 0.73 mmol) and Pd/C (300 mg) in MeOH(20 mL) was stirred at 50 °C for 0.5 h under H₂. The reaction mixture was filtered through Celite^{®} and the filter cake was washed with DCM/MeOH (10/1, 20 mL). The filtrate was concentrated in vacuo to give the crude, which was purified by preparative HPLC to give **(R)-1-(2-(1-((3-(bis(3,4-dimethyl benzyl)amino)-1,2,4-triazin-5-yl)amino)ethyl)-6-cyclopropylimidazo[1,2-a]pyridin-8-yl)-3-methylimidazolidine-2,4-dione intermediate 1113** (380 mg, 80 %) as a white solid. ESI-MS [M +H]⁺: 644.1.

A mixture of **intermediate 1113** (380 mg, 0.59 mmol) in TFA (4 mL) was stirred at room temperature for 12 h. The reaction was quenched with water (10 mL) and extracted with EtOAc (3 x 10 mL). The combined organic layers were washed with brine (20 mL), dried over anhydrous Na₂SO₄, and concentrated in vacuo to give the crude product, which was purified by preparative TLC (eluent: DCM/MeOH = 10/1) to give **(R)-1-(2-(1-((3-amino-1,2,4-triazin-5-yl)amino)ethyl)-6-cyclopropylimidazo[1,2-a]pyridin-8-yl)-3-methylimidazolidine-2,4-dione intermediate 1114** (120 mg, 50%) as a white solid. ESI-MS [M +H]+: 408.2.

A mixture of **intermediate 1114** (120 mg, 0.29 mmol), (1S,2S)-2-(4-methylpyrimidin-2-yl)cyclopropane-1-carboxylic acid (34 mg, 0.29 mmol), HATU (220 mg, 0.58 mmol), and DIPEA (112 mg, 0.87 mmol) in DMF (4 mL) was stirred at 50 °C for 3 h under N₂. The reaction was quenched with water (10 mL) and extracted with EtOAc (3 x 10 mL). The combined organic layers were washed with brine (20 mL), dried over anhydrous Na₂SO₄, and concentrated in vacuo to give the crude product, which was purified by preparative TLC(eluent: DCM/MeOH=20/1) to give **(I-**665) (95 mg, 58%) as a yellow solid. ESI-MS [M +H]+: 568.2, δ 10.60 (s, 1H), 8.54 (d, J = 7.9 Hz, 1H), 8.48 (d, J = 5.1 Hz, 1H), 8.32 (s, 1H), 8.21 (s, 1H), 7.75 (s, 1H), 7.34 (d, J = 1.4 Hz, 1H), 7.15 (d, J = 5.1 Hz, 1H), 5.26 - 5.23 (m, 1H), 4.91 (s, 2H), 2.97 (s, 3H), 2.57 - 2.52 (m, 2H), 2.37 (s, 3H), 1.97 - 1.93 (m, 1H), 1.55 - 1.52 (m, 5H), 0.96 - 0.92 (m, 2H), 0.66 - 0.62 (m, 2H).

### Example 666: N-(5-((6-cyclopropyl-8-(3-methyl-2,4-dioxoimidazolidin-1-yl)imidazo[1,2-a]pyridin-2-yl)methoxy)-6-(trifluoromethyl)pyridazin-3-yl)-2-(4-methylpyrimidin-2-yl)cyclo propane-1-carboxamide (I-666)

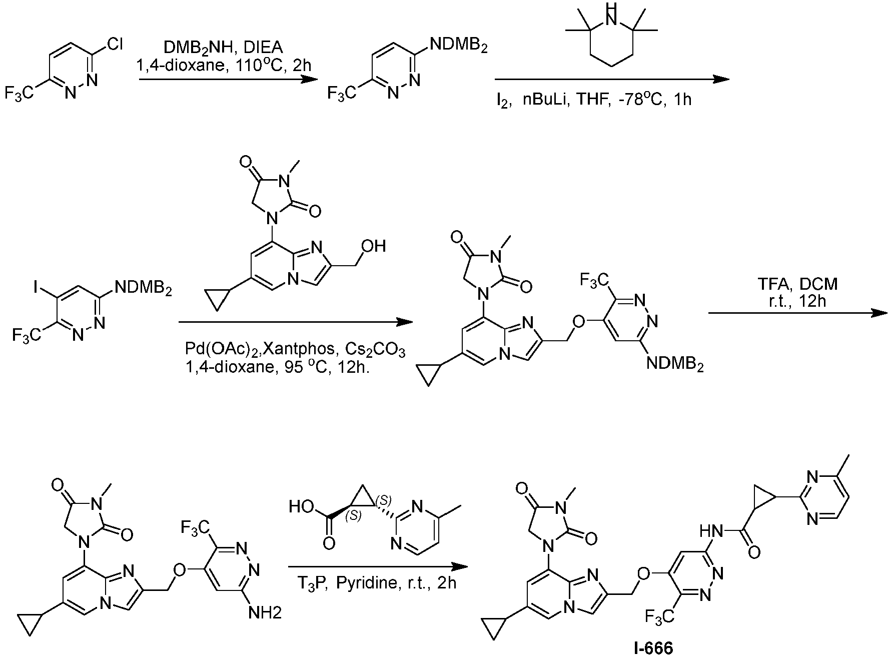

To a solution of 3-chloro-6-(trifluoromethyl)pyridazine(5 g, 27.4 mmol) in 1,4-dioxane (50 mL) was added DMB₂NH (9.5 g, 30.1 mmol) and DIEA (10.6 g, 82.2 mmol) at room temperature. After stirring at 110 ⁰C for 2 h, water (200 mL) was added and the mixture was extracted with DCM (3 x 50 mL). The combined organic layers were washed with brine, dried over Na₂SO₄, filtered and concentrated *in vacuo* to give the crude, which was purified by column chromatography (eluent: PE/EtOAc=20/1 ~ 5/1) to give **N,N-bis(3,4-dimethylbenzyl)-6-(trifluoromethyl)pyridazin-3-amine intermediate 1115** (7 g, 55%) as a yellow solid. ESI-MS [M +H]+: 464.2

To a solution of 2,2,6,6-tetramethylpiperidine (423 mg, 0.9 mmol) in THF (10 mL) was added *n*-BuLi (0.5 mL, 1.2 mmol) at -78 ^{O}C under N₂. The mixture was stirred at -78 ^{O}C for 1h and then a solution of **intermediate 1115** (463 mg, 1.0 mmol) in THF (1 mL) was added slowly. After 1 h, a solution of I₂ (379.5 mg, 1.5 mmol) in THF (1 mL) was added and the reaction mixture was stirred at -78 ⁰C for 1 h. The reaction was quenched with saturated aq. NH₄Cl (20 mL) and extracted with EtOAc (3 x 30 mL). The combined organic layers were washed with brine (30 mL), dried over anhydrous Na₂SO₄, and concentrated *in vacuo* to give the crude, which was purified by preparative TLC (eluent: PE/EtOAc= 5/1) to give **N,N-bis(3,4-dimethoxybenzyl)-5-iodo-6-(trifluoromethyl)pyridazin-3-amine intermediate 1116** (463 mg, 87%) as a yellow solid. ESI-MS [M +H]+: 590.2

To a mixture of **intermediate 1116** (138 mg, 0.46 mmol), **intermediate 454** (270 mg, 0.46 mmol) and Cs₂CO₃ (449.6 mg, 1.4 mmol) in 1,4-dioxane (5 mL) were added Pd(OAc)₂ (24.1 mg, 0.09 mmol) and Xantphos (79.8 mg, 0.14 mmol). After stirring at 95 °C for 12 h under N₂, the reaction mixture was filtered through Celite^{®} and the filter cake was washed with DCM/MeOH (10/1, 10 mL). The filtrate was concentrated in vacuo to give the crude, which was purified by preparative TLC (eluent: DCM/MeOH=20/1) to give **1-(2-(((6-(bis(3,4-dimethylbenzyl)amino)-3-(trifluoromethyl)pyridazin-4-yl)oxy)methyl)-6-cyclopropylimidazo[1,2-a]pyridin-8-yl)-3-methylimidazolidine-2,4-dione intermediate 1117** (160 mg, 46%) as a yellow solid. ESI-MS [M +H]+: 762.2.

***Synthesis of 1-(2-(((6-amino-3-(trifluoromethyl)pyridazin-4-yl)oxy)methyl)-6-cyclopropylimidazo[1,2-a]pyridin-8-yl)-3-methylimidazolidine-2,4-dione.*** To a mixture of **intermediate 1117** (300 mg, 0.58 mmol) in DCM (4 mL) was added TFA (4 ml) at room temperature. The resulting reaction was stirred at room temperature for 12 h. The reaction was quenched with water (10 mL) and extracted with EtOAc (3 x 10 mL). The combined organic layers were washed with brine (20 mL), dried over anhydrous Na₂SO₄, and concentrated in vacuo to give the crude product, which was purified by preparative TLC (eluent: DCM/MeOH = 10/1) to give 1-(2-(((6-amino-3-(trifluoromethyl)pyridazin-4-yl)oxy)methyl)-6-cyclopropylimidazo[1,2-a]pyridin-8-yl)-3-methylimidazolidine-2,4-dione (140 mg, 52%) as a white solid. ESI-MS [M +H]+: 462.2.

A mixture of 1-(2-(((6-amino-3-(trifluoromethyl)pyridazin-4-yl)oxy)methyl)-6-cyclopropylimidazo[1,2-a]pyridin-8-yl)-3-methylimidazolidine-2,4-dione (50 mg, 0.1 mmol), (1S,2S)-2-(4-methylpyrimidin-2-yl)cyclopropane-1-carboxylic acid (19 mg, 0.1mmol) and T₃P (343.0 mg, 1.1 mmol) in pyridine (1 mL) was stirred at room temperature for 2 h. Water (20 mL) was added and the mixture was extracted with EtOAc (3 x 20 mL). The combined organic layers were washed with brine, dried over Na₂SO₄, filtered and concentrated in vacuo to give the crude, which was purified by preparative TLC (eluent: DCM/MeOH=201) to give **(I-666)** (38 mg, 61%) as a white solid. ESI-MS [M +H]+: 622.1. δ 11.83 (s, 1H), 8.62 (d, J = 28.2 Hz, 1H), 8.55 (d, J = 5.0 Hz, 1H), 8.31 (s, 1H), 8.01 (s, 1H), 7.44 (s, 1H), 7.23 (d, J = 5.0 Hz, 1H), 5.53 (s, 2H), 4.96-4.94 (m, 2H), 2.99 (s, 3H), 2.67 - 2.56 (m, 2H), 2.43 (s, 3H), 1.99-1.96 (m, 1H), 1.67 - 1.46 (m, 2H), 0.98-0.94 (m, 2H), 0.69-0.65 (m, 2H).

### Example 667: (1S,2S)-N-(5-(2-(6-cyclopropyl-8-(3-methyl-2,4-dioxoimidazolidin-1-yl) imidazo[1,2-a]pyridin-2-yl)ethyl)pyridazin-3-yl)-2-(4-methylpyrimidin-2-yl)cyclopropane-1-carboxamide (I-667)

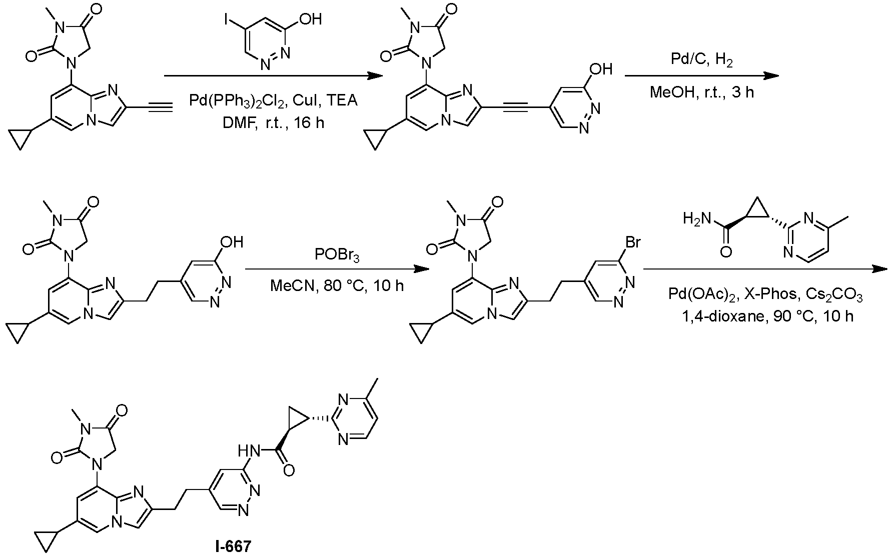

To a reaction mixture of **intermediate 1011** (0.37 g, 0.50 mmol), Pd (PPh₃)₂Cl₂ (0.10 g, 0.13 mmol), CuI (0.12 g, 0.63 mmol), and TEA (0.64 g, 5.0 mmol) in DMF (10 mL) was added 5-iodopyridazin-3-ol (0.56 g, 2.5 mmol). After stirring at room temperature for 16 h, the reaction was concentrated *in vacuo* to give the crude, which was purified by column chromatography on silica gel (eluent: MeOH/DCM = 1/20) to give the **1-(6-cyclopropyl-2-((6-hydroxypyridazin-4-yl)ethynyl) imidazo[1,2-a]pyridin-8-yl)-3-methylimidazolidine-2,4-dione intermediate 1118** (0.20 g, 41%) as yellow solid. ESI-MS [M +H]⁺: 389.1.

A reaction mixture of **intermediate 1118** (0.20 g, 0.52 mmol) and 10% Pd on carbon (0.20 g) in MeOH (20 mL) was stirred at room temperature for 3 h under H₂ atmosphere. The mixture was then filtered and concentrated *in vacuo* to give the **1-(6-cyclopropyl-2-(2-(6-hydroxypyridazin-4-yl)ethyl)imidazo[1,2-a]pyridin-8-yl)-3-methylimidazolidine-2,4-dione** (0.18 g, 89 %) as yellow solid, which was used in next step without purification. ESI-MS [M +H]⁺: 393.2.

A mixture of 1-(6-cyclopropyl-2-(2-(6-hydroxypyridazin-4-yl)ethyl)imidazo[1,2-a]pyridin-8-yl)-3-methylimidazolidine-2,4-dione (0.16 g, 0.40 mmol) in MeCN (15 mL) was added POBr₃ (1.1 g, 4.0 mmol). Then the reaction was stirred at 8 0°C for 10 h under N₂. After cooling to room temperature, the reaction was quenched in ice/water and neutralized with solid NaHCO₃. The product was extracted with EtOAc and the organic phase was washed with brine, dried over Na₂SO₄ and concentrated *in vacuo* to give the crude, which was purified by column chromatography on silica gel (eluent: MeOH/DCM = 1/20) to give the **1-(2-(2-(6-bromopyridazin-4-yl)ethyl)-6-cyclopropylimidazo[1,2-a]pyridin-8-yl)-3-methylimidazolidine-2,4-dione intermediate 1119** (95 mg, 52 %) as yellow solid. ESI-MS [M +H]⁺: 455.1.

To a reaction mixture of **intermediate 1119** (0.14 g, 0.50 mmol), **intermediate 55** (36 mg, 0.20 mmol), Cs₂CO₃ (0.20 mg, 0.60 mmol), and X-Phos (19 mg, 0.040 mmol) in 1,4-dioxane (10 mL) was added Pd(OAc)₂ (4.0 mg, 0.020 mmol). The reaction was stirred at 90 °C for 10 h under N₂ and cooled to room temperature. The reaction was concentrated in vacuo to give the crude, which was purified by column chromatography on silica gel (eluent: MeOH/DCM = 1/15) to give the (I-667) (19 mg, 17%) as white solid. ESI-MS [M +H]⁺: 552.2. δ 11.35 (s, 1H), 8.85 (d, J = 1.9 Hz, 1H), 8.53 (d, J = 5.1 Hz, 1H), 8.19 (d, J = 1.2 Hz, 2H), 7.62 (s, 1H), 7.32 (d, J = 1.5 Hz, 1H), 7.22 (d, J = 5.1 Hz, 1H), 4.92 - 4.87 (m, 2H), 3.09 - 3.02 (m, 4H), 2.98 (s, 3H), 2.69 - 2.67 (m, 1H), 2.56 - 2.54 (m, 1H), 2.42 (s, 3H), 1.97 -1.91 (m, 1H), 1.58 - 1.49 (m, 2H), 0.97 - 0.90 (m, 2H), 0.68 - 0.62 (m, 2H).

### Example 668: (1S,2S)-N-(5-(((6-cyclopropyl-8-(3-methyl-2,4-dioxoimidazolidin-1-yl) imidazo[1,2-a]pyridin-2-yl)methyl)(methyl)amino)-1,2,4-triazin-3-yl)-2-(4-methylpyrimidin-2-yl) cyclopropane-1-carboxamide (I-668)

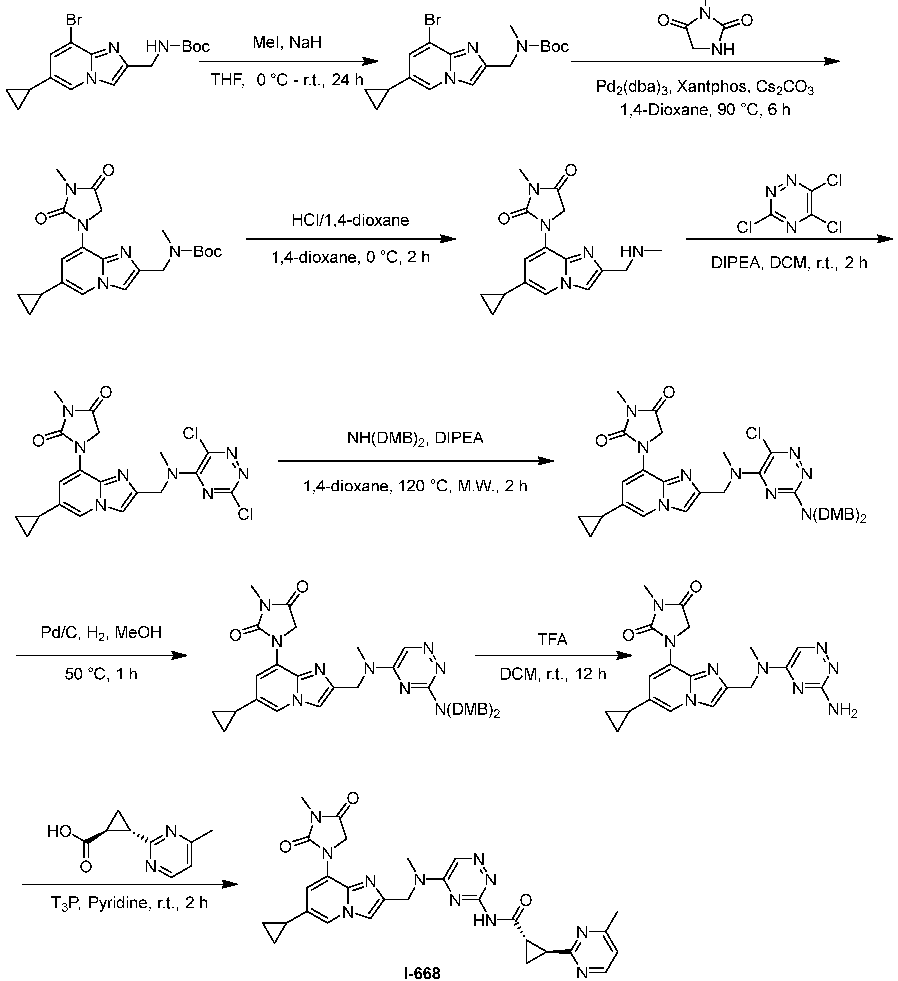

To a mixture of *tert-*butyl ((8-bromo-6-cyclopropylimidazo[1,2-a]pyridin-2-yl)methyl) carbamate (2.0 g, 5.5 mmol) in THF (35 mL) was added NaH (0.40 g, 9.8 mmol, 60% purity in mineral oil) at 0 °C for 30 min. Then a solution of MeI (1.712 g, 12.054 mmol) in THF (5 mL) was added and stirred at room temperature for 24 h. The mixture was quenched with saturated aq. NH₄Cl (50 mL) and extracted with EtOAc (3 x 100 mL). The combined organic extract was washed with brine (200 mL), dried over Na₂SO₄, concentrated *in vacuo* and purified by column chromatography on silica gel (eluent: PE/EtOAc = 2/1) to give ***tert*-butyl ((8-bromo-6-cyclopropylimidazo[1,2-a] pyridin-2-yl)methyl)(methyl)carbamate intermediate 1120** (1.3 g, 60%) as white solid. ESI-MS [M +H]+: 380.1.

A mixture of **intermediate 1047** (0.26 g, 0.84 mmol), 3,5,6-trichloro-1,2,4-triazine (0.23 g, 1.3 mmol), and DIPEA (0.54 g, 4.2 mmol) in DCM (15 mL) was stirred at room temperature for 2 h. The mixture was concentrated in vacuo and purified by preparative TLC (eluent: PE/EtOAc = 1/1) to give **1-(6-cyclopropyl-2-(((3,6-dichloro-1,2,4-triazin-5-yl)(methyl)amino)methyl)imidazo[1,2-a]pyridin-8-yl)-3-methylimidazolidine-2,4-dione intermediate 1121** (0.33 g, 85%) as yellow solid. ESI-MS [M +H]+: 293.1.

A mixture of **intermediate 1121** (0.33 g, 0.72 mmol), NH(DMB)₂ (0.68 g, 2.2 mmol), and DIPEA (0.28 g, 2.2 mmol) in 1,4-dioxane (8.0 mL) was stirred at 120 °C in a microwave reactor for 3 h. The mixture was concentrated in vacuo and purified by preparative TLC (eluent: PE/EtOAc = 1/1) to give **1-(2-(((3-(bis(2,4-dimethoxybenzyl)amino)-6-chloro-1,2,4-triazin-5-yl)(methyl)amino)methyl)-6-cyclopropylimidazo[1,2-a]pyridin-8-yl)-3-methylimidazolidine-2,4-dione intermediate 1122** (0.48 g, 90%) as light-yellow solid. ESI-MS [M +H]+: 742.2.

A mixture of **intermediate 1122** (0.24 g, 0.32 mmol) and 10% Pd on carbon (0.21 g, 1.9 mmol) in MeOH (20 mL) was stirred at 50 °C for 1 h under H₂. The mixture was filtered and concentrated in vacuo to give the crude product, which was purified by preparative TLC (eluent: DCM/MeOH = 10/1) to give **1-(2-(((3-(bis(2,4-dimethoxybenzyl)amino)-1,2,4-triazin-5-yl)(methyl)amino)methyl)-6-cyclopropylimidazo[1,2-a]pyridin-8-yl)-3-methylimidazolidine-2,4-dione intermediate 1123** (0.15 mg, 66%) as a yellow solid. ESI-MS [M +H]+: 708.3.

A mixture of **intermediate 1123** (0.15 g, 0.21 mmol) and TFA (0.73 g, 6.4 mmol) in DCM (10 mL) was stirred at room temperature for 12 h. The mixture was concentrated in vacuo and purified by preparative TLC (eluent: DCM/MeOH = 20/1) to give **1-(2-(((3-amino-1,2,4-triazin-5-yl)(methyl)amino)methyl)-6-cyclopropylimidazo[1,2-a]pyridin-8-yl)-3-methylimidazolidine-2,4-dione intermediate 1124** (75 mg, 87%) as white solid. ESI-MS [M +H]+: 408.1.

A mixture of **intermediate 1124** (75 mg, 0.18 mmol), (1S,2S)-2-(4-methylpyrimidin-2-yl)cyclopropane-1-carboxylic acid (49 mg, 0.28 mmol) and T₃P (0.94 g, 1.5 mmol) in pyridine (0.38 mL) was stirred at room temperature for 2 h. The mixture was quenched with saturated aq. NaHCO₃ (5.0 mL) and extracted with EtOAc (3 x 20 mL). The combined organic extract was washed with brine (20 mL), dried over Na₂SO₄, and concentrated in vacuo and purified by preparative TLC (eluent: DCM/MeOH = 10/1) to give **(I-668)** (16 mg, 15%) as yellow solid. ESI-MS [M +H]+: 568.3. δ 10.71 (d, J = 17.9 Hz, 1H), 8.88-8.68 (m, 1H), 8.51-8.47 (m, 1H), 8.22 (d, J = 15.6 Hz, 1H), 7.83 (d, J = 27.6 Hz, 1H), 7.37 (d, J = 11.2 Hz, 1H), 7.17 (d, J = 12.8 Hz, 1H), 4.95 - 4.71 (m, 4H), 3.16 (d, J = 26.3 Hz, 3H), 2.96 (s, 4H), 2.56 (d, J = 14.8 Hz, 1H), 2.39 (s, 3H), 2.03 - 1.87 (m, 1H), 1.55 (s, 2H), 0.97-0.90 (m, 2H), 0.67-0.61 (m, 2H).

### Example 669: (1S,2S)-N-(4-cyano-3-((6-cyclopropyl-8-(3-methyl-2,4-dioxoimidazolidin-1-yl)imidazo[1,2-a]pyridin-2-yl)methoxy)phenyl)-2-(4-methylpyrimidin-2-yl)cyclopropane-1-carboxamide (I-669)

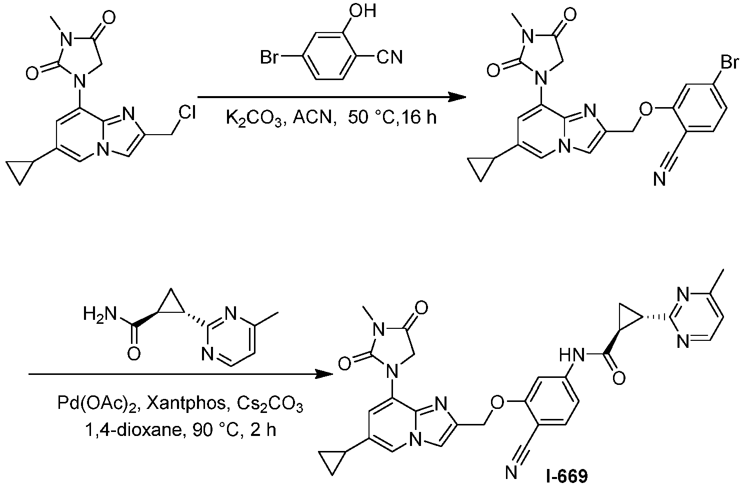

To a solution of **intermediate 451** (0.11 g, 0.36 mmol) in ACN (5.0 mL) was added 4-bromo-2-hydroxybenzonitrile (0.14 g, 0.72 mmol) and K₂CO₃ (0.50 g, 3.6 mmol). After the mixture was stirred at 50 °C for 16 h, water (20 mL) was added and extracted with EtOAc (3 x 20 mL). The combined organic layers were washed with brine, dried over Na₂SO₄, filtered and concentrated *in vacuo* to give the crude, which was purified by preparative TLC to give **4-bromo-2-((6-cyclo propyl-8-(3-methyl-2,4-dioxoimidazolidin-1-yl)imidazo[1,2-a]pyridin-2-yl)methoxy) benzonitrile intermediate 1125** (0.10 g, 58%) as a white solid. ESI-MS [M +H]+: 480.1.

To a solution of **intermediate 1125** (0.10 g, 0.21 mmol) in 1,4-dioxane (8.0 mL) was added **intermediate 55** (39 mg, 0.22 mmol), Cs₂CO₃ (0.20 g, 0.63 mmol),Pd₂(dba)₃ (19 mg, 0.021 mmol), and Xantphos (24 mg, 0.042 mmol). The mixture was stirred at 90 °C for 2 h under N₂. The reaction mixture was filtered through Celite^{®} and the filter cake was washed with DCM/MeOH (v/v = 10/1, 30 mL). The filtrate was concentrated in vacuo to give the crude, which was purified by prep-HPLC to give **(I-669)** (21 mg, 17%) as a white solid. ESI-MS [M +H]⁺:577.2 . δ 10.77 (s, 1H), 8.53 (d, J = 4.9 Hz, 1H), 8.31 (s, 1H), 7.94 (d, J = 22.7 Hz, 2H), 7.63 (d, J = 8.5 Hz, 1H), 7.40 (s, 1H), 7.21 - 7.19 (m, 2H), 5.32 (s, 2H), 4.93 (d, J = 18.4 Hz, 2H), 2.97 (s, 3H), 2.53 - 2.51 (m, 2H), 2.42 (s, 3H), 1.97- 1.94 (m, 1H), 1.62 - 1.48 (m, 2H), 0.96 - 0.94 (m, 2H), 0.68 - 0.66 (m, 2H).

### Example 670: (1S,2S)-N-(5-((6-cyclopropyl-8-(3-methyl-2,4-dioxoimidazolidin-1-yl)imidazo[1,2-a]pyridin-2-yl)methoxy)-6-(methylamino)pyridazin-3-yl)-2-(4-methylpyrimidin-2-yl)cyclopropane-1-carboxamide (I-670)

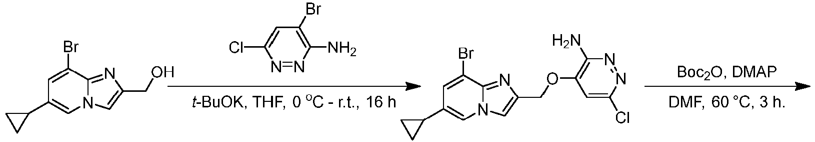

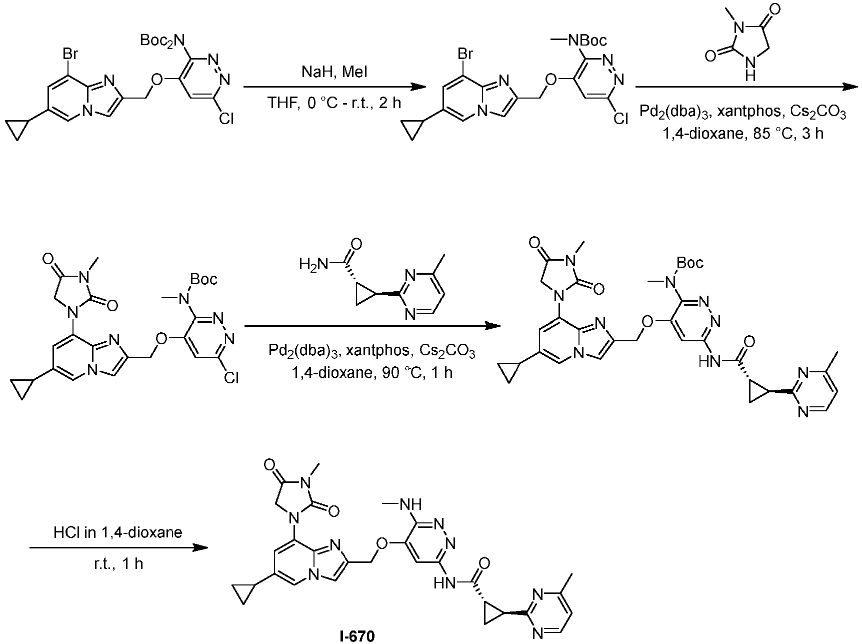

To mixture of **intermediate 453** (1.1 g, 4.0 mmol) and 4-bromo-6-chloropyridazin-3-amine (1.3 g, 6.0 mmol) in dry THF (40 mL) was added t-BuOK (1M in THF, 12 mL) at 0 °C . After stirring at room temperature for 16 h, the reaction was quenched with saturated aq. NH₄Cl (20 mL) and extracted with EtOAc (3 x 30 mL x 3). The combined organic layers were washed with brine (30 mL), dried over anhydrous Na₂SO₄, and concentrated *in vacuo* to give the crude, which was purified by column chromatography on silica gel (eluent: DCM/MeOH = 30/1) to give **4-((8-bromo-6-cyclopropylimidazo[1,2-a]pyridin-2-yl)methoxy)-6-chloropyridazin-3-amine intermediate 1126** (1.5 g, 95%) as a yellow solid. ESI-MS [M +H]⁺: 396.2.

A mixture of **intermediate 1126** (1.5 g, 3.8 mmol), Boc₂O (2.4 g, 11 mmol) and DMAP (0.46 g, 3.8 mmol) in DMF (30 mL) was stirred at 60 °C for 3 h. The reaction was quenched with water (50 mL) and extracted with EtOAc (3 x 30 mL). The combined organic layers were washed with brine (50 mL), dried over anhydrous Na₂SO₄, and concentrated in vacuo to give the crude, which was purified by column chromatography on silica gel (eluent: DCM/MeOH = 50/1) to give **tert-butyl(4-((8-bromo-6-cyclopropylimidazo[1,2-a]pyridin-2-yl)methoxy)-6-chloropyridazin-3-yl)(tert-butoxycarbonyl)carbamate intermediate 1127** (1.5 g, 66%) as a yellow solid. ESI-MS [M +H]⁺: 596.2.

To mixture of **intermediate 1127** (0.50 g, 0.84 mmol) in dry THF (15 mL) was added NaH (0.10 g, 2.5 mmol) at 0 °C for 0.5 h. Then a solution of MeI (0.36 g, 2.5 mmol) in dry THF (5.0 mL) was added and it was stirred at room temperature for another 2 h. The reaction was quenched with saturated aq. NH₄Cl (20 mL) and extracted with EtOAc (3 x 30 mL). The combined organic layers were washed with brine (30 mL), dried over anhydrous Na₂SO₄, and concentrated in vacuo to give the crude, which was purified by column chromatography on silica gel (eluent: DCM/MeOH = 20/1) to give **tert-butyl (4-((8-bromo-6-cyclopropylimidazo[1,2-a]pyridin-2-yl)methoxy)-6-chloropyridazin-3-yl)(methyl)carbamate intermediate 1128** (0.25 g, 59%) as a yellow solid. ESI-MS [M +H]⁺: 510.1.

To a mixture of **intermediate 1128**(0.20 g, 0.40 mmol), 3-methylimidazolidine-2,4-dione (46 mg, 0.40 mmol), and Cs₂CO₃ (0.39 g, 1.2 mmol) in 1,4-dioxane (10 mL) were added Pd₂(dba)₃ (37 mg, 0.040 mmol) and Xantphos (46 mg, 0.080 mmol). The reaction mixture was stirred at 85 °C for 3 h under N₂ and cooled to room temperature. The reaction mixture was filtered through Celite^{®} and the filter cake was washed with DCM/MeOH (v/v = 10/1, 30 mL). The filtrate was concentrated in vacuo to give the crude, which was purified by preparative TLC (eluent: DCM/MeOH = 20/1) to give **tert-butyl (6-chloro-4-((6-cyclopropyl-8-(3-methyl-2,4-dioxoimidazolidin-1-yl)imidazo[1,2-a]pyridin-2-yl)methoxy)pyridazin-3-yl)(methyl)carbamate intermediate 1129** (80 mg, 37%) as a yellow solid. ESI-MS [M +H]⁺: 542.2.

To a mixture **intermediate 1129** (0.12 g, 0.22 mmol), **intermediate 55** (40 mg, 0.22 mmol), and Cs₂CO₃ (0.22 g, 0.66 mmol) in 1,4-dioxane (10 mL) were added Pd₂(dba)₃ (20 mg, 0.022 mmol) and Xantphos (25 mg, 0.044 mmol). After stirring at 90 °C for 1 h under N₂, the reaction mixture was cooled to room temperature and filtered through Celite^{®}. The filter cake was washed with DCM/MeOH (v/v = 10/1, 30 mL) and the filtrate was concentrated in vacuo to give the crude, which was purified by preparative TLC (eluent: DCM/MeOH = 20/1) to give **tert-butyl (4-((6-cyclopropyl-8-(3-methyl-2,4-dioxoimidazolidin-1-yl)imidazo[1,2-a]pyridin-2-yl)methoxy)-6-((1S,2S)-2-(4-methylpyrimidin-2-yl)cyclopropane-1-carboxamido)pyridazin-3-yl)(methyl)carbamate intermediate 1130** (40 mg, 27%) as a yellow solid. ESI-MS [M +H]⁺: 683.2.

A mixture of **intermediate 1130** (40 mg, 0.060 mmol) in HCl (4M solution in 1,4-dioxane, 5.0 mL) was stirred at room temperature for 1 h. The reaction mixture was concentrated in vacuo to give the crude, which was purified by preparative HPLC to give **(I-670)** (12 mg, 35%) as a white solid. ESI-MS [M +H]⁺: 583.2. δ 10.82 (s, 1H), 8.53 (d, J = 5.1 Hz, 1H), 8.29 (s, 1H), 8.05 (s, 1H), 7.87 (s, 1H), 7.40 (d, J = 1.4 Hz, 1H), 7.21 (d, J = 5.1 Hz, 1H), 6.17 (q, J = 4.8 Hz, 1H), 5.31 (s, 2H), 4.94 (d, J = 4.0 Hz, 2H), 2.98 (s, 3H), 2.85 (d, J = 4.8 Hz, 3H), 2.57 - 2.54 (m, 2H), 2.42 (s, 3H), 2.00 - 1.95 (m, 1H), 1.53 - 1.49 (m, 2H), 1.03 - 0.86 (m, 2H), 0.73 - 0.63 (m, 2H).

### Example 671: (1S,2S)-N-(5-((6-cyclopropyl-8-(3-methyl-2,4-dioxoimidazolidin-1-yl)imidazo[1,2-a]pyridin-2-yl)methoxy)-1,2,4-triazin-3-yl)-2-(4-methylpyrimidin-2-yl)cyclopropane-1-carboxamide (I-671)

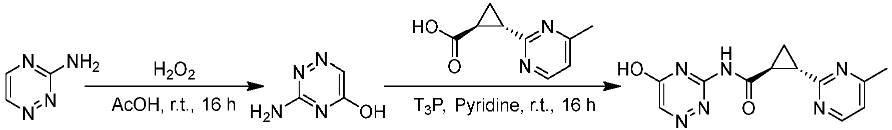

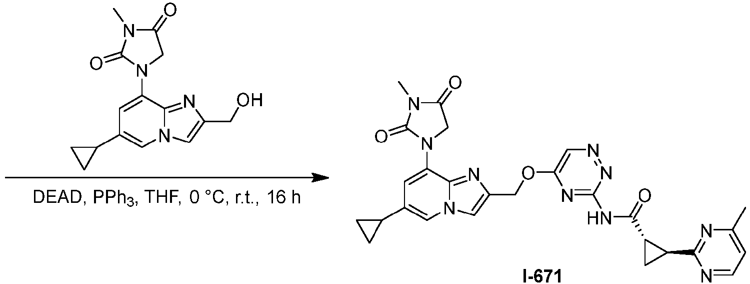

To a mixture of 1,2,4-triazin-3-amine (2.4 g, 25 mmol) in acetic acid (15 mL) was added dropwise H₂O₂ (30% w/w in H₂O, 2.5 mL, 50 mmol) at room temperature. After stirring for 16 h, the mixture was diluted with EtOAc (40 mL) and filtered. The filter cake was washed with EtOAc (2 x 30 mL) and then added MeOH (40 mL). The resulting solution was concentrated *in vacuo* to afford **3-amino-1,2,4-triazin-5-ol intermediate 1131** (2 g, 71 %) as a white solid. ESI-MS [M +H]⁺: 113.1.

To a reaction mixture of **intermediate 1131** (0.67 g, 6.0 mmol) and (1S,2S)-2-(4-methyl pyrimidin-2-yl)cyclopropane-1-carboxylic acid (0.59 g, 5.0 mmol) in pyridine (9.0 mL) was added T₃P (4.7 g, 15 mmol). After stirring at room temperature for 16 h, the reaction mixture was diluted with water (50 mL) and extracted with EtOAc (50 mL). The organic phase was washed with brine, dried over Na₂SO₄, filtered and concentrated *in vacuo* and the residue was purified by column chromatography on silica gel (eluent: DCM/MeOH = 15/1) to give **(1S,2S)-N-(5-hydroxy-1,2,4-triazin-3-yl)-2-(4-methylpyrimidin-2-yl)cyclopropane-1-carboxamide intermediate 1132** (0.70 g, 78 %) as yellow solid. ESI-MS [M +H]⁺: 273.1.

To a mixture of **intermediate 1132** (54 mg, 0.20 mmol) and PPh₃ (0.16 g, 0.60 mol) in THF (10 mL) at 0 °C was added **intermediate 454** (90 mg, 0.30 mmol), followed by DEAD (0.14 g, 0.80 mmol). After stirring the mixture at room temperature under N₂ for 16 hours, the reaction was concentrated in vacuo to give the crude, which was purified with preparative TLC (eluent: DCM/MeOH = 18/1) to give **(I-671)** (15 mg, 14 %) as yellow oil. ESI-MS [M +H]⁺: 555., δ 11.35 (s, 1H), 8.81 (s, 1H), 8.52 (d, J = 5.1 Hz, 1H), 8.29 (s, 1H), 8.08 (s, 1H), 7.39 (d, J = 1.4 Hz, 1H), 7.19 (d, J = 5.1 Hz, 1H), 5.49 (s, 2H), 4.88 (s, 2H), 2.97 (s, 3H), 2.90 - 2.88 (m, 1H), 2.63 - 2.60 (m, 1H), 2.40 (s, 3H), 1.99 - 1.94 (m, 1H), 1.63 - 1.59 (m, 2H), 0.97 - 0.94 (m, 2H), 0.69 -0.65 (m, 2H).

### Example 672: (1S,2S)-N-(5-(1-(6-cyclopropyl-8-(3-methyl-2,4-dioxoimidazolidin-1-yl) imidazo[1,2-a]pyridin-2-yl)ethoxy)-1,2,4-triazin-3-yl)-2-(4-methylpyrimidin-2-yl) cyclopropane-1-carboxamide (I-672)

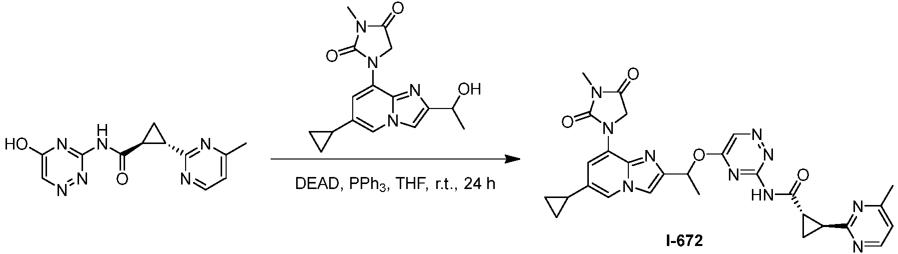

To a reaction mixture of **intermediate 1132** (54 mg, 0.20 mmol) and PPh₃ (0.16 g, 0.60 mmol) in THF (10 mL) was added 1-(6-cyclopropyl-2-(1-hydroxyethyl)imidazo[1,2-a]pyridin-8-yl)-3-methylimidazolidine-2,4-dione (94 mg, 0.30 mmol) at 0 °C. Then DEAD (0.14 g, 0.80 mmol) was added dropwise and the solution was stirred at room temperature under N₂ for 24 hours. The mixture was concentrated *in vacuo* to give the crude which was purified with preparative TLC (eluent: DCM/MeOH = 18/1) to give **(I-672)** (24 mg, 21 %) as yellow oil. ESI-MS [M +H] +: 569.1 δ 11.32 (s, 1H), 8.78 (s, 1H), 8.55 - 8.50 (m, 1H), 8.24 - 8.20 (m, 1H), 8.02 (d, J = 18.5 Hz, 1H), 7.41 - 7.36 (m, 1H), 7.22 - 7.19 (m, 1H), 6.42 - 6.38 (m, 1H), 4.95 - 4.84 (m, 2H), 2.97 (s, 3H), 2.85 - 2.83 (m, 1H), 2.65 - 2.59 (m, 1H), 2.41 (d, J = 5.6 Hz, 3H), 2.00 - 1.93 (m, 1H), 1.77 - 1.73 (m, 3H), 1.63 - 1.58 (m, 2H), 0.98 - 0.92 (m, 2H), 0.69 - 0.63 (m, 2H).

### Examples 673-674

### 2-(6-cyclopropyl-8-(3-methyl-2,4-dioxoimidazolidin-1-yl)imidazo[1,2-a]pyridin-2-yl)-1-(2-methyl-6-((1S,2S)-2-(4-methylpyrimidin-2-yl)cyclopropane-1-carboxamido)pyrimidin-4-yl)ethyl acetate (I-673) and (1S,2S)-N-(6-(2-(6-cyclopropyl-8-(3-methyl-2,4-dioxoimidazolidin-1-yl)imidazo[1,2-a]pyridin-2-yl)-1-hydroxyethyl)-2-methylpyrimidin-4-yl)-2-(4-methylpyrimidin-2-yl)cyclopropane-1-carboxamide (I-674)

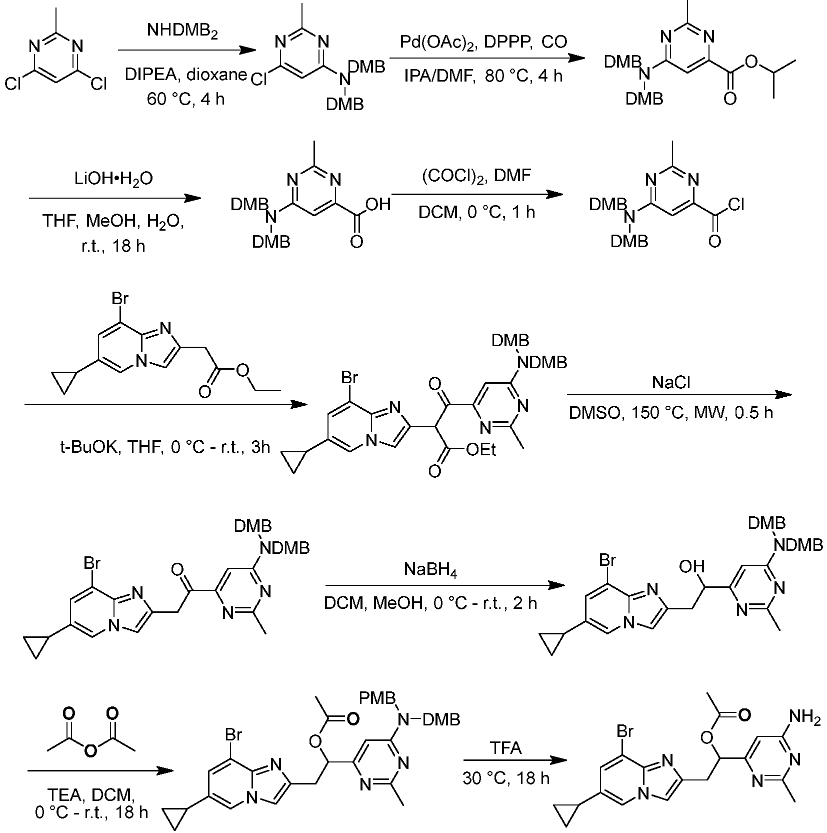

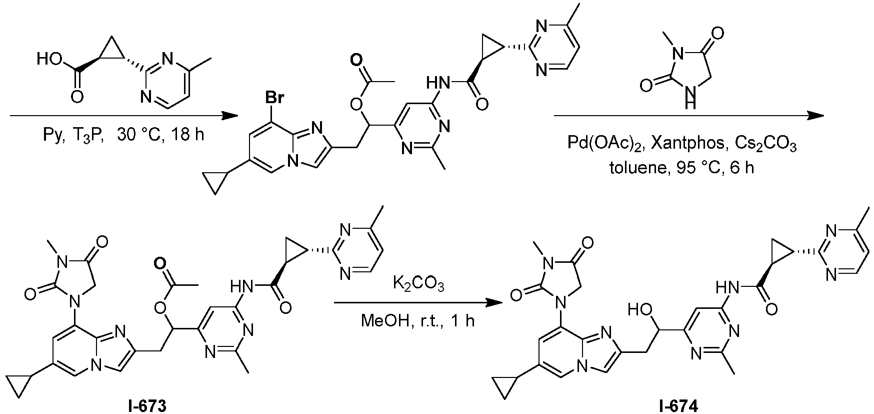

A mixture of 4,6-dichloro-2-methylpyrimidine (15 g, 93 mmol), bis(2,4-dimethoxy benzyl)amine (35 g, 0.11 mol), and DIPEA (36 g, 0.28 mol) in 1,4-dioxane (0.40 L) was stirred at 60 °C for 4 h. The mixture was quenched with saturated aq. NaHCO₃ (500 mL) and extracted with EtOAc (3 x 500 mL). The combined organic layers were washed with brine (500 mL), dried over Na₂SO₄ and concentrated *in vacuo.* The residue was purified by column chromatography on silica gel (eluent: EtOAc/PE = 0 - 30%) to afford **6-chloro-N,N-bis(2,4-dimethoxybenzyl)-2-methyl pyrimidin-4-amine intermediate 1133** (30 g, 73%) as an off-white solid. ESI-MS [M +H] +: 444.2

A mixture of **intermediate 1133** (10 g, 23 mmol), 1,3-Bis(diphenylphosphino)propane (0.95 g, 2.3 mmol), 1,3-Bis(diphenylphosphino)propane (0.95 g, 2.3 mmol), and Pd(OAc)₂ (0.52 g, 2.3 mmol) in IPA/DMF (10 mL/100 mL) was stirred at 80 °C for 4 h under CO. The mixture was quenched with water (1.0 L) and extracted with EtOAc (3 x 300 mL). The combined organic layers were washed with brine (300 mL), dried over Na₂SO₄ and concentrated *in vacuo.* The residue was purified by column chromatography on silica gel (eluent: EtOAc/PE = 0 - 30%) to afford **isopropyl 6-(bis(2,4-dimethoxybenzyl)amino)-2-methylpyrimidine-4-carboxylate intermediate 1134** (21g, 52% yield, for 3 batches) as an off-white solid. ESI-MS [M +H] +: 496.2

A mixture of **intermediate 1134** (10.0 g, 20.2 mmol) and LiOH•H₂O (1.7 g, 40 mmol) in THF/MeOH/ H₂O (100 mL/100 mL/100 mL) was stirred in at room temperature for 18 h. The mixture was adjusted to pH = 3 and extracted with IPA/CHCl₃ (5 x 300 mL). The combined organic layers were washed with brine (300 mL), dried over Na₂SO₄ and concentrated *in vacuo* to give 6-**(bis(2,4-dimethoxybenzyl)amino)-2-methylpyrimidine-4-carboxylic acid intermediate 1135** (9.0 g, crude) as a white solid, which was used in the next step directly. ESI-MS [M +H] +: 454.2

To a mixture of **intermediate 1135** (2.3 g, 5.0 mmol and oxalyl chloride (1.3 g, 10 mmol) in DCM (20 mL) was added DMF (one drop) and the mixture was stirred at room temperature for 1 h. The mixture was concentrated *in vacuo* to give **6-(bis(2,4-dimethoxybenzyl)amino)-2-methylpyrimidine-4-carbonyl chloride intermediate 1136** (2.5 g, crude) as a yellow solid, which was used in the next step without purification.

To a mixture of **intermediate 1017** (1.6 g, 5.0 mmol) in THF (30 mL) was added t-BuOK (16 mL, 16 mmol) at 0 °C and stirred at room temperature for 0.5 h. **Intermediate 1136** (2.5 g, 5.0 mmol) in THF (20 mL) was then added at 0 °C and the mixture was stirred at room temperature for 2.5 h. The reaction was quenched with saturated aq. NH₄Cl (100 mL) and extracted with EtOAc (3 x 50 mL). The combined organic layers were washed with brine (50 mL), dried over Na₂SO₄ and concentrated in vacuo to afford **ethyl 3-(6-(bis(2,4-dimethoxybenzyl)amino)-2-methylpyrimidin-4-yl)-2-(8-bromo-6-cyclopropylimidazo[1,2-a]pyridin-2-yl)-3-oxopropanoate intermediate 1137** (3.6 g, crude) as a brown solid which was used in next step directly. ESI-MS [M +H] +: 758.2

A mixture of **intermediate 1137(0.5** g, 0.66 mmol) and NaCl (46 mg, 0.79mmol) in DMSO/water (1.5 mL/0.10 mL) was stirred at 150 °C in a microwave reactor for 0.5 h. The mixture was quenched with water (7 x 200 mL) and extracted with EtOAc (3 x 50 mL). The combined organic layers were washed with brine (50 mL), dried over Na₂SO₄ and concentrated *in vacuo.* The residue was purified by column chromatography on silica gel (eluent: EtOAc/PE = 0 - 50%) to afford **1-(6-(bis(2,4-dimethoxybenzyl)amino)-2-methylpyrimidin-4-yl)-2-(8-bromo-6-cyclo propylimidazo[1,2-a]pyridin-2-yl)ethan-1-one intermediate 1138** (1.0 g, 32%, for 7 batches) as a brown solid. ESI-MS [M +H] +: 686.2

To a mixture of **intermediate 1138** (0.40 g, 0.58 mmol) in DCM/MeOH (10 mL/1.0 mL) was added NaBH₄ (44 mg, 1.2 mmol) at 0 °C. After stirring at room temperature for 2 h, the mixture was with poured into water (50 mL,) and extracted with EtOAc (3 x 30 mL). The combined organic layers were washed with brine (30 mL), dried over Na₂SO₄ and concentrated *in vacuo.* The residue was purified by column chromatography on silica gel (eluent: MeOH/DCM = 0 ~ 5 %) concentrated *in vacuo* to give **1-(6-(bis(2,4-dimethoxybenzyl)amino)-2-methylpyrimidin-4-yl)-2-(8-bromo-6-cyclopropylimidazo[1,2-a]pyridin-2-yl)ethan-1-ol intermediate 1139** (0.36 g, 90%) as a white solid. ESI-MS [M +H] +: 688.2

To a mixture of **intermediate 1139** (0.45 g, 0.66 mmol) and TEA ( 0.53 g, 5.3 mmol) in DCM (10 mL) was added acetic anhydride (0.34 g, 3.3 mmol) at 0 °C. After stirring at room temperature for 18 h, the mixture was quenched with saturated aq. NaHCO₃ (50 mL) and extracted with EtOAc (3 x 50 mL). The combined organic layers were washed with brine (50 mL), dried over Na₂SO₄ and concentrated in vacuo. The residue was purified by column chromatography on silica gel (eluent: MeOH/DCM = 0 - 3 %) to give **1-(6-(bis(2,4-dimethoxybenzyl)amino)-2-methyl pyrimidin-4-yl)-2-(8-bromo-6-cyclopropylimidazo[1,2-a]pyridin-2-yl)ethyl acetate intermediate 1140** (0.40 g, 83%) as a white solid. ESI-MS [M +H] +: 730.2

A mixture of **intermediate 1140** (0.42 g, 0.58 mmol) in TFA (5.0 mL) was stirred at 30 °C for 18 h. The mixture was quenched with saturated aq. NaHCO₃ (100 mL) and extracted with EtOAc (3 x 50 mL). The combined organic layers were washed with brine (30 mL), dried over Na₂SO₄ and concentrated *in vacuo.* The residue was purified by preparative TLC (eluent: MeOH/DCM = 1/12) to afford **1-(6-amino-2-methylpyrimidin-4-yl)-2-(8-bromo-6-cyclopropylimidazo[1,2-a]pyridin-2-yl)ethyl acetate intermediate 1141** (0.20 g, 81 %) as a white solid. ESI-MS [M +H] +: 430.1

To a mixture of **intermediate 1141** (0.20 g, 0.47 mmol), (1S,2S)-2-(4-methylpyrimidin-2-yl)cyclopropane-1-carboxylic acid (0.12 g, 0.70 mmol) in pyridine (1.0 mL) was added T₃P (3.0 g, 4.7 mmol) and the mixture was degassed with N₂. After stirring at 30 °C for 18 h, the mixture was quenched with water (50 mL) and extracted with EtOAc (3 x 30 mL). The combined organic layers were washed with brine (30 mL), dried over Na₂SO₄ and concentrated in vacuo. The residue was purified by preparative TLC (eluent: MeOH/DCM = 1/20) to afford **2-(8-bromo-6-cyclopropyl imidazo[1,2-a]pyridin-2-yl)-1-(2-methyl-6-((1S,2S)-2-(4-methylpyrimidin-2-yl)cyclopropane-1-carboxamido)pyrimidin-4-yl)ethyl acetate intermediate 1142** (0.18 g, 65 %) as a white solid. ESI-MS [M +H] +: 590.1

A mixture of **intermediate 1142** 2-(6-cyclopropyl-8-(3-methyl-2,4-dioxoimidazolidin-1-yl)imidazo[1,2-a]pyridin-2-yl)-1-(2-methyl-6-((1S,2S)-2-(4-methylpyrimidin-2-yl)cyclopropane-1-carboxamido)pyrimidin-4-yl)ethyl acetate (0.18 mg, 0.31 mmol), 3-methylimidazolidine-2,4-dione (0.11 mg, 0.92 mmol), Pd(OAc)₂ (14 mg, 0.062 mmol), Xantphos (53 mg, 0.092 mmol), and Cs₂CO₃ (0.20 mg, 0.62 mmol) in toluene (10 mL) was degassed with N₂ and stirred at 95 °C for 6 h. The mixture was quenched with water (50 mL) and extracted with EtOAc (3 x 30 mL). The combined organic layers were washed with brine (30 mL), dried over Na₂SO₄ and concentrated in vacuo. The residue was purified by preparative TLC (eluent: MeOH/DCM = 1/20) to afford (**I-673**) (0.10 g, 53 %) as a white solid. ESI-MS [M +H]+: 624.3. δ 11.32 (s, 1H), 8.53 (d, J = 5.1 Hz, 1H), 8.23 (s, 1H), 7.86 (s, 1H), 7.69 (s, 1H), 7.33 (d, J = 1.2 Hz, 1H), 7.21 (d, J = 5.0 Hz, 1H), 5.93-5.88 (m, 1H), 4.83 (s, 2H), 3.31 - 3.17 (m, 2H), 2.96 (s, 3H), 2.67 - 2.62 (m, 1H), 2.57 (s, 1H), 2.52 (s, 3H), 2.41 (s, 3H), 2.06 (s, 3H), 1.98-1.90 (m, 1H), 1.61 - 1.48 (m, 2H), 0.98-0.90 (m, 2H), 0.72-0.62 (m, 2H).

A mixture of 2-(6-cyclopropyl-8-(3-methyl-2,4-dioxoimidazolidin-1-yl)imidazo[1,2-a]pyridin-2-yl)-1-(2-methyl-6-((1S,2S)-2-(4-methylpyrimidin-2-yl)cyclopropane-1-carboxamido) pyrimidin-4-yl)ethyl acetate (60 mg, 0.096 mmol) and K₂CO₃ (27 mg, 0.19 mmol) in MeOH (5.0 mL) was stirred at room temperature for 1 h. The mixture was quenched with 1 N HCl (2.0 mL), then saturated aq. NaHCO₃ (50 mL) was added and the resulting mixture was extracted with EtOAc (3 x 30 mL). The combined organic layers were washed with brine (30 mL), dried over Na₂SO₄ and concentrated in vacuo. The residue was purified by preparative TLC (eluent: MeOH/DCM = 1/20) to afford (**I-674**)(25 mg, 45 %) as a white solid. ESI-MS [M +H]⁺: 582.0. δ 11.20 (s, 1H), 8.57 - 8.50 (m, 1H), 8.25 (d, J = 0.9 Hz, 1H), 8.08 (s, 1H), 7.67 (s, 1H), 7.30 (d, J = 1.1 Hz, 1H), 7.22 (d, J = 5.0 Hz, 1H), 5.64 (d, J = 5.1 Hz, 1H), 4.92-4.79 (m, 3H), 3.23-3.17 (m, 1H), 2.99-2.95 (m, 3H), 2.94-2.86 (m, 1H), 2.69-2.63 (m, 1H), 2.59 - 2.54 (m, 1H), 2.52 (s, 3H), 2.42 (s, 3H), 1.99-1.90 (m, 1H), 1.60-1.49 (m, 2H), 1.01 - 0.87 (m, 2H), 0.71 - 0.58 (m, 2H).

### Example 675: (1S,2S)-N-(6-((6-cyclopropyl-8-(3-methyl-2,4-dioxoimidazolidin-1-yl)imidazo[1,2-a]pyridin-2-yl)methoxy)-4-fluoropyridin-2-yl)-2-(4-methylpyrimidin-2-yl)cyclopropane-1-carboxamide (I-675)

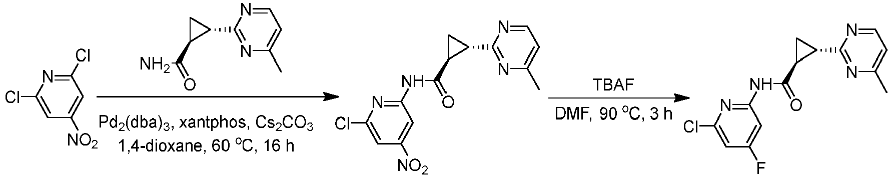

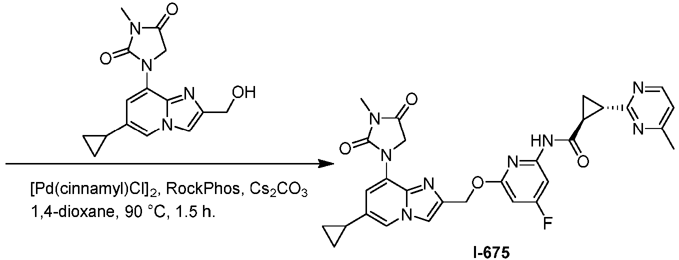

A mixture of 2,6-dichloro-4-nitropyridine (0.19 g, 1.0 mmol), (1S,2S)-2-(4-methylpyrimidin-2 -yl)cyclopropane-1-carboxamide (0.18 g, 1.0 mmol), and Cs₂CO₃ (0.98 g, 3.0 mmol) in 1,4-dioxane (10 mL) were added Pd₂(dba)₃ (46 mg, 0.050 mmol) and Xantphos (58 mg, 0.10 mmol). After stirring at 60 °C for 16 h under N₂, the reaction mixture was filtered through Celite^{®} and the filter cake was washed with DCM/MeOH (10/1, 30 mL). The filtrate was concentrated *in vacuo* to give the crude, which was purified by preparative TLC (eluent: DCM/MeOH = 20/1) to give **(1S,2S)-N-(6-chloro-4-nitropyridin-2-yl)-2-(4-methylpyrimidin-2-yl)cyclopropane-1-carboxamide intermediate 1143** (80 mg, 24%) as a yellow solid. ESI-MS [M +H]⁺: 334.2.

A mixture of **intermediate 1143** (0.10 g, 0.3 mmol) and TBAF (1M in THF, 0.75 mL) in DMF (5.0 mL) was stirred at 90 °C for 3 h under N₂. The reaction was quenched with water (30 mL) and extracted with EtOAc (3 x 30 mL). The combined organic layers were washed with brine (30 mL), dried over anhydrous Na₂SO₄, and concentrated *in vacuo* to give the crude product, which was purified by preparative TLC (eluent: DCM/MeOH = 30/1) to give **(1S,2S)-N-(6-chloro-4-fluoropyridin-2-yl)-2-(4-methylpyrimidin-2-yl)cyclopropane-1-carboxamide intermediate 1144** (55 mg, 60%) as a yellow solid. ESI-MS [M +H]⁺: 307.2.

To a mixture of **intermediate 1144** (40 mg, 0.13 mmol), 1-(6-cyclopropyl-2-(hydroxymethyl)imidazo[1,2-a] pyridin-8-yl)-3-methylimidazolidine-2,4-dione (20 mg, 0.11 mmol), and Cs₂CO₃ (0.11 mg, 0.33 mmol) in 1,4-dioxane (8.0 mL) were added Di-chlorobis[(1,2,3-)-1-phenyl-2-propenyl] dipalladium(II) (7.0 mg, 0.013 mmol) and RockPhos (12 mg, 0.026 mmol). After stirring at 90 °C for 1.5 h under N₂, the reaction mixture was filtered through Celite^{®} and the filter cake was washed with DCM/MeOH (10/1, 20 mL). The filtrate was concentrated in vacuo to give the crude, which was purified by preparative HPLC to give **(I-675)** (15 mg, 20%) as a white solid. ESI-MS [M +H]⁺: 571.2. δ 11.02 (s, 1H), 8.54 (d, J = 5.1 Hz, 1H), 8.42 (s, 1H), 8.10 (s, 1H), 7.63 - 7.39 (m, 2H), 7.23 (d, J = 5.1 Hz, 1H), 6.51 (dd, J = 9.6, 1.8 Hz, 1H), 5.47 (s, 2H), 4.80 (s, 2H), 2.97 (s, 3H), 2.62 - 2.52 (m, 2H), 2.43 (s, 3H), 2.06 - 1.92 (m, 1H), 1.56 (t, J = 7.2 Hz, 2H), 0.99 - 0.96 (m, 2H), 0.72 - 0.69 (m, 2H).

### Example 676: (1S,2S)-N-(5-((6-(1-fluorocyclopropyl)-8-(3-methyl-2,4-dioxoimidazolidin-1-ylimidazo[1,2-a]pyridin-2-yl)methoxy)pyridazin-3-yl)-2-(4-methylpyrimidin-2-yl)cyclopropane-1-carboxamide (I-676)

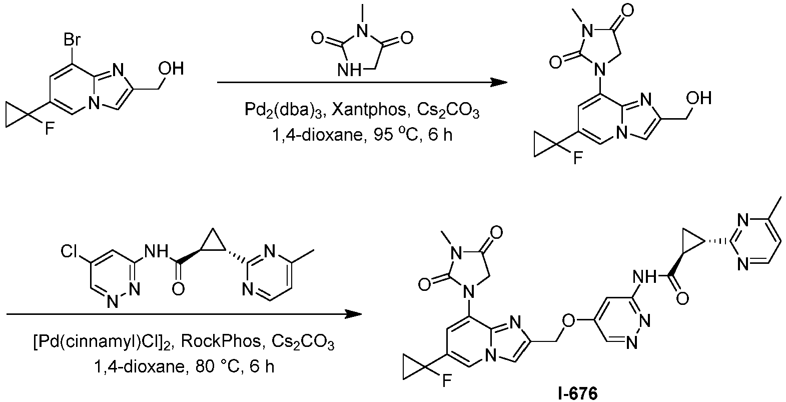

To a stirred solution of (8-bromo-6-(1-fluorocyclopropyl)imidazo[1,2-a]pyridin-2-yl)methanol (86 mg, 0.30 mmol), 3-methylimidazolidine-2,4-dione (0.10 g, 0.90 mmol), Xantphos (35 mg, 0.060 mmol), and Cs₂CO₃ (0.29 g, 0.90 mmol) in 1,4-dioxane (10 mL) was added Pd₂(dba)₃ (23 mg, 0.025 mmol). Then the reaction was stirred at 95 °C for 6 h and cooled to room temperature. The mixture was then concentrated *in vacuo* to give the crude, which was purified by column chromatography on silica gel (eluent: MeOH/DCM= 1/20) to give the **1-(6-(1-fluorocyclopropyl)-2-(hydroxymethyl)imidazo[1,2-a]pyridin-8-yl)-3-methylimidazolidine-2,4-dione intermediate 1145** (70 mg, 73 %) as yellow solid. ESI-MS [M +H]⁺: 319.1.

To a stirred solution of **intermediate 1145** (64 mg, 0.20 mmol), (1S,2S)-N-(5-chloropyridazin-3-yl)-2-(4-methylpyrimidin-2-yl)cyclopropane-1-carboxamide (87 mg, 0.30 mmol), RockPhos (19 mg, 0.040 mmol), and Cs₂CO₃ (0.20 mg, 0.60 mmol) in 1,4-dioxane (10 mL) was added Di-chlorobis[(1,2,3-)-1-phenyl-2-propenyl]dipalladium(II) (10 mg, 0.020 mmol). The resulting mixture was stirred at 80 °C for 6 h and then concentrated in vacuo to give the crude, which was purified with silica gel chromatography (eluent: (MeOH: DCM= 1:20) to give (I-676) (6 mg, 5 %) as white solid. ESI-MS [M +H]+: 572. δ 11.38 (s, 1H), 8.78 (d, J = 2.5 Hz, 1H), 8.59 (s, 1H), 8.54 (d, J = 5.1 Hz, 1H), 8.21 (d, J = 2.4 Hz, 1H), 8.12 (s, 1H), 7.66 (s, 1H), 7.22 (d, J = 5.1 Hz, 1H), 5.41 (s, 2H), 5.01 - 4.92 (m, 2H), 2.99 (s, 3H), 2.69 - 2.67(m, 1H), 2.58 - 2.55 (m, 1H), 2.42 (s, 3H), 1.57 - 1.45 (m, 4H), 1.16 (q, J = 7.8 Hz, 2H).

### Example 677: (1S,2S)-N-(5-((6-cyclopropyl-8-(3-methyl-2,4-dioxoimidazolidin-1-yl)imidazo[1,2-a]pyridin-2-yl)methoxy)-6-(hydroxymethyl)pyridazin-3-yl)-2-(4-methylpyrimidin-2-yl)cyclopropane-1-carboxamide (I-677)

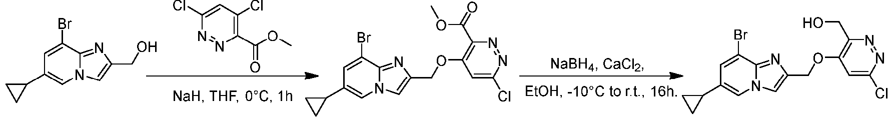

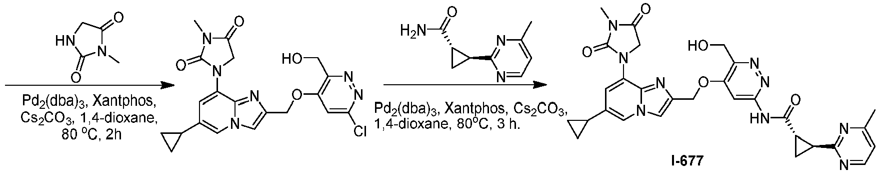

To a solution of **intermediate 453** (1.2 g, 4.5 mmol) in dry THF (20.0 mL) was added NaH (360 mg, 60% in mineral oil, 9.0 mmol) at 0 °C . After the reaction was stirred at 0 °C for 0.5 h, a solution of methyl 4,6-dichloropyridazine-3-carboxylate (1.38 g, 6.75 mmol) in dry THF (20.0 mL) was added and the resulting mixture was stirred at room temperature for another 2 h. The reaction was quenched with saturated aqueous NH₄Cl (50 mL) and extracted with EtOAc (50 mL x 3). The combined organic layers were washed with brine (50 mL), dried over anhydrous Na₂SO₄, and concentrated *in vacuo* to give the crude, which was purified by column chromatography (eluent: DCM/MeOH = 50/1) to give **methyl 4-((8-bromo-6-cyclopropylimidazo[1,2-a]pyridin-2-yl)methoxy)-6-chloropyridazine-3-carboxylate intermediate 1146** (1.7 g, 86%) as a yellow solid. ESI-MS [M +H]⁺: 438.1.

To a mixture of **intermediate 1146** (1.3 g, 3.0 mmol) and CaCl₂ (660 mg, 6.0 mmol) in dry THF (40.0 mL) was added NaBH₄ (338 mg, 8.9 mmol) at -10 °C . The resulting mixture was stirred at room temperature for 16 h. The reaction was quenched with saturated aqueous NH₄Cl (40 mL) and extracted with EtOAc (30 mL x 3). The combined organic layers were washed with brine (30 mL), dried over anhydrous Na₂SO₄, and concentrated *in vacuo* to give the crude, which was purified by column chromatography (eluent: DCM/MeOH = 20/1) to give **(4-((8-bromo-6-cyclopropylimidazo[1,2-a]pyridin-2-yl)methoxy)-6-chloropyridazin-3-yl)methanol intermediate 1147** (900 mg, 73%) as a yellow solid. ESI-MS [M +H]⁺: 410.2.

A mixture of **intermediate 1147** (410 mg, 1.0 mmol), 3-methylimidazolidine-2,4-dione (228 mg, 2.0 mmol), and Cs₂CO₃ (815 mg, 2.5 mmol) in 1,4-dioxane (20 mL) was added Pd₂(dba)₃ (46 mg, 0.05 mmol) and Xantphos (58 mg, 0.1 mmol). The reaction mixture was stirred at 80 °C for 2 h under N₂. The reaction mixture was filtered through Celite^{®} and the filter cake was washed with DCM/MeOH (10/1, 30 mL). The filtrate was concentrated in vacuo to give the crude, which was purified by preparative HPLC to give **1-(2-1((6-chloro-3-(hydroxymethyl)pyridazin-4-yl)oxy)methyl)-6-cyclopropylimidazo[1,2-a]pyridin-8-yl)-3-methylimidazolidine-2,4-dione intermediate 1148** (70 mg, 16%) as a white solid. ESI-MS [M +H]⁺: 443.2.

To a mixture of **intermediate 1148** (70 mg, 0.16 mmol), **intermediate 55** (28 mg, 0.16 mmol), and Cs₂CO₃ (129 mg, 0.39 mmol) in 1,4-dioxane (8 mL) was added Pd₂(dba)₃ (14.6 mg, 0.016 mmol) and Xantphos (18.3 mg, 0.032 mmol). The reaction mixture was stirred at 80 °C for 3 h under N₂. The reaction mixture was filtered through Celite^{®} and the filter cake was washed with DCM/MeOH (10/1, 20 mL). The filtrate was concentrated in vacuo to give the crude, which was purified by preparative TLC to give (**I-677**) (16 mg, 17%) as a white solid. ESI-MS [M +H]⁺: 584.2. δ 11.38 (s, 1H), 8.54 (d, J = 5.1 Hz, 1H), 8.30 (s, 2H), 8.01 (s, 1H), 7.41 (s, 1H), 7.22 (d, J = 5.1 Hz, 1H), 5.38 (s, 2H), 5.16 (t, J = 5.8 Hz, 1H), 5.03 - 4.82 (m, 2H), 4.63 (d, J = 3.9 Hz, 2H), 2.99 (s, 3H), 2.56 - 2.54 (m, 2H), 2.42 (s, 3H), 2.00 - 1.94 (m, 1H), 1.56 (t, J = 7.2 Hz, 2H), 1.03 - 0.87 (m, 2H), 0.69 - 0.65 (m,, 2H).

**Intermediate 1149:** herein is rac-(1*S**,2*S**)-2-(3-chlorophenyl)cyclopropane-1-carboxylic acid.

### Example 678: (1S,2S)-N-(6-(((R)-1-(6-(fluoromethyl)-8-(3-methyl-2,4-dioxoimidazolidin-1-yl)imidazo[1,2-a]pyridin-2-yl)ethyl)amino)pyrimidin-4-yl)-2-(4-methylpyrimidin-2-yl)cyclopropane-1-carboxamide (I-678)

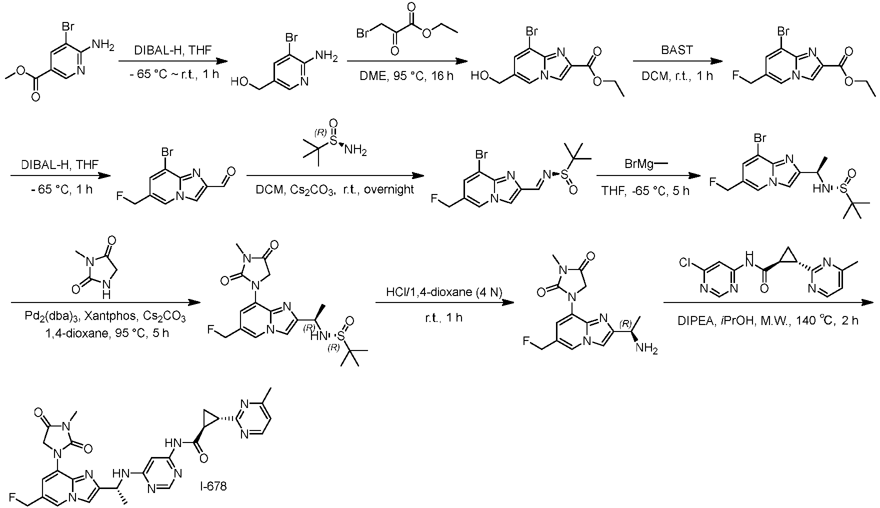

To a solution of methyl 6-amino-5-bromonicotinate (3.0 g, 13 mmol) in THF (30 mL) was added DIBAL-H (36 mL, 1.0 N in hexane, 36 mmol) dropwise at -65 °C. After stirring at -65 °C for 0.5 h, the mixture was warmed to room temperature and stirred for another 0.5 h. The reaction mixture was quenched with NaOH (1.0 M aq., 36 mL) and then extracted with EtOAc (50 mL x 3). The combined organics were washed with brine (100 mL), dried over Na₂SO₄, and concentrated *in vacuo.* The residue was purified by column chromatography on silica gel, eluting with 0~20% EtOAc in PE to give **(6-amino-5-bromopyridin-3-yl)methanol** (2.4 g, yield: 91%) as a white solid. ESI-MS [M +H]⁺: 203.0.

A mixture of ethyl 3-bromo-2-oxopropanoate (3.3 g, 17 mmol) and (6-amino-5-bromopyridin-3-yl)methanol (2.4 g, 12 mmol) in DME (50 mL) was stirred at 95 °C for 24 h. The mixture was cooled to room temperature and concentrated *in vacuo.* The residue was diluted with NaHCO₃ (sat. aq., 50 mL) and then extracted with DCM (50 mL x 3). The combined organic layers were washed with brine (30 mL), dried over Na₂SO₄, and concentrated *in vacuo.* The residue was purified by column chromatography on silica gel, eluting with 0~40% EtOAc in PE to give **ethyl 8-bromo-6-(hydroxymethyl)imidazo[1,2-a]pyridine-2-carboxylate** (1.0 g, yield: 28%) as a brown solid. ESI-MS [M +H]+: 299.0

To a solution of ethyl 8-bromo-6-(hydroxymethyl)imidazo[1,2-a]pyridine-2-carboxylate (1.0 g, 3.3 mmol) in DME (20 mL) was added BAST (1.8 mL, 10 mmol) at 0°C. After stirring at room temperature for 12 h, the reaction mixture was quenched with NaHCO₃ (Sat. aq., 50 mL) and extracted with DCM (50 mL x 3). The combined organics were washed with brine (30 mL), dried over Na₂SO₄, and concentrated *in vacuo.* The residue was purified by column chromatography on silica gel, eluting with 10~20% EtOAc in PE to give **ethyl 8-bromo-6-(fluoromethyl)imidazo[1,2-a]pyridine-2-carboxylate** (0.55 g, yield: 55%) as a white solid. ESI-MS [M +H]+: 301.0

To a solution of ethyl 8-bromo-6-(fluoromethyl)imidazo[1,2-a]pyridine-2-carboxylate (0.55 g, 1.8 mmol) in THF (20 mL) was added DIBAL-H (5.4 mL, 1.0 M in hexane, 5.4 mmol) at - 65 °C. After stirring at -65 °C for 1 h, the reaction mixture was quenched by adding sodium sulfate decahydrate at -65 °C, warmed to 0°C and stirred for 10 min. The reaction mixture was filtered and the filtrate was concentrated to give residue, which was purified by column chromatography on silica gel, eluting with 0~50% EtOAc in PE to give **8-bromo-6-(fluoromethyl)imidazo[1,2-a]pyridine-2-carbaldehyde** (0.30 g, yield: 65%) as a white solid. ESI-MS [M + H]⁺: 257.1.

A mixture of 8-bromo-6-(fluoromethyl)imidazo[1,2-a]pyridine-2-carbaldehyde (0.30 g, 1.2 mmol), (*R*)-2-methylpropane-2-sulfinamide (0.17 g, 1.4 mmol) and Cs₂CO₃ (0.78 g, 2.4 mmol) in DCM (25 mL) was stirred at room temperature overnight. The resulting mixture was filtered and the filtrate was concentrated *in vacuo.* The residue was purified by column chromatography on silica gel, eluting with 0~50% EtOAc in PE to give **(*R*,E)-N-((8-bromo-6-(fluoromethyl)imidazo[1,2-a]pyridin-2-yl)methylene)-2-methylpropane-2-sulfinamide** (0.30 g, yield: 69%) as a pale-yellow solid. ESI-MS [M +H]+: 360.1

To a solution of (*R*,E)-N-((8-bromo-6-(fluoromethyl)imidazo[1,2-a]pyridin-2-yl)methylene)-2-methylpropane-2-sulfinamide (0.30 g, 0.83 mmol) in THF (10 mL) was added methylmagnesium bromide (0.83 mL, 3 N in ether, 2.5 mmol) at -65 °C and the mixture was stirred at the same temperature for 5h. The reaction mixture was quenched with NH₄Cl (Sat. aq., 20 mL) at -65°C and then extracted with EtOAc (30 mL x 3). The combined organics were washed with brine (20 mL), dried over Na₂SO₄, and concentrated *in vacuo.* The residue was purified by column chromatography on silica gel, eluting with 0~50% MeOH in DCM to give **(*R*)-N-((*R*)-1-(8-bromo-6-(fluoromethyl) imidazo[1,2-a]pyridin-2-yl)ethyl)-2-methylpropane-2-sulfinamide** (0.20 g, yield: 64%) as pale-yellow solid. ESI-MS [M + H]⁺: 376.1.

A mixture of (R)-N-((R)-1-(8-bromo-6-(fluoromethyl)imidazo[1,2-a]pyridin-2-yl)ethyl)-2-methylpropane-2-sulfinamide (101 mg, 0.27 mmol), 3-methylimidazolidine-2,4-dione (160 mg, 1.4 mmol), Pd₂(dba)₃ (38 mg, 0.041 mmol), Xantphos (47 mg, 0.082 mmol) and Cs₂CO₃ (222 mg, 0.68 mmol) in 1,4-dioxane (10 mL) was stirred at 95 °C for 5 h under N₂ atmosphere. The mixture was cooled to room temperature and filtered through celite^{®}. The filter cake was washed with DCM/MeOH (5/1, 30 mL) and the filtrate was concentrated in vacuo. The residue was purified by column chromatography on silica gel, eluting with 0~4% MeOH in DCM to give **(R)-N-((R)-1-(6-(fluoromethyl)-8-(3-methyl-2,4-dioxoimidazolidin-1-yl)imidazo[1,2-a]pyridin-2-yl)ethyl)-2-methylpropane-2-sulfinamide** (100 mg, yield: 90%) as pale-yellow solid. ESI-MS [M +H]+: 410.2

A solution of (R)-N-((R)-1-(6-(fluoromethyl)-8-(3-methyl-2,4-dioxoimidazolidin-1-yl)imidazo[1,2-a]pyridin-2-yl)ethyl)-2-methylpropane-2-sulfinamide (100 mg, 0.24 mmol) and HCl (4 N in 1,4-dioxane, 3 mL) in DCM (5 mL) was stirred at room temperature for 1 h. The mixture was concentrated, and the residue was diluted with NH₃ (7 N in MeOH, 5 mL) at 0 °C and stirred at room temperature for 30 min. The resulting mixture was then then concentrated *in vacuo,* the residue was purified by Prep-TLC (eluting with DCM/MeOH=10:1) to give **(R)-1-(2-(1-aminoethyl)-6-(fluoromethyl)imidazo[1,2-a]pyridin-8-yl)-3-methylimidazolidine-2,4-dione** (50 mg, yield: 68%) as white solid.ESI-MS [M +H]+: 306.1

A mixture of (R)-1-(2-(1-aminoethyl)-6-(fluoromethyl)imidazo[1,2-a]pyridin-8-yl)-3-methylimidazolidine-2,4-dione (50 mg, 0.16 mmol), DIPEA (0.21 g, 1.6 mmol) and (1S,2S)-N-(6-chloropyrimidin-4-yl)-2-(4-methylpyrimidin-2-yl)cyclopropane-1-carboxamide (72 mg, 0.25 mmol) in iPrOH (2.0 mL) was stirred in sealed tube. After degassing with N₂ for 1 min, the mixture was irradiated in microwave at 140 °C for 2 h. The mixture was cooled to room temperature and concentrated in vacuo. The residue was diluted with H₂O (15 mL) and extracted with EtOAc (30 mL x 2). The combined organics were washed with brine (10 mL), dried over Na₂SO₄, and concentrated in vacuo. The residue was purified by Prep-HPLC to give (**I-678**) (13.8 mg, yield: 15%) as a white solid. ESI-MS [M +H]+: 559.1. Purity: 99.4% (214 nm), 99.5% (254 nm) 1H NMR (400 MHz, DMSO) δ 10.66 (s, 1H), 8.55 (d, J = 2.8 Hz, 1H), 8.52 (d, J = 5.1 Hz, 1H), 8.16 (s, 1H), 7.83 (s, 2H), 7.66 (s, 1H), 7.32 (s, 1H), 7.21 (d, J = 5.1 Hz, 1H), 5.48 - 5.36 (m, 3H), 5.03 - 4.94 (m, 2H), 2.98 (s, 3H), 2.64 - 2.55 (m, 2H), 2.41 (s, 3H), 1.57 - 1.49 (m, 5H).

### Example 679: (1S,2S)-N-(6-(((R)-1-(6-isopropyl-8-(3-methyl-2,4-dioxoimidazolidin-1-yl) imidazo[1,2-a]pyridin-2-yl)ethyl)amino)pyrimidin-4-yl)-2-(4-methylpyrimidin-2-yl)cyclopropane-1-carboxamide (I-679)

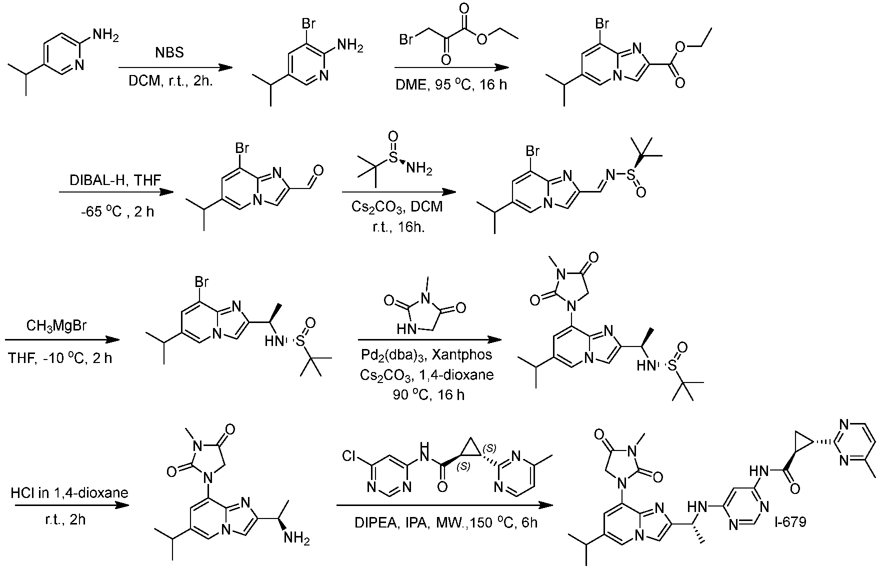

A mixture of 5-isopropylpyridin-2-amine (2.0 g, 14.7 mmol) and NBS (3.1 g, 17.6 mmol) in DCM (40 mL) was stirred at room temperature for 2 h. Water (50 mL) was added and the mixture was extracted with EtOAc (30 mL X 3). The combined organic layers were washed with brine (60 mL), dried over anhydrous Na₂SO₄, and concentrated *in vacuo* to give the crude, which was purified by column chromatography (eluent: PE/EtOAc = 3/1) to give **3-bromo-5-isopropylpyridin-2-amine** (3.0 g, yield: 95%) as a yellow oil. ESI-MS [M +H] +: 215.1.

A mixture of 3-bromo-5-isopropylpyridin-2-amine (3.0 g, 14.0 mmol) and ethyl 3-bromo-2-oxopropanoate (8.1 g, 42.0 mmol) in DME (50 mL) was stirred at 95 °C for 16 h. The mixture was cooled to room temperature and concentrated *in vacuo.* The residue was diluted with NaHCO₃ (sat. aq., 50 mL) and extracted with DCM (50 mL x 3). The combined organic layers were washed with brine (30 mL), dried over Na₂SO₄, and concentrated *in vacuo,* the residue was purified by column chromatography (eluent: PE/EtOAc = 3/1) to give **ethyl 8-bromo-6-isopropylimidazo[1,2-a]pyridine-2-carboxylate** (2.6 g, yield: 60%) as a brown oil. ESI-MS [M +H]+: 311.2.

To a solution of ethyl 8-bromo-6-isopropylimidazo[1,2-a]pyridine-2-carboxylate (550 mg, 1.8 mmol) in THF (15 mL) was added DIBAL-H (5.4 mL, 1.0 M in hexane, 5.4 mmol) at -65 °C under N₂. After the rection mixture was stirred at -65 °C for 2 h, the mixture was quenched with NaOH (1M aq., 10 mL) and extracted with EtOAc (30 mL X 3). The combined organic layers were washed with brine (20 mL), dried over anhydrous Na₂SO₄, and concentrated to give the crude, which was purified by column chromatography (eluent: PE/EtOAc = 5/1) to give **8-bromo-6-isopropylimidazo[1,2-a]pyridine-2-carbaldehyde** (350 mg, yield: 73%) as a yellow solid. ESI-MS [M +H]+: 267.1.

A mixture of 8-bromo-6-isopropylimidazo[1,2-a]pyridine-2-carbaldehyde (350 mg, 1.32 mmol), (R)-2-methylpropane-2-sulfinamide (240 mg, 1.98 mmol) and Cs₂CO₃ (1.3 g, 3.96 mmol) in DCM (30 mL) was stirred at room temperature for 16h. The resulting mixture was filtered and the filtrate was concentrated *in vacuo.* The residue was purified by column chromatography (eluent: PE/EtOAc = 3/1) to give **(R,E)-N-((8-bromo-6-isopropylimidazo[1,2-a]pyridin-2-yl)methylene)-2-methylpropane-2-sulfinamide** (450 mg, yield: 92%) as a yellow solid. ESI-MS [M +H]+: 372.1.

To a solution of (R,E)-N-((8-bromo-6-isopropylimidazo[1,2-a]pyridin-2-yl)methylene)-2-methylpropane-2-sulfinamide (450 mg, 1.2 mmol) in THF (10 mL) was added methylmagnesium bromide (2.0 mL, 3 M in ether, 6.0 mmol) at -10 °C under N₂ and the reaction mixture was stirred at -10 °C for 2 h. The mixture was quenched with NH₄Cl (sat. aq., 30 mL) and extracted with EtOAc (30 mL X 3). The combined organic layers were washed with brine (30 mL), dried over anhydrous Na₂SO₄, and concentrated to give the crude product, which was purified by column chromatography (eluent: DCM/MeOH = 20/1) to give **(R)-N-((R)-1-(8-bromo-6-isopropylimidazo[1,2-a]pyridin-2-yl)ethyl)-2-methylpropane-2-sulfinamide** (300 mg, yield: 65%) as a yellow solid. ESI-MS [M +H]+: 388.1.

To a mixture of (R)-N-((R)-1-(8-bromo-6-isopropylimidazo[1,2-a]pyridin-2-yl)ethyl)-2-methylpropane-2-sulfinamide (300 mg, 0.78 mmol), 3-methylimidazolidine-2,4-dione (133 mg, 1.17 mmol) and Cs₂CO₃ (763 mg, 2.34 mmol) in 1,4-dioxane (15 mL) was added Pd₂(dba)₃ (71 mg, 0.078 mmol) and Xantphos (90 mg, 0.16 mmol). The mixture was stirred at 90 °C for 16h under N₂. The reaction mixture was cooled to room temperature and filtered through celite^{®}, the filter cake was washed with DCM/MeOH (10/1, 30 mL). The filtrate was concentrated in vacuo, the residue was purified by column chromatography (eluent: DCM/MeOH = 20/1) to give **(R)-N-((R)-1-(6-isopropyl-8-(3-methyl-2,4-dioxoimidazolidin-1-yl)imidazo[1,2-a]pyridin-2-yl)ethyl)-2-methyl propane-2-sulfinamide** (80 mg, yield: 24%) as a yellow solid. ESI-MS [M +H]+: 420.2.

To a solution of (R)-N-((R)-1-(6-isopropyl-8-(3-methyl-2,4-dioxoimidazolidin-1-yl)imidazo[1,2-a]pyridin-2-yl)ethyl)-2-methylpropane-2-sulfinamide (80 mg, 0.19 mmol) in DCM (5 mL) was added HCl (4M solution in 1,4-dioxane, 2 mL) and the reaction mixture was stirred at room temperature for 2h. The mixture was concentrated, and the residue was diluted with NH₃ (7M solution in MeOH, 5 mL). The resulting mixture was concentrated *in vacuo,* the residue was purified by Prep-TLC (eluent: DCM/MeOH = 10/1) to give **(R)-1-(2-(1-aminoethyl)-6-isopropylimidazo[1,2-a]pyridin-8-yl)-3-methylimidazolidine-2,4-dione** (50 mg, yield: 83%) as a yellow solid. ESI-MS [M +H]+: 316.2.

A mixture of (R)-1-(2-(1-aminoethyl)-6-isopropylimidazo[1,2-a]pyridin-8-yl)-3-methylimidazolidine-2,4-dione (50 mg, 0.16 mmol), (1S,2S)-N-(6-chloropyrimidin-4-yl)-2-(4-methylpyrimidin-2-yl)cyclopropane-1-carboxamide (69 mg, 0.24 mmol) and DIPEA (103 mg, 0.80 mmol) in i-PrOH (5 mL) was stirred in a sealed tube. After degassing with N₂ for 1min, the reaction was irradiated in microwave at 150 °C for 6h. The mixture was cooled to room temperature and concentrated to give the crude, which was purified by Prep-TLC (eluent: DCM/MeOH = 20/1) to give **(I-679)**de (36 mg, yield: 40%) as a white solid. ESI-MS [M +H]+: 569.2. Purity: 99.16 (214 nm), 99.18 (254 nm). 1H NMR (400 MHz, DMSO) δ 10.65 (s, 1H), 8.52 (d, J = 5.1 Hz, 1H), 8.25 (s, 1H), 8.15 (d, J = 0.8 Hz, 1H), 7.78 (s, 1H), 7.69 (s, 1H), 7.52 (d, J = 1.4 Hz, 1H), 7.30 (s, 1H), 7.21 (d, J = 5.1 Hz, 1H), 5.32 (s, 1H), 4.95 (d, J = 1.5 Hz, 1H), 2.98 (s, 3H), 2.92-2.85 (m, 1H), 2.60-2.52 (m, 2H), 2.41 (s, 3H), 1.52-1.50 (m, 5H), 1.22 (d, J = 6.9 Hz, 6H).

### Example 680

### Inhibitory Activity of Exemplary Compounds against Plasma Kallikrein.

Compounds were evaluated for inhibition of the human activated kallikrein enzyme in two formats of an assay employing a fluorogenic peptide substrate. In one assay format, the concentrations of reagents were as follows: 20 mM Tris pH 7.5, 1 mM EDTA, 150 mM sodium chloride, 0.1% PEG-400, 0.1% Triton X-100, 500 pM activated kallikrein enzyme, 300 uM Pro-Phe-Arg-7-amido-4-methylcoumarin (PFR-AMC) substrate. Prior to reaction initiation with substrate, enzyme and inhibitors were preincubated for 30 min at room temperature. After initiation with substrate, reactions were incubated for 10 min at room temperature and fluorescence emission at 460 nm from 380 nm excitation measured with a microplate reader. In another assay format, the concentrations of reagents were as follows: 20 mM Tris pH 7.5, 1 mM EDTA, 150 mM sodium chloride, 0.1% PEG-400, 0.1% Triton X-100, 5 pM activated kallikrein enzyme, 300 uM PFR-AMC substrate. Prior to reaction initiation with substrate, enzyme and inhibitors were preincubated for 30 min at room temperature. After initiation with substrate, reactions were incubated for 18 hr at room temperature and fluorescence emission at 460 nm from 380 nm excitation measured with a microplate reader.

Table 49 provides the results of the assay in the format with 500 pM activated kallikrein assay. For the compounds listed in Table 49, the EC₅₀ values are reported according to the following ranges: A ≤ 1.0 nM; 1.0 nM < B ≤ 10 nM; 10 nM < C ≤ 100 nM; 100 nM < D ≤ 1000 nM; 1000 nM < E ≤ 6000 nM; 6000 nM < F.

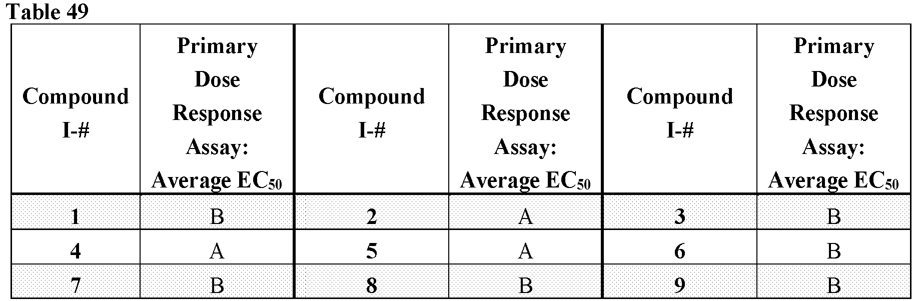

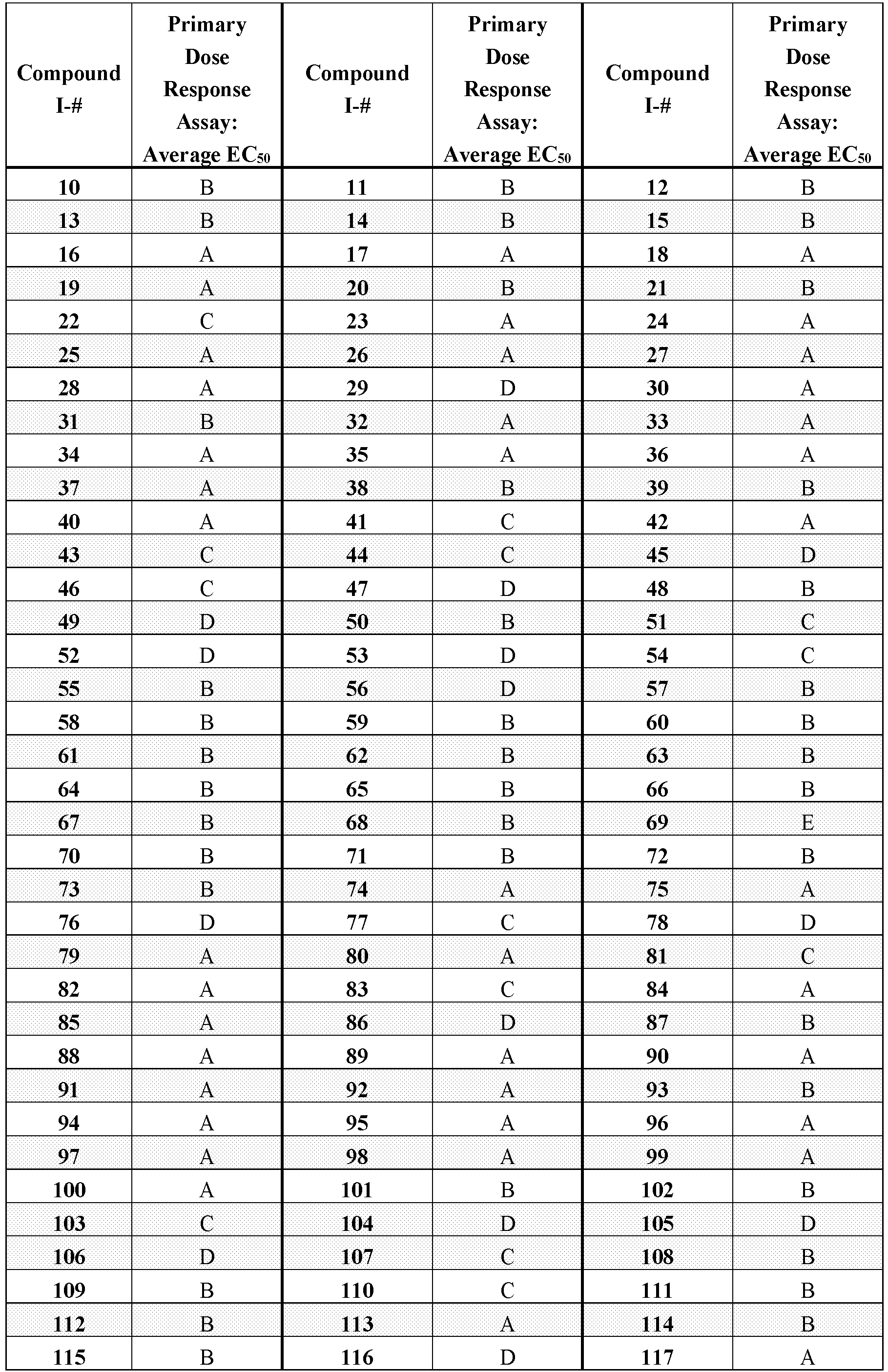

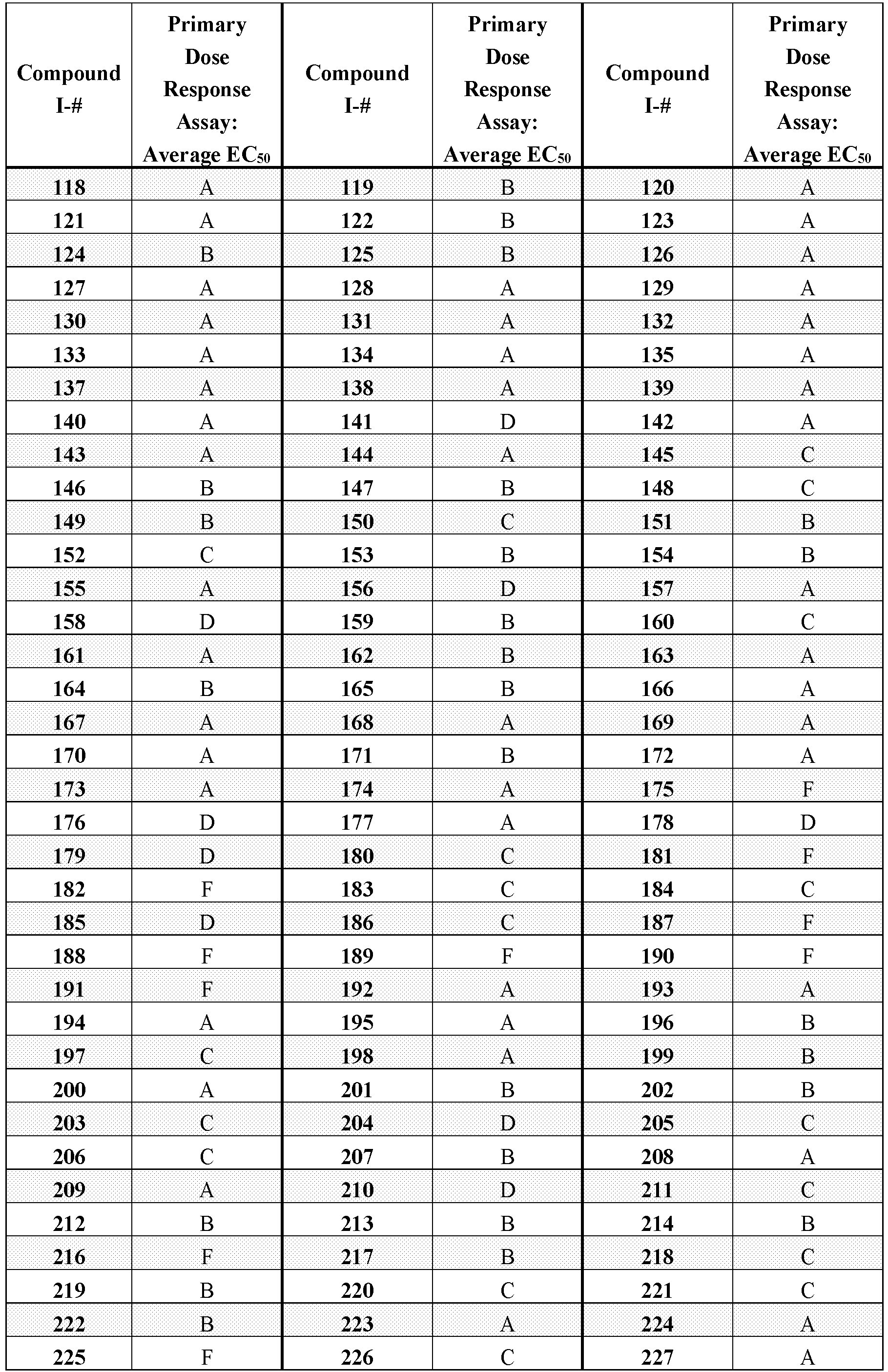

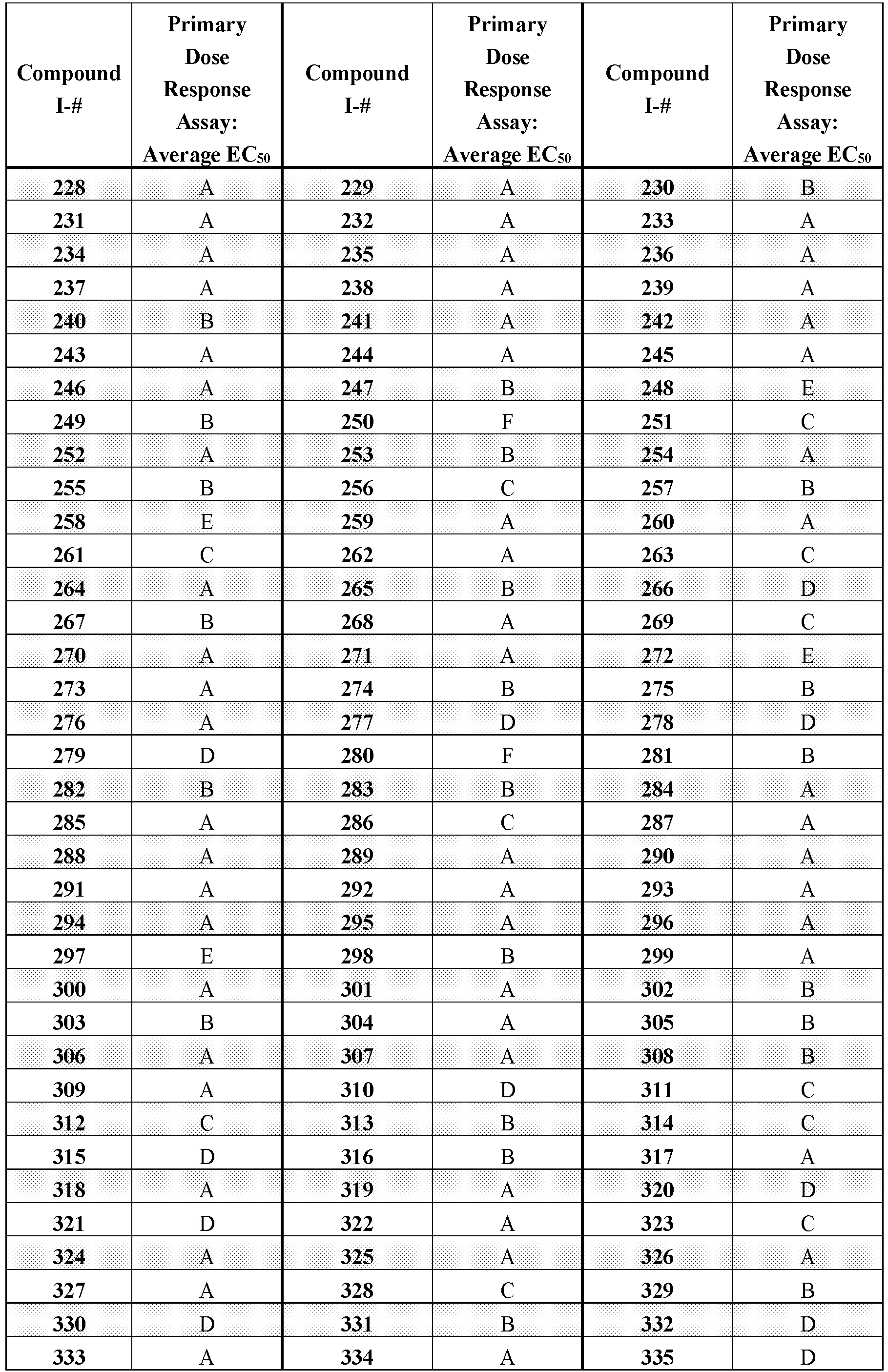

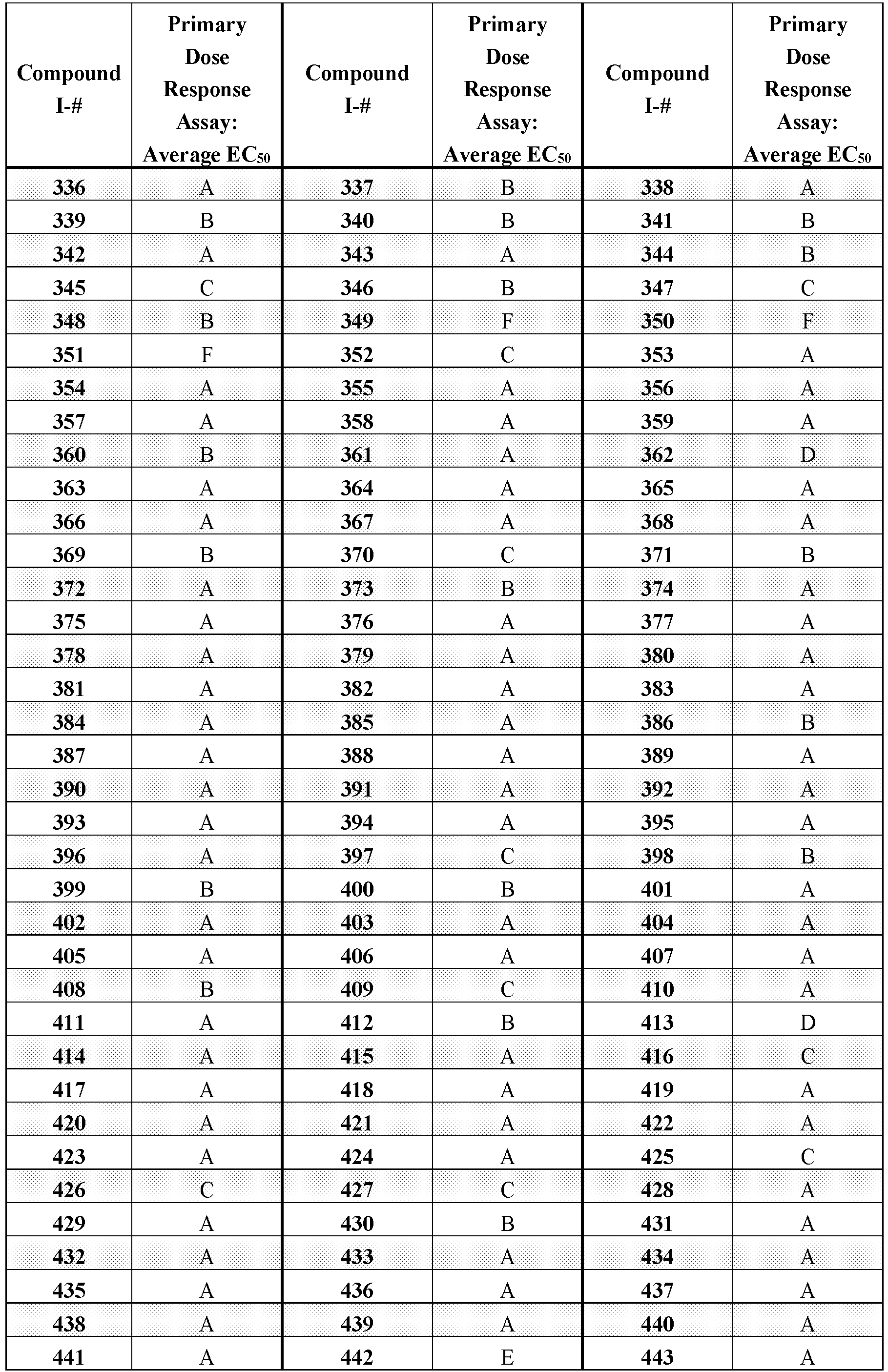

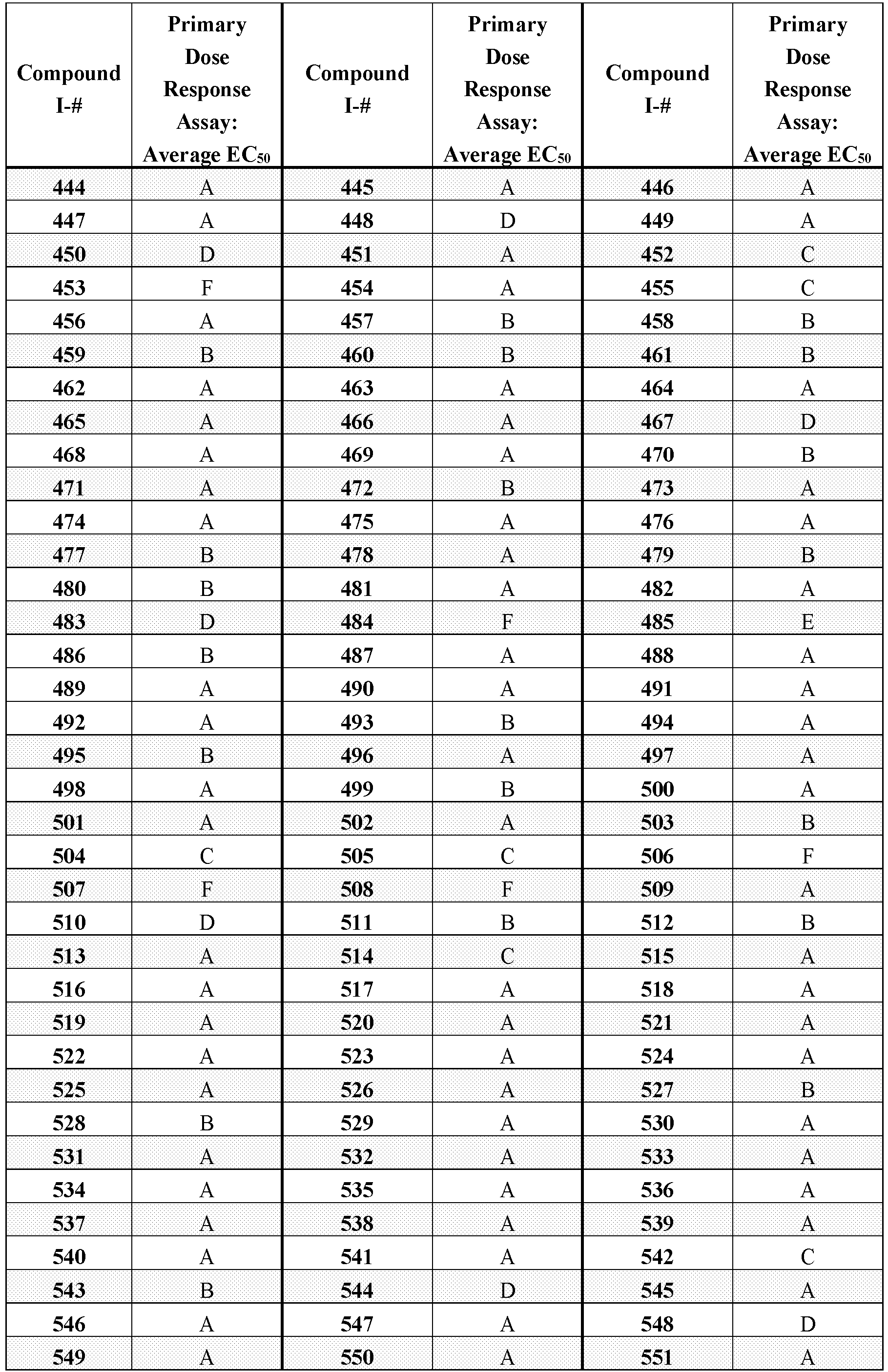

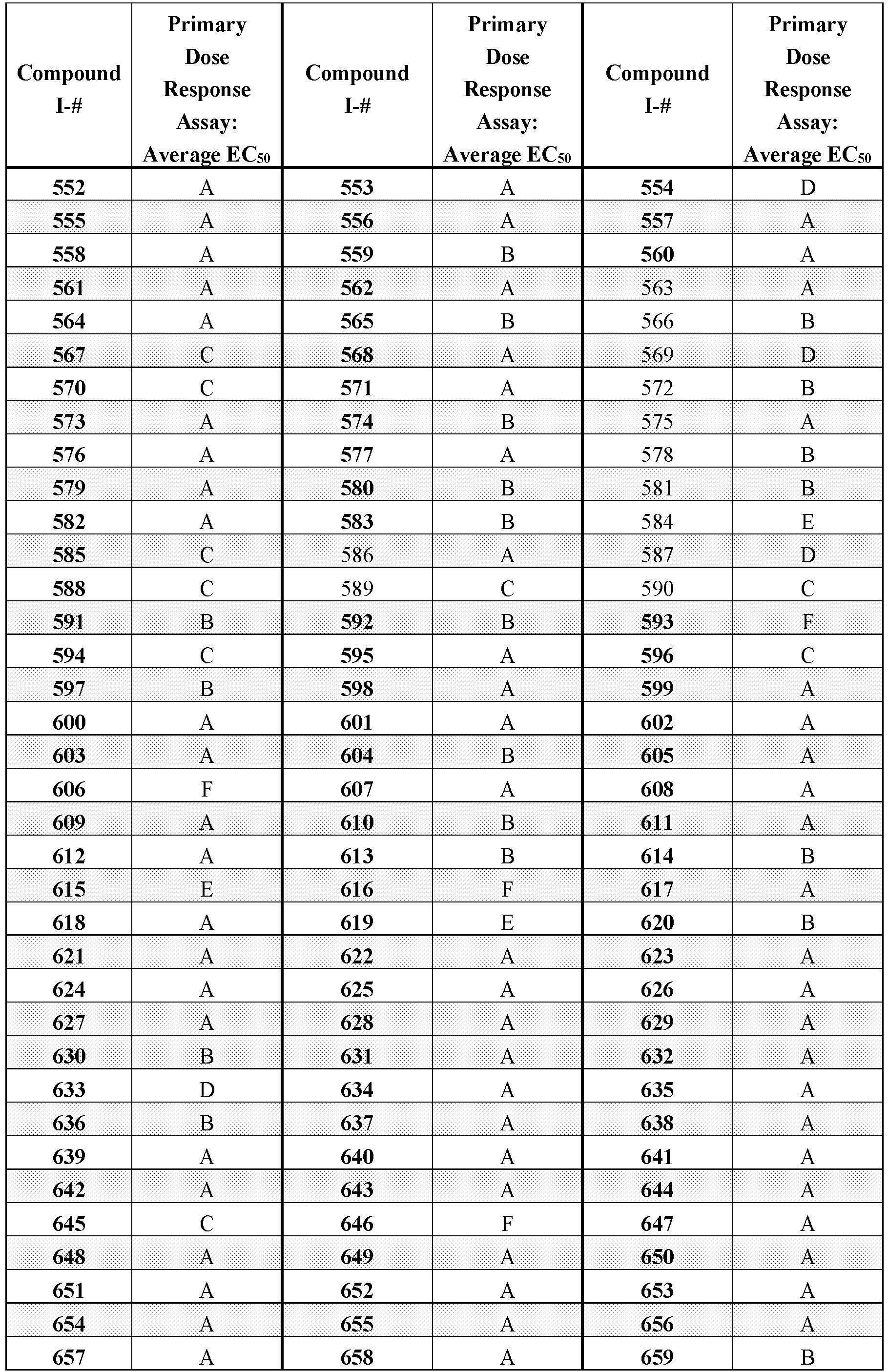

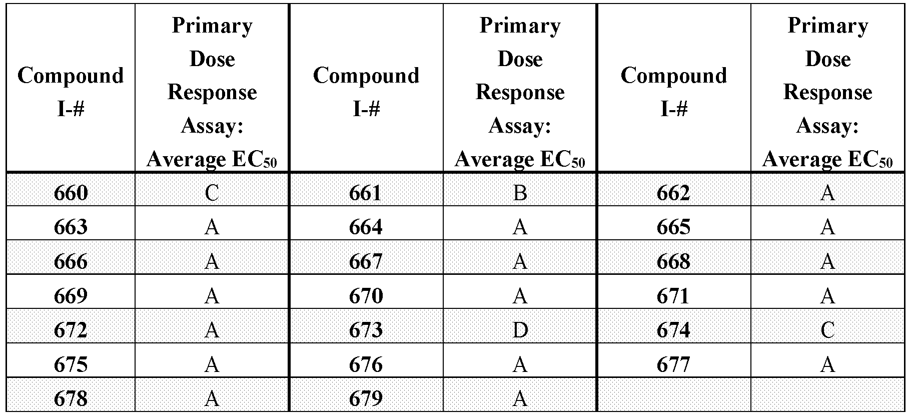

### Example 681

### Neat human and rat plasma assay

To analyze inhibition of plasma kallikrein in an ex vivo setting, the potency of compounds was measure in contact pathway-activated plasma assays. In a fluorogenic peptide substrate assay, test compounds dissolved in DMSO were added to sodium citrate collected human or rat plasma in a 96-well microplate. Alternatively, citrated plasma was collected from rats administered the compounds orally or by IV. 10 nM of human FXIIa (Enzyme Research Laboratories) diluted in pKal buffer (20 mM Tris-HCl, pH 7.5, 150 mM NaCl, 1 mM EDTA, 0.1% PEG-8000, and 0.1% Triton X-100) was added to the plasma, followed by the 100 µM of the profluorescent, synthetic plasma kallikrein substrate PFR-AMC (also diluted in pKal buffer). Final plasma concentration in the reaction was 78%. Fluorescence was immediately monitored by excitation/emission wavelengths of 360 nm/480 nm respectively over a period of 5 minutes in a microplate reader. The resulting linear increase in fluorescence emission (reflecting pKal proteolysis of PFR-AMC substrate) was fit to extract a proteolytic rate (fluorescent units over time), and this rate was subsequently plotted against compound inhibitor concentration. Resulting plots were fit to a standard 4-parameter IC50/IC90 equation to determine min/max values, IC50/90, and slope. All experimental steps were performed at room temperature. Table 50 provides results of the assay.

For the compounds listed in Table 50, the IC₉₀ values are reported according to the following ranges: A ≤ 400 nM; 400 nM < B ≤ 1500 nM; 1500 nM < C ≤ 10000 nM; 10000 nM < D ≤ 48000 nM; 48000 nM < E.

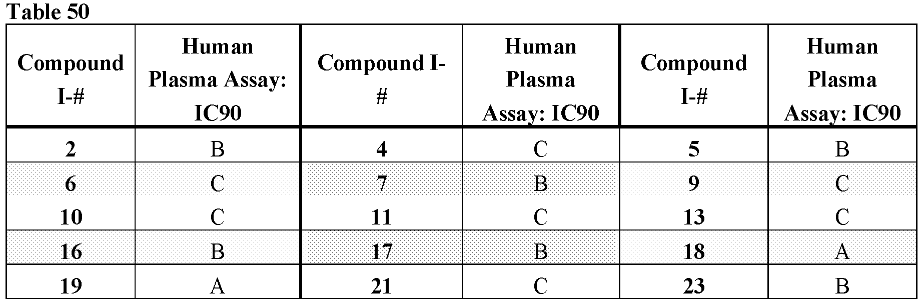

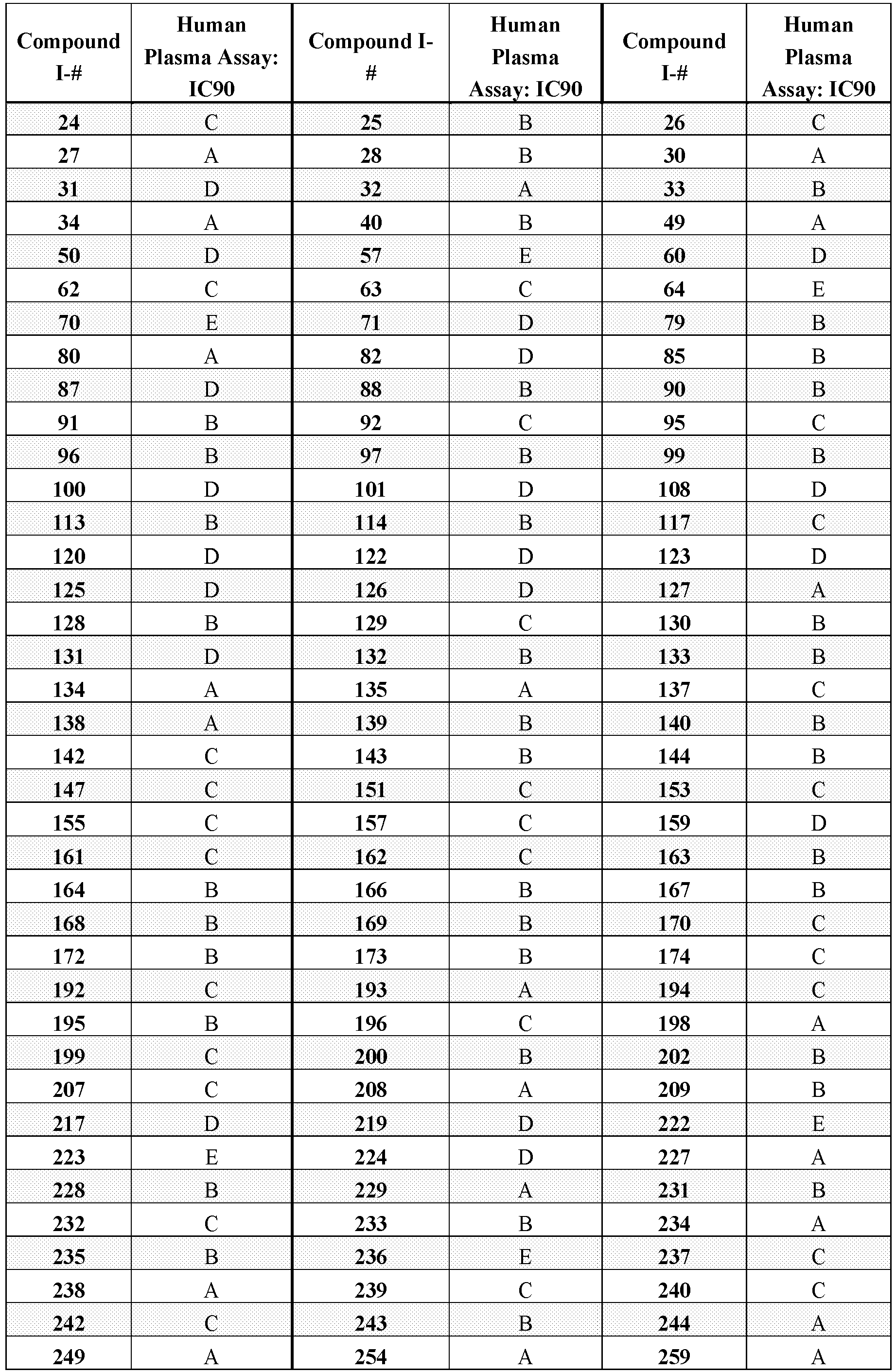

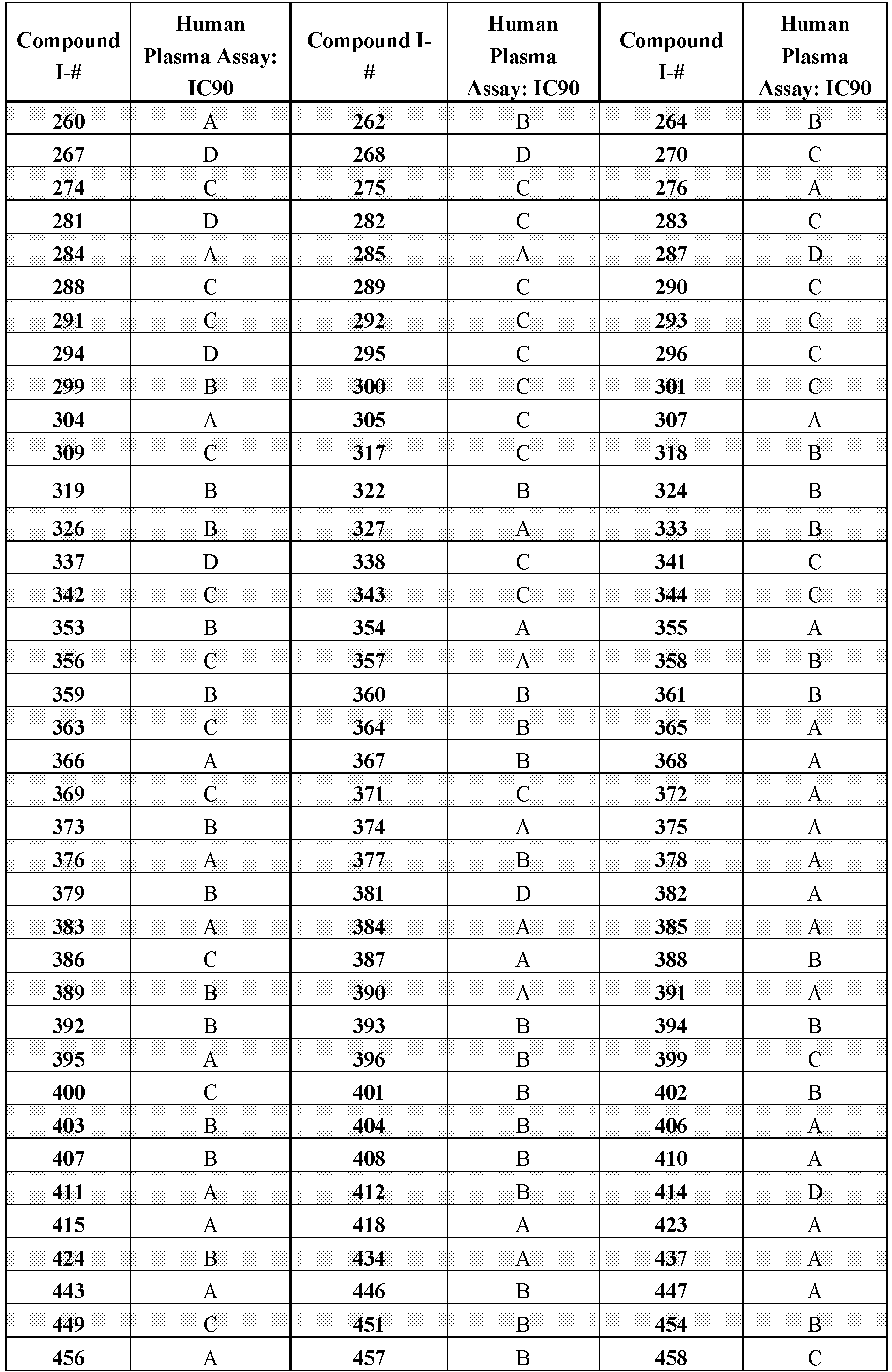

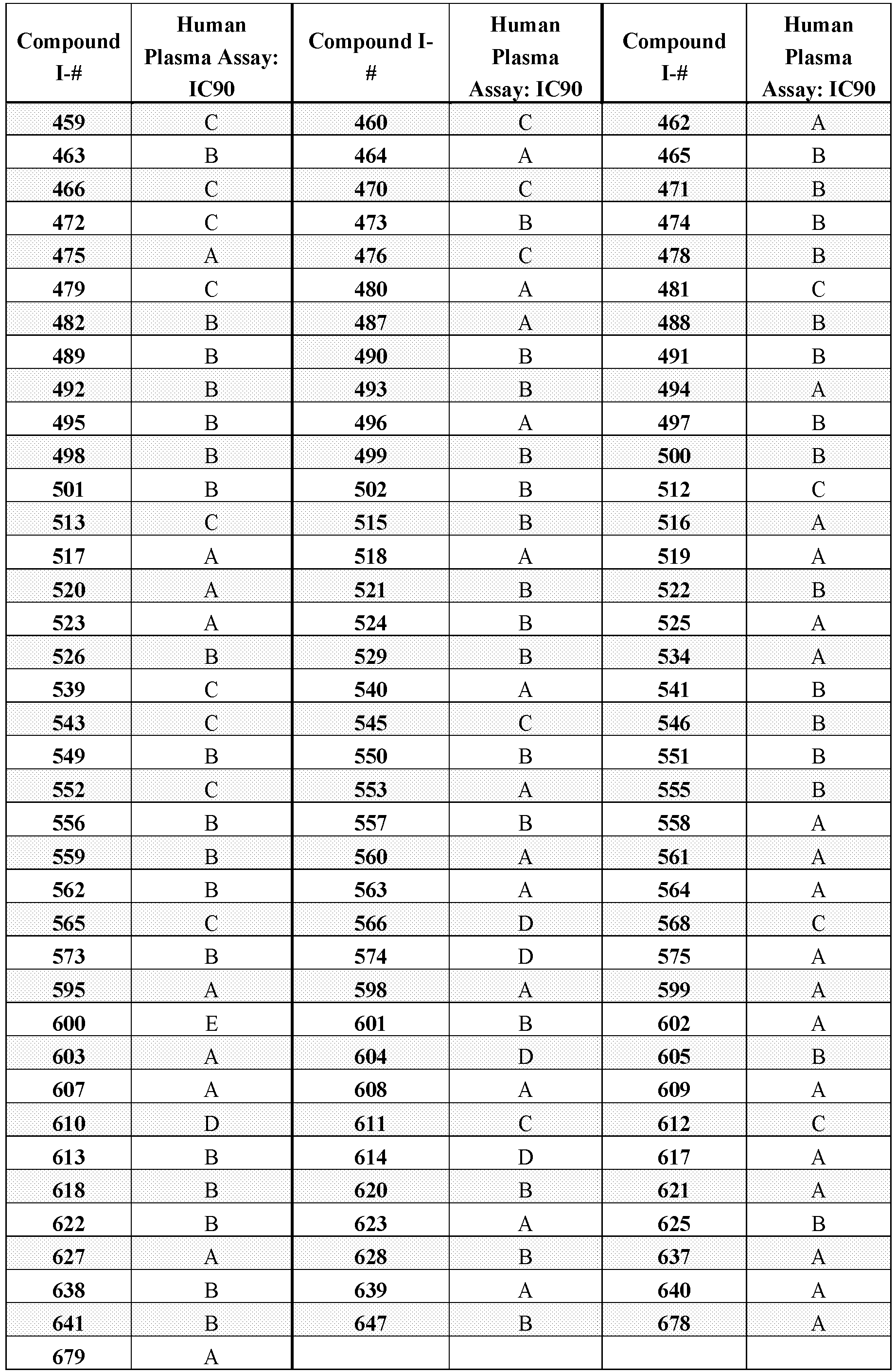

### Example 683

**Comparative Inhibitory Activity of Various Compounds against Plasma Kallikrein.** Table 51 provides comparative plasma kallikrein inhibition potency in the assay format with 500 pM activated kallikrein assay (described in detail in a preceding example). For the compounds listed in Table 51, the EC₅₀ values are reported according to the following ranges: A ≤ 1.0 nM; 1.0 nM < B ≤ 10 nM; 10 nM < C ≤ 100 nM; 100 nM < D ≤ 1000 nM; 1000 nM < E ≤ 6000 nM; 6000 nM < F.

**Table 51**

| **Compound Primary Dose Response Assay: Average EC50** | **Compound Primary Dose Response Assay: Average EC50** |
|---|---|
| 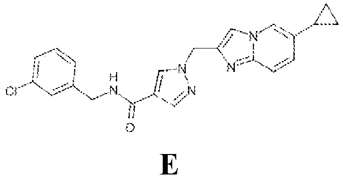 | 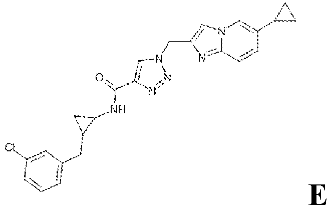 |
| 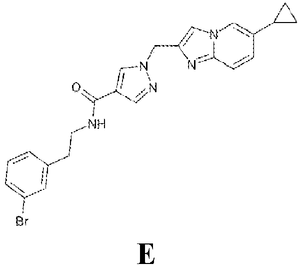 | 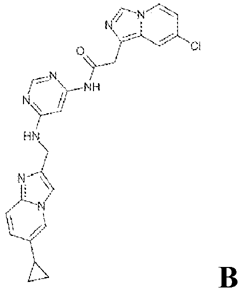 |
| 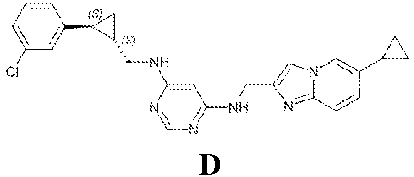 | 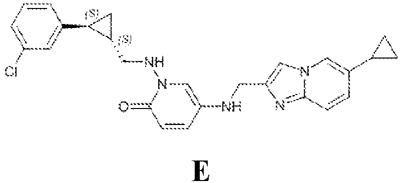 |
| 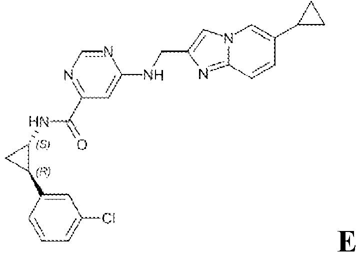 | 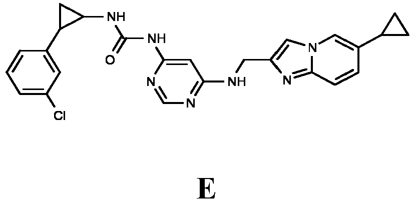 |

### EXEMPLARY ENUMERATED EMBODIMENTS 1

1. A compound of Formula (I): 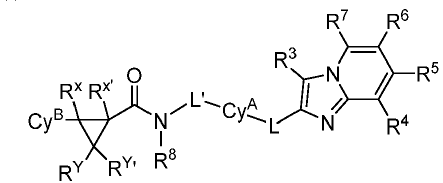
   or a pharmaceutically acceptable salt thereof,
   wherein:
      Cy^{A} is a phenylene or a 5- to 6-membered monocyclic heteroarylene having 1-3 heteroatoms independently selected from oxygen, nitrogen, and sulfur, or a 7- to 12-membered bicyclic heteroarylene having 1-4 heteroatoms independently selected from oxygen, nitrogen, and sulfur, wherein Cy^{A} is substituted with 0-4 -R^{A} groups;
         each R^{A} is independently selected from oxo, halogen, -CN, -C(O)R, -C(O)₂F, -C(O)N(R)₂, -NO₂, - N(R)₂, -N(R)C(O)R, -N(R)C(O)₂R, -N(R)S(O)₂R, -OR, -OC(O)R, -OC(O)N(R)₂, -SR, -S(O)R, - S(O)₂R, -S(O)N(R)₂, -S(O)₂N(R)₂, or an optionally substituted group selected from C₁-₆ aliphatic, phenyl, 3- to 7-membered saturated or partially unsaturated monocyclic carbocyclyl, or 3- to 7-membered saturated or partially unsaturated monocyclic heterocyclyl having 1-2 heteroatoms selected from oxygen, nitrogen, or sulfur;
      each R is independently hydrogen or an optionally substituted C₁₋₆ aliphatic group;
      each R^{Y} and R^{Y}' is independently selected from hydrogen, halogen, and an optionally substituted C₁₋₆ aliphatic group;
      each R^{x} and R^{x}' is independently selected from hydrogen, halogen, or -CN;
      Cy^{B} is selected from phenyl, 8- to 10-membered bicyclic aryl, a 5- to 6-membered heteroaryl having 1-3 heteroatoms independently selected from oxygen, nitrogen, and sulfur, or a 7- to 10-membered heteroaryl having 1-4 heteroatoms independently selected from oxygen, nitrogen, and sulfur, wherein Cy^{B} is substituted with 0-5 -R^{B} groups; or
      Cy^{B} and R^{x}, together with their intervening atoms, form a 6- to 12-membered spirocyclic ring system having 0-4 heteroatoms independently selected from oxygen, nitrogen, and sulfur, wherein the ring or rings formed by Cy^{B} and R^{x} may be substituted with 0-4 -R^{B} groups;
         each R^{B} is independently selected from oxo, halogen, -CN, -C(O)R, -C(O)₂R, -C(O)N(R)₂, -NO₂, - N(R)₂, -N(R)C(O)R, -N(R)C(O)₂R, -N(R)S(O)₂R, -OR, -OC(O)R, -OC(O)N(R)₂, -SR, -S(O)R, - S(O)₂R, -S(O)N(R)₂, -S(O)₂N(R)₂, or an optionally substituted group selected from C₁-₆ aliphatic, a 3- to 7-membered saturated or partially unsaturated monocyclic heterocyclyl having 1-2 heteroatoms selected from oxygen, nitrogen, or sulfur, or a 5- to 6-membered heteroaryl having 1-4 heteroatoms independently selected from oxygen, nitrogen, and sulfur;
      L is an optionally substituted C₁₋₃ hydrocarbon chain, wherein 1 to 3 methylene units are optionally and independently replaced with -O-, -NR^{z}-, -S-, -SO-, or -SO₂-; or L is an optionally substituted 5- to 6-membered saturated or partially unsaturated heterocyclene, having 1-3 heteroatoms independently selected from oxygen, nitrogen, and sulfur;
         each R^{z} is independently selected from hydrogen, -(CH₂)₀₋₃OR, -(CH₂)₀₋₃C(O)OR, or an optionally substituted C₁₋₆ aliphatic group;
      L' is a covalent bond or an optionally substituted C₁₋₃ hydrocarbon chain, wherein 1 to 3 methylene units are optionally and independently replaced with -O-, -NR^{z}-, -S-, -SO-, or SO₂-;
      each R³, R⁴, R⁵, R⁶, and R⁷ is independently selected from hydrogen or -L^{C}-R^{C}, wherein
         each L^{C} is independently selected from a covalent bond or an optionally substituted C₁₋₆ hydrocarbon chain, wherein 1 to 3 methylene units are optionally and independently replaced with -O- or -NR-;
         each R^{C} is independently selected from oxo, halogen, -CN, -C(O)R, -C(O)₂R, -C(O)N(R)₂, -NO₂, -N(R)₂, -N(R)C(O)R, -N(R)C(O)₂R, -N(R)S(O)₂R, -OR, -OC(O)R, - OC(O)N(R)₂, -SR, -S(O)R, -S(O)₂R, -S(O)N(R)₂, -S(O)₂N(R)₂, Cy^{C}, or an optionally substituted group selected from C₁-₆ aliphatic;
            each Cy^{C} is independently selected from a 3- to 7-membered saturated or partially unsaturated monocyclic carbocyclyl, a 3- to 7-membered saturated or partially unsaturated monocyclic heterocyclyl having 1-2 heteroatoms selected from oxygen, nitrogen, or sulfur, a 5- to 6-membered monocyclic heteroaryl having 1-3 heteroatoms independently selected from oxygen, nitrogen, and sulfur, phenyl, a 6- to 12- membered saturated or partially unsaturated fused bicyclic heterocyclyl having 1-3 heteroatoms independently selected from oxygen, nitrogen, or sulfur, a bridged bicycle, or a 6- to 12- membered saturated or partially unsaturated bicyclic spiroheterocyclyl having 1-3 heteroatoms independently selected from oxygen, nitrogen, or sulfur, wherein Cy^{C} is substituted with 0-4 -L^{D}-R^{D} groups;
            each L^{D} is independently selected from a covalent bond or an optionally substituted C₁₋₆ hydrocarbon chain, wherein 1 to 3 methylene units are optionally and independently replaced with -O- or -NR-;
            each R^{D} is independently selected from oxo, halogen, -CN, -C(O)R, -C(O)₂R, -C(O)N(R)₂, -NO₂, -N(R)₂, -N(R)C(O)R, -N(R)C(O)₂R, -N(R)S(O)₂R, -OR, -OC(O)R, -OC(O)N(R)₂, -SR, -S(O)R, -S(O)₂R, -S(O)N(R)₂, -S(O)₂N(R)₂, or an optionally substituted group selected from C₁-₆ aliphatic, phenyl, a 3- to 7-membered saturated or partially unsaturated monocyclic carbocyclyl, or a 3- to 7-membered saturated or partially unsaturated monocyclic heterocyclyl having 1-2 heteroatoms selected from oxygen, nitrogen, or sulfur; and
      R⁸ is selected from hydrogen, -OR, or an optionally substituted C₁₋₆ aliphatic group.
2. The compound of embodiment 1, wherein Cy^{A} is a phenylene, wherein Cy^{A} is substituted with 0-4 -R^{A} groups.
3. The compound of any one of the preceding embodiments, wherein Cy^{A} is a 5- to 6-membered monocyclic heteroarylene having 1-3 heteroatoms independently selected from oxygen, nitrogen, and sulfur, wherein Cy^{A} is substituted with 0-4 -R^{A} groups.
4. The compound of embodiment 3, wherein Cy^{A} is a 6-membered monocyclic heteroarylene having 1-3 heteroatoms independently selected from oxygen, nitrogen, and sulfur, wherein Cy^{A} is substituted with 0-4 -R^{A} groups.
5. The compound of embodiments 3 or 4, wherein Cy^{A} is pyridinediyl, pyrimidinediyl, pyridazinediyl, or triazinediyl substituted with 0-1 R^{A} groups.
6. The compound of embodiment 3, wherein Cy^{A} is a 5-membered monocyclic heteroarylene having 1-3 heteroatoms independently selected from oxygen, nitrogen, and sulfur, wherein Cy^{A} is substituted with 0-2 -R^{A} groups.
7. The compound of embodiment 6, wherein Cy^{A} is unsubstituted thiadiazolediyl, unsubstituted oxadiazolediyl, or unsubstituted triazolediyl.
8. The compound of embodiment 1, wherein Cy^{A} is a 7- to 12-membered bicyclic heteroarylene having 1-4 heteroatoms independently selected from oxygen, nitrogen, and sulfur, wherein Cy^{A} is substituted with 0-4 -R^{A} groups.
9. The compound of embodiment 8, wherein Cy^{A} is a 9-membered bicyclic heteroarylene having 3-4 heteroatoms independently selected from oxygen and nitrogen, or a 10-membered bicyclic heteroarylene having 3-4 heteroatoms independently selected from oxygen and nitrogen, wherein CyA is substituted with 0-1 -R^{A} groups.
10. The compound of embodiment 4, wherein Cy^{A} is selected from the group consisting of: 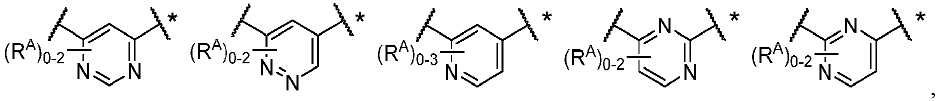 wherein * represents the point of attachment to L.
11. The compound of embodiment 10, wherein Cy^{A} is 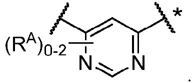
12. The compound of embodiment 10, wherein Cy^{A} is 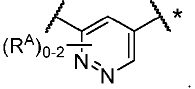
13. The compound of embodiment 10, wherein Cy^{A} is 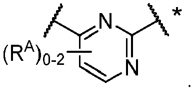
14. The compound of embodiment 6, wherein Cy^{A} is selected from the group consisting of: 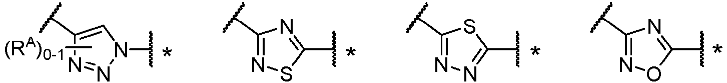 wherein * represents the point of attachment to L.
15. The compound of any one of the preceding embodiments, wherein each R^{A} is independently selected from oxo, halogen, -CN, -C(O)₂R, -N(R)₂, -OR, -SR, -S(O)R, -S(O)₂R, or an optionally substituted group selected from C₁₋₆ aliphatic, 3- to 7-membered saturated or partially unsaturated monocyclic carbocyclyl, or 3- to 7-membered saturated or partially unsaturated monocyclic heterocyclyl having 1-2 heteroatoms selected from oxygen, nitrogen, or sulfur.
16. The compound of any one of the preceding embodiments, wherein Cy^{A} is substituents on an optionally substituted R^{A} group are independently halogen, -(CH₂)₀₋₄OR°, or -(CH₂)₀₋₄N(R°)₂, wherein each R° is independently as defined above and described in classes and subclasses herein.
17. The compound of any one of the preceding embodiments, wherein Cy^{B} is selected from phenyl, a 5- to 6-membered heteroaryl having 1-3 heteroatoms independently selected from oxygen, nitrogen, and sulfur or a 7- to 10-membered heteroaryl having 1-4 heteroatoms independently selected from oxygen, nitrogen, and sulfur, wherein Cy^{B} is substituted with 0-4 -R^{B} groups.
18. The compound of any one of the preceding embodiments, wherein Cy^{B} is phenyl or a 5- to 6-membered heteroaryl having 1-3 heteroatoms independently selected from oxygen, nitrogen, and sulfur, wherein Cy^{B} is substituted with 0-4 -R^{B} groups.
19. The compound of any one of the preceding embodiments, wherein Cy^{B} is phenyl, wherein Cy^{B} is substituted with 0-4 -R^{B} groups.
20. The compound of any one of embodiments 1-18, wherein Cy^{B} is a 6-membered heteroaryl having 1-3 nitrogens, wherein Cy^{B} is substituted with 0-4 -R^{B} groups.
21. The compound of embodiment 20, wherein Cy^{B} a pyrimidinyl group substituted with 0-2 -R^{B} groups, a pyridinyl group substituted with 0-2 -R^{B} groups, a pyrazinyl group substituted with 0-1 -R^{B} groups, a pyridazinyl group substituted with 0-1 -R^{B} groups, or a 1,3,5-triazinyl group substituted with 0-1 -R^{B} groups.
22. The compound of any one of embodiments 1-18, wherein Cy^{B} is a 5-membered heteroaryl having 1-2 heteroatoms independently selected from oxygen, nitrogen, and sulfur, wherein Cy^{B} is substituted with 0-4 -R^{B} groups.
23. The compound of embodiment 22, wherein Cy^{B} is a thienyl group substituted with 0-2 -R^{B} groups, a thiazolyl group substituted with 0-1 -R^{B} groups, or a thiadiazolyl group substituted with 0-1 - R^{B} groups.
24. The compound of any one of embodiments 1-18, wherein Cy^{B} is selected from the group consisting of: 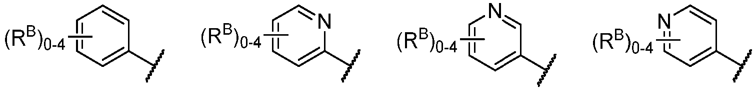 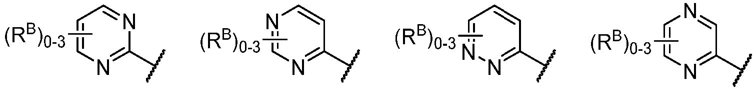 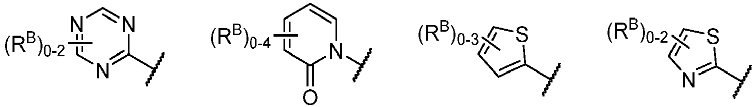 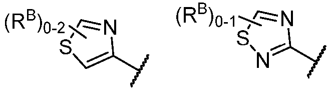
25. The compound of any one of embodiments 1-18, wherein Cy^{B} is: 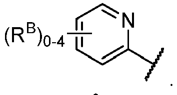
26. The compound of any one of embodiments 1-18, wherein Cy^{B} is 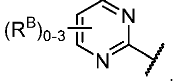
27. The compound of any one of embodiments 1-18, wherein Cy^{B} is 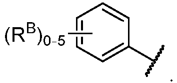
28. The compound of any one of embodiments 1-16, wherein Cy^{B} and R^{x}, together with their intervening atoms, form a 6- to 12-membered spirocyclic ring system having 0-4 heteroatoms independently selected from oxygen, nitrogen, and sulfur, wherein the ring or rings formed by Cy^{B} and R^{x} may be substituted with 0-4 -R^{B} groups.
29. The compound of embodiment 28, wherein Cy^{B} and R^{x}, together with their intervening atoms, form a 6- to 12-membered spirocyclic ring system selected from: 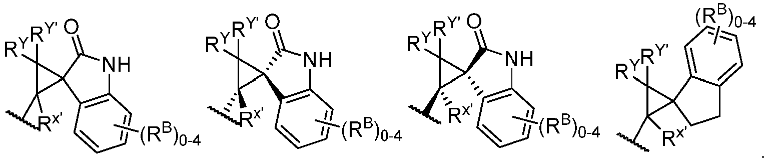
30. The compound of any one of the preceding embodiments, wherein each R^{B} is independently selected from oxo, halogen, -CN, -NO₂, -N(R)₂, -N(R)C(O)₂R, -OR, or an optionally substituted group selected from C1-6 aliphatic or a 5-membered heteroaryl having 1-4 heteroatoms independently selected from oxygen, nitrogen, and sulfur.
31. The compound of any one of the preceding embodiments, wherein substituents on an optionally substituted R^{B} group are independently selected from oxo, halogen, and -(CH₂)₀₋₄OR°, wherein R° is as defined above and described in classes and subclasses herein.
32. The compound of any one of the preceding embodiments, wherein each of R^{x} and R^{x}' is independently selected from hydrogen and halogen.
33. The compound of any one of the preceding embodiments, wherein each of R^{x} and R^{x}' is hydrogen.
34. The compound of any one of the preceding embodiments, wherein one of R^{x} and R^{x}' is hydrogen and the other is halogen.
35. The compound of any one of the preceding embodiments, wherein each of R^{Y} and R^{Y}' is independently selected from hydrogen and halogen.
36. The compound of any one of the preceding embodiments, wherein each of R^{Y} and R^{Y}' is hydrogen.
37. The compound of any one of the preceding embodiments, wherein L is an optionally substituted C₁₋₃ hydrocarbon chain, wherein 1-3 methylene units are optionally replaced with -O-, -NR^{z}-, -S-, or - SO₂-.
38. The compound of any one of the preceding embodiments, wherein L is an optionally substituted C₁₋₃ hydrocarbon chain, wherein 1 methylene unit is optionally replaced with -O-, -NR^{z}-, -S-, or -SO₂-.
39. The compound of any one of the preceding embodiments, wherein L is an optionally substituted C₁ hydrocarbon chain.
40. The compound of embodiment 39, wherein Cy^{A} is bicyclic.
41. The compound of any one of the preceding embodiments, wherein L is an optionally substituted C₂ hydrocarbon chain, wherein 1 methylene unit is optionally replaced with -NR^{z}- or -O-.
42. The compound of any one of the preceding embodiments, wherein L is an optionally substituted C₂ hydrocarbon chain, wherein the methylene unit connected to Cy^{A} is replaced with -NR^{z}- or -O-.
43. The compound of any one of the preceding embodiments, wherein L is an optionally substituted C₂ hydrocarbon chain, wherein the methylene unit connected to Cy^{A} is replaced with -NR^{z}-.
44. The compound of embodiment 43, wherein L is *-NHCH(Me)-, wherein * represents the point of attachment to Cy^{A}.
45. The compound of embodiment 43, wherein L is *-NHCH₂-, wherein * represents the point of attachment to Cy^{A}.
46. The compound of any one of embodiments 1-42, wherein L is an optionally substituted C₂ hydrocarbon chain, wherein the methylene unit connected to Cy^{A} is replaced with -O-.
47. The compound of embodiment 46, wherein L is *-OCH(Me)-, wherein * represents the point of attachment to Cy^{A}.
48. The compound of embodiment 46, wherein L is *-OCH₂-, wherein * represents the point of attachment to Cy^{A}.
49. The compound of any one of the preceding embodiments, wherein L comprises a two-atom spacer between Cy^{A} and 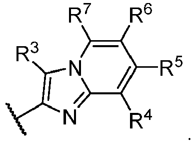
50. The compound of any one of the preceding embodiments, wherein L is an optionally substituted 5-membered saturated or partially unsaturated heterocyclene, having 1 heteroatom independently selected from oxygen, nitrogen, and sulfur.
51. The compound of any one of the preceding embodiments, wherein L is optionally substituted 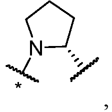 wherein * represents the point of attachment to Cy^{A}.
52. The compound of any one of embodiments 41-51, wherein Cy^{A} is monocyclic.
53. The compound of any one of the preceding embodiments, wherein optional substituents on L are independently selected from -(CH₂)₀₋₄R°, -(CH₂)₀₋₄OR°, -(CH₂)₀₋₄OC(O)R°, and -(CH₂)₀₋₄N(R°)₂, wherein each R° is independently as defined above and described in classes and subclasses herein.
54. The compound of any one of the preceding embodiments, wherein L' is a covalent bond or a methylene unit optionally substituted with -(CH₂)₀₋₄R°, wherein R° is independently as defined above and described in classes and subclasses herein.
55. The compound of any one of the preceding embodiments, wherein L' is a covalent bond.
56. The compound of any one of the preceding embodiments, wherein each of R³, R⁴, R⁵, R⁶, and R⁷ is independently selected from hydrogen or L^{C}-R^{C}, wherein each L^{C} is independently selected from a covalent bond or an optionally substituted C₁₋₆ hydrocarbon chain, wherein 1 to 3 methylene units are optionally and independently replaced with -O- or -NR-; and wherein each R^{C} is independently selected from halogen, -CN, -C(O)R, -C(O)₂R, -C(O)N(R)₂, -N(R)₂, -N(R)C(O)R, -N(R)C(O)₂R, -N(R)S(O)₂R, - S(O)₂R, -S(O)₂N(R)₂, Cy^{C}, or an optionally substituted group selected from C₁-₆ aliphatic
57. The compound of any one of the preceding embodiments, wherein R³ is selected from hydrogen or L^{C}-R^{C}, wherein L^{C} is a covalent bond and R^{C} is halogen.
58. The compound of any one of the preceding embodiments, wherein R³ is hydrogen.
59. The compound of any one of the preceding embodiments, wherein R⁴ is selected from hydrogen or L^{C}-R^{C}, wherein L^{C} is selected from a covalent bond or an optionally substituted _{C1-6} hydrocarbon chain, wherein 1 to 3 methylene units are optionally and independently replaced with -O- or -NR-; and wherein R^{C} is selected from halogen, -CN, -C(O)R, -C(O)₂R, -C(O)N(R)₂, -N(R)₂, -N(R)C(O)R, - N(R)C(O)₂R, -N(R)S(O)₂R, -OR, -S(O)₂R, -S(O)₂N(R)₂, Cy^{C}, or an optionally substituted group selected from C₁₋₆ aliphatic.
60. The compound of any one of the preceding embodiments, wherein L^{C} is a covalent bond, R^{C} is Cy^{C} and Cy^{C} is a 5-membered saturated or partially unsaturated monocyclic heterocyclyl having 1-2 heteroatoms selected from oxygen, nitrogen, or sulfur.
61. The compound of any one of the preceding embodiments, wherein R⁴ is selected from the group consisting of: 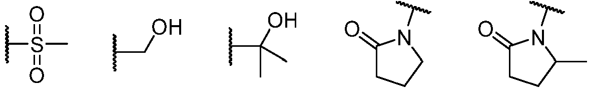 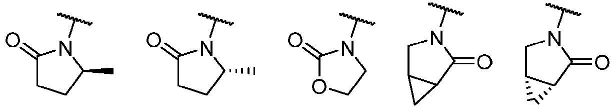 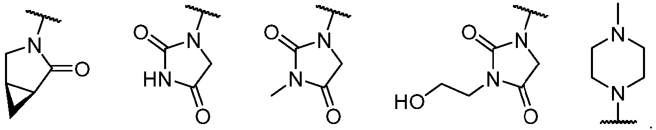
62. The compound of any one of the preceding embodiments, wherein optional substituents on a C₁₋₆ aliphatic group of R⁴ are selected from -(CH₂)₀₋₄R°, -(CH₂)₀₋₄OR°, -CN, -(CH₂)₀₋₄N(R°)₂, and -(CH₂)₀₋₄C(O)OR°, wherein each R° is independently as defined above and described in classes and subclasses herein.
63. The compound of any one of the preceding embodiments, wherein Cy^{C} is selected from a 3- to 7-membered saturated or partially unsaturated monocyclic heterocyclyl having 1-2 heteroatoms selected from oxygen, nitrogen, or sulfur, a 5- to 6-membered monocyclic heteroaryl having 1-3 heteroatoms independently selected from oxygen, nitrogen, and sulfur, a 6- to 12- membered saturated or partially unsaturated fused bicyclic heterocyclyl having 1-3 heteroatoms independently selected from oxygen, nitrogen, or sulfur, a bridged bicycle, or a 6- to 12- membered saturated or partially unsaturated bicyclic spiroheterocyclyl having 1-3 heteroatoms independently selected from oxygen, nitrogen, or sulfur, wherein Cy^{C} is substituted with 0-4 -L^{D}-R^{D} groups.
64. The compound of any one of the preceding embodiments, wherein R^{D} is selected from oxo, halogen, -C(O)₂R, -N(R)₂, -OR, or an optionally substituted group selected from C₁-₆ aliphatic, phenyl, a 3- to 7-membered saturated or partially unsaturated monocyclic carbocyclyl, or a 3- to 7-membered saturated or partially unsaturated monocyclic heterocyclyl having 1-2 heteroatoms selected from oxygen, nitrogen, or sulfur.
65. The compound of any one of the preceding embodiments, wherein optional substituents on R^{D} are selected from halogen, -(CH₂)₀₋₄R°, -(CH₂)₀₋₄OR°, -(CH₂)₀₋₄N(R°)₂, -(CH₂)₀₋₄C(O)OR°, and - OP(O)(OR°)₂, wherein each R° is independently as defined above and described in classes and subclasses herein.
66. The compound of any one of the preceding embodiments, wherein L^{D} is a covalent bond.
67. The compound of any one of the preceding embodiments, wherein R⁵ is hydrogen.
68. The compound of any one of embodiments1-66, wherein R⁵ is L^{C}-R^{C}, wherein L^{C} is a covalent bond and R^{C} is Cy^{C}.
69. The compound of any one of the preceding embodiments, wherein Cy^{C} is a cyclopropyl group.
70. The compound of any one of the preceding embodiments, wherein R⁶ is selected from hydrogen or L^{C}-R^{C}, wherein L^{C} is a covalent bond, and wherein R^{C} is selected from halogen, -N(R)₂, -OR, Cy^{C}, or an optionally substituted C₁₋₆ aliphatic group.
71. The compound of any one of the preceding embodiments, wherein Cy^{C} is a cyclopropyl group substituted with 0-4 L^{D}-R^{D} groups.
72. The compound of any one of the preceding embodiments, wherein L^{C} is a covalent bond, and wherein R^{C} is an optionally substituted C₁₋₆ aliphatic group.
73. The compound of any one of the preceding embodiments, wherein Cy^{C} is an unsubstituted cyclopropyl group.
74. The compound of any one of the preceding embodiments, wherein L^{D} is a covalent bond and R^{D} is selected from halogen and optionally substituted C₁₋₆ aliphatic.
75. The compound of any one of the preceding embodiments, wherein R⁷ is selected from hydrogen or L^{C}-R^{C}, wherein L^{C} is a covalent bond, and wherein R^{C} is Cy^{C}.
76. The compound of any one of the preceding embodiments, wherein R⁷ is hydrogen.
77. The compound of any one of the preceding embodiments, wherein Cy^{C} is: 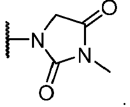
78. The compound of any one of the preceding embodiments, wherein R⁸ is hydrogen.
79. The compound of any one of embodiments1-77, wherein R⁸ is selected from -OR or an optionally substituted C₁₋₆ aliphatic group.
80. The compound of any one of the preceding embodiments, wherein the compound is of Formula (II): 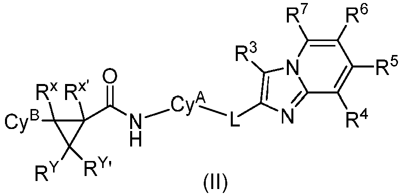 or a pharmaceutically acceptable salt thereof.
81. The compound of any one of the preceding embodiments, wherein the compound is of Formula (III-a), Formula (III-b), or Formula (III-c): 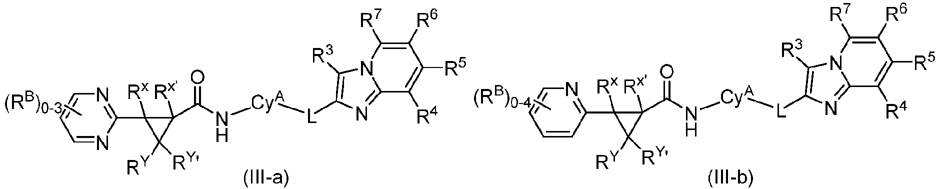 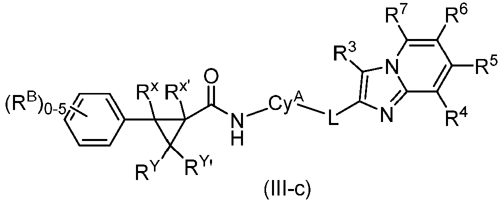 or a pharmaceutically acceptable salt thereof.
82. The compound of any one of the preceding embodiments, wherein the compound is of Formula (IV-a), Formula (IV-b), Formula (IV-c), Formula (IV-d), Formula (IV-e), or Formula (IV-e): 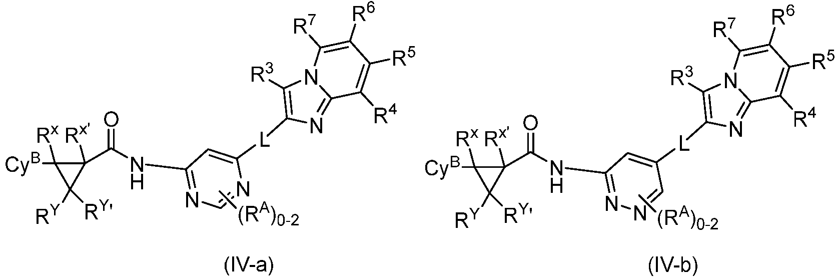 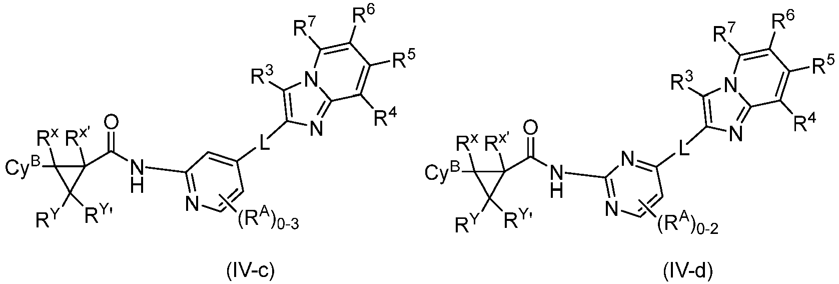 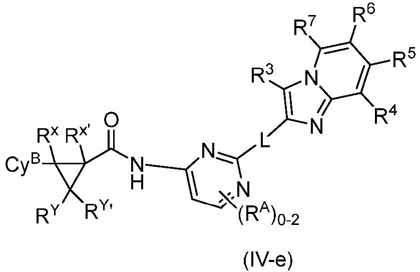 or a pharmaceutically acceptable salt thereof.
83. The compound of any one of the preceding embodiments, wherein the compound is of Formula (V-a), Formula (V-b), or Formula (V-c): 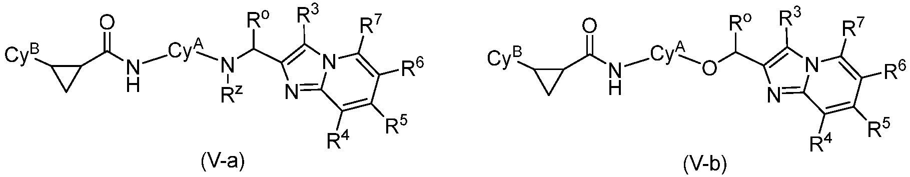 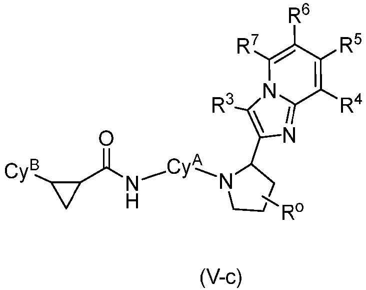 or a pharmaceutically acceptable salt thereof.
84. The compound of any one of the preceding embodiments, wherein the compound is of Formula (V-a-1), Formula (V-b-1), or Formula (V-c-1): 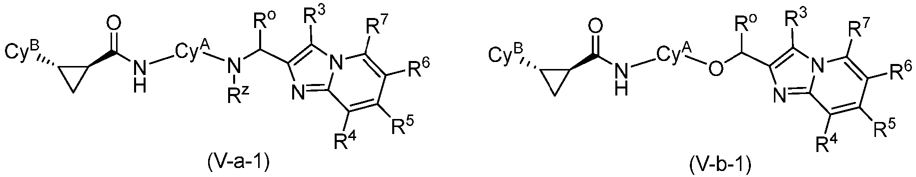 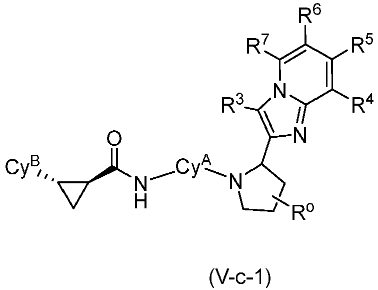 or a pharmaceutically acceptable salt thereof.
85. The compound of any one of the preceding embodiments, wherein the compound is of Formula (VI-a), Formula (VI-b), or Formula (VI-c): 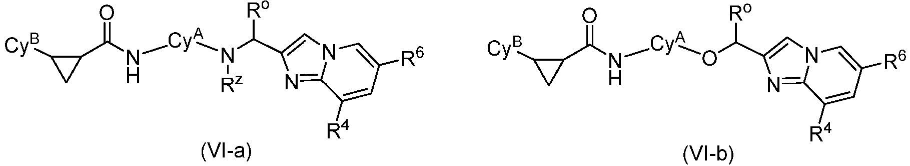 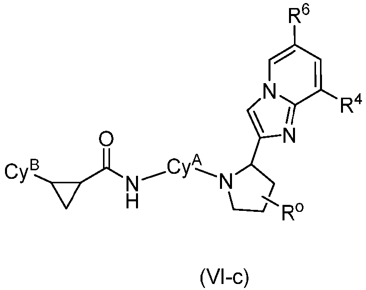
   or a pharmaceutically acceptable salt thereof,
   wherein each of Cy^{A}, Cy^{B}, R^{z}, and R° is defined and described in classes and subclasses herein, both singly and in combination;
   R⁴ is L^{C}-R^{C}, wherein L^{C} is a covalent bond and R^{C} is Cy^{C}, wherein Cy^{C} is a 5-membered saturated or partially unsaturated monocyclic heterocyclyl having 1-2 heteroatoms selected from nitrogen, and wherein Cy^{C} is substituted with 0-4 -L^{D}-R^{D} groups;
   R⁶ is L^{C}-R^{C}, wherein L^{C} is a covalent bond and R^{C} is C₁₋₆ aliphatic or Cy^{C}, wherein Cy^{C} is cyclopropyl optionally substituted with halogen.
86. The compound of any one of the preceding embodiments, wherein R⁴ is a ring selected from: 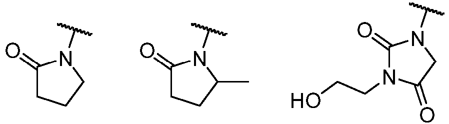 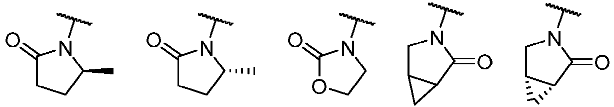 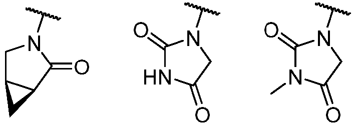
87. The compound of any one of the preceding embodiments, wherein R⁴ is a ring selected from: 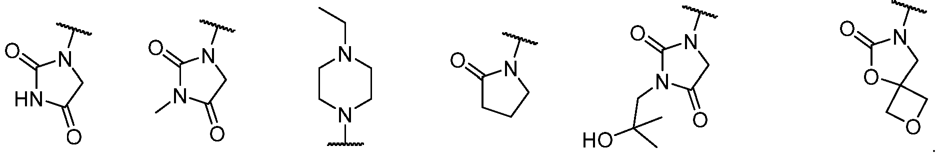
88. The compound of any one of the preceding embodiments, wherein the moiety: 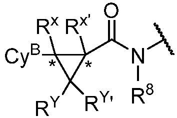 (including where one or more of R^{x}, R^{x}', R^{Y}, or R^{Y}' is hydrogen) is in the relative trans configuration with respect to the Cy^{B} and amide group attached to the two stereocenters marked with an *.
89. The compound of embodiment 88, wherein the compound is a racemic mixture with respect to the two stereocenters marked with an *.
90. The compound of any one of embodiments 1-88, wherein the absolute stereochemistry of the moiety: 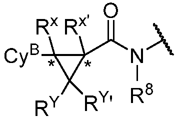 (including where one or more of R^{x}, R^{x}', R^{Y}, or R^{Y}' is hydrogen) is as follows: 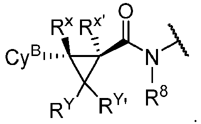
91. The compound of any one of embodiments 1-88, wherein the absolute stereochemistry of the moiety: 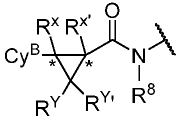 (including where one or more of R^{x}, R^{x}', R^{Y}, or R^{Y}' is hydrogen) is as follows: 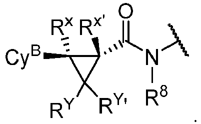
92. The compound of any one of the preceding embodiments, wherein each R° is independently selected from hydrogen, C₁₋₆ aliphatic, or -OH.
93. The compound of any one of the preceding embodiments, wherein R° of L is methyl.
94. The compound of any one of the preceding embodiments, wherein the compound is selected from compounds I-1 through I-679, or a pharmaceutically acceptable salt thereof.
95. A pharmaceutical composition comprising a compound of any one of the preceding embodiments.
96. The pharmaceutical composition comprising a compound of any one of the preceding embodiments, further comprising a pharmaceutically acceptable excipient.
97. The composition of embodiment 95 or 96, wherein the composition is suitable for oral administration.
98. A method of treating a plasma kallikrein-mediated disease or disorder using a compound or composition of any one of the preceding embodiments.
99. The method of embodiment 98, wherein the disease or disorder is hereditary angioedema.
100. The method of embodiment 98, wherein the disease or disorder is diabetic macular edema.
101. A method of treating hereditary angioedema comprising administering to a patient in need thereof a compound or composition of any one of the preceding embodiments.
102. A method of treating diabetic macular edema comprising administering to a patient in need thereof a compound or composition of any one of the preceding embodiments.
103. The method of any one of embodiment 99 or 101, wherein administration of the compound partially or completely inhibits, delays onset of, reduces severity of, and/or reduces incidence of one or more symptoms, features, and/or causes of a hereditary angioedema.
104. The method of embodiment 103, wherein the compound is administered orally.

### EXEMPLARY ENUMERATED EMBODIMENTS 2

1. A compound of Formula (**I**): 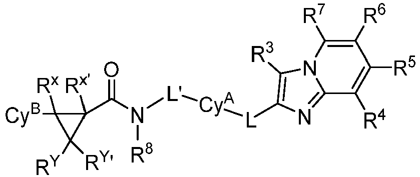
   or a pharmaceutically acceptable salt thereof,
   wherein:
      **Cy**^{A} is a phenylene or a 5- to 6-membered monocyclic heteroarylene having 1-3 heteroatoms independently selected from oxygen, nitrogen, and sulfur, or a 7- to 12-membered bicyclic heteroarylene having 1-4 heteroatoms independently selected from oxygen, nitrogen, and sulfur, wherein Cy^{A} is substituted with 0-4 -R^{A} groups;
         each **R^{A}** is independently selected from oxo, halogen, -CN, -C(O)R, -C(O)₂R, -C(O)N(R)₂, -NO₂, - N(R)₂, -N(R)C(O)R, -N(R)C(O)₂R, -N(R)S(O)₂R, -OR, -OC(O)R, -OC(O)N(R)₂, -SR, -S(O)R, - S(O)₂R, -S(O)N(R)₂, -S(O)₂N(R)₂, or an optionally substituted group selected from C₁-₆ aliphatic, phenyl, 3- to 7-membered saturated or partially unsaturated monocyclic carbocyclyl, or 3- to 7-membered saturated or partially unsaturated monocyclic heterocyclyl having 1-2 heteroatoms selected from oxygen, nitrogen, or sulfur;
      each **R** is independently hydrogen or an optionally substituted C₁₋₆ aliphatic group;
      each **R^{Y}** and **R^{Y}'** is independently selected from hydrogen, halogen, and an optionally substituted C₁₋₆ aliphatic group;
      each **R^{x}** and **R^{x}'** is independently selected from hydrogen, halogen, or -CN;
      **Cy^{B}** is selected from phenyl, 8- to 10-membered bicyclic aryl, a 5- to 6-membered heteroaryl having 1-3 heteroatoms independently selected from oxygen, nitrogen, and sulfur, or a 7- to 10-membered heteroaryl having 1-4 heteroatoms independently selected from oxygen, nitrogen, and sulfur, wherein Cy^{B} is substituted with 0-5 -R^{B} groups; or
      **Cy^{B}** and **R^{x},** together with their intervening atoms, form a 6- to 12-membered spirocyclic ring system having 0-4 heteroatoms independently selected from oxygen, nitrogen, and sulfur, wherein the ring or rings formed by Cy^{B} and R^{x} may be substituted with 0-4 -R^{B} groups;
         each **R^{B}** is independently selected from oxo, halogen, -CN, -C(O)R, -C(O)₂R, -C(O)N(R)₂, -NO₂, - N(R)₂, -N(R)C(O)R, -N(R)C(O)₂R, -N(R)S(O)₂R, -OR, -OC(O)R, -OC(O)N(R)₂, -SR, -S(O)R, - S(O)₂R, -S(O)N(R)₂, -S(O)₂N(R)₂, or an optionally substituted group selected from C₁-₆ aliphatic, a 3- to 7-membered saturated or partially unsaturated monocyclic heterocyclyl having 1-2 heteroatoms selected from oxygen, nitrogen, or sulfur, or a 5- to 6-membered heteroaryl having 1-4 heteroatoms independently selected from oxygen, nitrogen, and sulfur;
      **L** is an optionally substituted C₁₋₃ hydrocarbon chain, wherein 1 to 3 methylene units are optionally and independently replaced with -O-, -NR^{z}-, -S-, -SO-, or -SO₂-; or L is an optionally substituted 5- to 6-membered saturated or partially unsaturated heterocyclene, having 1-3 heteroatoms independently selected from oxygen, nitrogen, and sulfur;
         each R^{z} is independently selected from hydrogen, -(CH₂)₀₋₃OR, -(CH₂)₀₋₃C(O)OR, or an optionally substituted C₁₋₆ aliphatic group;
      **L'** is a covalent bond or an optionally substituted C₁₋₃ hydrocarbon chain, wherein 1 to 3 methylene units are optionally and independently replaced with -O-, -NR^{z}-, -S-, -SO-, or SO₂-;
      each **R³, R⁴, R⁵, R⁶,** and **R⁷** is independently selected from hydrogen or -L^{C}-R^{C}, wherein
         each **L^{C}** is independently selected from a covalent bond or an optionally substituted C₁₋₆ hydrocarbon chain, wherein 1 to 3 methylene units are optionally and independently replaced with -O- or -NR-;
         each **R^{C}** is independently selected from oxo, halogen, -CN, -C(O)R, -C(O)₂R, -C(O)N(R)₂, -NO₂, -N(R)₂, -N(R)C(O)R, -N(R)C(O)₂R, -N(R)S(O)₂R, -OR, -OC(O)R, -OC(O)N(R)₂, -SR, -S(O)R, -S(O)₂R, -S(O)N(R)₂, -S(O)₂N(R)₂, Cy^{C}, or an optionally substituted group selected from C₁-₆ aliphatic;
            each **Cy^{C}** is independently selected from a 3- to 7-membered saturated or partially unsaturated monocyclic carbocyclyl, a 3- to 7-membered saturated or partially unsaturated monocyclic heterocyclyl having 1-2 heteroatoms selected from oxygen, nitrogen, or sulfur, a 5- to 6-membered monocyclic heteroaryl having 1-3 heteroatoms independently selected from oxygen, nitrogen, and sulfur, phenyl, a 6- to 12- membered saturated or partially unsaturated fused bicyclic heterocyclyl having 1-3 heteroatoms independently selected from oxygen, nitrogen, or sulfur, a bridged bicycle, or a 6- to 12- membered saturated or partially unsaturated bicyclic spiroheterocyclyl having 1-3 heteroatoms independently selected from oxygen, nitrogen, or sulfur, wherein Cy^{C} is substituted with 0-4 -L^{D}-R^{D} groups;
            each **L^{D}** is independently selected from a covalent bond or an optionally substituted C₁₋₆ hydrocarbon chain, wherein 1 to 3 methylene units are optionally and independently replaced with -O- or -NR-;
            each **R^{D}** is independently selected from oxo, halogen, -CN, -C(O)R, -C(O)₂R, -C(O)N(R)₂, -NO₂, -N(R)₂, -N(R)C(O)R, -N(R)C(O)₂R, -N(R)S(O)₂R, -OR, -OC(O)R, -OC(O)N(R)₂, -SR, -S(O)R, -S(O)₂R, -S(O)N(R)₂, -S(O)₂N(R)₂, or an optionally substituted group selected from C₁-₆ aliphatic, phenyl, a 3- to 7-membered saturated or partially unsaturated monocyclic carbocyclyl, or a 3- to 7-membered saturated or partially unsaturated monocyclic heterocyclyl having 1-2 heteroatoms selected from oxygen, nitrogen, or sulfur; and
      **R⁸** is selected from hydrogen, -OR, or an optionally substituted C₁₋₆ aliphatic group.
2. The compound of claim 1, wherein L' is a covalent bond.
3. The compound of claim 1 or 2, wherein the compound is of Formula (II): 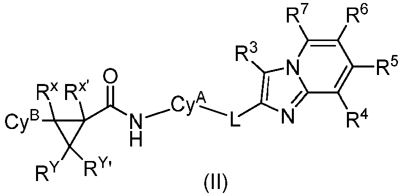 or a pharmaceutically acceptable salt thereof, wherein R³, R⁴, R⁵, R⁶, R⁷, R^{X}, R^{X}', R^{Y}, R^{Y}', Cy^{A}, Cy^{B} and L are defined for compounds of formula (I).
4. The compound of claim 1 or 2, wherein the compound is of Formula (III-a), Formula (III-b), or Formula (III-c): 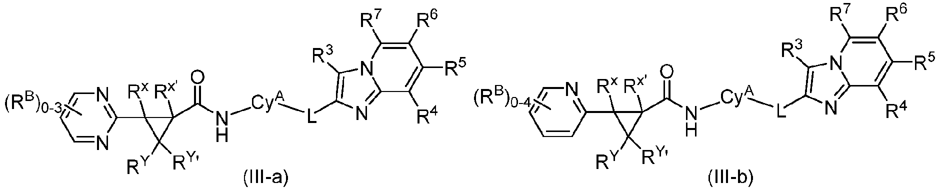 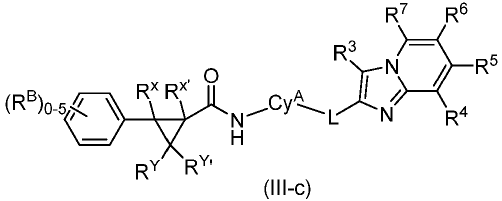
   or a pharmaceutically acceptable salt thereof wherein R³, R⁴, R⁵, R⁶, R⁷, R^{B}, R^{X}, R^{X}', R^{Y}, R^{Y'},
   Cy^{A}, and L are defined for compounds of formula (I).
5. The compound of claim 1, wherein the compound is of Formula (IV-a), Formula (IV-b), Formula (IV-c), Formula (IV-d), Formula (IV-e), or Formula (IV-e): 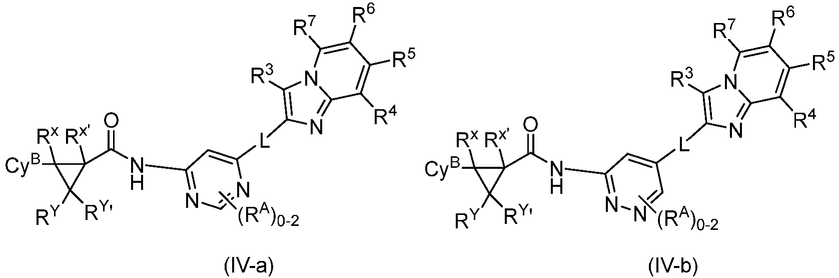 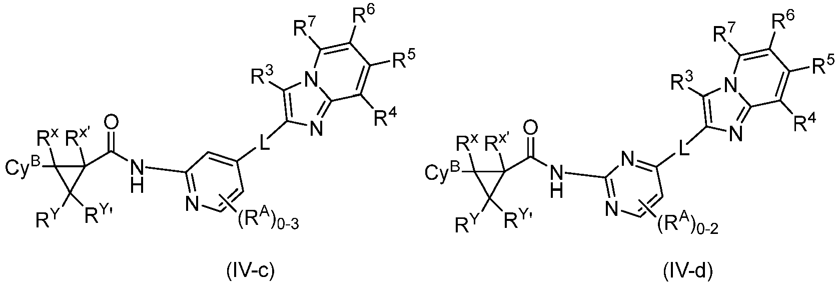 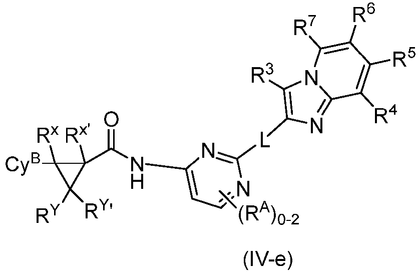 or a pharmaceutically acceptable salt thereof wherein R³, R⁴, R⁵, R⁶, R⁷, R^{X}, R^{X}', R^{Y}, R^{Y}', R^{A} and Cy^{B} are defined for compounds of formula (I).
6. The compound of claim 1 or 2, wherein the compound is of Formula (V-a), Formula (V-b), or Formula (V-c): 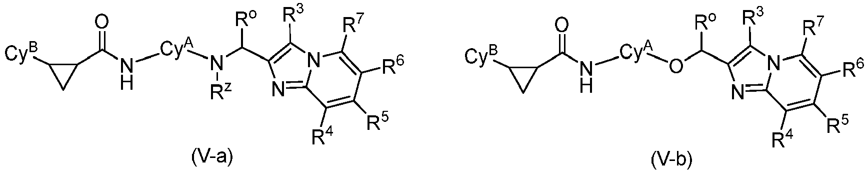 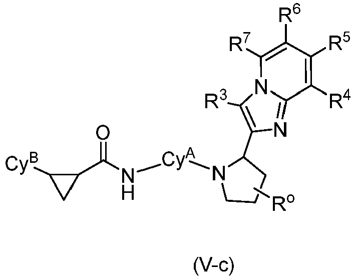 or a pharmaceutically acceptable salt thereof wherein R³, R⁴, R⁵, R⁶, R⁷, R^{X}, R^{X}', R^{Y}, R^{Y}', Cy^{A} and Cy^{B} are defined for compounds of formula (I).
7. The compound of claim 1, wherein the compound is of Formula (V-a-1), Formula (V-b-1), or Formula (V-c-1): 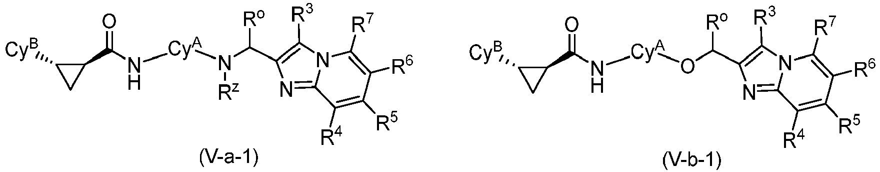 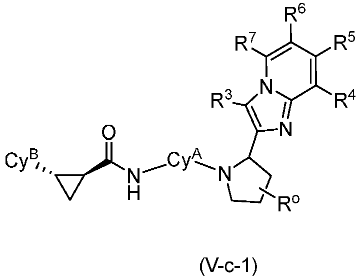 or a pharmaceutically acceptable salt thereof wherein R³, R⁴, R⁵, R⁶, R⁷, Cy^{A} and Cy^{B} are defined for compounds of formula (I), and R° is hydrogen or C₁₋₆ aliphatic.
8. The compound of claim 7, wherein the compound is of Formula (VI-a), Formula (VI-b), or Formula (VI-c): 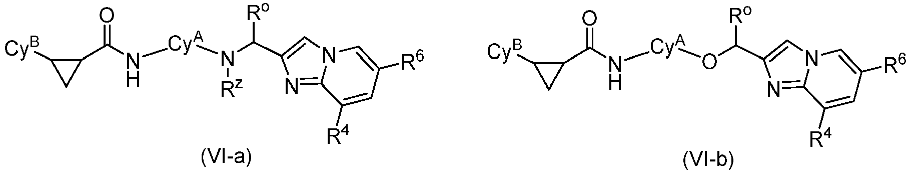 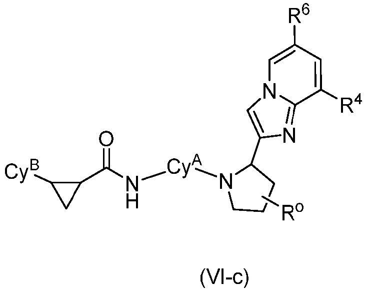
   or a pharmaceutically acceptable salt thereof
   wherein each of Cy^{A}, Cy^{B}, R^{z}, and R° is defined for compounds of formula (V);
   R⁴ is L^{C}-R^{C}, wherein L^{C} is a covalent bond and R^{C} is Cy^{C}, wherein Cy^{C} is a 5-membered saturated or partially unsaturated monocyclic heterocyclyl having 1-2 heteroatoms selected from nitrogen, and wherein Cy^{C} is substituted with 0-4 -L^{D}-R^{D} groups;
   R⁶ is L^{C}-R^{C}, wherein L^{C} is a covalent bond and R^{C} is C₁₋₆ aliphatic or Cy^{C}, wherein Cy^{C} is cyclopropyl optionally substituted with halogen.
9. The compound of any one of the claims 1 to 5, wherein L is an optionally substituted C₁₋₃ hydrocarbon chain, wherein 1-3 methylene units are optionally replaced with -O-, -NR^{z}-, -S-, or -SO₂-.
10. The compound of any one of claims 1-5, wherein L is *-NHCH(Me)-, wherein * represents the point of attachment to Cy^{A}.
11. The compound of any one of claims 1-5, wherein L is *-NHCH₂-, wherein * represents the point of attachment to Cy^{A}.
12. The compound of any one of claims 1-5, wherein L is *-OCH(Me)-, wherein * represents the point of attachment to Cy^{A}.
13. The compound of any one of claims 1-5, wherein L is *-OCH₂-, wherein * represents the point of attachment to Cy^{A}.
14. The compound of any one of claims 1-5, wherein L comprises a two-atom spacer between Cy^{A} and 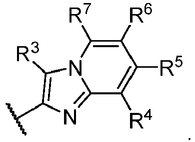
15. The compound of any one of claims 1 to 4, 6 and 8-14, wherein Cy^{A} is a 5- to 6-membered monocyclic heteroarylene having 1-3 heteroatoms independently selected from oxygen, nitrogen, and sulfur (for example a pyrimidinediyl or pyridinediyl, such as pyrimidinediyl), wherein Cy^{A} is substituted with 0-4 -R^{A} groups (for example 0 or 1).
16. The compound of claim 15, wherein Cy^{A} is selected from the group consisting of: 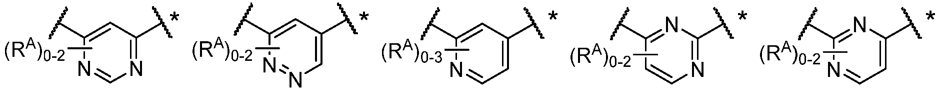 wherein * represents the point of attachment to L.
17. The compound of any one of claims 1 to 4, 6 and 8 to 14, wherein Cy^{A} is selected from the group consisting of: 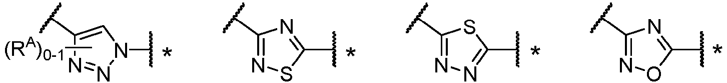 wherein * represents the point of attachment to L.
18. The compound of any one of the preceding claims, wherein each R^{A} is independently selected from oxo, halogen, -CN, -C(O)₂R, -N(R)₂, -OR, -SR, -S(O)R, -S(O)₂R, or an optionally substituted group selected from C₁₋₆ aliphatic, 3- to 7-membered saturated or partially unsaturated monocyclic carbocyclyl, or 3- to 7-membered saturated or partially unsaturated monocyclic heterocyclyl having 1-2 heteroatoms selected from oxygen, nitrogen, or sulfur, in particular an optionally substituted group selected from C₁₋₆ aliphatic, such as methyl.
19. The compound of any one of the preceding claims, wherein Cy^{B} is selected from phenyl, a 5- to 6-membered heteroaryl having 1-3 heteroatoms independently selected from oxygen, nitrogen, and sulfur or a 7- to 10-membered heteroaryl having 1-4 heteroatoms independently selected from oxygen, nitrogen, and sulfur, wherein Cy^{B} is substituted with 0-4 -R^{B} groups (for example 0 or 1 groups) 5- to 6-membered heteroaryl having 1-3 heteroatoms independently selected from oxygen, nitrogen, and sulfur (such as pyridine or pyrimidine, in particular pyrimidine) substituted with 0-4 -R^{B} groups.
20. The compound of any one of the preceding claims, wherein Cy^{B} is selected from the group consisting of: 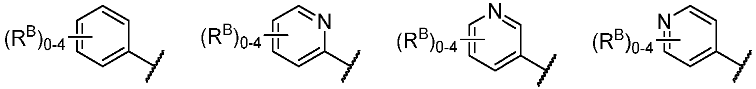 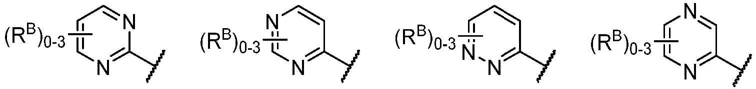 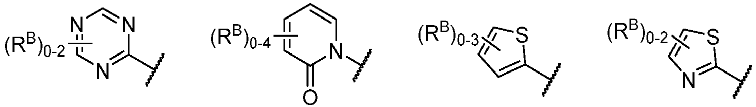 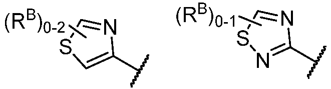
21. The compound of any one of the preceding claims, wherein each R^{B} is independently selected from oxo, halogen, -CN, -NO₂, -N(R)₂, -N(R)C(O)₂R, -OR, or an optionally substituted group selected from C₁₋₆ aliphatic or a 5-membered heteroaryl having 1-4 heteroatoms independently selected from oxygen, nitrogen, and sulfur, for example where the optionally substituted group is selected from C₁₋₆ aliphatic, such as methyl.
22. The compound of any one of the preceding claims, wherein each of R^{x} and/or R^{x'} is hydrogen.
23. The compound of any one of the preceding claims, wherein each of R^{Y} and/or R^{Y'} is hydrogen.
24. The compound of any one of the preceding claims, wherein each of R³, R⁴, R⁵, R⁶, and R⁷ is independently selected from hydrogen or L^{C}-R^{C}, wherein each L^{C} is independently selected from a covalent bond or an optionally substituted C₁₋₆ hydrocarbon chain, wherein 1 to 3 methylene units are optionally and independently replaced with -O- or -NR-; and wherein each R^{C} is independently selected from halogen, -CN, -C(O)R, -C(O)₂R, -C(O)N(R)₂, -N(R)₂, -N(R)C(O)R, -N(R)C(O)₂R, -N(R)S(O)₂R, -S(O)₂R, -S(O)₂N(R)₂, Cy^{C}, or an optionally substituted group selected from C₁₋₆ aliphatic.
25. The compound of any one of the preceding claims, wherein R³ is hydrogen.
26. The compound of any one of the preceding claims, wherein R⁴ is selected from the group consisting of: 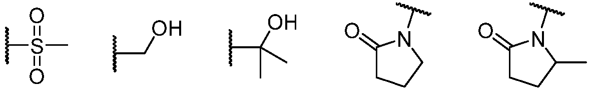 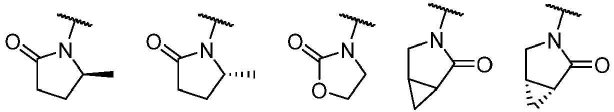 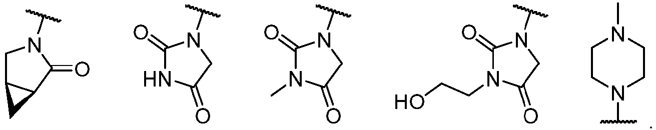
27. The compound of any one of the preceding claims, wherein R⁵ is hydrogen
28. The compound of any one of the preceding claims, wherein R⁶ is selected from hydrogen or L^{C}-R^{C}, wherein L^{C} is a covalent bond, and wherein R^{C} is selected from halogen, -N(R)₂, -OR, Cy^{C}, or an optionally substituted C₁₋₆ aliphatic group.
29. The compound of claim 27, wherein Cy^{C} is an unsubstituted cyclopropyl group.
30. The compound of any one of the preceding claims, wherein R⁷ is hydrogen.
31. The compound of claim 1, wherein the compound is selected from those in Table A.
32. A pharmaceutical composition comprising a compound of any one of the preceding claims.
33. A method of treating a plasma kallikrein-mediated disease or disorder using a compound or composition of any one of the preceding claims.
34. The method of claim 33, wherein the disease or disorder is hereditary angioedema or diabetic macular edema.

## Claims

1. A compound of Formula **(I):** 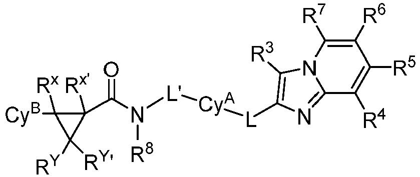
or a pharmaceutically acceptable salt thereof,
wherein:
**Cy^{A}** is a 5- to 6-membered monocyclic heteroarylene having 1-3 heteroatoms independently selected from oxygen, nitrogen, and sulfur, or a 7- to 12-membered bicyclic heteroarylene having 1-4 heteroatoms independently selected from oxygen, nitrogen, and sulfur, wherein Cy^{A} is substituted with 0-4 -R^{A} groups;
each **R^{A}** is independently selected from oxo, halogen, -CN, -C(O)R, -C(O)₂R, -C(O)N(R)₂, -NO₂, - N(R)₂, -N(R)C(O)R, -N(R)C(O)₂R, -N(R)S(O)₂R, -OR, -OC(O)R, -OC(O)N(R)₂, -SR, -S(O)R, - S(O)₂R, -S(O)N(R)₂, -S(O)₂N(R)₂, or an optionally substituted group selected from C₁₋₆ aliphatic, phenyl, 3- to 7-membered saturated or partially unsaturated monocyclic carbocyclyl, or 3- to 7-membered saturated or partially unsaturated monocyclic heterocyclyl having 1-2 heteroatoms selected from oxygen, nitrogen, or sulfur;
each **R** is independently hydrogen or an optionally substituted C₁₋₆ aliphatic group;
each **R^{Y}** and **R**^{**Y**'} is independently selected from hydrogen, halogen, and an optionally substituted C₁₋₆ aliphatic group;
each **R^{x}** and **R^{x}**' is independently selected from hydrogen, halogen, or -CN;
**Cy^{B}** is selected from phenyl, 8- to 10-membered bicyclic aryl, a 5- to 6-membered heteroaryl having 1-3 heteroatoms independently selected from oxygen, nitrogen, and sulfur, or a 7- to 10-membered heteroaryl having 1-4 heteroatoms independently selected from oxygen, nitrogen, and sulfur, wherein Cy^{B} is substituted with 0-5 -R^{B} groups; or
**Cy^{B}** and **R^{x},** together with their intervening atoms, form a 6- to 12-membered spirocyclic ring system having 0-4 heteroatoms independently selected from oxygen, nitrogen, and sulfur, wherein the ring or rings formed by Cy^{B} and R^{X} may be substituted with 0-4 -R^{B} groups;
each **R^{B}** is independently selected from oxo, halogen, -CN, -C(O)R, -C(O)₂R, -C(O)N(R)₂, -NO₂, - N(R)₂, -N(R)C(O)R, -N(R)C(O)₂R, -N(R)S(O)₂R, -OR, -OC(O)R, -OC(O)N(R)₂, -SR, -S(O)R, - S(O)₂R, -S(O)N(R)₂, -S(O)₂N(R)₂, or an optionally substituted group selected from C₁₋₆ aliphatic, a 3- to 7-membered saturated or partially unsaturated monocyclic heterocyclyl having 1-2 heteroatoms selected from oxygen, nitrogen, or sulfur, or a 5- to 6-membered heteroaryl having 1-4 heteroatoms independently selected from oxygen, nitrogen, and sulfur;
**L** is an optionally substituted C₁₋₃ hydrocarbon chain, wherein 1 to 3 methylene units are optionally and independently replaced with -O-, -NR^{z}-, -S-, -SO-, or -SO₂-; or L is an optionally substituted 5- to 6-membered saturated or partially unsaturated heterocyclene, having 1-3 heteroatoms independently selected from oxygen, nitrogen, and sulfur;
each R^{z} is independently selected from hydrogen, -(CH₂)₀₋₃OR, -(CH₂)₀₋₃C(O)OR, or an optionally substituted C₁₋₆ aliphatic group;
**L'** is a covalent bond or an optionally substituted C₁₋₃ hydrocarbon chain, wherein 1 to 3 methylene units are optionally and independently replaced with -O-, -NR^{z}-, -S-, -SO-, or SO₂-;
each **R³**, **R⁴**, **R⁵**, **R⁶**, and **R⁷** is independently selected from hydrogen or -L^{C}-R^{C}, wherein
each **L^{C}** is independently selected from a covalent bond or an optionally substituted C₁₋₆ hydrocarbon chain, wherein 1 to 3 methylene units are optionally and independently replaced with -O- or -NR-;
each **R^{C}** is independently selected from oxo, halogen, -CN, -C(O)R, -C(O)₂R, -C(O)N(R)₂, -NO₂, -N(R)₂, -N(R)C(O)R, -N(R)C(O)₂R, -N(R)S(O)₂R, -OR, -OC(O)R, -OC(O)N(R)₂, -SR, -S(O)R, -S(O)₂R, -S(O)N(R)₂, -S(O)₂N(R)₂, Cy^{C}, or an optionally substituted group selected from C₁₋₆ aliphatic;
each **Cy^{C}** is independently selected from a 3- to 7-membered saturated or partially unsaturated monocyclic carbocyclyl, a 3- to 7-membered saturated or partially unsaturated monocyclic heterocyclyl having 1-2 heteroatoms selected from oxygen, nitrogen, or sulfur, a 5- to 6-membered monocyclic heteroaryl having 1-3 heteroatoms independently selected from oxygen, nitrogen, and sulfur, phenyl, a 6- to 12- membered saturated or partially unsaturated fused bicyclic heterocyclyl having 1-3 heteroatoms independently selected from oxygen, nitrogen, or sulfur, a bridged bicycle, or a 6- to 12- membered saturated or partially unsaturated bicyclic spiroheterocyclyl having 1-3 heteroatoms independently selected from oxygen, nitrogen, or sulfur, wherein Cy^{C} is substituted with 0-4 -L^{D}-R^{D} groups;
each **L^{D}** is independently selected from a covalent bond or an optionally substituted C₁₋₆ hydrocarbon chain, wherein 1 to 3 methylene units are optionally and independently replaced with -O- or -NR-;
each **R^{D}** is independently selected from oxo, halogen, -CN, -C(O)R, -C(O)₂R, -C(O)N(R)₂, -NO₂, -N(R)₂, -N(R)C(O)R, -N(R)C(O)₂R, -N(R)S(O)₂R, -OR, -OC(O)R, -OC(O)N(R)₂, -SR, -S(O)R, -S(O)₂R, -S(O)N(R)₂, -S(O)₂N(R)₂, or an optionally substituted group selected from C₁₋₆ aliphatic, phenyl, a 3- to 7-membered saturated or partially unsaturated monocyclic carbocyclyl, or a 3- to 7-membered saturated or partially unsaturated monocyclic heterocyclyl having 1-2 heteroatoms selected from oxygen, nitrogen, or sulfur; and
**R⁸** is selected from hydrogen, -OR, or an optionally substituted C₁₋₆ aliphatic group, wherein
L comprises a two-atom spacer between Cy^{A} and

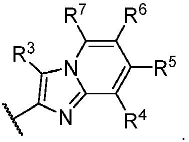

2. The compound of claim 1, wherein L' is a covalent bond.

3. The compound of claim 1 or 2, wherein the compound is of: Formula (II): 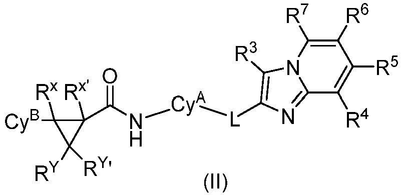
wherein R³, R⁴, R⁵, R⁶, R⁷, R^{X}, R^{X}', R^{Y}, R^{Y'}, Cy^{A}, Cy^{B} and L are defined for formula (I);
Formula (III-a), Formula (III-b), or Formula (III-c): 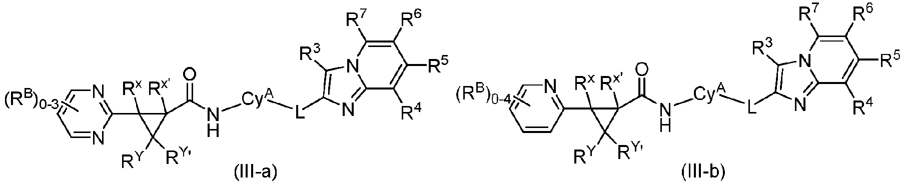 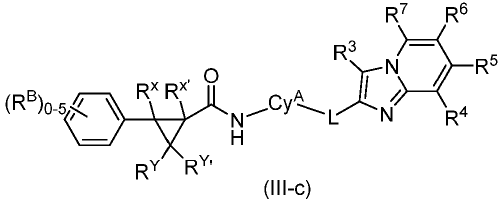
wherein R³, R⁴, R⁵, R⁶, R⁷, R^{B}, R^{X}, R^{X}', R^{Y}, R^{Y'}, Cy^{A}, and L are defined for formula (I);
Formula (IV-a), Formula (IV-b), Formula (IV-c), Formula (IV-d), Formula (IV-e), or Formula (IV-e): 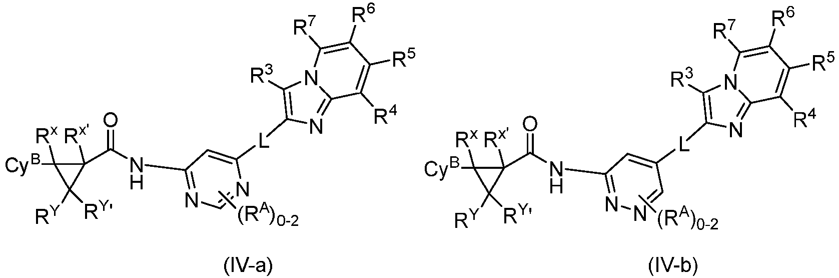 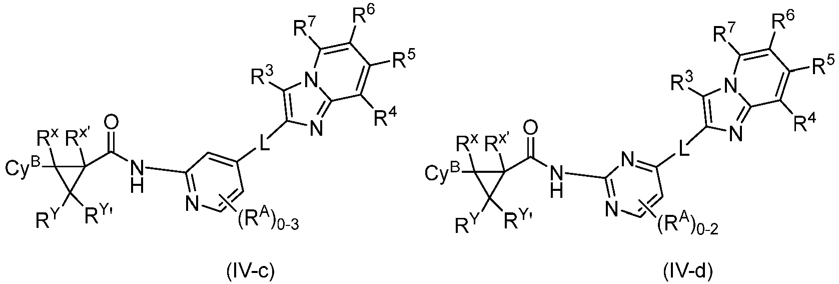 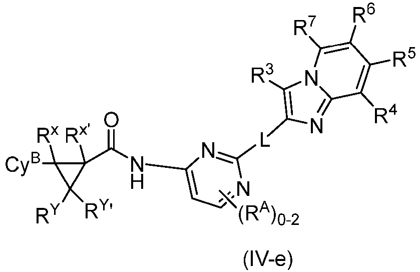
wherein R³, R⁴, R⁵, R⁶, R⁷, R^{X}, R^{X}', R^{Y}, R^{Y'}, R^{A} and Cy^{B} are defined for formula (I):
Formula (V-a), Formula (V-b), or Formula (V-c): 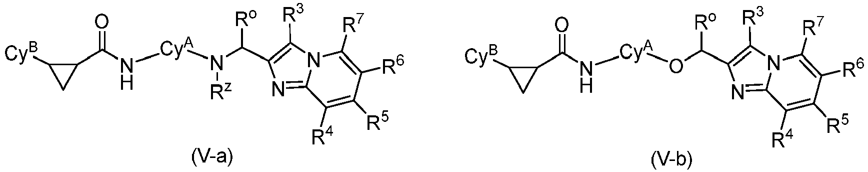 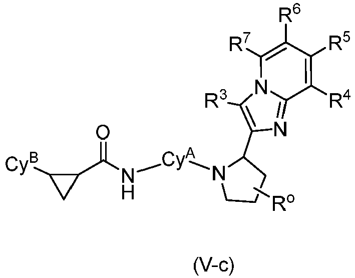
wherein R³, R⁴, R⁵, R⁶, R⁷, R^{X}, R^{X}', R^{Y}, R^{Y'}, Cy^{A} and Cy^{B} are defined for formula (I);
Formula (V-a-1), Formula (V-b-1), or Formula (V-c-1): 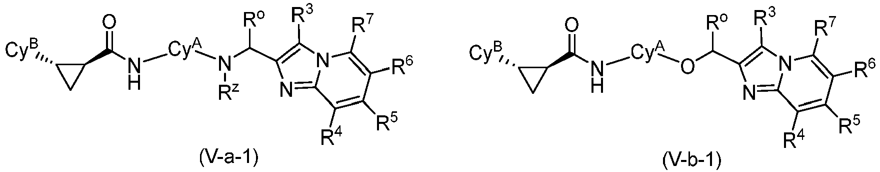 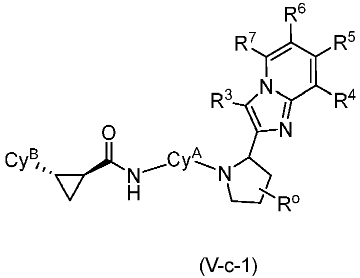
wherein R³, R⁴, R⁵, R⁶, R⁷, Cy^{A} and Cy^{B} are defined for formula (I), and R° is hydrogen or C₁₋₆ aliphatic.
Formula (VI-a), Formula (VI-b), or Formula (VI-c): 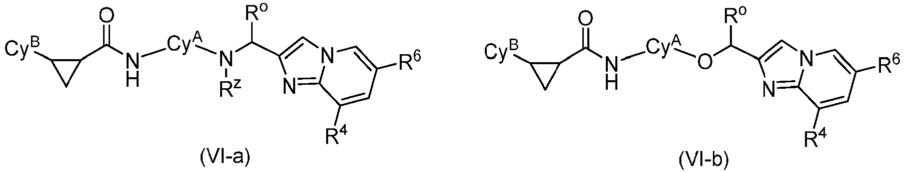 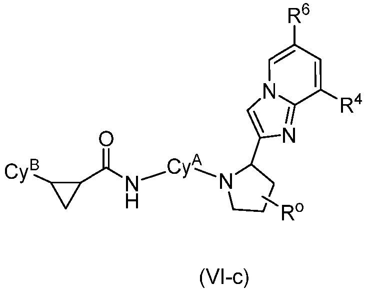
wherein each of Cy^{A}, Cy^{B}, R^{z}, and R° is defined for compounds of formula (V);
R⁴ is L^{C}-R^{C}, wherein L^{C} is a covalent bond and R^{C} is Cy^{C}, wherein Cy^{C} is a 5-membered saturated or partially unsaturated monocyclic heterocyclyl having 1-2 heteroatoms selected from nitrogen, and
wherein Cy^{C} is substituted with 0-4 -L^{D}-R^{D} groups;
R⁶ is L^{C}-R^{C}, wherein L^{C} is a covalent bond and R^{C} is C₁₋₆ aliphatic or Cy^{C}, wherein Cy^{C} is cyclopropyl optionally substituted with halogen; or
a pharmaceutically acceptable salt thereof.

4. The compound of any one of the claims 1 to 3, wherein L is an optionally substituted C₁₋₃ hydrocarbon chain, wherein 1-3 methylene units are optionally replaced with -O-, -NR^{z}-, -S-, or -SO₂-.

5. The compound of any one of claims 1 to 3, wherein L is *-NHCH(Me)-, wherein * represents the point of attachment to Cy^{A}.

6. The compound of any one of claims 1 to 3, wherein L is *-NHCH₂-, wherein * represents the point of attachment to Cy^{A}.

7. The compound of any one of claims 1 to 3, wherein L is *-OCH(Me)-, wherein * represents the point of attachment to Cy^{A}.

8. The compound of any one of claims 1 to 3 wherein L is *-OCH₂-, wherein * represents the point of attachment to Cy^{A}.

9. The compound of any one of claims 1 to 8, wherein Cy^{A} is a 5- to 6-membered monocyclic heteroarylene having 1-3 heteroatoms independently selected from oxygen, nitrogen, and sulfur (for example a pyrimidinediyl or pyridinediyl, such as pyrimidinediyl), wherein Cy^{A} is substituted with 0-4 -R^{A} groups (for example 0 or 1).

10. The compound of claim 9, wherein Cy^{A} is selected from the group consisting of: 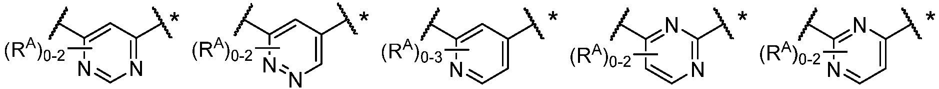 wherein * represents the point of attachment to L.

11. The compound of any one of claims 1 to 10, wherein Cy^{A} is selected from the group consisting of: 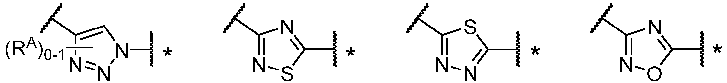 wherein * represents the point of attachment to L.

12. The compound of any one of the preceding claims, wherein each R^{A} is independently selected from oxo, halogen, -CN, -C(O)₂R, -N(R)₂, -OR, -SR, -S(O)R, -S(O)₂R, or an optionally substituted group selected from C₁₋₆ aliphatic, 3- to 7-membered saturated or partially unsaturated monocyclic carbocyclyl, or 3- to 7-membered saturated or partially unsaturated monocyclic heterocyclyl having 1-2 heteroatoms selected from oxygen, nitrogen, or sulfur, in particular an optionally substituted group selected from C₁₋₆ aliphatic, such as methyl.

13. The compound of any one of the preceding claims, wherein Cy^{B} is selected from phenyl, a 5- to 6-membered heteroaryl having 1-3 heteroatoms independently selected from oxygen, nitrogen, and sulfur or a 7- to 10-membered heteroaryl having 1-4 heteroatoms independently selected from oxygen, nitrogen, and sulfur, wherein Cy^{B} is substituted with 0-4 -R^{B} groups (for example 0 or 1 groups) 5- to 6-membered heteroaryl having 1-3 heteroatoms independently selected from oxygen, nitrogen, and sulfur (such as pyridine or pyrimidine, in particular pyrimidine) substituted with 0-4 -R^{B} groups.

14. The compound of any one of the preceding claims, wherein Cy^{B} is selected from the group consisting of:

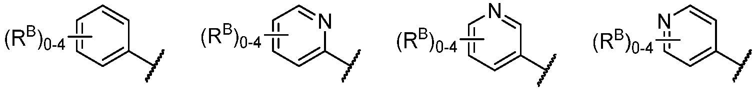

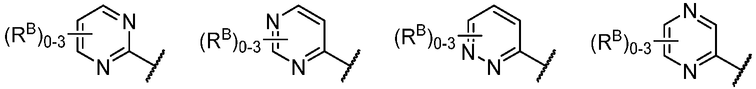

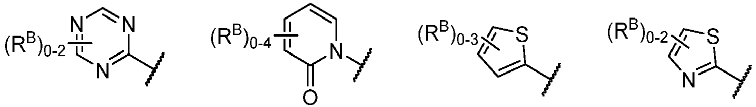

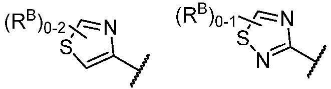

15. The compound of any one of the preceding claims, wherein each R^{B} is independently selected from oxo, halogen, -CN, -NO₂, -N(R)₂, -N(R)C(O)₂R, -OR, or an optionally substituted group selected from C₁₋₆ aliphatic or a 5-membered heteroaryl having 1-4 heteroatoms independently selected from oxygen, nitrogen, and sulfur, for example where the optionally substituted group is selected from C₁₋₆ aliphatic, such as methyl.

16. The compound of any one of the preceding claims, wherein each of R^{X} and/or R^{x'} is hydrogen.

17. The compound of any one of the preceding claims, wherein each of R^{Y} and/or R^{Y'} is hydrogen.

18. The compound of any one of the preceding claims, wherein each of R³, R⁴, R⁵, R⁶, and R⁷ is independently selected from hydrogen or L^{C}-R^{C}, wherein each L^{C} is independently selected from a covalent bond or an optionally substituted C₁₋₆ hydrocarbon chain, wherein 1 to 3 methylene units are optionally and independently replaced with -O- or -NR-; and wherein each R^{C} is independently selected from halogen, -CN, -C(O)R, -C(O)₂R, -C(O)N(R)₂, -N(R)₂, -N(R)C(O)R, -N(R)C(O)₂R, -N(R)S(O)₂R, -S(O)₂R, -S(O)₂N(R)₂, Cy^{C}, or an optionally substituted group selected from C₁₋₆ aliphatic.

19. The compound of any one of the preceding claims, wherein R³ is hydrogen.

20. The compound of any one of the preceding claims, wherein R⁴ is selected from the group consisting of:

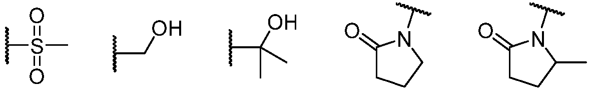

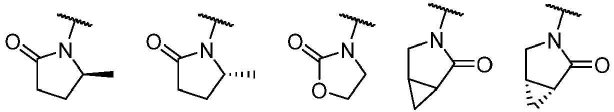

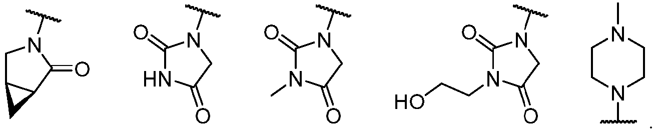

21. The compound of any one of the preceding claims, wherein R⁵ is hydrogen

22. The compound of any one of the preceding claims, wherein R⁶ is selected from hydrogen or L^{C}-R^{C}, wherein L^{C} is a covalent bond, and wherein R^{C} is selected from halogen, -N(R)₂, -OR, Cy^{C}, or an optionally substituted C₁₋₆ aliphatic group.

23. The compound of any one of the preceding claims, wherein R⁷ is hydrogen.

24. The compound of claim 1, wherein the compound is selected from those in Table A.

25. A pharmaceutical composition comprising a compound of any one of the preceding claims.

26. A method of treating a plasma kallikrein-mediated disease or disorder using a compound or composition of any one of the preceding claims.

27. The method of claim 26, wherein the disease or disorder is hereditary angioedema or diabetic macular edema.
